(19) Europäisches Patentamt
European Patent Office
Office européen des brevets

(11) EP 1 375 517 A1

# (12) EUROPEAN PATENT APPLICATION

(43) Date of publication:
**02.01.2004 Bulletin 2004/01**

(51) Int Cl.[7]: **C07K 14/72**, G06F 19/00

(21) Application number: **03076899.8**

(22) Date of filing: **17.06.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **21.06.2002 US 390610 P**

(71) Applicant: **SMITHKLINE BEECHAM CORPORATION**
**Philadelphia Pennsylvania 19101 (US)**

(72) Inventors:
- **Bledsoe, Randy K., c/o GlaxoSmithKline Research Triangle Park, NC 27709 (US)**
- **Lambert, Millard Hurst III, c/o GlaxoSmithKline Research Triangle Park, NC 27709 (US)**
- **Montana, Valerie, Gail c/o GlaxoSmithKline Research Triangle Park, NC 27709 (US)**
- **Stewart, Eugene Lee, c/o GlaxoSmithKline Research Triangle Park, NC 27709 (US)**
- **Xu, Eric Huayiang, Van Andel Research Institut Grand Rapids, MI 49503 (US)**

(74) Representative: **Giddings, Peter John et al**
**GlaxoSmithkline**
**Corporate Intellectual Property (CN9.25.1)**
**980 Great West Road**
**Brentford, Middlesex TW8 9GS (GB)**

## (54) Structure of a glucocorticoid receptor ligand binding domain comprising an expanded binding pocket and methods employing same

(57) A solved three-dimensional crystal structure of a glucocorticord receptor (GR) α ligand binding domain polypeptide is disclosed, in the form of a crystalline glucocorticord receptor α ligand binding domain polypeptide in complex with the ligand fluticasone propionate (FP) and a peptide derived from the co-activator TIF2. The GR/FP/TIF2 structure includes an expanded binding pocket not seen in other GR structures. Methods of designing steroid and non-steroid modulators of the biological activity of GR and other nuclear receptors (NRs) are also disclosed. In another aspect of the present invention homology models of androgen receptor (AR), progesterone receptor (PR) and mineralcorticoid receptor (MR) are disclosed, as well as methods of forming homology models for other NRs. Methods of forming a soluble GR/FP/TIF2 complex are also disclosed.

EP 1 375 517 A1

SEQUENCE LISTING

<110> Xu, H Eric

Bledsoe, Randy K

Montana, Valerie G.

Stewart, Eugene L.

Lambert, Millard H.

<120> STRUCTURE OF A GLUCOCORTICOID RECEPTOR LIGAND BINDING DOMAIN COMPRISING AN EXPANDED BINDING POCKET AND METHODS EMPLOYING SAME

<130> PU4803WO

<160> 11

<170> PatentIn version 3.1

<210> 1

<211> 2334

<212> DNA

<213> Homo sapiens

<220>

<221> CDS

<222> (1)..(2334)

<223>

<400> 1

```
atg gac tcc aaa gaa tca tta act cct ggt aga gaa gaa aac ccc agc        48
Met Asp Ser Lys Glu Ser Leu Thr Pro Gly Arg Glu Glu Asn Pro Ser
1               5                   10                  15

agt gtg ctt gct cag gag agg gga gat gtg atg gac ttc tat aaa acc        96
Ser Val Leu Ala Gln Glu Arg Gly Asp Val Met Asp Phe Tyr Lys Thr
            20                  25                  30
```

```
cta aga gga gga gct act gtg aag gtt tct gcg tct tca ccc tca ctg        144
Leu Arg Gly Gly Ala Thr Val Lys Val Ser Ala Ser Ser Pro Ser Leu
        35                  40                  45

gct gtc gct tct caa tca gac tcc aag cag cga aga ctt ttg gtt gat        192
Ala Val Ala Ser Gln Ser Asp Ser Lys Gln Arg Arg Leu Leu Val Asp
    50                  55                  60

ttt cca aaa ggc tca gta agc aat gcg cag cag cca gat ctg tcc aaa        240
Phe Pro Lys Gly Ser Val Ser Asn Ala Gln Gln Pro Asp Leu Ser Lys
65                  70                  75                  80

gca gtt tca ctc tca atg gga ctg tat atg gga gag aca gaa aca aaa        288
Ala Val Ser Leu Ser Met Gly Leu Tyr Met Gly Glu Thr Glu Thr Lys
                85                  90                  95

gtg atg gga aat gac ctg gga ttc cca cag cag ggc caa atc agc ctt        336
Val Met Gly Asn Asp Leu Gly Phe Pro Gln Gln Gly Gln Ile Ser Leu
            100                 105                 110

tcc tcg ggg gaa aca gac tta aag ctt ttg gaa gaa agc att gca aac        384
Ser Ser Gly Glu Thr Asp Leu Lys Leu Leu Glu Glu Ser Ile Ala Asn
        115                 120                 125

ctc aat agg tcg acc agt gtt cca gag aac ccc aag agt tca gca tcc        432
Leu Asn Arg Ser Thr Ser Val Pro Glu Asn Pro Lys Ser Ser Ala Ser
    130                 135                 140

act gct gtg tct gct gcc ccc aca gag aag gag ttt cca aaa act cac        480
Thr Ala Val Ser Ala Ala Pro Thr Glu Lys Glu Phe Pro Lys Thr His
145                 150                 155                 160

tct gat gta tct tca gaa cag caa cat ttg aag ggc cag act ggc acc        528
Ser Asp Val Ser Ser Glu Gln Gln His Leu Lys Gly Gln Thr Gly Thr
                165                 170                 175

aac ggt ggc aat gtg aaa ttg tat acc aca gac caa agc acc ttt gac        576
Asn Gly Gly Asn Val Lys Leu Tyr Thr Thr Asp Gln Ser Thr Phe Asp
            180                 185                 190

att ttg cag gat ttg gag ttt tct tct ggg tcc cca ggt aaa gag acg        624
Ile Leu Gln Asp Leu Glu Phe Ser Ser Gly Ser Pro Gly Lys Glu Thr
        195                 200                 205

aat gag agt cct tgg aga tca gac ctg ttg ata gat gaa aac tgt ttg        672
Asn Glu Ser Pro Trp Arg Ser Asp Leu Leu Ile Asp Glu Asn Cys Leu
    210                 215                 220

ctt tct cct ctg gcg gga gaa gac gat tca ttc ctt ttg gaa gga aac        720
Leu Ser Pro Leu Ala Gly Glu Asp Asp Ser Phe Leu Leu Glu Gly Asn
225                 230                 235                 240

tcg aat gag gac tgc aag cct ctc att tta ccg gac act aaa ccc aaa        768
Ser Asn Glu Asp Cys Lys Pro Leu Ile Leu Pro Asp Thr Lys Pro Lys
                245                 250                 255

att aag gat aat gga gat ctg gtt ttg tca agc ccc agt aat gta aca        816
Ile Lys Asp Asn Gly Asp Leu Val Leu Ser Ser Pro Ser Asn Val Thr
            260                 265                 270

ctg ccc caa gtg aaa aca gaa aaa gaa gat ttc atc gaa ctc tgc acc        864
```

```
Leu Pro Gln Val Lys Thr Glu Lys Glu Asp Phe Ile Glu Leu Cys Thr
        275                 280                 285

cct ggg gta att aag caa gag aaa ctg ggc aca gtt tac tgt cag gca      912
Pro Gly Val Ile Lys Gln Glu Lys Leu Gly Thr Val Tyr Cys Gln Ala
    290                 295                 300

agc ttt cct gga gca aat ata att ggt aat aaa atg tct gcc att tct      960
Ser Phe Pro Gly Ala Asn Ile Ile Gly Asn Lys Met Ser Ala Ile Ser
305                 310                 315                 320

gtt cat ggt gtg agt acc tct gga gga cag atg tac cac tat gac atg     1008
Val His Gly Val Ser Thr Ser Gly Gly Gln Met Tyr His Tyr Asp Met
                325                 330                 335

aat aca gca tcc ctt tct caa cag cag gat cag aag cct att ttt aat     1056
Asn Thr Ala Ser Leu Ser Gln Gln Gln Asp Gln Lys Pro Ile Phe Asn
            340                 345                 350

gtc att cca cca att ccc gtt ggt tcc gaa aat tgg aat agg tgc caa     1104
Val Ile Pro Pro Ile Pro Val Gly Ser Glu Asn Trp Asn Arg Cys Gln
        355                 360                 365

gga tct gga gat gac aac ttg act tct ctg ggg act ctg aac ttc cct     1152
Gly Ser Gly Asp Asp Asn Leu Thr Ser Leu Gly Thr Leu Asn Phe Pro
    370                 375                 380

ggt cga aca gtt ttt tct aat ggc tat tca agc ccc agc atg aga cca     1200
Gly Arg Thr Val Phe Ser Asn Gly Tyr Ser Ser Pro Ser Met Arg Pro
385                 390                 395                 400

gat gta agc tct cct cca tcc agc tcc tca aca gca aca aca gga cca     1248
Asp Val Ser Ser Pro Pro Ser Ser Ser Ser Thr Ala Thr Thr Gly Pro
                405                 410                 415

cct ccc aaa ctc tgc ctg gtg tgc tct gat gaa gct tca gga tgt cat     1296
Pro Pro Lys Leu Cys Leu Val Cys Ser Asp Glu Ala Ser Gly Cys His
            420                 425                 430

tat gga gtc tta act tgt gga agc tgt aaa gtt ttc ttc aaa aga gca     1344
Tyr Gly Val Leu Thr Cys Gly Ser Cys Lys Val Phe Phe Lys Arg Ala
        435                 440                 445

gtg gaa gga cag cac aat tac cta tgt gct gga agg aat gat tgc atc     1392
Val Glu Gly Gln His Asn Tyr Leu Cys Ala Gly Arg Asn Asp Cys Ile
    450                 455                 460

atc gat aaa att cga aga aaa aac tgc cca gca tgc cgc tat cga aaa     1440
Ile Asp Lys Ile Arg Arg Lys Asn Cys Pro Ala Cys Arg Tyr Arg Lys
465                 470                 475                 480

tgt ctt cag gct gga atg aac ctg gaa gct cga aaa aca aag aaa aaa     1488
Cys Leu Gln Ala Gly Met Asn Leu Glu Ala Arg Lys Thr Lys Lys Lys
                485                 490                 495

ata aaa gga att cag cag gcc act aca gga gtc tca caa gaa acc tct     1536
Ile Lys Gly Ile Gln Gln Ala Thr Thr Gly Val Ser Gln Glu Thr Ser
            500                 505                 510

gaa aat cct ggt aac aaa aca ata gtt cct gca acg tta cca caa ctc     1584
Glu Asn Pro Gly Asn Lys Thr Ile Val Pro Ala Thr Leu Pro Gln Leu
```

```
            515                 520                 525

acc cct acc ctg gtg tca ctg ttg gag gtt att gaa cct gaa gtg tta       1632
Thr Pro Thr Leu Val Ser Leu Leu Glu Val Ile Glu Pro Glu Val Leu
    530                 535                 540

tat gca gga tat gat agc tct gtt cca gac tca act tgg agg atc atg       1680
Tyr Ala Gly Tyr Asp Ser Ser Val Pro Asp Ser Thr Trp Arg Ile Met
545                 550                 555                 560

act acg ctc aac atg tta gga ggg cgg caa gtg att gca gca gtg aaa       1728
Thr Thr Leu Asn Met Leu Gly Gly Arg Gln Val Ile Ala Ala Val Lys
                565                 570                 575

tgg gca aag gca ata cca ggt ttc agg aac tta cac ctg gat gac caa       1776
Trp Ala Lys Ala Ile Pro Gly Phe Arg Asn Leu His Leu Asp Asp Gln
            580                 585                 590

atg acc cta ctg cag tac tcc tgg atg ttt ctt atg gca ttt gct ctg       1824
Met Thr Leu Leu Gln Tyr Ser Trp Met Phe Leu Met Ala Phe Ala Leu
        595                 600                 605

ggg tgg aga tca tat aga caa tca agt gca aac ctg ctg tgt ttt gct       1872
Gly Trp Arg Ser Tyr Arg Gln Ser Ser Ala Asn Leu Leu Cys Phe Ala
    610                 615                 620

cct gat ctg att att aat gag cag aga atg act cta ccc tgc atg tac       1920
Pro Asp Leu Ile Ile Asn Glu Gln Arg Met Thr Leu Pro Cys Met Tyr
625                 630                 635                 640

gac caa tgt aaa cac atg ctg tat gtt tcc tct gag tta cac agg ctt       1968
Asp Gln Cys Lys His Met Leu Tyr Val Ser Ser Glu Leu His Arg Leu
                645                 650                 655

cag gta tct tat gaa gag tat ctc tgt atg aaa acc tta ctg ctt ctc       2016
Gln Val Ser Tyr Glu Glu Tyr Leu Cys Met Lys Thr Leu Leu Leu Leu
            660                 665                 670

tct tca gtt cct aag gac ggt ctg aag agc caa gag cta ttt gat gaa       2064
Ser Ser Val Pro Lys Asp Gly Leu Lys Ser Gln Glu Leu Phe Asp Glu
        675                 680                 685

att aga atg acc tac atc aaa gag cta gga aaa gcc att gtc aag agg       2112
Ile Arg Met Thr Tyr Ile Lys Glu Leu Gly Lys Ala Ile Val Lys Arg
    690                 695                 700

gaa gga aac tcc agc cag aac tgg cag cgg ttt tat caa ctg aca aaa       2160
Glu Gly Asn Ser Ser Gln Asn Trp Gln Arg Phe Tyr Gln Leu Thr Lys
705                 710                 715                 720

ctc ttg gat tct atg cat gaa gtg gtt gaa aat ctc ctt aac tat tgc       2208
Leu Leu Asp Ser Met His Glu Val Val Glu Asn Leu Leu Asn Tyr Cys
                725                 730                 735

ttc caa aca ttt ttg gat aag acc atg agt att gaa ttc ccc gag atg       2256
Phe Gln Thr Phe Leu Asp Lys Thr Met Ser Ile Glu Phe Pro Glu Met
            740                 745                 750

tta gct gaa atc atc acc aat cag ata cca aaa tat tca aat gga aat       2304
Leu Ala Glu Ile Ile Thr Asn Gln Ile Pro Lys Tyr Ser Asn Gly Asn
        755                 760                 765
```

```
atc aaa aaa ctt ctg ttt cat caa aag tga                                  2334
Ile Lys Lys Leu Leu Phe His Gln Lys
    770                 775
```

<210> 2

<211> 777

<212> PRT

<213> Homo sapiens

<400> 2

```
Met Asp Ser Lys Glu Ser Leu Thr Pro Gly Arg Glu Glu Asn Pro Ser
1               5                   10                  15

Ser Val Leu Ala Gln Glu Arg Gly Asp Val Met Asp Phe Tyr Lys Thr
            20                  25                  30

Leu Arg Gly Gly Ala Thr Val Lys Val Ser Ala Ser Ser Pro Ser Leu
        35                  40                  45

Ala Val Ala Ser Gln Ser Asp Ser Lys Gln Arg Arg Leu Leu Val Asp
    50                  55                  60

Phe Pro Lys Gly Ser Val Ser Asn Ala Gln Gln Pro Asp Leu Ser Lys
65                  70                  75                  80

Ala Val Ser Leu Ser Met Gly Leu Tyr Met Gly Glu Thr Glu Thr Lys
                85                  90                  95

Val Met Gly Asn Asp Leu Gly Phe Pro Gln Gln Gly Gln Ile Ser Leu
            100                 105                 110

Ser Ser Gly Glu Thr Asp Leu Lys Leu Leu Glu Glu Ser Ile Ala Asn
        115                 120                 125

Leu Asn Arg Ser Thr Ser Val Pro Glu Asn Pro Lys Ser Ser Ala Ser
    130                 135                 140

Thr Ala Val Ser Ala Ala Pro Thr Glu Lys Glu Phe Pro Lys Thr His
145                 150                 155                 160

Ser Asp Val Ser Ser Glu Gln Gln His Leu Lys Gly Gln Thr Gly Thr
                165                 170                 175

Asn Gly Gly Asn Val Lys Leu Tyr Thr Thr Asp Gln Ser Thr Phe Asp
```

180 185 190

Ile Leu Gln Asp Leu Glu Phe Ser Ser Gly Ser Pro Gly Lys Glu Thr
195 200 205

Asn Glu Ser Pro Trp Arg Ser Asp Leu Leu Ile Asp Glu Asn Cys Leu
210 215 220

Leu Ser Pro Leu Ala Gly Glu Asp Asp Ser Phe Leu Leu Glu Gly Asn
225 230 235 240
Ser Asn Glu Asp Cys Lys Pro Leu Ile Leu Pro Asp Thr Lys Pro Lys
245 250 255

Ile Lys Asp Asn Gly Asp Leu Val Leu Ser Ser Pro Ser Asn Val Thr
260 265 270

Leu Pro Gln Val Lys Thr Glu Lys Glu Asp Phe Ile Glu Leu Cys Thr
275 280 285

Pro Gly Val Ile Lys Gln Glu Lys Leu Gly Thr Val Tyr Cys Gln Ala
290 295 300

Ser Phe Pro Gly Ala Asn Ile Ile Gly Asn Lys Met Ser Ala Ile Ser
305 310 315 320

Val His Gly Val Ser Thr Ser Gly Gly Gln Met Tyr His Tyr Asp Met
325 330 335

Asn Thr Ala Ser Leu Ser Gln Gln Gln Asp Gln Lys Pro Ile Phe Asn
340 345 350

Val Ile Pro Pro Ile Pro Val Gly Ser Glu Asn Trp Asn Arg Cys Gln
355 360 365

Gly Ser Gly Asp Asp Asn Leu Thr Ser Leu Gly Thr Leu Asn Phe Pro
370 375 380

Gly Arg Thr Val Phe Ser Asn Gly Tyr Ser Ser Pro Ser Met Arg Pro
385 390 395 400

Asp Val Ser Ser Pro Pro Ser Ser Ser Ser Thr Ala Thr Thr Gly Pro
405 410 415

Pro Pro Lys Leu Cys Leu Val Cys Ser Asp Glu Ala Ser Gly Cys His
420 425 430

Tyr Gly Val Leu Thr Cys Gly Ser Cys Lys Val Phe Phe Lys Arg Ala
435 440 445

```
Val Glu Gly Gln His Asn Tyr Leu Cys Ala Gly Arg Asn Asp Cys Ile
    450                 455                 460

Ile Asp Lys Ile Arg Arg Lys Asn Cys Pro Ala Cys Arg Tyr Arg Lys
465                 470                 475                 480

Cys Leu Gln Ala Gly Met Asn Leu Glu Ala Arg Lys Thr Lys Lys Lys
                485                 490                 495

Ile Lys Gly Ile Gln Gln Ala Thr Thr Gly Val Ser Gln Glu Thr Ser
            500                 505                 510
Glu Asn Pro Gly Asn Lys Thr Ile Val Pro Ala Thr Leu Pro Gln Leu
        515                 520                 525

Thr Pro Thr Leu Val Ser Leu Leu Glu Val Ile Glu Pro Glu Val Leu
    530                 535                 540

Tyr Ala Gly Tyr Asp Ser Ser Val Pro Asp Ser Thr Trp Arg Ile Met
545                 550                 555                 560

Thr Thr Leu Asn Met Leu Gly Gly Arg Gln Val Ile Ala Ala Val Lys
                565                 570                 575

Trp Ala Lys Ala Ile Pro Gly Phe Arg Asn Leu His Leu Asp Asp Gln
            580                 585                 590

Met Thr Leu Leu Gln Tyr Ser Trp Met Phe Leu Met Ala Phe Ala Leu
        595                 600                 605

Gly Trp Arg Ser Tyr Arg Gln Ser Ser Ala Asn Leu Leu Cys Phe Ala
    610                 615                 620

Pro Asp Leu Ile Ile Asn Glu Gln Arg Met Thr Leu Pro Cys Met Tyr
625                 630                 635                 640

Asp Gln Cys Lys His Met Leu Tyr Val Ser Ser Glu Leu His Arg Leu
                645                 650                 655

Gln Val Ser Tyr Glu Glu Tyr Leu Cys Met Lys Thr Leu Leu Leu Leu
            660                 665                 670

Ser Ser Val Pro Lys Asp Gly Leu Lys Ser Gln Glu Leu Phe Asp Glu
        675                 680                 685

Ile Arg Met Thr Tyr Ile Lys Glu Leu Gly Lys Ala Ile Val Lys Arg
    690                 695                 700
```

```
Glu Gly Asn Ser Ser Gln Asn Trp Gln Arg Phe Tyr Gln Leu Thr Lys
705                 710                 715                 720

Leu Leu Asp Ser Met His Glu Val Val Glu Asn Leu Leu Asn Tyr Cys
                725                 730                 735

Phe Gln Thr Phe Leu Asp Lys Thr Met Ser Ile Glu Phe Pro Glu Met
            740                 745                 750

Leu Ala Glu Ile Ile Thr Asn Gln Ile Pro Lys Tyr Ser Asn Gly Asn
        755                 760                 765

Ile Lys Lys Leu Leu Phe His Gln Lys
    770                 775
```

<210> 3

<211> 2334

<212> DNA

<213> Homo sapiens

<220>

<221> CDS

<222> (1)..(2334)

<223>

<400> 3

```
atg gac tcc aaa gaa tca tta act cct ggt aga gaa gaa aac ccc agc       48
Met Asp Ser Lys Glu Ser Leu Thr Pro Gly Arg Glu Glu Asn Pro Ser
1               5                   10                  15

agt gtg ctt gct cag gag agg gga gat gtg atg gac ttc tat aaa acc       96
Ser Val Leu Ala Gln Glu Arg Gly Asp Val Met Asp Phe Tyr Lys Thr
            20                  25                  30

cta aga gga gga gct act gtg aag gtt tct gcg tct tca ccc tca ctg      144
Leu Arg Gly Gly Ala Thr Val Lys Val Ser Ala Ser Ser Pro Ser Leu
        35                  40                  45

gct gtc gct tct caa tca gac tcc aag cag cga aga ctt ttg gtt gat      192
Ala Val Ala Ser Gln Ser Asp Ser Lys Gln Arg Arg Leu Leu Val Asp
    50                  55                  60

ttt cca aaa ggc tca gta agc aat gcg cag cag cca gat ctg tcc aaa      240
Phe Pro Lys Gly Ser Val Ser Asn Ala Gln Gln Pro Asp Leu Ser Lys
65                  70                  75                  80

gca gtt tca ctc tca atg gga ctg tat atg gga gag aca gaa aca aaa      288
Ala Val Ser Leu Ser Met Gly Leu Tyr Met Gly Glu Thr Glu Thr Lys
                85                  90                  95
```

```
gtg atg gga aat gac ctg gga ttc cca cag cag ggc caa atc agc ctt      336
Val Met Gly Asn Asp Leu Gly Phe Pro Gln Gln Gly Gln Ile Ser Leu
            100                 105                 110

tcc tcg ggg gaa aca gac tta aag ctt ttg gaa gaa agc att gca aac      384
Ser Ser Gly Glu Thr Asp Leu Lys Leu Leu Glu Glu Ser Ile Ala Asn
        115                 120                 125

ctc aat agg tcg acc agt gtt cca gag aac ccc aag agt tca gca tcc      432
Leu Asn Arg Ser Thr Ser Val Pro Glu Asn Pro Lys Ser Ser Ala Ser
    130                 135                 140

act gct gtg tct gct gcc ccc aca gag aag gag ttt cca aaa act cac      480
Thr Ala Val Ser Ala Ala Pro Thr Glu Lys Glu Phe Pro Lys Thr His
145                 150                 155                 160

tct gat gta tct tca gaa cag caa cat ttg aag ggc cag act ggc acc      528
Ser Asp Val Ser Ser Glu Gln Gln His Leu Lys Gly Gln Thr Gly Thr
                165                 170                 175

aac ggt ggc aat gtg aaa ttg tat acc aca gac caa agc acc ttt gac      576
Asn Gly Gly Asn Val Lys Leu Tyr Thr Thr Asp Gln Ser Thr Phe Asp
            180                 185                 190

att ttg cag gat ttg gag ttt tct tct ggg tcc cca ggt aaa gag acg      624
Ile Leu Gln Asp Leu Glu Phe Ser Ser Gly Ser Pro Gly Lys Glu Thr
        195                 200                 205

aat gag agt cct tgg aga tca gac ctg ttg ata gat gaa aac tgt ttg      672
Asn Glu Ser Pro Trp Arg Ser Asp Leu Leu Ile Asp Glu Asn Cys Leu
    210                 215                 220

ctt tct cct ctg gcg gga gaa gac gat tca ttc ctt ttg gaa gga aac      720
Leu Ser Pro Leu Ala Gly Glu Asp Asp Ser Phe Leu Leu Glu Gly Asn
225                 230                 235                 240

tcg aat gag gac tgc aag cct ctc att tta ccg gac act aaa ccc aaa      768
Ser Asn Glu Asp Cys Lys Pro Leu Ile Leu Pro Asp Thr Lys Pro Lys
                245                 250                 255

att aag gat aat gga gat ctg gtt ttg tca agc ccc agt aat gta aca      816
Ile Lys Asp Asn Gly Asp Leu Val Leu Ser Ser Pro Ser Asn Val Thr
            260                 265                 270

ctg ccc caa gtg aaa aca gaa aaa gaa gat ttc atc gaa ctc tgc acc      864
Leu Pro Gln Val Lys Thr Glu Lys Glu Asp Phe Ile Glu Leu Cys Thr
        275                 280                 285

cct ggg gta att aag caa gag aaa ctg ggc aca gtt tac tgt cag gca      912
Pro Gly Val Ile Lys Gln Glu Lys Leu Gly Thr Val Tyr Cys Gln Ala
    290                 295                 300

agc ttt cct gga gca aat ata att ggt aat aaa atg tct gcc att tct      960
Ser Phe Pro Gly Ala Asn Ile Ile Gly Asn Lys Met Ser Ala Ile Ser
305                 310                 315                 320

gtt cat ggt gtg agt acc tct gga gga cag atg tac cac tat gac atg     1008
Val His Gly Val Ser Thr Ser Gly Gly Gln Met Tyr His Tyr Asp Met
                325                 330                 335

aat aca gca tcc ctt tct caa cag cag gat cag aag cct att ttt aat     1056
```

```
Asn Thr Ala Ser Leu Ser Gln Gln Gln Asp Gln Lys Pro Ile Phe Asn
            340                 345                 350

gtc att cca cca att ccc gtt ggt tcc gaa aat tgg aat agg tgc caa      1104
Val Ile Pro Pro Ile Pro Val Gly Ser Glu Asn Trp Asn Arg Cys Gln
        355                 360                 365

gga tct gga gat gac aac ttg act tct ctg ggg act ctg aac ttc cct      1152
Gly Ser Gly Asp Asp Asn Leu Thr Ser Leu Gly Thr Leu Asn Phe Pro
    370                 375                 380

ggt cga aca gtt ttt tct aat ggc tat tca agc ccc agc atg aga cca      1200
Gly Arg Thr Val Phe Ser Asn Gly Tyr Ser Ser Pro Ser Met Arg Pro
385                 390                 395                 400

gat gta agc tct cct cca tcc agc tcc tca aca gca aca aca gga cca      1248
Asp Val Ser Ser Pro Pro Ser Ser Ser Ser Thr Ala Thr Thr Gly Pro
                405                 410                 415

cct ccc aaa ctc tgc ctg gtg tgc tct gat gaa gct tca gga tgt cat      1296
Pro Pro Lys Leu Cys Leu Val Cys Ser Asp Glu Ala Ser Gly Cys His
            420                 425                 430

tat gga gtc tta act tgt gga agc tgt aaa gtt ttc ttc aaa aga gca      1344
Tyr Gly Val Leu Thr Cys Gly Ser Cys Lys Val Phe Phe Lys Arg Ala
        435                 440                 445

gtg gaa gga cag cac aat tac cta tgt gct gga agg aat gat tgc atc      1392
Val Glu Gly Gln His Asn Tyr Leu Cys Ala Gly Arg Asn Asp Cys Ile
    450                 455                 460

atc gat aaa att cga aga aaa aac tgc cca gca tgc cgc tat cga aaa      1440
Ile Asp Lys Ile Arg Arg Lys Asn Cys Pro Ala Cys Arg Tyr Arg Lys
465                 470                 475                 480

tgt ctt cag gct gga atg aac ctg gaa gct cga aaa aca aag aaa aaa      1488
Cys Leu Gln Ala Gly Met Asn Leu Glu Ala Arg Lys Thr Lys Lys Lys
                485                 490                 495

ata aaa gga att cag cag gcc act aca gga gtc tca caa gaa acc tct      1536
Ile Lys Gly Ile Gln Gln Ala Thr Thr Gly Val Ser Gln Glu Thr Ser
            500                 505                 510

gaa aat cct ggt aac aaa aca ata gtt cct gca acg tta cca caa ctc      1584
Glu Asn Pro Gly Asn Lys Thr Ile Val Pro Ala Thr Leu Pro Gln Leu
        515                 520                 525

acc cct acc ctg gtg tca ctg ttg gag gtt att gaa cct gaa gtg tta      1632
Thr Pro Thr Leu Val Ser Leu Leu Glu Val Ile Glu Pro Glu Val Leu
    530                 535                 540

tat gca gga tat gat agc tct gtt cca gac tca act tgg agg atc atg      1680
Tyr Ala Gly Tyr Asp Ser Ser Val Pro Asp Ser Thr Trp Arg Ile Met
545                 550                 555                 560

act acg ctc aac atg tta gga ggg cgg caa gtg att gca gca gtg aaa      1728
Thr Thr Leu Asn Met Leu Gly Gly Arg Gln Val Ile Ala Ala Val Lys
                565                 570                 575

tgg gca aag gca ata cca ggt ttc agg aac tta cac ctg gat gac caa      1776
Trp Ala Lys Ala Ile Pro Gly Phe Arg Asn Leu His Leu Asp Asp Gln
```

580 585 590

atg acc cta ctg cag tac tcc tgg atg tcc ctt atg gca ttt gct ctg 1824
Met Thr Leu Leu Gln Tyr Ser Trp Met Ser Leu Met Ala Phe Ala Leu
595 600 605

ggg tgg aga tca tat aga caa tca agt gca aac ctg ctg tgt ttt gct 1872
Gly Trp Arg Ser Tyr Arg Gln Ser Ser Ala Asn Leu Leu Cys Phe Ala
610 615 620

cct gat ctg att att aat gag cag aga atg act cta ccc tgc atg tac 1920
Pro Asp Leu Ile Ile Asn Glu Gln Arg Met Thr Leu Pro Cys Met Tyr
625 630 635 640

gac caa tgt aaa cac atg ctg tat gtt tcc tct gag tta cac agg ctt 1968
Asp Gln Cys Lys His Met Leu Tyr Val Ser Ser Glu Leu His Arg Leu
645 650 655

cag gta tct tat gaa gag tat ctc tgt atg aaa acc tta ctg ctt ctc 2016
Gln Val Ser Tyr Glu Glu Tyr Leu Cys Met Lys Thr Leu Leu Leu Leu
660 665 670

tct tca gtt cct aag gac ggt ctg aag agc caa gag cta ttt gat gaa 2064
Ser Ser Val Pro Lys Asp Gly Leu Lys Ser Gln Glu Leu Phe Asp Glu
675 680 685

att aga atg acc tac atc aaa gag cta gga aaa gcc att gtc aag agg 2112
Ile Arg Met Thr Tyr Ile Lys Glu Leu Gly Lys Ala Ile Val Lys Arg
690 695 700

gaa gga aac tcc agc cag aac tgg cag cgg ttt tat caa ctg aca aaa 2160
Glu Gly Asn Ser Ser Gln Asn Trp Gln Arg Phe Tyr Gln Leu Thr Lys
705 710 715 720

ctc ttg gat tct atg cat gaa gtg gtt gaa aat ctc ctt aac tat tgc 2208
Leu Leu Asp Ser Met His Glu Val Val Glu Asn Leu Leu Asn Tyr Cys
725 730 735

ttc caa aca ttt ttg gat aag acc atg agt att gaa ttc ccc gag atg 2256
Phe Gln Thr Phe Leu Asp Lys Thr Met Ser Ile Glu Phe Pro Glu Met
740 745 750

tta gct gaa atc atc acc aat cag ata cca aaa tat tca aat gga aat 2304
Leu Ala Glu Ile Ile Thr Asn Gln Ile Pro Lys Tyr Ser Asn Gly Asn
755 760 765

atc aaa aaa ctt ctg ttt cat caa aag tga 2334
Ile Lys Lys Leu Leu Phe His Gln Lys
770 775

<210> 4

<211> 777

<212> PRT

<213> Homo sapiens

<400> 4

```
Met Asp Ser Lys Glu Ser Leu Thr Pro Gly Arg Glu Glu Asn Pro Ser
1               5                   10                  15

Ser Val Leu Ala Gln Glu Arg Gly Asp Val Met Asp Phe Tyr Lys Thr
            20                  25                  30

Leu Arg Gly Gly Ala Thr Val Lys Val Ser Ala Ser Ser Pro Ser Leu
        35                  40                  45

Ala Val Ala Ser Gln Ser Asp Ser Lys Gln Arg Arg Leu Leu Val Asp
    50                  55                  60

Phe Pro Lys Gly Ser Val Ser Asn Ala Gln Gln Pro Asp Leu Ser Lys
65                  70                  75                  80

Ala Val Ser Leu Ser Met Gly Leu Tyr Met Gly Glu Thr Glu Thr Lys
                85                  90                  95

Val Met Gly Asn Asp Leu Gly Phe Pro Gln Gln Gly Gln Ile Ser Leu
            100                 105                 110

Ser Ser Gly Glu Thr Asp Leu Lys Leu Leu Glu Glu Ser Ile Ala Asn
        115                 120                 125

Leu Asn Arg Ser Thr Ser Val Pro Glu Asn Pro Lys Ser Ser Ala Ser
    130                 135                 140

Thr Ala Val Ser Ala Ala Pro Thr Glu Lys Glu Phe Pro Lys Thr His
145                 150                 155                 160

Ser Asp Val Ser Ser Glu Gln Gln His Leu Lys Gly Gln Thr Gly Thr
                165                 170                 175

Asn Gly Gly Asn Val Lys Leu Tyr Thr Thr Asp Gln Ser Thr Phe Asp
            180                 185                 190

Ile Leu Gln Asp Leu Glu Phe Ser Ser Gly Ser Pro Gly Lys Glu Thr
        195                 200                 205

Asn Glu Ser Pro Trp Arg Ser Asp Leu Leu Ile Asp Glu Asn Cys Leu
    210                 215                 220

Leu Ser Pro Leu Ala Gly Glu Asp Asp Ser Phe Leu Leu Glu Gly Asn
225                 230                 235                 240
```

```
Ser Asn Glu Asp Cys Lys Pro Leu Ile Leu Pro Asp Thr Lys Pro Lys
                245                 250                 255

Ile Lys Asp Asn Gly Asp Leu Val Leu Ser Ser Pro Ser Asn Val Thr
            260                 265                 270

Leu Pro Gln Val Lys Thr Glu Lys Glu Asp Phe Ile Glu Leu Cys Thr
        275                 280                 285

Pro Gly Val Ile Lys Gln Glu Lys Leu Gly Thr Val Tyr Cys Gln Ala
    290                 295                 300

Ser Phe Pro Gly Ala Asn Ile Ile Gly Asn Lys Met Ser Ala Ile Ser
305                 310                 315                 320

Val His Gly Val Ser Thr Ser Gly Gly Gln Met Tyr His Tyr Asp Met
                325                 330                 335

Asn Thr Ala Ser Leu Ser Gln Gln Gln Asp Gln Lys Pro Ile Phe Asn
            340                 345                 350

Val Ile Pro Pro Ile Pro Val Gly Ser Glu Asn Trp Asn Arg Cys Gln
        355                 360                 365

Gly Ser Gly Asp Asp Asn Leu Thr Ser Leu Gly Thr Leu Asn Phe Pro
    370                 375                 380
Gly Arg Thr Val Phe Ser Asn Gly Tyr Ser Ser Pro Ser Met Arg Pro
385                 390                 395                 400

Asp Val Ser Ser Pro Pro Ser Ser Ser Ser Thr Ala Thr Thr Gly Pro
                405                 410                 415

Pro Pro Lys Leu Cys Leu Val Cys Ser Asp Glu Ala Ser Gly Cys His
            420                 425                 430

Tyr Gly Val Leu Thr Cys Gly Ser Cys Lys Val Phe Phe Lys Arg Ala
        435                 440                 445

Val Glu Gly Gln His Asn Tyr Leu Cys Ala Gly Arg Asn Asp Cys Ile
    450                 455                 460

Ile Asp Lys Ile Arg Arg Lys Asn Cys Pro Ala Cys Arg Tyr Arg Lys
465                 470                 475                 480

Cys Leu Gln Ala Gly Met Asn Leu Glu Ala Arg Lys Thr Lys Lys Lys
                485                 490                 495

Ile Lys Gly Ile Gln Gln Ala Thr Thr Gly Val Ser Gln Glu Thr Ser
```

500 505 510

Glu Asn Pro Gly Asn Lys Thr Ile Val Pro Ala Thr Leu Pro Gln Leu
515 520 525

Thr Pro Thr Leu Val Ser Leu Leu Glu Val Ile Glu Pro Glu Val Leu
530 535 540

Tyr Ala Gly Tyr Asp Ser Ser Val Pro Asp Ser Thr Trp Arg Ile Met
545 550 555 560

Thr Thr Leu Asn Met Leu Gly Gly Arg Gln Val Ile Ala Ala Val Lys
565 570 575

Trp Ala Lys Ala Ile Pro Gly Phe Arg Asn Leu His Leu Asp Asp Gln
580 585 590

Met Thr Leu Leu Gln Tyr Ser Trp Met Ser Leu Met Ala Phe Ala Leu
595 600 605

Gly Trp Arg Ser Tyr Arg Gln Ser Ser Ala Asn Leu Leu Cys Phe Ala
610 615 620

Pro Asp Leu Ile Ile Asn Glu Gln Arg Met Thr Leu Pro Cys Met Tyr
625 630 635 640

Asp Gln Cys Lys His Met Leu Tyr Val Ser Ser Glu Leu His Arg Leu
645 650 655
Gln Val Ser Tyr Glu Glu Tyr Leu Cys Met Lys Thr Leu Leu Leu Leu
660 665 670

Ser Ser Val Pro Lys Asp Gly Leu Lys Ser Gln Glu Leu Phe Asp Glu
675 680 685

Ile Arg Met Thr Tyr Ile Lys Glu Leu Gly Lys Ala Ile Val Lys Arg
690 695 700

Glu Gly Asn Ser Ser Gln Asn Trp Gln Arg Phe Tyr Gln Leu Thr Lys
705 710 715 720

Leu Leu Asp Ser Met His Glu Val Val Glu Asn Leu Leu Asn Tyr Cys
725 730 735

Phe Gln Thr Phe Leu Asp Lys Thr Met Ser Ile Glu Phe Pro Glu Met
740 745 750

Leu Ala Glu Ile Ile Thr Asn Gln Ile Pro Lys Tyr Ser Asn Gly Asn
755 760 765

```
Ile Lys Lys Leu Leu Phe His Gln Lys
    770                 775
```

<210> 5

<211> 774

<212> DNA

<213> Homo sapiens

<220>

<221> CDS

<222> (1)..(771)

<223>

<400> 5

```
gtt cct gca acg tta cca caa ctc acc cct acc ctg gtg tca ctg ttg       48
Val Pro Ala Thr Leu Pro Gln Leu Thr Pro Thr Leu Val Ser Leu Leu
1               5                   10                  15

gag gtt att gaa cct gaa gtg tta tat gca gga tat gat agc tct gtt       96
Glu Val Ile Glu Pro Glu Val Leu Tyr Ala Gly Tyr Asp Ser Ser Val
            20                  25                  30

cca gac tca act tgg agg atc atg act acg ctc aac atg tta gga ggg      144
Pro Asp Ser Thr Trp Arg Ile Met Thr Thr Leu Asn Met Leu Gly Gly
        35                  40                  45

cgg caa gtg att gca gca gtg aaa tgg gca aag gca ata cca ggt ttc      192
Arg Gln Val Ile Ala Ala Val Lys Trp Ala Lys Ala Ile Pro Gly Phe
    50                  55                  60

agg aac tta cac ctg gat gac caa atg acc cta ctg cag tac tcc tgg      240
Arg Asn Leu His Leu Asp Asp Gln Met Thr Leu Leu Gln Tyr Ser Trp
65                  70                  75                  80

atg ttt ctt atg gca ttt gct ctg ggg tgg aga tca tat aga caa tca      288
Met Phe Leu Met Ala Phe Ala Leu Gly Trp Arg Ser Tyr Arg Gln Ser
                85                  90                  95

agt gca aac ctg ctg tgt ttt gct cct gat ctg att att aat gag cag      336
Ser Ala Asn Leu Leu Cys Phe Ala Pro Asp Leu Ile Ile Asn Glu Gln
            100                 105                 110

aga atg act cta ccc tgc atg tac gac caa tgt aaa cac atg ctg tat      384
Arg Met Thr Leu Pro Cys Met Tyr Asp Gln Cys Lys His Met Leu Tyr
        115                 120                 125

gtt tcc tct gag tta cac agg ctt cag gta tct tat gaa gag tat ctc      432
Val Ser Ser Glu Leu His Arg Leu Gln Val Ser Tyr Glu Glu Tyr Leu
    130                 135                 140
```

```
tgt atg aaa acc tta ctg ctt ctc tct tca gtt cct aag gac ggt ctg        480
Cys Met Lys Thr Leu Leu Leu Leu Ser Ser Val Pro Lys Asp Gly Leu
145                 150                 155                 160

aag agc caa gag cta ttt gat gaa att aga atg acc tac atc aaa gag        528
Lys Ser Gln Glu Leu Phe Asp Glu Ile Arg Met Thr Tyr Ile Lys Glu
                165                 170                 175

cta gga aaa gcc att gtc aag agg gaa gga aac tcc agc cag aac tgg        576
Leu Gly Lys Ala Ile Val Lys Arg Glu Gly Asn Ser Ser Gln Asn Trp
            180                 185                 190

cag cgg ttt tat caa ctg aca aaa ctc ttg gat tct atg cat gaa gtg        624
Gln Arg Phe Tyr Gln Leu Thr Lys Leu Leu Asp Ser Met His Glu Val
        195                 200                 205

gtt gaa aat ctc ctt aac tat tgc ttc caa aca ttt ttg gat aag acc        672
Val Glu Asn Leu Leu Asn Tyr Cys Phe Gln Thr Phe Leu Asp Lys Thr
    210                 215                 220

atg agt att gaa ttc ccc gag atg tta gct gaa atc atc acc aat cag        720
Met Ser Ile Glu Phe Pro Glu Met Leu Ala Glu Ile Ile Thr Asn Gln
225                 230                 235                 240

ata cca aaa tat tca aat gga aat atc aaa aaa ctt ctg ttt cat caa        768
Ile Pro Lys Tyr Ser Asn Gly Asn Ile Lys Lys Leu Leu Phe His Gln
                245                 250                 255

aag tga                                                                774
Lys
```

```
<210>  6

<211>  257

<212>  PRT

<213>  Homo sapiens

<400>  6

Val Pro Ala Thr Leu Pro Gln Leu Thr Pro Thr Leu Val Ser Leu Leu
1               5                   10                  15

Glu Val Ile Glu Pro Glu Val Leu Tyr Ala Gly Tyr Asp Ser Ser Val
            20                  25                  30

Pro Asp Ser Thr Trp Arg Ile Met Thr Thr Leu Asn Met Leu Gly Gly
        35                  40                  45

Arg Gln Val Ile Ala Ala Val Lys Trp Ala Lys Ala Ile Pro Gly Phe
    50                  55                  60

Arg Asn Leu His Leu Asp Asp Gln Met Thr Leu Leu Gln Tyr Ser Trp
65                  70                  75                  80
```

Met Phe Leu Met Ala Phe Ala Leu Gly Trp Arg Ser Tyr Arg Gln Ser
85 90 95

Ser Ala Asn Leu Leu Cys Phe Ala Pro Asp Leu Ile Ile Asn Glu Gln
100 105 110

Arg Met Thr Leu Pro Cys Met Tyr Asp Gln Cys Lys His Met Leu Tyr
115 120 125

Val Ser Ser Glu Leu His Arg Leu Gln Val Ser Tyr Glu Glu Tyr Leu
130 135 140

Cys Met Lys Thr Leu Leu Leu Leu Ser Ser Val Pro Lys Asp Gly Leu
145 150 155 160

Lys Ser Gln Glu Leu Phe Asp Glu Ile Arg Met Thr Tyr Ile Lys Glu
165 170 175

Leu Gly Lys Ala Ile Val Lys Arg Glu Gly Asn Ser Ser Gln Asn Trp
180 185 190

Gln Arg Phe Tyr Gln Leu Thr Lys Leu Leu Asp Ser Met His Glu Val
195 200 205

Val Glu Asn Leu Leu Asn Tyr Cys Phe Gln Thr Phe Leu Asp Lys Thr
210 215 220

Met Ser Ile Glu Phe Pro Glu Met Leu Ala Glu Ile Ile Thr Asn Gln
225 230 235 240

Ile Pro Lys Tyr Ser Asn Gly Asn Ile Lys Lys Leu Leu Phe His Gln
245 250 255

Lys

<210> 7

<211> 774

<212> DNA

<213> Homo sapiens

<220>

<221> CDS

<222> (1)..(771)

<223>

<400> 7

```
gtt cct gca acg tta cca caa ctc acc cct acc ctg gtg tca ctg ttg        48
Val Pro Ala Thr Leu Pro Gln Leu Thr Pro Thr Leu Val Ser Leu Leu
1               5                   10                  15

gag gtt att gaa cct gaa gtg tta tat gca gga tat gat agc tct gtt        96
Glu Val Ile Glu Pro Glu Val Leu Tyr Ala Gly Tyr Asp Ser Ser Val
            20                  25                  30

cca gac tca act tgg agg atc atg act acg ctc aac atg tta gga ggg       144
Pro Asp Ser Thr Trp Arg Ile Met Thr Thr Leu Asn Met Leu Gly Gly
        35                  40                  45

cgg caa gtg att gca gca gtg aaa tgg gca aag gca ata cca ggt ttc       192
Arg Gln Val Ile Ala Ala Val Lys Trp Ala Lys Ala Ile Pro Gly Phe
    50                  55                  60

agg aac tta cac ctg gat gac caa atg acc cta ctg cag tac tcc tgg       240
Arg Asn Leu His Leu Asp Asp Gln Met Thr Leu Leu Gln Tyr Ser Trp
65                  70                  75                  80

atg tcc ctt atg gca ttt gct ctg ggg tgg aga tca tat aga caa tca       288
Met Ser Leu Met Ala Phe Ala Leu Gly Trp Arg Ser Tyr Arg Gln Ser
                85                  90                  95

agt gca aac ctg ctg tgt ttt gct cct gat ctg att att aat gag cag       336
Ser Ala Asn Leu Leu Cys Phe Ala Pro Asp Leu Ile Ile Asn Glu Gln
            100                 105                 110

aga atg act cta ccc tgc atg tac gac caa tgt aaa cac atg ctg tat       384
Arg Met Thr Leu Pro Cys Met Tyr Asp Gln Cys Lys His Met Leu Tyr
        115                 120                 125

gtt tcc tct gag tta cac agg ctt cag gta tct tat gaa gag tat ctc       432
Val Ser Ser Glu Leu His Arg Leu Gln Val Ser Tyr Glu Glu Tyr Leu
    130                 135                 140

tgt atg aaa acc tta ctg ctt ctc tct tca gtt cct aag gac ggt ctg       480
Cys Met Lys Thr Leu Leu Leu Leu Ser Ser Val Pro Lys Asp Gly Leu
145                 150                 155                 160

aag agc caa gag cta ttt gat gaa att aga atg acc tac atc aaa gag       528
Lys Ser Gln Glu Leu Phe Asp Glu Ile Arg Met Thr Tyr Ile Lys Glu
                165                 170                 175

cta gga aaa gcc att gtc aag agg gaa gga aac tcc agc cag aac tgg       576
Leu Gly Lys Ala Ile Val Lys Arg Glu Gly Asn Ser Ser Gln Asn Trp
            180                 185                 190

cag cgg ttt tat caa ctg aca aaa ctc ttg gat tct atg cat gaa gtg       624
Gln Arg Phe Tyr Gln Leu Thr Lys Leu Leu Asp Ser Met His Glu Val
        195                 200                 205

gtt gaa aat ctc ctt aac tat tgc ttc caa aca ttt ttg gat aag acc       672
Val Glu Asn Leu Leu Asn Tyr Cys Phe Gln Thr Phe Leu Asp Lys Thr
```

210 215 220

atg agt att gaa ttc ccc gag atg tta gct gaa atc atc acc aat cag 720
Met Ser Ile Glu Phe Pro Glu Met Leu Ala Glu Ile Ile Thr Asn Gln
225 230 235 240

ata cca aaa tat tca aat gga aat atc aaa aaa ctt ctg ttt cat caa 768
Ile Pro Lys Tyr Ser Asn Gly Asn Ile Lys Lys Leu Leu Phe His Gln
245 250 255

aag tga 774
Lys

<210> 8

<211> 257

<212> PRT

<213> Homo sapiens

<400> 8

Val Pro Ala Thr Leu Pro Gln Leu Thr Pro Thr Leu Val Ser Leu Leu
1 5 10 15

Glu Val Ile Glu Pro Glu Val Leu Tyr Ala Gly Tyr Asp Ser Ser Val
20 25 30

Pro Asp Ser Thr Trp Arg Ile Met Thr Thr Leu Asn Met Leu Gly Gly
35 40 45

Arg Gln Val Ile Ala Ala Val Lys Trp Ala Lys Ala Ile Pro Gly Phe
50 55 60

Arg Asn Leu His Leu Asp Asp Gln Met Thr Leu Leu Gln Tyr Ser Trp
65 70 75 80

Met Ser Leu Met Ala Phe Ala Leu Gly Trp Arg Ser Tyr Arg Gln Ser
85 90 95

Ser Ala Asn Leu Leu Cys Phe Ala Pro Asp Leu Ile Ile Asn Glu Gln
100 105 110

Arg Met Thr Leu Pro Cys Met Tyr Asp Gln Cys Lys His Met Leu Tyr
115 120 125

Val Ser Ser Glu Leu His Arg Leu Gln Val Ser Tyr Glu Glu Tyr Leu
130 135 140

Cys Met Lys Thr Leu Leu Leu Leu Ser Ser Val Pro Lys Asp Gly Leu
145 150 155 160

Lys Ser Gln Glu Leu Phe Asp Glu Ile Arg Met Thr Tyr Ile Lys Glu
165 170 175

Leu Gly Lys Ala Ile Val Lys Arg Glu Gly Asn Ser Ser Gln Asn Trp
180 185 190

Gln Arg Phe Tyr Gln Leu Thr Lys Leu Leu Asp Ser Met His Glu Val
195 200 205

Val Glu Asn Leu Leu Asn Tyr Cys Phe Gln Thr Phe Leu Asp Lys Thr
210 215 220

Met Ser Ile Glu Phe Pro Glu Met Leu Ala Glu Ile Ile Thr Asn Gln
225 230 235 240

Ile Pro Lys Tyr Ser Asn Gly Asn Ile Lys Lys Leu Leu Phe His Gln
245 250 255

Lys

<210> 9

<211> 14

<212> PRT

<213> Homo sapiens

<400> 9

Lys Glu Asn Ala Leu Leu Arg Tyr Leu Leu Asp Lys Asp Asp
1 5 10

<210> 10

<211> 5

<212> PRT

<213> Homo sapiens

<220>

<221> misc_feature

<222> (1)..(5)

<223> X is any amino acid

<400> 10

Leu Xaa Xaa Leu Leu
1 5

<210> 11

<211> 6

<212> PRT

<213> Homo sapiens

<400> 11

Leu Leu Arg Tyr Leu Leu
1 5

**Description**

Technical Field

The present invention relates generally to a glucocorticoid receptor polypeptide, to a glucocorticoid receptor ligand binding domain polypeptide, and to the structure of a glucocorticoid receptor ligand binding domain bound to fluticasone propionate and a co-activator peptide. This stucture reveals an expanded binding pocket having a configuration and volume not observed in other GR structures, which explains the observed binding of some ligands to GR. In one aspect, the invention relates to methods by which a soluble complex comprising glucocorticoid ligand binding domain, fluticasone propionate and a co-activator can be generated. Methods by which modulators and ligands of nuclear receptors, particularly steroid receptors, and more particularly glucosteroid receptors, and the ligand binding domains thereof, can be identified are also disclosed. The invention further relates to homology models of nuclear receptors, preferably the ligand binding domains of nuclear receptors, which can be generated using the structure of a glucocorticoid receptor of the present invention, as well as docking models of an association between a ligand and a nuclear receptor.

| Abbreviations | |
|---|---|
| ATP | adenosine triphosphate |
| ADP | adenosine diphosphate |
| APS | Advanced Photon Source |
| AR | androgen receptor |
| CAT | chloramphenicol acyltransferase |
| CCD | charge-coupled device |
| cDNA | complementary DNA |
| DBD | DNA binding domain |
| DEX | dexamethasone |
| DHT | dihydrotestosterone |
| DMSO | dimethyl sulfoxide |
| DNA | deoxyribonucleic acid |
| DTT | dithiothreitol |
| EDTA | ethylenediaminetetraacetic acid |
| ER | estrogen receptor |
| FP | fluticasone propionate |
| GR | glucocorticoid receptor |
| GR$\alpha$ | glucocorticoid receptor $\alpha$ |
| GRE | glucocorticoid responsive element |
| HEPES | N-2-hydroxyethylpiperazine-N'-2-ethanesulfonic acid |
| HSP | heat shock protein |
| kDa | kilodalton(s) |
| LBD | ligand binding domain |
| MM | molecular mechanics |
| MR | mineralcorticoid receptor |
| NDP | nucleotide diphosphate |
| NID | nuclear receptor interaction domain |
| NR | nuclear receptor |
| NTP | nucleotide triphosphate |
| PAGE | polyacrylamide gel electrophoresis |
| PCR | polymerase chain reaction |
| PG | progesterone |
| pl | isoelectric point |
| PPAR | peroxisome proliferator-activated receptor |
| PR | progesterone receptor |
| QSAR | quantitative structure-activity relationship |

(continued)

| Abbreviations | |
|---|---|
| RAR | retinoid acid receptor |
| RXR | retinoid X receptor |
| SAR | structure-activity relationship |
| SDS | sodium dodecyl sulfate |
| SDS-PAGE | sodium dodecyl sulfate polyacrylamide gel electrophoresis |
| SR | steroid receptor |
| TIF2 | transcription intermediary factor 2 |
| TR | thyroid receptor |
| VDR | vitamin D receptor |

| Amino Acid Abbreviations | | |
|---|---|---|
| Single-Letter Code | Three-Letter Code | Name |
| A | Ala | Alanine |
| V | Val | Valine |
| L | Leu | Leucine |
| I | Ile | Isoleucine |
| P | Pro | Proline |
| F | Phe | Phenylalanine |
| W | Trp | Tryptophan |
| M | Met | Methionine |
| G | Gly | Glycine |
| S | Ser | Serine |
| T | Thr | Threonine |
| C | Cys | Cysteine |
| Y | Tyr | Tyrosine |
| N | Asn | Asparagine |
| Q | Gln | Glutamine |
| D | Asp | Aspartic Acid |
| E | Glu | Glutamic Acid |
| K | Lys | Lysine |
| R | Arg | Arginine |
| H | His | Histidine |

## Functionally Equivalent Codons

| Amino Acid | Codons |
|---|---|

| | | | |
|---|---|---|---|
| Alanine | Ala | A | GCA GCC GCG GCU |
| Cysteine | Cys | C | UGC UGU |
| Aspartic Acid | Asp | D | GAC GAU |
| Glumatic acid | Glu | E | GAA GAG |
| Phenylalanine | Phe | F | UUC UUU |
| Glycine | Gly | G | GGA GGC GGG GGU |
| Histidine | His | H | CAC CAU |
| Isoleucine | Ile | I | AUA AUC AUU |
| Lysine | Lys | K | AAA AAG |
| Methionine | Met | M | AUG |
| Asparagine | Asn | N | AAC AAU |
| Proline | Pro | P | CCA CCC CCG CCU |
| Glutamine | Gln | Q | CAA CAG |
| Threonine | Thr | T | ACA ACC ACG ACU |
| Valine | Val | V | GUA GUC GUG GUU |
| Tryptophan | Trp | W | UGG |
| Tyrosine | Tyr | Y | UAC UAU |
| Leucine | Leu | L | UUA UUG CUA CUC CUG CUU |
| Arginine | Arg | R | AGA AGG CGA CGC CGG CGU |
| Serine | Ser | S | ACG AGU UCA UCC UCG UCU |

Background Art

Nuclear receptors represent a superfamily of proteins that specifically bind a physiologically relevant small molecule, such as a hormone or vitamin. As a result of a molecule binding to a nuclear receptor, the nuclear receptor changes the ability of a cell to transcribe DNA, i.e. nuclear receptors modulate the transcription of DNA. However, they can also have transcription independent actions.

Unlike integral membrane receptors and membrane-associated receptors, nuclear receptors reside in either the cytoplasm or nucleus of eukaryotic cells. Thus, nuclear receptors comprise a class of intracellular, soluble, ligand-regulated transcription factors. Nuclear receptors include but are not limited to receptors for androgens, mineralcorticoids, progestins, estrogens, thyroid hormones, vitamin D, retinoids, eicosanoids, peroxisome proliferators and, pertinently, glucocorticoids. Many nuclear receptors, identified by either sequence homology to known receptors (See, e.g., Drewes et al., (1996) *Mol. Cell. Biol.* 16:925-31) or based on their affinity for specific DNA binding sites in gene promoters (See, e.g., Sladek et al., *Genes Dev.* 4:2353-65), have unascertained ligands and are therefore commonly termed "orphan receptors."

Glucocorticoids are an example of a cellular molecule that has been associated with cellular proliferation.

Glucocorticoids are known to induce growth arrest in the G1-phase of the cell cycle in a variety of cells, both *in vivo* and *in vitro,* and have been shown to be useful in the treatment of certain cancers. The glucocorticoid receptor (GR) belongs to an important class of transcription factors that alter the expression of target genes in response to a specific hormone signal. Accumulated evidence indicates that receptor associated proteins play key roles in regulating glucocorticoid signaling. The list of cellular proteins that can bind and co-purify with the GR is constantly expanding.

Glucocorticoids are also used for their anti-inflammatory effect on the skin, joints, and tendons. They are important for treatment of disorders in which inflammation is thought to be caused by immune system activity. Representative disorders of this sort include but are not limited to rheumatoid arthritis, inflammatory bowel disease, glomerulonephritis, and connective tissue diseases like systemic lupus erythmatosus. Glucocorticoids are also used to treat asthma (e.g. fluticasone propionate, a component of the asthma medication ADVAIR$^{TM}$ marketed by GlaxoSmithKline) and are widely used with other drugs to prevent the rejection of organ transplants. Some cancers of the blood (leukemias) and lymphatic system (lymphomas) can also respond to corticosteroid drugs.

Glucocorticoids exert several effects in tissues that express receptors for them. They regulate the expression of several genes either positively or negatively and in a direct or indirect manner. They are also known to arrest the growth of certain lymphoid cells and in some cases cause cell death (Harmon et al., (1979) *J. Cell Physiol.* 98: 267-278; Yamamoto, (1985) *Ann. Rev. Genet.* 19: 209-252; Evans, (1988) *Science* 240:889-895; Beato, (1989) *Cell* 56:335-344; Thompson, (1989) *Cancer Res.* 49: 2259s-2265s.). Due in part to their ability to kill cells, glucocorticoids have been used for decades in the treatment of leukemias, lymphomas, breast cancer, solid tumors and other diseases involving irregular cell growth, e.g. psoriasis. The inclusion of glucocorticoids in chemotherapeutic regimens has contributed to a high rate of cure of certain leukemias and lymphomas which were formerly lethal (Homo-Delarche, (1984) *Cancer Res.* 44: 431-437). Although it is clear that glucocorticoids exert these effects after binding to their receptors, the mechanism of killing cells is not completely understood, although several hypotheses have been proposed. Among the more prominent hypotheses are: the deinduction of critical lymphokines, oncogenes and growth factors; the induction of supposed "lysis genes;" alterations in calcium ion influx; the induction of endonucleases; and the induction of a cyclic AMP-dependent protein kinase (McConkey et al., (1989) *Arch. Biochem. Biophys.* 269: 365-370; Cohen & Duke, (1984) *J. Immunol.* 152: 38-42; Eastman-Reks & Vedeckis, (1986) *Cancer Res.* 46: 2457-2462; Kelso & Munck, (1984) *J. Immunol.* 133:784-791; Gruol et al., (1989) *Molec. Endocrinol.* 3: 2119-2127; Yuh & Thompson. (1989) *J. Biol. Chem.* 264: 10904-10910).

Fluticasone propionate (FP) is a coricosteroid that forms one active component of the GlaxoSmithKline product ADVAIR™, which is indicated for treatment of asthma. Fluticasone propionate is a GR modulator. As an asthma medicine, fluticasone propionate reduces swelling and inflammation inside the lungs of a patient. The precise mechanism of this effect is not presently known. Fluticasone propionate has been found to have an affinity for GR 18 times that of dexamethasone, another commonly employed corticosteroid. The present invention offers some insight into this observed pattern of affinity for GR.

Polypeptides, e.g. the glucocorticoid receptor ligand binding domain, have a three-dimensional structure determined by the primary amino acid sequence and the environment surrounding the polypeptide. This three-dimensional structure establishes the polypeptide's activity, stability, binding affinity, binding specificity, and other biochemical attributes. Thus, knowledge of a protein's three-dimensional structure can provide much guidance in designing agents that mimic, inhibit, or improve its biological activity.

The three-dimensional structure of a polypeptide can be determined in a number of ways. Many of the most precise methods employ X-ray crystallography (See. e.g.. Van Holde, (1971) Physical Biochemistry. Prentice-Hall, New Jersey, pp. 221-39). This technique relies on the ability of crystalline lattices to diffract X-rays or other forms of radiation. Diffraction experiments suitable for determining the three-dimensional structure of macromolecules typically require high-quality crystals. Unfortunately, such crystals have been unavailable for the ligand binding domain of a human glucocorticoid receptor, as well as many other proteins of interest. Thus, high-quality diffracting crystals of the ligand binding domain of a human glucocorticoid receptor in complex with a ligand would greatly assist in the elucidation of its three-dimensional structure.

Clearly, the solved crystal structure of the ligand binding domain of a glucocorticoid receptor polypeptide in complex with a ligand and a co-activator peptide would be useful in the process of the rational design of modulators of activity mediated by the glucocorticoid receptor. Evaluation of the available sequence data shows that GRα is particularly similar to MR, PR and AR. The GRα LBD has approximately 56%, 54% and 50% sequence identity to the MR, PR and AR LBDs, respectively. The GRβ amino acid sequence is identical to the GRα amino acid sequence for residues 1-727, but the remaining 15 residues in GRβ show no significant similarity to the remaining 50 residues in GRα. If no X-ray structure were available for GRα, then one could build a model for GRα using the available X-ray structures of PR and/or AR as templates. These theoretical models have some utility, but cannot be as accurate as a true X-ray structure, such as the X-ray structure disclosed here. Because of their limited accuracy, a model for GRα will generally be less useful than an X-ray structure for the design of agonists, antagonists and modulators of GRα.

Additionally, a solved GRα-co-activator peptide-fluticasone propionate crystal structure would provide structural details and insights necessary to design a modulator of GRα that maximizes preferred requirements for any modulator, i.e. potency and specificity. By exploiting the structural details obtained from a GRα-co-activator peptide-fluticasone propionate crystal structure, it would be possible to design a GRα modulator that, despite GRα's similarity with other steroid receptors and nuclear receptors, exploits the unique structural features of the ligand binding domain of human GRα. A GRα modulator developed using structure-assisted design would take advantage of heretofore unknown GRα structural considerations and thus be more effective than a modulator developed using homology-based design or other GRα structures. Potential or existent homology models or existing crystal structures cannot provide the necessary degree of specificity. A GRα modulator designed using the structural coordinates of a crystalline form of the ligand binding domain of GRα in complex with fluticasone propionate and a co-activator peptide would also provide a starting point for the development of modulators of other nuclear receptors.

Although several journal articles have referred to GR mutants having "increased ligand efficacy" in cell-based assays, it has not been mentioned that such mutants could have improved solution properties so that they could provide a suitable reagent for purification, assay, and crystallization. See Garabedian & Yamamoto, (1992) *Mol. Biol. Cell* 3: 1245-1257; Kralli et al., (1995) *Proc. Natl. Acad. Sci.* 92: 4701-4705; Bohen, (1995) *J. Biol. Chem.* 270: 29433-29438; Bohen, (1998) *Mol. Cell. Biol.* 18: 3330-3339; Freeman et al., (2000) *Genes Dev. 14:* 422-434.

Indeed, it is well documented that GR associates with molecular chaperones (e.g. heat shock proteins (HSPs) such as hsp90, hsc70, and p23). In the past, it has been considered that GR would either not be active or soluble if purified away from these binding partners. In fact, it has even been mentioned that GR must be in complex with hsp90 in order to adopt a high affinity steroid binding conformation. See Xu et al., (1998) *J. Biol. Chem.* 273: 13918-13924; Rajapandi et al., (2000) *J. Biol. Chem.* 275: 22597-22604.

Still other journal articles have reported *E.coli* expression of GST-GR, but also noted a failure to purify the purported polypeptide. See Ohara-Nemoto et al., (1990) *J. Steroid Biochem. Molec. Biol.* 37: 481-490; Caamano et al., (1994) *Annal. NYAcad. Sci.* 746: 68-77.

The structure of GR in complex with dexamethasone was previously solved ("the Dex structure"), the atomic coordinates of which are presented in Table 3. While offering unprecedented insight into the structure of GR in complex with a ligand, this structure does not adequately answer the question surrounding the higher affinity of GR for FP than for dexamethasone. Nor does the GR/Dex structure explain the structural requirements for association of FP with GR and other NRs. For example, examination of the GR/Dex structure initially suggests that the binding pocket of GR, AR, MR and PR is too small to accommodate the FP ligand. Nor can available GR, AR, MR and PR models adequately explain the mode of FP association with these NRs. Examination of these models indicates that the ligand binding pocket is sterically limited in its ability to accommodate FP and other ligands, such as steroidal molecules having large substituents at the C-17α position and non-steroidal molecules having substituents predicted to fill the same space as would be filled by the proprionate group of FP. These larger ligands, including FP, are nonetheless known to bind these NRs, presumably by expanding the ligand binding pocket in some way. Until the disclosure of the present invention, the details of this expansion, including the identity of movements of structural features of a GR protein, were not known, and would have been exceptionally difficult to predict with protein modelling software. A crystal structure of FP in complex with GR would provide insight into the binding of larger ligands to not only GR, but other NRs as well, including AR, MR and PR. Such a structure could also form a basis for the construction of homology models and docking models of these and other nuclear receptors.

Importantly, a GR/FP structure could be employed in modulator design. This structure would be particularly valuable because it would provide insight into the structural features of GR that are involved in binding FP. Since available structures and models cannot adequately account for the binding of FP and certain other ligands and in fact suggest that, based on a steric evaluation of the ligand-receptor interaction, such binding would not be likely to be productive, a solved structure of GR in complex with FP would be of particular value to researchers involved with the rational design of NR modulators, particularly modulators of GR, AR, PR and MR. Further, such a structure could form the basis of one or more homology models and docking models; these models would be particularly valuable since they would account for receptor-specific features that a general NR model could not. The generation of such models would be of assistance in designing receptor-specific modulators.

What is needed, therefore, is a purified, soluble GRα LBD polypeptide in complex with a steroidal ligand having a substituent larger than a hydroxyl group at the C17-α position, preferably also with a co-activator peptide, for use in structural studies, as well as methods for making the same. Such methods would also find application in the preparation of modified NRs in general.

What is also needed is a crystallized form of a GRα ligand binding domain, preferably in complex with fluticasone propionate and a co-activator peptide. Acquisition of crystals of the GRα ligand binding domain polypeptide in complex with fluticasone propionate and a co-activator peptide facilitates a determination of a three-dimensional structure of a GRα ligand binding domain (LBD) polypeptide in the conformation adopted by GRα when it binds fluticasone propionate and a co-activator peptide. Knowledge of this three dimensional structure can facilitate the design of modulators of GR-mediated activity. Such modulators can lead to therapeutic compounds to treat a wide range of conditions,

including inflammation, tissue rejection, auto-immunity, malignancies such as leukemias and lymphomas, Cushing's syndrome, acute adrenal insufficiency, congenital adrenal hyperplasia, rheumatic fever, polyarteritis nodosa, granulomatous polyarteritis, inhibition of myeloid cell lines, immune proliferation/apoptosis, HPA axis suppression and regulation, hypercortisolemia, modulation of the TH1/TH2 cytokine balance, chronic kidney disease, stroke and spinal cord injury, hypercalcemia, hypergylcemia, acute adrenal insufficiency, chronic primary adrenal insufficiency, secondary adrenal insufficiency, congenital adrenal hyperplasia, cerebral edema, thrombocytopenia, Little's syndrome, inflammatory bowel disease, systemic lupus erythematosus, polyartitis nodosa, Wegener's granulomatosis, giant cell arteritis, rheumatoid arthritis, osteoarthritis, hay fever, allergic rhinitis, urticaria, angioneurotic edema, chronic obstructive pulmonary disease, asthma, tendonitis, bursitis, Crohn's disease, ulcerative colitis, autoimmune chronic active hepatitis, organ transplantation, hepatitis, cirrhosis, inflammatory scalp alopecia, panniculitis, psoriasis, discoid lupus erythematosus, inflamed cysts, atopic dermatitis, pyoderma gangrenosum, pemphigus vulgaris, bullous pemphigoid, systemic lupus erythematosus, dermatomyositis, herpes gestationis, eosinophilic fasciitis, relapsing polychondritis, inflammatory vasculitis, sarcoidosis, Sweet's disease, type 1 reactive leprosy, capillary hemangiomas, contact dermatitis, atopic dermatitis, lichen planus, exfoliative dermatitus, erythema nodosum, acne, hirsutism, toxic epidermal necrolysis, erythema multiform, cutaneous T-cell lymphoma. Other applications of a GR modulator developed in accordance with the present invention can be employed to treat Human Immunodeficiency Virus (HIV), cell apoptosis, and can be employed in treating cancerous conditions including, but not limited to, Kaposi's sarcoma, immune system activation and modulation, desensitization of inflammatory responses, IL-1 expression, natural killer cell development, lymphocytic leukemia, treatment of retinitis pigmentosa. Other applications for such a modulator comprise modulating cognitive performance, memory and learning enhancement, depression, addiction, mood disorders, chronic fatigue syndrome, schizophrenia, stroke, sleep disorders, anxiety, immunostimulants, repressors, wound healing and a role as a tissue repair agent or in anti-retroviral therapy.

## Summary of the Invention

A crystalline GR polypeptide complex comprising an expanded binding pocket is disclosed. Preferably, the crystalline form has lattice constants of of $a = b = 127.656$ Å, $c = 87.725$ Å, $\alpha = 90°$, $\beta = 90°$, $\gamma = 120°$. Preferably, the crystalline form is a hexagonal crystalline form. More preferably, the crystalline form has a space group of $P6_1$. Even more preferably, the GR ligand binding domain polypeptide comprises the amino acid sequence shown in SEQ ID NOs: 6 and 8. Even more preferably, the GR ligand binding domain has a crystalline structure further characterized by the coordinates corresponding to Table 2.

Preferably, the GR polypeptide complex comprises a ligand and a co-activator peptide. Optionally, the crystalline form contains two GR ligand binding domain polypeptides in the asymmetric unit. Preferably, the crystalline form is such that the three-dimensional structure of the crystallized GR ligand binding domain polypeptide can be determined to a resolution of about 3.0 Å or better. Even more preferably, the crystalline form contains one or more atoms having a molecular weight of 40 grams/mol or greater.

A method for determining the three-dimensional structure of a crystallized GR polypeptide complex comprising an expanded binding pocket to a resolution of about 3.0 Å or better is disclosed. In a preferred embodiment, the method comprises: (a) crystallizing a GR ligand binding domain polypeptide; and (b) analyzing the GR ligand binding domain polypeptide to determine the three-dimensional structure of the crystallized GR ligand binding domain polypeptide, whereby the three-dimensional structure of a crystallized GR polypeptide complex comprising an expanded binding pocket is determined to a resolution of about 3.0 Å or better.

Preferably, the complex comprises a ligand, preferably fluticasone propionate, and a co-activator peptide, preferably a TIF2 peptide. It is also preferable that the GR ligand binding domain polypeptide comprises the amino acid sequence of SEQ ID NOs: 6 and 8, and that the TIF2 peptide comprises SEQ ID NO: 9. Even more preferably, the three-dimensional structure is further characterized by the coordinates corresponding to Table 2.

A method of generating a crystallized GR polypeptide complex comprising an expanded binding pocket and a ligand known or suspected to be unable to associate with a known GR structure is disclosed. In a preferred embodiment, the method comprises: (a) providing a solution comprising a GR polypeptide and a ligand known or suspected to be unable to associate with a known GR structure; and (b) crystallizing the GR ligand binding domain polypeptide using the hanging drop method, whereby a crystallized GR polypeptide complex comprising an expanded binding pocket and a ligand known or suspected to be unable to associate with a known GR structure is generated.

Preferably, the complex comprises a ligand, preferably fluticasone propionate, and a co-activator peptide, preferably a TIF2 peptide. It is also preferable that the GR ligand binding domain polypeptide comprises the amino acid sequence of SEQ ID NOs: 6 or 8, and that the TIF2 peptide comprises SEQ ID NO: 9. Even more preferably, the complex is further characterized by the coordinates corresponding to Table 2.

A method for identifying a GR modulator is disclosed. In a preferred embodiment, the method comprises: (a) providing atomic coordinates of a GR polypeptide complex comprising an expanded binding pocket to a computerized

modeling system; and (b) modeling a ligand that fits spatially into the large pocket volume of the GR polypeptide complex to thereby identify a GR modulator.

Preferably, the complex comprises a ligand, preferably fluticasone propionate, and a co-activator peptide, preferably a TIF2 peptide. It is also preferable that the GR polypeptide comprises the amino acid sequence of SEQ ID NOs: 6 or 8, and that the TIF2 peptide comprises SEQ ID NO: 9. Even more preferably, the complex is further characterized by the coordinates corresponding to Table 2.

A method of designing a modulator that selectively modulates the activity of a GRα polypeptide comprising an expanded binding pocket is disclosed. In a preferred embodiment, the method comprises: (a) providing a crystalline form of a GRα polypeptide complex comprising an expanded binding pocket; (b) determining the three-dimensional structure of the crystalline form of the GRα ligand binding domain polypeptide; and (c) synthesizing a modulator based on the three-dimensional structure of the crystalline form of the GRα ligand binding domain polypeptide, whereby a modulator that selectively modulates the activity of a GRα polypeptide comprising an expanded binding pocket is designed.

Preferably, the complex comprises a ligand, preferably fluticasone propionate, and a co-activator peptide, preferably a TIF2 peptide. It is also preferable that the GR ligand binding domain polypeptide comprises the amino acid sequence of SEQ ID NOs: 6 or 8, and that the TIF2 peptide comprises SEQ ID NO: 9. Even more preferably, the three-dimensional structure is further characterized by the coordinates corresponding to Table 2.

A method of forming a homology model of an NR is disclosed. In a preferred embodiment, the method comprises: (a) providing a template amino acid sequence comprising a GR polypeptide comprising an expanded binding pocket; (b) providing a target NR amino acid sequence; (c) aligning the target sequence and the template sequence to form a homology model.

Preferably, the GR polypeptide comprises the amino acid sequence of SEQ ID NOs: 6 or 8, and that the TIF2 peptide comprises SEQ ID NO: 9.

A method of designing a modulator of a nuclear receptor is disclosed. In a preferred embodiment, the method comprises: (a) designing a potential modulator of a nuclear receptor that will make interactions with amino acids in the ligand binding site of the nuclear receptor based upon atomic structure coordinates of a NR polypeptide complex comprising an expanded binding pocket; (b) synthesizing the modulator; and (c) determining whether the potential modulator modulates the activity of the nuclear receptor, whereby a modulator of a nuclear receptor is designed.

Preferably, the complex comprises a ligand, preferably fluticasone propionate, and a co-activator peptide, preferably a TIF2 peptide. It is also preferable that the NR polypeptide comprises the amino acid sequence of SEQ ID NOs: 6 or 8, and that the TIF2 peptide comprises SEQ ID NO: 9. Even more preferably, the atomic structural coordinates are further characterized by the coordinates corresponding to Table 2.

A method of modeling an interaction between an NR and a non-steroid ligand is disclosed. In a preferred embodiment, the method comprises: (a) providing a homology model of a target NR generated using a crystalline GR polypeptide complex comprising an expanded binding pocket; (b) providing atomic coordinates of a non-steroid ligand; and (c) docking the non-steroid ligand with the homology model to form a NR/ligand model.

Preferably, the complex comprises a ligand, preferably fluticasone propionate, and a co-activator peptide, preferably a TIF2 peptide. It is also preferable that the GR polypeptide comprises the amino acid sequence of SEQ ID NOs: 6 or 8, and that the TIF2 peptide comprises SEQ ID NO: 9. Even more preferably, the complex is further characterized by the coordinates corresponding to Table 2.

A method of designing a non-steroid modulator of a target NR using a homology model is disclosed. In a preferred embodiment, the method comprises: (a) modeling an interaction between a target NR and a non-steroid ligand using a homology model generated using a crystalline GR polypeptide complex comprising an expanded binding pocket; (b) evaluating the interaction between the target NR and the non-steroid ligand to determine a first binding efficiency; (c) modifying the structure of the non-steroid ligand to form a modified ligand; (d) modeling an interaction between the modified ligand and the target NR; (e) evaluating the interaction between the target NR and the modified ligand to determine a second binding efficiency; and (f) repeating steps (c)-(e) a desired number of times if the second binding efficiency is less than the first binding efficiency.

Preferably, the complex comprises a ligand, preferably fluticasone propionate, and a co-activator peptide, preferably a TIF2 peptide. It is also preferable that the GR polypeptide comprises the amino acid sequence of SEQ ID NOs: 6 or 8, and that the TIF2 peptide comprises SEQ ID NO: 9. Even more preferably, the complex is further characterized by the coordinates corresponding to Table 2.

A data structure embodied in a computer-readable medium is disclosed. In a preferred embodiment, the data structure comprises: a first data field containing data representing spatial coordinates of an NR LBD comprising an expanded binding pocket, wherein the first data field is derived by combining at least a part of a second data field with at least a part of a third data field, and wherein (a) the second data field contains data representing spatial coordinates of the atoms comprising a GR LBD comprising an expanded binding pocket in complex with a ligand; and (b) the third data field contains data representing spatial coordinates of the atoms comprising a NR LBD. Preferably, the data of

the third data field comprises data selected from the data embodied in one of Table 3, Table 8, Table 9 and Table 10. It is also preferable that the GR LBD comprises the amino acid sequence of SEQ ID NOs: 6 or 8, and that the TIF2 peptide comprises SEQ ID NO: 9. Even more preferably, the complex is further characterized by the coordinates corresponding to Table 2.

A method for designing a homology model of the ligand binding domain of an NR wherein the homology model may be displayed as a three-dimensional image. In a preferred embodiment, the method comprises: (a) providing an amino acid sequence and an crystallographic structure of the ligand binding domain of a GRα polypeptide, (b) modifying said crystallographic structure to take account of differences between the amino acid configuration of the ligand binding domains of the NR on the one hand and the GRα polypeptide on the other hand, (c) verifying the accuracy of the homology model by comparing it with experimentally-determined NR protein and ligand properties, and if required, modifying the homology model for greater consistency with those binding properties.

A computational method of iteratively generating a homology model of the ligand binding domain of an NR, wherein the homology model is capable of being displayed as a three-dimensional image is disclosed. In a preferred embodiment, the method comprises: (a) entering into a computer a machine readable representation of an amino acid sequence of a ligand binding domain of a target NR polypeptide and a machine readable representation of a crystallographic structure of a ligand binding domain of a GRα polypeptide; (b) identifying a difference between an amino acid configuration of a ligand binding domain of a target NR and a GRα polypeptide; (c) modifying the machine readable representation of the crystallographic structure based on a difference identified in step (b) to thereby form a modified crystallographic structure; (d) comparing the modified crystallographic structure with an experimentally-determined property of one of the target NR and a ligand of the target NR; and (e) repeating steps (b) and (d) a desired number of times.

Accordingly, it is an object of the present invention to provide a three dimensional structure of the ligand binding domain of a GR. The object is achieved in whole or in part by the present invention.

An object of the invention having been stated hereinabove, other objects will be evident as the description proceeds, when taken in connection with the accompanying Drawings and Laboratory Examples as best described hereinbelow.

Brief Description of the Drawings

Figure 1 is an autoradiogram of a polyacrylamide gel depicting the isolation of a GR mutant of the present invention. In this figure, Lane 1 contains the insoluble pellet fraction. Lane 2 contains the soluble supernatant fraction. Lane 3 contains pooled eluent from the initial $Ni^{2+}$ column. Lane 4 contains the sample after thrombin digestion. Lane 5 contains the flow through fraction after reload of the $Ni^{2+}$ column. Lane 6 contains the protein after anion exchange. The positions of molecular mass (kDa) markers are indicated on the left side of the figure. Figure 2 is a ribbon diagram showing an overview of the GR/TIF2/FP dimer complex. The ribbon representation of the two GR LBD is shown with gray and white, respectively, with the N-terminus and the C-terminus of the protein indicated. The fluticasone propionate molecules (FP) and TIF2 coactivator motifs are also identified.

Figure 3 is an electron density map (gray net) for the FP ligand and the surrounding residues (white sticks). The map was calculated with the 2Fo-Fc coefficient and is shown with 1 sigma cutoff. The propionate group of the FP molecule is also indicated.

Figure 4 is a ribbon diagram depicting the superposition of the GR/TIF2/FP and the GR/TIF2/Dex structures and showing the expanded binding pocket formed by rearrangement of helices 3, 6, 7 and 10, and the loop preceeding the AF-2 helix. Arrows indicate structural changes that expand the GR pocket to form an expanded binding pocket.

Figure 5A is a cartoon showing a semi-transparent surface representing the available pocket volume in GR subunit A in the GR/TIF2/Dex structure. Residues that surround the pocket are also presented.

Figure 5B is a cartoon showing a semi-transparent surface representing the available pocket volume in GR subunit B in the GR/TIF2/Dex structure. Residues that surround the pocket are also presented.

Figure 6A is a cartoon showing the expanded ligand-binding pocket of GR subunit A in the GR/TIF2/FP structure by a semi-transparent surface representing the available pocket volume. Residues that surround the pocket are also presented.

Figure 6B is a cartoon showing the expanded ligand-binding pocket of GR subunit B in the GR/TIF2/FP structure by a semi-transparent surface representing the available pocket volume. Residues that surround the pocket are also presented.

Figure 7A is a cartoon that uses a semi-transparent surface to show the extra pocket volume that is available to a ligand in the GR/TIF2/FP structure but is not available in the GR/TIF2/Dex structure. Residues around the pocket are also shown. In this figure GR subunit A is depicted.

Figure 7B is a cartoon that uses a semi-transparent surface to show the extra pocket volume that is available to a ligand in the GR/TIF2/FP structure but not available in the GR/TIF2/Dex structure. The surface was generated in the same manner as in Figure 7A. Key residues around the pocket are also shown. In this figure GR subunit B is depicted.

Figure 8A is a schematic representation of molecular interactions between the bound FP ligand and residues in subunit A of the GR protein. The dashed lines depict some of the significant interactions of 5.0 angstroms or less, although several less important interactions have been omitted for clarity.

Figure 8B is a schematic representation of molecular interactions between the bound FP ligand and residues in subunit B of the GR protein. The dashed lines depict some of the significant interactions of 5.0 angstroms or less, although several less important interactions have been omitted for clarity.

Figure 9 is a docking model of the Schering ligand, benzoxazin-1-one, bound to a GR LBD model derived from the GR/TIF2/FP crystal structure. The ligand is shown with a CPK drawing.

Figure 10 is a stick drawing of the ligand binding pocket of the GR structural model showing various interactions between the benzoxazin-1-one ligand and the amino acid residues that comprise the binding pocket.

Figure 11 is an orthogonal view of Figure 9 and illustrates the fitting of the p-fluorophenolic side chain of the benzoxazin-1-one into the expanded binding pocket of the GR structural model.

Figure 12 is a depiction of the overlay of the GR/TIF2/Dex crystal structure (grey) with the GR/benzoxazin-1-one model (white) comparing the geometries of the ligands and the relative locations of the amino acid side chains that comprise the GR expanded binding pocket.

Figure 13 a docking model of the A-222977 ligand bound to a GR LBD model generated using the GR/TIF2/FP crystal structure. The ligand is shown as a CPK drawing.

Figure 14 is a stick drawing of the ligand binding pocket of the GR structural model showing key interactions between A-222977 and the amino acid residues that comprise the binding pocket.

Figure 15 is an orthogonal view of Figure 13 and illustrates the protrusion of methyl-sulfonyl-methoxyl-phenyl side chain of A-222977 into the expanded binding pocket of the GR structural model.

Figure 16 is a depiction of the overlay of the GR/Dex crystal structure (grey) with the GR/A-222977 (white) comparing the geometries of the ligands and the relative locations of the amino acid side chains that comprise the GR expanded binding pocket. Figure 17 is a sequence alignment of amino acid residues comprising the ligand binding domains of GR, MR, PR and AR.

Figure 18A is a ribbon drawing depicting the AR LBD homology model derived from the GR/TIF2/FP crystal structure

Figure 18B is a ribbon diagram depicting a known AR/DHT LBD crystal structure; the ligand binding pocket, rendered as a solid surface, reveals no additional volume and no expanded binding pocket.

Figure 19 is a ribbon drawing of a docking model of bicalutamide bound to the LBD of the AR homology model derived from the GR/TIF2/FP crystal stucture. The ligand is shown in a CPK drawing.

Figure 20 is an orthogonal view of the structure depicted in Figure 18A and shows the LBD of the AR homology model in complex with bicalutamide.

Figure 21 is a stick drawing of the ligand binding pocket of the AR homology model showing interactions between bicalutamide and the amino acid residues that comprise the binding pocket.

Figure 22 is an orthogonal view of Figure 20 and illustrates the protrusion of the *p*-fluorophenyl group of bicalutamide into the expanded binding pocket of the AR homology model.

Figure 23A is a ribbon drawing depicting the PR LBD homology model derived from the GR/TIF2/FP crystal structure; the PR ligand binding pocket, which is rendered as a solid surface, comprises an additional extension, similar to the additional volume of the GR expanded binding pocket.

Figure 23B is a ribbon diagram depicting a known PR/PG LBD crystal structure; the ligand binding pocket, rendered as a solid surface, reveals no expanded binding pocket.

Figure 24 is a ribbon drawing of a docking model of RWJ-60130 bound to the LBD of the PR homology model derived from the GR/TIF2/FP crystal structure. The ligand is shown in a CPK drawing.

Figure 25 is an orthogonal view of Figure 23 showing the LBD of the PR homology model bound with RWJ-60130.

Figure 26 is a stick drawing of the ligand binding pocket of the PR homology model showing interactions between RWJ-60130 and the amino acid residues that comprise the binding pocket.

Figure 27 is an orthogonal view of Figure 25 and illustrates the protrusion of the p-fiodophenyl group of RWJ-60130 into the expanded binding pocket of the PR homology model.

Figure 28A is a ribbon drawing depicting an MR LBD homology model derived from the GR/TIF2/FP crystal structure; the MR ligand binding pocket, which is rendered as a solid surface, contains an additional extension, similar to that found in the GR expanded binding pocket.

Figure 28B is a ribbon drawing depicting an MR LBD homology model derived from the GR/TIF2/FP crystal structure; the PR ligand binding pocket, which is rendered as a solid surface, contains a smaller side pocket, similar to

the GR/Dex ligand binding pocket, which does not show the presence of an expanded binding pocket.

Brief Description of Sequences in the Sequence Listing

SEQ ID NOs: 1 and 2 are, respectively, a DNA sequence encoding a wild type full-length human glucocorticoid receptor (GenBank Accession No. 31679) and the amino acid sequence (GenBank Accession No. 121069) of a human glucocorticoid receptor encoded by the DNA sequence.

SEQ ID NOs: 3 and 4 are, respectively, a DNA sequence encoding a F602S full-length human glucocorticoid receptor and the amino acid sequence of a human glucocorticoid receptor encoded by the DNA sequence.

SEQ ID NOs: 5 and 6 are, respectively, a DNA sequence encoding a wild type ligand binding domain of a human glucocorticoid receptor and the amino acid sequence of a human glucocorticoid receptor encoded by the DNA sequence.

SEQ ID NOs: 7 and 8 are, respectively, a DNA sequence encoding a ligand binding domain (residues 521-777) of a human glucocorticoid receptor containing a phenylalanine to serine mutation at residue 602 and the amino acid sequence of a human glucocorticoid receptor encoded by the DNA sequence.

SEQ ID NO: 9 is an amino acid sequence of amino acid residues 740-753 of the human TIF2 protein.

SEQ ID NO: 10 is an LXXLL motif of a human TIF2 protein.

SEQ ID NO: 11 is an LLRYLL motif of a human TIF2 protein.

Detailed Description of the Invention

The present invention discloses a crystal stucture of a ligand binding domain of GR in complex with a fluticasone propionate ligand and a peptide derived from the co-actiavtor TIF2. This structure reveals an expanded binding pocket comprising additional volume that accommodates the propionate moiety of the FP ligand. The presence of this additional volume is not observed in previous known GR/ligand structures, such as the structure of GR in complex with dexamethasone (characterized by the atomic coordinates of Table 3). The presence of the additional volume in the ligand binding pocket, which contributes to an "expanded binding pocket," accounts for observed ligand binding modes and can form the basis of homology models of GR and other nuclear receptors, including an androgen receptor, a progesterone receptor and a mineralcorticoid receptor. These homology models also form aspects of the present invention. Additionally, the expanded binding pocket can contribute to docking models that can be employed to understand and clarify the binding of a ligand to a nuclear receptor. Such homology and docking models can be employed in the design of nuclear receptor modulators.

The present invention provides for the generation of a complex comprising a soluble GR LBD bound to fluticasone propionate and a TIF2 co-activator peptide. The present invention also provides for the ability to crystallize the above complex and to determine its crystal structure. The GR LBD employed in the present invention comprises a single F602S mutation at residue 602. Thus, an aspect of the present invention comprises the use of both targeted and random mutagenesis of the GR gene to produce a recombinant protein with improved solution characteristics for the purposes of, for example, crystallization, characterization of biologically relevant protein-protein interactions, and compound screening assays. The present invention, which relates to GR LBD mutation F602S as well as other LBD mutations, demonstrates that GR can be overexpressed using an *E.coli* expression system and that active GR protein can be purified, assayed, and crystallized.

Until disclosure of the present invention presented herein, the ability to obtain crystalline forms of the ligand binding domain of GR (e.g. GRα) in complex with fluticasone propionate and a co-activator peptide has not been realized. And until disclosure of the present invention presented herein, a detailed three-dimensional crystal structure of a GRα LBD polypeptide in complex with fluticasone propionate and a co-activator peptide has not been solved. Moreover, nuclear receptor structures known in the art do not comprise an expanded binding pocket and therefore cannot fully explain the observed binding of some known ligands to various NRs.

In another aspect, the present invention provides for the generation of NR, SR and GR polypeptides and NR, SR or GR mutants (preferably GRα and GRα LBD mutants), and the ability to solve the crystal structures of those that crystallize. Indeed, a GRα LBD having a point mutation was crystallized and solved in one aspect of the present invention. Thus, an aspect of the present invention involves the use of both targeted and random mutagenesis of the GR gene for the production of a recombinant protein with improved solution characteristics for the purpose of crystallization, characterization of biologically relevant protein-protein interactions, and compound screening assays. The present invention, relating to GR LBD F602S and other LBD mutations, shows that GR can be overexpressed using an *E.coli* expression system and that active GR protein can be purified, assayed, and crystallized.

In addition to providing structural information, crystalline polypeptides provide other advantages. For example, the crystallization process itself further purifies the polypeptide, and satisfies one of the classical criteria for homogeneity. In fact, crystallization frequently provides unparalleled purification quality, removing impurities that are not removed by other purification methods such as HPLC, dialysis, conventional column chromatography, and other methods. Moreover, crystalline polypeptides are sometimes stable at ambient temperatures and free of protease contamination and other degradation associated with solution storage. Crystalline polypeptides can also be useful as pharmaceutical preparations. Finally, crystallization techniques in general are largely free of problems such as denaturation associated with other stabilization methods (e.g., lyophilization). Once crystallization has been accomplished, crystallographic data provides useful structural information that can assist the design of compounds that can serve as modulators (e.g. agonists or antagonists), as described herein below. In addition, the crystal structure provides information useful to map a ligand binding site, which can then be mimicked by a chemical entity that can serve as an antagonist or agonist.

I. Definitions

Following long-standing patent law convention, the terms "a" and "an" mean "one or more" when used in this application, including the claims.

As used herein, the term "about," when referring to a value or to an amount of mass, weight, time, volume, concentration or percentage is meant to encompass variations of ±20% or ±10%, more preferably ±5%, even more preferably ±1%, and still more preferably ±0.1% from the specified amount, as such variations are appropriate to perform the disclosed method.

As used herein, the terms "active position of the AF2 helix" and "active conformation of the AF2 helix" are used interchangeably and mean an AF2 helix having a position and/or orientation similar to that of an AF2 helix in a GR/TIF2/FP structure (e.g. as characterized by the atomic structural coordinates of Table 2), or similar to that of an AF2 helix in a GR/TIF2/Dex structure (e.g. as characterized by the atomic structural coordinates of Table 3). For example, with respect to GR, the "active position" is further characterized in GR by contacts between Leu757 in the AF2 helix and Trp600, Cys736, Phe737 and Phe740 in helices 5, 11, 11 and 11, respectively. The position and/or orientation of an AF2 helix in a structure comprising GR can be compared with that of an AF2 helix in a structure comprising a GR/FP complex by rotating and/or translating the GR structure so as to superimpose the backbone atoms of helices 1 through 10 onto the corresponding backbone atoms of helices 1 through 10 of a GR/TIF2/FP structure. A similar procedure can be employed to compare a structure of GR with that of another nuclear receptor, such as ERα or ERβ. If, after superimposition, a majority of the backbone atoms of the core of the AF2 helix of the GR structure, (e.g. residues 752-757), lie within 1.0 angstroms of the position of corresponding backbone atoms of the AF2 helix of the GR/FP structure, then the AF2 helix is defined as being in an active position or active conformation. If more than half of the atoms lie more than 1.0 angstroms from their counterparts in the GR/FP structure, then the AF2 helix is considered to be in a position or conformation different from the active position or conformation.

In some cases, the AF2 helix might be disordered, or dynamically mobile. If several of the backbone atoms of the AF2 helix residues 752-757 are disordered so that they are not clearly defined in the electron density of an X-ray crystallographic experiment, then the AF2 helix as a whole is defined as assuming multiple positions and/or conformations. This ensemble of alternative positions or conformations might include positions or conformations that could be characterized as "active positions" or "active conformations." However, the disorder indicates that the "active position" or "active conformation" does not constitute an adequate fraction of the ensemble, and in this case the AF2 helix cannot be considered to be in the "active position" or "active conformation".

Other examples of a nuclear receptor where the AF2 helix is in an "active position" include the X-ray structures of the estrogen receptor α (ERα) bound to estradiol (Brzozowski et al., (1997) *Nature* 389:753) and diethylstilbesterol (DES) (Shiau et al., (1998) *Cell* 95:927). Examples of a nuclear receptor where the AF2 helix is not in an "active position" are the X-ray structures of the estrogen receptor α (ERα) bound to raloxifene (Brzozowski et al., (1997) *Nature* 389:753) and tamoxifen (Shiau et al., (1998) *Cell* 95:927). Binding of coactivator, and AF2-dependent activation of gene transcription, normally requires that the AF2 helix be in the "active position" (Nolte et al., (1998) *Nature* 395:137; Shiau et al., (1998) *Cell* 95:927). This creates a "charge-clamp" structure that holds the coactivator in its required position (Nolte et al., (1998) *Nature* 395:137). GR antagonists, such as RU-486, would be expected to displace the AF2 helix out of the "active position" and into some other position, such as the coactivator binding site as seen with raloxifene and tamoxifen in ERα (Brzozowski et al., (1997) *Nature* 389:753; Shiau et al., (1998) *Cell* 95:927).

The movement of the AF2 helix often induces other conformational changes in the protein that might not be compatible with agonist binding or activation of transcription. Also, the movement of the AF2 helix leaves the ligand binding pocket open to the exterior of the protein. These conformational modifications can make the structure unsuitable for structure-based design and docking calculations where the goal is the design of agonists or modulators where the protein remains predominantly in or near the active conformation.

As used herein, the term "agonist" means an agent that supplements or potentiates the bioactivity of a functional gene or protein or of a polypeptide encoded by a gene that is up- or down-regulated by a polypeptide and/or a polypeptide encoded by a gene that contains a binding site or response element in its promoter region. By way of specific example, an "agonist' is a compound that interacts with the steroid hormone receptor to promote a transcriptional response. An agonist can induce changes in a receptor that places the receptor in an active conformation that allows them to influence transcription, either positively or negatively. There can be several different ligand-induced changes in the receptor's conformation. The term "agonist" specifically encompasses partial agonists.

As used herein, the terms "α-helix", "alpha-helix" and "alpha helix" are used interchangeably and mean the conformation of a polypeptide chain wherein the polypeptide backbone is wound around the long axis of the molecule in a left-handed or right-handed direction, and the R groups of the amino acids protrude outward from the helical backbone, wherein the repeating unit of the structure is a single turnoff the helix, which extends about 0.56 nm along the long axis.

As used herein, the term "antagonist" means an agent that decreases or inhibits the bioactivity of a functional gene or protein, or that decrease or inhibit the bioactivity of a naturally occurring or engineered non-functional gene or protein. Alternatively, an antagonist can decrease or inhibit the bioactivity of a functional gene or polypeptide encoded by a gene that is up- or down-regulated by a polypeptide and/or contains a binding site or response element in its promoter region. An antagonist can also decrease or inhibit the bioactivity of a naturally occurring or engineered non-functional gene or polypeptide encoded by a gene that is up- or down-regulated by a polypeptide, and/or contains a binding site or response element in its promoter region. By way of specific example, an "antagonist" is a compound that interacts with the steroid hormone receptor to inhibit a transcriptional response. An antagonist can bind to a receptor but fail to induce conformational changes that alter the receptor's transcriptional regulatory properties or physiologically relevant conformations. Binding of an antagonist can also block the binding and therefore the actions of an agonist. The term "antagonist" specifically encompasses partial antagonists.

As used herein, the terms "backbone" and "backbone atoms" are the N, Ca, C and O atoms of a protein that are common to all twenty of the amino acids normally present in a protein. See G. E. Schulz and R. H. Schirmer, Principles of Protein Structure, Springer-Verlag, New York.

As used herein, the terms "β-sheet", "beta-sheet" and "beta sheet" are used interchangeably and mean the conformation of a polypeptide chain stretched into an extended zig-zig conformation. Portions of polypeptide chains that run "parallel" all run in the same direction. Polypeptide chains that are "antiparallel" run in the opposite direction from the parallel chains.

As used herein, the terms "binding pocket of an NR ligand binding domain", "NR ligand binding pocket," "NR ligand binding pocket" and "NR binding pocket" are used interchangeably, and refer to the large cavity within the NR ligand binding domain where a ligand can bind. This cavity can be empty, or can contain water molecules or other molecules from the solvent, or can contain ligand atoms. The binding pocket includes regions of space near the "main" binding pocket that not occupied by atoms of the NR but that are near the "main" binding pocket, and that are contiguous with the "main" binding pocket. For GR, the main binding pocket comprises the region of space encompassed by the residues shown in Figure 8.

As used herein, the term "biological activity" means any observable effect flowing from interaction between an NR (preferably a GR) polypeptide and a ligand. Representative, but non-limiting, examples of biological activity in the context of the present invention include transcription regulation, ligand binding and peptide binding.

As used herein, the terms "candidate substance" and "candidate compound" are used interchangeably and refer to a substance that is believed to interact with another moiety, for example a given ligand that is believed to interact with a complete target NR (preferably a GR) polypeptide or fragment thereof, and which can be subsequently evaluated for such an interaction. Representative candidate substances or compounds include xenobiotics such as drugs and other therapeutic agents, carcinogens and environmental pollutants, natural products and extracts, as well as endobiotics such as glucocorticosteroids, steroids, fatty acids and prostaglandins. Other examples of candidate compounds that can be investigated using the methods of the present invention include, but are not restricted to, agonists and antagonists of a GR polypeptide or other polypeptide, toxins and venoms, viral epitopes, hormones (e. g., glucocorticosteroids, opioid peptides, steroids, etc.), hormone receptors, peptides, enzymes, enzyme substrates, co-factors, lectins, sugars, oligonucleotides or nucleic acids, oligosaccharides, proteins, small molecules and monoclonal antibodies.

As used herein, the terms "cells," "host cells" or "recombinant host cells" are used interchangeably and mean not only to the particular subject cell, but also to the progeny or potential progeny of such a cell. Because certain modifications can occur in succeeding generations due to either mutation or environmental influences, such progeny might not, in fact, be identical to the parent cell, but are still included within the scope of the term as used herein.

As used herein, the terms "chimeric protein" or "fusion protein" are used interchangeably and mean a fusion of a first amino acid sequence encoding a target polypeptide with a second amino acid sequence defining a polypeptide domain foreign to, and not homologous with, any domain of a target polypeptide. A chimeric protein can include a foreign domain that is found in an organism that also expresses the first protein, or it can be an "interspecies" or "intergenic" fusion of protein structures expressed by different kinds of organisms. In general, a fusion protein can be represented by the general formula X--target--Y, wherein "target" represents a portion of the protein that is derived from a target polypeptide, and X and Y are independently absent or represent amino acid sequences that are not

related to a target sequence in an organism, including naturally occurring mutants. Representative target polypeptides include, but are not limited to, GR, AR, MR, PR and other NRs.

As used herein, the term "co-activator" means an entity that has the ability to enhance transcription when it is bound to at least one other entity. The association of a co-activator with an entity has the ultimate effect of enhancing the transciption of one or more sequences of DNA. In the context of the present invention, transcription is preferably nuclear receptor-mediated. By way of specific example, in the present invention TIF2 (the human analog of mouse glucocorticoid receptor interaction protein 1 (GRIP1)) can bind to a site on the glucorticoid receptor, an event that can enhance transcription. TIF2 is therefore a co-activator of the glucocorticoid receptor. Other GR co-activators can include SRC1.

As used herein, the term "co-repressor" means an entity that has the ability to repress transcription when it is bound to at least one other entity. In the context of the present invention, transcription is preferably nuclear receptor-mediated. The association of a co-repressor with an entity has the ultimate effect of repressing the transciption of one or more sequences of DNA.

As used herein, the term "crystal lattice" means the array of points defined by the vertices of packed unit cells.

As used herein, the term "detecting" means confirming the presence of a target entity by observing the occurrence of a detectable signal, such as a radiologic or spectroscopic signal that will appear exclusively in the presence of the target entity.

As used herein, the term "DNA segment" means a DNA molecule that has been isolated free of total genomic DNA of a particular species. In a preferred embodiment, a DNA segment encoding a GR polypeptide refers to a DNA segment that comprises any of SEQ ID NOs: 1, 3, 5 and 7, but can optionally comprise fewer or additional nucleic acids, yet is isolated away from, or purified free from, total genomic DNA of a source species, such as *Homo sapiens.* Included within the term "DNA segment" are DNA segments and smaller fragments of such segments, and also recombinant vectors, including, for example, plasmids, cosmids, phages, viruses, and the like.

As used herein, the term "DNA sequence encoding a GR polypeptide" can refer to one or more coding sequences within a particular individual. Moreover, certain differences in nucleotide sequences can exist between individual organisms, which are called alleles. It is possible that such allelic differences might or might not result in differences in the amino acid sequence of the encoded polypeptide yet still encode a protein with the same biological activity. As is well known, genes for a particular polypeptide can exist in single or multiple copies within the genome of an individual. Such duplicate genes can be identical or can have certain modifications, including nucleotide substitutions, additions or deletions, all of which still code for polypeptides having substantially the same activity.

As used herein, the phrase "enhancer-promoter" means a composite unit that contains both enhancer and promoter elements. An enhancer-promoter is operatively linked to a coding sequence that encodes at least one gene product.

As used herein, the term "expanded binding pocket" means an NR ligand binding pocket in which atoms in the protein have shifted so as to increase the volume available to the ligand. The GR/FP structure disclosed in Table 2 provides an example in which, in the A-subunit, the pocket volume increases by approximately 58 cubic angstroms compared with the corresponding subunit of the GR/Dex structure, as described in Table 3, and in which, in the B-subunit, the pocket volume increases by approximately 138 cubic angstroms compared with the corresponding subunit of the GR/Dex structure. In this example, the expansion in the pocket volume is due to movements in atoms comprising residues M560, L563, M639, Q642, M646, and Y735.

Although a GR expanded binding pocket has been described, other NRs can also comprise an expanded binding pocket. For example, residues that are homologous to those listed for GR (i.e. M560, L563, M639, Q642, M646, and Y735) can be sterically displaced in other NRs. Figure 17, which depicts an alignment of several NRs, can be employed to identify residues homologous to those identified for GR. Figures 8A and 8B identify residues of GR subunit A and subunit B, respectively, that interact with an FP ligand. Steric displacement of any residue in an NR that is homologous to those identified in Figures 8A and 8B for a given NR can also contribute to an expanded binding pocket. Thus, an expanded binding pocket can be formed by steric displacement of one or more residues homologous to the GR residues identified in Figures 8A, 8B and 17.

An expanded binding pocket can also be characterized in terms of steric displacement of secondary structure elements. Referring again to GR, when FP is bound to the ligand binding site, helices 3, 6, 7, 10 and the loop preceding the AF-2 helix are sterically displaced, leading to an increase in pocket volume as compared with a GR/Dex structure, as characterized by the atomic coordinates of Table 3. Displacement of homologous secondary structure in other NRs can lead to an increase in the pocket volume. Figure 17 identifies homologous secondary structure for several nuclear receptors.

An expanded NR binding pocket comprises a greater volume than the ligand binding pocket volume in other structures of the same NR. The term "binding pocket volume," which refers to the volume of a binding pocket further defines the term "expanded binding pocket," can also be characterized by reference to the following Table of Pocket Volume Data, which tabulates some representative pocket volumes. In the Table of Pocket Volume Data, pocket volumes were calculated with the program GRASP, using a grid spacing of 0.20 angstroms for construction of the molecular surface, with the atomic radius values of Bondi (Bondi, (1964) *J. Phys. Chem.* 68:441-451), and using a procedure in the MVP program to close all openings and channels connecting the pocket with the exterior of the protein. Ligand volumes were also calculated with the program GRASP, using the same grid spacing and atomic radius values. The specific radius values are as follows: hydrogen, 1.20 angstroms (Å); carbon, 1.70 Å; oxygen, 1.52 Å; nitrogen, 1.55 Å; sulfur, 1.80 Å; fluorine, 1.47 Å; chlorine, 1.75 Å; bromine, 1.85 Å; iodine, 1.98 Å. Hydrogen atoms are modeled onto the protein and the ligand using standard bond lengths and angles, and are represented explicitly in the volume calculations. The MVP program closes openings and channels by covering the entire protein with several layers of closely spaced spheres of radius 1.4 angstroms, and then classifying the spheres as either "inside" or "outside" the protein, based on the degree to which the protein buries the spheres. For the pocket volume calculations, the spheres classified as "outside" are loaded into GRASP together with the protein atoms. This procedure effectively closes all the openings and channels that connect the pocket to the outside of the protein, and allows GRASP to calculate a meaningful cavity volume for the pocket. In the following Table of Pocket Volume Data, all volumes are given in cubic angstroms.

| Table of Pocket Volume Data | | | | | |
|---|---|---|---|---|---|
| | | subunit-A | | subunit-B | |
| protein | ligand | pocket | ligand | pocket | ligand |
| GR | fluticasone proionate | 658 | 476 | 716 | 477 |
| GR | dexamethasone | 600 | 390 | 578 | 389 |
| PR | progesterone | 557 | 349 | 570 | 351 |
| AR | dihydrotestosterone | 422 | 319 | no B subunit | |

The term "expanded binding pocket," then, can refer to an NR ligand binding pocket in which the pocket volume is increased by about 50 cubic angstoms over that of a ligand binding pocket in a different structure of the same NR. By way of example, a GR LBD of the present invention comprising an expanded binding pocket (e.g. as characterized by the atomic structural coordinates of Table 2) can exhibit an increase in pocket volume of between about 50 and about 150 cubic angstroms over a GR structure lacking an expanded binding pocket, (e.g. as characterized by the atomic coordinates of Table 3). In other examples, an AR LBD comprising an expanded binding pocket (e.g. as characterized by the atomic structural coordinates of Table 4) can exhibit an increase in pocket volume of between about 50 and about 150 cubic angstroms over an AR structure lacking an expanded binding pocket (e.g. as characterized by the atomic structural coordinates of Tables 8 and 9). A MR LBD comprising an expanded binding pocket (e.g. as characterized by the atomic structural coordinates of Table 11) can exhibit an increase in pocket volume of between about 50 and about 150 cubic angstroms over a MR structure lacking an expanded binding pocket. A PR LBD comprising an expanded binding pocket (e.g. as characterized by the atomic structural coordinates of Table 5) can exhibit an increase in pocket volume of between about 50 and about 150 cubic angstroms over a PR structure lacking an expanded binding pocket (e.g. as characterized by the atomic structural coordinates of Table 10).

In a preferred embodiment, a GR structure with an expanded binding pocket can comprise a crystalline GR polypeptide, with or without ligand, and with or without coactivator peptide, and atomic coordinates thereof, where the AF2 helix is located in the active position, and where atoms in the residues Met560, Met639, Gln642, Cys643, Met646, and Tyr735 have shifted from their positions in a GR/Dex structure, e.g. as characterized by the atomic structural coordinates of Table 3, by a heavy-atom RMS deviation of at least about 0.50 angstroms, or by a backbone heavy-atom RMS deviation of at least about 0.35 angstroms.

In another preferred embodiment, a GR structure with an expanded binding pocket can comprise a crystalline GR polypeptide, with or without ligand, and with or without coactivator peptide, and atomic coordinates thereof, where the AF2 helix is located in the active position, and where atoms in the residues Met560, Met639, Gln642, Cys643, Met646, and Tyr735 have shifted from their positions in a GR/Dex structure, e.g. as characterized by the atomic structural coordinates of Table 3, so as to increase the volume of a binding pocket by at least about 5% compared with a GR/Dex structure, e.g. as characterized by the atomic structural coordiates of Table 3.

In yet another preferred embodiment, a GR structure with an expanded binding pocket can comrprise a crystalline GR polypeptide, with or without ligand, and with or without coactivator peptide, and atomic coordinates thereof, where the AF2 helix is located in the active position, and where atoms in and around the ligand binding site have shifted from their positions in the GR/Dex structure so as to accomodate without atomic overlap steroidal ligands with C17-$\alpha$ substituents comprising 2-20 heavy atoms.

In a further preferred embodiment, a GR structure with an expanded binding pocket can comprise a crystalline GR polypeptide, with or without ligand, and with or without coactivator peptide, and atomic coordinates thereof, where

the AF2 helix is located in the active position, and where atoms in and around the ligand binding site have shifted from their positions in the GR/Dex structure so as to accomodate without atomic overlap non-steroidal ligands such as benzoxazin-1-one and A-222977.

In an additional preferred embodiment, a GR structure with an expanded binding pocket can comprise a crystalline GR polypeptide, with or without ligand, and with or without coactivator peptide, and atomic coordinates thereof, where the AF2 helix is located in the active position, and where atoms in and around the ligand binding site have shifted from their positions in the GR/Dex structure so that fluticasone propionate can be docked into the binding site with a favorable binding energy, as computed with molecular modeling software such as MVP, Discover, AMBER or CHARMM, using common force fields such as CFF91 or MMFF94, and where all atoms in the protein are held fixed.

In another preferred embodiment, a GR structure with an expanded binding pocket can comprise a crystalline GR polypeptide, with or without ligand, and with or without coactivator peptide, and atomic coordinates thereof, where the AF2 helix is located in the active position, and where atoms in and around the ligand binding site have shifted from their positions in the GR/Dex structure so that non-steroidal GR ligands, such as benzoxazin-1-one and A-222977, can be docked into the binding site with a favorable binding energy, as computed with molecular modeling software such as MVP, Discover, AMBER or CHARMM, using common force fields such as CFF91 or MMFF94, and where all atoms in the protein are held fixed.

As used herein, the term "expression" generally refers to the cellular processes by which a biologically active polypeptide is produced.

As used herein, the term "gene" is used for simplicity to refer to a functional protein, polypeptide or peptide encoding unit. As will be understood by those in the art, this functional term includes both genomic sequences and cDNA sequences. Preferred embodiments of genomic and cDNA sequences are disclosed herein.

As used herein, the term "glucocorticoid" means a steroid hormone glucocorticoid. "Glucocorticoids" are agonists for the glucocorticoid receptor. Compounds which mimic glucocorticoids can also be defined as glucocorticoid receptor agonists. A preferred glucocorticoid receptor agonist is fluticasone propionate. Other common glucocorticoid receptor agonists include cortisol, cortisone, prednisolone, prednisone, methylprednisolone, trimcinolone, hydrocortisone, and corticosterone. As used herein, glucocorticoid is intended to include, for example, the following generic and brand name corticosteroids: cortisone (CORTONE ACETATE, ADRESON, ALTESONA, CORTELAN, CORTISTAB, CORTISYL, CORTOGEN, CORTONE, SCHEROSON); dexamethasone--oral (DECADRON-ORAL, DEXAMETH, DEXONE, HEXADROL-ORAL, DEXAMETHASONE INTENSOL, DEXONE 0.5, DEXONE 0.75, DEXONE 1.5, DEXONE 4); hydrocortisone--oral (CORTEF, HYDROCORTONE); hydrocortisone cypionate (CORTEF ORAL SUSPENSION); methylprednisolone--oral (MEDROL-ORAL); prednisolone--oral (PRELONE, DELTA-CORTEF, PEDIAPRED, ADNISOLONE, CORTALONE, DELTACORTRIL, DELTASOLONE, DELTASTAB, DI-ADRESON F, ENCORTOLONE, HYDROCORTANCYL, MEDISOLONE, METICORTELONE, OPREDSONE, PANAAFCORTELONE, PRECORTISYL, PRENISOLONA, SCHERISOLONA, SCHERISOLONE); prednisone (DELTASONE, LIQUID PRED, METICORTEN, ORASONE 1, ORASONE 5, ORASONE 10, ORASONE 20, ORASONE 50, PREDNICEN-M, PREDNISONE INTENSOL, STERAPRED, STERAPRED DS, ADASONE, CARTANCYL, COLISONE, CORDROL, CORTAN, DACORTIN, DECORTIN, DECORTISYL, DELCORTIN, DELLACORT, DELTA-DOME, DELTACORTENE, DELTISONA, DIADRESON, ECONOSONE, ENCORTON, FERNISONE, NISONA, NOVOPREDNISONE, PANAFCORT, PANASOL, PARACORT, PARMENISON, PEHACORT, PREDELTIN, PREDNICORT, PREDNICOT, PREDNIDIB, PREDNIMENT, RECTODELT, ULTRACORTEN, WINPRED); triamcinolone--oral (KENACORT, ARISTOCORT, ATOLONE, SHOLOG A, TRAMACORT-D, TRI-MED, TRIAMCOT, TRISTO-PLEX, TRYLONE D, U-TRI-LONE).

As used herein, the term "glucocorticoid receptor," abbreviated herein as "GR," means the receptor for a steroid hormone glucocorticoid. A glucocorticoid receptor is a steroid receptor and, consequently, a nuclear receptor, since steroid receptors are a subfamily of the superfamily of nuclear receptors. The term "GR" means any polypeptide sequence that can be aligned with human GR such that at least 70%, preferably at least 75%, of the amino acids are identical to the corresponding amino acid in the human GR. The term "GR" also encompasses nucleic acid sequences where the corresponding translated protein sequence can be considered to be a GR. The term "GR" includes invertebrate homologs, whether now known or hereafter identified; preferably, GR nucleic acids and polypeptides are isolated from eukaryotic sources. The term "GR" further includes vertebrate homologs of GR family members, including, but not limited to, mammalian and avian homologs. Representative mammalian homologs of GR family members include, but are not limited to, murine and human homologs. The term "GR" specifically encompasses all GR isoforms, including GR$\alpha$ and GR$\beta$. GR$\beta$ is a splicing variant with 100% identity to GR$\alpha$, except at the C-terminus, where 50 residues in GR$\alpha$ have been replaced with 15 residues in GR$\beta$.

As used herein, the terms "GR gene product", "GR protein", "GR polypeptide", and "GR peptide" are used interchangeably and mean peptides having amino acid sequences which are substantially identical to native amino acid sequences from the organism of interest and which are biologically active in that they comprise all or a part of the amino acid sequence of a GR polypeptide, or cross-react with antibodies raised against a GR polypeptide, or retain all or some of the biological activity (e.g., DNA or ligand binding ability and/or transcriptional regulation) of the native

amino acid sequence or protein. Such biological activity can include immunogenicity. Representative embodiments are set forth in SEQ ID NOs: 2, 4, 6, and 8. The terms "GR gene product", "GR protein", "GR polypeptide", and "GR peptide" also include analogs of a GR polypeptide. By "analog" is intended that a DNA or peptide sequence can contain alterations relative to the sequences disclosed herein, yet retain all or some of the biological activity of those sequences. Analogs can be derived from genomic nucleotide sequences as are disclosed herein or from other organisms, or can be created synthetically. Those skilled in the art will appreciate that other analogs, as yet undisclosed or undiscovered, can be used to design and/or construct GR analogs. There is no need for a "GR gene product", "GR protein", "GR polypeptide", or "GR peptide" to comprise all or substantially all of the amino acid sequence of a GR polypeptide gene product. Shorter or longer sequences are anticipated to be of use in the invention; shorter sequences are herein referred to as "segments". Thus, the terms "GR gene product", "GR protein", "GR polypeptide", and "GR peptide" also include fusion or recombinant GR polypeptides and proteins comprising sequences of the present invention. Methods of preparing such proteins are disclosed herein and are known in the art.

As used herein, the terms "GR gene" and "recombinant GR gene" mean a nucleic acid molecule comprising an open reading frame encoding a GR polypeptide of the present invention, including both exon and (optionally) intron sequences.

As used herein, "hexagonal unit cell" means a unit cell wherein $a = b \neq c$; and $\alpha = \beta = 90$, $\gamma = 120°$. The vectors a, b and c describe the unit cell edges and the angles $\alpha$, $\beta$, and $\gamma$ describe the unit cell angles. In a preferred embodiment of the present invention, the unit cell has lattice constants of a = b =127.656 Å, c = 87.725 Å, $\alpha = 90°$, $\beta = 90°$, $\gamma = 120°$. While preferred lattice constants are provided, a crystalline polypeptide of the present invention also comprises variations from the preferred lattice constants, wherein the varations range from about one to about two percent. Thus, for example, a crystalline polypeptide of the present invention can also comprise lattice constants a and b of about 126 Å or about 128 Å and lattice constant c of about 86 Å or about 88 Å.

As used herein, "homology model" or "homology modeling" means a simulated three-dimensional protein structure resulting from homology modeling, which encompasses the process of creating those simulated protein structures by systematic replacement of differing amino acid residues in a related template protein structure, that can either be a crystal structure or homology model itself, in order to produce a target protein structure.

As used herein, "docking model" means a simulated three-dimensional protein structure resulting from the manual or automated adjustment of the three-dimensional coordinates of a template protein structure, that can either be a crystal structure or homology model, and/or a bound ligand. A docking model differs from a homology model in that, when constructing a docking model, no systematic replacement of differing amino acids residues is required.

As used herein, "model" means either a homology model or a docking model depending on the context.

As used herein, the term "hybridization" means the binding of a probe molecule, e.g. a molecule to which a detectable moiety has been bound, to a target sample.

As used herein, the term "interact" means detectable interactions between molecules, such as can be detected using, for example, a yeast two hybrid assay. The term "interact" is also meant to include "binding" interactions between molecules. Interactions can, for example, be protein-protein or protein-nucleic acid in nature.

As used herein, the term "intron" means a DNA sequence present in a given gene that is not translated into protein.

As used herein, the term "isolated" means oligonucleotides substantially free of other nucleic acids, proteins, lipids, carbohydrates or other materials with which they can be associated, such association being either in cellular material or in a synthesis medium. The term can also be applied to polypeptides, in which case the polypeptide will be substantially free of nucleic acids, carbohydrates, lipids and other undesired polypeptides.

As used herein, the term "labeled" means the attachment of a moiety, capable of detection by spectroscopic, radiologic or other methods, to a probe molecule.

As used herein, the term "modified" means an alteration from an entity's normally occurring state. An entity can be modified by removing discrete chemical units or by adding discrete chemical units. The term "modified" encompasses detectable labels as well as those entities added as aids in purification.

As used herein, the term "modulate" means an increase, decrease, or other alteration of any or all chemical and biological activities or properties of a wild-type or mutant polypeptide, e.g. a wild-type or mutant GR polypeptide. The term "modulation" as used herein refers to both upregulation (i.e., activation or stimulation) and downregulation (i.e. inhibition or suppression) of a response, and includes responses that are upregulated in one cell type or tissue, and down-regulated in another cell type or tissue.

As used herein, the term "molecular replacement" means a method of solving a crystal structure of a chemical compound (e.g. a protein) that involves generating a preliminary model of a crystalline polypeptide whose structure coordinates are unknown (e.g. a wild type or mutant GR polypeptide or fragment or domain thereof), by orienting and positioning a molecule or model whose structure coordinates are known (e.g., a nuclear receptor) within the unit cell of the unknown crystal so as best to account for the observed diffraction pattern of the unknown crystal. Phases can then be calculated from this model and combined with the observed amplitudes to give an approximate Fourier synthesis

of the structure whose coordinates are unknown. This, in turn, can be subject to any of the several forms of refinement to provide a final, accurate structure of the unknown crystal. See, e.g., Lattman, (1985) *Method Enzymol.,* 115: 55-77; Rossmann (ed.), (1972) The Molecular Replacement Method, Gordon & Breach, New York, New York, United States of America. For example, using the structure coordinates of the ligand binding domain of GR provided by this invention, molecular replacement can be used to determine the structure coordinates of a crystalline mutant or homologue of the GR ligand binding domain, or of a different crystal form of the GR ligand binding domain.

As used herein, the term "mutation" carries its traditional connotation and means a change, inherited, naturally occurring or introduced, in a nucleic acid or polypeptide sequence, and is used in its sense as generally known to those of skill in the art.

As used herein, the terms "non-steroid" and "non-steroid compound" are used interchangeably and mean a compound that lacks the ring structure that defines steroid compounds, namely the structure:

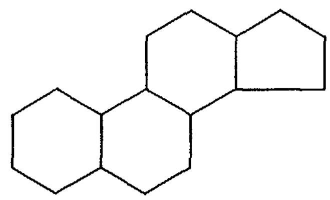

but retains the binding and functional activity of a steroid compound for an NR such as GR.

As used herein, the term "nuclear receptor", occasionally abbreviated herein as "NR", means a member of the superfamily of receptors that comprises at least the subfamilies of steroid receptors, thryroid hormone receptors, retinoic acid receptors and vitamin D receptors, and specifically encompasses GR. Thus, a given nuclear receptor can be further classified as a member of a subfamily while retaining its status as a nuclear receptor. The term "nuclear receptor" also encompasses fragments of a nuclear receptor.

As used herein, the phrase "operatively linked" means that an enhancer-promoter is connected to a coding sequence in such a way that the transcription of that coding sequence is controlled and regulated by that enhancer-promoter. Techniques for operatively linking an enhancer-promoter to a coding sequence are well known in the art; the precise orientation and location relative to a coding sequence of interest is dependent, *inter alia,* upon the specific nature of the enhancer-promoter.

As used herein, the term "partial agonist" means an entity that can bind to a receptor or other target and induce only part of the changes in the receptor or other target that are induced by agonists. The differences can be qualitative or quantitative. Thus, a partial agonist can induce some of the conformation changes induced by agonists, but not others, or it can only induce certain changes to a limited extent.

As used herein, the term "partial antagonist" means an entity that can bind to a receptor or other target and inhibit only part of the changes in the receptor or other target that are induced by antagonists. The differences can be qualitative or quantitative. Thus, a partial antagonist can inhibit some of the conformation changes induced by an antagonist, but not others, or it can inhibit certain changes to a limited extent.

As used herein, the term "pocket volume" means the volume of space within the protein that is available for occupation by a ligand. Any desired algorithm can be employed when calculating a pocket volume, although some algorithms are more accurate than others. In one approach, a pocket volume can be approximated by an ellipsoid with principle axes of length 2a, 2b and 2c, and its volume can be calculated as

$$V = (4/3) \times pi \times (a) \times (b) \times (c)$$

where pi=3.14159.

The walls of the pocket are formed from atoms comprising the nuclear receptor protein. In another approach, these atoms, and the atoms in the ligand, can be approximated as spheres with specified atomic radius values. With this representation, the walls of the pocket comprise numerous spheres. If two atoms are directly bonded together, then their spheres will overlap. The spheres can also overlap when atoms are connected together by bonds with one or two intervening atoms, but do not normally overlap significantly when atoms are more distantly connected, or when the atoms are not covalently connected. Consequently, in this representation, the walls of the pocket have numerous gaps, channels and spaces between the spheres. Ligand atoms may fit into some of the larger gaps, channels and spaces, but generally cannot fit into the smaller gaps, channels and spaces. This complication of the spherical atom representation led to the definition of a "molecular surface" where gaps and spaces too small to accommodate a water

molecule, or "probe," were effectively smoothed over. Some of the fundamental issues involved in the definition of a molecular surface and the calculation of molecular volumes are discussed in Richards, (1977) *Ann. Rev. Biophys. Bioeng.* 6:151-176. For a further discussion of the molecular surface and algorithms for its calculation, see Connolly, (1983) *Science* 221:709-713. Because of Connolly's contributions, the molecular surface is sometimes referred to as a "Connolly surface."

A pocket is generally defined as the region enclosed by the molecular surface, where the molecular surface is calculated using a probe radius of 1.4 angstroms. With nuclear receptors, there can often be channels connecting the pocket with the exterior of the protein. In this case, it is presumed that the channels are occluded in some manner so that a fully enclosed pocket can be defined. For example, a channel can be occluded by placing a water molecule at the narrowest point along the channel. The program MVP has an systematic algorithm for closing channels: the entire protein is first covered by several layers of closely-spaced water-sized spheres. The spheres are generated by placing the protein in a grid, and identifying grid points where a sphere of radius 1.4 angstroms can be accommodated without overlapping the sphere corresponding to any atom of the protein. In calculations reported herein, the grid spacing was taken as 0.3-0.8 angstroms. These spheres on the grid are then identified as either internal to the protein or external to the protein, based on the degree to which they are buried within the protein. The degree of burial is quantified by measuring the solid angle occluded by the protein at the grid point in question. In calculations reported herein, the sphere is considered to be buried if 90% or more of the solid angle is occluded by the protein.

A fully closed molecular surface can be generated for the ligand binding pocket with programs such as GRASP (Columbia University, New York, New York, United States of America) or Connolly's MS program by loading the protein together with the external water-sized spheres generated by MVP. The program GRASP can further be used to calculate the cavity volume. It is noted that the calculated cavity volume is sensitive to the grid spacing used in generating the molecular surface. The GRASP calculations reported herein used a grid spacing of 0.2 angstroms. Coarser spacings can lead to substantially inaccurate volumes. The internal grid spheres generated by MVP can also be used to estimate the volume of the pocket. In this case, MVP carries out a cluster analysis to group the internal spheres into clusters corresponding to different pockets and cavities within the protein. With nuclear receptors, the ligand binding pocket generally corresponds to the largest such cluster. The volume of the cluster can be calculated directly with the GRASP program. This approach tends to underestimate the volume of the pocket, since the internal grid spheres can never fill the pocket entirely. The spheres can fill the pocket more fully as the grid spacing is reduced. A grid spacing of 0.3 angstroms gives volumes in relatively good agreement with the alternative GRASP method described above. Other methods of calculating pocket volumes have been described in the literature. See, e.g., Kleywegt & Jones, (1994) *Acta Crystallogr. Section D* D50:178-185.

Aside from the algorithm used, the atomic radius values can also be considered. Generally, atomic volumes depend on the radius raised to the third power, so it is clear that calculated molecular volumes are sensitive to atomic radius values. Cavity volumes tend to decrease as radius values increase, and if the atomic radius values are too large, the calculated cavity volume will be too small. In the present invention, the following atomic radius values were employed: hydrogen, 1.20Å; carbon, 1.70Å; nitrogen, 1.55Å; oxygen, 1.52Å; sulfur, 1.80Å; fluorine, 1.47Å; chlorine, 1.75Å; bromine, 1.85Å; iodine, 1.98Å. See Bondi, (1964) *J. Phys. Chem.* 68:441-451. For all volume calculations reported herein, the hydrogens were represented explicitly. These hydrogen atoms are added to the protein with MVP using standard bond lengths and angles, followed by energy minimization with the CFF91 force field within MVP. Some other workers in the protein structure field often omit the hydrogens in surface and volume calculations, using an increased carbon radius to compensate. This "united atom" approximation can reduce the accuracy of a pocket volume calculation.

When comparing the volumes of two different proteins, or two different conformations of the same protein, it is preferable to use the same algorithm, parameters and atomic radius values.

As used herein, the term "polypeptide" means any polymer comprising any of the 20 protein amino acids, regardless of its size. Although "protein" is often used in reference to relatively large polypeptides, and "peptide" is often used in reference to small polypeptides, usage of these terms in the art overlaps and varies. The term "polypeptide" as used herein refers to peptides, polypeptides and proteins, unless otherwise noted. As used herein, the terms "protein", "polypeptide" and "peptide" are used interchangeably herein when referring to a gene product.

As used herein, the term "primer" means a sequence comprising two or more deoxyribonucleotides or ribonucleotides, preferably more than three, and more preferably more than eight and most preferably at least about 20 nucleotides of an exonic or intronic region. Such oligonucleotides are preferably between ten and thirty bases in length.

As used herein, the term "root mean squared (RMS) deviation" of a collection of atoms in one protein structure relative to the corresponding atoms in another protein structure refers to the average displacement of those atoms, after superimposition of the proteins, as computed according to the formula

$$\text{RMSDeviation} = \sqrt{\frac{1}{N}\sum_{i=1}^{N}\left[\left(x_i^1 - x_i^2\right)^2 + \left(y_i^1 - y_i^2\right)^2 + \left(z_i^1 - z_i^2\right)^2\right]}$$

where $xi^1$, $yi^1$, $zi^1$ are the coordinates of atom i in structure 1, and $xi^2$, $yi^2$, $zi^2$ are the coordinates of atom i in structure 2 (after superimposition of the two proteins), N is the number of atoms in the collection, and where the index i runs iteratively through the collection of N atoms for which the RMS deviation is to be calculated. The superimposition is a rotation and translation of the coordinates carried out using the backbone atoms in the core of the protein, and carried out so as to minimize the RMS deviation of these core backbone atoms. This can optionally include some or all the atoms in the collection for which the RMS deviation is calculated. For GR, the superimposition might be carried out using backbone atoms in helices 1-10, but would normally not include the AF2 helix or the loops connecting the helices. Various algorithms are available for generating the rotation matrix and translation vectors that superimpose two sets of protein backbone atoms. See, for example, Kabsch, (1978) *Acta Cryst.* A34, 827-828. These algorithms can be used together with sequence alignment algorithms to identify corresponding backbone atoms in two different protein structures. See, for example, Blundell et al., (1987) *Nature* 326:347-352. Hydrogen atoms are generally not clearly visible in the electron density, and there may be uncertainties in their placement using molecular modeling software. Consequently, hydrogen atoms are usually not included in the collections of atoms used in calculating RMS deviations. As used herein, the term heavy atom RMS deviation refers to an RMS deviation calculated by excluding the hydrogen atoms from the specified collection. In the analysis of protein structures, the side-chain atoms often shift more than the backbone atoms, and it may be useful to calculate RMS deviations using only the backbone heavy atoms. As used herein, the term backbone heavy-atom RMS deviation refers to an RMS deviation calculated using the backbone heavy atoms, commonly designated as N, Cα, C and O, but not including any of the side-chain atoms.

As used herein, the term "sequencing" means the determining the ordered linear sequence of nucleic acids or amino acids of a DNA or protein target sample, using conventional manual or automated laboratory techniques.

As used herein, the term "space group" means the arrangement of symmetry elements of a crystal.

As used herein, the term "steroid receptor" means a nuclear receptor that can bind or associate with a naturally occurring steroid compound. Steroid receptors are a subfamily of the superfamily of nuclear receptors. The subfamily of steroid receptors comprises glucocorticoid receptors and, therefore, a glucocorticoid receptor is a member of the subfamily of steroid receptors and the superfamily of nuclear receptors.

As used herein, the terms "structure coordinates," "structural coordinates," "spatial coordinates," "atomic structure coordinates," "three-dimensional coordinates" and "atomic coordinates" are used interchangeably and mean mathematical coordinates derived from mathematical equations related to the patterns obtained on diffraction of a monochromatic beam of X-rays by the atoms (scattering centers) of a molecule in crystal form. The diffraction data are used to calculate an electron density map of the repeating unit of the crystal. The electron density maps are used to establish the positions of the individual atoms within the unit cell of the crystal.

Those of skill in the art understand that a set of coordinates determined by X-ray crystallography is not without standard error. In general, the error in the coordinates tends to be reduced as the resolution is increased, since more experimental diffraction data is available for the model fitting and refinement. Thus, for example, more diffraction data can be collected from a crystal that diffracts to a resolution of 3.0 angstroms than from a crystal that diffracts to a lower resolution, such as 3.5 angstroms. Consequently, the refined structural coordinates will usually be more accurate when fitted and refined using data from a crystal that diffracts to higher resolution. The design of ligands and modulators for GR or any other NR depends on the accuracy of the structural coordinates. If the coordinates are not sufficiently accurate, then the design process will be ineffective. In most cases, it is very difficult or impossible to collect sufficient diffraction data to define atomic coordinates precisely when the crystals diffract to a resolution of only 3.5 angstroms or poorer. Thus, in most cases, it is difficult to use X-ray structures in structure-based ligand design when the X-ray structures are based on crystals that diffract to a resolution of only 3.5 angstroms or poorer. However, common experience has shown that crystals diffracting to 3.0 angstroms or better can yield X-ray structures with sufficient accuracy to greatly facilitate structure-based drug design. Further improvement in the resolution can further facilitate structure-based design, but the coordinates obtained at 3.0 angstroms resolution are generally adequate for most purposes.

Also, those of skill in the art will understand that NR proteins can adopt different conformations when different ligands are bound. In particular, NR proteins will adopt substantially different conformations when agonists and antagonists are bound. Subtle variations in the conformation can also occur when different agonists are bound, and when different antagonists are bound. These variations can be difficult or impossible to predict from a single X-ray structure. Generally, structure-based design of GR modulators depends to some degree on an understanding of the differences in conformation that occur when agonists and antagonists are bound. Thus, structure-based modulator design is most facilitated by the availability of X-ray structures of complexes with potent agonists as well as potent antagonists.

As used herein, the term "substantially pure" means that the polynucleotide or polypeptide is substantially free of the sequences and molecules with which it is associated in its natural state, and those molecules used in the isolation procedure. The term "substantially free" means that the sample is at least 50%, preferably at least 70%, more preferably 80% and most preferably 90% free of the materials and compounds with which is it associated in nature.

As used herein, the term "target cell" refers to a cell, into which it is desired to insert a nucleic acid sequence or polypeptide, or to otherwise effect a modification from conditions known to be standard in the unmodified cell. A nucleic acid sequence introduced into a target cell can be of variable length. Additionally, a nucleic acid sequence can enter a target cell as a component of a plasmid or other vector or as a naked sequence.

As used herein, the term "transcription" means a cellular process involving the interaction of an RNA polymerase with a gene that directs the expression as RNA of the structural information present in the coding sequences of the gene. The process includes, but is not limited to the following steps: (a) the transcription initiation, (b) transcript elongation, (c) transcript splicing, (d) transcript capping, (e) transcript termination, (f) transcript polyadenylation, (g) nuclear export of the transcript, (h) transcript editing, and (i) stabilizing the transcript.

As used herein, the term "transcription factor" means a cytoplasmic or nuclear protein which binds to such gene, or binds to an RNA transcript of such gene, or binds to another protein which binds to such gene or such RNA transcript or another protein which in turn binds to such gene or such RNA transcript, so as to thereby modulate expression of the gene. Such modulation can additionally be achieved by other mechanisms; the essence of "transcription factor for a gene" is that the level of transcription of the gene is altered in some way.

As used herein, the term "unit cell" means a basic parallelipiped shaped block. The entire volume of a crystal can be constructed by regular assembly of such blocks. Each unit cell comprises a complete representation of the unit of pattern, the repetition of which builds up the crystal. Thus, the term "unit cell" means the fundamental portion of a crystal structure that is repeated infinitely by translation in three dimensions. A unit cell is characterized by three vectors a, b, and c, not located in one plane, which form the edges of a parallelepiped. Angles $\alpha$, $\beta$ and $\gamma$ define the angles between the vectors: angle $\alpha$ is the angle between vectors b and c; angle $\beta$ is the angle between vectors a and c; and angle $\gamma$ is the angle between vectors a and b. The entire volume of a crystal can be constructed by regular assembly of unit cells; each unit cell comprises a complete representation of the unit of pattern, the repetition of which builds up the crystal.

II. Description of Tables

Table 1 is a table summarizing the crystal and data statistics obtained from the crystallized ligand binding domain of human GR in complex with the ligand fluticasone propionate and a coactivator peptide derived from TIF2. Data on the unit cell are presented, including data on the crystal space group, unit cell dimensions, molecules per asymmetric cell and crystal resolution.

Table 2 is a table presenting the atomic coordinate data for crystallized GR LBD in complex with fluticasone propionate and a TIF2 peptide.

Table 3 is a table presenting the atomic coordinate data for human GR in complex with dexamethasone and a TIF2 peptide employed in the molecular replacement solution of human GR ligand binding domain in complex with fluticasone propionate and a TIF2 peptide.

Table 4 is a table presenting the three-dimensional coordinates of AR in complex with bicalutamide obtained from homology modeling of the crystal structure coordinates of GR$\alpha$ in complex with FP.

Table 5 is a table presenting the three-dimensional coordinates of PR in complex with RWJ-60130 obtained from homology modeling of the crystal structure coordinates of GR$\alpha$ in complex with FP.

Table 6 is a table presenting a subset of three-dimensional coordinates of GR$\alpha$ in complex with the benzoxazin-1-one obtained from modeling of the crystal structure of GR$\alpha$ in complex with FP.

Table 7 is a table presenting a subset of three-dimensional coordinates of GR$\alpha$ in complex with A-222977 obtained from modeling of the crystal structure of GR$\alpha$ in complex with FP.

Table 8 is a table presenting three-dimensional coordinates of AR in complex with DHT (Sack et al., (2001) *Proc. Natl. Acad. Sci. U.S.A.* 98(9): 4904-4909; PDB ID No. 1137).

Table 9 is a table presenting three-dimensional coordinates of AR in complex with the ligand R1881 (Matias et al., (2000) *J. Biol. Chem.* 275(34): 26164-171; PDB ID No. 1E3G).

Table 10 is a table presenting three-dimensional coordinates of PR in complex with PG (Williams & Sigler, (1998) *Nature* 393:392-396; PDB ID No. 1A28).

Table 11 is a table presenting three-dimensional coordinates of MR obtained from homology modeling of the crystal structure coordinates of GR$\alpha$ in complex with FP.

III. General Considerations

The present invention will usually be applicable *mutatis mutandis* to nuclear receptors in general, more particularly to steroid receptors including MR, AR, PR, GR and isoforms thereof, and even more particularly to glucocorticoid receptors, as discussed herein, based, in part, on the patterns of nuclear receptor and steroid receptor structure and modulation. Some of these patterns have emerged as a consequence of the present disclosure, which in part discloses determining the three dimensional structure of the ligand binding domain of GRα having an expanded binding pocket in complex with fluticasone propionate and a fragment of the co-activator TIF2.

The nuclear receptor superfamily can be subdivided into two subfamilies: the GR subfamily (also referred to as the steroid receptors and denoted SRs), comprising GR, AR (androgen receptor), MR (mineralcorticoid receptor) and PR (progesterone receptor) and the thyroid hormone receptor (TR) subfamily, comprising TR, vitamin D receptor (VDR), retinoic acid receptor (RAR), retinoid X receptor (RXR), and most orphan receptors. This division has been made on the basis of DNA binding domain structures, interactions with heat shock proteins (HSP), and ability to form dimers.

Steroid receptors (SRs) form a subset of the superfamily of nuclear receptors. The glucocorticoid receptor is a steroid receptor and thus a member of the superfamily of nuclear receptors and the subset of steroid receptors. The human glucocorticoid receptor exists in two isoforms: GRα, which comprises 777 amino acids and GRβ, which comprises 742 amino acids. As noted, the alpha isoform of human glucocorticoid receptor comprises 777 amino acids and is predominantly cytoplasmic in its unactivated, non-DNA binding form. When activated, it translocates to the nucleus. In order to understand the role played by the glucocorticoid receptor in the different cell processes, the receptor was mapped by transfecting receptor-negative and glucocorticoid-resistant cells with different steroid receptor constructs and reporter genes like chloramphenicol acyltransferase (CAT) or luciferase which had been covalently linked to a glucocorticoid responsive element (GRE). From these and other studies, four major functional domains have become evident.

From the amino terminal end to the carboxyl terminal end, these functional domains include the tau 1, DNA binding, and ligand binding domains in succession. The tau 1 domain spans amino acid positions 77-262 and regulates gene activation. The DNA binding domain is from amino acid positions 421-486 and has nine cysteine residues, eight of which are organized in the form of two zinc fingers analogous to *Xenopus* transcription factor IIIA. The DNA binding domain binds to the regulatory sequences of certain genes that are induced or deinduced by glucocorticoids. Amino acids 521 to 777 form the ligand binding domain, which binds glucocorticoid to activate the receptor. This region of the receptor also comprises a nuclear localization signal. Deletion of this carboxyl terminal end results in a receptor that is constitutively active for gene induction (up to 30% of wild type activity) and even more active for cell kill (up to 150% of wild type activity) (Giguere et al., (1986) *Cell* 46: 645-652; Hollenberg et al., (1987) *Cell* 49: 39-46; Hollenberg & Evans, (1988) *Cell* 55: 899-906; Hollenberg et al., (1989) *Cancer Res.* 49: 2292s-2294s; Oro et al., (1988) *Cell* 55: 1109-1114; Evans, (1989) in Recent Progress in Hormone Research (Clark, ed.) Vol. 45, pp. 1-27, Academic Press, San Diego, California, United States of America; Green & Chambon, (1987) *Nature* 325: 75-78; Picard & Yamamoto, (1987) *EMBO J.* 6: 3333-3340; Picard et al., (1990) *Cell Regul.* 1: 291-299; Godowski et al., (1987) *Nature* 325: 365-368; Miesfeld et al., (1987) *Science* 236:423-427; Danielsen et al., (1989) *Cancer Res.* 49: 2286s-2291s; Danielsen et al., (1987) *Molec. Endocrinol.* 1: 816-822; Umesono & Evans, (1989) *Cell* 57: 1139-1146.). Despite the aforementioned indirect characterization of the structure of GRα, until the present disclosure, a detailed three-dimensional model of the ligand binding domain of GRα in complex with fluticasone propionate has not been achieved.

GR subgroup members are tightly bound by heat shock protein(s) (HSP) in the absence of ligand, dimerize following ligand binding and dissociation of HSP, and show homology in the DNA half sites to which they bind. These half sites also tend to be arranged as palindromes. TR subgroup members tend to be bound to DNA or other chromatin molecules when unliganded, can bind to DNA as monomers and dimers, but tend to form heterodimers, and bind DNA elements with a variety of orientations and spacings of the half sites, and also show homology with respect to the nucleotide sequences of the half sites. ER does not belong to either subfamily, since it resembles the GR subfamily in hsp interactions, and the TR subfamily in nuclear localization and DNA-binding properties.

Most members of the superfamily, including orphan receptors, possess at least two transcription activation subdomains, one of which is constitutive and resides in the amino terminal domain (AF-1), and the other of which (AF-2) resides in the ligand binding domain, whose activity is regulated by binding of an agonist ligand. The function of AF-2 requires an activation domain (also called transactivation domain) that is highly conserved among the receptor superfamily. Most LBDs contain an activation domain. Some mutations in this domain abolish AF-2 function, but leave ligand binding and other functions unaffected. Ligand binding allows the activation domain to serve as an interaction site for essential co-activator proteins that function to stimulate (or in some cases, inhibit) transcription.

Analysis and alignment of amino acid sequences, and X-ray and NMR structure determinations, have shown that nuclear receptors have a modular architecture with three main domains:

1) a variable amino-terminal domain;
2) a highly conserved DNA-binding domain (DBD); and
3) a less conserved carboxy-terminal ligand binding domain (LBD).

In addition, nuclear receptors can have linker segments of variable length between these major domains.

Sequence analysis and X-ray crystallography, including the disclosure of the present invention have confirmed that GR also has the same general modular architecture, with the same three domains. The function of GR in human cells presumably requires all three domains in a single amino acid sequence. However, the modularity of GR permits different domains of each protein to separately accomplish certain functions. Some of the functions of a domain within the full-length receptor are preserved when that particular domain is isolated from the remainder of the protein. Using conventional protein chemistry techniques, a modular domain can sometimes be separated from the parent protein. Using conventional molecular biology techniques, each domain can usually be separately expressed with its original function intact or, as discussed herein below, chimeras comprising two different proteins can be constructed, wherein the chimeras retain the properties of the individual functional domains of the respective nuclear receptors from which the chimeras were generated.

The carboxy-terminal activation subdomain is in close three-dimensional proximity in the LBD to the ligand, so as to allow for ligands bound to the LBD to coordinate (or interact) with amino acid(s) in the activation subdomain. As described herein, the LBD of a nuclear receptor can be expressed, crystallized, its three dimensional structure determined with a ligand bound (either using crystal data from the same receptor or a different receptor or a combination thereof), and computational methods used to design ligands to its LBD, particularly ligands that contain an extension moiety that coordinates the activation domain of the nuclear receptor.

The LBD is the second most highly conserved domain in these receptors. As its name suggests, the LBD binds ligands. With many nuclear receptors, including GR, binding of the ligand can induce a conformational change in the LBD that can, in turn, activate transcription of certain target genes. Whereas integrity of several different LBD sub-domains is important for ligand binding, truncated molecules containing only the LBD retain normal ligand-binding activity. This domain also participates in other functions, including dimerization, nuclear translocation and transcriptional activation, as described herein.

Nuclear receptors usually have HSP binding domains that present a region for binding to the LBD and can be modulated by the binding of a ligand to the LBD. For many of the nuclear receptors ligand binding induces a dissociation of heat shock proteins such that the receptors can form dimers in most cases, after which the receptors bind to DNA and regulate transcription. Consequently, a ligand that stabilizes the binding or contact of the heat shock protein binding domain with the LBD can be designed using the computational methods described herein.

With the receptors that are associated with the HSP in the absence of the ligand, dissociation of the HSP results in dimerization of the receptors. Dimerization is due to receptor domains in both the DBD and the LBD. Although the main stimulus for dimerization is dissociation of the HSP, the ligand-induced conformational changes in the receptors can have an additional facilitative influence. With the receptors that are not associated with HSP in the absence of the ligand, particularly with the TR, ligand binding can affect the pattern of dimerization. The influence depends on the DNA binding site context, and can also depend on the promoter context with respect to other proteins that can interact with the receptors. A common pattern is to discourage monomer formation, with a resulting preference for heterodimer formation over dimer formation on DNA.

Nuclear receptor LBDs usually have dimerization domains that present a region for binding to another nuclear receptor and can be modulated by the binding of a ligand to the LBD. Consequently, a ligand that disrupts the binding or contact of the dimerization domain can be designed using the computational methods described herein to produce a partial agonist or antagonist.

The amino terminal domain of GR is the least conserved of the three domains. This domain is involved in transcriptional activation and, its uniqueness might dictate selective receptor-DNA binding and activation of target genes by GR subtypes. This domain can display synergistic and antagonistic interactions with the domains of the LBD.

The DNA binding domain has the most highly conserved amino acid sequence among the GR domains. It typically comprises about 70 amino acids that fold into two zinc finger motifs, wherein a zinc atom coordinates four cysteines. The DBD comprises two perpendicularly oriented $\alpha$-helixes that extend from the base of the first and second zinc fingers. The two zinc fingers function in concert along with non-zinc finger residues to direct the GR to specific target sites on DNA and to align receptor dimer interfaces. Various amino acids in the DBD influence spacing between two half-sites (which usually comprises six nucleotides) for receptor dimerization. The optimal spacings facilitate cooperative interactions between DBDs, and D box residues are part of the dimerization interface. Other regions of the DBD facilitate DNA-protein and protein-protein interactions are involved in dimerization.

In nuclear receptors that bind to a HSP, the ligand-induced dissociation of HSP with consequent dimer formation allows, and therefore, promotes DNA binding. With receptors that are not associated (as in the absence of ligand), ligand binding tends to stimulate DNA binding of heterodimers and dimers, and to discourage monomer binding

to DNA. However, with DNA containing only a single half site, the ligand tends to stimulate the receptor's binding to DNA. The effects are modest and depend on the nature of the DNA site and probably on the presence of other proteins that can interact with the receptors. Nuclear receptors usually have DBD (DNA binding domains) that present a region for binding to DNA and this binding can be modulated by the binding of a ligand to the LBD.

The modularity of the members of the nuclear receptor superfamily permits different domains of each protein to separately accomplish different functions, although the domains can influence each other. The separate function of a domain is usually preserved when a particular domain is isolated from the remainder of the protein. Using conventional protein chemistry techniques a modular domain can sometimes be separated from the parent protein. By employing conventional molecular biology techniques each domain can usually be separately expressed with its original function intact or chimerics of two different nuclear receptors can be constructed, wherein the chimerics retain the properties of the individual functional domains of the respective nuclear receptors from which the chimerics were generated.

Various structures have indicated that most nuclear receptor LBDs adopt the same general folding pattern. This fold consists of 10-12 alpha helices arranged in a bundle, together with several beta-strands, and linking segments. A preferred GRα LBD structure of the present invention has 10-11 helices, depending on whether helix-3' is counted. Structural studies have shown that most of the alpha-helices and beta-strands have the same general position and orientation in all nuclear receptor structures, whether ligand is bound or not. However, the AF2 helix has been found in different positions and orientations relative to the main bundle, depending on the presence or absence of the ligand, and also on the chemical nature of the ligand. These structural studies have suggested that many nuclear receptors share a common mechanism of activation, where binding of activating ligands helps to stabilize the AF2 helix in a position and orientation adjacent to helices-3, -4, and -10, covering an opening to the ligand binding site. This position and orientation of the AF2 helix, which will be called the "active conformation", creates a binding site for co-activators. See, e.g., Nolte et al., (1998) *Nature* 395:137-43; Shiau et al., (1998) *Cell* 95: 927-37. This co-activator binding site has a central lipophilic pocket that can accommodate leucine side-chains from co-activators, as well as a "charge-clamp" structure consisting essentially of a lysine residue from helix-3 and a glutamic acid residue from the AF2 helix.

Structural studies have shown that co-activator peptides containing the sequence LXXLL (SEQ ID NO: 10) (where L is leucine and X can be a different amino acid in different cases) can bind to this co-activator binding site by making interactions with the charge clamp lysine and glutamic acid residues, as well as the central lipophilic region. This co-activator binding site is disrupted when the AF2 helix is shifted into other positions and orientations. In PPARγ, activating ligands such as rosiglitazone (BRL49653) make a hydrogen bonding interaction with tyrosine-473 in the AF2 helix. Nolte et al., (1998) *Nature* 395:137-43; Gampe et al., (2000) *Mol. Cell* 5: 545-55. Similarly, in GR, the dexamethasone ligand makes van der Waals interaction with the side chain of leucine-753 from the AF2 helix. This interaction is believed in part to stabilize the AF2 helix in the active conformation, thereby allowing co-activators to bind and thus activating transcription from target genes.

With certain antagonist ligands, or in the absence of any ligand, the AF2 helix can be held less tightly in the active conformation, or can be free to adopt other conformations. This would either destabilize or disrupt the co-activator binding site, thereby reducing or eliminating co-activator binding and transcription from certain target genes. Some of the functions of the GR protein depend on having the full-length amino acid sequence and certain partner molecules, such as co-activators and DNA. However, other functions, including ligand binding and ligand-dependent conformational changes, can be observed experimentally using isolated domains, chimeras and mutant molecules.

As described herein, the LBD of a GR can be mutated, expressed, crystallized, its three dimensional structure can be determined with a ligand (e.g. fluticasone propionate) bound as disclosed in the present invention. Computational methods can then be employed to design ligands to nuclear receptors, preferably to steroid receptors, and more preferably to glucocorticoid receptors.

## IV. The Fluticasone Ligand

Ligand binding can induce transcriptional activation functions in a variety of ways. One way is through the dissociation of the HSP from receptors. This dissociation, with consequent dimerization of the receptors and their binding to DNA or other proteins in the nuclear chromatin, allows transcriptional regulatory properties of the receptors to be manifest. This can be especially true of such functions on the amino terminus of the receptors.

Another way is by altering the receptor to interact with other proteins involved in transcription. These can be proteins that interact directly or indirectly with elements of the proximal promoter or proteins of the proximal promoter. Alternatively, the interactions can be through other transcription factors that themselves interact directly or indirectly with proteins of the proximal promoter. Several different proteins have been described that bind to the receptors in a ligand-dependent manner. In addition, it is possible that in some cases, the ligand-induced conformational changes do not affect the binding of other proteins to the receptor, but do affect their abilities to regulate transcription.

In one aspect of the present invention, a GR LBD was co-crystallized with a TIF2 peptide and the ligand fluticasone propionate. U.S. Patent No. 4,335,121 to Phillips et al., incorporated herein by reference, teaches an antiinflammatory steroid compound known by the chemical name (6α, 11β, 16α, 17α)-6,9-difluoro-11-hydroxy-16-methyl-3-oxo-17-(1-oxopropoxy)androsta-1,4-diene-17-carbothioic acid S-(fluoromethyl) ester and the generic name "fluticasone propionate." Fluticasone propionate in aerosol form, has been accepted by the medical community as useful in the treatment of asthma (see, e.g.. Nimmagadda et al., (1998) *Ann. Allerg. Asthma Im.* 81:35-40) and is marketed under the trademarks FLOVENT® and FLONASE®. Fluticasone propionate can also be used in the form of a physiologically acceptable solvate.

Fluticasone propionate has the chemical structure:

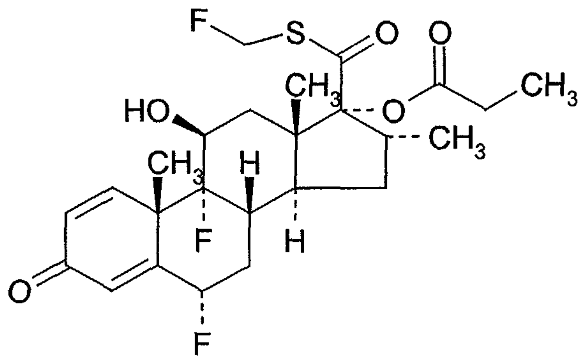

V. The TIF2 Co-activator

A peptide from the nuclear receptor co-activator TIF2 (SEQ ID NO: 9) was co-crystallized in one aspect of the present invention. Structurally, the nuclear receptor coactivator TIF2 comprises one domain that reacts with a nuclear receptor (nuclear receptor interaction domain, abbreviated "NID") and two autonomous activation domains, AD1 and AD2 (Voegel et al., (1998) *EMBO J.* 17: 507-519). The TIF2 NID comprises three NR-interacting modules, with each module comprising the motif, LXXLL (SEQ ID NO: 10) (Voegel et al., (1998) *EMBO J.* 17: 507-519). Mutation of the motif abrogates TIF2's ability to interact with the ligand-induced activation function-2 (AF-2) found in the ligand-binding domains (LBDs) of many NRs. Presently, it is thought that TIF2 AD1 activity is mediated by CREB binding protein (CBP), however, TIF2 AD2 activity does not appear to involve interaction with CBP (Voegel et al., (1998) *EMBO J.* 17: 507-519).

In the present invention, residues 740-753 of the TIF2 protein (SEQ ID NO: 9) were co-crystallized with GR and fluticasone propionate. These residues comprise the LXXLL (SEQ ID NO: 10) of AD-2, the third motif in the linear sequence of TIF2. The TIF2 fragment is 13 residues in length and was synthesized using an automated peptide synthesis apparatus. SEQ ID NO: 9, and other sequences corresponding to TIF2 and other co-activators and co-repressors, can be similarly synthesized using automated apparatuses.

VI. Production of GR and Other NR Polypeptides

In a preferred embodiment, the present invention provides for the first time a GR/TIF2/FP complex. The GR LBD polypeptide of the present invention is expressed as a soluble polypeptide in bacteria, more preferably, in *E. coli.* The GR polypeptides of the present invention, disclosed herein, can thus now provide a variety of host-expression vector systems to express an NR coding sequence. These include but are not limited to microorganisms such as bacteria transformed with recombinant bacteriophage DNA, plasmid DNA or cosmid DNA expression vectors containing an NR coding sequence; yeast transformed with recombinant yeast expression vectors containing an NR coding sequence; insect cell systems infected with recombinant virus expression vectors (e.g., baculovirus) containing an NR coding sequence; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing an NR coding sequence; or animal cell systems. The expression elements of these systems vary in their strength and specificities. Methods for constructing expression vectors that comprise a partial or the entire native or mutated NR and GR polypeptide coding sequence and appropriate transcriptlonal/translational control signals include *in vitro* recombinant DNA techniques, synthetic techniques and *in vivo* recombination/genetic recombination. See, for example, the techniques described throughout Sambrook et al., (1989) Molecular Cloning: A Laboratory Manual, Cold Spring Harbor Laboratory, New York, and Ausubel et al., (1989) Current Protocols in Molecular Biology, Greene Publishing Associates and Wiley Interscience, New York, both incorporated herein in their entirety.

Depending on the host/vector system utilized, any of a number of suitable transcription and translation elements, including constitutive and inducible promoters, can be used in the expression vector. For example, when cloning

in bacterial systems, inducible promoters such as pL of bacteriophage λ, plac, ptrp, ptac (ptrp-lac hybrid promoter) and the like can be used. When cloning in insect cell systems, promoters such as the baculovirus polyhedrin promoter can be used. When cloning in plant cell systems, promoters derived from the genome of plant cells, such as heat shock promoters; the promoter for the small subunit of RUBISCO; the promoter for the chlorophyll a/b binding protein) or from plant viruses (e.g., the 35S RNA promoter of CaMV; the coat protein promoter of TMV) can be used. When cloning in mammalian cell systems, promoters derived from the genome of mammalian cells (e.g., metallothionein promoter) or from mammalian viruses (e.g., the adenovirus late promoter; the vaccinia virus 7.5K promoter) can be used. When generating cell lines that contain multiple copies of the tyrosine kinase domain DNA, SV40-, BPV- and EBV-based vectors can be used with an appropriate selectable marker.

Adequate levels of expression of nuclear receptor LBDs can be obtained by the novel approaches described herein. High level expression in *E. coli* of ligand binding domains of TR and other nuclear receptors, including members of the steroid/thyroid receptor superfamily, such as the estrogen (ER), androgen (AR), mineralocorticoid (MR), progesterone (PR), RAR, RXR and vitamin D (VDR) receptors can also be achieved after review of the expression of a soluble GR polypeptide in bacteria, more preferably, *E. coli* disclosed herein. The GR polypeptides of the present invention, disclosed herein, can thus now provide a variety of host-expression vector systems. Yeast and other eukaryotic expression systems can be used with nuclear receptors that bind heat shock proteins since these nuclear receptors are generally more difficult to express in bacteria, with the exception of ER, which can be expressed in bacteria. In a preferred embodiment of the present invention, as disclosed in the Examples, a GR LBD is expressed in *E. coli.*

Representative nuclear receptors or their ligand binding domains have been cloned and sequenced, including human RARα, human RARγ, human RXRα, human RXRβ, human PPARα, human PPARβ or δ (delta), human PPARγ, human VDR, human ER (as described in Seielstad et al., (1995) *Mol. Endocrinol.* 9: 647-658), human GR, human PR, human MR, and human AR. The ligand binding domain of each of these nuclear receptors has been identified. Using this information in conjunction with the methods described herein, one of ordinary skill in the art can express and purify LBDs of any of the nuclear receptors, bind it to an appropriate ligand, and crystallize the nuclear receptor's LBD with a bound ligand, if desired.

Extracts of expressing cells are a suitable source of receptor for purification and preparation of crystals of the chosen receptor. To obtain such expression, a vector can be constructed in a manner similar to that employed for expression of the rat TR alpha (Apriletti et al., (1995) *Protein Expres. Purif.* 6: 368-370). The nucleotides encoding the amino acids encompassing the ligand binding domain of the receptor to be expressed can be inserted into an expression vector such as the one employed by Apriletti et al. (1995). Stretches of adjacent amino acid sequences can be included if more structural information is desired.

The native and mutated nuclear receptors in general, and more particularly SR and GR polypeptides, and fragments thereof, of the present invention can also be chemically synthesized in whole or part using techniques that are known in the art (See, e.g., Creighton, (1983) Proteins: Structures and Molecular Principles, W.H. Freeman & Co., New York, United States of America, incorporated herein in its entirety).

In a preferred embodiment, the present invention provides for the first time a soluble GR/TIF2/FP complex. The GR LBD polypeptide of the present invention is expressed as a soluble polypeptide in bacteria, more preferably, *E. coli,* and can be subsequently purified therefrom. Representative purification techniques are also disclosed in the Laboratory Examples, particularly Laboratory Examples 1 and 2. The GR polypeptides of the present invention, disclosed herein, can thus now provide the ability to employ additional purification techniques for both liganded and unliganded NRs. Thus, it is envisioned, based upon the disclosure of the present invention, that purification of the unliganded or liganded NR receptor can be obtained by conventional techniques, such as hydrophobic interaction chromatography (e.g., HPLC employing a reversed phase column), ion exchange chromatography (e.g., HPLC employing an IEC column), and heparin affinity chromatography. To achieve higher purification for improved crystals of nuclear receptors it is sometimes preferable to ligand shift purify the nuclear receptor using a column that separates the receptor according to charge, such as an ion exchange or hydrophobic interaction column, and then bind the eluted receptor with a ligand. The ligand induces a change in the receptor's surface charge such that when re-chromatographed on the same column, the receptor then elutes at the position of the liganded receptor and is removed by the original column run with the unliganded receptor. Typically, saturating concentrations of ligand can be used in the column and the protein can be preincubated with the ligand prior to passing it over the column.

More recently developed methods involve engineering a "tag" such as a plurality of histidine residues placed on an end of the protein, such as on the amino terminus, and then using a nickel chelation column for purification. See Janknecht, (1991) *Proc. Natl. Acad. Sci. U.S.A.* 88: 8972-8976 (1991), incorporated herein by reference.

## VII. Formation of NR Ligand Binding Domain Crystals

In one embodiment, the present invention provides crystals of GRα LBD. In a preferred embodiment, crystals are obtained using the methodology disclosed in the Laboratory Examples hereinbelow. IN this embodiment, the GRα

LBD crystals, which can be native crystals, derivative crystals or co-crystals, have hexagonal unit cells (a hexagonal unit cell is a unit cell wherein a = b ≠ c, and wherein $\alpha = \beta = 90°$, and $\gamma = 120°$) and space group symmetry $P6_1$. There are two GRα LBD molecules and two TIF2 peptides in the asymmetric unit. In this GRα crystalline form, the unit cell has dimensions of a = b =127.656 Å, c = 87.725 Å, and $\alpha = \beta = 90°$, and $\gamma = 120°$. This crystal form can be formed in a crystallization reservoir as described in the Laboratory Examples hereinbelow.

VII.A. Preparation of NR Crystals

The native and derivative co-crystals, and fragments thereof, disclosed in the present invention can be obtained by a variety of techniques, including batch, liquid bridge, dialysis, vapor diffusion and hanging drop methods (see, e.g., McPherson, (1982) Preparation and Analysis of Protein Crystals, John Wiley, New York; McPherson, (1990) *Eur. J. Biochem.* 189:1-23; Weber, (1991) *Adv. Protein Chem.* 41:1-36). In a preferred embodiment, the vapor diffusion and hanging drop methods are used for the crystallization of NR polypeptides and fragments thereof. A more preferred hanging drop method technique is disclosed in the Laboratory Examples.

In general, native crystals of the present invention are grown by dissolving substantially pure NR polypeptide or a fragment thereof in an aqueous buffer containing a precipitant at a concentration just below that necessary to precipitate the protein. Water is removed by controlled evaporation to produce precipitating conditions, which are maintained until crystal growth ceases.

In one embodiment of the invention, native crystals are grown by vapor diffusion (see, e.g., McPherson, (1982) Preparation and Analysis of Protein Crystals, John Wiley, New York; McPherson, (1990) *Eur. J. Biochem.* 189:1-23). In this method, the polypeptide/precipitant solution is allowed to equilibrate in a closed container with a larger aqueous reservoir having a precipitant concentration optimal for producing crystals. Generally, less than about 25 μL of NR polypeptide solution is mixed with an equal volume of reservoir solution, giving a precipitant concentration about half that required for crystallization. This solution is suspended as a droplet underneath a coverslip, which is sealed onto the top of the reservoir. The sealed container is allowed to stand until crystals grow. Crystals generally form within two to six weeks, and are suitable for data collection within approximately seven to ten weeks. Of course, those of skill in the art will recognize that the above-described crystallization procedures and conditions can be varied.

VII.B. Preparation of Derivative Crystals

Derivative crystals of the present invention, e.g. heavy atom derivative crystals, can be obtained by soaking native crystals in mother liquor containing salts of heavy metal atoms. Such derivative crystals are useful for phase analysis in the solution of crystals of the present invention. In a preferred embodiment of the present invention, for example, soaking a native crystal in a solution containing methyl-mercury chloride provides derivative crystals suitable for use as isomorphous replacements in determining the X-ray crystal structure of a NR polypeptide. Additional reagents useful for the preparation of the derivative crystals of the present invention will be apparent to those of skill in the art after review of the disclosure of the present invention presented herein.

VII.C. Preparation of Co-crystals

Co-crystals of the present invention can be obtained by soaking a native crystal in mother liquor containing compounds known or predicted to bind a NR polypeptide or a fragment thereof (including a NR LBD polypeptide or a fragment thereof). Alternatively, co-crystals can be obtained by co-crystallizing a NR polypeptide or a fragment thereof (including a NR LBD polypeptide or fragment thereof) in the presence of one or more compounds known or predicted to bind the polypeptide. In a preferred embodiment, as disclosed in the Examples, such a compound is fluticasone propionate.

VII.D. Solving a Crystal Structure of the Present Invention

Crystal structures of the present invention can be solved using a variety of techniques including, but not limited to, isomorphous replacement, anomalous scattering or molecular replacement methods. Computer software packages are also helpful in solving a crystal structure of the present invention. Applicable software packages include but are not limited to the CCP4 package disclosed in the Examples, the X-PLOR™ program (Brünger, (1992) *X-PLOR, Version 3.1. A System for X-ray Crystallography and NMR,* Yale University Press, New Haven, Connecticut; X-PLOR is available from Accelrys of San Diego, California, United States of America, Xtal View (McRee, (1992) *J. Mol. Graphics* 10: 44-46; X-tal View is available from the San Diego Supercomputer Center). SHELXS 97 (Sheldrick, (1990) *Acta Cryst.* A 46: 467; SHELX 97 is available from the Institute of Inorganic Chemistry, Georg-August-Universität, Göttingen, Germany), HEAVY (Terwilliger, Los Alamos National Laboratory) and SHAKE-AND-BAKE (Hauptman. (1997) *Curr. Opin. Struct.*

*Biol.* 7: 672-80; Weeks et al., (1993) *Acta Cryst.* D 49: 179; available from the Hauptman-Woodward Medical Research Institute, Buffalo, New York) can be used. See also, Ducruix & Geige. (1992) Crystallization of Nucleic Acids and Proteins: A Practical Approach. IRL Press, Oxford, England, and references cited therein.

VIII. Characterization and Solution of a GR Ligand Binding Domain Crystal

The ligand binding domains of many nuclear receptors share a degree of identity with one another. This observation can be beneficial to the characterization and solution of a NR crystal in general and a GR LBD crystal in particular. It is also noted that, within the ligand binding domains (LBDs), the sequence identity there is a degree of homology, which is summarized in the following table:

| Sequence Identity of NR LBDs | | | | |
|---|---|---|---|---|
| | GR | MR | PR | AR |
| GR | 100% | 56% | 54% | 50% |
| MR | 56% | 100% | 55% | 51% |
| PR | 54% | 55% | 100% | 55% |
| AR | 50% | 51% | 55% | 100% |

Turning to Figure 17, a figure depicting a sequence alignment of several NRs, this figure depicts structural and sequence homology between the several NRs, as well as similarities in the overall protein architecture. In Figure 17, secondary structures in GR, PR and AR are indicated by large boxes and by annotation underneath the sequences. The secondary structure attributed to MR is that demonstrated by a homology model of the present invention, as discussed hereinbelow and in the Laboratory Examples. For each line of the alignment, the three-digit number provides the residue number of the first residue in the line. Residues within 5.0 angstroms distance of a bound ligand are identified with small boxes. The bound ligands are FP, progesterone and dihydrotestosterone for GR, PR and AR, respectively, and subunit A was used for the distance calculations in all three cases. Three residues in GR, Met639, Cys643 and Phe740, lie within 5.0 angstroms distance to FP in the GR/FP structure, but do not lie within 5.0 angstroms distance to Dex in the GR/Dex structure. These three residues are denoted in Figure 17 by underlining. Met639 and Cys643 interact with the propionate group in FP, as shown in the schematic diagrams of Figures 8A and 8B, and are involved in the expanded ligand binding pocket. Phe740 lies approximately 5 angstroms from the F-$CH_2$-thioester group of FP, but fails to make any significant interaction, and is not shown in either of the schematic diagrams of Figures 8A and 8B.

This information, combined with the structural features observed in a GR/FP structure of the present invention, as discussed herein below, can facilitate the design of additional modulators of GR. Such modulators can comprise FP derivatives, which are preferred modulators.

VIII.A Unique Structural Features of the GR/FP/TF2 Structure

The structure of GR in complex with fluticasone propionate and a TIF2 co-activator peptide reveals several features of the GR structure that, prior to the present disclosure, have not been observed or reported. The detailed structural information about the GR LBD and the expanded binding pocket provided herein can be further exploited to design receptor specific agonists or antagonists.

One unique feature of the GRα/FP/TIF2 structure relates to the conformation of the GR expanded binding pocket observed when GR binds FP. The GR/FP/TIF2 crystal structure is a significant and unique addition to the knowledge of the three-dimensional structure of the GR and of the associated changes in that structure as a result of the binding of various glucocorticoids. As evidenced in the GR/TIF2/FP crystal structure, the binding of FP induces a conformational change in the GR protein that opens additional volume into which the proponiate side chain of FP extends, leading to an expanded binding pocket. The identification of the expanded binding pocket faciliates the ability to better interpret and explain the structure-activity relationship (SAR) observed for both steroidal and non-steroidal glucocorticoids. Thus, the GR/FP/TIF2 crystal structures disclosed herein can be employed to further explain glucocorticoid binding and GR's functional activity via an analysis of compounds as they occupy the added volume of the expanded binding pocket.

VIII.A.1. The Overall Structure of the GR/TF2/FP Complex

The GR/TIF2/fluticasone propionate complex of the present invention crystallized in the $P6_1$ space group with two complexes in each asymmetry unit. Data was collected from a single crystal to a resolution of 2.6 Å. The structure

was solved using the molecular replacement method. A GR/TIF2/dexamethasone structure was used as the initial search model (see Laboratory Example 5). The electron density map calculated with the molecular replacement solutions showed clear tracings for two GR LBD monomers (GR residues 521-777), the LXXLL motifs (SEQ ID NO: 10) of two TIF2 peptides, and two bound molecules of fluticasone propionate (see Figure 2). The statistics of data sets and the refined structures are summarized in Table 1.

In a preferred embodiment of the crystals, the two GR LBD monomers in each asymmetry unit are packed into a symmetric dimer. Each GR LBD is bound with a molecule of fluticasone propionate and a TIF2 coactivator peptide (see Figure 2). The structure of the GR LBD contains 11 a-helices and 4 small β-strands that fold into a three-layer helical domain with an overall organization closely resembling the structures of PR and AR (Matias et al., (2000) *J. Biol. Chem.* 275:26164-26171; Sack et al., (2001) *Proc. Natl. Acad Sci.* 98:4904-4909; Willams & Sigler, (1998) *Nature* 393:392-396). Helices 1 and 3 form one side of a helical sandwich whereas helices 7 and 10 form the other side. The middle layer of helices (helices 4, 5, 8, and 9) are present in the top half of the protein but are absent in the bottom half of the protein. This arrangement of helices thus creates a cavity in the bottom half of the GR LBD where the fluticasone propionate is bound, and forms an element of an expanded binding pocket. The conformation adopted by FP in the binding pocket is depicted in Figure 3. Figure 3 shows the propionate moiety and the space it occupies in the expanded binding pocket.

The AF-2 helix, which plays an essential function of ligand-dependent activation, adopts the so-called active or "agonist-bound" conformation that is packed against helices 3, 4, and 10 as an integrated part of the domain structure. Following the AF-2 helix is an extended strand that forms a conserved beta sheet with a β-strand between helices 8 and 9. The LLRYLL sequence (SEQ ID NO: 11) in the TIF2 motif forms a two-turn α-helix that docks the hydrophobic leucine side chains into a groove formed in part by the AF-2 helix and residues from helices 3, 3', 4 and 5 (see Figure 2). Both ends of the coactivator helix are clamped by E754 on the AF-2 helix and K579 on helix 3, respectively. This mode of coactivator binding further stabilizes the overall GR LBD structure and the arrangement of the dimer configuration.

VIII.A.2. Differences Between the GR/TIF2/FP Complex and a GR/Dex/TIF2 Complex

Although the GR/TIF2/FP complex is similar to the GR/TIF2/dexamethasone complex ("the Dex structure"; coordinates of this structure are presented in Table 3), there are a number of differences in their crystallization conditions and their detailed structures. First, the FP complex contains a TIF2 peptide that is 10 residues shorter than the TIF2 peptide used in the GR/TIF2/Dex complex. The crystals of the GR/TIF2/FP complex were obtained using $MgSO_4$ as precipitant, whereas ammonium formate was used to obtain crystals of the GR/TIF2/Dex complex. The crystallization conditions for the GR/TIF2/Dex complex were not preferred for the GR/TIF2/FP complex.

Second, despite the similar LBD structure and arrangement of the dimer configuration between the FP and the Dex structures, there is a dramatic difference in the ligand binding pocket that is occupied by the propionate group of the fluticasone. This ligand binding pocket is much smaller in size in the GR/Dex structure. Although the 17-α-hydroxyl of dexamethasone points toward this region of the ligand binding pocket, the volume of this ligand binding pocket is largely unoccupied in the Dex structure. The volume of the ligand binding pocket in the FP structure is significantly expanded to accommodate the larger propionate group of fluticasone in both LBD monomers of the dimer, and forms an expanded binding pocket. This expansion in the volume of the ligand binding pocket in the GR/TIF2/FP structure, as compared with the GR/TIF2/Dex structure, is readily seen when Figures 5A and 5B, showing the available pocket volume in the GR/Dex structure, are compared with Figures 6A and 6B, showing the available pocket volume in the GRTIF2/FP structure. The expanded binding pocket of the FP structure is also depicted in Figure 7A and 7B, where the additional pocket volume of the FP structure over that of the Dex structure is represented by a semi-transparent surface.

Referring again to Figure 5A, this figure depicts subunit A, and shows dexamethasone, selected side-chains from the protein, and a semi-transparent surface enclosing the volume that is available to oxygen-sized ligand atoms within the ligand binding region of the GR protein in the GR/Dex structure. Figure 5B depicts subunit B, and shows the corresponding ligand molecule, side-chains and pocket volume from subunit B of the same GR/Dex structure. Protein side-chains are depicted with ball and stick representation, using thin sticks and small balls. The dexamethasone ligand is also depicted by a ball and stick representation, but using thicker sticks and larger balls. The pocket volume is depicted by a surface generated over closely-space spheres within the pocket of the GR/Dex structure. The spheres have radius 1.4 angstroms, and are arranged on a rectangular grid with a spacing of 0.3 angstroms. The surface is a "quick" surface generated within the INSIGHTII molecular graphics program using the "very high" surface quality. Atoms are represented by various shades of gray, with carbon darker than nitrogen, which is darker than oxygen, which is darker than sulfur. Fluorine is represented by a shade similar to nitrogen, but can be distinguished from nitrogen because the protein has no fluorine atoms, and the dexamethasone molecule has no nitrogens. The shades are gray are further modified by the use of depth queueing to help distinguish foreground and background features.

Turning next to Figure 6A, this figure depicts GR subunit A, and shows FP, selected side-chains from the protein, and a semi-transparent surface enclosing the volume that is available to oxygen-sized ligand atoms within the ligand binding region of the GR protein in the GR/TIF2/FP structure. Figure 6B depicts GR subunit B, showing the corresponding ligand molecule, side-chains and pocket volume from GR subunit B of the same GR/TIF2/FP structure. This figure was generated using the same methods as Figures 5A and 5B and uses the same representation and shading for atoms and volumes.

Figure 7A depicts GR GR subunit A, and shows FP, selected side-chains from the protein in the GR/FP/TIF2 structure, and a semi-transparent surface enclosing the "extra volume" that is available in the GR/FP ligand binding pocket, but not in the GR/Dex ligand binding pocket. This "extra" volume is essentially the volume depicted in Figure 5A subtracted from the volume depicted in Figure 6A and contributes to the expanded binding pocket observed in the GR/TIF2/FP structure. The available volumes in the structures were represented computationally by a collection of closely-spaced water-sized spheres. The extra volume in the GR/TIF2/FP structure was identified computationally by comparing these two collections of water-sized spheres, represented by a collection of closely-spaced spheres of radius 0.2 angstroms, and then depicted by generation of the semi-transparent surface.

Figure 7B depicts GR subunit B, and shows the corresponding ligand molecule, side-chains and "extra volume" from GR subunit B. The representation and shading for atoms is the same as Figures 5A and 5B above. The "extra volume" is depicted by a surface generated over closely-space spheres occupying the region of the GR/TIF2/FP pocket, (see Figures 6A and 6B), that is not available in the GR/Dex structure, (see Figures 5A and 5B). The spheres used for the surface calculation have a radius of 0.2 angstroms, and are arranged on a rectangular grid with a spacing of 0.3 angstroms.

Figure 8A is a schematic representation of molecular interactions between the bound FP ligand and residues in the GR protein in subunit A. The dashed lines depict most of the significant interactions of 5.0 angstroms or less, although several of the less important interactions have been omitted for clarity. The propionate side-chain adopts different conformations in the two subunits, and the approximate conformation in subunit A is depicted schematically here. Several side-chains in the protein adopt different conformations in the two subunits. While these side-chain conformations are not represented explicitly, their interactions with the ligand, and differences in these interactions in GR subunits A and B, are represented.

Figure 8B is a schematic representation of molecular interactions between the found FP ligand and residues in the GR protein in GR subunit B. The dashed lines depict most of the significant interactions of 5.0 angstroms or less, although several of the less important interactions have been omitted for clarity. The propionate side-chain adopts different conformations in the two subunits, and the approximate conformation in GR subunit B is depicted schematically in Figure 8B.

There are no large conformational changes of helices or loops between the FP and Dex structures, consistent with the observation that both ligands bound with high affinity. Instead, the larger expanded binding pocket in the FP structure is formed by gently pushing out helices 3, 6, 7 and 10 and the loop preceeding the AF-2 helix, which make up the framework of the ligand binding pocket (see Figure 4). The subtle changes in the conformation of these helices and loops in the FP structure, which are highlighted in Figure 4 by arrows, would be difficult to predict by modeling the GR/TIF2/Dex structure.

The expanded binding pocket is surrounded by side chains of more than 10 residues, including M560, L563, F623, M639, Q642, M643, M646, Y735, C736, T739 and 1747. Conformations of these side chains generally favor formation of the larger expanded binding pocket in the FP structure. By way of example, in order to assume the observed positions, residues Q642 and Y735 in monomer B undego a large conformational changes. Residue Q642, on the other hand, flips out of pocket to the space that is normally occupied by Y735. The conformational changes of these two residues contribute to an expanded binding pocket in this LBD monomer (see Table 2). The expanded binding pocket in the FP structure is a feature making the present invention distinct from known GR structures (e.g. the GR/TIF2/Dex structure, atomic coordinates of which are presented in Table 3) and offers several advantages for structure-based drug discovery over the use of the GR/TIF2/Dex structure.

VIII.E. Generation of Easily-Solved NR Crystals

The present invention discloses a substantially pure GR LBD polypeptide in crystalline form. In a preferred embodiment, exemplified in the Figures and Laboratory Examples, GR$\alpha$ is crystallized with a bound ligand and a bound co-activator peptide. Crystals can be formed from NR LBD polypeptides that are usually expressed by a cell culture, such as *E. coli.* Bromo- and iodo-substitutions can be included during the preparation of crystal forms and can act as heavy atom substitutions in GR ligands and crystals of NRs. This method can be advantageous for the phasing of the crystal, which is a crucial, and sometimes limiting, step in solving the three-dimensional structure of a crystallized entity. Thus, the need for generating the heavy metal derivatives traditionally employed in crystallography can be eliminated. After the three-dimensional structure of a NR or an NR LBD with or without a ligand and/or a co-activator bound is

determined, the resultant three-dimensional structure can be used in computational methods to design synthetic ligands for a NR and for other NR polypeptides. Further activity structure relationships can be determined through routine testing employing assays disclosed herein and known in the art.

IX. Uses of NR Crvstals and the Three-Dimensional Structure of the Ligand Binding Domain of GRα

The solved crystal structure of the present invention is useful in the design of modulators of activity mediated by the glucocorticoid receptor and by other nuclear receptors. Evaluation of the available sequence data shows that GRα is particularly similar to MR, PR and AR. The GRα LBD has approximately 56%, 54% and 50% sequence identity to the MR, PR and AR LBDs, respectively. The GRβ amino acid sequence is identical to the GRα amino acid sequence for residues 1-726, but the remaining 16 residues in GRβ show no significant similarity to the remaining 51 residues in GRα.

The present GRα X-ray structure can also be used to build models for targets where no X-ray structure is available, such as MR. Additionally, targets whose X-ray structures have been solved (e.g. AR and PR), do not comprise an expanded binding pocket. Thus, these previously solved structures cannot be effectively employed in an attempt to model these structures in association with a ligand comprising a large 17α substituent. By employing a GRα X-ray structure of the present invention, however, such models can be generated. These generated models can aid in the design of compounds to selectively modulate any desired subset of GRα, MR, PR, AR and other related nuclear receptors.

Various models can be built, such as homology models and docking models. Indeed, homology models of AR, MR and PR form aspects of the present invention. These models incorporate the expanded binding pocket observed in the GR/TIF2/FP structure. Although a few NR structures are available, theses structures do not comprise an expanded binding pocket and are therefore of limited use in rational drug design.

IX.A. Design and Development of NR Modulators

The present invention, particularly the computational methods, can be used to design drugs for a variety of nuclear receptors, such as receptors for glucocorticoids (GRs), androgens (ARs), mineralocorticoids (MRs) and progestins (PRs).

The knowledge of the structure of the GRα ligand binding domain (LBD), an aspect of the present invention, provides a tool for investigating the mechanism of action of GRα and other NR polypeptides in a subject. For example, various computer modelleing programs, as described herein, can predict the binding of various ligand molecules to the LBD of GRβ, or another steroid receptor or, more generally, nuclear receptor. Upon discovering that such binding in fact takes place, knowledge of the protein structure then allows design and synthesis of small molecules that mimic the functional binding of the ligand to the LBD of GRα, and to the LBDs of other polypeptides. This is the method of "rational" drug design, further described herein.

Use of the isolated and purified GRα crystalline structure of the present invention in rational drug design is thus provided in accordance with the present invention. Additional rational drug design techniques are described in U. S. Patent Nos. 5,834,228 and 5,872,011, incorporated herein in their entirety.

Thus, in addition to the compounds described herein, other sterically similar compounds can be formulated to interact with the key structural regions of an NR, SR or GR in general, or of GRα in particular. The generation of a structural functional equivalent can be achieved by the techniques of modeling and chemical design known to those of skill in the art and described herein. It will be understood that all such sterically similar constructs fall within the scope of the present invention.

IX.A.1. Rational Drug Design

The three-dimensional structure of a FP bound GRα is unprecedented and will greatly aid in the development of new synthetic ligands for NR polypeptides, such as GR agonists and antagonists, including those that bind exclusively to any one of the GR subtypes. In addition, NRs are well suited to modern methods, including three-dimensional structure elucidation and combinatorial chemistry, such as those disclosed in U.S. Patent Nos. 5,463,564, and 6,236,946 incorporated herein by reference. Structure determination using X-ray crystallography is possible because of the solubility properties of NRs. Computer programs that use crystallography data when practicing the present invention will enable the rational design of ligands to these receptors.

Programs such as RASMOL (Biomolecular Structures Group, Glaxo Wellcome Research & Development Stevenage, Hertfordshire, UK Version 2.6, August 1995, Version 2.6.4, December 1998, © Roger Sayle 1992-1999) and Protein Explorer (Version 1.87, July 3, 2001, © Eric Martz, 2001 and available online at http://www.umass.edu/microbio/chime/explorer/index.htm) can be used with the atomic structural coordinates from crystals generated by

practicing the invention or used to practice the invention by generating three-dimensional models and/or determining the structures involved in ligand binding. Computer programs such as those sold under the registered trademark INSIGHTII® (available from Accelrys of San Diego, California, United States of America) and the programs GRASP (Nicholls et al., (1991) *Proteins* 11: 281) and SYBYL™ (available from Tripos, Inc. of St. Louis, Missouri, United States of America) allow for further manipulations and the ability to introduce new structures. In addition, high throughput binding and bioactivity assays can be devised using purified recombinant protein and modern reporter gene transcription assays known to those of skill in the art in order to refine the activity of a designed ligand.

A method of identifying modulators of the activity of an NR polypeptide using rational drug design is thus provided in accordance with the present invention. The method comprises designing a potential modulator for an NR polypeptide of the present invention that will form non-covalent interactions with amino acids in the ligand binding pocket based upon the crystalline structure of the GRα LBD polypeptide; synthesizing the modulator; and determining whether the potential modulator modulates the activity of the NR polypeptide. In a preferred embodiment, the modulator is designed for an SR polypeptide. In a more preferred embodiment, the modulator is designed for a GRα polypeptide. Preferably, the GRα polypeptide comprises the amino acid sequence of SEQ ID NOs: 2 and 4 and more preferably, the GRα LBD comprises the amino acid sequence of SEQ ID NOs: 6 and 8. The determination of whether the modulator modulates the biological activity of an NR polypeptide is made in accordance with the screening methods disclosed herein, or by other screening methods known to those of skill in the art. Modulators can be synthesized using techniques known to those of ordinary skill in the art.

In an alternative embodiment, a method of designing a modulator of an NR polypeptide in accordance with the present invention is disclosed comprising: (a) selecting a candidate NR ligand; (b) determining which amino acid or amino acids of an NR polypeptide interact with the ligand using a three-dimensional model of a crystallized GRα LBD in complex with a co-activator peptide and fluticasone propionate; (c) identifying in a biological assay for NR activity a degree to which the ligand modulates the activity of the NR polypeptide; (d) selecting a chemical modification of the ligand wherein the interaction between the amino acids of the NR polypeptide and the ligand is predicted to be modulated by the chemical modification; (e) synthesizing a chemical compound with the selected chemical modification to form a modified ligand; (f) contacting the modified ligand with the NR polypeptide; (g) identifying in a biological assay for NR activity a degree to which the modified ligand modulates the biological activity of the NR polypeptide; and (h) comparing the biological activity of the NR polypeptide in the presence of modified ligand with the biological activity of the NR polypeptide in the presence of the unmodified ligand, whereby a modulator of an NR polypeptide is designed.

An additional method of designing modulators of an NR or an NR LBD can comprise: (a) determining which amino acid or amino acids of an NR LBD interacts with a first chemical moiety (at least one) of the ligand using a three dimensional model of a crystallized protein comprising an NR LBD in complex with a bound ligand; and (b) selecting one or more chemical modifications of the first chemical moiety to produce a second chemical moiety with a structure to either decrease or increase an interaction between the interacting amino acid and the second chemical moiety compared to the interaction between the interacting amino acid and the first chemical moiety. A structure disclosed herein, namely a structure comprising a GRα LBD in complex with fluticasone propionate, can be employed in this method. This is a general strategy only, however, and variations on this disclosed protocol would be apparent to those of skill in the art upon consideration of the present disclosure.

Once a candidate modulator is synthesized as described herein and as will be known to those of skill in the art upon contemplation of the present invention, it can be tested using assays to establish its activity as an agonist, partial agonist or antagonist, and affinity, as described herein. After such testing, a candidate modulator can be further refined by generating LBD crystals with the candidate modulator bound to the LBD. The structure of the candidate modulator can then be further refined using the chemical modification methods described herein for three dimensional models to improve the activity or affinity of the candidate modulator and make second generation modulators with improved properties, such as that of a super agonist or antagonist, as described herein.

IX.A.2. Methods for Using the GRα LBD Structural Coordinates For Molecular Design

The present invention permits the use of molecular design techniques to design, select and synthesize chemical entities and compounds, including modulatory compounds, capable of binding to the ligand binding pocket or an accessory binding site of an NR and an NR LBD, in whole or in part. Correspondingly, the present invention also provides for the application of similar techniques in the design of modulators of any NR polypeptide.

In accordance with a preferred embodiment of the present invention, the structure coordinates of a crystalline GRα LBD in complex with a co-activator and fluticasone propionate can be employed to design compounds that bind to a GR LBD (more preferably a GRα LBD) and alter the properties of a GR LBD (for example, the dimerization ability, ligand binding ability or effect on transcription) in different ways. One aspect of the present invention provides for the design of compounds that can compete with natural or engineered ligands of a GR polypeptide by binding to all, or a portion of, the binding sites on a GR LBD. The present invention also provides for the design of compounds that can

bind to all, or a portion of, an accessory binding site on a GR that is already binding a ligand. Similarly, non-competitive agonists/ligands that bind to and modulate GR LBD activity, whether or not it is bound to another chemical entity, and partial agonists and antagonists can be designed using the GR LBD structure coordinates of this invention.

A second design approach is to probe an NR or an NR LBD (preferably a GRα or GRα LBD) crystal with molecules comprising a variety of different chemical entities to determine optimal sites for interaction between candidate NR or NR LBD modulators and the polypeptide. For example, high resolution X-ray diffraction data collected from crystals saturated with solvent allows the determination of the site where each type of solvent molecule adheres. Small molecules that bind tightly to those sites can then be designed and synthesized and tested for their NR modulator activity. Representative designs are also disclosed in published PCT application WO 99/26966.

Once a computationally-designed ligand is synthesized using the methods of the present invention or other methods known to those of skill in the art, assays can be used to establish its efficacy of the ligand as a modulator of NR (preferably GRα) activity. After such assays, the ligands can be further refined by generating intact NR or NR LBD crystals with a ligand and/or a co-activator peptide bound to the LBD. The structure of the ligand can then be further refined using the chemical modification methods described herein and known to those of skill in the art, in order to improve the modulation activity or the binding affinity of the ligand. This process can lead to second generation ligands with improved properties.

Ligands also can be selected that modulate NR responsive gene transcription by the method of altering the interaction of co-activators and co-repressors with their cognate NR. For example, agonistic ligands can be selected that block or dissociate a co-repressor from interacting with a GR, and/or that promote binding or association of a co-activator. Antagonistic ligands can be selected that block co-activator interaction and/or promote co-repressor interaction with a target receptor. Selection can be done via binding assays that screen for designed ligands having the desired modulatory properties. Preferably, interactions of a GRα polypeptide are targeted. A suitable assay for screening that can be employed, *mutatis mutandis* in the present invention, as described in Oberfield et al., (1999) *Proc. Natl. Acad. Sci. U. S. A.* 96(11): 6102-6, incorporated herein in its entirety by reference. Other examples of suitable screening assays for GR function include an *in vitro* peptide binding assay representing ligand-induced interaction with coactivator (Zhou et al., (1998) *Mol. Endocrinol.* 12: 1594-1604; Parks et al., (1999) *Science* 284: 1365-1368) or a cell-based reporter assay related to transcription from a GRE (see Jenkins et al., (2001) *Trends Endocrinol. Metab.* 12: 122-126) or a cell-based reporter assay related to repression of genes driven via NF-kB (DeBosscher et al., (2000) *Proc. Natl. Acad. Sci. U. S. A.* 97: 3919-3924).

IX.A.3. Methods of Designing NR LBD Modulator Compounds

Knowledge of the three-dimensional structure of the GR LBD complex of the present invention can facilitate a general model for modulator (e.g. agonist, partial agonist, antagonist and partial antagonist) design. Other ligand-receptor complexes belonging to the nuclear receptor superfamily can have a ligand binding pocket similar to that of GR and therefore the present invention can be employed in agonist/antagonist design for other members of the nuclear receptor superfamily and the steroid receptor subfamily. Examples of suitable receptors include those of the NR superfamily and those of the SR and TR subfamilies.

The design of candidate substances, also referred to as "compounds" or "candidate compounds", that augment or inhibit NR LBD-mediated activity according to the present invention generally involves consideration of two factors. First, the compound must be capable of physically and structurally associating with a NR LBD. Non-covalent molecular interactions important in the association of a NR LBD with its substrate include hydrogen bonding, van der Waals interactions and hydrophobic interactions.

The interaction between an atom of a LBD amino acid and an atom of an LBD ligand can be made by any force or attraction described in nature. Usually the interaction between the atom of the amino acid and the ligand will be the result of a hydrogen bonding interaction, charge interaction, hydrophobic interaction, van der Waals interaction or dipole interaction. In the case of the hydrophobic interaction it is recognized that this is not a per se interaction between the amino acid and ligand, but rather the usual result, in part, of the repulsion of water or other hydrophilic group from a hydrophobic surface. Reducing or enhancing the interaction of the LBD and a ligand can be measured by calculating or testing binding energies, computationally or using thermodynamic or kinetic methods as known in the art.

Second, the compound must be able to assume a conformation that allows it to associate with a NR LBD. Although certain portions of the compound might not directly participate in this association with a NR LBD, those portions can still influence the overall conformation of the molecule. This, in turn, can have a significant impact on potency. Such conformational requirements include the overall three-dimensional structure and orientation of the chemical entity or compound in relation to all or a portion of the binding site, e.g., the ligand binding pocket or an accessory binding site of a NR LBD, or the spacing between functional groups of a compound comprising several chemical entities that directly interact with a NR LBD.

Chemical modifications will often enhance or reduce interactions of an atom of a LBD amino acid and an atom of an LBD ligand. Altering a degree of steric hinderance is one approach that can be employed to alter the interaction of a LBD binding pocket with an activation domain. Chemical modifications are preferably introduced at C-, C-H, and C-OH positions in a ligand, where the carbon is part of the ligand structure that remains the same after modification is complete. In the case of C-H, C could have 1, 2 or 3 hydrogens, but typically only one hydrogen is replaced. An H or OH can be removed after modification is complete and replaced with a desired chemical moiety.

The potential modulatory or binding effect of a chemical compound on a NR LBD can be analyzed prior to its actual synthesis and testing by the use of computer modeling techniques that employ the coordinates of a crystalline GRα LBD polypeptide of the present invention. If the theoretical structure of the given compound suggests insufficient interaction and association between it and a NR LBD, synthesis and testing of the compound is obviated. However, if computer modeling indicates a strong interaction, the molecule can then be synthesized and tested for its ability to bind and modulate the activity of a NR LBD. In this manner, synthesis of unproductive or inoperative compounds can be minimized or avoided.

A modulatory or other binding compound of a NR LBD polypeptide (preferably a GRα LBD) can be computationally evaluated and designed via a series of steps in which chemical entities or fragments are screened and selected for their ability to associate with an individual binding site or other area of a crystalline GRα LBD polypeptide of the present invention and to interact with the amino acids disposed in the binding sites.

Interacting amino acids forming contacts with a ligand and the atoms of the interacting amino acids are usually 2 to 4 angstroms away from the center of the atoms of the ligand. Generally these distances are determined by computer as discussed herein and by McRee (McRee, (1993) Practical Protein Crystallography, Academic Press, New York), however distances can be determined manually once the three dimensional model is made. More commonly, the atoms of the ligand and the atoms of interacting amino acids are 3 to 4 angstroms apart. A ligand can also interact with distant amino acids, after chemical modification of the ligand to create a new ligand. Distant amino acids are generally not in contact with the ligand before chemical modification. A chemical modification can change the structure of the ligand to make as new ligand that interacts with a distant amino acid usually at least 4.5 angstroms away from the ligand. Often distant amino acids will not line the surface of the binding cavity for the ligand, as they are too far away from the ligand to be part of a pocket or surface of the binding cavity.

A variety of methods can be used to screen chemical entities or fragments for their ability to associate with an NR LBD and, more particularly, with the individual binding sites of an NR LBD, such as ligand binding pocket or an accessory binding site. This process can begin by visual inspection of, for example, the ligand binding pocket on a computer screen based on the GRα LBD atomic coordinates presented in Tables 2-11 as described herein. Selected fragments or chemical entities can then be positioned in a variety of orientations, or docked, within an individual binding site of a GRα LBD as defined herein above. Docking can be accomplished using software programs such as those available under the tradenames QUANTA™ (Accelrys of San Diego, California, United States of America) and SYBYL™ (Tripos, Inc., St. Louis, Missouri, United States of America), followed by energy minimization and molecular dynamics with standard molecular mechanics forcefields, such as CHARM (Brooks et al., (1983) *J. Comp. Chem.,* 8: 132) and AMBER 5 (Case et al., (1997), AMBER 5, University of California, San Francisco, California, United States of America; Pearlman et al., (1995) *Comput. Phys. Commun.* 91:1-41).

Specialized computer programs can also assist in the process of selecting fragments or chemical entities. These include:

1. GRID™ program, version 17 (Goodford, (1985) *J. Med. Chem.* 28:849-57), which is available from Molecular Discovery Ltd., Oxford, UK;
2. MCSS™ program (Miranker & Karplus, (1991) *Proteins* 11:29-34), which is available from Accelrys of San Diego, California, United States of America;
3. AUTODOCK™ 3.0 program (Goodsell & Olsen, (1990) *Proteins* 8:195-202), which is available from the Scripps Research Institute, La Jolla, California, United States of America;
4. DOCK™ 4.0 program (Kuntz et al., (1992) *J. Mol. Biol.* 161:269-88), which is available from the University of California, San Francisco, California, United States of America;
5. FLEX-X™ program (See, Rarey et al., (1996) *J. Comput. Aid. Mol. Des.* 10:41-54), which is available from Tripos, Inc., St. Louis, Missouri, United States of America;
6. MVP program (Lambert, (1997) in Practical Application of Computer-Aided Drug Design, (Charifson, ed.) Marcel-Dekker, New York, New York, United States of America, pp. 243-303); and
7. LUDI™ program (Bohm, (1992) *J. Comput. Aid. Mol. Des.* 6:61-78), which is available from Accelrys of San Diego, California, United States of America.

Once suitable chemical entities or fragments have been selected, they can be assembled into a single compound or modulator. Assembly can proceed by visual inspection of the relationship of the fragments to each other on

the three-dimensional image displayed on a computer screen in relation to the structure coordinates of a GRα LBD. Manual model building using software such as QUANTA™ or SYBYL™ typically follows.

Useful programs to aid one of ordinary skill in the art in connecting the individual chemical entities or fragments include:

1. CAVEAT™ program (Bartlett et al., (1989) *Special Pub., Royal Chem. Soc.* 78:182-96), which is available from the University of California, Berkeley, California, United States of America;
2. 3D Database systems, such as MACCS-3D™ system program, which is available from MDL Information Systems, San Leandro, California, United States of America. This area is reviewed in Martin, (1992) *J. Med. Chem.* 35:2145-54; and
3. HOOK™ program (Eisen et al., (1994). *Proteins* 19:199-221), which is available from Accelrys of San Diego, California, United States of America.

Instead of proceeding to build a GR LBD modulator (preferably a GRα LBD modulator) in a step-wise fashion one fragment or chemical entity at a time as described above, modulatory or other binding compounds can be designed as a whole or *de novo* using the structural coordinates of a crystalline GRα LBD polypeptide of the present invention and either an empty binding site or optionally including some portion(s) of a known modulator(s). Applicable methods can employ the following software programs:

1. LUDI™ program (Bohm, (1992) *J. Comput. Aid. Mol. Des.* 6:61-78), which is available from Accelrys of San Diego, California, United States of America;
2. LEGEND™ program (Nishibata & Itai, (1991) *Tetrahedron* 47:8985); and
3. LEAPFROG™, which is available from Tripos Associates, St. Louis, Missouri, United States of America.

Other molecular modeling techniques can also be employed in accordance with this invention. See, e.g., Cohen et al., (1990) *J. Med. Chem.* 33: 883-94. See also, Navia & Murcko, (1992) *Curr. Opin. Struc. Biol.* 2: 202-10; U.S. Patent No. 6,008,033, herein incorporated by reference.

Once a compound has been designed or selected by the above methods, the efficiency with which that compound can bind to a NR LBD can be tested and optimized by computational evaluation. By way of particular example, a compound that has been designed or selected to function as a NR LBD modulator should also preferably traverse a volume not overlapping that occupied by the binding site when it is bound to its native ligand. Additionally, an effective NR LBD modulator should preferably demonstrate a relatively small difference in energy between its bound and free states (i.e., a small deformation energy of binding). Thus, the most efficient NR LBD modulators should preferably be designed with a deformation energy of binding of not greater than about 10 kcal/mole, and preferably, not greater than 7 kcal/mole. It is possible for NR LBD modulators to interact with the polypeptide in more than one conformation that is similar in overall binding energy. In those cases, the deformation energy of binding is taken to be the difference between the energy of the free compound and the average energy of the conformations observed when the modulator binds to the polypeptide.

A compound designed or selected as binding to an NR polypeptide (preferably a GRα LBD polypeptide) can be further computationally optimized so that in its bound state it would preferably lack repulsive electrostatic interaction with the target polypeptide. Such non-complementary (e.g., electrostatic) interactions include repulsive charge-charge, dipole-dipole and charge-dipole interactions. Specifically, the sum of all electrostatic interactions between the modulator and the polypeptide when the modulator is bound to an NR LBD preferably make a neutral or favorable contribution to the enthalpy of binding.

Specific computer software is available in the art to evaluate compound deformation energy and electrostatic interaction. Examples of programs designed for such uses include:

1. Gaussian 98™, which is available from Gaussian, Inc., Pittsburgh, Pennsylvania, United States of America;
2. AMBER™ program, version 6.0, which is available from the University of California at San Francisco, San Francisco, California, United States of America;
3. QUANTA™ program, which is available from Accelrys of San Diego, California, United States of America;
4. CHARMm® program, which is available from Accelrys of San Diego, California, United States of America; and
5. Insight II® program, which is available from Accelrys of San Diego, California, United States of America.

These programs can be implemented using a suitable computer system. Other hardware systems and software packages will be apparent to those skilled in the art after review of the disclosure of the present invention presented herein.

Once an NR LBD modulating compound has been optimally selected or designed, as described above, substitutions can then be made in some of its atoms or side groups in order to improve or modify its binding properties. Generally, initial substitutions are conservative, i.e., the replacement group will have approximately the same size, shape, hydrophobicity and charge as the original group. It should, of course, be understood that components known in the art to alter conformation are preferably avoided. Such substituted chemical compounds can then be analyzed for efficiency of fit to an NR LBD binding site using the same computer-based approaches described in detail above.

IX.B. Design of Modulators Based on the Expanded Binding Pocket of GR Observed in the GR/FP/TIF2 Structure

The GR/FP/TIF2 expanded binding pocket described herein can be employed to explain a significant amount of the SAR in the non-steroidal class of compounds for these receptors. Additional insight into the SAR of the steroidal class of glucocorticoids can also be obtained using these models derived from the GR/FP/TIF2 crystal structure.

The expanded binding pocket of GR can also be employed in the design of novel steroidal and non-steroidal glucocorticoids. For example, *de novo* design of these ligands can be carried out in the context of the crystal structure using both intuition, manual processing of compounds, or various *de novo* drug design programs such as LUDI™ (Accelrys Inc., San Diego, California, United States of America) and LEAPFROG™ (Tripos Inc., St. Louis, Missouri, United States of America), as discussed herein.

The GR/FP/TIF2 crystal structure (particularly the region comprising additional volume seen in the binding pocket of the GR/TIF2/FP structure, which contributes to the expanded binding pocket) can be further employed to construct quantitative structure-activity relationship (QSAR) models through the crystal structure or combination of the crystal structure, calculated molecular descriptors, or calculated properties of the crystal structure such as those derived from molecular mechanics (MM) calculations.

Thus, the region comprising additional volume seen in the binding pocket of the GR/TIF2/FP structure can be used in various capacities to explain the SAR of various binders of these proteins, to design *de novo* high affinity ligands, to predict the binding affinities or functional activity based on a QSAR model, or to electronically screen small to large collections of compounds at high-throughput.

As an example of the utility of the expanded binding pocket in modeling non-steroidal glucocorticoids, a docking model study was performed. The study involved the benzoxazin-1-one compound (Schering AG, Berlin, Germany; the compound is described in published PCT patent application WO 02/10143, incorporated herein by reference), which has the IUPAC name 4-(5-fluoro-2-hydroxyphenyl)-2-hydroxy-4-methyl-2-trifluoromethyl-pentanoic acid (4-methyl-1-oxo-1H-benzo[d][1,2]oxazine-6-yl)-amide and the chemical structure:

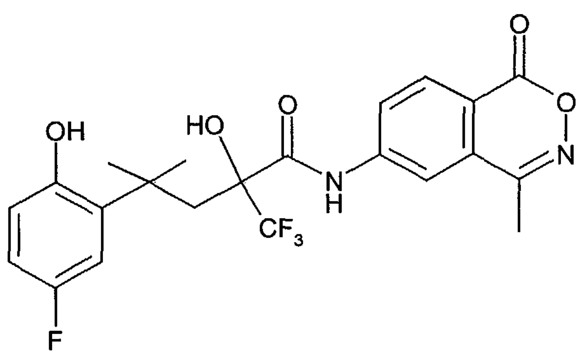

In one aspect of the present invention, this compound was modeled in the GR active site; the process and results of this modeling is presented hereinbelow in Example 6. Before the disclosure of the present invention, attempts to model this compound into the GR binding pocket were unsuccessful. Thus through the discovery of the expanded binding pocket, which forms another aspect of the present invention, a viable binding mode of this compound has been proposed.

In a further example, the non-steroidal compound A-222977 was modeled in the GR active site (see Laboratory Example 9). A-222977 has the IUPAC name 10-methoxy-2,2,4-trimethyl-5-(3-methylsulfonylmethoxyphenyl)-2,5-dihydro-1H-6-oxa-1-azachrysene and the chemical structure:

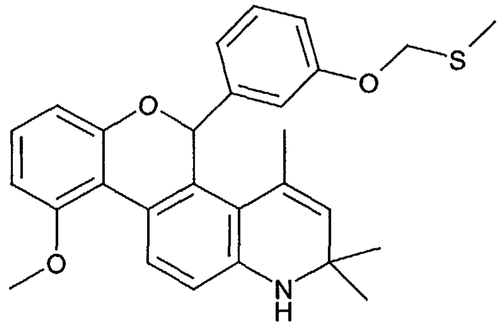

IX.C. Homology Modeling of Nuclear Receptors Using the GR/FP/TIF2 Crystal Structure

In yet another aspect of the present invention, the GR/FP structure disclosed herein can form a basis for generating homology models of other nuclear receptors. Homology modeling of a target protein generally involves the incremental substitution of amino acids of a related template protein in the attempt to produce a model of the target protein structure. This exercise assumes the template and target proteins to be related in their overall three-dimensional shape. This assumption is supported by other factors including similarity in primary amino acid sequence, receptor family membership, etc. A goal of creating a homology model can be, but need not be, to capture all of the detail usually found in a crystal structure. Preferably at least those essential portions of the protein's structure that are essential to describing its functional activity, small molecule binding properties, and other characteristics are considered. Therefore, to validate the utility of a homology model, it is preferable to infer from the model some explanation of experimentally observed data and/or information about the target protein, such as its binding affinities for various small molecules. Also, as further evidence relating a target protein's properties to its structure is acquired, it is possible to continue to refine various aspects of the homology model to account for this information. Thus, as more information is gathered and further experiments are conducted on the target protein, the homology model continues to improve and reflect the target protein's true functional nature.

For purposes of illustration, the generation of homology models of AR and PR based on a GR/FP/TIF2 structure of the present invention are discussed (see also Laboratory Examples 6-8). In the cases of AR and PR, crystal structures of these proteins have been determined previously for each of their respective natural steroidal ligands, dihydrotestosterone (DHT) (Sack et al., (2001) *Proc. Natl. Acad Sci.* 98:4904-4909.) and progesterone (PG) (Willams & Sigler, (1998) *Nature* 393:392-396), and the steroidal compound R1881 (Matias et al., (2000) *J. Biol. Chem.* 275: 26164-26171). Although these crystal structures account for aspects of the steroidal structure activity relationships (SAR) among these receptors, the structures fail to account for the SAR of the non-steroidal compounds that are known to bind either or both AR and PR. For example, in the case of AR, bicalutamide (N-[4-cyano-3-(trifluoromethyl)phenyl]-3-[(4-fluorophenyl)sulfonyl]-2-hydroxy-2-methylpropanamide) (U.S. Patent No. 4,636,505 and Tucker et al., (1988) *J. Med. Chem.* 31:954), a known, non-steroidal antagonist, binds AR with high-affinity, but this activity has not, and indeed cannot, be explained in the context of the AR crystal structures. Bicalutamide has the the IUPAC name *N*-(4-cyano-3-trifluoromethylphenyl)-3-(4-fluorobenzenesulfonyl)-2-hydroxy-2-methylpropionamide and the chemical structure:

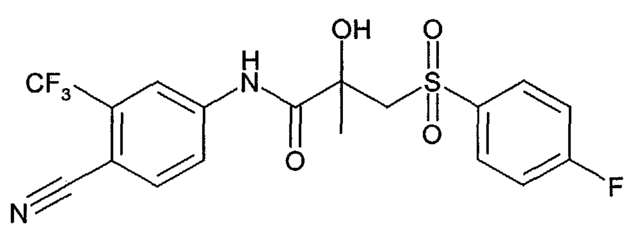

Similarly, RWJ-60130 (U.S. Patent No. 5,684,151; Palmer et al., (2001) *J. Steroid. Biochem. Mol. Biol.* 75:33-42), a known, potent, non-steroidal agonist, binds PR with a high-affinity, but, as with AR and bicalutamide, its activity has not and cannot be explained in the context of the PR crystal structures. RWJ-60130 has the IUPAC name 3-(4-chloro-3-trifluoromethylphenyl)-1-(4-iodobenzensulfonyl)-6-methyl-1,4,5,6-tetrahydropyridazine and the chemical structure:

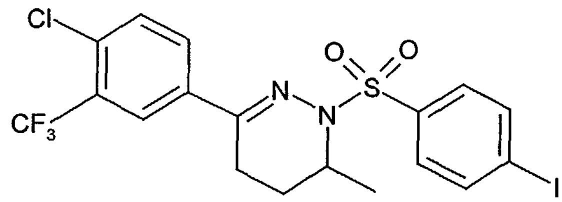

In both cases, the inexplicability of the compounds' high affinity is related to the size of the compounds; these non-steroidal ligands are simply too large to fit in the ligand binding pockets as depicted in the AR and PR crystal structures.

With the solution of a GR/FP/TIF2 crystal structure and the appearance of an expanded binding pocket as provided by the present invention, construction of AR and PR (and other NR) homology models that explain the SAR of these large, potent binders became possible. Also, given the high sequence identity in the LBD of GR to AR (50%) and PR (54%) and receptor family similarity (as depicted hereinabove), a similar expanded binding pocket is expected to materialize in AR and PR under appropriate conditions. Thus, the construction of AR and PR homology models bound with bicalutamide and RWJ-60130, respectively, can be undertaken using the crystal structure of GR bound with FP and a TIF2 peptide.

It is noted that prior to the disclosure of the present invention, accurate AR, MR and PR homology and docking models could not be generated. Although structures for AR, MR and PR have been published, these structures do not account for the expanded binding pocket observed in the present GR/TIF2/FP structure. The presence of the expanded binding pocket is useful in explaining the observed binding of ligands to NRs. Models that do not include the expanded binding pocket cannot adequately explain observed binding modes. Therefore, models generated employing previous known NR structures that do not include the expanded binding pocket are incomplete and are not the best representation of the NR structures for which the models were generated. Moreover, models lacking the expanded binding pocket are not the best models to employ in the rational design of NR modulators.

Thus, in one embodiment, a data structure embodied in a computer-readable medium is provided. Preferably, the data structure comprises: a first data field containing data representing spatial coordinates of an NR LBD comprising an expanded binding pocket, wherein the first data field is derived by combining at least a part of a second data field with at least a part of a third data field, and wherein (a) the second data field contains data representing spatial coordinates of the atoms comprising a GR LBD comprising an expanded binding pocket in complex with a ligand; and (b) the third data field contains data representing spatial coordinates of the atoms comprising a NR LBD.

IX.C.1. Applications of NR Homology Models

The NR (and particularly AR, MR and PR) homology models described herein can be employed to explain a majority of the SAR in the non-steroidal class of compounds for these receptors. Additional insight into the SAR of the steroidal class of compounds for NRs, such as AR and PR can also be obtained using these models.

These models can be employed in the design of novel steroidal and non-steroidal ligands for NRs (e.g. AR, MR and PR). For example, de novo design of NR ligands can be carried out in the context of these homology models using both intuition, manual processing of compounds, or various de novo drug design programs such as LUDI™ (Accelrys Inc., San Diego, California United States of America) and LEAPFROG™ (Tripos Inc., St. Louis, Missouri, United States of America).

The models can be used to construct quantitative structure-activity relationship (QSAR) models solely through the homology models or through the combination of the models, calculated molecular descriptors, or calculated properties of the homology models such as those derived from molecular mechanics (MM) calculations.

Thus, the homology models of the present invention can be employed in various capacities to explain the SAR of various binders of these proteins, de novo design of high affinity ligands, predict the binding affinities or functional activity based on a QSAR model, or electronically high-throughput screen small to large collections of compounds.

IX.C.2. Method of Forming a Homology Model of an NR

In one aspect of the present invention a method of forming a homology model of an NR is disclosed. In a preferred embodiment, the method comprises: (a) providing a template amino acid sequence comprising a GR complex comprising a large pocket volume as disclosed herein; (b) providing a target NR amino acid sequence; (c) aligning the target sequence and the template sequence to form a homology model. Preferably, the template amino acid comprises the LBD of GRα in complex with a co-activator peptide and fluticasone propionate.

This preferred method is best illustrated by way of specific example, namely the construction of an AR homology model. Those of ordinary skill in the art will appreciate that although the method is presented in the context of generating an AR homology model, the method can be employed *mutatis mutandis* to generate homology models for any NR.

In the formulation of an AR homology model based on the GR/FP/TIF2 structure of the present invention, sequence alignments of the AR and GR LBDs can be initially obtained using the alignment algorithm implemented in MVP (Lambert, (1997) in Practical Application of Computer-Aided Drug Design (Charifson, ed.), Marcel Dekker, New York, New York, United States of America, pp 243-303). Target NRs that can be characterized in terms of atomic coordinates are especially preferred, due to the relative ease of manipulation. In this specific example of the preferred method, the GR LBD, which is more preferably derived from the GR/FP/TIF2 structure disclosed herein, is the template amino acid sequence. The AR amino acid sequence is the target NR amino acid sequence in this example.

After three-dimensional alignment and coordinate translation of the GR/FP crystal structure into a standard orientation using MVP, a desired subunit can be selected for use in the homology model. For example, the second subunit of the GR/FP/TIF2 structure can be selected when constructing an AR homology model. Throughout the process of building a homology model, the Homology package in the INSIGHTII program (Accelrys Inc., San Diego, California, United States of America) or a similar computer software package can be used to visualize the proteins, extract the LBD sequences, manually align the sequences, transform the amino acid residues, manually manipulate the amino acid sidechain conformers, and export the three-dimensional coordinates in appropriate file formats.

A desired subunit (e.g. the second subunit of the GR/FP/TIF2 structure) can be loaded into the display area of INSIGHTII along with the target NR structure (e.g. the AR/DHT structure) for comparison purposes. Following any desired comparison, the Homology package can be used to extract the template and target (e.g. the GR and AR, respectively) primary amino acid sequences. The sequences are preferably extracted from crystal structure coordinate files, although a target NR amino acid sequence can also be manually built and manipulated. If desired, the sequences can then be manually aligned using Homology and by comparison with those alignments obtained using the MVP program.

Next, a transformation of the amino acid residues can be performed. A desired transformation can be carried out and initial three-dimensional coordinates of the NR homology model can be assigned using the AssignCoods method in the Homology modeling package or another suitable software package. When assigning coordinates to an NR in a homology model, corresponding residues in a template sequence can be employed. For example, when assigning the coordinates of residues I672-K883 in the AR homology model, the corresponding coordinates of residues T531-D742 in the GR/FP crystal structure were used. Additionally, when assigning the coordinates of residues M886-H917 in the AR homology model, the corresponding coordinates of residues K744-H775 in the GR/FP/TIF2 crystal structure were used. Finally, when assigning the coordinates of residues S884-H885 in the AR homology model, the corresponding coordinates from the AR/DHT crystal structure were used.

Following transformation and assignment of coordinates in an NR homology model, it might be desirable to manually manipulate the homology model. Desired manual modifications of amino acid side chain conformers can be carried out after comparing the conformations of corresponding residues in the initial homology model and the crystal structure of the target sequence.

Table 4 presents the three-dimensional coordinates of AR in complex with bicalutamide obtained from homology modeling of the crystal structure coordinates of GRα in complex with FP, as derived from the disclosed method. Table 5 presents the three-dimensional coordinates of PR in Complex with RWJ-60130 obtained from homology modeling of the crystal structure coordinates of GRα in complex with FP.

IX.C.3. Method of Modeling the Interaction Between an NR and a Ligand

In another aspect of the present invention, a method of modeling an interaction between an NR and a non-steroid ligand is provided. In a preferred embodiment, the method comprises: (a) providing a homology model of a target NR generated using a GR complex that comprises an expanded binding pocket as disclosed herein; (b) providing coordinates of a non-steroid ligand; (c) docking the non-steroid ligand with homology model to form a NR/ligand model; and (d) optimizing the geometry of the NR/ligand model, whereby an interaction between an NR and a non-steroid ligand is modeled.

As noted, a GR complex that comprises an expanded binding pocket as disclosed herein can be employed to model an interaction between an NR and a ligand. In the following section, a preferred method of modeling an interaction between an NR and a ligand is presented by way of specific example, namely modeling an interaction between PR and the ligand RWJ-60130. Those of ordinary skill in the art will appreciate that although the method is presented in the context of modeling an interaction between a PR and RWJ-60130, the method can be employed *mutatis mutandis* to model an interaction between any NR and a ligand.

First, a homology model can be constructed. Construction of such a model can be achieved by employing the method disclosed in detail in section IX.C.2. hereinabove. Although the precise steps of forming a homology model

for a PR using the GR/FP/TIF2 structure that forms an aspect of the present invention are not presented here, preferred steps mirror, *mutatis mutandis,* those presented hereinabove in the formation of an AR homology model. The follow discussion assumes the preparation of a PR homology model.

Continuing with the preferred method, initial coordinates for a non-steroid ligand are provided. Coordinates for a non-steroid ligand can be generated using any suitable software package; the software package CONCORD v4.0.4 (Tripos Inc., St. Louis, Missouri, United States of America) is especially preferred. In the present specific example, initial coordinates of the PR ligand RWJ-60130 are generated using CONCORD v4.0.4.

Next, any desired ligand conformers are generated. These ligand conformers can be generated using software adapted for that purpose. Preferred software includes the GROW algorithm available in MVP and optimized using the CVFF module, as implemented in MVP. In the context of the present PR example, a number of conformers of the initial RWJ-60130 geometry are generated.

Subsequently, the ligand conformers are docked into the homology model. This operation can be performed using, for example, the DOCK module of INSIGHTII. Each generated conformer can be automatically or manually docked into the homology model and evaluated for goodness of fit. The evaluation can comprise a computational analysis of the ligand-NR structure or it can be a simple visual inspection of the structure. The best fitting conformer is taken as representative of the conformation the ligand takes when it binds the NR. Continuing with the PR/RWJ-60130 complex example, each of the resulting conformers are hand-docked into the initial PR homology model and the best-fitting conformer is selected as the proposed binding conformation of RWJ-60130.

After docking of the best-fitting conformer into the NR, the complex is modified as desired, for example to correct residue numbering. MVP can be employed to perform any desired modifications. With reference to the example of the PR/RWJ-60130 complex, the complex is exported from INSIGHTII in the identical coordinate reference frame as the GR/FP/TIF2 crystal structure. MVP and the sequence alignments of GR and PR are employed to correct the residue numbering of the initial PR model.

Finally, optimization of the geometry of the NR/ligand model is performed. Again, suitable software can be employed to perform the optimization. Although any software can be employed, the CVFF software package of MVP is preferred for carrying out the optimization operation. Desirable settings and conditions for the optimization will be known to those of ordinary skill in the art upon consideration of the present disclosure. By way of specific example, geometry optimization of the PR/RWJ-60130 homology model complex is carried out using CVFF as implemented in MVP, as noted above. All atoms in the complex are fixed in space except for those atoms contained in RWJ-60130 and the initial PR model that were within a desired distance constraint, for example within 6 angstroms of any atom in RWJ-60130. The CVFF energy terms are calculated using only those atoms within desired distance constraint of the ligand, for example within 16 angstroms of (and including) RWJ-60130. Geometry optimization of the protein-ligand complex is preferably carried out using the conjugate gradient method as implemented in MVP and with a convergence criteria of a 0.1 change in the gradient.

Table 6 presents a subset of the three-dimensional coordinates of GR in complex with the Benzoxazin-1-one obtained from modeling of the crystal structure of GRα in complex with FP. Table 7 presents a subset of the three-dimensional coordinates of GRα in complex with A-222977 obtained from modeling of the crystal structure of GRα in complex with FP.

IX.C.4. Method of Designing a Non-steroid Modulator of an NR Using a Homology Model

In yet another embodiment of the present invention, a method of designing a non-steroid modulator of an NR using a homology model is disclosed. In a preferred embodiment, the method comprises: (a) modeling an interaction between an NR and a non-steroid ligand using the structure of a GR complex comprising a large pocket volume; (b) evaluating the interaction between the NR and the non-steroid ligand to determine a first binding efficiency; (c) modifying the structure of the non-steroid ligand to form a modified ligand; (d) modeling an interaction between the modified ligand and the NR; (e) evaluating the interaction between the NR and the modified ligand to determine a second binding efficiency; and (f) repeating steps (c)-(e) a desired number of times if the second binding efficiency is less than the first binding efficiency. The disclosed method can be applied to any NR.

In one embodiment, an interaction between an NR and a non-steroid ligand is modeled using the structure of a GRα LBD in complex with TIF2 and fluticasone propionate, an aspect of the present invention. Such an interaction can be modeled using the steps disclosed hereinabove in section IX.C.3.

Next, the interaction between the NR and the non-steroid ligand is evaluated in order to determine a first binding efficiency. The evaluation can be quantitative or qualitative. When a quantitative comparison is desired, software programs can be employed to calculate various binding parameters, which can be subsequently analyzed to arrive at one or more parameters that described aspects of binding efficiency.

Following an assessment of a first binding efficiency, the structure of the non-steroid ligand is modified to form a modified ligand. Such modification can include altering one or more properties of the ligand predicted to enhance

binding efficiency of the ligand to the NR. The modification(s) is preferably performed using a suitable software package. Modules of software packages INSIGHTII and/or MVP can be employed to accomplish any desired modification(s). The modification(s) can take any of a variety of forms, for example functional groups can be replaced and bond angles can be altered.

Then, an interaction between the modified ligand and the NR can be modeled. Again, the interaction can be modeled using the steps disclosed hereinabove and in section IX.C.3.

Finally, the interaction between the NR and the modified ligand is evaluated to determine a second binding efficiency. As described above, software programs can be employed to calculate various binding parameters and binding parameters. A quantitative assessment of a second binding efficiency is preferred.

Lastly, the above steps are repeated a desired number of times if the second binding efficiency is less than the first binding efficiency. By performing multiple iterations of the above method, a non-steroid ligand can be designed using a GR complex comprising a large pocket volume in accordance with the present invention.

IX.D. Method of Screening for Chemical and Biological Modulators of the Biological Activity of an NR

A candidate substance identified according to a screening assay of the present invention has an ability to modulate the biological activity of an NR or an NR LBD polypeptide. In a preferred embodiment, such a candidate compound can have utility in the treatment of disorders and/or conditions and/or biological events associated with the biological activity of an NR or an NR LBD polypeptide, including transcription modulation.

In a cell-free system, the method preferably comprises the steps of establishing a control system comprising a GRα polypeptide and a ligand which is capable of binding to the polypeptide; establishing a test system comprising a GRα polypeptide, the ligand, and a candidate compound; and determining whether the candidate compound modulates the activity of the polypeptide by comparison of the test and control systems. A representative ligand can comprise fluticasone propionate or other small molecule, and in this embodiment, the biological activity or property screened can include binding affinity or transcription regulation. The GRα polypeptide can be in soluble or crystalline form.

In another embodiment of the invention, a soluble or a crystalline form of a GRα polypeptide or a catalytic or immunogenic fragment or oligopeptide thereof, can be used for screening libraries of compounds in any of a variety of drug screening techniques. The fragment employed in such a screening can be affixed to a solid support. The formation of binding complexes, between a soluble or a crystalline GRα polypeptide and the agent being tested, will be detected. In a preferred embodiment, the soluble or crystalline GRα polypeptide has an amino acid sequence of any of SEQ ID NOs: 2 and 4. When a GRα LBD polypeptide is employed, a preferred embodiment includes a soluble or a crystalline GRα polypeptide having the amino acid sequence of any of SEQ ID NOs: 6 and 8.

Another technique for drug screening which can be used provides for high throughput screening of compounds having suitable binding affinity to the protein of interest as described in published PCT application WO 84/03564, herein incorporated by reference. In this method, as applied to a soluble or crystalline polypeptide of the present invention, large numbers of different small test compounds are synthesized on a solid substrate, such as plastic pins or some other surface. The test compounds are reacted with the soluble or crystalline polypeptide, or fragments thereof. Bound polypeptide is then detected by methods known to those of skill in the art. The soluble or crystalline polypeptide can also be placed directly onto plates for use in the aforementioned drug screening techniques.

In yet another embodiment, a method of screening for a modulator of an NR or an NR LBD polypeptide comprises: providing a library of test samples; contacting a soluble or a crystalline form of an NR or a soluble or crystalline form of an NR LBD polypeptide with each test sample; detecting an interaction between a test sample and a soluble or a crystalline form of an NR or a soluble or a crystalline form of an NR LBD polypeptide; identifying a test sample that interacts with a soluble or a crystalline form of an NR or a soluble or a crystalline form of an NR LBD polypeptide; and isolating a test sample that interacts with a soluble or a crystalline form of an NR or a soluble or a crystalline form of an NR LBD polypeptide.

In each of the foregoing embodiments, an interaction can be detected spectrophotometrically, radiologically, colorimetrically or immunologically. An interaction between a soluble or a crystalline form of an NR or a soluble or a crystalline form of an NR LBD polypeptide and a test sample can also be quantified using methodology known to those of skill in the art.

In accordance with the present invention there is also provided a rapid and high throughput screening method that relies on the methods described above. This screening method comprises separately contacting each of a plurality of substantially identical samples with a soluble or a crystalline form of an NR or a soluble or a crystalline form of an NR LBD and detecting a resulting binding complex. In such a screening method the plurality of samples preferably comprises more than about $10^4$ samples, or more preferably comprises more than about $5 \times 10^4$ samples.

In another embodiment, a method for identifying a substance that modulates GR LBD function is also provided. In a preferred embodiment, the method comprises: (a) isolating a GR polypeptide of the present invention; (b) exposing the isolated GR polypeptide to a plurality of substances; (c) assaying binding of a substance to the isolated GR polypeptide; and (d) selecting a substance that demonstrates specific binding to the isolated GR LBD polypeptide. By the term "exposing the GR polypeptide to a plurality of substances", it is meant both in pools and as mutiple samples of "discrete" pure substances.

IX.E. Method of Identifying Compounds Which Inhibit Ligand Binding

In one aspect of the present invention, an assay method for identifying a compound that inhibits binding of a ligand to an NR polypeptide is disclosed. A ligand, such as fluticasone propionate (which associates with at least GR), can be employed in the assay method as the ligand against which the inhibition by a test compound is gauged. In the following discussion of Section IX.E., it will be understood that although GR is used as an example, the method is equally applicable to any of NR polypeptide. The method comprises (a) incubating a GR polypeptide with a ligand in the presence of a test inhibitor compound; (b) determining an amount of ligand that is bound to the GR polypeptide, wherein decreased binding of ligand to the GR polypeptide in the presence of the test inhibitor compound relative to binding in the absence of the test inhibitor compound is indicative of inhibition; and (c) identifying the test compound as an inhibitor of ligand binding if decreased ligand binding is observed. Preferably, the ligand is fluticasone propionate.

In another aspect of the present invention, the disclosed assay method can be used in the structural refinement of candidate GR inhibitors. For example, multiple rounds of optimization can be followed by gradual structural changes in a strategy of inhibitor design. A strategy such as this is facilitated by the disclosure of the atomic coordinates of a GR complex in accordance with the present invention.

X. Design, Preparation and Structural Analysis of Additional NR Polypeptides and NR LBD Mutants and Structural Equivalents

The present invention provides for the generation of NR polypeptides and NR (preferably GRα and GRα LBD mutants), and the ability to solve the crystal structures of those that crystallize. Thus, an aspect of the present invention involves the use of both targeted and random mutagenesis of the GR gene for the production of a recombinant protein with improved or desired characteristics for the purpose of crystallization, characterization of biologically relevant protein-protein interactions, and compound screening assays, or for the production of a recombinant protein having another desirable characteristic(s). Polypeptide products produced by the methods of the present invention are also disclosed herein.

The structure coordinates of a NR LBD provided in accordance with the present invention also facilitate the identification of related proteins or enzymes analogous to GRα in function, structure or both, (for example, a GRβ) which can lead to novel therapeutic modes for treating or preventing a range of disease states. More particularly, through the provision of the mutagenesis approaches as well as the three-dimensional structure of a GRα LBD disclosed herein, desirable sites for mutation are identified.

X.A. Design and Preparation of Sterically Similar Compounds

A further aspect of the present invention is that sterically similar compounds can be formulated to mimic the key portions of an NR LBD structure. Such compounds are functional equivalents. The generation of a structural functional equivalent can be achieved by the techniques of modeling and chemical design known to those of skill in the art and described herein. Modeling and chemical design of NR and NR LBD structural equivalents can be based on the structure coordinates of a crystalline GRα LBD polypeptide of the present invention. It will be understood that all such sterically similar constructs fall within the scope of the present invention.

X.B. Design and Preparation of NR Polypeptides

The generation of chimeric GR polypeptides is also an aspect of the present invention. Such a chimeric polypeptide can comprise an NR LBD polypeptide or a portion of an NR LBD, (e.g. a GRα LBD) that is fused to a candidate polypeptide or a suitable region of the candidate polypeptide, for example GRβ. Throughout the present disclosure it is intended that the term "mutant" encompass not only mutants of an NR LBD polypeptide but chimeric proteins generated using an NR LBD as well. It is thus intended that the following discussion of mutant NR LBDs apply *mutatis mutandis* to chimeric NR polypeptides and NR LBD polypeptides and to structural equivalents thereof.

In accordance with the present invention, a mutation can be directed to a particular site or combination of sites of a wild-type NR LBD. For example, an accessory binding site or the binding pocket can be chosen for mutagenesis. Similarly, a residue having a location on, at or near the surface of the polypeptide can be replaced, resulting in an altered surface charge of one or more charge units, as compared to the wild-type NR and NR LBDs. Alternatively, an amino acid residue in an NR or an NR LBD can be chosen for replacement based on its hydrophilic or hydrophobic

characteristics.

Such mutants can be characterized by any one of several different properties, i.e. a "desired" or "predetermined" characteristic as compared with the wild type NR LBD. For example, such mutants can have an altered surface charge of one or more charge units, or can have an increase in overall stability. Other mutants can have altered substrate specificity in comparison with, or a higher specific activity than, a wild-type NR or an NR LBD.

NR and NR LBD mutants of the present invention can be generated in a number of ways. For example, the wild-type sequence of an NR or an NR LBD can be mutated at those sites identified using this invention as desirable for mutation, by means of oligonucleotide-directed mutagenesis or other conventional methods, such as deletion. Alternatively, mutants of an NR or an NR LBD can be generated by the site-specific replacement of a particular amino acid with an unnaturally occurring amino acid. In addition, NR or NR LBD mutants can be generated through replacement of an amino acid residue, for example, a particular cysteine or methionine residue, with selenocysteine or selenomethionine. This can be achieved by growing a host organism capable of expressing either the wild-type or mutant polypeptide on a growth medium depleted of either natural cysteine or methionine (or both) but enriched in selenocysteine or selenomethionine (or both).

As disclosed in the Examples presented below, mutations can be introduced into a DNA sequence coding for a NR or an NR LBD using synthetic oligonucleotides. These oligonucleotides contain nucleotide sequences flanking the desired mutation sites. Mutations can be generated in the full-length DNA sequence of a NR or an NR LBD or in any sequence coding for polypeptide fragments of an NR or an NR LBD.

According to the present invention, a mutated NR or NR LBD DNA sequence produced by the methods described above, or any alternative methods known in the art, can be expressed using an expression vector. An expression vector, as is well known to those of skill in the art, typically includes elements that permit autonomous replication in a host cell independent of the host genome, and one or more phenotypic markers for selection purposes. Either prior to or after insertion of the DNA sequences surrounding the desired NR or NR LBD mutant coding sequence, an expression vector also will include control sequences encoding a promoter, operator, ribosome binding site, translation initiation signal, and, optionally, a repressor gene or various activator genes and a signal for termination. In some embodiments, where secretion of the produced mutant is desired, nucleotides encoding a "signal sequence" can be inserted prior to an NR or an NR LBD mutant coding sequence. For expression under the direction of the control sequences, a desired DNA sequence must be operatively linked to the control sequences; that is, the sequence must have an appropriate start signal in front of the DNA sequence encoding the NR or NR LBD mutant, and the correct reading frame to permit expression of that sequence under the control of the control sequences and production of the desired product encoded by that NR or NR LBD sequence must be maintained.

After a review of the disclosure of the present invention presented herein, any of a wide variety of well-known available expression vectors can be useful to express a mutated coding sequence of this invention. These include for example, vectors consisting of segments of chromosomal, non-chromosomal and synthetic DNA sequences, such as various known derivatives of SV40, known bacterial plasmids, e.g., plasmids from *E. coli* including col E1, pCR1, pBR322, pMB9 and their derivatives, wider host range plasmids, e.g., RP4, phage DNAs, e.g., the numerous derivatives of phage λ, e.g., NM 989, and other DNA phages, e.g., M13 and filamentous single stranded DNA phages, yeast plasmids and vectors derived from combinations of plasmids and phage DNAs, such as plasmids which have been modified to employ phage DNA or other expression control sequences. In the preferred embodiments of this invention, vectors amenable to expression in a pET-based expression system are employed. The pET expression system is available from Novagen/Invitrogen, Inc. of Carlsbad, California. Expression and screening of a polypeptide of the present invention in bacteria, preferably *E. coli,* is a preferred aspect of the present invention.

In addition, any of a wide variety of expression control sequences--sequences that control the expression of a DNA sequence when operatively linked to it--can be used in these vectors to express the mutated DNA sequences according to this invention. Such useful expression control sequences, include, for example, the early and late promoters of SV40 for animal cells, the lac system, the trp system the TAC or TRC system, the major operator and promoter regions of phage λ, the control regions of fd coat protein, all for *E. coli,* the promoter for 3-phosphoglycerate kinase or other glycolytic enzymes, the promoters of acid phosphatase, e.g., Pho5, the promoters of the yeast α-mating factors for yeast, and other sequences known to control the expression of genes of prokaryotic or eukaryotic cells or their viruses, and various combinations thereof.

A wide variety of hosts are also useful for producing mutated NR, SR or GR and NR, SR or GR LBD polypeptides according to this invention. These hosts include, for example, bacteria, such as *E. coli. Bacillus* and *Streptomyces,* fungi, such as yeasts, and animal cells, such as CHO and COS-1 cells, plant cells, insect cells, such as SF9 cells, and transgenic host cells. Expression and screening of a polypeptide of the present invention in bacteria, preferably *E. coli,* is a preferred aspect of the present invention.

It should be understood that not all expression vectors and expression systems function in the same way to express mutated DNA sequences of this invention, and to produce modified NR, SR or GR and NR, SR or GR LBD polypeptides or NR, SR or GR or NR, SR or GR LBD mutants. Neither do all hosts function equally well with the same

expression system. One of skill in the art can, however, make a selection among these vectors, expression control sequences and hosts without undue experimentation and without departing from the scope of this invention. For example, an important consideration in selecting a vector will be the ability of the vector to replicate in a given host. The copy number of the vector, the ability to control that copy number, and the expression of any other proteins encoded by the vector, such as antibiotic markers, should also be considered.

In selecting an expression control sequence, a variety of factors should also be considered. These include, for example, the relative strength of the system, its controllability and its compatibility with the DNA sequence encoding a modified NR or NR LBD polypeptide of this invention, with particular regard to the formation of potential secondary and tertiary structures.

Hosts should be selected by consideration of their compatibility with the chosen vector, the toxicity of a modified polypeptide to them, their ability to express mature products, their ability to fold proteins correctly, their fermentation requirements, the ease of purification of a modified GR or GR LBD and safety. Within these parameters, one of skill in the art can select various vector/expression control system/host combinations that will produce useful amounts of a mutant polypeptide. A mutant polypeptide produced in these systems can be purified, for example, via the approaches disclosed in the Laboratory Examples.

Once a mutation(s) has been generated in the desired location, such as an active site or dimerization site, the mutants can be tested for any one of several properties of interest, i.e. "desired" or "predetermined" positions. For example, mutants can be screened for an altered charge at physiological pH. This property can be determined by measuring the mutant polypeptide isoelectric point (pl) and comparing the observed value with that of the wild-type parent. Isoelectric point can be measured by gel-electrophoresis according to the method of Wellner (Wellner, (1971) *Anal. Chem.* 43:597). A mutant polypeptide containing a replacement amino acid located at the surface of the enzyme, as provided by the structural information of this invention, can lead to an altered surface charge and an altered pl.

X.C. Generation of an NR or NR LBD Mutants

In another aspect of the present invention, a unique NR or NR LBD polypeptide is generated. Such a mutant can facilitate purification and the study of the structure and the ligand-binding abilities of a NR polypeptide. Thus, an aspect of the present invention involves the use of both targeted and random mutagenesis of the GR gene for the production of a recombinant protein with improved solution characteristics for the purpose of crystallization, characterization of biologically relevant protein-protein interactions, and compound screening assays , or for the production of a recombinant polypeptide having other characteristics of interest. Expression of the polypeptide in bacteria, preferably *E. coli,* is also an aspect of the present invention.

In one embodiment, targeted mutagenesis was performed using a sequence alignment of several nuclear receptors, primarily steroid receptors. Several residues that were hydrophobic in GR and hydrophilic in other receptors were chosen for mutagenesis. Most of these residues were predicted to be solvent exposed hydrophobic residues in GR. Therefore, mutations were made to change these hydrophobic residues to hydrophilic in attempt to improve the solubility and stability of *E.coli*-expressed GR LBD.

Random mutagenesis can be performed on residues where a significant difference, hydrophobic versus hydrophilic, is observed between GR and other steroid receptors based on sequence alignment. Such positions can be randomized by oligo-directed or cassette mutagenesis. A GR LBD protein library can be sorted by an appropriate display system to select mutants with improved solution properties. Residues in GR that meet the criteria for such an approach include: V538, V552, W557, F602, L636, Y648, Y660, L685, M691, V702, W712, L733, and Y764. In addition, residues predicted to neighbor these positions can also be randomized.

A method of modifying a test NR polypeptide is thus disclosed. The method can comprise: providing a test NR polypeptide sequence having a characteristic that is targeted for modification; aligning the test NR polypeptide sequence with at least one reference NR polypeptide sequence for which an X-ray structure is available, wherein the at least one reference NR polypeptide sequence has a characteristic that is desired for the test NR polypeptide; building a three-dimensional model for the test NR polypeptide using the three-dimensional coordinates of the X-ray structure (s) of the at least one reference polypeptide and its sequence alignment with the test NR polypeptide sequence; examining the three-dimensional model of the test NR polypeptide for differences with the at least one reference polypeptide that are associated with the desired characteristic; and mutating at least one amino acid residue in the test NR polypeptide sequence located at a difference identified above to a residue associated with the desired characteristic, whereby the test NR polypeptide is modified. By the term "associated with a desired characteristic" it is meant that a residue is found in the reference polypeptide at a point of difference wherein the difference provides a desired characteristic or phenotype in the reference polypeptide.

A method of altering the solubility of a test NR polypeptide is also disclosed in accordance with the present invention. In a preferred embodiment, the method comprises: (a) providing a reference NR polypeptide sequence and a test NR polypeptide sequence; (b) comparing the reference NR polypeptide sequence and the test NR polypeptide

sequence to identify one or more residues in the test NR sequence that are more or less hydrophilic than a corresponding residue in the reference NR polypeptide sequence; and (c) mutating the residue in the test NR polypeptide sequence identified in step (b) to a residue having a different hydrophilicity, whereby the solubility of the test NR polypeptide is altered.

By the term "altering" it is meant any change in the solubility of the test NR polypeptide, including preferably a change to make the polypeptide more soluble. Such approaches to obtain soluble proteins for crystallization studies have been successfully demonstrated in the case of HIV integration intergrase and the human leptin cytokine. See Dyda et al., (1994) *Science* 266:1981-86; and Zhang et al., (1997) *Nature* 387:206-209.

Typically, such a change involves substituting a residue that is more hydrophilic than the wild type residue. Hydrophobicity and hydrophilicity criteria and comparision information are set forth herein below. Optionally, the reference NR polypeptide sequence is an AR or a PR sequence, and the test polypeptide sequence is a GR polypeptide sequence. Alternatively, the reference polypeptide sequence is a crystalline GR LBD. The comparing of step (b) is preferably by sequence alignment. More preferably, the screening is carried out in bacteria, even more preferably, in *E. coli.*

A method for modifying a test NR polypeptide to alter and preferably improve the solubility, stability in solution and other solution behavior, to alter and preferably improve the folding and stability of the folded structure, and to alter and preferably improve the ability to form ordered crystals is also provided in accordance with the present invention. The aforementioned characteristics are representative "desired" or "predetermined characteristics or phenotypes.

In a preferred embodiment, the method comprises: (a) providing a test NR polypeptide sequence for which the solubility, stability in solution, other solution behavior, tendency to fold properly, ability to form ordered crystals, or combination thereof is different from that desired; (b) aligning the test NR polypeptide sequence with the sequences of other reference NR polypeptides for which the X-ray structure is available and for which the solution properties, folding behavior and crystallization properties are closer to those desired; (c) building a three-dimensional model for the test NR polypeptide using the three-dimensional coordinates of the X-ray structure(s) of one or more of the reference polypeptides and their sequence alignment with the test NR polypetide sequence; (d) optionally, optimizing the side-chain conformations in the three-dimensional model by generating many alternative side-chain conformations, refining by energy minimization, and selecting side-chain conformations with lower energy; (e) examining the three-dimensional model for the test NR graphically for lipophilic side-chains that are exposed to solvent, for clusters of two or more lipophilic side-chains exposed to solvent, for lipophilic pockets and clefts on the surface of the protein model, and in particular for sites on the surface of the protein model that are more lipophilic than the corresponding sites on the structure(s) of the reference NR polypeptide(s); (f) for each residue identified in step (e), mutating the amino acid to an amino acid with different hydrophilicity, and usually to a more hydrophilic amino acid, whereby the exposed lipophilic sites are reduced, and the solution properties improved; (g) examining the three-dimensional model graphically at each site where the amino acid in the test NR polypeptide is different from the amino acid at the corresponding position in the reference NR polypeptide, and checking whether the amino acid in the test NR polypeptide makes favorable interactions with the atoms that lie around it in the three-dimensional model, considering the side-chain conformations predicted in steps (c) and, optionally step (d), as well as likely alternative conformations of the side-chains, and also considering the possible presence of water molecules (for this analysis, an amino acid is considered to make "favorable interactions with the atoms that lie around it" if these interactions are more favorable than the interactions that would be obtained if it was replaced by any of the 19 other naturally-occurring amino acids); (h) for each residue identified in step (g) as not making favorable interactions with the atoms that lie around it, mutating the residue to another amino acid that could make better interactions with the atoms that lie around it, thereby promoting the tendency for the test NR polypeptide to fold into a stable structure with improved solution properties, less tendency to unfold, and greater tendency to form ordered crystals; (i) examining the three-dimensional model graphically at each residue position where the amino acid in the test NR polypeptide is different from the amino acid at the corresponding position in the reference NR polypeptide, and checking whether the steric packing, hydrogen bonding and other energetic interactions could be improved by mutating that residue or any one or more of the surrounding residues lying within 8 angstroms in the three-dimensional model; (j) for each residue position identified in step (i) as potentially allowing an improvement in the packing, hydrogen bonding and energetic interactions, mutating those residues individually or in combination to residues that could improve the packing, hydrogen bonding and energetic interactions, thereby promoting the tendency for the test NR polypeptide to fold into a stable structure with improved solution properties, less tendency to unfold, and greater tendency to form ordered crystals.

By the term "graphically" it is meant through the use of computer aided graphics, such by the use of a software package disclosed herein above. Optionally, in this embodiment, the reference NR polypeptide is AR, or PR, when the test NR polypeptide is GR$\alpha$. Alternatively, the reference NR polypeptide is GR$\alpha$, and the test NR polypeptide is preferably GR$\beta$, AR, PR or MR.

An isolated GR polypeptide comprising a mutation in a ligand binding domain, wherein the mutation alters the solubility of the ligand binding domain, is also disclosed. An isolated GR polypeptide, or functional portion thereof,

having one or more mutations comprising a substitution of a hydrophobic amino acid residue by a hydrophilic amino acid residue in a ligand binding domain is also disclosed. Preferably, in each case, the mutation can be at a residue selected from the group consisting of V552, W557, F602, L636, Y648, W712, L741, L535, V538, C638, M691, V702, Y648, Y660, L685, M691, V702, W712, L733, Y764 and combinations thereof. More preferably, the mutation is selected from the group consisting of V552K, W557S, F602S, F602D, F602E, F602Y, F602T, F602N, F602C, L636E, Y648Q, W712S, L741R, L535T, V538S, C638S, M691T, V702T, W712T and combinations thereof. Even more preferably, the mutation is made by targeted point or randomizing mutagenesis. Hydrophobicity and hyrdrophilicity criteria and comparision information are set forth herein below.

As discussed above, the GRα gene can be translated from its mRNA by alternative initiation from an internal ATG codon (Yudt & Cidlowski, (2001) *Molec. Endocrinol.* 15: 1093-1103). This codon codes for methionine at position 27 and translation from this position produces a slightly smaller protein. These two isoforms, translated from the same gene, are referred to as GR-A and GR-B. It has been shown in a cellular system that the shorter GR-B form is more effective in initiating transcription from a GRE compared to GR-A. Additionally, another form of GR, called GRβ is produced by an alternative splicing event. The GRβ protein differs from GRα at the very C-terminus, where the final 50 amino acids are replaced with a 15 amino acid segment. These two isoforms are 100% identical up to amino acid 727. No sequence similarity exists between GRα and GRβ at the C-terminus beyond position 727. GRβ has been shown to be a dominant negative regulator of GRα-mediated gene transcription (Oakley, et al., (1996) *J. Biol. Chem.* 271: 9550-9559). It has been suggested that some of the tissue specific effects observed with glucocorticoid treatment may in part be due to the presence of varying amounts of isoform in certain cell-types. This method is also applicable to any other subfamily so organized. Thus, while the amino acid residue numbers referenced above pertain to GR-A, the polypeptides of the present invention also have a mutation at an analogous position in any polypeptide based on a sequence alignment (such as prepared by BLAST or other approach disclosed herein or known in the art) to GRα, which are not forth herein for convenience.

As used in the following discussion, the terms "engineered NR", "engineered NR LDB", "NR mutant", and "NR LBD mutant" refers to polypeptides having amino acid sequences that contain at least one mutation in the wild-type sequence, including at an analogous position in any polypeptide based on a sequence alignment to GRα. The terms also refer to NR and NR LBD polypeptides which are capable of exerting a biological effect in that they comprise all or a part of the amino acid sequence of an engineered mutant polypeptide of the present invention, or cross-react with antibodies raised against an engineered mutant polypeptide, or retain all or some or an enhanced degree of the biological activity of the engineered mutant amino acid sequence or protein. Such biological activity can include the binding of small molecules in general, the binding of glucocorticoids in particular and even more particularly the binding of dexamethasone.

The terms "engineered NR LBD" and "NR LBD mutant" also includes analogs of an engineered NR polypeptide or NR LBD mutant polypeptide. By "analog" is intended that a DNA or polypeptide sequence can contain alterations relative to the sequences disclosed herein, yet retain all or some or an enhanced degree of the biological activity of those sequences. Analogs can be derived from genomic nucleotide sequences or from other organisms, or can be created synthetically. Those of skill in the art will appreciate that other analogs, as yet undisclosed or undiscovered, can be used to design and/or construct mutant analogs. There is no need for an engineered mutant polypeptide to comprise all or substantially all of the amino acid sequence of the wild type polypeptide (e.g. SEQ ID NOs: 2, 4, 6 and 8). Shorter or longer sequences are anticipated to be of use in the invention; shorter sequences are herein referred to as "segments". Thus, the terms "engineered NR LBD" and "NR LBD mutant" also includes fusion, chimeric or recombinant engineered NR LBD or NR LBD mutant polypeptides and proteins comprising sequences of the present invention. Methods of preparing such proteins are disclosed herein above.

## X.D. Sequence Similarity and Identity

As used herein, the term "substantially similar" as applied to GR means that a particular sequence varies from nucleic acid sequence of any of SEQ ID NOs: 1, 3, 5, or 7, or the amino acid sequence of any of SEQ ID NOs: 2, 4, 6 or 8 by one or more deletions, substitutions, or additions, the net effect of which is to retain at least some of biological activity of the natural gene, gene product, or sequence. Such sequences include "mutant" or "polymorphic" sequences, or sequences in which the biological activity and/or the physical properties are altered to some degree but retains at least some or an enhanced degree of the original biological activity and/or physical properties. In determining nucleic acid sequences, all subject nucleic acid sequences capable of encoding substantially similar amino acid sequences are considered to be substantially similar to a reference nucleic acid sequence, regardless of differences in codon sequences or substitution of equivalent amino acids to create biologically functional equivalents.

X.D.1. Sequences That are Substantially Identical to an Engineered NR or NR LBD Mutant Sequence of the Present Invention

Nucleic acids that are substantially identical to a nucleic acid sequence of an engineered NR or NR LBD mutant of the present invention, e.g. allelic variants, genetically altered versions of the gene, etc., bind to an engineered NR or NR LBD mutant sequence under stringent hybridization conditions. By using probes, particularly labeled probes of DNA sequences, one can isolate homologous or related genes. The source of homologous genes can be any species, e.g. primate species; rodents, such as rats and mice, canines, felines, bovines, equines, yeast, nematodes, etc.

Between mammalian species, e.g. human and mouse, homologs have substantial sequence similarity, i.e. at least 75% sequence identity between nucleotide sequences. Sequence similarity is calculated based on a reference sequence, which can be a subset of a larger sequence, such as a conserved motif, coding region, flanking region, etc. A reference sequence will usually be at least about 18 nt long, more usually at least about 30 nt long, and can extend to the complete sequence that is being compared. Algorithms for sequence analysis are known in the art, such as BLAST, described in Altschul et al., (1990) *J. Mol. Biol.* 215:403-10. Software for performing BLAST analyses is publicly available through the National Center for Biotechnology Information (http://www.ncbi.nlm.nih.gov/).

This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached. The BLAST algorithm parameters W, T, and X determine the sensitivity and speed of the alignment. The BLASTN program (for nucleotide sequences) uses as defaults a wordlength W=11, an expectation E=10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix. See Henikoff & Henikoff, (1989) *Proc. Natl. Acad. Sci. U.S.A.* 89:10915.

In addition to calculating percent sequence identity, the BLAST algorithm also performs a statistical analysis of the similarity between two sequences. See, e.g., Karlin & Altschul, (1993) *Proc. Natl. Acad. Sci. U.S.A.* 90:5873-5887. One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.

Percent identity or percent similarity of a DNA or peptide sequence can be determined, for example, by comparing sequence information using the GAP computer program, available from the University of Wisconsin Geneticist Computer Group. The GAP program utilizes the alignment method of Needleman et al., (1970) *J. Mol. Biol.* 48:443, as revised by Smith et al., (1981) *Adv. Appl. Math.* 2:482. Briefly, the GAP program defines similarity as the number of aligned symbols (i.e., nucleotides or amino acids) that are similar, divided by the total number of symbols in the shorter of the two sequences. The preferred parameters for the GAP program are the default parameters, which do not impose a penalty for end gaps. See, e.g., Schwartz et al. (eds.), (1979), Atlas of Protein Sequence and Structure, National Biomedical Research Foundation, pp. 357-358, and Gribskov et al., (1986) *Nucl. Acids. Res.* 14:6745.

The term "similarity" is contrasted with the term "identity". Similarity is defined as above; "identity", however, means a nucleic acid or amino acid sequence having the same amino acid at the same relative position in a given family member of a gene family. Homology and similarity are generally viewed as broader terms than the term identity. Biochemically similar amino acids, for example leucine/isoleucine or glutamate/aspartate, can be present at the same position--these are not identical per se, but are biochemically "similar." As disclosed herein, these are referred to as conservative differences or conservative substitutions. This differs from a conservative mutation at the DNA level, which changes the nucleotide sequence without making a change in the encoded amino acid, e.g. TCC to TCA, both of which encode serine.

As used herein, DNA analog sequences are "substantially identical" to specific DNA sequences disclosed herein if: (a) the DNA analog sequence is derived from coding regions of the nucleic acid sequence shown in any one of SEQ ID NOs: 1, 3, 5 or 7 or (b) the DNA analog sequence is capable of hybridization with DNA sequences of (a) under stringent conditions and which encode a biologically active GRα or GRα LBD gene product; or (c) the DNA sequences are degenerate as a result of alternative genetic code to the DNA analog sequences defined in (a) and/or

(b). Substantially identical analog proteins and nucleic acids will have between about 70% and 80%, preferably between about 81% to about 90% or even more preferably between about 91% and 99% sequence identity with the corresponding sequence of the native protein or nucleic acid. Sequences having lesser degrees of identity but comparable biological activity are considered to be equivalents.

As used herein, "stringent conditions" means conditions of high stringency, for example 6X SSC, 0.2% polyvinylpyrrolidone, 0.2% Ficoll, 0.2% bovine serum albumin, 0.1% sodium dodecyl sulfate, 100 μg/ml salmon sperm DNA and 15% formamide at 68°C. For the purposes of specifying additional conditions of high stringency, preferred conditions are salt concentration of about 200 mM and temperature of about 45°C. One example of such stringent conditions is hybridization at 4X SSC, at 65°C, followed by a washing in 0.1XSSC at 65°C for one hour. Another exemplary stringent hybridization scheme uses 50% formamide, 4X SSC at 42°C.

In contrast, nucleic acids having sequence similarity are detected by hybridization under lower stringency conditions. Thus, sequence identity can be determined by hybridization under lower stringency conditions, for example, at 50°C or higher and 0.1X SSC (9 mM NaCl/0.9 mM sodium citrate) and the sequences will remain bound when subjected to washing at 55°C in 1X SSC.

As used herein, the term "complementary sequences" means nucleic acid sequences that are base-paired according to the standard Watson-Crick complementarity rules. The present invention also encompasses the use of nucleotide segments that are complementary to the sequences of the present invention.

Hybridization can also be used for assessing complementary sequences and/or isolating complementary nucleotide sequences. As discussed above, nucleic acid hybridization will be affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringent temperature conditions will generally include temperatures in excess of about 30°C, typically in excess of about 37°C, and preferably in excess of about 45°C. Stringent salt conditions will ordinarily be less than about 1,000 mM, typically less than about 500 mM, and preferably less than about 200 mM. However, the combination of parameters is much more important than the measure of any single parameter. See, e.g., Wetmur & Davidson, (1968) *J. Mol. Biol.* 31:349-70. Determining appropriate hybridization conditions to identify and/or isolate sequences containing high levels of homology is well known in the art. See, e.g., Sambrook et al., (1989) Molecular Cloning: A Laboratory Manual, Cold Spring Harbor, New York.

X.D.2. Functional Equivalents of an Engineered NR, SR or GR or NR, SR, GR LBD Mutant Nucleic Acid Sequence of the Present Invention

As used herein, the term "functionally equivalent codon" is used to refer to codons that encode the same amino acid, such as the ACG and AGU codons for serine. For example, GRα or GRα LBD-encoding nucleic acid sequences comprising any one of SEQ ID NOs: 1, 3, 5 or 7 that have functionally equivalent codons are covered by the present invention. Thus, when referring to the sequence example presented in SEQ ID NOs: 1, 3, 5 or 7, applicants provide substitution of functionally equivalent codons into the sequence example of in SEQ ID NOs: 1, 3, 5 or 7. Thus, applicants are in possession of amino acid and nucleic acids sequences which include such substitutions but which are not set forth herein in their entirety for convenience.

It will also be understood by those of skill in the art that amino acid and nucleic acid sequences can include additional residues, such as additional Nor C-terminal amino acids or 5' or 3' nucleic acid sequences, and yet still be essentially as set forth in one of the sequences disclosed herein, so long as the sequence retains biological protein activity where polypeptide expression is concerned. The addition of terminal sequences particularly applies to nucleic acid sequences which can, for example, include various non-coding sequences flanking either of the 5' or 3' portions of the coding region or can include various internal sequences, i.e., introns, which are known to occur within genes.

X.D.3. Biological Equivalents

The present invention envisions and includes biological equivalents of a engineered NR or NR LBD mutant polypeptide of the present invention. The term "biological equivalent" refers to proteins having amino acid sequences which are substantially identical to the amino acid sequence of an engineered NR LBD mutant of the present invention and which are capable of exerting a biological effect in that they are capable of binding small molecules or cross-reacting with anti-NR or NR LBD mutant antibodies raised against an engineered mutant NR or NR LBD polypeptide of the present invention.

For example, certain amino acids can be substituted for other amino acids in a protein structure without appreciable loss of interactive capacity with, for example, structures in the nucleus of a cell. Since it is the interactive capacity and nature of a protein that defines that protein's biological functional activity, certain amino acid sequence substitutions can be made in a protein sequence (or the nucleic acid sequence encoding it) to obtain a protein with the

same, enhanced, or antagonistic properties. Such properties can be achieved by interaction with the normal targets of the protein, but this need not be the case, and the biological activity of the invention is not limited to a particular mechanism of action. It is thus in accordance with the present invention that various changes can be made in the amino acid sequence of an engineered NR or NR LBD mutant polypeptide of the present invention or its underlying nucleic acid sequence without appreciable loss of biological utility or activity.

Biologically equivalent polypeptides, as used herein, are polypeptides in which certain, but not most or all, of the amino acids can be substituted. Thus, when referring to the sequence examples presented in any of SEQ ID NOs: 1, 3, 5 and 7, applicants envision substitution of codons that encode biologically equivalent amino acids, as described herein, into a sequence example of SEQ ID NOs: 1, 3, 5 and 7, respectively. Thus, applicants are in possession of amino acid and nucleic acids sequences which include such substitutions but which are not set forth herein in their entirety for convenience.

Alternatively, functionally equivalent proteins or peptides can be created via the application of recombinant DNA technology, in which changes in the protein structure can be engineered, based on considerations of the properties of the amino acids being exchanged, e.g. substitution of lie for Leu. Changes designed by man can be introduced through the application of site-directed mutagenesis techniques, e.g., to introduce improvements to the antigenicity of the protein or to test an engineered mutant polypeptide of the present invention in order to modulate lipid-binding or other activity, at the molecular level.

Amino acid substitutions, such as those which might be employed in modifying an engineered mutant polypeptide of the present invention are generally, but not necessarily, based on the relative similarity of the amino acid side-chain substituents, for example, their hydrophobicity, hydrophilicity, charge, size, and the like. An analysis of the size, shape and type of the amino acid side-chain substituents reveals that arginine, lysine and histidine are all positively charged residues; that alanine, glycine and serine are all of similar size; and that phenylalanine, tryptophan and tyrosine all have a generally similar shape. Therefore, based upon these considerations, arginine, lysine and histidine; alanine, glycine and serine; and phenylalanine, tryptophan and tyrosine; are defined herein as biologically functional equivalents. Those of skill in the art will appreciate other biologically functionally equivalent changes. It is implicit in the above discussion, however, that one of skill in the art can appreciate that a radical, rather than a conservative substitution is warranted in a given situation. Non-conservative substitutions in engineered mutant LBD polypeptides of the present invention are also an aspect of the present invention.

In making biologically functional equivalent amino acid substitutions, the hydropathic index of amino acids can be considered. Each amino acid has been assigned a hydropathic index on the basis of their hydrophobicity and charge characteristics, these are: isoleucine (+ 4.5); valine (+ 4.2); leucine (+ 3.8); phenylalanine (+ 2.8); cysteine (+ 2.5); methionine (+ 1.9); alanine (+ 1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is generally understood in the art (Kyte & Doolittle, (1982), *J. Mol. Biol.* 157:105-132, incorporated herein by reference). It is known that certain amino acids can be substituted for other amino acids having a similar hydropathic index or score and still retain a similar biological activity. In making changes based upon the hydropathic index, the substitution of amino acids whose hydropathic indices are within ±2 of the original value is preferred, those which are within ±1 of the original value are particularly preferred, and those within ±0.5 of the original value are even more particularly preferred.

It is also understood in the art that the substitution of like amino acids can be made effectively on the basis of hydrophilicity. U.S. Patent No. 4,554,101, incorporated herein by reference, states that the greatest local average hydrophilicity of a protein, as governed by the hydrophilicity of its adjacent amino acids, correlates with its immunogenicity and antigenicity, i.e. with a biological property of the protein. It is understood that an amino acid can be substituted for another having a similar hydrophilicity value and still obtain a biologically equivalent protein.

As detailed in U.S. Patent No. 4,554,101, the following hydrophilicity values have been assigned to amino acid residues: arginine (+3.0); lysine (+ 3.0); aspartate (+ 3.0±1); glutamate (+ 3.0±1); serine (+ 0.3); asparagine (+ 0.2); glutamine (+ 0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

In making changes based upon similar hydrophilicity values, the substitution of amino acids whose hydrophilicity values are within ±2 of the original value is preferred, those which are within ±1 of the original value are particularly preferred, and those within ±0.5 of the original value are even more particularly preferred.

While discussion has focused on functionally equivalent polypeptides arising from amino acid changes, it will be appreciated that these changes can be effected by alteration of the encoding DNA, taking into consideration also that the genetic code is degenerate and that two or more codons can code for the same amino acid.

Thus, it will also be understood that this invention is not limited to the particular amino acid and nucleic acid sequences of any of SEQ ID NOs: 1-11. Recombinant vectors and isolated DNA segments can therefore variously

include an engineered NR or NR LBD mutant polypeptide-encoding region itself, include coding regions bearing selected alterations or modifications in the basic coding region, or include larger polypeptides which nevertheless comprise an NR or NR LBD mutant polypeptide-encoding regions or can encode biologically functional equivalent proteins or polypeptides which have variant amino acid sequences. Biological activity of an engineered NR or NR LBD mutant polypeptide can be determined, for example, by transcription assays known to those of skill in the art.

The nucleic acid segments of the present invention, regardless of the length of the coding sequence itself, can be combined with other DNA sequences, such as promoters, enhancers, polyadenylation signals, additional restriction enzyme sites, multiple cloning sites, other coding segments, and the like, such that their overall length can vary considerably. It is therefore contemplated that a nucleic acid fragment of almost any length can be employed, with the total length preferably being limited by the ease of preparation and use in the intended recombinant DNA protocol. For example, nucleic acid fragments can be prepared which include a short stretch complementary to a nucleic acid sequence set forth in any of SEQ ID NOs: 1, 3, 5 and 7, such as about 10 nucleotides, and which are up to 10,000 or 5,000 base pairs in length. DNA segments with total lengths of about 4,000, 3,000, 2,000, 1,000, 500, 200, 100, and about 50 base pairs in length are also useful.

The DNA segments of the present invention encompass biologically functional equivalents of engineered NR, or NR LBD mutant polypeptides. Such sequences can rise as a consequence of codon redundancy and functional equivalency that are known to occur naturally within nucleic acid sequences and the proteins thus encoded. Alternatively, functionally equivalent proteins or polypeptides can be created via the application of recombinant DNA technology, in which changes in the protein structure can be engineered, based on considerations of the properties of the amino acids being exchanged. Changes can be introduced through the application of site-directed mutagenesis techniques, e.g., to introduce improvements to the antigenicity of the protein or to test variants of an engineered mutant of the present invention in order to examine the degree of binding activity, or other activity at the molecular level. Various site-directed mutagenesis techniques are known to those of skill in the art and can be employed in the present invention.

The invention further encompasses fusion proteins and peptides wherein an engineered mutant coding region of the present invention is aligned within the same expression unit with other proteins or peptides having desired functions, such as for purification or immunodetection purposes.

Recombinant vectors form important further aspects of the present invention. Particularly useful vectors are those in which the coding portion of the DNA segment is positioned under the control of a promoter. The promoter can be that naturally associated with an NR gene, as can be obtained by isolating the 5' non-coding sequences located upstream of the coding segment or exon, for example, using recombinant cloning and/or PCR technology and/or other methods known in the art, in conjunction with the compositions disclosed herein.

In other embodiments, certain advantages will be gained by positioning the coding DNA segment under the control of a recombinant, or heterologous, promoter. As used herein, a recombinant or heterologous promoter is a promoter that is not normally associated with an NR gene in its natural environment. Such promoters can include promoters isolated from bacterial, viral, eukaryotic, or mammalian cells. Naturally, it will be important to employ a promoter that effectively directs the expression of the DNA segment in the cell type chosen for expression. The use of promoter and cell type combinations for protein expression is generally known to those of skill in the art of molecular biology (see, e.g., Sambrook et al., (1989) Molecular Cloning: A Laboratory Manual, Cold Spring Harbor Laboratory, New York, United States of America, specifically incorporated herein by reference). The promoters employed can be constitutive or inducible and can be used under the appropriate conditions to direct high level expression of the introduced DNA segment, such as is advantageous in the large-scale production of recombinant proteins or peptides. One preferred promoter system contemplated for use in high-level expression is a T7 promoter-based system.

### X.E. Antibodies to an Engineered NR or NR LBD Mutant Polypeptide of the Present Invention

The present invention also provides an antibody that specifically binds a engineered NR or NR LBD mutant polypeptide and methods to generate same. The term "antibody" indicates an immunoglobulin protein, or functional portion thereof, including a polyclonal antibody, a monoclonal antibody, a chimeric antibody, a single chain antibody, Fab fragments, and a Fab expression library. "Functional portion" refers to the part of the protein that binds a molecule of interest. In a preferred embodiment, an antibody of the invention is a monoclonal antibody. Techniques for preparing and characterizing antibodies are well known in the art (see, e.g., Harlow & Lane, (1988) Antibodies: A Laboratory Manual, Cold Spring Harbor Laboratory Press, Cold Spring Harbor, New York, United States of America). A monoclonal antibody of the present invention can be readily prepared through use of well-known techniques such as the hybridoma techniques exemplified in U.S. Patent No 4,196,265 and the phage-displayed techniques disclosed in U.S. Patent No. 5,260,203.

The phrase "specifically (or selectively) binds to an antibody", or "specifically (or selectively) immunoreactive with", when referring to a protein or peptide, refers to a binding reaction which is determinative of the presence of the protein in a heterogeneous population of proteins and other biological materials. Thus, under designated immunoassay

conditions, the specified antibodies bind to a particular protein and do not show significant binding to other proteins present in the sample. Specific binding to an antibody under such conditions can require an antibody that is selected for its specificity for a particular protein. For example, antibodies raised to a protein with an amino acid sequence encoded by any of the nucleic acid sequences of the invention can be selected to obtain antibodies specifically immunoreactive with that protein and not with unrelated proteins.

The use of a molecular cloning approach to generate antibodies, particularly monoclonal antibodies, and more particularly single chain monoclonal antibodies, are also provided. The production of single chain antibodies has been described in the art. See, e.g., U.S. Patent No. 5,260,203. For this approach, combinatorial immunoglobulin phagemid libraries are prepared from RNA isolated from the spleen of the immunized animal, and phagemids expressing appropriate antibodies are selected by panning on endothelial tissue. The advantages of this approach over conventional hybridoma techniques are that approximately $10^4$ times as many antibodies can be produced and screened in a single round, and that new specificities are generated by heavy (H) and light (L) chain combinations in a single chain, which further increases the chance of finding appropriate antibodies. Thus, an antibody of the present invention, or a "derivative" of an antibody of the present invention, pertains to a single polypeptide chain binding molecule which has binding specificity and affinity substantially similar to the binding specificity and affinity of the light and heavy chain aggregate variable region of an antibody described herein.

The term "immunochemical reaction", as used herein, refers to any of a variety of immunoassay formats used to detect antibodies specifically bound to a particular protein, including but not limited to competitive and non-competitive assay systems using techniques such as radioimmunoassays, ELISA (enzyme linked immunosorbent assay), "sandwich" immunoassays, immunoradiometric assays, gel diffusion precipitation reactions, immunodiffusion assays, *in situ* immunoassays (e.g., using colloidal gold, enzyme or radioisotope labels), western blots, precipitation reactions, agglutination assays (e.g., gel agglutination assays, hemagglutination assays), complement fixation assays, immunofluorescence assays, protein A assays, and immunoelectrophoresis assays, etc. See Harlow & Lane, (1988) Antibodies: A Laboratory Manual, Cold Spring Harbor Laboratory Press, Cold Spring Harbor, New York, United States of America, for a description of immunoassay formats and conditions.

## X.F. Method for Detecting an Engineered NR or NR LBD Mutant Polypeptide or an Nucleic Acid Molecule Encoding the Same

In another aspect of the invention, a method is provided for detecting a level of an engineered NR or NR LBD mutant polypeptide using an antibody that specifically recognizes an engineered NR or NR LBD mutant polypeptide, or portion thereof. In a preferred embodiment, biological samples from an experimental subject and a control subject are obtained, and an engineered NR or NR LBD mutant polypeptide is detected in each sample by immunochemical reaction with the antibody. More preferably, the antibody recognizes amino acids of any one of SEQ ID NOs: 2, 4, 6 and 8, and is prepared according to a method of the present invention for producing such an antibody.

In one embodiment, an antibody is used to screen a biological sample for the presence of an engineered NR or NR LBD mutant polypeptide. A biological sample to be screened can be a biological fluid such as extracellular or intracellular fluid, or a cell or tissue extract or homogenate. A biological sample can also be an isolated cell (e.g., in culture) or a collection of cells such as in a tissue sample or histology sample. A tissue sample can be suspended in a liquid medium or fixed onto a solid support such as a microscope slide. In accordance with a screening assay method, a biological sample is exposed to an antibody immunoreactive with an engineered NR or NR LBD mutant polypeptide whose presence is being assayed, and the formation of antibody-polypeptide complexes is detected. Techniques for detecting such antibody-antigen conjugates or complexes are well known in the art and include but are not limited to centrifugation, affinity chromatography and the like, and binding of a labeled secondary antibody to the antibody-candidate receptor complex.

In another aspect of the invention, a method is provided for detecting a nucleic acid molecule that encodes an engineered NR or NR LBD mutant polypeptide. According to the method, a biological sample having nucleic acid material is procured and hybridized under stringent hybridization conditions to an engineered NR or NR LBD mutant polypeptide-encoding nucleic acid molecule of the present invention. Such hybridization enables a nucleic acid molecule of the biological sample and an engineered NR or NR LBD mutant polypeptide encoding-nucleic acid molecule to form a detectable duplex structure. Preferably, the an engineered NR or NR LBD mutant polypeptide encoding-nucleic acid molecule includes some or all nucleotides of any one of SEQ ID NOs: 1, 3, 5 and 7. It is also preferable that the biological sample comprises human nucleic acid material.

## XI. The Role of the Three-Dimensional Structure of the GRα LDB in Solving Additional NR, SR or GR Crystals

Because polypeptides can crystallize in more than one crystal form, the structural coordinates of a GRα LBD, or portions thereof, as provided by the present invention, are particularly useful in solving the structure of other crystal

forms of GRα and the crystalline forms of other NRs, SRs and GRs. The coordinates provided in the present invention can also be used to solve the structure of NR and NR LBD mutants (such as those described in Sections IX and X above), NR LDB co-complexes, or of the crystalline form of any other protein with significant amino acid sequence homology to any functional domain of a NR.

XI.A. Determining the Three-Dimensional Structure of a Polypeptide Using the Three-Dimensional Structure of the GRα LBD as a Template in Molecular Replacement

One method that can be employed for the purpose of solving additional GR crystal structures is molecular replacement. See generally, Rossmann (ed.), (1972) The Molecular Replacement Method, Gordon & Breach, New York, New York, United States of America. In the molecular replacement method, the unknown crystal structure, whether it is another crystal form of a GRα or a GRα LBD, (i.e. a GRα or a GRα LBD mutant), or an NR or an NR LBD polypeptide complexed with another compound (a "co-complex"), or the crystal of some other protein with significant amino acid sequence homology to any functional region of the GRα LBD, can be determined using the GRα LBD structure coordinates provided in Table 2. This method provides an accurate structural form for the unknown crystal more quickly and efficiently than attempting to determine such information *ab initio.*

In addition, in accordance with this invention, NR and NR LBD mutants can be crystallized in complex with known modulators. The crystal structures of a series of such complexes can then be solved by molecular replacement and compared with that of the wild-type NR or the wild-type NR LBD. Potential sites for modification within the various binding sites of the enzyme can thus be identified. This information provides an additional tool for determining the most efficient binding interactions, for example, increased hydrophobic interactions, between the GRα LBD and a chemical entity or compound.

All of the complexes referred to in the present disclosure can be studied using X-ray diffraction techniques (See, e.g., Blundell & Johnson (1985) *Method. Enzymol.,* 114A & 115B, (Wyckoff et al., eds.), Academic Press; McRee, (1993) Practical Protein Crystallography, Academic Press, New York, New York) and can be refined using computer software, such as the X-PLOR™ program (Brünger, (1992) *X-PLOR, Version 3.1. A System for X-ray Crystallography and NMR,* Yale University Press, New Haven, Connecticut; X-PLOR is available from Accelrys of San Diego, California, United States of America) and the XTAL-VIEW program (McRee, (1992) *J. Mol. Graphics* 10:44-46; McRee, (1993) Practical Protein Crystallography, Academic Press, San Diego, California, United States of America). This information can thus be used to optimize known classes of GR and GR LBD modulators, and more importantly, to design and synthesize novel classes of GR and GR LBD modulators.

Laboratory Examples

The following Laboratory Examples have been included to illustrate preferred modes of the invention. Certain aspects of the following Laboratory Examples are described in terms of techniques and procedures found or contemplated by the present inventors to work well in the practice of the invention. These Laboratory Examples are exemplified through the use of standard laboratory practices of the inventors. In light of the present disclosure and the general level of skill in the art, those of skill will appreciate that the following Laboratory Examples are intended to be exemplary only and that numerous changes, modifications and alterations can be employed without departing from the spirit and scope of the invention.

Laboratory Example 1

Expression Of a GRα Polypeptide

BL21(DE3) cells (Novagen/Invitrogen, Inc., Carlsbad, California, United States of America) were transformed with the expression plasmid 6xHisGST-GR(521-777) F602S pET24 following established protocols. Following overnight incubation at 37°C a single colony was used to inoculate a 10 ml LB culture containing 50 μg/ml kanamycin (Sigma, St. Louis, Missouri, United States of America). The culture was grown for ~8 hrs at 30°C and then a 500μl aliquot was used to inoculate flasks containing 1 liter CIRCLE GROW™ media (Bio 101, Inc., Vista, California, United States of America) and the required antibiotic. The cells were then grown at 22°C to an OD600 between 2 and 3 and then cooled to 18°C. Following a 30 min equilibration at that temperature, dexamethasone (Spectrum Chemical Co., Gardena, California, United States of America) (50 or 100 μM final concentration) was added. Induction of expression was achieved by adding IPTG (BACHEM, Philapdelphia, Pennsylvania, United States of America) (final concentration 1 mM) to the cultures. Expression at 18°C was continued for ~20 hrs. Cells were then harvested and frozen at -80°C.

In another example, GR LBD was expressed in the presence of 50 or 100 μM FP. This approach eliminated the step of exchanging dexamethasone with fluticasone propionate during the purification process. The GR LBD/FP

complex that was formed by expressing the GR LBD in the presence of 50 or 100 μM FP also formed crystals.

Laboratory Example 2

Purification Of a GR LBD (521-777) F602S Polypeptide Bound to Fluticasone Propionate

Approximately 37 g of cells were resuspended in 500 mL lysis buffer (50mM Tris pH =8.0, 150 mM NaCl, 2M urea, and 30 μM fluticasone propionate) and lysed by passing 3 times through a Rannie APV Lab 2000 homogenizer (Rannie APV, Copenhagen, Denmark). The lysate was subjected to centrifugation (30 minutes, 20,000g, 4°C). The cleared supernatant was filtered through coarse pre-filters and 50 mM Tris, pH= 8.0, containing 150 mM NaCl and 1M imidazole was added to obtain a final imidazole concentration of 50mM. This lysate was loaded onto a XK-26 column (Pharmacia, Peapack, New Jersey) packed with Sepharose [$Ni^{2+}$ charged] chelation resin (Pharmacia, Peapack, New Jersey) and pre-equilibrated with lysis buffer supplemented with 50mM imidazole. Following loading, the column was washed to baseline absorbance with equilibration buffer. This was followed by a linear (0 to 10%) glycerol and (2M to 0M) urea gradient. For elution the column was developed with a linear gradient from 50 to 500 mM imidazole in 50mM Tris pH =8.0, 150 mM NaCl, 10% glycerol and 30 μM fluticasone proprionate. Column fractions of interest were pooled and 500 units of thrombin protease (Amersham Pharmacia Biotech, Piscataway, New Jersey, United States of America) were added for the cleavage of the fusion protein. This solution was then dialyzed against 1 liter of 50 mM Tris pH = 8.0, 150 mM NaCl, 10% glycerol and 30 μM fluticasone proprionate for ~24 hrs at 4°C. The digested protein sample was filtered and then reloaded onto a fresh (previously equilibrated) $Ni^{++}$ charged column. The cleaved GR LBD was collected in the flow-through fraction. The diluted protein sample was concentrated with CENTRIPREP™ 10K centrifugal filtration devices (Amicon/Millipore, Bedford, Massachusetts, United States of America) to a volume of 45ml and then diluted 5 fold with 50 mM Tris pH=8.0, 10 % glycerol, 10 mM DTT, 0.5 mM EDTA and 30 μM fluticasone proprionate. The sample was then loaded onto a pre-equilibrated XK-26 column (Pharmacia, Peapack, New Jersey, United States of America) packed with Poros HQ resin (PerSeptive Biosystems, Framingham, Massachusetts, United States of America). The cleaved GR LBD was collected in the flowthrough. The NaCl concentration was adjusted to 500mM and the purified protein was concentrated to ~15 mg/ml using the CENTRIPREP™ 10K centrifugal filtration devices and then frozen at -80°C.

Figure 1 is an autoradiogram of a polyacrylamide gel summarizing the isolation of a GR mutant of the present invention. In this figure, Lane 1 contains the insoluble pellet fraction. Lane 2 contains the soluble supernatant fraction. Lane 3 contains pooled eluent fromtheinitial $Ni^{2+}$ column. Lane 4 contains the sample after thrombin digestion. Lane 5 contains the flow through fraction after reload of the $Ni^{2+}$ column. Lane 6 contains the protein after anion exchange. The positions of molecular mass (kDa) markers are indicated on the left side of the figure.

Laboratory Example 3

Preparation of a GR/TIF2/Fluticasone Proprionate (FP) Complex

The GR/TIF2/FP complex was prepared by adding a 1.2-fold excess of a TIF2 peptide containing sequence of KENALLRYLLDKDD (SEQ ID NO: 9) during the buffer exchange step as described below. The above complex was concentrated then diluted 1:1 with a buffer containing 500 mM NH40AC, 50 mMTris, pH 8.0, 10% glycerol, 10 mM dithiothreitol (DTT), 0.5mM EDTA and 0.05% β-octyl-glucoside and concentrated to 1 ml. The complex was diluted 1:9 with the above buffer and slowly concentrated to 7.5 mg/ml in the presence of an additional 1.2 fold excess of a TIF2 peptide (residues 740-753), aliquoted and stored at -80 °C.

Laboratory Example 4

Crystallization and Data Collection

The GR/TIF2/FP crystals were grown at room temperature in hanging drops containing 3.0 μl of the above protein-ligand solutions, and 0.5 μl of well buffer (60mM Bis-Tris-Propane, PH 7.5-8.5, and 1.5-1.7 M magnesium sulfate). Crystals appeared overnight and continuously grew to a size of up to 300 microns within several weeks. Before data collection, crystals were flash frozen in liquid nitrogen.

The GR/TIF2/FP crystals formed in the $P6_1$ space group, with a = b = 127.656 Å, c = 87.725 Å, α = β =90°, and γ =120°. Each asymmetry unit contains two molecules of the GR LBD with 58% of solvent content. Data were collected using a MAR165 CCD detector at the 17BM of the Advanced Photon Source (APS) of Argonne National Laboratory in Chicago, Illinois, United States of America. The observed reflections were reduced, merged and scaled with DENZO and SCALEPACK in the HKL2000 package (Otwinowski et al., (1993) in Proceedings of the CCP4 Study

Weekend: Data Collection and Processing. (Sawyer et al., eds), pp. 56-62, SERC Daresbury Laboratory, England).

Laboratory Example 5

Structure Determination and Refinement

A model of GR/TIF2/FP complex was built based on the crystal structure of a GR/TIF2/dexamethasone complex ("the Dex structure"; coordinates of the Dex structure are presented in Table 3). This model was used in molecular replacement search with the CCP4 AmoRe program (Collaborative Computational Project Number 4, 1994; Navaza, (1994) *Acta. Cryst.* A50:157-163) to determine the initial structure solutions. The calculated phase from the molecular replacement solutions was improved with solvent flattening, histogram matching and the two-fold noncrystallographic averaging as implemented in the CCP4 dm program, and produced a clear map for the GR LBD, the TIF2 peptide and the dexamethasone. Model building proceeded by employing the QUANTA software (Accelrys Inc., San Diego, California, United States of America), and refinement continued by employing the CNX software (Accelrys Inc., San Diego, California, United States of America; Brunger et al., (1998) *Acta. Crystallogr.* D54:905-921) and multiple cycle of manual rebuilding. The statistics of the structure are summarized in Table 1.

Laboratory Example 6

Construction of a Docking Model for the Componund Benzoxazin-1-one Using a GR/FP/TIF2 Structure

The second subunit of the GR structure was selected as the initial crystal structure in which to model the benzoxazin-1-one compound and loaded into the display area of INSIGHTII (Accelrys Inc., San Diego, California, United States of America). As a reference, the crystal structure of the bound FP molecule in that subunit was loaded into the same display area.

Initial coordinates of the benzoxazin-1-one were generated using CONCORD v4.0.4 (Tripos Inc., St. Louis, Missouri, United States of America). Conformers of the initial benzoxazin-1-one geometry were generated using the GROW algorithm available in MVP and optimized using CVFF as implemented in MVP (Lambert, (1997) in Practical Application of Computer-Aided Drug Design (Charifson, ed.), Marcel Dekker, New York, New York, United States of America, pp. 243-303). Each of the resulting conformers were then hand-docked into the GR crystal structure and the best-fitting conformer was selected as the proposed binding conformation of the benzoxazin-1-one.

The initial GR/benzoxazin-1-one docking model complex was exported from the INSIGHTII software in the identical coordinate reference frame as the GR/FP crystal structure. Geometry optimization of the GR/benzoxazin-1-one complex was carried out using CVFF as implemented in MVP. All atoms in the complex remained fixed in space except for those atoms contained in the benzoxazin-1-one and the initial GR structure that were within 6 angstroms of any atom in the benzoxazin-1-one. The CVFF energy terms were calculated using only those atoms within 16 angstroms of (and including) the benzoxazin-1-one. Geometry optimization of the protein-ligand complex was carried out using the conjugate gradient method as implemented in MVP and with a convergence criteria of a 0.1 change in the gradient.

Figure 9 depicts a docking model of a GR LBD with the benzoxazine-1-one ligand generated as described hereinabove. Figure 10 depicts various interactions formed between the benzoxazin-1-one ligand and GR residues that comprising the binding pocket. Intermolecular distances are indicated in the figure. Figure 11 depicts the docking of the benzoxazin-1-one ligand with the GR binding pocket. The docking model comprises an expanded binding pocket, which, as Figure 11 shows, accommodates the *p*-fluorophenoilc side chain of the ligand.

Figure 12 a depiction of the overlay of the GR/Dex crystal structure (grey) with the GR/benzoxazin-1-one model (white) comparing the geometries of the ligands and the relative locations of the amino acid side chains that compose the GR expanded binding pocket. Conformational differences between four residues (M560, M639, W642, and W735) allow for the additional volume of the expanded binding pocket. This added volume provides additional space in the binding pocket and allows the large *p*-fluorophenol group of the Schering compounds to extend beyond the dexamethasone D-ring and into this region. This added volume is observed in the GR/benzoxazin-1-one model but is not observed in the GR/Dex structure.

Table 6 presents a subset of atomic coordinates of GRα in complex with benzoxazin-1-one obtained from modeling of the crystal structure of GRα in complex with FP.

Laboratory Example 7

Construction of an AR Homology Model Bound with Bicalutamide Using a GR/FP/TIF2 Structure

A preferred method of constructing an NR homology model using a GR/TIF2/FP structure of the present

invention is disclosed. This method is illustrated by way of specific example, namely the construction of an AR homology model. Those of ordinary skill in the art will appreciate that although the method is presented in the context of generating an AR homology model, the method can be employed *mutatis mutandis* to generate homology models for all NRs.

In the formulation of an AR homology model based on the GR/TIF2/FP structure of the present invention, sequence alignments of the AR and GR LBDs were initially obtained using the alignment algorithm implemented in MVP (Lambert, (1997) in Practical Application of Computer-Aided Drug Design (Charifson, ed.), Marcel Dekker, New York, New York, United States of America, pp. 243-303). After three-dimensional alignment and coordinate translation of the GR/TIF2/FP crystal structure into a standard orientation using MVP, the second subunit of the GR/TIF2/FP structure was chosen for the AR homology model. Throughout the building the homology model, the Homology package in the INSIGHTII program (Accelrys Inc., San Diego, California, United States of America) was used to visualize the proteins, extract the LBD sequences, manually align the sequences, transform the amino acid residues, manually manipulate the amino acid sidechain conformers, and export the three-dimensional coordinates in appropriate file formats.

The second subunit of the GR/TIF2/FP structure was loaded into the display area of INSIGHTII along with the AR/DHT structure for comparison purposes. Using the Homology package, the GR/TIF2/FP and AR/DHT primary amino acid sequences were extracted from the crystal structures. The sequences were then manually aligned using Homology and by comparison with those alignments obtained using the MVP program.

The transformation of the amino acid residues was carried out and initial three-dimensional coordinates of the AR homology model were assigned using the AssignCoods method in the Homology modeling package. In assigning the coordinates of residues I672-K883 in the AR model, the corresponding coordinates of residues T531-D742 in the GR/TIF2/FP crystal structure were used. In assigning the coordinates of residues M886-H917 in the AR model, the corresponding coordinates of residues K744-H775 in the GR/TIF2/FP crystal structure were used. For the coordinates of residues S884-H885 in the AR model, the corresponding coordinates from the AR/DHT crystal structure were used. Manual modifications of amino acid side chain conformers were carried out after comparing the conformations of corresponding residues in the initial AR homology model and the AR/DHT crystal structure. The conformations of the following AR model residues were modified based on these comparisons: L880, M895, F697, K777, T877, and Q711.

Initial coordinates of bicalutamide were generated using CONCORD v4.0.4 (Tripos Inc., St. Louis, Missouri, United States of America). Conformers of the initial bicalutamide geometry were generated using the GROW algorithm available in MVP and optimized using CVFF as implemented in MVP. Each of the resulting conformers were then hand-docked into the initial AR homology model, and the best-fitting conformer was selected as the proposed binding conformation of bicalutamide.

The initial AR/bicalutamide homology model complex was exported from INSIGHTII in the identical coordinate reference frame as the GR/TIF2/FP crystal structure. Using MVP and the sequence alignments of GR and AR, the residue numbering of the initial AR model was corrected.

Geometry optimization of the AR/bicalutamide homology model complex was carried out using CVFF as implemented in MVP. All atoms in the complex remained fixed in space except for those atoms contained in bicalutamide and the initial AR model that were within 6 angstroms of any atom in bicalutamide. The CVFF energy terms were calculated using only those atoms within 16 angstroms of (and including) bicalutamide. Geometry optimization of the protein-ligand complex was carried out using the conjugate gradient method as implemented in MVP and with a convergence criteria of a 0.1 change in the gradient.

Figure 18A is a ribbon diagram that depicts an AR homology model formed using the GR/TIF2/FP structure of the present invention and the method disclosed hereinabove. The homology model comprises an expanded binding pocket similar to that observed in the GR/TIF2/FP structure of the present invention. The binding pocket is represented as a solid surface. By way of comparison, Figure 18B depicts a known AR/DHT LBD structure. This structure lacks an expanded binding pocket and cannot accommodate a bicalutamide ligand.

Figure 19 depicts a docking model of an AR LBD with the bicalutamide ligand generated as described hereinabove. The AF2, H3, H9 aned H10 helices are labeled. Figure 20 depicts an orthogonal view of the structure depicted in Figure 19 and shows the orientation of the ligand in the binding pocket of AR. Figure 21, which is a stick diagram, depicts various interactions formed between the bicalutamide ligand and AR residues that comprising the binding pocket. Intermolecular distances are indicated in the figure. Figure 21 depicts the docking of the benzoxazin-1-one ligand with the AR binding pocket. Figure 22 is a ribbon diagram that shows the extension of the p-fluorophenyl group of the bicalutamide ligand into the expanded binding pocket formed in the AR-bicalutamide model.

Table 4 presents the atomic coordinates of AR in complex with bicalutamide obtained from homology modeling of the crystal structure coordinates of GR$\alpha$ in complex with FP.

Laboratory Example 8

Construction of a PR Homology Model Bound with RWJ-60130 Using a GR/TF2/FP Crystal Structure

As noted, a GR/TIF2/FP structure of the present invention can be employed to construct a homology model of an NR. In the following section, a preferred method is presented by way of specific example, namely the construction of a PR homology model. In the following example, although PR is specifically recited, any NR can be employed and the following discussion is intended to illustrate one embodiment of this general method.

First, sequence alignments of the PR and GR LBDs were obtained using the alignment algorithm implemented in MVP. After three-dimensional alignment and coordinate translation of the GR/TIF2/FP crystal structure into a standard orientation using MVP, the second subunit of the GR/TIF2/FP structure was chosen for the PR homology modeling exercise.

The second subunit of the GR/TIF2/FP structure was loaded into the display area of INSIGHTII along with the PR/PG structure for comparison purposes. Using the Homology package, the GR/TIF2/FP and PR/PG primary amino acid sequences were extracted from the crystal structures. The sequences were then manually aligned using Homology and by comparison with those alignments obtained using the MVP program.

The transformation of the amino acid residues was carried out and initial three-dimensional coordinates of the PR homology model were assigned using the AssignCoods method in the Homology modeling package. In assigning the coordinates of residues Q682-Q897 and A900-K932 in the PR model, the corresponding coordinates of residues Q527-D742 and T744-Q776 in the GR/TIF2/FP crystal structure, respectively, were used. For the coordinates of residues S898-R899 in the PR model, the corresponding coordinates from the PR/PG crystal structure were used. Manual modifications of amino acid side chain conformers were carried out after comparing the conformations of corresponding residues in the initial PR homology model and the PR/PG crystal structure. The conformations of the following PR model residues were modified based on these comparisons: L799, W802, V823, N828, M909, L726, R740, S757, M759, and V760.

Initial coordinates of RWJ-60130 were generated using CONCORD v4.0.4. Conformers of the initial RWJ-60130 geometry were generated using the GROW algorithm available in MVP and optimized using CVFF as implemented in MVP. Each of the resulting conformers were then hand-docked into the initial PR homology model and the best-fitting conformer was selected as the proposed binding conformation of RWJ-60130.

The initial PR/RWJ-60130 homology model complex was exported from INSIGHTII in the identical coordinate reference frame as the GR/TIF2/FP crystal structure. Using MVP and the sequence alignments of GR and PR, the residue numbering of the initial PR model was corrected.

Geometry optimization of the PR/RWJ-60130 homology model complex was carried out using CVFF as implemented in MVP. All atoms in the complex remained fixed in space except for those atoms contained in RWJ-60130 and the initial PR model that were within 6 angstroms of any atom in RWJ-60130. The CVFF energy terms were calculated using only those atoms within 16 angstroms of (and including) RWJ-60130. Geometry optimization of the protein-ligand complex was carried out using the conjugate gradient method as implemented in MVP and with a convergence criteria of a 0.1 change in the gradient.

Figure 23A is a ribbon diagram depicting a PR LBD homology model formed using the method disclosed hereinabove and incorporating a GR/TIF2/FP structure of the present invention. The ligand binding pocket is depicted as a solid surface and comprises an expanded binding pocket, as seen in the GR/TIF2/FP structures of the present invention. On the other hand, Figure 23B depicts a known PR LBD structure, shown with the ligand progesterone positioned in the binding pocket. The PR/PG structure does not comprise an expanded binding pocket and cannot accommodate the ligand RWJ-60130.

Figure 24 is a ribbon diagram docking model depicting the association of the ligand RWJ-60130 with an AR LBD comprising an expanded binding pocket. The AR was modeled based on the GR/TIF2/FP structure of the present invention. Figure 25 is an orthogonal view of the structure depicted in Figure 24. Continuing, Figure 26 is a stick model of the interactions the RWJ-60130 ligand forms with the binding pocket of AR. Intermolecular distances are indicated. Figure 27 is an orthogonal view of the structure depicted in Figure 25. Figure 27 shows the extension of the p-fidodophenyl group of the RWJ-60130 ligand into the expanded binding pocket of the AR model. As noted, known AR models and structures that lack the expanded binding pocket cannot fully accommodate the RWJ-60130 ligand.

Table 5 presents atomic coordinates of PR in complex with RWJ-60130 obtained from homology modeling of the crystal structure coordinates of GR$\alpha$ in complex with FP.

Laboratory Example 9

Construction of a Binding Model for A-222977 Using the GR/TIF2/FP Crystal Structure

The second subunit of the GR structure was selected as the initial crystal structure in which to model A-222977 and loaded into the display area of INSIGHTII. As a reference, the crystal structure of the bound FP molecule in that subunit was loaded into the same display area.

Initial coordinates of A-222977 were generated using CONCORD v4.0.4. Conformers of the initial geometry were generated using the GROW algorithm available in MVP and optimized using CVFF as implemented in MVP. Each of the resulting conformers were then hand-docked into the GR crystal structure and the best-fitting conformer was selected as the proposed binding conformation of A-222977.

The initial GR/A-222977 model complex was exported from INSIGHTII in the identical coordinate reference frame as the GR/TIF2/FP crystal structure. Geometry optimization of the GR/A-222977 complex was carried out using CVFF as implemented in MVP. All atoms in the complex remained fixed in space except for those atoms contained in A-222977 and the initial GR structure that were within 6 angstroms of any atom in A-222977. The CVFF energy terms were calculated using only those atoms within 16 angstroms of (and including) A-222977. Geometry optimization of the protein-ligand complex was carried out using the conjugate gradient method as implemented in MVP and with a convergence criteria of a 0.1 change in the gradient.

Figure 13 is a docking model of the ligand A-222977 bound to GR. The GR is the GR/TIF2/FP structure that forms an aspect of the present invention. The model depicted in Figure 13 comprises the expanded binding pocket observed in the GR/TIF2/FP structure. Figure 15 is an orthogonal view of the structure of Figure 13. Figure 15 shows the extension of the methyl-sulfonyl-methoxyl-phenyl side chain of the A-222977 ligand into the expanded binding pocket formed in the GR structure. It is not possible to accurately dock the A-222977 ligand into the GR structure without the presence of the expanded binding pocket, due to the protrusion of the methyl-sulfonyl-methoxyl-phenyl side chain beyond the bounds of the binding pocket. Figure 14 is a stick drawing that depicts the interaction between the residues of the ligand binding pocket of GR, which comprises the expanded binding pocket, and the A-222977 ligand.

Figure 16 is an overlay of the GR/Dex structure with the GR/A-222977 structure. The ligands are represented as stick structures. Figure 16 illustrates several conformational differences between four residues (M560, M639, W642, and W735) contribute to the additional volume of the expanded binding pocket. The added volume encompassed by the expanded binding pocket provides additional space that allows the large methyl-sulfonyl-methoxyl-phenyl group of the A-222977 ligand to extend beyond the dexamethasone D-ring and into this region. Although this space is observed in the GR/A-222977 structure, it is not observed in the GR/Dex structure.

Table 7 presents a subset of atomic coordinates of GR$\alpha$ in complex with A-222977 obtained from modeling of the crystal structure of GR$\alpha$ in complex with FP.

Laboratory Example 11

Construction of a Homology Model for MR Using a GR/TIF2/FP Structure

A model for the human MR LBD was built with the program MVP using the amino acid sequences of human MR (Genbank entry M16801.1), human GR (Genbank entry X03225.1), human PR (Genbank entry X51730.1) and human AR (SwissProt entry ANDR_HUMAN), together with the X-ray structures of GR bound to FP (Table 2) and PR bound to progesterone (Williams & Sigler, PDB entry 1A28). The MVP program was first used to align the amino acid sequences. This alignment, Figure 17, has a single gap, occurring in the GR sequence between GR Asp742 and Lys743, at a position corresponding to MR Ser949, PR Ser898 and AR Ser884. This gap lies in the loop between helix-10 and the AF2 helix. The alignment establishes a corresponding template residue in GR for each residue in the MR LBD except for MR Ser949, which lies in the single gap position. The A subunit of the GR/TIF2/FP complex, Table 2, as was selected as the primary template for the MR model. This structure provides coordinates for GR residues 523-777. Using the residue correspondence from the sequence alignment, the MVP program generated coordinates for the backbone atoms of MR residues 729-948 and 950-984 by copying the corresponding coordinates in GR. The MVP program also copies coordinates for side-chain atoms in MR residues when the side-chain is identical to the corresponding residue in GR. Side-chains that differ from the corresponding side-chains in GR are built using standard bond lengths, angles and dihedral angles, but are built to adopt a confomation similar to that in GR when possible. Initially, no coordinates were generated for Ser949. Energy calculations were used to refine the side-chain conformations. The FP ligand was included in the energy calculations to prevent protein side-chains from moving into the volume normally occupied by the ligand. The protein and ligand were protonated as expected at pH 7, and modeled with the CFF91 force field, as implemented in MVP. A grow calculation was used to generate alternative, low energy conformations for

the side-chains lying within 10Å of the FP ligand. No energy refinement was applied to side-chains lying more than 10Å from the FP ligand. The grow calculation used repeated cycles of torsional coordinate miminization on partially grown side-chain arrangements, followed by cartesion coordinate minimization to an RMS gradient of 0.3 kcal/Å$^2$. Backbone atoms, and side-chains that are identical in MR and GR, were held fixed during the energy calculatons. After energy refinement of the side-chains in and around the ligand binding pocket, the helix-10/AF2 loop from PR was transplanted into the MR model. This transplant model was built by first superimposing the PR structure onto the GR and MR structures, replacing MR residues 945-950 with PR residues 894-904, renumbering these residues according to the MR numbering scheme, and mutating Ile947 to Arg, Gln948 to Glu, Arg950 to His and Ser953 to Lys. The entire model was then examined graphically within Insight-II. Side-chain conformations were adjusted graphically as necessary to avoid overlaps. Table 11 presents the three-dimensional coordinates for the MR homology model.

References

The references listed below as well as all references cited in the specification are incorporated herein by reference to the extent that they supplement, explain, provide a background for or teach methodology, techniques and/or compositions employed herein.


Altschul et al., (1990) *J. Mol. Biol.* 215: 403-10
Apriletti et al., (1995) *Protein Expres. Purif.* 6: 368-370
Ausubel et al., (1989) Current Protocols in Molecular Biology, Greene Publishing Associates and Wiley Interscience, New York
Bartlett et al., (1989) *Special Pub., Royal Chem. Soc.* 78: 182-96
Beato, (1989) *Cell* 56:335-344
Blundell & Johnson, (1985) *Method. Enzymol.* 114A & 115B, (Wyckoff et al., eds.), Academic Press
Bohen, (1995) *J. Biol. Chem.* 270: 29433-29438
Bohen, (1998) *Mol. Cell. Biol.* 18: 3330-3339
Bohm, (1992) *J. Comput. Aid. Mol.* Des. 6: 61-78
Brooks et al., (1983) *J. Comp. Chem.*, 8: 132
Brünger, (1992) *X-PLOR, Version 3.1. A System for X-ray Crystallography and NMR,* Yale University Press, New Haven, Connecticut
Caamano et al., (1994) *Annal. NY Acad. Sci.* 746: 68-77
Case et al., (1997), AMBER 5, University of California, San Francisco, California, United States of America
Cohen & Duke, (1984) *J. Immunol.* 152: 38-42
Cohen et al., (1990) *J. Meet. Chem.* 33: 883-94
Creighton, (1983) Proteins: Structures and Molecular Principles, W.H. Freeman & Co., New York, United States of America
Danielsen et al., (1987) *Molec. Endocrinol.* 1: 816-822
Danielsen et al., (1989) *Cancer Res.* 49: 2286s-2291s
DeBosscher et al., (2000) *Proc. Natl. Acad. Sci. U. S. A.* 97: 3919-3924
Drewes et al., (1996) *Mol. Cell. Biol.* 16:925-31
Ducruix & Geige, (1992) Crystallization of Nucleic Acids and Proteins: A Practical Approach, IRL Press, Oxford, England
Dyda et al., (1994) *Science* 266:1981-6
Eastman-Reks & Vedeckis, (1986) *Cancer Res.* 46: 2457-2462
Eisen et al., (1994). *Proteins* 19:199-221
Evans, (1989) in Recent Progress in Hormone Research (Clark, ed.) Vol. 45, pp. 1-27, Academic Press, San Diego, California, United States of America
Evans, (1988) *Science* 240:889-895
Freeman et al., (2000) *Genes Dev.* 14: 422-434
Gampe et al., (2000) *Mol. Cell* 5: 545-55
Garabedian & Yamamoto, (1992) *Mol. Biol. Cell 3: 1245-1257*
Giguere et al., (1986) *Cell 46:* 645-652
Godowski et al., (1987) *Nature* 325: 365-368
Goodford, (1985) *J. Med. Chem.* 28: 849-57
Goodsell & Olsen, (1990) *Proteins* 8: 195-202
Green & Chambon, (1987) *Nature* 325: 75-78
Gribskov et al., (1986) *Nucl. Acids. Res.* 14: 6745
Gruol et al., (1989) *Molec. Endocrinol.* 3: 2119-2127

Harlow & Lane, (1988) Antibodies: A Laboratory Manual, Cold Spring Harbor Laboratory Press, Cold Spring Harbor, New York, United States of America
Harmon et al., (1979) *J. Cell Physiol.* 98: 267-278
Hauptman, (1997) *Curr. Opin. Struct. Biol.* 7: 672-80
Henikoff & Henikoff, (1989) *Proc. Natl. Acad. Sci. U.S.A.* 89: 10915
Hollenberg & Evans, (1988) *Cell* 55: 899-906
Hollenberg et al., (1987) *Cell* 49: 39-46
Hollenberg et al., (1989) *Cancer* Res. 49: 2292s-2294s
Homo-Delarche, (1984) *Cancer Res.* 44: 431-437
Janknecht, (1991) *Proc. Natl. Acad. Sci. U.S.A.* 88: 8972-8976
Jenkins et al., (2001) *Trends Endocrinol. Metab.* 12: 122-126
Karlin & Altschul, (1993) *Proc. Natl. Acad. Sci. U.S.A.* 90: 5873-5887
Kelso & Munck, (1984) *J. Immunol.* 133:784-791
Kralli et al., (1995) *Proc. Natl. Acad. Sci.* 92: 4701-4705
Kuntz et al., (1992) *J. Mol. Biol.* 161: 269-88
Kyte & Doolittle, (1982), *J. Mol. Biol.* 157: 105-132
Lambert, (1997) in Practical Application of Computer-Aided Drug Design, (Charifson, ed.) Marcel-Dekker, New York, New York, United States of America, pp. 243-303
Lattman, (1985) *Method Enzymol.,* 115: 55-77
Martin, (1992) *J. Med. Chem.* 35: 2145-54
Matias et al., (2000) *J. Biol. Chem.* 275:26164-26171
McConkey et al., (1989) *Arch. Biochem. Biophys.* 269: 365-370
McPherson, (1982) Preparation and Analysis of Protein Crystals, John Wiley, New York
McPherson, (1990) *Eur. J. Biochem.* 189:1-23
McRee, (1992) *J. Mol. Graphics* 10: 44-46
McRee, (1993) Practical Protein Crystallography, Academic Press, San Diego, California, United States of America
Miesfeld et al., (1987) *Science* 236:423-427
Miranker & Karplus, (1991) *Proteins* 11: 29-34
Navia & Murcko, (1992) *Curr. Opin. Struc. Biol.* 2: 202-10
Needleman et al., (1970) *J. Mol. Biol.* 48: 443
Nicholls et al., (1991) *Proteins* 11: 281
Nimmagadda et al., (1998) *Ann. Allerg. Asthma Im.* 81:35-40
Nishibata & Itai, (1991) *Tetrahedron* 47: 8985
Nolte et al., (1998) *Nature* 395:137-43
Oakley et al., (1996) *J. Biol. Chem.* 271: 9550-9559
Oberfield et al., (1999) *Proc. Natl. Acad. Sci. U. S. A.* 96(11):6102-6
Ohara-Nemoto et al., (1990) *J. Steroid Biochem. Molec. Biol.* 37: 481-490
Oro et al., (1988) *Cell* 55: 1109-1114
Palmer et al., (2001) *J. Steroid. Biochem. Mol. Biol.* 75:33-42
Parks et al., (1999) *Science* 284: 1365-1368
Pearlman et al., (1995) *Comput. Phys. Commun.* 91: 1-41
Picard & Yamamoto, (1987) *EMBO J.* 6: 3333-3340
Picard et al., (1990) *Cell Regul.* 1: 291-299
Rajapandi et al., (2000) *J. Biol. Chem.* 275: 22597-22604
Rarey et al., (1996) *J. Comput. Aid. Mol. Des.* 10:41-54
Rossmann (ed.), (1972) The Molecular Replacement Method, Gordon & Breach, New York, New York, United States of America
Sack et al., (2001) *Proc. Natl. Acad Sci.* 98:4904-4909
Sambrook et al., (1989) Molecular Cloning: A Laboratory Manual, Cold Spring Harbor Laboratory, New York, United States of America
Schwartz et al. (eds.), (1979), Atlas of Protein Sequence and Structure, National Biomedical Research Foundation, pp. 357-358
Seielstad et al., (1995) *Mol. Endocrinol.* 9: 647-658
Sheldrick, (1990) *Acta Cryst.* A 46: 467
Shiau et al., (1998) *Cell* 95: 927-37
Sladek et al., *Genes Dev.* 4:2353-65
Smith et al., (1981) *Adv. Appl. Math.* 2:482
Thompson, (1989) *Cancer Res.* 49: 2259s-2265s

Tucker et al., (1988) J. Med. Chem. 31:954
Umesono & Evans, (1989) *Cell* 57: 1139-1146
Van Holde, (1971) Physical Biochemistry, Prentice-Hall, New Jersey, pp. 221-39
Voegel et al., (1998) *EMBO J.* 17: 507-519
Weber, (1991) *Adv. Protein Chem.* 41:1-36
Weeks et al., (1993) *Acta Cryst*. D 49: 179
Wellner, (1971) *Anal. Chem.* 43: 597
Wetmur & Davidson, (1968) *J. Mol. Biol.* 31: 349-70
Willams & Sigler, (1998) *Nature* 393:392-396
Xu et al., (1998) *J. Biol. Chem.* 273: 13918-13924
Yamamoto, (1985) *Ann. Rev. Genet.* 19: 209-252
Yudt & Cidlowski, (2001) *Molec. Endocrinol.* 15: 1093-1103
Yuh & Thompson, (1989) *J. Biol. Chem.* 264: 10904-10910
Zhang et al., (1997) *Nature* 387:206-9
Zhou et al., (1998) *Mol. Endocrinol.* 12: 1594-1604
U.S. Patent No. 4,196,265
U.S. Patent No. 4,554,101
U.S. Patent No. 5,260,203
U.S. Patent No. 5,463,564
U.S. Patent No. 5,684,151
U.S. Patent No. 5,834,228
U.S. Patent No. 5,872,011
U.S. Patent No. 6,008,033
U.S. Patent No. 6,236,946
WO 02/10143
WO 84/03564

TABLE 1

| STATISTICS OF CRYSTALLOGRAPHIC DATA AND STRUCTURE | |
|---|---|
| Crystals | GR/TIF2 in complex with Fluticasone Proprionate |
| Space Group | $P6_1$ |
| Resolution (Å) | 20.0- 2.5 |
| Unique Reflections ( N ) | 28,224 |
| Completeness (%) | 99.7 |
| I/σ | 26.8 |
| $R_{sym}{}^a$ (%) | 8.5 |
| Refinement Statistics: | |
| Resolution (Å) | 10.0-2.6 |
| R factor[b] (%) | 24.47 |
| R free (%) | 27.49 |
| R.M.S.D. | |
| Bond Lengths (Å) | 0.016 |
| R.M.S.D. Bond | |
| Angles(degrees) | 2.34 |
| Number of $H_2O$ | 145 |
| Total Non-hydrogen Atoms | 4589 |

R.M.S.D. is the root mean square deviation from ideal geometry.
[a] $R_{sym}=\Sigma \mid Iavg - Ii \mid \Sigma \mid i$
[b] $R_{factor}= \mid F_P - F_{pcalc} \mid / \sum Fp$,
where $F_p$ and $F_{pcalc}$ are observed and calculated structure factors, $R_{free}$ is calculated from a randomly chosen 8% of reflections that never be used in refinement and $R_{factor}$ is calculated for the remaining 92% of reflections.

TABLE 2

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1 | CB | ALA | 523 | 65.188 | 9.713 | -17.288 | 1.00 | 138.77 | A |
| 2 | C | ALA | 523 | 63.798 | 11.629 | -16.450 | 1.00 | 138.81 | A |
| 3 | O | ALA | 523 | 63.723 | 12.079 | -15.303 | 1.00 | 139.25 | A |
| 4 | N | ALA | 523 | 66.269 | 11.624 | -16.161 | 1.00 | 138.92 | A |
| 5 | CA | ALA | 523 | 65.144 | 11.215 | -17.046 | 1.00 | 138.73 | A |
| 6 | N | THR | 524 | 62.743 | 11.446 | -17.237 | 1.00 | 138.36 | A |
| 7 | CA | THR | 524 | 61.387 | 11.854 | -16.883 | 1.00 | 137.51 | A |
| 8 | CB | THR | 524 | 60.369 | 11.258 | -17.876 | 1.00 | 137.43 | A |
| 9 | OG1 | THR | 524 | 60.906 | 11.379 | -19.190 | 1.00 | 137.90 | A |
| 10 | CG2 | THR | 524 | 59.044 | 12.033 | -17.854 | 1.00 | 137.31 | A |
| 11 | C | THR | 524 | 60.839 | 11.661 | -15.489 | 1.00 | 136.92 | A |
| 12 | O | THR | 524 | 61.408 | 11.005 | -14.628 | 1.00 | 137.02 | A |
| 13 | N | LEU | 525 | 59.703 | 12.304 | -15.311 | 1.00 | 136.09 | A |
| 14 | CA | LEU | 525 | 58.954 | 12.290 | -14.094 | 1.00 | 134.94 | A |
| 15 | CB | LEU | 525 | 59.864 | 12.306 | -12.838 | 1.00 | 134.72 | A |
| 16 | CG | LEU | 525 | 60.146 | 11.153 | -11.810 | 1.00 | 134.77 | A |
| 17 | CD1 | LEU | 525 | 58.986 | 10.507 | -11.287 | 1.00 | 134.77 | A |
| 18 | CD2 | LEU | 525 | 60.920 | 10.054 | -12.398 | 1.00 | 134.85 | A |
| 19 | C | LEU | 525 | 58.023 | 13.514 | -14.176 | 1.00 | 133.93 | A |
| 20 | O | LEU | 525 | 58.206 | 14.401 | -15.010 | 1.00 | 134.00 | A |
| 21 | N | PRO | 526 | 57.023 | 13.566 | -13.292 | 1.00 | 132.74 | A |
| 22 | CD | PRO | 526 | 55.931 | 14.501 | -13.003 | 1.00 | 132.69 | A |
| 23 | CA | PRO | 526 | 57.030 | 12.420 | -12.404 | 1.00 | 131.51 | A |
| 24 | CB | PRO | 526 | 55.930 | 12.705 | -11.385 | 1.00 | 131.91 | A |
| 25 | CG | PRO | 526 | 55.174 | 13.816 | -11.901 | 1.00 | 132.44 | A |
| 26 | C | PRO | 526 | 57.009 | 10.986 | -12.877 | 1.00 | 130.00 | A |
| 27 | O | PRO | 526 | 57.248 | 10.597 | -14.033 | 1.00 | 129.99 | A |
| 28 | N | GLN | 527 | 56.831 | 10.220 | -11.829 | 1.00 | 128.09 | A |
| 29 | CA | GLN | 527 | 56.837 | 8.807 | -11.797 | 1.00 | 125.23 | A |
| 30 | CB | GLN | 527 | 55.643 | 8.376 | -10.987 | 1.00 | 125.24 | A |
| 31 | CG | GLN | 527 | 55.443 | 9.354 | -9.824 | 1.00 | 125.04 | A |
| 32 | CD | GLN | 527 | 56.752 | 9.725 | -9.106 | 1.00 | 124.76 | A |
| 33 | OE1 | GLN | 527 | 57.722 | 8.964 | -9.117 | 1.00 | 124.80 | A |
| 34 | NE2 | GLN | 527 | 56.770 | 10.892 | -8.461 | 1.00 | 124.17 | A |
| 35 | C | GLN | 527 | 56.986 | 8.041 | -13.057 | 1.00 | 122.94 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 36 | O | GLN | 527 | 56.017 | 7.593 | -13.642 | 1.00 | 123.03 | A |
| 37 | N | LEU | 528 | 58.213 | 7.963 | -13.535 | 1.00 | 119.79 | A |
| 38 | CA | LEU | 528 | 58.407 | 7.079 | -14.638 | 1.00 | 116.21 | A |
| 39 | CB | LEU | 528 | 59.246 | 7.658 | -15.761 | 1.00 | 116.64 | A |
| 40 | CG | LEU | 528 | 58.345 | 7.394 | -16.977 | 1.00 | 116.96 | A |
| 41 | CD1 | LEU | 528 | 59.130 | 7.575 | -18.243 | 1.00 | 117.15 | A |
| 42 | CD2 | LEU | 528 | 57.763 | 5.970 | -16.919 | 1.00 | 116.94 | A |
| 43 | C | LEU | 528 | 59.161 | 6.052 | -13.836 | 1.00 | 113.51 | A |
| 44 | O | LEU | 528 | 59.794 | 5.149 | -14.363 | 1.00 | 113.46 | A |
| 45 | N | THR | 529 | 59.050 | 6.229 | -12.520 | 1.00 | 110.03 | A |
| 46 | CA | THR | 529 | 59.669 | 5.385 | -11.513 | 1.00 | 106.14 | A |
| 47 | CB | THR | 529 | 61.184 | 5.482 | -11.576 | 1.00 | 106.80 | A |
| 48 | OG1 | THR | 529 | 61.553 | 6.661 | -12.300 | 1.00 | 107.42 | A |
| 49 | CG2 | THR | 529 | 61.748 | 4.253 | -12.268 | 1.00 | 107.43 | A |
| 50 | C | THR | 529 | 59.162 | 5.870 | -10.163 | 1.00 | 102.32 | A |
| 51 | O | THR | 529 | 59.786 | 6.705 | -9.505 | 1.00 | 102.18 | A |
| 52 | N | PRO | 530 | 58.041 | 5.292 | -9.722 | 1.00 | 98.65 | A |
| 53 | CD | PRO | 530 | 57.966 | 3.889 | -10.144 | 1.00 | 97.93 | A |
| 54 | CA | PRO | 530 | 57.228 | 5.477 | -8.520 | 1.00 | 96.17 | A |
| 55 | CB | PRO | 530 | 56.233 | 4.320 | -8.566 | 1.00 | 96.64 | A |
| 56 | CG | PRO | 530 | 56.596 | 3.514 | -9.758 | 1.00 | 96.77 | A |
| 57 | C | PRO | 530 | 57.911 | 5.556 | -7.183 | 1.00 | 93.87 | A |
| 58 | O | PRO | 530 | 58.930 | 4.922 | -6.916 | 1.00 | 93.23 | A |
| 59 | N | THR | 531 | 57.261 | 6.307 | -6.315 | 1.00 | 91.25 | A |
| 60 | CA | THR | 531 | 57.776 | 6.562 | -5.003 | 1.00 | 89.02 | A |
| 61 | CB | THR | 531 | 57.936 | 8.030 | -4.841 | 1.00 | 89.18 | A |
| 62 | OG1 | THR | 531 | 56.641 | 8.646 | -4.893 | 1.00 | 88.94 | A |
| 63 | CG2 | THR | 531 | 58.762 | 8.564 | -5.981 | 1.00 | 88.16 | A |
| 64 | C | THR | 531 | 56.845 | 6.079 | -3.941 | 1.00 | 87.31 | A |
| 65 | O | THR | 531 | 55.680 | 5.783 | -4.204 | 1.00 | 86.50 | A |
| 66 | N | LEU | 532 | 57.336 | 6.023 | -2.722 | 1.00 | 85.91 | A |
| 67 | CA | LEU | 532 | 56.425 | 5.574 | -1.727 | 1.00 | 84.44 | A |
| 68 | CB | LEU | 532 | 57.077 | 5.511 | -0.355 | 1.00 | 83.60 | A |
| 69 | CG | LEU | 532 | 56.840 | 4.026 | -0.104 | 1.00 | 82.34 | A |
| 70 | CD1 | LEU | 532 | 56.814 | 3.701 | 1.352 | 1.00 | 83.51 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 71 | CD2 | LEU | 532 | 55.483 | 3.652 | -0.701 | 1.00 | 81.89 | A |
| 72 | C | LEU | 532 | 55.159 | 6.426 | -1.720 | 1.00 | 84.30 | A |
| 73 | O | LEU | 532 | 54.047 | 5.897 | -1.757 | 1.00 | 85.02 | A |
| 74 | N | VAL | 533 | 55.338 | 7.742 | -1.743 | 1.00 | 82.66 | A |
| 75 | CA | VAL | 533 | 54.240 | 8.696 | -1.706 | 1.00 | 80.63 | A |
| 76 | CB | VAL | 533 | 54.801 | 10.064 | -1.381 | 1.00 | 79.39 | A |
| 77 | CG1 | VAL | 533 | 55.615 | 10.561 | -2.562 | 1.00 | 79.10 | A |
| 78 | CG2 | VAL | 533 | 53.691 | 11.005 | -1.029 | 1.00 | 78.78 | A |
| 79 | C | VAL | 533 | 53.401 | 8.825 | -2.987 | 1.00 | 80.22 | A |
| 80 | O | VAL | 533 | 52.344 | 9.457 | -2.994 | 1.00 | 80.48 | A |
| 81 | N | SER | 534 | 53.883 | 8.234 | -4.068 | 1.00 | 78.78 | A |
| 82 | CA | SER | 534 | 53.202 | 8.309 | -5.353 | 1.00 | 77.29 | A |
| 83 | CB | SER | 534 | 54.226 | 8.107 | -6.456 | 1.00 | 79.18 | A |
| 84 | OG | SER | 534 | 53.604 | 7.680 | -7.650 | 1.00 | 82.05 | A |
| 85 | C | SER | 534 | 52.180 | 7.214 | -5.427 | 1.00 | 75.67 | A |
| 86 | O | SER | 534 | 51.079 | 7.364 | -5.959 | 1.00 | 75.13 | A |
| 87 | N | LEU | 535 | 52.615 | 6.092 | -4.892 | 1.00 | 74.52 | A |
| 88 | CA | LEU | 535 | 51.854 | 4.884 | -4.835 | 1.00 | 73.20 | A |
| 89 | CB | LEU | 535 | 52.783 | 3.790 | -4.355 | 1.00 | 72.45 | A |
| 90 | CG | LEU | 535 | 52.371 | 2.441 | -4.887 | 1.00 | 71.93 | A |
| 91 | CD1 | LEU | 535 | 51.281 | 1.873 | -4.022 | 1.00 | 70.60 | A |
| 92 | CD2 | LEU | 535 | 51.903 | 2.610 | -6.308 | 1.00 | 72.73 | A |
| 93 | C | LEU | 535 | 50.730 | 5.105 | -3.851 | 1.00 | 73.41 | A |
| 94 | O | LEU | 535 | 49.601 | 4.640 | -4.032 | 1.00 | 73.44 | A |
| 95 | N | LEU | 536 | 51.055 | 5.844 | -2.804 | 1.00 | 73.02 | A |
| 96 | CA | LEU | 536 | 50.093 | 6.137 | -1.768 | 1.00 | 72.88 | A |
| 97 | CB | LEU | 536 | 50.814 | 6.850 | -0.629 | 1.00 | 71.91 | A |
| 98 | CG | LEU | 536 | 50.740 | 6.099 | 0.706 | 1.00 | 69.79 | A |
| 99 | CD1 | LEU | 536 | 51.084 | 4.641 | 0.531 | 1.00 | 67.04 | A |
| 100 | CD2 | LEU | 536 | 51.681 | 6.752 | 1.685 | 1.00 | 70.08 | A |
| 101 | C | LEU | 536 | 48.929 | 6.972 | -2.299 | 1.00 | 72.91 | A |
| 102 | O | LEU | 536 | 47.806 | 6.895 | -1.796 | 1.00 | 71.27 | A |
| 103 | N | GLU | 537 | 49.217 | 7.738 | -3.348 | 1.00 | 74.45 | A |
| 104 | CA | GLU | 537 | 48.268 | 8.637 | -3.988 | 1.00 | 74.79 | A |
| 105 | CB | GLU | 537 | 49.044 | 9.639 | -4.847 | 1.00 | 76.31 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 106 | CG | GLU | 537 | 48.580 | 11.065 | -4.663 | 1.00 | 78.43 | A |
| 107 | CD | GLU | 537 | 49.645 | 12.119 | -4.966 | 1.00 | 80.45 | A |
| 108 | OE1 | GLU | 537 | 50.867 | 11.850 | -4.838 | 1.00 | 82.51 | A |
| 109 | OE2 | GLU | 537 | 49.238 | 13.247 | -5.310 | 1.00 | 80.91 | A |
| 110 | C | GLU | 537 | 47.203 | 7.959 | -4.843 | 1.00 | 74.83 | A |
| 111 | O | GLU | 537 | 46.055 | 8.404 | -4.881 | 1.00 | 75.79 | A |
| 112 | N | VAL | 538 | 47.583 | 6.889 | -5.521 | 1.00 | 74.12 | A |
| 113 | CA | VAL | 538 | 46.663 | 6.190 | -6.395 | 1.00 | 73.20 | A |
| 114 | CB | VAL | 538 | 47.423 | 5.486 | -7.509 | 1.00 | 72.76 | A |
| 115 | CG1 | VAL | 538 | 48.767 | 6.147 | -7.685 | 1.00 | 71.81 | A |
| 116 | CG2 | VAL | 538 | 47.590 | 4.010 | -7.178 | 1.00 | 72.29 | A |
| 117 | C | VAL | 538 | 45.853 | 5.159 | -5.647 | 1.00 | 73.07 | A |
| 118 | O | VAL | 538 | 44.727 | 4.846 | -6.033 | 1.00 | 73.83 | A |
| 119 | N | ILE | 539 | 46.417 | 4.615 | -4.575 | 1.00 | 73.33 | A |
| 120 | CA | ILE | 539 | 45.664 | 3.608 | -3.854 | 1.00 | 74.29 | A |
| 121 | CB | ILE | 539 | 46.586 | 2.582 | -3.130 | 1.00 | 73.38 | A |
| 122 | CG2 | ILE | 539 | 47.786 | 2.259 | -4.001 | 1.00 | 72.86 | A |
| 123 | CG1 | ILE | 539 | 47.048 | 3.116 | -1.779 | 1.00 | 73.08 | A |
| 124 | CD1 | ILE | 539 | 47.600 | 2.026 | -0.885 | 1.00 | 72.35 | A |
| 125 | C | ILE | 539 | 44.676 | 4.212 | -2.865 | 1.00 | 74.46 | A |
| 126 | O | ILE | 539 | 43.901 | 3.487 | -2.251 | 1.00 | 73.49 | A |
| 127 | N | GLU | 540 | 44.691 | 5.536 | -2.725 | 1.00 | 76.23 | A |
| 128 | CA | GLU | 540 | 43.770 | 6.223 | -1.810 | 1.00 | 78.11 | A |
| 129 | CB | GLU | 540 | 44.092 | 7.730 | -1.758 | 1.00 | 78.20 | A |
| 130 | CG | GLU | 540 | 43.208 | 8.584 | -0.829 | 1.00 | 79.83 | A |
| 131 | CD | GLU | 540 | 43.279 | 8.199 | 0.649 | 1.00 | 80.76 | A |
| 132 | OE1 | GLU | 540 | 44.294 | 8.501 | 1.311 | 1.00 | 81.61 | A |
| 133 | OE2 | GLU | 540 | 42.309 | 7.597 | 1.157 | 1.00 | 80.13 | A |
| 134 | C | GLU | 540 | 42.341 | 5.984 | -2.309 | 1.00 | 78.89 | A |
| 135 | O | GLU | 540 | 42.016 | 6.288 | -3.462 | 1.00 | 79.27 | A |
| 136 | N | PRO | 541 | 41.480 | 5.397 | -1.464 | 1.00 | 79.34 | A |
| 137 | CD | PRO | 541 | 41.713 | 4.622 | -0.233 | 1.00 | 79.21 | A |
| 138 | CA | PRO | 541 | 40.126 | 5.182 | -1.970 | 1.00 | 80.63 | A |
| 139 | CB | PRO | 541 | 39.403 | 4.466 | -0.819 | 1.00 | 79.43 | A |
| 140 | CG | PRO | 541 | 40.335 | 4.536 | 0.354 | 1.00 | 78.38 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 141 | C | PRO | 541 | 39.450 | 6.469 | -2.371 | 1.00 | 82.68 | A |
| 142 | O | PRO | 541 | 39.811 | 7.548 | -1.892 | 1.00 | 82.05 | A |
| 143 | N | GLU | 542 | 38.494 | 6.370 | -3.283 | 1.00 | 85.63 | A |
| 144 | CA | GLU | 542 | 37.803 | 7.576 | -3.659 | 1.00 | 88.82 | A |
| 145 | CB | GLU | 542 | 37.681 | 7.713 | -5.182 | 1.00 | 90.75 | A |
| 146 | CG | GLU | 542 | 36.797 | 6.830 | -5.997 | 1.00 | 93.88 | A |
| 147 | CD | GLU | 542 | 36.660 | 7.477 | -7.363 | 1.00 | 96.19 | A |
| 148 | OE1 | GLU | 542 | 37.715 | 7.811 | -7.953 | 1.00 | 97.39 | A |
| 149 | OE2 | GLU | 542 | 35.523 | 7.687 | -7.836 | 1.00 | 97.14 | A |
| 150 | C | GLU | 542 | 36.491 | 7.641 | -2.888 | 1.00 | 89.50 | A |
| 151 | O | GLU | 542 | 35.965 | 6.619 | -2.456 | 1.00 | 89.69 | A |
| 152 | N | VAL | 543 | 35.994 | 8.859 | -2.684 | 1.00 | 90.04 | A |
| 153 | CA | VAL | 543 | 34.820 | 9.119 | -1.843 | 1.00 | 90.75 | A |
| 154 | CB | VAL | 543 | 34.644 | 10.627 | -1.691 | 1.00 | 91.20 | A |
| 155 | CG1 | VAL | 543 | 33.946 | 10.911 | -0.379 | 1.00 | 90.59 | A |
| 156 | CG2 | VAL | 543 | 36.004 | 11.325 | -1.750 | 1.00 | 90.81 | A |
| 157 | C | VAL | 543 | 33.417 | 8.509 | -2.014 | 1.00 | 91.22 | A |
| 158 | O | VAL | 543 | 32.778 | 8.651 | -3.057 | 1.00 | 91.07 | A |
| 159 | N | LEU | 544 | 32.926 | 7.891 | -0.936 | 1.00 | 91.90 | A |
| 160 | CA | LEU | 544 | 31.607 | 7.231 | -0.896 | 1.00 | 93.16 | A |
| 161 | CB | LEU | 544 | 31.695 | 5.928 | -0.076 | 1.00 | 93.10 | A |
| 162 | CG | LEU | 544 | 32.356 | 4.691 | -0.694 | 1.00 | 93.35 | A |
| 163 | CD1 | LEU | 544 | 31.473 | 3.432 | -0.558 | 1.00 | 93.98 | A |
| 164 | CD2 | LEU | 544 | 32.607 | 4.994 | -2.153 | 1.00 | 93.53 | A |
| 165 | C | LEU | 544 | 30.382 | 8.011 | -0.378 | 1.00 | 94.32 | A |
| 166 | O | LEU | 544 | 30.268 | 8.268 | 0.826 | 1.00 | 94.86 | A |
| 167 | N | TYR | 545 | 29.456 | 8.340 | -1.289 | 1.00 | 94.95 | A |
| 168 | CA | TYR | 545 | 28.207 | 9.051 | -0.955 | 1.00 | 94.71 | A |
| 169 | CB | TYR | 545 | 27.465 | 9.466 | -2.233 | 1.00 | 96.89 | A |
| 170 | CG | TYR | 545 | 28.215 | 10.538 | -2.990 | 1.00 | 99.74 | A |
| 171 | CD1 | TYR | 545 | 29.253 | 10.210 | -3.860 | 1.00 | 101.09 | A |
| 172 | CE1 | TYR | 545 | 30.049 | 11.206 | -4.440 | 1.00 | 102.49 | A |
| 173 | CD2 | TYR | 545 | 27.978 | 11.887 | -2.732 | 1.00 | 101.22 | A |
| 174 | CE2 | TYR | 545 | 28.764 | 12.886 | -3.299 | 1.00 | 102.50 | A |
| 175 | CZ | TYR | 545 | 29.800 | 12.540 | -4.151 | 1.00 | 103.07 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 176 | OH | TYR | 545 | 30.613 | 13.525 | -4.675 | 1.00 | 103.71 | A |
| 177 | C | TYR | 545 | 27.346 | 8.126 | -0.100 | 1.00 | 93.35 | A |
| 178 | O | TYR | 545 | 27.512 | 6.914 | -0.165 | 1.00 | 92.28 | A |
| 179 | N | ALA | 546 | 26.415 | 8.678 | 0.677 | 1.00 | 92.46 | A |
| 180 | CA | ALA | 546 | 25.627 | 7.836 | 1.583 | 1.00 | 91.68 | A |
| 181 | CB | ALA | 546 | 25.512 | 8.536 | 2.941 | 1.00 | 91.47 | A |
| 182 | C | ALA | 546 | 24.259 | 7.289 | 1.195 | 1.00 | 90.98 | A |
| 183 | O | ALA | 546 | 23.712 | 6.466 | 1.930 | 1.00 | 91.08 | A |
| 184 | N | GLY | 547 | 23.696 | 7.714 | 0.069 | 1.00 | 90.37 | A |
| 185 | CA | GLY | 547 | 22.380 | 7.214 | -0.302 | 1.00 | 89.38 | A |
| 186 | C | GLY | 547 | 21.359 | 7.574 | 0.767 | 1.00 | 88.66 | A |
| 187 | O | GLY | 547 | 20.486 | 6.780 | 1.119 | 1.00 | 88.42 | A |
| 188 | N | TYR | 548 | 21.480 | 8.778 | 1.298 | 1.00 | 88.15 | A |
| 189 | CA | TYR | 548 | 20.574 | 9.246 | 2.327 | 1.00 | 86.95 | A |
| 190 | CB | TYR | 548 | 21.255 | 10.322 | 3.156 | 1.00 | 85.67 | A |
| 191 | CG | TYR | 548 | 20.496 | 10.787 | 4.372 | 1.00 | 84.73 | A |
| 192 | CD1 | TYR | 548 | 20.476 | 10.024 | 5.538 | 1.00 | 84.74 | A |
| 193 | CE1 | TYR | 548 | 19.896 | 10.510 | 6.706 | 1.00 | 84.64 | A |
| 194 | CD2 | TYR | 548 | 19.892 | 12.043 | 4.396 | 1.00 | 83.83 | A |
| 195 | CE2 | TYR | 548 | 19.310 | 12.534 | 5.553 | 1.00 | 84.34 | A |
| 196 | CZ | TYR | 548 | 19.321 | 11.763 | 6.707 | 1.00 | 84.86 | A |
| 197 | OH | TYR | 548 | 18.801 | 12.254 | 7.882 | 1.00 | 86.53 | A |
| 198 | C | TYR | 548 | 19.379 | 9.840 | 1.624 | 1.00 | 87.42 | A |
| 199 | O | TYR | 548 | 19.469 | 10.266 | 0.474 | 1.00 | 87.52 | A |
| 200 | N | ASP | 549 | 18.247 | 9.865 | 2.300 | 1.00 | 88.18 | A |
| 201 | CA | ASP | 549 | 17.071 | 10.438 | 1.694 | 1.00 | 88.70 | A |
| 202 | CB | ASP | 549 | 15.908 | 9.461 | 1.770 | 1.00 | 90.10 | A |
| 203 | CG | ASP | 549 | 14.632 | 10.070 | 1.281 | 1.00 | 91.67 | A |
| 204 | OD1 | ASP | 549 | 14.725 | 10.951 | 0.404 | 1.00 | 94.32 | A |
| 205 | OD2 | ASP | 549 | 13.552 | 9.676 | 1.763 | 1.00 | 91.48 | A |
| 206 | C | ASP | 549 | 16.744 | 11.720 | 2.433 | 1.00 | 88.75 | A |
| 207 | O | ASP | 549 | 16.139 | 11.697 | 3.494 | 1.00 | 88.40 | A |
| 208 | N | SER | 550 | 17.150 | 12.851 | 1.876 | 1.00 | 89.12 | A |
| 209 | CA | SER | 550 | 16.888 | 14.115 | 2.544 | 1.00 | 89.61 | A |
| 210 | CB | SER | 550 | 18.044 | 15.106 | 2.319 | 1.00 | 90.04 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 211 | OG | SER | 550 | 18.345 | 15.283 | 0.947 | 1.00 | 90.73 | A |
| 212 | C | SER | 550 | 15.564 | 14.714 | 2.123 | 1.00 | 89.18 | A |
| 213 | O | SER | 550 | 15.241 | 15.849 | 2.479 | 1.00 | 89.03 | A |
| 214 | N | SER | 551 | 14.791 | 13.964 | 1.357 | 1.00 | 88.84 | A |
| 215 | CA | SER | 551 | 13.493 | 14.483 | 0.995 | 1.00 | 89.26 | A |
| 216 | CB | SER | 551 | 12.878 | 13.704 | -0.168 | 1.00 | 89.54 | A |
| 217 | OG | SER | 551 | 13.113 | 12.324 | -0.037 | 1.00 | 91.73 | A |
| 218 | C | SER | 551 | 12.634 | 14.390 | 2.256 | 1.00 | 88.19 | A |
| 219 | O | SER | 551 | 11.603 | 15.030 | 2.341 | 1.00 | 88.72 | A |
| 220 | N | VAL | 552 | 13.058 | 13.610 | 3.250 | 1.00 | 87.55 | A |
| 221 | CA | VAL | 552 | 12.284 | 13.527 | 4.495 | 1.00 | 87.25 | A |
| 222 | CB | VAL | 552 | 11.830 | 12.070 | 4.820 | 1.00 | 87.97 | A |
| 223 | CG1 | VAL | 552 | 11.975 | 11.175 | 3.599 | 1.00 | 87.37 | A |
| 224 | CG2 | VAL | 552 | 12.606 | 11.529 | 6.003 | 1.00 | 87.92 | A |
| 225 | C | VAL | 552 | 13.094 | 14.092 | 5.670 | 1.00 | 86.73 | A |
| 226 | O | VAL | 552 | 14.326 | 14.022 | 5.676 | 1.00 | 86.64 | A |
| 227 | N | PRO | 553 | 12.410 | 14.634 | 6.696 | 1.00 | 86.61 | A |
| 228 | CD | PRO | 553 | 10.956 | 14.803 | 6.833 | 1.00 | 86.60 | A |
| 229 | CA | PRO | 553 | 13.114 | 15.213 | 7.850 | 1.00 | 87.41 | A |
| 230 | CB | PRO | 553 | 12.014 | 15.962 | 8.591 | 1.00 | 87.09 | A |
| 231 | CG | PRO | 553 | 10.807 | 15.185 | 8.288 | 1.00 | 87.29 | A |
| 232 | C | PRO | 553 | 13.975 | 14.371 | 8.786 | 1.00 | 87.77 | A |
| 233 | O | PRO | 553 | 13.570 | 13.322 | 9.289 | 1.00 | 88.30 | A |
| 234 | N | ASP | 554 | 15.171 | 14.905 | 9.019 | 1.00 | 87.98 | A |
| 235 | CA | ASP | 554 | 16.184 | 14.298 | 9.868 | 1.00 | 88.66 | A |
| 236 | CB | ASP | 554 | 17.364 | 15.258 | 10.095 | 1.00 | 88.19 | A |
| 237 | CG | ASP | 554 | 17.900 | 15.894 | 8.820 | 1.00 | 88.24 | A |
| 238 | OD1 | ASP | 554 | 17.439 | 15.598 | 7.695 | 1.00 | 88.21 | A |
| 239 | OD2 | ASP | 554 | 18.817 | 16.722 | 8.975 | 1.00 | 88.62 | A |
| 240 | C | ASP | 554 | 15.693 | 13.903 | 11.254 | 1.00 | 89.18 | A |
| 241 | O | ASP | 554 | 14.574 | 14.205 | 11.670 | 1.00 | 89.35 | A |
| 242 | N | SER | 555 | 16.592 | 13.245 | 11.972 | 1.00 | 89.93 | A |
| 243 | CA | SER | 555 | 16.379 | 12.799 | 13.331 | 1.00 | 90.27 | A |
| 244 | CB | SER | 555 | 15.277 | 11.750 | 13.419 | 1.00 | 90.19 | A |
| 245 | OG | SER | 555 | 15.647 | 10.558 | 12.754 | 1.00 | 89.00 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 246 | C | SER | 555 | 17.715 | 12.181 | 13.672 | 1.00 | 91.27 | A |
| 247 | O | SER | 555 | 18.445 | 11.746 | 12.780 | 1.00 | 90.85 | A |
| 248 | N | THR | 556 | 18.041 | 12.149 | 14.950 | 1.00 | 92.81 | A |
| 249 | CA | THR | 556 | 19.317 | 11.597 | 15.357 | 1.00 | 94.66 | A |
| 250 | CB | THR | 556 | 19.533 | 11.760 | 16.870 | 1.00 | 95.26 | A |
| 251 | OG1 | THR | 556 | 20.067 | 13.062 | 17.143 | 1.00 | 95.78 | A |
| 252 | CG2 | THR | 556 | 20.489 | 10.713 | 17.379 | 1.00 | 95.54 | A |
| 253 | C | THR | 556 | 19.557 | 10.141 | 14.977 | 1.00 | 95.12 | A |
| 254 | O | THR | 556 | 20.654 | 9.819 | 14.514 | 1.00 | 94.58 | A |
| 255 | N | TRP | 557 | 18.561 | 9.266 | 15.168 | 1.00 | 96.53 | A |
| 256 | CA | TRP | 557 | 18.725 | 7.837 | 14.850 | 1.00 | 97.64 | A |
| 257 | CB | TRP | 557 | 17.556 | 6.977 | 15.403 | 1.00 | 100.93 | A |
| 258 | CG | TRP | 557 | 16.527 | 6.440 | 14.377 | 1.00 | 105.32 | A |
| 259 | CD2 | TRP | 557 | 16.552 | 5.170 | 13.683 | 1.00 | 106.95 | A |
| 260 | CE2 | TRP | 557 | 15.425 | 5.135 | 12.826 | 1.00 | 107.45 | A |
| 261 | CE3 | TRP | 557 | 17.409 | 4.057 | 13.709 | 1.00 | 107.83 | A |
| 262 | CD1 | TRP | 557 | 15.417 | 7.096 | 13.913 | 1.00 | 106.74 | A |
| 263 | NE1 | TRP | 557 | 14.754 | 6.320 | 12.983 | 1.00 | 107.65 | A |
| 264 | CZ2 | TRP | 557 | 15.142 | 4.040 | 11.988 | 1.00 | 107.79 | A |
| 265 | CZ3 | TRP | 557 | 17.123 | 2.963 | 12.875 | 1.00 | 108.24 | A |
| 266 | CH2 | TRP | 557 | 15.996 | 2.967 | 12.032 | 1.00 | 107.96 | A |
| 267 | C | TRP | 557 | 18.884 | 7.612 | 13.355 | 1.00 | 96.37 | A |
| 268 | O | TRP | 557 | 19.697 | 6.803 | 12.916 | 1.00 | 96.21 | A |
| 269 | N | ARG | 558 | 18.117 | 8.351 | 12.580 | 1.00 | 95.03 | A |
| 270 | CA | ARG | 558 | 18.164 | 8.243 | 11.139 | 1.00 | 93.86 | A |
| 271 | CB | ARG | 558 | 17.054 | 9.068 | 10.566 | 1.00 | 93.78 | A |
| 272 | CG | ARG | 558 | 16.719 | 8.624 | 9.224 | 1.00 | 94.44 | A |
| 273 | CD | ARG | 558 | 15.975 | 9.679 | 8.550 | 1.00 | 94.58 | A |
| 274 | NE | ARG | 558 | 16.253 | 9.641 | 7.134 | 1.00 | 95.72 | A |
| 275 | CZ | ARG | 558 | 16.025 | 10.672 | 6.351 | 1.00 | 96.79 | A |
| 276 | NH1 | ARG | 558 | 15.513 | 11.775 | 6.873 | 1.00 | 98.36 | A |
| 277 | NH2 | ARG | 558 | 16.334 | 10.612 | 5.077 | 1.00 | 96.49 | A |
| 278 | C | ARG | 558 | 19.461 | 8.773 | 10.561 | 1.00 | 93.25 | A |
| 279 | O | ARG | 558 | 19.887 | 8.406 | 9.467 | 1.00 | 93.99 | A |
| 280 | N | ILE | 559 | 20.070 | 9.683 | 11.291 | 1.00 | 91.60 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 281 | CA | ILE | 559 | 21.280 | 10.294 | 10.812 | 1.00 | 89.71 | A |
| 282 | CB | ILE | 559 | 21.492 | 11.590 | 11.494 | 1.00 | 88.91 | A |
| 283 | CG2 | ILE | 559 | 22.970 | 11.817 | 11.655 | 1.00 | 87.58 | A |
| 284 | CG1 | ILE | 559 | 20.833 | 12.699 | 10.697 | 1.00 | 88.10 | A |
| 285 | CD1 | ILE | 559 | 21.767 | 13.323 | 9.711 | 1.00 | 86.84 | A |
| 286 | C | ILE | 559 | 22.514 | 9.481 | 11.063 | 1.00 | 89.28 | A |
| 287 | O | ILE | 559 | 23.505 | 9.565 | 10.329 | 1.00 | 88.46 | A |
| 288 | N | MET | 560 | 22.486 | 8.742 | 12.149 | 1.00 | 88.75 | A |
| 289 | CA | MET | 560 | 23.651 | 7.976 | 12.426 | 1.00 | 88.01 | A |
| 290 | CB | MET | 560 | 24.089 | 8.152 | 13.864 | 1.00 | 87.86 | A |
| 291 | CG | MET | 560 | 22.989 | 8.134 | 14.830 | 1.00 | 87.31 | A |
| 292 | SD | MET | 560 | 23.689 | 8.279 | 16.446 | 1.00 | 90.24 | A |
| 293 | CE | MET | 560 | 22.239 | 8.344 | 17.386 | 1.00 | 88.07 | A |
| 294 | C | MET | 560 | 23.471 | 6.542 | 12.062 | 1.00 | 87.94 | A |
| 295 | O | MET | 560 | 24.281 | 5.703 | 12.418 | 1.00 | 89.01 | A |
| 296 | N | THR | 561 | 22.401 | 6.202 | 11.378 | 1.00 | 87.34 | A |
| 297 | CA | THR | 561 | 22.443 | 4.835 | 10.977 | 1.00 | 86.95 | A |
| 298 | CB | THR | 561 | 21.093 | 4.175 | 10.912 | 1.00 | 87.31 | A |
| 299 | OG1 | THR | 561 | 20.928 | 3.364 | 12.087 | 1.00 | 87.10 | A |
| 300 | CG2 | THR | 561 | 21.020 | 3.289 | 9.681 | 1.00 | 87.30 | A |
| 301 | C | THR | 561 | 23.112 | 4.874 | 9.612 | 1.00 | 86.05 | A |
| 302 | O | THR | 561 | 24.027 | 4.104 | 9.345 | 1.00 | 86.32 | A |
| 303 | N | THR | 562 | 22.686 | 5.803 | 8.762 | 1.00 | 85.38 | A |
| 304 | CA | THR | 562 | 23.254 | 5.931 | 7.431 | 1.00 | 85.67 | A |
| 305 | CB | THR | 562 | 22.661 | 7.175 | 6.763 | 1.00 | 85.65 | A |
| 306 | OG1 | THR | 562 | 21.230 | 7.079 | 6.820 | 1.00 | 85.77 | A |
| 307 | CG2 | THR | 562 | 23.098 | 7.282 | 5.312 | 1.00 | 85.39 | A |
| 308 | C | THR | 562 | 24.774 | 6.055 | 7.544 | 1.00 | 85.77 | A |
| 309 | O | THR | 562 | 25.539 | 5.598 | 6.665 | 1.00 | 86.58 | A |
| 310 | N | LEU | 563 | 25.190 | 6.642 | 8.661 | 1.00 | 84.92 | A |
| 311 | CA | LEU | 563 | 26.579 | 6.904 | 8.938 | 1.00 | 82.98 | A |
| 312 | CB | LEU | 563 | 26.574 | 7.878 | 10.095 | 1.00 | 82.31 | A |
| 313 | CG | LEU | 563 | 27.148 | 9.271 | 9.752 | 1.00 | 81.13 | A |
| 314 | CD1 | LEU | 563 | 26.867 | 9.649 | 8.298 | 1.00 | 79.57 | A |
| 315 | CD2 | LEU | 563 | 26.558 | 10.334 | 10.667 | 1.00 | 79.28 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 316 | C | LEU | 563 | 27.298 | 5.591 | 9.252 | 1.00 | 82.41 | A |
| 317 | O | LEU | 563 | 28.455 | 5.334 | 8.868 | 1.00 | 82.20 | A |
| 318 | N | ASN | 564 | 26.593 | 4.751 | 9.975 | 1.00 | 81.88 | A |
| 319 | CA | ASN | 564 | 27.155 | 3.478 | 10.274 | 1.00 | 81.91 | A |
| 320 | CB | ASN | 564 | 26.246 | 2.805 | 11.249 | 1.00 | 81.34 | A |
| 321 | CG | ASN | 564 | 26.562 | 3.217 | 12.637 | 1.00 | 81.57 | A |
| 322 | OD1 | ASN | 564 | 26.978 | 4.352 | 12.886 | 1.00 | 81.79 | A |
| 323 | ND2 | ASN | 564 | 26.387 | 2.297 | 13.559 | 1.00 | 82.16 | A |
| 324 | C | ASN | 564 | 27.242 | 2.755 | 8.952 | 1.00 | 81.96 | A |
| 325 | O | ASN | 564 | 28.304 | 2.329 | 8.539 | 1.00 | 82.86 | A |
| 326 | N | MET | 565 | 26.113 | 2.617 | 8.281 | 1.00 | 81.73 | A |
| 327 | CA | MET | 565 | 26.088 | 1.995 | 6.978 | 1.00 | 81.37 | A |
| 328 | CB | MET | 565 | 24.744 | 2.265 | 6.354 | 1.00 | 82.20 | A |
| 329 | CG | MET | 565 | 23.649 | 1.696 | 7.190 | 1.00 | 83.63 | A |
| 330 | SD | MET | 565 | 23.641 | -0.044 | 6.893 | 1.00 | 87.40 | A |
| 331 | CE | MET | 565 | 23.455 | -0.696 | 8.521 | 1.00 | 85.88 | A |
| 332 | C | MET | 565 | 27.200 | 2.581 | 6.113 | 1.00 | 80.49 | A |
| 333 | O | MET | 565 | 27.821 | 1.862 | 5.333 | 1.00 | 81.60 | A |
| 334 | N | LEU | 566 | 27.450 | 3.884 | 6.224 | 1.00 | 78.75 | A |
| 335 | CA | LEU | 566 | 28.545 | 4.438 | 5.437 | 1.00 | 77.41 | A |
| 336 | CB | LEU | 566 | 28.605 | 5.973 | 5.503 | 1.00 | 76.81 | A |
| 337 | CG | LEU | 566 | 29.358 | 6.582 | 4.305 | 1.00 | 76.68 | A |
| 338 | CD1 | LEU | 566 | 29.976 | 7.929 | 4.627 | 1.00 | 75.40 | A |
| 339 | CD2 | LEU | 566 | 30.456 | 5.629 | 3.906 | 1.00 | 78.41 | A |
| 340 | C | LEU | 566 | 29.786 | 3.827 | 6.097 | 1.00 | 77.16 | A |
| 341 | O | LEU | 566 | 30.806 | 3.581 | 5.453 | 1.00 | 75.61 | A |
| 342 | N | GLY | 567 | 29.669 | 3.566 | 7.396 | 1.00 | 77.62 | A |
| 343 | CA | GLY | 567 | 30.752 | 2.961 | 8.154 | 1.00 | 77.48 | A |
| 344 | C | GLY | 567 | 31.245 | 1.671 | 7.527 | 1.00 | 78.30 | A |
| 345 | O | GLY | 567 | 32.411 | 1.585 | 7.145 | 1.00 | 78.23 | A |
| 346 | N | GLY | 568 | 30.371 | 0.671 | 7.410 | 1.00 | 78.02 | A |
| 347 | CA | GLY | 568 | 30.776 | -0.588 | 6.815 | 1.00 | 77.12 | A |
| 348 | C | GLY | 568 | 31.491 | -0.437 | 5.479 | 1.00 | 76.89 | A |
| 349 | O | GLY | 568 | 32.611 | -0.917 | 5.312 | 1.00 | 78.89 | A |
| 350 | N | ARG | 569 | 30.860 | 0.234 | 4.525 | 1.00 | 75.25 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 351 | CA | ARG | 569 | 31.463 | 0.391 | 3.212 | 1.00 | 75.08 | A |
| 352 | CB | ARG | 569 | 30.550 | 1.219 | 2.304 | 1.00 | 76.25 | A |
| 353 | CG | ARG | 569 | 29.293 | 0.458 | 1.878 | 1.00 | 77.82 | A |
| 354 | CD | ARG | 569 | 28.574 | 1.141 | 0.734 | 1.00 | 78.35 | A |
| 355 | NE | ARG | 569 | 28.004 | 2.413 | 1.141 | 1.00 | 80.79 | A |
| 356 | CZ | ARG | 569 | 28.127 | 3.537 | 0.439 | 1.00 | 82.46 | A |
| 357 | NH1 | ARG | 569 | 28.808 | 3.533 | -0.698 | 1.00 | 82.95 | A |
| 358 | NH2 | ARG | 569 | 27.573 | 4.660 | 0.871 | 1.00 | 82.74 | A |
| 359 | C | ARG | 569 | 32.859 | 0.990 | 3.251 | 1.00 | 74.49 | A |
| 360 | O | ARG | 569 | 33.778 | 0.496 | 2.595 | 1.00 | 74.53 | A |
| 361 | N | GLN | 570 | 33.016 | 2.056 | 4.028 | 1.00 | 73.65 | A |
| 362 | CA | GLN | 570 | 34.304 | 2.722 | 4.155 | 1.00 | 71.97 | A |
| 363 | CB | GLN | 570 | 34.160 | 3.957 | 5.032 | 1.00 | 72.89 | A |
| 364 | CG | GLN | 570 | 34.204 | 5.270 | 4.286 | 1.00 | 72.15 | A |
| 365 | CD | GLN | 570 | 33.699 | 6.384 | 5.147 | 1.00 | 71.88 | A |
| 366 | OE1 | GLN | 570 | 33.539 | 7.513 | 4.692 | 1.00 | 72.77 | A |
| 367 | NE2 | GLN | 570 | 33.434 | 6.074 | 6.412 | 1.00 | 71.54 | A |
| 368 | C | GLN | 570 | 35.344 | 1.797 | 4.762 | 1.00 | 70.46 | A |
| 369 | O | GLN | 570 | 36.477 | 1.728 | 4.286 | 1.00 | 70.24 | A |
| 370 | N | VAL | 571 | 34.959 | 1.100 | 5.824 | 1.00 | 68.82 | A |
| 371 | CA | VAL | 571 | 35.850 | 0.165 | 6.499 | 1.00 | 68.22 | A |
| 372 | CB | VAL | 571 | 35.133 | -0.539 | 7.669 | 1.00 | 68.99 | A |
| 373 | CG1 | VAL | 571 | 35.836 | -1.845 | 8.004 | 1.00 | 66.89 | A |
| 374 | CG2 | VAL | 571 | 35.114 | 0.371 | 8.886 | 1.00 | 69.13 | A |
| 375 | C | VAL | 571 | 36.358 | -0.887 | 5.525 | 1.00 | 67.65 | A |
| 376 | O | VAL | 571 | 37.553 | -1.197 | 5.492 | 1.00 | 67.31 | A |
| 377 | N | ILE | 572 | 35.436 | -1.443 | 4.742 | 1.00 | 67.57 | A |
| 378 | CA | ILE | 572 | 35.784 | -2.451 | 3.747 | 1.00 | 67.90 | A |
| 379 | CB | ILE | 572 | 34.517 | -2.964 | 3.017 | 1.00 | 68.96 | A |
| 380 | CG2 | ILE | 572 | 34.870 | -3.480 | 1.617 | 1.00 | 68.69 | A |
| 381 | CG1 | ILE | 572 | 33.863 | -4.063 | 3.870 | 1.00 | 69.67 | A |
| 382 | CD1 | ILE | 572 | 32.347 | -4.003 | 3.945 | 1.00 | 68.89 | A |
| 383 | C | ILE | 572 | 36.760 | -1.832 | 2.768 | 1.00 | 67.12 | A |
| 384 | O | ILE | 572 | 37.777 | -2.428 | 2.448 | 1.00 | 68.93 | A |
| 385 | N | ALA | 573 | 36.464 | -0.630 | 2.308 | 1.00 | 66.15 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 386 | CA | ALA | 573 | 37.356 | 0.054 | 1.383 | 1.00 | 66.46 | A |
| 387 | CB | ALA | 573 | 36.840 | 1.478 | 1.108 | 1.00 | 65.79 | A |
| 388 | C | ALA | 573 | 38.771 | 0.116 | 1.967 | 1.00 | 66.93 | A |
| 389 | O | ALA | 573 | 39.766 | -0.012 | 1.238 | 1.00 | 65.81 | A |
| 390 | N | ALA | 574 | 38.843 | 0.312 | 3.287 | 1.00 | 67.23 | A |
| 391 | CA | ALA | 574 | 40.110 | 0.419 | 4.014 | 1.00 | 67.61 | A |
| 392 | CB | ALA | 574 | 39.846 | 0.728 | 5.486 | 1.00 | 67.60 | A |
| 393 | C | ALA | 574 | 40.942 | -0.847 | 3.894 | 1.00 | 67.98 | A |
| 394 | O | ALA | 574 | 42.168 | -0.789 | 3.797 | 1.00 | 67.69 | A |
| 395 | N | VAL | 575 | 40.265 | -1.992 | 3.912 | 1.00 | 67.17 | A |
| 396 | CA | VAL | 575 | 40.937 | -3.273 | 3.792 | 1.00 | 66.42 | A |
| 397 | CB | VAL | 575 | 39.946 | -4.428 | 3.976 | 1.00 | 65.00 | A |
| 398 | CG1 | VAL | 575 | 40.670 | -5.734 | 3.877 | 1.00 | 64.48 | A |
| 399 | CG2 | VAL | 575 | 39.261 | -4.314 | 5.314 | 1.00 | 64.37 | A |
| 400 | C | VAL | 575 | 41.645 | -3.404 | 2.428 | 1.00 | 66.62 | A |
| 401 | O | VAL | 575 | 42.841 | -3.686 | 2.380 | 1.00 | 66.81 | A |
| 402 | N | LYS | 576 | 40.918 | -3.183 | 1.329 | 1.00 | 66.28 | A |
| 403 | CA | LYS | 576 | 41.495 | -3.282 | -0.024 | 1.00 | 66.17 | A |
| 404 | CB | LYS | 576 | 40.403 | -3.082 | -1.076 | 1.00 | 67.84 | A |
| 405 | CG | LYS | 576 | 40.917 | -2.933 | -2.521 | 1.00 | 70.31 | A |
| 406 | CD | LYS | 576 | 39.757 | -2.724 | -3.500 | 1.00 | 72.81 | A |
| 407 | CE | LYS | 576 | 38.675 | -3.803 | -3.306 | 1.00 | 74.86 | A |
| 408 | NZ | LYS | 576 | 37.379 | -3.492 | -4.002 | 1.00 | 76.42 | A |
| 409 | C | LYS | 576 | 42.601 | -2.249 | -0.253 | 1.00 | 65.27 | A |
| 410 | O | LYS | 576 | 43.366 | -2.322 | -1.221 | 1.00 | 66.35 | A |
| 411 | N | TRP | 577 | 42.652 | -1.283 | 0.652 | 1.00 | 63.60 | A |
| 412 | CA | TRP | 577 | 43.617 | -0.188 | 0.636 | 1.00 | 60.50 | A |
| 413 | CB | TRP | 577 | 42.988 | 1.044 | 1.280 | 1.00 | 57.29 | A |
| 414 | CG | TRP | 577 | 43.969 | 2.074 | 1.708 | 1.00 | 52.50 | A |
| 415 | CD2 | TRP | 577 | 44.414 | 2.330 | 3.042 | 1.00 | 50.81 | A |
| 416 | CE2 | TRP | 577 | 45.370 | 3.360 | 2.971 | 1.00 | 50.82 | A |
| 417 | CE3 | TRP | 577 | 44.113 | 1.769 | 4.293 | 1.00 | 49.45 | A |
| 418 | CD1 | TRP | 577 | 44.642 | 2.943 | 0.904 | 1.00 | 51.34 | A |
| 419 | NE1 | TRP | 577 | 45.484 | 3.721 | 1.654 | 1.00 | 50.82 | A |
| 420 | CZ2 | TRP | 577 | 46.011 | 3.873 | 4.105 | 1.00 | 50.90 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 421 | CZ3 | TRP | 577 | 44.754 | 2.274 | 5.421 | 1.00 | 49.27 | A |
| 422 | CH2 | TRP | 577 | 45.697 | 3.309 | 5.318 | 1.00 | 49.59 | A |
| 423 | C | TRP | 577 | 44.842 | -0.583 | 1.437 | 1.00 | 60.60 | A |
| 424 | O | TRP | 577 | 45.975 | -0.357 | 1.018 | 1.00 | 59.49 | A |
| 425 | N | ALA | 578 | 44.591 | -1.143 | 2.617 | 1.00 | 61.86 | A |
| 426 | CA | ALA | 578 | 45.657 | -1.570 | 3.507 | 1.00 | 63.00 | A |
| 427 | CB | ALA | 578 | 45.073 | -2.239 | 4.745 | 1.00 | 61.78 | A |
| 428 | C | ALA | 578 | 46.541 | -2.546 | 2.759 | 1.00 | 64.13 | A |
| 429 | O | ALA | 578 | 47.762 | -2.444 | 2.791 | 1.00 | 65.39 | A |
| 430 | N | LYS | 579 | 45.901 | -3.480 | 2.065 | 1.00 | 64.92 | A |
| 431 | CA | LYS | 579 | 46.608 | -4.491 | 1.308 | 1.00 | 64.28 | A |
| 432 | CB | LYS | 579 | 45.612 | -5.540 | 0.792 | 1.00 | 64.80 | A |
| 433 | CG | LYS | 579 | 44.584 | -6.000 | 1.845 | 1.00 | 65.52 | A |
| 434 | CD | LYS | 579 | 43.721 | -7.234 | 1.436 | 1.00 | 65.27 | A |
| 435 | CE | LYS | 579 | 43.635 | -7.474 | -0.085 | 1.00 | 65.83 | A |
| 436 | NZ | LYS | 579 | 42.891 | -8.722 | -0.466 | 1.00 | 66.54 | A |
| 437 | C | LYS | 579 | 47.427 | -3.916 | 0.150 | 1.00 | 64.31 | A |
| 438 | O | LYS | 579 | 48.215 | -4.634 | -0.447 | 1.00 | 66.40 | A |
| 439 | N | ALA | 580 | 47.268 | -2.637 | -0.180 | 1.00 | 63.18 | A |
| 440 | CA | ALA | 580 | 48.057 | -2.074 | -1.278 | 1.00 | 63.23 | A |
| 441 | CB | ALA | 580 | 47.194 | -1.179 | -2.159 | 1.00 | 63.30 | A |
| 442 | C | ALA | 580 | 49.260 | -1.291 | -0.765 | 1.00 | 63.73 | A |
| 443 | O | ALA | 580 | 50.146 | -0.917 | -1.542 | 1.00 | 63.06 | A |
| 444 | N | ILE | 581 | 49.273 | -1.032 | 0.543 | 1.00 | 64.21 | A |
| 445 | CA | ILE | 581 | 50.377 | -0.316 | 1.172 | 1.00 | 64.66 | A |
| 446 | CB | ILE | 581 | 50.115 | -0.041 | 2.670 | 1.00 | 63.87 | A |
| 447 | CG2 | ILE | 581 | 51.358 | 0.554 | 3.325 | 1.00 | 62.27 | A |
| 448 | CG1 | ILE | 581 | 48.940 | 0.927 | 2.812 | 1.00 | 62.68 | A |
| 449 | CD1 | ILE | 581 | 47.956 | 0.530 | 3.881 | 1.00 | 63.04 | A |
| 450 | C | ILE | 581 | 51.552 | -1.258 | 1.035 | 1.00 | 66.30 | A |
| 451 | O | ILE | 581 | 51.620 | -2.304 | 1.689 | 1.00 | 66.21 | A |
| 452 | N | PRO | 582 | 52.480 | -0.923 | 0.142 | 1.00 | 67.08 | A |
| 453 | CD | PRO | 582 | 52.523 | 0.254 | -0.741 | 1.00 | 67.75 | A |
| 454 | CA | PRO | 582 | 53.637 | -1.791 | -0.037 | 1.00 | 68.22 | A |
| 455 | CB | PRO | 582 | 54.645 | -0.891 | -0.770 | 1.00 | 68.28 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 456 | CG | PRO | 582 | 53.981 | 0.516 | -0.825 | 1.00 | 68.57 | A |
| 457 | C | PRO | 582 | 54.154 | -2.320 | 1.307 | 1.00 | 69.73 | A |
| 458 | O | PRO | 582 | 54.501 | -1.537 | 2.192 | 1.00 | 70.65 | A |
| 459 | N | GLY | 583 | 54.167 | -3.641 | 1.471 | 1.00 | 71.35 | A |
| 460 | CA | GLY | 583 | 54.663 | -4.223 | 2.709 | 1.00 | 72.71 | A |
| 461 | C | GLY | 583 | 53.642 | -4.649 | 3.749 | 1.00 | 74.07 | A |
| 462 | O | GLY | 583 | 53.910 | -5.513 | 4.584 | 1.00 | 74.51 | A |
| 463 | N | PHE | 584 | 52.461 | -4.058 | 3.728 | 1.00 | 74.52 | A |
| 464 | CA | PHE | 584 | 51.485 | -4.449 | 4.716 | 1.00 | 74.52 | A |
| 465 | CB | PHE | 584 | 50.236 | -3.586 | 4.614 | 1.00 | 69.69 | A |
| 466 | CG | PHE | 584 | 49.161 | -3.994 | 5.563 | 1.00 | 64.19 | A |
| 467 | CD1 | PHE | 584 | 49.244 | -3.660 | 6.910 | 1.00 | 62.21 | A |
| 468 | CD2 | PHE | 584 | 48.078 | -4.729 | 5.115 | 1.00 | 61.91 | A |
| 469 | CE1 | PHE | 584 | 48.268 | -4.070 | 7.809 | 1.00 | 60.54 | A |
| 470 | CE2 | PHE | 584 | 47.091 | -5.147 | 6.004 | 1.00 | 61.98 | A |
| 471 | CZ | PHE | 584 | 47.180 | -4.811 | 7.354 | 1.00 | 60.81 | A |
| 472 | C | PHE | 584 | 51.089 | -5.915 | 4.565 | 1.00 | 77.99 | A |
| 473 | O | PHE | 584 | 50.806 | -6.590 | 5.566 | 1.00 | 78.31 | A |
| 474 | N | ARG | 585 | 51.066 | -6.429 | 3.337 | 1.00 | 80.71 | A |
| 475 | CA | ARG | 585 | 50.634 | -7.812 | 3.215 | 1.00 | 82.98 | A |
| 476 | CB | ARG | 585 | 49.729 | -8.017 | 1.993 | 1.00 | 83.84 | A |
| 477 | CG | ARG | 585 | 50.239 | -7.765 | 0.590 | 1.00 | 86.72 | A |
| 478 | CD | ARG | 585 | 49.051 | -8.111 | -0.318 | 1.00 | 89.26 | A |
| 479 | NE | ARG | 585 | 48.267 | -9.140 | 0.371 | 1.00 | 92.25 | A |
| 480 | CZ | ARG | 585 | 47.235 | -9.818 | -0.119 | 1.00 | 93.54 | A |
| 481 | NH1 | ARG | 585 | 46.797 | -9.610 | -1.359 | 1.00 | 94.06 | A |
| 482 | NH2 | ARG | 585 | 46.652 | -10.737 | 0.641 | 1.00 | 93.54 | A |
| 483 | C | ARG | 585 | 51.662 | -8.917 | 3.324 | 1.00 | 83.93 | A |
| 484 | O | ARG | 585 | 51.379 | -10.069 | 3.003 | 1.00 | 84.40 | A |
| 485 | N | ASN | 586 | 52.845 | -8.573 | 3.802 | 1.00 | 85.01 | A |
| 486 | CA | ASN | 586 | 53.871 | -9.570 | 4.022 | 1.00 | 85.88 | A |
| 487 | CB | ASN | 586 | 55.231 | -9.061 | 3.564 | 1.00 | 86.01 | A |
| 488 | CG | ASN | 586 | 55.328 | -8.972 | 2.059 | 1.00 | 86.96 | A |
| 489 | OD1 | ASN | 586 | 56.236 | -8.339 | 1.522 | 1.00 | 88.16 | A |
| 490 | ND2 | ASN | 586 | 54.388 | -9.613 | 1.363 | 1.00 | 86.48 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 491 | C | ASN | 586 | 53.846 | -9.776 | 5.518 | 1.00 | 86.24 | A |
| 492 | O | ASN | 586 | 54.624 | -10.539 | 6.077 | 1.00 | 87.32 | A |
| 493 | N | LEU | 587 | 52.932 | -9.071 | 6.169 | 1.00 | 86.65 | A |
| 494 | CA | LEU | 587 | 52.792 | -9.198 | 7.600 | 1.00 | 87.77 | A |
| 495 | CB | LEU | 587 | 52.088 | -7.970 | 8.188 | 1.00 | 87.74 | A |
| 496 | CG | LEU | 587 | 52.935 | -6.697 | 8.210 | 1.00 | 86.52 | A |
| 497 | CD1 | LEU | 587 | 52.116 | -5.534 | 8.724 | 1.00 | 86.30 | A |
| 498 | CD2 | LEU | 587 | 54.145 | -6.924 | 9.095 | 1.00 | 86.53 | A |
| 499 | C | LEU | 587 | 51.979 | -10.450 | 7.850 | 1.00 | 88.42 | A |
| 500 | O | LEU | 587 | 51.260 | -10.930 | 6.968 | 1.00 | 87.25 | A |
| 501 | N | HIS | 588 | 52.108 | -10.989 | 9.053 | 1.00 | 89.97 | A |
| 502 | CA | HIS | 588 | 51.378 | -12.191 | 9.390 | 1.00 | 91.55 | A |
| 503 | CB | HIS | 588 | 51.642 | -12.574 | 10.835 | 1.00 | 93.32 | A |
| 504 | CG | HIS | 588 | 51.068 | -13.911 | 11.192 | 1.00 | 95.30 | A |
| 505 | CD2 | HIS | 588 | 50.156 | -14.245 | 12.137 | 1.00 | 95.82 | A |
| 506 | ND1 | HIS | 588 | 51.401 | -15.047 | 10.527 | 1.00 | 96.00 | A |
| 507 | CE1 | HIS | 588 | 50.715 | -16.075 | 11.050 | 1.00 | 96.43 | A |
| 508 | NE2 | HIS | 588 | 49.967 | -15.608 | 12.013 | 1.00 | 96.43 | A |
| 509 | C | HIS | 588 | 49.887 | -11.952 | 9.184 | 1.00 | 91.17 | A |
| 510 | O | HIS | 588 | 49.297 | -11.142 | 9.893 | 1.00 | 91.33 | A |
| 511 | N | LEU | 589 | 49.284 | -12.651 | 8.221 | 1.00 | 89.87 | A |
| 512 | CA | LEU | 589 | 47.860 | -12.477 | 7.945 | 1.00 | 88.11 | A |
| 513 | CB | LEU | 589 | 47.284 | -13.700 | 7.221 | 1.00 | 88.40 | A |
| 514 | CG | LEU | 589 | 45.765 | -13.669 | 6.969 | 1.00 | 88.28 | A |
| 515 | CD1 | LEU | 589 | 45.400 | -14.733 | 5.962 | 1.00 | 88.56 | A |
| 516 | CD2 | LEU | 589 | 44.979 | -13.904 | 8.259 | 1.00 | 88.39 | A |
| 517 | C | LEU | 589 | 47.047 | -12.209 | 9.208 | 1.00 | 87.37 | A |
| 518 | O | LEU | 589 | 45.969 | -11.624 | 9.127 | 1.00 | 86.71 | A |
| 519 | N | ASP | 590 | 47.541 | -12.642 | 10.363 | 1.00 | 86.54 | A |
| 520 | CA | ASP | 590 | 46.833 | -12.406 | 11.625 | 1.00 | 85.87 | A |
| 521 | CB | ASP | 590 | 47.294 | -13.340 | 12.734 | 1.00 | 86.86 | A |
| 522 | CG | ASP | 590 | 46.610 | -14.679 | 12.702 | 1.00 | 87.52 | A |
| 523 | OD1 | ASP | 590 | 45.526 | -14.809 | 12.090 | 1.00 | 87.78 | A |
| 524 | OD2 | ASP | 590 | 47.167 | -15.605 | 13.323 | 1.00 | 87.71 | A |
| 525 | C | ASP | 590 | 47.067 | -11.002 | 12.120 | 1.00 | 84.92 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 526 | O | ASP | 590 | 46.244 | -10.458 | 12.849 | 1.00 | 84.94 | A |
| 527 | N | ASP | 591 | 48.230 | -10.454 | 11.769 | 1.00 | 84.52 | A |
| 528 | CA | ASP | 591 | 48.620 | -9.091 | 12.130 | 1.00 | 83.62 | A |
| 529 | CB | ASP | 591 | 50.107 | -8.838 | 11.850 | 1.00 | 84.12 | A |
| 530 | CG | ASP | 591 | 51.022 | -9.693 | 12.697 | 1.00 | 84.28 | A |
| 531 | OD1 | ASP | 591 | 50.727 | -9.862 | 13.899 | 1.00 | 84.06 | A |
| 532 | OD2 | ASP | 591 | 52.042 | -10.183 | 12.160 | 1.00 | 83.73 | A |
| 533 | C | ASP | 591 | 47.808 | -8.185 | 11.229 | 1.00 | 82.14 | A |
| 534 | O | ASP | 591 | 47.205 | -7.207 | 11.669 | 1.00 | 81.59 | A |
| 535 | N | GLN | 592 | 47.815 | -8.529 | 9.946 | 1.00 | 80.47 | A |
| 536 | CA | GLN | 592 | 47.067 | -7.782 | 8.969 | 1.00 | 78.87 | A |
| 537 | CB | GLN | 592 | 46.947 | -8.594 | 7.690 | 1.00 | 77.24 | A |
| 538 | CG | GLN | 592 | 48.091 | -8.281 | 6.782 | 1.00 | 76.69 | A |
| 539 | CD | GLN | 592 | 48.068 | -9.036 | 5.487 | 1.00 | 76.67 | A |
| 540 | OE1 | GLN | 592 | 47.017 | -9.193 | 4.855 | 1.00 | 76.15 | A |
| 541 | NE2 | GLN | 592 | 49.241 | -9.495 | 5.059 | 1.00 | 75.91 | A |
| 542 | C | GLN | 592 | 45.706 | -7.450 | 9.538 | 1.00 | 78.83 | A |
| 543 | O | GLN | 592 | 45.148 | -6.390 | 9.245 | 1.00 | 79.67 | A |
| 544 | N | MET | 593 | 45.212 | -8.347 | 10.393 | 1.00 | 78.36 | A |
| 545 | CA | MET | 593 | 43.912 | -8.231 | 11.058 | 1.00 | 78.31 | A |
| 546 | CB | MET | 593 | 43.275 | -9.615 | 11.244 | 1.00 | 79.96 | A |
| 547 | CG | MET | 593 | 42.329 | -10.103 | 10.149 | 1.00 | 82.51 | A |
| 548 | SD | MET | 593 | 41.604 | -11.744 | 10.579 | 1.00 | 86.38 | A |
| 549 | CE | MET | 593 | 39.882 | -11.316 | 10.995 | 1.00 | 84.03 | A |
| 550 | C | MET | 593 | 43.900 | -7.547 | 12.428 | 1.00 | 77.60 | A |
| 551 | O | MET | 593 | 42.843 | -7.081 | 12.859 | 1.00 | 77.34 | A |
| 552 | N | THR | 594 | 45.020 | -7.507 | 13.145 | 1.00 | 76.82 | A |
| 553 | CA | THR | 594 | 44.984 | -6.861 | 14.458 | 1.00 | 76.93 | A |
| 554 | CB | THR | 594 | 46.140 | -7.311 | 15.391 | 1.00 | 77.94 | A |
| 555 | OG1 | THR | 594 | 46.650 | -8.574 | 14.956 | 1.00 | 78.86 | A |
| 556 | CG2 | THR | 594 | 45.627 | -7.462 | 16.828 | 1.00 | 77.65 | A |
| 557 | C | THR | 594 | 45.098 | -5.365 | 14.225 | 1.00 | 76.19 | A |
| 558 | O | THR | 594 | 44.451 | -4.551 | 14.896 | 1.00 | 75.57 | A |
| 559 | N | LEU | 595 | 45.936 | -5.021 | 13.252 | 1.00 | 74.76 | A |
| 560 | CA | LEU | 595 | 46.147 | -3.637 | 12.882 | 1.00 | 72.91 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 561 | CB | LEU | 595 | 47.243 | -3.554 | 11.804 | 1.00 | 70.46 | A |
| 562 | CG | LEU | 595 | 48.681 | -3.258 | 12.261 | 1.00 | 67.61 | A |
| 563 | CD1 | LEU | 595 | 48.841 | -3.596 | 13.722 | 1.00 | 66.68 | A |
| 564 | CD2 | LEU | 595 | 49.676 | -4.028 | 11.409 | 1.00 | 66.99 | A |
| 565 | C | LEU | 595 | 44.820 | -3.030 | 12.400 | 1.00 | 72.82 | A |
| 566 | O | LEU | 595 | 44.428 | -1.966 | 12.874 | 1.00 | 73.34 | A |
| 567 | N | LEU | 596 | 44.103 | -3.706 | 11.505 | 1.00 | 71.86 | A |
| 568 | CA | LEU | 596 | 42.837 | -3.154 | 11.025 | 1.00 | 71.08 | A |
| 569 | CB | LEU | 596 | 42.307 | -3.985 | 9.868 | 1.00 | 67.81 | A |
| 570 | CG | LEU | 596 | 42.995 | -3.546 | 8.584 | 1.00 | 65.63 | A |
| 571 | CD1 | LEU | 596 | 42.589 | -4.452 | 7.451 | 1.00 | 65.47 | A |
| 572 | CD2 | LEU | 596 | 42.625 | -2.111 | 8.286 | 1.00 | 64.45 | A |
| 573 | C | LEU | 596 | 41.751 | -2.993 | 12.083 | 1.00 | 72.89 | A |
| 574 | O | LEU | 596 | 40.990 | -2.023 | 12.061 | 1.00 | 72.60 | A |
| 575 | N | GLN | 597 | 41.685 | -3.940 | 13.014 | 1.00 | 75.30 | A |
| 576 | CA | GLN | 597 | 40.688 | -3.913 | 14.080 | 1.00 | 77.33 | A |
| 577 | CB | GLN | 597 | 40.550 | -5.284 | 14.700 | 1.00 | 79.16 | A |
| 578 | CG | GLN | 597 | 39.994 | -6.316 | 13.777 | 1.00 | 82.61 | A |
| 579 | CD | GLN | 597 | 40.095 | -7.697 | 14.372 | 1.00 | 84.85 | A |
| 580 | OE1 | GLN | 597 | 39.459 | -8.633 | 13.890 | 1.00 | 85.84 | A |
| 581 | NE2 | GLN | 597 | 40.908 | -7.838 | 15.420 | 1.00 | 85.44 | A |
| 582 | C | GLN | 597 | 41.068 | -2.959 | 15.177 | 1.00 | 77.62 | A |
| 583 | O | GLN | 597 | 40.239 | -2.546 | 15.991 | 1.00 | 77.11 | A |
| 584 | N | TYR | 598 | 42.335 | -2.626 | 15.233 | 1.00 | 78.31 | A |
| 585 | CA | TYR | 598 | 42.748 | -1.717 | 16.265 | 1.00 | 79.61 | A |
| 586 | CB | TYR | 598 | 44.192 | -1.982 | 16.624 | 1.00 | 82.08 | A |
| 587 | CG | TYR | 598 | 44.386 | -3.073 | 17.637 | 1.00 | 84.90 | A |
| 588 | CD1 | TYR | 598 | 43.519 | -4.168 | 17.717 | 1.00 | 85.68 | A |
| 589 | CE1 | TYR | 598 | 43.735 | -5.174 | 18.653 | 1.00 | 86.83 | A |
| 590 | CD2 | TYR | 598 | 45.457 | -3.014 | 18.504 | 1.00 | 86.14 | A |
| 591 | CE2 | TYR | 598 | 45.684 | -4.000 | 19.426 | 1.00 | 87.17 | A |
| 592 | CZ | TYR | 598 | 44.831 | -5.071 | 19.504 | 1.00 | 87.67 | A |
| 593 | OH | TYR | 598 | 45.103 | -6.015 | 20.464 | 1.00 | 89.91 | A |
| 594 | C | TYR | 598 | 42.607 | -0.302 | 15.767 | 1.00 | 78.74 | A |
| 595 | O | TYR | 598 | 42.158 | 0.582 | 16.490 | 1.00 | 79.40 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 596 | N | SER | 599 | 42.949 | -0.111 | 14.502 | 1.00 | 77.13 | A |
| 597 | CA | SER | 599 | 42.945 | 1.215 | 13.915 | 1.00 | 75.64 | A |
| 598 | CB | SER | 599 | 44.271 | 1.425 | 13.187 | 1.00 | 76.43 | A |
| 599 | OG | SER | 599 | 44.381 | 0.534 | 12.088 | 1.00 | 77.42 | A |
| 600 | C | SER | 599 | 41.816 | 1.623 | 12.978 | 1.00 | 73.69 | A |
| 601 | O | SER | 599 | 41.908 | 2.674 | 12.347 | 1.00 | 73.01 | A |
| 602 | N | TRP | 600 | 40.752 | 0.840 | 12.882 | 1.00 | 72.01 | A |
| 603 | CA | TRP | 600 | 39.696 | 1.228 | 11.962 | 1.00 | 70.02 | A |
| 604 | CB | TRP | 600 | 38.635 | 0.102 | 11.860 | 1.00 | 68.48 | A |
| 605 | CG | TRP | 600 | 37.721 | -0.054 | 13.036 | 1.00 | 67.90 | A |
| 606 | CD2 | TRP | 600 | 36.434 | 0.550 | 13.179 | 1.00 | 67.96 | A |
| 607 | CE2 | TRP | 600 | 35.960 | 0.229 | 14.472 | 1.00 | 67.86 | A |
| 608 | CE3 | TRP | 600 | 35.616 | 1.302 | 12.326 | 1.00 | 66.28 | A |
| 609 | CD1 | TRP | 600 | 37.984 | -0.689 | 14.220 | 1.00 | 67.57 | A |
| 610 | NE1 | TRP | 600 | 36.932 | -0.516 | 15.094 | 1.00 | 67.79 | A |
| 611 | CZ2 | TRP | 600 | 34.733 | 0.684 | 14.957 | 1.00 | 67.32 | A |
| 612 | CZ3 | TRP | 600 | 34.400 | 1.751 | 12.797 | 1.00 | 67.02 | A |
| 613 | CH2 | TRP | 600 | 33.959 | 1.426 | 14.098 | 1.00 | 67.80 | A |
| 614 | C | TRP | 600 | 39.062 | 2.615 | 12.252 | 1.00 | 69.26 | A |
| 615 | O | TRP | 600 | 38.704 | 3.343 | 11.321 | 1.00 | 68.40 | A |
| 616 | N | MET | 601 | 38.954 | 3.014 | 13.516 | 1.00 | 68.05 | A |
| 617 | CA | MET | 601 | 38.354 | 4.317 | 13.800 | 1.00 | 67.13 | A |
| 618 | CB | MET | 601 | 37.794 | 4.349 | 15.218 | 1.00 | 68.03 | A |
| 619 | CG | MET | 601 | 36.990 | 5.601 | 15.536 | 1.00 | 68.98 | A |
| 620 | SD | MET | 601 | 35.331 | 5.636 | 14.826 | 1.00 | 69.62 | A |
| 621 | CE | MET | 601 | 34.434 | 4.799 | 16.078 | 1.00 | 69.04 | A |
| 622 | C | MET | 601 | 39.378 | 5.439 | 13.613 | 1.00 | 66.32 | A |
| 623 | O | MET | 601 | 39.055 | 6.524 | 13.120 | 1.00 | 65.08 | A |
| 624 | N | SER | 602 | 40.612 | 5.174 | 14.016 | 1.00 | 65.58 | A |
| 625 | CA | SER | 602 | 41.668 | 6.157 | 13.858 | 1.00 | 64.76 | A |
| 626 | CB | SER | 602 | 43.018 | 5.519 | 14.185 | 1.00 | 65.19 | A |
| 627 | OG | SER | 602 | 44.010 | 6.500 | 14.419 | 1.00 | 66.76 | A |
| 628 | C | SER | 602 | 41.610 | 6.568 | 12.387 | 1.00 | 63.83 | A |
| 629 | O | SER | 602 | 41.581 | 7.753 | 12.058 | 1.00 | 63.81 | A |
| 630 | N | LEU | 603 | 41.546 | 5.564 | 11.512 | 1.00 | 63.22 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 631 | CA | LEU | 603 | 41.485 | 5.746 | 10.059 | 1.00 | 61.75 | A |
| 632 | CB | LEU | 603 | 41.555 | 4.393 | 9.364 | 1.00 | 60.69 | A |
| 633 | CG | LEU | 603 | 42.781 | 3.526 | 9.588 | 1.00 | 59.23 | A |
| 634 | CD1 | LEU | 603 | 42.470 | 2.124 | 9.100 | 1.00 | 59.85 | A |
| 635 | CD2 | LEU | 603 | 43.981 | 4.120 | 8.876 | 1.00 | 58.03 | A |
| 636 | C | LEU | 603 | 40.227 | 6.451 | 9.553 | 1.00 | 61.71 | A |
| 637 | O | LEU | 603 | 40.308 | 7.366 | 8.740 | 1.00 | 60.08 | A |
| 638 | N | MET | 604 | 39.071 | 5.974 | 10.005 | 1.00 | 62.13 | A |
| 639 | CA | MET | 604 | 37.786 | 6.525 | 9.603 | 1.00 | 62.89 | A |
| 640 | CB | MET | 604 | 36.645 | 5.671 | 10.186 | 1.00 | 64.15 | A |
| 641 | CG | MET | 604 | 35.998 | 4.681 | 9.194 | 1.00 | 64.86 | A |
| 642 | SD | MET | 604 | 37.123 | 4.017 | 7.921 | 1.00 | 69.00 | A |
| 643 | CE | MET | 604 | 36.833 | 5.103 | 6.468 | 1.00 | 67.97 | A |
| 644 | C | MET | 604 | 37.675 | 7.995 | 10.031 | 1.00 | 63.55 | A |
| 645 | O | MET | 604 | 37.309 | 8.854 | 9.228 | 1.00 | 64.56 | A |
| 646 | N | ALA | 605 | 38.025 | 8.294 | 11.283 | 1.00 | 63.18 | A |
| 647 | CA | ALA | 605 | 37.959 | 9.674 | 11.761 | 1.00 | 61.86 | A |
| 648 | CB | ALA | 605 | 38.021 | 9.719 | 13.291 | 1.00 | 61.77 | A |
| 649 | C | ALA | 605 | 39.052 | 10.556 | 11.155 | 1.00 | 61.28 | A |
| 650 | O | ALA | 605 | 38.801 | 11.720 | 10.857 | 1.00 | 63.43 | A |
| 651 | N | PHE | 606 | 40.256 | 10.033 | 10.963 | 1.00 | 59.86 | A |
| 652 | CA | PHE | 606 | 41.308 | 10.852 | 10.368 | 1.00 | 59.36 | A |
| 653 | CB | PHE | 606 | 42.662 | 10.124 | 10.405 | 1.00 | 57.40 | A |
| 654 | CG | PHE | 606 | 43.820 | 10.993 | 10.011 | 1.00 | 55.08 | A |
| 655 | CD1 | PHE | 606 | 44.153 | 12.110 | 10.770 | 1.00 | 53.58 | A |
| 656 | CD2 | PHE | 606 | 44.515 | 10.755 | 8.837 | 1.00 | 54.57 | A |
| 657 | CE1 | PHE | 606 | 45.162 | 12.979 | 10.367 | 1.00 | 53.02 | A |
| 658 | CE2 | PHE | 606 | 45.537 | 11.627 | 8.423 | 1.00 | 54.27 | A |
| 659 | CZ | PHE | 606 | 45.852 | 12.741 | 9.187 | 1.00 | 52.34 | A |
| 660 | C | PHE | 606 | 40.945 | 11.236 | 8.916 | 1.00 | 60.57 | A |
| 661 | O | PHE | 606 | 41.380 | 12.270 | 8.401 | 1.00 | 61.01 | A |
| 662 | N | ALA | 607 | 40.140 | 10.412 | 8.257 | 1.00 | 61.24 | A |
| 663 | CA | ALA | 607 | 39.734 | 10.706 | 6.890 | 1.00 | 60.99 | A |
| 664 | CB | ALA | 607 | 39.229 | 9.456 | 6.212 | 1.00 | 62.48 | A |
| 665 | C | ALA | 607 | 38.654 | 11.786 | 6.870 | 1.00 | 60.76 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 666 | O | ALA | 607 | 38.758 | 12.748 | 6.107 | 1.00 | 60.48 | A |
| 667 | N | LEU | 608 | 37.611 | 11.629 | 7.687 | 1.00 | 59.85 | A |
| 668 | CA | LEU | 608 | 36.561 | 12.644 | 7.738 | 1.00 | 60.59 | A |
| 669 | CB | LEU | 608 | 35.499 | 12.267 | 8.773 | 1.00 | 60.18 | A |
| 670 | CG | LEU | 608 | 34.414 | 13.234 | 9.264 | 1.00 | 61.39 | A |
| 671 | CD1 | LEU | 608 | 33.099 | 13.239 | 8.441 | 1.00 | 62.18 | A |
| 672 | CD2 | LEU | 608 | 34.119 | 12.747 | 10.668 | 1.00 | 60.55 | A |
| 673 | C | LEU | 608 | 37.247 | 13.966 | 8.093 | 1.00 | 61.63 | A |
| 674 | O | LEU | 608 | 37.031 | 14.972 | 7.416 | 1.00 | 61.28 | A |
| 675 | N | GLY | 609 | 38.095 | 13.974 | 9.125 | 1.00 | 61.90 | A |
| 676 | CA | GLY | 609 | 38.785 | 15.206 | 9.448 | 1.00 | 62.07 | A |
| 677 | C | GLY | 609 | 39.165 | 15.873 | 8.130 | 1.00 | 62.66 | A |
| 678 | O | GLY | 609 | 38.727 | 16.974 | 7.810 | 1.00 | 63.76 | A |
| 679 | N | TRP | 610 | 39.955 | 15.162 | 7.336 | 1.00 | 62.36 | A |
| 680 | CA | TRP | 610 | 40.419 | 15.637 | 6.040 | 1.00 | 60.84 | A |
| 681 | CB | TRP | 610 | 41.207 | 14.528 | 5.364 | 1.00 | 57.02 | A |
| 682 | CG | TRP | 610 | 41.865 | 14.975 | 4.130 | 1.00 | 52.45 | A |
| 683 | CD2 | TRP | 610 | 43.040 | 15.777 | 4.054 | 1.00 | 50.06 | A |
| 684 | CE2 | TRP | 610 | 43.335 | 15.952 | 2.684 | 1.00 | 48.16 | A |
| 685 | CE3 | TRP | 610 | 43.842 | 16.405 | 5.019 | 1.00 | 49.35 | A |
| 686 | CD1 | TRP | 610 | 41.511 | 14.679 | 2.839 | 1.00 | 50.18 | A |
| 687 | NE1 | TRP | 610 | 42.404 | 15.259 | 1.967 | 1.00 | 48.49 | A |
| 688 | CZ2 | TRP | 610 | 44.446 | 16.684 | 2.250 | 1.00 | 49.59 | A |
| 689 | CZ3 | TRP | 610 | 44.948 | 17.142 | 4.592 | 1.00 | 49.89 | A |
| 690 | CH2 | TRP | 610 | 45.227 | 17.292 | 3.210 | 1.00 | 50.85 | A |
| 691 | C | TRP | 610 | 39.353 | 16.134 | 5.059 | 1.00 | 61.87 | A |
| 692 | O | TRP | 610 | 39.468 | 17.219 | 4.504 | 1.00 | 62.11 | A |
| 693 | N | ARG | 611 | 38.337 | 15.321 | 4.808 | 1.00 | 63.21 | A |
| 694 | CA | ARG | 611 | 37.300 | 15.718 | 3.873 | 1.00 | 64.73 | A |
| 695 | CB | ARG | 611 | 36.250 | 14.611 | 3.737 | 1.00 | 65.43 | A |
| 696 | CG | ARG | 611 | 36.749 | 13.336 | 3.066 | 1.00 | 64.87 | A |
| 697 | CD | ARG | 611 | 35.582 | 12.508 | 2.553 | 1.00 | 64.94 | A |
| 698 | NE | ARG | 611 | 34.683 | 12.093 | 3.628 | 1.00 | 66.13 | A |
| 699 | CZ | ARG | 611 | 35.034 | 11.266 | 4.605 | 1.00 | 66.82 | A |
| 700 | NH1 | ARG | 611 | 36.270 | 10.765 | 4.637 | 1.00 | 67.98 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 701 | NH2 | ARG | 611 | 34.147 | 10.908 | 5.532 | 1.00 | 64.34 | A |
| 702 | C | ARG | 611 | 36.646 | 16.998 | 4.379 | 1.00 | 66.30 | A |
| 703 | O | ARG | 611 | 36.212 | 17.848 | 3.596 | 1.00 | 67.09 | A |
| 704 | N | SER | 612 | 36.604 | 17.134 | 5.696 | 1.00 | 67.15 | A |
| 705 | CA | SER | 612 | 36.007 | 18.294 | 6.336 | 1.00 | 67.98 | A |
| 706 | CB | SER | 612 | 35.798 | 18.004 | 7.807 | 1.00 | 66.31 | A |
| 707 | OG | SER | 612 | 34.810 | 17.005 | 7.922 | 1.00 | 66.23 | A |
| 708 | C | SER | 612 | 36.830 | 19.548 | 6.183 | 1.00 | 69.48 | A |
| 709 | O | SER | 612 | 36.322 | 20.611 | 5.834 | 1.00 | 69.46 | A |
| 710 | N | TYR | 613 | 38.107 | 19.429 | 6.473 | 1.00 | 71.12 | A |
| 711 | CA | TYR | 613 | 38.963 | 20.571 | 6.348 | 1.00 | 74.12 | A |
| 712 | CB | TYR | 613 | 40.343 | 20.137 | 6.799 | 1.00 | 72.89 | A |
| 713 | CG | TYR | 613 | 41.520 | 20.770 | 6.137 | 1.00 | 72.72 | A |
| 714 | CD1 | TYR | 613 | 41.827 | 22.116 | 6.316 | 1.00 | 71.49 | A |
| 715 | CE1 | TYR | 613 | 43.039 | 22.638 | 5.861 | 1.00 | 71.14 | A |
| 716 | CD2 | TYR | 613 | 42.437 | 19.967 | 5.467 | 1.00 | 73.13 | A |
| 717 | CE2 | TYR | 613 | 43.636 | 20.472 | 5.009 | 1.00 | 73.12 | A |
| 718 | CZ | TYR | 613 | 43.941 | 21.799 | 5.214 | 1.00 | 71.86 | A |
| 719 | OH | TYR | 613 | 45.173 | 22.250 | 4.807 | 1.00 | 71.29 | A |
| 720 | C | TYR | 613 | 38.914 | 21.029 | 4.893 | 1.00 | 76.62 | A |
| 721 | O | TYR | 613 | 38.594 | 22.176 | 4.598 | 1.00 | 77.91 | A |
| 722 | N | ARG | 614 | 39.149 | 20.108 | 3.982 | 1.00 | 79.53 | A |
| 723 | CA | ARG | 614 | 39.169 | 20.433 | 2.563 | 1.00 | 82.42 | A |
| 724 | CB | ARG | 614 | 39.712 | 19.213 | 1.827 | 1.00 | 83.40 | A |
| 725 | CG | ARG | 614 | 41.000 | 18.718 | 2.460 | 1.00 | 84.51 | A |
| 726 | CD | ARG | 614 | 42.108 | 19.638 | 2.084 | 1.00 | 84.98 | A |
| 727 | NE | ARG | 614 | 42.150 | 19.612 | 0.637 | 1.00 | 87.19 | A |
| 728 | CZ | ARG | 614 | 43.238 | 19.787 | -0.094 | 1.00 | 89.15 | A |
| 729 | NH1 | ARG | 614 | 44.414 | 20.020 | 0.490 | 1.00 | 89.01 | A |
| 730 | NH2 | ARG | 614 | 43.150 | 19.678 | -1.414 | 1.00 | 89.92 | A |
| 731 | C | ARG | 614 | 37.866 | 20.913 | 1.904 | 1.00 | 83.98 | A |
| 732 | O | ARG | 614 | 37.896 | 21.448 | 0.794 | 1.00 | 83.82 | A |
| 733 | N | GLN | 615 | 36.731 | 20.766 | 2.586 | 1.00 | 85.72 | A |
| 734 | CA | GLN | 615 | 35.446 | 21.126 | 1.990 | 1.00 | 87.80 | A |
| 735 | CB | GLN | 615 | 34.641 | 19.833 | 1.811 | 1.00 | 88.02 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 736 | CG | GLN | 615 | 33.302 | 19.930 | 1.105 | 1.00 | 89.29 | A |
| 737 | CD | GLN | 615 | 32.464 | 18.668 | 1.302 | 1.00 | 90.00 | A |
| 738 | OE1 | GLN | 615 | 31.230 | 18.697 | 1.191 | 1.00 | 89.30 | A |
| 739 | NE2 | GLN | 615 | 33.131 | 17.549 | 1.598 | 1.00 | 90.17 | A |
| 740 | C | GLN | 615 | 34.621 | 22.153 | 2.763 | 1.00 | 89.16 | A |
| 741 | O | GLN | 615 | 33.419 | 22.280 | 2.541 | 1.00 | 89.38 | A |
| 742 | N | SER | 616 | 35.259 | 22.894 | 3.663 | 1.00 | 90.80 | A |
| 743 | CA | SER | 616 | 34.543 | 23.885 | 4.457 | 1.00 | 92.99 | A |
| 744 | CB | SER | 616 | 33.235 | 23.296 | 5.016 | 1.00 | 92.42 | A |
| 745 | OG | SER | 616 | 33.445 | 22.457 | 6.143 | 1.00 | 90.39 | A |
| 746 | C | SER | 616 | 35.431 | 24.338 | 5.603 | 1.00 | 95.02 | A |
| 747 | O | SER | 616 | 34.953 | 24.572 | 6.711 | 1.00 | 95.57 | A |
| 748 | N | SER | 617 | 36.723 | 24.445 | 5.333 | 1.00 | 96.67 | A |
| 749 | CA | SER | 617 | 37.700 | 24.867 | 6.337 | 1.00 | 98.08 | A |
| 750 | CB | SER | 617 | 37.677 | 26.400 | 6.509 | 1.00 | 98.21 | A |
| 751 | OG | SER | 617 | 37.701 | 27.099 | 5.276 | 1.00 | 98.06 | A |
| 752 | C | SER | 617 | 37.510 | 24.244 | 7.730 | 1.00 | 98.69 | A |
| 753 | O | SER | 617 | 37.895 | 24.856 | 8.724 | 1.00 | 99.28 | A |
| 754 | N | ALA | 618 | 36.912 | 23.060 | 7.831 | 1.00 | 98.75 | A |
| 755 | CA | ALA | 618 | 36.743 | 22.448 | 9.156 | 1.00 | 99.29 | A |
| 756 | CB | ALA | 618 | 38.080 | 22.494 | 9.915 | 1.00 | 98.81 | A |
| 757 | C | ALA | 618 | 35.631 | 23.069 | 10.026 | 1.00 | 99.45 | A |
| 758 | O | ALA | 618 | 35.706 | 23.014 | 11.264 | 1.00 | 99.79 | A |
| 759 | N | ASN | 619 | 34.616 | 23.645 | 9.378 | 1.00 | 98.51 | A |
| 760 | CA | ASN | 619 | 33.466 | 24.275 | 10.044 | 1.00 | 97.30 | A |
| 761 | CB | ASN | 619 | 32.907 | 25.407 | 9.168 | 1.00 | 97.72 | A |
| 762 | CG | ASN | 619 | 33.993 | 26.254 | 8.533 | 1.00 | 97.81 | A |
| 763 | OD1 | ASN | 619 | 33.766 | 26.909 | 7.510 | 1.00 | 97.90 | A |
| 764 | ND2 | ASN | 619 | 35.175 | 26.254 | 9.137 | 1.00 | 97.55 | A |
| 765 | C | ASN | 619 | 32.370 | 23.216 | 10.191 | 1.00 | 96.23 | A |
| 766 | O | ASN | 619 | 31.673 | 23.120 | 11.208 | 1.00 | 95.29 | A |
| 767 | N | LEU | 620 | 32.238 | 22.430 | 9.127 | 1.00 | 94.73 | A |
| 768 | CA | LEU | 620 | 31.260 | 21.363 | 9.020 | 1.00 | 92.84 | A |
| 769 | CB | LEU | 620 | 30.476 | 21.547 | 7.705 | 1.00 | 92.98 | A |
| 770 | CG | LEU | 620 | 29.802 | 22.919 | 7.492 | 1.00 | 92.80 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 771 | CD1 | LEU | 620 | 28.936 | 22.943 | 6.226 | 1.00 | 92.07 | A |
| 772 | CD2 | LEU | 620 | 28.939 | 23.220 | 8.704 | 1.00 | 92.73 | A |
| 773 | C | LEU | 620 | 31.983 | 19.998 | 9.044 | 1.00 | 91.47 | A |
| 774 | O | LEU | 620 | 33.207 | 19.941 | 8.910 | 1.00 | 91.02 | A |
| 775 | N | LEU | 621 | 31.229 | 18.918 | 9.256 | 1.00 | 89.67 | A |
| 776 | CA | LEU | 621 | 31.757 | 17.547 | 9.256 | 1.00 | 87.87 | A |
| 777 | CB | LEU | 621 | 31.206 | 16.722 | 10.430 | 1.00 | 87.01 | A |
| 778 | CG | LEU | 621 | 31.886 | 16.853 | 11.791 | 1.00 | 86.70 | A |
| 779 | CD1 | LEU | 621 | 31.304 | 15.830 | 12.766 | 1.00 | 86.15 | A |
| 780 | CD2 | LEU | 621 | 33.378 | 16.639 | 11.623 | 1.00 | 85.78 | A |
| 781 | C | LEU | 621 | 31.230 | 16.961 | 7.962 | 1.00 | 86.90 | A |
| 782 | O | LEU | 621 | 30.029 | 16.809 | 7.805 | 1.00 | 86.82 | A |
| 783 | N | CYS | 622 | 32.112 | 16.619 | 7.032 | 1.00 | 85.92 | A |
| 784 | CA | CYS | 622 | 31.639 | 16.104 | 5.753 | 1.00 | 85.59 | A |
| 785 | CB | CYS | 622 | 32.449 | 16.744 | 4.629 | 1.00 | 85.97 | A |
| 786 | SG | CYS | 622 | 32.515 | 18.552 | 4.739 | 1.00 | 88.73 | A |
| 787 | C | CYS | 622 | 31.669 | 14.596 | 5.612 | 1.00 | 84.70 | A |
| 788 | O | CYS | 622 | 32.503 | 14.057 | 4.892 | 1.00 | 85.08 | A |
| 789 | N | PHE | 623 | 30.751 | 13.902 | 6.264 | 1.00 | 83.91 | A |
| 790 | CA | PHE | 623 | 30.761 | 12.459 | 6.154 | 1.00 | 83.55 | A |
| 791 | CB | PHE | 623 | 29.637 | 11.864 | 7.015 | 1.00 | 81.77 | A |
| 792 | CG | PHE | 623 | 29.897 | 11.980 | 8.515 | 1.00 | 80.87 | A |
| 793 | CD1 | PHE | 623 | 29.644 | 13.162 | 9.210 | 1.00 | 80.74 | A |
| 794 | CD2 | PHE | 623 | 30.380 | 10.890 | 9.235 | 1.00 | 80.88 | A |
| 795 | CE1 | PHE | 623 | 29.870 | 13.256 | 10.601 | 1.00 | 80.30 | A |
| 796 | CE2 | PHE | 623 | 30.612 | 10.979 | 10.615 | 1.00 | 80.14 | A |
| 797 | CZ | PHE | 623 | 30.350 | 12.159 | 11.297 | 1.00 | 80.61 | A |
| 798 | C | PHE | 623 | 30.754 | 11.990 | 4.674 | 1.00 | 84.77 | A |
| 799 | O | PHE | 623 | 31.382 | 10.986 | 4.343 | 1.00 | 85.60 | A |
| 800 | N | ALA | 624 | 30.089 | 12.730 | 3.790 | 1.00 | 85.86 | A |
| 801 | CA | ALA | 624 | 30.081 | 12.431 | 2.348 | 1.00 | 87.32 | A |
| 802 | CB | ALA | 624 | 28.888 | 11.576 | 1.985 | 1.00 | 86.51 | A |
| 803 | C | ALA | 624 | 29.974 | 13.821 | 1.713 | 1.00 | 88.99 | A |
| 804 | O | ALA | 624 | 29.353 | 14.699 | 2.307 | 1.00 | 89.36 | A |
| 805 | N | PRO | 625 | 30.590 | 14.071 | 0.532 | 1.00 | 90.51 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 806 | CD | PRO | 625 | 31.601 | 13.375 | -0.288 | 1.00 | 91.20 | A |
| 807 | CA | PRO | 625 | 30.404 | 15.451 | 0.055 | 1.00 | 91.39 | A |
| 808 | CB | PRO | 625 | 31.248 | 15.492 | -1.223 | 1.00 | 91.01 | A |
| 809 | CG | PRO | 625 | 32.366 | 14.540 | -0.909 | 1.00 | 90.40 | A |
| 810 | C | PRO | 625 | 28.929 | 15.814 | -0.180 | 1.00 | 91.81 | A |
| 811 | O | PRO | 625 | 28.590 | 16.979 | -0.420 | 1.00 | 91.56 | A |
| 812 | N | ASP | 626 | 28.071 | 14.797 | -0.077 | 1.00 | 91.89 | A |
| 813 | CA | ASP | 626 | 26.623 | 14.915 | -0.260 | 1.00 | 91.87 | A |
| 814 | CB | ASP | 626 | 26.103 | 13.750 | -1.069 | 1.00 | 93.67 | A |
| 815 | CG | ASP | 626 | 26.079 | 12.476 | -0.245 | 1.00 | 95.47 | A |
| 816 | OD1 | ASP | 626 | 27.157 | 12.118 | 0.255 | 1.00 | 97.06 | A |
| 817 | OD2 | ASP | 626 | 25.008 | 11.853 | -0.069 | 1.00 | 95.89 | A |
| 818 | C | ASP | 626 | 25.877 | 14.820 | 1.071 | 1.00 | 91.25 | A |
| 819 | O | ASP | 626 | 24.658 | 14.974 | 1.104 | 1.00 | 92.29 | A |
| 820 | N | LEU | 627 | 26.581 | 14.500 | 2.148 | 1.00 | 90.06 | A |
| 821 | CA | LEU | 627 | 25.926 | 14.362 | 3.447 | 1.00 | 88.48 | A |
| 822 | CB | LEU | 627 | 25.801 | 12.876 | 3.806 | 1.00 | 86.93 | A |
| 823 | CG | LEU | 627 | 25.003 | 12.516 | 5.059 | 1.00 | 86.55 | A |
| 824 | CD1 | LEU | 627 | 25.598 | 13.155 | 6.301 | 1.00 | 86.49 | A |
| 825 | CD2 | LEU | 627 | 23.588 | 12.986 | 4.868 | 1.00 | 86.46 | A |
| 826 | C | LEU | 627 | 26.721 | 15.104 | 4.520 | 1.00 | 87.74 | A |
| 827 | O | LEU | 627 | 27.639 | 14.539 | 5.114 | 1.00 | 88.20 | A |
| 828 | N | ILE | 628 | 26.352 | 16.356 | 4.776 | 1.00 | 86.51 | A |
| 829 | CA | ILE | 628 | 27.046 | 17.208 | 5.752 | 1.00 | 84.81 | A |
| 830 | CB | ILE | 628 | 27.296 | 18.638 | 5.173 | 1.00 | 83.92 | A |
| 831 | CG2 | ILE | 628 | 27.963 | 19.516 | 6.212 | 1.00 | 83.50 | A |
| 832 | CG1 | ILE | 628 | 28.228 | 18.585 | 3.964 | 1.00 | 83.63 | A |
| 833 | CD1 | ILE | 628 | 27.752 | 17.707 | 2.855 | 1.00 | 83.66 | A |
| 834 | C | ILE | 628 | 26.327 | 17.405 | 7.091 | 1.00 | 84.24 | A |
| 835 | O | ILE | 628 | 25.099 | 17.397 | 7.167 | 1.00 | 84.45 | A |
| 836 | N | ILE | 629 | 27.097 | 17.566 | 8.161 | 1.00 | 83.48 | A |
| 837 | CA | ILE | 629 | 26.461 | 17.844 | 9.428 | 1.00 | 82.78 | A |
| 838 | CB | ILE | 629 | 26.999 | 17.048 | 10.607 | 1.00 | 81.63 | A |
| 839 | CG2 | ILE | 629 | 26.599 | 17.734 | 11.903 | 1.00 | 79.75 | A |
| 840 | CG1 | ILE | 629 | 26.470 | 15.604 | 10.547 | 1.00 | 82.25 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 841 | CD1 | ILE | 629 | 25.110 | 15.415 | 9.861 | 1.00 | 81.23 | A |
| 842 | C | ILE | 629 | 26.697 | 19.304 | 9.659 | 1.00 | 83.83 | A |
| 843 | O | ILE | 629 | 27.797 | 19.755 | 9.985 | 1.00 | 83.14 | A |
| 844 | N | ASN | 630 | 25.607 | 20.021 | 9.437 | 1.00 | 85.68 | A |
| 845 | CA | ASN | 630 | 25.502 | 21.453 | 9.547 | 1.00 | 86.72 | A |
| 846 | CB | ASN | 630 | 24.607 | 21.962 | 8.456 | 1.00 | 86.20 | A |
| 847 | CG | ASN | 630 | 23.272 | 21.261 | 8.477 | 1.00 | 85.18 | A |
| 848 | OD1 | ASN | 630 | 22.946 | 20.552 | 9.426 | 1.00 | 84.86 | A |
| 849 | ND2 | ASN | 630 | 22.492 | 21.447 | 7.437 | 1.00 | 84.97 | A |
| 850 | C | ASN | 630 | 24.868 | 21.858 | 10.857 | 1.00 | 88.22 | A |
| 851 | O | ASN | 630 | 24.323 | 21.039 | 11.613 | 1.00 | 87.63 | A |
| 852 | N | GLU | 631 | 24.872 | 23.179 | 11.018 | 1.00 | 90.31 | A |
| 853 | CA | GLU | 631 | 24.398 | 23.891 | 12.187 | 1.00 | 91.48 | A |
| 854 | CB | GLU | 631 | 24.382 | 25.385 | 11.921 | 1.00 | 93.00 | A |
| 855 | CG | GLU | 631 | 23.793 | 26.104 | 13.115 | 1.00 | 96.45 | A |
| 856 | CD | GLU | 631 | 23.464 | 27.561 | 12.874 | 1.00 | 98.56 | A |
| 857 | OE1 | GLU | 631 | 22.255 | 27.889 | 12.773 | 1.00 | 98.65 | A |
| 858 | OE2 | GLU | 631 | 24.414 | 28.374 | 12.797 | 1.00 | 99.30 | A |
| 859 | C | GLU | 631 | 23.060 | 23.541 | 12.736 | 1.00 | 91.50 | A |
| 860 | O | GLU | 631 | 22.827 | 23.560 | 13.943 | 1.00 | 91.38 | A |
| 861 | N | GLN | 632 | 22.164 | 23.270 | 11.820 | 1.00 | 91.77 | A |
| 862 | CA | GLN | 632 | 20.832 | 22.950 | 12.183 | 1.00 | 92.07 | A |
| 863 | CB | GLN | 632 | 19.965 | 23.198 | 10.992 | 1.00 | 93.94 | A |
| 864 | CG | GLN | 632 | 19.286 | 24.573 | 10.860 | 1.00 | 97.21 | A |
| 865 | CD | GLN | 632 | 19.518 | 25.646 | 11.945 | 1.00 | 98.67 | A |
| 866 | OE1 | GLN | 632 | 19.142 | 25.505 | 13.107 | 1.00 | 99.98 | A |
| 867 | NE2 | GLN | 632 | 20.095 | 26.764 | 11.526 | 1.00 | 99.13 | A |
| 868 | C | GLN | 632 | 20.837 | 21.487 | 12.542 | 1.00 | 90.67 | A |
| 869 | O | GLN | 632 | 20.097 | 21.044 | 13.410 | 1.00 | 91.24 | A |
| 870 | N | ARG | 633 | 21.686 | 20.733 | 11.873 | 1.00 | 89.18 | A |
| 871 | CA | ARG | 633 | 21.775 | 19.313 | 12.161 | 1.00 | 87.17 | A |
| 872 | CB | ARG | 633 | 22.453 | 18.598 | 11.021 | 1.00 | 86.70 | A |
| 873 | CG | ARG | 633 | 21.431 | 17.895 | 10.184 | 1.00 | 84.53 | A |
| 874 | CD | ARG | 633 | 22.020 | 17.447 | 8.896 | 1.00 | 83.94 | A |
| 875 | NE | ARG | 633 | 21.077 | 16.992 | 7.877 | 1.00 | 82.31 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 876 | CZ | ARG | 633 | 21.490 | 16.771 | 6.637 | 1.00 | 80.78 | A |
| 877 | NH1 | ARG | 633 | 22.767 | 16.982 | 6.379 | 1.00 | 81.48 | A |
| 878 | NH2 | ARG | 633 | 20.673 | 16.342 | 5.682 | 1.00 | 78.84 | A |
| 879 | C | ARG | 633 | 22.586 | 19.091 | 13.403 | 1.00 | 86.42 | A |
| 880 | O | ARG | 633 | 22.417 | 18.149 | 14.163 | 1.00 | 86.50 | A |
| 881 | N | MET | 634 | 23.508 | 19.990 | 13.583 | 1.00 | 85.41 | A |
| 882 | CA | MET | 634 | 24.422 | 19.923 | 14.676 | 1.00 | 85.16 | A |
| 883 | CB | MET | 634 | 25.333 | 21.071 | 14.564 | 1.00 | 83.80 | A |
| 884 | CG | MET | 634 | 26.532 | 20.722 | 15.242 | 1.00 | 83.67 | A |
| 885 | SD | MET | 634 | 26.813 | 18.971 | 14.989 | 1.00 | 83.03 | A |
| 886 | CE | MET | 634 | 28.456 | 18.959 | 15.223 | 1.00 | 81.55 | A |
| 887 | C | MET | 634 | 23.750 | 20.066 | 15.958 | 1.00 | 85.63 | A |
| 888 | O | MET | 634 | 24.225 | 19.776 | 17.058 | 1.00 | 85.09 | A |
| 889 | N | THR | 635 | 22.601 | 20.597 | 15.781 | 1.00 | 87.23 | A |
| 890 | CA | THR | 635 | 21.881 | 20.899 | 16.896 | 1.00 | 88.52 | A |
| 891 | CB | THR | 635 | 21.350 | 22.196 | 16.587 | 1.00 | 87.94 | A |
| 892 | OG1 | THR | 635 | 20.215 | 22.470 | 17.380 | 1.00 | 89.21 | A |
| 893 | CG2 | THR | 635 | 20.985 | 22.182 | 15.195 | 1.00 | 88.12 | A |
| 894 | C | THR | 635 | 20.853 | 19.841 | 17.202 | 1.00 | 89.56 | A |
| 895 | O | THR | 635 | 19.970 | 20.116 | 17.985 | 1.00 | 90.59 | A |
| 896 | N | LEU | 636 | 20.892 | 18.633 | 16.625 | 1.00 | 89.94 | A |
| 897 | CA | LEU | 636 | 19.861 | 17.719 | 17.107 | 1.00 | 90.42 | A |
| 898 | CB | LEU | 636 | 19.555 | 16.490 | 16.236 | 1.00 | 91.24 | A |
| 899 | CG | LEU | 636 | 19.136 | 16.816 | 14.826 | 1.00 | 92.26 | A |
| 900 | CD1 | LEU | 636 | 20.324 | 17.384 | 14.235 | 1.00 | 92.82 | A |
| 901 | CD2 | LEU | 636 | 18.739 | 15.622 | 14.013 | 1.00 | 92.86 | A |
| 902 | C | LEU | 636 | 20.401 | 17.252 | 18.416 | 1.00 | 90.58 | A |
| 903 | O | LEU | 636 | 21.542 | 17.553 | 18.802 | 1.00 | 90.48 | A |
| 904 | N | PRO | 637 | 19.594 | 16.478 | 19.111 | 1.00 | 90.59 | A |
| 905 | CD | PRO | 637 | 18.305 | 15.875 | 18.738 | 1.00 | 90.32 | A |
| 906 | CA | PRO | 637 | 20.019 | 15.991 | 20.398 | 1.00 | 91.07 | A |
| 907 | CB | PRO | 637 | 19.091 | 14.825 | 20.639 | 1.00 | 90.73 | A |
| 908 | CG | PRO | 637 | 17.847 | 15.305 | 20.031 | 1.00 | 90.37 | A |
| 909 | C | PRO | 637 | 21.425 | 15.600 | 20.619 | 1.00 | 91.70 | A |
| 910 | O | PRO | 637 | 22.349 | 16.393 | 20.827 | 1.00 | 93.14 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 911 | N | CYS | 638 | 21.589 | 14.316 | 20.539 | 1.00 | 91.74 | A |
| 912 | CA | CYS | 638 | 22.857 | 13.860 | 20.917 | 1.00 | 91.86 | A |
| 913 | CB | CYS | 638 | 22.672 | 12.435 | 21.382 | 1.00 | 91.44 | A |
| 914 | SG | CYS | 638 | 22.362 | 12.383 | 23.136 | 1.00 | 92.33 | A |
| 915 | C | CYS | 638 | 24.004 | 14.008 | 19.970 | 1.00 | 91.43 | A |
| 916 | O | CYS | 638 | 24.977 | 13.305 | 20.113 | 1.00 | 92.21 | A |
| 917 | N | MET | 639 | 23.955 | 14.977 | 19.074 | 1.00 | 90.28 | A |
| 918 | CA | MET | 639 | 25.003 | 15.076 | 18.076 | 1.00 | 89.06 | A |
| 919 | CB | MET | 639 | 24.474 | 15.815 | 16.875 | 1.00 | 89.67 | A |
| 920 | CG | MET | 639 | 25.057 | 15.363 | 15.576 | 1.00 | 89.96 | A |
| 921 | SD | MET | 639 | 23.803 | 14.471 | 14.669 | 1.00 | 91.78 | A |
| 922 | CE | MET | 639 | 24.470 | 12.873 | 14.665 | 1.00 | 91.55 | A |
| 923 | C | MET | 639 | 26.280 | 15.742 | 18.432 | 1.00 | 88.30 | A |
| 924 | O | MET | 639 | 27.390 | 15.236 | 18.249 | 1.00 | 88.54 | A |
| 925 | N | TYR | 640 | 26.089 | 16.955 | 18.882 | 1.00 | 87.19 | A |
| 926 | CA | TYR | 640 | 27.202 | 17.757 | 19.177 | 1.00 | 85.96 | A |
| 927 | CB | TYR | 640 | 26.724 | 19.072 | 19.707 | 1.00 | 83.25 | A |
| 928 | CG | TYR | 640 | 27.903 | 19.924 | 19.724 | 1.00 | 81.17 | A |
| 929 | CD1 | TYR | 640 | 28.386 | 20.449 | 18.536 | 1.00 | 80.77 | A |
| 930 | CE1 | TYR | 640 | 29.621 | 21.033 | 18.475 | 1.00 | 79.85 | A |
| 931 | CD2 | TYR | 640 | 28.685 | 20.016 | 20.866 | 1.00 | 79.86 | A |
| 932 | CE2 | TYR | 640 | 29.923 | 20.591 | 20.822 | 1.00 | 79.30 | A |
| 933 | CZ | TYR | 640 | 30.388 | 21.095 | 19.622 | 1.00 | 79.94 | A |
| 934 | OH | TYR | 640 | 31.638 | 21.656 | 19.564 | 1.00 | 79.45 | A |
| 935 | C | TYR | 640 | 28.230 | 17.187 | 20.119 | 1.00 | 86.91 | A |
| 936 | O | TYR | 640 | 29.423 | 17.431 | 19.984 | 1.00 | 87.67 | A |
| 937 | N | ASP | 641 | 27.773 | 16.429 | 21.093 | 1.00 | 88.23 | A |
| 938 | CA | ASP | 641 | 28.693 | 15.872 | 22.061 | 1.00 | 89.33 | A |
| 939 | CB | ASP | 641 | 27.933 | 15.217 | 23.184 | 1.00 | 91.25 | A |
| 940 | CG | ASP | 641 | 27.827 | 16.076 | 24.402 | 1.00 | 92.97 | A |
| 941 | OD1 | ASP | 641 | 28.852 | 16.625 | 24.886 | 1.00 | 93.24 | A |
| 942 | OD2 | ASP | 641 | 26.684 | 16.169 | 24.889 | 1.00 | 93.94 | A |
| 943 | C | ASP | 641 | 29.574 | 14.812 | 21.474 | 1.00 | 89.35 | A |
| 944 | O | ASP | 641 | 30.653 | 14.546 | 21.979 | 1.00 | 89.26 | A |
| 945 | N | GLN | 642 | 29.045 | 14.178 | 20.434 | 1.00 | 89.50 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 946 | CA | GLN | 642 | 29.666 | 13.099 | 19.688 | 1.00 | 89.13 | A |
| 947 | CB | GLN | 642 | 28.526 | 12.237 | 19.044 | 1.00 | 90.27 | A |
| 948 | CG | GLN | 642 | 27.962 | 10.822 | 19.594 | 1.00 | 91.79 | A |
| 949 | CD | GLN | 642 | 26.681 | 10.417 | 18.792 | 1.00 | 93.88 | A |
| 950 | OE1 | GLN | 642 | 26.221 | 9.266 | 18.765 | 1.00 | 94.69 | A |
| 951 | NE2 | GLN | 642 | 26.098 | 11.421 | 18.152 | 1.00 | 94.27 | A |
| 952 | C | GLN | 642 | 30.568 | 13.716 | 18.613 | 1.00 | 88.31 | A |
| 953 | O | GLN | 642 | 31.765 | 13.430 | 18.522 | 1.00 | 87.84 | A |
| 954 | N | CYS | 643 | 29.961 | 14.599 | 17.822 | 1.00 | 87.09 | A |
| 955 | CA | CYS | 643 | 30.607 | 15.296 | 16.691 | 1.00 | 86.13 | A |
| 956 | CB | CYS | 643 | 29.579 | 16.175 | 15.965 | 1.00 | 85.59 | A |
| 957 | SG | CYS | 643 | 28.434 | 15.246 | 14.937 | 1.00 | 86.84 | A |
| 958 | C | CYS | 643 | 31.835 | 16.167 | 16.978 | 1.00 | 85.11 | A |
| 959 | O | CYS | 643 | 32.634 | 16.451 | 16.086 | 1.00 | 85.48 | A |
| 960 | N | LYS | 644 | 31.978 | 16.570 | 18.224 | 1.00 | 83.90 | A |
| 961 | CA | LYS | 644 | 33.050 | 17.445 | 18.689 | 1.00 | 82.99 | A |
| 962 | CB | LYS | 644 | 32.847 | 17.650 | 20.164 | 1.00 | 83.94 | A |
| 963 | CG | LYS | 644 | 33.903 | 18.465 | 20.828 | 1.00 | 84.87 | A |
| 964 | CD | LYS | 644 | 33.751 | 18.350 | 22.330 | 1.00 | 85.64 | A |
| 965 | CE | LYS | 644 | 32.292 | 18.434 | 22.762 | 1.00 | 86.34 | A |
| 966 | NZ | LYS | 644 | 32.193 | 18.431 | 24.245 | 1.00 | 85.91 | A |
| 967 | C | LYS | 644 | 34.451 | 16.938 | 18.505 | 1.00 | 82.08 | A |
| 968 | O | LYS | 644 | 35.424 | 17.654 | 18.224 | 1.00 | 81.31 | A |
| 969 | N | HIS | 645 | 34.526 | 15.664 | 18.775 | 1.00 | 81.67 | A |
| 970 | CA | HIS | 645 | 35.747 | 14.987 | 18.710 | 1.00 | 81.48 | A |
| 971 | CB | HIS | 645 | 35.510 | 13.663 | 19.317 | 1.00 | 83.43 | A |
| 972 | CG | HIS | 645 | 35.703 | 13.776 | 20.783 | 1.00 | 85.32 | A |
| 973 | CD2 | HIS | 645 | 36.806 | 14.279 | 21.392 | 1.00 | 86.28 | A |
| 974 | ND1 | HIS | 645 | 34.689 | 13.785 | 21.664 | 1.00 | 86.22 | A |
| 975 | CE1 | HIS | 645 | 35.135 | 14.319 | 22.824 | 1.00 | 87.26 | A |
| 976 | NE2 | HIS | 645 | 36.392 | 14.616 | 22.663 | 1.00 | 87.68 | A |
| 977 | C | HIS | 645 | 36.286 | 14.967 | 17.372 | 1.00 | 80.40 | A |
| 978 | O | HIS | 645 | 37.442 | 15.286 | 17.131 | 1.00 | 79.49 | A |
| 979 | N | MET | 646 | 35.403 | 14.670 | 16.460 | 1.00 | 79.68 | A |
| 980 | CA | MET | 646 | 35.847 | 14.655 | 15.125 | 1.00 | 78.11 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 981 | CB | MET | 646 | 34.794 | 14.004 | 14.287 | 1.00 | 78.69 | A |
| 982 | CG | MET | 646 | 34.894 | 12.531 | 14.452 | 1.00 | 78.50 | A |
| 983 | SD | MET | 646 | 33.445 | 11.861 | 15.209 | 1.00 | 80.06 | A |
| 984 | CE | MET | 646 | 32.393 | 11.981 | 13.892 | 1.00 | 82.37 | A |
| 985 | C | MET | 646 | 36.199 | 16.020 | 14.595 | 1.00 | 76.66 | A |
| 986 | O | MET | 646 | 37.186 | 16.151 | 13.878 | 1.00 | 76.52 | A |
| 987 | N | LEU | 647 | 35.414 | 17.042 | 14.944 | 1.00 | 75.12 | A |
| 988 | CA | LEU | 647 | 35.692 | 18.380 | 14.416 | 1.00 | 74.23 | A |
| 989 | CB | LEU | 647 | 34.616 | 19.401 | 14.824 | 1.00 | 73.81 | A |
| 990 | CG | LEU | 647 | 33.384 | 19.749 | 13.952 | 1.00 | 72.95 | A |
| 991 | CD1 | LEU | 647 | 32.473 | 20.475 | 14.890 | 1.00 | 73.14 | A |
| 992 | CD2 | LEU | 647 | 33.651 | 20.650 | 12.712 | 1.00 | 74.46 | A |
| 993 | C | LEU | 647 | 37.038 | 18.786 | 14.921 | 1.00 | 73.06 | A |
| 994 | O | LEU | 647 | 37.798 | 19.493 | 14.267 | 1.00 | 73.18 | A |
| 995 | N | TYR | 648 | 37.343 | 18.325 | 16.105 | 1.00 | 71.81 | A |
| 996 | CA | TYR | 648 | 38.626 | 18.614 | 16.615 | 1.00 | 70.55 | A |
| 997 | CB | TYR | 648 | 38.770 | 17.967 | 17.942 | 1.00 | 71.32 | A |
| 998 | CG | TYR | 648 | 40.193 | 17.938 | 18.201 | 1.00 | 72.59 | A |
| 999 | CD1 | TYR | 648 | 40.922 | 16.773 | 18.043 | 1.00 | 72.98 | A |
| 1000 | CE1 | TYR | 648 | 42.276 | 16.793 | 18.159 | 1.00 | 74.89 | A |
| 1001 | CD2 | TYR | 648 | 40.849 | 19.122 | 18.479 | 1.00 | 73.43 | A |
| 1002 | CE2 | TYR | 648 | 42.193 | 19.160 | 18.595 | 1.00 | 74.28 | A |
| 1003 | CZ | TYR | 648 | 42.909 | 17.992 | 18.446 | 1.00 | 75.87 | A |
| 1004 | OH | TYR | 648 | 44.259 | 18.004 | 18.666 | 1.00 | 78.05 | A |
| 1005 | C | TYR | 648 | 39.727 | 18.082 | 15.672 | 1.00 | 69.47 | A |
| 1006 | O | TYR | 648 | 40.782 | 18.697 | 15.527 | 1.00 | 69.10 | A |
| 1007 | N | VAL | 649 | 39.535 | 16.936 | 15.042 | 1.00 | 68.99 | A |
| 1008 | CA | VAL | 649 | 40.641 | 16.531 | 14.205 | 1.00 | 68.88 | A |
| 1009 | CB | VAL | 649 | 40.677 | 14.969 | 13.955 | 1.00 | 69.22 | A |
| 1010 | CG1 | VAL | 649 | 39.292 | 14.369 | 13.993 | 1.00 | 68.78 | A |
| 1011 | CG2 | VAL | 649 | 41.391 | 14.663 | 12.652 | 1.00 | 69.29 | A |
| 1012 | C | VAL | 649 | 40.674 | 17.386 | 12.935 | 1.00 | 68.29 | A |
| 1013 | O | VAL | 649 | 41.747 | 17.830 | 12.515 | 1.00 | 67.00 | A |
| 1014 | N | SER | 650 | 39.507 | 17.680 | 12.368 | 1.00 | 69.10 | A |
| 1015 | CA | SER | 650 | 39.425 | 18.528 | 11.170 | 1.00 | 69.39 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1016 | CB | SER | 650 | 37.979 | 18.727 | 10.752 | 1.00 | 69.29 | A |
| 1017 | OG | SER | 650 | 37.915 | 19.900 | 9.974 | 1.00 | 71.17 | A |
| 1018 | C | SER | 650 | 40.015 | 19.922 | 11.379 | 1.00 | 69.02 | A |
| 1019 | O | SER | 650 | 40.648 | 20.502 | 10.486 | 1.00 | 68.81 | A |
| 1020 | N | SER | 651 | 39.758 | 20.475 | 12.557 | 1.00 | 68.62 | A |
| 1021 | CA | SER | 651 | 40.248 | 21.799 | 12.916 | 1.00 | 68.72 | A |
| 1022 | CB | SER | 651 | 39.724 | 22.166 | 14.315 | 1.00 | 70.09 | A |
| 1023 | OG | SER | 651 | 40.748 | 22.677 | 15.162 | 1.00 | 72.58 | A |
| 1024 | C | SER | 651 | 41.772 | 21.790 | 12.913 | 1.00 | 67.36 | A |
| 1025 | O | SER | 651 | 42.430 | 22.683 | 12.379 | 1.00 | 66.28 | A |
| 1026 | N | GLU | 652 | 42.310 | 20.739 | 13.514 | 1.00 | 66.84 | A |
| 1027 | CA | GLU | 652 | 43.737 | 20.543 | 13.652 | 1.00 | 65.75 | A |
| 1028 | CB | GLU | 652 | 43.950 | 19.303 | 14.497 | 1.00 | 65.94 | A |
| 1029 | CG | GLU | 652 | 45.026 | 19.432 | 15.539 | 1.00 | 68.92 | A |
| 1030 | CD | GLU | 652 | 45.053 | 20.768 | 16.255 | 1.00 | 70.91 | A |
| 1031 | OE1 | GLU | 652 | 44.020 | 21.469 | 16.341 | 1.00 | 72.27 | A |
| 1032 | OE2 | GLU | 652 | 46.141 | 21.107 | 16.753 | 1.00 | 73.20 | A |
| 1033 | C | GLU | 652 | 44.472 | 20.448 | 12.323 | 1.00 | 64.24 | A |
| 1034 | O | GLU | 652 | 45.601 | 20.931 | 12.202 | 1.00 | 64.18 | A |
| 1035 | N | LEU | 653 | 43.838 | 19.818 | 11.339 | 1.00 | 63.37 | A |
| 1036 | CA | LEU | 653 | 44.407 | 19.689 | 9.997 | 1.00 | 62.68 | A |
| 1037 | CB | LEU | 653 | 43.515 | 18.797 | 9.117 | 1.00 | 60.31 | A |
| 1038 | CG | LEU | 653 | 43.496 | 17.298 | 9.453 | 1.00 | 58.08 | A |
| 1039 | CD1 | LEU | 653 | 42.502 | 16.529 | 8.584 | 1.00 | 56.65 | A |
| 1040 | CD2 | LEU | 653 | 44.889 | 16.766 | 9.259 | 1.00 | 55.94 | A |
| 1041 | C | LEU | 653 | 44.437 | 21.100 | 9.425 | 1.00 | 63.49 | A |
| 1042 | O | LEU | 653 | 45.454 | 21.574 | 8.919 | 1.00 | 63.20 | A |
| 1043 | N | HIS | 654 | 43.303 | 21.773 | 9.532 | 1.00 | 65.44 | A |
| 1044 | CA | HIS | 654 | 43.155 | 23.131 | 9.050 | 1.00 | 66.40 | A |
| 1045 | CB | HIS | 654 | 41.757 | 23.639 | 9.408 | 1.00 | 68.67 | A |
| 1046 | CG | HIS | 654 | 41.466 | 24.995 | 8.860 | 1.00 | 70.94 | A |
| 1047 | CD2 | HIS | 654 | 40.952 | 25.380 | 7.669 | 1.00 | 72.19 | A |
| 1048 | ND1 | HIS | 654 | 41.829 | 26.151 | 9.517 | 1.00 | 72.01 | A |
| 1049 | CE1 | HIS | 654 | 41.561 | 27.190 | 8.743 | 1.00 | 72.60 | A |
| 1050 | NE2 | HIS | 654 | 41.031 | 26.749 | 7.620 | 1.00 | 73.26 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1051 | C | HIS | 654 | 44.217 | 24.058 | 9.639 | 1.00 | 66.64 | A |
| 1052 | O | HIS | 654 | 44.982 | 24.702 | 8.912 | 1.00 | 66.69 | A |
| 1053 | N | ARG | 655 | 44.253 | 24.114 | 10.964 | 1.00 | 66.13 | A |
| 1054 | CA | ARG | 655 | 45.198 | 24.945 | 11.685 | 1.00 | 65.54 | A |
| 1055 | CB | ARG | 655 | 45.095 | 24.674 | 13.168 | 1.00 | 66.61 | A |
| 1056 | CG | ARG | 655 | 45.840 | 25.672 | 14.004 | 1.00 | 68.07 | A |
| 1057 | CD | ARG | 655 | 46.173 | 25.076 | 15.332 | 1.00 | 68.45 | A |
| 1058 | NE | ARG | 655 | 47.501 | 24.497 | 15.290 | 1.00 | 71.04 | A |
| 1059 | CZ | ARG | 655 | 47.952 | 23.630 | 16.183 | 1.00 | 73.26 | A |
| 1060 | NH1 | ARG | 655 | 47.162 | 23.252 | 17.177 | 1.00 | 74.95 | A |
| 1061 | NH2 | ARG | 655 | 49.193 | 23.158 | 16.096 | 1.00 | 73.03 | A |
| 1062 | C | ARG | 655 | 46.624 | 24.670 | 11.277 | 1.00 | 65.54 | A |
| 1063 | O | ARG | 655 | 47.460 | 25.575 | 11.222 | 1.00 | 66.10 | A |
| 1064 | N | LEU | 656 | 46.920 | 23.408 | 11.014 | 1.00 | 65.42 | A |
| 1065 | CA | LEU | 656 | 48.272 | 23.039 | 10.645 | 1.00 | 65.44 | A |
| 1066 | CB | LEU | 656 | 48.584 | 21.653 | 11.206 | 1.00 | 64.44 | A |
| 1067 | CG | LEU | 656 | 49.148 | 21.728 | 12.619 | 1.00 | 63.25 | A |
| 1068 | CD1 | LEU | 656 | 49.549 | 20.337 | 13.064 | 1.00 | 63.29 | A |
| 1069 | CD2 | LEU | 656 | 50.358 | 22.650 | 12.625 | 1.00 | 62.26 | A |
| 1070 | C | LEU | 656 | 48.571 | 23.094 | 9.150 | 1.00 | 65.58 | A |
| 1071 | O | LEU | 656 | 49.705 | 22.815 | 8.734 | 1.00 | 64.71 | A |
| 1072 | N | GLN | 657 | 47.558 | 23.501 | 8.375 | 1.00 | 66.26 | A |
| 1073 | CA | GLN | 657 | 47.599 | 23.588 | 6.907 | 1.00 | 67.33 | A |
| 1074 | CB | GLN | 657 | 48.272 | 24.883 | 6.412 | 1.00 | 70.37 | A |
| 1075 | CG | GLN | 657 | 47.332 | 26.121 | 6.337 | 1.00 | 74.91 | A |
| 1076 | CD | GLN | 657 | 46.109 | 25.932 | 5.418 | 1.00 | 77.90 | A |
| 1077 | OE1 | GLN | 657 | 44.957 | 26.078 | 5.853 | 1.00 | 79.08 | A |
| 1078 | NE2 | GLN | 657 | 46.362 | 25.622 | 4.145 | 1.00 | 79.44 | A |
| 1079 | C | GLN | 657 | 48.306 | 22.374 | 6.365 | 1.00 | 66.01 | A |
| 1080 | O | GLN | 657 | 49.342 | 22.467 | 5.708 | 1.00 | 67.61 | A |
| 1081 | N | VAL | 658 | 47.714 | 21.224 | 6.663 | 1.00 | 64.56 | A |
| 1082 | CA | VAL | 658 | 48.246 | 19.938 | 6.260 | 1.00 | 63.29 | A |
| 1083 | CB | VAL | 658 | 47.627 | 18.787 | 7.098 | 1.00 | 61.27 | A |
| 1084 | CG1 | VAL | 658 | 48.073 | 17.451 | 6.544 | 1.00 | 60.69 | A |
| 1085 | CG2 | VAL | 658 | 48.063 | 18.902 | 8.562 | 1.00 | 59.00 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1086 | C | VAL | 658 | 48.093 | 19.630 | 4.779 | 1.00 | 63.70 | A |
| 1087 | O | VAL | 658 | 47.011 | 19.686 | 4.196 | 1.00 | 64.37 | A |
| 1088 | N | SER | 659 | 49.249 | 19.304 | 4.214 | 1.00 | 64.31 | A |
| 1089 | CA | SER | 659 | 49.510 | 18.938 | 2.829 | 1.00 | 64.00 | A |
| 1090 | CB | SER | 659 | 51.038 | 18.836 | 2.715 | 1.00 | 65.00 | A |
| 1091 | OG | SER | 659 | 51.521 | 18.491 | 1.435 | 1.00 | 68.63 | A |
| 1092 | C | SER | 659 | 48.833 | 17.594 | 2.491 | 1.00 | 63.59 | A |
| 1093 | O | SER | 659 | 48.599 | 16.781 | 3.384 | 1.00 | 63.80 | A |
| 1094 | N | TYR | 660 | 48.512 | 17.353 | 1.219 | 1.00 | 62.62 | A |
| 1095 | CA | TYR | 660 | 47.894 | 16.080 | 0.862 | 1.00 | 60.90 | A |
| 1096 | CB | TYR | 660 | 47.483 | 16.023 | -0.603 | 1.00 | 57.88 | A |
| 1097 | CG | TYR | 660 | 46.622 | 14.816 | -0.889 | 1.00 | 55.47 | A |
| 1098 | CD1 | TYR | 660 | 45.585 | 14.484 | -0.024 | 1.00 | 54.48 | A |
| 1099 | CE1 | TYR | 660 | 44.729 | 13.439 | -0.283 | 1.00 | 52.21 | A |
| 1100 | CD2 | TYR | 660 | 46.791 | 14.045 | -2.044 | 1.00 | 53.60 | A |
| 1101 | CE2 | TYR | 660 | 45.926 | 12.977 | -2.318 | 1.00 | 52.33 | A |
| 1102 | CZ | TYR | 660 | 44.892 | 12.692 | -1.427 | 1.00 | 52.28 | A |
| 1103 | OH | TYR | 660 | 43.969 | 11.695 | -1.661 | 1.00 | 53.74 | A |
| 1104 | C | TYR | 660 | 48.888 | 14.964 | 1.086 | 1.00 | 61.71 | A |
| 1105 | O | TYR | 660 | 48.546 | 13.894 | 1.581 | 1.00 | 62.27 | A |
| 1106 | N | GLU | 661 | 50.129 | 15.213 | 0.693 | 1.00 | 62.99 | A |
| 1107 | CA | GLU | 661 | 51.145 | 14.205 | 0.853 | 1.00 | 64.16 | A |
| 1108 | CB | GLU | 661 | 52.430 | 14.641 | 0.165 | 1.00 | 65.20 | A |
| 1109 | CG | GLU | 661 | 52.264 | 14.520 | -1.334 | 1.00 | 68.41 | A |
| 1110 | CD | GLU | 661 | 53.567 | 14.573 | -2.093 | 1.00 | 70.81 | A |
| 1111 | OE1 | GLU | 661 | 54.627 | 14.807 | -1.463 | 1.00 | 69.79 | A |
| 1112 | OE2 | GLU | 661 | 53.525 | 14.381 | -3.331 | 1.00 | 73.10 | A |
| 1113 | C | GLU | 661 | 51.338 | 13.912 | 2.319 | 1.00 | 63.87 | A |
| 1114 | O | GLU | 661 | 51.208 | 12.760 | 2.734 | 1.00 | 64.43 | A |
| 1115 | N | GLU | 662 | 51.626 | 14.943 | 3.107 | 1.00 | 62.73 | A |
| 1116 | CA | GLU | 662 | 51.788 | 14.740 | 4.535 | 1.00 | 60.94 | A |
| 1117 | CB | GLU | 662 | 51.820 | 16.072 | 5.260 | 1.00 | 62.39 | A |
| 1118 | CG | GLU | 662 | 53.120 | 16.809 | 5.171 | 1.00 | 63.89 | A |
| 1119 | CD | GLU | 662 | 52.926 | 18.276 | 5.467 | 1.00 | 65.88 | A |
| 1120 | OE1 | GLU | 662 | 51.759 | 18.720 | 5.449 | 1.00 | 66.26 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1121 | OE2 | GLU | 662 | 53.923 | 18.992 | 5.705 | 1.00 | 66.78 | A |
| 1122 | C | GLU | 662 | 50.611 | 13.923 | 5.068 | 1.00 | 59.42 | A |
| 1123 | O | GLU | 662 | 50.791 | 13.031 | 5.897 | 1.00 | 59.90 | A |
| 1124 | N | TYR | 663 | 49.408 | 14.232 | 4.587 | 1.00 | 57.33 | A |
| 1125 | CA | TYR | 663 | 48.182 | 13.546 | 5.016 | 1.00 | 57.29 | A |
| 1126 | CB | TYR | 663 | 46.944 | 14.179 | 4.372 | 1.00 | 55.05 | A |
| 1127 | CG | TYR | 663 | 45.719 | 13.267 | 4.361 | 1.00 | 52.55 | A |
| 1128 | CD1 | TYR | 663 | 44.988 | 13.044 | 5.526 | 1.00 | 52.08 | A |
| 1129 | CE1 | TYR | 663 | 43.829 | 12.242 | 5.521 | 1.00 | 51.10 | A |
| 1130 | CD2 | TYR | 663 | 45.272 | 12.661 | 3.180 | 1.00 | 51.77 | A |
| 1131 | CE2 | TYR | 663 | 44.114 | 11.856 | 3.161 | 1.00 | 50.54 | A |
| 1132 | CZ | TYR | 663 | 43.390 | 11.656 | 4.340 | 1.00 | 51.46 | A |
| 1133 | OH | TYR | 663 | 42.207 | 10.922 | 4.350 | 1.00 | 52.74 | A |
| 1134 | C | TYR | 663 | 48.114 | 12.068 | 4.685 | 1.00 | 57.96 | A |
| 1135 | O | TYR | 663 | 47.530 | 11.268 | 5.430 | 1.00 | 58.51 | A |
| 1136 | N | LEU | 664 | 48.652 | 11.726 | 3.520 | 1.00 | 58.19 | A |
| 1137 | CA | LEU | 664 | 48.629 | 10.366 | 3.047 | 1.00 | 57.31 | A |
| 1138 | CB | LEU | 664 | 49.030 | 10.342 | 1.576 | 1.00 | 56.51 | A |
| 1139 | CG | LEU | 664 | 47.854 | 10.677 | 0.655 | 1.00 | 54.66 | A |
| 1140 | CD1 | LEU | 664 | 48.206 | 10.343 | -0.776 | 1.00 | 54.06 | A |
| 1141 | CD2 | LEU | 664 | 46.639 | 9.867 | 1.068 | 1.00 | 52.20 | A |
| 1142 | C | LEU | 664 | 49.547 | 9.511 | 3.890 | 1.00 | 57.26 | A |
| 1143 | O | LEU | 664 | 49.257 | 8.342 | 4.184 | 1.00 | 56.63 | A |
| 1144 | N | CYS | 665 | 50.652 | 10.128 | 4.290 | 1.00 | 56.70 | A |
| 1145 | CA | CYS | 665 | 51.629 | 9.475 | 5.118 | 1.00 | 57.53 | A |
| 1146 | CB | CYS | 665 | 52.910 | 10.292 | 5.130 | 1.00 | 58.19 | A |
| 1147 | SG | CYS | 665 | 53.830 | 10.095 | 3.584 | 1.00 | 63.72 | A |
| 1148 | C | CYS | 665 | 51.099 | 9.299 | 6.527 | 1.00 | 57.62 | A |
| 1149 | O | CYS | 665 | 51.156 | 8.203 | 7.082 | 1.00 | 59.12 | A |
| 1150 | N | MET | 666 | 50.557 | 10.362 | 7.109 | 1.00 | 57.03 | A |
| 1151 | CA | MET | 666 | 50.042 | 10.272 | 8.468 | 1.00 | 56.19 | A |
| 1152 | CB | MET | 666 | 49.433 | 11.608 | 8.895 | 1.00 | 55.82 | A |
| 1153 | CG | MET | 666 | 50.475 | 12.699 | 9.005 | 1.00 | 57.38 | A |
| 1154 | SD | MET | 666 | 49.817 | 14.370 | 9.027 | 1.00 | 58.37 | A |
| 1155 | CE | MET | 666 | 49.222 | 14.475 | 10.771 | 1.00 | 59.18 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1156 | C | MET | 666 | 49.012 | 9.180 | 8.638 | 1.00 | 55.71 | A |
| 1157 | O | MET | 666 | 48.930 | 8.555 | 9.693 | 1.00 | 56.57 | A |
| 1158 | N | LYS | 667 | 48.225 | 8.937 | 7.603 | 1.00 | 55.23 | A |
| 1159 | CA | LYS | 667 | 47.178 | 7.935 | 7.730 | 1.00 | 54.64 | A |
| 1160 | CB | LYS | 667 | 46.097 | 8.190 | 6.698 | 1.00 | 53.25 | A |
| 1161 | CG | LYS | 667 | 44.903 | 7.301 | 6.845 | 1.00 | 52.56 | A |
| 1162 | CD | LYS | 667 | 43.726 | 7.893 | 6.096 | 1.00 | 52.52 | A |
| 1163 | CE | LYS | 667 | 44.050 | 8.168 | 4.637 | 1.00 | 51.07 | A |
| 1164 | NZ | LYS | 667 | 44.186 | 6.900 | 3.877 | 1.00 | 54.84 | A |
| 1165 | C | LYS | 667 | 47.693 | 6.506 | 7.631 | 1.00 | 54.78 | A |
| 1166 | O | LYS | 667 | 47.056 | 5.577 | 8.127 | 1.00 | 55.27 | A |
| 1167 | N | THR | 668 | 48.838 | 6.332 | 6.983 | 1.00 | 55.22 | A |
| 1168 | CA | THR | 668 | 49.433 | 5.013 | 6.850 | 1.00 | 55.52 | A |
| 1169 | CB | THR | 668 | 50.550 | 5.021 | 5.773 | 1.00 | 54.91 | A |
| 1170 | OG1 | THR | 668 | 50.007 | 5.511 | 4.538 | 1.00 | 55.09 | A |
| 1171 | CG2 | THR | 668 | 51.083 | 3.620 | 5.535 | 1.00 | 54.29 | A |
| 1172 | C | THR | 668 | 49.996 | 4.765 | 8.243 | 1.00 | 54.90 | A |
| 1173 | O | THR | 668 | 49.851 | 3.674 | 8.793 | 1.00 | 54.89 | A |
| 1174 | N | LEU | 669 | 50.602 | 5.803 | 8.828 | 1.00 | 55.28 | A |
| 1175 | CA | LEU | 669 | 51.151 | 5.714 | 10.182 | 1.00 | 55.00 | A |
| 1176 | CB | LEU | 669 | 51.890 | 7.001 | 10.566 | 1.00 | 52.74 | A |
| 1177 | CG | LEU | 669 | 53.266 | 7.224 | 9.928 | 1.00 | 51.51 | A |
| 1178 | CD1 | LEU | 669 | 53.925 | 8.432 | 10.516 | 1.00 | 51.09 | A |
| 1179 | CD2 | LEU | 669 | 54.144 | 6.030 | 10.175 | 1.00 | 51.14 | A |
| 1180 | C | LEU | 669 | 50.049 | 5.444 | 11.214 | 1.00 | 55.52 | A |
| 1181 | O | LEU | 669 | 50.321 | 4.968 | 12.311 | 1.00 | 56.72 | A |
| 1182 | N | LEU | 670 | 48.805 | 5.734 | 10.875 | 1.00 | 55.19 | A |
| 1183 | CA | LEU | 670 | 47.737 | 5.504 | 11.832 | 1.00 | 55.37 | A |
| 1184 | CB | LEU | 670 | 46.566 | 6.432 | 11.512 | 1.00 | 54.24 | A |
| 1185 | CG | LEU | 670 | 46.048 | 7.483 | 12.498 | 1.00 | 53.91 | A |
| 1186 | CD1 | LEU | 670 | 47.100 | 8.401 | 13.059 | 1.00 | 51.87 | A |
| 1187 | CD2 | LEU | 670 | 45.063 | 8.287 | 11.712 | 1.00 | 56.32 | A |
| 1188 | C | LEU | 670 | 47.282 | 4.044 | 11.849 | 1.00 | 56.26 | A |
| 1189 | O | LEU | 670 | 46.659 | 3.590 | 12.806 | 1.00 | 56.76 | A |
| 1190 | N | LEU | 671 | 47.583 | 3.314 | 10.784 | 1.00 | 57.62 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1191 | CA | LEU | 671 | 47.216 | 1.910 | 10.685 | 1.00 | 58.03 | A |
| 1192 | CB | LEU | 671 | 47.306 | 1.483 | 9.207 | 1.00 | 54.95 | A |
| 1193 | CG | LEU | 671 | 47.458 | 0.030 | 8.739 | 1.00 | 52.63 | A |
| 1194 | CD1 | LEU | 671 | 46.215 | -0.742 | 9.077 | 1.00 | 51.93 | A |
| 1195 | CD2 | LEU | 671 | 47.696 | -0.023 | 7.234 | 1.00 | 51.62 | A |
| 1196 | C | LEU | 671 | 48.233 | 1.167 | 11.559 | 1.00 | 61.12 | A |
| 1197 | O | LEU | 671 | 47.900 | 0.228 | 12.291 | 1.00 | 62.38 | A |
| 1198 | N | LEU | 672 | 49.470 | 1.653 | 11.511 | 1.00 | 63.00 | A |
| 1199 | CA | LEU | 672 | 50.578 | 1.073 | 12.259 | 1.00 | 63.98 | A |
| 1200 | CB | LEU | 672 | 51.843 | 1.143 | 11.395 | 1.00 | 60.71 | A |
| 1201 | CG | LEU | 672 | 51.592 | 0.994 | 9.888 | 1.00 | 58.47 | A |
| 1202 | CD1 | LEU | 672 | 52.875 | 1.217 | 9.095 | 1.00 | 56.96 | A |
| 1203 | CD2 | LEU | 672 | 51.013 | -0.383 | 9.621 | 1.00 | 56.80 | A |
| 1204 | C | LEU | 672 | 50.826 | 1.802 | 13.586 | 1.00 | 66.42 | A |
| 1205 | O | LEU | 672 | 51.977 | 1.885 | 14.027 | 1.00 | 67.06 | A |
| 1206 | N | SER | 673 | 49.768 | 2.304 | 14.231 | 1.00 | 68.97 | A |
| 1207 | CA | SER | 673 | 49.936 | 3.061 | 15.476 | 1.00 | 72.55 | A |
| 1208 | CB | SER | 673 | 49.203 | 4.398 | 15.391 | 1.00 | 71.35 | A |
| 1209 | OG | SER | 673 | 47.818 | 4.198 | 15.201 | 1.00 | 70.80 | A |
| 1210 | C | SER | 673 | 49.584 | 2.383 | 16.798 | 1.00 | 75.85 | A |
| 1211 | O | SER | 673 | 49.707 | 2.988 | 17.867 | 1.00 | 76.67 | A |
| 1212 | N | SER | 674 | 49.095 | 1.152 | 16.737 | 1.00 | 79.23 | A |
| 1213 | CA | SER | 674 | 48.844 | 0.423 | 17.968 | 1.00 | 82.13 | A |
| 1214 | CB | SER | 674 | 47.492 | 0.781 | 18.613 | 1.00 | 81.41 | A |
| 1215 | OG | SER | 674 | 46.385 | 0.252 | 17.915 | 1.00 | 84.00 | A |
| 1216 | C | SER | 674 | 48.970 | -1.056 | 17.652 | 1.00 | 83.87 | A |
| 1217 | O | SER | 674 | 48.424 | -1.556 | 16.661 | 1.00 | 84.21 | A |
| 1218 | N | VAL | 675 | 49.776 | -1.715 | 18.477 | 1.00 | 85.78 | A |
| 1219 | CA | VAL | 675 | 50.056 | -3.137 | 18.373 | 1.00 | 87.96 | A |
| 1220 | CB | VAL | 675 | 51.556 | -3.381 | 18.110 | 1.00 | 86.94 | A |
| 1221 | CG1 | VAL | 675 | 51.926 | -2.896 | 16.726 | 1.00 | 86.95 | A |
| 1222 | CG2 | VAL | 675 | 52.390 | -2.658 | 19.159 | 1.00 | 87.12 | A |
| 1223 | C | VAL | 675 | 49.668 | -3.783 | 19.704 | 1.00 | 90.28 | A |
| 1224 | O | VAL | 675 | 49.483 | -3.092 | 20.709 | 1.00 | 89.69 | A |
| 1225 | N | PRO | 676 | 49.544 | -5.124 | 19.726 | 1.00 | 93.03 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1226 | CD | PRO | 676 | 49.961 | -6.005 | 18.630 | 1.00 | 93.57 | A |
| 1227 | CA | PRO | 676 | 49.182 | -5.919 | 20.904 | 1.00 | 95.42 | A |
| 1228 | CB | PRO | 676 | 49.235 | -7.354 | 20.380 | 1.00 | 94.95 | A |
| 1229 | CG | PRO | 676 | 49.127 | -7.206 | 18.883 | 1.00 | 94.18 | A |
| 1230 | C | PRO | 676 | 50.243 | -5.673 | 21.963 | 1.00 | 97.54 | A |
| 1231 | O | PRO | 676 | 51.352 | -5.257 | 21.626 | 1.00 | 98.58 | A |
| 1232 | N | LYS | 677 | 49.933 | -5.929 | 23.229 | 1.00 | 99.68 | A |
| 1233 | CA | LYS | 677 | 50.917 | -5.693 | 24.286 | 1.00 | 101.53 | A |
| 1234 | CB | LYS | 677 | 50.295 | -5.899 | 25.670 | 1.00 | 102.63 | A |
| 1235 | CG | LYS | 677 | 51.160 | -5.381 | 26.824 | 1.00 | 103.79 | A |
| 1236 | CD | LYS | 677 | 51.244 | -6.398 | 27.965 | 1.00 | 104.31 | A |
| 1237 | CE | LYS | 677 | 52.012 | -7.633 | 27.522 | 1.00 | 104.35 | A |
| 1238 | NZ | LYS | 677 | 51.800 | -8.827 | 28.387 | 1.00 | 105.00 | A |
| 1239 | C | LYS | 677 | 52.107 | -6.628 | 24.116 | 1.00 | 102.18 | A |
| 1240 | O | LYS | 677 | 53.147 | -6.458 | 24.761 | 1.00 | 101.68 | A |
| 1241 | N | ASP | 678 | 51.949 | -7.615 | 23.240 | 1.00 | 102.84 | A |
| 1242 | CA | ASP | 678 | 53.009 | -8.571 | 22.980 | 1.00 | 104.29 | A |
| 1243 | CB | ASP | 678 | 52.524 | -9.988 | 23.281 | 1.00 | 106.29 | A |
| 1244 | CG | ASP | 678 | 51.908 | -10.107 | 24.656 | 1.00 | 108.07 | A |
| 1245 | OD1 | ASP | 678 | 52.320 | -9.342 | 25.551 | 1.00 | 108.43 | A |
| 1246 | OD2 | ASP | 678 | 51.025 | -10.974 | 24.842 | 1.00 | 109.39 | A |
| 1247 | C | ASP | 678 | 53.469 | -8.488 | 21.532 | 1.00 | 104.24 | A |
| 1248 | O | ASP | 678 | 53.859 | -9.493 | 20.935 | 1.00 | 104.59 | A |
| 1249 | N | GLY | 679 | 53.424 | -7.286 | 20.968 | 1.00 | 103.43 | A |
| 1250 | CA | GLY | 679 | 53.840 | -7.135 | 19.590 | 1.00 | 101.95 | A |
| 1251 | C | GLY | 679 | 53.097 | -8.131 | 18.724 | 1.00 | 101.17 | A |
| 1252 | O | GLY | 679 | 52.297 | -8.936 | 19.203 | 1.00 | 100.48 | A |
| 1253 | N | LEU | 680 | 53.382 | -8.097 | 17.432 | 1.00 | 100.20 | A |
| 1254 | CA | LEU | 680 | 52.707 | -8.983 | 16.504 | 1.00 | 98.61 | A |
| 1255 | CB | LEU | 680 | 52.425 | -8.208 | 15.229 | 1.00 | 98.06 | A |
| 1256 | CG | LEU | 680 | 52.093 | -6.769 | 15.601 | 1.00 | 97.47 | A |
| 1257 | CD1 | LEU | 680 | 52.560 | -5.848 | 14.516 | 1.00 | 97.75 | A |
| 1258 | CD2 | LEU | 680 | 50.615 | -6.630 | 15.855 | 1.00 | 97.02 | A |
| 1259 | C | LEU | 680 | 53.547 | -10.203 | 16.202 | 1.00 | 97.47 | A |
| 1260 | O | LEU | 680 | 54.561 | -10.459 | 16.850 | 1.00 | 97.58 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1261 | N | LYS | 681 | 53.120 | -10.968 | 15.211 | 1.00 | 96.17 | A |
| 1262 | CA | LYS | 681 | 53.865 | -12.143 | 14.837 | 1.00 | 95.16 | A |
| 1263 | CB | LYS | 681 | 52.937 | -13.202 | 14.245 | 1.00 | 95.18 | A |
| 1264 | CG | LYS | 681 | 51.846 | -13.706 | 15.192 | 1.00 | 94.91 | A |
| 1265 | CD | LYS | 681 | 51.184 | -14.927 | 14.581 | 1.00 | 94.17 | A |
| 1266 | CE | LYS | 681 | 49.836 | -15.278 | 15.191 | 1.00 | 93.86 | A |
| 1267 | NZ | LYS | 681 | 49.185 | -16.380 | 14.418 | 1.00 | 92.63 | A |
| 1268 | C | LYS | 681 | 54.937 | -11.770 | 13.828 | 1.00 | 95.02 | A |
| 1269 | O | LYS | 681 | 55.736 | -12.613 | 13.437 | 1.00 | 95.32 | A |
| 1270 | N | SER | 682 | 54.962 | -10.511 | 13.397 | 1.00 | 95.00 | A |
| 1271 | CA | SER | 682 | 55.981 | -10.080 | 12.435 | 1.00 | 94.85 | A |
| 1272 | CB | SER | 682 | 55.377 | -9.946 | 11.029 | 1.00 | 94.61 | A |
| 1273 | OG | SER | 682 | 54.906 | -11.193 | 10.549 | 1.00 | 92.86 | A |
| 1274 | C | SER | 682 | 56.639 | -8.761 | 12.842 | 1.00 | 94.58 | A |
| 1275 | O | SER | 682 | 57.167 | -8.028 | 12.004 | 1.00 | 94.56 | A |
| 1276 | N | GLN | 683 | 56.616 | -8.482 | 14.144 | 1.00 | 94.40 | A |
| 1277 | CA | GLN | 683 | 57.192 | -7.269 | 14.710 | 1.00 | 94.48 | A |
| 1278 | CB | GLN | 683 | 57.517 | -7.477 | 16.180 | 1.00 | 95.06 | A |
| 1279 | CG | GLN | 683 | 56.384 | -7.111 | 17.096 | 1.00 | 97.14 | A |
| 1280 | CD | GLN | 683 | 56.186 | -5.606 | 17.210 | 1.00 | 98.36 | A |
| 1281 | OE1 | GLN | 683 | 55.147 | -5.148 | 17.678 | 1.00 | 99.32 | A |
| 1282 | NE2 | GLN | 683 | 57.189 | -4.835 | 16.795 | 1.00 | 98.03 | A |
| 1283 | C | GLN | 683 | 58.425 | -6.725 | 14.029 | 1.00 | 94.16 | A |
| 1284 | O | GLN | 683 | 58.599 | -5.516 | 13.948 | 1.00 | 94.61 | A |
| 1285 | N | GLU | 684 | 59.301 | -7.596 | 13.563 | 1.00 | 94.49 | A |
| 1286 | CA | GLU | 684 | 60.494 | -7.084 | 12.915 | 1.00 | 95.02 | A |
| 1287 | CB | GLU | 684 | 61.445 | -8.230 | 12.567 | 1.00 | 97.72 | A |
| 1288 | CG | GLU | 684 | 61.936 | -9.001 | 13.790 | 1.00 | 101.32 | A |
| 1289 | CD | GLU | 684 | 62.713 | -10.245 | 13.404 | 1.00 | 104.63 | A |
| 1290 | OE1 | GLU | 684 | 62.269 | -10.940 | 12.460 | 1.00 | 106.64 | A |
| 1291 | OE2 | GLU | 684 | 63.753 | -10.534 | 14.043 | 1.00 | 105.52 | A |
| 1292 | C | GLU | 684 | 60.092 | -6.292 | 11.671 | 1.00 | 93.12 | A |
| 1293 | O | GLU | 684 | 60.516 | -5.148 | 11.509 | 1.00 | 93.19 | A |
| 1294 | N | LEU | 685 | 59.257 | -6.893 | 10.814 | 1.00 | 90.38 | A |
| 1295 | CA | LEU | 685 | 58.783 | -6.237 | 9.591 | 1.00 | 88.13 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1296 | CB | LEU | 685 | 57.904 | -7.198 | 8.760 | 1.00 | 87.12 | A |
| 1297 | CG | LEU | 685 | 58.232 | -7.458 | 7.278 | 1.00 | 86.54 | A |
| 1298 | CD1 | LEU | 685 | 56.980 | -7.975 | 6.550 | 1.00 | 86.04 | A |
| 1299 | CD2 | LEU | 685 | 58.720 | -6.181 | 6.613 | 1.00 | 85.14 | A |
| 1300 | C | LEU | 685 | 57.970 | -4.993 | 9.975 | 1.00 | 87.42 | A |
| 1301 | O | LEU | 685 | 58.225 | -3.894 | 9.482 | 1.00 | 87.78 | A |
| 1302 | N | PHE | 686 | 56.990 | -5.178 | 10.860 | 1.00 | 85.68 | A |
| 1303 | CA | PHE | 686 | 56.161 | -4.083 | 11.334 | 1.00 | 83.88 | A |
| 1304 | CB | PHE | 686 | 55.399 | -4.497 | 12.587 | 1.00 | 82.21 | A |
| 1305 | CG | PHE | 686 | 54.501 | -3.431 | 13.117 | 1.00 | 80.95 | A |
| 1306 | CD1 | PHE | 686 | 53.229 | -3.267 | 12.590 | 1.00 | 80.85 | A |
| 1307 | CD2 | PHE | 686 | 54.948 | -2.542 | 14.090 | 1.00 | 80.25 | A |
| 1308 | CE1 | PHE | 686 | 52.399 | -2.243 | 13.024 | 1.00 | 80.58 | A |
| 1309 | CE2 | PHE | 686 | 54.127 | -1.505 | 14.533 | 1.00 | 80.77 | A |
| 1310 | CZ | PHE | 686 | 52.851 | -1.352 | 13.995 | 1.00 | 80.71 | A |
| 1311 | C | PHE | 686 | 57.013 | -2.869 | 11.693 | 1.00 | 84.16 | A |
| 1312 | O | PHE | 686 | 56.863 | -1.787 | 11.125 | 1.00 | 84.35 | A |
| 1313 | N | ASP | 687 | 57.906 | -3.051 | 12.657 | 1.00 | 84.16 | A |
| 1314 | CA | ASP | 687 | 58.755 | -1.955 | 13.088 | 1.00 | 84.15 | A |
| 1315 | CB | ASP | 687 | 59.742 | -2.445 | 14.165 | 1.00 | 86.11 | A |
| 1316 | CG | ASP | 687 | 59.088 | -2.582 | 15.559 | 1.00 | 87.77 | A |
| 1317 | OD1 | ASP | 687 | 59.737 | -3.133 | 16.479 | 1.00 | 88.21 | A |
| 1318 | OD2 | ASP | 687 | 57.928 | -2.131 | 15.738 | 1.00 | 88.58 | A |
| 1319 | C | ASP | 687 | 59.476 | -1.326 | 11.895 | 1.00 | 83.05 | A |
| 1320 | O | ASP | 687 | 59.734 | -0.123 | 11.886 | 1.00 | 82.98 | A |
| 1321 | N | GLU | 688 | 59.757 | -2.135 | 10.878 | 1.00 | 81.82 | A |
| 1322 | CA | GLU | 688 | 60.446 | -1.684 | 9.664 | 1.00 | 81.05 | A |
| 1323 | CB | GLU | 688 | 60.962 | -2.903 | 8.894 | 1.00 | 84.35 | A |
| 1324 | CG | GLU | 688 | 62.429 | -2.835 | 8.520 | 1.00 | 89.90 | A |
| 1325 | CD | GLU | 688 | 62.642 | -2.602 | 7.037 | 1.00 | 93.24 | A |
| 1326 | OE1 | GLU | 688 | 62.207 | -3.464 | 6.232 | 1.00 | 94.85 | A |
| 1327 | OE2 | GLU | 688 | 63.244 | -1.557 | 6.680 | 1.00 | 94.38 | A |
| 1328 | C | GLU | 688 | 59.543 | -0.851 | 8.748 | 1.00 | 78.17 | A |
| 1329 | O | GLU | 688 | 59.974 | 0.133 | 8.142 | 1.00 | 77.26 | A |
| 1330 | N | ILE | 689 | 58.290 | -1.274 | 8.628 | 1.00 | 74.88 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1331 | CA | ILE | 689 | 57.334 | -0.562 | 7.807 | 1.00 | 71.74 | A |
| 1332 | CB | ILE | 689 | 56.039 | -1.363 | 7.701 | 1.00 | 70.89 | A |
| 1333 | CG2 | ILE | 689 | 55.087 | -0.735 | 6.694 | 1.00 | 70.61 | A |
| 1334 | CG1 | ILE | 689 | 56.393 | -2.772 | 7.253 | 1.00 | 70.37 | A |
| 1335 | CD1 | ILE | 689 | 55.212 | -3.643 | 7.021 | 1.00 | 70.93 | A |
| 1336 | C | ILE | 689 | 57.126 | 0.753 | 8.527 | 1.00 | 70.52 | A |
| 1337 | O | ILE | 689 | 57.470 | 1.809 | 8.012 | 1.00 | 70.29 | A |
| 1338 | N | ARG | 690 | 56.600 | 0.701 | 9.745 | 1.00 | 68.49 | A |
| 1339 | CA | ARG | 690 | 56.413 | 1.936 | 10.470 | 1.00 | 67.14 | A |
| 1340 | CB | ARG | 690 | 56.263 | 1.660 | 11.948 | 1.00 | 66.55 | A |
| 1341 | CG | ARG | 690 | 55.840 | 2.865 | 12.729 | 1.00 | 65.66 | A |
| 1342 | CD | ARG | 690 | 54.881 | 2.378 | 13.767 | 1.00 | 66.86 | A |
| 1343 | NE | ARG | 690 | 54.332 | 3.440 | 14.589 | 1.00 | 69.11 | A |
| 1344 | CZ | ARG | 690 | 55.062 | 4.204 | 15.386 | 1.00 | 71.32 | A |
| 1345 | NH1 | ARG | 690 | 56.371 | 4.020 | 15.458 | 1.00 | 71.35 | A |
| 1346 | NH2 | ARG | 690 | 54.481 | 5.144 | 16.123 | 1.00 | 72.01 | A |
| 1347 | C | ARG | 690 | 57.566 | 2.926 | 10.263 | 1.00 | 66.75 | A |
| 1348 | O | ARG | 690 | 57.328 | 4.088 | 9.972 | 1.00 | 66.46 | A |
| 1349 | N | MET | 691 | 58.814 | 2.478 | 10.398 | 1.00 | 67.31 | A |
| 1350 | CA | MET | 691 | 59.941 | 3.402 | 10.241 | 1.00 | 68.50 | A |
| 1351 | CB | MET | 691 | 61.241 | 2.753 | 10.733 | 1.00 | 70.62 | A |
| 1352 | CG | MET | 691 | 62.461 | 3.684 | 10.735 | 1.00 | 73.77 | A |
| 1353 | SD | MET | 691 | 62.232 | 5.254 | 11.652 | 1.00 | 79.07 | A |
| 1354 | CE | MET | 691 | 61.883 | 4.634 | 13.390 | 1.00 | 75.73 | A |
| 1355 | C | MET | 691 | 60.115 | 3.933 | 8.813 | 1.00 | 67.84 | A |
| 1356 | O | MET | 691 | 60.691 | 5.005 | 8.602 | 1.00 | 67.23 | A |
| 1357 | N | THR | 692 | 59.617 | 3.186 | 7.835 | 1.00 | 67.57 | A |
| 1358 | CA | THR | 692 | 59.706 | 3.621 | 6.452 | 1.00 | 66.26 | A |
| 1359 | CB | THR | 692 | 59.374 | 2.483 | 5.479 | 1.00 | 66.50 | A |
| 1360 | OG1 | THR | 692 | 60.548 | 1.686 | 5.260 | 1.00 | 67.25 | A |
| 1361 | CG2 | THR | 692 | 58.880 | 3.037 | 4.162 | 1.00 | 66.69 | A |
| 1362 | C | THR | 692 | 58.730 | 4.770 | 6.255 | 1.00 | 65.64 | A |
| 1363 | O | THR | 692 | 59.078 | 5.784 | 5.642 | 1.00 | 65.69 | A |
| 1364 | N | TYR | 693 | 57.513 | 4.621 | 6.779 | 1.00 | 64.80 | A |
| 1365 | CA | TYR | 693 | 56.536 | 5.701 | 6.664 | 1.00 | 64.71 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1366 | CB | TYR | 693 | 55.103 | 5.201 | 6.886 | 1.00 | 64.94 | A |
| 1367 | CG | TYR | 693 | 54.677 | 4.298 | 5.766 | 1.00 | 66.69 | A |
| 1368 | CD1 | TYR | 693 | 54.722 | 4.748 | 4.440 | 1.00 | 67.11 | A |
| 1369 | CE1 | TYR | 693 | 54.519 | 3.881 | 3.386 | 1.00 | 67.34 | A |
| 1370 | CD2 | TYR | 693 | 54.393 | 2.955 | 6.001 | 1.00 | 66.21 | A |
| 1371 | CE2 | TYR | 693 | 54.190 | 2.078 | 4.954 | 1.00 | 67.07 | A |
| 1372 | CZ | TYR | 693 | 54.264 | 2.543 | 3.647 | 1.00 | 68.00 | A |
| 1373 | OH | TYR | 693 | 54.152 | 1.651 | 2.603 | 1.00 | 69.03 | A |
| 1374 | C | TYR | 693 | 56.824 | 6.877 | 7.589 | 1.00 | 64.42 | A |
| 1375 | O | TYR | 693 | 56.325 | 7.969 | 7.344 | 1.00 | 65.00 | A |
| 1376 | N | ILE | 694 | 57.605 | 6.694 | 8.650 | 1.00 | 63.88 | A |
| 1377 | CA | ILE | 694 | 57.900 | 7.859 | 9.472 | 1.00 | 62.99 | A |
| 1378 | CB | ILE | 694 | 58.571 | 7.515 | 10.821 | 1.00 | 62.36 | A |
| 1379 | CG2 | ILE | 694 | 59.061 | 8.789 | 11.501 | 1.00 | 61.36 | A |
| 1380 | CG1 | ILE | 694 | 57.553 | 6.852 | 11.746 | 1.00 | 62.16 | A |
| 1381 | CD1 | ILE | 694 | 58.137 | 6.409 | 13.056 | 1.00 | 61.49 | A |
| 1382 | C | ILE | 694 | 58.853 | 8.701 | 8.640 | 1.00 | 62.62 | A |
| 1383 | O | ILE | 694 | 58.890 | 9.919 | 8.757 | 1.00 | 62.37 | A |
| 1384 | N | LYS | 695 | 59.611 | 8.042 | 7.773 | 1.00 | 63.30 | A |
| 1385 | CA | LYS | 695 | 60.541 | 8.761 | 6.924 | 1.00 | 64.01 | A |
| 1386 | CB | LYS | 695 | 61.656 | 7.820 | 6.452 | 1.00 | 65.96 | A |
| 1387 | CG | LYS | 695 | 62.589 | 7.316 | 7.569 | 1.00 | 68.75 | A |
| 1388 | CD | LYS | 695 | 63.633 | 6.371 | 6.975 | 1.00 | 71.84 | A |
| 1389 | CE | LYS | 695 | 64.652 | 5.841 | 7.993 | 1.00 | 74.93 | A |
| 1390 | NZ | LYS | 695 | 65.646 | 4.889 | 7.367 | 1.00 | 75.72 | A |
| 1391 | C | LYS | 695 | 59.804 | 9.390 | 5.733 | 1.00 | 63.18 | A |
| 1392 | O | LYS | 695 | 60.101 | 10.514 | 5.335 | 1.00 | 63.38 | A |
| 1393 | N | GLU | 696 | 58.837 | 8.671 | 5.177 | 1.00 | 62.48 | A |
| 1394 | CA | GLU | 696 | 58.059 | 9.179 | 4.058 | 1.00 | 62.80 | A |
| 1395 | CB | GLU | 696 | 56.969 | 8.156 | 3.715 | 1.00 | 62.84 | A |
| 1396 | CG | GLU | 696 | 56.901 | 7.590 | 2.268 | 1.00 | 65.44 | A |
| 1397 | CD | GLU | 696 | 57.947 | 8.126 | 1.269 | 1.00 | 68.55 | A |
| 1398 | OE1 | GLU | 696 | 59.132 | 7.725 | 1.358 | 1.00 | 69.98 | A |
| 1399 | OE2 | GLU | 696 | 57.574 | 8.933 | 0.380 | 1.00 | 69.05 | A |
| 1400 | C | GLU | 696 | 57.442 | 10.532 | 4.494 | 1.00 | 63.48 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1401 | O | GLU | 696 | 57.417 | 11.494 | 3.721 | 1.00 | 63.32 | A |
| 1402 | N | LEU | 697 | 56.960 | 10.593 | 5.743 | 1.00 | 63.97 | A |
| 1403 | CA | LEU | 697 | 56.350 | 11.799 | 6.336 | 1.00 | 63.42 | A |
| 1404 | CB | LEU | 697 | 55.832 | 11.516 | 7.764 | 1.00 | 62.28 | A |
| 1405 | CG | LEU | 697 | 55.230 | 12.693 | 8.555 | 1.00 | 61.21 | A |
| 1406 | CD1 | LEU | 697 | 53.962 | 13.141 | 7.867 | 1.00 | 59.88 | A |
| 1407 | CD2 | LEU | 697 | 54.919 | 12.305 | 10.000 | 1.00 | 61.08 | A |
| 1408 | C | LEU | 697 | 57.360 | 12.939 | 6.401 | 1.00 | 63.31 | A |
| 1409 | O | LEU | 697 | 57.017 | 14.101 | 6.195 | 1.00 | 63.33 | A |
| 1410 | N | GLY | 698 | 58.604 | 12.604 | 6.708 | 1.00 | 63.85 | A |
| 1411 | CA | GLY | 698 | 59.631 | 13.623 | 6.771 | 1.00 | 64.63 | A |
| 1412 | C | GLY | 698 | 59.873 | 14.137 | 5.370 | 1.00 | 65.37 | A |
| 1413 | O | GLY | 698 | 60.160 | 15.311 | 5.164 | 1.00 | 65.08 | A |
| 1414 | N | LYS | 699 | 59.754 | 13.247 | 4.394 | 1.00 | 66.39 | A |
| 1415 | CA | LYS | 699 | 59.947 | 13.649 | 3.011 | 1.00 | 68.06 | A |
| 1416 | CB | LYS | 699 | 59.919 | 12.437 | 2.109 | 1.00 | 68.79 | A |
| 1417 | CG | LYS | 699 | 61.185 | 11.646 | 2.136 | 1.00 | 71.18 | A |
| 1418 | CD | LYS | 699 | 60.963 | 10.397 | 1.352 | 1.00 | 71.92 | A |
| 1419 | CE | LYS | 699 | 62.237 | 9.864 | 0.785 | 1.00 | 72.64 | A |
| 1420 | NZ | LYS | 699 | 61.851 | 8.836 | -0.208 | 1.00 | 73.27 | A |
| 1421 | C | LYS | 699 | 58.845 | 14.604 | 2.604 | 1.00 | 69.09 | A |
| 1422 | O | LYS | 699 | 59.098 | 15.610 | 1.933 | 1.00 | 69.42 | A |
| 1423 | N | ALA | 700 | 57.620 | 14.277 | 3.011 | 1.00 | 68.96 | A |
| 1424 | CA | ALA | 700 | 56.471 | 15.105 | 2.711 | 1.00 | 68.91 | A |
| 1425 | CB | ALA | 700 | 55.235 | 14.525 | 3.356 | 1.00 | 68.39 | A |
| 1426 | C | ALA | 700 | 56.747 | 16.496 | 3.250 | 1.00 | 70.45 | A |
| 1427 | O | ALA | 700 | 56.729 | 17.464 | 2.500 | 1.00 | 70.63 | A |
| 1428 | N | ILE | 701 | 57.017 | 16.581 | 4.556 | 1.00 | 72.26 | A |
| 1429 | CA | ILE | 701 | 57.319 | 17.847 | 5.249 | 1.00 | 74.11 | A |
| 1430 | CB | ILE | 701 | 57.961 | 17.556 | 6.637 | 1.00 | 72.88 | A |
| 1431 | CG2 | ILE | 701 | 58.462 | 18.835 | 7.280 | 1.00 | 71.61 | A |
| 1432 | CG1 | ILE | 701 | 56.943 | 16.850 | 7.529 | 1.00 | 72.83 | A |
| 1433 | CD1 | ILE | 701 | 57.216 | 16.974 | 9.009 | 1.00 | 72.05 | A |
| 1434 | C | ILE | 701 | 58.282 | 18.721 | 4.445 | 1.00 | 76.37 | A |
| 1435 | O | ILE | 701 | 57.977 | 19.860 | 4.077 | 1.00 | 75.84 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1436 | N | VAL | 702 | 59.453 | 18.162 | 4.193 | 1.00 | 79.02 | A |
| 1437 | CA | VAL | 702 | 60.505 | 18.815 | 3.441 | 1.00 | 83.18 | A |
| 1438 | CB | VAL | 702 | 61.653 | 17.827 | 3.195 | 1.00 | 83.12 | A |
| 1439 | CG1 | VAL | 702 | 62.631 | 18.405 | 2.202 | 1.00 | 83.67 | A |
| 1440 | CG2 | VAL | 702 | 62.348 | 17.502 | 4.507 | 1.00 | 82.28 | A |
| 1441 | C | VAL | 702 | 60.052 | 19.381 | 2.094 | 1.00 | 85.97 | A |
| 1442 | O | VAL | 702 | 60.257 | 20.561 | 1.813 | 1.00 | 86.89 | A |
| 1443 | N | LYS | 703 | 59.466 | 18.527 | 1.262 | 1.00 | 89.29 | A |
| 1444 | CA | LYS | 703 | 58.980 | 18.906 | -0.070 | 1.00 | 92.79 | A |
| 1445 | CB | LYS | 703 | 58.138 | 17.740 | -0.638 | 1.00 | 90.47 | A |
| 1446 | CG | LYS | 703 | 58.014 | 17.642 | -2.173 | 1.00 | 89.42 | A |
| 1447 | CD | LYS | 703 | 57.321 | 16.325 | -2.633 | 1.00 | 88.53 | A |
| 1448 | CE | LYS | 703 | 57.388 | 16.120 | -4.166 | 1.00 | 87.74 | A |
| 1449 | NZ | LYS | 703 | 56.350 | 15.184 | -4.728 | 1.00 | 87.46 | A |
| 1450 | C | LYS | 703 | 58.139 | 20.197 | 0.041 | 1.00 | 96.58 | A |
| 1451 | O | LYS | 703 | 58.300 | 21.149 | -0.738 | 1.00 | 96.91 | A |
| 1452 | N | ARG | 704 | 57.266 | 20.194 | 1.047 | 1.00 | 100.79 | A |
| 1453 | CA | ARG | 704 | 56.327 | 21.260 | 1.393 | 1.00 | 104.87 | A |
| 1454 | CB | ARG | 704 | 55.784 | 20.985 | 2.806 | 1.00 | 105.73 | A |
| 1455 | CG | ARG | 704 | 54.977 | 22.104 | 3.412 | 1.00 | 107.49 | A |
| 1456 | CD | ARG | 704 | 54.209 | 21.704 | 4.659 | 1.00 | 109.11 | A |
| 1457 | NE | ARG | 704 | 53.494 | 22.890 | 5.106 | 1.00 | 111.38 | A |
| 1458 | CZ | ARG | 704 | 54.049 | 23.883 | 5.794 | 1.00 | 112.38 | A |
| 1459 | NH1 | ARG | 704 | 55.331 | 23.825 | 6.150 | 1.00 | 112.57 | A |
| 1460 | NH2 | ARG | 704 | 53.349 | 24.978 | 6.055 | 1.00 | 112.76 | A |
| 1461 | C | ARG | 704 | 56.906 | 22.651 | 1.359 | 1.00 | 107.58 | A |
| 1462 | O | ARG | 704 | 56.307 | 23.618 | 0.874 | 1.00 | 108.13 | A |
| 1463 | N | GLU | 705 | 58.102 | 22.745 | 1.878 | 1.00 | 110.53 | A |
| 1464 | CA | GLU | 705 | 58.710 | 24.028 | 1.971 | 1.00 | 113.44 | A |
| 1465 | CB | GLU | 705 | 58.866 | 24.321 | 3.440 | 1.00 | 114.43 | A |
| 1466 | CG | GLU | 705 | 59.182 | 23.034 | 4.168 | 1.00 | 115.34 | A |
| 1467 | CD | GLU | 705 | 59.016 | 23.153 | 5.646 | 1.00 | 116.03 | A |
| 1468 | OE1 | GLU | 705 | 59.832 | 23.860 | 6.269 | 1.00 | 116.67 | A |
| 1469 | OE2 | GLU | 705 | 58.065 | 22.547 | 6.184 | 1.00 | 116.66 | A |
| 1470 | C | GLU | 705 | 60.034 | 24.037 | 1.301 | 1.00 | 114.62 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1471 | O | GLU | 705 | 60.574 | 23.003 | 0.902 | 1.00 | 114.77 | A |
| 1472 | N | GLY | 706 | 60.543 | 25.241 | 1.152 | 1.00 | 116.18 | A |
| 1473 | CA | GLY | 706 | 61.852 | 25.398 | 0.588 | 1.00 | 118.63 | A |
| 1474 | C | GLY | 706 | 62.672 | 25.661 | 1.834 | 1.00 | 120.26 | A |
| 1475 | O | GLY | 706 | 63.853 | 25.330 | 1.897 | 1.00 | 121.20 | A |
| 1476 | N | ASN | 707 | 62.000 | 26.216 | 2.845 | 1.00 | 120.91 | A |
| 1477 | CA | ASN | 707 | 62.581 | 26.609 | 4.137 | 1.00 | 121.19 | A |
| 1478 | CB | ASN | 707 | 61.710 | 27.710 | 4.716 | 1.00 | 122.25 | A |
| 1479 | CG | ASN | 707 | 60.240 | 27.387 | 4.585 | 1.00 | 123.17 | A |
| 1480 | OD1 | ASN | 707 | 59.792 | 26.332 | 5.026 | 1.00 | 123.06 | A |
| 1481 | ND2 | ASN | 707 | 59.481 | 28.285 | 3.963 | 1.00 | 123.82 | A |
| 1482 | C | ASN | 707 | 62.791 | 25.529 | 5.210 | 1.00 | 120.52 | A |
| 1483 | O | ASN | 707 | 61.919 | 25.294 | 6.052 | 1.00 | 120.14 | A |
| 1484 | N | SER | 708 | 63.978 | 24.927 | 5.197 | 1.00 | 120.09 | A |
| 1485 | CA | SER | 708 | 64.367 | 23.864 | 6.126 | 1.00 | 119.78 | A |
| 1486 | CB | SER | 708 | 65.610 | 23.146 | 5.586 | 1.00 | 120.58 | A |
| 1487 | OG | SER | 708 | 65.402 | 22.691 | 4.257 | 1.00 | 121.41 | A |
| 1488 | C | SER | 708 | 64.639 | 24.344 | 7.555 | 1.00 | 118.82 | A |
| 1489 | O | SER | 708 | 65.222 | 23.619 | 8.368 | 1.00 | 118.32 | A |
| 1490 | N | SER | 709 | 64.208 | 25.565 | 7.850 | 1.00 | 117.85 | A |
| 1491 | CA | SER | 709 | 64.394 | 26.147 | 9.174 | 1.00 | 116.73 | A |
| 1492 | CB | SER | 709 | 64.188 | 27.660 | 9.091 | 1.00 | 117.22 | A |
| 1493 | OG | SER | 709 | 65.042 | 28.217 | 8.105 | 1.00 | 118.10 | A |
| 1494 | C | SER | 709 | 63.429 | 25.517 | 10.191 | 1.00 | 115.52 | A |
| 1495 | O | SER | 709 | 63.830 | 25.193 | 11.317 | 1.00 | 115.87 | A |
| 1496 | N | GLN | 710 | 62.168 | 25.339 | 9.791 | 1.00 | 112.97 | A |
| 1497 | CA | GLN | 710 | 61.169 | 24.729 | 10.669 | 1.00 | 109.62 | A |
| 1498 | CB | GLN | 710 | 59.958 | 25.657 | 10.849 | 1.00 | 111.67 | A |
| 1499 | CG | GLN | 710 | 60.193 | 26.759 | 11.894 | 1.00 | 113.15 | A |
| 1500 | CD | GLN | 710 | 58.910 | 27.387 | 12.420 | 1.00 | 114.64 | A |
| 1501 | OE1 | GLN | 710 | 58.939 | 28.182 | 13.364 | 1.00 | 115.78 | A |
| 1502 | NE2 | GLN | 710 | 57.776 | 27.035 | 11.815 | 1.00 | 114.86 | A |
| 1503 | C | GLN | 710 | 60.720 | 23.340 | 10.198 | 1.00 | 106.11 | A |
| 1504 | O | GLN | 710 | 59.542 | 22.973 | 10.293 | 1.00 | 105.36 | A |
| 1505 | N | ASN | 711 | 61.682 | 22.573 | 9.692 | 1.00 | 101.53 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1506 | CA | ASN | 711 | 61.406 | 21.225 | 9.242 | 1.00 | 96.35 | A |
| 1507 | CB | ASN | 711 | 62.540 | 20.705 | 8.362 | 1.00 | 95.93 | A |
| 1508 | CG | ASN | 711 | 62.287 | 20.980 | 6.892 | 1.00 | 96.22 | A |
| 1509 | OD1 | ASN | 711 | 63.138 | 20.721 | 6.041 | 1.00 | 96.50 | A |
| 1510 | ND2 | ASN | 711 | 61.101 | 21.502 | 6.587 | 1.00 | 95.88 | A |
| 1511 | C | ASN | 711 | 61.200 | 20.339 | 10.461 | 1.00 | 93.06 | A |
| 1512 | O | ASN | 711 | 60.173 | 19.673 | 10.568 | 1.00 | 92.98 | A |
| 1513 | N | TRP | 712 | 62.142 | 20.328 | 11.395 | 1.00 | 88.90 | A |
| 1514 | CA | TRP | 712 | 61.896 | 19.508 | 12.573 | 1.00 | 85.12 | A |
| 1515 | CB | TRP | 712 | 63.139 | 19.345 | 13.448 | 1.00 | 82.71 | A |
| 1516 | CG | TRP | 712 | 64.206 | 18.565 | 12.816 | 1.00 | 78.88 | A |
| 1517 | CD2 | TRP | 712 | 64.273 | 17.140 | 12.692 | 1.00 | 77.30 | A |
| 1518 | CE2 | TRP | 712 | 65.454 | 16.845 | 11.979 | 1.00 | 77.10 | A |
| 1519 | CE3 | TRP | 712 | 63.495 | 16.079 | 13.175 | 1.00 | 76.79 | A |
| 1520 | CD1 | TRP | 712 | 65.278 | 19.061 | 12.155 | 1.00 | 77.85 | A |
| 1521 | NE1 | TRP | 712 | 66.032 | 18.038 | 11.638 | 1.00 | 77.39 | A |
| 1522 | CZ2 | TRP | 712 | 65.824 | 15.539 | 11.650 | 1.00 | 77.27 | A |
| 1523 | CZ3 | TRP | 712 | 63.869 | 14.773 | 12.857 | 1.00 | 77.22 | A |
| 1524 | CH2 | TRP | 712 | 65.043 | 14.516 | 12.126 | 1.00 | 76.39 | A |
| 1525 | C | TRP | 712 | 60.781 | 20.095 | 13.427 | 1.00 | 84.16 | A |
| 1526 | O | TRP | 712 | 60.240 | 19.394 | 14.280 | 1.00 | 84.15 | A |
| 1527 | N | GLN | 713 | 60.426 | 21.359 | 13.233 | 1.00 | 83.31 | A |
| 1528 | CA | GLN | 713 | 59.352 | 21.887 | 14.059 | 1.00 | 82.44 | A |
| 1529 | CB | GLN | 713 | 59.316 | 23.418 | 14.051 | 1.00 | 85.53 | A |
| 1530 | CG | GLN | 713 | 58.358 | 24.025 | 15.110 | 1.00 | 89.96 | A |
| 1531 | CD | GLN | 713 | 58.571 | 23.473 | 16.534 | 1.00 | 92.69 | A |
| 1532 | OE1 | GLN | 713 | 57.728 | 22.737 | 17.066 | 1.00 | 92.02 | A |
| 1533 | NE2 | GLN | 713 | 59.701 | 23.832 | 17.152 | 1.00 | 93.58 | A |
| 1534 | C | GLN | 713 | 58.014 | 21.327 | 13.595 | 1.00 | 80.69 | A |
| 1535 | O | GLN | 713 | 57.169 | 20.991 | 14.417 | 1.00 | 80.26 | A |
| 1536 | N | ARG | 714 | 57.840 | 21.202 | 12.281 | 1.00 | 78.63 | A |
| 1537 | CA | ARG | 714 | 56.596 | 20.673 | 11.715 | 1.00 | 76.05 | A |
| 1538 | CB | ARG | 714 | 56.511 | 20.977 | 10.226 | 1.00 | 75.18 | A |
| 1539 | CG | ARG | 714 | 55.148 | 20.658 | 9.665 | 1.00 | 74.15 | A |
| 1540 | CD | ARG | 714 | 55.015 | 21.162 | 8.264 | 1.00 | 73.11 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1541 | NE | ARG | 714 | 53.718 | 20.828 | 7.681 | 1.00 | 72.23 | A |
| 1542 | CZ | ARG | 714 | 52.573 | 21.419 | 8.001 | 1.00 | 71.10 | A |
| 1543 | NH1 | ARG | 714 | 52.542 | 22.374 | 8.917 | 1.00 | 70.94 | A |
| 1544 | NH2 | ARG | 714 | 51.459 | 21.068 | 7.372 | 1.00 | 69.74 | A |
| 1545 | C | ARG | 714 | 56.448 | 19.173 | 11.918 | 1.00 | 75.24 | A |
| 1546 | O | ARG | 714 | 55.332 | 18.655 | 12.017 | 1.00 | 75.01 | A |
| 1547 | N | PHE | 715 | 57.573 | 18.475 | 11.953 | 1.00 | 74.26 | A |
| 1548 | CA | PHE | 715 | 57.544 | 17.043 | 12.167 | 1.00 | 73.79 | A |
| 1549 | CB | PHE | 715 | 58.935 | 16.456 | 12.019 | 1.00 | 73.23 | A |
| 1550 | CG | PHE | 715 | 58.969 | 14.973 | 12.143 | 1.00 | 73.54 | A |
| 1551 | CD1 | PHE | 715 | 58.715 | 14.169 | 11.038 | 1.00 | 74.69 | A |
| 1552 | CD2 | PHE | 715 | 59.213 | 14.372 | 13.367 | 1.00 | 74.25 | A |
| 1553 | CE1 | PHE | 715 | 58.720 | 12.791 | 11.148 | 1.00 | 74.64 | A |
| 1554 | CE2 | PHE | 715 | 59.219 | 12.990 | 13.494 | 1.00 | 74.97 | A |
| 1555 | CZ | PHE | 715 | 58.968 | 12.196 | 12.383 | 1.00 | 75.12 | A |
| 1556 | C | PHE | 715 | 57.049 | 16.804 | 13.586 | 1.00 | 73.88 | A |
| 1557 | O | PHE | 715 | 56.413 | 15.791 | 13.875 | 1.00 | 73.98 | A |
| 1558 | N | TYR | 716 | 57.356 | 17.756 | 14.466 | 1.00 | 74.00 | A |
| 1559 | CA | TYR | 716 | 56.950 | 17.682 | 15.862 | 1.00 | 73.76 | A |
| 1560 | CB | TYR | 716 | 57.698 | 18.738 | 16.681 | 1.00 | 74.90 | A |
| 1561 | CG | TYR | 716 | 57.303 | 18.724 | 18.134 | 1.00 | 76.12 | A |
| 1562 | CD1 | TYR | 716 | 57.656 | 17.657 | 18.961 | 1.00 | 76.63 | A |
| 1563 | CE1 | TYR | 716 | 57.199 | 17.579 | 20.270 | 1.00 | 77.14 | A |
| 1564 | CD2 | TYR | 716 | 56.493 | 19.725 | 18.659 | 1.00 | 76.34 | A |
| 1565 | CE2 | TYR | 716 | 56.028 | 19.657 | 19.964 | 1.00 | 77.79 | A |
| 1566 | CZ | TYR | 716 | 56.383 | 18.581 | 20.764 | 1.00 | 77.71 | A |
| 1567 | OH | TYR | 716 | 55.878 | 18.483 | 22.037 | 1.00 | 77.98 | A |
| 1568 | C | TYR | 716 | 55.445 | 17.900 | 16.008 | 1.00 | 73.41 | A |
| 1569 | O | TYR | 716 | 54.773 | 17.154 | 16.720 | 1.00 | 73.40 | A |
| 1570 | N | GLN | 717 | 54.932 | 18.930 | 15.341 | 1.00 | 72.86 | A |
| 1571 | CA | GLN | 717 | 53.506 | 19.246 | 15.381 | 1.00 | 72.51 | A |
| 1572 | CB | GLN | 717 | 53.199 | 20.517 | 14.570 | 1.00 | 74.03 | A |
| 1573 | CG | GLN | 717 | 53.717 | 21.832 | 15.172 | 1.00 | 77.70 | A |
| 1574 | CD | GLN | 717 | 53.451 | 23.065 | 14.288 | 1.00 | 80.63 | A |
| 1575 | OE1 | GLN | 717 | 53.797 | 23.094 | 13.098 | 1.00 | 81.29 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1576 | NE2 | GLN | 717 | 52.848 | 24.096 | 14.881 | 1.00 | 81.96 | A |
| 1577 | C | GLN | 717 | 52.728 | 18.074 | 14.791 | 1.00 | 71.31 | A |
| 1578 | O | GLN | 717 | 51.828 | 17.516 | 15.418 | 1.00 | 71.30 | A |
| 1579 | N | LEU | 718 | 53.089 | 17.687 | 13.575 | 1.00 | 68.89 | A |
| 1580 | CA | LEU | 718 | 52.396 | 16.596 | 12.931 | 1.00 | 66.70 | A |
| 1581 | CB | LEU | 718 | 53.019 | 16.309 | 11.578 | 1.00 | 65.16 | A |
| 1582 | CG | LEU | 718 | 52.851 | 17.456 | 10.603 | 1.00 | 64.70 | A |
| 1583 | CD1 | LEU | 718 | 53.411 | 17.030 | 9.276 | 1.00 | 64.31 | A |
| 1584 | CD2 | LEU | 718 | 51.387 | 17.819 | 10.472 | 1.00 | 65.25 | A |
| 1585 | C | LEU | 718 | 52.322 | 15.303 | 13.717 | 1.00 | 66.09 | A |
| 1586 | O | LEU | 718 | 51.271 | 14.677 | 13.761 | 1.00 | 66.32 | A |
| 1587 | N | THR | 719 | 53.429 | 14.894 | 14.331 | 1.00 | 66.23 | A |
| 1588 | CA | THR | 719 | 53.441 | 13.637 | 15.074 | 1.00 | 67.26 | A |
| 1589 | CB | THR | 719 | 54.880 | 13.161 | 15.289 | 1.00 | 67.26 | A |
| 1590 | OG1 | THR | 719 | 55.556 | 14.070 | 16.161 | 1.00 | 67.19 | A |
| 1591 | CG2 | THR | 719 | 55.618 | 13.100 | 13.947 | 1.00 | 65.43 | A |
| 1592 | C | THR | 719 | 52.715 | 13.758 | 16.417 | 1.00 | 68.33 | A |
| 1593 | O | THR | 719 | 52.254 | 12.758 | 16.995 | 1.00 | 67.95 | A |
| 1594 | N | LYS | 720 | 52.623 | 15.003 | 16.885 | 1.00 | 69.49 | A |
| 1595 | CA | LYS | 720 | 51.937 | 15.361 | 18.120 | 1.00 | 70.54 | A |
| 1596 | CB | LYS | 720 | 52.232 | 16.839 | 18.424 | 1.00 | 73.28 | A |
| 1597 | CG | LYS | 720 | 52.196 | 17.259 | 19.894 | 1.00 | 77.37 | A |
| 1598 | CD | LYS | 720 | 53.224 | 16.488 | 20.738 | 1.00 | 80.58 | A |
| 1599 | CE | LYS | 720 | 52.565 | 15.638 | 21.853 | 1.00 | 82.40 | A |
| 1600 | NZ | LYS | 720 | 51.608 | 14.564 | 21.400 | 1.00 | 81.87 | A |
| 1601 | C | LYS | 720 | 50.438 | 15.137 | 17.809 | 1.00 | 69.73 | A |
| 1602 | O | LYS | 720 | 49.680 | 14.604 | 18.629 | 1.00 | 70.78 | A |
| 1603 | N | LEU | 721 | 50.029 | 15.530 | 16.599 | 1.00 | 67.65 | A |
| 1604 | CA | LEU | 721 | 48.649 | 15.353 | 16.146 | 1.00 | 65.04 | A |
| 1605 | CB | LEU | 721 | 48.450 | 15.934 | 14.740 | 1.00 | 63.50 | A |
| 1606 | CG | LEU | 721 | 47.028 | 16.423 | 14.419 | 1.00 | 62.27 | A |
| 1607 | CD1 | LEU | 721 | 46.820 | 16.517 | 12.919 | 1.00 | 60.98 | A |
| 1608 | CD2 | LEU | 721 | 46.008 | 15.489 | 15.027 | 1.00 | 62.51 | A |
| 1609 | C | LEU | 721 | 48.348 | 13.860 | 16.093 | 1.00 | 64.45 | A |
| 1610 | O | LEU | 721 | 47.318 | 13.407 | 16.581 | 1.00 | 65.22 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1611 | N | LEU | 722 | 49.249 | 13.108 | 15.467 | 1.00 | 63.64 | A |
| 1612 | CA | LEU | 722 | 49.095 | 11.668 | 15.344 | 1.00 | 62.92 | A |
| 1613 | CB | LEU | 722 | 50.359 | 11.048 | 14.764 | 1.00 | 60.54 | A |
| 1614 | CG | LEU | 722 | 50.490 | 11.222 | 13.260 | 1.00 | 57.75 | A |
| 1615 | CD1 | LEU | 722 | 51.771 | 10.603 | 12.779 | 1.00 | 55.67 | A |
| 1616 | CD2 | LEU | 722 | 49.301 | 10.577 | 12.589 | 1.00 | 55.86 | A |
| 1617 | C | LEU | 722 | 48.832 | 11.061 | 16.695 | 1.00 | 64.43 | A |
| 1618 | O | LEU | 722 | 47.985 | 10.175 | 16.833 | 1.00 | 64.56 | A |
| 1619 | N | ASP | 723 | 49.575 | 11.544 | 17.689 | 1.00 | 66.00 | A |
| 1620 | CA | ASP | 723 | 49.440 | 11.071 | 19.063 | 1.00 | 67.32 | A |
| 1621 | CB | ASP | 723 | 50.504 | 11.708 | 19.957 | 1.00 | 68.83 | A |
| 1622 | CG | ASP | 723 | 51.815 | 10.946 | 19.958 | 1.00 | 71.08 | A |
| 1623 | OD1 | ASP | 723 | 51.917 | 9.887 | 19.293 | 1.00 | 71.58 | A |
| 1624 | OD2 | ASP | 723 | 52.751 | 11.416 | 20.646 | 1.00 | 72.22 | A |
| 1625 | C | ASP | 723 | 48.061 | 11.390 | 19.635 | 1.00 | 67.50 | A |
| 1626 | O | ASP | 723 | 47.419 | 10.530 | 20.233 | 1.00 | 67.38 | A |
| 1627 | N | SER | 724 | 47.605 | 12.622 | 19.460 | 1.00 | 67.60 | A |
| 1628 | CA | SER | 724 | 46.306 | 12.979 | 19.998 | 1.00 | 68.26 | A |
| 1629 | CB | SER | 724 | 46.055 | 14.481 | 19.846 | 1.00 | 69.65 | A |
| 1630 | OG | SER | 724 | 46.043 | 14.873 | 18.485 | 1.00 | 72.65 | A |
| 1631 | C | SER | 724 | 45.170 | 12.186 | 19.355 | 1.00 | 68.41 | A |
| 1632 | O | SER | 724 | 44.018 | 12.323 | 19.749 | 1.00 | 68.56 | A |
| 1633 | N | MET | 725 | 45.477 | 11.354 | 18.361 | 1.00 | 68.11 | A |
| 1634 | CA | MET | 725 | 44.421 | 10.567 | 17.737 | 1.00 | 66.74 | A |
| 1635 | CB | MET | 725 | 44.856 | 10.002 | 16.384 | 1.00 | 65.02 | A |
| 1636 | CG | MET | 725 | 44.938 | 11.011 | 15.260 | 1.00 | 63.01 | A |
| 1637 | SD | MET | 725 | 43.372 | 11.815 | 14.850 | 1.00 | 61.30 | A |
| 1638 | CE | MET | 725 | 42.296 | 10.467 | 14.601 | 1.00 | 60.48 | A |
| 1639 | C | MET | 725 | 44.038 | 9.417 | 18.650 | 1.00 | 67.46 | A |
| 1640 | O | MET | 725 | 42.928 | 8.894 | 18.555 | 1.00 | 68.23 | A |
| 1641 | N | HIS | 726 | 44.948 | 9.000 | 19.524 | 1.00 | 67.20 | A |
| 1642 | CA | HIS | 726 | 44.596 | 7.924 | 20.433 | 1.00 | 67.38 | A |
| 1643 | CB | HIS | 726 | 45.810 | 7.409 | 21.227 | 1.00 | 64.41 | A |
| 1644 | CG | HIS | 726 | 46.804 | 6.622 | 20.417 | 1.00 | 60.65 | A |
| 1645 | CD2 | HIS | 726 | 48.071 | 6.926 | 20.043 | 1.00 | 59.49 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1646 | ND1 | HIS | 726 | 46.566 | 5.346 | 19.969 | 1.00 | 59.00 | A |
| 1647 | CE1 | HIS | 726 | 47.649 | 4.886 | 19.352 | 1.00 | 58.49 | A |
| 1648 | NE2 | HIS | 726 | 48.571 | 5.825 | 19.385 | 1.00 | 58.18 | A |
| 1649 | C | HIS | 726 | 43.563 | 8.519 | 21.389 | 1.00 | 69.98 | A |
| 1650 | O | HIS | 726 | 42.660 | 7.820 | 21.825 | 1.00 | 71.06 | A |
| 1651 | N | GLU | 727 | 43.679 | 9.811 | 21.704 | 1.00 | 73.24 | A |
| 1652 | CA | GLU | 727 | 42.711 | 10.453 | 22.604 | 1.00 | 76.05 | A |
| 1653 | CB | GLU | 727 | 43.221 | 11.847 | 23.070 | 1.00 | 78.51 | A |
| 1654 | CG | GLU | 727 | 42.424 | 13.114 | 22.613 | 1.00 | 84.07 | A |
| 1655 | CD | GLU | 727 | 43.301 | 14.398 | 22.491 | 1.00 | 87.46 | A |
| 1656 | OE1 | GLU | 727 | 44.458 | 14.402 | 22.987 | 1.00 | 88.22 | A |
| 1657 | OE2 | GLU | 727 | 42.829 | 15.406 | 21.901 | 1.00 | 87.58 | A |
| 1658 | C | GLU | 727 | 41.345 | 10.570 | 21.935 | 1.00 | 76.51 | A |
| 1659 | O | GLU | 727 | 40.334 | 10.193 | 22.520 | 1.00 | 76.62 | A |
| 1660 | N | VAL | 728 | 41.287 | 11.059 | 20.705 | 1.00 | 77.36 | A |
| 1661 | CA | VAL | 728 | 39.977 | 11.185 | 20.094 | 1.00 | 77.61 | A |
| 1662 | CB | VAL | 728 | 39.992 | 12.131 | 18.867 | 1.00 | 77.17 | A |
| 1663 | CG1 | VAL | 728 | 41.342 | 12.768 | 18.725 | 1.00 | 77.42 | A |
| 1664 | CG2 | VAL | 728 | 39.603 | 11.395 | 17.614 | 1.00 | 77.94 | A |
| 1665 | C | VAL | 728 | 39.371 | 9.829 | 19.740 | 1.00 | 78.18 | A |
| 1666 | O | VAL | 728 | 38.155 | 9.655 | 19.855 | 1.00 | 78.24 | A |
| 1667 | N | VAL | 729 | 40.188 | 8.864 | 19.325 | 1.00 | 79.36 | A |
| 1668 | CA | VAL | 729 | 39.628 | 7.555 | 19.008 | 1.00 | 80.65 | A |
| 1669 | CB | VAL | 729 | 40.693 | 6.546 | 18.563 | 1.00 | 80.37 | A |
| 1670 | CG1 | VAL | 729 | 40.092 | 5.154 | 18.530 | 1.00 | 80.54 | A |
| 1671 | CG2 | VAL | 729 | 41.194 | 6.905 | 17.183 | 1.00 | 81.12 | A |
| 1672 | C | VAL | 729 | 38.950 | 7.020 | 20.259 | 1.00 | 81.61 | A |
| 1673 | O | VAL | 729 | 37.795 | 6.606 | 20.220 | 1.00 | 82.29 | A |
| 1674 | N | GLU | 730 | 39.672 | 7.029 | 21.369 | 1.00 | 82.79 | A |
| 1675 | CA | GLU | 730 | 39.101 | 6.566 | 22.620 | 1.00 | 84.17 | A |
| 1676 | CB | GLU | 730 | 40.008 | 7.016 | 23.754 | 1.00 | 85.48 | A |
| 1677 | CG | GLU | 730 | 39.686 | 6.476 | 25.117 | 1.00 | 88.56 | A |
| 1678 | CD | GLU | 730 | 40.765 | 6.857 | 26.102 | 1.00 | 90.50 | A |
| 1679 | OE1 | GLU | 730 | 41.174 | 8.040 | 26.096 | 1.00 | 91.35 | A |
| 1680 | OE2 | GLU | 730 | 41.207 | 5.982 | 26.876 | 1.00 | 92.37 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1681 | C | GLU | 730 | 37.671 | 7.137 | 22.766 | 1.00 | 84.44 | A |
| 1682 | O | GLU | 730 | 36.709 | 6.375 | 22.882 | 1.00 | 84.46 | A |
| 1683 | N | ASN | 731 | 37.529 | 8.462 | 22.726 | 1.00 | 85.16 | A |
| 1684 | CA | ASN | 731 | 36.214 | 9.114 | 22.839 | 1.00 | 85.37 | A |
| 1685 | CB | ASN | 731 | 36.325 | 10.622 | 22.682 | 1.00 | 86.19 | A |
| 1686 | CG | ASN | 731 | 36.897 | 11.274 | 23.882 | 1.00 | 86.88 | A |
| 1687 | OD1 | ASN | 731 | 36.493 | 10.986 | 25.005 | 1.00 | 88.09 | A |
| 1688 | ND2 | ASN | 731 | 37.840 | 12.177 | 23.667 | 1.00 | 87.13 | A |
| 1689 | C | ASN | 731 | 35.213 | 8.666 | 21.803 | 1.00 | 85.12 | A |
| 1690 | O | ASN | 731 | 34.036 | 8.481 | 22.102 | 1.00 | 84.67 | A |
| 1691 | N | LEU | 732 | 35.676 | 8.545 | 20.573 | 1.00 | 84.94 | A |
| 1692 | CA | LEU | 732 | 34.789 | 8.139 | 19.511 | 1.00 | 85.15 | A |
| 1693 | CB | LEU | 732 | 35.490 | 8.262 | 18.167 | 1.00 | 83.41 | A |
| 1694 | CG | LEU | 732 | 35.707 | 9.696 | 17.691 | 1.00 | 82.65 | A |
| 1695 | CD1 | LEU | 732 | 36.028 | 9.661 | 16.211 | 1.00 | 82.43 | A |
| 1696 | CD2 | LEU | 732 | 34.458 | 10.530 | 17.939 | 1.00 | 81.93 | A |
| 1697 | C | LEU | 732 | 34.269 | 6.727 | 19.718 | 1.00 | 86.42 | A |
| 1698 | O | LEU | 732 | 33.076 | 6.476 | 19.564 | 1.00 | 85.99 | A |
| 1699 | N | LEU | 733 | 35.165 | 5.818 | 20.099 | 1.00 | 88.47 | A |
| 1700 | CA | LEU | 733 | 34.834 | 4.407 | 20.310 | 1.00 | 90.36 | A |
| 1701 | CB | LEU | 733 | 36.145 | 3.619 | 20.442 | 1.00 | 89.51 | A |
| 1702 | CG | LEU | 733 | 36.535 | 2.352 | 19.657 | 1.00 | 89.28 | A |
| 1703 | CD1 | LEU | 733 | 35.949 | 2.280 | 18.252 | 1.00 | 88.11 | A |
| 1704 | CD2 | LEU | 733 | 38.058 | 2.351 | 19.600 | 1.00 | 88.74 | A |
| 1705 | C | LEU | 733 | 33.886 | 4.098 | 21.488 | 1.00 | 92.45 | A |
| 1706 | O | LEU | 733 | 32.949 | 3.309 | 21.325 | 1.00 | 92.88 | A |
| 1707 | N | ASN | 734 | 34.111 1 | 4.699 | 22.662 | 1.00 | 94.41 | A |
| 1708 | CA | ASN | 734 | 33.239 | 4.446 | 23.826 | 1.00 | 96.34 | A |
| 1709 | CB | ASN | 734 | 33.569 | 5.380 | 24.990 | 1.00 | 98.01 | A |
| 1710 | CG | ASN | 734 | 34.943 | 5.145 | 25.555 | 1.00 | 100.48 | A |
| 1711 | OD1 | ASN | 734 | 35.434 | 4.013 | 25.576 | 1.00 | 101.32 | A |
| 1712 | ND2 | ASN | 734 | 35.571 | 6.209 | 26.039 | 1.00 | 101.67 | A |
| 1713 | C | ASN | 734 | 31.785 | 4.673 | 23.465 | 1.00 | 96.76 | A |
| 1714 | O | ASN | 734 | 30.903 | 3.870 | 23.769 | 1.00 | 96.85 | A |
| 1715 | N | TYR | 735 | 31.568 | 5.817 | 22.828 | 1.00 | 97.87 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1716 | CA | TYR | 735 | 30.272 | 6.279 | 22.366 | 1.00 | 98.53 | A |
| 1717 | CB | TYR | 735 | 30.446 | 7.677 | 21.791 | 1.00 | 100.86 | A |
| 1718 | CG | TYR | 735 | 29.259 | 8.590 | 21.899 | 1.00 | 103.46 | A |
| 1719 | CD1 | TYR | 735 | 27.960 | 8.131 | 21.665 | 1.00 | 104.41 | A |
| 1720 | CE1 | TYR | 735 | 26.864 | 8.992 | 21.773 | 1.00 | 106.25 | A |
| 1721 | CD2 | TYR | 735 | 29.445 | 9.927 | 22.244 | 1.00 | 104.91 | A |
| 1722 | CE2 | TYR | 735 | 28.374 | 10.799 | 22.357 | 1.00 | 106.48 | A |
| 1723 | CZ | TYR | 735 | 27.081 | 10.331 | 22.119 | 1.00 | 107.25 | A |
| 1724 | OH | TYR | 735 | 26.037 | 11.228 | 22.198 | 1.00 | 108.22 | A |
| 1725 | C | TYR | 735 | 29.767 | 5.363 | 21.258 | 1.00 | 97.72 | A |
| 1726 | O | TYR | 735 | 28.607 | 5.437 | 20.856 | 1.00 | 98.21 | A |
| 1727 | N | CYS | 736 | 30.652 | 4.519 | 20.744 | 1.00 | 96.14 | A |
| 1728 | CA | CYS | 736 | 30.293 | 3.611 | 19.666 | 1.00 | 94.51 | A |
| 1729 | CB | CYS | 736 | 31.473 | 3.426 | 18.719 | 1.00 | 93.20 | A |
| 1730 | SG | CYS | 736 | 31.103 | 2.373 | 17.310 | 1.00 | 89.33 | A |
| 1731 | C | CYS | 736 | 29.884 | 2.268 | 20.225 | 1.00 | 94.66 | A |
| 1732 | O | CYS | 736 | 28.961 | 1.629 | 19.719 | 1.00 | 93.86 | A |
| 1733 | N | PHE | 737 | 30.605 | 1.849 | 21.262 | 1.00 | 95.33 | A |
| 1734 | CA | PHE | 737 | 30.339 | 0.596 | 21.952 | 1.00 | 96.63 | A |
| 1735 | CB | PHE | 737 | 31.484 | 0.264 | 22.928 | 1.00 | 95.49 | A |
| 1736 | CG | PHE | 737 | 32.804 | -0.094 | 22.260 | 1.00 | 94.69 | A |
| 1737 | CD1 | PHE | 737 | 32.864 | -0.453 | 20.917 | 1.00 | 94.73 | A |
| 1738 | CD2 | PHE | 737 | 33.984 | -0.136 | 23.011 | 1.00 | 94.55 | A |
| 1739 | CE1 | PHE | 737 | 34.076 | -0.846 | 20.323 | 1.00 | 94.35 | A |
| 1740 | CE2 | PHE | 737 | 35.203 | -0.528 | 22.428 | 1.00 | 94.76 | A |
| 1741 | CZ | PHE | 737 | 35.247 | -0.885 | 21.083 | 1.00 | 94.34 | A |
| 1742 | C | PHE | 737 | 29.034 | 0.811 | 22.732 | 1.00 | 98.44 | A |
| 1743 | O | PHE | 737 | 28.105 | 0.003 | 22.647 | 1.00 | 98.71 | A |
| 1744 | N | GLN | 738 | 28.978 | 1.917 | 23.474 | 1.00 | 100.30 | A |
| 1745 | CA | GLN | 738 | 27.801 | 2.279 | 24.269 | 1.00 | 102.02 | A |
| 1746 | CB | GLN | 738 | 27.975 | 3.716 | 24.854 | 1.00 | 102.80 | A |
| 1747 | CG | GLN | 738 | 27.106 | 4.083 | 26.120 | 1.00 | 103.60 | A |
| 1748 | CD | GLN | 738 | 27.583 | 5.347 | 26.908 | 1.00 | 104.17 | A |
| 1749 | OE1 | GLN | 738 | 26.990 | 6.429 | 26.806 | 1.00 | 104.86 | A |
| 1750 | NE2 | GLN | 738 | 28.649 | 5.190 | 27.697 | 1.00 | 104.42 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1751 | C | GLN | 738 | 26.573 | 2.160 | 23.346 | 1.00 | 103.16 | A |
| 1752 | O | GLN | 738 | 25.699 | 1.328 | 23.592 | 1.00 | 103.35 | A |
| 1753 | N | THR | 739 | 26.519 | 2.936 | 22.268 | 1.00 | 104.30 | A |
| 1754 | CA | THR | 739 | 25.366 | 2.839 | 21.377 | 1.00 | 105.68 | A |
| 1755 | CB | THR | 739 | 25.511 | 3.778 | 20.162 | 1.00 | 105.48 | A |
| 1756 | OG1 | THR | 739 | 25.991 | 5.052 | 20.613 | 1.00 | 106.89 | A |
| 1757 | CG2 | THR | 739 | 24.153 | 3.998 | 19.494 | 1.00 | 105.18 | A |
| 1758 | C | THR | 739 | 25.164 | 1.375 | 20.940 | 1.00 | 106.67 | A |
| 1759 | O | THR | 739 | 24.187 | 0.754 | 21.346 | 1.00 | 107.32 | A |
| 1760 | N | PHE | 740 | 26.085 | 0.808 | 20.161 | 1.00 | 107.75 | A |
| 1761 | CA | PHE | 740 | 25.950 | -0.590 | 19.719 | 1.00 | 108.45 | A |
| 1762 | CB | PHE | 740 | 27.286 | -1.127 | 19.209 | 1.00 | 108.59 | A |
| 1763 | CG | PHE | 740 | 27.268 | -2.597 | 18.882 | 1.00 | 108.29 | A |
| 1764 | CD1 | PHE | 740 | 26.881 | -3.041 | 17.620 | 1.00 | 108.66 | A |
| 1765 | CD2 | PHE | 740 | 27.671 | -3.534 | 19.826 | 1.00 | 108.06 | A |
| 1766 | CE1 | PHE | 740 | 26.896 | -4.404 | 17.307 | 1.00 | 108.89 | A |
| 1767 | CE2 | PHE | 740 | 27.689 | -4.898 | 19.524 | 1.00 | 108.14 | A |
| 1768 | CZ | PHE | 740 | 27.306 | -5.332 | 18.261 | 1.00 | 108.57 | A |
| 1769 | C | PHE | 740 | 25.428 | -1.560 | 20.785 | 1.00 | 108.81 | A |
| 1770 | O | PHE | 740 | 24.731 | -2.519 | 20.468 | 1.00 | 108.65 | A |
| 1771 | N | LEU | 741 | 25.797 | -1.336 | 22.042 | 1.00 | 109.39 | A |
| 1772 | CA | LEU | 741 | 25.322 | -2.207 | 23.108 | 1.00 | 110.49 | A |
| 1773 | CB | LEU | 741 | 26.425 | -2.500 | 24.115 | 1.00 | 109.37 | A |
| 1774 | CG | LEU | 741 | 27.685 | -3.170 | 23.590 | 1.00 | 108.46 | A |
| 1775 | CD1 | LEU | 741 | 28.582 | -3.425 | 24.770 | 1.00 | 108.19 | A |
| 1776 | CD2 | LEU | 741 | 27.365 | -4.469 | 22.876 | 1.00 | 108.56 | A |
| 1777 | C | LEU | 741 | 24.182 | -1.518 | 23.822 | 1.00 | 112.02 | A |
| 1778 | O | LEU | 741 | 24.322 | -1.081 | 24.963 | 1.00 | 112.34 | A |
| 1779 | N | ASP | 742 | 23.056 | -1.419 | 23.128 | 1.00 | 113.82 | A |
| 1780 | CA | ASP | 742 | 21.864 | -0.782 | 23.665 | 1.00 | 115.47 | A |
| 1781 | CB | ASP | 742 | 22.182 | 0.625 | 24.156 | 1.00 | 116.03 | A |
| 1782 | CG | ASP | 742 | 20.959 | 1.333 | 24.685 | 1.00 | 117.14 | A |
| 1783 | OD1 | ASP | 742 | 21.000 | 2.572 | 24.835 | 1.00 | 117.82 | A |
| 1784 | OD2 | ASP | 742 | 19.954 | 0.643 | 24.954 | 1.00 | 117.31 | A |
| 1785 | C | ASP | 742 | 20.805 | -0.706 | 22.575 | 1.00 | 116.45 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1786 | O | ASP | 742 | 20.649 | 0.323 | 21.916 | 1.00 | 116.14 | A |
| 1787 | N | LYS | 743 | 20.084 | -1.810 | 22.392 | 1.00 | 118.04 | A |
| 1788 | CA | LYS | 743 | 19.032 | -1.910 | 21.386 | 1.00 | 119.53 | A |
| 1789 | CB | LYS | 743 | 18.431 | -3.330 | 21.389 | 1.00 | 120.38 | A |
| 1790 | CG | LYS | 743 | 18.709 | -4.182 | 20.127 | 1.00 | 121.19 | A |
| 1791 | CD | LYS | 743 | 18.195 | -3.494 | 18.848 | 1.00 | 121.91 | A |
| 1792 | CE | LYS | 743 | 18.211 | -4.400 | 17.597 | 1.00 | 122.04 | A |
| 1793 | NZ | LYS | 743 | 16.987 | -5.256 | 17.431 | 1.00 | 122.03 | A |
| 1794 | C | LYS | 743 | 17.930 | -0.876 | 21.627 | 1.00 | 119.72 | A |
| 1795 | O | LYS | 743 | 17.253 | -0.451 | 20.688 | 1.00 | 119.76 | A |
| 1796 | N | THR | 744 | 17.758 | -0.472 | 22.883 | 1.00 | 120.14 | A |
| 1797 | CA | THR | 744 | 16.744 | 0.516 | 23.243 | 1.00 | 120.66 | A |
| 1798 | CB | THR | 744 | 16.883 | 0.942 | 24.736 | 1.00 | 120.95 | A |
| 1799 | OG1 | THR | 744 | 17.456 | -0.137 | 25.488 | 1.00 | 121.58 | A |
| 1800 | CG2 | THR | 744 | 15.515 | 1.266 | 25.333 | 1.00 | 120.72 | A |
| 1801 | C | THR | 744 | 16.910 | 1.748 | 22.342 | 1.00 | 120.72 | A |
| 1802 | O | THR | 744 | 15.952 | 2.489 | 22.106 | 1.00 | 121.13 | A |
| 1803 | N | MET | 745 | 18.124 | 1.947 | 21.835 | 1.00 | 120.51 | A |
| 1804 | CA | MET | 745 | 18.434 | 3.075 | 20.950 | 1.00 | 119.90 | A |
| 1805 | CB | MET | 745 | 19.936 | 3.371 | 20.968 | 1.00 | 120.29 | A |
| 1806 | CG | MET | 745 | 20.316 | 4.645 | 21.706 | 1.00 | 120.44 | A |
| 1807 | SD | MET | 745 | 22.034 | 4.572 | 22.232 | 1.00 | 121.93 | A |
| 1808 | CE | MET | 745 | 21.949 | 5.228 | 23.955 | 1.00 | 121.39 | A |
| 1809 | C | MET | 745 | 17.988 | 2.794 | 19.520 | 1.00 | 118.93 | A |
| 1810 | O | MET | 745 | 17.574 | 3.701 | 18.800 | 1.00 | 119.40 | A |
| 1811 | N | SER | 746 | 18.093 | 1.537 | 19.109 | 1.00 | 117.59 | A |
| 1812 | CA | SER | 746 | 17.665 | 1.146 | 17.776 | 1.00 | 116.37 | A |
| 1813 | CB | SER | 746 | 16.156 | 1.302 | 17.664 | 1.00 | 116.67 | A |
| 1814 | OG | SER | 746 | 15.513 | 0.352 | 18.490 | 1.00 | 117.32 | A |
| 1815 | C | SER | 746 | 18.342 | 1.886 | 16.635 | 1.00 | 115.59 | A |
| 1816 | O | SER | 746 | 17.679 | 2.505 | 15.802 | 1.00 | 114.80 | A |
| 1817 | N | ILE | 747 | 19.668 | 1.807 | 16.627 | 1.00 | 114.95 | A |
| 1818 | CA | ILE | 747 | 20.531 | 2.397 | 15.609 | 1.00 | 113.53 | A |
| 1819 | CB | ILE | 747 | 21.545 | 3.387 | 16.244 | 1.00 | 113.48 | A |
| 1820 | CG2 | ILE | 747 | 22.594 | 3.816 | 15.228 | 1.00 | 113.56 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1821 | CG1 | ILE | 747 | 20.798 | 4.621 | 16.749 | 1.00 | 113.34 | A |
| 1822 | CD1 | ILE | 747 | 21.491 | 5.322 | 17.880 | 1.00 | 113.67 | A |
| 1823 | C | ILE | 747 | 21.238 | 1.158 | 15.051 | 1.00 | 112.59 | A |
| 1824 | O | ILE | 747 | 21.829 | 0.380 | 15.803 | 1.00 | 112.41 | A |
| 1825 | N | GLU | 748 | 21.146 | 0.974 | 13.738 | 1.00 | 111.41 | A |
| 1826 | CA | GLU | 748 | 21.718 | -0.186 | 13.061 | 1.00 | 110.45 | A |
| 1827 | CB | GLU | 748 | 20.920 | -0.492 | 11.819 | 1.00 | 112.15 | A |
| 1828 | CG | GLU | 748 | 19.542 | -0.943 | 12.075 | 1.00 | 115.24 | A |
| 1829 | CD | GLU | 748 | 18.913 | -1.396 | 10.796 | 1.00 | 117.08 | A |
| 1830 | OE1 | GLU | 748 | 19.522 | -2.261 | 10.127 | 1.00 | 117.86 | A |
| 1831 | OE2 | GLU | 748 | 17.825 | -0.885 | 10.456 | 1.00 | 118.10 | A |
| 1832 | C | GLU | 748 | 23.161 | -0.154 | 12.621 | 1.00 | 108.52 | A |
| 1833 | O | GLU | 748 | 23.593 | 0.773 | 11.946 | 1.00 | 108.69 | A |
| 1834 | N | PHE | 749 | 23.891 | -1.208 | 12.951 | 1.00 | 106.14 | A |
| 1835 | CA | PHE | 749 | 25.277 | -1.304 | 12.545 | 1.00 | 103.42 | A |
| 1836 | CB | PHE | 749 | 26.137 | -1.709 | 13.739 | 1.00 | 102.12 | A |
| 1837 | CG | PHE | 749 | 26.427 | -0.574 | 14.688 | 1.00 | 100.46 | A |
| 1838 | CD1 | PHE | 749 | 25.485 | -0.142 | 15.620 | 1.00 | 99.45 | A |
| 1839 | CD2 | PHE | 749 | 27.681 | 0.022 | 14.684 | 1.00 | 99.75 | A |
| 1840 | CE1 | PHE | 749 | 25.793 | 0.876 | 16.522 | 1.00 | 98.95 | A |
| 1841 | CE2 | PHE | 749 | 27.999 | 1.034 | 15.576 | 1.00 | 99.15 | A |
| 1842 | CZ | PHE | 749 | 27.061 | 1.449 | 16.506 | 1.00 | 98.78 | A |
| 1843 | C | PHE | 749 | 25.366 | -2.336 | 11.424 | 1.00 | 102.03 | A |
| 1844 | O | PHE | 749 | 24.849 | -3.444 | 11.552 | 1.00 | 101.37 | A |
| 1845 | N | PRO | 750 | 26.038 | -1.998 | 10.306 | 1.00 | 101.19 | A |
| 1846 | CD | PRO | 750 | 27.100 | -0.995 | 10.107 | 1.00 | 101.23 | A |
| 1847 | CA | PRO | 750 | 26.090 | -3.003 | 9.250 | 1.00 | 100.72 | A |
| 1848 | CB | PRO | 750 | 26.774 | -2.262 | 8.113 | 1.00 | 99.98 | A |
| 1849 | CG | PRO | 750 | 27.812 | -1.500 | 8.837 | 1.00 | 100.74 | A |
| 1850 | C | PRO | 750 | 26.859 | -4.235 | 9.708 | 1.00 | 100.57 | A |
| 1851 | O | PRO | 750 | 27.192 | -4.365 | 10.885 | 1.00 | 100.03 | A |
| 1852 | N | GLU | 751 | 27.145 | -5.131 | 8.766 | 1.00 | 100.58 | A |
| 1853 | CA | GLU | 751 | 27.847 | -6.379 | 9.056 | 1.00 | 100.49 | A |
| 1854 | CB | GLU | 751 | 27.693 | -7.331 | 7.875 | 1.00 | 101.63 | A |
| 1855 | CG | GLU | 751 | 27.095 | -8.689 | 8.227 | 1.00 | 103.13 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1856 | CD | GLU | 751 | 28.000 | -9.557 | 9.106 | 1.00 | 105.13 | A |
| 1857 | OE1 | GLU | 751 | 29.247 | -9.505 | 8.971 | 1.00 | 106.54 | A |
| 1858 | OE2 | GLU | 751 | 27.449 | -10.321 | 9.922 | 1.00 | 104.89 | A |
| 1859 | C | GLU | 751 | 29.328 | -6.227 | 9.382 | 1.00 | 99.40 | A |
| 1860 | O | GLU | 751 | 29.791 | -6.650 | 10.444 | 1.00 | 98.23 | A |
| 1861 | N | MET | 752 | 30.074 | -5.650 | 8.448 | 1.00 | 98.65 | A |
| 1862 | CA | MET | 752 | 31.494 | -5.439 | 8.648 | 1.00 | 97.97 | A |
| 1863 | CB | MET | 752 | 32.015 | -4.443 | 7.605 | 1.00 | 99.04 | A |
| 1864 | CG | MET | 752 | 33.487 | -4.148 | 7.738 | 1.00 | 100.18 | A |
| 1865 | SD | MET | 752 | 34.387 | -5.685 | 7.901 | 1.00 | 101.15 | A |
| 1866 | CE | MET | 752 | 34.323 | -6.213 | 6.236 | 1.00 | 101.26 | A |
| 1867 | C | MET | 752 | 31.717 | -4.907 | 10.066 | 1.00 | 96.84 | A |
| 1868 | O | MET | 752 | 32.232 | -5.619 | 10.927 | 1.00 | 96.54 | A |
| 1869 | N | LEU | 753 | 31.299 | -3.661 | 10.295 | 1.00 | 95.99 | A |
| 1870 | CA | LEU | 753 | 31.412 | -2.979 | 11.591 | 1.00 | 94.98 | A |
| 1871 | CB | LEU | 753 | 30.655 | -1.645 | 11.555 | 1.00 | 93.18 | A |
| 1872 | CG | LEU | 753 | 31.327 | -0.282 | 11.406 | 1.00 | 91.40 | A |
| 1873 | CD1 | LEU | 753 | 32.026 | -0.142 | 10.073 | 1.00 | 90.81 | A |
| 1874 | CD2 | LEU | 753 | 30.256 | 0.774 | 11.527 | 1.00 | 91.13 | A |
| 1875 | C | LEU | 753 | 30.841 | -3.813 | 12.730 | 1.00 | 95.34 | A |
| 1876 | O | LEU | 753 | 31.353 | -3.790 | 13.854 | 1.00 | 94.92 | A |
| 1877 | N | ALA | 754 | 29.756 | -4.515 | 12.436 | 1.00 | 96.40 | A |
| 1878 | CA | ALA | 754 | 29.087 | -5.365 | 13.413 | 1.00 | 97.52 | A |
| 1879 | CB | ALA | 754 | 28.056 | -6.244 | 12.714 | 1.00 | 97.46 | A |
| 1880 | C | ALA | 754 | 30.127 | -6.233 | 14.113 | 1.00 | 98.02 | A |
| 1881 | O | ALA | 754 | 30.257 | -6.198 | 15.338 | 1.00 | 97.73 | A |
| 1882 | N | GLU | 755 | 30.864 | -6.987 | 13.304 | 1.00 | 98.67 | A |
| 1883 | CA | GLU | 755 | 31.921 | -7.905 | 13.742 | 1.00 | 99.35 | A |
| 1884 | CB | GLU | 755 | 32.477 | -8.629 | 12.536 | 1.00 | 100.91 | A |
| 1885 | CG | GLU | 755 | 31.977 | -10.010 | 12.322 | 1.00 | 103.15 | A |
| 1886 | CD | GLU | 755 | 32.582 | -10.587 | 11.074 | 1.00 | 104.45 | A |
| 1887 | OE1 | GLU | 755 | 33.424 | -9.892 | 10.461 | 1.00 | 105.05 | A |
| 1888 | OE2 | GLU | 755 | 32.222 | -11.724 | 10.705 | 1.00 | 105.40 | A |
| 1889 | C | GLU | 755 | 33.116 | -7.305 | 14.467 | 1.00 | 98.62 | A |
| 1890 | O | GLU | 755 | 33.579 | -7.841 | 15.476 | 1.00 | 98.37 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1891 | N | ILE | 756 | 33.640 | -6.227 | 13.892 | 1.00 | 97.94 | A |
| 1892 | CA | ILE | 756 | 34.804 | -5.512 | 14.401 | 1.00 | 97.06 | A |
| 1893 | CB | ILE | 756 | 35.249 | -4.470 | 13.376 | 1.00 | 95.95 | A |
| 1894 | CG2 | ILE | 756 | 36.513 | -3.783 | 13.830 | 1.00 | 95.40 | A |
| 1895 | CG1 | ILE | 756 | 35.484 | -5.154 | 12.036 | 1.00 | 94.93 | A |
| 1896 | CD1 | ILE | 756 | 35.420 | -4.204 | 10.888 | 1.00 | 95.21 | A |
| 1897 | C | ILE | 756 | 34.544 | -4.832 | 15.740 | 1.00 | 97.25 | A |
| 1898 | O | ILE | 756 | 35.461 | -4.693 | 16.556 | 1.00 | 97.09 | A |
| 1899 | N | ILE | 757 | 33.304 | -4.387 | 15.960 | 1.00 | 97.46 | A |
| 1900 | CA | ILE | 757 | 32.953 | -3.754 | 17.227 | 1.00 | 98.19 | A |
| 1901 | CB | ILE | 757 | 31.636 | -2.904 | 17.129 | 1.00 | 97.65 | A |
| 1902 | CG2 | ILE | 757 | 31.194 | -2.467 | 18.522 | 1.00 | 97.21 | A |
| 1903 | CG1 | ILE | 757 | 31.890 | -1.625 | 16.312 | 1.00 | 97.77 | A |
| 1904 | CD1 | ILE | 757 | 30.718 | -1.146 | 15.454 | 1.00 | 97.25 | A |
| 1905 | C | ILE | 757 | 32.805 | -4.872 | 18.265 | 1.00 | 99.15 | A |
| 1906 | O | ILE | 757 | 33.306 | -4.746 | 19.382 | 1.00 | 99.19 | A |
| 1907 | N | THR | 758 | 32.141 | -5.968 | 17.884 | 1.00 | 100.27 | A |
| 1908 | CA | THR | 758 | 31.964 | -7.130 | 18.768 | 1.00 | 101.43 | A |
| 1909 | CB | THR | 758 | 31.082 | -8.230 | 18.101 | 1.00 | 101.61 | A |
| 1910 | OG1 | THR | 758 | 31.053 | -8.021 | 16.686 | 1.00 | 101.89 | A |
| 1911 | CG2 | THR | 758 | 29.660 | -8.206 | 18.650 | 1.00 | 101.75 | A |
| 1912 | C | THR | 758 | 33.339 | -7.733 | 19.066 | 1.00 | 102.09 | A |
| 1913 | O | THR | 758 | 33.629 | -8.149 | 20.188 | 1.00 | 101.89 | A |
| 1914 | N | ASN | 759 | 34.175 | -7.774 | 18.035 | 1.00 | 103.34 | A |
| 1915 | CA | ASN | 759 | 35.540 | -8.289 | 18.108 | 1.00 | 105.16 | A |
| 1916 | CB | ASN | 759 | 36.231 | -8.027 | 16.760 | 1.00 | 105.72 | A |
| 1917 | CG | ASN | 759 | 37.652 | -8.573 | 16.687 | 1.00 | 106.18 | A |
| 1918 | OD1 | ASN | 759 | 38.488 | -8.313 | 17.556 | 1.00 | 105.89 | A |
| 1919 | ND2 | ASN | 759 | 37.934 | -9.320 | 15.625 | 1.00 | 106.34 | A |
| 1920 | C | ASN | 759 | 36.288 | -7.557 | 19.223 | 1.00 | 106.31 | A |
| 1921 | O | ASN | 759 | 36.597 | -8.120 | 20.273 | 1.00 | 106.83 | A |
| 1922 | N | GLN | 760 | 36.542 | -6.283 | 18.957 | 1.00 | 107.80 | A |
| 1923 | CA | GLN | 760 | 37.273 | -5.351 | 19.815 | 1.00 | 109.24 | A |
| 1924 | CB | GLN | 760 | 37.375 | -4.032 | 19.067 | 1.00 | 109.55 | A |
| 1925 | CG | GLN | 760 | 38.163 | -4.174 | 17.815 | 1.00 | 110.53 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1926 | CD | GLN | 760 | 39.548 | -4.664 | 18.132 | 1.00 | 111.49 | A |
| 1927 | OE1 | GLN | 760 | 39.918 | -5.791 | 17.804 | 1.00 | 111.90 | A |
| 1928 | NE2 | GLN | 760 | 40.323 | -3.823 | 18.806 | 1.00 | 112.26 | A |
| 1929 | C | GLN | 760 | 36.782 | -5.052 | 21.223 | 1.00 | 109.82 | A |
| 1930 | O | GLN | 760 | 37.575 | -4.916 | 22.161 | 1.00 | 108.95 | A |
| 1931 | N | ILE | 761 | 35.468 | -4.924 | 21.334 | 1.00 | 111.38 | A |
| 1932 | CA | ILE | 761 | 34.793 | -4.577 | 22.573 | 1.00 | 113.13 | A |
| 1933 | CB | ILE | 761 | 33.247 | -4.839 | 22.414 | 1.00 | 112.25 | A |
| 1934 | CG2 | ILE | 761 | 32.949 | -6.322 | 22.323 | 1.00 | 112.15 | A |
| 1935 | CG1 | ILE | 761 | 32.470 | -4.200 | 23.561 | 1.00 | 111.65 | A |
| 1936 | CD1 | ILE | 761 | 32.594 | -4.922 | 24.877 | 1.00 | 111.27 | A |
| 1937 | C | ILE | 761 | 35.364 | -5.187 | 23.862 | 1.00 | 114.87 | A |
| 1938 | O | ILE | 761 | 35.448 | -4.502 | 24.885 | 1.00 | 115.06 | A |
| 1939 | N | PRO | 762 | 35.787 | -6.461 | 23.834 | 1.00 | 116.56 | A |
| 1940 | CD | PRO | 762 | 35.597 | -7.519 | 22.831 | 1.00 | 116.88 | A |
| 1941 | CA | PRO | 762 | 36.330 | -7.031 | 25.069 | 1.00 | 117.98 | A |
| 1942 | CB | PRO | 762 | 36.454 | -8.516 | 24.737 | 1.00 | 117.53 | A |
| 1943 | CG | PRO | 762 | 35.390 | -8.723 | 23.703 | 1.00 | 117.09 | A |
| 1944 | C | PRO | 762 | 37.671 | -6.424 | 25.465 | 1.00 | 119.34 | A |
| 1945 | O | PRO | 762 | 37.813 | -5.854 | 26.545 | 1.00 | 119.13 | A |
| 1946 | N | LYS | 763 | 38.649 | -6.529 | 24.571 | 1.00 | 121.52 | A |
| 1947 | CA | LYS | 763 | 39.988 | -6.025 | 24.853 | 1.00 | 123.89 | A |
| 1948 | CB | LYS | 763 | 41.025 | -6.777 | 24.007 | 1.00 | 124.50 | A |
| 1949 | CG | LYS | 763 | 41.377 | -6.076 | 22.701 | 1.00 | 125.11 | A |
| 1950 | CD | LYS | 763 | 40.640 | -6.658 | 21.513 | 1.00 | 125.37 | A |
| 1951 | CE | LYS | 763 | 41.386 | -7.881 | 21.002 | 1.00 | 126.09 | A |
| 1952 | NZ | LYS | 763 | 40.789 | -8.514 | 19.792 | 1.00 | 126.47 | A |
| 1953 | C | LYS | 763 | 40.250 | -4.519 | 24.709 | 1.00 | 125.24 | A |
| 1954 | O | LYS | 763 | 41.364 | -4.077 | 24.995 | 1.00 | 125.51 | A |
| 1955 | N | TYR | 764 | 39.271 | -3.728 | 24.259 | 1.00 | 126.66 | A |
| 1956 | CA | TYR | 764 | 39.504 | -2.280 | 24.123 | 1.00 | 127.39 | A |
| 1957 | CB | TYR | 764 | 38.628 | -1.648 | 23.025 | 1.00 | 128.05 | A |
| 1958 | CG | TYR | 764 | 39.451 | -0.985 | 21.937 | 1.00 | 129.02 | A |
| 1959 | CD1 | TYR | 764 | 39.575 | -1.577 | 20.681 | 1.00 | 129.21 | A |
| 1960 | CE1 | TYR | 764 | 40.445 | -1.064 | 19.718 | 1.00 | 129.52 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1961 | CD2 | TYR | 764 | 40.206 | 0.163 | 22.203 | 1.00 | 129.22 | A |
| 1962 | CE2 | TYR | 764 | 41.085 | 0.687 | 21.243 | 1.00 | 129.70 | A |
| 1963 | CZ | TYR | 764 | 41.203 | 0.061 | 20.006 | 1.00 | 129.81 | A |
| 1964 | OH | TYR | 764 | 42.124 | 0.504 | 19.083 | 1.00 | 129.43 | A |
| 1965 | C | TYR | 764 | 39.221 | -1.570 | 25.434 | 1.00 | 127.40 | A |
| 1966 | O | TYR | 764 | 38.978 | -0.365 | 25.451 | 1.00 | 127.67 | A |
| 1967 | N | SER | 765 | 39.274 | -2.330 | 26.525 | 1.00 | 127.42 | A |
| 1968 | CA | SER | 765 | 38.999 | -1.807 | 27.858 | 1.00 | 127.37 | A |
| 1969 | CB | SER | 765 | 37.552 | -2.121 | 28.245 | 1.00 | 127.92 | A |
| 1970 | OG | SER | 765 | 36.660 | -1.906 | 27.166 | 1.00 | 128.95 | A |
| 1971 | C | SER | 765 | 39.908 | -2.405 | 28.928 | 1.00 | 126.95 | A |
| 1972 | O | SER | 765 | 40.246 | -1.738 | 29.908 | 1.00 | 127.13 | A |
| 1973 | N | ASN | 766 | 40.290 | -3.668 | 28.735 | 1.00 | 126.33 | A |
| 1974 | CA | ASN | 766 | 41.121 | -4.420 | 29.682 | 1.00 | 125.68 | A |
| 1975 | CB | ASN | 766 | 41.166 | -5.899 | 29.271 | 1.00 | 126.48 | A |
| 1976 | CG | ASN | 766 | 39.788 | -6.497 | 29.051 | 1.00 | 127.50 | A |
| 1977 | OD1 | ASN | 766 | 38.850 | -6.221 | 29.803 | 1.00 | 128.09 | A |
| 1978 | ND2 | ASN | 766 | 39.660 | -7.339 | 28.026 | 1.00 | 127.55 | A |
| 1979 | C | ASN | 766 | 42.559 | -3.958 | 29.915 | 1.00 | 124.54 | A |
| 1980 | O | ASN | 766 | 42.970 | -3.691 | 31.052 | 1.00 | 124.51 | A |
| 1981 | N | GLY | 767 | 43.324 | -3.891 | 28.833 | 1.00 | 122.93 | A |
| 1982 | CA | GLY | 767 | 44.722 | -3.509 | 28.903 | 1.00 | 120.80 | A |
| 1983 | C | GLY | 767 | 45.416 | -4.524 | 28.018 | 1.00 | 119.01 | A |
| 1984 | O | GLY | 767 | 46.310 | -5.259 | 28.440 | 1.00 | 118.79 | A |
| 1985 | N | ASN | 768 | 44.973 | -4.564 | 26.773 | 1.00 | 117.24 | A |
| 1986 | CA | ASN | 768 | 45.504 | -5.506 | 25.810 | 1.00 | 115.20 | A |
| 1987 | CB | ASN | 768 | 44.318 | -6.304 | 25.223 | 1.00 | 116.68 | A |
| 1988 | CG | ASN | 768 | 43.414 | -6.926 | 26.320 | 1.00 | 118.09 | A |
| 1989 | OD1 | ASN | 768 | 43.106 | -6.281 | 27.331 | 1.00 | 118.30 | A |
| 1990 | ND2 | ASN | 768 | 42.984 | -8.171 | 26.105 | 1.00 | 118.06 | A |
| 1991 | C | ASN | 768 | 46.307 | -4.776 | 24.718 | 1.00 | 113.01 | A |
| 1992 | O | ASN | 768 | 47.374 | -5.237 | 24.309 | 1.00 | 112.58 | A |
| 1993 | N | ILE | 769 | 45.805 | -3.608 | 24.305 | 1.00 | 109.95 | A |
| 1994 | CA | ILE | 769 | 46.388 | -2.762 | 23.252 | 1.00 | 106.52 | A |
| 1995 | CB | ILE | 769 | 45.260 | -1.996 | 22.527 | 1.00 | 106.78 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 1996 | CG2 | ILE | 769 | 45.826 | -1.167 | 21.379 | 1.00 | 106.69 | A |
| 1997 | CG1 | ILE | 769 | 44.197 | -2.994 | 22.053 | 1.00 | 106.81 | A |
| 1998 | CD1 | ILE | 769 | 42.973 | -2.369 | 21.435 | 1.00 | 106.29 | A |
| 1999 | C | ILE | 769 | 47.463 | -1.732 | 23.664 | 1.00 | 103.81 | A |
| 2000 | O | ILE | 769 | 47.264 | -0.935 | 24.582 | 1.00 | 103.59 | A |
| 2001 | N | LYS | 770 | 48.585 | -1.735 | 22.947 | 1.00 | 100.30 | A |
| 2002 | CA | LYS | 770 | 49.691 | -0.824 | 23.218 | 1.00 | 96.45 | A |
| 2003 | CB | LYS | 770 | 51.001 | -1.579 | 23.072 | 1.00 | 95.81 | A |
| 2004 | CG | LYS | 770 | 52.227 | -0.777 | 23.354 | 1.00 | 94.18 | A |
| 2005 | CD | LYS | 770 | 53.407 | -1.532 | 22.808 | 1.00 | 94.37 | A |
| 2006 | CE | LYS | 770 | 54.692 | -0.768 | 23.017 | 1.00 | 93.82 | A |
| 2007 | NZ | LYS | 770 | 55.821 | -1.401 | 22.284 | 1.00 | 93.42 | A |
| 2008 | C | LYS | 770 | 49.676 | 0.360 | 22.250 | 1.00 | 94.29 | A |
| 2009 | O | LYS | 770 | 50.038 | 0.228 | 21.082 | 1.00 | 94.06 | A |
| 2010 | N | LYS | 771 | 49.272 | 1.517 | 22.756 | 1.00 | 91.57 | A |
| 2011 1 | CA | LYS | 771 | 49.174 | 2.727 | 21.949 | 1.00 | 88.83 | A |
| 2012 | CB | LYS | 771 | 48.176 | 3.678 | 22.654 | 1.00 | 88.60 | A |
| 2013 | CG | LYS | 771 | 46.931 | 2.902 | 23.212 | 1.00 | 89.92 | A |
| 2014 | CD | LYS | 771 | 45.687 | 3.749 | 23.550 | 1.00 | 90.42 | A |
| 2015 | CE | LYS | 771 | 45.830 | 4.541 | 24.848 | 1.00 | 92.04 | A |
| 2016 | NZ | LYS | 771 | 45.010 | 5.799 | 24.853 | 1.00 | 92.59 | A |
| 2017 | C | LYS | 771 | 50.547 | 3.392 | 21.692 | 1.00 | 86.56 | A |
| 2018 | O | LYS | 771 | 51.009 | 4.180 | 22.506 | 1.00 | 86.88 | A |
| 2019 | N | LEU | 772 | 51.200 | 3.067 | 20.571 | 1.00 | 83.82 | A |
| 2020 | CA | LEU | 772 | 52.510 | 3.650 | 20.229 | 1.00 | 81.98 | A |
| 2021 | CB | LEU | 772 | 52.970 | 3.194 | 18.838 | 1.00 | 80.07 | A |
| 2022 | CG | LEU | 772 | 52.856 | 1.704 | 18.545 | 1.00 | 78.83 | A |
| 2023 | CD1 | LEU | 772 | 53.382 | 1.346 | 17.159 | 1.00 | 78.33 | A |
| 2024 | CD2 | LEU | 772 | 53.635 | 0.984 | 19.605 | 1.00 | 78.33 | A |
| 2025 | C | LEU | 772 | 52.424 | 5.176 | 20.219 | 1.00 | 82.19 | A |
| 2026 | O | LEU | 772 | 51.397 | 5.735 | 19.839 | 1.00 | 82.51 | A |
| 2027 | N | LEU | 773 | 53.492 | 5.857 | 20.620 | 1.00 | 81.85 | A |
| 2028 | CA | LEU | 773 | 53.476 | 7.320 | 20.617 | 1.00 | 81.81 | A |
| 2029 | CB | LEU | 773 | 53.261 | 7.871 | 22.027 | 1.00 | 80.64 | A |
| 2030 | CG | LEU | 773 | 51.990 | 7.525 | 22.798 | 1.00 | 79.78 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2031 | CD1 | LEU | 773 | 52.296 | 7.672 | 24.267 | 1.00 | 80.42 | A |
| 2032 | CD2 | LEU | 773 | 50.826 | 8.418 | 22.396 | 1.00 | 79.59 | A |
| 2033 | C | LEU | 773 | 54.786 | 7.878 | 20.082 | 1.00 | 82.31 | A |
| 2034 | O | LEU | 773 | 55.849 | 7.320 | 20.341 | 1.00 | 82.88 | A |
| 2035 | N | PHE | 774 | 54.705 | 8.972 | 19.331 | 1.00 | 82.39 | A |
| 2036 | CA | PHE | 774 | 55.895 | 9.612 | 18.798 | 1.00 | 82.78 | A |
| 2037 | CB | PHE | 774 | 55.538 | 10.531 | 17.630 | 1.00 | 80.87 | A |
| 2038 | CG | PHE | 774 | 55.321 | 9.799 | 16.353 | 1.00 | 78.91 | A |
| 2039 | CD1 | PHE | 774 | 56.395 | 9.479 | 15.539 | 1.00 | 78.24 | A |
| 2040 | CD2 | PHE | 774 | 54.064 | 9.292 | 16.038 | 1.00 | 78.78 | A |
| 2041 | CE1 | PHE | 774 | 56.226 | 8.654 | 14.428 | 1.00 | 78.15 | A |
| 2042 | CE2 | PHE | 774 | 53.881 | 8.465 | 14.931 | 1.00 | 78.23 | A |
| 2043 | CZ | PHE | 774 | 54.966 | 8.143 | 14.127 | 1.00 | 78.32 | A |
| 2044 | C | PHE | 774 | 56.498 | 10.406 | 19.933 | 1.00 | 84.83 | A |
| 2045 | O | PHE | 774 | 57.704 | 10.639 | 19.968 | 1.00 | 84.81 | A |
| 2046 | N | HIS | 775 | 55.638 | 10.796 | 20.873 | 1.00 | 88.32 | A |
| 2047 | CA | HIS | 775 | 56.039 | 11.578 | 22.041 | 1.00 | 91.73 | A |
| 2048 | CB | HIS | 775 | 55.558 | 13.012 | 21.895 | 1.00 | 90.90 | A |
| 2049 | CG | HIS | 775 | 55.970 | 13.634 | 20.612 | 1.00 | 90.80 | A |
| 2050 | CD2 | HIS | 775 | 55.263 | 14.275 | 19.656 | 1.00 | 90.67 | A |
| 2051 | ND1 | HIS | 775 | 57.265 | 13.551 | 20.143 | 1.00 | 90.62 | A |
| 2052 | CE1 | HIS | 775 | 57.333 | 14.107 | 18.950 | 1.00 | 91.01 | A |
| 2053 | NE2 | HIS | 775 | 56.131 | 14.554 | 18.629 | 1.00 | 91.35 | A |
| 2054 | C | HIS | 775 | 55.506 | 11.026 | 23.340 | 1.00 | 94.76 | A |
| 2055 | O | HIS | 775 | 54.304 | 10.790 | 23.480 | 1.00 | 94.90 | A |
| 2056 | N | GLN | 776 | 56.398 | 10.841 | 24.303 | 1.00 | 98.84 | A |
| 2057 | CA | GLN | 776 | 55.996 | 10.334 | 25.601 | 1.00 | 103.31 | A |
| 2058 | CB | GLN | 776 | 57.179 | 9.633 | 26.291 | 1.00 | 104.45 | A |
| 2059 | CG | GLN | 776 | 58.582 | 10.114 | 25.886 | 1.00 | 106.57 | A |
| 2060 | CD | GLN | 776 | 58.999 | 11.424 | 26.551 | 1.00 | 108.35 | A |
| 2061 | OE1 | GLN | 776 | 58.837 | 12.508 | 25.981 | 1.00 | 108.79 | A |
| 2062 | NE2 | GLN | 776 | 59.538 | 11.323 | 27.767 | 1.00 | 108.32 | A |
| 2063 | C | GLN | 776 | 55.384 | 11.412 | 26.511 | 1.00 | 105.64 | A |
| 2064 | O | GLN | 776 | 54.217 | 11.295 | 26.883 | 1.00 | 106.06 | A |
| 2065 | N | LYS | 777 | 56.149 | 12.466 | 26.822 | 1.00 | 108.50 | A |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2066 | CA | LYS | 777 | 55.725 | 13.569 | 27.721 | 1.00 | 111.14 | A |
| 2067 | CB | LYS | 777 | 55.127 | 14.778 | 26.940 | 1.00 | 111.13 | A |
| 2068 | CG | LYS | 777 | 55.167 | 16.163 | 27.704 | 1.00 | 111.26 | A |
| 2069 | CD | LYS | 777 | 53.761 | 16.804 | 27.916 | 1.00 | 110.94 | A |
| 2070 | CE | LYS | 777 | 53.775 | 18.216 | 28.568 | 1.00 | 110.88 | A |
| 2071 | NZ | LYS | 777 | 54.016 | 18.255 | 30.049 | 1.00 | 110.23 | A |
| 2072 | C | LYS | 777 | 54.728 | 13.077 | 28.778 | 1.00 | 112.33 | A |
| 2073 | O | LYS | 777 | 53.497 | 13.179 | 28.558 | 1.00 | 112.91 | A |
| 2074 | OXT | LYS | 777 | 55.202 | 12.561 | 29.818 | 1.00 | 113.31 | A |
| 2075 | CB | LYS | 740 | 36.212 | -17.177 | 16.526 | 1.00 | 126.78 | B |
| 2076 | CG | LYS | 740 | 36.506 | -17.638 | 17.979 | 1.00 | 128.08 | B |
| 2077 | CD | LYS | 740 | 37.327 | -18.909 | 18.063 | 1.00 | 129.35 | B |
| 2078 | CE | LYS | 740 | 38.818 | -18.624 | 18.192 | 1.00 | 129.72 | B |
| 2079 | NZ | LYS | 740 | 39.630 | -19.761 | 17.686 | 1.00 | 130.22 | B |
| 2080 | C | LYS | 740 | 37.246 | -15.433 | 15.072 | 1.00 | 124.52 | B |
| 2081 | O | LYS | 740 | 37.988 | -14.994 | 14.192 | 1.00 | 124.88 | B |
| 2082 | N | LYS | 740 | 38.637 | -16.592 | 16.502 | 1.00 | 126.10 | B |
| 2083 | CA | LYS | 740 | 37.432 | -16.814 | 15.655 | 1.00 | 125.59 | B |
| 2084 | N | GLU | 741 | 36.239 | -14.769 | 15.618 | 1.00 | 122.85 | B |
| 2085 | CA | GLU | 741 | 35.871 | -13.404 | 15.324 | 1.00 | 120.66 | B |
| 2086 | CB | GLU | 741 | 36.304 | -12.556 | 16.511 | 1.00 | 121.44 | B |
| 2087 | CG | GLU | 741 | 35.820 | -13.120 | 17.844 | 1.00 | 121.77 | B |
| 2088 | CD | GLU | 741 | 36.524 | -14.406 | 18.271 | 1.00 | 122.23 | B |
| 2089 | OE1 | GLU | 741 | 37.767 | -14.406 | 18.439 | 1.00 | 122.26 | B |
| 2090 | OE2 | GLU | 741 | 35.811 | -15.408 | 18.452 | 1.00 | 121.76 | B |
| 2091 | C | GLU | 741 | 36.317 | -12.740 | 14.032 | 1.00 | 118.57 | B |
| 2092 | O | GLU | 741 | 37.480 | -12.798 | 13.620 | 1.00 | 118.23 | B |
| 2093 | N | ASN | 742 | 35.345 | -12.103 | 13.398 | 1.00 | 116.10 | B |
| 2094 | CA | ASN | 742 | 35.600 | -11.375 | 12.184 | 1.00 | 113.26 | B |
| 2095 | CB | ASN | 742 | 36.529 | -10.220 | 12.514 | 1.00 | 114.33 | B |
| 2096 | CG | ASN | 742 | 36.603 | -9.208 | 11.418 | 1.00 | 115.13 | B |
| 2097 | OD1 | ASN | 742 | 37.244 | -8.171 | 11.572 | 1.00 | 116.52 | B |
| 2098 | ND2 | ASN | 742 | 35.948 | -9.494 | 10.297 | 1.00 | 114.81 | B |
| 2099 | C | ASN | 742 | 36.204 | -12.247 | 11.094 | 1.00 | 110.51 | B |
| 2100 | O | ASN | 742 | 37.313 | -11.999 | 10.623 | 1.00 | 110.08 | B |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2101 | N | ALA | 743 | 35.473 | -13.285 | 10.702 | 1.00 | 106.82 | B |
| 2102 | CA | ALA | 743 | 35.952 | -14.143 | 9.640 | 1.00 | 102.91 | B |
| 2103 | CB | ALA | 743 | 35.161 | -15.450 | 9.610 | 1.00 | 103.22 | B |
| 2104 | C | ALA | 743 | 35.745 | -13.344 | 8.355 | 1.00 | 100.03 | B |
| 2105 | O | ALA | 743 | 36.397 | -13.611 | 7.345 | 1.00 | 99.90 | B |
| 2106 | N | LEU | 744 | 34.861 | -12.344 | 8.400 | 1.00 | 95.91 | B |
| 2107 | CA | LEU | 744 | 34.588 | -11.513 | 7.224 | 1.00 | 92.30 | B |
| 2108 | CB | LEU | 744 | 33.374 | -10.603 | 7.455 | 1.00 | 91.74 | B |
| 2109 | CG | LEU | 744 | 32.438 | -10.333 | 6.259 | 1.00 | 91.66 | B |
| 2110 | CD1 | LEU | 744 | 31.463 | -9.223 | 6.623 | 1.00 | 91.11 | B |
| 2111 | CD2 | LEU | 744 | 33.221 | -9.924 | 5.023 | 1.00 | 92.05 | B |
| 2112 | C | LEU | 744 | 35.793 | -10.662 | 6.779 | 1.00 | 90.60 | B |
| 2113 | O | LEU | 744 | 35.905 | -10.346 | 5.596 | 1.00 | 90.25 | B |
| 2114 | N | LEU | 745 | 36.680 | -10.274 | 7.695 | 1.00 | 87.95 | B |
| 2115 | CA | LEU | 745 | 37.872 | -9.492 | 7.312 | 1.00 | 85.36 | B |
| 2116 | CB | LEU | 745 | 38.474 | -8.746 | 8.502 | 1.00 | 84.07 | B |
| 2117 | CG | LEU | 745 | 38.669 | -7.237 | 8.665 | 1.00 | 82.29 | B |
| 2118 | CD1 | LEU | 745 | 39.514 | -7.118 | 9.916 | 1.00 | 80.83 | B |
| 2119 | CD2 | LEU | 745 | 39.362 | -6.537 | 7.497 | 1.00 | 80.58 | B |
| 2120 | C | LEU | 745 | 38.921 | -10.486 | 6.839 | 1.00 | 84.43 | B |
| 2121 | O | LEU | 745 | 39.672 | -10.226 | 5.894 | 1.00 | 84.16 | B |
| 2122 | N | ARG | 746 | 38.987 | -11.616 | 7.541 | 1.00 | 83.85 | B |
| 2123 | CA | ARG | 746 | 39.926 | -12.673 | 7.209 | 1.00 | 83.05 | B |
| 2124 | CB | ARG | 746 | 39.707 | -13.898 | 8.097 | 1.00 | 83.28 | B |
| 2125 | CG | ARG | 746 | 40.511 | -15.126 | 7.673 | 1.00 | 84.87 | B |
| 2126 | CD | ARG | 746 | 40.606 | -16.167 | 8.792 | 1.00 | 86.65 | B |
| 2127 | NE | ARG | 746 | 41.854 | -16.040 | 9.554 | 1.00 | 89.65 | B |
| 2128 | CZ | ARG | 746 | 41.934 | -15.874 | 10.874 | 1.00 | 90.19 | B |
| 2129 | NH1 | ARG | 746 | 40.829 | -15.809 | 11.618 | 1.00 | 90.02 | B |
| 2130 | NH2 | ARG | 746 | 43.128 | -15.766 | 11.455 | 1.00 | 88.90 | B |
| 2131 | C | ARG | 746 | 39.689 | -13.043 | 5.768 | 1.00 | 82.22 | B |
| 2132 | O | ARG | 746 | 40.614 | -13.204 | 4.998 | 1.00 | 82.49 | B |
| 2133 | N | TYR | 747 | 38.428 | -13.155 | 5.409 | 1.00 | 82.19 | B |
| 2134 | CA | TYR | 747 | 38.059 | -13.508 | 4.058 | 1.00 | 82.92 | B |
| 2135 | CB | TYR | 747 | 36.555 | -13.717 | 4.010 | 1.00 | 82.63 | B |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2136 | CG | TYR | 747 | 36.065 | -13.789 | 2.616 | 1.00 | 82.87 | B |
| 2137 | CD1 | TYR | 747 | 36.594 | -14.724 | 1.734 | 1.00 | 83.81 | B |
| 2138 | CE1 | TYR | 747 | 36.209 | -14.756 | 0.422 | 1.00 | 84.67 | B |
| 2139 | CD2 | TYR | 747 | 35.130 | -12.884 | 2.147 | 1.00 | 82.83 | B |
| 2140 | CE2 | TYR | 747 | 34.732 | -12.903 | 0.833 | 1.00 | 84.05 | B |
| 2141 | CZ | TYR | 747 | 35.276 | -13.840 | -0.026 | 1.00 | 85.10 | B |
| 2142 | OH | TYR | 747 | 34.880 | -13.863 | -1.336 | 1.00 | 86.88 | B |
| 2143 | C | TYR | 747 | 38.479 | -12.448 | 3.022 | 1.00 | 83.86 | B |
| 2144 | O | TYR | 747 | 38.943 | -12.768 | 1.917 | 1.00 | 84.05 | B |
| 2145 | N | LEU | 748 | 38.296 | -11.189 | 3.414 | 1.00 | 84.91 | B |
| 2146 | CA | LEU | 748 | 38.593 | -9.994 | 2.622 | 1.00 | 85.00 | B |
| 2147 | CB | LEU | 748 | 38.142 | -8.763 | 3.391 | 1.00 | 85.05 | B |
| 2148 | CG | LEU | 748 | 36.733 | -8.194 | 3.274 | 1.00 | 84.64 | B |
| 2149 | CD1 | LEU | 748 | 36.515 | -7.239 | 4.426 | 1.00 | 85.29 | B |
| 2150 | CD2 | LEU | 748 | 36.573 | -7.473 | 1.947 | 1.00 | 83.89 | B |
| 2151 | C | LEU | 748 | 40.063 | -9.807 | 2.299 | 1.00 | 85.69 | B |
| 2152 | O | LEU | 748 | 40.429 | -9.385 | 1.197 | 1.00 | 85.46 | B |
| 2153 | N | LEU | 749 | 40.886 | -10.089 | 3.294 | 1.00 | 87.36 | B |
| 2154 | CA | LEU | 749 | 42.329 | -9.958 | 3.191 | 1.00 | 90.50 | B |
| 2155 | CB | LEU | 749 | 42.948 | -10.171 | 4.574 | 1.00 | 87.67 | B |
| 2156 | CG | LEU | 749 | 42.539 | -9.094 | 5.578 | 1.00 | 85.91 | B |
| 2157 | CD1 | LEU | 749 | 43.126 | -9.325 | 6.963 | 1.00 | 84.27 | B |
| 2158 | CD2 | LEU | 749 | 43.012 | -7.775 | 5.016 | 1.00 | 83.58 | B |
| 2159 | C | LEU | 749 | 42.971 | -10.913 | 2.199 | 1.00 | 94.62 | B |
| 2160 | O | LEU | 749 | 44.057 | -10.645 | 1.683 | 1.00 | 95.29 | B |
| 2161 | N | ASP | 750 | 42.293 | -12.010 | 1.906 | 1.00 | 99.41 | B |
| 2162 | CA | ASP | 750 | 42.859 | -13.007 | 1.025 | 1.00 | 103.90 | B |
| 2163 | CB | ASP | 750 | 42.303 | -14.362 | 1.502 | 1.00 | 103.66 | B |
| 2164 | CG | ASP | 750 | 42.735 | -14.686 | 2.957 | 1.00 | 103.87 | B |
| 2165 | OD1 | ASP | 750 | 43.483 | -15.661 | 3.178 | 1.00 | 103.14 | B |
| 2166 | OD2 | ASP | 750 | 42.347 | -13.947 | 3.885 | 1.00 | 104.73 | B |
| 2167 | C | ASP | 750 | 42.856 | -12.811 | -0.534 | 1.00 | 108.04 | B |
| 2168 | O | ASP | 750 | 43.917 | -12.898 | -1.140 | 1.00 | 108.70 | B |
| 2169 | N | LYS | 751 | 41.728 | -12.458 | -1.161 | 1.00 | 112.20 | B |
| 2170 | CA | LYS | 751 | 41.571 | -12.287 | -2.648 | 1.00 | 117.11 | B |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2171 | CB | LYS | 751 | 40.158 | -12.770 | -2.987 | 1.00 | 117.68 | B |
| 2172 | CG | LYS | 751 | 39.095 | -12.327 | -1.933 | 1.00 | 118.08 | B |
| 2173 | CD | LYS | 751 | 39.319 | -10.912 | -1.354 | 1.00 | 118.35 | B |
| 2174 | CE | LYS | 751 | 38.613 | -9.832 | -2.158 | 1.00 | 118.66 | B |
| 2175 | NZ | LYS | 751 | 37.151 | -10.073 | -2.164 | 1.00 | 118.89 | B |
| 2176 | C | LYS | 751 | 41.736 | -10.959 | -3.494 | 1.00 | 120.57 | B |
| 2177 | O | LYS | 751 | 40.815 | -10.163 | -3.467 | 1.00 | 120.77 | B |
| 2178 | N | ASP | 752 | 42.787 | -10.662 | -4.272 | 1.00 | 124.55 | B |
| 2179 | CA | ASP | 752 | 42.641 | -9.379 | -5.042 | 1.00 | 128.27 | B |
| 2180 | CB | ASP | 752 | 43.242 | -8.168 | -4.270 | 1.00 | 128.97 | B |
| 2181 | CG | ASP | 752 | 42.370 | -6.877 | -4.391 | 1.00 | 130.06 | B |
| 2182 | OD1 | ASP | 752 | 42.081 | -6.432 | -5.529 | 1.00 | 130.65 | B |
| 2183 | OD2 | ASP | 752 | 41.975 | -6.299 | -3.348 | 1.00 | 130.15 | B |
| 2184 | C | ASP | 752 | 43.041 | -9.284 | -6.550 | 1.00 | 129.74 | B |
| 2185 | O | ASP | 752 | 42.531 | -8.403 | -7.257 | 1.00 | 130.16 | B |
| 2186 | N | ASP | 753 | 43.903 | -10.186 | -7.031 | 1.00 | 131.88 | B |
| 2187 | CA | ASP | 753 | 44.382 | -10.237 | -8.438 | 1.00 | 132.79 | B |
| 2188 | CB | ASP | 753 | 44.786 | -11.700 | -8.767 | 1.00 | 133.26 | B |
| 2189 | CG | ASP | 753 | 45.584 | -11.841 | -10.074 | 1.00 | 133.42 | B |
| 2190 | OD1 | ASP | 753 | 44.982 | -11.747 | -11.168 | 1.00 | 133.48 | B |
| 2191 | OD2 | ASP | 753 | 46.815 | -12.067 | -10.003 | 1.00 | 133.16 | B |
| 2192 | C | ASP | 753 | 43.364 | -9.707 | -9.474 | 1.00 | 133.27 | B |
| 2193 | O | ASP | 753 | 43.309 | -8.468 | -9.660 | 1.00 | 133.47 | B |
| 2194 | OXT | ASP | 753 | 42.624 | -10.518 | -10.079 | 1.00 | 133.68 | B |
| 2195 | CB | LEU | 525 | 40.004 | 46.082 | 16.396 | 1.00 | 136.92 | D |
| 2196 | CG | LEU | 525 | 39.293 | 45.409 | 15.212 | 1.00 | 137.30 | D |
| 2197 | CD1 | LEU | 525 | 39.125 | 46.375 | 14.046 | 1.00 | 136.87 | D |
| 2198 | CD2 | LEU | 525 | 40.098 | 44.179 | 14.790 | 1.00 | 137.28 | D |
| 2199 | C | LEU | 525 | 37.974 | 47.434 | 17.108 | 1.00 | 135.85 | D |
| 2200 | O | LEU | 525 | 37.316 | 46.395 | 17.075 | 1.00 | 136.26 | D |
| 2201 | N | LEU | 525 | 40.125 | 47.532 | 18.398 | 1.00 | 136.62 | D |
| 2202 | CA | LEU | 525 | 39.512 | 47.393 | 17.042 | 1.00 | 136.44 | D |
| 2203 | N | PRO | 526 | 37.390 | 48.641 | 17.246 | 1.00 | 134.93 | D |
| 2204 | CD | PRO | 526 | 38.029 | 49.714 | 18.037 | 1.00 | 134.90 | D |
| 2205 | CA | PRO | 526 | 35.929 | 48.780 | 17.315 | 1.00 | 133.52 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2206 | CB | PRO | 526 | 35.730 | 49.375 | 18.690 | 1.00 | 134.35 | D |
| 2207 | CG | PRO | 526 | 36.830 | 50.415 | 18.709 | 1.00 | 134.90 | D |
| 2208 | C | PRO | 526 | 35.316 | 49.692 | 16.243 | 1.00 | 131.67 | D |
| 2209 | O | PRO | 526 | 35.089 | 50.875 | 16.504 | 1.00 | 131.68 | D |
| 2210 | N | GLN | 527 | 35.017 | 49.166 | 15.064 | 1.00 | 129.15 | D |
| 2211 | CA | GLN | 527 | 34.438 | 50.023 | 14.038 | 1.00 | 126.46 | D |
| 2212 | CB | GLN | 527 | 34.671 | 49.401 | 12.657 | 1.00 | 126.52 | D |
| 2213 | CG | GLN | 527 | 34.626 | 47.894 | 12.636 | 1.00 | 126.83 | D |
| 2214 | CD | GLN | 527 | 33.233 | 47.385 | 12.865 | 1.00 | 127.18 | D |
| 2215 | OE1 | GLN | 527 | 32.264 | 48.096 | 12.616 | 1.00 | 126.89 | D |
| 2216 | NE2 | GLN | 527 | 33.117 | 46.143 | 13.324 | 1.00 | 128.15 | D |
| 2217 | C | GLN | 527 | 32.955 | 50.431 | 14.241 | 1.00 | 124.32 | D |
| 2218 | O | GLN | 527 | 32.591 | 51.569 | 13.921 | 1.00 | 124.28 | D |
| 2219 | N | LEU | 528 | 32.128 | 49.533 | 14.798 | 1.00 | 121.31 | D |
| 2220 | CA | LEU | 528 | 30.684 | 49.773 | 15.072 | 1.00 | 117.75 | D |
| 2221 | CB | LEU | 528 | 30.468 | 50.286 | 16.503 | 1.00 | 118.19 | D |
| 2222 | CG | LEU | 528 | 30.502 | 49.369 | 17.727 | 1.00 | 118.67 | D |
| 2223 | CD1 | LEU | 528 | 31.761 | 48.525 | 17.715 | 1.00 | 118.31 | D |
| 2224 | CD2 | LEU | 528 | 30.435 | 50.226 | 18.991 | 1.00 | 119.12 | D |
| 2225 | C | LEU | 528 | 29.995 | 50.749 | 14.125 | 1.00 | 114.85 | D |
| 2226 | O | LEU | 528 | 29.206 | 51.591 | 14.558 | 1.00 | 114.46 | D |
| 2227 | N | THR | 529 | 30.275 | 50.614 | 12.840 | 1.00 | 111.72 | D |
| 2228 | CA | THR | 529 | 29.727 | 51.501 | 11.831 | 1.00 | 107.57 | D |
| 2229 | CB | THR | 529 | 30.647 | 52.757 | 11.723 | 1.00 | 107.58 | D |
| 2230 | OG1 | THR | 529 | 29.965 | 53.809 | 11.038 | 1.00 | 108.51 | D |
| 2231 | CG2 | THR | 529 | 31.949 | 52.426 | 11.009 | 1.00 | 106.76 | D |
| 2232 | C | THR | 529 | 29.655 | 50.697 | 10.519 | 1.00 | 104.56 | D |
| 2233 | O | THR | 529 | 30.679 | 50.281 | 9.969 | 1.00 | 104.85 | D |
| 2234 | N | PRO | 530 | 28.434 | 50.463 | 10.012 | 1.00 | 100.85 | D |
| 2235 | CD | PRO | 530 | 27.221 | 51.206 | 10.399 | 1.00 | 100.11 | D |
| 2236 | CA | PRO | 530 | 28.190 | 49.697 | 8.785 | 1.00 | 97.13 | D |
| 2237 | CB | PRO | 530 | 26.672 | 49.710 | 8.679 | 1.00 | 98.21 | D |
| 2238 | CG | PRO | 530 | 26.344 | 51.081 | 9.162 | 1.00 | 98.89 | D |
| 2239 | C | PRO | 530 | 28.842 | 50.242 | 7.531 | 1.00 | 93.39 | D |
| 2240 | O | PRO | 530 | 28.791 | 51.438 | 7.261 | 1.00 | 92.57 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2241 | N | THR | 531 | 29.470 | 49.353 | 6.775 | 1.00 | 89.92 | D |
| 2242 | CA | THR | 531 | 30.077 | 49.750 | 5.522 | 1.00 | 86.89 | D |
| 2243 | CB | THR | 531 | 31.470 | 49.126 | 5.304 | 1.00 | 86.90 | D |
| 2244 | OG1 | THR | 531 | 31.412 | 47.706 | 5.492 | 1.00 | 86.36 | D |
| 2245 | CG2 | THR | 531 | 32.469 | 49.726 | 6.266 | 1.00 | 86.26 | D |
| 2246 | C | THR | 531 | 29.102 | 49.196 | 4.519 | 1.00 | 85.27 | D |
| 2247 | O | THR | 531 | 28.306 | 48.316 | 4.846 | 1.00 | 85.29 | D |
| 2248 | N | LEU | 532 | 29.142 | 49.701 | 3.301 | 1.00 | 82.89 | D |
| 2249 | CA | LEU | 532 | 28.206 | 49.223 | 2.306 | 1.00 | 80.97 | D |
| 2250 | CB | LEU | 532 | 28.380 | 50.042 | 1.047 | 1.00 | 79.96 | D |
| 2251 | CG | LEU | 532 | 27.321 | 49.899 | -0.032 | 1.00 | 79.61 | D |
| 2252 | CD1 | LEU | 532 | 25.947 | 49.459 | 0.495 | 1.00 | 79.10 | D |
| 2253 | CD2 | LEU | 532 | 27.255 | 51.256 | -0.674 | 1.00 | 79.67 | D |
| 2254 | C | LEU | 532 | 28.357 | 47.725 | 2.018 | 1.00 | 80.18 | D |
| 2255 | O | LEU | 532 | 27.367 | 47.000 | 1.884 | 1.00 | 80.38 | D |
| 2256 | N | VAL | 533 | 29.597 | 47.261 | 1.925 | 1.00 | 78.45 | D |
| 2257 | CA | VAL | 533 | 29.858 | 45.854 | 1.672 | 1.00 | 76.93 | D |
| 2258 | CB | VAL | 533 | 31.319 | 45.637 | 1.290 | 1.00 | 75.90 | D |
| 2259 | CG1 | VAL | 533 | 32.198 | 46.022 | 2.461 | 1.00 | 75.17 | D |
| 2260 | CG2 | VAL | 533 | 31.556 | 44.189 | 0.891 | 1.00 | 75.29 | D |
| 2261 | C | VAL | 533 | 29.583 | 45.085 | 2.964 | 1.00 | 76.53 | D |
| 2262 | O | VAL | 533 | 29.442 | 43.862 | 2.963 | 1.00 | 76.16 | D |
| 2263 | N | SER | 534 | 29.520 | 45.818 | 4.073 | 1.00 | 75.87 | D |
| 2264 | CA | SER | 534 | 29.273 | 45.220 | 5.383 | 1.00 | 75.27 | D |
| 2265 | CB | SER | 534 | 29.453 | 46.268 | 6.473 | 1.00 | 76.08 | D |
| 2266 | OG | SER | 534 | 29.059 | 45.758 | 7.732 | 1.00 | 78.01 | D |
| 2267 | C | SER | 534 | 27.856 | 44.695 | 5.459 | 1.00 | 74.06 | D |
| 2268 | O | SER | 534 | 27.574 | 43.601 | 5.941 | 1.00 | 73.87 | D |
| 2269 | N | LEU | 535 | 26.963 | 45.531 | 4.978 | 1.00 | 73.15 | D |
| 2270 | CA | LEU | 535 | 25.563 | 45.243 | 4.961 | 1.00 | 72.35 | D |
| 2271 | CB | LEU | 535 | 24.859 | 46.543 | 4.681 | 1.00 | 71.21 | D |
| 2272 | CG | LEU | 535 | 23.382 | 46.585 | 4.898 | 1.00 | 69.96 | D |
| 2273 | CD1 | LEU | 535 | 23.089 | 47.679 | 5.872 | 1.00 | 70.31 | D |
| 2274 | CD2 | LEU | 535 | 22.712 | 46.857 | 3.603 | 1.00 | 69.87 | D |
| 2275 | C | LEU | 535 | 25.298 | 44.234 | 3.861 | 1.00 | 72.57 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2276 | O | LEU | 535 | 24.392 | 43.407 | 3.954 | 1.00 | 73.83 | D |
| 2277 | N | LEU | 536 | 26.099 | 44.301 | 2.807 | 1.00 | 72.53 | D |
| 2278 | CA | LEU | 536 | 25.931 | 43.381 | 1.694 | 1.00 | 72.36 | D |
| 2279 | CB | LEU | 536 | 26.849 | 43.814 | 0.551 | 1.00 | 71.10 | D |
| 2280 | CG | LEU | 536 | 26.370 | 43.781 | -0.901 | 1.00 | 69.77 | D |
| 2281 | CD1 | LEU | 536 | 25.018 | 44.439 | -1.100 | 1.00 | 69.03 | D |
| 2282 | CD2 | LEU | 536 | 27.415 | 44.504 | -1.704 | 1.00 | 70.87 | D |
| 2283 | C | LEU | 536 | 26.256 | 41.957 | 2.180 | 1.00 | 72.62 | D |
| 2284 | O | LEU | 536 | 25.642 | 40.979 | 1.755 | 1.00 | 71.52 | D |
| 2285 | N | GLU | 537 | 27.210 | 41.845 | 3.092 | 1.00 | 73.92 | D |
| 2286 | CA | GLU | 537 | 27.545 | 40.540 | 3.608 | 1.00 | 74.65 | D |
| 2287 | CB | GLU | 537 | 28.921 | 40.555 | 4.242 | 1.00 | 75.29 | D |
| 2288 | CG | GLU | 537 | 29.255 | 39.278 | 4.954 | 1.00 | 78.75 | D |
| 2289 | CD | GLU | 537 | 30.683 | 39.277 | 5.451 | 1.00 | 81.31 | D |
| 2290 | OE1 | GLU | 537 | 31.079 | 38.285 | 6.108 | 1.00 | 82.53 | D |
| 2291 | OE2 | GLU | 537 | 31.407 | 40.268 | 5.177 | 1.00 | 80.17 | D |
| 2292 | C | GLU | 537 | 26.515 | 40.027 | 4.614 | 1.00 | 74.98 | D |
| 2293 | O | GLU | 537 | 26.170 | 38.852 | 4.566 | 1.00 | 76.28 | D |
| 2294 | N | VAL | 538 | 26.001 | 40.870 | 5.515 | 1.00 | 74.17 | D |
| 2295 | CA | VAL | 538 | 25.034 | 40.334 | 6.481 | 1.00 | 72.78 | D |
| 2296 | CB | VAL | 538 | 24.761 | 41.266 | 7.717 | 1.00 | 72.66 | D |
| 2297 | CG1 | VAL | 538 | 26.006 | 42.046 | 8.094 | 1.00 | 72.43 | D |
| 2298 | CG2 | VAL | 538 | 23.564 | 42.173 | 7.459 | 1.00 | 72.20 | D |
| 2299 | C | VAL | 538 | 23.680 | 39.951 | 5.898 | 1.00 | 72.50 | D |
| 2300 | O | VAL | 538 | 22.906 | 39.274 | 6.572 | 1.00 | 72.58 | D |
| 2301 | N | ILE | 539 | 23.375 | 40.364 | 4.666 | 1.00 | 71.88 | D |
| 2302 | CA | ILE | 539 | 22.085 | 40.005 | 4.067 | 1.00 | 71.03 | D |
| 2303 | CB | ILE | 539 | 21.374 | 41.207 | 3.399 | 1.00 | 70.22 | D |
| 2304 | CG2 | ILE | 539 | 21.461 | 42.433 | 4.285 | 1.00 | 70.64 | D |
| 2305 | CG1 | ILE | 539 | 21.997 | 41.492 | 2.033 | 1.00 | 69.87 | D |
| 2306 | CD1 | ILE | 539 | 21.296 | 42.585 | 1.269 | 1.00 | 68.95 | D |
| 2307 | C | ILE | 539 | 22.209 | 38.920 | 3.006 | 1.00 | 71.27 | D |
| 2308 | O | ILE | 539 | 21.273 | 38.684 | 2.240 | 1.00 | 71.30 | D |
| 2309 | N | GLU | 540 | 23.365 | 38.269 | 2.954 | 1.00 | 71.95 | D |
| 2310 | CA | GLU | 540 | 23.598 | 37.216 | 1.980 | 1.00 | 73.04 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2311 | CB | GLU | 540 | 25.062 | 36.776 | 2.025 | 1.00 | 73.24 | D |
| 2312 | CG | GLU | 540 | 25.494 | 35.779 | 0.948 | 1.00 | 75.23 | D |
| 2313 | CD | GLU | 540 | 24.963 | 36.111 | -0.445 | 1.00 | 77.05 | D |
| 2314 | OE1 | GLU | 540 | 24.820 | 37.315 | -0.762 | 1.00 | 77.82 | D |
| 2315 | OE2 | GLU | 540 | 24.703 | 35.165 | -1.225 | 1.00 | 76.49 | D |
| 2316 | C | GLU | 540 | 22.692 | 36.060 | 2.337 | 1.00 | 73.74 | D |
| 2317 | O | GLU | 540 | 22.561 | 35.719 | 3.505 | 1.00 | 74.01 | D |
| 2318 | N | PRO | 541 | 21.993 | 35.496 | 1.355 | 1.00 | 75.38 | D |
| 2319 | CD | PRO | 541 | 21.359 | 36.155 | 0.205 | 1.00 | 75.60 | D |
| 2320 | CA | PRO | 541 | 21.174 | 34.389 | 1.854 | 1.00 | 77.84 | D |
| 2321 | CB | PRO | 541 | 20.270 | 34.029 | 0.671 | 1.00 | 77.22 | D |
| 2322 | CG | PRO | 541 | 20.590 | 35.041 | -0.424 | 1.00 | 76.96 | D |
| 2323 | C | PRO | 541 | 22.058 | 33.228 | 2.297 | 1.00 | 80.33 | D |
| 2324 | O | PRO | 541 | 23.264 | 33.203 | 2.028 | 1.00 | 80.53 | D |
| 2325 | N | GLU | 542 | 21.472 | 32.270 | 2.992 | 1.00 | 83.14 | D |
| 2326 | CA | GLU | 542 | 22.262 | 31.149 | 3.446 | 1.00 | 86.12 | D |
| 2327 | CB | GLU | 542 | 21.979 | 30.892 | 4.904 | 1.00 | 88.33 | D |
| 2328 | CG | GLU | 542 | 20.557 | 30.563 | 5.158 | 1.00 | 92.41 | D |
| 2329 | CD | GLU | 542 | 20.435 | 29.825 | 6.445 | 1.00 | 96.11 | D |
| 2330 | OE1 | GLU | 542 | 21.234 | 30.135 | 7.359 | 1.00 | 97.98 | D |
| 2331 | OE2 | GLU | 542 | 19.555 | 28.944 | 6.546 | 1.00 | 97.94 | D |
| 2332 | C | GLU | 542 | 21.913 | 29.929 | 2.617 | 1.00 | 86.19 | D |
| 2333 | O | GLU | 542 | 20.767 | 29.780 | 2.193 | 1.00 | 86.63 | D |
| 2334 | N | VAL | 543 | 22.895 | 29.060 | 2.388 | 1.00 | 86.32 | D |
| 2335 | CA | VAL | 543 | 22.674 | 27.871 | 1.570 | 1.00 | 86.78 | D |
| 2336 | CB | VAL | 543 | 23.891 | 26.926 | 1.557 | 1.00 | 87.17 | D |
| 2337 | CG1 | VAL | 543 | 25.039 | 27.566 | 0.808 | 1.00 | 87.13 | D |
| 2338 | CG2 | VAL | 543 | 24.291 | 26.566 | 2.983 | 1.00 | 87.29 | D |
| 2339 | C | VAL | 543 | 21.484 | 27.046 | 1.987 | 1.00 | 86.72 | D |
| 2340 | O | VAL | 543 | 21.296 | 26.740 | 3.160 | 1.00 | 86.85 | D |
| 2341 | N | LEU | 544 | 20.673 | 26.674 | 1.012 | 1.00 | 86.80 | D |
| 2342 | CA | LEU | 544 | 19.520 | 25.858 | 1.312 | 1.00 | 87.62 | D |
| 2343 | CB | LEU | 544 | 18.229 | 26.624 | 1.018 | 1.00 | 87.72 | D |
| 2344 | CG | LEU | 544 | 17.996 | 27.129 | -0.398 | 1.00 | 87.80 | D |
| 2345 | CD1 | LEU | 544 | 16.643 | 27.828 | -0.472 | 1.00 | 86.38 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2346 | CD2 | LEU | 544 | 19.120 | 28.074 | -0.789 | 1.00 | 89.07 | D |
| 2347 | C | LEU | 544 | 19.594 | 24.560 | 0.517 | 1.00 | 88.38 | D |
| 2348 | O | LEU | 544 | 19.826 | 24.568 | -0.700 | 1.00 | 88.44 | D |
| 2349 | N | TYR | 545 | 19.416 | 23.452 | 1.239 | 1.00 | 88.99 | D |
| 2350 | CA | TYR | 545 | 19.479 | 22.099 | 0.689 | 1.00 | 88.20 | D |
| 2351 | CB | TYR | 545 | 19.524 | 21.055 | 1.807 | 1.00 | 88.66 | D |
| 2352 | CG | TYR | 545 | 20.809 | 21.107 | 2.584 | 1.00 | 90.06 | D |
| 2353 | CD1 | TYR | 545 | 20.794 | 21.162 | 3.974 | 1.00 | 90.04 | D |
| 2354 | CE1 | TYR | 545 | 21.966 | 21.335 | 4.698 | 1.00 | 90.29 | D |
| 2355 | CD2 | TYR | 545 | 22.040 | 21.210 | 1.927 | 1.00 | 91.31 | D |
| 2356 | CE2 | TYR | 545 | 23.225 | 21.382 | 2.642 | 1.00 | 92.00 | D |
| 2357 | CZ | TYR | 545 | 23.177 | 21.452 | 4.034 | 1.00 | 91.41 | D |
| 2358 | OH | TYR | 545 | 24.319 | 21.694 | 4.770 | 1.00 | 90.69 | D |
| 2359 | C | TYR | 545 | 18.373 | 21.743 | -0.259 | 1.00 | 87.58 | D |
| 2360 | O | TYR | 545 | 17.314 | 22.373 | -0.286 | 1.00 | 87.17 | D |
| 2361 | N | ALA | 546 | 18.630 | 20.686 | -1.018 | 1.00 | 87.43 | D |
| 2362 | CA | ALA | 546 | 17.706 | 20.212 | -2.027 | 1.00 | 87.23 | D |
| 2363 | CB | ALA | 546 | 18.491 | 19.655 | -3.188 | 1.00 | 86.89 | D |
| 2364 | C | ALA | 546 | 16.667 | 19.191 | -1.578 | 1.00 | 87.25 | D |
| 2365 | O | ALA | 546 | 15.695 | 18.962 | -2.292 | 1.00 | 88.37 | D |
| 2366 | N | GLY | 547 | 16.856 | 18.576 | -0.415 | 1.00 | 86.70 | D |
| 2367 | CA | GLY | 547 | 15.893 | 17.580 | 0.027 | 1.00 | 84.97 | D |
| 2368 | C | GLY | 547 | 15.772 | 16.465 | -1.001 | 1.00 | 84.36 | D |
| 2369 | O | GLY | 547 | 14.670 | 16.076 | -1.389 | 1.00 | 84.24 | D |
| 2370 | N | TYR | 548 | 16.914 | 15.955 | -1.458 | 1.00 | 83.34 | D |
| 2371 | CA | TYR | 548 | 16.942 | 14.882 | -2.449 | 1.00 | 82.17 | D |
| 2372 | CB | TYR | 548 | 18.166 | 15.024 | -3.364 | 1.00 | 80.47 | D |
| 2373 | CG | TYR | 548 | 18.223 | 14.157 | -4.630 | 1.00 | 78.31 | D |
| 2374 | CD1 | TYR | 548 | 17.406 | 14.426 | -5.733 | 1.00 | 77.47 | D |
| 2375 | CE1 | TYR | 548 | 17.579 | 13.748 | -6.955 | 1.00 | 76.81 | D |
| 2376 | CD2 | TYR | 548 | 19.206 | 13.165 | -4.776 | 1.00 | 77.12 | D |
| 2377 | CE2 | TYR | 548 | 19.384 | 12.485 | -5.987 | 1.00 | 76.61 | D |
| 2378 | CZ | TYR | 548 | 18.579 | 12.784 | -7.072 | 1.00 | 76.85 | D |
| 2379 | OH | TYR | 548 | 18.807 | 12.151 | -8.275 | 1.00 | 76.36 | D |
| 2380 | C | TYR | 548 | 17.026 | 13.547 | -1.740 | 1.00 | 82.76 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2381 | O | TYR | 548 | 17.520 | 13.440 | -0.611 | 1.00 | 82.35 | D |
| 2382 | N | ASP | 549 | 16.520 | 12.535 | -2.419 | 1.00 | 83.15 | D |
| 2383 | CA | ASP | 549 | 16.562 | 11.184 | -1.922 | 1.00 | 83.21 | D |
| 2384 | CB | ASP | 549 | 15.230 | 10.485 | -2.134 | 1.00 | 83.27 | D |
| 2385 | CG | ASP | 549 | 15.216 | 9.110 | -1.531 | 1.00 | 83.93 | D |
| 2386 | OD1 | ASP | 549 | 16.305 | 8.643 | -1.146 | 1.00 | 85.23 | D |
| 2387 | OD2 | ASP | 549 | 14.132 | 8.502 | -1.443 | 1.00 | 84.20 | D |
| 2388 | C | ASP | 549 | 17.609 | 10.556 | -2.817 | 1.00 | 83.68 | D |
| 2389 | O | ASP | 549 | 17.330 | 10.226 | -3.971 | 1.00 | 83.55 | D |
| 2390 | N | SER | 550 | 18.821 | 10.430 | -2.298 | 1.00 | 84.19 | D |
| 2391 | CA | SER | 550 | 19.920 | 9.853 | -3.054 | 1.00 | 85.11 | D |
| 2392 | CB | SER | 550 | 21.225 | 10.584 | -2.720 | 1.00 | 84.86 | D |
| 2393 | OG | SER | 550 | 21.524 | 10.497 | -1.335 | 1.00 | 85.47 | D |
| 2394 | C | SER | 550 | 20.028 | 8.384 | -2.686 | 1.00 | 85.74 | D |
| 2395 | O | SER | 550 | 21.019 | 7.718 | -2.992 | 1.00 | 85.66 | D |
| 2396 | N | SER | 551 | 18.982 | 7.897 | -2.023 | 1.00 | 86.44 | D |
| 2397 | CA | SER | 551 | 18.902 | 6.515 | -1.570 | 1.00 | 86.68 | D |
| 2398 | CB | SER | 551 | 17.908 | 6.387 | -0.431 | 1.00 | 86.76 | D |
| 2399 | OG | SER | 551 | 16.603 | 6.257 | -0.965 | 1.00 | 88.21 | D |
| 2400 | C | SER | 551 | 18.418 | 5.639 | -2.696 | 1.00 | 86.60 | D |
| 2401 | O | SER | 551 | 18.192 | 4.441 | -2.527 | 1.00 | 86.16 | D |
| 2402 | N | VAL | 552 | 18.212 | 6.235 | -3.854 | 1.00 | 87.52 | D |
| 2403 | CA | VAL | 552 | 17.744 | 5.444 | -4.955 | 1.00 | 88.87 | D |
| 2404 | CB | VAL | 552 | 16.263 | 5.469 | -4.935 | 1.00 | 88.89 | D |
| 2405 | CG1 | VAL | 552 | 15.800 | 5.509 | -3.488 | 1.00 | 88.42 | D |
| 2406 | CG2 | VAL | 552 | 15.778 | 6.674 | -5.638 | 1.00 | 88.55 | D |
| 2407 | C | VAL | 552 | 18.349 | 6.072 | -6.192 | 1.00 | 89.48 | D |
| 2408 | O | VAL | 552 | 18.434 | 7.303 | -6.289 | 1.00 | 89.94 | D |
| 2409 | N | PRO | 553 | 18.749 | 5.233 | -7.166 | 1.00 | 90.53 | D |
| 2410 | CD | PRO | 553 | 18.293 | 3.842 | -7.338 | 1.00 | 91.20 | D |
| 2411 | CA | PRO | 553 | 19.382 | 5.726 | -8.389 | 1.00 | 91.54 | D |
| 2412 | CB | PRO | 553 | 19.267 | 4.570 | -9.388 | 1.00 | 91.76 | D |
| 2413 | CG | PRO | 553 | 18.260 | 3.673 | -8.844 | 1.00 | 91.49 | D |
| 2414 | C | PRO | 553 | 18.968 | 7.021 | -8.977 | 1.00 | 92.07 | D |
| 2415 | O | PRO | 553 | 17.865 | 7.522 | -8.784 | 1.00 | 92.26 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2416 | N | ASP | 554 | 19.915 | 7.570 | -9.712 | 1.00 | 92.21 | D |
| 2417 | CA | ASP | 554 | 19.671 | 8.819 | -10.342 | 1.00 | 92.25 | D |
| 2418 | CB | ASP | 554 | 20.943 | 9.625 | -10.433 | 1.00 | 91.71 | D |
| 2419 | CG | ASP | 554 | 21.565 | 9.814 | -9.105 | 1.00 | 91.92 | D |
| 2420 | OD1 | ASP | 554 | 20.807 | 9.769 | -8.111 | 1.00 | 92.26 | D |
| 2421 | OD2 | ASP | 554 | 22.793 | 10.007 | -9.047 | 1.00 | 91.66 | D |
| 2422 | C | ASP | 554 | 19.149 | 8.605 | -11.701 | 1.00 | 92.49 | D |
| 2423 | O | ASP | 554 | 19.017 | 7.491 | -12.204 | 1.00 | 93.01 | D |
| 2424 | N | SER | 555 | 18.841 | 9.728 | -12.295 | 1.00 | 92.63 | D |
| 2425 | CA | SER | 555 | 18.365 | 9.750 | -13.628 | 1.00 | 93.51 | D |
| 2426 | CB | SER | 555 | 17.024 | 9.031 | -13.751 | 1.00 | 93.19 | D |
| 2427 | OG | SER | 555 | 15.938 | 9.844 | -13.371 | 1.00 | 93.50 | D |
| 2428 | C | SER | 555 | 18.253 | 11.225 | -13.787 | 1.00 | 94.69 | D |
| 2429 | O | SER | 555 | 17.974 | 11.959 | -12.834 | 1.00 | 95.13 | D |
| 2430 | N | THR | 556 | 18.534 | 11.675 | -14.992 | 1.00 | 95.90 | D |
| 2431 | CA | THR | 556 | 18.504 | 13.080 | -15.220 | 1.00 | 96.67 | D |
| 2432 | CB | THR | 556 | 18.659 | 13.418 | -16.575 | 1.00 | 97.22 | D |
| 2433 | OG1 | THR | 556 | 19.705 | 12.608 | -17.088 | 1.00 | 98.43 | D |
| 2434 | CG2 | THR | 556 | 19.003 | 14.886 | -16.674 | 1.00 | 97.19 | D |
| 2435 | C | THR | 556 | 17.242 | 13.616 | -14.836 | 1.00 | 96.67 | D |
| 2436 | O | THR | 556 | 17.239 | 14.267 | -13.831 | 1.00 | 96.78 | D |
| 2437 | N | TRP | 557 | 16.198 | 13.399 | -15.645 | 1.00 | 97.24 | D |
| 2438 | CA | TRP | 557 | 14.885 | 13.878 | -15.294 | 1.00 | 97.34 | D |
| 2439 | CB | TRP | 557 | 13.843 | 12.915 | -15.846 | 1.00 | 100.14 | D |
| 2440 | CG | TRP | 557 | 13.864 | 13.048 | -17.342 | 1.00 | 103.86 | D |
| 2441 | CD2 | TRP | 557 | 13.027 | 12.435 | -18.291 | 1.00 | 105.46 | D |
| 2442 | CE2 | TRP | 557 | 13.343 | 12.882 | -19.587 | 1.00 | 106.20 | D |
| 2443 | CE3 | TRP | 557 | 11.906 | 11.550 | -18.197 | 1.00 | 105.69 | D |
| 2444 | CD1 | TRP | 557 | 14.738 | 13.832 | -18.082 | 1.00 | 104.90 | D |
| 2445 | NE1 | TRP | 557 | 14.464 | 13.754 | -19.414 | 1.00 | 105.88 | D |
| 2446 | CZ2 | TRP | 557 | 12.747 | 12.485 | -20.763 | 1.00 | 106.15 | D |
| 2447 | CZ3 | TRP | 557 | 11.277 | 11.129 | -19.365 | 1.00 | 105.93 | D |
| 2448 | CH2 | TRP | 557 | 11.674 | 11.619 | -20.623 | 1.00 | 106.20 | D |
| 2449 | C | TRP | 557 | 15.061 | 13.885 | -13.803 | 1.00 | 95.80 | D |
| 2450 | O | TRP | 557 | 15.879 | 14.624 | -13.353 | 1.00 | 95.83 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2451 | N | ARG | 558 | 14.400 | 13.116 | -12.985 | 1.00 | 93.50 | D |
| 2452 | CA | ARG | 558 | 14.743 | 13.277 | -11.573 | 1.00 | 91.63 | D |
| 2453 | CB | ARG | 558 | 15.415 | 12.048 | -11.058 | 1.00 | 91.10 | D |
| 2454 | CG | ARG | 558 | 15.020 | 11.954 | -9.656 | 1.00 | 90.49 | D |
| 2455 | CD | ARG | 558 | 14.884 | 10.562 | -9.315 | 1.00 | 89.68 | D |
| 2456 | NE | ARG | 558 | 15.970 | 10.187 | -8.440 | 1.00 | 89.44 | D |
| 2457 | CZ | ARG | 558 | 15.933 | 10.373 | -7.130 | 1.00 | 89.73 | D |
| 2458 | NH1 | ARG | 558 | 14.861 | 10.929 | -6.578 | 1.00 | 88.39 | D |
| 2459 | NH2 | ARG | 558 | 16.943 | 9.976 | -6.368 | 1.00 | 89.50 | D |
| 2460 | C | ARG | 558 | 15.577 | 14.447 | -11.003 | 1.00 | 90.86 | D |
| 2461 | O | ARG | 558 | 15.171 | 15.095 | -10.053 | 1.00 | 90.88 | D |
| 2462 | N | ILE | 559 | 16.756 | 14.695 | -11.544 | 1.00 | 89.58 | D |
| 2463 | CA | ILE | 559 | 17.604 | 15.729 | -10.998 | 1.00 | 88.94 | D |
| 2464 | CB | ILE | 559 | 18.964 | 15.397 | -11.261 | 1.00 | 88.55 | D |
| 2465 | CG2 | ILE | 559 | 19.823 | 16.584 | -11.025 | 1.00 | 88.39 | D |
| 2466 | CG1 | ILE | 559 | 19.338 | 14.230 | -10.403 | 1.00 | 88.44 | D |
| 2467 | CD1 | ILE | 559 | 20.792 | 14.151 | -10.319 | 1.00 | 88.21 | D |
| 2468 | C | ILE | 559 | 17.438 | 17.109 | -11.513 | 1.00 | 88.86 | D |
| 2469 | O | ILE | 559 | 17.733 | 18.112 | -10.853 | 1.00 | 88.86 | D |
| 2470 | N | MET | 560 | 17.098 | 17.147 | -12.772 | 1.00 | 88.53 | D |
| 2471 | CA | MET | 560 | 16.863 | 18.409 | -13.360 | 1.00 | 88.39 | D |
| 2472 | CB | MET | 560 | 16.426 | 18.226 | -14.761 | 1.00 | 88.54 | D |
| 2473 | CG | MET | 560 | 17.550 | 17.945 | -15.603 | 1.00 | 89.76 | D |
| 2474 | SD | MET | 560 | 17.064 | 17.908 | -17.282 | 1.00 | 92.87 | D |
| 2475 | CE | MET | 560 | 18.550 | 18.420 | -18.053 | 1.00 | 92.53 | D |
| 2476 | C | MET | 560 | 15.653 | 18.747 | -12.618 | 1.00 | 88.07 | D |
| 2477 | O | MET | 560 | 15.393 | 19.883 | -12.229 | 1.00 | 88.78 | D |
| 2478 | N | THR | 561 | 14.867 | 17.727 | -12.389 | 1.00 | 86.83 | D |
| 2479 | CA | THR | 561 | 13.682 | 18.152 | -11.800 | 1.00 | 85.30 | D |
| 2480 | CB | THR | 561 | 12.662 | 17.110 | -11.851 | 1.00 | 84.89 | D |
| 2481 | OG1 | THR | 561 | 11.788 | 17.422 | -12.945 | 1.00 | 84.68 | D |
| 2482 | CG2 | THR | 561 | 11.909 | 17.094 | -10.585 | 1.00 | 84.42 | D |
| 2483 | C | THR | 561 | 13.902 | 18.736 | -10.448 | 1.00 | 84.31 | D |
| 2484 | O | THR | 561 | 13.522 | 19.882 | -10.225 | 1.00 | 84.37 | D |
| 2485 | N | THR | 562 | 14.555 | 17.990 | -9.569 | 1.00 | 83.16 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2486 | CA | THR | 562 | 14.833 | 18.504 | -8.251 | 1.00 | 82.17 | D |
| 2487 | CB | THR | 562 | 15.786 | 17.565 | -7.420 | 1.00 | 82.07 | D |
| 2488 | OG1 | THR | 562 | 17.047 | 17.455 | -8.089 | 1.00 | 82.61 | D |
| 2489 | CG2 | THR | 562 | 15.185 | 16.154 | -7.228 | 1.00 | 82.32 | D |
| 2490 | C | THR | 562 | 15.475 | 19.901 | -8.403 | 1.00 | 81.33 | D |
| 2491 | O | THR | 562 | 15.039 | 20.807 | -7.712 | 1.00 | 82.35 | D |
| 2492 | N | LEU | 563 | 16.442 | 20.097 | -9.317 | 1.00 | 79.93 | D |
| 2493 | CA | LEU | 563 | 17.134 | 21.413 | -9.487 | 1.00 | 78.45 | D |
| 2494 | CB | LEU | 563 | 18.268 | 21.323 | -10.557 | 1.00 | 77.66 | D |
| 2495 | CG | LEU | 563 | 19.514 | 20.947 | -9.730 | 1.00 | 78.54 | D |
| 2496 | CD1 | LEU | 563 | 18.945 | 20.372 | -8.463 | 1.00 | 79.05 | D |
| 2497 | CD2 | LEU | 563 | 20.458 | 19.923 | -10.336 | 1.00 | 78.61 | D |
| 2498 | C | LEU | 563 | 16.264 | 22.641 | -9.693 | 1.00 | 77.89 | D |
| 2499 | O | LEU | 563 | 16.525 | 23.707 | -9.131 | 1.00 | 77.49 | D |
| 2500 | N | ASN | 564 | 15.211 | 22.470 | -10.470 | 1.00 | 77.41 | D |
| 2501 | CA | ASN | 564 | 14.231 | 23.514 | -10.738 | 1.00 | 76.89 | D |
| 2502 | CB | ASN | 564 | 13.261 | 23.004 | -11.770 | 1.00 | 75.58 | D |
| 2503 | CG | ASN | 564 | 13.809 | 23.077 | -13.162 | 1.00 | 74.85 | D |
| 2504 | OD1 | ASN | 564 | 15.013 | 23.104 | -13.393 | 1.00 | 75.81 | D |
| 2505 | ND2 | ASN | 564 | 12.922 | 23.088 | -14.103 | 1.00 | 75.21 | D |
| 2506 | C | ASN | 564 | 13.465 | 23.900 | -9.473 | 1.00 | 76.70 | D |
| 2507 | O | ASN | 564 | 13.204 | 25.076 | -9.222 | 1.00 | 76.62 | D |
| 2508 | N | MET | 565 | 13.062 | 22.901 | -8.699 | 1.00 | 77.18 | D |
| 2509 | CA | MET | 565 | 12.367 | 23.187 | -7.457 | 1.00 | 77.91 | D |
| 2510 | CB | MET | 565 | 12.112 | 21.901 | -6.675 | 1.00 | 79.42 | D |
| 2511 | CG | MET | 565 | 10.648 | 21.571 | -6.452 | 1.00 | 81.29 | D |
| 2512 | SD | MET | 565 | 9.742 | 21.346 | -7.996 | 1.00 | 83.79 | D |
| 2513 | CE | MET | 565 | 8.275 | 22.311 | -7.661 | 1.00 | 84.35 | D |
| 2514 | C | MET | 565 | 13.367 | 24.047 | -6.695 | 1.00 | 77.81 | D |
| 2515 | O | MET | 565 | 13.056 | 25.148 | -6.229 | 1.00 | 77.62 | D |
| 2516 | N | LEU | 566 | 14.592 | 23.542 | -6.602 | 1.00 | 77.54 | D |
| 2517 | CA | LEU | 566 | 15.623 | 24.263 | -5.894 | 1.00 | 77.35 | D |
| 2518 | CB | LEU | 566 | 16.983 | 23.594 | -6.052 | 1.00 | 76.46 | D |
| 2519 | CG | LEU | 566 | 17.908 | 24.447 | -5.190 | 1.00 | 76.14 | D |
| 2520 | CD1 | LEU | 566 | 17.281 | 24.541 | -3.813 | 1.00 | 76.45 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2521 | CD2 | LEU | 566 | 19.301 | 23.887 | -5.113 | 1.00 | 76.14 | D |
| 2522 | C | LEU | 566 | 15.749 | 25.705 | -6.346 | 1.00 | 78.03 | D |
| 2523 | O | LEU | 566 | 15.956 | 26.598 | -5.524 | 1.00 | 78.22 | D |
| 2524 | N | GLY | 567 | 15.644 | 25.925 | -7.653 | 1.00 | 78.32 | D |
| 2525 | CA | GLY | 567 | 15.766 | 27.264 | -8.199 | 1.00 | 78.16 | D |
| 2526 | C | GLY | 567 | 14.644 | 28.156 | -7.715 | 1.00 | 78.69 | D |
| 2527 | O | GLY | 567 | 14.853 | 29.334 | -7.410 | 1.00 | 78.69 | D |
| 2528 | N | GLY | 568 | 13.451 | 27.572 | -7.658 | 1.00 | 78.97 | D |
| 2529 | CA | GLY | 568 | 12.291 | 28.295 | -7.192 | 1.00 | 77.68 | D |
| 2530 | C | GLY | 568 | 12.574 | 28.751 | -5.785 | 1.00 | 77.16 | D |
| 2531 | O | GLY | 568 | 12.456 | 29.926 | -5.460 | 1.00 | 78.00 | D |
| 2532 | N | ARG | 569 | 12.983 | 27.846 | -4.922 | 1.00 | 75.95 | D |
| 2533 | CA | ARG | 569 | 13.219 | 28.320 | -3.589 | 1.00 | 76.63 | D |
| 2534 | CB | ARG | 569 | 13.493 | 27.128 | -2.691 | 1.00 | 78.37 | D |
| 2535 | CG | ARG | 569 | 12.355 | 26.113 | -2.806 | 1.00 | 81.13 | D |
| 2536 | CD | ARG | 569 | 12.650 | 24.904 | -1.976 | 1.00 | 83.19 | D |
| 2537 | NE | ARG | 569 | 13.118 | 25.333 | -0.668 | 1.00 | 84.03 | D |
| 2538 | CZ | ARG | 569 | 14.210 | 24.856 | -0.090 | 1.00 | 85.57 | D |
| 2539 | NH1 | ARG | 569 | 14.933 | 23.933 | -0.712 | 1.00 | 85.65 | D |
| 2540 | NH2 | ARG | 569 | 14.591 | 25.317 | 1.094 | 1.00 | 86.82 | D |
| 2541 | C | ARG | 569 | 14.304 | 29.402 | -3.504 | 1.00 | 76.48 | D |
| 2542 | O | ARG | 569 | 14.198 | 30.301 | -2.673 | 1.00 | 77.05 | D |
| 2543 | N | GLN | 570 | 15.307 | 29.353 | -4.390 | 1.00 | 75.83 | D |
| 2544 | CA | GLN | 570 | 16.409 | 30.332 | -4.394 | 1.00 | 74.43 | D |
| 2545 | CB | GLN | 570 | 17.633 | 29.763 | -5.110 | 1.00 | 75.14 | D |
| 2546 | CG | GLN | 570 | 18.513 | 28.845 | -4.290 | 1.00 | 74.93 | D |
| 2547 | CD | GLN | 570 | 19.487 | 28.081 | -5.164 | 1.00 | 74.79 | D |
| 2548 | OE1 | GLN | 570 | 20.479 | 27.532 | -4.681 | 1.00 | 75.77 | D |
| 2549 | NE2 | GLN | 570 | 19.197 | 28.032 | -6.463 | 1.00 | 73.98 | D |
| 2550 | C | GLN | 570 | 16.112 | 31.684 | -5.026 | 1.00 | 74.13 | D |
| 2551 | O | GLN | 570 | 16.736 | 32.687 | -4.669 | 1.00 | 74.57 | D |
| 2552 | N | VAL | 571 | 15.197 | 31.707 | -5.994 | 1.00 | 72.68 | D |
| 2553 | CA | VAL | 571 | 14.834 | 32.951 | -6.664 | 1.00 | 71.57 | D |
| 2554 | CB | VAL | 571 | 14.183 | 32.649 | -8.039 | 1.00 | 71.29 | D |
| 2555 | CG1 | VAL | 571 | 13.528 | 33.895 | -8.630 | 1.00 | 70.57 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2556 | CG2 | VAL | 571 | 15.264 | 32.134 | -8.985 | 1.00 | 70.98 | D |
| 2557 | C | VAL | 571 | 13.911 | 33.739 | -5.736 | 1.00 | 70.76 | D |
| 2558 | O | VAL | 571 | 13.829 | 34.972 | -5.805 | 1.00 | 70.66 | D |
| 2559 | N | ILE | 572 | 13.244 | 33.004 | -4.851 | 1.00 | 69.37 | D |
| 2560 | CA | ILE | 572 | 12.356 | 33.588 | -3.860 | 1.00 | 67.55 | D |
| 2561 | CB | ILE | 572 | 11.428 | 32.507 | -3.247 | 1.00 | 67.43 | D |
| 2562 | CG2 | ILE | 572 | 11.025 | 32.883 | -1.821 | 1.00 | 66.71 | D |
| 2563 | CG1 | ILE | 572 | 10.210 | 32.330 | -4.166 | 1.00 | 65.46 | D |
| 2564 | CD1 | ILE | 572 | 9.499 | 31.009 | -4.031 | 1.00 | 64.46 | D |
| 2565 | C | ILE | 572 | 13.246 | 34.234 | -2.804 | 1.00 | 66.03 | D |
| 2566 | O | ILE | 572 | 12.946 | 35.316 | -2.317 | 1.00 | 67.56 | D |
| 2567 | N | ALA | 573 | 14.355 | 33.588 | -2.471 | 1.00 | 64.63 | D |
| 2568 | CA | ALA | 573 | 15.275 | 34.170 | -1.501 | 1.00 | 64.65 | D |
| 2569 | CB | ALA | 573 | 16.281 | 33.116 | -1.028 | 1.00 | 64.20 | D |
| 2570 | C | ALA | 573 | 16.009 | 35.348 | -2.159 | 1.00 | 65.16 | D |
| 2571 | O | ALA | 573 | 16.571 | 36.213 | -1.466 | 1.00 | 63.91 | D |
| 2572 | N | ALA | 574 | 15.980 | 35.367 | -3.499 | 1.00 | 65.57 | D |
| 2573 | CA | ALA | 574 | 16.626 | 36.403 | -4.334 | 1.00 | 65.65 | D |
| 2574 | CB | ALA | 574 | 16.706 | 35.916 | -5.791 | 1.00 | 64.41 | D |
| 2575 | C | ALA | 574 | 15.873 | 37.732 | -4.267 | 1.00 | 65.77 | D |
| 2576 | O | ALA | 574 | 16.442 | 38.816 | -4.435 | 1.00 | 64.59 | D |
| 2577 | N | VAL | 575 | 14.572 | 37.624 | -4.037 | 1.00 | 66.30 | D |
| 2578 | CA | VAL | 575 | 13.717 | 38.774 | -3.916 | 1.00 | 67.41 | D |
| 2579 | CB | VAL | 575 | 12.261 | 38.365 | -4.194 | 1.00 | 67.80 | D |
| 2580 | CG1 | VAL | 575 | 11.342 | 39.580 | -4.126 | 1.00 | 66.72 | D |
| 2581 | CG2 | VAL | 575 | 12.183 | 37.695 | -5.572 | 1.00 | 68.22 | D |
| 2582 | C | VAL | 575 | 13.903 | 39.353 | -2.500 | 1.00 | 68.51 | D |
| 2583 | O | VAL | 575 | 14.030 | 40.573 | -2.363 | 1.00 | 68.32 | D |
| 2584 | N | LYS | 576 | 13.937 | 38.516 | -1.448 | 1.00 | 68.40 | D |
| 2585 | CA | LYS | 576 | 14.159 | 39.085 | -0.103 | 1.00 | 69.53 | D |
| 2586 | CB | LYS | 576 | 14.044 | 38.055 | 1.073 | 1.00 | 72.11 | D |
| 2587 | CG | LYS | 576 | 12.641 | 37.402 | 1.379 | 1.00 | 77.34 | D |
| 2588 | CD | LYS | 576 | 12.456 | 36.763 | 2.842 | 1.00 | 80.60 | D |
| 2589 | CE | LYS | 576 | 13.443 | 35.602 | 3.211 | 1.00 | 83.74 | D |
| 2590 | NZ | LYS | 576 | 13.115 | 34.750 | 4.436 | 1.00 | 83.62 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2591 | C | LYS | 576 | 15.593 | 39.643 | -0.132 | 1.00 | 68.77 | D |
| 2592 | O | LYS | 576 | 15.895 | 40.648 | 0.514 | 1.00 | 70.13 | D |
| 2593 | N | TRP | 577 | 16.489 | 39.007 | -0.878 | 1.00 | 66.43 | D |
| 2594 | CA | TRP | 577 | 17.842 | 39.536 | -0.913 | 1.00 | 63.78 | D |
| 2595 | CB | TRP | 577 | 18.790 | 38.561 | -1.620 | 1.00 | 61.85 | D |
| 2596 | CG | TRP | 577 | 20.107 | 39.167 | -1.989 | 1.00 | 58.40 | D |
| 2597 | CD2 | TRP | 577 | 20.517 | 39.533 | -3.302 | 1.00 | 57.61 | D |
| 2598 | CE2 | TRP | 577 | 21.836 | 40.028 | -3.210 | 1.00 | 57.50 | D |
| 2599 | CE3 | TRP | 577 | 19.920 | 39.441 | -4.565 | 1.00 | 56.82 | D |
| 2600 | CD1 | TRP | 577 | 21.136 | 39.486 | -1.158 | 1.00 | 57.68 | D |
| 2601 | NE1 | TRP | 577 | 22.182 | 40.009 | -1.884 | 1.00 | 58.31 | D |
| 2602 | CZ2 | TRP | 577 | 22.542 | 40.486 | -4.322 | 1.00 | 57.43 | D |
| 2603 | CZ3 | TRP | 577 | 20.617 | 39.894 | -5.668 | 1.00 | 56.94 | D |
| 2604 | CH2 | TRP | 577 | 21.928 | 40.389 | -5.545 | 1.00 | 57.05 | D |
| 2605 | C | TRP | 577 | 17.856 | 40.901 | -1.609 | 1.00 | 62.95 | D |
| 2606 | O | TRP | 577 | 18.328 | 41.881 | -1.035 | 1.00 | 61.99 | D |
| 2607 | N | ALA | 578 | 17.324 | 40.959 | -2.826 | 1.00 | 62.89 | D |
| 2608 | CA | ALA | 578 | 17.288 | 42.201 | -3.610 | 1.00 | 63.57 | D |
| 2609 | CB | ALA | 578 | 16.480 | 41.988 | -4.895 | 1.00 | 62.98 | D |
| 2610 | C | ALA | 578 | 16.724 | 43.380 | -2.821 | 1.00 | 64.15 | D |
| 2611 | O | ALA | 578 | 17.341 | 44.446 | -2.769 | 1.00 | 63.71 | D |
| 2612 | N | LYS | 579 | 15.557 | 43.178 | -2.213 | 1.00 | 64.96 | D |
| 2613 | CA | LYS | 579 | 14.901 | 44.196 | -1.406 | 1.00 | 65.52 | D |
| 2614 | CB | LYS | 579 | 13.617 | 43.610 | -0.812 | 1.00 | 67.19 | D |
| 2615 | CG | LYS | 579 | 12.524 | 43.330 | -1.856 | 1.00 | 68.64 | D |
| 2616 | CD | LYS | 579 | 11.399 | 42.409 | -1.353 | 1.00 | 69.03 | D |
| 2617 | CE | LYS | 579 | 11.014 | 42.666 | 0.106 | 1.00 | 69.80 | D |
| 2618 | NZ | LYS | 579 | 9.561 | 42.402 | 0.366 | 1.00 | 70.70 | D |
| 2619 | C | LYS | 579 | 15.809 | 44.754 | -0.293 | 1.00 | 65.43 | D |
| 2620 | O | LYS | 579 | 15.728 | 45.933 | 0.044 | 1.00 | 65.59 | D |
| 2621 | N | ALA | 580 | 16.687 | 43.930 | 0.264 | 1.00 | 64.59 | D |
| 2622 | CA | ALA | 580 | 17.577 | 44.407 | 1.325 | 1.00 | 64.93 | D |
| 2623 | CB | ALA | 580 | 18.038 | 43.234 | 2.192 | 1.00 | 65.44 | D |
| 2624 | C | ALA | 580 | 18.793 | 45.178 | 0.817 | 1.00 | 64.74 | D |
| 2625 | O | ALA | 580 | 19.532 | 45.780 | 1.604 | 1.00 | 64.88 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2626 | N | ILE | 581 | 19.013 | 45.157 | -0.492 | 1.00 | 64.45 | D |
| 2627 | CA | ILE | 581 | 20.152 | 45.867 | -1.048 | 1.00 | 64.93 | D |
| 2628 | CB | ILE | 581 | 20.382 | 45.492 | -2.515 | 1.00 | 63.01 | D |
| 2629 | CG2 | ILE | 581 | 21.375 | 46.449 | -3.160 | 1.00 | 62.56 | D |
| 2630 | CG1 | ILE | 581 | 20.908 | 44.068 | -2.592 | 1.00 | 60.84 | D |
| 2631 | CD1 | ILE | 581 | 20.782 | 43.502 | -3.944 | 1.00 | 59.17 | D |
| 2632 | C | ILE | 581 | 19.916 | 47.364 | -0.949 | 1.00 | 66.48 | D |
| 2633 | O | ILE | 581 | 18.937 | 47.900 | -1.473 | 1.00 | 67.02 | D |
| 2634 | N | PRO | 582 | 20.806 | 48.055 | -0.238 | 1.00 | 67.71 | D |
| 2635 | CD | PRO | 582 | 21.885 | 47.468 | 0.572 | 1.00 | 68.94 | D |
| 2636 | CA | PRO | 582 | 20.747 | 49.499 | -0.039 | 1.00 | 69.60 | D |
| 2637 | CB | PRO | 582 | 22.138 | 49.810 | 0.481 | 1.00 | 69.24 | D |
| 2638 | CG | PRO | 582 | 22.394 | 48.676 | 1.353 | 1.00 | 69.39 | D |
| 2639 | C | PRO | 582 | 20.452 | 50.200 | -1.349 | 1.00 | 70.14 | D |
| 2640 | O | PRO | 582 | 21.234 | 50.111 | -2.296 | 1.00 | 70.73 | D |
| 2641 | N | GLY | 583 | 19.318 | 50.885 | -1.408 | 1.00 | 70.94 | D |
| 2642 | CA | GLY | 583 | 18.947 | 51.599 | -2.611 | 1.00 | 72.37 | D |
| 2643 | C | GLY | 583 | 18.149 | 50.832 | -3.652 | 1.00 | 74.21 | D |
| 2644 | O | GLY | 583 | 17.795 | 51.397 | -4.676 | 1.00 | 74.83 | D |
| 2645 | N | PHE | 584 | 17.844 | 49.555 | -3.422 | 1.00 | 74.85 | D |
| 2646 | CA | PHE | 584 | 17.092 | 48.813 | -4.429 | 1.00 | 74.38 | D |
| 2647 | CB | PHE | 584 | 17.361 | 47.309 | -4.321 | 1.00 | 70.65 | D |
| 2648 | CG | PHE | 584 | 16.647 | 46.498 | -5.369 | 1.00 | 67.79 | D |
| 2649 | CD1 | PHE | 584 | 17.111 | 46.468 | -6.684 | 1.00 | 65.83 | D |
| 2650 | CD2 | PHE | 584 | 15.470 | 45.821 | -5.056 | 1.00 | 66.67 | D |
| 2651 | CE1 | PHE | 584 | 16.404 | 45.783 | -7.677 | 1.00 | 63.42 | D |
| 2652 | CE2 | PHE | 584 | 14.756 | 45.138 | -6.039 | 1.00 | 65,18 | D |
| 2653 | CZ | PHE | 584 | 15.225 | 45.120 | -7.353 | 1.00 | 64.10 | D |
| 2654 | C | PHE | 584 | 15.587 | 49.053 | -4.351 | 1.00 | 76.56 | D |
| 2655 | O | PHE | 584 | 14.924 | 49.153 | -5.385 | 1.00 | 76.30 | D |
| 2656 | N | ARG | 585 | 15.058 | 49.140 | -3.131 | 1.00 | 79.68 | D |
| 2657 | CA | ARG | 585 | 13.620 | 49.343 | -2.898 | 1.00 | 81.83 | D |
| 2658 | CB | ARG | 585 | 13.277 | 49.230 | -1.404 | 1.00 | 82.57 | D |
| 2659 | CG | ARG | 585 | 13.320 | 47.822 | -0.844 | 1.00 | 85.17 | D |
| 2660 | CD | ARG | 585 | 12.282 | 47.604 | 0.253 | 1.00 | 87.14 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2661 | NE | ARG | 585 | 10.921 | 47.879 | -0.208 | 1.00 | 89.83 | D |
| 2662 | CZ | ARG | 585 | 10.378 | 47.395 | -1.327 | 1.00 | 90.53 | D |
| 2663 | NH1 | ARG | 585 | 11.073 | 46.596 | -2.130 | 1.00 | 89.47 | D |
| 2664 | NH2 | ARG | 585 | 9.130 | 47.717 | -1.650 | 1.00 | 90.67 | D |
| 2665 | C | ARG | 585 | 13.105 | 50.680 | -3.394 | 1.00 | 82.68 | D |
| 2666 | O | ARG | 585 | 11.961 | 50.800 | -3.841 | 1.00 | 82.83 | D |
| 2667 | N | ASN | 586 | 13.957 | 51.689 | -3.302 | 1.00 | 83.37 | D |
| 2668 | CA | ASN | 586 | 13.582 | 53.021 | -3.717 | 1.00 | 84.07 | D |
| 2669 | CB | ASN | 586 | 14.559 | 54.021 | -3.111 | 1.00 | 85.16 | D |
| 2670 | CG | ASN | 586 | 14.559 | 53.959 | -1.593 | 1.00 | 86.57 | D |
| 2671 | OD1 | ASN | 586 | 15.605 | 53.793 | -0.958 | 1.00 | 86.08 | D |
| 2672 | ND2 | ASN | 586 | 13.368 | 54.076 | -1.004 | 1.00 | 87.02 | D |
| 2673 | C | ASN | 586 | 13.482 | 53.180 | -5.218 | 1.00 | 83.72 | D |
| 2674 | O | ASN | 586 | 13.251 | 54.274 | -5.715 | 1.00 | 84.47 | D |
| 2675 | N | LEU | 587 | 13.668 | 52.105 | -5.963 | 1.00 | 83.66 | D |
| 2676 | CA | LEU | 587 | 13.506 | 52.244 | -7.394 | 1.00 | 83.96 | D |
| 2677 | CB | LEU | 587 | 14.377 | 51.266 | -8.177 | 1.00 | 82.49 | D |
| 2678 | CG | LEU | 587 | 15.893 | 51.449 | -8.222 | 1.00 | 80.62 | D |
| 2679 | CD1 | LEU | 587 | 16.502 | 50.072 | -8.268 | 1.00 | 80.84 | D |
| 2680 | CD2 | LEU | 587 | 16.339 | 52.269 | -9.424 | 1.00 | 78.79 | D |
| 2681 | C | LEU | 587 | 12.049 | 51.908 | -7.606 | 1.00 | 85.36 | D |
| 2682 | O | LEU | 587 | 11.378 | 51.373 | -6.721 | 1.00 | 85.16 | D |
| 2683 | N | HIS | 588 | 11.561 | 52.223 | -8.792 | 1.00 | 86.53 | D |
| 2684 | CA | HIS | 588 | 10.186 | 51.949 | -9.110 | 1.00 | 87.58 | D |
| 2685 | CB | HIS | 588 | 9.908 | 52.395 | -10.533 | 1.00 | 88.54 | D |
| 2686 | CG | HIS | 588 | 8.461 | 52.636 | -10.806 | 1.00 | 90.27 | D |
| 2687 | CD2 | HIS | 588 | 7.739 | 53.783 | -10.820 | 1.00 | 90.56 | D |
| 2688 | ND1 | HIS | 588 | 7.567 | 51.616 | -11.046 | 1.00 | 90.27 | D |
| 2689 | CE1 | HIS | 588 | 6.358 | 52.122 | -11.200 | 1.00 | 90.42 | D |
| 2690 | NE2 | HIS | 588 | 6.437 | 53.436 | -11.068 | 1.00 | 90.75 | D |
| 2691 | C | HIS | 588 | 9.900 | 50.461 | -8.957 | 1.00 | 87.99 | D |
| 2692 | O | HIS | 588 | 10.651 | 49.636 | -9.464 | 1.00 | 88.40 | D |
| 2693 | N | LEU | 589 | 8.821 | 50.129 | -8.260 | 1.00 | 88.47 | D |
| 2694 | CA | LEU | 589 | 8.421 | 48.740 | -8.052 | 1.00 | 88.53 | D |
| 2695 | CB | LEU | 589 | 7.093 | 48.679 | -7.306 | 1.00 | 88.25 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2696 | CG | LEU | 589 | 6.842 | 47.395 | -6.527 | 1.00 | 89.05 | D |
| 2697 | CD1 | LEU | 589 | 6.931 | 47.743 | -5.056 | 1.00 | 89.76 | D |
| 2698 | CD2 | LEU | 589 | 5.482 | 46.787 | -6.860 | 1.00 | 88.71 | D |
| 2699 | C | LEU | 589 | 8.256 | 48.003 | -9.382 | 1.00 | 88.65 | D |
| 2700 | O | LEU | 589 | 7.991 | 46.803 | -9.407 | 1.00 | 88.78 | D |
| 2701 | N | ASP | 590 | 8.396 | 48.735 | -10.483 | 1.00 | 88.97 | D |
| 2702 | CA | ASP | 590 | 8.269 | 48.163 | -11.819 | 1.00 | 89.67 | D |
| 2703 | CB | ASP | 590 | 7.587 | 49.166 | -12.758 | 1.00 | 91.42 | D |
| 2704 | CG | ASP | 590 | 6.149 | 48.786 | -13.080 | 1.00 | 92.34 | D |
| 2705 | OD1 | ASP | 590 | 5.466 | 48.223 | -12.196 | 1.00 | 93.01 | D |
| 2706 | OD2 | ASP | 590 | 5.702 | 49.064 | -14.217 | 1.00 | 93.07 | D |
| 2707 | C | ASP | 590 | 9.641 | 47.794 | -12.365 | 1.00 | 89.21 | D |
| 2708 | O | ASP | 590 | 9.766 | 46.872 | -13.174 | 1.00 | 89.72 | D |
| 2709 | N | ASP | 591 | 10.663 | 48.528 | -11.941 | 1.00 | 88.41 | D |
| 2710 | CA | ASP | 591 | 12.033 | 48.252 | -12.365 | 1.00 | 87.48 | D |
| 2711 | CB | ASP | 591 | 12.925 | 49.470 | -12.157 | 1.00 | 89.21 | D |
| 2712 | CG | ASP | 591 | 12.417 | 50.683 | -12.885 | 1.00 | 90.55 | D |
| 2713 | OD1 | ASP | 591 | 11.775 | 50.505 | -13.943 | 1.00 | 91.32 | D |
| 2714 | OD2 | ASP | 591 | 12.670 | 51.811 | -12.407 | 1.00 | 90.79 | D |
| 2715 | C | ASP | 591 | 12.514 | 47.125 | -11.480 | 1.00 | 85.89 | D |
| 2716 | O | ASP | 591 | 13.210 | 46.213 | -11.928 | 1.00 | 85.48 | D |
| 2717 | N | GLN | 592 | 12.132 | 47.227 | -10.208 | 1.00 | 84.16 | D |
| 2718 | CA | GLN | 592 | 12.450 | 46.231 | -9.204 | 1.00 | 82.32 | D |
| 2719 | CB | GLN | 592 | 11.654 | 46.496 | -7.914 | 1.00 | 81.57 | D |
| 2720 | CG | GLN | 592 | 12.383 | 47.381 | -6.904 | 1.00 | 82.24 | D |
| 2721 | CD | GLN | 592 | 11.593 | 47.633 | -5.622 | 1.00 | 82.59 | D |
| 2722 | OE1 | GLN | 592 | 11.225 | 46.704 | -4.896 | 1.00 | 82.42 | D |
| 2723 | NE2 | GLN | 592 | 11.340 | 48.905 | -5.332 | 1.00 | 83.09 | D |
| 2724 | C | GLN | 592 | 12.046 | 44.891 | -9.790 | 1.00 | 81.43 | D |
| 2725 | O | GLN | 592 | 12.658 | 43.863 | -9.513 | 1.00 | 81.40 | D |
| 2726 | N | MET | 593 | 11.014 | 44.913 | -10.625 | 1.00 | 80.58 | D |
| 2727 | CA | MET | 593 | 10.529 | 43.694 | -11.249 | 1.00 | 79.77 | D |
| 2728 | CB | MET | 593 | 9.027 | 43.756 | -11.480 | 1.00 | 81.53 | D |
| 2729 | CG | MET | 593 | 8.224 | 43.102 | -10.394 | 1.00 | 83.90 | D |
| 2730 | SD | MET | 593 | 6.539 | 42.873 | -10.938 | 1.00 | 86.70 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2731 | CE | MET | 593 | 5.819 | 44.357 | -10.312 | 1.00 | 86.41 | D |
| 2732 | C | MET | 593 | 11.189 | 43.379 | -12.563 | 1.00 | 78.53 | D |
| 2733 | O | MET | 593 | 11.140 | 42.234 | -13.012 | 1.00 | 78.60 | D |
| 2734 | N | THR | 594 | 11.784 | 44.377 | -13.208 | 1.00 | 76.34 | D |
| 2735 | CA | THR | 594 | 12.429 | 44.086 | -14.476 | 1.00 | 74.92 | D |
| 2736 | CB | THR | 594 | 12.608 | 45.340 | -15.353 | 1.00 | 75.23 | D |
| 2737 | OG1 | THR | 594 | 11.648 | 46.336 | -14.979 | 1.00 | 75.76 | D |
| 2738 | CG2 | THR | 594 | 12.388 | 44.976 | -16.822 | 1.00 | 74.71 | D |
| 2739 | C | THR | 594 | 13.798 | 43.504 | -14.174 | 1.00 | 73.12 | D |
| 2740 | O | THR | 594 | 14.198 | 42.481 | -14.741 | 1.00 | 72.69 | D |
| 2741 | N | LEU | 595 | 14.498 | 44.156 | -13.255 | 1.00 | 70.86 | D |
| 2742 | CA | LEU | 595 | 15.822 | 43.721 | -12.869 | 1.00 | 68.87 | D |
| 2743 | CB | LEU | 595 | 16.363 | 44.672 | -11.783 | 1.00 | 67.46 | D |
| 2744 | CG | LEU | 595 | 16.546 | 46.137 | -12.248 | 1.00 | 65.81 | D |
| 2745 | CD1 | LEU | 595 | 17.104 | 46.982 | -11.111 | 1.00 | 65.46 | D |
| 2746 | CD2 | LEU | 595 | 17.484 | 46.212 | -13.454 | 1.00 | 64.25 | D |
| 2747 | C | LEU | 595 | 15.837 | 42.233 | -12.444 | 1.00 | 68.29 | D |
| 2748 | O | LEU | 595 | 16.655 | 41.460 | -12.945 | 1.00 | 67.78 | D |
| 2749 | N | LEU | 596 | 14.919 | 41.815 | -11.571 | 1.00 | 67.24 | D |
| 2750 | CA | LEU | 596 | 14.867 | 40.417 | -11.145 | 1.00 | 66.35 | D |
| 2751 | CB | LEU | 596 | 13.898 | 40.242 | -10.015 | 1.00 | 64.75 | D |
| 2752 | CG | LEU | 596 | 14.549 | 40.758 | -8.763 | 1.00 | 65.03 | D |
| 2753 | CD1 | LEU | 596 | 13.510 | 40.709 | -7.689 | 1.00 | 65.87 | D |
| 2754 | CD2 | LEU | 596 | 15.772 | 39.920 | -8.402 | 1.00 | 63.70 | D |
| 2755 | C | LEU | 596 | 14.450 | 39.468 | -12.223 | 1.00 | 67.95 | D |
| 2756 | O | LEU | 596 | 14.648 | 38.260 | -12.121 | 1.00 | 68.48 | D |
| 2757 | N | GLN | 597 | 13.834 | 40.003 | -13.256 | 1.00 | 70.46 | D |
| 2758 | CA | GLN | 597 | 13.385 | 39.144 | -14.322 | 1.00 | 71.83 | D |
| 2759 | CB | GLN | 597 | 12.092 | 39.680 | -14.893 | 1.00 | 74.64 | D |
| 2760 | CG | GLN | 597 | 10.889 | 39.131 | -14.174 | 1.00 | 77.64 | D |
| 2761 | CD | GLN | 597 | 9.690 | 40.015 | -14.335 | 1.00 | 80.72 | D |
| 2762 | OE1 | GLN | 597 | 9.518 | 40.672 | -15.371 | 1.00 | 81.13 | D |
| 2763 | NE2 | GLN | 597 | 8.839 | 40.040 | -13.312 | 1.00 | 82.34 | D |
| 2764 | C | GLN | 597 | 14.433 | 39.010 | -15.389 | 1.00 | 71.17 | D |
| 2765 | O | GLN | 597 | 14.538 | 37.981 | -16.054 | 1.00 | 70.16 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2766 | N | TYR | 598 | 15.222 | 40.060 | -15.547 | 1.00 | 71.62 | D |
| 2767 | CA | TYR | 598 | 16.282 | 40.034 | -16.530 | 1.00 | 73.19 | D |
| 2768 | CB | TYR | 598 | 16.699 | 41.442 | -16.884 | 1.00 | 75.94 | D |
| 2769 | CG | TYR | 598 | 15.858 | 42.139 | -17.912 | 1.00 | 78.54 | D |
| 2770 | CD1 | TYR | 598 | 14.469 | 42.030 | -17.920 | 1.00 | 79.49 | D |
| 2771 | CE1 | TYR | 598 | 13.705 | 42.761 | -18.828 | 1.00 | 80.77 | D |
| 2772 | CD2 | TYR | 598 | 16.459 | 42.988 | -18.832 | 1.00 | 79.85 | D |
| 2773 | CE2 | TYR | 598 | 15.718 | 43.719 | -19.729 | 1.00 | 80.37 | D |
| 2774 | CZ | TYR | 598 | 14.350 | 43.607 | -19.726 | 1.00 | 80.56 | D |
| 2775 | OH | TYR | 598 | 13.651 | 44.360 | -20.628 | 1.00 | 81.35 | D |
| 2776 | C | TYR | 598 | 17.509 | 39.318 | -15.991 | 1.00 | 72.98 | D |
| 2777 | O | TYR | 598 | 18.318 | 38.818 | -16.761 | 1.00 | 73.46 | D |
| 2778 | N | SER | 599 | 17.653 | 39.246 | -14.671 | 1.00 | 71.89 | D |
| 2779 | CA | SER | 599 | 18.855 | 38.629 | -14.141 | 1.00 | 70.45 | D |
| 2780 | CB | SER | 599 | 19.754 | 39.726 | -13.554 | 1.00 | 70.74 | D |
| 2781 | OG | SER | 599 | 19.116 | 40.418 | -12.498 | 1.00 | 71.65 | D |
| 2782 | C | SER | 599 | 18.773 | 37.475 | -13.152 | 1.00 | 69.27 | D |
| 2783 | O | SER | 599 | 19.778 | 37.130 | -12.543 | 1.00 | 69.11 | D |
| 2784 | N | TRP | 600 | 17.620 | 36.846 | -12.991 | 1.00 | 68.14 | D |
| 2785 | CA | TRP | 600 | 17.553 | 35.758 | -12.021 | 1.00 | 66.50 | D |
| 2786 | CB | TRP | 600 | 16.104 | 35.228 | -11.880 | 1.00 | 64.24 | D |
| 2787 | CG | TRP | 600 | 15.610 | 34.419 | -13.029 | 1.00 | 62.65 | D |
| 2788 | CD2 | TRP | 600 | 15.688 | 32.996 | -13.158 | 1.00 | 62.35 | D |
| 2789 | CE2 | TRP | 600 | 15.247 | 32.668 | -14.461 | 1.00 | 62.55 | D |
| 2790 | CE3 | TRP | 600 | 16.088 | 31.963 | -12.298 | 1.00 | 61.35 | D |
| 2791 | CD1 | TRP | 600 | 15.129 | 34.887 | -14.220 | 1.00 | 62.62 | D |
| 2792 | NE1 | TRP | 600 | 14.913 | 33.842 | -15.086 | 1.00 | 62.81 | D |
| 2793 | CZ2 | TRP | 600 | 15.205 | 31.350 | -14.928 | 1.00 | 62.62 | D |
| 2794 | CZ3 | TRP | 600 | 16.046 | 30.648 | -12.763 | 1.00 | 61.50 | D |
| 2795 | CH2 | TRP | 600 | 15.607 | 30.356 | -14.070 | 1.00 | 62.18 | D |
| 2796 | C | TRP | 600 | 18.545 | 34.604 | -12.305 | 1.00 | 66.75 | D |
| 2797 | O | TRP | 600 | 19.058 | 33.988 | -11.365 | 1.00 | 66.87 | D |
| 2798 | N | MET | 601 | 18.847 | 34.327 | -13.576 | 1.00 | 65.69 | D |
| 2799 | CA | MET | 601 | 19.759 | 33.225 | -13.902 | 1.00 | 64.40 | D |
| 2800 | CB | MET | 601 | 19.572 | 32.783 | -15.357 | 1.00 | 65.00 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2801 | CG | MET | 601 | 20.405 | 31.563 | -15.746 | 1.00 | 64.60 | D |
| 2802 | SD | MET | 601 | 19.781 | 30.024 | -15.098 | 1.00 | 63.03 | D |
| 2803 | CE | MET | 601 | 18.606 | 29.722 | -16.311 | 1.00 | 63.59 | D |
| 2804 | C | MET | 601 | 21.216 | 33.612 | -13.676 | 1.00 | 63.06 | D |
| 2805 | O | MET | 601 | 22.030 | 32.811 | -13.209 | 1.00 | 61.71 | D |
| 2806 | N | SER | 602 | 21.531 | 34.849 | -14.033 | 1.00 | 62.11 | D |
| 2807 | CA | SER | 602 | 22.858 | 35.392 | -13.847 | 1.00 | 60.77 | D |
| 2808 | CB | SER | 602 | 22.879 | 36.826 | -14.363 | 1.00 | 61.16 | D |
| 2809 | OG | SER | 602 | 24.160 | 37.417 | -14.248 | 1.00 | 65.49 | D |
| 2810 | C | SER | 602 | 23.113 | 35.321 | -12.340 | 1.00 | 59.92 | D |
| 2811 1 | O | SER | 602 | 24.177 | 34.898 | -11.900 | 1.00 | 58.97 | D |
| 2812 | N | LEU | 603 | 22.111 | 35.709 | -11.548 | 1.00 | 59.96 | D |
| 2813 | CA | LEU | 603 | 22.215 | 35.657 | -10.088 | 1.00 | 60.03 | D |
| 2814 | CB | LEU | 603 | 20.967 | 36.270 | -9.451 | 1.00 | 59.15 | D |
| 2815 | CG | LEU | 603 | 20.845 | 37.787 | -9.443 | 1.00 | 58.29 | D |
| 2816 | CD1 | LEU | 603 | 19.555 | 38.185 | -8.783 | 1.00 | 59.06 | D |
| 2817 | CD2 | LEU | 603 | 22.015 | 38.374 | -8.704 | 1.00 | 59.29 | D |
| 2818 | C | LEU | 603 | 22.367 | 34.218 | -9.556 | 1.00 | 60.99 | D |
| 2819 | O | LEU | 603 | 23.199 | 33.949 | -8.671 | 1.00 | 59.56 | D |
| 2820 | N | MET | 604 | 21.544 | 33.317 | -10.092 | 1.00 | 61.05 | D |
| 2821 | CA | MET | 604 | 21.535 | 31.914 | -9.701 | 1.00 | 61.42 | D |
| 2822 | CB | MET | 604 | 20.301 | 31.212 | -10.291 | 1.00 | 62.57 | D |
| 2823 | CG | MET | 604 | 18.977 | 31.722 | -9.720 | 1.00 | 63.60 | D |
| 2824 | SD | MET | 604 | 18.923 | 31.632 | -7.902 | 1.00 | 66.37 | D |
| 2825 | CE | MET | 604 | 19.713 | 33.227 | -7.348 | 1.00 | 61.36 | D |
| 2826 | C | MET | 604 | 22.818 | 31.186 | -10.104 | 1.00 | 61.75 | D |
| 2827 | O | MET | 604 | 23.401 | 30.451 | -9.303 | 1.00 | 63.54 | D |
| 2828 | N | ALA | 605 | 23.259 | 31.396 | -11.343 | 1.00 | 61.11 | D |
| 2829 | CA | ALA | 605 | 24.484 | 30.759 | -11.826 | 1.00 | 58.27 | D |
| 2830 | CB | ALA | 605 | 24.652 | 30.980 | -13.328 | 1.00 | 56.44 | D |
| 2831 | C | ALA | 605 | 25.693 | 31.308 | -11.081 | 1.00 | 57.37 | D |
| 2832 | O | ALA | 605 | 26.508 | 30.533 | -10.584 | 1.00 | 58.59 | D |
| 2833 | N | PHE | 606 | 25.816 | 32.632 | -10.982 | 1.00 | 55.71 | D |
| 2834 | CA | PHE | 606 | 26.969 | 33.185 | -10.289 | 1.00 | 54.38 | D |
| 2835 | CB | PHE | 606 | 26.883 | 34.693 | -10.154 | 1.00 | 53.77 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2836 | CG | PHE | 606 | 28.211 | 35.350 | -9.951 | 1.00 | 52.02 | D |
| 2837 | CD1 | PHE | 606 | 29.221 | 35.169 | -10.889 | 1.00 | 50.21 | D |
| 2838 | CD2 | PHE | 606 | 28.464 | 36.127 | -8.818 | 1.00 | 51.90 | D |
| 2839 | CE1 | PHE | 606 | 30.454 | 35.765 | -10.725 | 1.00 | 50.29 | D |
| 2840 | CE2 | PHE | 606 | 29.697 | 36.732 | -8.636 | 1.00 | 50.51 | D |
| 2841 | CZ | PHE | 606 | 30.699 | 36.545 | -9.591 | 1.00 | 51.66 | D |
| 2842 | C | PHE | 606 | 27.121 | 32.578 | -8.907 | 1.00 | 55.10 | D |
| 2843 | O | PHE | 606 | 28.213 | 32.161 | -8.539 | 1.00 | 55.15 | D |
| 2844 | N | ALA | 607 | 26.039 | 32.527 | -8.134 | 1.00 | 55.44 | D |
| 2845 | CA | ALA | 607 | 26.102 | 31.931 | -6.805 | 1.00 | 54.39 | D |
| 2846 | CB | ALA | 607 | 24.742 | 31.971 | -6.134 | 1.00 | 54.87 | D |
| 2847 | C | ALA | 607 | 26.618 | 30.484 | -6.876 | 1.00 | 54.05 | D |
| 2848 | O | ALA | 607 | 27.568 | 30.149 | -6.172 | 1.00 | 53.71 | D |
| 2849 | N | LEU | 608 | 26.015 | 29.628 | -7.702 | 1.00 | 52.77 | D |
| 2850 | CA | LEU | 608 | 26.504 | 28.249 | -7.822 | 1.00 | 54.52 | D |
| 2851 | CB | LEU | 608 | 25.737 | 27.488 | -8.918 | 1.00 | 53.99 | D |
| 2852 | CG | LEU | 608 | 26.095 | 26.110 | -9.530 | 1.00 | 53.37 | D |
| 2853 | CD1 | LEU | 608 | 25.505 | 24.894 | -8.789 | 1.00 | 52.75 | D |
| 2854 | CD2 | LEU | 608 | 25.500 | 26.137 | -10.928 | 1.00 | 51.27 | D |
| 2855 | C | LEU | 608 | 27.997 | 28.321 | -8.167 | 1.00 | 55.73 | D |
| 2856 | O | LEU | 608 | 28.820 | 27.621 | -7.574 | 1.00 | 55.83 | D |
| 2857 | N | GLY | 609 | 28.352 | 29.177 | -9.119 | 1.00 | 56.30 | D |
| 2858 | CA | GLY | 609 | 29.750 | 29.289 | -9.466 | 1.00 | 55.84 | D |
| 2859 | C | GLY | 609 | 30.519 | 29.339 | -8.169 | 1.00 | 56.33 | D |
| 2860 | O | GLY | 609 | 31.360 | 28.493 | -7.891 | 1.00 | 58.34 | D |
| 2861 | N | TRP | 610 | 30.194 | 30.320 | -7.348 | 1.00 | 56.64 | D |
| 2862 | CA | TRP | 610 | 30.837 | 30.510 | -6.063 | 1.00 | 56.47 | D |
| 2863 | CB | TRP | 610 | 30.164 | 31.686 | -5.382 | 1.00 | 54.87 | D |
| 2864 | CG | TRP | 610 | 30.715 | 31.983 | -4.082 | 1.00 | 54.25 | D |
| 2865 | CD2 | TRP | 610 | 31.922 | 32.688 | -3.819 | 1.00 | 54.11 | D |
| 2866 | CE2 | TRP | 610 | 32.062 | 32.760 | -2.413 | 1.00 | 53.58 | D |
| 2867 | CE3 | TRP | 610 | 32.924 | 33.244 | -4.630 | 1.00 | 54.73 | D |
| 2868 | CD1 | TRP | 610 | 30.168 | 31.670 | -2.872 | 1.00 | 53.99 | D |
| 2869 | NE1 | TRP | 610 | 30.971 | 32.139 | -1.861 | 1.00 | 52.96 | D |
| 2870 | CZ2 | TRP | 610 | 33.145 | 33.398 | -1.798 | 1.00 | 53.93 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2871 | CZ3 | TRP | 610 | 34.006 | 33.879 | -4.020 | 1.00 | 55.04 | D |
| 2872 | CH2 | TRP | 610 | 34.109 | 33.941 | -2.614 | 1.00 | 54.37 | D |
| 2873 | C | TRP | 610 | 30.861 | 29.278 | -5.126 | 1.00 | 56.15 | D |
| 2874 | O | TRP | 610 | 31.909 | 28.875 | -4.650 | 1.00 | 55.81 | D |
| 2875 | N | ARG | 611 | 29.709 | 28.696 | -4.850 | 1.00 | 57.57 | D |
| 2876 | CA | ARG | 611 | 29.642 | 27.532 | -3.979 | 1.00 | 60.30 | D |
| 2877 | CB | ARG | 611 | 28.205 | 27.011 | -3.916 | 1.00 | 60.79 | D |
| 2878 | CG | ARG | 611 | 27.260 | 27.895 | -3.152 | 1.00 | 61.01 | D |
| 2879 | CD | ARG | 611 | 26.050 | 27.105 | -2.743 | 1.00 | 62.23 | D |
| 2880 | NE | ARG | 611 | 25.221 | 26.733 | -3.885 | 1.00 | 65.70 | D |
| 2881 | CZ | ARG | 611 | 24.575 | 27.609 | -4.658 | 1.00 | 67.49 | D |
| 2882 | NH1 | ARG | 611 | 24.672 | 28.915 | -4.411 | 1.00 | 67.58 | D |
| 2883 | NH2 | ARG | 611 | 23.815 | 27.175 | -5.662 | 1.00 | 67.11 | D |
| 2884 | C | ARG | 611 | 30.557 | 26.396 | -4.437 | 1.00 | 62.21 | D |
| 2885 | O | ARG | 611 | 31.111 | 25.658 | -3.619 | 1.00 | 62.76 | D |
| 2886 | N | SER | 612 | 30.700 | 26.267 | -5.752 | 1.00 | 63.60 | D |
| 2887 | CA | SER | 612 | 31.515 | 25.235 | -6.376 | 1.00 | 64.24 | D |
| 2888 | CB | SER | 612 | 31.249 | 25.222 | -7.871 | 1.00 | 63.20 | D |
| 2889 | OG | SER | 612 | 29.893 | 24.903 | -8.109 | 1.00 | 64.79 | D |
| 2890 | C | SER | 612 | 32.992 | 25.440 | -6.165 | 1.00 | 65.94 | D |
| 2891 | O | SER | 612 | 33.743 | 24.518 | -5.849 | 1.00 | 66.25 | D |
| 2892 | N | TYR | 613 | 33.395 | 26.674 | -6.379 | 1.00 | 67.61 | D |
| 2893 | CA | TYR | 613 | 34.766 | 27.065 | -6.253 | 1.00 | 70.25 | D |
| 2894 | CB | TYR | 613 | 34.877 | 28.493 | -6.765 | 1.00 | 68.40 | D |
| 2895 | CG | TYR | 613 | 35.904 | 29.345 | -6.083 | 1.00 | 66.72 | D |
| 2896 | CD1 | TYR | 613 | 37.257 | 29.017 | -6.129 | 1.00 | 66.00 | D |
| 2897 | CE1 | TYR | 613 | 38.216 | 29.854 | -5.587 | 1.00 | 65.63 | D |
| 2898 | CD2 | TYR | 613 | 35.529 | 30.534 | -5.465 | 1.00 | 65.87 | D |
| 2899 | CE2 | TYR | 613 | 36.478 | 31.385 | -4.920 | 1.00 | 66.64 | D |
| 2900 | CZ | TYR | 613 | 37.824 | 31.042 | -4.986 | 1.00 | 66.27 | D |
| 2901 | OH | TYR | 613 | 38.779 | 31.900 | -4.487 | 1.00 | 66.46 | D |
| 2902 | C | TYR | 613 | 35.274 | 26.960 | -4.833 | 1.00 | 73.21 | D |
| 2903 | O | TYR | 613 | 36.445 | 26.684 | -4.603 | 1.00 | 74.48 | D |
| 2904 | N | ARG | 614 | 34.403 | 27.146 | -3.862 | 1.00 | 76.37 | D |
| 2905 | CA | ARG | 614 | 34.881 | 27.117 | -2.496 | 1.00 | 80.08 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2906 | CB | ARG | 614 | 34.064 | 28.077 | -1.675 | 1.00 | 81.71 | D |
| 2907 | CG | ARG | 614 | 34.106 | 29.440 | -2.230 | 1.00 | 84.04 | D |
| 2908 | CD | ARG | 614 | 34.100 | 30.368 | -1.095 | 1.00 | 85.85 | D |
| 2909 | NE | ARG | 614 | 35.334 | 31.126 | -1.078 | 1.00 | 88.32 | D |
| 2910 | CZ | ARG | 614 | 35.980 | 31.443 | 0.033 | 1.00 | 90.04 | D |
| 2911 | NH1 | ARG | 614 | 35.501 | 31.042 | 1.207 | 1.00 | 90.01 | D |
| 2912 | NH2 | ARG | 614 | 37.086 | 32.173 | -0.031 | 1.00 | 90.37 | D |
| 2913 | C | ARG | 614 | 34.884 | 25.786 | -1.798 | 1.00 | 82.06 | D |
| 2914 | O | ARG | 614 | 35.673 | 25.558 | -0.881 | 1.00 | 81.66 | D |
| 2915 | N | GLN | 615 | 33.996 | 24.919 | -2.257 | 1.00 | 84.29 | D |
| 2916 | CA | GLN | 615 | 33.783 | 23.607 | -1.683 | 1.00 | 86.72 | D |
| 2917 | CB | GLN | 615 | 32.291 | 23.295 | -1.776 | 1.00 | 87.00 | D |
| 2918 | CG | GLN | 615 | 31.757 | 22.321 | -0.758 | 1.00 | 87.90 | D |
| 2919 | CD | GLN | 615 | 30.383 | 21.805 | -1.139 | 1.00 | 88.85 | D |
| 2920 | OE1 | GLN | 615 | 29.469 | 22.583 | -1.439 | 1.00 | 89.58 | D |
| 2921 | NE2 | GLN | 615 | 30.230 | 20.486 | -1.129 | 1.00 | 88.70 | D |
| 2922 | C | GLN | 615 | 34.570 | 22.480 | -2.330 | 1.00 | 88.12 | D |
| 2923 | O | GLN | 615 | 34.636 | 21.376 | -1.793 | 1.00 | 88.63 | D |
| 2924 | N | SER | 616 | 35.154 | 22.743 | -3.493 | 1.00 | 89.98 | D |
| 2925 | CA | SER | 616 | 35.897 | 21.714 | -4.201 | 1.00 | 92.24 | D |
| 2926 | CB | SER | 616 | 34.935 | 20.628 | -4.707 | 1.00 | 91.52 | D |
| 2927 | OG | SER | 616 | 33.666 | 21.166 | -5.037 | 1.00 | 90.46 | D |
| 2928 | C | SER | 616 | 36.699 | 22.297 | -5.351 | 1.00 | 94.27 | D |
| 2929 | O | SER | 616 | 36.747 | 21.737 | -6.454 | 1.00 | 94.33 | D |
| 2930 | N | SER | 617 | 37.288 | 23.457 | -5.076 | 1.00 | 96.24 | D |
| 2931 | CA | SER | 617 | 38.160 | 24.176 | -5.995 | 1.00 | 98.57 | D |
| 2932 | CB | SER | 617 | 39.554 | 23.553 | -5.872 | 1.00 | 99.03 | D |
| 2933 | OG | SER | 617 | 39.571 | 22.606 | -4.807 | 1.00 | 99.55 | D |
| 2934 | C | SER | 617 | 37.755 | 24.286 | -7.482 | 1.00 | 99.70 | D |
| 2935 | O | SER | 617 | 38.627 | 24.410 | -8.349 | 1.00 | 100.43 | D |
| 2936 | N | ALA | 618 | 36.451 | 24.269 | -7.761 | 1.00 | 99.99 | D |
| 2937 | CA | ALA | 618 | 35.910 | 24.355 | -9.129 | 1.00 | 100.55 | D |
| 2938 | CB | ALA | 618 | 36.607 | 25.472 | -9.927 | 1.00 | 100.28 | D |
| 2939 | C | ALA | 618 | 36.023 | 23.019 | -9.877 | 1.00 | 101.11 | D |
| 2940 | O | ALA | 618 | 36.252 | 22.988 | -11.095 | 1.00 | 102.13 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2941 | N | ASN | 619 | 35.846 | 21.925 | -9.140 | 1.00 | 100.05 | D |
| 2942 | CA | ASN | 619 | 35.920 | 20.572 | -9.686 | 1.00 | 98.66 | D |
| 2943 | CB | ASN | 619 | 36.717 | 19.698 | -8.729 | 1.00 | 98.89 | D |
| 2944 | CG | ASN | 619 | 38.173 | 20.061 | -8.710 | 1.00 | 99.76 | D |
| 2945 | OD1 | ASN | 619 | 38.989 | 19.409 | -9.362 | 1.00 | 100.38 | D |
| 2946 | ND2 | ASN | 619 | 38.513 | 21.118 | -7.984 | 1.00 | 100.19 | D |
| 2947 | C | ASN | 619 | 34.537 | 19.956 | -9.898 | 1.00 | 97.79 | D |
| 2948 | O | ASN | 619 | 34.263 | 19.341 | -10.933 | 1.00 | 97.87 | D |
| 2949 | N | LEU | 620 | 33.689 | 20.138 | -8.889 | 1.00 | 95.84 | D |
| 2950 | CA | LEU | 620 | 32.311 | 19.639 | -8.847 | 1.00 | 93.00 | D |
| 2951 | CB | LEU | 620 | 32.115 | 18.877 | -7.520 | 1.00 | 92.86 | D |
| 2952 | CG | LEU | 620 | 32.410 | 17.366 | -7.399 | 1.00 | 93.21 | D |
| 2953 | CD1 | LEU | 620 | 33.616 | 16.984 | -8.224 | 1.00 | 93.46 | D |
| 2954 | CD2 | LEU | 620 | 32.620 | 16.984 | -5.936 | 1.00 | 93.04 | D |
| 2955 | C | LEU | 620 | 31.305 | 20.815 | -8.945 | 1.00 | 90.83 | D |
| 2956 | O | LEU | 620 | 31.656 | 21.951 | -8.634 | 1.00 | 90.07 | D |
| 2957 | N | LEU | 621 | 30.080 | 20.559 | -9.417 | 1.00 | 88.70 | D |
| 2958 | CA | LEU | 621 | 29.042 | 21.602 | -9.478 | 1.00 | 86.58 | D |
| 2959 | CB | LEU | 621 | 28.116 | 21.448 | -10.702 | 1.00 | 85.41 | D |
| 2960 | CG | LEU | 621 | 28.548 | 22.141 | -12.005 | 1.00 | 84.56 | D |
| 2961 | CD1 | LEU | 621 | 27.426 | 22.091 | -13.034 | 1.00 | 83.62 | D |
| 2962 | CD2 | LEU | 621 | 28.920 | 23.591 | -11.705 | 1.00 | 83.64 | D |
| 2963 | C | LEU | 621 | 28.257 | 21.351 | -8.201 | 1.00 | 85.65 | D |
| 2964 | O | LEU | 621 | 27.712 | 20.267 | -8.015 | 1.00 | 84.66 | D |
| 2965 | N | CYS | 622 | 28.195 | 22.352 | -7.329 | 1.00 | 84.99 | D |
| 2966 | CA | CYS | 622 | 27.547 | 22.173 | -6.033 | 1.00 | 84.82 | D |
| 2967 | CB | CYS | 622 | 28.555 | 22.513 | -4.943 | 1.00 | 84.44 | D |
| 2968 | SG | CYS | 622 | 30.165 | 21.733 | -5.213 | 1.00 | 84.00 | D |
| 2969 | C | CYS | 622 | 26.256 | 22.923 | -5.775 | 1.00 | 85.04 | D |
| 2970 | O | CYS | 622 | 26.237 | 23.915 | -5.046 | 1.00 | 85.22 | D |
| 2971 | N | PHE | 623 | 25.166 | 22.418 | -6.321 | 1.00 | 85.24 | D |
| 2972 | CA | PHE | 623 | 23.879 | 23.058 | -6.161 | 1.00 | 85.95 | D |
| 2973 | CB | PHE | 623 | 22.842 | 22.244 | -6.917 | 1.00 | 84.99 | D |
| 2974 | CG | PHE | 623 | 23.133 | 22.232 | -8.381 | 1.00 | 84.67 | D |
| 2975 | CD1 | PHE | 623 | 24.170 | 21.456 | -8.896 | 1.00 | 84.97 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 2976 | CD2 | PHE | 623 | 22.378 | 22.995 | -9.256 | 1.00 | 84.22 | D |
| 2977 | CE1 | PHE | 623 | 24.531 | 21.545 | -10.242 | 1.00 | 84.00 | D |
| 2978 | CE2 | PHE | 623 | 22.733 | 23.093 | -10.600 | 1.00 | 83.93 | D |
| 2979 | CZ | PHE | 623 | 23.779 | 22.324 | -11.103 | 1.00 | 84.03 | D |
| 2980 | C | PHE | 623 | 23.451 | 23.362 | -4.722 | 1.00 | 87.69 | D |
| 2981 | O | PHE | 623 | 23.098 | 24.506 | -4.424 | 1.00 | 87.48 | D |
| 2982 | N | ALA | 624 | 23.492 | 22.364 | -3.844 | 1.00 | 89.72 | D |
| 2983 | CA | ALA | 624 | 23.135 | 22.522 | -2.422 | 1.00 | 91.56 | D |
| 2984 | CB | ALA | 624 | 21.650 | 22.219 | -2.214 | 1.00 | 91.67 | D |
| 2985 | C | ALA | 624 | 23.989 | 21.484 | -1.693 | 1.00 | 92.97 | D |
| 2986 | O | ALA | 624 | 23.729 | 20.308 | -1.854 | 1.00 | 93.10 | D |
| 2987 | N | PRO | 625 | 25.007 | 21.894 | -0.893 | 1.00 | 94.78 | D |
| 2988 | CD | PRO | 625 | 25.468 | 23.245 | -0.512 | 1.00 | 95.11 | D |
| 2989 | CA | PRO | 625 | 25.824 | 20.873 | -0.211 | 1.00 | 95.23 | D |
| 2990 | CB | PRO | 625 | 26.256 | 21.600 | 1.061 | 1.00 | 95.14 | D |
| 2991 | CG | PRO | 625 | 26.614 | 22.958 | 0.500 | 1.00 | 94.67 | D |
| 2992 | C | PRO | 625 | 25.285 | 19.441 | 0.026 | 1.00 | 94.97 | D |
| 2993 | O | PRO | 625 | 26.069 | 18.542 | 0.330 | 1.00 | 95.36 | D |
| 2994 | N | ASP | 626 | 23.970 | 19.242 | -0.116 | 1.00 | 94.20 | D |
| 2995 | CA | ASP | 626 | 23.327 | 17.927 | -0.008 | 1.00 | 93.55 | D |
| 2996 | CB | ASP | 626 | 21.992 | 18.026 | 0.797 | 1.00 | 95.17 | D |
| 2997 | CG | ASP | 626 | 20.697 | 17.895 | -0.073 | 1.00 | 96.51 | D |
| 2998 | OD1 | ASP | 626 | 20.722 | 18.077 | -1.311 | 1.00 | 96.93 | D |
| 2999 | OD2 | ASP | 626 | 19.616 | 17.628 | 0.506 | 1.00 | 97.12 | D |
| 3000 | C | ASP | 626 | 23.083 | 17.412 | -1.444 | 1.00 | 92.62 | D |
| 3001 | O | ASP | 626 | 22.289 | 16.493 | -1.655 | 1.00 | 92.92 | D |
| 3002 | N | LEU | 627 | 23.749 | 18.013 | -2.436 | 1.00 | 91.17 | D |
| 3003 | CA | LEU | 627 | 23.584 | 17.593 | -3.840 | 1.00 | 89.74 | D |
| 3004 | CB | LEU | 627 | 22.161 | 17.873 | -4.367 | 1.00 | 88.17 | D |
| 3005 | CG | LEU | 627 | 21.783 | 17.274 | -5.744 | 1.00 | 87.34 | D |
| 3006 | CD1 | LEU | 627 | 20.342 | 17.608 | -6.076 | 1.00 | 87.00 | D |
| 3007 | CD2 | LEU | 627 | 22.674 | 17.807 | -6.855 | 1.00 | 87.14 | D |
| 3008 | C | LEU | 627 | 24.587 | 18.216 | -4.805 | 1.00 | 89.11 | D |
| 3009 | O | LEU | 627 | 24.358 | 19.297 | -5.368 | 1.00 | 88.73 | D |
| 3010 | N | ILE | 628 | 25.674 | 17.476 | -5.005 | 1.00 | 88.58 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3011 | CA | ILE | 628 | 26.777 | 17.834 | -5.882 | 1.00 | 87.30 | D |
| 3012 | CB | ILE | 628 | 28.120 | 17.568 | -5.204 | 1.00 | 86.84 | D |
| 3013 | CG2 | ILE | 628 | 29.256 | 17.957 | -6.120 | 1.00 | 86.63 | D |
| 3014 | CG1 | ILE | 628 | 28.175 | 18.310 | -3.873 | 1.00 | 86.64 | D |
| 3015 | CD1 | ILE | 628 | 29.556 | 18.369 | -3.261 | 1.00 | 86.21 | D |
| 3016 | C | ILE | 628 | 26.743 | 16.966 | -7.128 | 1.00 | 86.45 | D |
| 3017 | O | ILE | 628 | 26.406 | 15.783 | -7.074 | 1.00 | 85.46 | D |
| 3018 | N | ILE | 629 | 27.087 | 17.543 | -8.264 | 1.00 | 86.29 | D |
| 3019 | CA | ILE | 629 | 27.103 | 16.735 | -9.443 | 1.00 | 86.10 | D |
| 3020 | CB | ILE | 629 | 26.472 | 17.449 | -10.621 | 1.00 | 84.49 | D |
| 3021 | CG2 | ILE | 629 | 27.090 | 16.998 | -11.939 | 1.00 | 82.56 | D |
| 3022 | CG1 | ILE | 629 | 24.978 | 17.158 | -10.572 | 1.00 | 83.17 | D |
| 3023 | CD1 | ILE | 629 | 24.175 | 18.036 | -11.424 | 1.00 | 84.20 | D |
| 3024 | C | ILE | 629 | 28.527 | 16.352 | -9.684 | 1.00 | 87.80 | D |
| 3025 | O | ILE | 629 | 29.310 | 17.095 | -10.275 | 1.00 | 87.90 | D |
| 3026 | N | ASN | 630 | 28.840 | 15.190 | -9.129 | 1.00 | 90.07 | D |
| 3027 | CA | ASN | 630 | 30.127 | 14.525 | -9.210 | 1.00 | 91.27 | D |
| 3028 | CB | ASN | 630 | 30.121 | 13.359 | -8.248 | 1.00 | 90.47 | D |
| 3029 | CG | ASN | 630 | 28.829 | 12.602 | -8.339 | 1.00 | 90.42 | D |
| 3030 | OD1 | ASN | 630 | 28.088 | 12.776 | -9.314 | 1.00 | 90.62 | D |
| 3031 | ND2 | ASN | 630 | 28.536 | 11.777 | -7.348 | 1.00 | 89.63 | D |
| 3032 | C | ASN | 630 | 30.222 | 13.976 | -10.627 | 1.00 | 92.33 | D |
| 3033 | O | ASN | 630 | 29.381 | 14.260 | -11.495 | 1.00 | 91.85 | D |
| 3034 | N | GLU | 631 | 31.228 | 13.141 | -10.814 | 1.00 | 94.20 | D |
| 3035 | CA | GLU | 631 | 31.525 | 12.554 | -12.090 | 1.00 | 95.53 | D |
| 3036 | CB | GLU | 631 | 32.843 | 11.896 | -12.061 | 1.00 | 97.16 | D |
| 3037 | CG | GLU | 631 | 33.465 | 11.820 | -13.378 | 1.00 | 99.89 | D |
| 3038 | CD | GLU | 631 | 34.587 | 10.855 | -13.290 | 1.00 | 101.71 | D |
| 3039 | OE1 | GLU | 631 | 35.675 | 11.248 | -12.812 | 1.00 | 102.39 | D |
| 3040 | OE2 | GLU | 631 | 34.359 | 9.683 | -13.654 | 1.00 | 101.65 | D |
| 3041 | C | GLU | 631 | 30.538 | 11.546 | -12.304 | 1.00 | 95.35 | D |
| 3042 | O | GLU | 631 | 29.768 | 11.682 | -13.201 | 1.00 | 95.58 | D |
| 3043 | N | GLN | 632 | 30.609 | 10.478 | -11.553 | 1.00 | 95.08 | D |
| 3044 | CA | GLN | 632 | 29.542 | 9.588 | -11.723 | 1.00 | 95.45 | D |
| 3045 | CB | GLN | 632 | 29.154 | 9.224 | -10.365 | 1.00 | 97.31 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3046 | CG | GLN | 632 | 28.446 | 8.037 | -10.306 | 1.00 | 100.89 | D |
| 3047 | CD | GLN | 632 | 27.588 | 8.184 | -9.158 | 1.00 | 103.01 | D |
| 3048 | OE1 | GLN | 632 | 28.060 | 8.523 | -8.072 | 1.00 | 103.65 | D |
| 3049 | NE2 | GLN | 632 | 26.299 | 7.994 | -9.362 | 1.00 | 103.97 | D |
| 3050 | C | GLN | 632 | 28.442 | 10.450 | -12.433 | 1.00 | 94.61 | D |
| 3051 | O | GLN | 632 | 28.445 | 10.522 | -13.637 | 1.00 | 95.69 | D |
| 3052 | N | ARG | 633 | 27.594 | 11.210 | -11.729 | 1.00 | 92.32 | D |
| 3053 | CA | ARG | 633 | 26.507 | 11.993 | -12.391 | 1.00 | 89.05 | D |
| 3054 | CB | ARG | 633 | 25.913 | 12.967 | -11.405 | 1.00 | 87.98 | D |
| 3055 | CG | ARG | 633 | 25.233 | 12.225 | -10.339 | 1.00 | 86.09 | D |
| 3056 | CD | ARG | 633 | 24.990 | 13.092 | -9.180 | 1.00 | 84.99 | D |
| 3057 | NE | ARG | 633 | 24.176 | 12.385 | -8.209 | 1.00 | 83.88 | D |
| 3058 | CZ | ARG | 633 | 23.971 | 12.830 | -6.983 | 1.00 | 82.20 | D |
| 3059 | NH1 | ARG | 633 | 24.530 | 13.968 | -6.618 | 1.00 | 81.44 | D |
| 3060 | NH2 | ARG | 633 | 23.217 | 12.146 | -6.134 | 1.00 | 82.05 | D |
| 3061 | C | ARG | 633 | 26.688 | 12.732 | -13.706 | 1.00 | 88.02 | D |
| 3062 | O | ARG | 633 | 25.868 | 12.640 | -14.616 | 1.00 | 87.50 | D |
| 3063 | N | MET | 634 | 27.749 | 13.509 | -13.764 | 1.00 | 86.51 | D |
| 3064 | CA | MET | 634 | 28.118 | 14.278 | -14.926 | 1.00 | 85.73 | D |
| 3065 | CB | MET | 634 | 29.471 | 14.854 | -14.672 | 1.00 | 85.01 | D |
| 3066 | CG | MET | 634 | 29.759 | 16.030 | -15.488 | 1.00 | 85.81 | D |
| 3067 | SD | MET | 634 | 29.367 | 17.489 | -14.539 | 1.00 | 85.72 | D |
| 3068 | CE | MET | 634 | 28.927 | 18.527 | -15.819 | 1.00 | 85.08 | D |
| 3069 | C | MET | 634 | 28.259 | 13.421 | -16.158 | 1.00 | 85.55 | D |
| 3070 | O | MET | 634 | 28.533 | 13.913 | -17.241 | 1.00 | 84.84 | D |
| 3071 | N | THR | 635 | 28.101 | 12.127 | -16.003 | 1.00 | 86.00 | D |
| 3072 | CA | THR | 635 | 28.322 | 11.281 | -17.142 | 1.00 | 86.49 | D |
| 3073 | CB | THR | 635 | 28.999 | 10.036 | -16.635 | 1.00 | 86.24 | D |
| 3074 | OG1 | THR | 635 | 29.025 | 10.105 | -15.217 | 1.00 | 88.35 | D |
| 3075 | CG2 | THR | 635 | 30.436 | 10.000 | -17.055 | 1.00 | 86.02 | D |
| 3076 | C | THR | 635 | 27.029 | 10.987 | -17.878 | 1.00 | 86.60 | D |
| 3077 | O | THR | 635 | 27.055 | 10.407 | -18.967 | 1.00 | 86.62 | D |
| 3078 | N | LEU | 636 | 25.935 | 11.501 | -17.293 | 1.00 | 86.56 | D |
| 3079 | CA | LEU | 636 | 24.538 | 11.315 | -17.714 | 1.00 | 86.65 | D |
| 3080 | CB | LEU | 636 | 23.580 | 11.669 | -16.578 | 1.00 | 86.61 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3081 | CG | LEU | 636 | 23.837 | 10.975 | -15.244 | 1.00 | 87.09 | D |
| 3082 | CD1 | LEU | 636 | 23.459 | 11.935 | -14.143 | 1.00 | 87.05 | D |
| 3083 | CD2 | LEU | 636 | 23.067 | 9.690 | -15.100 | 1.00 | 86.70 | D |
| 3084 | C | LEU | 636 | 23.950 | 11.908 | -18.952 | 1.00 | 87.19 | D |
| 3085 | O | LEU | 636 | 23.710 | 13.106 | -19.057 | 1.00 | 86.40 | D |
| 3086 | N | PRO | 637 | 23.473 | 11.013 | -19.798 | 1.00 | 88.07 | D |
| 3087 | CD | PRO | 637 | 22.642 | 9.979 | -19.135 | 1.00 | 88.43 | D |
| 3088 | CA | PRO | 637 | 22.888 | 11.410 | -21.067 | 1.00 | 89.09 | D |
| 3089 | CB | PRO | 637 | 21.454 | 10.914 | -20.970 | 1.00 | 89.17 | D |
| 3090 | CG | PRO | 637 | 21.539 | 9.716 | -20.122 | 1.00 | 88.86 | D |
| 3091 | C | PRO | 637 | 22.920 | 12.871 | -21.475 | 1.00 | 89.60 | D |
| 3092 | O | PRO | 637 | 23.318 | 13.197 | -22.575 | 1.00 | 90.04 | D |
| 3093 | N | CYS | 638 | 22.562 | 13.798 | -20.631 | 1.00 | 89.77 | D |
| 3094 | CA | CYS | 638 | 22.496 | 15.085 | -21.249 | 1.00 | 90.59 | D |
| 3095 | CB | CYS | 638 | 21.027 | 15.318 | -21.522 | 1.00 | 90.92 | D |
| 3096 | SG | CYS | 638 | 20.576 | 15.454 | -23.222 | 1.00 | 91.46 | D |
| 3097 | C | CYS | 638 | 23.031 | 16.160 | -20.395 | 1.00 | 90.69 | D |
| 3098 | O | CYS | 638 | 22.797 | 17.335 | -20.639 | 1.00 | 91.18 | D |
| 3099 | N | MET | 639 | 23.819 | 15.758 | -19.429 | 1.00 | 90.76 | D |
| 3100 | CA | MET | 639 | 24.239 | 16.699 | -18.451 | 1.00 | 90.74 | D |
| 3101 | CB | MET | 639 | 24.472 | 15.923 | -17.159 | 1.00 | 91.74 | D |
| 3102 | CG | MET | 639 | 23.666 | 16.490 | -16.026 | 1.00 | 92.82 | D |
| 3103 | SD | MET | 639 | 22.527 | 15.549 | -15.057 | 1.00 | 96.14 | D |
| 3104 | CE | MET | 639 | 23.011 | 16.324 | -13.615 | 1.00 | 95.51 | D |
| 3105 | C | MET | 639 | 25.365 | 17.641 | -18.712 | 1.00 | 90.77 | D |
| 3106 | O | MET | 639 | 25.222 | 18.866 | -18.588 | 1.00 | 91.47 | D |
| 3107 | N | TYR | 640 | 26.493 | 17.061 | -19.070 | 1.00 | 90.29 | D |
| 3108 | CA | TYR | 640 | 27.665 | 17.840 | -19.283 | 1.00 | 89.21 | D |
| 3109 | CB | TYR | 640 | 28.749 | 16.976 | -19.896 | 1.00 | 85.42 | D |
| 3110 | CG | TYR | 640 | 30.015 | 17.737 | -19.953 | 1.00 | 81.76 | D |
| 3111 | CD1 | TYR | 640 | 30.667 | 18.086 | -18.782 | 1.00 | 80.23 | D |
| 3112 | CE1 | TYR | 640 | 31.734 | 18.942 | -18.800 | 1.00 | 79.42 | D |
| 3113 | CD2 | TYR | 640 | 30.474 | 18.254 | -21.153 | 1.00 | 80.19 | D |
| 3114 | CE2 | TYR | 640 | 31.544 | 19.112 | -21.184 | 1.00 | 78.89 | D |
| 3115 | CZ | TYR | 640 | 32.167 | 19.457 | -20.002 | 1.00 | 78.88 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3116 | OH | TYR | 640 | 33.227 | 20.326 | -20.008 | 1.00 | 78.74 | D |
| 3117 | C | TYR | 640 | 27.324 | 18.970 | -20.202 | 1.00 | 90.99 | D |
| 3118 | O | TYR | 640 | 27.515 | 20.150 | -19.900 | 1.00 | 91.28 | D |
| 3119 | N | ASP | 641 | 26.739 | 18.605 | -21.313 | 1.00 | 92.71 | D |
| 3120 | CA | ASP | 641 | 26.447 | 19.619 | -22.257 | 1.00 | 93.94 | D |
| 3121 | CB | ASP | 641 | 25.557 | 19.076 | -23.309 | 1.00 | 97.46 | D |
| 3122 | CG | ASP | 641 | 25.977 | 19.569 | -24.610 | 1.00 | 100.36 | D |
| 3123 | OD1 | ASP | 641 | 26.403 | 20.747 | -24.622 | 1.00 | 102.08 | D |
| 3124 | OD2 | ASP | 641 | 25.917 | 18.810 | -25.589 | 1.00 | 101.89 | D |
| 3125 | C | ASP | 641 | 25.902 | 20.934 | -21.775 | 1.00 | 92.79 | D |
| 3126 | O | ASP | 641 | 26.134 | 21.979 | -22.380 | 1.00 | 92.04 | D |
| 3127 | N | GLN | 642 | 25.203 | 20.886 | -20.665 | 1.00 | 92.46 | D |
| 3128 | CA | GLN | 642 | 24.613 | 22.071 | -20.127 | 1.00 | 92.28 | D |
| 3129 | CB | GLN | 642 | 23.176 | 21.768 | -19.850 | 1.00 | 93.82 | D |
| 3130 | CG | GLN | 642 | 22.514 | 21.281 | -21.065 | 1.00 | 97.01 | D |
| 3131 | CD | GLN | 642 | 21.906 | 22.436 | -21.770 | 1.00 | 99.33 | D |
| 3132 | OE1 | GLN | 642 | 21.991 | 23.566 | -21.291 | 1.00 | 100.64 | D |
| 3133 | NE2 | GLN | 642 | 21.251 | 22.175 | -22.891 | 1.00 | 100.09 | D |
| 3134 | C | GLN | 642 | 25.269 | 22.435 | -18.842 | 1.00 | 90.53 | D |
| 3135 | O | GLN | 642 | 25.014 | 23.495 | -18.306 | 1.00 | 90.55 | D |
| 3136 | N | CYS | 643 | 26.110 | 21.576 | -18.307 | 1.00 | 88.94 | D |
| 3137 | CA | CYS | 643 | 26.657 | 21.944 | -17.011 | 1.00 | 88.17 | D |
| 3138 | CB | CYS | 643 | 26.827 | 20.735 | -16.132 | 1.00 | 88.74 | D |
| 3139 | SG | CYS | 643 | 25.287 | 20.237 | -15.425 | 1.00 | 91.87 | D |
| 3140 | C | CYS | 643 | 27.954 | 22.519 | -17.224 | 1.00 | 86.91 | D |
| 3141 | O | CYS | 643 | 28.692 | 22.913 | -16.323 | 1.00 | 86.37 | D |
| 3142 | N | LYS | 644 | 28.215 | 22.657 | -18.472 | 1.00 | 85.71 | D |
| 3143 | CA | LYS | 644 | 29.502 | 23.050 | -18.703 | 1.00 | 84.89 | D |
| 3144 | CB | LYS | 644 | 29.854 | 22.517 | -20.028 | 1.00 | 86.51 | D |
| 3145 | CG | LYS | 644 | 29.634 | 23.541 | -21.026 | 1.00 | 89.18 | D |
| 3146 | CD | LYS | 644 | 30.440 | 23.203 | -22.199 | 1.00 | 91.39 | D |
| 3147 | CE | LYS | 644 | 29.585 | 22.496 | -23.213 | 1.00 | 92.90 | D |
| 3148 | NZ | LYS | 644 | 30.446 | 21.988 | -24.311 | 1.00 | 93.59 | D |
| 3149 | C | LYS | 644 | 29.645 | 24.519 | -18.613 | 1.00 | 83.42 | D |
| 3150 | O | LYS | 644 | 30.699 | 25.003 | -18.233 | 1.00 | 82.60 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3151 | N | HIS | 645 | 28.588 | 25.246 | -18.936 | 1.00 | 81.98 | D |
| 3152 | CA | HIS | 645 | 28.753 | 26.666 | -18.883 | 1.00 | 80.20 | D |
| 3153 | CB | HIS | 645 | 27.705 | 27.352 | -19.712 | 1.00 | 80.02 | D |
| 3154 | CG | HIS | 645 | 27.935 | 27.162 | -21.171 | 1.00 | 79.83 | D |
| 3155 | CD2 | HIS | 645 | 28.877 | 27.635 | -21.990 | 1.00 | 79.20 | D |
| 3156 | ND1 | HIS | 645 | 27.103 | 26.362 | -21.947 | 1.00 | 79.63 | D |
| 3157 | CE1 | HIS | 645 | 27.551 | 26.380 | -23.189 | 1.00 | 79.43 | D |
| 3158 | NE2 | HIS | 645 | 28.624 | 27.145 | -23.247 | 1.00 | 79.34 | D |
| 3159 | C | HIS | 645 | 28.752 | 27.168 | -17.486 | 1.00 | 78.77 | D |
| 3160 | O | HIS | 645 | 29.191 | 28.277 | -17.229 | 1.00 | 77.58 | D |
| 3161 | N | MET | 646 | 28.283 | 26.332 | -16.578 | 1.00 | 77.65 | D |
| 3162 | CA | MET | 646 | 28.278 | 26.699 | -15.176 | 1.00 | 76.48 | D |
| 3163 | CB | MET | 646 | 27.324 | 25.832 | -14.421 | 1.00 | 78.58 | D |
| 3164 | CG | MET | 646 | 25.939 | 26.304 | -14.567 | 1.00 | 80.03 | D |
| 3165 | SD | MET | 646 | 24.817 | 25.168 | -13.806 | 1.00 | 84.57 | D |
| 3166 | CE | MET | 646 | 24.055 | 24.523 | -15.228 | 1.00 | 84.51 | D |
| 3167 | C | MET | 646 | 29.646 | 26.433 | -14.645 | 1.00 | 74.60 | D |
| 3168 | O | MET | 646 | 30.246 | 27.246 | -13.946 | 1.00 | 74.05 | D |
| 3169 | N | LEU | 647 | 30.099 | 25.230 | -14.946 | 1.00 | 72.75 | D |
| 3170 | CA | LEU | 647 | 31.411 | 24.790 | -14.567 | 1.00 | 71.39 | D |
| 3171 | CB | LEU | 647 | 31.784 | 23.611 | -15.428 | 1.00 | 71.41 | D |
| 3172 | CG | LEU | 647 | 31.425 | 22.331 | -14.726 | 1.00 | 71.96 | D |
| 3173 | CD1 | LEU | 647 | 31.553 | 21.158 | -15.662 | 1.00 | 71.20 | D |
| 3174 | CD2 | LEU | 647 | 32.354 | 22.209 | -13.544 | 1.00 | 71.68 | D |
| 3175 | C | LEU | 647 | 32.356 | 25.896 | -14.896 | 1.00 | 70.66 | D |
| 3176 | O | LEU | 647 | 33.367 | 26.131 | -14.243 | 1.00 | 71.06 | D |
| 3177 | N | TYR | 648 | 32.001 | 26.580 | -15.958 | 1.00 | 69.70 | D |
| 3178 | CA | TYR | 648 | 32.823 | 27.626 | -16.451 | 1.00 | 68.49 | D |
| 3179 | CB | TYR | 648 | 32.243 | 28.113 | -17.748 | 1.00 | 67.48 | D |
| 3180 | CG | TYR | 648 | 32.924 | 29.359 | -18.130 | 1.00 | 67.54 | D |
| 3181 | CD1 | TYR | 648 | 34.227 | 29.323 | -18.597 | 1.00 | 67.45 | D |
| 3182 | CE1 | TYR | 648 | 34.919 | 30.471 | -18.849 | 1.00 | 68.42 | D |
| 3183 | CD2 | TYR | 648 | 32.311 | 30.589 | -17.932 | 1.00 | 68.18 | D |
| 3184 | CE2 | TYR | 648 | 32.987 | 31.753 | -18.175 | 1.00 | 68.77 | D |
| 3185 | CZ | TYR | 648 | 34.296 | 31.689 | -18.646 | 1.00 | 70.60 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3186 | OH | TYR | 648 | 34.963 | 32.853 | -18.948 | 1.00 | 72.77 | D |
| 3187 | C | TYR | 648 | 33.058 | 28.813 | -15.535 | 1.00 | 68.18 | D |
| 3188 | O | TYR | 648 | 34.143 | 29.395 | -15.524 | 1.00 | 68.25 | D |
| 3189 | N | VAL | 649 | 32.045 | 29.181 | -14.776 | 1.00 | 67.31 | D |
| 3190 | CA | VAL | 649 | 32.165 | 30.349 | -13.925 | 1.00 | 66.61 | D |
| 3191 | CB | VAL | 649 | 30.755 | 30.921 | -13.625 | 1.00 | 65.52 | D |
| 3192 | CG1 | VAL | 649 | 29.736 | 30.299 | -14.581 | 1.00 | 63.01 | D |
| 3193 | CG2 | VAL | 649 | 30.363 | 30.668 | -12.194 | 1.00 | 64.50 | D |
| 3194 | C | VAL | 649 | 32.921 | 30.034 | -12.642 | 1.00 | 67.05 | D |
| 3195 | O | VAL | 649 | 33.734 | 30.830 | -12.167 | 1.00 | 66.32 | D |
| 3196 | N | SER | 650 | 32.631 | 28.856 | -12.106 | 1.00 | 68.46 | D |
| 3197 | CA | SER | 650 | 33.239 | 28.336 | -10.887 | 1.00 | 69.14 | D |
| 3198 | CB | SER | 650 | 32.687 | 26.951 | -10.620 | 1.00 | 67.35 | D |
| 3199 | OG | SER | 650 | 33.743 | 26.091 | -10.281 | 1.00 | 68.16 | D |
| 3200 | C | SER | 650 | 34.720 | 28.204 | -11.135 | 1.00 | 70.15 | D |
| 3201 | O | SER | 650 | 35.566 | 28.269 | -10.237 | 1.00 | 71.52 | D |
| 3202 | N | SER | 651 | 35.010 | 27.995 | -12.402 | 1.00 | 69.47 | D |
| 3203 | CA | SER | 651 | 36.356 | 27.803 | -12.836 | 1.00 | 68.31 | D |
| 3204 | CB | SER | 651 | 36.304 | 27.110 | -14.181 | 1.00 | 69.50 | D |
| 3205 | OG | SER | 651 | 37.318 | 27.608 | -15.023 | 1.00 | 70.83 | D |
| 3206 | C | SER | 651 | 37.086 | 29.121 | -12.925 | 1.00 | 66.66 | D |
| 3207 | O | SER | 651 | 38.290 | 29.205 | -12.729 | 1.00 | 65.24 | D |
| 3208 | N | GLU | 652 | 36.343 | 30.169 | -13.216 | 1.00 | 66.35 | D |
| 3209 | CA | GLU | 652 | 36.957 | 31.461 | -13.348 | 1.00 | 65.96 | D |
| 3210 | CB | GLU | 652 | 36.062 | 32.329 | -14.197 | 1.00 | 66.68 | D |
| 3211 | CG | GLU | 652 | 36.131 | 31.903 | -15.622 | 1.00 | 67.67 | D |
| 3212 | CD | GLU | 652 | 37.481 | 32.258 | -16.202 | 1.00 | 69.84 | D |
| 3213 | OE1 | GLU | 652 | 37.724 | 33.462 | -16.411 | 1.00 | 70.18 | D |
| 3214 | OE2 | GLU | 652 | 38.309 | 31.351 | -16.428 | 1.00 | 72.00 | D |
| 3215 | C | GLU | 652 | 37.179 | 32.061 | -11.996 | 1.00 | 64.92 | D |
| 3216 | O | GLU | 652 | 38.165 | 32.756 | -11.753 | 1.00 | 63.63 | D |
| 3217 | N | LEU | 653 | 36.246 | 31.781 | -11.109 | 1.00 | 65.42 | D |
| 3218 | CA | LEU | 653 | 36.338 | 32.277 | -9.755 | 1.00 | 66.47 | D |
| 3219 | CB | LEU | 653 | 35.169 | 31.757 | -8.957 | 1.00 | 66.41 | D |
| 3220 | CG | LEU | 653 | 33.934 | 32.597 | -9.220 | 1.00 | 64.68 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3221 | CD1 | LEU | 653 | 32.747 | 31.964 | -8.559 | 1.00 | 62.94 | D |
| 3222 | CD2 | LEU | 653 | 34.178 | 33.993 | -8.682 | 1.00 | 62.56 | D |
| 3223 | C | LEU | 653 | 37.602 | 31.727 | -9.171 | 1.00 | 67.66 | D |
| 3224 | O | LEU | 653 | 38.385 | 32.399 | -8.511 | 1.00 | 67.27 | D |
| 3225 | N | HIS | 654 | 37.780 | 30.457 | -9.428 | 1.00 | 69.12 | D |
| 3226 | CA | HIS | 654 | 38.923 | 29.801 | -8.923 | 1.00 | 70.49 | D |
| 3227 | CB | HIS | 654 | 38.836 | 28.367 | -9.293 | 1.00 | 74.35 | D |
| 3228 | CG | HIS | 654 | 40.008 | 27.578 | -8.800 | 1.00 | 78.11 | D |
| 3229 | CD2 | HIS | 654 | 41.218 | 27.362 | -9.405 | 1.00 | 78.68 | D |
| 3230 | ND1 | HIS | 654 | 40.019 | 26.988 | -7.601 | 1.00 | 79.49 | D |
| 3231 | CE1 | HIS | 654 | 41.218 | 26.385 | -7.421 | 1.00 | 80.11 | D |
| 3232 | NE2 | HIS | 654 | 41.924 | 26.610 | -8.493 | 1.00 | 79.67 | D |
| 3233 | C | HIS | 654 | 40.182 | 30.367 | -9.518 | 1.00 | 70.41 | D |
| 3234 | O | HIS | 654 | 41.132 | 30.725 | -8.821 | 1.00 | 71.08 | D |
| 3235 | N | ARG | 655 | 40.202 | 30.409 | -10.832 | 1.00 | 69.80 | D |
| 3236 | CA | ARG | 655 | 41.370 | 30.896 | -11.512 | 1.00 | 68.29 | D |
| 3237 | CB | ARG | 655 | 41.127 | 30.849 | -13.002 | 1.00 | 68.53 | D |
| 3238 | CG | ARG | 655 | 42.064 | 31.738 | -13.755 | 1.00 | 67.31 | D |
| 3239 | CD | ARG | 655 | 41.808 | 31.616 | -15.211 | 1.00 | 67.45 | D |
| 3240 | NE | ARG | 655 | 40.945 | 32.665 | -15.733 | 1.00 | 68.01 | D |
| 3241 | CZ | ARG | 655 | 41.322 | 33.934 | -15.833 | 1.00 | 69.26 | D |
| 3242 | NH1 | ARG | 655 | 42.533 | 34.286 | -15.436 | 1.00 | 70.31 | D |
| 3243 | NH2 | ARG | 655 | 40.525 | 34.834 | -16.396 | 1.00 | 70.41 | D |
| 3244 | C | ARG | 655 | 41.724 | 32.309 | -11.109 | 1.00 | 67.75 | D |
| 3245 | O | ARG | 655 | 42.889 | 32.670 | -10.956 | 1.00 | 67.53 | D |
| 3246 | N | LEU | 656 | 40.700 | 33.120 | -10.945 | 1.00 | 67.39 | D |
| 3247 | CA | LEU | 656 | 40.917 | 34.501 | -10.607 | 1.00 | 66.65 | D |
| 3248 | CB | LEU | 656 | 39.739 | 35.301 | -11.088 | 1.00 | 66.57 | D |
| 3249 | CG | LEU | 656 | 40.126 | 36.495 | -11.943 | 1.00 | 67.03 | D |
| 3250 | CD1 | LEU | 656 | 41.318 | 36.181 | -12.810 | 1.00 | 65.91 | D |
| 3251 | CD2 | LEU | 656 | 38.925 | 36.851 | -12.783 | 1.00 | 66.92 | D |
| 3252 | C | LEU | 656 | 41.099 | 34.703 | -9.133 | 1.00 | 66.46 | D |
| 3253 | O | LEU | 656 | 41.387 | 35.812 | -8.677 | 1.00 | 64.34 | D |
| 3254 | N | GLN | 657 | 40.896 | 33.630 | -8.380 | 1.00 | 66.91 | D |
| 3255 | CA | GLN | 657 | 41.064 | 33.735 | -6.958 | 1.00 | 67.19 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3256 | CB | GLN | 657 | 42.526 | 33.983 | -6.678 | 1.00 | 69.55 | D |
| 3257 | CG | GLN | 657 | 43.331 | 32.749 | -6.906 | 1.00 | 71.85 | D |
| 3258 | CD | GLN | 657 | 43.089 | 31.780 | -5.786 | 1.00 | 74.34 | D |
| 3259 | OE1 | GLN | 657 | 42.589 | 30.672 | -5.987 | 1.00 | 74.41 | D |
| 3260 | NE2 | GLN | 657 | 43.426 | 32.208 | -4.573 | 1.00 | 74.95 | D |
| 3261 | C | GLN | 657 | 40.238 | 34.900 | -6.507 | 1.00 | 66.94 | D |
| 3262 | O | GLN | 657 | 40.766 | 35.902 | -6.047 | 1.00 | 68.33 | D |
| 3263 | N | VAL | 658 | 38.934 | 34.785 | -6.657 | 1.00 | 65.98 | D |
| 3264 | CA | VAL | 658 | 38.076 | 35.866 | -6.254 | 1.00 | 64.44 | D |
| 3265 | CB | VAL | 658 | 36.810 | 35.879 | -7.126 | 1.00 | 63.24 | D |
| 3266 | CG1 | VAL | 658 | 35.711 | 36.664 | -6.451 | 1.00 | 60.80 | D |
| 3267 | CG2 | VAL | 658 | 37.155 | 36.489 | -8.486 | 1.00 | 61.41 | D |
| 3268 | C | VAL | 658 | 37.750 | 35.737 | -4.781 | 1.00 | 64.62 | D |
| 3269 | O | VAL | 658 | 37.513 | 34.645 | -4.290 | 1.00 | 64.05 | D |
| 3270 | N | SER | 659 | 37.756 | 36.866 | -4.079 | 1.00 | 65.57 | D |
| 3271 | CA | SER | 659 | 37.469 | 36.893 | -2.645 | 1.00 | 66.30 | D |
| 3272 | CB | SER | 659 | 38.306 | 37.966 | -1.978 | 1.00 | 66.15 | D |
| 3273 | OG | SER | 659 | 37.759 | 39.241 | -2.264 | 1.00 | 67.74 | D |
| 3274 | C | SER | 659 | 35.999 | 37.189 | -2.358 | 1.00 | 67.03 | D |
| 3275 | O | SER | 659 | 35.339 | 37.878 | -3.133 | 1.00 | 68.36 | D |
| 3276 | N | TYR | 660 | 35.496 | 36.696 | -1.227 | 1.00 | 67.15 | D |
| 3277 | CA | TYR | 660 | 34.092 | 36.910 | -0.847 | 1.00 | 66.17 | D |
| 3278 | CB | TYR | 660 | 33.857 | 36.533 | 0.615 | 1.00 | 64.78 | D |
| 3279 | CG | TYR | 660 | 32.403 | 36.313 | 0.959 | 1.00 | 63.18 | D |
| 3280 | CD1 | TYR | 660 | 31.643 | 35.416 | 0.226 | 1.00 | 62.24 | D |
| 3281 | CE1 | TYR | 660 | 30.355 | 35.103 | 0.591 | 1.00 | 61.50 | D |
| 3282 | CD2 | TYR | 660 | 31.815 | 36.910 | 2.084 | 1.00 | 62.26 | D |
| 3283 | CE2 | TYR | 660 | 30.503 | 36.593 | 2.462 | 1.00 | 60.49 | D |
| 3284 | CZ | TYR | 660 | 29.791 | 35.680 | 1.709 | 1.00 | 60.89 | D |
| 3285 | OH | TYR | 660 | 28.535 | 35.259 | 2.082 | 1.00 | 62.14 | D |
| 3286 | C | TYR | 660 | 33.652 | 38.352 | -1.005 | 1.00 | 66.01 | D |
| 3287 | O | TYR | 660 | 32.531 | 38.640 | -1.433 | 1.00 | 64.21 | D |
| 3288 | N | GLU | 661 | 34.522 | 39.270 | -0.629 | 1.00 | 66.98 | D |
| 3289 | CA | GLU | 661 | 34.122 | 40.644 | -0.743 | 1.00 | 67.54 | D |
| 3290 | CB | GLU | 661 | 35.070 | 41.543 | 0.040 | 1.00 | 69.27 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3291 | CG | GLU | 661 | 34.532 | 41.760 | 1.448 | 1.00 | 71.42 | D |
| 3292 | CD | GLU | 661 | 35.342 | 42.747 | 2.256 | 1.00 | 74.29 | D |
| 3293 | OE1 | GLU | 661 | 35.698 | 43.822 | 1.714 | 1.00 | 73.64 | D |
| 3294 | OE2 | GLU | 661 | 35.610 | 42.449 | 3.444 | 1.00 | 75.81 | D |
| 3295 | C | GLU | 661 | 33.951 | 41.068 | -2.186 | 1.00 | 66.87 | D |
| 3296 | O | GLU | 661 | 32.961 | 41.727 | -2.508 | 1.00 | 67.41 | D |
| 3297 | N | GLU | 662 | 34.868 | 40.688 | -3.071 | 1.00 | 64.88 | D |
| 3298 | CA | GLU | 662 | 34.686 | 41.042 | -4.477 | 1.00 | 63.49 | D |
| 3299 | CB | GLU | 662 | 35.873 | 40.599 | -5.314 | 1.00 | 62.99 | D |
| 3300 | CG | GLU | 662 | 37.162 | 41.179 | -4.843 | 1.00 | 64.63 | D |
| 3301 | CD | GLU | 662 | 38.352 | 40.431 | -5.382 | 1.00 | 65.77 | D |
| 3302 | OE1 | GLU | 662 | 38.304 | 39.176 | -5.401 | 1.00 | 65.65 | D |
| 3303 | OE2 | GLU | 662 | 39.334 | 41.096 | -5.771 | 1.00 | 66.42 | D |
| 3304 | C | GLU | 662 | 33.425 | 40.344 | -4.991 | 1.00 | 62.59 | D |
| 3305 | O | GLU | 662 | 32.602 | 40.943 | -5.669 | 1.00 | 62.76 | D |
| 3306 | N | TYR | 663 | 33.275 | 39.067 | -4.666 | 1.00 | 61.55 | D |
| 3307 | CA | TYR | 663 | 32.116 | 38.296 | -5.105 | 1.00 | 61.53 | D |
| 3308 | CB | TYR | 663 | 32.173 | 36.903 | -4.511 | 1.00 | 60.18 | D |
| 3309 | CG | TYR | 663 | 30.842 | 36.207 | -4.514 | 1.00 | 58.99 | D |
| 3310 | CD1 | TYR | 663 | 30.358 | 35.603 | -5.679 | 1.00 | 58.48 | D |
| 3311 | CE1 | TYR | 663 | 29.173 | 34.869 | -5.674 | 1.00 | 57.76 | D |
| 3312 | CD2 | TYR | 663 | 30.098 | 36.076 | -3.342 | 1.00 | 58.44 | D |
| 3313 | CE2 | TYR | 663 | 28.902 | 35.341 | -3.324 | 1.00 | 58.34 | D |
| 3314 | CZ | TYR | 663 | 28.453 | 34.738 | -4.497 | 1.00 | 57.94 | D |
| 3315 | OH | TYR | 663 | 27.308 | 33.970 | -4.496 | 1.00 | 57.87 | D |
| 3316 | C | TYR | 663 | 30.767 | 38.908 | -4.724 | 1.00 | 62.00 | D |
| 3317 | O | TYR | 663 | 29.829 | 38.924 | -5.520 | 1.00 | 62.20 | D |
| 3318 | N | LEU | 664 | 30.665 | 39.354 | -3.481 | 1.00 | 62.05 | D |
| 3319 | CA | LEU | 664 | 29.445 | 39.953 | -2.986 | 1.00 | 61.47 | D |
| 3320 | CB | LEU | 664 | 29.637 | 40.414 | -1.538 | 1.00 | 59.38 | D |
| 3321 | CG | LEU | 664 | 29.465 | 39.343 | -0.468 | 1.00 | 56.38 | D |
| 3322 | CD1 | LEU | 664 | 29.558 | 39.964 | 0.898 | 1.00 | 56.57 | D |
| 3323 | CD2 | LEU | 664 | 28.120 | 38.685 | -0.643 | 1.00 | 54.70 | D |
| 3324 | C | LEU | 664 | 29.041 | 41.139 | -3.858 | 1.00 | 61.76 | D |
| 3325 | O | LEU | 664 | 27.897 | 41.235 | -4.315 | 1.00 | 62.66 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3326 | N | CYS | 665 | 30.001 | 42.036 | -4.062 | 1.00 | 60.92 | D |
| 3327 | CA | CYS | 665 | 29.814 | 43.223 | -4.871 | 1.00 | 60.90 | D |
| 3328 | CB | CYS | 665 | 31.074 | 44.068 | -4.861 | 1.00 | 61.24 | D |
| 3329 | SG | CYS | 665 | 31.426 | 44.837 | -3.286 | 1.00 | 65.61 | D |
| 3330 | C | CYS | 665 | 29.496 | 42.865 | -6.306 | 1.00 | 60.48 | D |
| 3331 | O | CYS | 665 | 28.707 | 43.530 | -6.963 | 1.00 | 61.40 | D |
| 3332 | N | MET | 666 | 30.117 | 41.818 | -6.803 | 1.00 | 60.26 | D |
| 3333 | CA | MET | 666 | 29.875 | 41.421 | -8.171 | 1.00 | 61.32 | D |
| 3334 | CB | MET | 666 | 30.901 | 40.380 | -8.603 | 1.00 | 62.78 | D |
| 3335 | CG | MET | 666 | 32.263 | 40.951 | -8.889 | 1.00 | 64.66 | D |
| 3336 | SD | MET | 666 | 33.317 | 39.659 | -9.479 | 1.00 | 66.42 | D |
| 3337 | CE | MET | 666 | 33.925 | 39.166 | -8.028 | 1.00 | 65.54 | D |
| 3338 | C | MET | 666 | 28.473 | 40.873 | -8.410 | 1.00 | 61.55 | D |
| 3339 | O | MET | 666 | 27.900 | 41.065 | -9.487 | 1.00 | 61.02 | D |
| 3340 | N | LYS | 667 | 27.924 | 40.178 | -7.419 | 1.00 | 61.13 | D |
| 3341 | CA | LYS | 667 | 26.595 | 39.607 | -7.568 | 1.00 | 60.57 | D |
| 3342 | CB | LYS | 667 | 26.350 | 38.592 | -6.466 | 1.00 | 59.74 | D |
| 3343 | CG | LYS | 667 | 25.239 | 37.616 | -6.751 | 1.00 | 59.22 | D |
| 3344 | CD | LYS | 667 | 25.237 | 36.517 | -5.690 | 1.00 | 60.32 | D |
| 3345 | CE | LYS | 667 | 24.654 | 36.991 | -4.345 | 1.00 | 62.35 | D |
| 3346 | NZ | LYS | 667 | 25.373 | 38.129 | -3.686 | 1.00 | 63.45 | D |
| 3347 | C | LYS | 667 | 25.547 | 40.710 | -7.513 | 1.00 | 60.96 | D |
| 3348 | O | LYS | 667 | 24.472 | 40.594 | -8.112 | 1.00 | 60.77 | D |
| 3349 | N | THR | 668 | 25.864 | 41.775 | -6.782 | 1.00 | 60.79 | D |
| 3350 | CA | THR | 668 | 24.973 | 42.909 | -6.658 | 1.00 | 60.41 | D |
| 3351 | CB | THR | 668 | 25.477 | 43.872 | -5.594 | 1.00 | 61.34 | D |
| 3352 | OG1 | THR | 668 | 25.395 | 43.225 | -4.321 | 1.00 | 63.92 | D |
| 3353 | CG2 | THR | 668 | 24.646 | 45.148 | -5.575 | 1.00 | 61.41 | D |
| 3354 | C | THR | 668 | 24.996 | 43.561 | -8.023 | 1.00 | 59.41 | D |
| 3355 | O | THR | 668 | 23.960 | 43.901 | -8.568 | 1.00 | 59.47 | D |
| 3356 | N | LEU | 669 | 26.195 | 43.708 | -8.580 | 1.00 | 58.91 | D |
| 3357 | CA | LEU | 669 | 26.346 | 44.279 | -9.906 | 1.00 | 57.37 | D |
| 3358 | CB | LEU | 669 | 27.816 | 44.348 | -10.293 | 1.00 | 55.15 | D |
| 3359 | CG | LEU | 669 | 28.451 | 45.738 | -10.271 | 1.00 | 54.09 | D |
| 3360 | CD1 | LEU | 669 | 27.548 | 46.728 | -9.596 | 1.00 | 53.96 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3361 | CD2 | LEU | 669 | 29.782 | 45.684 | -9.566 | 1.00 | 54.12 | D |
| 3362 | C | LEU | 669 | 25.577 | 43.457 | -10.937 | 1.00 | 57.74 | D |
| 3363 | O | LEU | 669 | 25.001 | 44.015 | -11.849 | 1.00 | 59.33 | D |
| 3364 | N | LEU | 670 | 25.540 | 42.139 | -10.809 | 1.00 | 57.67 | D |
| 3365 | CA | LEU | 670 | 24.787 | 41.377 | -11.803 | 1.00 | 58.56 | D |
| 3366 | CB | LEU | 670 | 24.977 | 39.878 | -11.623 | 1.00 | 58.10 | D |
| 3367 | CG | LEU | 670 | 26.318 | 39.252 | -11.968 | 1.00 | 58.50 | D |
| 3368 | CD1 | LEU | 670 | 26.221 | 37.821 | -11.498 | 1.00 | 57.91 | D |
| 3369 | CD2 | LEU | 670 | 26.640 | 39.314 | -13.463 | 1.00 | 56.69 | D |
| 3370 | C | LEU | 670 | 23.296 | 41.656 | -11.773 | 1.00 | 59.22 | D |
| 3371 | O | LEU | 670 | 22.615 | 41.449 | -12.768 | 1.00 | 59.43 | D |
| 3372 | N | LEU | 671 | 22.786 | 42.095 | -10.629 | 1.00 | 60.29 | D |
| 3373 | CA | LEU | 671 | 21.359 | 42.389 | -10.500 | 1.00 | 59.73 | D |
| 3374 | CB | LEU | 671 | 21.003 | 42.662 | -9.035 | 1.00 | 57.31 | D |
| 3375 | CG | LEU | 671 | 19.589 | 43.177 | -8.782 | 1.00 | 55.23 | D |
| 3376 | CD1 | LEU | 671 | 18.578 | 42.139 | -9.272 | 1.00 | 53.61 | D |
| 3377 | CD2 | LEU | 671 | 19.409 | 43.482 | -7.299 | 1.00 | 52.46 | D |
| 3378 | C | LEU | 671 | 21.092 | 43.632 | -11.334 | 1.00 | 60.95 | D |
| 3379 | O | LEU | 671 | 20.006 | 43.804 | -11.891 | 1.00 | 61.87 | D |
| 3380 | N | LEU | 672 | 22.117 | 44.476 | -11.420 | 1.00 | 60.54 | D |
| 3381 | CA | LEU | 672 | 22.058 | 45.718 | -12.168 | 1.00 | 60.50 | D |
| 3382 | CB | LEU | 672 | 22.810 | 46.795 | -11.402 | 1.00 | 58.78 | D |
| 3383 | CG | LEU | 672 | 22.413 | 46.856 | -9.938 | 1.00 | 58.99 | D |
| 3384 | CD1 | LEU | 672 | 23.175 | 47.964 | -9.225 | 1.00 | 57.12 | D |
| 3385 | CD2 | LEU | 672 | 20.914 | 47.072 | -9.865 | 1.00 | 58.80 | D |
| 3386 | C | LEU | 672 | 22.726 | 45.538 | -13.515 | 1.00 | 62.31 | D |
| 3387 | O | LEU | 672 | 23.130 | 46.520 | -14.136 | 1.00 | 63.27 | D |
| 3388 | N | SER | 673 | 22.826 | 44.295 | -13.972 | 1.00 | 64.36 | D |
| 3389 | CA | SER | 673 | 23.527 | 43.991 | -15.217 | 1.00 | 67.18 | D |
| 3390 | CB | SER | 673 | 24.130 | 42.588 | -15.146 | 1.00 | 66.06 | D |
| 3391 | OG | SER | 673 | 23.144 | 41.590 | -15.336 | 1.00 | 63.96 | D |
| 3392 | C | SER | 673 | 22.840 | 44.150 | -16.570 | 1.00 | 70.84 | D |
| 3393 | O | SER | 673 | 23.468 | 43.919 | -17.608 | 1.00 | 71.75 | D |
| 3394 | N | SER | 674 | 21.562 | 44.495 | -16.587 | 1.00 | 73.68 | D |
| 3395 | CA | SER | 674 | 20.911 | 44.726 | -17.872 | 1.00 | 75.75 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3396 | CB | SER | 674 | 20.813 | 43.430 | -18.723 | 1.00 | 74.70 | D |
| 3397 | OG | SER | 674 | 19.856 | 42.496 | -18.265 | 1.00 | 74.10 | D |
| 3398 | C | SER | 674 | 19.570 | 45.403 | -17.662 | 1.00 | 76.88 | D |
| 3399 | O | SER | 674 | 18.859 | 45.143 | -16.694 | 1.00 | 76.90 | D |
| 3400 | N | VAL | 675 | 19.266 | 46.333 | -18.554 | 1.00 | 79.18 | D |
| 3401 | CA | VAL | 675 | 18.034 | 47.082 | -18.465 | 1.00 | 82.02 | D |
| 3402 | CB | VAL | 675 | 18.333 | 48.572 | -18.122 | 1.00 | 81.32 | D |
| 3403 | CG1 | VAL | 675 | 18.840 | 48.676 | -16.689 | 1.00 | 80.99 | D |
| 3404 | CG2 | VAL | 675 | 19.375 | 49.143 | -19.084 | 1.00 | 81.53 | D |
| 3405 | C | VAL | 675 | 17.282 | 46.979 | -19.783 | 1.00 | 84.34 | D |
| 3406 | O | VAL | 675 | 17.825 | 46.508 | -20.788 | 1.00 | 84.33 | D |
| 3407 | N | PRO | 676 | 16.006 | 47.390 | -19.792 | 1.00 | 86.71 | D |
| 3408 | CD | PRO | 676 | 15.247 | 48.061 | -18.726 | 1.00 | 86.46 | D |
| 3409 | CA | PRO | 676 | 15.222 | 47.326 | -21.019 | 1.00 | 89.31 | D |
| 3410 | CB | PRO | 676 | 13.828 | 47.717 | -20.540 | 1.00 | 88.04 | D |
| 3411 | CG | PRO | 676 | 14.130 | 48.722 | -19.485 | 1.00 | 86.61 | D |
| 3412 | C | PRO | 676 | 15.832 | 48.341 | -21.978 | 1.00 | 92.02 | D |
| 3413 | O | PRO | 676 | 16.330 | 49.381 | -21.537 | 1.00 | 92.35 | D |
| 3414 | N | LYS | 677 | 15.842 | 48.036 | -23.272 | 1.00 | 94.90 | D |
| 3415 | CA | LYS | 677 | 16.407 | 48.972 | -24.244 | 1.00 | 97.63 | D |
| 3416 | CB | LYS | 677 | 16.648 | 48.301 | -25.598 | 1.00 | 98.89 | D |
| 3417 | CG | LYS | 677 | 16.682 | 49.263 | -26.803 | 1.00 | 101.15 | D |
| 3418 | CD | LYS | 677 | 17.638 | 50.452 | -26.628 | 1.00 | 103.43 | D |
| 3419 | CE | LYS | 677 | 17.643 | 51.363 | -27.875 | 1.00 | 104.79 | D |
| 3420 | NZ | LYS | 677 | 18.011 | 50.655 | -29.151 | 1.00 | 104.66 | D |
| 3421 | C | LYS | 677 | 15.422 | 50.100 | -24.414 | 1.00 | 98.84 | D |
| 3422 | O | LYS | 677 | 14.555 | 50.055 | -25.273 | 1.00 | 99.87 | D |
| 3423 | N | ASP | 678 | 15.573 | 51.114 | -23.582 | 1.00 | 99.57 | D |
| 3424 | CA | ASP | 678 | 14.702 | 52.270 | -23.583 | 1.00 | 100.29 | D |
| 3425 | CB | ASP | 678 | 13.225 | 51.869 | -23.734 | 1.00 | 101.68 | D |
| 3426 | CG | ASP | 678 | 12.772 | 51.830 | -25.198 | 1.00 | 103.54 | D |
| 3427 | OD1 | ASP | 678 | 13.319 | 52.599 | -26.020 | 1.00 | 103.41 | D |
| 3428 | OD2 | ASP | 678 | 11.855 | 51.040 | -25.518 | 1.00 | 104.45 | D |
| 3429 | C | ASP | 678 | 14.951 | 52.811 | -22.196 | 1.00 | 100.06 | D |
| 3430 | O | ASP | 678 | 14.945 | 54.020 | -21.966 | 1.00 | 100.74 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3431 | N | GLY | 679 | 15.194 | 51.884 | -21.272 | 1.00 | 98.77 | D |
| 3432 | CA | GLY | 679 | 15.483 | 52.273 | -19.906 | 1.00 | 96.64 | D |
| 3433 | C | GLY | 679 | 14.346 | 52.099 | -18.930 | 1.00 | 95.05 | D |
| 3434 | O | GLY | 679 | 13.198 | 51.851 | -19.309 | 1.00 | 95.43 | D |
| 3435 | N | LEU | 680 | 14.677 | 52.258 | -17.653 | 1.00 | 93.10 | D |
| 3436 | CA | LEU | 680 | 13.712 | 52.103 | -16.580 | 1.00 | 91.98 | D |
| 3437 | CB | LEU | 680 | 14.398 | 51.536 | -15.332 | 1.00 | 91.95 | D |
| 3438 | CG | LEU | 680 | 15.723 | 50.795 | -15.513 | 1.00 | 91.77 | D |
| 3439 | CD1 | LEU | 680 | 16.303 | 50.412 | -14.171 | 1.00 | 91.91 | D |
| 3440 | CD2 | LEU | 680 | 15.494 | 49.569 | -16.329 | 1.00 | 92.35 | D |
| 3441 | C | LEU | 680 | 13.025 | 53.407 | -16.199 | 1.00 | 90.95 | D |
| 3442 | O | LEU | 680 | 13.467 | 54.493 | -16.570 | 1.00 | 91.02 | D |
| 3443 | N | LYS | 681 | 11.943 | 53.271 | -15.442 | 1.00 | 90.04 | D |
| 3444 | CA | LYS | 681 | 11.173 | 54.410 | -14.964 | 1.00 | 89.16 | D |
| 3445 | CB | LYS | 681 | 9.975 | 53.931 | -14.143 | 1.00 | 89.45 | D |
| 3446 | CG | LYS | 681 | 8.980 | 53.086 | -14.913 | 1.00 | 89.94 | D |
| 3447 | CD | LYS | 681 | 7.670 | 52.959 | -14.154 | 1.00 | 90.41 | D |
| 3448 | CE | LYS | 681 | 6.800 | 51.866 | -14.745 | 1.00 | 91.27 | D |
| 3449 | NZ | LYS | 681 | 5.491 | 51.754 | -14.056 | 1.00 | 91.68 | D |
| 3450 | C | LYS | 681 | 12.050 | 55.290 | -14.087 | 1.00 | 88.34 | D |
| 3451 | O | LYS | 681 | 11.866 | 56.508 | -14.019 | 1.00 | 88.29 | D |
| 3452 | N | SER | 682 | 13.008 | 54.656 | -13.419 | 1.00 | 87.39 | D |
| 3453 | CA | SER | 682 | 13.913 | 55.358 | -12.521 | 1.00 | 86.48 | D |
| 3454 | CB | SER | 682 | 13.803 | 54.761 | -11.117 | 1.00 | 87.14 | D |
| 3455 | OG | SER | 682 | 12.473 | 54.351 | -10.838 | 1.00 | 87.46 | D |
| 3456 | C | SER | 682 | 15.350 | 55.242 | -13.012 | 1.00 | 85.57 | D |
| 3457 | O | SER | 682 | 16.278 | 55.196 | -12.203 | 1.00 | 85.27 | D |
| 3458 | N | GLN | 683 | 15.521 | 55.197 | -14.330 | 1.00 | 84.91 | D |
| 3459 | CA | GLN | 683 | 16.844 | 55.073 | -14.948 | 1.00 | 84.23 | D |
| 3460 | CB | GLN | 683 | 16.787 | 55.515 | -16.413 | 1.00 | 83.77 | D |
| 3461 | CG | GLN | 683 | 18.108 | 55.393 | -17.157 | 1.00 | 84.51 | D |
| 3462 | CD | GLN | 683 | 18.644 | 53.969 | -17.198 | 1.00 | 85.77 | D |
| 3463 | OE1 | GLN | 683 | 18.017 | 53.065 | -17.766 | 1.00 | 85.99 | D |
| 3464 | NE2 | GLN | 683 | 19.807 | 53.761 | -16.595 | 1.00 | 85.67 | D |
| 3465 | C | GLN | 683 | 17.853 | 55.924 | -14.204 | 1.00 | 83.55 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3466 | O | GLN | 683 | 18.961 | 55.482 | -13.894 | 1.00 | 83.89 | D |
| 3467 | N | GLU | 684 | 17.447 | 57.147 | -13.921 | 1.00 | 83.39 | D |
| 3468 | CA | GLU | 684 | 18.256 | 58.112 | -13.203 | 1.00 | 83.70 | D |
| 3469 | CB | GLU | 684 | 17.345 | 59.309 | -12.795 | 1.00 | 86.45 | D |
| 3470 | CG | GLU | 684 | 15.864 | 58.957 | -12.344 | 1.00 | 89.45 | D |
| 3471 | CD | GLU | 684 | 14.753 | 59.125 | -13.432 | 1.00 | 91.23 | D |
| 3472 | OE1 | GLU | 684 | 14.992 | 58.827 | -14.626 | 1.00 | 91.14 | D |
| 3473 | OE2 | GLU | 684 | 13.614 | 59.535 | -13.077 | 1.00 | 91.97 | D |
| 3474 | C | GLU | 684 | 18.965 | 57.468 | -11.988 | 1.00 | 82.04 | D |
| 3475 | O | GLU | 684 | 20.201 | 57.396 | -11.931 | 1.00 | 81.53 | D |
| 3476 | N | LEU | 685 | 18.157 | 56.968 | -11.057 | 1.00 | 80.84 | D |
| 3477 | CA | LEU | 685 | 18.585 | 56.321 | -9.807 | 1.00 | 79.91 | D |
| 3478 | CB | LEU | 685 | 17.341 | 56.029 | -8.964 | 1.00 | 79.23 | D |
| 3479 | CG | LEU | 685 | 17.327 | 56.378 | -7.477 | 1.00 | 78.87 | D |
| 3480 | CD1 | LEU | 685 | 16.478 | 55.346 | -6.740 | 1.00 | 78.47 | D |
| 3481 | CD2 | LEU | 685 | 18.742 | 56.376 | -6.921 | 1.00 | 79.03 | D |
| 3482 | C | LEU | 685 | 19.368 | 55.013 | -10.008 | 1.00 | 79.20 | D |
| 3483 | O | LEU | 685 | 20.294 | 54.682 | -9.259 | 1.00 | 78.90 | D |
| 3484 | N | PHE | 686 | 18.950 | 54.256 | -11.004 | 1.00 | 77.79 | D |
| 3485 | CA | PHE | 686 | 19.598 | 53.011 | -11.333 | 1.00 | 76.60 | D |
| 3486 | CB | PHE | 686 | 19.024 | 52.503 | -12.633 | 1.00 | 74.90 | D |
| 3487 | CG | PHE | 686 | 19.654 | 51.260 | -13.106 | 1.00 | 73.91 | D |
| 3488 | CD1 | PHE | 686 | 19.184 | 50.036 | -12.669 | 1.00 | 73.55 | D |
| 3489 | CD2 | PHE | 686 | 20.739 | 51.307 | -13.969 | 1.00 | 73.72 | D |
| 3490 | CE1 | PHE | 686 | 19.771 | 48.866 | -13.091 | 1.00 | 73.58 | D |
| 3491 | CE2 | PHE | 686 | 21.337 | 50.140 | -14.405 | 1.00 | 74.95 | D |
| 3492 | CZ | PHE | 686 | 20.856 | 48.912 | -13.959 | 1.00 | 74.68 | D |
| 3493 | C | PHE | 686 | 21.070 | 53.309 | -11.528 | 1.00 | 76.92 | D |
| 3494 | O | PHE | 686 | 21.942 | 52.774 | -10.845 | 1.00 | 76.88 | D |
| 3495 | N | ASP | 687 | 21.332 | 54.194 | -12.481 | 1.00 | 77.62 | D |
| 3496 | CA | ASP | 687 | 22.687 | 54.562 | -12.804 | 1.00 | 78.23 | D |
| 3497 | CB | ASP | 687 | 22.697 | 55.529 | -13.983 | 1.00 | 79.48 | D |
| 3498 | CG | ASP | 687 | 22.345 | 54.836 | -15.286 | 1.00 | 80.76 | D |
| 3499 | OD1 | ASP | 687 | 22.659 | 53.632 | -15.418 | 1.00 | 80.42 | D |
| 3500 | OD2 | ASP | 687 | 21.768 | 55.489 | -16.180 | 1.00 | 81.33 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3501 | C | ASP | 687 | 23.420 | 55.140 | -11.623 | 1.00 | 77.82 | D |
| 3502 | O | ASP | 687 | 24.649 | 55.192 | -11.610 | 1.00 | 77.25 | D |
| 3503 | N | GLU | 688 | 22.676 | 55.560 | -10.614 | 1.00 | 77.40 | D |
| 3504 | CA | GLU | 688 | 23.326 | 56.105 | -9.444 | 1.00 | 77.00 | D |
| 3505 | CB | GLU | 688 | 22.393 | 57.027 | -8.699 | 1.00 | 79.26 | D |
| 3506 | CG | GLU | 688 | 23.115 | 57.790 | -7.636 | 1.00 | 83.90 | D |
| 3507 | CD | GLU | 688 | 22.167 | 58.405 | -6.663 | 1.00 | 86.20 | D |
| 3508 | OE1 | GLU | 688 | 21.003 | 58.648 | -7.061 | 1.00 | 87.82 | D |
| 3509 | OE2 | GLU | 688 | 22.587 | 58.648 | -5.510 | 1.00 | 87.04 | D |
| 3510 | C | GLU | 688 | 23.724 | 54.954 | -8.538 | 1.00 | 75.46 | D |
| 3511 | O | GLU | 688 | 24.850 | 54.904 | -8.027 | 1.00 | 75.05 | D |
| 3512 | N | ILE | 689 | 22.789 | 54.032 | -8.343 | 1.00 | 73.02 | D |
| 3513 | CA | ILE | 689 | 23.026 | 52.871 | -7.514 | 1.00 | 70.61 | D |
| 3514 | CB | ILE | 689 | 21.731 | 52.082 | -7.351 | 1.00 | 71.16 | D |
| 3515 | CG2 | ILE | 689 | 21.977 | 50.724 | -6.687 | 1.00 | 69.86 | D |
| 3516 | CG1 | ILE | 689 | 20.775 | 52.947 | -6.542 | 1.00 | 71.37 | D |
| 3517 | CD1 | ILE | 689 | 19.540 | 52.267 | -6.190 | 1.00 | 72.58 | D |
| 3518 | C | ILE | 689 | 24.092 | 52.043 | -8.186 | 1.00 | 69.08 | D |
| 3519 | O | ILE | 689 | 25.093 | 51.694 | -7.572 | 1.00 | 68.64 | D |
| 3520 | N | ARG | 690 | 23.898 | 51.758 | -9.463 | 1.00 | 67.58 | D |
| 3521 | CA | ARG | 690 | 24.882 | 50.980 | -10.186 | 1.00 | 67.61 | D |
| 3522 | CB | ARG | 690 | 24.491 | 50.855 | -11.644 | 1.00 | 65.92 | D |
| 3523 | CG | ARG | 690 | 25.464 | 50.007 | -12.435 | 1.00 | 65.20 | D |
| 3524 | CD | ARG | 690 | 24.717 | 49.473 | -13.621 | 1.00 | 66.02 | D |
| 3525 | NE | ARG | 690 | 25.445 | 48.547 | -14.481 | 1.00 | 64.92 | D |
| 3526 | CZ | ARG | 690 | 26.646 | 48.781 | -14.997 | 1.00 | 65.68 | D |
| 3527 | NH1 | ARG | 690 | 27.285 | 49.912 | -14.727 | 1.00 | 65.29 | D |
| 3528 | NH2 | ARG | 690 | 27.179 | 47.904 | -15.839 | 1.00 | 66.43 | D |
| 3529 | C | ARG | 690 | 26.308 | 51.527 | -10.117 | 1.00 | 68.89 | D |
| 3530 | O | ARG | 690 | 27.264 | 50.756 | -10.127 | 1.00 | 70.04 | D |
| 3531 | N | MET | 691 | 26.465 | 52.846 | -10.065 | 1.00 | 70.17 | D |
| 3532 | CA | MET | 691 | 27.795 | 53.438 | -10.002 | 1.00 | 70.49 | D |
| 3533 | CB | MET | 691 | 27.708 | 54.940 | -10.291 | 1.00 | 73.73 | D |
| 3534 | CG | MET | 691 | 29.047 | 55.648 | -10.500 | 1.00 | 77.42 | D |
| 3535 | SD | MET | 691 | 29.970 | 54.941 | -11.898 | 1.00 | 82.38 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3536 | CE | MET | 691 | 31.370 | 54.338 | -11.036 | 1.00 | 81.52 | D |
| 3537 | C | MET | 691 | 28.418 | 53.205 | -8.626 | 1.00 | 69.68 | D |
| 3538 | O | MET | 691 | 29.625 | 52.981 | -8.506 | 1.00 | 68.71 | D |
| 3539 | N | THR | 692 | 27.579 | 53.256 | -7.591 | 1.00 | 69.09 | D |
| 3540 | CA | THR | 692 | 28.033 | 53.068 | -6.213 | 1.00 | 68.99 | D |
| 3541 | CB | THR | 692 | 26.918 | 53.278 | -5.201 | 1.00 | 68.68 | D |
| 3542 | OG1 | THR | 692 | 26.208 | 54.478 | -5.503 | 1.00 | 68.25 | D |
| 3543 | CG2 | THR | 692 | 27.518 | 53.382 | -3.812 | 1.00 | 68.52 | D |
| 3544 | C | THR | 692 | 28.572 | 51.672 | -5.977 | 1.00 | 69.04 | D |
| 3545 | O | THR | 692 | 29.567 | 51.491 | -5.258 | 1.00 | 68.26 | D |
| 3546 | N | TYR | 693 | 27.901 | 50.672 | -6.543 | 1.00 | 68.04 | D |
| 3547 | CA | TYR | 693 | 28.422 | 49.339 | -6.376 | 1.00 | 67.90 | D |
| 3548 | CB | TYR | 693 | 27.340 | 48.267 | -6.564 | 1.00 | 67.99 | D |
| 3549 | CG | TYR | 693 | 26.389 | 48.214 | -5.384 | 1.00 | 68.55 | D |
| 3550 | CD1 | TYR | 693 | 26.855 | 47.903 | -4.103 | 1.00 | 67.96 | D |
| 3551 | CE1 | TYR | 693 | 26.014 | 47.999 | -2.991 | 1.00 | 68.49 | D |
| 3552 | CD2 | TYR | 693 | 25.056 | 48.599 | -5.523 | 1.00 | 67.38 | D |
| 3553 | CE2 | TYR | 693 | 24.216 | 48.696 | -4.424 | 1.00 | 67.56 | D |
| 3554 | CZ | TYR | 693 | 24.699 | 48.402 | -3.160 | 1.00 | 68.22 | D |
| 3555 | OH | TYR | 693 | 23.879 | 48.568 | -2.066 | 1.00 | 68.00 | D |
| 3556 | C | TYR | 693 | 29.597 | 49.128 | -7.320 | 1.00 | 67.31 | D |
| 3557 | O | TYR | 693 | 30.340 | 48.184 | -7.142 | 1.00 | 67.67 | D |
| 3558 | N | ILE | 694 | 29.810 | 49.978 | -8.321 | 1.00 | 67.26 | D |
| 3559 | CA | ILE | 694 | 30.994 | 49.711 | -9.133 | 1.00 | 68.01 | D |
| 3560 | CB | ILE | 694 | 30.998 | 50.412 | -10.506 | 1.00 | 67.45 | D |
| 3561 | CG2 | ILE | 694 | 32.387 | 50.264 | -11.151 | 1.00 | 64.83 | D |
| 3562 | CG1 | ILE | 694 | 29.964 | 49.750 | -11.419 | 1.00 | 66.41 | D |
| 3563 | CD1 | ILE | 694 | 29.460 | 50.643 | -12.516 | 1.00 | 63.94 | D |
| 3564 | C | ILE | 694 | 32.218 | 50.151 | -8.346 | 1.00 | 68.58 | D |
| 3565 | O | ILE | 694 | 33.273 | 49.533 | -8.434 | 1.00 | 67.69 | D |
| 3566 | N | LYS | 695 | 32.073 | 51.216 | -7.566 | 1.00 | 69.57 | D |
| 3567 | CA | LYS | 695 | 33.187 | 51.680 | -6.755 | 1.00 | 70.84 | D |
| 3568 | CB | LYS | 695 | 32.959 | 53.124 | -6.318 | 1.00 | 72.69 | D |
| 3569 | CG | LYS | 695 | 33.000 | 54.142 | -7.451 | 1.00 | 74.90 | D |
| 3570 | CD | LYS | 695 | 32.390 | 55.440 | -6.948 | 1.00 | 76.94 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3571 | CE | LYS | 695 | 32.702 | 56.654 | -7.812 | 1.00 | 78.21 | D |
| 3572 | NZ | LYS | 695 | 32.248 | 57.896 | -7.098 | 1.00 | 78.41 | D |
| 3573 | C | LYS | 695 | 33.293 | 50.757 | -5.539 | 1.00 | 70.27 | D |
| 3574 | O | LYS | 695 | 34.367 | 50.579 | -4.967 | 1.00 | 70.82 | D |
| 3575 | N | GLU | 696 | 32.168 | 50.170 | -5.155 | 1.00 | 69.31 | D |
| 3576 | CA | GLU | 696 | 32.146 | 49.253 | -4.033 | 1.00 | 68.61 | D |
| 3577 | CB | GLU | 696 | 30.702 | 48.845 | -3.749 | 1.00 | 69.60 | D |
| 3578 | CG | GLU | 696 | 30.362 | 48.549 | -2.281 | 1.00 | 71.86 | D |
| 3579 | CD | GLU | 696 | 31.072 | 49.454 | -1.271 | 1.00 | 72.47 | D |
| 3580 | OE1 | GLU | 696 | 30.972 | 50.702 | -1.370 | 1.00 | 73.30 | D |
| 3581 | OE2 | GLU | 696 | 31.730 | 48.892 | -0.364 | 1.00 | 72.64 | D |
| 3582 | C | GLU | 696 | 32.998 | 48.053 | -4.456 | 1.00 | 68.13 | D |
| 3583 | O | GLU | 696 | 33.702 | 47.452 | -3.635 | 1.00 | 68.67 | D |
| 3584 | N | LEU | 697 | 32.942 | 47.722 | -5.747 | 1.00 | 66.69 | D |
| 3585 | CA | LEU | 697 | 33.730 | 46.618 | -6.278 | 1.00 | 64.43 | D |
| 3586 | CB | LEU | 697 | 33.386 | 46.321 | -7.745 | 1.00 | 61.53 | D |
| 3587 | CG | LEU | 697 | 34.306 | 45.230 | -8.301 | 1.00 | 60.09 | D |
| 3588 | CD1 | LEU | 697 | 34.268 | 44.066 | -7.340 | 1.00 | 58.33 | D |
| 3589 | CD2 | LEU | 697 | 33.902 | 44.789 | -9.690 | 1.00 | 58.82 | D |
| 3590 | C | LEU | 697 | 35.165 | 47.078 | -6.190 | 1.00 | 64.59 | D |
| 3591 | O | LEU | 697 | 36.064 | 46.325 | -5.824 | 1.00 | 65.27 | D |
| 3592 | N | GLY | 698 | 35.363 | 48.345 | -6.518 | 1.00 | 65.37 | D |
| 3593 | CA | GLY | 698 | 36.689 | 48.930 | -6.489 | 1.00 | 66.92 | D |
| 3594 | C | GLY | 698 | 37.407 | 48.840 | -5.154 | 1.00 | 67.72 | D |
| 3595 | O | GLY | 698 | 38.576 | 48.466 | -5.112 | 1.00 | 66.59 | D |
| 3596 | N | LYS | 699 | 36.716 | 49.198 | -4.072 | 1.00 | 69.34 | D |
| 3597 | CA | LYS | 699 | 37.292 | 49.149 | -2.725 | 1.00 | 70.05 | D |
| 3598 | CB | LYS | 699 | 36.300 | 49.663 | -1.706 | 1.00 | 70.00 | D |
| 3599 | CG | LYS | 699 | 35.875 | 51.067 | -1.896 | 1.00 | 71.97 | D |
| 3600 | CD | LYS | 699 | 34.768 | 51.376 | -0.924 | 1.00 | 72.69 | D |
| 3601 | CE | LYS | 699 | 34.729 | 52.853 | -0.670 | 1.00 | 74.06 | D |
| 3602 | NZ | LYS | 699 | 33.664 | 53.216 | 0.290 | 1.00 | 77.09 | D |
| 3603 | C | LYS | 699 | 37.630 | 47.721 | -2.332 | 1.00 | 71.03 | D |
| 3604 | O | LYS | 699 | 38.694 | 47.449 | -1.771 | 1.00 | 70.60 | D |
| 3605 | N | ALA | 700 | 36.690 | 46.819 | -2.591 | 1.00 | 71.91 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3606 | CA | ALA | 700 | 36.875 | 45.422 | -2.268 | 1.00 | 72.27 | D |
| 3607 | CB | ALA | 700 | 35.713 | 44.601 | -2.790 | 1.00 | 71.48 | D |
| 3608 | C | ALA | 700 | 38.171 | 45.004 | -2.938 | 1.00 | 73.71 | D |
| 3609 | O | ALA | 700 | 38.994 | 44.321 | -2.333 | 1.00 | 73.83 | D |
| 3610 | N | ILE | 701 | 38.351 | 45.434 | -4.184 | 1.00 | 75.21 | D |
| 3611 | CA | ILE | 701 | 39.564 | 45.110 | -4.927 | 1.00 | 77.37 | D |
| 3612 | CB | ILE | 701 | 39.509 | 45.654 | -6.370 | 1.00 | 76.34 | D |
| 3613 | CG2 | ILE | 701 | 40.884 | 45.535 | -7.015 | 1.00 | 74.87 | D |
| 3614 | CG1 | ILE | 701 | 38.471 | 44.881 | -7.185 | 1.00 | 76.01 | D |
| 3615 | CD1 | ILE | 701 | 38.142 | 45.515 | -8.531 | 1.00 | 75.37 | D |
| 3616 | C | ILE | 701 | 40.832 | 45.656 | -4.255 | 1.00 | 79.07 | D |
| 3617 | O | ILE | 701 | 41.787 | 44.909 | -4.047 | 1.00 | 79.07 | D |
| 3618 | N | VAL | 702 | 40.846 | 46.952 | -3.919 | 1.00 | 81.82 | D |
| 3619 | CA | VAL | 702 | 42.015 | 47.567 | -3.280 | 1.00 | 84.95 | D |
| 3620 | CB | VAL | 702 | 41.891 | 49.117 | -3.042 | 1.00 | 83.97 | D |
| 3621 | CG1 | VAL | 702 | 41.611 | 49.843 | -4.326 | 1.00 | 82.62 | D |
| 3622 | CG2 | VAL | 702 | 40.838 | 49.409 | -1.989 | 1.00 | 84.13 | D |
| 3623 | C | VAL | 702 | 42.358 | 47.002 | -1.914 | 1.00 | 87.59 | D |
| 3624 | O | VAL | 702 | 43.524 | 46.983 | -1.539 | 1.00 | 88.96 | D |
| 3625 | N | LYS | 703 | 41.356 | 46.562 | -1.157 | 1.00 | 89.94 | D |
| 3626 | CA | LYS | 703 | 41.632 | 46.050 | 0.176 | 1.00 | 93.02 | D |
| 3627 | CB | LYS | 703 | 40.491 | 46.447 | 1.146 | 1.00 | 92.54 | D |
| 3628 | CG | LYS | 703 | 39.544 | 45.321 | 1.640 | 1.00 | 92.88 | D |
| 3629 | CD | LYS | 703 | 38.695 | 45.782 | 2.857 | 1.00 | 93.01 | D |
| 3630 | CE | LYS | 703 | 38.111 | 44.620 | 3.689 | 1.00 | 92.79 | D |
| 3631 | NZ | LYS | 703 | 37.288 | 45.073 | 4.865 | 1.00 | 91.91 | D |
| 3632 | C | LYS | 703 | 41.898 | 44.549 | 0.243 | 1.00 | 95.67 | D |
| 3633 | O | LYS | 703 | 41.770 | 43.948 | 1.301 | 1.00 | 96.69 | D |
| 3634 | N | ARG | 704 | 42.300 | 43.942 | -0.870 | 1.00 | 98.62 | D |
| 3635 | CA | ARG | 704 | 42.564 | 42.501 | -0.878 | 1.00 | 101.79 | D |
| 3636 | CB | ARG | 704 | 41.570 | 41.781 | -1.805 | 1.00 | 101.22 | D |
| 3637 | CG | ARG | 704 | 41.793 | 40.262 | -1.931 | 1.00 | 102.41 | D |
| 3638 | CD | ARG | 704 | 41.167 | 39.671 | -3.209 | 1.00 | 102.72 | D |
| 3639 | NE | ARG | 704 | 42.151 | 39.345 | -4.247 | 1.00 | 103.97 | D |
| 3640 | CZ | ARG | 704 | 42.771 | 38.170 | -4.365 | 1.00 | 104.85 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3641 | NH1 | ARG | 704 | 42.519 | 37.190 | -3.505 | 1.00 | 105.29 | D |
| 3642 | NH2 | ARG | 704 | 43.647 | 37.972 | -5.346 | 1.00 | 104.19 | D |
| 3643 | C | ARG | 704 | 43.962 | 42.238 | -1.396 | 1.00 | 104.43 | D |
| 3644 | O | ARG | 704 | 44.690 | 41.370 | -0.910 | 1.00 | 104.44 | D |
| 3645 | N | GLU | 705 | 44.303 | 43.031 | -2.403 | 1.00 | 107.72 | D |
| 3646 | CA | GLU | 705 | 45.556 | 42.951 | -3.126 | 1.00 | 110.81 | D |
| 3647 | CB | GLU | 705 | 45.508 | 43.963 | -4.280 | 1.00 | 111.68 | D |
| 3648 | CG | GLU | 705 | 45.679 | 43.371 | -5.688 | 1.00 | 113.18 | D |
| 3649 | CD | GLU | 705 | 44.861 | 42.108 | -5.932 | 1.00 | 115.18 | D |
| 3650 | OE1 | GLU | 705 | 43.628 | 42.131 | -5.713 | 1.00 | 116.70 | D |
| 3651 | OE2 | GLU | 705 | 45.454 | 41.089 | -6.355 | 1.00 | 116.07 | D |
| 3652 | C | GLU | 705 | 46.853 | 43.097 | -2.338 | 1.00 | 112.03 | D |
| 3653 | O | GLU | 705 | 47.368 | 42.128 | -1.781 | 1.00 | 112.47 | D |
| 3654 | N | GLY | 706 | 47.378 | 44.313 | -2.286 | 1.00 | 113.33 | D |
| 3655 | CA | GLY | 706 | 48.642 | 44.519 | -1.616 | 1.00 | 115.12 | D |
| 3656 | C | GLY | 706 | 49.598 | 44.812 | -2.756 | 1.00 | 116.18 | D |
| 3657 | O | GLY | 706 | 50.261 | 43.918 | -3.292 | 1.00 | 116.43 | D |
| 3658 | N | ASN | 707 | 49.609 | 46.086 | -3.131 | 1.00 | 116.60 | D |
| 3659 | CA | ASN | 707 | 50.415 | 46.664 | -4.208 | 1.00 | 116.29 | D |
| 3660 | CB | ASN | 707 | 50.762 | 45.618 | -5.279 | 1.00 | 116.94 | D |
| 3661 | CG | ASN | 707 | 52.236 | 45.209 | -5.260 | 1.00 | 117.97 | D |
| 3662 | OD1 | ASN | 707 | 52.875 | 45.106 | -6.312 | 1.00 | 118.42 | D |
| 3663 | ND2 | ASN | 707 | 52.775 | 44.960 | -4.069 | 1.00 | 118.55 | D |
| 3664 | C | ASN | 707 | 49.514 | 47.771 | -4.776 | 1.00 | 115.24 | D |
| 3665 | O | ASN | 707 | 48.949 | 48.546 | -4.003 | 1.00 | 115.85 | D |
| 3666 | N | SER | 708 | 49.359 | 47.857 | -6.093 | 1.00 | 113.05 | D |
| 3667 | CA | SER | 708 | 48.501 | 48.896 | -6.666 | 1.00 | 111.02 | D |
| 3668 | CB | SER | 708 | 48.887 | 50.285 | -6.133 | 1.00 | 111.30 | D |
| 3669 | OG | SER | 708 | 48.094 | 50.649 | -5.015 | 1.00 | 110.98 | D |
| 3670 | C | SER | 708 | 48.501 | 48.939 | -8.184 | 1.00 | 109.52 | D |
| 3671 | O | SER | 708 | 47.654 | 49.603 | -8.788 | 1.00 | 109.82 | D |
| 3672 | N | SER | 709 | 49.454 | 48.266 | -8.813 | 1.00 | 106.92 | D |
| 3673 | CA | SER | 709 | 49.455 | 48.261 | -10.267 | 1.00 | 104.39 | D |
| 3674 | CB | SER | 709 | 50.868 | 48.082 | -10.827 | 1.00 | 104.45 | D |
| 3675 | OG | SER | 709 | 51.334 | 46.758 | -10.667 | 1.00 | 104.39 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3676 | C | SER | 709 | 48.564 | 47.098 | -10.668 | 1.00 | 102.82 | D |
| 3677 | O | SER | 709 | 47.962 | 47.094 | -11.744 | 1.00 | 102.65 | D |
| 3678 | N | GLN | 710 | 48.465 | 46.108 | -9.786 | 1.00 | 100.52 | D |
| 3679 | CA | GLN | 710 | 47.622 | 44.966 | -10.069 | 1.00 | 97.80 | D |
| 3680 | CB | GLN | 710 | 48.159 | 43.697 | -9.379 | 1.00 | 100.41 | D |
| 3681 | CG | GLN | 710 | 48.133 | 43.680 | -7.855 | 1.00 | 104.43 | D |
| 3682 | CD | GLN | 710 | 48.672 | 44.953 | -7.233 | 1.00 | 106.82 | D |
| 3683 | OE1 | GLN | 710 | 49.775 | 45.397 | -7.559 | 1.00 | 108.23 | D |
| 3684 | NE2 | GLN | 710 | 47.898 | 45.543 | -6.329 | 1.00 | 108.12 | D |
| 3685 | C | GLN | 710 | 46.203 | 45.298 | -9.622 | 1.00 | 94.01 | D |
| 3686 | O | GLN | 710 | 45.322 | 44.442 | -9.640 | 1.00 | 93.94 | D |
| 3687 | N | ASN | 711 | 45.978 | 46.553 | -9.240 | 1.00 | 89.32 | D |
| 3688 | CA | ASN | 711 | 44.640 | 46.949 | -8.823 | 1.00 | 84.80 | D |
| 3689 | CB | ASN | 711 | 44.688 | 48.108 | -7.839 | 1.00 | 84.11 | D |
| 3690 | CG | ASN | 711 | 44.719 | 47.619 | -6.411 | 1.00 | 84.22 | D |
| 3691 | OD1 | ASN | 711 | 44.669 | 48.406 | -5.474 | 1.00 | 84.93 | D |
| 3692 | ND2 | ASN | 711 | 44.802 | 46.301 | -6.237 | 1.00 | 83.76 | D |
| 3693 | C | ASN | 711 1 | 43.714 | 47.252 | -9.981 | 1.00 | 81.96 | D |
| 3694 | O | ASN | 711 | 42.601 | 46.735 | -10.017 | 1.00 | 81.02 | D |
| 3695 | N | TRP | 712 | 44.139 | 48.079 | -10.930 | 1.00 | 78.84 | D |
| 3696 | CA | TRP | 712 | 43.273 | 48.298 | -12.073 | 1.00 | 75.28 | D |
| 3697 | CB | TRP | 712 | 43.780 | 49.379 | -12.988 | 1.00 | 74.46 | D |
| 3698 | CG | TRP | 712 | 43.507 | 50.684 | -12.489 | 1.00 | 73.88 | D |
| 3699 | CD2 | TRP | 712 | 42.538 | 51.601 | -12.996 | 1.00 | 74.08 | D |
| 3700 | CE2 | TRP | 712 | 42.647 | 52.778 | -12.231 | 1.00 | 73.76 | D |
| 3701 | CE3 | TRP | 712 | 41.587 | 51.548 | -14.032 | 1.00 | 74.01 | D |
| 3702 | CD1 | TRP | 712 | 44.137 | 51.304 | -11.464 | 1.00 | 73.42 | D |
| 3703 | NE1 | TRP | 712 | 43.631 | 52.565 | -11.297 | 1.00 | 73.37 | D |
| 3704 | CZ2 | TRP | 712 | 41.841 | 53.901 | -12.456 | 1.00 | 73.06 | D |
| 3705 | CZ3 | TRP | 712 | 40.780 | 52.663 | -14.258 | 1.00 | 72.71 | D |
| 3706 | CH2 | TRP | 712 | 40.918 | 53.828 | -13.474 | 1.00 | 72.39 | D |
| 3707 | C | TRP | 712 | 43.287 | 47.025 | -12.863 | 1.00 | 74.11 | D |
| 3708 | O | TRP | 712 | 42.325 | 46.687 | -13.535 | 1.00 | 74.07 | D |
| 3709 | N | GLN | 713 | 44.388 | 46.304 | -12.817 | 1.00 | 72.50 | D |
| 3710 | CA | GLN | 713 | 44.379 | 45.095 | -13.582 | 1.00 | 71.13 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3711 | CB | GLN | 713 | 45.753 | 44.459 | -13.606 | 1.00 | 72.88 | D |
| 3712 | CG | GLN | 713 | 45.816 | 43.299 | -14.564 | 1.00 | 73.89 | D |
| 3713 | CD | GLN | 713 | 47.108 | 42.544 | -14.428 | 1.00 | 75.34 | D |
| 3714 | OE1 | GLN | 713 | 47.438 | 41.708 | -15.269 | 1.00 | 76.79 | D |
| 3715 | NE2 | GLN | 713 | 47.847 | 42.826 | -13.359 | 1.00 | 75.96 | D |
| 3716 | C | GLN | 713 | 43.349 | 44.128 | -13.016 | 1.00 | 69.75 | D |
| 3717 | O | GLN | 713 | 42.760 | 43.357 | -13.764 | 1.00 | 69.68 | D |
| 3718 | N | ARG | 714 | 43.125 | 44.181 | -11.701 | 1.00 | 68.00 | D |
| 3719 | CA | ARG | 714 | 42.162 | 43.294 | -11.021 | 1.00 | 66.56 | D |
| 3720 | CB | ARG | 714 | 42.267 | 43.521 | -9.508 | 1.00 | 66.48 | D |
| 3721 | CG | ARG | 714 | 41.495 | 42.575 | -8.623 | 1.00 | 64.93 | D |
| 3722 | CD | ARG | 714 | 42.015 | 41.157 | -8.695 | 1.00 | 64.89 | D |
| 3723 | NE | ARG | 714 | 41.283 | 40.327 | -7.752 | 1.00 | 64.24 | D |
| 3724 | CZ | ARG | 714 | 41.209 | 39.007 | -7.806 | 1.00 | 64.10 | D |
| 3725 | NH1 | ARG | 714 | 41.824 | 38.344 | -8.770 | 1.00 | 63.98 | D |
| 3726 | NH2 | ARG | 714 | 40.496 | 38.351 | -6.906 | 1.00 | 64.35 | D |
| 3727 | C | ARG | 714 | 40.757 | 43.636 | -11.510 | 1.00 | 65.72 | D |
| 3728 | O | ARG | 714 | 40.010 | 42.788 | -12.009 | 1.00 | 64.06 | D |
| 3729 | N | PHE | 715 | 40.422 | 44.905 | -11.342 | 1.00 | 65.51 | D |
| 3730 | CA | PHE | 715 | 39.158 | 45.457 | -11.757 | 1.00 | 66.56 | D |
| 3731 | CB | PHE | 715 | 39.241 | 46.968 | -11.699 | 1.00 | 65.63 | D |
| 3732 | CG | PHE | 715 | 37.954 | 47.644 | -11.950 | 1.00 | 66.28 | D |
| 3733 | CD1 | PHE | 715 | 36.831 | 47.302 | -11.209 | 1.00 | 67.11 | D |
| 3734 | CD2 | PHE | 715 | 37.858 | 48.655 | -12.890 | 1.00 | 66.76 | D |
| 3735 | CE1 | PHE | 715 | 35.629 | 47.956 | -11.397 | 1.00 | 66.49 | D |
| 3736 | CE2 | PHE | 715 | 36.650 | 49.324 | -13.090 | 1.00 | 66.75 | D |
| 3737 | CZ | PHE | 715 | 35.535 | 48.974 | -12.339 | 1.00 | 66.22 | D |
| 3738 | C | PHE | 715 | 38.876 | 45.034 | -13.182 | 1.00 | 67.61 | D |
| 3739 | O | PHE | 715 | 37.832 | 44.448 | -13.488 | 1.00 | 69.43 | D |
| 3740 | N | TYR | 716 | 39.818 | 45.349 | -14.056 | 1.00 | 67.76 | D |
| 3741 | CA | TYR | 716 | 39.691 | 45.012 | -15.452 | 1.00 | 67.50 | D |
| 3742 | CB | TYR | 716 | 40.993 | 45.341 | -16.172 | 1.00 | 68.51 | D |
| 3743 | CG | TYR | 716 | 40.999 | 44.951 | -17.618 | 1.00 | 69.44 | D |
| 3744 | CD1 | TYR | 716 | 40.438 | 45.773 | -18.594 | 1.00 | 69.90 | D |
| 3745 | CE1 | TYR | 716 | 40.411 1 | 45.381 | -19.929 | 1.00 | 71.02 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3746 | CD2 | TYR | 716 | 41.538 | 43.733 | -18.006 | 1.00 | 70.06 | D |
| 3747 | CE2 | TYR | 716 | 41.516 | 43.332 | -19.321 | 1.00 | 71.47 | D |
| 3748 | CZ | TYR | 716 | 40.953 | 44.155 | -20.282 | 1.00 | 71.73 | D |
| 3749 | OH | TYR | 716 | 40.934 | 43.726 | -21.590 | 1.00 | 73.37 | D |
| 3750 | C | TYR | 716 | 39.327 | 43.532 | -15.592 | 1.00 | 67.55 | D |
| 3751 | O | TYR | 716 | 38.400 | 43.191 | -16.321 | 1.00 | 68.03 | D |
| 3752 | N | GLN | 717 | 40.020 | 42.655 | -14.879 | 1.00 | 67.25 | D |
| 3753 | CA | GLN | 717 | 39.711 | 41.230 | -14.973 | 1.00 | 68.01 | D |
| 3754 | CB | GLN | 717 | 40.794 | 40.384 | -14.327 | 1.00 | 69.66 | D |
| 3755 | CG | GLN | 717 | 42.189 | 40.709 | -14.740 | 1.00 | 72.77 | D |
| 3756 | CD | GLN | 717 | 43.173 | 39.799 | -14.052 | 1.00 | 75.40 | D |
| 3757 | OE1 | GLN | 717 | 43.559 | 40.028 | -12.899 | 1.00 | 76.60 | D |
| 3758 | NE2 | GLN | 717 | 43.566 | 38.738 | -14.744 | 1.00 | 76.67 | D |
| 3759 | C | GLN | 717 | 38.387 | 40.863 | -14.307 | 1.00 | 67.82 | D |
| 3760 | O | GLN | 717 | 37.608 | 40.067 | -14.848 | 1.00 | 67.36 | D |
| 3761 | N | LEU | 718 | 38.132 | 41.405 | -13.120 | 1.00 | 66.52 | D |
| 3762 | CA | LEU | 718 | 36.879 | 41.066 | -12.470 | 1.00 | 64.77 | D |
| 3763 | CB | LEU | 718 | 36.784 | 41.711 | -11.078 | 1.00 | 62.27 | D |
| 3764 | CG | LEU | 718 | 37.739 | 41.100 | -10.040 | 1.00 | 60.26 | D |
| 3765 | CD1 | LEU | 718 | 37.496 | 41.700 | -8.674 | 1.00 | 58.48 | D |
| 3766 | CD2 | LEU | 718 | 37.529 | 39.596 | -9.976 | 1.00 | 60.78 | D |
| 3767 | C | LEU | 718 | 35.731 | 41.493 | -13.390 | 1.00 | 64.36 | D |
| 3768 | O | LEU | 718 | 34.859 | 40.686 | -13.713 | 1.00 | 63.38 | D |
| 3769 | N | THR | 719 | 35.760 | 42.740 | -13.855 | 1.00 | 64.39 | D |
| 3770 | CA | THR | 719 | 34.704 | 43.227 | -14.738 | 1.00 | 65.59 | D |
| 3771 | CB | THR | 719 | 34.833 | 44.729 | -14.972 | 1.00 | 64.69 | D |
| 3772 | OG1 | THR | 719 | 36.133 | 45.020 | -15.505 | 1.00 | 63.56 | D |
| 3773 | CG2 | THR | 719 | 34.637 | 45.477 | -13.660 | 1.00 | 64.71 | D |
| 3774 | C | THR | 719 | 34.648 | 42.507 | -16.090 | 1.00 | 66.44 | D |
| 3775 | O | THR | 719 | 33.690 | 42.674 | -16.853 | 1.00 | 66.27 | D |
| 3776 | N | LYS | 720 | 35.666 | 41.709 | -16.396 | 1.00 | 67.02 | D |
| 3777 | CA | LYS | 720 | 35.650 | 40.980 | -17.654 | 1.00 | 66.94 | D |
| 3778 | CB | LYS | 720 | 37.048 | 40.497 | -18.036 | 1.00 | 69.93 | D |
| 3779 | CG | LYS | 720 | 37.235 | 40.307 | -19.547 | 1.00 | 74.14 | D |
| 3780 | CD | LYS | 720 | 37.418 | 41.656 | -20.262 | 1.00 | 77.44 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3781 | CE | LYS | 720 | 37.399 | 41.534 | -21.799 | 1.00 | 79.51 | D |
| 3782 | NZ | LYS | 720 | 37.765 | 42.813 | -22.503 | 1.00 | 80.62 | D |
| 3783 | C | LYS | 720 | 34.741 | 39.789 | -17.405 | 1.00 | 65.29 | D |
| 3784 | O | LYS | 720 | 33.925 | 39.420 | -18.247 | 1.00 | 65.38 | D |
| 3785 | N | LEU | 721 | 34.881 | 39.207 | -16.218 | 1.00 | 63.82 | D |
| 3786 | CA | LEU | 721 | 34.073 | 38.068 | -15.818 | 1.00 | 62.57 | D |
| 3787 | CB | LEU | 721 | 34.473 | 37.616 | -14.420 | 1.00 | 60.12 | D |
| 3788 | CG | LEU | 721 | 34.373 | 36.116 | -14.199 | 1.00 | 60.11 | D |
| 3789 | CD1 | LEU | 721 | 34.104 | 35.890 | -12.732 | 1.00 | 59.49 | D |
| 3790 | CD2 | LEU | 721 | 33.261 | 35.499 | -15.051 | 1.00 | 59.33 | D |
| 3791 | C | LEU | 721 | 32.596 | 38.467 | -15.823 | 1.00 | 62.94 | D |
| 3792 | O | LEU | 721 | 31.732 | 37.701 | -16.263 | 1.00 | 63.51 | D |
| 3793 | N | LEU | 722 | 32.315 | 39.666 | -15.324 | 1.00 | 62.68 | D |
| 3794 | CA | LEU | 722 | 30.951 | 40.170 | -15.287 | 1.00 | 62.47 | D |
| 3795 | CB | LEU | 722 | 30.926 | 41.583 | -14.698 | 1.00 | 61.57 | D |
| 3796 | CG | LEU | 722 | 31.240 | 41.519 | -13.203 | 1.00 | 60.98 | D |
| 3797 | CD1 | LEU | 722 | 31.132 | 42.877 | -12.563 | 1.00 | 59.96 | D |
| 3798 | CD2 | LEU | 722 | 30.267 | 40.535 | -12.553 | 1.00 | 59.00 | D |
| 3799 | C | LEU | 722 | 30.347 | 40.167 | -16.675 | 1.00 | 63.61 | D |
| 3800 | O | LEU | 722 | 29.330 | 39.525 | -16.898 | 1.00 | 64.24 | D |
| 3801 | N | ASP | 723 | 30.983 | 40.866 | -17.609 | 1.00 | 63.93 | D |
| 3802 | CA | ASP | 723 | 30.493 | 40.952 | -18.983 | 1.00 | 63.89 | D |
| 3803 | CB | ASP | 723 | 31.462 | 41.748 | -19.844 | 1.00 | 64.74 | D |
| 3804 | CG | ASP | 723 | 31.485 | 43.200 | -19.481 | 1.00 | 66.67 | D |
| 3805 | OD1 | ASP | 723 | 30.530 | 43.657 | -18.817 | 1.00 | 68.28 | D |
| 3806 | OD2 | ASP | 723 | 32.445 | 43.890 | -19.868 | 1.00 | 65.20 | D |
| 3807 | C | ASP | 723 | 30.280 | 39.608 | -19.652 | 1.00 | 64.20 | D |
| 3808 | O | ASP | 723 | 29.408 | 39.456 | -20.509 | 1.00 | 63.99 | D |
| 3809 | N | SER | 724 | 31.089 | 38.635 | -19.263 | 1.00 | 64.29 | D |
| 3810 | CA | SER | 724 | 31.009 | 37.311 | -19.855 | 1.00 | 64.18 | D |
| 3811 | CB | SER | 724 | 32.353 | 36.610 | -19.706 | 1.00 | 64.03 | D |
| 3812 | OG | SER | 724 | 32.261 | 35.556 | -18.769 | 1.00 | 65.88 | D |
| 3813 | C | SER | 724 | 29.912 | 36.478 | -19.218 | 1.00 | 63.99 | D |
| 3814 | O | SER | 724 | 29.685 | 35.322 | -19.585 | 1.00 | 63.80 | D |
| 3815 | N | MET | 725 | 29.234 | 37.086 | -18.256 | 1.00 | 63.58 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3816 | CA | MET | 725 | 28.157 | 36.433 | -17.533 | 1.00 | 62.50 | D |
| 3817 | CB | MET | 725 | 27.944 | 37.184 | -16.225 | 1.00 | 60.73 | D |
| 3818 | CG | MET | 725 | 27.507 | 36.347 | -15.077 | 1.00 | 59.86 | D |
| 3819 | SD | MET | 725 | 28.599 | 34.988 | -14.747 | 1.00 | 57.82 | D |
| 3820 | CE | MET | 725 | 27.340 | 33.823 | -14.381 | 1.00 | 56.09 | D |
| 3821 | C | MET | 725 | 26.911 | 36.479 | -18.410 | 1.00 | 62.36 | D |
| 3822 | O | MET | 725 | 26.014 | 35.645 | -18.289 | 1.00 | 61.95 | D |
| 3823 | N | HIS | 726 | 26.867 | 37.474 | -19.294 | 1.00 | 62.70 | D |
| 3824 | CA | HIS | 726 | 25.754 | 37.598 | -20.213 | 1.00 | 63.08 | D |
| 3825 | CB | HIS | 726 | 25.856 | 38.883 | -21.038 | 1.00 | 60.89 | D |
| 3826 | CG | HIS | 726 | 25.427 | 40.114 | -20.295 | 1.00 | 59.03 | D |
| 3827 | CD2 | HIS | 726 | 24.277 | 40.399 | -19.634 | 1.00 | 58.37 | D |
| 3828 | ND1 | HIS | 726 | 26.228 | 41.224 | -20.171 | 1.00 | 58.24 | D |
| 3829 | CE1 | HIS | 726 | 25.595 | 42.144 | -19.460 | 1.00 | 57.37 | D |
| 3830 | NE2 | HIS | 726 | 24.413 | 41.669 | -19.126 | 1.00 | 58.49 | D |
| 3831 | C | HIS | 726 | 25.850 | 36.383 | -21.115 | 1.00 | 64.70 | D |
| 3832 | O | HIS | 726 | 24.873 | 35.676 | -21.303 | 1.00 | 65.01 | D |
| 3833 | N | GLU | 727 | 27.031 | 36.091 | -21.641 | 1.00 | 67.88 | D |
| 3834 | CA | GLU | 727 | 27.092 | 34.931 | -22.504 | 1.00 | 70.51 | D |
| 3835 | CB | GLU | 727 | 28.390 | 34.864 | -23.293 | 1.00 | 72.69 | D |
| 3836 | CG | GLU | 727 | 28.196 | 34.036 | -24.552 | 1.00 | 77.47 | D |
| 3837 | CD | GLU | 727 | 29.485 | 33.473 | -25.082 | 1.00 | 82.02 | D |
| 3838 | OE1 | GLU | 727 | 30.495 | 34.219 | -25.072 | 1.00 | 84.51 | D |
| 3839 | OE2 | GLU | 727 | 29.483 | 32.291 | -25.512 | 1.00 | 83.29 | D |
| 3840 | C | GLU | 727 | 26.842 | 33.585 | -21.825 | 1.00 | 71.14 | D |
| 3841 | O | GLU | 727 | 26.305 | 32.685 | -22.477 | 1.00 | 71.67 | D |
| 3842 | N | VAL | 728 | 27.211 | 33.395 | -20.557 | 1.00 | 71.39 | D |
| 3843 | CA | VAL | 728 | 26.890 | 32.080 | -19.992 | 1.00 | 71.03 | D |
| 3844 | CB | VAL | 728 | 27.794 | 31.638 | -18.750 | 1.00 | 70.84 | D |
| 3845 | CG1 | VAL | 728 | 28.705 | 32.749 | -18.298 | 1.00 | 70.06 | D |
| 3846 | CG2 | VAL | 728 | 26.921 | 31.121 | -17.609 | 1.00 | 69.97 | D |
| 3847 | C | VAL | 728 | 25.397 | 32.019 | -19.657 | 1.00 | 70.61 | D |
| 3848 | O | VAL | 728 | 24.783 | 30.964 | -19.800 | 1.00 | 70.41 | D |
| 3849 | N | VAL | 729 | 24.806 | 33.146 | -19.252 | 1.00 | 71.03 | D |
| 3850 | CA | VAL | 729 | 23.368 | 33.165 | -18.948 | 1.00 | 72.03 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3851 | CB | VAL | 729 | 22.929 | 34.527 | -18.317 | 1.00 | 72.41 | D |
| 3852 | CG1 | VAL | 729 | 21.547 | 34.949 | -18.822 | 1.00 | 72.84 | D |
| 3853 | CG2 | VAL | 729 | 22.873 | 34.382 | -16.811 | 1.00 | 72.63 | D |
| 3854 | C | VAL | 729 | 22.560 | 32.872 | -20.216 | 1.00 | 72.63 | D |
| 3855 | O | VAL | 729 | 21.558 | 32.166 | -20.173 | 1.00 | 72.16 | D |
| 3856 | N | GLU | 730 | 22.996 | 33.403 | -21.351 | 1.00 | 74.69 | D |
| 3857 | CA | GLU | 730 | 22.289 | 33.131 | -22.592 | 1.00 | 77.21 | D |
| 3858 | CB | GLU | 730 | 23.013 | 33.756 | -23.783 | 1.00 | 78.90 | D |
| 3859 | CG | GLU | 730 | 22.218 | 33.663 | -25.068 | 1.00 | 83.32 | D |
| 3860 | CD | GLU | 730 | 22.836 | 34.458 | -26.198 | 1.00 | 86.77 | D |
| 3861 | OE1 | GLU | 730 | 23.326 | 35.580 | -25.931 | 1.00 | 88.49 | D |
| 3862 | OE2 | GLU | 730 | 22.811 | 33.962 | -27.351 | 1.00 | 88.56 | D |
| 3863 | C | GLU | 730 | 22.195 | 31.610 | -22.771 | 1.00 | 77.66 | D |
| 3864 | O | GLU | 730 | 21.104 | 31.057 | -22.852 | 1.00 | 77.32 | D |
| 3865 | N | ASN | 731 | 23.342 | 30.934 | -22.801 | 1.00 | 78.69 | D |
| 3866 | CA | ASN | 731 | 23.362 | 29.482 | -22.972 | 1.00 | 79.39 | D |
| 3867 | CB | ASN | 731 | 24.808 | 29.046 | -23.172 | 1.00 | 80.51 | D |
| 3868 | CG | ASN | 731 | 25.367 | 29.753 | -24.340 | 1.00 | 81.74 | D |
| 3869 | OD1 | ASN | 731 | 26.524 | 29.646 | -24.728 | 1.00 | 83.00 | D |
| 3870 | ND2 | ASN | 731 | 24.483 | 30.534 | -24.946 | 1.00 | 84.04 | D |
| 3871 | C | ASN | 731 | 22.649 | 28.770 | -21.864 | 1.00 | 79.09 | D |
| 3872 | O | ASN | 731 | 22.147 | 27.659 | -22.040 | 1.00 | 78.14 | D |
| 3873 | N | LEU | 732 | 22.550 | 29.441 | -20.731 | 1.00 | 79.35 | D |
| 3874 | CA | LEU | 732 | 21.838 | 28.846 | -19.639 | 1.00 | 79.99 | D |
| 3875 | CB | LEU | 732 | 22.253 | 29.475 | -18.331 | 1.00 | 78.02 | D |
| 3876 | CG | LEU | 732 | 23.448 | 28.662 | -17.877 | 1.00 | 76.80 | D |
| 3877 | CD1 | LEU | 732 | 23.803 | 29.107 | -16.496 | 1.00 | 76.83 | D |
| 3878 | CD2 | LEU | 732 | 23.124 | 27.158 | -17.905 | 1.00 | 76.26 | D |
| 3879 | C | LEU | 732 | 20.349 | 28.977 | -19.856 | 1.00 | 81.89 | D |
| 3880 | O | LEU | 732 | 19.600 | 28.065 | -19.510 | 1.00 | 83.06 | D |
| 3881 | N | LEU | 733 | 19.913 | 30.090 | -20.442 | 1.00 | 83.07 | D |
| 3882 | CA | LEU | 733 | 18.487 | 30.283 | -20.705 | 1.00 | 83.87 | D |
| 3883 | CB | LEU | 733 | 18.172 | 31.759 | -20.959 | 1.00 | 82.93 | D |
| 3884 | CG | LEU | 733 | 18.209 | 32.702 | -19.752 | 1.00 | 82.42 | D |
| 3885 | CD1 | LEU | 733 | 18.026 | 34.120 | -20.244 | 1.00 | 81.62 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3886 | CD2 | LEU | 733 | 17.132 | 32.332 | -18.738 | 1.00 | 81.48 | D |
| 3887 | C | LEU | 733 | 17.976 | 29.439 | -21.873 | 1.00 | 85.02 | D |
| 3888 | O | LEU | 733 | 16.902 | 28.843 | -21.764 | 1.00 | 84.76 | D |
| 3889 | N | ASN | 734 | 18.718 | 29.374 | -22.979 | 1.00 | 86.42 | D |
| 3890 | CA | ASN | 734 | 18.259 | 28.555 | -24.100 | 1.00 | 88.57 | D |
| 3891 | CB | ASN | 734 | 19.306 | 28.424 | -25.236 | 1.00 | 88.99 | D |
| 3892 | CG | ASN | 734 | 19.540 | 29.733 | -26.048 | 1.00 | 90.34 | D |
| 3893 | OD1 | ASN | 734 | 18.745 | 30.685 | -26.026 | 1.00 | 89.17 | D |
| 3894 | ND2 | ASN | 734 | 20.652 | 29.748 | -26.793 | 1.00 | 91.10 | D |
| 3895 | C | ASN | 734 | 17.838 | 27.123 | -23.646 | 1.00 | 90.13 | D |
| 3896 | O | ASN | 734 | 16.761 | 26.706 | -24.049 | 1.00 | 90.87 | D |
| 3897 | N | TYR | 735 | 18.631 | 26.380 | -22.837 | 1.00 | 91.96 | D |
| 3898 | CA | TYR | 735 | 18.235 | 24.990 | -22.412 | 1.00 | 93.75 | D |
| 3899 | CB | TYR | 735 | 19.265 | 24.176 | -21.528 | 1.00 | 96.29 | D |
| 3900 | CG | TYR | 735 | 19.019 | 22.640 | -21.651 | 1.00 | 99.41 | D |
| 3901 | CD1 | TYR | 735 | 19.517 | 21.657 | -20.741 | 1.00 | 101.17 | D |
| 3902 | CE1 | TYR | 735 | 19.286 | 20.262 | -20.977 | 1.00 | 102.99 | D |
| 3903 | CD2 | TYR | 735 | 18.300 | 22.187 | -22.738 | 1.00 | 100.65 | D |
| 3904 | CE2 | TYR | 735 | 18.064 | 20.851 | -22.963 | 1.00 | 101.77 | D |
| 3905 | CZ | TYR | 735 | 18.540 | 19.893 | -22.097 | 1.00 | 103.45 | D |
| 3906 | OH | TYR | 735 | 18.225 | 18.584 | -22.388 | 1.00 | 104.95 | D |
| 3907 | C | TYR | 735 | 17.015 | 25.084 | -21.563 | 1.00 | 93.60 | D |
| 3908 | O | TYR | 735 | 16.179 | 24.194 | -21.555 | 1.00 | 94.18 | D |
| 3909 | N | CYS | 736 | 16.907 | 26.177 | -20.843 | 1.00 | 92.87 | D |
| 3910 | CA | CYS | 736 | 15.803 | 26.297 | -19.942 | 1.00 | 92.13 | D |
| 3911 | CB | CYS | 736 | 16.125 | 27.357 | -18.909 | 1.00 | 91.98 | D |
| 3912 | SG | CYS | 736 | 14.842 | 27.599 | -17.686 | 1.00 | 92.43 | D |
| 3913 | C | CYS | 736 | 14.489 | 26.567 | -20.621 | 1.00 | 91.67 | D |
| 3914 | O | CYS | 736 | 13.510 | 25.862 | -20.381 | 1.00 | 91.52 | D |
| 3915 | N | PHE | 737 | 14.457 | 27.583 | -21.468 | 1.00 | 91.07 | D |
| 3916 | CA | PHE | 737 | 13.220 | 27.872 | -22.154 | 1.00 | 91.08 | D |
| 3917 | CB | PHE | 737 | 13.420 | 29.045 | -23.104 | 1.00 | 89.07 | D |
| 3918 | CG | PHE | 737 | 13.666 | 30.364 | -22.404 | 1.00 | 87.03 | D |
| 3919 | CD1 | PHE | 737 | 13.388 | 30.521 | -21.044 | 1.00 | 85.41 | D |
| 3920 | CD2 | PHE | 737 | 14.114 | 31.465 | -23.125 | 1.00 | 86.11 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3921 | CE1 | PHE | 737 | 13.544 | 31.759 | -20.422 | 1.00 | 84.26 | D |
| 3922 | CE2 | PHE | 737 | 14.274 | 32.706 | -22.512 | 1.00 | 84.63 | D |
| 3923 | CZ | PHE | 737 | 13.991 | 32.854 | -21.158 | 1.00 | 83.57 | D |
| 3924 | C | PHE | 737 | 12.821 | 26.598 | -22.896 | 1.00 | 92.41 | D |
| 3925 | O | PHE | 737 | 11.734 | 26.054 | -22.685 | 1.00 | 92.34 | D |
| 3926 | N | GLN | 738 | 13.730 | 26.120 | -23.740 | 1.00 | 93.76 | D |
| 3927 | CA | GLN | 738 | 13.534 | 24.904 | -24.514 | 1.00 | 95.09 | D |
| 3928 | CB | GLN | 738 | 14.888 | 24.425 | -25.027 | 1.00 | 96.20 | D |
| 3929 | CG | GLN | 738 | 14.873 | 23.403 | -26.136 | 1.00 | 97.39 | D |
| 3930 | CD | GLN | 738 | 16.275 | 23.176 | -26.656 | 1.00 | 98.13 | D |
| 3931 | OE1 | GLN | 738 | 17.001 | 22.299 | -26.178 | 1.00 | 98.35 | D |
| 3932 | NE2 | GLN | 738 | 16.680 | 23.997 | -27.621 | 1.00 | 98.37 | D |
| 3933 | C | GLN | 738 | 12.922 | 23.873 | -23.585 | 1.00 | 95.89 | D |
| 3934 | O | GLN | 738 | 11.711 | 23.743 | -23.530 | 1.00 | 96.25 | D |
| 3935 | N | THR | 739 | 13.750 | 23.155 | -22.837 | 1.00 | 97.17 | D |
| 3936 | CA | THR | 739 | 13.224 | 22.160 | -21.915 | 1.00 | 98.69 | D |
| 3937 | CB | THR | 739 | 14.197 | 21.930 | -20.764 | 1.00 | 98.17 | D |
| 3938 | OG1 | THR | 739 | 14.904 | 23.141 | -20.495 | 1.00 | 98.69 | D |
| 3939 | CG2 | THR | 739 | 15.193 | 20.839 | -21.115 | 1.00 | 98.02 | D |
| 3940 | C | THR | 739 | 11.831 | 22.533 | -21.369 | 1.00 | 100.03 | D |
| 3941 | O | THR | 739 | 10.915 | 21.708 | -21.389 | 1.00 | 100.63 | D |
| 3942 | N | PHE | 740 | 11.654 | 23.771 | -20.921 | 1.00 | 101.15 | D |
| 3943 | CA | PHE | 740 | 10.358 | 24.205 | -20.388 | 1.00 | 102.20 | D |
| 3944 | CB | PHE | 740 | 10.470 | 25.629 | -19.856 | 1.00 | 100.56 | D |
| 3945 | CG | PHE | 740 | 9.179 | 26.181 | -19.325 | 1.00 | 98.93 | D |
| 3946 | CD1 | PHE | 740 | 8.824 | 26.007 | -17.994 | 1.00 | 98.31 | D |
| 3947 | CD2 | PHE | 740 | 8.327 | 26.901 | -20.152 | 1.00 | 97.96 | D |
| 3948 | CE1 | PHE | 740 | 7.647 | 26.542 | -17.487 | 1.00 | 97.83 | D |
| 3949 | CE2 | PHE | 740 | 7.148 | 27.440 | -19.658 | 1.00 | 97.24 | D |
| 3950 | CZ | PHE | 740 | 6.806 | 27.263 | -18.320 | 1.00 | 97.30 | D |
| 3951 | C | PHE | 740 | 9.197 | 24.140 | -21.385 | 1.00 | 104.02 | D |
| 3952 | O | PHE | 740 | 8.043 | 23.978 | -20.987 | 1.00 | 103.68 | D |
| 3953 | N | LEU | 741 | 9.495 | 24.291 | -22.671 | 1.00 | 106.46 | D |
| 3954 | CA | LEU | 741 | 8.459 | 24.238 | -23.696 | 1.00 | 109.10 | D |
| 3955 | CB | LEU | 741 | 8.630 | 25.383 | -24.699 | 1.00 | 108.94 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3956 | CG | LEU | 741 | 8.799 | 26.819 | -24.195 | 1.00 | 108.65 | D |
| 3957 | CD1 | LEU | 741 | 8.890 | 27.741 | -25.405 | 1.00 | 108.53 | D |
| 3958 | CD2 | LEU | 741 | 7.642 | 27.233 | -23.299 | 1.00 | 108.26 | D |
| 3959 | C | LEU | 741 | 8.493 | 22.901 | -24.436 | 1.00 | 111.39 | D |
| 3960 | O | LEU | 741 | 8.385 | 22.853 | -25.666 | 1.00 | 111.44 | D |
| 3961 | N | ASP | 742 | 8.663 | 21.821 | -23.676 | 1.00 | 114.02 | D |
| 3962 | CA | ASP | 742 | 8.684 | 20.465 | -24.222 | 1.00 | 116.52 | D |
| 3963 | CB | ASP | 742 | 10.064 | 20.088 | -24.764 | 1.00 | 117.48 | D |
| 3964 | CG | ASP | 742 | 10.055 | 18.732 | -25.451 | 1.00 | 119.20 | D |
| 3965 | OD1 | ASP | 742 | 11.132 | 18.125 | -25.638 | 1.00 | 119.74 | D |
| 3966 | OD2 | ASP | 742 | 8.949 | 18.278 | -25.818 | 1.00 | 120.17 | D |
| 3967 | C | ASP | 742 | 8.329 | 19.488 | -23.115 | 1.00 | 117.76 | D |
| 3968 | O | ASP | 742 | 9.193 | 19.111 | -22.319 | 1.00 | 117.68 | D |
| 3969 | N | LYS | 743 | 7.073 | 19.060 | -23.048 | 1.00 | 119.41 | D |
| 3970 | CA | LYS | 743 | 6.746 | 18.138 | -21.985 | 1.00 | 120.92 | D |
| 3971 | CB | LYS | 743 | 5.381 | 18.418 | -21.393 | 1.00 | 121.59 | D |
| 3972 | CG | LYS | 743 | 5.537 | 18.534 | -19.898 | 1.00 | 122.24 | D |
| 3973 | CD | LYS | 743 | 4.242 | 18.414 | -19.164 | 1.00 | 122.77 | D |
| 3974 | CE | LYS | 743 | 3.312 | 19.572 | -19.487 | 1.00 | 122.90 | D |
| 3975 | NZ | LYS | 743 | 3.995 | 20.761 | -20.080 | 1.00 | 122.44 | D |
| 3976 | C | LYS | 743 | 6.885 | 16.671 | -22.315 | 1.00 | 121.35 | D |
| 3977 | O | LYS | 743 | 6.565 | 15.808 | -21.495 | 1.00 | 121.83 | D |
| 3978 | N | THR | 744 | 7.360 | 16.393 | -23.523 | 1.00 | 121.55 | D |
| 3979 | CA | THR | 744 | 7.625 | 15.018 | -23.914 | 1.00 | 121.69 | D |
| 3980 | CB | THR | 744 | 8.228 | 14.930 | -25.334 | 1.00 | 122.15 | D |
| 3981 | OG1 | THR | 744 | 8.139 | 16.208 | -25.970 | 1.00 | 122.30 | D |
| 3982 | CG2 | THR | 744 | 7.487 | 13.903 | -26.177 | 1.00 | 122.06 | D |
| 3983 | C | THR | 744 | 8.741 | 14.707 | -22.920 | 1.00 | 121.24 | D |
| 3984 | O | THR | 744 | 8.856 | 13.595 | -22.399 | 1.00 | 120.96 | D |
| 3985 | N | MET | 745 | 9.549 | 15.732 | -22.654 | 1.00 | 120.64 | D |
| 3986 | CA | MET | 745 | 10.672 | 15.622 | -21.737 | 1.00 | 120.07 | D |
| 3987 | CB | MET | 745 | 11.523 | 16.881 | -21.791 | 1.00 | 120.38 | D |
| 3988 | CG | MET | 745 | 12.995 | 16.582 | -21.729 | 1.00 | 120.61 | D |
| 3989 | SD | MET | 745 | 13.895 | 17.821 | -22.630 | 1.00 | 121.79 | D |
| 3990 | CE | MET | 745 | 14.003 | 17.040 | -24.248 | 1.00 | 121.33 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 3991 | C | MET | 745 | 10.191 | 15.375 | -20.318 | 1.00 | 119.16 | D |
| 3992 | O | MET | 745 | 10.806 | 14.616 | -19.571 | 1.00 | 119.47 | D |
| 3993 | N | SER | 746 | 9.109 | 16.041 | -19.942 | 1.00 | 117.72 | D |
| 3994 | CA | SER | 746 | 8.517 | 15.831 | -18.627 | 1.00 | 116.53 | D |
| 3995 | CB | SER | 746 | 8.322 | 14.328 | -18.392 | 1.00 | 116.85 | D |
| 3996 | OG | SER | 746 | 7.499 | 13.735 | -19.384 | 1.00 | 117.32 | D |
| 3997 | C | SER | 746 | 9.189 | 16.409 | -17.376 | 1.00 | 115.35 | D |
| 3998 | O | SER | 746 | 8.905 | 15.921 | -16.282 | 1.00 | 115.42 | D |
| 3999 | N | ILE | 747 | 10.063 | 17.403 | -17.490 | 1.00 | 113.39 | D |
| 4000 | CA | ILE | 747 | 10.676 | 17.964 | -16.278 | 1.00 | 111.23 | D |
| 4001 | CB | ILE | 747 | 12.094 | 18.519 | -16.557 | 1.00 | 111.42 | D |
| 4002 | CG2 | ILE | 747 | 12.783 | 18.904 | -15.243 | 1.00 | 110.85 | D |
| 4003 | CG1 | ILE | 747 | 12.935 | 17.448 | -17.254 | 1.00 | 111.16 | D |
| 4004 | CD1 | ILE | 747 | 14.181 | 17.984 | -17.879 | 1.00 | 111.76 | D |
| 4005 | C | ILE | 747 | 9.747 | 19.085 | -15.788 | 1.00 | 109.87 | D |
| 4006 | O | ILE | 747 | 9.137 | 19.766 | -16.607 | 1.00 | 109.61 | D |
| 4007 | N | GLU | 748 | 9.640 | 19.276 | -14.474 | 1.00 | 108.51 | D |
| 4008 | CA | GLU | 748 | 8.734 | 20.295 | -13.913 | 1.00 | 107.39 | D |
| 4009 | CB | GLU | 748 | 7.711 | 19.600 | -12.988 | 1.00 | 109.23 | D |
| 4010 | CG | GLU | 748 | 6.248 | 19.527 | -13.495 | 1.00 | 111.40 | D |
| 4011 | CD | GLU | 748 | 5.994 | 18.463 | -14.571 | 1.00 | 112.97 | D |
| 4012 | OE1 | GLU | 748 | 6.115 | 17.248 | -14.275 | 1.00 | 113.35 | D |
| 4013 | OE2 | GLU | 748 | 5.662 | 18.848 | -15.719 | 1.00 | 113.44 | D |
| 4014 | C | GLU | 748 | 9.369 | 21.484 | -13.147 | 1.00 | 105.43 | D |
| 4015 | O | GLU | 748 | 10.173 | 21.279 | -12.241 | 1.00 | 104.77 | D |
| 4016 | N | PHE | 749 | 8.982 | 22.716 | -13.505 | 1.00 | 103.47 | D |
| 4017 | CA | PHE | 749 | 9.480 | 23.942 | -12.843 | 1.00 | 101.74 | D |
| 4018 | CB | PHE | 749 | 9.757 | 25.045 | -13.883 | 1.00 | 100.69 | D |
| 4019 | CG | PHE | 749 | 10.476 | 24.565 | -15.115 | 1.00 | 100.55 | D |
| 4020 | CD1 | PHE | 749 | 11.843 | 24.716 | -15.251 | 1.00 | 99.85 | D |
| 4021 | CD2 | PHE | 749 | 9.778 | 23.929 | -16.126 | 1.00 | 100.99 | D |
| 4022 | CE1 | PHE | 749 | 12.479 | 24.254 | -16.373 | 1.00 | 100.32 | D |
| 4023 | CE2 | PHE | 749 | 10.434 | 23.445 | -17.259 | 1.00 | 101.43 | D |
| 4024 | CZ | PHE | 749 | 11.784 | 23.600 | -17.388 | 1.00 | 100.64 | D |
| 4025 | C | PHE | 749 | 8.407 | 24.441 | -11.841 | 1.00 | 100.85 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4026 | O | PHE | 749 | 7.214 | 24.417 | -12.153 | 1.00 | 100.84 | D |
| 4027 | N | PRO | 750 | 8.810 | 24.926 | -10.648 | 1.00 | 99.97 | D |
| 4028 | CD | PRO | 750 | 10.177 | 25.299 | -10.225 | 1.00 | 99.64 | D |
| 4029 | CA | PRO | 750 | 7.833 | 25.398 | -9.656 | 1.00 | 99.71 | D |
| 4030 | CB | PRO | 750 | 8.692 | 25.643 | -8.426 | 1.00 | 98.99 | D |
| 4031 | CG | PRO | 750 | 9.921 | 26.205 | -9.021 | 1.00 | 98.91 | D |
| 4032 | C | PRO | 750 | 7.045 | 26.641 | -10.059 | 1.00 | 99.72 | D |
| 4033 | O | PRO | 750 | 7.082 | 27.064 | -11.209 | 1.00 | 99.97 | D |
| 4034 | N | GLU | 751 | 6.329 | 27.216 | -9.095 | 1.00 | 99.33 | D |
| 4035 | CA | GLU | 751 | 5.531 | 28.418 | -9.330 | 1.00 | 98.80 | D |
| 4036 | CB | GLU | 751 | 4.831 | 28.844 | -8.036 | 1.00 | 99.78 | D |
| 4037 | CG | GLU | 751 | 3.619 | 28.013 | -7.714 | 1.00 | 100.77 | D |
| 4038 | CD | GLU | 751 | 2.597 | 28.070 | -8.830 | 1.00 | 101.15 | D |
| 4039 | OE1 | GLU | 751 | 2.945 | 27.748 | -9.985 | 1.00 | 101.44 | D |
| 4040 | OE2 | GLU | 751 | 1.443 | 28.448 | -8.555 | 1.00 | 101.81 | D |
| 4041 | C | GLU | 751 | 6.397 | 29.560 | -9.814 | 1.00 | 97.74 | D |
| 4042 | O | GLU | 751 | 6.224 | 30.073 | -10.927 | 1.00 | 96.52 | D |
| 4043 | N | MET | 752 | 7.323 | 29.954 | -8.949 | 1.00 | 96.53 | D |
| 4044 | CA | MET | 752 | 8.229 | 31.031 | -9.258 | 1.00 | 95.56 | D |
| 4045 | CB | MET | 752 | 9.331 | 31.122 | -8.192 | 1.00 | 96.57 | D |
| 4046 | CG | MET | 752 | 9.238 | 32.386 | -7.314 | 1.00 | 98.76 | D |
| 4047 | SD | MET | 752 | 8.453 | 33.847 | -8.124 | 1.00 | 100.33 | D |
| 4048 | CE | MET | 752 | 9.799 | 34.664 | -8.854 | 1.00 | 100.14 | D |
| 4049 | C | MET | 752 | 8.815 | 30.868 | -10.663 | 1.00 | 94.50 | D |
| 4050 | O | MET | 752 | 8.251 | 31.409 | -11.607 | 1.00 | 94.59 | D |
| 4051 | N | LEU | 753 | 9.899 | 30.113 | -10.825 | 1.00 | 93.21 | D |
| 4052 | CA | LEU | 753 | 10.528 | 29.942 | -12.149 | 1.00 | 91.78 | D |
| 4053 | CB | LEU | 753 | 11.487 | 28.764 | -12.166 | 1.00 | 90.35 | D |
| 4054 | CG | LEU | 753 | 12.819 | 28.895 | -11.456 | 1.00 | 89.15 | D |
| 4055 | CD1 | LEU | 753 | 12.644 | 29.374 | -10.035 | 1.00 | 88.34 | D |
| 4056 | CD2 | LEU | 753 | 13.457 | 27.539 | -11.491 | 1.00 | 88.65 | D |
| 4057 | C | LEU | 753 | 9.593 | 29.749 | -13.317 | 1.00 | 91.44 | D |
| 4058 | O | LEU | 753 | 9.887 | 30.188 | -14.428 | 1.00 | 91.23 | D |
| 4059 | N | ALA | 754 | 8.487 | 29.066 | -13.082 | 1.00 | 91.90 | D |
| 4060 | CA | ALA | 754 | 7.531 | 28.820 | -14.147 | 1.00 | 93.06 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4061 | CB | ALA | 754 | 6.399 | 27.933 | -13.633 | 1.00 | 92.79 | D |
| 4062 | C | ALA | 754 | 6.964 | 30.111 | -14.759 | 1.00 | 93.82 | D |
| 4063 | O | ALA | 754 | 6.846 | 30.200 | -15.981 | 1.00 | 93.25 | D |
| 4064 | N | GLU | 755 | 6.633 | 31.101 | -13.914 | 1.00 | 94.99 | D |
| 4065 | CA | GLU | 755 | 6.051 | 32.400 | -14.331 | 1.00 | 96.19 | D |
| 4066 | CB | GLU | 755 | 5.422 | 33.117 | -13.095 | 1.00 | 98.50 | D |
| 4067 | CG | GLU | 755 | 4.302 | 34.197 | -13.363 | 1.00 | 101.75 | D |
| 4068 | CD | GLU | 755 | 2.905 | 33.824 | -12.782 | 1.00 | 103.89 | D |
| 4069 | OE1 | GLU | 755 | 2.461 | 32.671 | -12.992 | 1.00 | 104.62 | D |
| 4070 | OE2 | GLU | 755 | 2.244 | 34.678 | -12.134 | 1.00 | 103.97 | D |
| 4071 | C | GLU | 755 | 7.081 | 33.324 | -15.006 | 1.00 | 95.52 | D |
| 4072 | O | GLU | 755 | 6.794 | 33.972 | -16.019 | 1.00 | 95.25 | D |
| 4073 | N | ILE | 756 | 8.281 | 33.373 | -14.431 | 1.00 | 94.75 | D |
| 4074 | CA | ILE | 756 | 9.363 | 34.199 | -14.948 | 1.00 | 93.22 | D |
| 4075 | CB | ILE | 756 | 10.523 | 34.227 | -13.969 | 1.00 | 92.05 | D |
| 4076 | CG2 | ILE | 756 | 11.499 | 35.314 | -14.336 | 1.00 | 90.95 | D |
| 4077 | CG1 | ILE | 756 | 9.994 | 34.519 | -12.577 | 1.00 | 91.74 | D |
| 4078 | CD1 | ILE | 756 | 11.064 | 34.473 | -11.556 | 1.00 | 92.95 | D |
| 4079 | C | ILE | 756 | 9.834 | 33.633 | -16.273 | 1.00 | 93.44 | D |
| 4080 | O | ILE | 756 | 10.051 | 34.383 | -17.224 | 1.00 | 93.81 | D |
| 4081 | N | ILE | 757 | 10.003 | 32.315 | -16.353 | 1.00 | 93.62 | D |
| 4082 | CA | ILE | 757 | 10.420 | 31.750 | -17.627 | 1.00 | 93.95 | D |
| 4083 | CB | ILE | 757 | 10.494 | 30.209 | -17.588 | 1.00 | 91.75 | D |
| 4084 | CG2 | ILE | 757 | 10.756 | 29.669 | -18.975 | 1.00 | 90.90 | D |
| 4085 | CG1 | ILE | 757 | 11.662 | 29.777 | -16.699 | 1.00 | 91.48 | D |
| 4086 | CD1 | ILE | 757 | 11.660 | 28.320 | -16.315 | 1.00 | 91.10 | D |
| 4087 | C | ILE | 757 | 9.367 | 32.228 | -18.623 | 1.00 | 95.85 | D |
| 4088 | O | ILE | 757 | 9.673 | 33.038 | -19.497 | 1.00 | 95.78 | D |
| 4089 | N | THR | 758 | 8.127 | 31.764 | -18.448 | 1.00 | 98.52 | D |
| 4090 | CA | THR | 758 | 6.979 | 32.135 | -19.294 | 1.00 | 100.72 | D |
| 4091 | CB | THR | 758 | 5.662 | 31.878 | -18.565 | 1.00 | 100.17 | D |
| 4092 | OG1 | THR | 758 | 5.839 | 32.147 | -17.172 | 1.00 | 99.25 | D |
| 4093 | CG2 | THR | 758 | 5.203 | 30.454 | -18.766 | 1.00 | 100.59 | D |
| 4094 | C | THR | 758 | 6.944 | 33.598 | -19.713 | 1.00 | 102.85 | D |
| 4095 | O | THR | 758 | 6.645 | 33.923 | -20.865 | 1.00 | 103.24 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4096 | N | ASN | 759 | 7.210 | 34.481 | -18.761 | 1.00 | 104.57 | D |
| 4097 | CA | ASN | 759 | 7.213 | 35.896 | -19.060 | 1.00 | 106.54 | D |
| 4098 | CB | ASN | 759 | 7.334 | 36.708 | -17.770 | 1.00 | 107.18 | D |
| 4099 | CG | ASN | 759 | 7.717 | 38.146 | -18.032 | 1.00 | 108.22 | D |
| 4100 | OD1 | ASN | 759 | 7.420 | 38.700 | -19.092 | 1.00 | 108.54 | D |
| 4101 | ND2 | ASN | 759 | 8.376 | 38.768 | -17.058 | 1.00 | 108.86 | D |
| 4102 | C | ASN | 759 | 8.356 | 36.251 | -20.000 | 1.00 | 107.90 | D |
| 4103 | O | ASN | 759 | 8.145 | 36.866 | -21.041 | 1.00 | 108.22 | D |
| 4104 | N | GLN | 760 | 9.566 | 35.838 | -19.641 | 1.00 | 109.61 | D |
| 4105 | CA | GLN | 760 | 10.746 | 36.144 | -20.438 | 1.00 | 111.29 | D |
| 4106 | CB | GLN | 760 | 12.011 | 35.838 | -19.632 | 1.00 | 111.83 | D |
| 4107 | CG | GLN | 760 | 12.186 | 36.680 | -18.385 | 1.00 | 113.09 | D |
| 4108 | CD | GLN | 760 | 12.179 | 38.167 | -18.681 | 1.00 | 113.70 | D |
| 4109 | OE1 | GLN | 760 | 12.858 | 38.636 | -19.599 | 1.00 | 114.19 | D |
| 4110 | NE2 | GLN | 760 | 11.419 | 38.924 | -17.896 | 1.00 | 113.41 | D |
| 4111 | C | GLN | 760 | 10.891 | 35.473 | -21.799 | 1.00 | 112.18 | D |
| 4112 | O | GLN | 760 | 11.477 | 36.061 | -22.708 | 1.00 | 111.69 | D |
| 4113 | N | ILE | 761 | 10.367 | 34.259 | -21.953 | 1.00 | 113.58 | D |
| 4114 | CA | ILE | 761 | 10.535 | 33.528 | -23.207 | 1.00 | 115.12 | D |
| 4115 | CB | ILE | 761 | 9.503 | 32.391 | -23.353 | 1.00 | 114.35 | D |
| 4116 | CG2 | ILE | 761 | 9.809 | 31.596 | -24.622 | 1.00 | 114.19 | D |
| 4117 | CG1 | ILE | 761 | 9.552 | 31.469 | -22.123 | 1.00 | 113.79 | D |
| 4118 | CD1 | ILE | 761 | 10.328 | 30.173 | -22.312 | 1.00 | 113.02 | D |
| 4119 | C | ILE | 761 | 10.548 | 34.363 | -24.495 | 1.00 | 116.87 | D |
| 4120 | O | ILE | 761 | 11.522 | 34.313 | -25.248 | 1.00 | 116.74 | D |
| 4121 | N | PRO | 762 | 9.484 | 35.145 | -24.769 | 1.00 | 118.80 | D |
| 4122 | CD | PRO | 762 | 8.257 | 35.427 | -24.001 | 1.00 | 119.13 | D |
| 4123 | CA | PRO | 762 | 9.517 | 35.936 | -26.007 | 1.00 | 120.40 | D |
| 4124 | CB | PRO | 762 | 8.115 | 36.547 | -26.059 | 1.00 | 119.76 | D |
| 4125 | CG | PRO | 762 | 7.785 | 36.732 | -24.614 | 1.00 | 119.02 | D |
| 4126 | C | PRO | 762 | 10.620 | 36.994 | -26.021 | 1.00 | 122.03 | D |
| 4127 | O | PRO | 762 | 11.647 | 36.822 | -26.673 | 1.00 | 122.16 | D |
| 4128 | N | LYS | 763 | 10.406 | 38.073 | -25.277 | 1.00 | 123.92 | D |
| 4129 | CA | LYS | 763 | 11.341 | 39.196 | -25.192 | 1.00 | 126.19 | D |
| 4130 | CB | LYS | 763 | 10.961 | 40.075 | -23.995 | 1.00 | 125.74 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4131 | CG | LYS | 763 | 11.310 | 39.457 | -22.655 | 1.00 | 125.82 | D |
| 4132 | CD | LYS | 763 | 11.180 | 40.457 | -21.515 | 1.00 | 125.86 | D |
| 4133 | CE | LYS | 763 | 9.724 | 40.759 | -21.192 | 1.00 | 125.93 | D |
| 4134 | NZ | LYS | 763 | 9.571 | 41.658 | -20.007 | 1.00 | 125.34 | D |
| 4135 | C | LYS | 763 | 12.860 | 38.919 | -25.132 | 1.00 | 127.80 | D |
| 4136 | O | LYS | 763 | 13.655 | 39.762 | -25.567 | 1.00 | 127.81 | D |
| 4137 | N | TYR | 764 | 13.267 | 37.765 | -24.596 | 1.00 | 129.73 | D |
| 4138 | CA | TYR | 764 | 14.693 | 37.418 | -24.475 | 1.00 | 131.28 | D |
| 4139 | CB | TYR | 764 | 14.902 | 36.263 | -23.481 | 1.00 | 132.18 | D |
| 4140 | CG | TYR | 764 | 15.676 | 36.665 | -22.250 | 1.00 | 133.74 | D |
| 4141 | CD1 | TYR | 764 | 15.034 | 36.789 | -21.018 | 1.00 | 134.37 | D |
| 4142 | CE1 | TYR | 764 | 15.704 | 37.278 | -19.901 | 1.00 | 134.88 | D |
| 4143 | CD2 | TYR | 764 | 17.024 | 37.026 | -22.333 | 1.00 | 134.58 | D |
| 4144 | CE2 | TYR | 764 | 17.707 | 37.520 | -21.218 | 1.00 | 135.24 | D |
| 4145 | CZ | TYR | 764 | 17.036 | 37.647 | -20.007 | 1.00 | 135.32 | D |
| 4146 | OH | TYR | 764 | 17.675 | 38.185 | -18.917 | 1.00 | 135.01 | D |
| 4147 | C | TYR | 764 | 15.368 | 37.017 | -25.772 | 1.00 | 131.67 | D |
| 4148 | O | TYR | 764 | 16.127 | 37.783 | -26.367 | 1.00 | 131.64 | D |
| 4149 | N | SER | 765 | 15.083 | 35.780 | -26.172 | 1.00 | 132.33 | D |
| 4150 | CA | SER | 765 | 15.635 | 35.159 | -27.370 | 1.00 | 132.82 | D |
| 4151 | CB | SER | 765 | 14.650 | 34.105 | -27.914 | 1.00 | 133.31 | D |
| 4152 | OG | SER | 765 | 13.399 | 34.665 | -28.285 | 1.00 | 133.84 | D |
| 4153 | C | SER | 765 | 16.065 | 36.119 | -28.483 | 1.00 | 132.44 | D |
| 4154 | O | SER | 765 | 17.177 | 36.004 | -29.004 | 1.00 | 132.68 | D |
| 4155 | N | ASN | 766 | 15.198 | 37.067 | -28.832 | 1.00 | 131.53 | D |
| 4156 | CA | ASN | 766 | 15.490 | 38.038 | -29.891 | 1.00 | 130.64 | D |
| 4157 | CB | ASN | 766 | 14.310 | 38.993 | -30.082 | 1.00 | 131.00 | D |
| 4158 | CG | ASN | 766 | 13.031 | 38.461 | -29.492 | 1.00 | 131.52 | D |
| 4159 | OD1 | ASN | 766 | 12.942 | 38.226 | -28.287 | 1.00 | 131.69 | D |
| 4160 | ND2 | ASN | 766 | 12.023 | 38.272 | -30.334 | 1.00 | 132.24 | D |
| 4161 | C | ASN | 766 | 16.739 | 38.884 | -29.629 | 1.00 | 129.71 | D |
| 4162 | O | ASN | 766 | 17.771 | 38.718 | -30.286 | 1.00 | 129.98 | D |
| 4163 | N | GLY | 767 | 16.615 | 39.807 | -28.673 | 1.00 | 128.24 | D |
| 4164 | CA | GLY | 767 | 17.712 | 40.697 | -28.317 | 1.00 | 125.71 | D |
| 4165 | C | GLY | 767 | 17.254 | 42.145 | -28.215 | 1.00 | 123.58 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4166 | O | GLY | 767 | 18.001 | 43.076 | -28.523 | 1.00 | 123.67 | D |
| 4167 | N | ASN | 768 | 16.013 | 42.326 | -27.761 | 1.00 | 121.21 | D |
| 4168 | CA | ASN | 768 | 15.382 | 43.638 | -27.618 | 1.00 | 117.93 | D |
| 4169 | CB | ASN | 768 | 13.907 | 43.490 | -28.011 | 1.00 | 118.56 | D |
| 4170 | CG | ASN | 768 | 13.661 | 42.254 | -28.898 | 1.00 | 119.51 | D |
| 4171 | OD1 | ASN | 768 | 12.763 | 41.449 | -28.629 | 1.00 | 119.65 | D |
| 4172 | ND2 | ASN | 768 | 14.463 | 42.109 | -29.953 | 1.00 | 119.47 | D |
| 4173 | C | ASN | 768 | 15.529 | 44.166 | -26.171 | 1.00 | 115.24 | D |
| 4174 | O | ASN | 768 | 14.715 | 44.963 | -25.687 | 1.00 | 115.25 | D |
| 4175 | N | ILE | 769 | 16.599 | 43.698 | -25.517 | 1.00 | 111.53 | D |
| 4176 | CA | ILE | 769 | 16.996 | 44.010 | -24.130 | 1.00 | 106.35 | D |
| 4177 | CB | ILE | 769 | 17.010 | 42.700 | -23.276 | 1.00 | 106.72 | D |
| 4178 | CG2 | ILE | 769 | 18.139 | 42.736 | -22.253 | 1.00 | 105.96 | D |
| 4179 | CG1 | ILE | 769 | 15.637 | 42.466 | -22.633 | 1.00 | 107.12 | D |
| 4180 | CD1 | ILE | 769 | 15.483 | 41.092 | -21.955 | 1.00 | 106.83 | D |
| 4181 | C | ILE | 769 | 18.427 | 44.586 | -24.175 | 1.00 | 102.35 | D |
| 4182 | O | ILE | 769 | 19.226 | 44.166 | -25.013 | 1.00 | 101.64 | D |
| 4183 | N | LYS | 770 | 18.767 | 45.512 | -23.283 | 1.00 | 97.33 | D |
| 4184 | CA | LYS | 770 | 20.114 | 46.079 | -23.315 | 1.00 | 92.92 | D |
| 4185 | CB | LYS | 770 | 20.057 | 47.599 | -23.311 | 1.00 | 92.48 | D |
| 4186 | CG | LYS | 770 | 21.417 | 48.285 | -23.326 | 1.00 | 91.34 | D |
| 4187 | CD | LYS | 770 | 21.191 | 49.751 | -23.030 | 1.00 | 91.61 | D |
| 4188 | CE | LYS | 770 | 22.458 | 50.541 | -22.808 | 1.00 | 91.57 | D |
| 4189 | NZ | LYS | 770 | 22.092 | 51.940 | -22.420 | 1.00 | 90.52 | D |
| 4190 | C | LYS | 770 | 21.066 | 45.627 | -22.216 | 1.00 | 90.16 | D |
| 4191 | O | LYS | 770 | 21.028 | 46.128 | -21.090 | 1.00 | 89.69 | D |
| 4192 | N | LYS | 771 | 21.932 | 44.690 | -22.579 | 1.00 | 86.93 | D |
| 4193 | CA | LYS | 771 | 22.933 | 44.147 | -21.675 | 1.00 | 83.50 | D |
| 4194 | CB | LYS | 771 | 23.574 | 42.904 | -22.294 | 1.00 | 82.69 | D |
| 4195 | CG | LYS | 771 | 22.605 | 41.810 | -22.745 | 1.00 | 82.70 | D |
| 4196 | CD | LYS | 771 | 23.387 | 40.601 | -23.265 | 1.00 | 83.42 | D |
| 4197 | CE | LYS | 771 | 22.882 | 40.087 | -24.616 | 1.00 | 84.30 | D |
| 4198 | NZ | LYS | 771 | 21.669 | 39.229 | -24.522 | 1.00 | 85.18 | D |
| 4199 | C | LYS | 771 | 24.018 | 45.201 | -21.475 | 1.00 | 81.53 | D |
| 4200 | O | LYS | 771 | 24.694 | 45.568 | -22.429 | 1.00 | 82.12 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4201 | N | LEU | 772 | 24.186 | 45.699 | -20.255 | 1.00 | 78.85 | D |
| 4202 | CA | LEU | 772 | 25.231 | 46.689 | -20.003 | 1.00 | 76.42 | D |
| 4203 | CB | LEU | 772 | 25.041 | 47.322 | -18.636 | 1.00 | 74.60 | D |
| 4204 | CG | LEU | 772 | 23.598 | 47.702 | -18.351 | 1.00 | 73.45 | D |
| 4205 | CD1 | LEU | 772 | 23.524 | 48.369 | -17.001 | 1.00 | 73.02 | D |
| 4206 | CD2 | LEU | 772 | 23.085 | 48.628 | -19.433 | 1.00 | 72.99 | D |
| 4207 | C | LEU | 772 | 26.570 | 45.963 | -20.024 | 1.00 | 75.82 | D |
| 4208 | O | LEU | 772 | 26.662 | 44.825 | -19.574 | 1.00 | 76.10 | D |
| 4209 | N | LEU | 773 | 27.610 | 46.601 | -20.539 | 1.00 | 74.99 | D |
| 4210 | CA | LEU | 773 | 28.918 | 45.951 | -20.573 | 1.00 | 74.05 | D |
| 4211 1 | CB | LEU | 773 | 29.267 | 45.552 | -22.010 | 1.00 | 72.45 | D |
| 4212 | CG | LEU | 773 | 28.437 | 44.416 | -22.609 | 1.00 | 71.06 | D |
| 4213 | CD1 | LEU | 773 | 29.066 | 43.982 | -23.912 | 1.00 | 71.54 | D |
| 4214 | CD2 | LEU | 773 | 28.400 | 43.230 | -21.661 | 1.00 | 70.94 | D |
| 4215 | C | LEU | 773 | 30.004 | 46.862 | -20.008 | 1.00 | 74.26 | D |
| 4216 | O | LEU | 773 | 29.873 | 48.077 | -20.072 | 1.00 | 73.55 | D |
| 4217 | N | PHE | 774 | 31.059 | 46.285 | -19.429 | 1.00 | 75.24 | D |
| 4218 | CA | PHE | 774 | 32.150 | 47.111 | -18.900 | 1.00 | 76.92 | D |
| 4219 | CB | PHE | 774 | 32.838 | 46.449 | -17.713 | 1.00 | 73.36 | D |
| 4220 | CG | PHE | 774 | 32.052 | 46.531 | -16.459 | 1.00 | 69.34 | D |
| 4221 | CD1 | PHE | 774 | 32.009 | 47.707 | -15.728 | 1.00 | 67.48 | D |
| 4222 | CD2 | PHE | 774 | 31.282 | 45.461 | -16.055 | 1.00 | 67.60 | D |
| 4223 | CE1 | PHE | 774 | 31.204 | 47.812 | -14.611 | 1.00 | 66.27 | D |
| 4224 | CE2 | PHE | 774 | 30.478 | 45.558 | -14.942 | 1.00 | 67.12 | D |
| 4225 | CZ | PHE | 774 | 30.433 | 46.734 | -14.219 | 1.00 | 66.31 | D |
| 4226 | C | PHE | 774 | 33.172 | 47.334 | -19.988 | 1.00 | 80.21 | D |
| 4227 | O | PHE | 774 | 33.761 | 48.404 | -20.093 | 1.00 | 81.43 | D |
| 4228 | N | HIS | 775 | 33.377 | 46.298 | -20.792 | 1.00 | 83.76 | D |
| 4229 | CA | HIS | 775 | 34.315 | 46.337 | -21.900 | 1.00 | 87.34 | D |
| 4230 | CB | HIS | 775 | 35.406 | 45.269 | -21.716 | 1.00 | 87.97 | D |
| 4231 | CG | HIS | 775 | 36.023 | 45.254 | -20.355 | 1.00 | 89.06 | D |
| 4232 | CD2 | HIS | 775 | 36.094 | 44.285 | -19.412 | 1.00 | 89.60 | D |
| 4233 | ND1 | HIS | 775 | 36.681 | 46.347 | -19.817 | 1.00 | 89.65 | D |
| 4234 | CE1 | HIS | 775 | 37.127 | 46.045 | -18.614 | 1.00 | 90.20 | D |
| 4235 | NE2 | HIS | 775 | 36.781 | 44.793 | -18.343 | 1.00 | 90.43 | D |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4236 | C | HIS | 775 | 33.501 | 46.008 | -23.154 | 1.00 | 90.31 | D |
| 4237 | O | HIS | 775 | 32.754 | 45.028 | -23.157 | 1.00 | 90.35 | D |
| 4238 | N | GLN | 776 | 33.632 | 46.820 | -24.204 | 1.00 | 94.37 | D |
| 4239 | CA | GLN | 776 | 32.908 | 46.573 | -25.460 | 1.00 | 98.31 | D |
| 4240 | CB | GLN | 776 | 32.564 | 47.907 | -26.159 | 1.00 | 98.95 | D |
| 4241 | CG | GLN | 776 | 33.657 | 48.474 | -27.093 | 1.00 | 101.45 | D |
| 4242 | CD | GLN | 776 | 33.554 | 47.974 | -28.542 | 1.00 | 102.22 | D |
| 4243 | OE1 | GLN | 776 | 34.518 | 48.065 | -29.312 | 1.00 | 101.71 | D |
| 4244 | NE2 | GLN | 776 | 32.381 | 47.461 | -28.917 | 1.00 | 102.39 | D |
| 4245 | C | GLN | 776 | 33.792 | 45.706 | -26.379 | 1.00 | 100.33 | D |
| 4246 | O | GLN | 776 | 34.989 | 45.974 | -26.503 | 1.00 | 100.20 | D |
| 4247 | N | LYS | 777 | 33.216 | 44.672 | -27.002 | 1.00 | 102.72 | D |
| 4248 | CA | LYS | 777 | 33.980 | 43.789 | -27.907 | 1.00 | 104.94 | D |
| 4249 | CB | LYS | 777 | 33.202 | 42.488 | -28.214 | 1.00 | 105.65 | D |
| 4250 | CG | LYS | 777 | 33.654 | 41.700 | -29.491 | 1.00 | 107.33 | D |
| 4251 | CD | LYS | 777 | 35.101 | 41.162 | -29.441 | 1.00 | 108.50 | D |
| 4252 | CE | LYS | 777 | 35.521 | 40.502 | -30.770 | 1.00 | 108.49 | D |
| 4253 | NZ | LYS | 777 | 35.344 | 41.407 | -31.944 | 1.00 | 109.12 | D |
| 4254 | C | LYS | 777 | 34.319 | 44.498 | -29.219 | 1.00 | 105.76 | D |
| 4255 | O | LYS | 777 | 35.517 | 44.512 | -29.589 | 1.00 | 105.96 | D |
| 4256 | OXT | LYS | 777 | 33.383 | 45.021 | -29.864 | 1.00 | 106.30 | D |
| 4257 | CB | LYS | 740 | -1.776 | 39.602 | -15.630 | 1.00 | 141.89 | E |
| 4258 | CG | LYS | 740 | -2.166 | 39.561 | -14.148 | 1.00 | 142.72 | E |
| 4259 | CD | LYS | 740 | -3.686 | 39.652 | -13.989 | 1.00 | 143.70 | E |
| 4260 | CE | LYS | 740 | -4.115 | 39.873 | -12.539 | 1.00 | 144.27 | E |
| 4261 | NZ | LYS | 740 | -5.554 | 40.272 | -12.441 | 1.00 | 144.10 | E |
| 4262 | C | LYS | 740 | 0.614 | 38.879 | -15.391 | 1.00 | 140.04 | E |
| 4263 | O | LYS | 740 | 0.331 | 38.231 | -14.381 | 1.00 | 139.91 | E |
| 4264 | N | LYS | 740 | -0.112 | 40.246 | -17.356 | 1.00 | 141.08 | E |
| 4265 | CA | LYS | 740 | -0.313 | 39.979 | -15.902 | 1.00 | 140.87 | E |
| 4266 | N | GLU | 741 | 1.719 | 38.664 | -16.097 | 1.00 | 138.85 | E |
| 4267 | CA | GLU | 741 | 2.688 | 37.662 | -15.678 | 1.00 | 137.18 | E |
| 4268 | CB | GLU | 741 | 3.798 | 37.516 | -16.727 | 1.00 | 138.20 | E |
| 4269 | CG | GLU | 741 | 4.326 | 36.108 | -16.872 | 1.00 | 139.42 | E |
| 4270 | CD | GLU | 741 | 3.287 | 35.187 | -17.456 | 1.00 | 140.29 | E |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4271 | OE1 | GLU | 741 | 2.190 | 35.093 | -16.867 | 1.00 | 140.93 | E |
| 4272 | OE2 | GLU | 741 | 3.559 | 34.562 | -18.502 | 1.00 | 140.86 | E |
| 4273 | C | GLU | 741 | 3.290 | 38.208 | -14.386 | 1.00 | 134.99 | E |
| 4274 | O | GLU | 741 | 3.029 | 39.350 | -14.006 | 1.00 | 134.69 | E |
| 4275 | N | ASN | 742 | 4.084 | 37.385 | -13.708 | 1.00 | 132.25 | E |
| 4276 | CA | ASN | 742 | 4.755 | 37.799 | -12.483 | 1.00 | 129.12 | E |
| 4277 | CB | ASN | 742 | 5.828 | 38.841 | -12.837 | 1.00 | 130.35 | E |
| 4278 | CG | ASN | 742 | 6.642 | 38.448 | -14.068 | 1.00 | 130.69 | E |
| 4279 | OD1 | ASN | 742 | 6.467 | 39.011 | -15.150 | 1.00 | 130.62 | E |
| 4280 | ND2 | ASN | 742 | 7.525 | 37.466 | -13.906 | 1.00 | 130.78 | E |
| 4281 | C | ASN | 742 | 3.846 | 38.329 | -11.365 | 1.00 | 126.11 | E |
| 4282 | O | ASN | 742 | 4.167 | 39.325 | -10.706 | 1.00 | 125.74 | E |
| 4283 | N | ALA | 743 | 2.718 | 37.661 | -11.156 | 1.00 | 122.24 | E |
| 4284 | CA | ALA | 743 | 1.797 | 38.047 | -10.095 | 1.00 | 117.75 | E |
| 4285 | CB | ALA | 743 | 0.469 | 37.301 | -10.236 | 1.00 | 118.53 | E |
| 4286 | C | ALA | 743 | 2.469 | 37.695 | -8.769 | 1.00 | 114.59 | E |
| 4287 | O | ALA | 743 | 2.385 | 38.455 | -7.806 | 1.00 | 114.16 | E |
| 4288 | N | LEU | 744 | 3.154 | 36.553 | -8.717 | 1.00 | 110.51 | E |
| 4289 | CA | LEU | 744 | 3.823 | 36.170 | -7.480 | 1.00 | 106.08 | E |
| 4290 | CB | LEU | 744 | 4.327 | 34.718 | -7.543 | 1.00 | 105.35 | E |
| 4291 | CG | LEU | 744 | 4.090 | 33.917 | -6.245 | 1.00 | 104.62 | E |
| 4292 | CD1 | LEU | 744 | 4.547 | 32.469 | -6.396 | 1.00 | 103.98 | E |
| 4293 | CD2 | LEU | 744 | 4.827 | 34.584 | -5.099 | 1.00 | 104.60 | E |
| 4294 | C | LEU | 744 | 4.973 | 37.134 | -7.143 | 1.00 | 103.50 | E |
| 4295 | O | LEU | 744 | 5.201 | 37.421 | -5.971 | 1.00 | 103.23 | E |
| 4296 | N | LEU | 745 | 5.682 | 37.650 | -8.151 | 1.00 | 100.22 | E |
| 4297 | CA | LEU | 745 | 6.791 | 38.587 | -7.894 | 1.00 | 97.11 | E |
| 4298 | CB | LEU | 745 | 7.598 | 38.878 | -9.172 | 1.00 | 95.13 | E |
| 4299 | CG | LEU | 745 | 9.114 | 38.639 | -9.295 | 1.00 | 92.39 | E |
| 4300 | CD1 | LEU | 745 | 9.534 | 39.232 | -10.627 | 1.00 | 91.20 | E |
| 4301 | CD2 | LEU | 745 | 9.924 | 39.266 | -8.162 | 1.00 | 90.98 | E |
| 4302 | C | LEU | 745 | 6.295 | 39.921 | -7.340 | 1.00 | 95.69 | E |
| 4303 | O | LEU | 745 | 6.781 | 40.383 | -6.305 | 1.00 | 95.37 | E |
| 4304 | N | ARG | 746 | 5.340 | 40.553 | -8.026 | 1.00 | 94.08 | E |
| 4305 | CA | ARG | 746 | 4.847 | 41.827 | -7.528 | 1.00 | 91.98 | E |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4306 | CB | ARG | 746 | 3.666 | 42.370 | -8.359 | 1.00 | 92.78 | E |
| 4307 | CG | ARG | 746 | 3.300 | 43.811 | -7.933 | 1.00 | 95.34 | E |
| 4308 | CD | ARG | 746 | 2.281 | 44.580 | -8.816 | 1.00 | 96.94 | E |
| 4309 | NE | ARG | 746 | 2.756 | 45.145 | -10.091 | 1.00 | 99.07 | E |
| 4310 | CZ | ARG | 746 | 2.598 | 44.566 | -11.287 | 1.00 | 100.49 | E |
| 4311 | NH1 | ARG | 746 | 1.992 | 43.386 | -11.381 | 1.00 | 100.23 | E |
| 4312 | NH2 | ARG | 746 | 2.962 | 45.204 | -12.397 | 1.00 | 100.52 | E |
| 4313 | C | ARG | 746 | 4.439 | 41.650 | -6.072 | 1.00 | 90.46 | E |
| 4314 | O | ARG | 746 | 4.742 | 42.493 | -5.237 | 1.00 | 89.72 | E |
| 4315 | N | TYR | 747 | 3.785 | 40.536 | -5.754 | 1.00 | 89.44 | E |
| 4316 | CA | TYR | 747 | 3.362 | 40.281 | -4.379 | 1.00 | 88.36 | E |
| 4317 | CB | TYR | 747 | 2.558 | 38.960 | -4.304 | 1.00 | 86.74 | E |
| 4318 | CG | TYR | 747 | 2.370 | 38.390 | -2.904 | 1.00 | 85.09 | E |
| 4319 | CD1 | TYR | 747 | 1.515 | 38.992 | -1.981 | 1.00 | 84.23 | E |
| 4320 | CE1 | TYR | 747 | 1.416 | 38.512 | -0.669 | 1.00 | 84.20 | E |
| 4321 | CD2 | TYR | 747 | 3.110 | 37.283 | -2.487 | 1.00 | 84.97 | E |
| 4322 | CE2 | TYR | 747 | 3.020 | 36.801 | -1.187 | 1.00 | 84.52 | E |
| 4323 | CZ | TYR | 747 | 2.179 | 37.422 | -0.280 | 1.00 | 84.47 | E |
| 4324 | OH | TYR | 747 | 2.144 | 36.982 | 1.024 | 1.00 | 84.78 | E |
| 4325 | C | TYR | 747 | 4.571 | 40.244 | -3.431 | 1.00 | 88.64 | E |
| 4326 | O | TYR | 747 | 4.569 | 40.907 | -2.394 | 1.00 | 88.55 | E |
| 4327 | N | LEU | 748 | 5.602 | 39.494 | -3.810 | 1.00 | 89.68 | E |
| 4328 | CA | LEU | 748 | 6.813 | 39.348 | -2.999 | 1.00 | 89.96 | E |
| 4329 | CB | LEU | 748 | 7.723 | 38.291 | -3.635 | 1.00 | 88.64 | E |
| 4330 | CG | LEU | 748 | 7.137 | 36.874 | -3.615 | 1.00 | 87.96 | E |
| 4331 | CD1 | LEU | 748 | 7.795 | 36.025 | -4.684 | 1.00 | 87.83 | E |
| 4332 | CD2 | LEU | 748 | 7.322 | 36.254 | -2.236 | 1.00 | 86.92 | E |
| 4333 | C | LEU | 748 | 7.592 | 40.642 | -2.778 | 1.00 | 90.99 | E |
| 4334 | O | LEU | 748 | 8.220 | 40.826 | -1.734 | 1.00 | 90.21 | E |
| 4335 | N | LEU | 749 | 7.535 | 41.541 | -3.758 | 1.00 | 93.07 | E |
| 4336 | CA | LEU | 749 | 8.243 | 42.816 | -3.678 | 1.00 | 95.67 | E |
| 4337 | CB | LEU | 749 | 8.470 | 43.365 | -5.094 | 1.00 | 92.83 | E |
| 4338 | CG | LEU | 749 | 9.614 | 42.671 | -5.837 | 1.00 | 90.56 | E |
| 4339 | CD1 | LEU | 749 | 9.654 | 43.080 | -7.295 | 1.00 | 88.14 | E |
| 4340 | CD2 | LEU | 749 | 10.916 | 43.016 | -5.133 | 1.00 | 89.43 | E |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4341 | C | LEU | 749 | 7.578 | 43.886 | -2.811 | 1.00 | 98.99 | E |
| 4342 | O | LEU | 749 | 8.202 | 44.904 | -2.501 | 1.00 | 99.50 | E |
| 4343 | N | ASP | 750 | 6.330 | 43.648 | -2.404 | 1.00 | 103.16 | E |
| 4344 | CA | ASP | 750 | 5.579 | 44.607 | -1.587 | 1.00 | 107.11 | E |
| 4345 | CB | ASP | 750 | 4.152 | 44.727 | -2.135 | 1.00 | 106.92 | E |
| 4346 | CG | ASP | 750 | 4.053 | 45.700 | -3.301 | 1.00 | 107.42 | E |
| 4347 | OD1 | ASP | 750 | 4.995 | 45.742 | -4.122 | 1.00 | 107.42 | E |
| 4348 | OD2 | ASP | 750 | 3.034 | 46.421 | -3.408 | 1.00 | 107.60 | E |
| 4349 | C | ASP | 750 | 5.542 | 44.392 | -0.063 | 1.00 | 110.57 | E |
| 4350 | O | ASP | 750 | 5.804 | 45.327 | 0.692 | 1.00 | 110.50 | E |
| 4351 | N | LYS | 751 | 5.227 | 43.182 | 0.398 | 1.00 | 114.91 | E |
| 4352 | CA | LYS | 751 | 5.157 | 42.897 | 1.845 | 1.00 | 119.84 | E |
| 4353 | CB | LYS | 751 | 4.738 | 41.430 | 2.053 | 1.00 | 120.51 | E |
| 4354 | CG | LYS | 751 | 5.442 | 40.409 | 1.165 | 1.00 | 121.28 | E |
| 4355 | CD | LYS | 751 | 5.130 | 39.006 | 1.655 | 1.00 | 122.93 | E |
| 4356 | CE | LYS | 751 | 5.522 | 38.865 | 3.125 | 1.00 | 124.03 | E |
| 4357 | NZ | LYS | 751 | 4.915 | 37.676 | 3.787 | 1.00 | 124.84 | E |
| 4358 | C | LYS | 751 | 6.425 | 43.218 | 2.677 | 1.00 | 122.73 | E |
| 4359 | O | LYS | 751 | 7.049 | 44.262 | 2.478 | 1.00 | 122.86 | E |
| 4360 | N | ASP | 752 | 6.765 | 42.351 | 3.641 | 1.00 | 126.13 | E |
| 4361 | CA | ASP | 752 | 7.964 | 42.504 | 4.492 | 1.00 | 129.40 | E |
| 4362 | CB | ASP | 752 | 9.183 | 42.759 | 3.586 | 1.00 | 130.18 | E |
| 4363 | CG | ASP | 752 | 10.443 | 42.073 | 4.084 | 1.00 | 131.66 | E |
| 4364 | OD1 | ASP | 752 | 10.786 | 42.244 | 5.275 | 1.00 | 132.08 | E |
| 4365 | OD2 | ASP | 752 | 11.097 | 41.367 | 3.281 | 1.00 | 132.63 | E |
| 4366 | C | ASP | 752 | 7.960 | 43.562 | 5.638 | 1.00 | 130.58 | E |
| 4367 | O | ASP | 752 | 7.487 | 44.684 | 5.459 | 1.00 | 131.17 | E |
| 4368 | N | ASP | 753 | 8.496 | 43.162 | 6.807 | 1.00 | 132.38 | E |
| 4369 | CA | ASP | 753 | 8.673 | 43.970 | 8.046 | 1.00 | 133.53 | E |
| 4370 | CB | ASP | 753 | 7.392 | 44.726 | 8.497 | 1.00 | 134.59 | E |
| 4371 | CG | ASP | 753 | 7.602 | 45.573 | 9.796 | 1.00 | 134.88 | E |
| 4372 | OD1 | ASP | 753 | 7.631 | 45.005 | 10.915 | 1.00 | 135.11 | E |
| 4373 | OD2 | ASP | 753 | 7.740 | 46.814 | 9.695 | 1.00 | 134.97 | E |
| 4374 | C | ASP | 753 | 9.092 | 43.000 | 9.154 | 1.00 | 133.95 | E |
| 4375 | O | ASP | 753 | 10.293 | 42.981 | 9.496 | 1.00 | 134.41 | E |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4376 | OXT | ASP | 753 | 8.223 | 42.246 | 9.644 | 1.00 | 134.72 | E |
| 4377 | C1 | FLU | 1 | 31.469 | 6.647 | 9.774 | 1.00 | 68.41 | C |
| 4378 | C2 | FLU | 1 | 31.819 | 7.274 | 8.637 | 1.00 | 66.97 | C |
| 4379 | C3 | FLU | 1 | 33.096 | 8.036 | 8.648 | 1.00 | 68.13 | C |
| 4380 | C4 | FLU | 1 | 33.846 | 8.156 | 9.949 | 1.00 | 67.54 | C |
| 4381 | C5 | FLU | 1 | 33.446 | 7.519 | 11.039 | 1.00 | 68.42 | C |
| 4382 | C6 | FLU | 1 | 34.232 | 7.698 | 12.303 | 1.00 | 69.88 | C |
| 4383 | F61 | FLU | 1 | 35.211 | 8.643 | 12.190 | 1.00 | 71.79 | C |
| 4384 | C7 | FLU | 1 | 33.332 | 8.128 | 13.460 | 1.00 | 70.88 | C |
| 4385 | C8 | FLU | 1 | 32.025 | 7.330 | 13.516 | 1.00 | 72.02 | C |
| 4386 | C9 | FLU | 1 | 31.297 | 7.119 | 12.193 | 1.00 | 71.23 | C |
| 4387 | C10 | FLU | 1 | 32.213 | 6.638 | 11.065 | 1.00 | 69.64 | C |
| 4388 | C11 | FLU | 1 | 29.932 | 6.437 | 12.283 | 1.00 | 71.39 | C |
| 4389 | C12 | FLU | 1 | 29.105 | 6.683 | 13.539 | 1.00 | 72.71 | C |
| 4390 | C13 | FLU | 1 | 29.890 | 6.661 | 14.857 | 1.00 | 73.76 | C |
| 4391 | C14 | FLU | 1 | 31.136 | 7.539 | 14.738 | 1.00 | 72.68 | C |
| 4392 | C15 | FLU | 1 | 31.614 | 7.926 | 16.141 | 1.00 | 74.16 | C |
| 4393 | C16 | FLU | 1 | 30.383 | 7.643 | 16.996 | 1.00 | 74.79 | C |
| 4394 | C17 | FLU | 1 | 29.218 | 7.330 | 16.053 | 1.00 | 76.56 | C |
| 4395 | C18 | FLU | 1 | 30.305 | 5.197 | 15.044 | 1.00 | 73.36 | C |
| 4396 | C19 | FLU | 1 | 32.695 | 5.192 | 11.181 | 1.00 | 69.59 | C |
| 4397 | C20 | FLU | 1 | 28.267 | 6.341 | 16.712 | 1.00 | 77.91 | C |
| 4398 | S21 | FLU | 1 | 26.498 | 6.507 | 16.475 | 1.00 | 78.08 | C |
| 4399 | C21 | FLU | 1 | 25.978 | 4.792 | 16.637 | 1.00 | 79.64 | C |
| 4400 | F21 | FLU | 1 | 26.415 | 4.104 | 15.543 | 1.00 | 82.24 | C |
| 4401 | C22 | FLU | 1 | 30.250 | 8.586 | 18.187 | 1.00 | 74.40 | C |
| 4402 | P1 | FLU | 1 | 30.899 | 8.375 | 11.854 | 1.00 | 74.42 | C |
| 4403 | 01 | FLU | 1 | 33.524 | 8.563 | 7.621 | 1.00 | 70.89 | C |
| 4404 | 02 | FLU | 1 | 30.058 | 5.029 | 12.153 | 1.00 | 70.24 | C |
| 4405 | 03 | FLU | 1 | 28.413 | 8.454 | 15.697 | 1.00 | 79.76 | C |
| 4406 | 04 | FLU | 1 | 28.685 | 5.416 | 17.406 | 1.00 | 79.85 | C |
| 4407 | C | FLU | 1 | 28.738 | 9.781 | 15.735 | 1.00 | 81.43 | C |
| 4408 | CC | FLU | 1 | 27.622 | 10.758 | 15.411 | 1.00 | 80.02 | C |
| 4409 | CC3 | FLU | 1 | 27.540 | 10.867 | 13.889 | 1.00 | 78.83 | C |
| 4410 | O | FLU | 1 | 29.829 | 10.282 | 16.003 | 1.00 | 82.80 | C |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4411 | C1 | FLU | 2 | 18.731 | 25.721 | -9.807 | 1.00 | 66.81 | F |
| 4412 | C2 | FLU | 2 | 19.386 | 25.696 | -8.652 | 1.00 | 65.83 | F |
| 4413 | C3 | FLU | 2 | 20.603 | 26.567 | -8.540 | 1.00 | 66.00 | F |
| 4414 | C4 | FLU | 2 | 21.052 | 27.321 | -9.758 | 1.00 | 65.97 | F |
| 4415 | C5 | FLU | 2 | 20.346 | 27.301 | -10.888 | 1.00 | 66.75 | F |
| 4416 | C6 | FLU | 2 | 20.870 | 28.079 | -12.060 | 1.00 | 67.70 | F |
| 4417 | F61 | FLU | 2 | 22.113 | 28.603 | -11.869 | 1.00 | 67.61 | F |
| 4418 | C7 | FLU | 2 | 20.975 | 27.192 | -13.299 | 1.00 | 67.74 | F |
| 4419 | C8 | FLU | 2 | 19.740 | 26.304 | -13.471 | 1.00 | 70.03 | F |
| 4420 | C9 | FLU | 2 | 19.217 | 25.580 | -12.225 | 1.00 | 69.01 | F |
| 4421 | C10 | FLU | 2 | 19.073 | 26.521 | -11.026 | 1.00 | 67.85 | F |
| 4422 | C11 | FLU | 2 | 18.071 | 24.593 | -12.461 | 1.00 | 69.61 | F |
| 4423 | C12 | FLU | 2 | 18.023 | 23.880 | -13.813 | 1.00 | 72.06 | F |
| 4424 | C13 | FLU | 2 | 18.345 | 24.746 | -15.029 | 1.00 | 73.82 | F |
| 4425 | C14 | FLU | 2 | 19.647 | 25.492 | -14.762 | 1.00 | 72.07 | F |
| 4426 | C15 | FLU | 2 | 20.287 | 25.940 | -16.075 | 1.00 | 73.54 | F |
| 4427 | C16 | FLU | 2 | 19.659 | 24.961 | -17.062 | 1.00 | 74.63 | F |
| 4428 | C17 | FLU | 2 | 18.758 | 23.991 | -16.287 | 1.00 | 77.21 | F |
| 4429 | C18 | FLU | 2 | 17.155 | 25.681 | -15.219 | 1.00 | 72.30 | F |
| 4430 | C19 | FLU | 2 | 17.921 | 27.513 | -11.113 | 1.00 | 68.89 | F |
| 4431 | C20 | FLU | 2 | 17.540 | 23.530 | -17.071 | 1.00 | 79.04 | F |
| 4432 | S21 | FLU | 2 | 16.760 | 21.975 | -16.623 | 1.00 | 81.55 | F |
| 4433 | C21 | FLU | 2 | 15.234 | 22.064 | -17.575 | 1.00 | 83.80 | F |
| 4434 | F21 | FLU | 2 | 14.313 | 22.715 | -16.813 | 1.00 | 86.86 | F |
| 4435 | C22 | FLU | 2 | 20.799 | 24.580 | -17.998 | 1.00 | 74.95 | F |
| 4436 | F1 | FLU | 2 | 20.223 | 24.708 | -11.913 | 1.00 | 70.32 | F |
| 4437 | 01 | FLU | 2 | 21.233 | 26.659 | -7.481 | 1.00 | 68.73 | F |
| 4438 | 02 | FLU | 2 | 16.816 | 25.254 | -12.348 | 1.00 | 69.31 | F |
| 4439 | 03 | FLU | 2 | 19.455 | 22.843 | -15.815 | 1.00 | 80.25 | F |
| 4440 | 04 | FLU | 2 | 17.083 | 24.202 | -17.994 | 1.00 | 79.76 | F |
| 4441 | C | FLU | 2 | 19.977 | 21.889 | -16.658 | 1.00 | 82.09 | F |
| 4442 | CC | FLU | 2 | 20.497 | 20.676 | -15.905 | 1.00 | 81.78 | F |
| 4443 | CC3 | FLU | 2 | 21.989 | 20.751 | -15.572 | 1.00 | 78.23 | F |
| 4444 | O | FLU | 2 | 20.024 | 21.982 | -17.881 | 1.00 | 84.06 | F |
| 4445 | O | HOH | | 19.523 | 33.396 | -3.571 | 1.00 | 76.62 | W |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4446 | O | HOH | 2 | 42.941 | 37.375 | -17.594 | 1.00 | 73.82 | W |
| 4447 | O | HOH | 3 | -5.577 | 41.115 | -21.499 | 1.00 | 86.78 | W |
| 4448 | O | HOH | 4 | 38.453 | 47.403 | -23.099 | 1.00 | 91.58 | W |
| 4449 | O | HOH | 5 | 26.829 | 53.228 | -14.715 | 1.00 | 76.19 | W |
| 4450 | O | HOH | 6 | 27.841 | 43.647 | -16.662 | 1.00 | 83.67 | W |
| 4451 | O | HOH | 7 | 37.308 | 4.182 | 2.628 | 1.00 | 82.13 | W |
| 4452 | O | HOH | 8 | 51.563 | 23.152 | 18.316 | 1.00 | 77.47 | W |
| 4453 | O | HOH | 9 | 14.159 | 62.331 | -10.476 | 1.00 | 72.93 | W |
| 4454 | O | HOH | 10 | 41.691 | 4.638 | 29.350 | 1.00 | 81.82 | W |
| 4455 | O | HOH | 11 | 51.782 | 5.887 | 14.391 | 1.00 | 69.86 | W |
| 4456 | O | HOH | 12 | 28.889 | -4.501 | 5.566 | 1.00 | 74.77 | W |
| 4457 | O | HOH | 13 | 49.515 | 19.573 | 17.146 | 1.00 | 84.85 | W |
| 4458 | O | HOH | 14 | 3.901 | 51.708 | -24.615 | 1.00 | 75.63 | W |
| 4459 | O | HOH | 15 | 44.526 | -5.219 | 33.428 | 1.00 | 92.56 | W |
| 4460 | O | HOH | 16 | 49.031 | 2.748 | 26.331 | 1.00 | 85.58 | W |
| 4461 | O | HOH | 17 | 69.550 | 9.656 | -15.186 | 1.00 | 95.60 | W |
| 4462 | O | HOH | 18 | 37.583 | 46.290 | -30.177 | 1.00 | 94.72 | W |
| 4463 | O | HOH | 19 | 44.712 | -10.424 | 23.759 | 1.00 | 93.68 | W |
| 4464 | O | HOH | 20 | 10.095 | 47.678 | 8.481 | 1.00 | 94.91 | W |
| 4465 | O | HOH | 21 | 23.412 | 55.862 | -18.634 | 1.00 | 64.40 | W |
| 4466 | O | HOH | 22 | 45.467 | 40.765 | -18.463 | 1.00 | 82.28 | W |
| 4467 | O | HOH | 23 | 63.473 | 2.606 | 6.965 | 1.00 | 78.36 | W |
| 4468 | O | HOH | 24 | 60.128 | 12.787 | 22.992 | 1.00 | 78.64 | W |
| 4469 | O | HOH | 25 | 43.814 | -4.792 | -2.342 | 1.00 | 73.85 | W |
| 4470 | O | HOH | 26 | 35.802 | 58.962 | -5.624 | 1.00 | 86.33 | W |
| 4471 | O | HOH | 27 | 18.584 | 43.655 | -32.292 | 1.00 | 110.47 | W |
| 4472 | O | HOH | 28 | 1.260 | 31.718 | -15.461 | 1.00 | 94.95 | W |
| 4473 | O | HOH | 29 | 39.707 | 25.903 | 12.225 | 1.00 | 81.11 | W |
| 4474 | O | HOH | 30 | 8.864 | 39.637 | -26.908 | 1.00 | 102.41 | W |
| 4475 | O | HOH | 31 | 25.220 | 8.573 | -6.280 | 1.00 | 84.78 | W |
| 4476 | O | HOH | 32 | 44.012 | 3.517 | 19.660 | 1.00 | 79.65 | W |
| 4477 | O | HOH | 33 | 61.617 | 5.971 | 3.196 | 1.00 | 76.45 | W |
| 4478 | O | HOH | 34 | 33.361 | 11.235 | 25.669 | 1.00 | 87.33 | W |
| 4479 | O | HOH | 35 | 48.747 | 6.563 | 26.113 | 1.00 | 94.00 | W |
| 4480 | O | HOH | 36 | 35.557 | 39.674 | 3.256 | 1.00 | 84.29 | W |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4481 | O | HOH | 37 | 43.568 | 3.880 | 17.239 | 1.00 | 80.95 | W |
| 4482 | O | HOH | 38 | -2.757 | 39.376 | -25.950 | 1.00 | 94.87 | W |
| 4483 | O | HOH | 39 | 37.467 | 5.537 | 29.129 | 1.00 | 102.58 | W |
| 4484 | O | HOH | 40 | 18.443 | 9.340 | -17.496 | 1.00 | 75.91 | W |
| 4485 | O | HOH | 41 | 58.946 | 27.891 | 16.135 | 1.00 | 117.13 | W |
| 4486 | O | HOH | 42 | 29.763 | 47.759 | -26.838 | 1.00 | 87.96 | W |
| 4487 | O | HOH | 43 | 38.372 | -23.387 | 18.058 | 1.00 | 88.20 | W |
| 4488 | O | HOH | 44 | 2.160 | 53.587 | -26.952 | 1.00 | 103.43 | W |
| 4489 | O | HOH | 45 | 15.637 | 33.354 | 6.623 | 1.00 | 78.76 | W |
| 4490 | O | HOH | 46 | 36.791 | 8.067 | 1.836 | 1.00 | 92.22 | W |
| 4491 | O | HOH | 47 | 41.420 | -19.727 | 12.465 | 1.00 | 90.65 | W |
| 4492 | O | HOH | 48 | 35.606 | 42.694 | -34.099 | 1.00 | 92.07 | W |
| 4493 | O | HOH | 49 | 9.972 | 40.686 | -28.976 | 1.00 | 90.64 | W |
| 4494 | O | HOH | 50 | 56.197 | 25.170 | 9.346 | 1.00 | 102.45 | W |
| 4495 | O | HOH | 51 | 45.027 | -14.654 | -8.801 | 1.00 | 73.69 | W |
| 4496 | O | HOH | 52 | 1.523 | 47.124 | -14.313 | 1.00 | 79.60 | W |
| 4497 | O | HOH | 53 | 48.094 | 20.357 | -1.285 | 1.00 | 63.38 | W |
| 4498 | O | HOH | 54 | -3.646 | 36.748 | -23.378 | 1.00 | 89.36 | W |
| 4499 | O | HOH | 55 | 54.686 | -16.296 | 13.451 | 1.00 | 93.02 | W |
| 4500 | O | HOH | 56 | 18.176 | 19.988 | -29.385 | 1.00 | 85.25 | W |
| 4501 | O | HOH | 57 | 18.017 | 7.401 | 19.131 | 1.00 | 89.90 | W |
| 4502 | O | HOH | 58 | 46.405 | 10.360 | -8.409 | 1.00 | 79.17 | W |
| 4503 | O | HOH | 59 | 60.377 | -2.468 | 19.274 | 1.00 | 93.48 | W |
| 4504 | O | HOH | 60 | -2.437 | 45.083 | -20.632 | 1.00 | 102.27 | W |
| 4505 | O | HOH | 61 | 38.901 | 33.047 | -19.920 | 1.00 | 80.62 | W |
| 4506 | O | HOH | 62 | 28.293 | 32.129 | -28.814 | 1.00 | 87.71 | W |
| 4507 | O | HOH | 63 | 44.732 | 10.741 | -4.492 | 1.00 | 81.65 | W |
| 4508 | O | HOH | 64 | 24.682 | 4.218 | -1.857 | 1.00 | 76.04 | W |
| 4509 | O | HOH | 65 | 31.637 | 0.342 | 25.661 | 1.00 | 82.29 | W |
| 4510 | O | HOH | 66 | 4.471 | 24.618 | -9.189 | 1.00 | 90.31 | W |
| 4511 1 | O | HOH | 67 | 30.941 | 3.201 | 26.651 | 1.00 | 103.40 | W |
| 4512 | O | HOH | 68 | 39.603 | 1.672 | 28.491 | 1.00 | 113.23 | W |
| 4513 | O | HOH | 69 | 30.027 | 53.782 | 2.347 | 1.00 | 86.93 | W |
| 4514 | O | HOH | 70 | 24.112 | 8.345 | 20.698 | 1.00 | 83.64 | W |
| 4515 | O | HOH | 71 | 11.238 | 11.421 | -10.129 | 1.00 | 104.98 | W |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4516 | O | HOH | 72 | 10.919 | 8.389 | -11.074 | 1.00 | 108.91 | W |
| 4517 | O | HOH | 73 | 37.382 | 35.007 | 1.398 | 1.00 | 60.56 | W |
| 4518 | O | HOH | 74 | 44.332 | 42.288 | -22.956 | 1.00 | 99.96 | W |
| 4519 | O | HOH | 75 | 9.555 | 39.664 | 1.611 | 1.00 | 103.96 | W |
| 4520 | O | HOH | 76 | 22.671 | 31.229 | -1.881 | 1.00 | 85.23 | W |
| 4521 | O | HOH | 77 | 4.772 | 47.026 | 10.208 | 1.00 | 105.31 | W |
| 4522 | O | HOH | 78 | 50.024 | 16.208 | -4.476 | 1.00 | 84.94 | W |
| 4523 | O | HOH | 79 | 48.969 | -19.537 | 13.191 | 1.00 | 101.64 | W |
| 4524 | O | HOH | 80 | 9.808 | 51.313 | -0.626 | 1.00 | 83.97 | W |
| 4525 | O | HOH | 81 | 57.763 | 14.751 | 31.604 | 1.00 | 84.36 | W |
| 4526 | O | HOH | 82 | 58.922 | -4.343 | 19.998 | 1.00 | 97.27 | W |
| 4527 | O | HOH | 83 | 53.567 | -16.970 | 16.934 | 1.00 | 74.06 | W |
| 4528 | O | HOH | 84 | 62.029 | 10.217 | -3.169 | 1.00 | 82.95 | W |
| 4529 | O | HOH | 85 | 50.109 | 40.904 | -11.762 | 1.00 | 102.45 | W |
| 4530 | O | HOH | 86 | 1.821 | 49.898 | -13.245 | 1.00 | 99.91 | W |
| 4531 | O | HOH | 87 | 21.200 | 13.279 | 0.465 | 1.00 | 79.27 | W |
| 4532 | O | HOH | 88 | 63.358 | -1.153 | 3.412 | 1.00 | 102.97 | W |
| 4533 | O | HOH | 89 | 18.070 | 25.741 | 14.704 | 1.00 | 86.42 | W |
| 4534 | O | HOH | 90 | 60.672 | 12.708 | 31.162 | 1.00 | 112.67 | W |
| 4535 | O | HOH | 91 | 13.164 | -6.141 | 17.266 | 1.00 | 110.15 | W |
| 4536 | O | HOH | 92 | 10.846 | 49.963 | -21.798 | 1.00 | 90.71 | W |
| 4537 | O | HOH | 93 | 34.418 | 24.160 | -18.821 | 1.00 | 87.02 | W |
| 4538 | O | HOH | 94 | 18.611 1 | 25.870 | -27.603 | 1.00 | 112.94 | W |
| 4539 | O | HOH | 95 | 5.242 | 48.331 | 1.314 | 1.00 | 78.27 | W |
| 4540 | O | HOH | 96 | 43.848 | -9.310 | 18.979 | 1.00 | 109.50 | W |
| 4541 | O | HOH | 97 | 22.575 | 6.419 | -8.204 | 1.00 | 80.78 | W |
| 4542 | O | HOH | 98 | 44.607 | 25.465 | 2.534 | 1.00 | 86.75 | W |
| 4543 | O | HOH | 99 | 25.294 | 0.805 | 26.895 | 1.00 | 79.15 | W |
| 4544 | O | HOH | 100 | 15.873 | 13.445 | 16.080 | 1.00 | 65.33 | W |
| 4545 | O | HOH | 101 | 46.669 | -4.925 | -3.439 | 1.00 | 63.97 | W |
| 4546 | O | HOH | 102 | 10.476 | 57.087 | -17.694 | 1.00 | 73.69 | W |
| 4547 | O | HOH | 103 | 32.866 | 39.274 | -27.212 | 1.00 | 95.95 | W |
| 4548 | O | HOH | 104 | 42.351 | -21.786 | 13.383 | 1.00 | 84.99 | W |
| 4549 | O | HOH | 105 | -0.138 | 38.199 | -23.756 | 1.00 | 105.98 | W |
| 4550 | O | HOH | 106 | 28.478 | 19.343 | -28.838 | 1.00 | 93.72 | W |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4551 | O | HOH | 107 | 26.576 | 57.319 | -16.982 | 1.00 | 115.17 | W |
| 4552 | O | HOH | 108 | 65.453 | 11.244 | 1.860 | 1.00 | 100.82 | W |
| 4553 | O | HOH | 109 | -0.708 | 38.981 | -21.728 | 1.00 | 89.17 | W |
| 4554 | O | HOH | 110 | 27.824 | -8.474 | 20.771 | 1.00 | 86.00 | W |
| 4555 | O | HOH | 111 1 | 39.931 | 48.171 | -29.933 | 1.00 | 125.52 | W |
| 4556 | O | HOH | 112 | 45.275 | -19.198 | 12.656 | 1.00 | 101.55 | W |
| 4557 | O | HOH | 113 | 45.279 | 29.204 | 13.170 | 1.00 | 81.25 | W |
| 4558 | O | HOH | 114 | 39.604 | -9.905 | -10.328 | 1.00 | 73.44 | W |
| 4559 | O | HOH | 115 | 64.611 | 12.168 | -0.278 | 1.00 | 95.59 | W |
| 4560 | O | HOH | 116 | 68.609 | 24.621 | 6.796 | 1.00 | 99.37 | W |
| 4561 | O | HOH | 117 | 25.054 | -7.146 | 20.234 | 1.00 | 85.14 | W |
| 4562 | O | HOH | 118 | 46.697 | 4.148 | 27.896 | 1.00 | 105.02 | W |
| 4563 | O | HOH | 119 | 22.193 | 26.077 | -24.741 | 1.00 | 69.48 | W |
| 4564 | O | HOH | 120 | 26.353 | 57.873 | -19.525 | 1.00 | 108.32 | W |
| 4565 | O | HOH | 121 | 8.026 | 55.457 | -17.860 | 1.00 | 98.34 | W |
| 4566 | O | HOH | 122 | 12.559 | 11.828 | -24.035 | 1.00 | 93.10 | W |
| 4567 | O | HOH | 123 | -4.915 | 40.336 | -23.651 | 1.00 | 96.08 | W |
| 4568 | O | HOH | 124 | 7.424 | 56.806 | -15.154 | 1.00 | 95.41 | W |
| 4569 | O | HOH | 125 | 9.243 | 9.680 | -21.618 | 1.00 | 88.75 | W |
| 4570 | O | HOH | 126 | 41.604 | 39.684 | -21.569 | 1.00 | 113.19 | W |
| 4571 | O | HOH | 127 | 39.225 | -16.888 | 3.197 | 1.00 | 66.34 | W |
| 4572 | O | HOH | 128 | 40.747 | 40.837 | -23.085 | 1.00 | 85.74 | W |
| 4573 | O | HOH | 129 | 36.354 | 54.080 | 16.892 | 1.00 | 107.37 | W |
| 4574 | O | HOH | 130 | 40.021 | -4.808 | 33.204 | 1.00 | 91.23 | W |
| 4575 | O | HOH | 131 | 55.071 | 24.231 | -2.554 | 1.00 | 70.80 | W |
| 4576 | O | HOH | 132 | 46.616 | 48.721 | -2.372 | 1.00 | 83.01 | W |
| 4577 | O | HOH | 133 | 26.663 | 54.965 | -17.649 | 1.00 | 94.09 | W |
| 4578 | O | HOH | 134 | 60.447 | 24.164 | -2.252 | 1.00 | 108.34 | W |
| 4579 | O | HOH | 135 | 37.786 | 54.011 | -0.139 | 1.00 | 112.49 | W |
| 4580 | O | HOH | 136 | 39.669 | 27.027 | 16.006 | 1.00 | 113.82 | W |
| 4581 | O | HOH | 137 | 54.615 | 6.585 | 26.415 | 1.00 | 97.63 | W |
| 4582 | O | HOH | 138 | 38.455 | 26.713 | 14.349 | 1.00 | 110.00 | W |
| 4583 | O | HOH | 139 | 25.536 | 31.838 | -27.978 | 1.00 | 118.35 | W |
| 4584 | O | HOH | 140 | 43.859 | 28.725 | 11.124 | 1.00 | 88.60 | W |
| 4585 | O | HOH | 141 | 39.097 | 47.972 | -32.369 | 1.00 | 104.31 | W |

TABLE 2 (continued)

| ATOMIC STRUCTURE COORDINATE DATA OBTAINED FROM X-RAY DIFFRACTION FROM THE LIGAND BINDING DOMAIN OF GRα IN COMPLEX WITH FLUTICASONE PROPIONATE AND A TIF2 FRAGMENT | | | | | | | | | |
|---|---|---|---|---|---|---|---|---|---|
| ATOM | ATOM TYPE | RESIDUE | # | X | Y | Z | OCC | B | ATOM |
| 4586 | O | HOH | 142 | 25.807 | 33.585 | -30.771 | 1.00 | 99.66 | W |
| 4587 | O | HOH | 143 | 39.247 | 53.006 | -1.353 | 1.00 | 94.50 | W |
| 4588 | O | HOH | 144 | 45.655 | -13.890 | -5.094 | 1.00 | 92.07 | W |
| 4589 | O | HOH | 145 | 34.471 | -14.556 | 13.467 | 1.00 | 82.57 | W |

TABLE 3

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1 | CB | GLN | 527 | 60.207 | 9.806 | 35.497 | 1.00 | 60.77 |
| 2 | CG | GLN | 527 | 60.501 | 11.318 | 35.564 | 1.00 | 60.74 |
| 3 | CD | GLN | 527 | 60.595 | 11.993 | 34.172 | 1.00 | 63.52 |
| 4 | OE1 | GLN | 527 | 60.493 | 13.224 | 34.058 | 1.00 | 61.80 |
| 5 | NE2 | GLN | 527 | 60.794 | 11.187 | 33.121 | 1.00 | 61.21 |
| 6 | C | GLN | 527 | 62.073 | 8.590 | 36.647 | 1.00 | 62.83 |
| 7 | O | GLN | 527 | 63.240 | 8.191 | 36.724 | 1.00 | 59.67 |
| 8 | N | GLN | 527 | 61.009 | 7.618 | 34.618 | 1.00 | 58.91 |
| 9 | CA | GLN | 527 | 61.426 | 8.890 | 35.289 | 1.00 | 62.13 |
| 10 | N | LEU | 528 | 61.308 | 8.776 | 37.716 | 1.00 | 62.73 |
| 11 | CA | LEU | 528 | 61.816 | 8.538 | 39.064 | 1.00 | 65.02 |
| 12 | CB | LEU | 528 | 62.105 | 9.889 | 39.733 | 1.00 | 62.65 |
| 13 | CG | LEU | 528 | 62.864 | 10.872 | 38.813 | 1.00 | 59.23 |
| 14 | CD1 | LEU | 528 | 62.071 | 12.198 | 38.675 | 1.00 | 63.52 |
| 15 | CD2 | LEU | 528 | 64.283 | 11.105 | 39.356 | 1.00 | 60.04 |
| 16 | C | LEU | 528 | 60.823 | 7.690 | 39.888 | 1.00 | 59.38 |
| 17 | O | LEU | 528 | 60.586 | 6.527 | 39.527 | 1.00 | 63.35 |
| 18 | N | THR | 529 | 60.247 | 8.256 | 40.960 | 1.00 | 60.40 |
| 19 | CA | THR | 529 | 59.282 | 7.539 | 41.835 | 1.00 | 60.79 |
| 20 | CB | THR | 529 | 57.841 | 8.227 | 41.847 | 1.00 | 63.67 |
| 21 | OG1 | THR | 529 | 57.918 | 9.561 | 42.382 | 1.00 | 60.60 |
| 22 | CG2 | THR | 529 | 56.867 | 7.410 | 42.706 | 1.00 | 62.04 |
| 23 | C | THR | 529 | 59.134 | 6.056 | 41.397 | 1.00 | 61.38 |
| 24 | O | THR | 529 | 58.454 | 5.754 | 40.398 | 1.00 | 59.93 |
| 25 | N | PRO | 530 | 59.743 | 5.117 | 42.163 | 1.00 | 61.16 |
| 26 | CD | PRO | 530 | 60.110 | 5.411 | 43.563 | 1.00 | 60.38 |
| 27 | CA | PRO | 530 | 59.753 | 3.660 | 41.928 | 1.00 | 62.39 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 28 | CB | PRO | 530 | 60.388 | 3.109 | 43.213 | 1.00 | 58.06 |
| 29 | CG | PRO | 530 | 59.914 | 4.071 | 44.249 | 1.00 | 64.31 |
| 30 | C | PRO | 530 | 58.453 | 2.927 | 41.537 | 1.00 | 63.39 |
| 31 | O | PRO | 530 | 57.400 | 3.542 | 41.363 | 1.00 | 59.17 |
| 32 | N | THR | 531 | 58.554 | 1.603 | 41.419 | 1.00 | 62.27 |
| 33 | CA | THR | 531 | 57.455 | 0.742 | 40.997 | 1.00 | 61.68 |
| 34 | CB | THR | 531 | 57.989 | -0.404 | 40.058 | 1.00 | 60.38 |
| 35 | OG1 | THR | 531 | 57.209 | -0.461 | 38.853 | 1.00 | 60.25 |
| 36 | CG2 | THR | 531 | 57.937 | -1.760 | 40.757 | 1.00 | 60.67 |
| 37 | C | THR | 531 | 56.629 | 0.125 | 42.117 | 1.00 | 60.82 |
| 38 | O | THR | 531 | 55.533 | -0.361 | 41.864 | 1.00 | 62.20 |
| 39 | N | LEU | 532 | 57.122 | 0.128 | 43.348 | 1.00 | 60.85 |
| 40 | CA | LEU | 532 | 56.324 | -0.465 | 44.418 | 1.00 | 60.11 |
| 41 | CB | LEU | 532 | 57.183 | -0.775 | 45.637 | 1.00 | 64.22 |
| 42 | CG | LEU | 532 | 56.388 | -1.514 | 46.704 | 1.00 | 63.74 |
| 43 | CD1 | LEU | 532 | 55.677 | -2.694 | 46.082 | 1.00 | 62.66 |
| 44 | CD2 | LEU | 532 | 57.317 | -1.968 | 47.806 | 1.00 | 63.22 |
| 45 | C | LEU | 532 | 55.143 | 0.422 | 44.817 | 1.00 | 62.08 |
| 46 | O | LEU | 532 | 54.047 | -0.075 | 45.061 | 1.00 | 61.27 |
| 47 | N | VAL | 533 | 55.366 | 1.733 | 44.883 | 1.00 | 59.27 |
| 48 | CA | VAL | 533 | 54.297 | 2.677 | 45.222 | 1.00 | 62.90 |
| 49 | CB | VAL | 533 | 54.858 | 4.050 | 45.638 | 1.00 | 64.91 |
| 50 | CG1 | VAL | 533 | 55.572 | 4.693 | 44.465 | 1.00 | 60.86 |
| 51 | CG2 | VAL | 533 | 53.746 | 4.941 | 46.102 | 1.00 | 61.00 |
| 52 | C | VAL | 533 | 53.422 | 2.874 | 43.979 | 1.00 | 62.21 |
| 53 | O | VAL | 533 | 52.281 | 3.321 | 44.065 | 1.00 | 61.72 |
| 54 | N | SER | 534 | 53.981 | 2.553 | 42.817 | 1.00 | 60.92 |
| 55 | CA | SER | 534 | 53.249 | 2.665 | 41.564 | 1.00 | 61.24 |
| 56 | CB | SER | 534 | 54.196 | 2.474 | 40.386 | 1.00 | 61.92 |
| 57 | OG | SER | 534 | 53.468 | 2.355 | 39.183 | 1.00 | 61.38 |
| 58 | C | SER | 534 | 52.209 | 1.557 | 41.566 | 1.00 | 64.31 |
| 59 | O | SER | 534 | 51.105 | 1.691 | 41.027 | 1.00 | 62.62 |
| 60 | N | LEU | 535 | 52.581 | 0.452 | 42.193 | 1.00 | 61.91 |
| 61 | CA | LEU | 535 | 51.697 | -0.684 | 42.288 | 1.00 | 60.97 |
| 62 | CB | LEU | 535 | 52.479 | -1.922 | 42.730 | 1.00 | 66.65 |
| 63 | CG | LEU | 535 | 51.949 | -3.225 | 42.131 | 1.00 | 63.58 |
| 64 | CD1 | LEU | 535 | 52.657 | -3.505 | 40.827 | 1.00 | 62.14 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 65 | CD2 | LEU | 535 | 52.175 | -4.364 | 43.090 | 1.00 | 61.95 |
| 66 | C | LEU | 535 | 50.588 | -0.353 | 43.285 | 1.00 | 59.91 |
| 67 | O | LEU | 535 | 49.432 | -0.684 | 43.060 | 1.00 | 63.02 |
| 68 | N | LEU | 536 | 50.933 | 0.315 | 44.381 | 1.00 | 62.58 |
| 69 | CA | LEU | 536 | 49.932 | 0.683 | 45.376 | 1.00 | 59.17 |
| 70 | CB | LEU | 536 | 50.583 | 1.413 | 46.541 | 1.00 | 61.74 |
| 71 | CG | LEU | 536 | 51.501 | 0.625 | 47.460 | 1.00 | 58.87 |
| 72 | CD1 | LEU | 536 | 51.953 | 1.545 | 48.553 | 1.00 | 59.54 |
| 73 | CD2 | LEU | 536 | 50.781 | -0.575 | 48.045 | 1.00 | 63.64 |
| 74 | C | LEU | 536 | 48.821 | 1.569 | 44.812 | 1.00 | 63.31 |
| 75 | O | LEU | 536 | 47.672 | 1.489 | 45.256 | 1.00 | 61.67 |
| 76 | N | GLU | 537 | 49.171 | 2.415 | 43.845 | 1.00 | 59.21 |
| 77 | CA | GLU | 537 | 48.231 | 3.343 | 43.213 | 1.00 | 59.76 |
| 78 | CB | GLU | 537 | 48.984 | 4.292 | 42.302 | 1.00 | 59.81 |
| 79 | CG | GLU | 537 | 48.816 | 5.744 | 42.625 | 1.00 | 60.10 |
| 80 | CD | GLU | 537 | 48.907 | 6.616 | 41.385 | 1.00 | 64.34 |
| 81 | OE1 | GLU | 537 | 47.868 | 6.813 | 40.707 | 1.00 | 57.41 |
| 82 | OE2 | GLU | 537 | 50.024 | 7.091 | 41.084 | 1.00 | 62.84 |
| 83 | C | GLU | 537 | 47.139 | 2.698 | 42.371 | 1.00 | 61.66 |
| 84 | O | GLU | 537 | 45.973 | 3.101 | 42.433 | 1.00 | 60.28 |
| 85 | N | VAL | 538 | 47.536 | 1.717 | 41.564 | 1.00 | 60.48 |
| 86 | CA | VAL | 538 | 46.606 | 1.045 | 40.674 | 1.00 | 63.41 |
| 87 | CB | VAL | 538 | 47.325 | 0.448 | 39.442 | 1.00 | 64.15 |
| 88 | CG1 | VAL | 538 | 48.334 | 1.444 | 38.903 | 1.00 | 60.29 |
| 89 | CG2 | VAL | 538 | 47.973 | -0.883 | 39.797 | 1.00 | 63.88 |
| 90 | C | VAL | 538 | 45.768 | -0.046 | 41.311 | 1.00 | 57.99 |
| 91 | O | VAL | 538 | 44.828 | -0.530 | 40.683 | 1.00 | 58.71 |
| 92 | N | ILE | 539 | 46.094 | -0.454 | 42.535 | 1.00 | 61.14 |
| 93 | CA | ILE | 539 | 45.282 | -1.484 | 43.186 | 1.00 | 60.23 |
| 94 | CB | ILE | 539 | 46.141 | -2.499 | 44.010 | 1.00 | 65.32 |
| 95 | CG2 | ILE | 539 | 47.243 | -3.066 | 43.140 | 1.00 | 61.32 |
| 96 | CG1 | ILE | 539 | 46.775 | -1.833 | 45.228 | 1.00 | 63.80 |
| 97 | CD1 | ILE | 539 | 47.356 | -2.833 | 46.207 | 1.00 | 60.85 |
| 98 | C | ILE | 539 | 44.259 | -0.811 | 44.097 | 1.00 | 61.40 |
| 99 | O | ILE | 539 | 43.321 | -1.447 | 44.573 | 1.00 | 63.49 |
| 100 | N | GLU | 540 | 44.451 | 0.489 | 44.310 | 1.00 | 61.12 |
| 101 | CA | GLU | 540 | 43.584 | 1.307 | 45.153 | 1.00 | 60.76 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 102 | CB | GLU | 540 | 44.109 | 2.753 | 45.173 | 1.00 | 58.26 |
| 103 | CG | GLU | 540 | 43.466 | 3.684 | 46.191 | 1.00 | 61.15 |
| 104 | CD | GLU | 540 | 43.598 | 3.183 | 47.619 | 1.00 | 61.95 |
| 105 | OE1 | GLU | 540 | 44.656 | 2.591 | 47.950 | 1.00 | 59.71 |
| 106 | OE2 | GLU | 540 | 42.649 | 3.397 | 48.410 | 1.00 | 62.96 |
| 107 | C | GLU | 540 | 42.169 | 1.264 | 44.585 | 1.00 | 61.78 |
| 108 | O | GLU | 540 | 41.928 | 1.709 | 43.459 | 1.00 | 61.36 |
| 109 | N | PRO | 541 | 41.214 | 0.713 | 45.352 | 1.00 | 63.77 |
| 110 | CD | PRO | 541 | 41.365 | 0.053 | 46.659 | 1.00 | 58.98 |
| 111 | CA | PRO | 541 | 39.830 | 0.632 | 44.876 | 1.00 | 60.14 |
| 112 | CB | PRO | 541 | 39.131 | -0.149 | 45.988 | 1.00 | 59.62 |
| 113 | CG | PRO | 541 | 39.978 | 0.122 | 47.195 | 1.00 | 60.56 |
| 114 | C | PRO | 541 | 39.180 | 1.991 | 44.592 | 1.00 | 62.36 |
| 115 | O | PRO | 541 | 39.455 | 2.982 | 45.283 | 1.00 | 59.45 |
| 116 | N | GLU | 542 | 38.332 | 2.039 | 43.563 | 1.00 | 60.43 |
| 117 | CA | GLU | 542 | 37.653 | 3.279 | 43.198 | 1.00 | 62.04 |
| 118 | CB | GLU | 542 | 37.091 | 3.201 | 41.770 | 1.00 | 62.84 |
| 119 | CG | GLU | 542 | 36.130 | 2.050 | 41.511 | 1.00 | 63.24 |
| 120 | CD | GLU | 542 | 35.745 | 1.911 | 40.031 | 1.00 | 63.39 |
| 121 | OE1 | GLU | 542 | 36.622 | 2.095 | 39.153 | 1.00 | 60.50 |
| 122 | OE2 | GLU | 542 | 34.568 | 1.599 | 39.743 | 1.00 | 59.31 |
| 123 | C | GLU | 542 | 36.548 | 3.515 | 44.208 | 1.00 | 63.11 |
| 124 | O | GLU | 542 | 35.941 | 2.564 | 44.697 | 1.00 | 59.70 |
| 125 | N | VAL | 543 | 36.304 | 4.783 | 44.528 | 1.00 | 61.53 |
| 126 | CA | VAL | 543 | 35.299 | 5.148 | 45.518 | 1.00 | 63.47 |
| 127 | CB | VAL | 543 | 35.334 | 6.661 | 45.801 | 1.00 | 62.60 |
| 128 | CG1 | VAL | 543 | 34.467 | 6.984 | 46.987 | 1.00 | 60.93 |
| 129 | CG2 | VAL | 543 | 36.762 | 7.103 | 46.064 | 1.00 | 59.59 |
| 130 | C | VAL | 543 | 33.886 | 4.748 | 45.126 | 1.00 | 61.39 |
| 131 | O | VAL | 543 | 33.495 | 4.877 | 43.965 | 1.00 | 60.79 |
| 132 | N | LEU | 544 | 33.128 | 4.267 | 46.109 | 1.00 | 62.56 |
| 133 | CA | LEU | 544 | 31.759 | 3.836 | 45.882 | 1.00 | 60.63 |
| 134 | CB | LEU | 544 | 31.501 | 2.486 | 46.547 | 1.00 | 63.18 |
| 135 | CG | LEU | 544 | 32.666 | 1.512 | 46.682 | 1.00 | 61.92 |
| 136 | CD1 | LEU | 544 | 33.702 | 2.114 | 47.638 | 1.00 | 62.67 |
| 137 | CD2 | LEU | 544 | 32.163 | 0.172 | 47.225 | 1.00 | 61.02 |
| 138 | C | LEU | 544 | 30.754 | 4.844 | 46.423 | 1.00 | 58.48 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 139 | O | LEU | 544 | 31.097 | 5.715 | 47.225 | 1.00 | 59.01 |
| 140 | N | TYR | 545 | 29.508 | 4.698 | 45.974 | 1.00 | 60.35 |
| 141 | CA | TYR | 545 | 28.394 | 5.559 | 46.356 | 1.00 | 58.86 |
| 142 | CB | TYR | 545 | 27.616 | 5.977 | 45.105 | 1.00 | 59.62 |
| 143 | CG | TYR | 545 | 28.421 | 6.799 | 44.122 | 1.00 | 60.54 |
| 144 | CD1 | TYR | 545 | 29.815 | 6.803 | 44.162 | 1.00 | 59.00 |
| 145 | CE1 | TYR | 545 | 30.561 | 7.563 | 43.270 | 1.00 | 61.22 |
| 146 | CD2 | TYR | 545 | 27.791 | 7.579 | 43.153 | 1.00 | 63.95 |
| 147 | CE2 | TYR | 545 | 28.534 | 8.348 | 42.256 | 1.00 | 59.17 |
| 148 | CZ | TYR | 545 | 29.914 | 8.336 | 42.325 | 1.00 | 60.43 |
| 149 | OH | TYR | 545 | 30.654 | 9.120 | 41.478 | 1.00 | 60.96 |
| 150 | C | TYR | 545 | 27.501 | 4.743 | 47.269 | 1.00 | 64.48 |
| 151 | O | TYR | 545 | 27.449 | 3.517 | 47.151 | 1.00 | 60.43 |
| 152 | N | ALA | 546 | 26.789 | 5.415 | 48.168 | 1.00 | 62.22 |
| 153 | CA | ALA | 546 | 25.918 | 4.720 | 49.112 | 1.00 | 61.72 |
| 154 | CB | ALA | 546 | 25.780 | 5.540 | 50.378 | 1.00 | 60.83 |
| 155 | C | ALA | 546 | 24.536 | 4.377 | 48.570 | 1.00 | 61.54 |
| 156 | O | ALA | 546 | 23.886 | 3.461 | 49.065 | 1.00 | 58.65 |
| 157 | N | GLY | 547 | 24.089 | 5.100 | 47.549 | 1.00 | 60.89 |
| 158 | CA | GLY | 547 | 22.768 | 4.841 | 47.014 | 1.00 | 59.44 |
| 159 | C | GLY | 547 | 21.765 | 5.212 | 48.088 | 1.00 | 59.45 |
| 160 | O | GLY | 547 | 20.849 | 4.460 | 48.392 | 1.00 | 58.64 |
| 161 | N | TYR | 548 | 21.966 | 6.387 | 48.671 | 1.00 | 59.64 |
| 162 | CA | TYR | 548 | 21.119 | 6.921 | 49.733 | 1.00 | 61.47 |
| 163 | CB | TYR | 548 | 21.912 | 7.970 | 50.520 | 1.00 | 64.12 |
| 164 | CG | TYR | 548 | 21.244 | 8.421 | 51.783 | 1.00 | 58.90 |
| 165 | CD1 | TYR | 548 | 21.049 | 7.534 | 52.833 | 1.00 | 61.61 |
| 166 | CE1 | TYR | 548 | 20.414 | 7.927 | 53.992 | 1.00 | 63.67 |
| 167 | CD2 | TYR | 548 | 20.785 | 9.726 | 51.926 | 1.00 | 60.05 |
| 168 | CE2 | TYR | 548 | 20.144 | 10.129 | 53.084 | 1.00 | 60.57 |
| 169 | CZ | TYR | 548 | 19.964 | 9.218 | 54.112 | 1.00 | 64.94 |
| 170 | OH | TYR | 548 | 19.319 | 9.579 | 55.262 | 1.00 | 63.47 |
| 171 | C | TYR | 548 | 19.907 | 7.569 | 49.080 | 1.00 | 63.87 |
| 172 | O | TYR | 548 | 19.755 | 7.481 | 47.867 | 1.00 | 60.53 |
| 173 | N | ASP | 549 | 19.043 | 8.207 | 49.871 | 1.00 | 60.86 |
| 174 | CA | ASP | 549 | 17.881 | 8.882 | 49.307 | 1.00 | 62.83 |
| 175 | CB | ASP | 549 | 16.590 | 8.410 | 49.958 | 1.00 | 61.82 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 176 | CG | ASP | 549 | 15.487 | 8.213 | 48.935 | 1.00 | 59.25 |
| 177 | OD1 | ASP | 549 | 14.321 | 7.942 | 49.306 | 1.00 | 61.06 |
| 178 | OD2 | ASP | 549 | 15.810 | 8.328 | 47.734 | 1.00 | 61.75 |
| 179 | C | ASP | 549 | 17.979 | 10.402 | 49.411 | 1.00 | 62.83 |
| 180 | O | ASP | 549 | 18.158 | 11.075 | 48.400 | 1.00 | 60.57 |
| 181 | N | SER | 550 | 17.875 | 10.954 | 50.617 | 1.00 | 59.81 |
| 182 | CA | SER | 550 | 17.953 | 12.415 | 50.793 | 1.00 | 62.38 |
| 183 | CB | SER | 550 | 19.325 | 12.951 | 50.386 | 1.00 | 56.99 |
| 184 | OG | SER | 550 | 19.438 | 13.020 | 48.978 | 1.00 | 62.06 |
| 185 | C | SER | 550 | 16.894 | 13.126 | 49.957 | 1.00 | 62.44 |
| 186 | O | SER | 550 | 16.893 | 14.350 | 49.843 | 1.00 | 61.89 |
| 187 | N | SER | 551 | 16.018 | 12.343 | 49.343 | 1.00 | 61.48 |
| 188 | CA | SER | 551 | 14.924 | 12.875 | 48.557 | 1.00 | 60.05 |
| 189 | CB | SER | 551 | 14.507 | 11.886 | 47.487 | 1.00 | 62.39 |
| 190 | OG | SER | 551 | 13.838 | 10.800 | 48.100 | 1.00 | 61.65 |
| 191 | C | SER | 551 | 13.850 | 12.904 | 49.615 | 1.00 | 60.87 |
| 192 | O | SER | 551 | 12.799 | 13.512 | 49.452 | 1.00 | 59.31 |
| 193 | N | VAL | 552 | 14.142 | 12.200 | 50.703 | 1.00 | 61.91 |
| 194 | CA | VAL | 552 | 13.252 | 12.096 | 51.849 | 1.00 | 60.13 |
| 195 | CB | VAL | 552 | 12.584 | 10.695 | 51.895 | 1.00 | 60.55 |
| 196 | CG1 | VAL | 552 | 11.242 | 10.744 | 51.187 | 1.00 | 59.77 |
| 197 | CG2 | VAL | 552 | 13.461 | 9.674 | 51.211 | 1.00 | 62.73 |
| 198 | C | VAL | 552 | 14.035 | 12.388 | 53.141 | 1.00 | 58.44 |
| 199 | O | VAL | 552 | 15.269 | 12.482 | 53.116 | 1.00 | 60.59 |
| 200 | N | PRO | 553 | 13.326 | 12.571 | 54.278 | 1.00 | 59.91 |
| 201 | CD | PRO | 553 | 11.861 | 12.614 | 54.440 | 1.00 | 61.19 |
| 202 | CA | PRO | 553 | 13.974 | 12.859 | 55.559 | 1.00 | 59.95 |
| 203 | CB | PRO | 553 | 12.865 | 12.572 | 56.556 | 1.00 | 62.02 |
| 204 | CG | PRO | 553 | 11.701 | 13.166 | 55.851 | 1.00 | 62.09 |
| 205 | C | PRO | 553 | 15.263 | 12.093 | 55.839 | 1.00 | 62.80 |
| 206 | O | PRO | 553 | 15.525 | 11.035 | 55.259 | 1.00 | 61.14 |
| 207 | N | ASP | 554 | 16.058 | 12.646 | 56.748 | 1.00 | 58.85 |
| 208 | CA | ASP | 554 | 17.357 | 12.084 | 57.104 | 1.00 | 60.06 |
| 209 | CB | ASP | 554 | 18.462 | 13.098 | 56.755 | 1.00 | 61.56 |
| 210 | CG | ASP | 554 | 18.836 | 13.106 | 55.280 | 1.00 | 62.42 |
| 211 | OD1 | ASP | 554 | 17.961 | 12.964 | 54.390 | 1.00 | 59.77 |
| 212 | OD2 | ASP | 554 | 20.038 | 13.286 | 55.014 | 1.00 | 59.95 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 213 | C | ASP | 554 | 17.535 | 11.703 | 58.575 | 1.00 | 56.92 |
| 214 | O | ASP | 554 | 18.402 | 12.273 | 59.229 | 1.00 | 61.20 |
| 215 | N | SER | 555 | 16.767 | 10.761 | 59.116 | 1.00 | 62.60 |
| 216 | CA | SER | 555 | 16.970 | 10.398 | 60.526 | 1.00 | 63.72 |
| 217 | CB | SER | 555 | 15.998 | 9.296 | 60.948 | 1.00 | 63.32 |
| 218 | OG | SER | 555 | 16.267 | 8.089 | 60.255 | 1.00 | 60.75 |
| 219 | C | SER | 555 | 18.404 | 9.905 | 60.749 | 1.00 | 61.32 |
| 220 | O | SER | 555 | 19.093 | 9.556 | 59.794 | 1.00 | 59.49 |
| 221 | N | THR | 556 | 18.855 | 9.877 | 62.002 | 1.00 | 63.20 |
| 222 | CA | THR | 556 | 20.211 | 9.407 | 62.308 | 1.00 | 62.68 |
| 223 | CB | THR | 556 | 20.554 | 9.487 | 63.826 | 1.00 | 62.64 |
| 224 | OG1 | THR | 556 | 20.893 | 10.831 | 64.183 | 1.00 | 62.26 |
| 225 | CG2 | THR | 556 | 21.739 | 8.582 | 64.158 | 1.00 | 62.40 |
| 226 | C | THR | 556 | 20.387 | 7.955 | 61.902 | 1.00 | 62.17 |
| 227 | O | THR | 556 | 21.196 | 7.633 | 61.030 | 1.00 | 63.77 |
| 228 | N | TRP | 557 | 19.624 | 7.082 | 62.554 | 1.00 | 63.04 |
| 229 | CA | TRP | 557 | 19.696 | 5.652 | 62.294 | 1.00 | 60.39 |
| 230 | CB | TRP | 557 | 18.505 | 4.923 | 62.964 | 1.00 | 61.26 |
| 231 | CG | TRP | 557 | 17.324 | 4.805 | 62.064 | 1.00 | 64.02 |
| 232 | CD2 | TRP | 557 | 17.074 | 3.747 | 61.123 | 1.00 | 60.71 |
| 233 | CE2 | TRP | 557 | 15.970 | 4.142 | 60.332 | 1.00 | 58.32 |
| 234 | CE3 | TRP | 557 | 17.684 | 2.511 | 60.865 | 1.00 | 62.45 |
| 235 | CD1 | TRP | 557 | 16.378 | 5.760 | 61.825 | 1.00 | 60.50 |
| 236 | NE1 | TRP | 557 | 15.562 | 5.373 | 60.780 | 1.00 | 59.73 |
| 237 | CZ2 | TRP | 557 | 15.464 | 3.341 | 59.296 | 1.00 | 61.79 |
| 238 | CZ3 | TRP | 557 | 17.184 | 1.716 | 59.836 | 1.00 | 62.55 |
| 239 | CH2 | TRP | 557 | 16.084 | 2.136 | 59.065 | 1.00 | 57.76 |
| 240 | C | TRP | 557 | 19.731 | 5.362 | 60.783 | 1.00 | 61.95 |
| 241 | O | TRP | 557 | 20.479 | 4.493 | 60.332 | 1.00 | 59.61 |
| 242 | N | ARG | 558 | 18.946 | 6.099 | 60.001 | 1.00 | 61.85 |
| 243 | CA | ARG | 558 | 18.898 | 5.873 | 58.555 | 1.00 | 64.57 |
| 244 | CB | ARG | 558 | 17.744 | 6.651 | 57.926 | 1.00 | 59.03 |
| 245 | CG | ARG | 558 | 17.303 | 6.107 | 56.582 | 1.00 | 62.63 |
| 246 | CD | ARG | 558 | 16.012 | 6.780 | 56.133 | 1.00 | 59.07 |
| 247 | NE | ARG | 558 | 16.221 | 7.958 | 55.288 | 1.00 | 61.76 |
| 248 | CZ | ARG | 558 | 16.594 | 7.911 | 54.011 | 1.00 | 63.52 |
| 249 | NH1 | ARG | 558 | 16.805 | 6.745 | 53.420 | 1.00 | 63.08 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 250 | NH2 | ARG | 558 | 16.750 | 9.031 | 53.319 | 1.00 | 60.97 |
| 251 | C | ARG | 558 | 20.200 | 6.222 | 57.841 | 1.00 | 64.20 |
| 252 | O | ARG | 558 | 20.573 | 5.566 | 56.869 | 1.00 | 65.47 |
| 253 | N | ILE | 559 | 20.877 | 7.266 | 58.307 | 1.00 | 62.87 |
| 254 | CA | ILE | 559 | 22.156 | 7.678 | 57.726 | 1.00 | 59.66 |
| 255 | CB | ILE | 559 | 22.639 | 9.040 | 58.329 | 1.00 | 62.98 |
| 256 | CG2 | ILE | 559 | 24.101 | 9.278 | 57.993 | 1.00 | 59.64 |
| 257 | CG1 | ILE | 559 | 21.794 | 10.196 | 57.791 | 1.00 | 61.72 |
| 258 | CD1 | ILE | 559 | 22.091 | 10.556 | 56.351 | 1.00 | 60.58 |
| 259 | C | ILE | 559 | 23.152 | 6.585 | 58.119 | 1.00 | 62.05 |
| 260 | O | ILE | 559 | 23.838 | 5.995 | 57.274 | 1.00 | 61.48 |
| 261 | N | MET | 560 | 23.188 | 6.332 | 59.425 | 1.00 | 60.47 |
| 262 | CA | MET | 560 | 24.056 | 5.340 | 60.036 | 1.00 | 59.72 |
| 263 | CB | MET | 560 | 23.799 | 5.286 | 61.554 | 1.00 | 61.50 |
| 264 | CG | MET | 560 | 24.863 | 6.016 | 62.358 | 1.00 | 59.68 |
| 265 | SD | MET | 560 | 24.765 | 5.946 | 64.183 | 1.00 | 62.00 |
| 266 | CE | MET | 560 | 25.827 | 7.029 | 64.314 | 1.00 | 56.75 |
| 267 | C | MET | 560 | 23.910 | 3.950 | 59.421 | 1.00 | 63.13 |
| 268 | O | MET | 560 | 24.908 | 3.299 | 59.122 | 1.00 | 60.43 |
| 269 | N | THR | 561 | 22.680 | 3.493 | 59.215 | 1.00 | 59.86 |
| 270 | CA | THR | 561 | 22.487 | 2.183 | 58.619 | 1.00 | 62.03 |
| 271 | CB | THR | 561 | 21.005 | 1.771 | 58.603 | 1.00 | 60.73 |
| 272 | OG1 | THR | 561 | 20.483 | 1.777 | 59.938 | 1.00 | 58.44 |
| 273 | CG2 | THR | 561 | 20.862 | 0.370 | 58.025 | 1.00 | 59.27 |
| 274 | C | THR | 561 | 23.005 | 2.192 | 57.190 | 1.00 | 62.25 |
| 275 | O | THR | 561 | 23.565 | 1.211 | 56.724 | 1.00 | 59.53 |
| 276 | N | THR | 562 | 22.813 | 3.296 | 56.482 | 1.00 | 61.35 |
| 277 | CA | THR | 562 | 23.305 | 3.365 | 55.112 | 1.00 | 62.18 |
| 278 | CB | THR | 562 | 22.728 | 4.593 | 54.342 | 1.00 | 58.86 |
| 279 | OG1 | THR | 562 | 21.338 | 4.375 | 54.051 | 1.00 | 58.36 |
| 280 | CG2 | THR | 562 | 23.473 | 4.805 | 53.033 | 1.00 | 58.66 |
| 281 | C | THR | 562 | 24.830 | 3.432 | 55.157 | 1.00 | 62.40 |
| 282 | O | THR | 562 | 25.509 | 3.011 | 54.225 | 1.00 | 59.62 |
| 283 | N | LEU | 563 | 25.374 | 3.949 | 56.252 | 1.00 | 60.06 |
| 284 | CA | LEU | 563 | 26.825 | 4.026 | 56.382 | 1.00 | 61.98 |
| 285 | CB | LEU | 563 | 27.230 | 5.045 | 57.451 | 1.00 | 59.10 |
| 286 | CG | LEU | 563 | 27.004 | 6.519 | 57.119 | 1.00 | 62.03 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 287 | CD1 | LEU | 563 | 27.667 | 7.377 | 58.180 | 1.00 | 59.83 |
| 288 | CD2 | LEU | 563 | 27.574 | 6.827 | 55.745 | 1.00 | 63.51 |
| 289 | C | LEU | 563 | 27.406 | 2.657 | 56.730 | 1.00 | 63.21 |
| 290 | O | LEU | 563 | 28.592 | 2.410 | 56.529 | 1.00 | 59.14 |
| 291 | N | ASN | 564 | 26.567 | 1.773 | 57.264 | 1.00 | 59.68 |
| 292 | CA | ASN | 564 | 27.001 | 0.427 | 57.606 | 1.00 | 62.50 |
| 293 | CB | ASN | 564 | 26.110 | -0.166 | 58.689 | 1.00 | 61.63 |
| 294 | CG | ASN | 564 | 26.456 | 0.349 | 60.058 | 1.00 | 62.37 |
| 295 | OD1 | ASN | 564 | 27.625 | 0.513 | 60.381 | 1.00 | 62.36 |
| 296 | ND2 | ASN | 564 | 25.447 | 0.590 | 60.881 | 1.00 | 59.03 |
| 297 | C | ASN | 564 | 26.949 | -0.442 | 56.356 | 1.00 | 61.40 |
| 298 | O | ASN | 564 | 27.823 | -1.266 | 56.121 | 1.00 | 61.63 |
| 299 | N | MET | 565 | 25.923 | -0.251 | 55.543 | 1.00 | 62.42 |
| 300 | CA | MET | 565 | 25.804 | -1.022 | 54.320 | 1.00 | 63.45 |
| 301 | CB | MET | 565 | 24.483 | -0.701 | 53.632 | 1.00 | 59.10 |
| 302 | CG | MET | 565 | 23.266 | -0.999 | 54.488 | 1.00 | 62.52 |
| 303 | SD | MET | 565 | 23.154 | -2.742 | 54.883 | 1.00 | 63.35 |
| 304 | CE | MET | 565 | 22.918 | -2.702 | 56.627 | 1.00 | 60.16 |
| 305 | C | MET | 565 | 26.967 | -0.669 | 53.410 | 1.00 | 62.27 |
| 306 | O | MET | 565 | 27.475 | -1.509 | 52.677 | 1.00 | 62.01 |
| 307 | N | LEU | 566 | 27.382 | 0.590 | 53.462 | 1.00 | 62.25 |
| 308 | CA | LEU | 566 | 28.495 | 1.064 | 52.656 | 1.00 | 60.20 |
| 309 | CB | LEU | 566 | 28.596 | 2.594 | 52.741 | 1.00 | 59.50 |
| 310 | CG | LEU | 566 | 29.801 | 3.267 | 52.076 | 1.00 | 64.18 |
| 311 | CD1 | LEU | 566 | 29.685 | 3.195 | 50.565 | 1.00 | 61.46 |
| 312 | CD2 | LEU | 566 | 29.869 | 4.700 | 52.516 | 1.00 | 62.10 |
| 313 | C | LEU | 566 | 29.756 | 0.424 | 53.218 | 1.00 | 62.85 |
| 314 | O | LEU | 566 | 30.576 | -0.116 | 52.474 | 1.00 | 60.20 |
| 315 | N | GLY | 567 | 29.886 | 0.477 | 54.542 | 1.00 | 59.45 |
| 316 | CA | GLY | 567 | 31.040 | -0.095 | 55.207 | 1.00 | 59.94 |
| 317 | C | GLY | 567 | 31.316 | -1.516 | 54.768 | 1.00 | 60.71 |
| 318 | O | GLY | 567 | 32.461 | -1.890 | 54.520 | 1.00 | 59.79 |
| 319 | N | GLY | 568 | 30.261 | -2.310 | 54.667 | 1.00 | 61.99 |
| 320 | CA | GLY | 568 | 30.417 | -3.687 | 54.254 | 1.00 | 59.73 |
| 321 | C | GLY | 568 | 31.050 | -3.836 | 52.888 | 1.00 | 60.65 |
| 322 | O | GLY | 568 | 32.008 | -4.590 | 52.724 | 1.00 | 63.15 |
| 323 | N | ARG | 569 | 30.529 | -3.112 | 51.907 | 1.00 | 58.99 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 324 | CA | ARG | 569 | 31.049 | -3.208 | 50.557 | 1.00 | 60.81 |
| 325 | CB | ARG | 569 | 30.109 | -2.483 | 49.600 | 1.00 | 60.57 |
| 326 | CG | ARG | 569 | 28.696 | -3.029 | 49.701 | 1.00 | 61.60 |
| 327 | CD | ARG | 569 | 27.806 | -2.602 | 48.556 | 1.00 | 64.73 |
| 328 | NE | ARG | 569 | 27.561 | -1.168 | 48.564 | 1.00 | 61.17 |
| 329 | CZ | ARG | 569 | 27.939 | -0.352 | 47.590 | 1.00 | 60.00 |
| 330 | NH1 | ARG | 569 | 28.577 | -0.841 | 46.532 | 1.00 | 60.46 |
| 331 | NH2 | ARG | 569 | 27.681 | 0.946 | 47.680 | 1.00 | 63.69 |
| 332 | C | ARG | 569 | 32.462 | -2.676 | 50.447 | 1.00 | 61.15 |
| 333 | O | ARG | 569 | 33.249 | -3.137 | 49.620 | 1.00 | 60.59 |
| 334 | N | GLN | 570 | 32.788 | -1.713 | 51.295 | 1.00 | 60.73 |
| 335 | CA | GLN | 570 | 34.123 | -1.132 | 51.300 | 1.00 | 62.31 |
| 336 | CB | GLN | 570 | 34.143 | 0.150 | 52.120 | 1.00 | 59.04 |
| 337 | CG | GLN | 570 | 33.608 | 1.361 | 51.417 | 1.00 | 62.03 |
| 338 | CD | GLN | 570 | 33.782 | 2.606 | 52.247 | 1.00 | 56.35 |
| 339 | OE1 | GLN | 570 | 33.460 | 3.698 | 51.801 | 1.00 | 62.86 |
| 340 | NE2 | GLN | 570 | 34.295 | 2.449 | 53.467 | 1.00 | 63.17 |
| 341 | C | GLN | 570 | 35.144 | -2.093 | 51.882 | 1.00 | 61.15 |
| 342 | O | GLN | 570 | 36.293 | -2.134 | 51.441 | 1.00 | 60.50 |
| 343 | N | VAL | 571 | 34.732 | -2.837 | 52.903 | 1.00 | 60.99 |
| 344 | CA | VAL | 571 | 35.615 | -3.792 | 53.554 | 1.00 | 61.91 |
| 345 | CB | VAL | 571 | 35.054 | -4.200 | 54.930 | 1.00 | 58.42 |
| 346 | CG1 | VAL | 571 | 35.822 | -5.393 | 55.485 | 1.00 | 61.27 |
| 347 | CG2 | VAL | 571 | 35.160 | -3.007 | 55.891 | 1.00 | 60.58 |
| 348 | C | VAL | 571 | 35.805 | -5.007 | 52.665 | 1.00 | 62.66 |
| 349 | O | VAL | 571 | 36.698 | -5.820 | 52.885 | 1.00 | 58.99 |
| 350 | N | ILE | 572 | 34.958 | -5.116 | 51.652 | 1.00 | 63.61 |
| 351 | CA | ILE | 572 | 35.042 | -6.206 | 50.695 | 1.00 | 63.76 |
| 352 | CB | ILE | 572 | 33.649 | -6.539 | 50.103 | 1.00 | 60.98 |
| 353 | CG2 | ILE | 572 | 33.794 | -7.443 | 48.883 | 1.00 | 63.63 |
| 354 | CG1 | ILE | 572 | 32.782 | -7.192 | 51.183 | 1.00 | 61.03 |
| 355 | CD1 | ILE | 572 | 31.346 | -7.366 | 50.801 | 1.00 | 62.17 |
| 356 | C | ILE | 572 | 35.999 | -5.772 | 49.589 | 1.00 | 60.35 |
| 357 | O | ILE | 572 | 36.733 | -6.587 | 49.042 | 1.00 | 62.13 |
| 358 | N | ALA | 573 | 35.984 | -4.481 | 49.265 | 1.00 | 62.76 |
| 359 | CA | ALA | 573 | 36.879 | -3.936 | 48.251 | 1.00 | 58.47 |
| 360 | CB | ALA | 573 | 36.502 | -2.496 | 47.940 | 1.00 | 61.48 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 361 | C | ALA | 573 | 38.271 | -3.997 | 48.872 | 1.00 | 61.14 |
| 362 | O | ALA | 573 | 39.294 | -4.088 | 48.180 | 1.00 | 60.46 |
| 363 | N | ALA | 574 | 38.273 | -3.964 | 50.200 | 1.00 | 60.84 |
| 364 | CA | ALA | 574 | 39.477 | -4.008 | 51.003 | 1.00 | 61.42 |
| 365 | CB | ALA | 574 | 39.098 | -3.888 | 52.465 | 1.00 | 60.18 |
| 366 | C | ALA | 574 | 40.294 | -5.282 | 50.771 | 1.00 | 58.96 |
| 367 | O | ALA | 574 | 41.506 | -5.217 | 50.518 | 1.00 | 61.38 |
| 368 | N | VAL | 575 | 39.631 | -6.435 | 50.861 | 1.00 | 59.99 |
| 369 | CA | VAL | 575 | 40.296 | -7.720 | 50.664 | 1.00 | 59.60 |
| 370 | CB | VAL | 575 | 39.309 | -8.898 | 50.732 | 1.00 | 56.06 |
| 371 | CG1 | VAL | 575 | 40.070 | -10.197 | 50.570 | 1.00 | 62.55 |
| 372 | CG2 | VAL | 575 | 38.547 | -8.880 | 52.057 | 1.00 | 63.34 |
| 373 | C | VAL | 575 | 41.009 | -7.779 | 49.318 | 1.00 | 60.96 |
| 374 | O | VAL | 575 | 42.222 | -7.981 | 49.264 | 1.00 | 62.47 |
| 375 | N | LYS | 576 | 40.265 | -7.584 | 48.236 | 1.00 | 59.97 |
| 376 | CA | LYS | 576 | 40.851 | -7.628 | 46.901 | 1.00 | 62.25 |
| 377 | CB | LYS | 576 | 39.770 | -7.391 | 45.860 | 1.00 | 60.99 |
| 378 | CG | LYS | 576 | 40.115 | -7.866 | 44.462 | 1.00 | 61.35 |
| 379 | CD | LYS | 576 | 38.905 | -7.708 | 43.568 | 1.00 | 63.13 |
| 380 | CE | LYS | 576 | 37.667 | -8.234 | 44.276 | 1.00 | 62.07 |
| 381 | NZ | LYS | 576 | 36.420 | -7.912 | 43.531 | 1.00 | 59.76 |
| 382 | C | LYS | 576 | 41.957 | -6.593 | 46.742 | 1.00 | 63.69 |
| 383 | O | LYS | 576 | 42.673 | -6.573 | 45.742 | 1.00 | 59.32 |
| 384 | N | TRP | 577 | 42.074 | -5.723 | 47.734 | 1.00 | 62.59 |
| 385 | CA | TRP | 577 | 43.091 | -4.694 | 47.734 | 1.00 | 62.15 |
| 386 | CB | TRP | 577 | 42.556 | -3.432 | 48.424 | 1.00 | 60.50 |
| 387 | CG | TRP | 577 | 43.620 | -2.458 | 48.780 | 1.00 | 63.03 |
| 388 | CD2 | TRP | 577 | 44.140 | -2.200 | 50.090 | 1.00 | 58.79 |
| 389 | CE2 | TRP | 577 | 45.189 | -1.272 | 49.945 | 1.00 | 64.04 |
| 390 | CE3 | TRP | 577 | 43.824 | -2.668 | 51.372 | 1.00 | 60.56 |
| 391 | CD1 | TRP | 577 | 44.346 | -1.698 | 47.924 | 1.00 | 62.09 |
| 392 | NE1 | TRP | 577 | 45.293 | -0.983 | 48.611 | 1.00 | 61.93 |
| 393 | CZ2 | TRP | 577 | 45.930 | -0.798 | 51.032 | 1.00 | 61.40 |
| 394 | CZ3 | TRP | 577 | 44.566 | -2.197 | 52.458 | 1.00 | 59.59 |
| 395 | CH2 | TRP | 577 | 45.607 | -1.271 | 52.277 | 1.00 | 61.92 |
| 396 | C | TRP | 577 | 44.263 | -5.272 | 48.509 | 1.00 | 64.09 |
| 397 | O | TRP | 577 | 45.403 | -5.238 | 48.055 | 1.00 | 61.89 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 398 | N | ALA | 578 | 43.958 | -5.824 | 49.678 | 1.00 | 60.10 |
| 399 | CA | ALA | 578 | 44.974 | -6.411 | 50.541 | 1.00 | 61.99 |
| 400 | CB | ALA | 578 | 44.342 | -6.937 | 51.828 | 1.00 | 57.97 |
| 401 | C | ALA | 578 | 45.704 | -7.526 | 49.828 | 1.00 | 61.84 |
| 402 | O | ALA | 578 | 46.890 | -7.718 | 50.034 | 1.00 | 60.47 |
| 403 | N | LYS | 579 | 44.988 | -8.251 | 48.979 | 1.00 | 61.79 |
| 404 | CA | LYS | 579 | 45.573 | -9.354 | 48.233 | 1.00 | 60.65 |
| 405 | CB | LYS | 579 | 44.472 | -10.320 | 47.784 | 1.00 | 62.20 |
| 406 | CG | LYS | 579 | 43.479 | -10.656 | 48.893 | 1.00 | 63.14 |
| 407 | CD | LYS | 579 | 42.688 | -11.944 | 48.636 | 1.00 | 58.27 |
| 408 | CE | LYS | 579 | 41.775 | -11.862 | 47.419 | 1.00 | 58.47 |
| 409 | NZ | LYS | 579 | 41.093 | -13.167 | 47.129 | 1.00 | 62.99 |
| 410 | C | LYS | 579 | 46.389 | -8.895 | 47.024 | 1.00 | 61.02 |
| 411 | O | LYS | 579 | 47.014 | -9.713 | 46.356 | 1.00 | 63.16 |
| 412 | N | ALA | 580 | 46.383 | -7.596 | 46.738 | 1.00 | 61.84 |
| 413 | CA | ALA | 580 | 47.153 | -7.079 | 45.610 | 1.00 | 58.94 |
| 414 | CB | ALA | 580 | 46.339 | -6.062 | 44.817 | 1.00 | 61.14 |
| 415 | C | ALA | 580 | 48.439 | -6.446 | 46.137 | 1.00 | 60.40 |
| 416 | O | ALA | 580 | 49.378 | -6.190 | 45.374 | 1.00 | 60.45 |
| 417 | N | ILE | 581 | 48.465 | -6.197 | 47.445 | 1.00 | 60.82 |
| 418 | CA | ILE | 581 | 49.631 | -5.631 | 48.111 | 1.00 | 60.29 |
| 419 | CB | ILE | 581 | 49.375 | -5.412 | 49.630 | 1.00 | 58.24 |
| 420 | CG2 | ILE | 581 | 50.654 | -4.997 | 50.324 | 1.00 | 63.40 |
| 421 | CG1 | ILE | 581 | 48.295 | -4.353 | 49.847 | 1.00 | 62.12 |
| 422 | CD1 | ILE | 581 | 47.769 | -4.324 | 51.257 | 1.00 | 62.13 |
| 423 | C | ILE | 581 | 50.690 | -6.706 | 47.965 | 1.00 | 62.02 |
| 424 | O | ILE | 581 | 50.541 | -7.805 | 48.500 | 1.00 | 61.29 |
| 425 | N | PRO | 582 | 51.773 | -6.412 | 47.233 | 1.00 | 62.24 |
| 426 | CD | PRO | 582 | 52.137 | -5.123 | 46.623 | 1.00 | 61.16 |
| 427 | CA | PRO | 582 | 52.837 | -7.397 | 47.041 | 1.00 | 64.30 |
| 428 | CB | PRO | 582 | 53.983 | -6.563 | 46.486 | 1.00 | 61.32 |
| 429 | CG | PRO | 582 | 53.294 | -5.515 | 45.720 | 1.00 | 57.84 |
| 430 | C | PRO | 582 | 53.208 | -8.082 | 48.341 | 1.00 | 62.26 |
| 431 | O | PRO | 582 | 53.291 | -7.451 | 49.390 | 1.00 | 61.55 |
| 432 | N | GLY | 583 | 53.413 | -9.386 | 48.268 | 1.00 | 60.04 |
| 433 | CA | GLY | 583 | 53.788 | -10.138 | 49.447 | 1.00 | 63.24 |
| 434 | C | GLY | 583 | 52.721 | -10.313 | 50.509 | 1.00 | 58.28 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 435 | O | GLY | 583 | 52.976 | -10.962 | 51.519 | 1.00 | 61.03 |
| 436 | N | PHE | 584 | 51.527 | -9.758 | 50.320 | 1.00 | 59.53 |
| 437 | CA | PHE | 584 | 50.517 | -9.932 | 51.354 | 1.00 | 60.33 |
| 438 | CB | PHE | 584 | 49.356 | -8.960 | 51.200 | 1.00 | 59.83 |
| 439 | CG | PHE | 584 | 48.314 | -9.107 | 52.276 | 1.00 | 62.69 |
| 440 | CD1 | PHE | 584 | 48.583 | -8.699 | 53.576 | 1.00 | 65.80 |
| 441 | CD2 | PHE | 584 | 47.075 | -9.677 | 52.000 | 1.00 | 59.15 |
| 442 | CE1 | PHE | 584 | 47.636 | -8.854 | 54.586 | 1.00 | 63.90 |
| 443 | CE2 | PHE | 584 | 46.123 | -9.837 | 53.000 | 1.00 | 62.77 |
| 444 | CZ | PHE | 584 | 46.405 | -9.423 | 54.296 | 1.00 | 62.23 |
| 445 | C | PHE | 584 | 49.960 | -11.336 | 51.359 | 1.00 | 59.87 |
| 446 | O | PHE | 584 | 49.874 | -11.967 | 52.415 | 1.00 | 60.23 |
| 447 | N | ARG | 585 | 49.584 | -11.848 | 50.193 | 1.00 | 61.22 |
| 448 | CA | ARG | 585 | 49.021 | -13.183 | 50.207 | 1.00 | 62.87 |
| 449 | CB | ARG | 585 | 48.025 | -13.405 | 49.042 | 1.00 | 61.05 |
| 450 | CG | ARG | 585 | 48.486 | -13.212 | 47.602 | 1.00 | 62.14 |
| 451 | CD | ARG | 585 | 47.253 | -13.326 | 46.690 | 1.00 | 59.51 |
| 452 | NE | ARG | 585 | 46.321 | -14.325 | 47.226 | 1.00 | 57.83 |
| 453 | CZ | ARG | 585 | 45.253 | -14.826 | 46.592 | 1.00 | 62.40 |
| 454 | NH1 | ARG | 585 | 44.934 | -14.430 | 45.360 | 1.00 | 64.51 |
| 455 | NH2 | ARG | 585 | 44.509 | -15.752 | 47.194 | 1.00 | 62.15 |
| 456 | C | ARG | 585 | 50.053 | -14.290 | 50.303 | 1.00 | 61.04 |
| 457 | O | ARG | 585 | 49.781 | -15.436 | 49.962 | 1.00 | 59.18 |
| 458 | N | ASN | 586 | 51.232 | -13.935 | 50.811 | 1.00 | 59.32 |
| 459 | CA | ASN | 586 | 52.319 | -14.893 | 51.021 | 1.00 | 62.11 |
| 460 | CB | ASN | 586 | 53.659 | -14.329 | 50.545 | 1.00 | 57.88 |
| 461 | CG | ASN | 586 | 53.910 | -14.596 | 49.071 | 1.00 | 62.73 |
| 462 | OD1 | ASN | 586 | 54.772 | -13.964 | 48.450 | 1.00 | 64.87 |
| 463 | ND2 | ASN | 586 | 53.164 | -15.551 | 48.504 | 1.00 | 63.32 |
| 464 | C | ASN | 586 | 52.396 | -15.218 | 52.503 | 1.00 | 61.24 |
| 465 | O | ASN | 586 | 53.093 | -16.138 | 52.916 | 1.00 | 62.45 |
| 466 | N | LEU | 587 | 51.692 | -14.446 | 53.314 | 1.00 | 62.98 |
| 467 | CA | LEU | 587 | 51.677 | -14.732 | 54.735 | 1.00 | 63.89 |
| 468 | CB | LEU | 587 | 51.210 | -13.502 | 55.522 | 1.00 | 63.58 |
| 469 | CG | LEU | 587 | 52.163 | -12.299 | 55.501 | 1.00 | 63.59 |
| 470 | CD1 | LEU | 587 | 51.405 | -11.009 | 55.348 | 1.00 | 58.78 |
| 471 | CD2 | LEU | 587 | 52.967 | -12.280 | 56.773 | 1.00 | 60.86 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 472 | C | LEU | 587 | 50.679 | -15.879 | 54.848 | 1.00 | 61.92 |
| 473 | O | LEU | 587 | 50.000 | -16.209 | 53.865 | 1.00 | 61.57 |
| 474 | N | HIS | 588 | 50.598 | -16.497 | 56.024 | 1.00 | 57.84 |
| 475 | CA | HIS | 588 | 49.676 | -17.609 | 56.235 | 1.00 | 62.30 |
| 476 | CB | HIS | 588 | 49.674 | -18.016 | 57.710 | 1.00 | 63.47 |
| 477 | CG | HIS | 588 | 49.180 | -19.411 | 57.962 | 1.00 | 62.99 |
| 478 | CD2 | HIS | 588 | 49.817 | -20.502 | 58.447 | 1.00 | 58.47 |
| 479 | ND1 | HIS | 588 | 47.886 | -19.808 | 57.705 | 1.00 | 57.63 |
| 480 | CE1 | HIS | 588 | 47.748 | -21.083 | 58.021 | 1.00 | 60.07 |
| 481 | NE2 | HIS | 588 | 48.905 | -21.527 | 58.474 | 1.00 | 61.65 |
| 482 | C | HIS | 588 | 48.304 | -17.100 | 55.839 | 1.00 | 61.24 |
| 483 | O | HIS | 588 | 48.137 | -15.900 | 55.641 | 1.00 | 58.81 |
| 484 | N | LEU | 589 | 47.325 | -17.990 | 55.714 | 1.00 | 60.37 |
| 485 | CA | LEU | 589 | 45.990 | -17.542 | 55.346 | 1.00 | 63.23 |
| 486 | CB | LEU | 589 | 45.219 | -18.626 | 54.588 | 1.00 | 60.24 |
| 487 | CG | LEU | 589 | 44.233 | -18.127 | 53.516 | 1.00 | 59.80 |
| 488 | CD1 | LEU | 589 | 43.798 | -19.286 | 52.630 | 1.00 | 65.35 |
| 489 | CD2 | LEU | 589 | 43.025 | -17.486 | 54.148 | 1.00 | 61.31 |
| 490 | C | LEU | 589 | 45.249 | -17.184 | 56.616 | 1.00 | 59.85 |
| 491 | O | LEU | 589 | 44.150 | -16.645 | 56.563 | 1.00 | 59.20 |
| 492 | N | ASP | 590 | 45.852 | -17.469 | 57.763 | 1.00 | 60.93 |
| 493 | CA | ASP | 590 | 45.200 | -17.158 | 59.027 | 1.00 | 61.82 |
| 494 | CB | ASP | 590 | 45.551 | -18.204 | 60.097 | 1.00 | 62.69 |
| 495 | CG | ASP | 590 | 44.823 | -19.529 | 59.898 | 1.00 | 58.29 |
| 496 | OD1 | ASP | 590 | 44.642 | -19.955 | 58.738 | 1.00 | 59.06 |
| 497 | OD2 | ASP | 590 | 44.447 | -20.159 | 60.910 | 1.00 | 64.34 |
| 498 | C | ASP | 590 | 45.608 | -15.771 | 59.504 | 1.00 | 59.77 |
| 499 | O | ASP | 590 | 44.915 | -15.153 | 60.314 | 1.00 | 60.73 |
| 500 | N | ASP | 591 | 46.734 | -15.278 | 59.001 | 1.00 | 59.62 |
| 501 | CA | ASP | 591 | 47.211 | -13.960 | 59.401 | 1.00 | 61.48 |
| 502 | CB | ASP | 591 | 48.733 | -13.861 | 59.240 | 1.00 | 60.79 |
| 503 | CG | ASP | 591 | 49.479 | -14.900 | 60.065 | 1.00 | 57.79 |
| 504 | OD1 | ASP | 591 | 49.012 | -15.239 | 61.176 | 1.00 | 66.77 |
| 505 | OD2 | ASP | 591 | 50.543 | -15.366 | 59.606 | 1.00 | 65.51 |
| 506 | C | ASP | 591 | 46.531 | -12.927 | 58.529 | 1.00 | 62.56 |
| 507 | O | ASP | 591 | 46.255 | -11.812 | 58.967 | 1.00 | 59.26 |
| 508 | N | GLN | 592 | 46.278 | -13.323 | 57.285 | 1.00 | 62.10 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 509 | CA | GLN | 592 | 45.613 | -12.473 | 56.316 | 1.00 | 61.30 |
| 510 | CB | GLN | 592 | 45.432 | -13.227 | 54.998 | 1.00 | 62.26 |
| 511 | CG | GLN | 592 | 46.751 | -13.456 | 54.277 | 1.00 | 60.59 |
| 512 | CD | GLN | 592 | 46.595 | -14.100 | 52.909 | 1.00 | 59.32 |
| 513 | OE1 | GLN | 592 | 45.597 | -13.887 | 52.213 | 1.00 | 62.27 |
| 514 | NE2 | GLN | 592 | 47.600 | -14.875 | 52.505 | 1.00 | 62.12 |
| 515 | C | GLN | 592 | 44.269 | -12.097 | 56.906 | 1.00 | 64.75 |
| 516 | O | GLN | 592 | 43.768 | -10.993 | 56.706 | 1.00 | 62.08 |
| 517 | N | MET | 593 | 43.701 | -13.028 | 57.660 | 1.00 | 59.14 |
| 518 | CA | MET | 593 | 42.413 | -12.815 | 58.302 | 1.00 | 61.78 |
| 519 | CB | MET | 593 | 41.740 | -14.162 | 58.597 | 1.00 | 58.91 |
| 520 | CG | MET | 593 | 41.290 | -14.935 | 57.357 | 1.00 | 64.30 |
| 521 | SD | MET | 593 | 40.510 | -16.524 | 57.776 | 1.00 | 58.06 |
| 522 | CE | MET | 593 | 39.514 | -16.029 | 59.273 | 1.00 | 61.88 |
| 523 | C | MET | 593 | 42.571 | -12.014 | 59.594 | 1.00 | 61.38 |
| 524 | O | MET | 593 | 41.802 | -11.089 | 59.837 | 1.00 | 60.42 |
| 525 | N | THR | 594 | 43.565 | -12.361 | 60.415 | 1.00 | 61.83 |
| 526 | CA | THR | 594 | 43.781 | -11.648 | 61.674 | 1.00 | 61.88 |
| 527 | CB | THR | 594 | 44.924 | -12.267 | 62.518 | 1.00 | 57.74 |
| 528 | OG1 | THR | 594 | 45.252 | -13.571 | 62.023 | 1.00 | 65.18 |
| 529 | CG2 | THR | 594 | 44.489 | -12.393 | 63.977 | 1.00 | 62.23 |
| 530 | C | THR | 594 | 44.127 | -10.194 | 61.378 | 1.00 | 62.61 |
| 531 | O | THR | 594 | 43.673 | -9.279 | 62.071 | 1.00 | 60.17 |
| 532 | N | LEU | 595 | 44.927 | -9.987 | 60.337 | 1.00 | 60.31 |
| 533 | CA | LEU | 595 | 45.325 | -8.647 | 59.938 | 1.00 | 61.48 |
| 534 | CB | LEU | 595 | 46.372 | -8.712 | 58.826 | 1.00 | 62.70 |
| 535 | CG | LEU | 595 | 47.788 | -9.074 | 59.266 | 1.00 | 60.61 |
| 536 | CD1 | LEU | 595 | 48.711 | -9.054 | 58.067 | 1.00 | 63.20 |
| 537 | CD2 | LEU | 595 | 48.268 | -8.083 | 60.316 | 1.00 | 62.58 |
| 538 | C | LEU | 595 | 44.128 | -7.823 | 59.475 | 1.00 | 63.38 |
| 539 | O | LEU | 595 | 43.835 | -6.779 | 60.051 | 1.00 | 60.70 |
| 540 | N | LEU | 596 | 43.439 | -8.290 | 58.436 | 1.00 | 60.66 |
| 541 | CA | LEU | 596 | 42.282 | -7.571 | 57.924 | 1.00 | 60.47 |
| 542 | CB | LEU | 596 | 41.703 | -8.278 | 56.699 | 1.00 | 60.10 |
| 543 | CG | LEU | 596 | 42.351 | -7.811 | 55.392 | 1.00 | 60.73 |
| 544 | CD1 | LEU | 596 | 42.036 | -8.767 | 54.254 | 1.00 | 59.40 |
| 545 | CD2 | LEU | 596 | 41.859 | -6.407 | 55.073 | 1.00 | 62.48 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 546 | C | LEU | 596 | 41.223 | -7.424 | 58.989 | 1.00 | 58.32 |
| 547 | O | LEU | 596 | 40.451 | -6.480 | 58.965 | 1.00 | 61.48 |
| 548 | N | GLN | 597 | 41.201 | -8.354 | 59.935 | 1.00 | 62.48 |
| 549 | CA | GLN | 597 | 40.230 | -8.327 | 61.031 | 1.00 | 64.28 |
| 550 | CB | GLN | 597 | 40.128 | -9.712 | 61.685 | 1.00 | 59.49 |
| 551 | CG | GLN | 597 | 38.936 | -10.561 | 61.279 | 1.00 | 64.66 |
| 552 | CD | GLN | 597 | 38.972 | -11.940 | 61.920 | 1.00 | 60.50 |
| 553 | OE1 | GLN | 597 | 39.080 | -12.078 | 63.149 | 1.00 | 60.07 |
| 554 | NE2 | GLN | 597 | 38.881 | -12.975 | 61.087 | 1.00 | 65.01 |
| 555 | C | GLN | 597 | 40.612 | -7.314 | 62.110 | 1.00 | 61.61 |
| 556 | O | GLN | 597 | 39.780 | -6.933 | 62.932 | 1.00 | 61.06 |
| 557 | N | TYR | 598 | 41.875 | -6.896 | 62.097 | 1.00 | 64.61 |
| 558 | CA | TYR | 598 | 42.418 | -5.958 | 63.075 | 1.00 | 60.76 |
| 559 | CB | TYR | 598 | 43.761 | -6.468 | 63.588 | 1.00 | 59.35 |
| 560 | CG | TYR | 598 | 43.692 | -7.564 | 64.613 | 1.00 | 63.67 |
| 561 | CD1 | TYR | 598 | 42.509 | -8.257 | 64.850 | 1.00 | 61.84 |
| 562 | CE1 | TYR | 598 | 42.451 | -9.262 | 65.812 | 1.00 | 61.13 |
| 563 | CD2 | TYR | 598 | 44.820 | -7.906 | 65.358 | 1.00 | 61.13 |
| 564 | CE2 | TYR | 598 | 44.774 | -8.915 | 66.322 | 1.00 | 62.04 |
| 565 | CZ | TYR | 598 | 43.588 | -9.583 | 66.544 | 1.00 | 60.04 |
| 566 | OH | TYR | 598 | 43.536 | -10.549 | 67.519 | 1.00 | 62.57 |
| 567 | C | TYR | 598 | 42.639 | -4.553 | 62.549 | 1.00 | 62.24 |
| 568 | O | TYR | 598 | 43.158 | -3.690 | 63.256 | 1.00 | 61.45 |
| 569 | N | SER | 599 | 42.278 | -4.312 | 61.305 | 1.00 | 58.28 |
| 570 | CA | SER | 599 | 42.491 | -2.988 | 60.774 | 1.00 | 62.69 |
| 571 | CB | SER | 599 | 43.837 | -2.949 | 60.046 | 1.00 | 62.55 |
| 572 | OG | SER | 599 | 44.008 | -4.083 | 59.216 | 1.00 | 62.72 |
| 573 | C | SER | 599 | 41.365 | -2.525 | 59.867 | 1.00 | 64.40 |
| 574 | O | SER | 599 | 41.398 | -1.405 | 59.367 | 1.00 | 62.40 |
| 575 | N | TRP | 600 | 40.358 | -3.375 | 59.677 | 1.00 | 59.48 |
| 576 | CA | TRP | 600 | 39.245 | -3.026 | 58.807 | 1.00 | 62.88 |
| 577 | CB | TRP | 600 | 38.073 | -4.031 | 58.932 | 1.00 | 64.17 |
| 578 | CG | TRP | 600 | 37.282 | -3.951 | 60.198 | 1.00 | 62.02 |
| 579 | CD2 | TRP | 600 | 36.105 | -3.166 | 60.420 | 1.00 | 58.67 |
| 580 | CE2 | TRP | 600 | 35.754 | -3.311 | 61.781 | 1.00 | 61.68 |
| 581 | CE3 | TRP | 600 | 35.314 | -2.350 | 59.603 | 1.00 | 62.68 |
| 582 | CD1 | TRP | 600 | 37.583 | -4.533 | 61.395 | 1.00 | 58.92 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 583 | NE1 | TRP | 600 | 36.672 | -4.151 | 62.355 | 1.00 | 64.28 |
| 584 | CZ2 | TRP | 600 | 34.648 | -2.666 | 62.342 | 1.00 | 61.17 |
| 585 | CZ3 | TRP | 600 | 34.217 | -1.711 | 60.159 | 1.00 | 58.94 |
| 586 | CH2 | TRP | 600 | 33.894 | -1.871 | 61.516 | 1.00 | 61.08 |
| 587 | C | TRP | 600 | 38.789 | -1.630 | 59.169 | 1.00 | 62.61 |
| 588 | O | TRP | 600 | 38.533 | -0.805 | 58.308 | 1.00 | 62.45 |
| 589 | N | MET | 601 | 38.744 | -1.344 | 60.458 | 1.00 | 63.90 |
| 590 | CA | MET | 601 | 38.298 | -0.049 | 60.884 | 1.00 | 60.96 |
| 591 | CB | MET | 601 | 37.968 | -0.064 | 62.351 | 1.00 | 60.46 |
| 592 | CG | MET | 601 | 37.139 | 1.112 | 62.702 | 1.00 | 61.28 |
| 593 | SD | MET | 601 | 35.774 | 1.420 | 61.631 | 1.00 | 59.33 |
| 594 | CE | MET | 601 | 34.684 | 1.638 | 62.889 | 1.00 | 64.63 |
| 595 | C | MET | 601 | 39.225 | 1.129 | 60.577 | 1.00 | 61.08 |
| 596 | O | MET | 601 | 38.758 | 2.167 | 60.114 | 1.00 | 60.27 |
| 597 | N | SER | 602 | 40.521 | 0.979 | 60.854 | 1.00 | 61.23 |
| 598 | CA | SER | 602 | 41.488 | 2.035 | 60.581 | 1.00 | 59.98 |
| 599 | CB | SER | 602 | 42.872 | 1.647 | 61.083 | 1.00 | 60.99 |
| 600 | OG | SER | 602 | 42.783 | 1.022 | 62.350 | 1.00 | 66.17 |
| 601 | C | SER | 602 | 41.536 | 2.214 | 59.079 | 1.00 | 60.99 |
| 602 | O | SER | 602 | 41.609 | 3.327 | 58.581 | 1.00 | 64.11 |
| 603 | N | LEU | 603 | 41.494 | 1.108 | 58.351 | 1.00 | 59.44 |
| 604 | CA | LEU | 603 | 41.522 | 1.185 | 56.901 | 1.00 | 61.46 |
| 605 | CB | LEU | 603 | 41.402 | -0.212 | 56.280 | 1.00 | 59.31 |
| 606 | CG | LEU | 603 | 42.646 | -1.097 | 56.346 | 1.00 | 61.54 |
| 607 | CD1 | LEU | 603 | 42.415 | -2.362 | 55.549 | 1.00 | 63.99 |
| 608 | CD2 | LEU | 603 | 43.828 | -0.346 | 55.787 | 1.00 | 63.36 |
| 609 | C | LEU | 603 | 40.386 | 2.061 | 56.408 | 1.00 | 60.47 |
| 610 | O | LEU | 603 | 40.599 | 3.062 | 55.731 | 1.00 | 63.39 |
| 611 | N | MET | 604 | 39.173 | 1.688 | 56.784 | 1.00 | 63.54 |
| 612 | CA | MET | 604 | 38.000 | 2.417 | 56.365 | 1.00 | 62.81 |
| 613 | CB | MET | 604 | 36.770 | 1.623 | 56.723 | 1.00 | 58.90 |
| 614 | CG | MET | 604 | 36.632 | 0.429 | 55.842 | 1.00 | 59.86 |
| 615 | SD | MET | 604 | 37.633 | 0.438 | 54.374 | 1.00 | 62.53 |
| 616 | CE | MET | 604 | 36.663 | -0.510 | 53.559 | 1.00 | 60.72 |
| 617 | C | MET | 604 | 37.898 | 3.832 | 56.856 | 1.00 | 60.43 |
| 618 | O | MET | 604 | 37.397 | 4.695 | 56.132 | 1.00 | 62.37 |
| 619 | N | ALA | 605 | 38.375 | 4.076 | 58.072 | 1.00 | 59.95 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 620 | CA | ALA | 605 | 38.357 | 5.409 | 58.664 | 1.00 | 60.49 |
| 621 | CB | ALA | 605 | 38.667 | 5.317 | 60.132 | 1.00 | 59.15 |
| 622 | C | ALA | 605 | 39.381 | 6.309 | 57.985 | 1.00 | 61.50 |
| 623 | O | ALA | 605 | 39.071 | 7.427 | 57.583 | 1.00 | 59.82 |
| 624 | N | PHE | 606 | 40.608 | 5.810 | 57.870 | 1.00 | 63.59 |
| 625 | CA | PHE | 606 | 41.700 | 6.554 | 57.258 | 1.00 | 60.15 |
| 626 | CB | PHE | 606 | 42.981 | 5.713 | 57.285 | 1.00 | 63.75 |
| 627 | CG | PHE | 606 | 44.237 | 6.490 | 56.999 | 1.00 | 64.30 |
| 628 | CD1 | PHE | 606 | 44.723 | 7.424 | 57.913 | 1.00 | 61.77 |
| 629 | CD2 | PHE | 606 | 44.957 | 6.265 | 55.829 | 1.00 | 60.74 |
| 630 | CE1 | PHE | 606 | 45.910 | 8.118 | 57.665 | 1.00 | 64.00 |
| 631 | CE2 | PHE | 606 | 46.145 | 6.955 | 55.575 | 1.00 | 62.47 |
| 632 | CZ | PHE | 606 | 46.620 | 7.879 | 56.496 | 1.00 | 63.95 |
| 633 | C | PHE | 606 | 41.362 | 6.933 | 55.825 | 1.00 | 61.96 |
| 634 | O | PHE | 606 | 41.751 | 7.991 | 55.356 | 1.00 | 60.07 |
| 635 | N | ALA | 607 | 40.644 | 6.063 | 55.126 | 1.00 | 62.00 |
| 636 | CA | ALA | 607 | 40.264 | 6.338 | 53.745 | 1.00 | 57.50 |
| 637 | CB | ALA | 607 | 39.888 | 5.051 | 53.039 | 1.00 | 59.69 |
| 638 | C | ALA | 607 | 39.105 | 7.324 | 53.684 | 1.00 | 64.88 |
| 639 | O | ALA | 607 | 38.931 | 8.030 | 52.703 | 1.00 | 59.60 |
| 640 | N | LEU | 608 | 38.292 | 7.361 | 54.723 | 1.00 | 59.93 |
| 641 | CA | LEU | 608 | 37.196 | 8.307 | 54.725 | 1.00 | 61.70 |
| 642 | CB | LEU | 608 | 36.222 | 7.972 | 55.883 | 1.00 | 59.57 |
| 643 | CG | LEU | 608 | 35.125 | 8.918 | 56.402 | 1.00 | 62.57 |
| 644 | CD1 | LEU | 608 | 34.229 | 9.360 | 55.287 | 1.00 | 63.51 |
| 645 | CD2 | LEU | 608 | 34.298 | 8.246 | 57.488 | 1.00 | 59.98 |
| 646 | C | LEU | 608 | 37.862 | 9.662 | 54.935 | 1.00 | 61.71 |
| 647 | O | LEU | 608 | 37.500 | 10.645 | 54.294 | 1.00 | 57.56 |
| 648 | N | GLY | 609 | 38.869 | 9.692 | 55.806 | 1.00 | 59.60 |
| 649 | CA | GLY | 609 | 39.583 | 10.920 | 56.086 | 1.00 | 60.49 |
| 650 | C | GLY | 609 | 40.232 | 11.505 | 54.850 | 1.00 | 59.17 |
| 651 | O | GLY | 609 | 40.189 | 12.710 | 54.625 | 1.00 | 61.65 |
| 652 | N | TRP | 610 | 40.835 | 10.650 | 54.039 | 1.00 | 62.47 |
| 653 | CA | TRP | 610 | 41.488 | 11.102 | 52.823 | 1.00 | 61.40 |
| 654 | CB | TRP | 610 | 42.141 | 9.917 | 52.123 | 1.00 | 62.68 |
| 655 | CG | TRP | 610 | 42.744 | 10.264 | 50.817 | 1.00 | 62.61 |
| 656 | CD2 | TRP | 610 | 43.955 | 10.991 | 50.604 | 1.00 | 61.10 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 657 | CE2 | TRP | 610 | 44.139 | 11.095 | 49.209 | 1.00 | 62.45 |
| 658 | CE3 | TRP | 610 | 44.906 | 11.565 | 51.457 | 1.00 | 63.78 |
| 659 | CD1 | TRP | 610 | 42.254 | 9.965 | 49.582 | 1.00 | 58.08 |
| 660 | NE1 | TRP | 610 | 43.086 | 10.459 | 48.608 | 1.00 | 62.17 |
| 661 | CZ2 | TRP | 610 | 45.238 | 11.751 | 48.646 | 1.00 | 60.53 |
| 662 | CZ3 | TRP | 610 | 46.001 | 12.219 | 50.896 | 1.00 | 62.27 |
| 663 | CH2 | TRP | 610 | 46.156 | 12.305 | 49.505 | 1.00 | 60.31 |
| 664 | C | TRP | 610 | 40.517 | 11.797 | 51.874 | 1.00 | 60.80 |
| 665 | O | TRP | 610 | 40.797 | 12.866 | 51.358 | 1.00 | 60.72 |
| 666 | N | ARG | 611 | 39.368 | 11.191 | 51.639 | 1.00 | 61.36 |
| 667 | CA | ARG | 611 | 38.412 | 11.790 | 50.738 | 1.00 | 58.33 |
| 668 | CB | ARG | 611 | 37.254 | 10.817 | 50.486 | 1.00 | 62.33 |
| 669 | CG | ARG | 611 | 37.684 | 9.490 | 49.873 | 1.00 | 60.18 |
| 670 | CD | ARG | 611 | 36.476 | 8.686 | 49.426 | 1.00 | 59.83 |
| 671 | NE | ARG | 611 | 35.604 | 8.333 | 50.544 | 1.00 | 61.17 |
| 672 | CZ | ARG | 611 | 35.817 | 7.308 | 51.366 | 1.00 | 59.54 |
| 673 | NH1 | ARG | 611 | 36.875 | 6.528 | 51.187 | 1.00 | 61.47 |
| 674 | NH2 | ARG | 611 | 34.988 | 7.072 | 52.376 | 1.00 | 62.25 |
| 675 | C | ARG | 611 | 37.898 | 13.128 | 51.277 | 1.00 | 62.93 |
| 676 | O | ARG | 611 | 37.610 | 14.051 | 50.502 | 1.00 | 61.13 |
| 677 | N | SER | 612 | 37.806 | 13.234 | 52.603 | 1.00 | 60.26 |
| 678 | CA | SER | 612 | 37.321 | 14.450 | 53.263 | 1.00 | 63.90 |
| 679 | CB | SER | 612 | 37.057 | 14.172 | 54.736 | 1.00 | 62.00 |
| 680 | OG | SER | 612 | 36.011 | 13.234 | 54.875 | 1.00 | 59.62 |
| 681 | C | SER | 612 | 38.263 | 15.637 | 53.137 | 1.00 | 61.68 |
| 682 | O | SER | 612 | 37.831 | 16.776 | 52.975 | 1.00 | 59.32 |
| 683 | N | TYR | 613 | 39.552 | 15.352 | 53.226 | 1.00 | 64.48 |
| 684 | CA | TYR | 613 | 40.600 | 16.351 | 53.111 | 1.00 | 60.97 |
| 685 | CB | TYR | 613 | 41.920 | 15.725 | 53.587 | 1.00 | 56.65 |
| 686 | CG | TYR | 613 | 43.169 | 16.122 | 52.830 | 1.00 | 60.73 |
| 687 | CD1 | TYR | 613 | 43.569 | 17.456 | 52.746 | 1.00 | 64.60 |
| 688 | CE1 | TYR | 613 | 44.737 | 17.812 | 52.086 | 1.00 | 60.79 |
| 689 | CD2 | TYR | 613 | 43.971 | 15.153 | 52.229 | 1.00 | 61.29 |
| 690 | CE2 | TYR | 613 | 45.142 | 15.500 | 51.564 | 1.00 | 63.40 |
| 691 | CZ | TYR | 613 | 45.522 | 16.830 | 51.497 | 1.00 | 61.75 |
| 692 | OH | TYR | 613 | 46.690 | 17.173 | 50.854 | 1.00 | 62.46 |
| 693 | C | TYR | 613 | 40.712 | 16.822 | 51.667 | 1.00 | 62.41 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 694 | O | TYR | 613 | 40.954 | 17.996 | 51.395 | 1.00 | 61.34 |
| 695 | N | ARG | 614 | 40.511 | 15.896 | 50.745 | 1.00 | 61.73 |
| 696 | CA | ARG | 614 | 40.623 | 16.190 | 49.328 | 1.00 | 61.64 |
| 697 | CB | ARG | 614 | 40.835 | 14.880 | 48.545 | 1.00 | 62.80 |
| 698 | CG | ARG | 614 | 42.274 | 14.328 | 48.621 | 1.00 | 58.30 |
| 699 | CD | ARG | 614 | 42.908 | 14.348 | 47.242 | 1.00 | 60.57 |
| 700 | NE | ARG | 614 | 44.369 | 14.448 | 47.262 | 1.00 | 61.63 |
| 701 | CZ | ARG | 614 | 45.056 | 15.421 | 47.868 | 1.00 | 63.66 |
| 702 | NH1 | ARG | 614 | 44.414 | 16.386 | 48.521 | 1.00 | 61.59 |
| 703 | NH2 | ARG | 614 | 46.389 | 15.451 | 47.797 | 1.00 | 64.70 |
| 704 | C | ARG | 614 | 39.440 | 16.960 | 48.776 | 1.00 | 58.09 |
| 705 | O | ARG | 614 | 39.613 | 17.922 | 48.041 | 1.00 | 63.07 |
| 706 | N | GLN | 615 | 38.239 | 16.538 | 49.137 | 1.00 | 64.09 |
| 707 | CA | GLN | 615 | 37.033 | 17.192 | 48.660 | 1.00 | 61.67 |
| 708 | CB | GLN | 615 | 35.840 | 16.259 | 48.801 | 1.00 | 62.84 |
| 709 | CG | GLN | 615 | 35.738 | 15.162 | 47.795 | 1.00 | 62.14 |
| 710 | CD | GLN | 615 | 34.290 | 14.775 | 47.573 | 1.00 | 58.76 |
| 711 | OE1 | GLN | 615 | 33.532 | 14.598 | 48.525 | 1.00 | 62.70 |
| 712 | NE2 | GLN | 615 | 33.897 | 14.651 | 46.314 | 1.00 | 61.03 |
| 713 | C | GLN | 615 | 36.677 | 18.478 | 49.396 | 1.00 | 59.82 |
| 714 | O | GLN | 615 | 36.200 | 19.441 | 48.784 | 1.00 | 60.64 |
| 715 | N | SER | 616 | 36.901 | 18.480 | 50.709 | 1.00 | 62.12 |
| 716 | CA | SER | 616 | 36.522 | 19.615 | 51.545 | 1.00 | 62.52 |
| 717 | CB | SER | 616 | 35.199 | 19.297 | 52.239 | 1.00 | 61.86 |
| 718 | OG | SER | 616 | 35.408 | 18.310 | 53.240 | 1.00 | 59.96 |
| 719 | C | SER | 616 | 37.514 | 20.090 | 52.612 | 1.00 | 61.77 |
| 720 | O | SER | 616 | 37.110 | 20.501 | 53.703 | 1.00 | 63.13 |
| 721 | N | SER | 617 | 38.804 | 20.026 | 52.321 | 1.00 | 59.65 |
| 722 | CA | SER | 617 | 39.796 | 20.502 | 53.279 | 1.00 | 60.19 |
| 723 | CB | SER | 617 | 39.818 | 22.033 | 53.253 | 1.00 | 60.71 |
| 724 | OG | SER | 617 | 39.578 | 22.511 | 51.942 | 1.00 | 63.01 |
| 725 | C | SER | 617 | 39.569 | 20.029 | 54.724 | 1.00 | 59.81 |
| 726 | O | SER | 617 | 40.164 | 20.577 | 55.654 | 1.00 | 63.66 |
| 727 | N | ALA | 618 | 38.700 | 19.036 | 54.903 | 1.00 | 64.87 |
| 728 | CA | ALA | 618 | 38.393 | 18.444 | 56.210 | 1.00 | 62.57 |
| 729 | CB | ALA | 618 | 39.673 | 18.327 | 57.064 | 1.00 | 60.83 |
| 730 | C | ALA | 618 | 37.277 | 19.059 | 57.053 | 1.00 | 59.66 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 731 | O | ALA | 618 | 37.238 | 18.817 | 58.260 | 1.00 | 60.80 |
| 732 | N | ASN | 619 | 36.375 | 19.839 | 56.451 | 1.00 | 63.98 |
| 733 | CA | ASN | 619 | 35.262 | 20.411 | 57.227 | 1.00 | 61.08 |
| 734 | CB | ASN | 619 | 35.129 | 21.934 | 57.042 | 1.00 | 61.69 |
| 735 | CG | ASN | 619 | 35.946 | 22.453 | 55.912 | 1.00 | 62.23 |
| 736 | OD1 | ASN | 619 | 35.664 | 22.172 | 54.751 | 1.00 | 61.72 |
| 737 | ND2 | ASN | 619 | 36.980 | 23.217 | 56.239 | 1.00 | 61.09 |
| 738 | C | ASN | 619 | 33.907 | 19.755 | 56.958 | 1.00 | 60.70 |
| 739 | O | ASN | 619 | 32.856 | 20.374 | 57.157 | 1.00 | 60.32 |
| 740 | N | LEU | 620 | 33.951 | 18.505 | 56.500 | 1.00 | 59.87 |
| 741 | CA | LEU | 620 | 32.767 | 17.686 | 56.237 | 1.00 | 59.97 |
| 742 | CB | LEU | 620 | 31.777 | 18.358 | 55.270 | 1.00 | 59.52 |
| 743 | CG | LEU | 620 | 32.162 | 19.088 | 53.990 | 1.00 | 61.28 |
| 744 | CD1 | LEU | 620 | 31.041 | 18.989 | 52.971 | 1.00 | 64.28 |
| 745 | CD2 | LEU | 620 | 32.459 | 20.539 | 54.330 | 1.00 | 65.45 |
| 746 | C | LEU | 620 | 33.147 | 16.307 | 55.712 | 1.00 | 61.40 |
| 747 | O | LEU | 620 | 33.869 | 16.178 | 54.720 | 1.00 | 61.10 |
| 748 | N | LEU | 621 | 32.660 | 15.280 | 56.407 | 1.00 | 58.95 |
| 749 | CA | LEU | 621 | 32.926 | 13.891 | 56.050 | 1.00 | 61.88 |
| 750 | CB | LEU | 621 | 32.394 | 12.947 | 57.123 | 1.00 | 63.38 |
| 751 | CG | LEU | 621 | 33.031 | 13.049 | 58.503 | 1.00 | 60.66 |
| 752 | CD1 | LEU | 621 | 32.383 | 12.036 | 59.434 | 1.00 | 59.80 |
| 753 | CD2 | LEU | 621 | 34.524 | 12.808 | 58.390 | 1.00 | 62.77 |
| 754 | C | LEU | 621 | 32.283 | 13.540 | 54.728 | 1.00 | 62.48 |
| 755 | O | LEU | 621 | 31.092 | 13.751 | 54.531 | 1.00 | 61.33 |
| 756 | N | CYS | 622 | 33.077 | 12.972 | 53.833 | 1.00 | 59.02 |
| 757 | CA | CYS | 622 | 32.585 | 12.609 | 52.523 | 1.00 | 62.42 |
| 758 | CB | CYS | 622 | 33.453 | 13.304 | 51.479 | 1.00 | 59.42 |
| 759 | SG | CYS | 622 | 33.715 | 15.064 | 51.889 | 1.00 | 59.94 |
| 760 | C | CYS | 622 | 32.566 | 11.094 | 52.329 | 1.00 | 59.63 |
| 761 | O | CYS | 622 | 33.248 | 10.552 | 51.451 | 1.00 | 58.28 |
| 762 | N | PHE | 623 | 31.766 | 10.421 | 53.156 | 1.00 | 62.39 |
| 763 | CA | PHE | 623 | 31.645 | 8.972 | 53.088 | 1.00 | 60.88 |
| 764 | CB | PHE | 623 | -30.387 | 8.490 | 53.841 | 1.00 | 59.68 |
| 765 | CG | PHE | 623 | 30.461 | 8.686 | 55.344 | 1.00 | 58.52 |
| 766 | CD1 | PHE | 623 | 30.338 | 9.948 | 55.906 | 1.00 | 66.23 |
| 767 | CD2 | PHE | 623 | 30.688 | 7.612 | 56.191 | 1.00 | 63.42 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 768 | CE1 | PHE | 623 | 30.443 | 10.139 | 57.292 | 1.00 | 60.25 |
| 769 | CE2 | PHE | 623 | 30.795 | 7.796 | 57.576 | 1.00 | 58.96 |
| 770 | CZ | PHE | 623 | 30.673 | 9.059 | 58.124 | 1.00 | 57.60 |
| 771 | C | PHE | 623 | 31.618 | 8.532 | 51.630 | 1.00 | 61.75 |
| 772 | O | PHE | 623 | 32.502 | 7.802 | 51.179 | 1.00 | 60.41 |
| 773 | N | ALA | 624 | 30.624 | 8.995 | 50.888 | 1.00 | 64.49 |
| 774 | CA | ALA | 624 | 30.517 | 8.644 | 49.476 | 1.00 | 61.68 |
| 775 | CB | ALA | 624 | 29.429 | 7.592 | 49.276 | 1.00 | 60.07 |
| 776 | C | ALA | 624 | 30.179 | 9.912 | 48.700 | 1.00 | 60.93 |
| 777 | O | ALA | 624 | 30.002 | 10.981 | 49.297 | 1.00 | 60.98 |
| 778 | N | PRO | 625 | 30.130 | 9.828 | 47.355 | 1.00 | 62.43 |
| 779 | CD | PRO | 625 | 30.706 | 8.811 | 46.459 | 1.00 | 62.52 |
| 780 | CA | PRO | 625 | 29.795 | 11.035 | 46.593 | 1.00 | 59.45 |
| 781 | CB | PRO | 625 | 29.949 | 10.582 | 45.146 | 1.00 | 62.18 |
| 782 | CG | PRO | 625 | 31.089 | 9.653 | 45.245 | 1.00 | 59.91 |
| 783 | C | PRO | 625 | 28.366 | 11.397 | 46.928 | 1.00 | 62.94 |
| 784 | O | PRO | 625 | 28.111 | 12.382 | 47.622 | 1.00 | 59.36 |
| 785 | N | ASP | 626 | 27.433 | 10.572 | 46.468 | 1.00 | 58.86 |
| 786 | CA | ASP | 626 | 26.036 | 10.848 | 46.741 | 1.00 | 60.61 |
| 787 | CB | ASP | 626 | 25.126 | 9.882 | 45.939 | 1.00 | 65.12 |
| 788 | CG | ASP | 626 | 25.227 | 8.421 | 46.393 | 1.00 | 60.28 |
| 789 | OD1 | ASP | 626 | 25.311 | 8.160 | 47.612 | 1.00 | 60.31 |
| 790 | OD2 | ASP | 626 | 25.189 | 7.526 | 45.518 | 1.00 | 59.51 |
| 791 | C | ASP | 626 | 25.680 | 10.825 | 48.248 | 1.00 | 58.60 |
| 792 | O | ASP | 626 | 24.510 | 10.636 | 48.616 | 1.00 | 62.03 |
| 793 | N | LEU | 627 | 26.668 | 11.051 | 49.119 | 1.00 | 63.43 |
| 794 | CA | LEU | 627 | 26.392 | 11.020 | 50.552 | 1.00 | 61.63 |
| 795 | CB | LEU | 627 | 26.175 | 9.573 | 51.007 | 1.00 | 58.45 |
| 796 | CG | LEU | 627 | 25.874 | 9.407 | 52.496 | 1.00 | 63.46 |
| 797 | CD1 | LEU | 627 | 24.401 | 9.669 | 52.770 | 1.00 | 60.41 |
| 798 | CD2 | LEU | 627 | 26.241 | 8.013 | 52.919 | 1.00 | 65.05 |
| 799 | C | LEU | 627 | 27.435 | 11.665 | 51.459 | 1.00 | 61.00 |
| 800 | O | LEU | 627 | 28.320 | 10.988 | 51.985 | 1.00 | 62.27 |
| 801 | N | ILE | 628 | 27.301 | 12.965 | 51.682 | 1.00 | 59.87 |
| 802 | CA | ILE | 628 | 28.230 | 13.686 | 52.537 | 1.00 | 61.72 |
| 803 | CB | ILE | 628 | 28.796 | 14.887 | 51.787 | 1.00 | 61.79 |
| 804 | CG2 | ILE | 628 | 29.848 | 15.575 | 52.618 | 1.00 | 61.82 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 805 | CG1 | ILE | 628 | 29.391 | 14.418 | 50.461 | 1.00 | 58.81 |
| 806 | CD1 | ILE | 628 | 29.806 | 15.542 | 49.554 | 1.00 | 61.23 |
| 807 | C | ILE | 628 | 27.541 | 14.162 | 53.815 | 1.00 | 59.57 |
| 808 | O | ILE | 628 | 26.396 | 14.611 | 53.779 | 1.00 | 61.09 |
| 809 | N | ILE | 629 | 28.221 | 14.044 | 54.951 | 1.00 | 60.22 |
| 810 | CA | ILE | 629 | 27.638 | 14.493 | 56.208 | 1.00 | 60.98 |
| 811 | CB | ILE | 629 | 28.261 | 13.766 | 57.423 | 1.00 | 65.92 |
| 812 | CG2 | ILE | 629 | 28.292 | 14.681 | 58.647 | 1.00 | 62.88 |
| 813 | CG1 | ILE | 629 | 27.419 | 12.536 | 57.768 | 1.00 | 64.60 |
| 814 | CD1 | ILE | 629 | 26.917 | 11.766 | 56.571 | 1.00 | 63.67 |
| 815 | C | ILE | 629 | 27.852 | 15.989 | 56.319 | 1.00 | 60.46 |
| 816 | O | ILE | 629 | 28.935 | 16.452 | 56.676 | 1.00 | 60.38 |
| 817 | N | ASN | 630 | 26.797 | 16.729 | 55.994 | 1.00 | 61.49 |
| 818 | CA | ASN | 630 | 26.789 | 18.187 | 56.015 | 1.00 | 61.95 |
| 819 | CB | ASN | 630 | 25.655 | 18.685 | 55.149 | 1.00 | 60.74 |
| 820 | CG | ASN | 630 | 24.348 | 18.042 | 55.516 | 1.00 | 63.39 |
| 821 | OD1 | ASN | 630 | 24.011 | 17.949 | 56.688 | 1.00 | 62.29 |
| 822 | ND2 | ASN | 630 | 23.603 | 17.591 | 54.525 | 1.00 | 63.08 |
| 823 | C | ASN | 630 | 26.616 | 18.786 | 57.402 | 1.00 | 63.46 |
| 824 | O | ASN | 630 | 26.311 | 18.085 | 58.369 | 1.00 | 63.49 |
| 825 | N | GLU | 631 | 26.794 | 20.103 | 57.475 | 1.00 | 60.97 |
| 826 | CA | GLU | 631 | 26.658 | 20.840 | 58.729 | 1.00 | 59.85 |
| 827 | CB | GLU | 631 | 26.743 | 22.349 | 58.484 | 1.00 | 62.90 |
| 828 | CG | GLU | 631 | 26.784 | 23.166 | 59.774 | 1.00 | 60.34 |
| 829 | CD | GLU | 631 | 25.819 | 24.340 | 59.761 | 1.00 | 60.36 |
| 830 | OE1 | GLU | 631 | 24.688 | 24.184 | 60.288 | 1.00 | 62.28 |
| 831 | OE2 | GLU | 631 | 26.191 | 25.406 | 59.213 | 1.00 | 56.78 |
| 832 | C | GLU | 631 | 25.313 | 20.519 | 59.367 | 1.00 | 59.95 |
| 833 | O | GLU | 631 | 25.223 | 20.250 | 60.564 | 1.00 | 61.19 |
| 834 | N | GLN | 632 | 24.268 | 20.540 | 58.552 | 1.00 | 60.53 |
| 835 | CA | GLN | 632 | 22.933 | 20.248 | 59.046 | 1.00 | 60.62 |
| 836 | CB | GLN | 632 | 21.930 | 20.354 | 57.895 | 1.00 | 64.85 |
| 837 | CG | GLN | 632 | 22.121 | 21.610 | 57.031 | 1.00 | 59.39 |
| 838 | CD | GLN | 632 | 22.081 | 22.917 | 57.841 | 1.00 | 63.65 |
| 839 | OE1 | GLN | 632 | 21.068 | 23.248 | 58.473 | 1.00 | 59.92 |
| 840 | NE2 | GLN | 632 | 23.193 | 23.663 | 57.821 | 1.00 | 62.67 |
| 841 | C | GLN | 632 | 22.873 | 18.860 | 59.697 | 1.00 | 63.23 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 842 | O | GLN | 632 | 22.554 | 18.741 | 60.882 | 1.00 | 61.37 |
| 843 | N | ARG | 633 | 23.213 | 17.827 | 58.929 | 1.00 | 62.09 |
| 844 | CA | ARG | 633 | 23.190 | 16.444 | 59.406 | 1.00 | 61.43 |
| 845 | CB | ARG | 633 | 23.762 | 15.504 | 58.345 | 1.00 | 60.38 |
| 846 | CG | ARG | 633 | 22.863 | 15.388 | 57.142 | 1.00 | 58.68 |
| 847 | CD | ARG | 633 | 23.419 | 14.459 | 56.102 | 1.00 | 63.71 |
| 848 | NE | ARG | 633 | 22.589 | 14.486 | 54.905 | 1.00 | 62.14 |
| 849 | CZ | ARG | 633 | 22.885 | 13.852 | 53.780 | 1.00 | 60.28 |
| 850 | NH1 | ARG | 633 | 23.996 | 13.136 | 53.704 | 1.00 | 60.27 |
| 851 | NH2 | ARG | 633 | 22.075 | 13.937 | 52.733 | 1.00 | 60.98 |
| 852 | C | ARG | 633 | 23.833 | 16.137 | 60.753 | 1.00 | 64.07 |
| 853 | O | ARG | 633 | 23.495 | 15.117 | 61.348 | 1.00 | 60.29 |
| 854 | N | MET | 634 | 24.758 | 16.970 | 61.236 | 1.00 | 61.53 |
| 855 | CA | MET | 634 | 25.334 | 16.721 | 62.560 | 1.00 | 60.63 |
| 856 | CB | MET | 634 | 26.429 | 17.747 | 62.859 | 1.00 | 60.06 |
| 857 | CG | MET | 634 | 27.598 | 17.688 | 61.874 | 1.00 | 53.30 |
| 858 | SD | MET | 634 | 28.604 | 16.178 | 62.057 | 1.00 | 63.04 |
| 859 | CE | MET | 634 | 30.133 | 16.562 | 61.162 | 1.00 | 60.95 |
| 860 | C | MET | 634 | 24.150 | 16.834 | 63.555 | 1.00 | 60.65 |
| 861 | O | MET | 634 | 23.899 | 17.897 | 64.149 | 1.00 | 63.14 |
| 862 | N | THR | 635 | 23.420 | 15.714 | 63.670 | 1.00 | 63.54 |
| 863 | CA | THR | 635 | 22.220 | 15.523 | 64.504 | 1.00 | 61.73 |
| 864 | CB | THR | 635 | 21.180 | 14.557 | 63.819 | 1.00 | 59.70 |
| 865 | OG1 | THR | 635 | 20.987 | 14.911 | 62.442 | 1.00 | 57.94 |
| 866 | CG2 | THR | 635 | 19.829 | 14.609 | 64.552 | 1.00 | 63.34 |
| 867 | C | THR | 635 | 22.593 | 14.861 | 65.825 | 1.00 | 61.33 |
| 868 | O | THR | 635 | 23.570 | 15.251 | 66.464 | 1.00 | 63.49 |
| 869 | N | LEU | 636 | 21.796 | 13.851 | 66.198 | 1.00 | 60.18 |
| 870 | CA | LEU | 636 | 21.953 | 13.057 | 67.420 | 1.00 | 61.62 |
| 871 | CB | LEU | 636 | 22.112 | 11.577 | 67.095 | 1.00 | 60.70 |
| 872 | CG | LEU | 636 | 22.867 | 10.855 | 68.213 | 1.00 | 62.64 |
| 873 | CD1 | LEU | 636 | 21.904 | 10.048 | 69.070 | 1.00 | 63.15 |
| 874 | CD2 | LEU | 636 | 23.910 | 9.960 | 67.603 | 1.00 | 60.79 |
| 875 | C | LEU | 636 | 23.183 | 13.478 | 68.172 | 1.00 | 61.59 |
| 876 | O | LEU | 636 | 24.287 | 13.372 | 67.626 | 1.00 | 60.57 |
| 877 | N | PRO | 637 | 23.029 | 13.907 | 69.442 | 1.00 | 59.83 |
| 878 | CD | PRO | 637 | 22.008 | 13.402 | 70.379 | 1.00 | 57.71 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 879 | CA | PRO | 637 | 24.225 | 14.321 | 70.182 | 1.00 | 61.11 |
| 880 | CB | PRO | 637 | 23.810 | 14.133 | 71.639 | 1.00 | 61.28 |
| 881 | CG | PRO | 637 | 22.862 | 12.966 | 71.555 | 1.00 | 61.68 |
| 882 | C | PRO | 637 | 25.417 | 13.433 | 69.793 | 1.00 | 61.18 |
| 883 | O | PRO | 637 | 26.457 | 13.928 | 69.331 | 1.00 | 61.45 |
| 884 | N | CYS | 638 | 25.243 | 12.117 | 69.920 | 1.00 | 63.53 |
| 885 | CA | CYS | 638 | 26.333 | 11.211 | 69.588 | 1.00 | 61.78 |
| 886 | CB | CYS | 638 | 26.024 | 9.787 | 70.065 | 1.00 | 62.20 |
| 887 | SG | CYS | 638 | 24.449 | 9.498 | 70.917 | 1.00 | 60.82 |
| 888 | C | CYS | 638 | 26.722 | 11.209 | 68.101 | 1.00 | 61.50 |
| 889 | O | CYS | 638 | 27.863 | 10.844 | 67.765 | 1.00 | 61.11 |
| 890 | N | MET | 639 | 25.816 | 11.625 | 67.214 | 1.00 | 57.65 |
| 891 | CA | MET | 639 | 26.186 | 11.638 | 65.817 | 1.00 | 60.56 |
| 892 | CB | MET | 639 | 25.103 | 12.211 1 | 64.924 | 1.00 | 66.32 |
| 893 | CG | MET | 639 | 25.612 | 12.161 | 63.532 | 1.00 | 60.47 |
| 894 | SD | MET | 639 | 25.084 | 11.084 | 62.238 | 1.00 | 57.31 |
| 895 | CE | MET | 639 | 25.984 | 11.962 | 61.115 | 1.00 | 58.45 |
| 896 | C | MET | 639 | 27.478 | 12.449 | 65.637 | 1.00 | 62.26 |
| 897 | O | MET | 639 | 28.361 | 12.083 | 64.857 | 1.00 | 61.35 |
| 898 | N | TYR | 640 | 27.589 | 13.543 | 66.383 | 1.00 | 61.78 |
| 899 | CA | TYR | 640 | 28.797 | 14.339 | 66.340 | 1.00 | 61.48 |
| 900 | CB | TYR | 640 | 28.569 | 15.755 | 66.872 | 1.00 | 60.05 |
| 901 | CG | TYR | 640 | 29.871 | 16.511 | 66.956 | 1.00 | 61.42 |
| 902 | CD1 | TYR | 640 | 30.530 | 16.927 | 65.795 | 1.00 | 62.42 |
| 903 | CE1 | TYR | 640 | 31.800 | 17.472 | 65.846 | 1.00 | 61.93 |
| 904 | CD2 | TYR | 640 | 30.519 | 16.680 | 68.175 | 1.00 | 59.45 |
| 905 | CE2 | TYR | 640 | 31.785 | 17.222 | 68.235 | 1.00 | 63.13 |
| 906 | CZ | TYR | 640 | 32.425 | 17.612 | 67.068 | 1.00 | 58.88 |
| 907 | OH | TYR | 640 | 33.711 | 18.103 | 67.130 | 1.00 | 60.76 |
| 908 | C | TYR | 640 | 29.842 | 13.646 | 67.215 | 1.00 | 60.30 |
| 909 | O | TYR | 640 | 31.041 | 13.847 | 67.034 | 1.00 | 63.06 |
| 910 | N | ASP | 641 | 29.397 | 12.830 | 68.168 | 1.00 | 61.14 |
| 911 | CA | ASP | 641 | 30.349 | 12.136 | 69.035 | 1.00 | 62.18 |
| 912 | CB | ASP | 641 | 29.674 | 11.669 | 70.323 | 1.00 | 59.68 |
| 913 | CG | ASP | 641 | 29.145 | 12.828 | 71.135 | 1.00 | 60.96 |
| 914 | OD1 | ASP | 641 | 27.930 | 13.090 | 71.062 | 1.00 | 61.07 |
| 915 | OD2 | ASP | 641 | 29.950 | 13.493 | 71.824 | 1.00 | 63.74 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 916 | C | ASP | 641 | 30.991 | 10.971 | 68.313 | 1.00 | 60.81 |
| 917 | O | ASP | 641 | 32.047 | 10.482 | 68.721 | 1.00 | 60.52 |
| 918 | N | GLN | 642 | 30.349 | 10.542 | 67.229 | 1.00 | 59.76 |
| 919 | CA | GLN | 642 | 30.860 | 9.456 | 66.396 | 1.00 | 63.97 |
| 920 | CB | GLN | 642 | 29.721 | 8.763 | 65.687 | 1.00 | 61.51 |
| 921 | CG | GLN | 642 | 28.690 | 8.348 | 66.642 | 1.00 | 61.79 |
| 922 | CD | GLN | 642 | 27.547 | 7.724 | 65.978 | 1.00 | 62.69 |
| 923 | OE1 | GLN | 642 | 27.709 | 6.684 | 65.308 | 1.00 | 61.01 |
| 924 | NE2 | GLN | 642 | 26.355 | 8.326 | 66.145 | 1.00 | 60.03 |
| 925 | C | GLN | 642 | 31.766 | 10.069 | 65.359 | 1.00 | 62.81 |
| 926 | O | GLN | 642 | 32.954 | 9.760 | 65.294 | 1.00 | 61.10 |
| 927 | N | CYS | 643 | 31.190 | 10.957 | 64.556 | 1.00 | 60.88 |
| 928 | CA | CYS | 643 | 31.931 | 11.628 | 63.504 | 1.00 | 61.65 |
| 929 | CB | CYS | 643 | 30.977 | 12.508 | 62.694 | 1.00 | 61.95 |
| 930 | SG | CYS | 643 | 29.662 | 11.585 | 61.843 | 1.00 | 63.26 |
| 931 | C | CYS | 643 | 33.081 | 12.455 | 64.071 | 1.00 | 61.30 |
| 932 | O | CYS | 643 | 34.102 | 12.652 | 63.418 | 1.00 | 61.90 |
| 933 | N | LYS | 644 | 32.911 | 12.923 | 65.299 | 1.00 | 62.82 |
| 934 | CA | LYS | 644 | 33.923 | 13.730 | 65.951 | 1.00 | 58.73 |
| 935 | CB | LYS | 644 | 33.634 | 13.827 | 67.449 | 1.00 | 63.83 |
| 936 | CG | LYS | 644 | 34.630 | 14.686 | 68.207 | 1.00 | 59.96 |
| 937 | CD | LYS | 644 | 34.226 | 14.891 | 69.665 | 1.00 | 59.57 |
| 938 | CE | LYS | 644 | 35.160 | 15.902 | 70.358 | 1.00 | 59.22 |
| 939 | NZ | LYS | 644 | 35.201 | 17.239 | 69.668 | 1.00 | 61.66 |
| 940 | C | LYS | 644 | 35.328 | 13.182 | 65.747 | 1.00 | 61.72 |
| 941 | O | LYS | 644 | 36.296 | 13.941 | 65.673 | 1.00 | 61.77 |
| 942 | N | HIS | 645 | 35.451 | 11.864 | 65.655 | 1.00 | 61.41 |
| 943 | CA | HIS | 645 | 36.769 | 11.262 | 65.474 | 1.00 | 59.96 |
| 944 | CB | HIS | 645 | 36.856 | 9.943 | 66.209 | 1.00 | 60.26 |
| 945 | CG | HIS | 645 | 37.109 | 10.100 | 67.667 | 1.00 | 59.75 |
| 946 | CD2 | HIS | 645 | 38.254 | 10.338 | 68.346 | 1.00 | 58.42 |
| 947 | ND1 | HIS | 645 | 36.103 | 10.041 | 68.606 | 1.00 | 61.77 |
| 948 | CE1 | HIS | 645 | 36.621 | 10.232 | 69.805 | 1.00 | 58.75 |
| 949 | NE2 | HIS | 645 | 37.924 | 10.415 | 69.675 | 1.00 | 57.48 |
| 950 | C | HIS | 645 | 37.165 | 11.038 | 64.037 | 1.00 | 60.84 |
| 951 | O | HIS | 645 | 38.352 | 11.000 | 63.727 | 1.00 | 62.12 |
| 952 | N | MET | 646 | 36.172 | 10.856 | 63.174 | 1.00 | 63.11 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 953 | CA | MET | 646 | 36.432 | 10.651 | 61.759 | 1.00 | 62.74 |
| 954 | CB | MET | 646 | 35.135 | 10.211 | 61.023 | 1.00 | 61.50 |
| 955 | CG | MET | 646 | 34.686 | 8.771 | 61.338 | 1.00 | 58.88 |
| 956 | SD | MET | 646 | 32.994 | 8.315 | 60.876 | 1.00 | 61.92 |
| 957 | CE | MET | 646 | 32.426 | 8.134 | 62.441 | 1.00 | 62.24 |
| 958 | C | MET | 646 | 36.948 | 11.972 | 61.168 | 1.00 | 63.61 |
| 959 | O | MET | 646 | 37.709 | 11.962 | 60.197 | 1.00 | 63.12 |
| 960 | N | LEU | 647 | 36.543 | 13.093 | 61.772 | 1.00 | 60.39 |
| 961 | CA | LEU | 647 | 36.963 | 14.419 | 61.325 | 1.00 | 61.16 |
| 962 | CB | LEU | 647 | 36.105 | 15.510 | 61.965 | 1.00 | 62.09 |
| 963 | CG | LEU | 647 | 34.661 | 15.778 | 61.551 | 1.00 | 62.69 |
| 964 | CD1 | LEU | 647 | 34.144 | 16.850 | 62.479 | 1.00 | 59.66 |
| 965 | CD2 | LEU | 647 | 34.553 | 16.232 | 60.098 | 1.00 | 61.60 |
| 966 | C | LEU | 647 | 38.400 | 14.652 | 61.731 | 1.00 | 62.99 |
| 967 | O | LEU | 647 | 39.164 | 15.329 | 61.042 | 1.00 | 62.41 |
| 968 | N | TYR | 648 | 38.750 | 14.087 | 62.876 | 1.00 | 60.24 |
| 969 | CA | TYR | 648 | 40.087 | 14.202 | 63.431 | 1.00 | 60.92 |
| 970 | CB | TYR | 648 | 40.190 | 13.339 | 64.685 | 1.00 | 59.63 |
| 971 | CG | TYR | 648 | 41.486 | 13.510 | 65.428 | 1.00 | 60.33 |
| 972 | CD1 | TYR | 648 | 42.672 | 12.950 | 64.952 | 1.00 | 62.96 |
| 973 | CE1 | TYR | 648 | 43.876 | 13.160 | 65.609 | 1.00 | 61.51 |
| 974 | CD2 | TYR | 648 | 41.537 | 14.280 | 66.585 | 1.00 | 57.67 |
| 975 | CE2 | TYR | 648 | 42.735 | 14.500 | 67.252 | 1.00 | 62.62 |
| 976 | CZ | TYR | 648 | 43.902 | 13.938 | 66.759 | 1.00 | 60.16 |
| 977 | OH | TYR | 648 | 45.089 | 14.157 | 67.426 | 1.00 | 64.40 |
| 978 | C | TYR | 648 | 41.164 | 13.787 | 62.435 | 1.00 | 61.32 |
| 979 | O | TYR | 648 | 42.045 | 14.575 | 62.099 | 1.00 | 62.83 |
| 980 | N | VAL | 649 | 41.107 | 12.545 | 61.971 | 1.00 | 65.90 |
| 981 | CA | VAL | 649 | 42.105 | 12.079 | 61.027 | 1.00 | 60.14 |
| 982 | CB | VAL | 649 | 41.862 | 10.578 | 60.642 | 1.00 | 59.02 |
| 983 | CG1 | VAL | 649 | 40.528 | 10.109 | 61.218 | 1.00 | 60.92 |
| 984 | CG2 | VAL | 649 | 41.930 | 10.372 | 59.122 | 1.00 | 59.70 |
| 985 | C | VAL | 649 | 42.072 | 12.982 | 59.814 | 1.00 | 61.99 |
| 986 | O | VAL | 649 | 43.105 | 13.378 | 59.297 | 1.00 | 60.55 |
| 987 | N | SER | 650 | 40.873 | 13.339 | 59.390 | 1.00 | 61.60 |
| 988 | CA | SER | 650 | 40.705 | 14.191 | 58.226 | 1.00 | 61.66 |
| 989 | CB | SER | 650 | 39.224 | 14.356 | 57.914 | 1.00 | 61.34 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 990 | OG | SER | 650 | 39.069 | 14.979 | 56.662 | 1.00 | 64.56 |
| 991 | C | SER | 650 | 41.344 | 15.555 | 58.429 | 1.00 | 61.44 |
| 992 | O | SER | 650 | 41.800 | 16.181 | 57.476 | 1.00 | 58.88 |
| 993 | N | SER | 651 | 41.365 | 16.013 | 59.677 | 1.00 | 59.26 |
| 994 | CA | SER | 651 | 41.960 | 17.298 | 60.029 | 1.00 | 62.47 |
| 995 | CB | SER | 651 | 41.578 | 17.637 | 61.483 | 1.00 | 58.62 |
| 996 | OG | SER | 651 | 42.537 | 18.441 | 62.154 | 1.00 | 60.41 |
| 997 | C | SER | 651 | 43.480 | 17.204 | 59.849 | 1.00 | 61.76 |
| 998 | O | SER | 651 | 44.087 | 18.019 | 59.164 | 1.00 | 62.09 |
| 999 | N | GLU | 652 | 44.070 | 16.172 | 60.441 | 1.00 | 60.41 |
| 1000 | CA | GLU | 652 | 45.509 | 15.927 | 60.395 | 1.00 | 60.48 |
| 1001 | CB | GLU | 652 | 45.837 | 14.680 | 61.220 | 1.00 | 65.10 |
| 1002 | CG | GLU | 652 | 45.488 | 14.822 | 62.677 | 1.00 | 59.36 |
| 1003 | CD | GLU | 652 | 46.160 | 16.021 | 63.289 | 1.00 | 62.29 |
| 1004 | OE1 | GLU | 652 | 47.399 | 15.970 | 63.444 | 1.00 | 60.10 |
| 1005 | OE2 | GLU | 652 | 45.451 | 17.014 | 63.592 | 1.00 | 60.61 |
| 1006 | C | GLU | 652 | 46.100 | 15.773 | 59.001 | 1.00 | 59.16 |
| 1007 | O | GLU | 652 | 47.238 | 16.166 | 58.755 | 1.00 | 59.92 |
| 1008 | N | LEU | 653 | 45.335 | 15.180 | 58.094 | 1.00 | 60.01 |
| 1009 | CA | LEU | 653 | 45.807 | 14.984 | 56.731 | 1.00 | 61.61 |
| 1010 | CB | LEU | 653 | 44.874 | 14.048 | 55.960 | 1.00 | 64.78 |
| 1011 | CG | LEU | 653 | 44.860 | 12.600 | 56.432 | 1.00 | 63.03 |
| 1012 | CD1 | LEU | 653 | 43.723 | 11.868 | 55.768 | 1.00 | 60.96 |
| 1013 | CD2 | LEU | 653 | 46.179 | 11.941 | 56.122 | 1.00 | 62.70 |
| 1014 | C | LEU | 653 | 45.878 | 16.328 | 56.037 | 1.00 | 60.72 |
| 1015 | O | LEU | 653 | 46.805 | 16.588 | 55.269 | 1.00 | 61.12 |
| 1016 | N | HIS | 654 | 44.895 | 17.182 | 56.303 | 1.00 | 62.78 |
| 1017 | CA | HIS | 654 | 44.894 | 18.497 | 55.698 | 1.00 | 61.32 |
| 1018 | CB | HIS | 654 | 43.513 | 19.141 | 55.805 | 1.00 | 63.28 |
| 1019 | CG | HIS | 654 | 43.517 | 20.607 | 55.518 | 1.00 | 61.88 |
| 1020 | CD2 | HIS | 654 | 43.210 | 21.296 | 54.394 | 1.00 | 59.76 |
| 1021 | ND1 | HIS | 654 | 43.946 | 21.543 | 56.436 | 1.00 | 58.15 |
| 1022 | CE1 | HIS | 654 | 43.905 | 22.744 | 55.889 | 1.00 | 62.74 |
| 1023 | NE2 | HIS | 654 | 43.463 | 22.622 | 54.650 | 1.00 | 60.79 |
| 1024 | C | HIS | 654 | 45.935 | 19.319 | 56.440 | 1.00 | 62.39 |
| 1025 | O | HIS | 654 | 46.667 | 20.112 | 55.851 | 1.00 | 62.10 |
| 1026 | N | ARG | 655 | 46.012 | 19.098 | 57.743 | 1.00 | 62.07 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1027 | CA | ARG | 655 | 46.968 | 19.804 | 58.572 | 1.00 | 62.85 |
| 1028 | CB | ARG | 655 | 46.882 | 19.277 | 60.008 | 1.00 | 58.45 |
| 1029 | CG | ARG | 655 | 47.082 | 20.314 | 61.111 | 1.00 | 57.53 |
| 1030 | CD | ARG | 655 | 48.522 | 20.368 | 61.565 | 1.00 | 58.48 |
| 1031 | NE | ARG | 655 | 48.968 | 19.079 | 62.082 | 1.00 | 61.43 |
| 1032 | CZ | ARG | 655 | 50.206 | 18.831 | 62.503 | 1.00 | 59.99 |
| 1033 | NH1 | ARG | 655 | 51.125 | 19.790 | 62.472 | 1.00 | 61.84 |
| 1034 | NH2 | ARG | 655 | 50.537 | 17.625 | 62.950 | 1.00 | 60.75 |
| 1035 | C | ARG | 655 | 48.367 | 19.599 | 57.999 | 1.00 | 60.66 |
| 1036 | O | ARG | 655 | 49.086 | 20.566 | 57.753 | 1.00 | 62.69 |
| 1037 | N | LEU | 656 | 48.735 | 18.340 | 57.759 | 1.00 | 60.05 |
| 1038 | CA | LEU | 656 | 50.060 | 18.008 | 57.224 | 1.00 | 61.69 |
| 1039 | CB | LEU | 656 | 50.575 | 16.697 | 57.832 | 1.00 | 60.63 |
| 1040 | CG | LEU | 656 | 50.902 | 16.651 | 59.330 | 1.00 | 60.12 |
| 1041 | CD1 | LEU | 656 | 51.059 | 15.205 | 59.759 | 1.00 | 61.83 |
| 1042 | CD2 | LEU | 656 | 52.161 | 17.440 | 59.632 | 1.00 | 61.33 |
| 1043 | C | LEU | 656 | 50.164 | 17.922 | 55.706 | 1.00 | 60.82 |
| 1044 | O | LEU | 656 | 51.187 | 17.491 | 55.184 | 1.00 | 63.77 |
| 1045 | N | GLN | 657 | 49.119 | 18.321 | 54.995 | 1.00 | 62.25 |
| 1046 | CA | GLN | 657 | 49.165 | 18.291 | 53.543 | 1.00 | 62.13 |
| 1047 | CB | GLN | 657 | 50.018 | 19.442 | 53.026 | 1.00 | 59.95 |
| 1048 | CG | GLN | 657 | 49.412 | 20.805 | 53.219 | 1.00 | 60.54 |
| 1049 | CD | GLN | 657 | 48.109 | 20.944 | 52.480 | 1.00 | 59.94 |
| 1050 | OE1 | GLN | 657 | 47.043 | 20.616 | 52.997 | 1.00 | 61.59 |
| 1051 | NE2 | GLN | 657 | 48.189 | 21.413 | 51.250 | 1.00 | 59.69 |
| 1052 | C | GLN | 657 | 49.756 | 16.998 | 53.027 | 1.00 | 64.37 |
| 1053 | O | GLN | 657 | 50.684 | 17.013 | 52.230 | 1.00 | 60.80 |
| 1054 | N | VAL | 658 | 49.233 | 15.876 | 53.487 | 1.00 | 59.26 |
| 1055 | CA | VAL | 658 | 49.730 | 14.589 | 53.048 | 1.00 | 60.79 |
| 1056 | CB | VAL | 658 | 49.044 | 13.466 | 53.856 | 1.00 | 61.35 |
| 1057 | CG1 | VAL | 658 | 49.169 | 12.130 | 53.154 | 1.00 | 61.79 |
| 1058 | CG2 | VAL | 658 | 49.663 | 13.406 | 55.240 | 1.00 | 61.37 |
| 1059 | C | VAL | 658 | 49.494 | 14.416 | 51.552 | 1.00 | 60.15 |
| 1060 | O | VAL | 658 | 48.452 | 14.798 | 51.025 | 1.00 | 60.95 |
| 1061 | N | SER | 659 | 50.485 | 13.862 | 50.869 | 1.00 | 63.41 |
| 1062 | CA | SER | 659 | 50.399 | 13.615 | 49.438 | 1.00 | 58.10 |
| 1063 | CB | SER | 659 | 51.797 | 13.685 | 48.834 | 1.00 | 59.86 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1064 | OG | SER | 659 | 52.018 | 12.643 | 47.905 | 1.00 | 62.03 |
| 1065 | C | SER | 659 | 49.777 | 12.242 | 49.157 | 1.00 | 62.11 |
| 1066 | O | SER | 659 | 49.781 | 11.353 | 50.015 | 1.00 | 61.38 |
| 1067 | N | TYR | 660 | 49.243 | 12.062 | 47.956 | 1.00 | 60.30 |
| 1068 | CA | TYR | 660 | 48.633 | 10.789 | 47.601 | 1.00 | 61.68 |
| 1069 | CB | TYR | 660 | 48.100 | 10.860 | 46.177 | 1.00 | 61.85 |
| 1070 | CG | TYR | 660 | 47.411 | 9.605 | 45.727 | 1.00 | 59.67 |
| 1071 | CD1 | TYR | 660 | 46.561 | 8.911 | 46.581 | 1.00 | 63.51 |
| 1072 | CE1 | TYR | 660 | 45.893 | 7.783 | 46.152 | 1.00 | 60.60 |
| 1073 | CD2 | TYR | 660 | 47.576 | 9.134 | 44.431 | 1.00 | 60.37 |
| 1074 | CE2 | TYR | 660 | 46.911 | 8.011 | 43.990 | 1.00 | 64.13 |
| 1075 | CZ | TYR | 660 | 46.072 | 7.339 | 44.851 | 1.00 | 63.85 |
| 1076 | OH | TYR | 660 | 45.393 | 6.229 | 44.402 | 1.00 | 62.07 |
| 1077 | C | TYR | 660 | 49.584 | 9.594 | 47.749 | 1.00 | 59.81 |
| 1078 | O | TYR | 660 | 49.175 | 8.510 | 48.165 | 1.00 | 64.86 |
| 1079 | N | GLU | 661 | 50.853 | 9.789 | 47.411 | 1.00 | 60.25 |
| 1080 | CA | GLU | 661 | 51.814 | 8.703 | 47.527 | 1.00 | 62.48 |
| 1081 | CB | GLU | 661 | 53.119 | 9.034 | 46.788 | 1.00 | 61.23 |
| 1082 | CG | GLU | 661 | 53.209 | 8.405 | 45.395 | 1.00 | 63.67 |
| 1083 | CD | GLU | 661 | 54.517 | 8.708 | 44.672 | 1.00 | 65.78 |
| 1084 | OE1 | GLU | 661 | 55.602 | 8.472 | 45.247 | 1.00 | 60.21 |
| 1085 | OE2 | GLU | 661 | 54.462 | 9.174 | 43.517 | 1.00 | 60.28 |
| 1086 | C | GLU | 661 | 52.096 | 8.354 | 48.980 | 1.00 | 61.69 |
| 1087 | O | GLU | 661 | 52.247 | 7.183 | 49.312 | 1.00 | 61.32 |
| 1088 | N | GLU | 662 | 52.160 | 9.348 | 49.854 | 1.00 | 62.68 |
| 1089 | CA | GLU | 662 | 52.405 | 9.048 | 51.252 | 1.00 | 63.61 |
| 1090 | CB | GLU | 662 | 52.605 | 10.340 | 52.032 | 1.00 | 61.04 |
| 1091 | CG | GLU | 662 | 53.485 | 11.321 | 51.309 | 1.00 | 61.12 |
| 1092 | CD | GLU | 662 | 53.768 | 12.555 | 52.117 | 1.00 | 62.85 |
| 1093 | OE1 | GLU | 662 | 52.822 | 13.164 | 52.637 | 1.00 | 58.86 |
| 1094 | OE2 | GLU | 662 | 54.945 | 12.931 | 52.227 | 1.00 | 61.02 |
| 1095 | C | GLU | 662 | 51.193 | 8.277 | 51.784 | 1.00 | 59.86 |
| 1096 | O | GLU | 662 | 51.333 | 7.263 | 52.466 | 1.00 | 63.08 |
| 1097 | N | TYR | 663 | 50.007 | 8.771 | 51.436 | 1.00 | 61.62 |
| 1098 | CA | TYR | 663 | 48.716 | 8.186 | 51.812 | 1.00 | 62.54 |
| 1099 | CB | TYR | 663 | 47.601 | 8.921 | 51.068 | 1.00 | 63.89 |
| 1100 | CG | TYR | 663 | 46.266 | 8.230 | 51.167 | 1.00 | 61.84 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1101 | CD1 | TYR | 663 | 45.619 | 8.109 | 52.392 | 1.00 | 59.60 |
| 1102 | CE1 | TYR | 663 | 44.407 | 7.458 | 52.498 | 1.00 | 58.52 |
| 1103 | CD2 | TYR | 663 | 45.659 | 7.676 | 50.043 | 1.00 | 58.65 |
| 1104 | CE2 | TYR | 663 | 44.441 | 7.019 | 50.138 | 1.00 | 60.83 |
| 1105 | CZ | TYR | 663 | 43.820 | 6.916 | 51.368 | 1.00 | 62.58 |
| 1106 | OH | TYR | 663 | 42.601 | 6.287 | 51.477 | 1.00 | 61.21 |
| 1107 | C | TYR | 663 | 48.565 | 6.680 | 51.537 | 1.00 | 59.94 |
| 1108 | O | TYR | 663 | 48.090 | 5.918 | 52.389 | 1.00 | 60.92 |
| 1109 | N | LEU | 664 | 48.930 | 6.274 | 50.325 | 1.00 | 60.84 |
| 1110 | CA | LEU | 664 | 48.846 | 4.881 | 49.908 | 1.00 | 61.56 |
| 1111 | CB | LEU | 664 | 49.261 | 4.757 | 48.438 | 1.00 | 60.37 |
| 1112 | CG | LEU | 664 | 48.363 | 5.402 | 47.382 | 1.00 | 64.33 |
| 1113 | CD1 | LEU | 664 | 49.036 | 5.350 | 46.023 | 1.00 | 64.49 |
| 1114 | CD2 | LEU | 664 | 47.032 | 4.687 | 47.351 | 1.00 | 59.02 |
| 1115 | C | LEU | 664 | 49.744 | 4.001 | 50.777 | 1.00 | 60.21 |
| 1116 | O | LEU | 664 | 49.369 | 2.889 | 51.161 | 1.00 | 61.89 |
| 1117 | N | CYS | 665 | 50.933 | 4.519 | 51.071 | 1.00 | 61.72 |
| 1118 | CA | CYS | 665 | 51.915 | 3.823 | 51.882 | 1.00 | 58.12 |
| 1119 | CB | CYS | 665 | 53.272 | 4.508 | 51.737 | 1.00 | 62.31 |
| 1120 | SG | CYS | 665 | 54.006 | 4.295 | 50.123 | 1.00 | 59.12 |
| 1121 | C | CYS | 665 | 51.516 | 3.771 | 53.348 | 1.00 | 59.84 |
| 1122 | O | CYS | 665 | 51.726 | 2.766 | 54.024 | 1.00 | 61.71 |
| 1123 | N | MET | 666 | 50.953 | 4.862 | 53.845 | 1.00 | 63.34 |
| 1124 | CA | MET | 666 | 50.524 | 4.910 | 55.228 | 1.00 | 58.59 |
| 1125 | CB | MET | 666 | 50.199 | 6.341 | 55.627 | 1.00 | 59.71 |
| 1126 | CG | MET | 666 | 51.408 | 7.189 | 55.867 | 1.00 | 62.55 |
| 1127 | SD | MET | 666 | 50.921 | 8.891 | 56.036 | 1.00 | 61.95 |
| 1128 | CE | MET | 666 | 50.313 | 8.927 | 57.659 | 1.00 | 64.15 |
| 1129 | C | MET | 666 | 49.303 | 4.023 | 55.437 | 1.00 | 63.86 |
| 1130 | O | MET | 666 | 49.146 | 3.420 | 56.495 | 1.00 | 59.48 |
| 1131 | N | LYS | 667 | 48.446 | 3.950 | 54.421 | 1.00 | 58.08 |
| 1132 | CA | LYS | 667 | 47.241 | 3.129 | 54.482 | 1.00 | 61.17 |
| 1133 | CB | LYS | 667 | 46.318 | 3.429 | 53.305 | 1.00 | 66.33 |
| 1134 | CG | LYS | 667 | 45.013 | 2.663 | 53.338 | 1.00 | 59.73 |
| 1135 | CD | LYS | 667 | 43.951 | 3.382 | 52.532 | 1.00 | 60.07 |
| 1136 | CE | LYS | 667 | 44.313 | 3.462 | 51.063 | 1.00 | 62.82 |
| 1137 | NZ | LYS | 667 | 44.134 | 2.158 | 50.390 | 1.00 | 62.44 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1138 | C | LYS | 667 | 47.592 | 1.658 | 54.468 | 1.00 | 63.84 |
| 1139 | O | LYS | 667 | 46.867 | 0.838 | 55.011 | 1.00 | 61.95 |
| 1140 | N | THR | 668 | 48.705 | 1.318 | 53.838 | 1.00 | 63.32 |
| 1141 | CA | THR | 668 | 49.114 | -0.069 | 53.801 | 1.00 | 61.88 |
| 1142 | CB | THR | 668 | 50.080 | -0.304 | 52.657 | 1.00 | 60.27 |
| 1143 | OG1 | THR | 668 | 49.463 | 0.124 | 51.439 | 1.00 | 62.16 |
| 1144 | CG2 | THR | 668 | 50.417 | -1.775 | 52.547 | 1.00 | 58.76 |
| 1145 | C | THR | 668 | 49.761 | -0.413 | 55.137 | 1.00 | 62.68 |
| 1146 | O | THR | 668 | 49.707 | -1.559 | 55.591 | 1.00 | 62.61 |
| 1147 | N | LEU | 669 | 50.350 | 0.597 | 55.773 | 1.00 | 62.26 |
| 1148 | CA | LEU | 669 | 50.995 | 0.427 | 57.068 | 1.00 | 61.58 |
| 1149 | CB | LEU | 669 | 51.888 | 1.626 | 57.378 | 1.00 | 62.50 |
| 1150 | CG | LEU | 669 | 53.265 | 1.643 | 56.712 | 1.00 | 57.56 |
| 1151 | CD1 | LEU | 669 | 54.041 | 2.847 | 57.205 | 1.00 | 60.97 |
| 1152 | CD2 | LEU | 669 | 54.012 | 0.355 | 57.037 | 1.00 | 59.22 |
| 1153 | C | LEU | 669 | 49.987 | 0.249 | 58.194 | 1.00 | 61.00 |
| 1154 | O | LEU | 669 | 50.354 | -0.119 | 59.310 | 1.00 | 61.30 |
| 1155 | N | LEU | 670 | 48.718 | 0.520 | 57.911 | 1.00 | 62.31 |
| 1156 | CA | LEU | 670 | 47.686 | 0.365 | 58.925 | 1.00 | 62.17 |
| 1157 | CB | LEU | 670 | 46.511 | 1.305 | 58.638 | 1.00 | 64.37 |
| 1158 | CG | LEU | 670 | 46.784 | 2.784 | 58.942 | 1.00 | 60.87 |
| 1159 | CD1 | LEU | 670 | 45.516 | 3.597 | 58.766 | 1.00 | 63.22 |
| 1160 | CD2 | LEU | 670 | 47.293 | 2.922 | 60.365 | 1.00 | 58.85 |
| 1161 | C | LEU | 670 | 47.227 | -1.090 | 58.976 | 1.00 | 63.40 |
| 1162 | O | LEU | 670 | 46.846 | -1.599 | 60.026 | 1.00 | 63.58 |
| 1163 | N | LEU | 671 | 47.281 | -1.750 | 57.827 | 1.00 | 61.89 |
| 1164 | CA | LEU | 671 | 46.913 | -3.150 | 57.716 | 1.00 | 60.74 |
| 1165 | CB | LEU | 671 | 46.946 | -3.574 | 56.249 | 1.00 | 62.54 |
| 1166 | CG | LEU | 671 | 46.501 | -4.997 | 55.921 | 1.00 | 62.48 |
| 1167 | CD1 | LEU | 671 | 45.015 | -5.180 | 56.251 | 1.00 | 63.63 |
| 1168 | CD2 | LEU | 671 | 46.768 | -5.260 | 54.449 | 1.00 | 63.52 |
| 1169 | C | LEU | 671 | 47.967 | -3.928 | 58.500 | 1.00 | 61.46 |
| 1170 | O | LEU | 671 | 47.688 | -4.971 | 59.110 | 1.00 | 60.81 |
| 1171 | N | LEU | 672 | 49.182 | -3.388 | 58.479 | 1.00 | 62.75 |
| 1172 | CA | LEU | 672 | 50.320 | -3.974 | 59.163 | 1.00 | 62.92 |
| 1173 | CB | LEU | 672 | 51.562 | -3.883 | 58.280 | 1.00 | 60.56 |
| 1174 | CG | LEU | 672 | 51.399 | -4.164 | 56.786 | 1.00 | 62.90 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1175 | CD1 | LEU | 672 | 52.776 | -4.394 | 56.183 | 1.00 | 60.90 |
| 1176 | CD2 | LEU | 672 | 50.519 | -5.373 | 56.558 | 1.00 | 61.77 |
| 1177 | C | LEU | 672 | 50.576 | -3.232 | 60.468 | 1.00 | 61.14 |
| 1178 | O | LEU | 672 | 51.722 | -3.064 | 60.883 | 1.00 | 63.28 |
| 1179 | N | SER | 673 | 49.502 | -2.795 | 61.116 | 1.00 | 62.86 |
| 1180 | CA | SER | 673 | 49.616 | -2.056 | 62.368 | 1.00 | 62.27 |
| 1181 | CB | SER | 673 | 48.582 | -0.910 | 62.405 | 1.00 | 61.42 |
| 1182 | OG | SER | 673 | 47.241 | -1.383 | 62.404 | 1.00 | 59.49 |
| 1183 | C | SER | 673 | 49.468 | -2.925 | 63.616 | 1.00 | 63.23 |
| 1184 | O | SER | 673 | 50.026 | -2.608 | 64.664 | 1.00 | 59.71 |
| 1185 | N | SER | 674 | 48.720 | -4.017 | 63.517 | 1.00 | 61.92 |
| 1186 | CA | SER | 674 | 48.538 | -4.875 | 64.680 | 1.00 | 60.38 |
| 1187 | CB | SER | 674 | 47.225 | -4.506 | 65.401 | 1.00 | 62.70 |
| 1188 | OG | SER | 674 | 46.204 | -4.115 | 64.495 | 1.00 | 56.91 |
| 1189 | C | SER | 674 | 48.590 | -6.373 | 64.405 | 1.00 | 61.52 |
| 1190 | O | SER | 674 | 48.122 | -6.849 | 63.377 | 1.00 | 61.47 |
| 1191 | N | VAL | 675 | 49.192 | -7.101 | 65.336 | 1.00 | 64.16 |
| 1192 | CA | VAL | 675 | 49.305 | -8.556 | 65.256 | 1.00 | 61.09 |
| 1193 | CB | VAL | 675 | 50.722 | -9.025 | 64.816 | 1.00 | 62.88 |
| 1194 | CG1 | VAL | 675 | 50.962 | -8.679 | 63.362 | 1.00 | 59.26 |
| 1195 | CG2 | VAL | 675 | 51.787 | -8.394 | 65.710 | 1.00 | 61.45 |
| 1196 | C | VAL | 675 | 49.039 | -9.116 | 66.652 | 1.00 | 62.60 |
| 1197 | O | VAL | 675 | 49.265 | -8.433 | 67.656 | 1.00 | 61.24 |
| 1198 | N | PRO | 676 | 48.550 | -10.363 | 66.735 | 1.00 | 59.77 |
| 1199 | CD | PRO | 676 | 48.219 | -11.265 | 65.616 | 1.00 | 57.51 |
| 1200 | CA | PRO | 676 | 48.260 | -11.001 | 68.020 | 1.00 | 62.72 |
| 1201 | CB | PRO | 676 | 48.026 | -12.452 | 67.623 | 1.00 | 60.61 |
| 1202 | CG | PRO | 676 | 47.362 | -12.317 | 66.283 | 1.00 | 59.30 |
| 1203 | C | PRO | 676 | 49.424 | -10.847 | 68.994 | 1.00 | 61.35 |
| 1204 | O | PRO | 676 | 50.585 | -10.758 | 68.576 | 1.00 | 63.84 |
| 1205 | N | LYS | 677 | 49.117 | -10.818 | 70.290 | 1.00 | 62.54 |
| 1206 | CA | LYS | 677 | 50.154 | -10.673 | 71.314 | 1.00 | 60.54 |
| 1207 | CB | LYS | 677 | 49.510 | -10.646 | 72.702 | 1.00 | 61.63 |
| 1208 | CG | LYS | 677 | 50.472 | -10.433 | 73.853 | 1.00 | 58.65 |
| 1209 | CD | LYS | 677 | 49.861 | -10.969 | 75.143 | 1.00 | 62.01 |
| 1210 | CE | LYS | 677 | 50.912 | -11.109 | 76.241 | 1.00 | 63.79 |
| 1211 | NZ | LYS | 677 | 50.350 | -11.727 | 77.491 | 1.00 | 60.49. |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1212 | C | LYS | 677 | 51.162 | -11.828 | 71.222 | 1.00 | 61.45 |
| 1213 | O | LYS | 677 | 52.102 | -11.917 | 72.023 | 1.00 | 59.62 |
| 1214 | N | ASP | 678 | 50.955 | -12.698 | 70.231 | 1.00 | 59.93 |
| 1215 | CA | ASP | 678 | 51.809 | -13.862 | 69.994 | 1.00 | 60.14 |
| 1216 | CB | ASP | 678 | 51.041 | -15.126 | 70.367 | 1.00 | 60.09 |
| 1217 | CG | ASP | 678 | 50.390 | -15.023 | 71.735 | 1.00 | 61.43 |
| 1218 | OD1 | ASP | 678 | 51.110 | -14.940 | 72.749 | 1.00 | 62.42 |
| 1219 | OD2 | ASP | 678 | 49.151 | -15.016 | 71.797 | 1.00 | 60.77 |
| 1220 | C | ASP | 678 | 52.273 | -13.947 | 68.535 | 1.00 | 63.29 |
| 1221 | O | ASP | 678 | 52.771 | -14.981 | 68.090 | 1.00 | 59.36 |
| 1222 | N | GLY | 679 | 52.098 | -12.854 | 67.797 | 1.00 | 59.82 |
| 1223 | CA | GLY | 679 | 52.512 | -12.823 | 66.408 | 1.00 | 58.14 |
| 1224 | C | GLY | 679 | 51.639 | -13.640 | 65.481 | 1.00 | 58.50 |
| 1225 | O | GLY | 679 | 50.600 | -14.179 | 65.870 | 1.00 | 58.88 |
| 1226 | N | LEU | 680 | 52.082 | -13.723 | 64.237 | 1.00 | 62.95 |
| 1227 | CA | LEU | 680 | 51.375 | -14.459 | 63.209 | 1.00 | 63.18 |
| 1228 | CB | LEU | 680 | 51.233 | -13.564 | 61.981 | 1.00 | 65.44 |
| 1229 | CG | LEU | 680 | 50.941 | -12.101 | 62.324 | 1.00 | 60.24 |
| 1230 | CD1 | LEU | 680 | 50.994 | -11.260 | 61.069 | 1.00 | 60.21 |
| 1231 | CD2 | LEU | 680 | 49.582 | -11.979 | 62.974 | 1.00 | 60.11 |
| 1232 | C | LEU | 680 | 52.221 | -15.685 | 62.881 | 1.00 | 63.74 |
| 1233 | O | LEU | 680 | 53.430 | -15.689 | 63.110 | 1.00 | 62.10 |
| 1234 | N | LYS | 681 | 51.598 | -16.729 | 62.354 | 1.00 | 60.36 |
| 1235 | CA | LYS | 681 | 52.348 | -17.922 | 62.000 | 1.00 | 60.78 |
| 1236 | CB | LYS | 681 | 51.406 | -18.969 | 61.418 | 1.00 | 60.11 |
| 1237 | CG | LYS | 681 | 50.209 | -19.253 | 62.289 | 1.00 | 61.43 |
| 1238 | CD | LYS | 681 | 49.295 | -20.221 | 61.579 | 1.00 | 63.30 |
| 1239 | CE | LYS | 681 | 47.908 | -20.186 | 62.160 | 1.00 | 61.77 |
| 1240 | NZ | LYS | 681 | 46.983 | -20.886 | 61.244 | 1.00 | 62.13 |
| 1241 | C | LYS | 681 | 53.429 | -17.551 | 60.973 | 1.00 | 61.58 |
| 1242 | O | LYS | 681 | 54.401 | -18.286 | 60.784 | 1.00 | 63.01 |
| 1243 | N | SER | 682 | 53.250 | -16.410 | 60.309 | 1.00 | 60.35 |
| 1244 | CA | SER | 682 | 54.211 | -15.932 | 59.314 | 1.00 | 62.36 |
| 1245 | CB | SER | 682 | 53.515 | -15.613 | 57.989 | 1.00 | 61.24 |
| 1246 | OG | SER | 682 | 53.066 | -16.788 | 57.346 | 1.00 | 63.18 |
| 1247 | C | SER | 682 | 54.885 | -14.674 | 59.826 | 1.00 | 64.68 |
| 1248 | O | SER | 682 | 55.289 | -13.814 | 59.051 | 1.00 | 59.53 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1249 | N | GLN | 683 | 55.012 | -14.579 | 61.140 | 1.00 | 64.92 |
| 1250 | CA | GLN | 683 | 55.614 | -13.411 | 61.754 | 1.00 | 62.89 |
| 1251 | CB | GLN | 683 | 55.862 | -13.679 | 63.240 | 1.00 | 63.65 |
| 1252 | CG | GLN | 683 | 56.282 | -12.452 | 64.059 | 1.00 | 64.38 |
| 1253 | CD | GLN | 683 | 55.318 | -11.274 | 63.954 | 1.00 | 64.26 |
| 1254 | OE1 | GLN | 683 | 55.688 | -10.205 | 63.476 | 1.00 | 63.72 |
| 1255 | NE2 | GLN | 683 | 54.085 | -11.465 | 64.407 | 1.00 | 61.52 |
| 1256 | C | GLN | 683 | 56.893 | -12.938 | 61.069 | 1.00 | 60.69 |
| 1257 | O | GLN | 683 | 56.981 | -11.782 | 60.669 | 1.00 | 62.35 |
| 1258 | N | GLU | 684 | 57.880 | -13.811 | 60.913 | 1.00 | 60.59 |
| 1259 | CA | GLU | 684 | 59.119 | -13.378 | 60.279 | 1.00 | 61.72 |
| 1260 | CB | GLU | 684 | 60.039 | -14.567 | 59.970 | 1.00 | 61.83 |
| 1261 | CG | GLU | 684 | 60.015 | -15.013 | 58.511 | 1.00 | 61.93 |
| 1262 | CD | GLU | 684 | 61.383 | -15.418 | 57.979 | 1.00 | 60.75 |
| 1263 | OE1 | GLU | 684 | 61.457 | -15.813 | 56.792 | 1.00 | 60.52 |
| 1264 | OE2 | GLU | 684 | 62.375 | -15.342 | 58.744 | 1.00 | 58.72 |
| 1265 | C | GLU | 684 | 58.801 | -12.623 | 58.993 | 1.00 | 60.55 |
| 1266 | O | GLU | 684 | 59.196 | -11.474 | 58.823 | 1.00 | 63.55 |
| 1267 | N | LEU | 685 | 58.064 | -13.263 | 58.100 | 1.00 | 63.64 |
| 1268 | CA | LEU | 685 | 57.710 | -12.649 | 56.834 | 1.00 | 57.80 |
| 1269 | CB | LEU | 685 | 56.921 | -13.649 | 55.985 | 1.00 | 59.68 |
| 1270 | CG | LEU | 685 | 57.165 | -13.734 | 54.476 | 1.00 | 63.20 |
| 1271 | CD1 | LEU | 685 | 55.839 | -14.063 | 53.829 | 1.00 | 59.43 |
| 1272 | CD2 | LEU | 685 | 57.699 | -12.434 | 53.902 | 1.00 | 62.25 |
| 1273 | C | LEU | 685 | 56.882 | -11.370 | 57.040 | 1.00 | 62.31 |
| 1274 | O | LEU | 685 | 56.953 | -10.432 | 56.242 | 1.00 | 61.77 |
| 1275 | N | PHE | 686 | 56.095 | -11.326 | 58.109 | 1.00 | 60.23 |
| 1276 | CA | PHE | 686 | 55.272 | -10.149 | 58.374 | 1.00 | 61.37 |
| 1277 | CB | PHE | 686 | 54.409 | -10.365 | 59.609 | 1.00 | 64.14 |
| 1278 | CG | PHE | 686 | 53.663 | -9.143 | 60.023 | 1.00 | 61.62 |
| 1279 | CD1 | PHE | 686 | 52.639 | -8.650 | 59.236 | 1.00 | 60.24 |
| 1280 | CD2 | PHE | 686 | 54.008 | -8.460 | 61.178 | 1.00 | 62.13 |
| 1281 | CE1 | PHE | 686 | 51.971 | -7.493 | 59.592 | 1.00 | 58.68 |
| 1282 | CE2 | PHE | 686 | 53.347 | -7.304 | 61.543 | 1.00 | 61.95 |
| 1283 | CZ | PHE | 686 | 52.326 | -6.818 | 60.749 | 1.00 | 61.43 |
| 1284 | C | PHE | 686 | 56.109 | -8.897 | 58.596 | 1.00 | 59.14 |
| 1285 | O | PHE | 686 | 56.247 | -8.051 | 57.714 | 1.00 | 61.44 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1286 | N | ASP | 687 | 56.651 | -8.791 | 59.802 | 1.00 | 64.05 |
| 1287 | CA | ASP | 687 | 57.483 | -7.668 | 60.195 | 1.00 | 60.85 |
| 1288 | CB | ASP | 687 | 58.102 | -7.960 | 61.567 | 1.00 | 56.84 |
| 1289 | CG | ASP | 687 | 59.033 | -9.162 | 61.536 | 1.00 | 64.67 |
| 1290 | OD1 | ASP | 687 | 59.482 | -9.610 | 62.622 | 1.00 | 60.21 |
| 1291 | OD2 | ASP | 687 | 59.322 | -9.655 | 60.414 | 1.00 | 63.02 |
| 1292 | C | ASP | 687 | 58.573 | -7.383 | 59.142 | 1.00 | 61.59 |
| 1293 | O | ASP | 687 | 59.262 | -6.358 | 59.214 | 1.00 | 61.60 |
| 1294 | N | GLU | 688 | 58.725 | -8.289 | 58.172 | 1.00 | 60.82 |
| 1295 | CA | GLU | 688 | 59.700 | -8.104 | 57.098 | 1.00 | 59.68 |
| 1296 | CB | GLU | 688 | 60.246 | -9.433 | 56.604 | 1.00 | 62.54 |
| 1297 | CG | GLU | 688 | 61.434 | -9.252 | 55.674 | 1.00 | 60.98 |
| 1298 | CD | GLU | 688 | 61.479 | -10.289 | 54.569 | 1.00 | 58.58 |
| 1299 | OE1 | GLU | 688 | 61.323 | -9.898 | 53.387 | 1.00 | 61.73 |
| 1300 | OE2 | GLU | 688 | 61.663 | -11.490 | 54.882 | 1.00 | 60.24 |
| 1301 | C | GLU | 688 | 59.031 | -7.387 | 55.931 | 1.00 | 60.97 |
| 1302 | O | GLU | 688 | 59.684 | -6.719 | 55.137 | 1.00 | 62.27 |
| 1303 | N | ILE | 689 | 57.721 | -7.552 | 55.809 | 1.00 | 61.23 |
| 1304 | CA | ILE | 689 | 56.979 | -6.864 | 54.767 | 1.00 | 61.00 |
| 1305 | CB | ILE | 689 | 55.641 | -7.592 | 54.451 | 1.00 | 63.41 |
| 1306 | CG2 | ILE | 689 | 54.655 | -6.650 | 53.759 | 1.00 | 59.25 |
| 1307 | CG1 | ILE | 689 | 55.916 | -8.808 | 53.568 | 1.00 | 57.54 |
| 1308 | CD1 | ILE | 689 | 54.667 | -9.526 | 53.121 | 1.00 | 62.29 |
| 1309 | C | ILE | 689 | 56.704 | -5.479 | 55.345 | 1.00 | 58.97 |
| 1310 | O | ILE | 689 | 56.778 | -4.473 | 54.645 | 1.00 | 61.54 |
| 1311 | N | ARG | 690 | 56.411 | -5.439 | 56.641 | 1.00 | 60.16 |
| 1312 | CA | ARG | 690 | 56.135 | -4.185 | 57.319 | 1.00 | 61.28 |
| 1313 | CB | ARG | 690 | 55.855 | -4.434 | 58.799 | 1.00 | 60.29 |
| 1314 | CG | ARG | 690 | 55.548 | -3.170 | 59.582 | 1.00 | 60.74 |
| 1315 | CD | ARG | 690 | 54.679 | -3.480 | 60.770 | 1.00 | 56.91 |
| 1316 | NE | ARG | 690 | 54.190 | -2.280 | 61.437 | 1.00 | 66.99 |
| 1317 | CZ | ARG | 690 | 54.967 | -1.378 | 62.026 | 1.00 | 64.04 |
| 1318 | NH1 | ARG | 690 | 56.283 | -1.533 | 62.029 | 1.00 | 58.19 |
| 1319 | NH2 | ARG | 690 | 54.427 | -0.327 | 62.623 | 1.00 | 62.52 |
| 1320 | C | ARG | 690 | 57.274 | -3.179 | 57.177 | 1.00 | 57.93 |
| 1321 | O | ARG | 690 | 57.037 | -1.977 | 57.067 | 1.00 | 59.25 |
| 1322 | N | MET | 691 | 58.512 | -3.660 | 57.190 | 1.00 | 58.75 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1323 | CA | MET | 691 | 59.664 | -2.783 | 57.048 | 1.00 | 64.96 |
| 1324 | CB | MET | 691 | 60.928 | -3.542 | 57.450 | 1.00 | 61.53 |
| 1325 | CG | MET | 691 | 62.247 | -3.016 | 56.886 | 1.00 | 59.40 |
| 1326 | SD | MET | 691 | 62.942 | -4.201 | 55.673 | 1.00 | 61.45 |
| 1327 | CE | MET | 691 | 63.225 | -5.669 | 56.764 | 1.00 | 60.93 |
| 1328 | C | MET | 691 | 59.775 | -2.254 | 55.621 | 1.00 | 61.68 |
| 1329 | O | MET | 691 | 60.130 | -1.099 | 55.405 | 1.00 | 63.95 |
| 1330 | N | THR | 692 | 59.459 | -3.097 | 54.646 | 1.00 | 59.70 |
| 1331 | CA | THR | 692 | 59.519 | -2.698 | 53.243 | 1.00 | 60.57 |
| 1332 | CB | THR | 692 | 59.105 | -3.855 | 52.323 | 1.00 | 62.51 |
| 1333 | OG1 | THR | 692 | 59.796 | -5.046 | 52.714 | 1.00 | 59.90 |
| 1334 | CG2 | THR | 692 | 59.437 | -3.523 | 50.879 | 1.00 | 60.93 |
| 1335 | C | THR | 692 | 58.586 | -1.523 | 52.962 | 1.00 | 60.46 |
| 1336 | O | THR | 692 | 58.890 | -0.655 | 52.143 | 1.00 | 59.04 |
| 1337 | N | TYR | 693 | 57.439 | -1.516 | 53.634 | 1.00 | 61.66 |
| 1338 | CA | TYR | 693 | 56.459 | -0.458 | 53.461 | 1.00 | 60.32 |
| 1339 | CB | TYR | 693 | 55.045 | -1.033 | 53.553 | 1.00 | 59.81 |
| 1340 | CG | TYR | 693 | 54.665 | -1.802 | 52.302 | 1.00 | 61.04 |
| 1341 | CD1 | TYR | 693 | 54.552 | -1.153 | 51.073 | 1.00 | 63.46 |
| 1342 | CE1 | TYR | 693 | 54.292 | -1.865 | 49.909 | 1.00 | 65.96 |
| 1343 | CD2 | TYR | 693 | 54.497 | -3.185 | 52.332 | 1.00 | 59.82 |
| 1344 | CE2 | TYR | 693 | 54.236 | -3.906 | 51.170 | 1.00 | 63.54 |
| 1345 | CZ | TYR | 693 | 54.137 | -3.242 | 49.967 | 1.00 | 62.96 |
| 1346 | OH | TYR | 693 | 53.901 | -3.961 | 48.822 | 1.00 | 59.68 |
| 1347 | C | TYR | 693 | 56.663 | 0.664 | 54.458 | 1.00 | 58.94 |
| 1348 | O | TYR | 693 | 55.877 | 1.600 | 54.527 | 1.00 | 59.49 |
| 1349 | N | ILE | 694 | 57.720 | 0.555 | 55.248 | 1.00 | 63.16 |
| 1350 | CA | ILE | 694 | 58.052 | 1.598 | 56.195 | 1.00 | 61.35 |
| 1351 | CB | ILE | 694 | 58.734 | 1.042 | 57.462 | 1.00 | 59.89 |
| 1352 | CG2 | ILE | 694 | 59.652 | 2.101 | 58.077 | 1.00 | 62.13 |
| 1353 | CG1 | ILE | 694 | 57.668 | 0.580 | 58.457 | 1.00 | 63.95 |
| 1354 | CD1 | ILE | 694 | 58.216 | 0.168 | 59.793 | 1.00 | 61.57 |
| 1355 | C | ILE | 694 | 59.028 | 2.478 | 55.430 | 1.00 | 58.86 |
| 1356 | O | ILE | 694 | 58.989 | 3.701 | 55.541 | 1.00 | 62.76 |
| 1357 | N | LYS | 695 | 59.890 | 1.838 | 54.643 | 1.00 | 61.80 |
| 1358 | CA | LYS | 695 | 60.869 | 2.539 | 53.821 | 1.00 | 60.19 |
| 1359 | CB | LYS | 695 | 61.987 | 1.607 | 53.364 | 1.00 | 61.05 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1360 | CG | LYS | 695 | 62.768 | 0.892 | 54.438 | 1.00 | 60.54 |
| 1361 | CD | LYS | 695 | 63.876 | 0.081 | 53.767 | 1.00 | 63.44 |
| 1362 | CE | LYS | 695 | 64.516 | -0.947 | 54.699 | 1.00 | 61.61 |
| 1363 | NZ | LYS | 695 | 65.330 | -1.962 | 53.942 | 1.00 | 62.18 |
| 1364 | C | LYS | 695 | 60.206 | 3.074 | 52.559 | 1.00 | 61.44 |
| 1365 | O | LYS | 695 | 60.706 | 4.010 | 51.946 | 1.00 | 61.23 |
| 1366 | N | GLU | 696 | 59.101 | 2.452 | 52.156 | 1.00 | 61.65 |
| 1367 | CA | GLU | 696 | 58.381 | 2.862 | 50.961 | 1.00 | 60.70 |
| 1368 | CB | GLU | 696 | 57.289 | 1.851 | 50.635 | 1.00 | 62.74 |
| 1369 | CG | GLU | 696 | 56.806 | 1.901 | 49.200 | 1.00 | 63.74 |
| 1370 | CD | GLU | 696 | 57.927 | 1.725 | 48.187 | 1.00 | 63.24 |
| 1371 | OE1 | GLU | 696 | 58.903 | 1.002 | 48.494 | 1.00 | 61.23 |
| 1372 | OE2 | GLU | 696 | 57.824 | 2.299 | 47.079 | 1.00 | 62.98 |
| 1373 | C | GLU | 696 | 57.775 | 4.215 | 51.260 | 1.00 | 61.27 |
| 1374 | O | GLU | 696 | 57.594 | 5.051 | 50.366 | 1.00 | 61.63 |
| 1375 | N | LEU | 697 | 57.468 | 4.414 | 52.540 | 1.00 | 59.99 |
| 1376 | CA | LEU | 697 | 56.912 | 5.668 | 53.022 | 1.00 | 63.89 |
| 1377 | CB | LEU | 697 | 56.314 | 5.493 | 54.418 | 1.00 | 58.18 |
| 1378 | CG | LEU | 697 | 55.831 | 6.810 | 55.016 | 1.00 | 62.97 |
| 1379 | CD1 | LEU | 697 | 54.754 | 7.370 | 54.127 | 1.00 | 59.80 |
| 1380 | CD2 | LEU | 697 | 55.319 | 6.606 | 56.425 | 1.00 | 59.24 |
| 1381 | C | LEU | 697 | 58.029 | 6.709 | 53.072 | 1.00 | 61.66 |
| 1382 | O | LEU | 697 | 57.807 | 7.871 | 52.774 | 1.00 | 60.35 |
| 1383 | N | GLY | 698 | 59.228 | 6.283 | 53.460 | 1.00 | 59.57 |
| 1384 | CA | GLY | 698 | 60.348 | 7.198 | 53.523 | 1.00 | 61.15 |
| 1385 | C | GLY | 698 | 60.570 | 7.770 | 52.146 | 1.00 | 60.91 |
| 1386 | O | GLY | 698 | 60.748 | 8.977 | 51.988 | 1.00 | 61.15 |
| 1387 | N | LYS | 699 | 60.557 | 6.880 | 51.156 | 1.00 | 60.97 |
| 1388 | CA | LYS | 699 | 60.729 | 7.219 | 49.745 | 1.00 | 60.90 |
| 1389 | CB | LYS | 699 | 60.526 | 5.983 | 48.875 | 1.00 | 66.06 |
| 1390 | CG | LYS | 699 | 61.729 | 5.098 | 48.621 | 1.00 | 61.25 |
| 1391 | CD | LYS | 699 | 61.290 | 3.930 | 47.737 | 1.00 | 59.41 |
| 1392 | CE | LYS | 699 | 62.371 | 3.498 | 46.765 | 1.00 | 61.62 |
| 1393 | NZ | LYS | 699 | 63.066 | 2.246 | 47.178 | 1.00 | 60.87 |
| 1394 | C | LYS | 699 | 59.710 | 8.256 | 49.289 | 1.00 | 58.54 |
| 1395 | O | LYS | 699 | 60.019 | 9.127 | 48.482 | 1.00 | 59.98 |
| 1396 | N | ALA | 700 | 58.483 | 8.128 | 49.777 | 1.00 | 63.02 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1397 | CA | ALA | 700 | 57.423 | 9.048 | 49.408 | 1.00 | 61.82 |
| 1398 | CB | ALA | 700 | 56.094 | 8.540 | 49.926 | 1.00 | 65.26 |
| 1399 | C | ALA | 700 | 57.712 | 10.422 | 49.977 | 1.00 | 60.36 |
| 1400 | O | ALA | 700 | 57.545 | 11.434 | 49.298 | 1.00 | 62.77 |
| 1401 | N | ILE | 701 | 58.137 | 10.446 | 51.235 | 1.00 | 65.16 |
| 1402 | CA | ILE | 701 | 58.471 | 11.683 | 51.917 | 1.00 | 64.84 |
| 1403 | CB | ILE | 701 | 58.931 | 11.406 | 53.338 | 1.00 | 61.36 |
| 1404 | CG2 | ILE | 701 | 59.509 | 12.670 | 53.953 | 1.00 | 63.03 |
| 1405 | CG1 | ILE | 701 | 57.761 | 10.874 | 54.151 | 1.00 | 61.61 |
| 1406 | CD1 | ILE | 701 | 58.167 | 10.364 | 55.495 | 1.00 | 60.05 |
| 1407 | C | ILE | 701 | 59.574 | 12.455 | 51.195 | 1.00 | 61.64 |
| 1408 | O | ILE | 701 | 59.597 | 13.683 | 51.228 | 1.00 | 58.62 |
| 1409 | N | VAL | 702 | 60.500 | 11.749 | 50.555 | 1.00 | 63.95 |
| 1410 | CA | VAL | 702 | 61.560 | 12.438 | 49.831 | 1.00 | 57.69 |
| 1411 | CB | VAL | 702 | 62.815 | 11.532 | 49.665 | 1.00 | 63.59 |
| 1412 | CG1 | VAL | 702 | 63.310 | 11.093 | 51.024 | 1.00 | 59.75 |
| 1413 | CG2 | VAL | 702 | 62.494 | 10.330 | 48.819 | 1.00 | 64.22 |
| 1414 | C | VAL | 702 | 61.038 | 12.907 | 48.466 | 1.00 | 61.62 |
| 1415 | O | VAL | 702 | 61.328 | 14.014 | 48.031 | 1.00 | 61.64 |
| 1416 | N | LYS | 703 | 60.244 | 12.065 | 47.814 | 1.00 | 61.58 |
| 1417 | CA | LYS | 703 | 59.666 | 12.387 | 46.516 | 1.00 | 60.75 |
| 1418 | CB | LYS | 703 | 58.475 | 11.474 | 46.201 | 1.00 | 56.80 |
| 1419 | CG | LYS | 703 | 57.419 | 12.161 | 45.293 | 1.00 | 59.93 |
| 1420 | CD | LYS | 703 | 55.967 | 11.815 | 45.660 | 1.00 | 60.60 |
| 1421 | CE | LYS | 703 | 54.962 | 12.848 | 45.088 | 1.00 | 57.15 |
| 1422 | NZ | LYS | 703 | 55.070 | 13.064 | 43.605 | 1.00 | 62.62 |
| 1423 | C | LYS | 703 | 59.169 | 13.814 | 46.392 | 1.00 | 61.49 |
| 1424 | O | LYS | 703 | 59.454 | 14.472 | 45.404 | 1.00 | 58.34 |
| 1425 | N | ARG | 704 | 58.390 | 14.271 | 47.367 | 1.00 | 63.00 |
| 1426 | CA | ARG | 704 | 57.826 | 15.621 | 47.321 | 1.00 | 60.60 |
| 1427 | CB | ARG | 704 | 56.331 | 15.590 | 47.661 | 1.00 | 62.95 |
| 1428 | CG | ARG | 704 | 56.026 | 15.010 | 49.033 | 1.00 | 61.71 |
| 1429 | CD | ARG | 704 | 54.728 | 15.553 | 49.548 | 1.00 | 60.30 |
| 1430 | NE | ARG | 704 | 54.931 | 16.417 | 50.702 | 1.00 | 62.33 |
| 1431 | CZ | ARG | 704 | 53.983 | 17.200 | 51.204 | 1.00 | 60.19 |
| 1432 | NH1 | ARG | 704 | 52.784 | 17.213 | 50.640 | 1.00 | 61.68 |
| 1433 | NH2 | ARG | 704 | 54.225 | 17.965 | 52.263 | 1.00 | 63.26 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1434 | C | ARG | 704 | 58.520 | 16.607 | 48.249 | 1.00 | 63.14 |
| 1435 | O | ARG | 704 | 58.732 | 17.772 | 47.885 | 1.00 | 60.41 |
| 1436 | N | GLU | 705 | 58.842 | 16.155 | 49.458 | 1.00 | 60.89 |
| 1437 | CA | GLU | 705 | 59.528 | 17.018 | 50.412 | 1.00 | 62.58 |
| 1438 | CB | GLU | 705 | 59.814 | 16.288 | 51.730 | 1.00 | 60.91 |
| 1439 | CG | GLU | 705 | 58.605 | 16.091 | 52.605 | 1.00 | 62.30 |
| 1440 | CD | GLU | 705 | 57.847 | 17.384 | 52.817 | 1.00 | 64.22 |
| 1441 | OE1 | GLU | 705 | 56.684 | 17.460 | 52.347 | 1.00 | 58.55 |
| 1442 | OE2 | GLU | 705 | 58.419 | 18.317 | 53.440 | 1.00 | 61.42 |
| 1443 | C | GLU | 705 | 60.848 | 17.456 | 49.801 | 1.00 | 62.10 |
| 1444 | O | GLU | 705 | 61.869 | 16.761 | 49.939 | 1.00 | 59.78 |
| 1445 | N | GLY | 706 | 60.823 | 18.597 | 49.115 | 1.00 | 60.22 |
| 1446 | CA | GLY | 706 | 62.036 | 19.100 | 48.500 | 1.00 | 61.75 |
| 1447 | C | GLY | 706 | 63.159 | 19.239 | 49.518 | 1.00 | 60.44 |
| 1448 | O | GLY | 706 | 64.168 | 18.519 | 49.450 | 1.00 | 61.54 |
| 1449 | N | ASN | 707 | 62.974 | 20.148 | 50.477 | 1.00 | 61.06 |
| 1450 | CA | ASN | 707 | 63.989 | 20.387 | 51.491 | 1.00 | 60.61 |
| 1451 | CB | ASN | 707 | 63.561 | 21.505 | 52.443 | 1.00 | 63.06 |
| 1452 | CG | ASN | 707 | 64.731 | 22.048 | 53.258 | 1.00 | 60.50 |
| 1453 | OD1 | ASN | 707 | 64.663 | 23.152 | 53.803 | 1.00 | 61.18 |
| 1454 | ND2 | ASN | 707 | 65.815 | 21.269 | 53.342 | 1.00 | 61.75 |
| 1455 | C | ASN | 707 | 64.355 | 19.143 | 52.281 | 1.00 | 61.56 |
| 1456 | O | ASN | 707 | 63.685 | 18.767 | 53.250 | 1.00 | 63.56 |
| 1457 | N | SER | 708 | 65.446 | 18.525 | 51.837 | 1.00 | 62.02 |
| 1458 | CA | SER | 708 | 66.024 | 17.326 | 52.427 | 1.00 | 60.22 |
| 1459 | CB | SER | 708 | 67.379 | 17.070 | 51.737 | 1.00 | 58.91 |
| 1460 | OG | SER | 708 | 68.112 | 15.998 | 52.305 | 1.00 | 62.15 |
| 1461 | C | SER | 708 | 66.200 | 17.500 | 53.945 | 1.00 | 63.60 |
| 1462 | O | SER | 708 | 66.754 | 16.635 | 54.624 | 1.00 | 60.95 |
| 1463 | N | SER | 709 | 65.713 | 18.619 | 54.474 | 1.00 | 61.83 |
| 1464 | CA | SER | 709 | 65.826 | 18.922 | 55.894 | 1.00 | 61.35 |
| 1465 | CB | SER | 709 | 66.279 | 20.373 | 56.065 | 1.00 | 65.65 |
| 1466 | OG | SER | 709 | 67.479 | 20.615 | 55.332 | 1.00 | 62.14 |
| 1467 | C | SER | 709 | 64.516 | 18.684 | 56.641 | 1.00 | 59.54 |
| 1468 | O | SER = | 709 | 64.497 | 18.583 | 57.874 | 1.00 | 61.29 |
| 1469 | N | GLN | 710 | 63.416 | 18.586 | 55.900 | 1.00 | 62.57 |
| 1470 | CA | GLN | 710 | 62.131 | 18.347 | 56.533 | 1.00 | 61.59 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1471 | CB | GLN | 710 | 61.087 | 19.333 | 56.007 | 1.00 | 58.05 |
| 1472 | CG | GLN | 710 | 61.469 | 20.792 | 56.249 | 1.00 | 62.98 |
| 1473 | CD | GLN | 710 | 60.344 | 21.776 | 55.943 | 1.00 | 59.84 |
| 1474 | OE1 | GLN | 710 | 59.363 | 21.882 | 56.696 | 1.00 | 60.97 |
| 1475 | NE2 | GLN | 710 | 60.481 | 22.502 | 54.831 | 1.00 | 65.14 |
| 1476 | C | GLN | 710 | 61.683 | 16.917 | 56.297 | 1.00 | 58.31 |
| 1477 | O | GLN | 710 | 60.512 | 16.586 | 56.466 | 1.00 | 60.42 |
| 1478 | N | ASN | 711 | 62.625 | 16.063 | 55.916 | 1.00 | 61.21 |
| 1479 | CA | ASN | 711 | 62.309 | 14.666 | 55.673 | 1.00 | 63.02 |
| 1480 | CB | ASN | 711 | 63.407 | 14.033 | 54.819 | 1.00 | 60.49 |
| 1481 | CG | ASN | 711 | 63.508 | 14.675 | 53.449 | 1.00 | 65.80 |
| 1482 | OD1 | ASN | 711 | 62.565 | 15.303 | 52.977 | 1.00 | 63.88 |
| 1483 | ND2 | ASN | 711 | 64.646 | 14.507 | 52.801 | 1.00 | 60.91 |
| 1484 | C | ASN | 711 | 62.090 | 13.879 | 56.974 | 1.00 | 63.26 |
| 1485 | O | ASN | 711 | 61.055 | 13.238 | 57.155 | 1.00 | 58.14 |
| 1486 | N | TRP | 712 | 63.054 | 13.930 | 57.883 | 1.00 | 61.93 |
| 1487 | CA | TRP | 712 | 62.915 | 13.234 | 59.148 | 1.00 | 59.49 |
| 1488 | CB | TRP | 712 | 64.259 | 13.185 | 59.833 | 1.00 | 62.44 |
| 1489 | CG | TRP | 712 | 65.169 | 12.333 | 59.088 | 1.00 | 62.60 |
| 1490 | CD2 | TRP | 712 | 65.485 | 10.980 | 59.388 | 1.00 | 61.74 |
| 1491 | CE2 | TRP | 712 | 66.331 | 10.516 | 58.366 | 1.00 | 62.04 |
| 1492 | CE3 | TRP | 712 | 65.130 | 10.108 | 60.426 | 1.00 | 60.92 |
| 1493 | CD1 | TRP | 712 | 65.815 | 12.637 | 57.934 | 1.00 | 57.10 |
| 1494 | NE1 | TRP | 712 | 66.517 | 11.552 | 57.490 | 1.00 | 63.63 |
| 1495 | CZ2 | TRP | 712 | 66.832 | 9.215 | 58.345 | 1.00 | 63.94 |
| 1496 | CZ3 | TRP | 712 | 65.625 | 8.817 | 60.407 | 1.00 | 65.64 |
| 1497 | CH2 | TRP | 712 | 66.470 | 8.381 | 59.370 | 1.00 | 62.08 |
| 1498 | C | TRP | 712 | 61.903 | 13.954 | 60.021 | 1.00 | 59.14 |
| 1499 | O | TRP | 712 | 61.372 | 13.410 | 60.996 | 1.00 | 59.32 |
| 1500 | N | GLN | 713 | 61.637 | 15.191 | 59.640 | 1.00 | 60.06 |
| 1501 | CA | GLN | 713 | 60.705 | 16.043 | 60.345 | 1.00 | 63.93 |
| 1502 | CB | GLN | 713 | 60.853 | 17.455 | 59.793 | 1.00 | 59.11 |
| 1503 | CG | GLN | 713 | 60.727 | 18.564 | 60.802 | 1.00 | 61.74 |
| 1504 | CD | GLN | 713 | 59.352 | 19.135 | 60.838 | 1.00 | 60.27 |
| 1505 | OE1 | GLN | 713 | 58.660 | 19.154 | 59.824 | 1.00 | 58.27 |
| 1506 | NE2 | GLN | 713 | 58.942 | 19.628 | 61.998 | 1.00 | 65.68 |
| 1507 | C | GLN | 713 | 59.296 | 15.522 | 60.107 | 1.00 | 57.87 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1508 | O | GLN | 713 | 58.472 | 15.465 | 61.018 | 1.00 | 63.21 |
| 1509 | N | ARG | 714 | 59.051 | 15.125 | 58.864 | 1.00 | 62.15 |
| 1510 | CA | ARG | 714 | 57.758 | 14.626 | 58.424 | 1.00 | 57.88 |
| 1511 | CB | ARG | 714 | 57.668 | 14.782 | 56.907 | 1.00 | 65.30 |
| 1512 | CG | ARG | 714 | 56.272 | 14.971 | 56.382 | 1.00 | 62.96 |
| 1513 | CD | ARG | 714 | 56.301 | 15.439 | 54.940 | 1.00 | 62.68 |
| 1514 | NE | ARG | 714 | 55.029 | 15.200 | 54.267 | 1.00 | 62.08 |
| 1515 | CZ | ARG | 714 | 53.899 | 15.828 | 54.561 | 1.00 | 62.26 |
| 1516 | NH1 | ARG | 714 | 53.877 | 16.741 | 55.515 | 1.00 | 59.12 |
| 1517 | NH2 | ARG | 714 | 52.788 | 15.535 | 53.906 | 1.00 | 57.33 |
| 1518 | C | ARG | 714 | 57.573 | 13.171 | 58.831 | 1.00 | 60.95 |
| 1519 | O | ARG | 714 | 56.531 | 12.787 | 59.368 | 1.00 | 60.00 |
| 1520 | N | PHE | 715 | 58.594 | 12.363 | 58.571 | 1.00 | 59.93 |
| 1521 | CA | PHE | 715 | 58.551 | 10.960 | 58.940 | 1.00 | 61.38 |
| 1522 | CB | PHE | 715 | 59.934 | 10.328 | 58.799 | 1.00 | 60.45 |
| 1523 | CG | PHE | 715 | 59.921 | 8.834 | 58.843 | 1.00 | 63.37 |
| 1524 | CD1 | PHE | 715 | 59.016 | 8.119 | 58.063 | 1.00 | 61.57 |
| 1525 | CD2 | PHE | 715 | 60.824 | 8.136 | 59.636 | 1.00 | 59.17 |
| 1526 | CE1 | PHE | 715 | 59.005 | 6.732 | 58.067 | 1.00 | 60.07 |
| 1527 | CE2 | PHE | 715 | 60.824 | 6.740 | 59.648 | 1.00 | 58.03 |
| 1528 | CZ | PHE | 715 | 59.904 | 6.038 | 58.856 | 1.00 | 61.29 |
| 1529 | C | PHE | 715 | 58.118 | 10.923 | 60.394 | 1.00 | 61.16 |
| 1530 | O | PHE | 715 | 57.409 | 10.017 | 60.821 | 1.00 | 61.52 |
| 1531 | N | TYR | 716 | 58.541 | 11.925 | 61.153 | 1.00 | 62.10 |
| 1532 | CA | TYR | 716 | 58.178 | 11.989 | 62.550 | 1.00 | 61.74 |
| 1533 | CB | TYR | 716 | 58.962 | 13.092 | 63.256 | 1.00 | 60.58 |
| 1534 | CG | TYR | 716 | 58.729 | 13.117 | 64.748 | 1.00 | 60.86 |
| 1535 | CD1 | TYR | 716 | 59.376 | 12.213 | 65.586 | 1.00 | 58.32 |
| 1536 | CE1 | TYR | 716 | 59.159 | 12.226 | 66.952 | 1.00 | 59.67 |
| 1537 | CD2 | TYR | 716 | 57.852 | 14.032 | 65.318 | 1.00 | 60.64 |
| 1538 | CE2 | TYR | 716 | 57.625 | 14.052 | 66.679 | 1.00 | 63.17 |
| 1539 | CZ | TYR | 716 | 58.283 | 13.152 | 67.493 | 1.00 | 58.37 |
| 1540 | OH | TYR | 716 | 58.090 | 13.194 | 68.856 | 1.00 | 59.28 |
| 1541 | C | TYR | 716 | 56.688 | 12.280 | 62.662 | 1.00 | 61.28 |
| 1542 | O | TYR | 716 | 55.952 | 11.578 | 63.356 | 1.00 | 61.70 |
| 1543 | N | GLN | 717 | 56.249 | 13.318 | 61.967 | 1.00 | 62.49 |
| 1544 | CA | GLN | 717 | 54.858 | 13.716 | 62.009 | 1.00 | 63.77 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1545 | CB | GLN | 717 | 54.694 | 15.061 | 61.304 | 1.00 | 63.18 |
| 1546 | CG | GLN | 717 | 55.613 | 16.126 | 61.888 | 1.00 | 62.93 |
| 1547 | CD | GLN | 717 | 55.418 | 17.512 | 61.288 | 1.00 | 62.21 |
| 1548 | OE1 | GLN | 717 | 55.545 | 17.709 | 60.073 | 1.00 | 59.68 |
| 1549 | NE2 | GLN | 717 | 55.125 | 18.487 | 62.147 | 1.00 | 58.70 |
| 1550 | C | GLN | 717 | 53.910 | 12.674 | 61.426 | 1.00 | 61.09 |
| 1551 | O | GLN | 717 | 52.907 | 12.338 | 62.064 | 1.00 | 61.62 |
| 1552 | N | LEU | 718 | 54.228 | 12.154 | 60.237 | 1.00 | 62.25 |
| 1553 | CA | LEU | 718 | 53.384 | 11.144 | 59.589 | 1.00 | 60.93 |
| 1554 | CB | LEU | 718 | 53.880 | 10.833 | 58.166 | 1.00 | 61.18 |
| 1555 | CG | LEU | 718 | 53.823 | 11.915 | 57.078 | 1.00 | 60.69 |
| 1556 | CD1 | LEU | 718 | 54.322 | 11.353 | 55.764 | 1.00 | 59.94 |
| 1557 | CD2 | LEU | 718 | 52.411 | 12.419 | 56.916 | 1.00 | 64.72 |
| 1558 | C | LEU | 718 | 53.308 | 9.847 | 60.391 | 1.00 | 61.41 |
| 1559 | O | LEU | 718 | 52.241 | 9.270 | 60.530 | 1.00 | 62.27 |
| 1560 | N | THR | 719 | 54.441 | 9.387 | 60.911 | 1.00 | 62.60 |
| 1561 | CA | THR | 719 | 54.469 | 8.162 | 61.706 | 1.00 | 61.77 |
| 1562 | CB | THR | 719 | 55.902 | 7.717 | 62.001 | 1.00 | 63.38 |
| 1563 | OG1 | THR | 719 | 56.590 | 8.749 | 62.715 | 1.00 | 59.25 |
| 1564 | CG2 | THR | 719 | 56.626 | 7.420 | 60.716 | 1.00 | 64.41 |
| 1565 | C | THR | 719 | 53.744 | 8.362 | 63.034 | 1.00 | 64.18 |
| 1566 | O | THR | 719 | 53.365 | 7.398 | 63.702 | 1.00 | 59.43 |
| 1567 | N | LYS | 720 | 53.562 | 9.619 | 63.421 | 1.00 | 60.48 |
| 1568 | CA | LYS | 720 | 52.864 | 9.913 | 64.652 | 1.00 | 60.21 |
| 1569 | CB | LYS | 720 | 53.414 | 11.194 | 65.281 | 1.00 | 63.61 |
| 1570 | CG | LYS | 720 | 52.661 | 11.630 | 66.532 | 1.00 | 60.31 |
| 1571 | CD | LYS | 720 | 52.340 | 10.450 | 67.448 | 1.00 | 57.45 |
| 1572 | CE | LYS | 720 | 51.254 | 10.801 | 68.472 | 1.00 | 57.33 |
| 1573 | NZ | LYS | 720 | 50.621 | 9.562 | 69.037 | 1.00 | 61.89 |
| 1574 | C | LYS | 720 | 51.364 | 10.023 | 64.345 | 1.00 | 60.76 |
| 1575 | O | LYS | 720 | 50.523 | 10.016 | 65.246 | 1.00 | 60.43 |
| 1576 | N | LEU | 721 | 51.030 | 10.110 | 63.061 | 1.00 | 65.16 |
| 1577 | CA | LEU | 721 | 49.634 | 10.162 | 62.659 | 1.00 | 59.83 |
| 1578 | CB | LEU | 721 | 49.505 | 10.724 | 61.235 | 1.00 | 59.18 |
| 1579 | CG | LEU | 721 | 48.264 | 11.548 | 60.867 | 1.00 | 63.18 |
| 1580 | CD1 | LEU | 721 | 48.219 | 11.751 | 59.366 | 1.00 | 62.00 |
| 1581 | CD2 | LEU | 721 | 47.005 | 10.851 | 61.327 | 1.00 | 62.08 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1582 | C | LEU | 721 | 49.163 | 8.696 | 62.703 | 1.00 | 61.87 |
| 1583 | O | LEU | 721 | 48.041 | 8.405 | 63.117 | 1.00 | 62.35 |
| 1584 | N | LEU | 722 | 50.052 | 7.788 | 62.282 | 1.00 | 62.99 |
| 1585 | CA | LEU | 722 | 49.813 | 6.339 | 62.250 | 1.00 | 58.34 |
| 1586 | CB | LEU | 722 | 50.988 | 5.635 | 61.570 | 1.00 | 59.92 |
| 1587 | CG | LEU | 722 | 51.194 | 5.933 | 60.084 | 1.00 | 60.23 |
| 1588 | CD1 | LEU | 722 | 52.500 | 5.333 | 59.636 | 1.00 | 58.33 |
| 1589 | CD2 | LEU | 722 | 50.050 | 5.373 | 59.264 | 1.00 | 59.18 |
| 1590 | C | LEU | 722 | 49.624 | 5.754 | 63.651 | 1.00 | 62.26 |
| 1591 | O | LEU | 722 | 48.827 | 4.835 | 63.860 | 1.00 | 60.80 |
| 1592 | N | ASP | 723 | 50.389 | 6.282 | 64.597 | 1.00 | 60.29 |
| 1593 | CA | ASP | 723 | 50.321 | 5.870 | 65.989 | 1.00 | 61.98 |
| 1594 | CB | ASP | 723 | 51.409 | 6.583 | 66.780 | 1.00 | 62.74 |
| 1595 | CG | ASP | 723 | 52.652 | 5.759 | 66.935 | 1.00 | 62.09 |
| 1596 | OD1 | ASP | 723 | 52.840 | 4.814 | 66.146 | 1.00 | 62.64 |
| 1597 | OD2 | ASP | 723 | 53.445 | 6.066 | 67.848 | 1.00 | 60.13 |
| 1598 | C | ASP | 723 | 48.970 | 6.275 | 66.554 | 1.00 | 61.71 |
| 1599 | O | ASP | 723 | 48.281 | 5.496 | 67.202 | 1.00 | 61.64 |
| 1600 | N | SER | 724 | 48.612 | 7.524 | 66.305 | 1.00 | 62.20 |
| 1601 | CA | SER | 724 | 47.362 | 8.090 | 66.784 | 1.00 | 61.12 |
| 1602 | CB | SER | 724 | 47.329 | 9.579 | 66.449 | 1.00 | 63.08 |
| 1603 | OG | SER | 724 | 47.513 | 9.767 | 65.057 | 1.00 | 61.03 |
| 1604 | C | SER | 724 | 46.098 | 7.419 | 66.246 | 1.00 | 59.27 |
| 1605 | O | SER | 724 | 45.015 | 7.664 | 66.772 | 1.00 | 61.79 |
| 1606 | N | MET | 725 | 46.229 | 6.594 | 65.203 | 1.00 | 60.63 |
| 1607 | CA | MET | 725 | 45.077 | 5.893 | 64.615 | 1.00 | 60.05 |
| 1608 | CB | MET | 725 | 45.425 | 5.228 | 63.272 | 1.00 | 60.16 |
| 1609 | CG | MET | 725 | 45.452 | 6.151 | 62.055 | 1.00 | 66.62 |
| 1610 | SD | MET | 725 | 43.992 | 7.180 | 61.838 | 1.00 | 58.95 |
| 1611 1 | CE | MET | 725 | 42.904 | 6.134 | 61.000 | 1.00 | 61.36 |
| 1612 | C | MET | 725 | 44.573 | 4.833 | 65.576 | 1.00 | 64.25 |
| 1613 | O | MET | 725 | 43.382 | 4.543 | 65.627 | 1.00 | 59.89 |
| 1614 | N | HIS | 726 | 45.492 | 4.251 | 66.334 | 1.00 | 62.22 |
| 1615 | CA | HIS | 726 | 45.122 | 3.249 | 67.313 | 1.00 | 60.86 |
| 1616 | CB | HIS | 726 | 46.356 | 2.746 | 68.064 | 1.00 | 58.90 |
| 1617 | CG | HIS | 726 | 47.183 | 1.772 | 67.286 | 1.00 | 59.73 |
| 1618 | CD2 | HIS | 726 | 48.518 | 1.706 | 67.070 | 1.00 | 60.15 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1619 | ND1 | HIS | 726 | 46.635 | 0.682 | 66.646 | 1.00 | 61.70 |
| 1620 | CE1 | HIS | 726 | 47.598 | -0.014 | 66.069 | 1.00 | 61.05 |
| 1621 | NE2 | HIS | 726 | 48.750 | 0.586 | 66.311 | 1.00 | 60.76 |
| 1622 | C | HIS | 726 | 44.141 | 3.880 | 68.291 | 1.00 | 61.09 |
| 1623 | O | HIS | 726 | 43.146 | 3.268 | 68.650 | 1.00 | 60.43 |
| 1624 | N | GLU | 727 | 44.425 | 5.109 | 68.714 | 1.00 | 61.91 |
| 1625 | CA | GLU | 727 | 43.548 | 5.825 | 69.642 | 1.00 | 58.28 |
| 1626 | CB | GLU | 727 | 44.218 | 7.109 | 70.159 | 1.00 | 63.92 |
| 1627 | CG | GLU | 727 | 43.254 | 8.102 | 70.846 | 1.00 | 59.43 |
| 1628 | CD | GLU | 727 | 43.073 | 9.413 | 70.053 | 1.00 | 61.79 |
| 1629 | OE1 | GLU | 727 | 44.079 | 10.150 | 69.874 | 1.00 | 60.96 |
| 1630 | OE2 | GLU | 727 | 41.931 | 9.707 | 69.607 | 1.00 | 63.98 |
| 1631 | C | GLU | 727 | 42.220 | 6.187 | 68.993 | 1.00 | 60.97 |
| 1632 | O | GLU | 727 | 41.163 | 6.009 | 69.596 | 1.00 | 58.72 |
| 1633 | N | VAL | 728 | 42.263 | 6.699 | 67.769 | 1.00 | 60.50 |
| 1634 | CA | VAL | 728 | 41.022 | 7.073 | 67.105 | 1.00 | 60.74 |
| 1635 | CB | VAL | 728 | 41.289 | 7.922 | 65.828 | 1.00 | 58.90 |
| 1636 | CG1 | VAL | 728 | 42.719 | 7.804 | 65.415 | 1.00 | 58.73 |
| 1637 | CG2 | VAL | 728 | 40.382 | 7.494 | 64.703 | 1.00 | 65.58 |
| 1638 | C | VAL | 728 | 40.192 | 5.842 | 66.773 | 1.00 | 59.91 |
| 1639 | O | VAL | 728 | 38.971 | 5.844 | 66.931 | 1.00 | 61.17 |
| 1640 | N | VAL | 729 | 40.872 | 4.792 | 66.326 | 1.00 | 61.77 |
| 1641 | CA | VAL | 729 | 40.243 | 3.522 | 65.974 | 1.00 | 62.39 |
| 1642 | CB | VAL | 729 | 41.277 | 2.578 | 65.332 | 1.00 | 60.27 |
| 1643 | CG1 | VAL | 729 | 40.946 | 1.131 | 65.606 | 1.00 | 61.53 |
| 1644 | CG2 | VAL | 729 | 41.298 | 2.815 | 63.866 | 1.00 | 60.01 |
| 1645 | C | VAL | 729 | 39.586 | 2.830 | 67.173 | 1.00 | 61.69 |
| 1646 | O | VAL | 729 | 38.718 | 1.971 | 67.009 | 1.00 | 59.65 |
| 1647 | N | GLU | 730 | 39.999 | 3.199 | 68.377 | 1.00 | 59.90 |
| 1648 | CA | GLU | 730 | 39.411 | 2.601 | 69.558 | 1.00 | 60.27 |
| 1649 | CB | GLU | 730 | 40.308 | 2.812 | 70.755 | 1.00 | 59.45 |
| 1650 | CG | GLU | 730 | 39.878 | 2.014 | 71.942 | 1.00 | 66.48 |
| 1651 | CD | GLU | 730 | 40.681 | 2.348 | 73.167 | 1.00 | 62.84 |
| 1652 - | OE1 | GLU | 730 | 41.912 | 2.547 | 73.039 | 1.00 | 61.03 |
| 1653 | OE2 | GLU | 730 | 40.081 | 2.400 | 74.263 | 1.00 | 60.34 |
| 1654 | C | GLU | 730 | 38.085 | 3.291 | 69.804 | 1.00 | 62.24 |
| 1655 | O | GLU | 730 | 37.031 | 2.660 | 69.869 | 1.00 | 60.10 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1656 | N | ASN | 731 | 38.154 | 4.606 | 69.941 | 1.00 | 61.01 |
| 1657 | CA | ASN | 731 | 36.967 | 5.409 | 70.157 | 1.00 | 60.41 |
| 1658 | CB | ASN | 731 | 37.356 | 6.880 | 70.212 | 1.00 | 62.98 |
| 1659 | CG | ASN | 731 | 37.593 | 7.333 | 71.613 | 1.00 | 59.54 |
| 1660 | OD1 | ASN | 731 | 36.657 | 7.385 | 72.410 | 1.00 | 58.59 |
| 1661 | ND2 | ASN | 731 | 38.841 | 7.638 | 71.945 | 1.00 | 58.18 |
| 1662 | C | ASN | 731 | 35.917 | 5.165 | 69.081 | 1.00 | 61.30 |
| 1663 | O | ASN | 731 | 34.720 | 5.345 | 69.317 | 1.00 | 60.95 |
| 1664 | N | LEU | 732 | 36.364 | 4.750 | 67.902 | 1.00 | 60.39 |
| 1665 | CA | LEU | 732 | 35.442 | 4.475 | 66.820 | 1.00 | 61.55 |
| 1666 | CB | LEU | 732 | 36.141 | 4.550 | 65.471 | 1.00 | 59.24 |
| 1667 | CG | LEU | 732 | 36.184 | 5.931 | 64.813 | 1.00 | 64.00 |
| 1668 | CD1 | LEU | 732 | 36.712 | 5.787 | 63.374 | 1.00 | 62.90 |
| 1669 | CD2 | LEU | 732 | 34.771 | 6.561 | 64.818 | 1.00 | 60.26 |
| 1670 | C | LEU | 732 | 34.841 | 3.108 | 66.997 | 1.00 | 60.27 |
| 1671 | O | LEU | 732 | 33.648 | 2.922 | 66.774 | 1.00 | 61.79 |
| 1672 | N | LEU | 733 | 35.673 | 2.148 | 67.399 | 1.00 | 58.72 |
| 1673 | CA | LEU | 733 | 35.214 | 0.770 | 67.612 | 1.00 | 60.63 |
| 1674 | CB | LEU | 733 | 36.376 | -0.153 | 67.969 | 1.00 | 60.85 |
| 1675 | CG | LEU | 733 | 37.087 | -0.798 | 66.782 | 1.00 | 56.83 |
| 1676 | CD1 | LEU | 733 | 38.344 | -1.474 | 67.266 | 1.00 | 63.66 |
| 1677 | CD2 | LEU | 733 | 36.159 | -1.786 | 66.083 | 1.00 | 56.09 |
| 1678 | C | LEU | 733 | 34.158 | 0.663 | 68.696 | 1.00 | 60.11 |
| 1679 | O | LEU | 733 | 33.092 | 0.098 | 68.458 | 1.00 | 60.20 |
| 1680 | N | ASN | 734 | 34.456 | 1.192 | 69.883 | 1.00 | 64.88 |
| 1681 | CA | ASN | 734 | 33.503 | 1.148 | 70.988 | 1.00 | 60.87 |
| 1682 | CB | ASN | 734 | 33.874 | 2.130 | 72.099 | 1.00 | 64.42 |
| 1683 | CG | ASN | 734 | 35.076 | 1.683 | 72.896 | 1.00 | 60.85 |
| 1684 | OD1 | ASN | 734 | 35.499 | 0.526 | 72.817 | 1.00 | 60.20 |
| 1685 | ND2 | ASN | 734 | 35.627 | 2.597 | 73.686 | 1.00 | 62.04 |
| 1686 | C | ASN | 734 | 32.157 | 1.544 | 70.455 | 1.00 | 59.98 |
| 1687 | O | ASN | 734 | 31.209 | 0.755 | 70.478 | 1.00 | 59.10 |
| 1688 | N | TYR | 735 | 32.085 | 2.778 | 69.969 | 1.00 | 61.85 |
| 1689 | CA | TYR | 735 | 30.844 | 3.294 | 69.421 | 1.00 | 63.27 |
| 1690 | CB | TYR | 735 | 31.075 | 4.640 | 68.743 | 1.00 | 60.68 |
| 1691 | CG | TYR | 735 | 29.867 | 5.515 | 68.853 | 1.00 | 62.56 |
| 1692 | CD1 | TYR | 735 | 28.777 | 5.328 | 67.975 | 1.00 | 60.84 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1693 | CE1 | TYR | 735 | 27.586 | 6.030 | 68.168 | 1.00 | 60.10 |
| 1694 | CD2 | TYR | 735 | 29.722 | 6.421 | 69.889 | 1.00 | 62.93 |
| 1695 | CE2 | TYR | 735 | 28.543 | 7.127 | 70.084 | 1.00 | 62.59 |
| 1696 | CZ | TYR | 735 | 27.483 | 6.916 | 69.221 | 1.00 | 60.99 |
| 1697 | OH | TYR | 735 | 26.305 | 7.561 | 69.418 | 1.00 | 56.15 |
| 1698 | C | TYR | 735 | 30.261 | 2.306 | 68.418 | 1.00 | 57.10 |
| 1699 | O | TYR | 735 | 29.052 | 2.158 | 68.321 | 1.00 | 60.19 |
| 1700 | N | CYS | 736 | 31.126 | 1.621 | 67.682 | 1.00 | 62.53 |
| 1701 | CA | CYS | 736 | 30.673 | 0.644 | 66.707 | 1.00 | 60.21 |
| 1702 | CB | CYS | 736 | 31.842 | 0.208 | 65.815 | 1.00 | 62.29 |
| 1703 | SG | CYS | 736 | 31.461 | -1.142 | 64.660 | 1.00 | 62.77 |
| 1704 | C | CYS | 736 | 30.063 | -0.572 | 67.399 | 1.00 | 62.99 |
| 1705 | O | CYS | 736 | 28.857 | -0.795 | 67.290 | 1.00 | 61.76 |
| 1706 | N | PHE | 737 | 30.892 | -1.346 | 68.111 | 1.00 | 60.76 |
| 1707 | CA | PHE | 737 | 30.435 | -2.546 | 68.835 | 1.00 | 61.61 |
| 1708 | CB | PHE | 737 | 31.454 | -3.013 | 69.889 | 1.00 | 65.67 |
| 1709 | CG | PHE | 737 | 32.718 | -3.575 | 69.321 | 1.00 | 61.17 |
| 1710 | CD1 | PHE | 737 | 32.732 | -4.173 | 68.069 | 1.00 | 62.43 |
| 1711 | CD2 | PHE | 737 | 33.904 | -3.515 | 70.049 | 1.00 | 62.09 |
| 1712 | CE1 | PHE | 737 | 33.908 | -4.704 | 67.544 | 1.00 | 60.80 |
| 1713 | CE2 | PHE | 737 | 35.083 | -4.042 | 69.536 | 1.00 | 61.51 |
| 1714 | CZ | PHE | 737 | 35.086 | -4.638 | 68.279 | 1.00 | 59.32 |
| 1715 | C | PHE | 737 | 29.151 | -2.266 | 69.581 | 1.00 | 60.98 |
| 1716 | O | PHE | 737 | 28.103 | -2.865 | 69.312 | 1.00 | 61.98 |
| 1717 | N | GLN | 738 | 29.274 | -1.356 | 70.542 | 1.00 | 64.21 |
| 1718 | CA | GLN | 738 | 28.183 | -0.934 | 71.399 | 1.00 | 63.22 |
| 1719 | CB | GLN | 738 | 28.613 | 0.301 | 72.173 | 1.00 | 64.29 |
| 1720 | CG | GLN | 738 | 27.542 | 0.896 | 73.021 | 1.00 | 59.02 |
| 1721 | CD | GLN | 738 | 27.555 | 2.392 | 72.920 | 1.00 | 61.20 |
| 1722 | OE1 | GLN | 738 | 27.116 | 2.962 | 71.916 | 1.00 | 60.80 |
| 1723 | NE2 | GLN | 738 | 28.082 | 3.048 | 73.948 | 1.00 | 57.12 |
| 1724 | C | GLN | 738 | 26.917 | -0.640 | 70.606 | 1.00 | 61.18 |
| 1725 | O | GLN | 738 | 25.827 | -1.017 | 71.009 | 1.00 | 61.34 |
| 1726 | N | THR | 739 | 27.056 | 0.037 | 69.478 | 1.00 | 61.45 |
| 1727 | CA | THR | 739 | 25.893 | 0.326 | 68.678 | 1.00 | 60.85 |
| 1728 | CB | THR | 739 | 26.208 | 1.297 | 67.542 | 1.00 | 55.90 |
| 1729 | OG1 | THR | 739 | 26.212 | 2.649 | 68.047 | 1.00 | 60.51 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1730 | CG2 | THR | 739 | 25.193 | 1.200 | 66.444 | 1.00 | 62.24 |
| 1731 | C | THR | 739 | 25.330 | -0.969 | 68.085 | 1.00 | 62.53 |
| 1732 | O | THR | 739 | 24.122 | -1.107 | 67.898 | 1.00 | 60.85 |
| 1733 | N | PHE | 740 | 26.221 | -1.906 | 67.784 | 1.00 | 61.25 |
| 1734 | CA | PHE | 740 | 25.859 | -3.197 | 67.215 | 1.00 | 59.86 |
| 1735 | CB | PHE | 740 | 27.110 | -3.854 | 66.641 | 1.00 | 60.28 |
| 1736 | CG | PHE | 740 | 26.937 | -5.301 | 66.311 | 1.00 | 63.82 |
| 1737 | CD1 | PHE | 740 | 26.434 | -5.690 | 65.079 | 1.00 | 62.86 |
| 1738 | CD2 | PHE | 740 | 27.297 | -6.278 | 67.236 | 1.00 | 61.71 |
| 1739 | CE1 | PHE | 740 | 26.295 | -7.042 | 64.763 | 1.00 | 60.86 |
| 1740 | CE2 | PHE | 740 | 27.165 | -7.623 | 66.938 | 1.00 | 60.83 |
| 1741 | CZ | PHE | 740 | 26.663 | -8.013 | 65.696 | 1.00 | 60.77 |
| 1742 | C | PHE | 740 | 25.200 | -4.140 | 68.238 | 1.00 | 60.76 |
| 1743 | O | PHE | 740 | 24.420 | -5.028 | 67.866 | 1.00 | 60.72 |
| 1744 | N | LEU | 741 | 25.538 | -3.965 | 69.515 | 1.00 | 62.82 |
| 1745 | CA | LEU | 741 | 24.976 | -4.793 | 70.584 | 1.00 | 61.57 |
| 1746 | CB | LEU | 741 | 25.991 | -5.012 | 71.708 | 1.00 | 59.14 |
| 1747 | CG | LEU | 741 | 27.187 | -5.904 | 71.404 | 1.00 | 62.48 |
| 1748 | CD1 | LEU | 741 | 28.083 | -5.956 | 72.627 | 1.00 | 58.13 |
| 1749 | CD2 | LEU | 741 | 26.708 | -7.294 | 71.021 | 1.00 | 60.31 |
| 1750 | C | LEU | 741 | 23.770 | -4.087 | 71.165 | 1.00 | 60.19 |
| 1751 | O | LEU | 741 | 23.577 | -4.055 | 72.389 | 1.00 | 59.64 |
| 1752 | N | ASP | 742 | 22.960 | -3.508 | 70.290 | 1.00 | 61.92 |
| 1753 | CA | ASP | 742 | 21.789 | -2.797 | 70.762 | 1.00 | 60.22 |
| 1754 | CB | ASP | 742 | 22.185 | -1.372 | 71.179 | 1.00 | 61.70 |
| 1755 | CG | ASP | 742 | 21.021 | -0.598 | 71.793 | 1.00 | 60.82 |
| 1756 | OD1 | ASP | 742 | 21.258 | 0.438 | 72.473 | 1.00 | 60.77 |
| 1757 | OD2 | ASP | 742 | 19.863 | -1.028 | 71.587 | 1.00 | 61.09 |
| 1758 | C | ASP | 742 | 20.689 | -2.769 | 69.710 | 1.00 | 61.90 |
| 1759 | O | ASP | 742 | 20.530 | -1.782 | 68.995 | 1.00 | 61.23 |
| 1760 | N | LYS | 743 | 19.934 | -3.864 | 69.623 | 1.00 | 63.29 |
| 1761 | CA | LYS | 743 | 18.833 | -3.975 | 68.664 | 1.00 | 62.18 |
| 1762 | CB | LYS | 743 | 18.045 | -5.273 | 68.888 | 1.00 | 63.55 |
| 1763 | CG | LYS | 743 | 18.054 | -6.243 | 67.705 | 1.00 | 62.77 |
| 1764 | CD | LYS | 743 | 17.301 | -5.691 | 66.489 | 1.00 | 59.38 |
| 1765 | CE | LYS | 743 | 17.291 | -6.724 | 65.349 | 1.00 | 62.80 |
| 1766 | NZ | LYS | 743 | 16.446 | -6.335 | 64.166 | 1.00 | 57.45 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1767 | C | LYS | 743 | 17.899 | -2.786 | 68.822 | 1.00 | 62.93 |
| 1768 | O | LYS | 743 | 17.407 | -2.248 | 67.833 | 1.00 | 58.22 |
| 1769 | N | THR | 744 | 17.669 | -2.383 | 70.069 | 1.00 | 61.23 |
| 1770 | CA | THR | 744 | 16.808 | -1.247 | 70.400 | 1.00 | 61.48 |
| 1771 | CB | THR | 744 | 17.148 | -0.715 | 71.802 | 1.00 | 63.18 |
| 1772 | OG1 | THR | 744 | 17.199 | -1.817 | 72.719 | 1.00 | 59.84 |
| 1773 | CG2 | THR | 744 | 16.112 | 0.308 | 72.265 | 1.00 | 60.98 |
| 1774 | C | THR | 744 | 16.913 | -0.077 | 69.411 | 1.00 | 59.67 |
| 1775 | O | THR | 744 | 15.913 | 0.580 | 69.118 | 1.00 | 64.20 |
| 1776 | N | MET | 745 | 18.117 | 0.184 | 68.903 | 1.00 | 61.36 |
| 1777 | CA | MET | 745 | 18.322 | 1.282 | 67.961 | 1.00 | 62.05 |
| 1778 | CB | MET | 745 | 19.703 | 1.909 | 68.158 | 1.00 | 63.01 |
| 1779 | CG | MET | 745 | 20.029 | 2.189 | 69.614 | 1.00 | 61.21 |
| 1780 | SD | MET | 745 | 21.418 | 3.324 | 69.877 | 1.00 | 62.14 |
| 1781 | CE | MET | 745 | 20.934 | 4.087 | 71.538 | 1.00 | 61.78 |
| 1782 | C | MET | 745 | 18.175 | 0.824 | 66.517 | 1.00 | 59.56 |
| 1783 | O | MET | 745 | 18.382 | 1.605 | 65.585 | 1.00 | 62.58 |
| 1784 | N | SER | 746 | 17.817 | -0.442 | 66.334 | 1.00 | 59.78 |
| 1785 | CA | SER | 746 | 17.624 | -1.000 | 64.996 | 1.00 | 58.09 |
| 1786 | CB | SER | 746 | 16.169 | -0.786 | 64.541 | 1.00 | 58.34 |
| 1787 | OG | SER | 746 | 15.252 | -1.368 | 65.455 | 1.00 | 63.83 |
| 1788 | C | SER | 746 | 18.592 | -0.429 | 63.940 | 1.00 | 59.09 |
| 1789 | O | SER | 746 | 18.176 | 0.027 | 62.867 | 1.00 | 61.57 |
| 1790 | N | ILE | 747 | 19.882 | -0.432 | 64.269 | 1.00 | 62.10 |
| 1791 | CA | ILE | 747 | 20.905 | 0.022 | 63.342 | 1.00 | 61.22 |
| 1792 | CB | ILE | 747 | 22.021 | 0.814 | 64.054 | 1.00 | 63.05 |
| 1793 | CG2 | ILE | 747 | 23.241 | 0.930 | 63.145 | 1.00 | 64.60 |
| 1794 | CG1 | ILE | 747 | 21.499 | 2.205 | 64.426 | 1.00 | 61.38 |
| 1795 | CD1 | ILE | 747 | 22.576 | 3.198 | 64.804 | 1.00 | 63.35 |
| 1796 | C | ILE | 747 | 21.465 | -1.260 | 62.749 | 1.00 | 62.54 |
| 1797 | O | ILE | 747 | 22.169 | -2.011 | 63.419 | 1.00 | 60.09 |
| 1798 | N | GLU | 748 | 21.125 | -1.507 | 61.491 | 1.00 | 60.25 |
| 1799 | CA | GLU | 748 | 21.541 | -2.711 | 60.787 | 1.00 | 60.97 |
| 1800 | CB | GLU | 748 | 20.497 | -3.038 | 59.709 | 1.00 | 63.45 |
| 1801 | CG | GLU | 748 | 20.877 | -4.168 | 58.756 | 1.00 | 60.81 |
| 1802 | CD | GLU | 748 | 19.678 | -4.723 | 57.973 | 1.00 | 59.13 |
| 1803 | OE1 | GLU | 748 | 19.013 | -3.932 | 57.262 | 1.00 | 62.39 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1804 | OE2 | GLU | 748 | 19.406 | -5.950 | 58.072 | 1.00 | 61.13 |
| 1805 | C | GLU | 748 | 22.940 | -2.658 | 60.175 | 1.00 | 61.28 |
| 1806 | O | GLU | 748 | 23.391 | -1.616 | 59.690 | 1.00 | 62.62 |
| 1807 | N | PHE | 749 | 23.622 | -3.800 | 60.231 | 1.00 | 62.73 |
| 1808 | CA | PHE | 749 | 24.963 | -3.959 | 59.667 | 1.00 | 59.35 |
| 1809 | CB | PHE | 749 | 25.974 | -4.421 | 60.733 | 1.00 | 59.45 |
| 1810 | CG | PHE | 749 | 26.299 | -3.389 | 61.759 | 1.00 | 64.74 |
| 1811 | CD1 | PHE | 749 | 25.399 | -3.085 | 62.770 | 1.00 | 59.18 |
| 1812 | CD2 | PHE | 749 | 27.514 | -2.721 | 61.721 | 1.00 | 57.96 |
| 1813 | CE1 | PHE | 749 | 25.708 | -2.119 | 63.742 | 1.00 | 61.52 |
| 1814 | CE2 | PHE | 749 | 27.839 | -1.753 | 62.685 | 1.00 | 65.31 |
| 1815 | CZ | PHE | 749 | 26.934 | -1.451 | 63.697 | 1.00 | 59.87 |
| 1816 | C | PHE | 749 | 24.872 | -5.048 | 58.599 | 1.00 | 60.75 |
| 1817 | O | PHE | 749 | 24.047 | -5.952 | 58.705 | 1.00 | 62.03 |
| 1818 | N | PRO | 750 | 25.705 | -4.970 | 57.550 | 1.00 | 61.23 |
| 1819 | CD | PRO | 750 | 26.645 | -3.910 | 57.166 | 1.00 | 60.04 |
| 1820 | CA | PRO | 750 | 25.652 | -6.003 | 56.516 | 1.00 | 61.34 |
| 1821 | CB | PRO | 750 | 26.584 | -5.468 | 55.434 | 1.00 | 64.04 |
| 1822 | CG | PRO | 750 | 26.608 | -4.015 | 55.668 | 1.00 | 60.17 |
| 1823 | C | PRO | 750 | 26.240 | -7.235 | 57.172 | 1.00 | 60.83 |
| 1824 | O | PRO | 750 | 26.788 | -7.143 | 58.271 | 1.00 | 59.53 |
| 1825 | N | GLU | 751 | 26.139 | -8.386 | 56.523 | 1.00 | 60.43 |
| 1826 | CA | GLU | 751 | 26.719 | -9.572 | 57.122 | 1.00 | 61.33 |
| 1827 | CB | GLU | 751 | 26.350 | -10.834 | 56.311 | 1.00 | 62.90 |
| 1828 | CG | GLU | 751 | 25.560 | -10.608 | 55.002 | 1.00 | 60.51 |
| 1829 | CD | GLU | 751 | 26.436 | -10.142 | 53.837 | 1.00 | 57.86 |
| 1830 | OE1 | GLU | 751 | 27.420 | -10.844 | 53.519 | 1.00 | 60.97 |
| 1831 | OE2 | GLU | 751 | 26.138 | -9.084 | 53.240 | 1.00 | 59.16 |
| 1832 | C | GLU | 751 | 28.255 | -9.389 | 57.219 | 1.00 | 58.48 |
| 1833 | O | GLU | 751 | 28.850 | -9.561 | 58.292 | 1.00 | 59.47 |
| 1834 | N | MET | 752 | 28.891 | -9.004 | 56.115 | 1.00 | 64.26 |
| 1835 | CA | MET | 752 | 30.336 | -8.808 | 56.109 | 1.00 | 61.15 |
| 1836 | CB | MET | 752 | 30.777 | -8.060 | 54.857 | 1.00 | 63.84 |
| 1837 | CG | MET | 752 | 32.255 | -7.666 | 54.898 | 1.00 | 64.44 |
| 1838 | SD | MET | 752 | 33.383 | -9.076 | 55.043 | 1.00 | 67.15 |
| 1839 | CE | MET | 752 | 33.649 | -9.386 | 53.307 | 1.00 | 59.96 |
| 1840 | C | MET | 752 | 30.884 | -8.072 | 57.323 | 1.00 | 62.38 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1841 | O | MET | 752 | 31.963 | -8.394 | 57.811 | 1.00 | 60.81 |
| 1842 | N | LEU | 753 | 30.163 | -7.067 | 57.796 | 1.00 | 59.19 |
| 1843 | CA | LEU | 753 | 30.627 | -6.323 | 58.957 | 1.00 | 62.39 |
| 1844 | CB | LEU | 753 | 30.187 | -4.862 | 58.881 | 1.00 | 61.65 |
| 1845 | CG | LEU | 753 | 31.214 | -3.835 | 58.397 | 1.00 | 61.49 |
| 1846 | CD1 | LEU | 753 | 31.713 | -4.171 | 57.010 | 1.00 | 61.11 |
| 1847 | CD2 | LEU | 753 | 30.567 | -2.471 | 58.411 | 1.00 | 63.29 |
| 1848 | C | LEU | 753 | 30.114 | -6.946 | 60.241 | 1.00 | 58.84 |
| 1849 | O | LEU | 753 | 30.779 | -6.885 | 61.273 | 1.00 | 61.39 |
| 1850 | N | ALA | 754 | 28.927 | -7.541 | 60.177 | 1.00 | 62.56 |
| 1851 | CA | ALA | 754 | 28.337 | -8.194 | 61.343 | 1.00 | 61.99 |
| 1852 | CB | ALA | 754 | 26.963 | -8.775 | 60.983 | 1.00 | 59.37 |
| 1853 | C | ALA | 754 | 29.287 | -9.313 | 61.747 | 1.00 | 66.01 |
| 1854 | O | ALA | 754 | 29.619 | -9.482 | 62.922 | 1.00 | 63.54 |
| 1855 | N | GLU | 755 | 29.724 | -10.044 | 60.722 | 1.00 | 63.92 |
| 1856 | CA | GLU | 755 | 30.632 | -11.194 | 60.795 | 1.00 | 59.42 |
| 1857 | CB | GLU | 755 | 30.674 | -11.851 | 59.407 | 1.00 | 60.24 |
| 1858 | CG | GLU | 755 | 31.733 | -12.933 | 59.190 | 1.00 | 62.29 |
| 1859 | CD | GLU | 755 | 31.348 | -14.260 | 59.811 | 1.00 | 59.60 |
| 1860 | OE1 | GLU | 755 | 30.318 | -14.834 | 59.388 | 1.00 | 60.67 |
| 1861 | OE2 | GLU | 755 | 32.075 | -14.728 | 60.719 | 1.00 | 62.46 |
| 1862 | C | GLU | 755 | 32.068 | -10.912 | 61.252 | 1.00 | 60.75 |
| 1863 | O | GLU | 755 | 32.906 | -11.809 | 61.240 | 1.00 | 61.42 |
| 1864 | N | ILE | 756 | 32.363 | -9.677 | 61.636 | 1.00 | 60.43 |
| 1865 | CA | ILE | 756 | 33.709 | -9.328 | 62.074 | 1.00 | 65.41 |
| 1866 | CB | ILE | 756 | 34.369 | -8.336 | 61.114 | 1.00 | 58.24 |
| 1867 | CG2 | ILE | 756 | 35.741 | -7.964 | 61.623 | 1.00 | 57.70 |
| 1868 | CG1 | ILE | 756 | 34.478 | -8.957 | 59.729 | 1.00 | 59.57 |
| 1869 | CD1 | ILE | 756 | 35.178 | -8.090 | 58.743 | 1.00 | 61.24 |
| 1870 | C | ILE | 756 | 33.625 | -8.693 | 63.439 | 1.00 | 63.18 |
| 1871 | O | ILE | 756 | 34.373 | -9.043 | 64.351 | 1.00 | 58.77 |
| 1872 | N | ILE | 757 | 32.705 | -7.740 | 63.548 | 1.00 | 59.89 |
| 1873 | CA | ILE | 757 | 32.434 | -7.024 | 64.785 | 1.00 | 61.90 |
| 1874 | CB | ILE | 757 | 31.115 | -6.254 | 64.668 | 1.00 | 64.77 |
| 1875 | CG2 | ILE | 757 | 30.778 | -5.602 | 65.991 | 1.00 | 62.61 |
| 1876 | CG1 | ILE | 757 | 31.224 | -5.237 | 63.529 | 1.00 | 62.60 |
| 1877 | CD1 | ILE | 757 | 29.902 | -4.649 | 63.097 | 1.00 | 59.02 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1878 | C | ILE | 757 | 32.298 | -8.069 | 65.879 | 1.00 | 60.76 |
| 1879 | O | ILE | 757 | 32.990 | -8.016 | 66.890 | 1.00 | 60.98 |
| 1880 | N | THR | 758 | 31.396 | -9.022 | 65.660 | 1.00 | 59.72 |
| 1881 | CA | THR | 758 | 31.184 | -10.104 | 66.608 | 1.00 | 61.21 |
| 1882 | CB | THR | 758 | 30.224 | -11.141 | 66.017 | 1.00 | 58.63 |
| 1883 | OG1 | THR | 758 | 30.260 | -11.028 | 64.592 | 1.00 | 60.88 |
| 1884 | CG2 | THR | 758 | 28.792 | -10.925 | 66.527 | 1.00 | 60.75 |
| 1885 | C | THR | 758 | 32.540 | -10.770 | 66.885 | 1.00 | 61.53 |
| 1886 | O | THR | 758 | 33.167 | -10.549 | 67.926 | 1.00 | 61.44 |
| 1887 | N | ASN | 759 | 32.979 | -11.572 | 65.924 | 1.00 | 62.04 |
| 1888 | CA | ASN | 759 | 34.240 | -12.305 | 65.965 | 1.00 | 60.58 |
| 1889 | CB | ASN | 759 | 34.426 | -13.001 | 64.623 | 1.00 | 60.54 |
| 1890 | CG | ASN | 759 | 33.242 | -12.774 | 63.689 | 1.00 | 60.06 |
| 1891 | OD1 | ASN | 759 | 32.581 | -11.723 | 63.736 | 1.00 | 59.27 |
| 1892 | ND2 | ASN | 759 | 32.976 | -13.747 | 62.825 | 1.00 | 59.97 |
| 1893 | C | ASN | 759 | 35.470 | -11.432 | 66.249 | 1.00 | 59.08 |
| 1894 | O | ASN | 759 | 36.564 | -11.710 | 65.734 | 1.00 | 63.02 |
| 1895 | N | GLN | 760 | 35.282 | -10.388 | 67.059 | 1.00 | 60.43 |
| 1896 | CA | GLN | 760 | 36.336 | -9.442 | 67.438 | 1.00 | 61.43 |
| 1897 | CB | GLN | 760 | 36.620 | -8.446 | 66.302 | 1.00 | 56.98 |
| 1898 | CG | GLN | 760 | 37.445 | -8.966 | 65.121 | 1.00 | 61.31 |
| 1899 | CD | GLN | 760 | 38.839 | -9.446 | 65.514 | 1.00 | 63.88 |
| 1900 | OE1 | GLN | 760 | 39.445 | -8.949 | 66.463 | 1.00 | 59.43 |
| 1901 | NE2 | GLN | 760 | 39.356 | -10.409 | 64.769 | 1.00 | 62.96 |
| 1902 | C | GLN | 760 | 35.850 | -8.659 | 68.651 | 1.00 | 59.31 |
| 1903 | O | GLN | 760 | 36.625 | -8.353 | 69.563 | 1.00 | 59.88 |
| 1904 | N | ILE | 761 | 34.546 | -8.371 | 68.649 | 1.00 | 61.08 |
| 1905 | CA | ILE | 761 | 33.861 | -7.606 | 69.704 | 1.00 | 64.40 |
| 1906 | CB | ILE | 761 | 32.318 | -7.582 | 69.469 | 1.00 | 60.77 |
| 1907 | CG2 | ILE | 761 | 31.759 | -8.983 | 69.575 | 1.00 | 61.46 |
| 1908 | CG1 | ILE | 761 | 31.626 | -6.686 | 70.500 | 1.00 | 61.86 |
| 1909 | CD1 | ILE | 761 | 30.115 | -6.703 | 70.390 | 1.00 | 62.34 |
| 1910 | C | ILE | 761 | 34.111 | -8.077 | 71.139 | 1.00 | 63.06 |
| 1911 | O | ILE | 761 | 33.900 | -7.317 | 72.087 | 1.00 | 60.11 |
| 1912 | N | PRO | 762 | 34.543 | -9.338 | 71.319 | 1.00 | 58.19 |
| 1913 | CD | PRO | 762 | 34.581 | -10.454 | 70.353 | 1.00 | 61.52 |
| 1914 | CA | PRO | 762 | 34.800 | -9.840 | 72.670 | 1.00 | 60.05 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1915 | CB | PRO | 762 | 34.718 | -11.350 | 72.482 | 1.00 | 60.47 |
| 1916 | CG | PRO | 762 | 35.309 | -11.528 | 71.122 | 1.00 | 62.98 |
| 1917 | C | PRO | 762 | 36.160 | -9.419 | 73.230 | 1.00 | 59.07 |
| 1918 | O | PRO | 762 | 36.257 | -8.697 | 74.232 | 1.00 | 61.15 |
| 1919 | N | LYS | 763 | 37.203 | -9.887 | 72.558 | 1.00 | 59.41 |
| 1920 | CA | LYS | 763 | 38.580 | -9.637 | 72.960 | 1.00 | 61.27 |
| 1921 | CB | LYS | 763 | 39.424 | -10.882 | 72.594 | 1.00 | 61.66 |
| 1922 | CG | LYS | 763 | 40.926 | -10.651 | 72.347 | 1.00 | 62.08 |
| 1923 | CD | LYS | 763 | 41.206 | -9.910 | 71.025 | 1.00 | 58.99 |
| 1924 | CE | LYS | 763 | 40.834 | -10.723 | 69.776 | 1.00 | 60.53 |
| 1925 | NZ | LYS | 763 | 39.378 | -11.026 | 69.623 | 1.00 | 62.94 |
| 1926 | C | LYS | 763 | 39.256 | -8.359 | 72.438 | 1.00 | 63.01 |
| 1927 | O | LYS | 763 | 40.164 | -7.846 | 73.086 | 1.00 | 63.43 |
| 1928 | N | TYR | 764 | 38.820 | -7.847 | 71.289 | 1.00 | 64.75 |
| 1929 | CA | TYR | 764 | 39.428 | -6.653 | 70.673 | 1.00 | 58.42 |
| 1930 | CB | TYR | 764 | 38.399 | -5.865 | 69.876 | 1.00 | 62.63 |
| 1931 | CG | TYR | 764 | 38.901 | -5.590 | 68.486 | 1.00 | 65.16 |
| 1932 | CD1 | TYR | 764 | 38.708 | -6.516 | 67.467 | 1.00 | 57.96 |
| 1933 | CE1 | TYR | 764 | 39.190 | -6.287 | 66.180 | 1.00 | 61.30 |
| 1934 | CD2 | TYR | 764 | 39.600 | -4.422 | 68.192 | 1.00 | 61.22 |
| 1935 | CE2 | TYR | 764 | 40.092 | -4.183 | 66.902 | 1.00 | 61.36 |
| 1936 | CZ | TYR | 764 | 39.876 | -5.118 | 65.901 | 1.00 | 59.50 |
| 1937 | OH | TYR | 764 | 40.304 | -4.863 | 64.612 | 1.00 | 62.78 |
| 1938 | C | TYR | 764 | 40.204 | -5.654 | 71.535 | 1.00 | 61.96 |
| 1939 | O | TYR | 764 | 41.310 | -5.963 | 72.003 | 1.00 | 61.04 |
| 1940 | N | SER | 765 | 39.653 | -4.443 | 71.701 | 1.00 | 60.29 |
| 1941 | CA | SER | 765 | 40.324 | -3.419 | 72.519 | 1.00 | 61.03 |
| 1942 | CB | SER | 765 | 39.780 | -1.999 | 72.208 | 1.00 | 57.40 |
| 1943 | OG | SER | 765 | 38.359 | -1.934 | 72.144 | 1.00 | 59.00 |
| 1944 | C | SER | 765 | 40.207 | -3.760 | 74.016 | 1.00 | 59.80 |
| 1945 | O | SER | 765 | 39.574 | -3.046 | 74.809 | 1.00 | 60.59 |
| 1946 | N | ASN | 766 | 40.833 | -4.884 | 74.371 | 1.00 | 59.36 |
| 1947 | CA | ASN | 766 | 40.867 | -5.404 | 75.738 | 1.00 | 63.02 |
| 1948 | CB | ASN | 766 | 40.390 | -6.867 | 75.745 | 1.00 | 59.32 |
| 1949 | CG | ASN | 766 | 38.959 | -7.018 | 75.239 | 1.00 | 61.45 |
| 1950 | OD1 | ASN | 766 | 38.593 | -6.456 | 74.197 | 1.00 | 60.60 |
| 1951 | ND2 | ASN | 766 | 38.143 | -7.782 | 75.971 | 1.00 | 64.82 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1952 | C | ASN | 766 | 42.301 | -5.305 | 76.311 | 1.00 | 62.76 |
| 1953 | O | ASN | 766 | 42.503 | -4.889 | 77.469 | 1.00 | 60.59 |
| 1954 | N | GLY | 767 | 43.281 | -5.673 | 75.478 | 1.00 | 59.11 |
| 1955 | CA | GLY | 767 | 44.689 | -5.653 | 75.860 | 1.00 | 59.91 |
| 1956 | C | GLY | 767 | 45.366 | -6.867 | 75.232 | 1.00 | 61.75 |
| 1957 | O | GLY | 767 | 46.588 | -6.912 | 75.041 | 1.00 | 61.17 |
| 1958 | N | ASN | 768 | 44.532 | -7.846 | 74.887 | 1.00 | 62.80 |
| 1959 | CA | ASN | 768 | 44.942 | -9.109 | 74.276 | 1.00 | 62.46 |
| 1960 | CB | ASN | 768 | 43.717 | -10.032 | 74.246 | 1.00 | 59.72 |
| 1961 | CG | ASN | 768 | 42.798 | -9.832 | 75.467 | 1.00 | 60.42 |
| 1962 | OD1 | ASN | 768 | 41.697 | -10.404 | 75.538 | 1.00 | 59.89 |
| 1963 | ND2 | ASN | 768 | 43.248 | -9.020 | 76.427 | 1.00 | 63.45 |
| 1964 | C | ASN | 768 | 45.543 | -8.940 | 72.855 | 1.00 | 63.10 |
| 1965 | O | ASN | 768 | 46.095 | -9.882 | 72.282 | 1.00 | 62.80 |
| 1966 | N | ILE | 769 | 45.418 | -7.744 | 72.284 | 1.00 | 58.10 |
| 1967 | CA | ILE | 769 | 45.984 | -7.464 | 70.967 | 1.00 | 60.18 |
| 1968 | CB | ILE | 769 | 45.006 | -6.713 | 70.036 | 1.00 | 61.64 |
| 1969 | CG2 | ILE | 769 | 45.569 | -6.703 | 68.614 | 1.00 | 59.97 |
| 1970 | CG1 | ILE | 769 | 43.623 | -7.361 | 70.051 | 1.00 | 65.54 |
| 1971 | CD1 | ILE | 769 | 42.605 | -6.626 | 69.175 | 1.00 | 65.14 |
| 1972 | C | ILE | 769 | 47.192 | -6.544 | 71.150 | 1.00 | 59.44 |
| 1973 | O | ILE | 769 | 47.217 | -5.701 | 72.058 | 1.00 | 61.97 |
| 1974 | N | LYS | 770 | 48.175 | -6.692 | 70.267 | 1.00 | 61.57 |
| 1975 | CA | LYS | 770 | 49.391 | -5.890 | 70.314 | 1.00 | 60.09 |
| 1976 | CB | LYS | 770 | 50.601 | -6.778 | 70.033 | 1.00 | 61.51 |
| 1977 | CG | LYS | 770 | 51.961 | -6.172 | 70.339 | 1.00 | 61.08 |
| 1978 | CD | LYS | 770 | 53.041 | -7.224 | 70.047 | 1.00 | 59.40 |
| 1979 | CE | LYS | 770 | 54.344 | -6.982 | 70.801 | 1.00 | 62.15 |
| 1980 | NZ | LYS | 770 | 55.339 | -8.089 | 70.604 | 1.00 | 63.19 |
| 1981 | C | LYS | 770 | 49.333 | -4.776 | 69.277 | 1.00 | 63.90 |
| 1982 | O | LYS | 770 | 49.439 | -5.031 | 68.071 | 1.00 | 62.08 |
| 1983 | N | LYS | 771 | 49.161 | -3.545 | 69.754 | 1.00 | 59.06 |
| 1984 | CA | LYS | 771 | 49.103 | -2.376 | 68.884 | 1.00 | 61.08 |
| 1985 | CB | LYS | 771 | 48.386 | -1.212 | 69.589 | 1.00 | 63.15 |
| 1986 | CG | LYS | 771 | 49.188 | -0.525 | 70.712 | 1.00 | 63.84 |
| 1987 | CD | LYS | 771 | 48.443 | 0.681 | 71.308 | 1.00 | 63.12 |
| 1988 | CE | LYS | 771 | 49.384 | 1.588 | 72.100 | 1.00 | 59.83 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 1989 | NZ | LYS | 771 | 48.821 | 2.970 | 72.186 | 1.00 | 60.16 |
| 1990 | C | LYS | 771 | 50.532 | -1.976 | 68.550 | 1.00 | 60.42 |
| 1991 | O | LYS | 771 | 51.276 | -1.561 | 69.430 | 1.00 | 62.03 |
| 1992 | N | LEU | 772 | 50.928 | -2.120 | 67.290 | 1.00 | 60.80 |
| 1993 | CA | LEU | 772 | 52.285 | -1.756 | 66.890 | 1.00 | 58.97 |
| 1994 | CB | LEU | 772 | 52.629 | -2.368 | 65.533 | 1.00 | 62.77 |
| 1995 | CG | LEU | 772 | 52.781 | -3.885 | 65.492 | 1.00 | 63.82 |
| 1996 | CD1 | LEU | 772 | 52.780 | -4.346 | 64.046 | 1.00 | 62.61 |
| 1997 | CD2 | LEU | 772 | 54.071 | -4.295 | 66.203 | 1.00 | 63.11 |
| 1998 | C | LEU | 772 | 52.405 | -0.243 | 66.812 | 1.00 | 61.57 |
| 1999 | O | LEU | 772 | 51.513 | 0.428 | 66.289 | 1.00 | 60.62 |
| 2000 | N | LEU | 773 | 53.499 | 0.296 | 67.341 | 1.00 | 59.76 |
| 2001 | CA | LEU | 773 | 53.704 | 1.734 | 67.317 | 1.00 | 60.00 |
| 2002 | CB | LEU | 773 | 53.602 | 2.320 | 68.725 | 1.00 | 59.66 |
| 2003 | CG | LEU | 773 | 52.221 | 2.321 | 69.380 | 1.00 | 62.80 |
| 2004 | CD1 | LEU | 773 | 52.290 | 2.999 | 70.729 | 1.00 | 61.23 |
| 2005 | CD2 | LEU | 773 | 51.233 | 3.053 | 68.490 | 1.00 | 65.68 |
| 2006 | C | LEU | 773 | 55.051 | 2.094 | 66.727 | 1.00 | 62.80 |
| 2007 | O | LEU | 773 | 55.911 | 1.234 | 66.517 | 1.00 | 61.81 |
| 2008 | N | PHE | 774 | 55.219 | 3.382 | 66.456 | 1.00 | 60.94 |
| 2009 | CA | PHE | 774 | 56.451 | 3.917 | 65.897 | 1.00 | 61.49 |
| 2010 | CB | PHE | 774 | 56.128 | 4.864 | 64.743 | 1.00 | 63.45 |
| 2011 | CG | PHE | 774 | 55.889 | 4.169 | 63.451 | 1.00 | 61.20 |
| 2012 | CD1 | PHE | 774 | 56.936 | 3.532 | 62.802 | 1.00 | 63.76 |
| 2013 | CD2 | PHE | 774 | 54.621 | 4.105 | 62.902 | 1.00 | 61.89 |
| 2014 | CE1 | PHE | 774 | 56.727 | 2.838 | 61.627 | 1.00 | 56.93 |
| 2015 | CE2 | PHE | 774 | 54.395 | 3.409 | 61.720 | 1.00 | 59.92 |
| 2016 | CZ | PHE | 774 | 55.451 | 2.773 | 61.081 | 1.00 | 62.56 |
| 2017 | C | PHE | 774 | 57.175 | 4.681 | 66.984 | 1.00 | 60.20 |
| 2018 | O | PHE | 774 | 58.400 | 4.702 | 67.046 | 1.00 | 60.03 |
| 2019 | N | HIS | 775 | 56.384 | 5.301 | 67.849 | 1.00 | 62.48 |
| 2020 | CA | HIS | 775 | 56.905 | 6.104 | 68.934 | 1.00 | 64.64 |
| 2021 | CB | HIS | 775 | 56.466 | 7.558 | 68.730 | 1.00 | 61.69 |
| 2022 | CG | HIS | 775 | 56.898 | 8.120 | 67.417 | 1.00 | 59.70 |
| 2023 | CD2 | HIS | 775 | 56.188 | 8.565 | 66.356 | 1.00 | 62.49 |
| 2024 | ND1 | HIS | 775 | 58.223 | 8.191 | 67.047 | 1.00 | 60.30 |
| 2025 | CE1 | HIS | 775 | 58.313 | 8.652 | 65.813 | 1.00 | 59.19 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2026 | NE2 | HIS | 775 | 57.092 | 8.886 | 65.370 | 1.00 | 59.04 |
| 2027 | C | HIS | 775 | 56.428 | 5.596 | 70.277 | 1.00 | 64.58 |
| 2028 | O | HIS | 775 | 55.390 | 4.948 | 70.373 | 1.00 | 58.66 |
| 2029 | N | GLN | 776 | 57.203 | 5.895 | 71.311 | 1.00 | 60.50 |
| 2030 | CA | GLN | 776 | 56.864 | 5.502 | 72.669 | 1.00 | 61.43 |
| 2031 | CB | GLN | 776 | 58.154 | 5.293 | 73.457 | 1.00 | 59.86 |
| 2032 | CG | GLN | 776 | 59.281 | 6.243 | 73.042 | 1.00 | 57.49 |
| 2033 | CD | GLN | 776 | 60.679 | 5.638 | 73.241 | 1.00 | 60.53 |
| 2034 | OE1 | GLN | 776 | 61.696 | 6.265 | 72.919 | 1.00 | 61.97 |
| 2035 | NE2 | GLN | 776 | 60.730 | 4.412 | 73.772 | 1.00 | 60.63 |
| 2036 | C | GLN | 776 | 55.993 | 6.583 | 73.335 | 1.00 | 60.06 |
| 2037 | O | GLN | 776 | 56.098 | 7.765 | 72.919 | 1.00 | 61.84 |
| 2038 | OXT | GLN | 776 | 55.219 | 6.239 | 74.268 | 1.00 | 60.74 |
| 2039 | CB | GLU | 741 | 35.922 | -16.424 | 65.488 | 1.00 | 58.89 |
| 2040 | CG | GLU | 741 | 36.766 | -17.078 | 64.386 | 1.00 | 67.76 |
| 2041 | CD | GLU | 741 | 38.144 | -16.463 | 64.277 | 1.00 | 60.66 |
| 2042 | OE1 | GLU | 741 | 38.838 | -16.698 | 63.252 | 1.00 | 59.66 |
| 2043 | OE2 | GLU | 741 | 38.524 | -15.741 | 65.233 | 1.00 | 61.27 |
| 2044 | C | GLU | 741 | 33.996 | -16.854 | 64.024 | 1.00 | 61.38 |
| 2045 | O | GLU | 741 | 33.681 | -15.808 | 63.464 | 1.00 | 63.70 |
| 2046 | N | GLU | 741 | 34.336 | -18.280 | 65.946 | 1.00 | 62.41 |
| 2047 | CA | GLU | 741 | 34.460 | -16.870 | 65.464 | 1.00 | 59.35 |
| 2048 | N | GLU | 742 | 33.995 | -18.034 | 63.422 | 1.00 | 58.35 |
| 2049 | CA | GLU | 742 | 33.594 | -18.184 | 62.036 | 1.00 | 63.61 |
| 2050 | CB | GLU | 742 | 32.158 | -17.672 | 61.824 | 1.00 | 60.80 |
| 2051 | CG | GLU | 742 | 31.516 | -18.155 | 60.518 | 1.00 | 62.06 |
| 2052 | CD | GLU | 742 | 31.783 | -19.645 | 60.238 | 1.00 | 63.64 |
| 2053 | OE1 | GLU | 742 | 31.146 | -20.190 | 59.309 | 1.00 | 64.92 |
| 2054 | OE2 | GLU | 742 | 32.632 | -20.276 | 60.925 | 1.00 | 62.96 |
| 2055 | C | GLU | 742 | 34.541 | -17.482 | 61.064 | 1.00 | 61.07 |
| 2056 | O | GLU | 742 | 35.655 | -17.950 | 60.825 | 1.00 | 59.51 |
| 2057 | N | ASN | 743 | 34.090 | -16.355 | 60.522 | 1.00 | 61.83 |
| 2058 | CA | ASN | 743 | 34.850 | -15.586 | 59.535 | 1.00 | 60.95 |
| 2059 | CB | ASN | 743 | 36.300 | -15.368 | 59.987 | 1.00 | 61.82 |
| 2060 | CG | ASN | 743 | 36.418 | -14.360 | 61.118 | 1.00 | 64.29 |
| 2061 | OD1 | ASN | 743 | 36.646 | -14.729 | 62.277 | 1.00 | 58.19 |
| 2062 | ND2 | ASN | 743 | 36.262 | -13.077 | 60.787 | 1.00 | 58.54 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2063 | C | ASN | 743 | 34.842 | -16.318 | 58.192 | 1.00 | 60.80 |
| 2064 | O | ASN | 743 | 35.780 | -16.196 | 57.410 | 1.00 | 62.67 |
| 2065 | N | ALA | 744 | 33.779 | -17.075 | 57.935 | 1.00 | 59.69 |
| 2066 | CA | ALA | 744 | 33.640 | -17.827 | 56.696 | 1.00 | 61.76 |
| 2067 | CB | ALA | 744 | 32.350 | -18.623 | 56.729 | 1.00 | 60.04 |
| 2068 | C | ALA | 744 | 33.675 | -16.930 | 55.453 | 1.00 | 62.63 |
| 2069 | O | ALA | 744 | 34.423 | -17.199 | 54.503 | 1.00 | 58.54 |
| 2070 | N | LEU | 745 | 32.869 | -15.866 | 55.462 | 1.00 | 60.19 |
| 2071 | CA | LEU | 745 | 32.820 | -14.936 | 54.329 | 1.00 | 62.12 |
| 2072 | CB | LEU | 745 | 31.855 | -13.783 | 54.617 | 1.00 | 60.59 |
| 2073 | CG | LEU | 745 | 30.606 | -13.726 | 53.739 | 1.00 | 58.63 |
| 2074 | CD1 | LEU | 745 | 29.895 | -12.400 | 53.937 | 1.00 | 64.25 |
| 2075 | CD2 | LEU | 745 | 31.004 | -13.902 | 52.291 | 1.00 | 60.41 |
| 2076 | C | LEU | 745 | 34.190 | -14.360 | 53.952 | 1.00 | 61.72 |
| 2077 | O | LEU | 745 | 34.521 | -14.276 | 52.776 | 1.00 | 59.83 |
| 2078 | N | LEU | 746 | 34.978 | -13.961 | 54.946 | 1.00 | 57.79 |
| 2079 | CA | LEU | 746 | 36.311 | -13.413 | 54.691 | 1.00 | 60.45 |
| 2080 | CB | LEU | 746 | 36.898 | -12.849 | 55.989 | 1.00 | 61.06 |
| 2081 | CG | LEU | 746 | 37.673 | -11.534 | 55.956 | 1.00 | 61.13 |
| 2082 | CD1 | LEU | 746 | 38.380 | -11.374 | 57.275 | 1.00 | 61.70 |
| 2083 | CD2 | LEU | 746 | 38.664 | -11.520 | 54.823 | 1.00 | 61.56 |
| 2084 | C | LEU | 746 | 37.249 | -14.511 | 54.156 | 1.00 | 58.73 |
| 2085 | O | LEU | 746 | 37.905 | -14.356 | 53.120 | 1.00 | 62.68 |
| 2086 | N | ARG | 747 | 37.307 | -15.613 | 54.895 | 1.00 | 61.00 |
| 2087 | CA | ARG | 747 | 38.145 | -16.759 | 54.560 | 1.00 | 57.24 |
| 2088 | CB | ARG | 747 | 37.853 | -17.906 | 55.539 | 1.00 | 66.74 |
| 2089 | CG | ARG | 747 | 38.332 | -19.278 | 55.102 | 1.00 | 61.05 |
| 2090 | CD | ARG | 747 | 38.533 | -20.190 | 56.319 | 1.00 | 62.51 |
| 2091 | NE | ARG | 747 | 39.807 | -19.938 | 56.998 | 1.00 | 62.06 |
| 2092 | CZ | ARG | 747 | 39.952 | -19.867 | 58.321 | 1.00 | 63.78 |
| 2093 | NH1 | ARG | 747 | 38.894 | -20.023 | 59.118 | 1.00 | 60.31 |
| 2094 | NH2 | ARG | 747 | 41.156 | -19.637 | 58.848 | 1.00 | 60.80 |
| 2095 | C | ARG | 747 | 37.883 | -17.186 | 53.134 | 1.00 | 59.12 |
| 2096 | O | ARG | 747 | 38.793 | -17.589 | 52.418 | 1.00 | 62.02 |
| 2097 | N | TYR | 748 | 36.623 | -17.088 | 52.731 | 1.00 | 61.89 |
| 2098 | CA | TYR | 748 | 36.215 | -17.449 | 51.387 | 1.00 | 65.00 |
| 2099 | CB | TYR | 748 | 34.685 | -17.413 | 51.301 | 1.00 | 58.95 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2100 | CG | TYR | 748 | 34.136 | -17.350 | 49.897 | 1.00 | 62.12 |
| 2101 | CD1 | TYR | 748 | 34.256 | -18.429 | 49.021 | 1.00 | 63.79 |
| 2102 | CE1 | TYR | 748 | 33.805 | -18.342 | 47.715 | 1.00 | 61.69 |
| 2103 | CD2 | TYR | 748 | 33.544 | -16.185 | 49.430 | 1.00 | 61.63 |
| 2104 | CE2 | TYR | 748 | 33.091 | -16.084 | 48.127 | 1.00 | 60.08 |
| 2105 | CZ | TYR | 748 | 33.226 | -17.163 | 47.272 | 1.00 | 58.42 |
| 2106 | OH | TYR | 748 | 32.806 | -17.030 | 45.966 | 1.00 | 59.74 |
| 2107 | C | TYR | 748 | 36.832 | -16.447 | 50.419 | 1.00 | 61.75 |
| 2108 | O | TYR | 748 | 37.599 | -16.807 | 49.532 | 1.00 | 61.82 |
| 2109 | N | LEU | 749 | 36.501 | -15.178 | 50.628 | 1.00 | 60.52 |
| 2110 | CA | LEU | 749 | 36.974 | -14.069 | 49.800 | 1.00 | 61.53 |
| 2111 | CB | LEU | 749 | 36.380 | -12.757 | 50.303 | 1.00 | 63.00 |
| 2112 | CG | LEU | 749 | 34.873 | -12.741 | 50.535 | 1.00 | 57.09 |
| 2113 | CD1 | LEU | 749 | 34.508 | -11.427 | 51.167 | 1.00 | 62.85 |
| 2114 | CD2 | LEU | 749 | 34.113 | -12.954 | 49.233 | 1.00 | 62.37 |
| 2115 | C | LEU | 749 | 38.482 | -13.910 | 49.733 | 1.00 | 64.05 |
| 2116 | O | LEU | 749 | 38.991 | -13.275 | 48.807 | 1.00 | 61.97 |
| 2117 | N | LEU | 750 | 39.195 | -14.475 | 50.703 | 1.00 | 59.99 |
| 2118 | CA | LEU | 750 | 40.644 | -14.363 | 50.728 | 1.00 | 63.11 |
| 2119 | CB | LEU | 750 | 41.147 | -14.497 | 52.167 | 1.00 | 62.65 |
| 2120 | CG | LEU | 750 | 41.463 | -13.160 | 52.849 | 1.00 | 63.02 |
| 2121 | CD1 | LEU | 750 | 41.695 | -13.344 | 54.327 | 1.00 | 58.04 |
| 2122 | CD2 | LEU | 750 | 42.693 | -12.559 | 52.194 | 1.00 | 62.24 |
| 2123 | C | LEU | 750 | 41.436 | -15.294 | 49.803 | 1.00 | 64.30 |
| 2124 | O | LEU | 750 | 42.666 | -15.246 | 49.796 | 1.00 | 58.24 |
| 2125 | N | ASP | 751 | 40.745 | -16.129 | 49.024 | 1.00 | 59.30 |
| 2126 | CA | ASP | 751 | 41.396 | -17.048 | 48.068 | 1.00 | 62.49 |
| 2127 | CB | ASP | 751 | 41.860 | -18.337 | 48.764 | 1.00 | 62.90 |
| 2128 | CG | ASP | 751 | 40.921 | -18.784 | 49.855 | 1.00 | 62.09 |
| 2129 | OD1 | ASP | 751 | 40.342 | -17.900 | 50.526 | 1.00 | 62.68 |
| 2130 | OD2 | ASP | 751 | 40.779 | -20.014 | 50.051 | 1.00 | 60.33 |
| 2131 | C | ASP | 751 | 40.472 | -17.371 | 46.897 | 1.00 | 61.25 |
| 2132 | O | ASP | 751 | 39.476 | -18.065 | 47.053 | 1.00 | 61.96 |
| 2133 | N | LYS | 752 | 40.824 | -16.849 | 45.725 | 1.00 | 62.34 |
| 2134 | CA | LYS | 752 | 40.030 | -17.003 | 44.506 | 1.00 | 62.50 |
| 2135 | CB | LYS | 752 | 38.733 | -16.160 | 44.600 | 1.00 | 63.17 |
| 2136 | CG | LYS | 752 | 37.872 | -16.386 | 45.858 | 1.00 | 60.82 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2137 | CD | LYS | 752 | 36.923 | -15.230 | 46.159 | 1.00 | 62.16 |
| 2138 | CE | LYS | 752 | 35.726 | -15.186 | 45.223 | 1.00 | 60.30 |
| 2139 | NZ | LYS | 752 | 36.101 | -15.095 | 43.779 | 1.00 | 61.47 |
| 2140 | C | LYS | 752 | 40.895 | -16.438 | 43.384 | 1.00 | 63.98 |
| 2141 | O | LYS | 752 | 42.043 | -16.833 | 43.212 | 1.00 | 60.08 |
| 2142 | N | ASP | 753 | 40.322 | -15.496 | 42.641 | 1.00 | 61.50 |
| 2143 | CA | ASP | 753 | 41.004 | -14.809 | 41.552 | 1.00 | 60.87 |
| 2144 | CB | ASP | 753 | 41.969 | -13.778 | 42.151 | 1.00 | 61.81 |
| 2145 | CG | ASP | 753 | 41.293 | -12.886 | 43.212 | 1.00 | 65.36 |
| 2146 | OD1 | ASP | 753 | 40.845 | -13.417 | 44.262 | 1.00 | 58.98 |
| 2147 | OD2 | ASP | 753 | 41.204 | -11.653 | 42.996 | 1.00 | 62.40 |
| 2148 | C | ASP | 753 | 41.712 | -15.723 | 40.533 | 1.00 | 62.25 |
| 2149 | O | ASP | 753 | 41.905 | -16.929 | 40.770 | 1.00 | 62.51 |
| 2150 | N | ASP | 754 | 42.076 | -15.125 | 39.394 | 1.00 | 59.26 |
| 2151 | CA | ASP | 754 | 42.713 | -15.818 | 38.271 | 1.00 | 62.08 |
| 2152 | CB | ASP | 754 | 42.118 | -15.302 | 36.949 | 1.00 | 60.51 |
| 2153 | CG | ASP | 754 | 41.121 | -14.148 | 37.150 | 1.00 | 60.95 |
| 2154 | OD1 | ASP | 754 | 40.683 | -13.555 | 36.135 | 1.00 | 61.57 |
| 2155 | OD2 | ASP | 754 | 40.762 | -13.831 | 38.304 | 1.00 | 61.61 |
| 2156 | C | ASP | 754 | 44.236 | -15.678 | 38.234 | 1.00 | 60.21 |
| 2157 | O | ASP | 754 | 44.946 | -16.712 | 38.264 | 1.00 | 63.29 |
| 2158 | OXT | ASP | 754 | 44.707 | -14.526 | 38.169 | 1.00 | 61.39 |
| 2159 | CB | GLN | 527 | 44.425 | 43.308 | 57.458 | 1.00 | 59.84 |
| 2160 | CG | GLN | 527 | 45.330 | 43.181 | 58.697 | 1.00 | 63.06 |
| 2161 | CD | GLN | 527 | 46.173 | 41.895 | 58.675 | 1.00 | 61.64 |
| 2162 | OE1 | GLN | 527 | 46.913 | 41.596 | 59.623 | 1.00 | 62.44 |
| 2163 | NE2 | GLN | 527 | 46.065 | 41.137 | 57.583 | 1.00 | 63.46 |
| 2164 | C | GLN | 527 | 43.994 | 44.763 | 55.475 | 1.00 | 60.61 |
| 2165 | O | GLN | 527 | 44.711 | 45.517 | 54.798 | 1.00 | 61.62 |
| 2166 | N | GLN | 527 | 42.843 | 45.238 | 57.671 | 1.00 | 62.80 |
| 2167 | CA | GLN | 527 | 44.095 | 44.745 | 57.006 | 1.00 | 60.11 |
| 2168 | N | LEU | 528 | 43.105 | 43.912 | 54.955 | 1.00 | 62.93 |
| 2169 | CA | LEU | 528 | 42.857 | 43.747 | 53.516 | 1.00 | 58.92 |
| 2170 | CB | LEU | 528 | 43.696 | 42.579 | 52.979 | 1.00 | 61.24 |
| 2171 | CG | LEU | 528 | 45.210 | 42.751 | 53.124 | 1.00 | 58.97 |
| 2172 | CD1 | LEU | 528 | 45.858 | 41.410 | 53.501 | 1.00 | 57.95 |
| 2173 | CD2 | LEU | 528 | 45.778 | 43.371 | 51.831 | 1.00 | 60.61 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2174 | C | LEU | 528 | 41.369 | 43.449 | 53.303 | 1.00 | 63.94 |
| 2175 | O | LEU | 528 | 40.531 | 44.290 | 53.650 | 1.00 | 62.36 |
| 2176 | N | THR | 529 | 41.067 | 42.256 | 52.748 | 1.00 | 59.95 |
| 2177 | CA | THR | 529 | 39.689 | 41.769 | 52.487 | 1.00 | 61.84 |
| 2178 | CB | THR | 529 | 39.396 | 40.430 | 53.232 | 1.00 | 59.05 |
| 2179 | OG1 | THR | 529 | 40.375 | 39.442 | 52.866 | 1.00 | 63.83 |
| 2180 | CG2 | THR | 529 | 37.997 | 39.920 | 52.882 | 1.00 | 59.80 |
| 2181 | C | THR | 529 | 38.725 | 42.842 | 52.992 | 1.00 | 61.32 |
| 2182 | O | THR | 529 | 37.988 | 42.645 | 53.973 | 1.00 | 61.35 |
| 2183 | N | PRO | 530 | 38.704 | 43.977 | 52.271 | 1.00 | 64.25 |
| 2184 | CD | PRO | 530 | 38.650 | 43.494 | 50.878 | 1.00 | 59.35 |
| 2185 | CA | PRO | 530 | 38.021 | 45.276 | 52.317 | 1.00 | 58.74 |
| 2186 | CB | PRO | 530 | 37.610 | 45.502 | 50.860 | 1.00 | 60.27 |
| 2187 | CG | PRO | 530 | 37.368 | 44.132 | 50.384 | 1.00 | 65.84 |
| 2188 | C | PRO | 530 | 36.839 | 45.387 | 53.239 | 1.00 | 62.92 |
| 2189 | O | PRO | 530 | 36.948 | 45.328 | 54.474 | 1.00 | 60.28 |
| 2190 | N | THR | 531 | 35.706 | 45.591 | 52.590 | 1.00 | 63.85 |
| 2191 | CA | THR | 531 | 34.443 | 45.708 | 53.246 | 1.00 | 62.34 |
| 2192 | CB | THR | 531 | 34.253 | 47.135 | 53.851 | 1.00 | 63.80 |
| 2193 | OG1 | THR | 531 | 33.854 | 47.016 | 55.230 | 1.00 | 64.25 |
| 2194 | CG2 | THR | 531 | 33.218 | 47.940 | 53.067 | 1.00 | 61.34 |
| 2195 | C | THR | 531 | 33.526 | 45.410 | 52.081 | 1.00 | 61.78 |
| 2196 | O | THR | 531 | 32.505 | 44.758 | 52.251 | 1.00 | 61.93 |
| 2197 | N | LEU | 532 | 33.917 | 45.822 | 50.877 | 1.00 | 62.02 |
| 2198 | CA | LEU | 532 | 33.060 | 45.545 | 49.722 | 1.00 | 60.94 |
| 2199 | CB | LEU | 532 | 33.410 | 46.445 | 48.528 | 1.00 | 57.32 |
| 2200 | CG | LEU | 532 | 32.463 | 46.285 | 47.329 | 1.00 | 64.49 |
| 2201 | CD1 | LEU | 532 | 31.027 | 46.313 | 47.771 | 1.00 | 61.90 |
| 2202 | CD2 | LEU | 532 | 32.702 | 47.377 | 46.344 | 1.00 | 62.84 |
| 2203 | C | LEU | 532 | 33.077 | 44.077 | 49.283 | 1.00 | 61.37 |
| 2204 | O | LEU | 532 | 32.016 | 43.461 | 49.149 | 1.00 | 62.65 |
| 2205 | N | VAL | 533 | 34.266 | 43.518 | 49.052 | 1.00 | 61.22 |
| 2206 | CA | VAL | 533 | 34.366 | 42.119 | 48.635 | 1.00 | 60.75 |
| 2207 | CB | VAL | 533 | 35.781 | 41.753 | 48.114 | 1.00 | 59.58 |
| 2208 | CG1 | VAL | 533 | 36.697 | 41.400 | 49.264 | 1.00 | 60.31 |
| 2209 | CG2 | VAL | 533 | 35.695 | 40.576 | 47.185 | 1.00 | 59.77 |
| 2210 | C | VAL | 533 | 34.062 | 41.240 | 49.840 | 1.00 | 63.61 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2211 | O | VAL | 533 | 33.861 | 40.038 | 49.709 | 1.00 | 62.93 |
| 2212 | N | SER | 534 | 34.053 | 41.859 | 51.013 | 1.00 | 61.64 |
| 2213 | CA | SER | 534 | 33.774 | 41.170 | 52.260 | 1.00 | 60.61 |
| 2214 | CB | SER | 534 | 34.143 | 42.089 | 53.425 | 1.00 | 62.32 |
| 2215 | OG | SER | 534 | 34.391 | 41.364 | 54.612 | 1.00 | 60.70 |
| 2216 | C | SER | 534 | 32.276 | 40.880 | 52.270 | 1.00 | 62.67 |
| 2217 | O | SER | 534 | 31.799 | 39.906 | 52.854 | 1.00 | 59.90 |
| 2218 | N | LEU | 535 | 31.544 | 41.747 | 51.593 | 1.00 | 60.18 |
| 2219 | CA | LEU | 535 | 30.102 | 41.644 | 51.508 | 1.00 | 60.75 |
| 2220 | CB | LEU | 535 | 29.527 | 42.975 | 51.048 | 1.00 | 61.59 |
| 2221 | CG | LEU | 535 | 28.027 | 43.101 | 51.245 | 1.00 | 61.16 |
| 2222 | CD1 | LEU | 535 | 27.773 | 43.835 | 52.537 | 1.00 | 63.92 |
| 2223 | CD2 | LEU | 535 | 27.416 | 43.850 | 50.089 | 1.00 | 61.72 |
| 2224 | C | LEU | 535 | 29.688 | 40.547 | 50.543 | 1.00 | 60.18 |
| 2225 | O | LEU | 535 | 28.852 | 39.714 | 50.868 | 1.00 | 58.39 |
| 2226 | N | LEU | 536 | 30.269 | 40.552 | 49.349 | 1.00 | 60.57 |
| 2227 | CA | LEU | 536 | 29.950 | 39.536 | 48.355 | 1.00 | 63.14 |
| 2228 | CB | LEU | 536 | 30.813 | 39.719 | 47.115 | 1.00 | 61.69 |
| 2229 | CG | LEU | 536 | 30.671 | 40.980 | 46.284 | 1.00 | 63.99 |
| 2230 | CD1 | LEU | 536 | 31.622 | 40.864 | 45.118 | 1.00 | 60.60 |
| 2231 | CD2 | LEU | 536 | 29.247 | 41.145 | 45.801 | 1.00 | 62.81 |
| 2232 | C | LEU | 536 | 30.204 | 38.140 | 48.908 | 1.00 | 61.72 |
| 2233 | O | LEU | 536 | 29.703 | 37.141 | 48.379 | 1.00 | 60.04 |
| 2234 | N | GLU | 537 | 30.996 | 38.086 | 49.970 | 1.00 | 60.48 |
| 2235 | CA | GLU | 537 | 31.359 | 36.838 | 50.607 | 1.00 | 61.60 |
| 2236 | CB | GLU | 537 | 32.691 | 37.003 | 51.307 | 1.00 | 61.56 |
| 2237 | CG | GLU | 537 | 33.169 | 35.763 | 51.998 | 1.00 | 58.70 |
| 2238 | CD | GLU | 537 | 34.599 | 35.907 | 52.442 | 1.00 | 62.52 |
| 2239 | OE1 | GLU | 537 | 35.173 | 34.900 | 52.919 | 1.00 | 61.18 |
| 2240 | OE2 | GLU | 537 | 35.140 | 37.033 | 52.305 | 1.00 | 60.21 |
| 2241 | C | GLU | 537 | 30.344 | 36.288 | 51.592 | 1.00 | 59.30 |
| 2242 | O | GLU | 537 | 30.094 | 35.084 | 51.604 | 1.00 | 60.22 |
| 2243 | N | VAL | 538 | 29.773 | 37.155 | 52.424 | 1.00 | 61.88 |
| 2244 | CA | VAL | 538 | 28.781 | 36.714 | 53.399 | 1.00 | 62.81 |
| 2245 | CB | VAL | 538 | 28.636 | 37.687 | 54.591 | 1.00 | 57.08 |
| 2246 | CG1 | VAL | 538 | 29.993 | 38.283 | 54.957 | 1.00 | 61.43 |
| 2247 | CG2 | VAL | 538 | 27.611 | 38.757 | 54.268 | 1.00 | 60.61 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2248 | C | VAL | 538 | 27.420 | 36.586 | 52.752 | 1.00 | 61.99 |
| 2249 | O | VAL | 538 | 26.576 | 35.841 | 53.233 | 1.00 | 61.89 |
| 2250 | N | ILE | 539 | 27.203 | 37.322 | 51.669 | 1.00 | 61.34 |
| 2251 | CA | ILE | 539 | 25.931 | 37.263 | 50.977 | 1.00 | 60.88 |
| 2252 | CB | ILE | 539 | 25.628 | 38.548 | 50.209 | 1.00 | 57.53 |
| 2253 | CG2 | ILE | 539 | 26.034 | 39.737 | 51.035 | 1.00 | 63.74 |
| 2254 | CG1 | ILE | 539 | 26.365 | 38.550 | 48.869 | 1.00 | 64.95 |
| 2255 | CD1 | ILE | 539 | 25.847 | 39.584 | 47.898 | 1.00 | 61.40 |
| 2256 | C | ILE | 539 | 25.946 | 36.133 | 49.977 | 1.00 | 59.86 |
| 2257 | O | ILE | 539 | 24.984 | 35.934 | 49.251 | 1.00 | 60.95 |
| 2258 | N | GLU | 540 | 27.051 | 35.408 | 49.919 | 1.00 | 61.19 |
| 2259 | CA | GLU | 540 | 27.170 | 34.290 | 48.993 | 1.00 | 64.61 |
| 2260 | CB | GLU | 540 | 28.620 | 33.798 | 48.960 | 1.00 | 61.89 |
| 2261 | CG | GLU | 540 | 28.917 | 32.628 | 48.022 | 1.00 | 59.99 |
| 2262 | CD | GLU | 540 | 28.635 | 32.926 | 46.560 | 1.00 | 60.98 |
| 2263 | OE1 | GLU | 540 | 28.949 | 34.050 | 46.103 | 1.00 | 60.19 |
| 2264 | OE2 | GLU | 540 | 28.113 | 32.025 | 45.861 | 1.00 | 58.09 |
| 2265 | C | GLU | 540 | 26.241 | 33.181 | 49.469 | 1.00 | 60.25 |
| 2266 | O | GLU | 540 | 26.357 | 32.710 | 50.603 | 1.00 | 62.79 |
| 2267 | N | PRO | 541 | 25.289 | 32.766 | 48.614 | 1.00 | 59.70 |
| 2268 | CD | PRO | 541 | 24.961 | 33.287 | 47.275 | 1.00 | 61.01 |
| 2269 | CA | PRO | 541 | 24.362 | 31.703 | 49.013 | 1.00 | 61.09 |
| 2270 | CB | PRO | 541 | 23.361 | 31.665 | 47.861 | 1.00 | 63.13 |
| 2271 | CG | PRO | 541 | 24.138 | 32.177 | 46.694 | 1.00 | 57.00 |
| 2272 | C | PRO | 541 | 25.051 | 30.365 | 49.254 | 1.00 | 60.83 |
| 2273 | O | PRO | 541 | 25.979 | 29.988 | 48.535 | 1.00 | 60.28 |
| 2274 | N | GLU | 542 | 24.607 | 29.669 | 50.297 | 1.00 | 60.82 |
| 2275 | CA | GLU | 542 | 25.157 | 28.364 | 50.655 | 1.00 | 61.11 |
| 2276 | CB | GLU | 542 | 24.607 | 27.933 | 52.001 | 1.00 | 63.94 |
| 2277 | CG | GLU | 542 | 23.163 | 27.558 | 51.899 | 1.00 | 65.51 |
| 2278 | CD | GLU | 542 | 22.569 | 27.217 | 53.237 | 1.00 | 61.20 |
| 2279 | OE1 | GLU | 542 | 21.369 | 26.813 | 53.269 | 1.00 | 64.82 |
| 2280 | OE2 | GLU | 542 | 23.307 | 27.360 | 54.253 | 1.00 | 61.05 |
| 2281 | C | GLU | 542 | 24.668 | 27.393 | 49.584 | 1.00 | 60.85 |
| 2282 | O | GLU | 542 | 23.631 | 27.637 | 48.966 | 1.00 | 61.52 |
| 2283 | N | VAL | 543 | 25.369 | 26.291 | 49.353 | 1.00 | 62.89 |
| 2284 | CA | VAL | 543 | 24.879 | 25.397 | 48.316 | 1.00 | 61.20 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2285 | CB | VAL | 543 | 26.029 | 24.589 | 47.676 | 1.00 | 60.15 |
| 2286 | CG1 | VAL | 543 | 27.366 | 25.164 | 48.116 | 1.00 | 61.25 |
| 2287 | CG2 | VAL | 543 | 25.903 | 23.132 | 48.011 | 1.00 | 64.25 |
| 2288 | C | VAL | 543 | 23.745 | 24.465 | 48.760 | 1.00 | 61.19 |
| 2289 | O | VAL | 543 | 23.506 | 24.232 | 49.955 | 1.00 | 61.59 |
| 2290 | N | LEU | 544 | 23.037 | 23.944 | 47.769 | 1.00 | 60.75 |
| 2291 | CA | LEU | 544 | 21.910 | 23.068 | 48.018 | 1.00 | 62.70 |
| 2292 | CB | LEU | 544 | 20.686 | 23.557 | 47.244 | 1.00 | 61.76 |
| 2293 | CG | LEU | 544 | 20.315 | 25.041 | 47.237 | 1.00 | 62.21 |
| 2294 | CD1 | LEU | 544 | 19.968 | 25.490 | 48.639 | 1.00 | 60.39 |
| 2295 | CD2 | LEU | 544 | 21.464 | 25.856 | 46.654 | 1.00 | 61.37 |
| 2296 | C | LEU | 544 | 22.209 | 21.639 | 47.591 | 1.00 | 61.62 |
| 2297 | O | LEU | 544 | 23.047 | 21.388 | 46.721 | 1.00 | 61.82 |
| 2298 | N | TYR | 545 | 21.504 | 20.713 | 48.222 | 1.00 | 60.41 |
| 2299 | CA | TYR | 545 | 21.623 | 19.306 | 47.915 | 1.00 | 64.14 |
| 2300 | CB | TYR | 545 | 21.436 | 18.495 | 49.189 | 1.00 | 59.96 |
| 2301 | CG | TYR | 545 | 22.566 | 18.713 | 50.160 | 1.00 | 66.69 |
| 2302 | CD1 | TYR | 545 | 22.715 | 17.908 | 51.287 | 1.00 | 63.31 |
| 2303 | CE1 | TYR | 545 | 23.810 | 18.056 | 52.122 | 1.00 | 61.54 |
| 2304 | CD2 | TYR | 545 | 23.537 | 19.684 | 49.907 | 1.00 | 62.21 |
| 2305 | CE2 | TYR | 545 | 24.632 | 19.843 | 50.739 | 1.00 | 61.49 |
| 2306 | CZ | TYR | 545 | 24.769 | 19.022 | 51.842 | 1.00 | 59.50 |
| 2307 | OH | TYR | 545 | 25.898 | 19.142 | 52.619 | 1.00 | 60.93 |
| 2308 | C | TYR | 545 | 20.482 | 19.111 | 46.949 | 1.00 | 58.64 |
| 2309 | O | TYR | 545 | 19.553 | 19.912 | 46.956 | 1.00 | 62.56 |
| 2310 | N | ALA | 546 | 20.532 | 18.077 | 46.115 | 1.00 | 57.51 |
| 2311 | CA | ALA | 546 | 19.461 | 17.886 | 45.145 | 1.00 | 59.14 |
| 2312 | CB | ALA | 546 | 20.026 | 17.390 | 43.845 | 1.00 | 60.50 |
| 2313 | C | ALA | 546 | 18.347 | 16.965 | 45.595 | 1.00 | 62.16 |
| 2314 | O | ALA | 546 | 17.352 | 16.821 | 44.895 | 1.00 | 62.38 |
| 2315 | N | GLY | 547 | 18.493 | 16.338 | 46.755 | 1.00 | 59.60 |
| 2316 | CA | GLY | 547 | 17.441 | 15.445 | 47.203 | 1.00 | 63.46 |
| 2317 | C | GLY | 547 | 17.125 | 14.408 | 46.137 | 1.00 | 63.02 |
| 2318 | O | GLY | 547 | 15.968 | 14.068 | 45.890 | 1.00 | 60.60 |
| 2319 | N | TYR | 548 | 18.180 | 13.918 | 45.496 | 1.00 | 62.10 |
| 2320 | CA | TYR | 548 | 18.095 | 12.906 | 44.447 | 1.00 | 60.16 |
| 2321 | CB | TYR | 548 | 19.366 | 12.971 | 43.621 | 1.00 | 60.58 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2322 | CG | TYR | 548 | 19.357 | 12.107 | 42.403 | 1.00 | 61.55 |
| 2323 | CD1 | TYR | 548 | 18.522 | 12.406 | 41.331 | 1.00 | 65.33 |
| 2324 | CE1 | TYR | 548 | 18.550 | 11.657 | 40.175 | 1.00 | 62.35 |
| 2325 | CD2 | TYR | 548 | 20.218 | 11.023 | 42.292 | 1.00 | 62.44 |
| 2326 | CE2 | TYR | 548 | 20.251 | 10.267 | 41.142 | 1.00 | 64.59 |
| 2327 | CZ | TYR | 548 | 19.416 | 10.594 | 40.086 | 1.00 | 60.42 |
| 2328 | OH | TYR | 548 | 19.467 | 9.877 | 38.925 | 1.00 | 60.60 |
| 2329 | C | TYR | 548 | 17.979 | 11.519 | 45.080 | 1.00 | 60.53 |
| 2330 | O | TYR | 548 | 18.584 | 11.272 | 46.114 | 1.00 | 61.95 |
| 2331 | N | ASP | 549 | 17.227 | 10.603 | 44.480 | 1.00 | 60.31 |
| 2332 | CA | ASP | 549 | 17.135 | 9.281 | 45.088 | 1.00 | 60.89 |
| 2333 | CB | ASP | 549 | 16.206 | 8.359 | 44.317 | 1.00 | 61.64 |
| 2334 | CG | ASP | 549 | 15.653 | 7.256 | 45.196 | 1.00 | 62.87 |
| 2335 | OD1 | ASP | 549 | 16.437 | 6.715 | 45.997 | 1.00 | 61.92 |
| 2336 | OD2 | ASP | 549 | 14.446 | 6.929 | 45.100 | 1.00 | 58.12 |
| 2337 | C | ASP | 549 | 18.525 | 8.656 | 45.152 | 1.00 | 62.05 |
| 2338 | O | ASP | 549 | 19.176 | 8.728 | 46.190 | 1.00 | 59.43 |
| 2339 | N | SER | 550 | 18.977 | 8.052 | 44.049 | 1.00 | 61.36 |
| 2340 | CA | SER | 550 | 20.312 | 7.425 | 43.963 | 1.00 | 61.16 |
| 2341 | CB | SER | 550 | 21.301 | 8.127 | 44.910 | 1.00 | 61.33 |
| 2342 | OG | SER | 550 | 22.637 | 8.055 | 44.435 | 1.00 | 65.58 |
| 2343 | C | SER | 550 | 20.286 | 5.923 | 44.268 | 1.00 | 62.72 |
| 2344 | O | SER | 550 | 21.025 | 5.138 | 43.662 | 1.00 | 61.37 |
| 2345 | N | SER | 551 | 19.422 | 5.543 | 45.206 | 1.00 | 60.55 |
| 2346 | CA | SER | 551 | 19.262 | 4.155 | 45.623 | 1.00 | 62.47 |
| 2347 | CB | SER | 551 | 18.461 | 4.092 | 46.927 | 1.00 | 59.78 |
| 2348 | OG | SER | 551 | 17.138 | 4.551 | 46.727 | 1.00 | 65.75 |
| 2349 | C | SER | 551 | 18.548 | 3.348 | 44.544 | 1.00 | 61.26 |
| 2350 | O | SER | 551 | 18.187 | 2.189 | 44.749 | 1.00 | 64.05 |
| 2351 | N | VAL | 552 | 18.349 | 3.976 | 43.394 | 1.00 | 61.88 |
| 2352 | CA | VAL | 552 | 17.683 | 3.338 | 42.268 | 1.00 | 61.07 |
| 2353 | CB | VAL | 552 | 16.146 | 3.536 | 42.346 | 1.00 | 61.01 |
| 2354 | CG1 | VAL | 552 | 15.781 | 4.362 | 43.582 | 1.00 | 62.13 |
| 2355 | CG2 | VAL | 552 | 15.642 | 4.194 | 41.084 | 1.00 | 63.59 |
| 2356 | C | VAL | 552 | 18.245 | 3.935 | 40.975 | 1.00 | 60.74 |
| 2357 | O | VAL | 552 | 18.423 | 5.151 | 40.872 | 1.00 | 60.94 |
| 2358 | N | PRO | 553 | 18.502 | 3.085 | 39.966 | 1.00 | 57.88 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2359 | CD | PRO | 553 | 17.755 | 1.824 | 39.869 | 1.00 | 60.27 |
| 2360 | CA | PRO | 553 | 19.058 | 3.418 | 38.648 | 1.00 | 60.29 |
| 2361 | CB | PRO | 553 | 18.407 | 2.387 | 37.718 | 1.00 | 59.38 |
| 2362 | CG | PRO | 553 | 17.208 | 1.918 | 38.478 | 1.00 | 59.68 |
| 2363 | C | PRO | 553 | 18.933 | 4.837 | 38.105 | 1.00 | 61.75 |
| 2364 | O | PRO | 553 | 17.864 | 5.452 | 38.132 | 1.00 | 60.81 |
| 2365 | N | ASP | 554 | 20.055 | 5.352 | 37.616 | 1.00 | 59.48 |
| 2366 | CA | ASP | 554 | 20.061 | 6.676 | 37.032 | 1.00 | 59.15 |
| 2367 | CB | ASP | 554 | 21.477 | 7.212 | 36.819 | 1.00 | 62.17 |
| 2368 | CG | ASP | 554 | 22.222 | 7.452 | 38.101 | 1.00 | 62.93 |
| 2369 | OD1 | ASP | 554 | 21.591 | 7.655 | 39.164 | 1.00 | 60.70 |
| 2370 | OD2 | ASP | 554 | 23.467 | 7.455 | 38.017 | 1.00 | 60.19 |
| 2371 | C | ASP | 554 | 19.433 | 6.505 | 35.667 | 1.00 | 61.49 |
| 2372 | O | ASP | 554 | 18.898 | 5.446 | 35.354 | 1.00 | 59.51 |
| 2373 | N | SER | 555 | 19.536 | 7.557 | 34.859 | 1.00 | 60.31 |
| 2374 | CA | SER | 555 | 19.023 | 7.601 | 33.492 | 1.00 | 60.74 |
| 2375 | CB | SER | 555 | 17.576 | 7.113 | 33.419 | 1.00 | 64.15 |
| 2376 | OG | SER | 555 | 16.687 | 8.099 | 33.896 | 1.00 | 60.71 |
| 2377 | C | SER | 555 | 19.092 | 9.069 | 33.108 | 1.00 | 61.31 |
| 2378 | O | SER | 555 | 18.776 | 9.929 | 33.927 | 1.00 | 59.93 |
| 2379 | N | THR | 556 | 19.525 | 9.358 | 31.883 | 1.00 | 62.16 |
| 2380 | CA | THR | 556 | 19.636 | 10.742 | 31.434 | 1.00 | 61.52 |
| 2381 | CB | THR | 556 | 19.673 | 10.857 | 29.895 | 1.00 | 59.01 |
| 2382 | OG1 | THR | 556 | 20.850 | 10.212 | 29.391 | 1.00 | 60.60 |
| 2383 | CG2 | THR | 556 | 19.677 | 12.330 | 29.475 | 1.00 | 60.87 |
| 2384 | C | THR | 556 | 18.422 | 11.505 | 31.913 | 1.00 | 61.95 |
| 2385 | O | THR | 556 | 18.517 | 12.377 | 32.788 | 1.00 | 60.46 |
| 2386 | N | TRP | 557 | 17.285 | 11.145 | 31.318 | 1.00 | 60.92 |
| 2387 | CA | TRP | 557 | 15.986 | 11.734 | 31.611 | 1.00 | 63.76 |
| 2388 | CB | TRP | 557 | 14.864 | 10.739 | 31.251 | 1.00 | 61.57 |
| 2389 | CG | TRP | 557 | 13.719 | 10.807 | 32.225 | 1.00 | 57.77 |
| 2390 | CD2 | TRP | 557 | 12.895 | 11.949 | 32.491 | 1.00 | 60.28 |
| 2391 | CE2 | TRP | 557 | 12.066 | 11.627 | 33.595 | 1.00 | 61.10 |
| 2392 | CE3 | TRP | 557 | 12.785 | 13.219 | 31.911 | 1.00 | 57.69 |
| 2393 | CD1 | TRP | 557 | 13.357 | 9.855 | 33.145 | 1.00 | 63.49 |
| 2394 | NE1 | TRP | 557 | 12.369 | 10.344 | 33.974 | 1.00 | 60.45 |
| 2395 | CZ2 | TRP | 557 | 11.132 | 12.535 | 34.132 | 1.00 | 61.64 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2396 | CZ3 | TRP | 557 | 11.857 | 14.124 | 32.452 | 1.00 | 61.15 |
| 2397 | CH2 | TRP | 557 | 11.045 | 13.773 | 33.548 | 1.00 | 61.06 |
| 2398 | C | TRP | 557 | 15.816 | 12.176 | 33.063 | 1.00 | 61.03 |
| 2399 | O | TRP | 557 | 15.546 | 13.342 | 33.335 | 1.00 | 63.66 |
| 2400 | N | ARG | 558 | 15.972 | 11.228 | 33.982 | 1.00 | 62.37 |
| 2401 | CA | ARG | 558 | 15.798 | 11.466 | 35.413 | 1.00 | 63.98 |
| 2402 | CB | ARG | 558 | 15.806 | 10.116 | 36.134 | 1.00 | 61.30 |
| 2403 | CG | ARG | 558 | 15.389 | 10.127 | 37.590 | 1.00 | 62.22 |
| 2404 | CD | ARG | 558 | 15.189 | 8.686 | 38.070 | 1.00 | 59.18 |
| 2405 | NE | ARG | 558 | 16.210 | 8.204 | 39.005 | 1.00 | 60.75 |
| 2406 | CZ | ARG | 558 | 16.280 | 8.542 | 40.294 | 1.00 | 62.74 |
| 2407 | NH1 | ARG | 558 | 15.392 | 9.378 | 40.820 | 1.00 | 65.45 |
| 2408 | NH2 | ARG | 558 | 17.222 | 8.022 | 41.074 | 1.00 | 61.39 |
| 2409 | C | ARG | 558 | 16.814 | 12.416 | 36.056 | 1.00 | 63.08 |
| 2410 | O | ARG | 558 | 16.524 | 13.027 | 37.085 | 1.00 | 62.80 |
| 2411 | N | ILE | 559 | 17.991 | 12.548 | 35.451 | 1.00 | 63.40 |
| 2412 | CA | ILE | 559 | 19.036 | 13.423 | 35.983 | 1.00 | 59.67 |
| 2413 | CB | ILE | 559 | 20.459 | 12.828 | 35.701 | 1.00 | 59.65 |
| 2414 | CG2 | ILE | 559 | 21.437 | 13.905 | 35.250 | 1.00 | 56.03 |
| 2415 | CG1 | ILE | 559 | 20.982 | 12.150 | 36.968 | 1.00 | 58.33 |
| 2416 | CD1 | ILE | 559 | 22.212 | 11.327 | 36.744 | 1.00 | 61.31 |
| 2417 | C | ILE | 559 | 18.939 | 14.862 | 35.469 | 1.00 | 64.14 |
| 2418 | O | ILE | 559 | 18.843 | 15.792 | 36.281 | 1.00 | 63.80 |
| 2419 | N | MET | 560 | 18.964 | 15.060 | 34.151 | 1.00 | 58.15 |
| 2420 | CA | MET | 560 | 18.871 | 16.419 | 33.646 | 1.00 | 61.08 |
| 2421 | CB | MET | 560 | 18.753 | 16.460 | 32.095 | 1.00 | 60.65 |
| 2422 | CG | MET | 560 | 20.117 | 16.352 | 31.322 | 1.00 | 63.74 |
| 2423 | SD | MET | 560 | 20.038 | 16.357 | 29.422 | 1.00 | 63.49 |
| 2424 | CE | MET | 560 | 21.750 | 16.939 | 28.970 | 1.00 | 61.12 |
| 2425 | C | MET | 560 | 17.634 | 17.014 | 34.325 | 1.00 | 63.02 |
| 2426 | O | MET | 560 | 17.666 | 18.156 | 34.780 | 1.00 | 60.37 |
| 2427 | N | THR | 561 | 16.572 | 16.217 | 34.457 | 1.00 | 60.33 |
| 2428 | CA | THR | 561 | 15.351 | 16.677 | 35.123 | 1.00 | 60.71 |
| 2429 | CB | THR | 561 | 14.306 | 15.540 | 35.292 | 1.00 | 58.90 |
| 2430 | OG1 | THR | 561 | 14.006 | 14.959 | 34.025 | 1.00 | 60.27 |
| 2431 | CG2 | THR | 561 | 13.019 | 16.080 | 35.867 | 1.00 | 58.67 |
| 2432 | C | THR | 561 | 15.616 | 17.259 | 36.520 | 1.00 | 60.71 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2433 | O | THR | 561 | 15.390 | 18.429 | 36.747 | 1.00 | 62.74 |
| 2434 | N | THR | 562 | 16.111 | 16.434 | 37.451 | 1.00 | 61.67 |
| 2435 | CA | THR | 562 | 16.387 | 16.821 | 38.860 | 1.00 | 62.60 |
| 2436 | CB | THR | 562 | 16.914 | 15.721 | 39.677 | 1.00 | 60.58 |
| 2437 | OG1 | THR | 562 | 18.152 | 15.292 | 39.081 | 1.00 | 59.96 |
| 2438 | CG2 | THR | 562 | 15.938 | 14.616 | 39.802 | 1.00 | 62.71 |
| 2439 | C | THR | 562 | 17.487 | 17.786 | 39.098 | 1.00 | 60.58 |
| 2440 | O | THR | 562 | 17.924 | 18.032 | 40.229 | 1.00 | 63.25 |
| 2441 | N | LEU | 563 | 17.993 | 18.325 | 38.047 | 1.00 | 60.71 |
| 2442 | CA | LEU | 563 | 19.148 | 19.110 | 38.123 | 1.00 | 61.15 |
| 2443 | CB | LEU | 563 | 20.009 | 18.485 | 37.164 | 1.00 | 61.40 |
| 2444 | CG | LEU | 563 | 21.445 | 18.268 | 37.247 | 1.00 | 60.76 |
| 2445 | CD1 | LEU | 563 | 21.882 | 17.094 | 38.118 | 1.00 | 59.56 |
| 2446 | CD2 | LEU | 563 | 21.705 | 18.012 | 35.810 | 1.00 | 62.29 |
| 2447 | C | LEU | 563 | 18.542 | 20.350 | 37.631 | 1.00 | 60.18 |
| 2448 | O | LEU | 563 | 19.115 | 21.393 | 37.453 | 1.00 | 62.51 |
| 2449 | N | ASN | 564 | 17.281 | 20.194 | 37.278 | 1.00 | 62.12 |
| 2450 | CA | ASN | 564 | 16.596 | 21.312 | 36.741 | 1.00 | 63.12 |
| 2451 | CB | ASN | 564 | 15.703 | 20.918 | 35.651 | 1.00 | 63.80 |
| 2452 | CG | ASN | 564 | 16.263 | 21.124 | 34.284 | 1.00 | 60.00 |
| 2453 | OD1 | ASN | 564 | 17.269 | 21.801 | 34.085 | 1.00 | 59.72 |
| 2454 | ND2 | ASN | 564 | 15.577 | 20.559 | 33.311 | 1.00 | 61.08 |
| 2455 | C | ASN | 564 | 15.740 | 21.719 | 37.867 | 1.00 | 61.56 |
| 2456 | O | ASN | 564 | 15.204 | 22.818 | 37.863 | 1.00 | 61.14 |
| 2457 | N | MET | 565 | 15.483 | 20.798 | 38.768 | 1.00 | 63.63 |
| 2458 | CA | MET | 565 | 14.643 | 21.083 | 39.872 | 1.00 | 60.96 |
| 2459 | CB | MET | 565 | 14.164 | 19.773 | 40.504 | 1.00 | 61.63 |
| 2460 | CG | MET | 565 | 12.686 | 19.546 | 40.369 | 1.00 | 65.53 |
| 2461 | SD | MET | 565 | 12.013 | 20.320 | 38.903 | 1.00 | 61.09 |
| 2462 | CE | MET | 565 | 10.337 | 20.392 | 39.357 | 1.00 | 60.22 |
| 2463 | C | MET | 565 | 15.560 | 21.814 | 40.802 | 1.00 | 62.76 |
| 2464 | O | MET | 565 | 15.162 | 22.713 | 41.539 | 1.00 | 60.13 |
| 2465 | N | LEU | 566 | 16.826 | 21.447 | 40.746 | 1.00 | 62.63 |
| 2466 | CA | LEU | 566 | 17.794 | 22.101 | 41.590 | 1.00 | 58.73 |
| 2467 | CB | LEU | 566 | 19.007 | 21.188 | 41.764 | 1.00 | 59.94 |
| 2468 | CG | LEU | 566 | 20.381 | 21.435 | 42.410 | 1.00 | 60.09 |
| 2469 | CD1 | LEU | 566 | 21.236 | 21.209 | 41.243 | 1.00 | 60.52 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2470 | CD2 | LEU | 566 | 20.687 | 22.805 | 43.045 | 1.00 | 59.08 |
| 2471 | C | LEU | 566 | 18.142 | 23.418 | 40.952 | 1.00 | 61.33 |
| 2472 | O | LEU | 566 | 18.373 | 24.398 | 41.652 | 1.00 | 61.14 |
| 2473 | N | GLY | 567 | 18.140 | 23.461 | 39.625 | 1.00 | 57.88 |
| 2474 | CA | GLY | 567 | 18.436 | 24.703 | 38.935 | 1.00 | 63.27 |
| 2475 | C | GLY | 567 | 17.469 | 25.785 | 39.351 | 1.00 | 60.49 |
| 2476 | O | GLY | 567 | 17.854 | 26.938 | 39.514 | 1.00 | 61.62 |
| 2477 | N | GLY | 568 | 16.206 | 25.412 | 39.526 | 1.00 | 63.72 |
| 2478 | CA | GLY | 568 | 15.212 | 26.382 | 39.936 | 1.00 | 60.34 |
| 2479 | C | GLY | 568 | 15.617 | 27.006 | 41.256 | 1.00 | 60.96 |
| 2480 | O | GLY | 568 | 15.913 | 28.195 | 41.332 | 1.00 | 61.39 |
| 2481 | N | ARG | 569 | 15.662 | 26.196 | 42.302 | 1.00 | 59.63 |
| 2482 | CA | ARG | 569 | 16.011 | 26.692 | 43.623 | 1.00 | 58.74 |
| 2483 | CB | ARG | 569 | 16.143 | 25.530 | 44.588 | 1.00 | 62.58 |
| 2484 | CG | ARG | 569 | 14.860 | 24.767 | 44.719 | 1.00 | 61.89 |
| 2485 | CD | ARG | 569 | 14.973 | 23.794 | 45.842 | 1.00 | 60.95 |
| 2486 | NE | ARG | 569 | 16.047 | 22.854 | 45.573 | 1.00 | 60.03 |
| 2487 | CZ | ARG | 569 | 16.734 | 22.229 | 46.513 | 1.00 | 60.68 |
| 2488 | NH1 | ARG | 569 | 16.459 | 22.447 | 47.793 | 1.00 | 65.06 |
| 2489 | NH2 | ARG | 569 | 17.697 | 21.391 | 46.169 | 1.00 | 63.36 |
| 2490 | C | ARG | 569 | 17.261 | 27.538 | 43.671 | 1.00 | 59.59 |
| 2491 | O | ARG | 569 | 17.395 | 28.397 | 44.539 | 1.00 | 58.49 |
| 2492 | N | GLN | 570 | 18.179 | 27.299 | 42.747 | 1.00 | 60.12 |
| 2493 | CA | GLN | 570 | 19.417 | 28.069 | 42.704 | 1.00 | 62.83 |
| 2494 | CB | GLN | 570 | 20.457 | 27.359 | 41.852 | 1.00 | 64.23 |
| 2495 | CG | GLN | 570 | 21.212 | 26.254 | 42.529 | 1.00 | 60.55 |
| 2496 | CD | GLN | 570 | 22.345 | 25.738 | 41.674 | 1.00 | 61.81 |
| 2497 | OE1 | GLN | 570 | 23.046 | 24.818 | 42.067 | 1.00 | 60.88 |
| 2498 | NE2 | GLN | 570 | 22.533 | 26.331 | 40.499 | 1.00 | 56.12 |
| 2499 | C | GLN | 570 | 19.195 | 29.462 | 42.135 | 1.00 | 59.62 |
| 2500 | O | GLN | 570 | 19.872 | 30.409 | 42.529 | 1.00 | 60.45 |
| 2501 | N | VAL | 571 | 18.273 | 29.571 | 41.182 | 1.00 | 64.99 |
| 2502 | CA | VAL | 571 | 17.953 | 30.851 | 40.576 | 1.00 | 62.98 |
| 2503 | CB | VAL | 571 | 17.141 | 30.653 | 39.278 | 1.00 | 62.20 |
| 2504 | CG1 | VAL | 571 | 16.363 | 31.906 | 38.929 | 1.00 | 61.93 |
| 2505 | CG2 | VAL | 571 | 18.090 | 30.312 | 38.140 | 1.00 | 61.13 |
| 2506 | C | VAL | 571 | 17.166 | 31.658 | 41.605 | 1.00 | 63.93 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2507 | O | VAL | 571 | 17.379 | 32.865 | 41.763 | 1.00 | 65.46 |
| 2508 | N | ILE | 572 | 16.271 | 30.981 | 42.317 | 1.00 | 61.03 |
| 2509 | CA | ILE | 572 | 15.483 | 31.623 | 43.360 | 1.00 | 58.70 |
| 2510 | CB | ILE | 572 | 14.548 | 30.605 | 44.045 | 1.00 | 66.72 |
| 2511 | CG2 | ILE | 572 | 14.006 | 31.169 | 45.350 | 1.00 | 58.22 |
| 2512 | CG1 | ILE | 572 | 13.425 | 30.220 | 43.081 | 1.00 | 61.85 |
| 2513 | CD1 | ILE | 572 | 12.411 | 29.251 | 43.663 | 1.00 | 61.02 |
| 2514 | C | ILE | 572 | 16.456 | 32.186 | 44.390 | 1.00 | 59.43 |
| 2515 | O | ILE | 572 | 16.240 | 33.257 | 44.948 | 1.00 | 61.61 |
| 2516 | N | ALA | 573 | 17.531 | 31.446 | 44.628 | 1.00 | 62.51 |
| 2517 | CA | ALA | 573 | 18.562 | 31.844 | 45.571 | 1.00 | 63.19 |
| 2518 | CB | ALA | 573 | 19.467 | 30.662 | 45.875 | 1.00 | 59.65 |
| 2519 | C | ALA | 573 | 19.390 | 32.994 | 45.016 | 1.00 | 60.08 |
| 2520 | O | ALA | 573 | 19.853 | 33.852 | 45.765 | 1.00 | 61.27 |
| 2521 | N | ALA | 574 | 19.573 | 33.004 | 43.700 | 1.00 | 62.86 |
| 2522 | CA | ALA | 574 | 20.350 | 34.039 | 43.027 | 1.00 | 62.91 |
| 2523 | CB | ALA | 574 | 20.500 | 33.690 | 41.553 | 1.00 | 60.40 |
| 2524 | C | ALA | 574 | 19.729 | 35.426 | 43.176 | 1.00 | 61.54 |
| 2525 | O | ALA | 574 | 20.402 | 36.441 | 42.977 | 1.00 | 62.14 |
| 2526 | N | VAL | 575 | 18.447 | 35.461 | 43.535 | 1.00 | 63.11 |
| 2527 | CA | VAL | 575 | 17.721 | 36.716 | 43.708 | 1.00 | 61.22 |
| 2528 | CB | VAL | 575 | 16.214 | 36.502 | 43.529 | 1.00 | 61.11 |
| 2529 | CG1 | VAL | 575 | 15.500 | 37.835 | 43.568 | 1.00 | 63.45 |
| 2530 | CG2 | VAL | 575 | 15.950 | 35.795 | 42.218 | 1.00 | 55.96 |
| 2531 | C | VAL | 575 | 17.970 | 37.386 | 45.063 | 1.00 | 61.67 |
| 2532 | O | VAL | 575 | 18.242 | 38.589 | 45.119 | 1.00 | 61.16 |
| 2533 | N | LYS | 576 | 17.866 | 36.618 | 46.148 | 1.00 | 63.44 |
| 2534 | CA | LYS | 576 | 18.103 | 37.169 | 47.477 | 1.00 | 61.90 |
| 2535 | CB | LYS | 576 | 17.913 | 36.120 | 48.576 | 1.00 | 60.65 |
| 2536 | CG | LYS | 576 | 16.569 | 35.385 | 48.633 | 1.00 | 62.95 |
| 2537 | CD | LYS | 576 | 16.370 | 34.812 | 50.045 | 1.00 | 61.64 |
| 2538 | CE | LYS | 576 | 15.453 | 33.594 | 50.101 | 1.00 | 61.27 |
| 2539 | NZ | LYS | 576 | 16.134 | 32.288 | 49.785 | 1.00 | 60.24 |
| 2540 | C | LYS | 576 | 19.554 | 37.616 | 47.498 | 1.00 | 62.75 |
| 2541 | O | LYS | 576 | 19.966 | 38.384 | 48.367 | 1.00 | 59.51 |
| 2542 | N | TRP | 577 | 20.320 | 37.103 | 46.534 | 1.00 | 64.04 |
| 2543 | CA | TRP | 577 | 21.741 | 37.407 | 46.382 | 1.00 | 63.24 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2544 | CB | TRP | 577 | 22.447 | 36.277 | 45.610 | 1.00 | 61.04 |
| 2545 | CG | TRP | 577 | 23.852 | 36.613 | 45.166 | 1.00 | 62.92 |
| 2546 | CD2 | TRP | 577 | 24.285 | 36.919 | 43.828 | 1.00 | 59.92 |
| 2547 | CE2 | TRP | 577 | 25.663 | 37.212 | 43.892 | 1.00 | 60.22 |
| 2548 | CE3 | TRP | 577 | 23.637 | 36.978 | 42.584 | 1.00 | 61.13 |
| 2549 | CD1 | TRP | 577 | 24.956 | 36.729 | 45.958 | 1.00 | 63.10 |
| 2550 | NE1 | TRP | 577 | 26.045 | 37.088 | 45.201 | 1.00 | 58.91 |
| 2551 | CZ2 | TRP | 577 | 26.411 | 37.557 | 42.763 | 1.00 | 58.75 |
| 2552 | CZ3 | TRP | 577 | 24.378 | 37.325 | 41.461 | 1.00 | 63.87 |
| 2553 | CH2 | TRP | 577 | 25.754 | 37.611 | 41.559 | 1.00 | 60.61 |
| 2554 | C | TRP | 577 | 21.923 | 38.719 | 45.634 | 1.00 | 60.28 |
| 2555 | O | TRP | 577 | 22.654 | 39.597 | 46.083 | 1.00 | 61.57 |
| 2556 | N | ALA | 578 | 21.251 | 38.837 | 44.490 | 1.00 | 60.75 |
| 2557 | CA | ALA | 578 | 21.338 | 40.032 | 43.661 | 1.00 | 62.79 |
| 2558 | CB | ALA | 578 | 20.522 | 39.847 | 42.395 | 1.00 | 63.55 |
| 2559 | C | ALA | 578 | 20.869 | 41.274 | 44.409 | 1.00 | 60.84 |
| 2560 | O | ALA | 578 | 21.347 | 42.370 | 44.156 | 1.00 | 60.70 |
| 2561 | N | LYS | 579 | 19.937 | 41.105 | 45.339 | 1.00 | 62.74 |
| 2562 | CA | LYS | 579 | 19.423 | 42.234 | 46.107 | 1.00 | 60.13 |
| 2563 | CB | LYS | 579 | 18.016 | 41.900 | 46.640 | 1.00 | 60.85 |
| 2564 | CG | LYS | 579 | 16.969 | 41.709 | 45.532 | 1.00 | 61.81 |
| 2565 | CD | LYS | 579 | 15.725 | 40.942 | 45.986 | 1.00 | 61.28 |
| 2566 | CE | LYS | 579 | 14.910 | 41.704 | 47.020 | 1.00 | 61.64 |
| 2567 | NZ | LYS | 579 | 13.708 | 40.953 | 47.492 | 1.00 | 62.20 |
| 2568 | C | LYS | 579 | 20.372 | 42.613 | 47.252 | 1.00 | 60.67 |
| 2569 | O | LYS | 579 | 20.251 | 43.683 | 47.835 | 1.00 | 59.96 |
| 2570 | N | ALA | 580 | 21.322 | 41.738 | 47.564 | 1.00 | 59.98 |
| 2571 | CA | ALA | 580 | 22.288 | 42.011 | 48.630 | 1.00 | 60.27 |
| 2572 | CB | ALA | 580 | 22.721 | 40.713 | 49.304 | 1.00 | 64.73 |
| 2573 | C | ALA | 580 | 23.505 | 42.722 | 48.059 | 1.00 | 58.96 |
| 2574 | O | ALA | 580 | 24.349 | 43.229 | 48.801 | 1.00 | 61.59 |
| 2575 | N | ILE | 581 | 23.590 | 42.738 | 46.731 | 1.00 | 63.39 |
| 2576 | CA | ILE | 581 | 24.690 | 43.380 | 46.030 | 1.00 | 60.52 |
| 2577 | CB | ILE | 581 | 24.789 | 42.908 | 44.559 | 1.00 | 60.74 |
| 2578 | CG2 | ILE | 581 | 25.911 | 43.650 | 43.840 | 1.00 | 63.37 |
| 2579 | CG1 | ILE | 581 | 25.069 | 41.409 | 44.494 | 1.00 | 60.26 |
| 2580 | CD1 | ILE | 581 | 24.930 | 40.862 | 43.091 | 1.00 | 61.59 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2581 | C | ILE | 581 | 24.426 | 44.873 | 46.025 | 1.00 | 63.62 |
| 2582 | O | ILE | 581 | 23.418 | 45.329 | 45.482 | 1.00 | 63.59 |
| 2583 | N | PRO | 582 | 25.329 | 45.655 | 46.634 | 1.00 | 62.76 |
| 2584 | CD | PRO | 582 | 26.596 | 45.257 | 47.270 | 1.00 | 63.28 |
| 2585 | CA | PRO | 582 | 25.162 | 47.104 | 46.681 | 1.00 | 64.52 |
| 2586 | CB | PRO | 582 | 26.505 | 47.589 | 47.226 | 1.00 | 62.45 |
| 2587 | CG | PRO | 582 | 26.934 | 46.473 | 48.106 | 1.00 | 59.21 |
| 2588 | C | PRO | 582 | 24.882 | 47.654 | 45.298 | 1.00 | 62.22 |
| 2589 | O | PRO | 582 | 25.518 | 47.252 | 44.323 | 1.00 | 61.69 |
| 2590 | N | GLY | 583 | 23.913 | 48.560 | 45.224 | 1.00 | 58.26 |
| 2591 | CA | GLY | 583 | 23.565 | 49.189 | 43.965 | 1.00 | 59.81 |
| 2592 | C | GLY | 583 | 22.640 | 48.446 | 43.028 | 1.00 | 61.92 |
| 2593 | O | GLY | 583 | 22.231 | 49.002 | 42.024 | 1.00 | 58.88 |
| 2594 | N | PHE | 584 | 22.302 | 47.201 | 43.327 | 1.00 | 62.92 |
| 2595 | CA | PHE | 584 | 21.418 | 46.459 | 42.438 | 1.00 | 61.83 |
| 2596 | CB | PHE | 584 | 21.563 | 44.953 | 42.677 | 1.00 | 62.46 |
| 2597 | CG | PHE | 584 | 20.863 | 44.104 | 41.650 | 1.00 | 57.96 |
| 2598 | CD1 | PHE | 584 | 21.406 | 43.921 | 40.390 | 1.00 | 63.14 |
| 2599 | CD2 | PHE | 584 | 19.646 | 43.514 | 41.938 | 1.00 | 62.84 |
| 2600 | CE1 | PHE | 584 | 20.746 | 43.166 | 39.437 | 1.00 | 61.42 |
| 2601 | CE2 | PHE | 584 | 18.980 | 42.759 | 40.991 | 1.00 | 60.47 |
| 2602 | CZ | PHE | 584 | 19.533 | 42.585 | 39.737 | 1.00 | 59.28 |
| 2603 | C | PHE | 584 | 19.958 | 46.883 | 42.624 | 1.00 | 60.13 |
| 2604 | O | PHE | 584 | 19.210 | 46.996 | 41.653 | 1.00 | 61.49 |
| 2605 | N | ARG | 585 | 19.561 | 47.131 | 43.870 | 1.00 | 60.78 |
| 2606 | CA | ARG | 585 | 18.190 | 47.535 | 44.160 | 1.00 | 63.10 |
| 2607 | CB | ARG | 585 | 17.832 | 47.252 | 45.627 | 1.00 | 59.03 |
| 2608 | CG | ARG | 585 | 17.716 | 45.757 | 45.943 | 1.00 | 57.77 |
| 2609 | CD | ARG | 585 | 17.222 | 45.476 | 47.365 | 1.00 | 61.69 |
| 2610 | NE | ARG | 585 | 15.825 | 45.858 | 47.587 | 1.00 | 62.10 |
| 2611 | CZ | ARG | 585 | 14.800 | 45.492 | 46.817 | 1.00 | 62.77 |
| 2612 | NH1 | ARG | 585 | 14.996 | 44.724 | 45.744 | 1.00 | 60.63 |
| 2613 | NH2 | ARG | 585 | 13.569 | 45.893 | 47.126 | 1.00 | 59.26 |
| 2614 | C | ARG | 585 | 17.952 | 48.996 | 43.843 | 1.00 | 59.85 |
| 2615 | O | ARG | 585 | 16.833 | 49.487 | 43.965 | 1.00 | 61.84 |
| 2616 | N | ASN | 586 | 19.003 | 49.689 | 43.424 | 1.00 | 60.92 |
| 2617 | CA | ASN | 586 | 18.871 | 51.094 | 43.090 | 1.00 | 62.56 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2618 | CB | ASN | 586 | 19.987 | 51.892 | 43.740 | 1.00 | 59.81 |
| 2619 | CG | ASN | 586 | 20.079 | 51.620 | 45.218 | 1.00 | 62.07 |
| 2620 | OD1 | ASN | 586 | 20.714 | 50.651 | 45.641 | 1.00 | 62.28 |
| 2621 | ND2 | ASN | 586 | 19.415 | 52.452 | 46.018 | 1.00 | 56.67 |
| 2622 | C | ASN | 586 | 18.865 | 51.271 | 41.592 | 1.00 | 63.34 |
| 2623 | O | ASN | 586 | 19.054 | 52.363 | 41.068 | 1.00 | 60.96 |
| 2624 | N | LEU | 587 | 18.648 | 50.163 | 40.907 | 1.00 | 62.25 |
| 2625 | CA | LEU | 587 | 18.551 | 50.166 | 39.467 | 1.00 | 60.33 |
| 2626 | CB | LEU | 587 | 19.304 | 48.971 | 38.887 | 1.00 | 63.97 |
| 2627 | CG | LEU | 587 | 20.823 | 49.075 | 38.847 | 1.00 | 59.84 |
| 2628 | CD1 | LEU | 587 | 21.410 | 47.701 | 38.948 | 1.00 | 62.41 |
| 2629 | CD2 | LEU | 587 | 21.262 | 49.748 | 37.572 | 1.00 | 64.48 |
| 2630 | C | LEU | 587 | 17.053 | 50.008 | 39.259 | 1.00 | 61.19 |
| 2631 | O | LEU | 587 | 16.355 | 49.541 | 40.164 | 1.00 | 61.87 |
| 2632 | N | HIS | 588 | 16.556 | 50.400 | 38.090 | 1.00 | 63.05 |
| 2633 | CA | HIS | 588 | 15.130 | 50.288 | 37.829 | 1.00 | 58.93 |
| 2634 | CB | HIS | 588 | 14.797 | 50.621 | 36.371 | 1.00 | 62.93 |
| 2635 | CG | HIS | 588 | 13.338 | 50.871 | 36.131 | 1.00 | 61.44 |
| 2636 | CD2 | HIS | 588 | 12.679 | 51.990 | 35.745 | 1.00 | 61.68 |
| 2637 | ND1 | HIS | 588 | 12.369 | 49.912 | 36.344 | 1.00 | 59.82 |
| 2638 | CE1 | HIS | 588 | 11.178 | 50.431 | 36.101 | 1.00 | 59.25 |
| 2639 | NE2 | HIS | 588 | 11.339 | 51.691 | 35.736 | 1.00 | 58.14 |
| 2640 | C | HIS | 588 | 14.723 | 48.860 | 38.128 | 1.00 | 61.41 |
| 2641 | O | HIS | 588 | 15.515 | 47.942 | 37.948 | 1.00 | 59.96 |
| 2642 | N | LEU | 589 | 13.492 | 48.686 | 38.598 | 1.00 | 59.88 |
| 2643 | CA | LEU | 589 | 12.974 | 47.370 | 38.929 | 1.00 | 61.54 |
| 2644 | CB | LEU | 589 | 11.602 | 47.509 | 39.599 | 1.00 | 60.80 |
| 2645 | CG | LEU | 589 | 10.980 | 46.337 | 40.367 | 1.00 | 62.36 |
| 2646 | CD1 | LEU | 589 | 10.643 | 45.192 | 39.424 | 1.00 | 59.67 |
| 2647 | CD2 | LEU | 589 | 11.934 | 45.887 | 41.449 | 1.00 | 61.30 |
| 2648 | C | LEU | 589 | 12.867 | 46.562 | 37.640 | 1.00 | 60.73 |
| 2649 | O | LEU | 589 | 12.841 | 45.332 | 37.667 | 1.00 | 59.26 |
| 2650 | N | ASP | 590 | 12.811 | 47.254 | 36.507 | 1.00 | 62.77 |
| 2651 | CA | ASP | 590 | 12.714 | 46.574 | 35.222 | 1.00 | 59.61 |
| 2652 | CB | ASP | 590 | 12.172 | 47.516 | 34.154 | 1.00 | 60.63 |
| 2653 | CG | ASP | 590 | 10.676 | 47.476 | 34.060 | 1.00 | 60.64 |
| 2654 | OD1 | ASP | 590 | 10.031 | 47.099 | 35.060 | 1.00 | 66.03 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2655 | OD2 | ASP | 590 | 10.140 | 47.830 | 32.989 | 1.00 | 62.74 |
| 2656 | C | ASP | 590 | 14.077 | 46.079 | 34.801 | 1.00 | 64.03 |
| 2657 | O | ASP | 590 | 14.194 | 45.131 | 34.020 | 1.00 | 61.02 |
| 2658 | N | ASP | 591 | 15.109 | 46.734 | 35.319 | 1.00 | 61.76 |
| 2659 | CA | ASP | 591 | 16.481 | 46.364 | 34.993 | 1.00 | 61.72 |
| 2660 | CB | ASP | 591 | 17.425 | 47.557 | 35.224 | 1.00 | 60.28 |
| 2661 | CG | ASP | 591 | 17.174 | 48.709 | 34.250 | 1.00 | 66.48 |
| 2662 | OD1 | ASP | 591 | 16.782 | 48.445 | 33.092 | 1.00 | 56.28 |
| 2663 | OD2 | ASP | 591 | 17.393 | 49.877 | 34.639 | 1.00 | 63.43 |
| 2664 | C | ASP | 591 | 16.937 | 45.160 | 35.813 | 1.00 | 60.81 |
| 2665 | O | ASP | 591 | 17.642 | 44.292 | 35.306 | 1.00 | 61.99 |
| 2666 | N | GLN | 592 | 16.515 | 45.120 | 37.075 | 1.00 | 59.60 |
| 2667 | CA | GLN | 592 | 16.852 | 44.035 | 37.981 | 1.00 | 59.54 |
| 2668 | CB | GLN | 592 | 16.145 | 44.209 | 39.327 | 1.00 | 60.92 |
| 2669 | CG | GLN | 592 | 16.268 | 45.571 | 39.962 | 1.00 | 63.19 |
| 2670 | CD | GLN | 592 | 15.991 | 45.536 | 41.460 | 1.00 | 61.96 |
| 2671 | OE1 | GLN | 592 | 15.303 | 44.641 | 41.967 | 1.00 | 59.41 |
| 2672 | NE2 | GLN | 592 | 16.522 | 46.518 | 42.176 | 1.00 | 61.73 |
| 2673 | C | GLN | 592 | 16.409 | 42.711 | 37.376 | 1.00 | 58.77 |
| 2674 | O | GLN | 592 | 17.034 | 41.668 | 37.606 | 1.00 | 60.09 |
| 2675 | N | MET | 593 | 15.319 | 42.762 | 36.611 | 1.00 | 61.89 |
| 2676 | CA | MET | 593 | 14.756 | 41.575 | 35.977 | 1.00 | 60.29 |
| 2677 | CB | MET | 593 | 13.257 | 41.746 | 35.768 | 1.00 | 61.65 |
| 2678 | CG | MET | 593 | 12.401 | 41.411 | 36.969 | 1.00 | 61.69 |
| 2679 | SD | MET | 593 | 10.676 | 41.232 | 36.456 | 1.00 | 62.68 |
| 2680 | CE | MET | 593 | 10.249 | 42.940 | 36.274 | 1.00 | 60.78 |
| 2681 | C | MET | 593 | 15.388 | 41.229 | 34.645 | 1.00 | 59.77 |
| 2682 | O | MET | 593 | 15.386 | 40.068 | 34.241 | 1.00 | 61.16 |
| 2683 | N | THR | 594 | 15.904 | 42.235 | 33.948 | 1.00 | 59.97 |
| 2684 | CA | THR | 594 | 16.541 | 42.008 | 32.655 | 1.00 | 61.96 |
| 2685 | CB | THR | 594 | 16.828 | 43.335 | 31.932 | 1.00 | 61.57 |
| 2686 | OG1 | THR | 594 | 15.726 | 44.234 | 32.127 | 1.00 | 61.11 |
| 2687 | CG2 | THR | 594 | 17.026 | 43.086 | 30.435 | 1.00 | 62.41 |
| 2688 | C | THR | 594 | 17.865 | 41.305 | 32.902 | 1.00 | 63.83 |
| 2689 | O | THR | 594 | 18.184 | 40.287 | 32.274 | 1.00 | 62.47 |
| 2690 | N | LEU | 595 | 18.634 | 41.865 | 33.829 | 1.00 | 61.96 |
| 2691 | CA | LEU | 595 | 19.924 | 41.308 | 34.184 | 1.00 | 62.86 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2692 | CB | LEU | 595 | 20.585 | 42.172 | 35.265 | 1.00 | 61.42 |
| 2693 | CG | LEU | 595 | 20.937 | 43.615 | 34.885 | 1.00 | 63.11 |
| 2694 | CD1 | LEU | 595 | 21.680 | 44.276 | 36.031 | 1.00 | 63.10 |
| 2695 | CD2 | LEU | 595 | 21.791 | 43.634 | 33.636 | 1.00 | 59.45 |
| 2696 | C | LEU | 595 | 19.784 | 39.859 | 34.660 | 1.00 | 61.56 |
| 2697 | O | LEU | 595 | 20.438 | 38.960 | 34.136 | 1.00 | 62.15 |
| 2698 | N | LEU | 596 | 18.920 | 39.629 | 35.640 | 1.00 | 63.33 |
| 2699 | CA | LEU | 596 | 18.728 | 38.283 | 36.156 | 1.00 | 61.25 |
| 2700 | CB | LEU | 596 | 17.830 | 38.313 | 37.387 | 1.00 | 59.50 |
| 2701 | CG | LEU | 596 | 18.518 | 38.469 | 38.735 | 1.00 | 63.65 |
| 2702 | CD1 | LEU | 596 | 17.484 | 38.837 | 39.769 | 1.00 | 60.39 |
| 2703 | CD2 | LEU | 596 | 19.232 | 37.190 | 39.109 | 1.00 | 62.18 |
| 2704 | C | LEU | 596 | 18.159 | 37.293 | 35.140 | 1.00 | 57.46 |
| 2705 | O | LEU | 596 | 18.306 | 36.079 | 35.310 | 1.00 | 60.98 |
| 2706 | N | GLN | 597 | 17.507 | 37.800 | 34.095 | 1.00 | 60.24 |
| 2707 | CA | GLN | 597 | 16.915 | 36.953 | 33.055 | 1.00 | 59.39 |
| 2708 | CB | GLN | 597 | 15.727 | 37.660 | 32.413 | 1.00 | 63.86 |
| 2709 | CG | GLN | 597 | 14.431 | 37.528 | 33.171 | 1.00 | 63.29 |
| 2710 | CD | GLN | 597 | 13.365 | 38.466 | 32.648 | 1.00 | 58.84 |
| 2711 | OE1 | GLN | 597 | 13.389 | 38.873 | 31.484 | 1.00 | 61.11 |
| 2712 | NE2 | GLN | 597 | 12.414 | 38.811 | 33.505 | 1.00 | 60.83 |
| 2713 | C | GLN | 597 | 17.927 | 36.620 | 31.973 | 1.00 | 60.76 |
| 2714 | O | GLN | 597 | 17.829 | 35.597 | 31.302 | 1.00 | 63.58 |
| 2715 | N | TYR | 598 | 18.900 | 37.501 | 31.806 | 1.00 | 59.20 |
| 2716 | CA | TYR | 598 | 19.923 | 37.315 | 30.807 | 1.00 | 61.48 |
| 2717 | CB | TYR | 598 | 20.378 | 38.678 | 30.311 | 1.00 | 64.95 |
| 2718 | CG | TYR | 598 | 19.364 | 39.407 | 29.466 | 1.00 | 59.33 |
| 2719 | CD1 | TYR | 598 | 18.119 | 38.844 | 29.177 | 1.00 | 59.16 |
| 2720 | CE1 | TYR | 598 | 17.213 | 39.496 | 28.344 | 1.00 | 62.34 |
| 2721 | CD2 | TYR | 598 | 19.673 | 40.645 | 28.903 | 1.00 | 58.48 |
| 2722 | CE2 | TYR | 598 | 18.771 | 41.303 | 28.067 | 1.00 | 61.12 |
| 2723 | CZ | TYR | 598 | 17.551 | 40.721 | 27.794 | 1.00 | 63.18 |
| 2724 | OH | TYR | 598 | 16.680 | 41.371 | 26.960 | 1.00 | 63.83 |
| 2725 | C | TYR | 598 | 21.130 | 36.532 | 31.320 | 1.00 | 63.31 |
| 2726 | O | TYR | 598 | 21.850 | 35.900 | 30.550 | 1.00 | 62.59 |
| 2727 | N | SER | 599 | 21.356 | 36.554 | 32.623 | 1.00 | 62.16 |
| 2728 | CA | SER | 599 | 22.511 | 35.859 | 33.157 | 1.00 | 62.80 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2729 | CB | SER | 599 | 23.420 | 36.885 | 33.845 | 1.00 | 60.71 |
| 2730 | OG | SER | 599 | 22.660 | 37.845 | 34.560 | 1.00 | 60.75 |
| 2731 | C | SER | 599 | 22.245 | 34.678 | 34.093 | 1.00 | 60.81 |
| 2732 | O | SER | 599 | 23.183 | 34.104 | 34.636 | 1.00 | 57.53 |
| 2733 | N | TRP | 600 | 20.986 | 34.293 | 34.272 | 1.00 | 62.86 |
| 2734 | CA | TRP | 600 | 20.683 | 33.187 | 35.180 | 1.00 | 60.85 |
| 2735 | CB | TRP | 600 | 19.186 | 32.813 | 35.134 | 1.00 | 60.84 |
| 2736 | CG | TRP | 600 | 18.745 | 32.104 | 33.887 | 1.00 | 61.86 |
| 2737 | CD2 | TRP | 600 | 18.561 | 30.697 | 33.726 | 1.00 | 61.60 |
| 2738 | CE2 | TRP | 600 | 18.300 | 30.461 | 32.362 | 1.00 | 64.40 |
| 2739 | CE3 | TRP | 600 | 18.599 | 29.611 | 34.602 | 1.00 | 59.75 |
| 2740 | CD1 | TRP | 600 | 18.574 | 32.653 | 32.650 | 1.00 | 60.08 |
| 2741 | NE1 | TRP | 600 | 18.311 | 31.672 | 31.724 | 1.00 | 59.13 |
| 2742 | CZ2 | TRP | 600 | 18.085 | 29.182 | 31.854 | 1.00 | 61.82 |
| 2743 | CZ3 | TRP | 600 | 18.383 | 28.342 | 34.097 | 1.00 | 63.01 |
| 2744 | CH2 | TRP | 600 | 18.131 | 28.137 | 32.737 | 1.00 | 62.11 |
| 2745 | C | TRP | 600 | 21.523 | 31.963 | 34.848 | 1.00 | 61.75 |
| 2746 | O | TRP | 600 | 21.973 | 31.238 | 35.732 | 1.00 | 60.91 |
| 2747 | N | MET | 601 | 21.761 | 31.749 | 33.564 | 1.00 | 62.84 |
| 2748 | CA | MET | 601 | 22.504 | 30.591 | 33.172 | 1.00 | 60.19 |
| 2749 | CB | MET | 601 | 22.084 | 30.133 | 31.794 | 1.00 | 61.93 |
| 2750 | CG | MET | 601 | 22.616 | 28.786 | 31.496 | 1.00 | 63.07 |
| 2751 | SD | MET | 601 | 21.595 | 27.427 | 31.465 | 1.00 | 62.35 |
| 2752 | CE | MET | 601 | 22.357 | 26.773 | 32.529 | 1.00 | 60.74 |
| 2753 | C | MET | 601 | 24.008 | 30.766 | 33.243 | 1.00 | 61.35 |
| 2754 | O | MET | 601 | 24.732 | 29.790 | 33.391 | 1.00 | 62.01 |
| 2755 | N | SER | 602 | 24.483 | 32.002 | 33.146 | 1.00 | 59.64 |
| 2756 | CA | SER | 602 | 25.914 | 32.253 | 33.227 | 1.00 | 61.85 |
| 2757 | CB | SER | 602 | 26.249 | 33.637 | 32.675 | 1.00 | 63.12 |
| 2758 | OG | SER | 602 | 27.643 | 33.887 | 32.746 | 1.00 | 63.90 |
| 2759 | C | SER | 602 | 26.356 | 32.163 | 34.684 | 1.00 | 63.19 |
| 2760 | O | SER | 602 | 27.478 | 31.765 | 34.976 | 1.00 | 59.89 |
| 2761 | N | LEU | 603 | 25.452 | 32.537 | 35.588 | 1.00 | 59.89 |
| 2762 | CA | LEU | 603 | 25.703 | 32.527 | 37.027 | 1.00 | 63.05 |
| 2763 | CB | LEU | 603 | 24.673 | 33.413 | 37.748 | 1.00 | 60.06 |
| 2764 | CG | LEU | 603 | 24.752 | 34.936 | 37.606 | 1.00 | 58.80 |
| 2765 | CD1 | LEU | 603 | 23.591 | 35.588 | 38.334 | 1.00 | 59.17 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2766 | CD2 | LEU | 603 | 26.051 | 35.422 | 38.175 | 1.00 | 63.89 |
| 2767 | C | LEU | 603 | 25.624 | 31.118 | 37.586 | 1.00 | 60.06 |
| 2768 | O | LEU | 603 | 26.337 | 30.753 | 38.519 | 1.00 | 58.30 |
| 2769 | N | MET | 604 | 24.745 | 30.323 | 37.004 | 1.00 | 62.50 |
| 2770 | CA | MET | 604 | 24.565 | 28.967 | 37.468 | 1.00 | 61.20 |
| 2771 | CB | MET | 604 | 23.151 | 28.541 | 37.166 | 1.00 | 59.34 |
| 2772 | CG | MET | 604 | 22.185 | 28.879 | 38.247 | 1.00 | 61.83 |
| 2773 | SD | MET | 604 | 22.610 | 30.141 | 39.388 | 1.00 | 62.09 |
| 2774 | CE | MET | 604 | 22.173 | 29.285 | 40.731 | 1.00 | 60.68 |
| 2775 | C | MET | 604 | 25.578 | 28.009 | 36.879 | 1.00 | 61.48 |
| 2776 | O | MET | 604 | 25.989 | 27.048 | 37.536 | 1.00 | 60.91 |
| 2777 | N | ALA | 605 | 25.988 | 28.292 | 35.646 | 1.00 | 58.39 |
| 2778 | CA | ALA | 605 | 26.970 | 27.493 | 34.943 | 1.00 | 62.30 |
| 2779 | CB | ALA | 605 | 26.934 | 27.812 | 33.472 | 1.00 | 63.55 |
| 2780 | C | ALA | 605 | 28.341 | 27.820 | 35.502 | 1.00 | 63.47 |
| 2781 | O | ALA | 605 | 29.194 | 26.944 | 35.590 | 1.00 | 60.63 |
| 2782 | N | PHE | 606 | 28.546 | 29.082 | 35.882 | 1.00 | 60.75 |
| 2783 | CA | PHE | 606 | 29.832 | 29.536 | 36.411 | 1.00 | 62.24 |
| 2784 | CB | PHE | 606 | 29.951 | 31.059 | 36.300 | 1.00 | 65.41 |
| 2785 | CG | PHE | 606 | 31.316 | 31.606 | 36.663 | 1.00 | 64.45 |
| 2786 | CD1 | PHE | 606 | 32.424 | 31.375 | 35.848 | 1.00 | 62.88 |
| 2787 | CD2 | PHE | 606 | 31.483 | 32.388 | 37.802 | 1.00 | 61.45 |
| 2788 | CE1 | PHE | 606 | 33.668 | 31.919 | 36.162 | 1.00 | 61.89 |
| 2789 | CE2 | PHE | 606 | 32.725 | 32.931 | 38.120 | 1.00 | 63.04 |
| 2790 | CZ | PHE | 606 | 33.814 | 32.696 | 37.296 | 1.00 | 66.17 |
| 2791 | C | PHE | 606 | 30.044 | 29.121 | 37.851 | 1.00 | 64.34 |
| 2792 | O | PHE | 606 | 31.154 | 28.764 | 38.234 | 1.00 | 61.04 |
| 2793 | N | ALA | 607 | 28.997 | 29.180 | 38.661 | 1.00 | 59.38 |
| 2794 | CA | ALA | 607 | 29.144 | 28.771 | 40.047 | 1.00 | 61.90 |
| 2795 | CB | ALA | 607 | 27.953 | 29.224 | 40.865 | 1.00 | 58.55 |
| 2796 | C | ALA | 607 | 29.269 | 27.246 | 40.073 | 1.00 | 58.23 |
| 2797 | O | ALA | 607 | 29.912 | 26.681 | 40.953 | 1.00 | 60.68 |
| 2798 | N | LEU | 608 | 28.656 | 26.571 | 39.110 | 1.00 | 61.24 |
| 2799 | CA | LEU | 608 | 28.771 | 25.121 | 39.086 | 1.00 | 60.02 |
| 2800 | CB | LEU | 608 | 27.928 | 24.526 | 37.958 | 1.00 | 62.86 |
| 2801 | CG | LEU | 608 | 27.703 | 23.018 | 37.693 | 1.00 | 60.90 |
| 2802 | CD1 | LEU | 608 | 27.926 | 22.923 | 36.222 | 1.00 | 62.46 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2803 | CD2 | LEU | 608 | 28.630 | 22.033 | 38.439 | 1.00 | 60.02 |
| 2804 | C | LEU | 608 | 30.240 | 24.783 | 38.858 | 1.00 | 61.60 |
| 2805 | O | LEU | 608 | 30.758 | 23.870 | 39.479 | 1.00 | 62.78 |
| 2806 | N | GLY | 609 | 30.917 | 25.511 | 37.974 | 1.00 | 63.89 |
| 2807 | CA | GLY | 609 | 32.319 | 25.229 | 37.746 | 1.00 | 60.11 |
| 2808 | C | GLY | 609 | 33.143 | 25.391 | 39.018 | 1.00 | 61.05 |
| 2809 | O | GLY | 609 | 34.080 | 24.631 | 39.266 | 1.00 | 62.95 |
| 2810 | N | TRP | 610 | 32.783 | 26.374 | 39.838 | 1.00 | 62.51 |
| 2811 | CA | TRP | 610 | 33.499 | 26.652 | 41.073 | 1.00 | 63.33 |
| 2812 | CB | TRP | 610 | 32.917 | 27.885 | 41.741 | 1.00 | 59.08 |
| 2813 | CG | TRP | 610 | 33.617 | 28.226 | 43.008 | 1.00 | 60.29 |
| 2814 | CD2 | TRP | 610 | 34.910 | 28.821 | 43.127 | 1.00 | 61.56 |
| 2815 | CE2 | TRP | 610 | 35.194 | 28.930 | 44.501 | 1.00 | 59.81 |
| 2816 | CE3 | TRP | 610 | 35.860 | 29.273 | 42.200 | 1.00 | 57.77 |
| 2817 | CD1 | TRP | 610 | 33.178 | 28.002 | 44.279 | 1.00 | 61.04 |
| 2818 | NE1 | TRP | 610 | 34.121 | 28.423 | 45.183 | 1.00 | 63.24 |
| 2819 | CZ2 | TRP | 610 | 36.387 | 29.472 | 44.973 | 1.00 | 58.16 |
| 2820 | CZ3 | TRP | 610 | 37.048 | 29.811 | 42.670 | 1.00 | 62.09 |
| 2821 | CH2 | TRP | 610 | 37.301 | 29.905 | 44.043 | 1.00 | 63.07 |
| 2822 | C | TRP | 610 | 33.516 | 25.510 | 42.073 | 1.00 | 62.56 |
| 2823 | O | TRP | 610 | 34.554 | 25.205 | 42.662 | 1.00 | 62.85 |
| 2824 | N | ARG | 611 | 32.360 | 24.896 | 42.288 | 1.00 | 64.01 |
| 2825 | CA | ARG | 611 | 32.268 | 23.784 | 43.222 | 1.00 | 61.00 |
| 2826 | CB | ARG | 611 | 30.803 | 23.396 | 43.440 | 1.00 | 60.82 |
| 2827 | CG | ARG | 611 | 29.973 | 24.437 | 44.180 | 1.00 | 60.63 |
| 2828 | CD | ARG | 611 | 28.568 | 23.899 | 44.480 | 1.00 | 63.65 |
| 2829 | NE | ARG | 611 | 27.830 | 23.608 | 43.250 | 1.00 | 60.05 |
| 2830 | CZ | ARG | 611 | 27.228 | 24.528 | 42.498 | 1.00 | 61.48 |
| 2831 | NH1 | ARG | 611 | 27.255 | 25.811 | 42.853 | 1.00 | 62.30 |
| 2832 | NH2 | ARG | 611 | 26.638 | 24.175 | 41.365 | 1.00 | 62.30 |
| 2833 | C | ARG | 611 | 33.049 | 22.606 | 42.648 | 1.00 | 64.56 |
| 2834 | O | ARG | 611 | 33.712 | 21.854 | 43.373 | 1.00 | 59.80 |
| 2835 | N | SER | 612 | 32.971 | 22.467 | 41.329 | 1.00 | 60.03 |
| 2836 | CA | SER | 612 | 33.664 | 21.403 | 40.624 | 1.00 | 62.69 |
| 2837 | CB | SER | 612 | 33.312 | 21.451 | 39.141 | 1.00 | 60.54 |
| 2838 | OG | SER | 612 | 31.976 | 21.038 | 38.947 | 1.00 | 61.90 |
| 2839 | C | SER | 612 | 35.163 | 21.542 | 40.815 | 1.00 | 60.18 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2840 | O | SER | 612 | 35.842 | 20.597 | 41.209 | 1.00 | 62.35 |
| 2841 | N | TYR | 613 | 35.663 | 22.738 | 40.538 | 1.00 | 59.25 |
| 2842 | CA | TYR | 613 | 37.074 | 23.058 | 40.677 | 1.00 | 60.56 |
| 2843 | CB | TYR | 613 | 37.265 | 24.534 | 40.311 | 1.00 | 63.42 |
| 2844 | CG | TYR | 613 | 38.515 | 25.215 | 40.829 | 1.00 | 66.04 |
| 2845 | CD1 | TYR | 613 | 39.771 | 24.631 | 40.692 | 1.00 | 61.54 |
| 2846 | CE1 | TYR | 613 | 40.925 | 25.308 | 41.091 | 1.00 | 59.89 |
| 2847 | CD2 | TYR | 613 | 38.443 | 26.491 | 41.384 | 1.00 | 61.40 |
| 2848 | CE2 | TYR | 613 | 39.586 | 27.172 | 41.782 | 1.00 | 60.60 |
| 2849 | CZ | TYR | 613 | 40.823 | 26.577 | 41.633 | 1.00 | 61.69 |
| 2850 | OH | TYR | 613 | 41.950 | 27.258 | 42.022 | 1.00 | 57.50 |
| 2851 | C | TYR | 613 | 37.624 | 22.765 | 42.074 | 1.00 | 61.96 |
| 2852 | O | TYR | 613 | 38.665 | 22.130 | 42.219 | 1.00 | 62.16 |
| 2853 | N | ARG | 614 | 36.913 | 23.204 | 43.102 | 1.00 | 65.05 |
| 2854 | CA | ARG | 614 | 37.380 | 23.004 | 44.463 | 1.00 | 59.34 |
| 2855 | CB | ARG | 614 | 36.724 | 24.017 | 45.395 | 1.00 | 60.68 |
| 2856 | CG | ARG | 614 | 36.950 | 25.445 | 45.007 | 1.00 | 63.19 |
| 2857 | CD | ARG | 614 | 36.724 | 26.354 | 46.190 | 1.00 | 59.94 |
| 2858 | NE | ARG | 614 | 37.945 | 26.927 | 46.773 | 1.00 | 59.42 |
| 2859 | CZ | ARG | 614 | 39.115 | 27.068 | 46.145 | 1.00 | 60.26 |
| 2860 | NH1 | ARG | 614 | 40.141 | 27.628 | 46.776 | 1.00 | 60.62 |
| 2861 | NH2 | ARG | 614 | 39.288 | 26.619 | 44.906 | 1.00 | 59.69 |
| 2862 | C | ARG | 614 | 37.144 | 21.620 | 45.019 | 1.00 | 60.75 |
| 2863 | O | ARG | 614 | 37.899 | 21.144 | 45.869 | 1.00 | 61.95 |
| 2864 | N | GLN | 615 | 36.093 | 20.967 | 44.549 | 1.00 | 60.97 |
| 2865 | CA | GLN | 615 | 35.780 | 19.654 | 45.074 | 1.00 | 61.36 |
| 2866 | CB | GLN | 615 | 34.282 | 19.387 | 44.957 | 1.00 | 59.74 |
| 2867 | CG | GLN | 615 | 33.666 | 18.942 | 46.273 | 1.00 | 65.30 |
| 2868 | CD | GLN | 615 | 32.416 | 18.106 | 46.097 | 1.00 | 64.80 |
| 2869 | OE1 | GLN | 615 | 32.019 | 17.380 | 47.007 | 1.00 | 59.65 |
| 2870 | NE2 | GLN | 615 | 31.787 | 18.204 | 44.928 | 1.00 | 63.88 |
| 2871 | C | GLN | 615 | 36.547 | 18.523 | 44.419 | 1.00 | 61.87 |
| 2872 | O | GLN | 615 | 36.984 | 17.588 | 45.093 | 1.00 | 61.19 |
| 2873 | N | SER | 616 | 36.726 | 18.615 | 43.109 | 1.00 | 62.69 |
| 2874 | CA | SER | 616 | 37.408 | 17.559 | 42.387 | 1.00 | 60.64 |
| 2875 | CB | SER | 616 | 36.380 | 16.632 | 41.757 | 1.00 | 64.46 |
| 2876 | OG | SER | 616 | 35.731 | 17.299 | 40.688 | 1.00 | 63.47 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2877 | C | SER | 616 | 38.347 | 18.047 | 41.298 | 1.00 | 59.36 |
| 2878 | O | SER | 616 | 38.444 | 17.424 | 40.246 | 1.00 | 61.79 |
| 2879 | N | SER | 617 | 39.021 | 19.163 | 41.534 | 1.00 | 62.74 |
| 2880 | CA | SER | 617 | 39.972 | 19.681 | 40.560 | 1.00 | 60.01 |
| 2881 | CB | SER | 617 | 41.253 | 18.847 | 40.638 | 1.00 | 56.86 |
| 2882 | OG | SER | 617 | 41.690 | 18.714 | 41.980 | 1.00 | 64.58 |
| 2883 | C | SER | 617 | 39.433 | 19.675 | 39.119 | 1.00 | 62.31 |
| 2884 | O | SER | 617 | 40.099 | 19.196 | 38.196 | 1.00 | 60.08 |
| 2885 | N | ALA | 618 | 38.230 | 20.213 | 38.931 | 1.00 | 62.60 |
| 2886 | CA | ALA | 618 | 37.600 | 20.261 | 37.612 | 1.00 | 62.84 |
| 2887 | CB | ALA | 618 | 38.399 | 21.165 | 36.676 | 1.00 | 61.64 |
| 2888 | C | ALA | 618 | 37.475 | 18.866 | 37.005 | 1.00 | 60.94 |
| 2889 | O | ALA | 618 | 37.175 | 18.725 | 35.820 | 1.00 | 63.12 |
| 2890 | N | ASN | 619 | 37.692 | 17.836 | 37.820 | 1.00 | 60.02 |
| 2891 | CA | ASN | 619 | 37.610 | 16.465 | 37.330 | 1.00 | 60.02 |
| 2892 | CB | ASN | 619 | 38.467 | 15.523 | 38.178 | 1.00 | 61.65 |
| 2893 | CG | ASN | 619 | 39.881 | 15.426 | 37.663 | 1.00 | 65.19 |
| 2894 | OD1 | ASN | 619 | 40.813 | 15.986 | 38.241 | 1.00 | 58.62 |
| 2895 | ND2 | ASN | 619 | 40.047 | 14.729 | 36.547 | 1.00 | 59.93 |
| 2896 | C | ASN | 619 | 36.205 | 15.922 | 37.241 | 1.00 | 63.97 |
| 2897 | O | ASN | 619 | 35.933 | 15.005 | 36.469 | 1.00 | 61.24 |
| 2898 | N | LEU | 620 | 35.305 | 16.487 | 38.028 | 1.00 | 61.46 |
| 2899 | CA | LEU | 620 | 33.925 | 16.044 | 37.999 | 1.00 | 61.85 |
| 2900 | CB | LEU | 620 | 33.599 | 15.266 | 39.271 | 1.00 | 63.36 |
| 2901 | CG | LEU | 620 | 34.516 | 14.087 | 39.589 | 1.00 | 60.08 |
| 2902 | CD1 | LEU | 620 | 33.992 | 13.354 | 40.805 | 1.00 | 65.17 |
| 2903 | CD2 | LEU | 620 | 34.578 | 13.145 | 38.408 | 1.00 | 59.21 |
| 2904 | C | LEU | 620 | 33.031 | 17.266 | 37.890 | 1.00 | 63.79 |
| 2905 | O | LEU | 620 | 33.520 | 18.400 | 37.844 | 1.00 | 62.56 |
| 2906 | N | LEU | 621 | 31.728 | 17.022 | 37.808 | 1.00 | 60.88 |
| 2907 | CA | LEU | 621 | 30.757 | 18.096 | 37.739 | 1.00 | 60.93 |
| 2908 | CB | LEU | 621 | 29.822 | 17.930 | 36.545 | 1.00 | 59.84 |
| 2909 | CG | LEU | 621 | 30.365 | 18.564 | 35.272 | 1.00 | 61.52 |
| 2910 | CD1 | LEU | 621 | 29.302 | 18.516 | 34.204 | 1.00 | 61.79 |
| 2911 | CD2 | LEU | 621 | 30.776 | 19.998 | 35.547 | 1.00 | 62.82 |
| 2912 | C | LEU | 621 | 30.001 | 17.973 | 39.033 | 1.00 | 59.84 |
| 2913 | O | LEU | 621 | 29.267 | 17.009 | 39.249 | 1.00 | 59.39 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2914 | N | CYS | 622 | 30.191 | 18.952 | 39.903 | 1.00 | 60.82 |
| 2915 | CA | CYS | 622 | 29.562 | 18.902 | 41.201 | 1.00 | 61.70 |
| 2916 | CB | CYS | 622 | 30.612 | 19.216 | 42.276 | 1.00 | 61.70 |
| 2917 | SG | CYS | 622 | 32.249 | 18.417 | 42.005 | 1.00 | 55.72 |
| 2918 | C | CYS | 622 | 28.360 | 19.822 | 41.333 | 1.00 | 60.92 |
| 2919 | O | CYS | 622 | 28.394 | 20.777 | 42.107 | 1.00 | 60.85 |
| 2920 | N | PHE | 623 | 27.299 | 19.518 | 40.584 | 1.00 | 62.83 |
| 2921 | CA | PHE | 623 | 26.057 | 20.298 | 40.618 | 1.00 | 61.52 |
| 2922 | CB | PHE | 623 | 24.944 | 19.578 | 39.827 | 1.00 | 63.16 |
| 2923 | CG | PHE | 623 | 25.174 | 19.562 | 38.332 | 1.00 | 64.30 |
| 2924 | CD1 | PHE | 623 | 25.946 | 18.565 | 37.734 | 1.00 | 62.97 |
| 2925 | CD2 | PHE | 623 | 24.667 | 20.580 | 37.527 | 1.00 | 59.44 |
| 2926 | CE1 | PHE | 623 | 26.214 | 18.585 | 36.354 | 1.00 | 60.51 |
| 2927 | CE2 | PHE | 623 | 24.931 | 20.607 | 36.152 | 1.00 | 61.00 |
| 2928 | CZ | PHE | 623 | 25.705 | 19.609 | 35.566 | 1.00 | 58.20 |
| 2929 | C | PHE | 623 | 25.631 | 20.512 | 42.074 | 1.00 | 62.99 |
| 2930 | O | PHE | 623 | 25.433 | 21.650 | 42.520 | 1.00 | 59.63 |
| 2931 | N | ALA | 624 | 25.489 | 19.404 | 42.798 | 1.00 | 60.19 |
| 2932 | CA | ALA | 624 | 25.146 | 19.426 | 44.220 | 1.00 | 60.71 |
| 2933 | CB | ALA | 624 | 23.953 | 18.540 | 44.505 | 1.00 | 65.97 |
| 2934 | C | ALA | 624 | 26.384 | 18.877 | 44.921 | 1.00 | 60.87 |
| 2935 | O | ALA | 624 | 27.278 | 18.328 | 44.276 | 1.00 | 61.88 |
| 2936 | N | PRO | 625 | 26.467 | 19.023 | 46.248 | 1.00 | 63.71 |
| 2937 | CD | PRO | 625 | 25.561 | 19.666 | 47.207 | 1.00 | 59.14 |
| 2938 | CA | PRO | 625 | 27.658 | 18.496 | 46.924 | 1.00 | 61.58 |
| 2939 | CB | PRO | 625 | 27.528 | 19.055 | 48.346 | 1.00 | 64.33 |
| 2940 | CG | PRO | 625 | 26.534 | 20.184 | 48.212 | 1.00 | 61.92 |
| 2941 | C | PRO | 625 | 27.593 | 16.960 | 46.904 | 1.00 | 61.41 |
| 2942 | O | PRO | 625 | 28.630 | 16.280 | 46.869 | 1.00 | 60.49 |
| 2943 | N | ASP | 626 | 26.353 | 16.450 | 46.913 | 1.00 | 61.38 |
| 2944 | CA | ASP | 626 | 26.036 | 15.016 | 46.914 | 1.00 | 61.12 |
| 2945 | CB | ASP | 626 | 25.050 | 14.730 | 48.038 | 1.00 | 59.24 |
| 2946 | CG | ASP | 626 | 23.643 | 15.219 | 47.706 | 1.00 | 60.93 |
| 2947 | OD1 | ASP | 626 | 23.518 | 16.271 | 47.036 | 1.00 | 62.15 |
| 2948 | OD2 | ASP | 626 | 22.658 | 14.564 | 48.112 | 1.00 | 64.40 |
| 2949 | C | ASP | 626 | 25.405 | 14.562 | 45.587 | 1.00 | 62.42 |
| 2950 | O | ASP | 626 | 24.526 | 13.703 | 45.568 | 1.00 | 60.54 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2951 | N | LEU | 627 | 25.834 | 15.152 | 44.483 | 1.00 | 60.30 |
| 2952 | CA | LEU | 627 | 25.293 | 14.792 | 43.183 | 1.00 | 61.38 |
| 2953 | CB | LEU | 627 | 24.007 | 15.569 | 42.915 | 1.00 | 59.07 |
| 2954 | CG | LEU | 627 | 23.311 | 15.347 | 41.573 | 1.00 | 62.30 |
| 2955 | CD1 | LEU | 627 | 22.632 | 13.994 | 41.525 | 1.00 | 59.92 |
| 2956 | CD2 | LEU | 627 | 22.300 | 16.440 | 41.381 | 1.00 | 64.94 |
| 2957 | C | LEU | 627 | 26.349 | 15.143 | 42.152 | 1.00 | 59.24 |
| 2958 | O | LEU | 627 | 26.321 | 16.224 | 41.550 | 1.00 | 60.06 |
| 2959 | N | ILE | 628 | 27.284 | 14.219 | 41.958 | 1.00 | 59.89 |
| 2960 | CA | ILE | 628 | 28.380 | 14.422 | 41.030 | 1.00 | 60.40 |
| 2961 | CB | ILE | 628 | 29.729 | 14.056 | 41.692 | 1.00 | 62.59 |
| 2962 | CG2 | ILE | 628 | 30.850 | 14.267 | 40.716 | 1.00 | 59.44 |
| 2963 | CG1 | ILE | 628 | 29.990 | 14.940 | 42.909 | 1.00 | 61.86 |
| 2964 | CD1 | ILE | 628 | 29.045 | 14.711 | 44.049 | 1.00 | 62.51 |
| 2965 | C | ILE | 628 | 28.234 | 13.609 | 39.750 | 1.00 | 64.22 |
| 2966 | O | ILE | 628 | 28.028 | 12.402 | 39.787 | 1.00 | 60.75 |
| 2967 | N | ILE | 629 | 28.323 | 14.272 | 38.608 | 1.00 | 60.54 |
| 2968 | CA | ILE | 629 | 28.239 | 13.540 | 37.370 | 1.00 | 62.37 |
| 2969 | CB | ILE | 629 | 28.044 | 14.470 | 36.165 | 1.00 | 65.41 |
| 2970 | CG2 | ILE | 629 | 28.371 | 13.733 | 34.877 | 1.00 | 61.37 |
| 2971 | CG1 | ILE | 629 | 26.619 | 15.024 | 36.170 | 1.00 | 63.05 |
| 2972 | CD1 | ILE | 629 | 25.623 | 14.193 | 36.983 | 1.00 | 60.63 |
| 2973 | C | ILE | 629 | 29.575 | 12.839 | 37.270 | 1.00 | 60.22 |
| 2974 | O | ILE | 629 | 30.580 | 13.454 | 36.929 | 1.00 | 58.35 |
| 2975 | N | ASN | 630 | 29.580 | 11.556 | 37.611 | 1.00 | 60.04 |
| 2976 | CA | ASN | 630 | 30.776 | 10.726 | 37.570 | 1.00 | 60.83 |
| 2977 | CB | ASN | 630 | 30.674 | 9.637 | 38.632 | 1.00 | 62.77 |
| 2978 | CG | ASN | 630 | 29.368 | 8.868 | 38.556 | 1.00 | 60.79 |
| 2979 | OD1 | ASN | 630 | 29.051 | 8.248 | 37.541 | 1.00 | 61.32 |
| 2980 | ND2 | ASN | 630 | 28.603 | 8.908 | 39.632 | 1.00 | 62.90 |
| 2981 | C | ASN | 630 | 30.949 | 10.085 | 36.197 | 1.00 | 60.61 |
| 2982 | O | ASN | 630 | 30.016 | 10.041 | 35.403 | 1.00 | 62.50 |
| 2983 | N | GLU | 631 | 32.151 | 9.592 | 35.926 | 1.00 | 63.77 |
| 2984 | CA | GLU | 631 | 32.472 | 8.954 | 34.653 | 1.00 | 60.65 |
| 2985 | CB | GLU | 631 | 33.804 | 8.219 | 34.786 | 1.00 | 62.06 |
| 2986 | CG | GLU | 631 | 34.021 | 7.046 | 33.841 | 1.00 | 63.14 |
| 2987 | CD | GLU | 631 | 35.255 | 6.229 | 34.232 | 1.00 | 59.80 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 2988 | OE1 | GLU | 631 | 36.349 | 6.836 | 34.405 | 1.00 | 61.43 |
| 2989 | OE2 | GLU | 631 | 35.122 | 4.988 | 34.369 | 1.00 | 58.68 |
| 2990 | C | GLU | 631 | 31.381 | 7.986 | 34.254 | 1.00 | 61.07 |
| 2991 | O | GLU | 631 | 30.879 | 8.014 | 33.132 | 1.00 | 61.02 |
| 2992 | N | GLN | 632 | 31.011 | 7.126 | 35.186 | 1.00 | 63.77 |
| 2993 | CA | GLN | 632 | 29.978 | 6.151 | 34.916 | 1.00 | 61.66 |
| 2994 | CB | GLN | 632 | 29.732 | 5.285 | 36.159 | 1.00 | 61.41 |
| 2995 | CG | GLN | 632 | 30.936 | 4.415 | 36.579 | 1.00 | 65.26 |
| 2996 | CD | GLN | 632 | 31.704 | 3.828 | 35.393 | 1.00 | 64.37 |
| 2997 | OE1 | GLN | 632 | 31.109 | 3.357 | 34.420 | 1.00 | 60.73 |
| 2998 | NE2 | GLN | 632 | 33.034 | 3.847 | 35.480 | 1.00 | 59.46 |
| 2999 | C | GLN | 632 | 28.695 | 6.851 | 34.466 | 1.00 | 61.12 |
| 3000 | O | GLN | 632 | 28.055 | 6.417 | 33.512 | 1.00 | 62.13 |
| 3001 | N | ARG | 633 | 28.334 | 7.946 | 35.134 | 1.00 | 59.27 |
| 3002 | CA | ARG | 633 | 27.125 | 8.682 | 34.767 | 1.00 | 61.29 |
| 3003 | CB | ARG | 633 | 26.821 | 9.786 | 35.775 | 1.00 | 59.65 |
| 3004 | CG | ARG | 633 | 26.235 | 9.274 | 37.069 | 1.00 | 62.42 |
| 3005 | CD | ARG | 633 | 25.223 | 10.258 | 37.602 | 1.00 | 60.12 |
| 3006 | NE | ARG | 633 | 24.486 | 9.732 | 38.743 | 1.00 | 61.79 |
| 3007 | CZ | ARG | 633 | 24.739 | 10.038 | 40.011 | 1.00 | 64.89 |
| 3008 | NH1 | ARG | 633 | 25.717 | 10.878 | 40.308 | 1.00 | 60.64 |
| 3009 | NH2 | ARG | 633 | 24.014 | 9.501 | 40.984 | 1.00 | 62.92 |
| 3010 | C | ARG | 633 | 27.151 | 9.274 | 33.360 | 1.00 | 61.86 |
| 3011 | O | ARG | 633 | 26.086 | 9.401 | 32.750 | 1.00 | 59.63 |
| 3012 | N | MET | 634 | 28.337 | 9.643 | 32.855 | 1.00 | 64.70 |
| 3013 | CA | MET | 634 | 28.465 | 10.180 | 31.497 | 1.00 | 60.91 |
| 3014 | CB | MET | 634 | 29.921 | 10.556 | 31.189 | 1.00 | 59.90 |
| 3015 | CG | MET | 634 | 30.438 | 11.791 | 31.950 | 1.00 | 60.63 |
| 3016 | SD | MET | 634 | 30.042 | 13.425 | 31.192 | 1.00 | 60.35 |
| 3017 | CE | MET | 634 | 30.985 | 14.531 | 32.251 | 1.00 | 64.03 |
| 3018 | C | MET | 634 | 27.956 | 9.086 | 30.543 | 1.00 | 60.81 |
| 3019 | O | MET | 634 | 28.727 | 8.350 | 29.899 | 1.00 | 62.71 |
| 3020 | N | THR | 635 | 26.622 | 8.989 | 30.531 | 1.00 | 62.02 |
| 3021 | CA | THR | 635 | 25.820 | 8.059 | 29.738 | 1.00 | 62.69 |
| 3022 | CB | THR | 635 | 24.313 | 8.140 | 30.124 | 1.00 | 62.75 |
| 3023 | OG1 | THR | 635 | 24.150 | 7.893 | 31.528 | 1.00 | 60.63 |
| 3024 | CG2 | THR | 635 | 23.486 | 7.141 | 29.303 | 1.00 | 61.43 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3025 | C | THR | 635 | 25.912 | 8.531 | 28.307 | 1.00 | 61.30 |
| 3026 | O | THR | 635 | 26.510 | 7.876 | 27.454 | 1.00 | 59.84 |
| 3027 | N | LEU | 636 | 25.315 | 9.694 | 28.077 | 1.00 | 61.92 |
| 3028 | CA | LEU | 636 | 25.270 | 10.317 | 26.765 | 1.00 | 60.40 |
| 3029 | CB | LEU | 636 | 23.901 | 10.968 | 26.586 | 1.00 | 63.75 |
| 3030 | CG | LEU | 636 | 22.679 | 10.156 | 26.167 | 1.00 | 61.04 |
| 3031 | CD1 | LEU | 636 | 22.623 | 8.760 | 26.811 | 1.00 | 59.92 |
| 3032 | CD2 | LEU | 636 | 21.476 | 11.011 | 26.536 | 1.00 | 55.92 |
| 3033 | C | LEU | 636 | 26.347 | 11.377 | 26.441 | 1.00 | 63.57 |
| 3034 | O | LEU | 636 | 26.834 | 12.087 | 27.327 | 1.00 | 58.28 |
| 3035 | N | PRO | 637 | 26.754 | 11.471 | 25.155 | 1.00 | 61.79 |
| 3036 | CD | PRO | 637 | 26.479 | 10.631 | 23.987 | 1.00 | 63.46 |
| 3037 | CA | PRO | 637 | 27.744 | 12.476 | 24.794 | 1.00 | 60.89 |
| 3038 | CB | PRO | 637 | 28.363 | 11.932 | 23.490 | 1.00 | 63.07 |
| 3039 | CG | PRO | 637 | 27.855 | 10.482 | 23.398 | 1.00 | 62.95 |
| 3040 | C | PRO | 637 | 26.780 | 13.647 | 24.551 | 1.00 | 61.80 |
| 3041 | O | PRO | 637 | 27.038 | 14.531 | 23.736 | 1.00 | 60.33 |
| 3042 | N | CYS | 638 | 25.605 | 13.523 | 25.193 | 1.00 | 63.35 |
| 3043 | CA | CYS | 638 | 24.557 | 14.549 | 25.225 | 1.00 | 58.89 |
| 3044 | CB | CYS | 638 | 23.122 | 14.023 | 25.351 | 1.00 | 62.48 |
| 3045 | SG | CYS | 638 | 22.633 | 12.668 | 24.333 | 1.00 | 61.93 |
| 3046 | C | CYS | 638 | 24.925 | 14.896 | 26.642 | 1.00 | 59.34 |
| 3047 | O | CYS | 638 | 25.366 | 16.010 | 26.968 | 1.00 | 61.64 |
| 3048 | N | MET | 639 | 24.773 | 13.878 | 27.486 | 1.00 | 59.95 |
| 3049 | CA | MET | 639 | 25.094 | 14.058 | 28.870 | 1.00 | 62.51 |
| 3050 | CB | MET | 639 | 24.794 | 12.794 | 29.647 | 1.00 | 56.25 |
| 3051 | CG | MET | 639 | 24.597 | 13.021 | 31.126 | 1.00 | 59.11 |
| 3052 | SD | MET | 639 | 23.446 | 14.225 | 31.808 | 1.00 | 60.73 |
| 3053 | CE | MET | 639 | 24.286 | 14.281 | 33.307 | 1.00 | 59.61 |
| 3054 | C | MET | 639 | 26.567 | 14.451 | 28.934 | 1.00 | 60.92 |
| 3055 | O | MET | 639 | 27.074 | 14.783 | 30.000 | 1.00 | 59.57 |
| 3056 | N | TYR | 640 | 27.244 | 14.409 | 27.782 | 1.00 | 60.93 |
| 3057 | CA | TYR | 640 | 28.622 | 14.866 | 27.693 | 1.00 | 62.32 |
| 3058 | CB | TYR | 640 | 29.585 | 13.827 | 27.110 | 1.00 | 61.41 |
| 3059 | CG | TYR | 640 | 30.961 | 14.446 | 26.910 | 1.00 | 62.45 |
| 3060 | CD1 | TYR | 640 | 31.797 | 14.687 | 28.006 | 1.00 | 59.75 |
| 3061 | CE1 | TYR | 640 | 32.996 | 15.372 | 27.862 | 1.00 | 59.57 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3062 | CD2 | TYR | 640 | 31.376 | 14.906 | 25.651 | 1.00 | 59.20 |
| 3063 | CE2 | TYR | 640 | 32.578 | 15.594 | 25.495 | 1.00 | 64.39 |
| 3064 | CZ | TYR | 640 | 33.381 | 15.827 | 26.608 | 1.00 | 62.16 |
| 3065 | OH | TYR | 640 | 34.554 | 16.542 | 26.485 | 1.00 | 62.68 |
| 3066 | C | TYR | 640 | 28.650 | 16.082 | 26.764 | 1.00 | 60.42 |
| 3067 | O | TYR | 640 | 29.264 | 17.104 | 27.075 | 1.00 | 62.43 |
| 3068 | N | ASP | 641 | 27.985 | 15.960 | 25.619 | 1.00 | 63.11 |
| 3069 | CA | ASP | 641 | 27.946 | 17.029 | 24.617 | 1.00 | 64.46 |
| 3070 | CB | ASP | 641 | 26.821 | 16.780 | 23.617 | 1.00 | 64.30 |
| 3071 | CG | ASP | 641 | 27.232 | 17.039 | 22.196 | 1.00 | 61.31 |
| 3072 | OD1 | ASP | 641 | 26.317 | 17.192 | 21.353 | 1.00 | 59.56 |
| 3073 | OD2 | ASP | 641 | 28.453 | 17.079 | 21.917 | 1.00 | 63.20 |
| 3074 | C | ASP | 641 | 27.729 | 18.401 | 25.222 | 1.00 | 61.02 |
| 3075 | O | ASP | 641 | 28.073 | 19.417 | 24.617 | 1.00 | 60.07 |
| 3076 | N | GLN | 642 | 27.124 | 18.417 | 26.406 | 1.00 | 63.30 |
| 3077 | CA | GLN | 642 | 26.801 | 19.653 | 27.115 | 1.00 | 60.83 |
| 3078 | CB | GLN | 642 | 25.298 | 19.837 | 27.180 | 1.00 | 63.77 |
| 3079 | CG | GLN | 642 | 24.570 | 18.590 | 26.781 | 1.00 | 59.93 |
| 3080 | CD | GLN | 642 | 24.905 | 18.192 | 25.345 | 1.00 | 61.47 |
| 3081 | OE1 | GLN | 642 | 24.656 | 17.063 | 24.922 | 1.00 | 59.50 |
| 3082 | NE2 | GLN | 642 | 25.462 | 19.135 | 24.580 | 1.00 | 60.78 |
| 3083 | C | GLN | 642 | 27.353 | 19.664 | 28.518 | 1.00 | 60.90 |
| 3084 | O | GLN | 642 | 27.430 | 20.714 | 29.136 | 1.00 | 60.17 |
| 3085 | N | CYS | 643 | 27.678 | 18.497 | 29.052 | 1.00 | 61.78 |
| 3086 | CA | CYS | 643 | 28.291 | 18.491 | 30.362 | 1.00 | 62.44 |
| 3087 | CB | CYS | 643 | 28.348 | 17.080 | 30.963 | 1.00 | 66.84 |
| 3088 | SG | CYS | 643 | 27.004 | 16.704 | 32.130 | 1.00 | 64.90 |
| 3089 | C | CYS | 643 | 29.691 | 18.976 | 30.015 | 1.00 | 61.39 |
| 3090 | O | CYS | 643 | 30.377 | 19.587 | 30.836 | 1.00 | 61.37 |
| 3091 | N | LYS | 644 | 30.093 | 18.726 | 28.768 | 1.00 | 63.75 |
| 3092 | CA | LYS | 644 | 31.415 | 19.128 | 28.308 | 1.00 | 59.22 |
| 3093 | CB | LYS | 644 | 31.708 | 18.603 | 26.889 | 1.00 | 57.84 |
| 3094 | CG | LYS | 644 | 31.163 | 19.462 | 25.740 | 1.00 | 63.56 |
| 3095 | CD | LYS | 644 | 31.637 | 18.994 | 24.350 | 1.00 | 63.09 |
| 3096 | CE | LYS | 644 | 33.034 | 19.520 | 23.983 | 1.00 | 63.23 |
| 3097 | NZ | LYS | 644 | 34.141 | 19.025 | 24.872 | 1.00 | 61.68 |
| 3098 | C | LYS | 644 | 31.560 | 20.641 | 28.319 | 1.00 | 61.63 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3099 | O | LYS | 644 | 32.672 | 21.157 | 28.379 | 1.00 | 62.21 |
| 3100 | N | HIS | 645 | 30.444 | 21.359 | 28.267 | 1.00 | 59.46 |
| 3101 | CA | HIS | 645 | 30.518 | 22.809 | 28.261 | 1.00 | 61.40 |
| 3102 | CB | HIS | 645 | 29.338 | 23.380 | 27.490 | 1.00 | 62.01 |
| 3103 | CG | HIS | 645 | 29.548 | 23.365 | 26.009 | 1.00 | 61.11 |
| 3104 | CD2 | HIS | 645 | 30.591 | 23.797 | 25.261 | 1.00 | 62.23 |
| 3105 | ND1 | HIS | 645 | 28.628 | 22.845 | 25.123 | 1.00 | 62.89 |
| 3106 | CE1 | HIS | 645 | 29.097 | 22.957 | 23.892 | 1.00 | 60.35 |
| 3107 | NE2 | HIS | 645 | 30.285 | 23.532 | 23.948 | 1.00 | 58.72 |
| 3108 | C | HIS | 645 | 30.626 | 23.413 | 29.652 | 1.00 | 60.22 |
| 3109 | O | HIS | 645 | 31.097 | 24.535 | 29.804 | 1.00 | 62.73 |
| 3110 | N | MET | 646 | 30.205 | 22.672 | 30.668 | 1.00 | 61.99 |
| 3111 | CA | MET | 646 | 30.320 | 23.173 | 32.027 | 1.00 | 60.02 |
| 3112 | CB | MET | 646 | 29.235 | 22.574 | 32.963 | 1.00 | 60.39 |
| 3113 | CG | MET | 646 | 27.846 | 22.502 | 32.348 | 1.00 | 59.13 |
| 3114 | SD | MET | 646 | 26.508 | 21.807 | 33.298 | 1.00 | 59.17 |
| 3115 | CE | MET | 646 | 25.617 | 21.251 | 31.946 | 1.00 | 56.97 |
| 3116 | C | MET | 646 | 31.712 | 22.761 | 32.539 | 1.00 | 61.22 |
| 3117 | O | MET | 646 | 32.329 | 23.495 | 33.304 | 1.00 | 59.33 |
| 3118 | N | LEU | 647 | 32.207 | 21.597 | 32.110 | 1.00 | 61.22 |
| 3119 | CA | LEU | 647 | 33.539 | 21.146 | 32.526 | 1.00 | 59.88 |
| 3120 | CB | LEU | 647 | 33.858 | 19.754 | 31.962 | 1.00 | 64.23 |
| 3121 | CG | LEU | 647 | 33.205 | 18.494 | 32.529 | 1.00 | 60.79 |
| 3122 | CD1 | LEU | 647 | 33.267 | 17.423 | 31.475 | 1.00 | 61.72 |
| 3123 | CD2 | LEU | 647 | 33.901 | 18.030 | 33.803 | 1.00 | 62.44 |
| 3124 | C | LEU | 647 | 34.571 | 22.141 | 31.997 | 1.00 | 62.52 |
| 3125 | O | LEU | 647 | 35.664 | 22.292 | 32.558 | 1.00 | 59.55 |
| 3126 | N | TYR | 648 | 34.220 | 22.816 | 30.907 | 1.00 | 61.57 |
| 3127 | CA | TYR | 648 | 35.126 | 23.785 | 30.320 | 1.00 | 63.01 |
| 3128 | CB | TYR | 648 | 34.597 | 24.318 | 28.997 | 1.00 | 64.69 |
| 3129 | CG | TYR | 648 | 35.477 | 25.427 | 28.499 | 1.00 | 60.08 |
| 3130 | CD1 | TYR | 648 | 36.741 | 25.148 | 27.989 | 1.00 | 62.71 |
| 3131 | CE1 | TYR | 648 | 37.617 | 26.170 | 27.642 | 1.00 | 56.16 |
| 3132 | CD2 | TYR | 648 | 35.104 | 26.764 | 28.646 | 1.00 | 65.58 |
| 3133 | CE2 | TYR | 648 | 35.974 | 27.795 | 28.305 | 1.00 | 64.32 |
| 3134 | CZ | TYR | 648 | 37.226 | 27.490 | 27.806 | 1.00 | 62.44 |
| 3135 | OH | TYR | 648 | 38.097 | 28.500 | 27.480 | 1.00 | 58.63 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3136 | C | TYR | 648 | 35.380 | 24.969 | 31.241 | 1.00 | 60.50 |
| 3137 | O | TYR | 648 | 36.510 | 25.423 | 31.369 | 1.00 | 59.77 |
| 3138 | N | VAL | 649 | 34.331 | 25.490 | 31.865 | 1.00 | 61.19 |
| 3139 | CA | VAL | 649 | 34.521 | 26.625 | 32.754 | 1.00 | 62.10 |
| 3140 | CB | VAL | 649 | 33.164 | 27.231 | 33.257 | 1.00 | 63.18 |
| 3141 | CG1 | VAL | 649 | 32.254 | 27.546 | 32.089 | 1.00 | 60.09 |
| 3142 | CG2 | VAL | 649 | 32.476 | 26.282 | 34.202 | 1.00 | 59.25 |
| 3143 | C | VAL | 649 | 35.313 | 26.111 | 33.941 | 1.00 | 63.47 |
| 3144 | O | VAL | 649 | 36.188 | 26.791 | 34.465 | 1.00 | 58.78 |
| 3145 | N | SER | 650 | 35.010 | 24.884 | 34.340 | 1.00 | 61.57 |
| 3146 | CA | SER | 650 | 35.664 | 24.258 | 35.471 | 1.00 | 62.45 |
| 3147 | CB | SER | 650 | 35.032 | 22.901 | 35.727 | 1.00 | 63.87 |
| 3148 | OG | SER | 650 | 35.312 | 22.468 | 37.037 | 1.00 | 57.30 |
| 3149 | C | SER | 650 | 37.152 | 24.102 | 35.217 | 1.00 | 61.59 |
| 3150 | O | SER | 650 | 37.966 | 24.254 | 36.123 | 1.00 | 59.77 |
| 3151 | N | SER | 651 | 37.506 | 23.796 | 33.977 | 1.00 | 61.81 |
| 3152 | CA | SER | 651 | 38.904 | 23.629 | 33.615 | 1.00 | 59.44 |
| 3153 | CB | SER | 651 | 39.029 | 23.147 | 32.175 | 1.00 | 61.10 |
| 3154 | OG | SER | 651 | 40.285 | 23.527 | 31.635 | 1.00 | 62.25 |
| 3155 | C | SER | 651 | 39.638 | 24.942 | 33.755 | 1.00 | 62.21 |
| 3156 | O | SER | 651 | 40.736 | 24.994 | 34.299 | 1.00 | 59.39 |
| 3157 | N | GLU | 652 | 39.019 | 25.998 | 33.248 | 1.00 | 60.68 |
| 3158 | CA | GLU | 652 | 39.590 | 27.333 | 33.296 | 1.00 | 62.47 |
| 3159 | CB | GLU | 652 | 38.683 | 28.294 | 32.534 | 1.00 | 62.00 |
| 3160 | CG | GLU | 652 | 38.551 | 27.905 | 31.087 | 1.00 | 60.93 |
| 3161 | CD | GLU | 652 | 39.896 | 27.841 | 30.412 | 1.00 | 62.60 |
| 3162 | OE1 | GLU | 652 | 40.389 | 28.912 | 29.994 | 1.00 | 61.37 |
| 3163 | OE2 | GLU | 652 | 40.466 | 26.727 | 30.323 | 1.00 | 62.86 |
| 3164 | C | GLU | 652 | 39.803 | 27.829 | 34.719 | 1.00 | 59.21 |
| 3165 | O | GLU | 652 | 40.843 | 28.404 | 35.040 | 1.00 | 60.26 |
| 3166 | N | LEU | 653 | 38.812 | 27.613 | 35.573 | 1.00 | 62.17 |
| 3167 | CA | LEU | 653 | 38.939 | 28.039 | 36.949 | 1.00 | 61.29 |
| 3168 | CB | LEU | 653 | 37.630 | 27.816 | 37.702 | 1.00 | 62.27 |
| 3169 | CG | LEU | 653 | 36.539 | 28.833 | 37.355 | 1.00 | 63.87 |
| 3170 | CD1 | LEU | 653 | 35.239 | 28.428 | 38.009 | 1.00 | 60.88 |
| 3171 | CD2 | LEU | 653 | 36.969 | 30.220 | 37.805 | 1.00 | 66.93 |
| 3172 | C | LEU | 653 | 40.065 | 27.252 | 37.579 | 1.00 | 61.64 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3173 | O | LEU | 653 | 40.705 | 27.711 | 38.526 | 1.00 | 62.53 |
| 3174 | N | HIS | 654 | 40.316 | 26.067 | 37.035 | 1.00 | 59.14 |
| 3175 | CA | HIS | 654 | 41.386 | 25.219 | 37.534 | 1.00 | 63.27 |
| 3176 | CB | HIS | 654 | 41.122 | 23.768 | 37.166 | 1.00 | 63.36 |
| 3177 | CG | HIS | 654 | 42.203 | 22.842 | 37.610 | 1.00 | 63.44 |
| 3178 | CD2 | HIS | 654 | 43.298 | 22.379 | 36.965 | 1.00 | 60.81 |
| 3179 | ND1 | HIS | 654 | 42.281 | 22.360 | 38.898 | 1.00 | 62.60 |
| 3180 | CE1 | HIS | 654 | 43.382 | 21.642 | 39.027 | 1.00 | 56.60 |
| 3181 | NE2 | HIS | 654 | 44.017 | 21.639 | 37.870 | 1.00 | 63.72 |
| 3182 | C | HIS | 654 | 42.719 | 25.654 | 36.928 | 1.00 | 61.86 |
| 3183 | O | HIS | 654 | 43.691 | 25.906 | 37.636 | 1.00 | 63.73 |
| 3184 | N | ARG | 655 | 42.744 | 25.732 | 35.605 | 1.00 | 62.82 |
| 3185 | CA | ARG | 655 | 43.929 | 26.133 | 34.867 | 1.00 | 60.68 |
| 3186 | CB | ARG | 655 | 43.559 | 26.326 | 33.394 | 1.00 | 56.53 |
| 3187 | CG | ARG | 655 | 44.577 | 27.074 | 32.574 | 1.00 | 62.54 |
| 3188 | CD | ARG | 655 | 43.921 | 27.870 | 31.451 | 1.00 | 58.99 |
| 3189 | NE | ARG | 655 | 44.865 | 28.859 | 30.951 | 1.00 | 62.01 |
| 3190 | CZ | ARG | 655 | 46.081 | 28.544 | 30.503 | 1.00 | 59.65 |
| 3191 | NH1 | ARG | 655 | 46.475 | 27.269 | 30.488 | 1.00 | 58.86 |
| 3192 | NH2 | ARG | 655 | 46.926 | 29.491 | 30.103 | 1.00 | 59.91 |
| 3193 | C | ARG | 655 | 44.525 | 27.419 | 35.430 | 1.00 | 59.56 |
| 3194 | O | ARG | 655 | 45.741 | 27.524 | 35.595 | 1.00 | 60.27 |
| 3195 | N | LEU | 656 | 43.664 | 28.389 | 35.735 | 1.00 | 64.27 |
| 3196 | CA | LEU | 656 | 44.102 | 29.687 | 36.250 | 1.00 | 58.78 |
| 3197 | CB | LEU | 656 | 43.099 | 30.751 | 35.833 | 1.00 | 62.45 |
| 3198 | CG | LEU | 656 | 43.072 | 30.957 | 34.328 | 1.00 | 57.52 |
| 3199 | CD1 | LEU | 656 | 41.832 | 31.704 | 33.943 | 1.00 | 65.67 |
| 3200 | CD2 | LEU | 656 | 44.305 | 31.714 | 33.895 | 1.00 | 62.85 |
| 3201 | C | LEU | 656 | 44.340 | 29.761 | 37.757 | 1.00 | 61.09 |
| 3202 | O | LEU | 656 | 44.995 | 30.688 | 38.244 | 1.00 | 62.48 |
| 3203 | N | GLN | 657 | 43.816 | 28.783 | 38.489 | 1.00 | 59.08 |
| 3204 | CA | GLN | 657 | 43.979 | 28.736 | 39.936 | 1.00 | 63.09 |
| 3205 | CB | GLN | 657 | 45.469 | 28.663 | 40.307 | 1.00 | 58.16 |
| 3206 | CG | GLN | 657 | 46.052 | 27.250 | 40.286 | 1.00 | 63.46 |
| 3207 | CD | GLN | 657 | 45.307 | 26.310 | 41.225 | 1.00 | 59.61 |
| 3208 | OE1 | GLN | 657 | 44.607 | 25.392 | 40.785 | 1.00 | 64.27 |
| 3209 | NE2 | GLN | 657 | 45.442 | 26.547 | 42.529 | 1.00 | 59.12 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3210 | C | GLN | 657 | 43.335 | 29.932 | 40.611 | 1.00 | 59.19 |
| 3211 | O | GLN | 657 | 43.926 | 30.539 | 41.498 | 1.00 | 61.22 |
| 3212 | N | VAL | 658 | 42.113 | 30.249 | 40.192 | 1.00 | 61.76 |
| 3213 | CA | VAL | 658 | 41.355 | 31.376 | 40.734 | 1.00 | 61.31 |
| 3214 | CB | VAL | 658 | 39.970 | 31.503 | 40.043 | 1.00 | 60.50 |
| 3215 | CG1 | VAL | 658 | 39.211 | 32.664 | 40.623 | 1.00 | 59.45 |
| 3216 | CG2 | VAL | 658 | 40.132 | 31.716 | 38.559 | 1.00 | 61.37 |
| 3217 | C | VAL | 658 | 41.115 | 31.283 | 42.240 | 1.00 | 58.30 |
| 3218 | O | VAL | 658 | 40.838 | 30.208 | 42.764 | 1.00 | 56.60 |
| 3219 | N | SER | 659 | 41.214 | 32.418 | 42.928 | 1.00 | 60.76 |
| 3220 | CA | SER | 659 | 40.979 | 32.465 | 44.369 | 1.00 | 59.93 |
| 3221 | CB | SER | 659 | 41.873 | 33.507 | 45.047 | 1.00 | 59.20 |
| 3222 | OG | SER | 659 | 41.582 | 34.817 | 44.608 | 1.00 | 62.34 |
| 3223 | C | SER | 659 | 39.518 | 32.789 | 44.656 | 1.00 | 62.94 |
| 3224 | O | SER | 659 | 38.784 | 33.247 | 43.780 | 1.00 | 58.10 |
| 3225 | N | TYR | 660 | 39.097 | 32.563 | 45.893 | 1.00 | 61.68 |
| 3226 | CA | TYR | 660 | 37.720 | 32.808 | 46.250 | 1.00 | 62.54 |
| 3227 | CB | TYR | 660 | 37.481 | 32.526 | 47.717 | 1.00 | 64.73 |
| 3228 | CG | TYR | 660 | 36.014 | 32.432 | 48.044 | 1.00 | 56.73 |
| 3229 | CD1 | TYR | 660 | 35.144 | 31.742 | 47.200 | 1.00 | 59.62 |
| 3230 | CE1 | TYR | 660 | 33.817 | 31.568 | 47.524 | 1.00 | 61.29 |
| 3231 | CD2 | TYR | 660 | 35.507 | 32.957 | 49.223 | 1.00 | 64.06 |
| 3232 | CE2 | TYR | 660 | 34.176 | 32.789 | 49.557 | 1.00 | 61.23 |
| 3233 | CZ | TYR | 660 | 33.336 | 32.085 | 48.705 | 1.00 | 60.34 |
| 3234 | OH | TYR | 660 | 32.032 | 31.840 | 49.064 | 1.00 | 61.17 |
| 3235 | C | TYR | 660 | 37.250 | 34.204 | 45.954 | 1.00 | 61.20 |
| 3236 | O | TYR | 660 | 36.162 | 34.383 | 45.433 | 1.00 | 65.37 |
| 3237 | N | GLU | 661 | 38.057 | 35.199 | 46.290 | 1.00 | 61.50 |
| 3238 | CA | GLU | 661 | 37.657 | 36.574 | 46.052 | 1.00 | 58.24 |
| 3239 | CB | GLU | 661 | 38.598 | 37.523 | 46.765 | 1.00 | 64.85 |
| 3240 | CG | GLU | 661 | 38.276 | 37.577 | 48.225 | 1.00 | 61.43 |
| 3241 | CD | GLU | 661 | 39.283 | 38.360 | 48.991 | 1.00 | 60.83 |
| 3242 | OE1 | GLU | 661 | 39.961 | 39.204 | 48.365 | 1.00 | 59.88 |
| 3243 | OE2 | GLU | 661 | 39.387 | 38.143 | 50.219 | 1.00 | 59.48 |
| 3244 | C | GLU | 661 | 37.548 | 36.918 | 44.591 | 1.00 | 58.50 |
| 3245 | O | GLU | 661 | 36.573 | 37.536 | 44.178 | 1.00 | 62.37 |
| 3246 | N | GLU | 662 | 38.529 | 36.516 | 43.798 | 1.00 | 63.69 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3247 | CA | GLU | 662 | 38.453 | 36.784 | 42.374 | 1.00 | 59.27 |
| 3248 | CB | GLU | 662 | 39.646 | 36.158 | 41.657 | 1.00 | 57.91 |
| 3249 | CG | GLU | 662 | 40.974 | 36.663 | 42.122 | 1.00 | 62.43 |
| 3250 | CD | GLU | 662 | 42.104 | 35.948 | 41.436 | 1.00 | 62.15 |
| 3251 | OE1 | GLU | 662 | 41.969 | 34.732 | 41.223 | 1.00 | 57.52 |
| 3252 | OE2 | GLU | 662 | 43.128 | 36.585 | 41.119 | 1.00 | 61.99 |
| 3253 | C | GLU | 662 | 37.138 | 36.188 | 41.831 | 1.00 | 61.83 |
| 3254 | O | GLU | 662 | 36.492 | 36.771 | 40.963 | 1.00 | 63.75 |
| 3255 | N | TYR | 663 | 36.751 | 35.031 | 42.361 | 1.00 | 59.76 |
| 3256 | CA | TYR | 663 | 35.525 | 34.336 | 41.962 | 1.00 | 62.22 |
| 3257 | CB | TYR | 663 | 35.439 | 32.992 | 42.694 | 1.00 | 58.01 |
| 3258 | CG | TYR | 663 | 34.073 | 32.342 | 42.676 | 1.00 | 62.53 |
| 3259 | CD1 | TYR | 663 | 33.536 | 31.831 | 41.499 | 1.00 | 60.26 |
| 3260 | CE1 | TYR | 663 | 32.298 | 31.201 | 41.495 | 1.00 | 62.40 |
| 3261 | CD2 | TYR | 663 | 33.330 | 32.212 | 43.850 | 1.00 | 58.63 |
| 3262 | CE2 | TYR | 663 | 32.096 | 31.590 | 43.855 | 1.00 | 62.25 |
| 3263 | CZ | TYR | 663 | 31.587 | 31.084 | 42.676 | 1.00 | 63.25 |
| 3264 | OH | TYR | 663 | 30.372 | 30.448 | 42.682 | 1.00 | 62.04 |
| 3265 | C | TYR | 663 | 34.240 | 35.125 | 42.228 | 1.00 | 61.08 |
| 3266 | O | TYR | 663 | 33.429 | 35.343 | 41.322 | 1.00 | 58.41 |
| 3267 | N | LEU | 664 | 34.055 | 35.528 | 43.480 | 1.00 | 60.59 |
| 3268 | CA | LEU | 664 | 32.876 | 36.270 | 43.884 | 1.00 | 61.06 |
| 3269 | CB | LEU | 664 | 32.976 | 36.618 | 45.369 | 1.00 | 63.96 |
| 3270 | CG | LEU | 664 | 33.063 | 35.440 | 46.343 | 1.00 | 63.81 |
| 3271 | CD1 | LEU | 664 | 33.322 | 35.929 | 47.750 | 1.00 | 60.79 |
| 3272 | CD2 | LEU | 664 | 31.786 | 34.656 | 46.283 | 1.00 | 58.66 |
| 3273 | C | LEU | 664 | 32.692 | 37.539 | 43.057 | 1.00 | 62.83 |
| 3274 | O | LEU | 664 | 31.558 | 37.955 | 42.812 | 1.00 | 59.88 |
| 3275 | N | CYS | 665 | 33.809 | 38.139 | 42.632 | 1.00 | 59.97 |
| 3276 | CA | CYS | 665 | 33.805 | 39.365 | 41.831 | 1.00 | 63.59 |
| 3277 | CB | CYS | 665 | 35.167 | 40.043 | 41.869 | 1.00 | 60.16 |
| 3278 | SG | CYS | 665 | 35.586 | 40.757 | 43.441 | 1.00 | 62.94 |
| 3279 | C | CYS | 665 | 33.475 | 39.091 | 40.388 | 1.00 | 60.01 |
| 3280 | O | CYS | 665 | 32.794 | 39.876 | 39.735 | 1.00 | 57.49 |
| 3281 | N | MET | 666 | 33.997 | 37.984 | 39.883 | 1.00 | 60.45 |
| 3282 | CA | MET | 666 | 33.752 | 37.601 | 38.510 | 1.00 | 61.35 |
| 3283 | CB | MET | 666 | 34.733 | 36.517 | 38.077 | 1.00 | 60.05 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3284 | CG | MET | 666 | 36.156 | 36.993 | 37.902 | 1.00 | 63.77 |
| 3285 | SD | MET | 666 | 37.274 | 35.592 | 37.856 | 1.00 | 59.75 |
| 3286 | CE | MET | 666 | 37.139 | 35.071 | 36.150 | 1.00 | 62.17 |
| 3287 | C | MET | 666 | 32.338 | 37.078 | 38.411 | 1.00 | 61.52 |
| 3288 | O | MET | 666 | 31.681 | 37.255 | 37.388 | 1.00 | 60.47 |
| 3289 | N | LYS | 667 | 31.869 | 36.433 | 39.475 | 1.00 | 61.04 |
| 3290 | CA | LYS | 667 | 30.516 | 35.898 | 39.482 | 1.00 | 62.69 |
| 3291 | CB | LYS | 667 | 30.261 | 35.036 | 40.726 | 1.00 | 61.46 |
| 3292 | CG | LYS | 667 | 28.966 | 34.228 | 40.671 | 1.00 | 59.84 |
| 3293 | CD | LYS | 667 | 28.678 | 33.497 | 41.975 | 1.00 | 63.25 |
| 3294 | CE | LYS | 667 | 28.483 | 34.471 | 43.123 | 1.00 | 59.23 |
| 3295 | NZ | LYS | 667 | 27.639 | 33.891 | 44.192 | 1.00 | 61.97 |
| 3296 | C | LYS | 667 | 29.554 | 37.066 | 39.461 | 1.00 | 63.05 |
| 3297 | O | LYS | 667 | 28.459 | 36.945 | 38.942 | 1.00 | 60.65 |
| 3298 | N | THR | 668 | 29.981 | 38.201 | 40.011 | 1.00 | 61.58 |
| 3299 | CA | THR | 668 | 29.146 | 39.398 | 40.061 | 1.00 | 60.37 |
| 3300 | CB | THR | 668 | 29.609 | 40.357 | 41.149 | 1.00 | 59.81 |
| 3301 | OG1 | THR | 668 | 29.776 | 39.634 | 42.370 | 1.00 | 59.26 |
| 3302 | CG2 | THR | 668 | 28.588 | 41.442 | 41.365 | 1.00 | 57.52 |
| 3303 | C | THR | 668 | 29.174 | 40.146 | 38.746 | 1.00 | 60.69 |
| 3304 | O | THR | 668 | 28.184 | 40.749 | 38.348 | 1.00 | 61.79 |
| 3305 | N | LEU | 669 | 30.320 | 40.111 | 38.076 | 1.00 | 58.82 |
| 3306 | CA | LEU | 669 | 30.479 | 40.774 | 36.786 | 1.00 | 60.62 |
| 3307 | CB | LEU | 669 | 31.947 | 40.863 | 36.412 | 1.00 | 60.70 |
| 3308 | CG | LEU | 669 | 32.673 | 41.944 | 37.192 | 1.00 | 61.83 |
| 3309 | CD1 | LEU | 669 | 34.131 | 41.996 | 36.761 | 1.00 | 63.30 |
| 3310 | CD2 | LEU | 669 | 31.981 | 43.275 | 36.953 | 1.00 | 62.82 |
| 3311 | C | LEU | 669 | 29.736 | 40.028 | 35.707 | 1.00 | 63.57 |
| 3312 | O | LEU | 669 | 29.574 | 40.521 | 34.599 | 1.00 | 63.32 |
| 3313 | N | LEU | 670 | 29.303 | 38.823 | 36.034 | 1.00 | 62.04 |
| 3314 | CA | LEU | 670 | 28.558 | 38.030 | 35.087 | 1.00 | 64.79 |
| 3315 | CB | LEU | 670 | 28.662 | 36.542 | 35.432 | 1.00 | 63.41 |
| 3316 | CG | LEU | 670 | 29.983 | 35.838 | 35.078 | 1.00 | 62.66 |
| 3317 | CD1 | LEU | 670 | 29.918 | 34.407 | 35.554 | 1.00 | 61.38 |
| 3318 | CD2 | LEU | 670 | 30.239 | 35.867 | 33.580 | 1.00 | 61.74 |
| 3319 | C | LEU | 670 | 27.111 | 38.495 | 35.114 | 1.00 | 60.65 |
| 3320 | O | LEU | 670 | 26.405 | 38.402 | 34.119 | 1.00 | 60.67 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3321 | N | LEU | 671 | 26.673 | 39.008 | 36.257 | 1.00 | 60.80 |
| 3322 | CA | LEU | 671 | 25.308 | 39.500 | 36.386 | 1.00 | 60.13 |
| 3323 | CB | LEU | 671 | 24.977 | 39.756 | 37.852 | 1.00 | 55.83 |
| 3324 | CG | LEU | 671 | 23.636 | 40.403 | 38.198 | 1.00 | 59.73 |
| 3325 | CD1 | LEU | 671 | 22.495 | 39.498 | 37.819 | 1.00 | 58.32 |
| 3326 | CD2 | LEU | 671 | 23.606 | 40.677 | 39.673 | 1.00 | 59.37 |
| 3327 | C | LEU | 671 | 25.178 | 40.804 | 35.613 | 1.00 | 60.50 |
| 3328 | O | LEU | 671 | 24.076 | 41.295 | 35.377 | 1.00 | 61.24 |
| 3329 | N | LEU | 672 | 26.320 | 41.354 | 35.219 | 1.00 | 59.65 |
| 3330 | CA | LEU | 672 | 26.355 | 42.613 | 34.492 | 1.00 | 62.17 |
| 3331 | CB | LEU | 672 | 27.128 | 43.650 | 35.309 | 1.00 | 60.10 |
| 3332 | CG | LEU | 672 | 26.917 | 43.688 | 36.822 | 1.00 | 65.92 |
| 3333 | CD1 | LEU | 672 | 27.728 | 44.819 | 37.407 | 1.00 | 60.54 |
| 3334 | CD2 | LEU | 672 | 25.460 | 43.885 | 37.148 | 1.00 | 58.59 |
| 3335 | C | LEU | 672 | 27.027 | 42.456 | 33.131 | 1.00 | 62.29 |
| 3336 | O | LEU | 672 | 27.489 | 43.430 | 32.554 | 1.00 | 61.76 |
| 3337 | N | SER | 673 | 27.070 | 41.237 | 32.613 | 1.00 | 60.43 |
| 3338 | CA | SER | 673 | 27.732 | 40.980 | 31.342 | 1.00 | 60.60 |
| 3339 | CB | SER | 673 | 28.212 | 39.538 | 31.317 | 1.00 | 60.81 |
| 3340 | OG | SER | 673 | 27.281 | 38.718 | 31.987 | 1.00 | 56.49 |
| 3341 | C | SER | 673 | 26.949 | 41.280 | 30.074 | 1.00 | 60.41 |
| 3342 | O | SER | 673 | 27.542 | 41.502 | 29.020 | 1.00 | 60.96 |
| 3343 | N | SER | 674 | 25.625 | 41.267 | 30.160 | 1.00 | 62.26 |
| 3344 | CA | SER | 674 | 24.800 | 41.565 | 28.995 | 1.00 | 62.20 |
| 3345 | CB | SER | 674 | 24.359 | 40.281 | 28.298 | 1.00 | 61.34 |
| 3346 | OG | SER | 674 | 23.730 | 39.420 | 29.221 | 1.00 | 62.88 |
| 3347 | C | SER | 674 | 23.581 | 42.371 | 29.402 | 1.00 | 60.51 |
| 3348 | O | SER | 674 | 22.904 | 42.050 | 30.376 | 1.00 | 62.02 |
| 3349 | N | VAL | 675 | 23.321 | 43.432 | 28.653 | 1.00 | 61.79 |
| 3350 | CA | VAL | 675 | 22.190 | 44.299 | 28.911 | 1.00 | 61.63 |
| 3351 | CB | VAL | 675 | 22.670 | 45.712 | 29.194 | 1.00 | 59.56 |
| 3352 | CG1 | VAL | 675 | 23.388 | 45.748 | 30.517 | 1.00 | 62.21 |
| 3353 | CG2 | VAL | 675 | 23.598 | 46.164 | 28.078 | 1.00 | 63.23 |
| 3354 | C | VAL | 675 | 21.325 | 44.320 | 27.658 | 1.00 | 62.05 |
| 3355 | O | VAL | 675 | 21.757 | 43.861 | 26.603 | 1.00 | 62.28 |
| 3356 | N | PRO | 676 | 20.077 | 44.817 | 27.764 | 1.00 | 60.51 |
| 3357 | CD | PRO | 676 | 19.330 | 45.132 | 28.991 | 1.00 | 63.50 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3358 | CA | PRO | 676 | 19.191 | 44.880 | 26.593 | 1.00 | 62.22 |
| 3359 | CB | PRO | 676 | 17.896 | 45.494 | 27.156 | 1.00 | 58.53 |
| 3360 | CG | PRO | 676 | 18.322 | 46.117 | 28.488 | 1.00 | 60.68 |
| 3361 | C | PRO | 676 | 19.839 | 45.746 | 25.514 | 1.00 | 58.14 |
| 3362 | O | PRO | 676 | 20.824 | 46.435 | 25.792 | 1.00 | 61.96 |
| 3363 | N | LYS | 677 | 19.309 | 45.710 | 24.293 | 1.00 | 60.64 |
| 3364 | CA | LYS | 677 | 19.906 | 46.501 | 23.215 | 1.00 | 59.47 |
| 3365 | CB | LYS | 677 | 19.025 | 46.521 | 21.970 | 1.00 | 61.69 |
| 3366 | CG | LYS | 677 | 19.782 | 46.912 | 20.707 | 1.00 | 61.32 |
| 3367 | CD | LYS | 677 | 18.832 | 47.051 | 19.514 | 1.00 | 61.48 |
| 3368 | CE | LYS | 677 | 19.604 | 47.129 | 18.198 | 1.00 | 62.13 |
| 3369 | NZ | LYS | 677 | 20.435 | 45.908 | 17.952 | 1.00 | 60.25 |
| 3370 | C | LYS | 677 | 20.145 | 47.929 | 23.686 | 1.00 | 59.29 |
| 3371 | O | LYS | 677 | 21.248 | 48.235 | 24.158 | 1.00 | 63.94 |
| 3372 | N | ASP | 678 | 19.129 | 48.796 | 23.580 | 1.00 | 61.98 |
| 3373 | CA | ASP | 678 | 19.302 | 50.178 | 24.028 | 1.00 | 61.19 |
| 3374 | CB | ASP | 678 | 18.178 | 51.083 | 23.506 | 1.00 | 60.11 |
| 3375 | CG | ASP | 678 | 18.515 | 52.582 | 23.647 | 1.00 | 60.40 |
| 3376 | OD1 | ASP | 678 | 18.311 | 53.325 | 22.652 | 1.00 | 60.21 |
| 3377 | OD2 | ASP | 678 | 18.980 | 53.011 | 24.745 | 1.00 | 61.19 |
| 3378 | C | ASP | 678 | 19.395 | 50.284 | 25.558 | 1.00 | 61.02 |
| 3379 | O | ASP | 678 | 18.592 | 50.955 | 26.210 | 1.00 | 61.94 |
| 3380 | N | GLY | 679 | 20.398 | 49.604 | 26.108 | 1.00 | 58.42 |
| 3381 | CA | GLY | 679 | 20.649 | 49.605 | 27.534 | 1.00 | 56.97 |
| 3382 | C | GLY | 679 | 19.449 | 49.444 | 28.438 | 1.00 | 56.85 |
| 3383 | O | GLY | 679 | 18.362 | 49.031 | 28.028 | 1.00 | 59.44 |
| 3384 | N | LEU | 680 | 19.674 | 49.788 | 29.696 | 1.00 | 63.29 |
| 3385 | CA | LEU | 680 | 18.655 | 49.704 | 30.727 | 1.00 | 62.45 |
| 3386 | CB | LEU | 680 | 19.297 | 49.181 | 32.017 | 1.00 | 64.86 |
| 3387 | CG | LEU | 680 | 20.118 | 47.895 | 31.832 | 1.00 | 59.84 |
| 3388 | CD1 | LEU | 680 | 20.946 | 47.595 | 33.068 | 1.00 | 59.62 |
| 3389 | CD2 | LEU | 680 | 19.181 | 46.760 | 31.543 | 1.00 | 61.01 |
| 3390 | C | LEU | 680 | 18.056 | 51.090 | 30.955 | 1.00 | 60.58 |
| 3391 | O | LEU | 680 | 18.433 | 52.063 | 30.298 | 1.00 | 62.60 |
| 3392 | N | LYS | 681 | 17.120 | 51.174 | 31.888 | 1.00 | 62.27 |
| 3393 | CA | LYS | 681 | 16.486 | 52.441 | 32.197 | 1.00 | 63.23 |
| 3394 | CB | LYS | 681 | 15.146 | 52.211 | 32.901 | 1.00 | 59.95 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3395 | CG | LYS | 681 | 14.188 | 51.417 | 32.034 | 1.00 | 60.87 |
| 3396 | CD | LYS | 681 | 12.837 | 51.183 | 32.665 | 1.00 | 61.02 |
| 3397 | CE | LYS | 681 | 12.004 | 50.295 | 31.740 | 1.00 | 64.07 |
| 3398 | NZ | LYS | 681 | 10.616 | 50.060 | 32.218 | 1.00 | 58.77 |
| 3399 | C | LYS | 681 | 17.414 | 53.242 | 33.072 | 1.00 | 60.18 |
| 3400 | O | LYS | 681 | 17.373 | 54.462 | 33.069 | 1.00 | 63.10 |
| 3401 | N | SER | 682 | 18.278 | 52.554 | 33.802 | 1.00 | 60.92 |
| 3402 | CA | SER | 682 | 19.214 | 53.240 | 34.681 | 1.00 | 62.50 |
| 3403 | CB | SER | 682 | 18.953 | 52.786 | 36.113 | 1.00 | 62.86 |
| 3404 | OG | SER | 682 | 17.564 | 52.589 | 36.296 | 1.00 | 61.69 |
| 3405 | C | SER | 682 | 20.682 | 52.993 | 34.272 | 1.00 | 58.65 |
| 3406 | O | SER | 682 | 21.558 | 52.781 | 35.120 | 1.00 | 60.76 |
| 3407 | N | GLN | 683 | 20.924 | 53.053 | 32.961 | 1.00 | 60.04 |
| 3408 | CA | GLN | 683 | 22.241 | 52.840 | 32.348 | 1.00 | 61.19 |
| 3409 | CB | GLN | 683 | 22.156 | 53.127 | 30.850 | 1.00 | 59.58 |
| 3410 | CG | GLN | 683 | 23.397 | 52.757 | 30.056 | 1.00 | 62.33 |
| 3411 | CD | GLN | 683 | 23.606 | 51.259 | 29.955 | 1.00 | 62.33 |
| 3412 | OE1 | GLN | 683 | 22.651 | 50.502 | 29.759 | 1.00 | 62.13 |
| 3413 | NE2 | GLN | 683 | 24.858 | 50.823 | 30.065 | 1.00 | 61.30 |
| 3414 | C | GLN | 683 | 23.397 | 53.655 | 32.934 | 1.00 | 61.70 |
| 3415 | O | GLN | 683 | 24.561 | 53.335 | 32.719 | 1.00 | 62.18 |
| 3416 | N | GLU | 684 | 23.083 | 54.710 | 33.666 | 1.00 | 64.02 |
| 3417 | CA | GLU | 684 | 24.117 | 55.539 | 34.257 | 1.00 | 60.92 |
| 3418 | CB | GLU | 684 | 23.541 | 56.904 | 34.590 | 1.00 | 62.70 |
| 3419 | CG | GLU | 684 | 22.396 | 56.780 | 35.574 | 1.00 | 62.30 |
| 3420 | CD | GLU | 684 | 21.884 | 58.112 | 36.063 | 1.00 | 61.33 |
| 3421 | OE1 | GLU | 684 | 21.260 | 58.120 | 37.153 | 1.00 | 61.85 |
| 3422 | OE2 | GLU | 684 | 22.092 | 59.135 | 35.363 | 1.00 | 62.66 |
| 3423 | C | GLU | 684 | 24.582 | 54.867 | 35.534 | 1.00 | 61.21 |
| 3424 | O | GLU | 684 | 25.741 | 54.979 | 35.924 | 1.00 | 63.65 |
| 3425 | N | LEU | 685 | 23.659 | 54.181 | 36.197 | 1.00 | 62.46 |
| 3426 | CA | LEU | 685 | 23.992 | 53.487 | 37.429 | 1.00 | 60.48 |
| 3427 | CB | LEU | 685 | 22.731 | 53.265 | 38.269 | 1.00 | 62.31 |
| 3428 | CG | LEU | 685 | 22.992 | 53.036 | 39.764 | 1.00 | 59.19 |
| 3429 | CD1 | LEU | 685 | 23.700 | 54.245 | 40.360 | 1.00 | 59.86 |
| 3430 | CD2 | LEU | 685 | 21.684 | 52.795 | 40.485 | 1.00 | 61.02 |
| 3431 | C | LEU | 685 | 24.657 | 52.148 | 37.086 | 1.00 | 61.95 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3432 | O | LEU | 685 | 25.524 | 51.662 | 37.804 | 1.00 | 61.31 |
| 3433 | N | PHE | 686 | 24.264 | 51.566 | 35.964 | 1.00 | 60.07 |
| 3434 | CA | PHE | 686 | 24.832 | 50.302 | 35.560 | 1.00 | 60.10 |
| 3435 | CB | PHE | 686 | 24.147 | 49.785 | 34.311 | 1.00 | 61.65 |
| 3436 | CG | PHE | 686 | 24.500 | 48.372 | 33.990 | 1.00 | 62.45 |
| 3437 | CD1 | PHE | 686 | 24.148 | 47.352 | 34.860 | 1.00 | 58.40 |
| 3438 | CD2 | PHE | 686 | 25.204 | 48.057 | 32.839 | 1.00 | 57.88 |
| 3439 | CE1 | PHE | 686 | 24.493 | 46.038 | 34.589 | 1.00 | 58.59 |
| 3440 | CE2 | PHE | 686 | 25.558 | 46.741 | 32.558 | 1.00 | 63.31 |
| 3441 | CZ | PHE | 686 | 25.201 | 45.733 | 33.433 | 1.00 | 61.15 |
| 3442 | C | PHE | 686 | 26.321 | 50.423 | 35.287 | 1.00 | 61.88 |
| 3443 | O | PHE | 686 | 27.150 | 49.890 | 36.038 | 1.00 | 60.58 |
| 3444 | N | ASP | 687 | 26.657 | 51.113 | 34.199 | 1.00 | 62.94 |
| 3445 | CA | ASP | 687 | 28.048 | 51.291 | 33.817 | 1.00 | 61.00 |
| 3446 | CB | ASP | 687 | 28.171 | 52.397 | 32.776 | 1.00 | 59.08 |
| 3447 | CG | ASP | 687 | 27.327 | 52.131 | 31.555 | 1.00 | 63.32 |
| 3448 | OD1 | ASP | 687 | 27.089 | 50.940 | 31.271 | 1.00 | 59.10 |
| 3449 | OD2 | ASP | 687 | 26.914 | 53.098 | 30.875 | 1.00 | 63.48 |
| 3450 | C | ASP | 687 | 28.853 | 51.645 | 35.051 | 1.00 | 60.66 |
| 3451 | O | ASP | 687 | 29.988 | 51.203 | 35.213 | 1.00 | 60.12 |
| 3452 | N | GLU | 688 | 28.238 | 52.424 | 35.934 | 1.00 | 62.70 |
| 3453 | CA | GLU | 688 | 28.869 | 52.872 | 37.172 | 1.00 | 64.06 |
| 3454 | CB | GLU | 688 | 27.967 | 53.930 | 37.803 | 1.00 | 59.69 |
| 3455 | CG | GLU | 688 | 28.530 | 54.718 | 38.963 | 1.00 | 60.90 |
| 3456 | CD | GLU | 688 | 27.609 | 55.883 | 39.319 | 1.00 | 59.61 |
| 3457 | OE1 | GLU | 688 | 27.607 | 56.905 | 38.575 | 1.00 | 59.94 |
| 3458 | OE2 | GLU | 688 | 26.871 | 55.765 | 40.328 | 1.00 | 61.24 |
| 3459 | C | GLU | 688 | 29.137 | 51.726 | 38.164 | 1.00 | 62.60 |
| 3460 | O | GLU | 688 | 30.199 | 51.659 | 38.784 | 1.00 | 62.63 |
| 3461 | N | ILE | 689 | 28.166 | 50.833 | 38.314 | 1.00 | 61.42 |
| 3462 | CA | ILE | 689 | 28.296 | 49.691 | 39.207 | 1.00 | 64.18 |
| 3463 | CB | ILE | 689 | 26.919 | 49.043 | 39.453 | 1.00 | 57.30 |
| 3464 | CG2 | ILE | 689 | 27.080 | 47.686 | 40.125 | 1.00 | 62.29 |
| 3465 | CG1 | ILE | 689 | 26.055 | 49.980 | 40.297 | 1.00 | 63.30 |
| 3466 | CD1 | ILE | 689 | 24.668 | 49.458 | 40.545 | 1.00 | 63.34 |
| 3467 | C | ILE | 689 | 29.235 | 48.654 | 38.590 | 1.00 | 62.15 |
| 3468 | O | ILE | 689 | 30.079 | 48.071 | 39.276 | 1.00 | 59.58 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3469 | N | ARG | 690 | 29.083 | 48.416 | 37.290 | 1.00 | 63.61 |
| 3470 | CA | ARG | 690 | 29.938 | 47.455 | 36.606 | 1.00 | 64.65 |
| 3471 | CB | ARG | 690 | 29.619 | 47.429 | 35.111 | 1.00 | 61.26 |
| 3472 | CG | ARG | 690 | 30.319 | 46.331 | 34.331 | 1.00 | 60.27 |
| 3473 | CD | ARG | 690 | 29.665 | 46.159 | 32.967 | 1.00 | 61.69 |
| 3474 | NE | ARG | 690 | 30.153 | 44.983 | 32.247 | 1.00 | 64.60 |
| 3475 | CZ | ARG | 690 | 31.331 | 44.907 | 31.640 | 1.00 | 64.30 |
| 3476 | NH1 | ARG | 690 | 32.158 | 45.946 | 31.657 | 1.00 | 61.69 |
| 3477 | NH2 | ARG | 690 | 31.682 | 43.793 | 31.015 | 1.00 | 61.35 |
| 3478 | C | ARG | 690 | 31.387 | 47.863 | 36.824 | 1.00 | 62.11 |
| 3479 | O | ARG | 690 | 32.208 | 47.070 | 37.268 | 1.00 | 63.96 |
| 3480 | N | MET | 691 | 31.690 | 49.118 | 36.521 | 1.00 | 58.67 |
| 3481 | CA | MET | 691 | 33.029 | 49.642 | 36.689 | 1.00 | 62.38 |
| 3482 | CB | MET | 691 | 33.015 | 51.144 | 36.408 | 1.00 | 63.73 |
| 3483 | CG | MET | 691 | 34.366 | 51.723 | 36.003 | 1.00 | 63.05 |
| 3484 | SD | MET | 691 | 35.189 | 50.799 | 34.661 | 1.00 | 60.35 |
| 3485 | CE | MET | 691 | 36.714 | 50.382 | 35.462 | 1.00 | 57.09 |
| 3486 | C | MET | 691 | 33.533 | 49.367 | 38.106 | 1.00 | 61.62 |
| 3487 | O | MET | 691 | 34.653 | 48.907 | 38.300 | 1.00 | 60.31 |
| 3488 | N | THR | 692 | 32.691 | 49.633 | 39.095 | 1.00 | 61.56 |
| 3489 | CA | THR | 692 | 33.053 | 49.428 | 40.490 | 1.00 | 59.70 |
| 3490 | CB | THR | 692 | 31.899 | 49.838 | 41.404 | 1.00 | 61.08 |
| 3491 | OG1 | THR | 692 | 31.493 | 51.167 | 41.074 | 1.00 | 61.61 |
| 3492 | CG2 | THR | 692 | 32.331 | 49.794 | 42.860 | 1.00 | 61.81 |
| 3493 | C | THR | 692 | 33.478 | 47.997 | 40.837 | 1.00 | 61.92 |
| 3494 | O | THR | 692 | 34.349 | 47.799 | 41.695 | 1.00 | 59.04 |
| 3495 | N | TYR | 693 | 32.850 | 47.013 | 40.190 | 1.00 | 58.91 |
| 3496 | CA | TYR | 693 | 33.183 | 45.605 | 40.419 | 1.00 | 59.83 |
| 3497 | CB | TYR | 693 | 31.961 | 44.711 | 40.241 | 1.00 | 59.81 |
| 3498 | CG | TYR | 693 | 31.053 | 44.801 | 41.437 | 1.00 | 63.50 |
| 3499 | CD1 | TYR | 693 | 31.565 | 44.640 | 42.728 | 1.00 | 59.52 |
| 3500 | CE1 | TYR | 693 | 30.757 | 44.793 | 43.844 | 1.00 | 63.13 |
| 3501 | CD2 | TYR | 693 | 29.703 | 45.109 | 41.297 | 1.00 | 59.74 |
| 3502 | CE2 | TYR | 693 | 28.892 | 45.259 | 42.409 | 1.00 | 61.29 |
| 3503 | CZ | TYR | 693 | 29.428 | 45.101 | 43.671 | 1.00 | 62.95 |
| 3504 | OH | TYR | 693 | 28.625 | 45.264 | 44.760 | 1.00 | 60.97 |
| 3505 | C | TYR | 693 | 34.310 | 45.138 | 39.530 | 1.00 | 62.66 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3506 | O | TYR | 693 | 34.856 | 44.059 | 39.732 | 1.00 | 60.97 |
| 3507 | N | ILE | 694 | 34.648 | 45.955 | 38.536 | 1.00 | 62.91 |
| 3508 | CA | ILE | 694 | 35.766 | 45.651 | 37.661 | 1.00 | 60.76 |
| 3509 | CB | ILE | 694 | 35.726 | 46.450 | 36.347 | 1.00 | 63.35 |
| 3510 | CG2 | ILE | 694 | 37.057 | 46.305 | 35.611 | 1.00 | 58.86 |
| 3511 | CG1 | ILE | 694 | 34.570 | 45.956 | 35.480 | 1.00 | 59.03 |
| 3512 | CD1 | ILE | 694 | 34.524 | 46.568 | 34.109 | 1.00 | 64.66 |
| 3513 | C | ILE | 694 | 36.947 | 46.106 | 38.496 | 1.00 | 59.29 |
| 3514 | O | ILE | 694 | 37.976 | 45.450 | 38.548 | 1.00 | 59.81 |
| 3515 | N | LYS | 695 | 36.788 | 47.235 | 39.171 | 1.00 | 60.10 |
| 3516 | CA | LYS | 695 | 37.850 | 47.718 | 40.031 | 1.00 | 59.42 |
| 3517 | CB | LYS | 695 | 37.530 | 49.111 | 40.577 | 1.00 | 62.71 |
| 3518 | CG | LYS | 695 | 37.525 | 50.194 | 39.543 | 1.00 | 56.74 |
| 3519 | CD | LYS | 695 | 37.579 | 51.556 | 40.199 | 1.00 | 62.01 |
| 3520 | CE | LYS | 695 | 37.453 | 52.666 | 39.170 | 1.00 | 62.05 |
| 3521 | NZ | LYS | 695 | 37.650 | 54.002 | 39.778 | 1.00 | 58.55 |
| 3522 | C | LYS | 695 | 37.996 | 46.741 | 41.193 | 1.00 | 60.49 |
| 3523 | O | LYS | 695 | 39.071 | 46.578 | 41.744 | 1.00 | 58.83 |
| 3524 | N | GLU | 696 | 36.906 | 46.084 | 41.559 | 1.00 | 58.96 |
| 3525 | CA | GLU | 696 | 36.924 | 45.135 | 42.663 | 1.00 | 63.14 |
| 3526 | CB | GLU | 696 | 35.489 | 44.813 | 43.080 | 1.00 | 62.85 |
| 3527 | CG | GLU | 696 | 35.338 | 44.392 | 44.513 | 1.00 | 61.99 |
| 3528 | CD | GLU | 696 | 35.888 | 45.410 | 45.497 | 1.00 | 62.60 |
| 3529 | OE1 | GLU | 696 | 35.609 | 46.616 | 45.353 | 1.00 | 60.89 |
| 3530 | OE2 | GLU | 696 | 36.596 | 44.999 | 46.438 | 1.00 | 59.40 |
| 3531 | C | GLU | 696 | 37.668 | 43.855 | 42.273 | 1.00 | 63.03 |
| 3532 | O | GLU | 696 | 38.281 | 43.198 | 43.120 | 1.00 | 61.00 |
| 3533 | N | LEU | 697 | 37.605 | 43.507 | 40.990 | 1.00 | 61.52 |
| 3534 | CA | LEU | 697 | 38.279 | 42.324 | 40.487 | 1.00 | 58.74 |
| 3535 | CB | LEU | 697 | 37.830 | 42.018 | 39.057 | 1.00 | 58.36 |
| 3536 | CG | LEU | 697 | 38.438 | 40.757 | 38.439 | 1.00 | 63.54 |
| 3537 | CD1 | LEU | 697 | 37.948 | 39.560 | 39.208 | 1.00 | 57.14 |
| 3538 | CD2 | LEU | 697 | 38.058 | 40.623 | 36.972 | 1.00 | 63.58 |
| 3539 | C | LEU | 697 | 39.766 | 42.633 | 40.499 | 1.00 | 60.87 |
| 3540 | O | LEU | 697 | 40.599 | 41.748 | 40.683 | 1.00 | 64.34 |
| 3541 | N | GLY | 698 | 40.087 | 43.907 | 40.303 | 1.00 | 63.62 |
| 3542 | CA | GLY | 698 | 41.472 | 44.331 | 40.285 | 1.00 | 60.55 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3543 | C | GLY | 698 | 42.053 | 44.398 | 41.677 | 1.00 | 61.55 |
| 3544 | O | GLY | 698 | 43.259 | 44.268 | 41.877 | 1.00 | 58.62 |
| 3545 | N | LYS | 699 | 41.180 | 44.621 | 42.644 | 1.00 | 60.41 |
| 3546 | CA | LYS | 699 | 41.596 | 44.689 | 44.027 | 1.00 | 61.47 |
| 3547 | CB | LYS | 699 | 40.437 | 45.189 | 44.903 | 1.00 | 62.58 |
| 3548 | CG | LYS | 699 | 40.251 | 46.712 | 44.942 | 1.00 | 64.44 |
| 3549 | CD | LYS | 699 | 39.111 | 47.088 | 45.889 | 1.00 | 59.70 |
| 3550 | CE | LYS | 699 | 39.375 | 48.368 | 46.707 | 1.00 | 60.92 |
| 3551 | NZ | LYS | 699 | 38.986 | 49.663 | 46.056 | 1.00 | 62.69 |
| 3552 | C | LYS | 699 | 42.000 | 43.284 | 44.447 | 1.00 | 64.59 |
| 3553 | O | LYS | 699 | 43.044 | 43.077 | 45.063 | 1.00 | 59.56 |
| 3554 | N | ALA | 700 | 41.161 | 42.322 | 44.080 | 1.00 | 58.13 |
| 3555 | CA | ALA | 700 | 41.376 | 40.927 | 44.415 | 1.00 | 62.66 |
| 3556 | CB | ALA | 700 | 40.219 | 40.103 | 43.908 | 1.00 | 59.89 |
| 3557 | C | ALA | 700 | 42.678 | 40.387 | 43.861 | 1.00 | 60.42 |
| 3558 | O | ALA | 700 | 43.430 | 39.743 | 44.577 | 1.00 | 61.59 |
| 3559 | N | ILE | 701 | 42.937 | 40.662 | 42.585 | 1.00 | 60.42 |
| 3560 | CA | ILE | 701 | 44.141 | 40.201 | 41.898 | 1.00 | 63.95 |
| 3561 | CB | ILE | 701 | 44.105 | 40.611 | 40.416 | 1.00 | 61.37 |
| 3562 | CG2 | ILE | 701 | 45.396 | 40.224 | 39.742 | 1.00 | 65.93 |
| 3563 | CG1 | ILE | 701 | 42.921 | 39.939 | 39.717 | 1.00 | 60.90 |
| 3564 | CD1 | ILE | 701 | 42.697 | 40.405 | 38.275 | 1.00 | 59.90 |
| 3565 | C | ILE | 701 | 45.465 | 40.675 | 42.514 | 1.00 | 61.05 |
| 3566 | O | ILE | 701 | 46.455 | 39.931 | 42.518 | 1.00 | 62.71 |
| 3567 | N | VAL | 702 | 45.495 | 41.901 | 43.030 | 1.00 | 64.20 |
| 3568 | CA | VAL | 702 | 46.723 | 42.409 | 43.637 | 1.00 | 61.51 |
| 3569 | CB | VAL | 702 | 46.690 | 43.949 | 43.841 | 1.00 | 61.28 |
| 3570 | CG1 | VAL | 702 | 46.285 | 44.645 | 42.546 | 1.00 | 61.75 |
| 3571 | CG2 | VAL | 702 | 45.753 | 44.305 | 44.983 | 1.00 | 63.42 |
| 3572 | C | VAL | 702 | 46.964 | 41.753 | 44.995 | 1.00 | 62.93 |
| 3573 | O | VAL | 702 | 48.101 | 41.598 | 45.432 | 1.00 | 62.05 |
| 3574 | N | LYS | 703 | 45.894 | 41.357 | 45.665 | 1.00 | 61.37 |
| 3575 | CA | LYS | 703 | 46.057 | 40.741 | 46.967 | 1.00 | 62.89 |
| 3576 | CB | LYS | 703 | 44.714 | 40.683 | 47.705 | 1.00 | 58.24 |
| 3577 | CG | LYS | 703 | 44.851 | 40.816 | 49.215 | 1.00 | 61.67 |
| 3578 | CD | LYS | 703 | 45.460 | 39.557 | 49.830 | 1.00 | 63.57 |
| 3579 | CE | LYS | 703 | 46.410 | 39.869 | 50.986 | 1.00 | 57.61 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3580 | NZ | LYS | 703 | 46.656 | 38.664 | 51.838 | 1.00 | 61.16 |
| 3581 | C | LYS | 703 | 46.645 | 39.355 | 46.776 | 1.00 | 60.39 |
| 3582 | O | LYS | 703 | 46.974 | 38.668 | 47.731 | 1.00 | 61.26 |
| 3583 | N | ARG | 704 | 46.790 | 38.951 | 45.523 | 1.00 | 62.02 |
| 3584 | CA | ARG | 704 | 47.369 | 37.649 | 45.214 | 1.00 | 61.58 |
| 3585 | CB | ARG | 704 | 46.408 | 36.817 | 44.368 | 1.00 | 61.15 |
| 3586 | CG | ARG | 704 | 45.272 | 36.177 | 45.112 | 1.00 | 66.70 |
| 3587 | CD | ARG | 704 | 44.871 | 34.956 | 44.350 | 1.00 | 62.98 |
| 3588 | NE | ARG | 704 | 45.731 | 34.788 | 43.185 | 1.00 | 58.04 |
| 3589 | CZ | ARG | 704 | 45.973 | 33.619 | 42.601 | 1.00 | 65.26 |
| 3590 | NH1 | ARG | 704 | 45.422 | 32.513 | 43.080 | 1.00 | 60.05 |
| 3591 | NH2 | ARG | 704 | 46.761 | 33.552 | 41.538 | 1.00 | 60.69 |
| 3592 | C | ARG | 704 | 48.686 | 37.792 | 44.445 | 1.00 | 62.07 |
| 3593 | O | ARG | 704 | 49.779 | 37.725 | 45.029 | 1.00 | 60.23 |
| 3594 | N | GLU | 705 | 48.555 | 37.997 | 43.130 | 1.00 | 61.20 |
| 3595 | CA | GLU | 705 | 49.686 | 38.134 | 42.212 | 1.00 | 61.45 |
| 3596 | CB | GLU | 705 | 49.179 | 38.271 | 40.776 | 1.00 | 59.83 |
| 3597 | CG | GLU | 705 | 49.038 | 36.941 | 40.015 | 1.00 | 61.58 |
| 3598 | CD | GLU | 705 | 48.539 | 35.764 | 40.875 | 1.00 | 62.88 |
| 3599 | OE1 | GLU | 705 | 47.511 | 35.908 | 41.595 | 1.00 | 61.37 |
| 3600 | OE2 | GLU | 705 | 49.181 | 34.685 | 40.806 | 1.00 | 62.83 |
| 3601 | C | GLU | 705 | 50.601 | 39.296 | 42.542 | 1.00 | 60.29 |
| 3602 | O | GLU | 705 | 50.212 | 40.468 | 42.446 | 1.00 | 61.23 |
| 3603 | N | GLY | 706 | 51.832 | 38.935 | 42.896 | 1.00 | 60.12 |
| 3604 | CA | GLY | 706 | 52.855 | 39.883 | 43.288 | 1.00 | 62.85 |
| 3605 | C | GLY | 706 | 53.083 | 41.191 | 42.556 | 1.00 | 58.46 |
| 3606 | O | GLY | 706 | 52.603 | 42.244 | 42.991 | 1.00 | 60.65 |
| 3607 | N | ASN | 707 | 53.818 | 41.141 | 41.449 | 1.00 | 62.51 |
| 3608 | CA | ASN | 707 | 54.158 | 42.366 | 40.729 | 1.00 | 63.30 |
| 3609 | CB | ASN | 707 | 55.516 | 42.196 | 40.013 | 1.00 | 60.74 |
| 3610 | CG | ASN | 707 | 55.676 | 40.835 | 39.356 | 1.00 | 60.19 |
| 3611 | OD1 | ASN | 707 | 55.354 | 39.797 | 39.950 | 1.00 | 59.76 |
| 3612 | ND2 | ASN | 707 | 56.195 | 40.833 | 38.128 | 1.00 | 59.34 |
| 3613 | C | ASN | 707 | 53.134 | 42.993 | 39.792 | 1.00 | 59.87 |
| 3614 | O | ASN | 707 | 52.054 | 42.451 | 39.569 | 1.00 | 63.44 |
| 3615 | N | SER | 708 | 53.501 | 44.161 | 39.265 | 1.00 | 60.90 |
| 3616 | CA | SER | 708 | 52.647 | 44.950 | 38.382 | 1.00 | 58.16 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3617 | CB | SER | 708 | 53.218 | 46.366 | 38.244 | 1.00 | 62.09 |
| 3618 | OG | SER | 708 | 53.403 | 46.974 | 39.516 | 1.00 | 61.85 |
| 3619 | C | SER | 708 | 52.432 | 44.354 | 36.998 | 1.00 | 61.28 |
| 3620 | O | SER | 708 | 51.400 | 44.607 | 36.372 | 1.00 | 58.94 |
| 3621 | N | SER | 709 | 53.393 | 43.567 | 36.516 | 1.00 | 61.19 |
| 3622 | CA | SER | 709 | 53.266 | 42.952 | 35.193 | 1.00 | 59.82 |
| 3623 | CB | SER | 709 | 54.650 | 42.527 | 34.665 | 1.00 | 62.14 |
| 3624 | OG | SER | 709 | 54.658 | 42.353 | 33.249 | 1.00 | 61.67 |
| 3625 | C | SER | 709 | 52.338 | 41.743 | 35.318 | 1.00 | 59.05 |
| 3626 | O | SER | 709 | 51.508 | 41.479 | 34.442 | 1.00 | 63.30 |
| 3627 | N | GLN | 710 | 52.481 | 41.022 | 36.426 | 1.00 | 63.44 |
| 3628 | CA | GLN | 710 | 51.652 | 39.851 | 36.691 | 1.00 | 59.41 |
| 3629 | CB | GLN | 710 | 52.289 | 39.036 | 37.833 | 1.00 | 63.27 |
| 3630 | CG | GLN | 710 | 53.572 | 38.329 | 37.354 | 1.00 | 60.06 |
| 3631 | CD | GLN | 710 | 54.362 | 37.611 | 38.453 | 1.00 | 57.38 |
| 3632 | OE1 | GLN | 710 | 53.781 | 37.012 | 39.369 | 1.00 | 60.97 |
| 3633 | NE2 | GLN | 710 | 55.701 | 37.647 | 38.348 | 1.00 | 63.13 |
| 3634 | C | GLN | 710 | 50.209 | 40.281 | 37.010 | 1.00 | 62.98 |
| 3635 | O | GLN | 710 | 49.250 | 39.584 | 36.667 | 1.00 | 61.41 |
| 3636 | N | ASN | 711 | 50.087 | 41.451 | 37.641 | 1.00 | 59.07 |
| 3637 | CA | ASN | 711 | 48.815 | 42.048 | 38.017 | 1.00 | 59.32 |
| 3638 | CB | ASN | 711 | 49.053 | 43.439 | 38.610 | 1.00 | 62.53 |
| 3639 | CG | ASN | 711 | 49.400 | 43.398 | 40.096 | 1.00 | 65.70 |
| 3640 | OD1 | ASN | 711 | 49.886 | 44.388 | 40.663 | 1.00 | 59.52 |
| 3641 | ND2 | ASN | 711 | 49.140 | 42.259 | 40.736 | 1.00 | 62.62 |
| 3642 | C | ASN | 711 | 47.918 | 42.167 | 36.792 | 1.00 | 61.19 |
| 3643 | 0 | ASN | 711 | 46.796 | 41.644 | 36.778 | 1.00 | 59.41 |
| 3644 | N | TRP | 712 | 48.418 | 42.850 | 35.762 | 1.00 | 60.32 |
| 3645 | CA | TRP | 712 | 47.660 | 43.044 | 34.534 | 1.00 | 65.58 |
| 3646 | CB | TRP | 712 | 48.270 | 44.168 | 33.711 | 1.00 | 59.75 |
| 3647 | CG | TRP | 712 | 48.272 | 45.426 | 34.444 | 1.00 | 58.80 |
| 3648 | CD2 | TRP | 712 | 47.148 | 46.271 | 34.668 | 1.00 | 64.37 |
| 3649 | CE2 | TRP | 712 | 47.577 | 47.322 | 35.506 | 1.00 | 59.23 |
| 3650 | CE3 | TRP | 712 | 45.812 | 46.240 | 34.245 | 1.00 | 62.90 |
| 3651 | CD1 | TRP | 712 | 49.313 | 45.976 | 35.124 | 1.00 | 62.87 |
| 3652 | NE1 | TRP | 712 | 48.905 | 47.118 | 35.770 | 1.00 | 62.24 |
| 3653 | CZ2 | TRP | 712 | 46.719 | 48.335 | 35.935 | 1.00 | 60.25 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3654 | CZ3 | TRP | 712 | 44.954 | 47.247 | 34.672 | 1.00 | 61.54 |
| 3655 | CH2 | TRP | 712 | 45.413 | 48.281 | 35.511 | 1.00 | 58.28 |
| 3656 | C | TRP | 712 | 47.538 | 41.811 | 33.663 | 1.00 | 61.45 |
| 3657 | O | TRP | 712 | 46.531 | 41.623 | 32.992 | 1.00 | 63.89 |
| 3658 | N | GLN | 713 | 48.561 | 40.974 | 33.646 | 1.00 | 62.44 |
| 3659 | CA | GLN | 713 | 48.493 | 39.778 | 32.823 | 1.00 | 60.84 |
| 3660 | CB | GLN | 713 | 49.865 | 39.088 | 32.761 | 1.00 | 59.49 |
| 3661 | CG | GLN | 713 | 50.495 | 39.114 | 31.371 | 1.00 | 61.40 |
| 3662 | CD | GLN | 713 | 49.624 | 38.414 | 30.336 | 1.00 | 60.98 |
| 3663 | OE1 | GLN | 713 | 49.306 | 38.980 | 29.285 | 1.00 | 60.97 |
| 3664 | NE2 | GLN | 713 | 49.232 | 37.173 | 30.629 | 1.00 | 62.19 |
| 3665 | C | GLN | 713 | 47.446 | 38.847 | 33.420 | 1.00 | 64.17 |
| 3666 | O | GLN | 713 | 46.843 | 38.037 | 32.722 | 1.00 | 62.77 |
| 3667 | N | ARG | 714 | 47.242 | 38.993 | 34.724 | 1.00 | 60.50 |
| 3668 | CA | ARG | 714 | 46.276 | 38.203 | 35.481 | 1.00 | 60.81 |
| 3669 | CB | ARG | 714 | 46.560 | 38.352 | 36.978 | 1.00 | 61.22 |
| 3670 | CG | ARG | 714 | 45.609 | 37.613 | 37.897 | 1.00 | 60.90 |
| 3671 | CD | ARG | 714 | 45.858 | 36.130 | 37.894 | 1.00 | 60.47 |
| 3672 | NE | ARG | 714 | 44.999 | 35.455 | 38.858 | 1.00 | 60.72 |
| 3673 | CZ | ARG | 714 | 44.777 | 34.145 | 38.860 | 1.00 | 62.42 |
| 3674 | NH1 | ARG | 714 | 45.358 | 33.382 | 37.943 | 1.00 | 57.63 |
| 3675 | NH2 | ARG | 714 | 43.967 | 33.601 | 39.762 | 1.00 | 61.24 |
| 3676 | C | ARG | 714 | 44.877 | 38.722 | 35.174 | 1.00 | 61.77 |
| 3677 | O | ARG | 714 | 43.930 | 37.949 | 35.007 | 1.00 | 61.51 |
| 3678 | N | PHE | 715 | 44.765 | 40.044 | 35.107 | 1.00 | 59.93 |
| 3679 | CA | PHE | 715 | 43.502 | 40.691 | 34.809 | 1.00 | 62.80 |
| 3680 | CB | PHE | 715 | 43.630 | 42.203 | 34.932 | 1.00 | 61.91 |
| 3681 | CG | PHE | 715 | 42.335 | 42.909 | 34.749 | 1.00 | 63.38 |
| 3682 | CD1 | PHE | 715 | 41.340 | 42.779 | 35.706 | 1.00 | 62.36 |
| 3683 | CD2 | PHE | 715 | 42.062 | 43.610 | 33.581 | 1.00 | 62.07 |
| 3684 | CE1 | PHE | 715 | 40.091 | 43.323 | 35.504 | 1.00 | 60.49 |
| 3685 | CE2 | PHE | 715 | 40.815 | 44.162 | 33.365 | 1.00 | 62.56 |
| 3686 | CZ | PHE | 715 | 39.823 | 44.017 | 34.328 | 1.00 | 61.44 |
| 3687 | C | PHE | 715 | 43.046 | 40.353 | 33.395 | 1.00 | 60.17 |
| 3688 | O | PHE | 715 | 41.849 | 40.288 | 33.115 | 1.00 | 59.69 |
| 3689 | N | TYR | 716 | 44.017 | 40.157 | 32.507 | 1.00 | 61.56 |
| 3690 | CA | TYR | 716 | 43.749 | 39.820 | 31.116 | 1.00 | 60.99 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3691 | CB | TYR | 716 | 45.043 | 39.870 | 30.297 | 1.00 | 62.72 |
| 3692 | CG | TYR | 716 | 44.810 | 39.609 | 28.828 | 1.00 | 61.29 |
| 3693 | CD1 | TYR | 716 | 44.115 | 40.530 | 28.047 | 1.00 | 57.90 |
| 3694 | CE1 | TYR | 716 | 43.790 | 40.253 | 26.728 | 1.00 | 62.63 |
| 3695 | CD2 | TYR | 716 | 45.188 | 38.399 | 28.242 | 1.00 | 65.06 |
| 3696 | CE2 | TYR | 716 | 44.866 | 38.111 | 26.923 | 1.00 | 60.30 |
| 3697 | CZ | TYR | 716 | 44.161 | 39.044 | 26.172 | 1.00 | 62.23 |
| 3698 | OH | TYR | 716 | 43.798 | 38.768 | 24.870 | 1.00 | 58.39 |
| 3699 | C | TYR | 716 | 43.157 | 38.419 | 31.036 | 1.00 | 62.09 |
| 3700 | O | TYR | 716 | 42.085 | 38.213 | 30.469 | 1.00 | 60.05 |
| 3701 | N | GLN | 717 | 43.875 | 37.460 | 31.611 | 1.00 | 62.41 |
| 3702 | CA | GLN | 717 | 43.449 | 36.071 | 31.623 | 1.00 | 60.55 |
| 3703 | CB | GLN | 717 | 44.409 | 35.222 | 32.465 | 1.00 | 59.06 |
| 3704 | CG | GLN | 717 | 45.855 | 35.166 | 31.978 | 1.00 | 60.27 |
| 3705 | CD | GLN | 717 | 46.758 | 34.357 | 32.919 | 1.00 | 60.87 |
| 3706 | OE1 | GLN | 717 | 46.844 | 34.639 | 34.124 | 1.00 | 61.07 |
| 3707 | NE2 | GLN | 717 | 47.437 | 33.352 | 32.369 | 1.00 | 62.22 |
| 3708 | C | GLN | 717 | 42.048 | 35.931 | 32.194 | 1.00 | 59.73 |
| 3709 | O | GLN | 717 | 41.156 | 35.383 | 31.545 | 1.00 | 64.26 |
| 3710 | N | LEU | 718 | 41.867 | 36.428 | 33.415 | 1.00 | 58.62 |
| 3711 | CA | LEU | 718 | 40.582 | 36.348 | 34.101 | 1.00 | 60.65 |
| 3712 | CB | LEU | 718 | 40.708 | 36.899 | 35.530 | 1.00 | 61.42 |
| 3713 | CG | LEU | 718 | 41.661 | 36.158 | 36.487 | 1.00 | 61.76 |
| 3714 | CD1 | LEU | 718 | 41.717 | 36.889 | 37.809 | 1.00 | 60.77 |
| 3715 | CD2 | LEU | 718 | 41.210 | 34.721 | 36.701 | 1.00 | 59.74 |
| 3716 | C | LEU | 718 | 39.427 | 37.034 | 33.375 | 1.00 | 59.70 |
| 3717 | O | LEU | 718 | 38.330 | 36.495 | 33.327 | 1.00 | 60.84 |
| 3718 | N | THR | 719 | 39.661 | 38.211 | 32.812 | 1.00 | 62.09 |
| 3719 | CA | THR | 719 | 38.608 | 38.925 | 32.095 | 1.00 | 59.89 |
| 3720 | CB | THR | 719 | 38.985 | 40.387 | 31.900 | 1.00 | 63.01 |
| 3721 | OG1 | THR | 719 | 40.248 | 40.465 | 31.237 | 1.00 | 63.25 |
| 3722 | CG2 | THR | 719 | 39.087 | 41.082 | 33.240 | 1.00 | 61.82 |
| 3723 | C | THR | 719 | 38.329 | 38.291 | 30.733 | 1.00 | 61.35 |
| 3724 | O | THR | 719 | 37.349 | 38.622 | 30.062 | 1.00 | 61.10 |
| 3725 | N | LYS | 720 | 39.203 | 37.371 | 30.341 | 1.00 | 62.98 |
| 3726 | CA | LYS | 720 | 39.074 | 36.651 | 29.079 | 1.00 | 61.71 |
| 3727 | CB | LYS | 720 | 40.460 | 36.209 | 28.588 | 1.00 | 62.48 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3728 | CG | LYS | 720 | 40.500 | 35.805 | 27.128 | 1.00 | 58.53 |
| 3729 | CD | LYS | 720 | 40.038 | 36.972 | 26.251 | 1.00 | 59.89 |
| 3730 | CE | LYS | 720 | 39.616 | 36.524 | 24.842 | 1.00 | 58.48 |
| 3731 | NZ | LYS | 720 | 38.772 | 37.570 | 24.209 | 1.00 | 60.64 |
| 3732 | C | LYS | 720 | 38.184 | 35.430 | 29.331 | 1.00 | 61.74 |
| 3733 | O | LYS | 720 | 37.479 | 34.962 | 28.438 | 1.00 | 61.10 |
| 3734 | N | LEU | 721 | 38.228 | 34.923 | 30.560 | 1.00 | 61.01 |
| 3735 | CA | LEU | 721 | 37.417 | 33.776 | 30.938 | 1.00 | 64.67 |
| 3736 | CB | LEU | 721 | 37.897 | 33.187 | 32.268 | 1.00 | 59.87 |
| 3737 | CG | LEU | 721 | 37.656 | 31.707 | 32.594 | 1.00 | 61.04 |
| 3738 | CD1 | LEU | 721 | 37.766 | 31.549 | 34.095 | 1.00 | 57.32 |
| 3739 | CD2 | LEU | 721 | 36.292 | 31.224 | 32.130 | 1.00 | 59.37 |
| 3740 | C | LEU | 721 | 35.983 | 34.277 | 31.078 | 1.00 | 62.25 |
| 3741 | O | LEU | 721 | 35.031 | 33.533 | 30.860 | 1.00 | 57.52 |
| 3742 | N | LEU | 722 | 35.830 | 35.540 | 31.455 | 1.00 | 62.01 |
| 3743 | CA | LEU | 722 | 34.503 | 36.113 | 31.591 | 1.00 | 62.92 |
| 3744 | CB | LEU | 722 | 34.578 | 37.497 | 32.246 | 1.00 | 61.91 |
| 3745 | CG | LEU | 722 | 34.841 | 37.559 | 33.754 | 1.00 | 58.92 |
| 3746 | CD1 | LEU | 722 | 34.946 | 38.986 | 34.193 | 1.00 | 62.12 |
| 3747 | CD2 | LEU | 722 | 33.728 | 36.876 | 34.507 | 1.00 | 62.52 |
| 3748 | C | LEU | 722 | 33.949 | 36.226 | 30.180 | 1.00 | 59.20 |
| 3749 | O | LEU | 722 | 32.883 | 35.697 | 29.854 | 1.00 | 60.43 |
| 3750 | N | ASP | 723 | 34.714 | 36.911 | 29.344 | 1.00 | 60.22 |
| 3751 | CA | ASP | 723 | 34.379 | 37.143 | 27.952 | 1.00 | 60.07 |
| 3752 | CB | ASP | 723 | 35.607 | 37.697 | 27.248 | 1.00 | 59.74 |
| 3753 | CG | ASP | 723 | 35.437 | 39.115 | 26.832 | 1.00 | 60.94 |
| 3754 | OD1 | ASP | 723 | 34.869 | 39.890 | 27.626 | 1.00 | 63.82 |
| 3755 | OD2 | ASP | 723 | 35.883 | 39.445 | 25.713 | 1.00 | 63.76 |
| 3756 | C | ASP | 723 | 33.909 | 35.899 | 27.214 | 1.00 | 61.21 |
| 3757 | O | ASP | 723 | 33.108 | 35.981 | 26.292 | 1.00 | 61.67 |
| 3758 | N | SER | 724 | 34.414 | 34.743 | 27.613 | 1.00 | 62.19 |
| 3759 | CA | SER | 724 | 34.054 | 33.521 | 26.923 | 1.00 | 62.38 |
| 3760 | CB | SER | 724 | 35.256 | 32.578 | 26.876 | 1.00 | 61.26 |
| 3761 | OG | SER | 724 | 35.743 | 32.308 | 28.175 | 1.00 | 62.27 |
| 3762 | C | SER | 724 | 32.869 | 32.823 | 27.539 | 1.00 | 63.42 |
| 3763 | O | SER | 724 | 32.419 | 31.785 | 27.054 | 1.00 | 60.80 |
| 3764 | N | MET | 725 | 32.352 | 33.395 | 28.613 | 1.00 | 61.24 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3765 | CA | MET | 725 | 31.209 | 32.794 | 29.265 | 1.00 | 60.73 |
| 3766 | CB | MET | 725 | 30.955 | 33.461 | 30.608 | 1.00 | 62.85 |
| 3767 | CG | MET | 725 | 30.460 | 32.506 | 31.654 | 1.00 | 63.01 |
| 3768 | SD | MET | 725 | 31.773 | 31.403 | 32.092 | 1.00 | 57.07 |
| 3769 | CE | MET | 725 | 30.949 | 30.380 | 33.178 | 1.00 | 59.44 |
| 3770 | C | MET | 725 | 30.008 | 32.987 | 28.352 | 1.00 | 59.13 |
| 3771 | O | MET | 725 | 29.022 | 32.254 | 28.437 | 1.00 | 62.11 |
| 3772 | N | HIS | 726 | 30.105 | 33.973 | 27.465 | 1.00 | 58.55 |
| 3773 | CA | HIS | 726 | 29.021 | 34.267 | 26.547 | 1.00 | 60.99 |
| 3774 | CB | HIS | 726 | 29.302 | 35.554 | 25.769 | 1.00 | 62.45 |
| 3775 | CG | HIS | 726 | 29.036 | 36.801 | 26.557 | 1.00 | 59.03 |
| 3776 | CD2 | HIS | 726 | 27.909 | 37.248 | 27.161 | 1.00 | 60.25 |
| 3777 | ND1 | HIS | 726 | 30.003 | 37.753 | 26.800 | 1.00 | 61.07 |
| 3778 | CE1 | HIS | 726 | 29.484 | 38.731 | 27.520 | 1.00 | 61.93 |
| 3779 | NE2 | HIS | 726 | 28.215 | 38.451 | 27.752 | 1.00 | 59.78 |
| 3780 | C | HIS | 726 | 28.773 | 33.116 | 25.601 | 1.00 | 58.35 |
| 3781 | O | HIS | 726 | 27.638 | 32.696 | 25.438 | 1.00 | 61.58 |
| 3782 | N | GLU | 727 | 29.816 | 32.571 | 24.993 | 1.00 | 62.79 |
| 3783 | CA | GLU | 727 | 29.574 | 31.461 | 24.086 | 1.00 | 60.53 |
| 3784 | CB | GLU | 727 | 30.693 | 31.307 | 23.055 | 1.00 | 59.90 |
| 3785 | CG | GLU | 727 | 32.005 | 30.809 | 23.578 | 1.00 | 57.32 |
| 3786 | CD | GLU | 727 | 32.838 | 30.187 | 22.473 | 1.00 | 59.99 |
| 3787 | OE1 | GLU | 727 | 34.057 | 30.013 | 22.680 | 1.00 | 62.44 |
| 3788 | OE2 | GLU | 727 | 32.275 | 29.862 | 21.400 | 1.00 | 63.27 |
| 3789 | C | GLU | 727 | 29.351 | 30.138 | 24.791 | 1.00 | 59.24 |
| 3790 | O | GLU | 727 | 28.779 | 29.233 | 24.203 | 1.00 | 62.98 |
| 3791 | N | VAL | 728 | 29.812 | 29.992 | 26.029 | 1.00 | 60.19 |
| 3792 | CA | VAL | 728 | 29.546 | 28.732 | 26.721 | 1.00 | 58.29 |
| 3793 | CB | VAL | 728 | 30.493 | 28.481 | 27.956 | 1.00 | 58.87 |
| 3794 | CG1 | VAL | 728 | 31.261 | 29.728 | 28.304 | 1.00 | 60.81 |
| 3795 | CG2 | VAL | 728 | 29.694 | 28.002 | 29.152 | 1.00 | 61.95 |
| 3796 | C | VAL | 728 | 28.082 | 28.825 | 27.145 | 1.00 | 59.41 |
| 3797 | O | VAL | 728 | 27.335 | 27.848 | 27.046 | 1.00 | 61.38 |
| 3798 | N | VAL | 729 | 27.670 | 30.014 | 27.585 | 1.00 | 59.91 |
| 3799 | CA | VAL | 729 | 26.283 | 30.238 | 27.971 | 1.00 | 60.73 |
| 3800 | CB | VAL | 729 | 26.072 | 31.664 | 28.504 | 1.00 | 63.53 |
| 3801 | CG1 | VAL | 729 | 24.602 | 32.062 | 28.406 | 1.00 | 62.52 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3802 | CG2 | VAL | 729 | 26.513 | 31.725 | 29.946 | 1.00 | 62.40 |
| 3803 | C | VAL | 729 | 25.397 | 30.004 | 26.749 | 1.00 | 62.11 |
| 3804 | O | VAL | 729 | 24.279 | 29.493 | 26.868 | 1.00 | 58.35 |
| 3805 | N | GLU | 730 | 25.894 | 30.372 | 25.571 | 1.00 | 59.30 |
| 3806 | CA | GLU | 730 | 25.133 | 30.146 | 24.347 | 1.00 | 61.37 |
| 3807 | CB | GLU | 730 | 25.940 | 30.546 | 23.125 | 1.00 | 57.56 |
| 3808 | CG | GLU | 730 | 25.202 | 31.467 | 22.199 | 1.00 | 61.79 |
| 3809 | CD | GLU | 730 | 25.875 | 31.569 | 20.859 | 1.00 | 62.47 |
| 3810 | OE1 | GLU | 730 | 27.039 | 32.032 | 20.811 | 1.00 | 62.43 |
| 3811 | OE2 | GLU | 730 | 25.235 | 31.178 | 19.858 | 1.00 | 61.70 |
| 3812 | C | GLU | 730 | 24.832 | 28.660 | 24.267 | 1.00 | 60.90 |
| 3813 | O | GLU | 730 | 23.701 | 28.244 | 24.449 | 1.00 | 63.19 |
| 3814 | N | ASN | 731 | 25.864 | 27.866 | 24.013 | 1.00 | 61.88 |
| 3815 | CA | ASN | 731 | 25.729 | 26.416 | 23.919 | 1.00 | 63.18 |
| 3816 | CB | ASN | 731 | 27.109 | 25.761 | 23.859 | 1.00 | 56.96 |
| 3817 | CG | ASN | 731 | 27.516 | 25.393 | 22.449 | 1.00 | 61.50 |
| 3818 | OD1 | ASN | 731 | 26.909 | 24.515 | 21.808 | 1.00 | 59.01 |
| 3819 | ND2 | ASN | 731 | 28.552 | 26.059 | 21.953 | 1.00 | 62.29 |
| 3820 | C | ASN | 731 | 24.927 | 25.773 | 25.045 | 1.00 | 62.97 |
| 3821 | O | ASN | 731 | 24.251 | 24.772 | 24.834 | 1.00 | 60.93 |
| 3822 | N | LEU | 732 | 25.002 | 26.330 | 26.246 | 1.00 | 59.50 |
| 3823 | CA | LEU | 732 | 24.241 | 25.737 | 27.326 | 1.00 | 57.74 |
| 3824 | CB | LEU | 732 | 24.826 | 26.120 | 28.668 | 1.00 | 61.00 |
| 3825 | CG | LEU | 732 | 25.964 | 25.172 | 29.078 | 1.00 | 60.69 |
| 3826 | CD1 | LEU | 732 | 26.278 | 25.584 | 30.458 | 1.00 | 63.28 |
| 3827 | CD2 | LEU | 732 | 25.589 | 23.663 | 29.058 | 1.00 | 63.30 |
| 3828 | C | LEU | 732 | 22.761 | 26.089 | 27.240 | 1.00 | 61.98 |
| 3829 | O | LEU | 732 | 21.912 | 25.269 | 27.574 | 1.00 | 63.87 |
| 3830 | N | LEU | 733 | 22.456 | 27.294 | 26.762 | 1.00 | 63.36 |
| 3831 | CA | LEU | 733 | 21.073 | 27.744 | 26.596 | 1.00 | 57.15 |
| 3832 | CB | LEU | 733 | 21.040 | 29.241 | 26.290 | 1.00 | 60.30 |
| 3833 | CG | LEU | 733 | 21.134 | 30.193 | 27.481 | 1.00 | 57.97 |
| 3834 | CD1 | LEU | 733 | 21.471 | 31.571 | 26.956 | 1.00 | 62.48 |
| 3835 | CD2 | LEU | 733 | 19.824 | 30.212 | 28.272 | 1.00 | 62.63 |
| 3836 | C | LEU | 733 | 20.354 | 26.997 | 25.470 | 1.00 | 63.32 |
| 3837 | O | LEU | 733 | 19.256 | 26.475 | 25.655 | 1.00 | 61.30 |
| 3838 | N | ASN | 734 | 20.965 | 26.972 | 24.292 | 1.00 | 57.57 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3839 | CA | ASN | 734 | 20.376 | 26.285 | 23.159 | 1.00 | 59.44 |
| 3840 | CB | ASN | 734 | 21.363 | 26.277 | 21.994 | 1.00 | 57.94 |
| 3841 | CG | ASN | 734 | 21.671 | 27.682 | 21.495 | 1.00 | 59.18 |
| 3842 | OD1 | ASN | 734 | 22.072 | 28.556 | 22.268 | 1.00 | 63.60 |
| 3843 | ND2 | ASN | 734 | 21.476 | 27.908 | 20.202 | 1.00 | 63.41 |
| 3844 | C | ASN | 734 | 20.038 | 24.872 | 23.594 | 1.00 | 60.75 |
| 3845 | O | ASN | 734 | 18.904 | 24.423 | 23.453 | 1.00 | 62.53 |
| 3846 | N | TYR | 735 | 21.017 | 24.177 | 24.151 | 1.00 | 61.37 |
| 3847 | CA | TYR | 735 | 20.762 | 22.823 | 24.597 | 1.00 | 58.79 |
| 3848 | CB | TYR | 735 | 22.058 | 22.201 | 25.158 | 1.00 | 58.40 |
| 3849 | CG | TYR | 735 | 22.087 | 20.717 | 24.978 | 1.00 | 60.44 |
| 3850 | CD1 | TYR | 735 | 21.309 | 19.890 | 25.780 | 1.00 | 60.76 |
| 3851 | CE1 | TYR | 735 | 21.240 | 18.509 | 25.562 | 1.00 | 60.42 |
| 3852 | CD2 | TYR | 735 | 22.815 | 20.134 | 23.938 | 1.00 | 61.19 |
| 3853 | CE2 | TYR | 735 | 22.750 | 18.758 | 23.696 | 1.00 | 62.36 |
| 3854 | CZ | TYR | 735 | 21.961 | 17.950 | 24.519 | 1.00 | 59.04 |
| 3855 | OH | TYR | 735 | 21.899 | 16.583 | 24.313 | 1.00 | 58.73 |
| 3856 | C | TYR | 735 | 19.649 | 22.877 | 25.657 | 1.00 | 61.17 |
| 3857 | O | TYR | 735 | 18.858 | 21.945 | 25.790 | 1.00 | 63.18 |
| 3858 | N | CYS | 736 | 19.574 | 23.995 | 26.373 | 1.00 | 63.51 |
| 3859 | CA | CYS | 736 | 18.563 | 24.202 | 27.403 | 1.00 | 65.29 |
| 3860 | CB | CYS | 736 | 18.922 | 25.433 | 28.228 | 1.00 | 64.15 |
| 3861 | SG | CYS | 736 | 17.642 | 25.957 | 29.339 | 1.00 | 60.76 |
| 3862 | C | CYS | 736 | 17.183 | 24.389 | 26.779 | 1.00 | 63.45 |
| 3863 | O | CYS | 736 | 16.251 | 23.645 | 27.090 | 1.00 | 61.32 |
| 3864 | N | PHE | 737 | 17.061 | 25.391 | 25.906 | 1.00 | 59.77 |
| 3865 | CA | PHE | 737 | 15.808 | 25.688 | 25.209 | 1.00 | 61.17 |
| 3866 | CB | PHE | 737 | 16.014 | 26.792 | 24.175 | 1.00 | 59.61 |
| 3867 | CG | PHE | 737 | 16.348 | 28.129 | 24.764 | 1.00 | 58.80 |
| 3868 | CD1 | PHE | 737 | 16.060 | 28.414 | 26.096 | 1.00 | 59.85 |
| 3869 | CD2 | PHE | 737 | 16.924 | 29.119 | 23.979 | 1.00 | 61.58 |
| 3870 | CE1 | PHE | 737 | 16.340 | 29.669 | 26.638 | 1.00 | 59.75 |
| 3871 | CE2 | PHE | 737 | 17.207 | 30.374 | 24.510 | 1.00 | 61.15 |
| 3872 | CZ | PHE | 737 | 16.914 | 30.649 | 25.843 | 1.00 | 59.90 |
| 3873 | C | PHE | 737 | 15.280 | 24.462 | 24.484 | 1.00 | 61.86 |
| 3874 | O | PHE | 737 | 14.153 | 24.024 | 24.714 | 1.00 | 64.04 |
| 3875 | N | GLN | 738 | 16.108 | 23.932 | 23.590 | 1.00 | 60.57 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3876 | CA | GLN | 738 | 15.786 | 22.752 | 22.798 | 1.00 | 61.63 |
| 3877 | CB | GLN | 738 | 17.078 | 22.181 | 22.220 | 1.00 | 62.59 |
| 3878 | CG | GLN | 738 | 16.989 | 20.805 | 21.575 | 1.00 | 61.50 |
| 3879 | CD | GLN | 738 | 18.368 | 20.144 | 21.521 | 1.00 | 62.73 |
| 3880 | OE1 | GLN | 738 | 18.581 | 19.062 | 22.093 | 1.00 | 60.75 |
| 3881 | NE2 | GLN | 738 | 19.321 | 20.808 | 20.853 | 1.00 | 61.08 |
| 3882 | C | GLN | 738 | 15.043 | 21.677 | 23.591 | 1.00 | 62.87 |
| 3883 | O | GLN | 738 | 13.970 | 21.235 | 23.180 | 1.00 | 62.00 |
| 3884 | N | THR | 739 | 15.595 | 21.262 | 24.725 | 1.00 | 62.54 |
| 3885 | CA | THR | 739 | 14.937 | 20.228 | 25.513 | 1.00 | 62.00 |
| 3886 | CB | THR | 739 | 15.883 | 19.572 | 26.529 | 1.00 | 61.40 |
| 3887 | OG1 | THR | 739 | 16.041 | 20.437 | 27.659 | 1.00 | 59.46 |
| 3888 | CG2 | THR | 739 | 17.234 | 19.302 | 25.902 | 1.00 | 67.45 |
| 3889 | C | THR | 739 | 13.721 | 20.740 | 26.282 | 1.00 | 60.54 |
| 3890 | O | THR | 739 | 12.911 | 19.949 | 26.758 | 1.00 | 60.32 |
| 3891 | N | PHE | 740 | 13.589 | 22.049 | 26.433 | 1.00 | 60.26 |
| 3892 | CA | PHE | 740 | 12.426 | 22.572 | 27.136 | 1.00 | 60.59 |
| 3893 | CB | PHE | 740 | 12.645 | 24.013 | 27.586 | 1.00 | 60.53 |
| 3894 | CG | PHE | 740 | 11.387 | 24.682 | 28.073 | 1.00 | 60.33 |
| 3895 | CD1 | PHE | 740 | 10.976 | 24.543 | 29.399 | 1.00 | 60.24 |
| 3896 | CD2 | PHE | 740 | 10.591 | 25.417 | 27.196 | 1.00 | 60.88 |
| 3897 | CE1 | PHE | 740 | 9.794 | 25.124 | 29.842 | 1.00 | 62.19 |
| 3898 | CE2 | PHE | 740 | 9.407 | 26.001 | 27.629 | 1.00 | 63.06 |
| 3899 | CZ | PHE | 740 | 9.005 | 25.857 | 28.954 | 1.00 | 59.45 |
| 3900 | C | PHE | 740 | 11.269 | 22.562 | 26.161 | 1.00 | 62.20 |
| 3901 | O | PHE | 740 | 10.102 | 22.514 | 26.560 | 1.00 | 60.22 |
| 3902 | N | LEU | 741 | 11.619 | 22.631 | 24.877 | 1.00 | 61.98 |
| 3903 | CA | LEU | 741 | 10.650 | 22.665 | 23.783 | 1.00 | 61.09 |
| 3904 | CB | LEU | 741 | 11.158 | 23.561 | 22.656 | 1.00 | 63.24 |
| 3905 | CG | LEU | 741 | 11.286 | 25.053 | 22.919 | 1.00 | 57.80 |
| 3906 | CD1 | LEU | 741 | 11.680 | 25.732 | 21.617 | 1.00 | 59.82 |
| 3907 | CD2 | LEU | 741 | 9.966 | 25.608 | 23.455 | 1.00 | 59.42 |
| 3908 | C | LEU | 741 | 10.313 | 21.316 | 23.170 | 1.00 | 60.22 |
| 3909 | O | LEU | 741 | 9.748 | 21.267 | 22.079 | 1.00 | 59.13 |
| 3910 | N | ASP | 742 | 10.662 | 20.230 | 23.845 | 1.00 | 61.37 |
| 3911 | CA | ASP | 742 | 10.388 | 18.914 | 23.309 | 1.00 | 61.75 |
| 3912 | CB | ASP | 742 | 11.679 | 18.315 | 22.733 | 1.00 | 62.29 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3913 | CG | ASP | 742 | 11.476 | 16.916 | 22.145 | 1.00 | 59.85 |
| 3914 | OD1 | ASP | 742 | 12.450 | 16.354 | 21.576 | 1.00 | 62.02 |
| 3915 | OD2 | ASP | 742 | 10.348 | 16.378 | 22.253 | 1.00 | 63.60 |
| 3916 | C | ASP | 742 | 9.843 | 18.041 | 24.420 | 1.00 | 61.37 |
| 3917 | O | ASP | 742 | 10.614 | 17.412 | 25.153 | 1.00 | 62.69 |
| 3918 | N | LYS | 743 | 8.517 | 18.018 | 24.564 | 1.00 | 61.79 |
| 3919 | CA | LYS | 743 | 7.882 | 17.183 | 25.595 | 1.00 | 58.96 |
| 3920 | CB | LYS | 743 | 6.381 | 17.501 | 25.727 | 1.00 | 62.19 |
| 3921 | CG | LYS | 743 | 6.056 | 18.836 | 26.404 | 1.00 | 57.47 |
| 3922 | CD | LYS | 743 | 4.545 | 19.047 | 26.473 | 1.00 | 59.18 |
| 3923 | CE | LYS | 743 | 4.180 | 20.313 | 27.232 | 1.00 | 61.36 |
| 3924 | NZ | LYS | 743 | 2.699 | 20.507 | 27.295 | 1.00 | 57.73 |
| 3925 | C | LYS | 743 | 8.055 | 15.688 | 25.281 | 1.00 | 61.20 |
| 3926 | O | LYS | 743 | 7.912 | 14.843 | 26.165 | 1.00 | 60.18 |
| 3927 | N | THR | 744 | 8.366 | 15.380 | 24.020 | 1.00 | 59.67 |
| 3928 | CA | THR | 744 | 8.580 | 14.007 | 23.554 | 1.00 | 61.15 |
| 3929 | CB | THR | 744 | 8.792 | 13.974 | 22.047 | 1.00 | 59.67 |
| 3930 | OG1 | THR | 744 | 7.881 | 14.890 | 21.426 | 1.00 | 62.19 |
| 3931 | CG2 | THR | 744 | 8.550 | 12.574 | 21.513 | 1.00 | 63.96 |
| 3932 | C | THR | 744 | 9.818 | 13.406 | 24.202 | 1.00 | 61.58 |
| 3933 | O | THR | 744 | 9.976 | 12.195 | 24.261 | 1.00 | 62.58 |
| 3934 | N | MET | 745 | 10.711 | 14.279 | 24.646 | 1.00 | 57.60 |
| 3935 | CA | MET | 745 | 11.933 | 13.887 | 25.334 | 1.00 | 62.60 |
| 3936 | CB | MET | 745 | 12.982 | 14.976 | 25.147 | 1.00 | 59.01 |
| 3937 | CG | MET | 745 | 14.366 | 14.645 | 25.612 | 1.00 | 60.84 |
| 3938 | SD | MET | 745 | 15.440 | 15.948 | 24.965 | 1.00 | 58.90 |
| 3939 | CE | MET | 745 | 16.317 | 15.039 | 23.610 | 1.00 | 62.07 |
| 3940 | C | MET | 745 | 11.435 | 13.843 | 26.774 | 1.00 | 63.96 |
| 3941 | O | MET | 745 | 11.973 | 13.137 | 27.629 | 1.00 | 64.77 |
| 3942 | N | SER | 746 | 10.394 | 14.635 | 27.025 | 1.00 | 59.79 |
| 3943 | CA | SER | 746 | 9.744 | 14.657 | 28.326 | 1.00 | 60.83 |
| 3944 | CB | SER | 746 | 9.149 | 13.249 | 28.576 | 1.00 | 61.05 |
| 3945 | OG | SER | 746 | 8.512 | 13.111 | 29.842 | 1.00 | 61.76 |
| 3946 | C | SER | 746 | 10.597 | 15.089 | 29.533 | 1.00 | 61.97 |
| 3947 | O | SER | 746 | 10.576 | 14.408 | 30.545 | 1.00 | 61.88 |
| 3948 | N | ILE | 747 | 11.322 | 16.207 | 29.443 | 1.00 | 60.50 |
| 3949 | CA | ILE | 747 | 12.140 | 16.678 | 30.571 | 1.00 | 61.46 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3950 | CB | ILE | 747 | 13.557 | 17.115 | 30.098 | 1.00 | 57.79 |
| 3951 | CG2 | ILE | 747 | 14.374 | 17.635 | 31.275 | 1.00 | 62.83 |
| 3952 | CG1 | ILE | 747 | 14.282 | 15.911 | 29.484 | 1.00 | 61.46 |
| 3953 | CD1 | ILE | 747 | 15.664 | 16.211 | 28.976 | 1.00 | 65.11 |
| 3954 | C | ILE | 747 | 11.441 | 17.829 | 31.318 | 1.00 | 63.68 |
| 3955 | O | ILE | 747 | 11.166 | 18.891 | 30.747 | 1.00 | 62.48 |
| 3956 | N | GLU | 748 | 11.167 | 17.597 | 32.601 | 1.00 | 59.16 |
| 3957 | CA | GLU | 748 | 10.457 | 18.546 | 33.466 | 1.00 | 62.34 |
| 3958 | CB | GLU | 748 | 9.803 | 17.736 | 34.620 | 1.00 | 60.97 |
| 3959 | CG | GLU | 748 | 8.628 | 18.410 | 35.400 | 1.00 | 63.60 |
| 3960 | CD | GLU | 748 | 7.998 | 17.505 | 36.516 | 1.00 | 59.56 |
| 3961 | OE1 | GLU | 748 | 8.753 | 16.918 | 37.340 | 1.00 | 61.60 |
| 3962 | OE2 | GLU | 748 | 6.744 | 17.396 | 36.574 | 1.00 | 62.18 |
| 3963 | C | GLU | 748 | 11.333 | 19.701 | 34.022 | 1.00 | 59.89 |
| 3964 | O | GLU | 748 | 12.503 | 19.498 | 34.367 | 1.00 | 59.20 |
| 3965 | N | PHE | 749 | 10.781 | 20.913 | 34.046 | 1.00 | 62.20 |
| 3966 | CA | PHE | 749 | 11.484 | 22.079 | 34.601 | 1.00 | 60.36 |
| 3967 | CB | PHE | 749 | 11.773 | 23.202 | 33.571 | 1.00 | 63.43 |
| 3968 | CG | PHE | 749 | 12.801 | 22.827 | 32.506 | 1.00 | 62.31 |
| 3969 | CD1 | PHE | 749 | 12.604 | 21.790 | 31.624 | 1.00 | 63.18 |
| 3970 | CD2 | PHE | 749 | 13.948 | 23.639 | 32.305 | 1.00 | 58.27 |
| 3971 | CE1 | PHE | 749 | 13.461 | 21.555 | 30.545 | 1.00 | 59.47 |
| 3972 | CE2 | PHE | 749 | 14.821 | 23.409 | 31.221 | 1.00 | 59.31 |
| 3973 | CZ | PHE | 749 | 14.566 | 22.379 | 30.341 | 1.00 | 60.26 |
| 3974 | C | PHE | 749 | 10.491 | 22.634 | 35.646 | 1.00 | 57.34 |
| 3975 | O | PHE | 749 | 9.296 | 22.348 | 35.598 | 1.00 | 62.05 |
| 3976 | N | PRO | 750 | 10.971 | 23.425 | 36.612 | 1.00 | 61.11 |
| 3977 | CD | PRO | 750 | 12.322 | 23.572 | 37.178 | 1.00 | 62.36 |
| 3978 | CA | PRO | 750 | 9.955 | 23.918 | 37.535 | 1.00 | 63.79 |
| 3979 | CB | PRO | 750 | 10.745 | 24.124 | 38.834 | 1.00 | 59.84 |
| 3980 | CG | PRO | 750 | 12.072 | 24.510 | 38.341 | 1.00 | 61.92 |
| 3981 | C | PRO | 750 | 9.283 | 25.174 | 37.042 | 1.00 | 61.44 |
| 3982 | O | PRO | 750 | 9.000 | 25.325 | 35.852 | 1.00 | 61.38 |
| 3983 | N | GLU | 751 | 9.016 | 26.087 | 37.962 | 1.00 | 61.10 |
| 3984 | CA | GLU | 751 | 8.375 | 27.335 | 37.604 | 1.00 | 59.57 |
| 3985 | CB | GLU | 751 | 7.535 | 27.828 | 38.778 | 1.00 | 60.79 |
| 3986 | CG | GLU | 751 | 6.534 | 26.804 | 39.190 | 1.00 | 60.87 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 3987 | CD | GLU | 751 | 5.716 | 26.358 | 38.013 | 1.00 | 58.45 |
| 3988 | OE1 | GLU | 751 | 6.004 | 26.825 | 36.889 | 1.00 | 58.76 |
| 3989 | OE2 | GLU | 751 | 4.768 | 25.564 | 38.205 | 1.00 | 62.99 |
| 3990 | C | GLU | 751 | 9.449 | 28.328 | 37.275 | 1.00 | 59.04 |
| 3991 | O | GLU | 751 | 9.644 | 28.698 | 36.115 | 1.00 | 63.48 |
| 3992 | N | MET | 752 | 10.154 | 28.736 | 38.319 | 1.00 | 62.70 |
| 3993 | CA | MET | 752 | 11.223 | 29.693 | 38.184 | 1.00 | 60.75 |
| 3994 | CB | MET | 752 | 12.241 | 29.499 | 39.306 | 1.00 | 60.00 |
| 3995 | CG | MET | 752 | 13.222 | 30.641 | 39.392 | 1.00 | 60.74 |
| 3996 | SD | MET | 752 | 12.305 | 32.203 | 39.387 | 1.00 | 61.02 |
| 3997 | CE | MET | 752 | 12.040 | 32.394 | 41.073 | 1.00 | 58.25 |
| 3998 | C | MET | 752 | 11.919 | 29.549 | 36.847 | 1.00 | 61.43 |
| 3999 | O | MET | 752 | 12.062 | 30.515 | 36.103 | 1.00 | 63.92 |
| 4000 | N | LEU | 753 | 12.329 | 28.326 | 36.537 | 1.00 | 61.53 |
| 4001 | CA | LEU | 753 | 13.044 | 28.081 | 35.307 | 1.00 | 63.94 |
| 4002 | CB | LEU | 753 | 13.749 | 26.729 | 35.370 | 1.00 | 61.07 |
| 4003 | CG | LEU | 753 | 15.278 | 26.834 | 35.432 | 1.00 | 59.22 |
| 4004 | CD1 | LEU | 753 | 15.720 | 27.666 | 36.636 | 1.00 | 60.65 |
| 4005 | CD2 | LEU | 753 | 15.870 | 25.436 | 35.488 | 1.00 | 56.88 |
| 4006 | C | LEU | 753 | 12.182 | 28.179 | 34.073 | 1.00 | 62.24 |
| 4007 | O | LEU | 753 | 12.539 | 28.882 | 33.138 | 1.00 | 61.75 |
| 4008 | N | ALA | 754 | 11.049 | 27.488 | 34.061 | 1.00 | 58.32 |
| 4009 | CA | ALA | 754 | 10.156 | 27.536 | 32.902 | 1.00 | 60.89 |
| 4010 | CB | ALA | 754 | 8.856 | 26.790 | 33.209 | 1.00 | 62.87 |
| 4011 | C | ALA | 754 | 9.851 | 28.995 | 32.581 | 1.00 | 60.33 |
| 4012 | O | ALA | 754 | 10.031 | 29.471 | 31.454 | 1.00 | 61.42 |
| 4013 | N | GLU | 755 | 9.406 | 29.698 | 33.615 | 1.00 | 62.19 |
| 4014 | CA | GLU | 755 | 9.040 | 31.101 | 33.535 | 1.00 | 60.87 |
| 4015 | CB | GLU | 755 | 8.481 | 31.550 | 34.891 | 1.00 | 60.79 |
| 4016 | CG | GLU | 755 | 7.821 | 32.911 | 34.858 | 1.00 | 62.93 |
| 4017 | CD | GLU | 755 | 6.333 | 32.829 | 35.097 | 1.00 | 56.10 |
| 4018 | OE1 | GLU | 755 | 5.741 | 31.746 | 34.841 | 1.00 | 59.07 |
| 4019 | OE2 | GLU | 755 | 5.761 | 33.857 | 35.531 | 1.00 | 65.63 |
| 4020 | C | GLU | 755 | 10.163 | 32.053 | 33.106 | 1.00 | 64.44 |
| 4021 | O | GLU | 755 | 10.006 | 33.269 | 33.209 | 1.00 | 61.31 |
| 4022 | N | ILE | 756 | 11.296 | 31.528 | 32.653 | 1.00 | 60.43 |
| 4023 | CA | ILE | 756 | 12.382 | 32.396 | 32.187 | 1.00 | 58.74 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4024 | CB | ILE | 756 | 13.664 | 32.292 | 33.024 | 1.00 | 62.34 |
| 4025 | CG2 | ILE | 756 | 14.819 | 32.942 | 32.280 | 1.00 | 62.52 |
| 4026 | CG1 | ILE | 756 | 13.487 | 33.000 | 34.355 | 1.00 | 61.56 |
| 4027 | CD1 | ILE | 756 | 14.750 | 33.012 | 35.170 | 1.00 | 59.91 |
| 4028 | C | ILE | 756 | 12.725 | 31.933 | 30.799 | 1.00 | 61.14 |
| 4029 | O | ILE | 756 | 12.985 | 32.732 | 29.904 | 1.00 | 61.35 |
| 4030 | N | ILE | 757 | 12.728 | 30.618 | 30.639 | 1.00 | 58.95 |
| 4031 | CA | ILE | 757 | 13.026 | 30.016 | 29.362 | 1.00 | 60.81 |
| 4032 | CB | ILE | 757 | 12.990 | 28.467 | 29.480 | 1.00 | 60.69 |
| 4033 | CG2 | ILE | 757 | 12.522 | 27.838 | 28.197 | 1.00 | 64.94 |
| 4034 | CG1 | ILE | 757 | 14.378 | 27.948 | 29.883 | 1.00 | 60.19 |
| 4035 | CD1 | ILE | 757 | 14.463 | 27.436 | 31.313 | 1.00 | 62.89 |
| 4036 | C | ILE | 757 | 12.012 | 30.542 | 28.348 | 1.00 | 59.56 |
| 4037 | O | ILE | 757 | 12.397 | 30.991 | 27.276 | 1.00 | 61.79 |
| 4038 | N | THR | 758 | 10.726 | 30.521 | 28.702 | 1.00 | 61.66 |
| 4039 | CA | THR | 758 | 9.677 | 31.014 | 27.795 | 1.00 | 61.59 |
| 4040 | CB | THR | 758 | 8.224 | 30.722 | 28.323 | 1.00 | 63.29 |
| 4041 | OG1 | THR | 758 | 8.188 | 30.811 | 29.755 | 1.00 | 62.91 |
| 4042 | CG2 | THR | 758 | 7.754 | 29.343 | 27.874 | 1.00 | 61.73 |
| 4043 | C | THR | 758 | 9.809 | 32.516 | 27.566 | 1.00 | 60.10 |
| 4044 | O | THR | 758 | 9.735 | 33.002 | 26.423 | 1.00 | 59.44 |
| 4045 | N | ASN | 759 | 10.023 | 33.242 | 28.656 | 1.00 | 59.76 |
| 4046 | CA | ASN | 759 | 10.154 | 34.691 | 28.608 | 1.00 | 61.77 |
| 4047 | CB | ASN | 759 | 10.160 | 35.242 | 30.034 | 1.00 | 62.16 |
| 4048 | CG | ASN | 759 | 9.352 | 34.371 | 30.981 | 1.00 | 60.61 |
| 4049 | OD1 | ASN | 759 | 9.601 | 33.164 | 31.072 | 1.00 | 60.73 |
| 4050 | ND2 | ASN | 759 | 8.379 | 34.965 | 31.683 | 1.00 | 58.61 |
| 4051 | C | ASN | 759 | 11.430 | 35.091 | 27.886 | 1.00 | 63.83 |
| 4052 | O | ASN | 759 | 11.725 | 36.278 | 27.737 | 1.00 | 59.18 |
| 4053 | N | GLN | 760 | 12.191 | 34.099 | 27.439 | 1.00 | 60.77 |
| 4054 | CA | GLN | 760 | 13.431 | 34.395 | 26.742 | 1.00 | 60.74 |
| 4055 | CB | GLN | 760 | 14.637 | 34.295 | 27.690 | 1.00 | 63.92 |
| 4056 | CG | GLN | 760 | 14.546 | 35.101 | 28.992 | 1.00 | 63.35 |
| 4057 | CD | GLN | 760 | 15.114 | 36.508 | 28.896 | 1.00 | 61.91 |
| 4058 | OE1 | GLN | 760 | 16.231 | 36.714 | 28.423 | 1.00 | 61.24 |
| 4059 | NE2 | GLN | 760 | 14.351 | 37.482 | 29.367 | 1.00 | 62.64 |
| 4060 | C | GLN | 760 | 13.687 | 33.482 | 25.554 | 1.00 | 61.67 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4061 | O | GLN | 760 | 14.390 | 33.898 | 24.631 | 1.00 | 58.73 |
| 4062 | N | ILE | 761 | 13.124 | 32.264 | 25.563 | 1.00 | 63.30 |
| 4063 | CA | ILE | 761 | 13.368 | 31.305 | 24.476 | 1.00 | 62.33 |
| 4064 | CB | ILE | 761 | 12.102 | 30.454 | 24.088 | 1.00 | 62.35 |
| 4065 | CG2 | ILE | 761 | 12.345 | 29.721 | 22.777 | 1.00 | 64.29 |
| 4066 | CG1 | ILE | 761 | 11.834 | 29.367 | 25.140 | 1.00 | 60.68 |
| 4067 | CD1 | ILE | 761 | 12.794 | 28.186 | 25.074 | 1.00 | 65.98 |
| 4068 | C | ILE | 761 | 13.925 | 32.069 | 23.273 | 1.00 | 60.92 |
| 4069 | O | ILE | 761 | 15.089 | 31.875 | 22.912 | 1.00 | 60.54 |
| 4070 | N | PRO | 762 | 13.128 | 32.938 | 22.626 | 1.00 | 60.85 |
| 4071 | CD | PRO | 762 | 11.810 | 32.652 | 22.044 | 1.00 | 60.12 |
| 4072 | CA | PRO | 762 | 13.999 | 33.474 | 21.571 | 1.00 | 60.84 |
| 4073 | CB | PRO | 762 | 13.355 | 32.980 | 20.264 | 1.00 | 58.66 |
| 4074 | CG | PRO | 762 | 12.240 | 32.017 | 20.716 | 1.00 | 58.17 |
| 4075 | C | PRO | 762 | 14.222 | 34.968 | 21.533 | 1.00 | 62.93 |
| 4076 | O | PRO | 762 | 14.168 | 35.566 | 20.457 | 1.00 | 59.88 |
| 4077 | N | LYS | 763 | 14.405 | 35.599 | 22.687 | 1.00 | 59.58 |
| 4078 | CA | LYS | 763 | 14.750 | 37.015 | 22.653 | 1.00 | 62.56 |
| 4079 | CB | LYS | 763 | 14.713 | 37.645 | 24.045 | 1.00 | 61.84 |
| 4080 | CG | LYS | 763 | 15.014 | 39.141 | 24.061 | 1.00 | 61.96 |
| 4081 | CD | LYS | 763 | 14.703 | 39.723 | 25.430 | 1.00 | 62.42 |
| 4082 | CE | LYS | 763 | 13.428 | 39.096 | 25.979 | 1.00 | 61.00 |
| 4083 | NZ | LYS | 763 | 12.992 | 39.651 | 27.285 | 1.00 | 62.85 |
| 4084 | C | LYS | 763 | 16.182 | 36.666 | 22.292 | 1.00 | 58.43 |
| 4085 | O | LYS | 763 | 16.780 | 37.217 | 21.354 | 1.00 | 59.83 |
| 4086 | N | TYR | 764 | 16.668 | 35.665 | 23.036 | 1.00 | 59.46 |
| 4087 | CA | TYR | 764 | 17.999 | 35.106 | 22.895 | 1.00 | 61.50 |
| 4088 | CB | TYR | 764 | 18.291 | 34.109 | 24.020 | 1.00 | 62.35 |
| 4089 | CG | TYR | 764 | 19.085 | 34.715 | 25.149 | 1.00 | 57.24 |
| 4090 | CD1 | TYR | 764 | 18.526 | 34.872 | 26.424 | 1.00 | 62.21 |
| 4091 | CE1 | TYR | 764 | 19.236 | 35.509 | 27.451 | 1.00 | 63.69 |
| 4092 | CD2 | TYR | 764 | 20.378 | 35.200 | 24.927 | 1.00 | 62.73 |
| 4093 | CE2 | TYR | 764 | 21.095 | 35.837 | 25.942 | 1.00 | 62.44 |
| 4094 | CZ | TYR | 764 | 20.516 | 35.992 | 27.195 | 1.00 | 65.70 |
| 4095 | OH | TYR | 764 | 21.206 | 36.670 | 28.168 | 1.00 | 63.44 |
| 4096 | C | TYR | 764 | 18.156 | 34.400 | 21.570 | 1.00 | 60.32 |
| 4097 | O | TYR | 764 | 17.580 | 34.816 | 20.556 | 1.00 | 60.80 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4098 | N | SER | 765 | 18.922 | 33.310 | 21.597 | 1.00 | 62.63 |
| 4099 | CA | SER | 765 | 19.209 | 32.535 | 20.391 | 1.00 | 62.57 |
| 4100 | CB | SER | 765 | 17.908 | 31.971 | 19.777 | 1.00 | 63.02 |
| 4101 | OG | SER | 765 | 18.172 | 31.232 | 18.586 | 1.00 | 63.09 |
| 4102 | C | SER | 765 | 19.904 | 33.487 | 19.403 | 1.00 | 61.20 |
| 4103 | O | SER | 765 | 21.121 | 33.703 | 19.474 | 1.00 | 60.85 |
| 4104 | N | ASN | 766 | 19.099 | 34.064 | 18.513 | 1.00 | 59.31 |
| 4105 | CA | ASN | 766 | 19.520 | 35.005 | 17.477 | 1.00 | 61.38 |
| 4106 | CB | ASN | 766 | 18.344 | 35.932 | 17.155 | 1.00 | 63.73 |
| 4107 | CG | ASN | 766 | 17.006 | 35.195 | 17.116 | 1.00 | 60.04 |
| 4108 | OD1 | ASN | 766 | 16.493 | 34.720 | 18.153 | 1.00 | 64.69 |
| 4109 | ND2 | ASN | 766 | 16.433 | 35.091 | 15.916 | 1.00 | 59.73 |
| 4110 | C | ASN | 766 | 20.764 | 35.857 | 17.800 | 1.00 | 64.20 |
| 4111 | O | ASN | 766 | 21.906 | 35.462 | 17.491 | 1.00 | 60.72 |
| 4112 | N | GLY | 767 | 20.523 | 37.032 | 18.396 | 1.00 | 59.36 |
| 4113 | CA | GLY | 767 | 21.589 | 37.961 | 18.766 | 1.00 | 63.17 |
| 4114 | C | GLY | 767 | 21.096 | 39.388 | 19.032 | 1.00 | 61.77 |
| 4115 | O | GLY | 767 | 21.905 | 40.321 | 19.172 | 1.00 | 58.54 |
| 4116 | N | ASN | 768 | 19.772 | 39.550 | 19.118 | 1.00 | 61.70 |
| 4117 | CA | ASN | 768 | 19.115 | 40.849 | 19.347 | 1.00 | 60.25 |
| 4118 | CB | ASN | 768 | 17.603 | 40.673 | 19.163 | 1.00 | 61.86 |
| 4119 | CG | ASN | 768 | 17.257 | 39.882 | 17.898 | 1.00 | 63.48 |
| 4120 | OD1 | ASN | 768 | 17.602 | 38.702 | 17.772 | 1.00 | 58.87 |
| 4121 | ND2 | ASN | 768 | 16.579 | 40.534 | 16.956 | 1.00 | 59.56 |
| 4122 | C | ASN | 768 | 19.400 | 41.566 | 20.692 | 1.00 | 61.21 |
| 4123 | O | ASN | 768 | 18.781 | 42.595 | 20.987 | 1.00 | 57.65 |
| 4124 | N | ILE | 769 | 20.323 | 41.011 | 21.490 | 1.00 | 60.01 |
| 4125 | CA | ILE | 769 | 20.764 | 41.563 | 22.792 | 1.00 | 60.44 |
| 4126 | CB | ILE | 769 | 20.851 | 40.456 | 23.891 | 1.00 | 60.23 |
| 4127 | CG2 | ILE | 769 | 21.520 | 41.004 | 25.161 | 1.00 | 65.73 |
| 4128 | CG1 | ILE | 769 | 19.461 | 39.920 | 24.234 | 1.00 | 64.63 |
| 4129 | CD1 | ILE | 769 | 19.506 | 38.773 | 25.256 | 1.00 | 63.56 |
| 4130 | C | ILE | 769 | 22.197 | 42.097 | 22.594 | 1.00 | 60.12 |
| 4131 | O | ILE | 769 | 22.744 | 41.990 | 21.495 | 1.00 | 59.38 |
| 4132 | N | LYS | 770 | 22.799 | 42.660 | 23.643 | 1.00 | 58.87 |
| 4133 | CA | LYS | 770 | 24.173 | 43.171 | 23.568 | 1.00 | 63.22 |
| 4134 | CB | LYS | 770 | 24.210 | 44.693 | 23.711 | 1.00 | 61.35 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4135 | CG | LYS | 770 | 25.615 | 45.304 | 23.656 | 1.00 | 60.38 |
| 4136 | CD | LYS | 770 | 25.617 | 46.682 | 24.324 | 1.00 | 59.38 |
| 4137 | CE | LYS | 770 | 26.765 | 47.580 | 23.858 | 1.00 | 63.47 |
| 4138 | NZ | LYS | 770 | 26.704 | 48.946 | 24.493 | 1.00 | 63.76 |
| 4139 | C | LYS | 770 | 25.039 | 42.568 | 24.665 | 1.00 | 59.00 |
| 4140 | O | LYS | 770 | 24.962 | 42.967 | 25.829 | 1.00 | 63.74 |
| 4141 | N | LYS | 771 | 25.868 | 41.604 | 24.292 | 1.00 | 59.72 |
| 4142 | CA | LYS | 771 | 26.742 | 40.984 | 25.268 | 1.00 | 60.78 |
| 4143 | CB | LYS | 771 | 27.024 | 39.525 | 24.871 | 1.00 | 59.33 |
| 4144 | CG | LYS | 771 | 27.854 | 39.345 | 23.619 | 1.00 | 62.03 |
| 4145 | CD | LYS | 771 | 28.351 | 37.906 | 23.466 | 1.00 | 63.82 |
| 4146 | CE | LYS | 771 | 29.538 | 37.838 | 22.501 | 1.00 | 59.76 |
| 4147 | NZ | LYS | 771 | 30.301 | 36.550 | 22.571 | 1.00 | 57.46 |
| 4148 | C | LYS | 771 | 28.044 | 41.798 | 25.413 | 1.00 | 61.42 |
| 4149 | O | LYS | 771 | 28.800 | 41.976 | 24.459 | 1.00 | 58.76 |
| 4150 | N | LEU | 772 | 28.271 | 42.302 | 26.623 | 1.00 | 61.95 |
| 4151 | CA | LEU | 772 | 29.444 | 43.107 | 26.948 | 1.00 | 60.44 |
| 4152 | CB | LEU | 772 | 29.187 | 43.864 | 28.260 | 1.00 | 59.38 |
| 4153 | CG | LEU | 772 | 27.923 | 44.730 | 28.267 | 1.00 | 63.13 |
| 4154 | CD1 | LEU | 772 | 27.630 | 45.253 | 29.656 | 1.00 | 60.11 |
| 4155 | CD2 | LEU | 772 | 28.102 | 45.873 | 27.289 | 1.00 | 62.17 |
| 4156 | C | LEU | 772 | 30.732 | 42.272 | 27.060 | 1.00 | 60.10 |
| 4157 | O | LEU | 772 | 30.764 | 41.233 | 27.718 | 1.00 | 60.79 |
| 4158 | N | LEU | 773 | 31.797 | 42.749 | 26.423 | 1.00 | 61.72 |
| 4159 | CA | LEU | 773 | 33.074 | 42.055 | 26.428 | 1.00 | 60.85 |
| 4160 | CB | LEU | 773 | 33.406 | 41.580 | 25.011 | 1.00 | 62.82 |
| 4161 | CG | LEU | 773 | 32.425 | 40.675 | 24.265 | 1.00 | 63.82 |
| 4162 | CD1 | LEU | 773 | 32.927 | 40.451 | 22.866 | 1.00 | 60.94 |
| 4163 | CD2 | LEU | 773 | 32.285 | 39.352 | 24.966 | 1.00 | 62.11 |
| 4164 | C | LEU | 773 | 34.205 | 42.942 | 26.933 | 1.00 | 63.04 |
| 4165 | O | LEU | 773 | 34.271 | 44.126 | 26.625 | 1.00 | 62.16 |
| 4166 | N | PHE | 774 | 35.101 | 42.352 | 27.712 | 1.00 | 60.13 |
| 4167 | CA | PHE | 774 | 36.246 | 43.072 | 28.248 | 1.00 | 61.57 |
| 4168 | CB | PHE | 774 | 36.893 | 42.279 | 29.377 | 1.00 | 66.09 |
| 4169 | CG | PHE | 774 | 36.280 | 42.543 | 30.698 | 1.00 | 61.67 |
| 4170 | CD1 | PHE | 774 | 36.524 | 43.741 | 31.355 | 1.00 | 59.42 |
| 4171 | CD2 | PHE | 774 | 35.385 | 41.650 | 31.248 | 1.00 | 56.29 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4172 | CE1 | PHE | 774 | 35.879 | 44.050 | 32.536 | 1.00 | 64.79 |
| 4173 | CE2 | PHE | 774 | 34.729 | 41.948 | 32.435 | 1.00 | 61.83 |
| 4174 | CZ | PHE | 774 | 34.978 | 43.155 | 33.080 | 1.00 | 62.07 |
| 4175 | C | PHE | 774 | 37.243 | 43.261 | 27.143 | 1.00 | 58.09 |
| 4176 | O | PHE | 774 | 38.081 | 44.155 | 27.187 | 1.00 | 63.24 |
| 4177 | N | HIS | 775 | 37.131 | 42.398 | 26.143 | 1.00 | 61.71 |
| 4178 | CA | HIS | 775 | 38.022 | 42.419 | 25.007 | 1.00 | 60.58 |
| 4179 | CB | HIS | 775 | 39.060 | 41.319 | 25.175 | 1.00 | 63.99 |
| 4180 | CG | HIS | 775 | 39.763 | 41.365 | 26.492 | 1.00 | 62.38 |
| 4181 | CD2 | HIS | 775 | 39.696 | 40.548 | 27.567 | 1.00 | 61.52 |
| 4182 | ND1 | HIS | 775 | 40.616 | 42.389 | 26.838 | 1.00 | 62.48 |
| 4183 | CE1 | HIS | 775 | 41.042 | 42.202 | 28.074 | 1.00 | 59.32 |
| 4184 | NE2 | HIS | 775 | 40.498 | 41.092 | 28.538 | 1.00 | 62.24 |
| 4185 | C | HIS | 775 | 37.236 | 42.196 | 23.732 | 1.00 | 61.77 |
| 4186 | O | HIS | 775 | 36.461 | 41.252 | 23.633 | 1.00 | 62.62 |
| 4187 | N | GLN | 776 | 37.425 | 43.083 | 22.765 | 1.00 | 58.75 |
| 4188 | CA | GLN | 776 | 36.759 | 42.955 | 21.484 | 1.00 | 58.47 |
| 4189 | CB | GLN | 776 | 36.460 | 44.340 | 20.893 | 1.00 | 58.08 |
| 4190 | CG | GLN | 776 | 37.681 | 45.247 | 20.680 | 1.00 | 63.95 |
| 4191 | CD | GLN | 776 | 38.236 | 45.221 | 19.250 | 1.00 | 62.81 |
| 4192 | OE1 | GLN | 776 | 39.158 | 45.979 | 18.924 | 1.00 | 62.48 |
| 4193 | NE2 | GLN | 776 | 37.680 | 44.353 | 18.397 | 1.00 | 60.08 |
| 4194 | C | GLN | 776 | 37.724 | 42.163 | 20.599 | 1.00 | 60.33 |
| 4195 | O | GLN | 776 | 37.269 | 41.235 | 19.894 | 1.00 | 61.93 |
| 4196 | OXT | GLN | 776 | 38.936 | 42.474 | 20.642 | 1.00 | 63.26 |
| 4197 | CB | LYS | 741 | 7.500 | 39.003 | 28.905 | 1.00 | 62.43 |
| 4198 | CG | LYS | 741 | 8.600 | 39.530 | 28.004 | 1.00 | 60.91 |
| 4199 | CD | LYS | 741 | 9.141 | 40.875 | 28.431 | 1.00 | 59.52 |
| 4200 | CE | LYS | 741 | 10.182 | 41.314 | 27.418 | 1.00 | 62.38 |
| 4201 | NZ | LYS | 741 | 10.807 | 42.617 | 27.779 | 1.00 | 64.43 |
| 4202 | C | LYS | 741 | 6.303 | 36.975 | 29.773 | 1.00 | 61.31 |
| 4203 | O | LYS | 741 | 6.054 | 35.766 | 29.829 | 1.00 | 59.09 |
| 4204 | N | LYS | 741 | 6.417 | 37.458 | 27.272 | 1.00 | 62.30 |
| 4205 | CA | LYS | 741 | 7.109 | 37.544 | 28.597 | 1.00 | 59.65 |
| 4206 | N | GLU | 742 | 5.905 | 37.867 | 30.689 | 1.00 | 62.69 |
| 4207 | CA | GLU | 742 | 5.163 | 37.547 | 31.917 | 1.00 | 61.65 |
| 4208 | CB | GLU | 742 | 4.672 | 36.083 | 31.926 | 1.00 | 61.06 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4209 | CG | GLU | 742 | 4.087 | 35.564 | 33.257 | 1.00 | 61.09 |
| 4210 | CD | GLU | 742 | 2.705 | 36.123 | 33.568 | 1.00 | 62.31 |
| 4211 | OE1 | GLU | 742 | 2.138 | 35.720 | 34.616 | 1.00 | 57.45 |
| 4212 | OE2 | GLU | 742 | 2.195 | 36.960 | 32.771 | 1.00 | 60.87 |
| 4213 | C | GLU | 742 | 6.112 | 37.794 | 33.099 | 1.00 | 63.06 |
| 4214 | O | GLU | 742 | 5.915 | 38.741 | 33.853 | 1.00 | 61.76 |
| 4215 | N | ASN | 743 | 7.151 | 36.967 | 33.238 | 1.00 | 63.03 |
| 4216 | CA | ASN | 743 | 8.116 | 37.101 | 34.341 | 1.00 | 61.82 |
| 4217 | CB | ASN | 743 | 9.276 | 38.040 | 33.958 | 1.00 | 58.35 |
| 4218 | CG | ASN | 743 | 10.217 | 37.445 | 32.909 | 1.00 | 59.24 |
| 4219 | OD1 | ASN | 743 | 10.071 | 37.693 | 31.699 | 1.00 | 60.28 |
| 4220 | ND2 | ASN | 743 | 11.198 | 36.658 | 33.372 | 1.00 | 60.70 |
| 4221 | C | ASN | 743 | 7.447 | 37.656 | 35.604 | 1.00 | 61.37 |
| 4222 | O | ASN | 743 | 8.010 | 38.522 | 36.284 | 1.00 | 60.15 |
| 4223 | N | ALA | 744 | 6.245 | 37.167 | 35.907 | 1.00 | 60.68 |
| 4224 | CA | ALA | 744 | 5.497 | 37.626 | 37.073 | 1.00 | 62.31 |
| 4225 | CB | ALA | 744 | 4.024 | 37.229 | 36.940 | 1.00 | 62.38 |
| 4226 | C | ALA | 744 | 6.080 | 37.067 | 38.364 | 1.00 | 61.35 |
| 4227 | O | ALA | 744 | 6.168 | 37.778 | 39.360 | 1.00 | 59.70 |
| 4228 | N | LEU | 745 | 6.490 | 35.801 | 38.346 | 1.00 | 61.20 |
| 4229 | CA | LEU | 745 | 7.062 | 35.182 | 39.538 | 1.00 | 59.93 |
| 4230 | CB | LEU | 745 | 7.419 | 33.710 | 39.276 | 1.00 | 63.15 |
| 4231 | CG | LEU | 745 | 7.255 | 32.720 | 40.448 | 1.00 | 63.24 |
| 4232 | CD1 | LEU | 745 | 8.022 | 31.429 | 40.158 | 1.00 | 59.45 |
| 4233 | CD2 | LEU | 745 | 7.759 | 33.342 | 41.745 | 1.00 | 59.90 |
| 4234 | C | LEU | 745 | 8.313 | 35.934 | 39.987 | 1.00 | 59.41 |
| 4235 | O | LEU | 745 | 8.520 | 36.123 | 41.182 | 1.00 | 59.83 |
| 4236 | N | LEU | 746 | 9.137 | 36.372 | 39.031 | 1.00 | 63.44 |
| 4237 | CA | LEU | 746 | 10.375 | 37.096 | 39.350 | 1.00 | 60.90 |
| 4238 | CB | LEU | 746 | 11.266 | 37.239 | 38.104 | 1.00 | 63.43 |
| 4239 | CG | LEU | 746 | 12.771 | 36.991 | 38.300 | 1.00 | 64.10 |
| 4240 | CD1 | LEU | 746 | 13.540 | 37.598 | 37.140 | 1.00 | 61.93 |
| 4241 | CD2 | LEU | 746 | 13.248 | 37.598 | 39.612 | 1.00 | 58.02 |
| 4242 | C | LEU | 746 | 10.120 | 38.485 | 39.950 | 1.00 | 65.26 |
| 4243 | O | LEU | 746 | 10.649 | 38.808 | 41.025 | 1.00 | 59.76 |
| 4244 | N | ARG | 747 | 9.334 | 39.308 | 39.255 | 1.00 | 61.19 |
| 4245 | CA | ARG | 747 | 9.012 | 40.641 | 39.762 | 1.00 | 61.73 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4246 | CB | ARG | 747 | 7.844 | 41.256 | 38.975 | 1.00 | 59.04 |
| 4247 | CG | ARG | 747 | 7.475 | 42.676 | 39.421 | 1.00 | 57.13 |
| 4248 | CD | ARG | 747 | 6.596 | 43.434 | 38.407 | 1.00 | 58.93 |
| 4249 | NE | ARG | 747 | 7.362 | 44.324 | 37.522 | 1.00 | 59.01 |
| 4250 | CZ | ARG | 747 | 7.556 | 44.118 | 36.221 | 1.00 | 59.60 |
| 4251 | NH1 | ARG | 747 | 7.039 | 43.043 | 35.624 | 1.00 | 63.96 |
| 4252 | NH2 | ARG | 747 | 8.272 | 44.987 | 35.518 | 1.00 | 60.37 |
| 4253 | C | ARG | 747 | 8.651 | 40.511 | 41.247 | 1.00 | 61.37 |
| 4254 | O | ARG | 747 | 9.155 | 41.257 | 42.090 | 1.00 | 62.28 |
| 4255 | N | TYR | 748 | 7.799 | 39.541 | 41.565 | 1.00 | 62.06 |
| 4256 | CA | TYR | 748 | 7.399 | 39.306 | 42.941 | 1.00 | 60.54 |
| 4257 | CB | TYR | 748 | 6.517 | 38.050 | 43.003 | 1.00 | 60.69 |
| 4258 | CG | TYR | 748 | 6.287 | 37.521 | 44.401 | 1.00 | 59.67 |
| 4259 | CD1 | TYR | 748 | 7.077 | 36.488 | 44.908 | 1.00 | 60.21 |
| 4260 | CE1 | TYR | 748 | 6.926 | 36.044 | 46.209 | 1.00 | 60.77 |
| 4261 | CD2 | TYR | 748 | 5.329 | 38.093 | 45.240 | 1.00 | 63.30 |
| 4262 | CE2 | TYR | 748 | 5.174 | 37.654 | 46.550 | 1.00 | 61.08 |
| 4263 | CZ | TYR | 748 | 5.977 | 36.631 | 47.027 | 1.00 | 62.49 |
| 4264 | OH | TYR | 748 | 5.864 | 36.204 | 48.331 | 1.00 | 59.76 |
| 4265 | C | TYR | 748 | 8.593 | 39.190 | 43.908 | 1.00 | 63.27 |
| 4266 | O | TYR | 748 | 8.702 | 39.969 | 44.857 | 1.00 | 60.19 |
| 4267 | N | LEU | 749 | 9.484 | 38.229 | 43.663 | 1.00 | 62.27 |
| 4268 | CA | LEU | 749 | 10.661 | 38.008 | 44.516 | 1.00 | 62.12 |
| 4269 | CB | LEU | 749 | 11.454 | 36.792 | 44.020 | 1.00 | 64.20 |
| 4270 | CG | LEU | 749 | 10.690 | 35.476 | 43.873 | 1.00 | 58.72 |
| 4271 | CD1 | LEU | 749 | 11.058 | 34.828 | 42.554 | 1.00 | 61.26 |
| 4272 | CD2 | LEU | 749 | 10.986 | 34.565 | 45.039 | 1.00 | 61.68 |
| 4273 | C | LEU | 749 | 11.589 | 39.223 | 44.561 | 1.00 | 61.27 |
| 4274 | O | LEU | 749 | 12.241 | 39.497 | 45.571 | 1.00 | 60.09 |
| 4275 | N | LEU | 750 | 11.658 | 39.946 | 43.455 | 1.00 | 60.39 |
| 4276 | CA | LEU | 750 | 12.503 | 41.120 | 43.397 | 1.00 | 59.95 |
| 4277 | CB | LEU | 750 | 12.603 | 41.607 | 41.959 | 1.00 | 59.67 |
| 4278 | CG | LEU | 750 | 14.026 | 41.742 | 41.404 | 1.00 | 65.62 |
| 4279 | CD1 | LEU | 750 | 15.031 | 40.919 | 42.205 | 1.00 | 61.72 |
| 4280 | CD2 | LEU | 750 | 14.005 | 41.302 | 39.953 | 1.00 | 61.66 |
| 4281 | C | LEU | 750 | 11.954 | 42.216 | 44.298 | 1.00 | 58.16 |
| 4282 | O | LEU | 750 | 12.712 | 42.855 | 45.032 | 1.00 | 61.93 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4283 | N | ASP | 751 | 10.637 | 42.423 | 44.242 | 1.00 | 60.02 |
| 4284 | CA | ASP | 751 | 9.969 | 43.428 | 45.073 | 1.00 | 61.63 |
| 4285 | CB | ASP | 751 | 8.539 | 43.658 | 44.616 | 1.00 | 59.82 |
| 4286 | CG | ASP | 751 | 8.381 | 44.973 | 43.912 | 1.00 | 60.49 |
| 4287 | OD1 | ASP | 751 | 9.166 | 45.214 | 42.968 | 1.00 | 59.40 |
| 4288 | OD2 | ASP | 751 | 7.491 | 45.767 | 44.298 | 1.00 | 59.17 |
| 4289 | C | ASP | 751 | 9.941 | 43.037 | 46.531 | 1.00 | 62.48 |
| 4290 | O | ASP | 751 | 10.367 | 43.813 | 47.383 | 1.00 | 63.09 |
| 4291 | N | LYS | 752 | 9.421 | 41.841 | 46.808 | 1.00 | 64.09 |
| 4292 | CA | LYS | 752 | 9.346 | 41.308 | 48.164 | 1.00 | 60.89 |
| 4293 | CB | LYS | 752 | 9.881 | 39.882 | 48.216 | 1.00 | 63.21 |
| 4294 | CG | LYS | 752 | 9.051 | 38.811 | 47.568 | 1.00 | 57.72 |
| 4295 | CD | LYS | 752 | 9.168 | 37.532 | 48.396 | 1.00 | 60.58 |
| 4296 | CE | LYS | 752 | 8.769 | 37.801 | 49.858 | 1.00 | 63.26 |
| 4297 | NZ | LYS | 752 | 8.598 | 36.571 | 50.686 | 1.00 | 60.07 |
| 4298 | C | LYS | 752 | 10.218 | 42.123 | 49.090 | 1.00 | 59.75 |
| 4299 | O | LYS | 752 | 11.426 | 42.228 | 48.869 | 1.00 | 61.26 |
| 4300 | N | ASP | 753 | 9.644 | 42.700 | 50.132 | 1.00 | 64.75 |
| 4301 | CA | ASP | 753 | 10.478 | 43.462 | 51.039 | 1.00 | 60.32 |
| 4302 | CB | ASP | 753 | 9.643 | 44.126 | 52.126 | 1.00 | 62.72 |
| 4303 | CG | ASP | 753 | 10.496 | 44.762 | 53.198 | 1.00 | 62.87 |
| 4304 | OD1 | ASP | 753 | 11.420 | 45.549 | 52.863 | 1.00 | 59.14 |
| 4305 | OD2 | ASP | 753 | 10.239 | 44.468 | 54.382 | 1.00 | 63.19 |
| 4306 | C | ASP | 753 | 11.455 | 42.468 | 51.647 | 1.00 | 60.33 |
| 4307 | O | ASP | 753 | 12.111 | 42.750 | 52.646 | 1.00 | 61.12 |
| 4308 | N | ALA | 754 | 11.528 | 41.304 | 51.008 | 1.00 | 59.93 |
| 4309 | CA | ALA | 754 | 12.396 | 40.177 | 51.356 | 1.00 | 61.16 |
| 4310 | CB | ALA | 754 | 12.896 | 39.509 | 50.053 | 1.00 | 64.36 |
| 4311 | C | ALA | 754 | 13.587 | 40.401 | 52.307 | 1.00 | 59.44 |
| 4312 | O | ALA | 754 | 14.700 | 39.937 | 52.047 | 1.00 | 61.71 |
| 4313 | N | THR | 755 | 13.355 | 41.108 | 53.403 | 1.00 | 60.67 |
| 4314 | CA | THR | 755 | 14.375 | 41.342 | 54.420 | 1.00 | 60.98 |
| 4315 | CB | THR | 755 | 15.250 | 42.613 | 54.137 | 1.00 | 63.96 |
| 4316 | OG1 | THR | 755 | 14.460 | 43.794 | 54.313 | 1.00 | 60.28 |
| 4317 | CG2 | THR | 755 | 15.824 | 42.582 | 52.696 | 1.00 | 57.43 |
| 4318 | C | THR | 755 | 13.505 | 41.499 | 55.671 | 1.00 | 59.27 |
| 4319 | O | THR | 755 | 13.323 | 42.586 | 56.237 | 1.00 | 60.51 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4320 | N | ALA | 756 | 12.918 | 40.356 | 56.024 | 1.00 | 62.21 |
| 4321 | CA | ALA | 756 | 12.025 | 40.165 | 57.162 | 1.00 | 59.83 |
| 4322 | CB | ALA | 756 | 11.075 | 38.993 | 56.870 | 1.00 | 60.21 |
| 4323 | C | ALA | 756 | 12.890 | 39.842 | 58.372 | 1.00 | 61.08 |
| 4324 | O | ALA | 756 | 12.461 | 39.941 | 59.531 | 1.00 | 61.80 |
| 4325 | N | ALA | 757 | 14.115 | 39.426 | 58.072 | 1.00 | 62.43 |
| 4326 | CA | ALA | 757 | 15.087 | 39.099 | 59.092 | 1.00 | 60.57 |
| 4327 | CB | ALA | 757 | 16.415 | 38.753 | 58.431 | 1.00 | 60.81 |
| 4328 | C | ALA | 757 | 15.211 | 40.367 | 59.932 | 1.00 | 61.35 |
| 4329 | O | ALA | 757 | 15.146 | 41.460 | 59.327 | 1.00 | 62.10 |
| 4330 | OXT | ALA | 757 | 15.354 | 40.253 | 61.169 | 1.00 | 58.48 |
| 4331 | O | HOH | 1 | 62.349 | -1.370 | 59.183 | 1.00 | 61.82 |
| 4332 | O | HOH | 2 | 63.098 | 9.775 | 56.010 | 1.00 | 63.21 |
| 4333 | O | HOH | 3 | 29.467 | 50.468 | 47.493 | 1.00 | 60.82 |
| 4334 | O | HOH | 4 | 24.799 | 1.025 | 51.054 | 1.00 | 63.04 |
| 4335 | O | HOH | 5 | 25.120 | 35.371 | 29.890 | 1.00 | 58.53 |
| 4336 | O | HOH | 6 | 62.603 | 13.819 | 69.179 | 1.00 | 62.10 |
| 4337 | O | HOH | 7 | 43.394 | -0.575 | 64.086 | 1.00 | 61.07 |
| 4338 | O | HOH | 8 | 33.029 | 27.080 | 24.812 | 1.00 | 63.53 |
| 4339 | O | HOH | 9 | 40.476 | 0.604 | 50.517 | 1.00 | 62.87 |
| 4340 | O | HOH | 10 | 42.083 | 33.017 | 29.431 | 1.00 | 59.31 |
| 4341 | O | HOH | 11 | 40.224 | -1.905 | 63.310 | 1.00 | 60.38 |
| 4342 | O | HOH | 12 | 29.926 | 49.219 | 30.317 | 1.00 | 60.19 |
| 4343 | O | HOH | 13 | 63.481 | 3.211 | 57.703 | 1.00 | 62.93 |
| 4344 | O | HOH | 14 | 45.679 | 44.833 | 38.756 | 1.00 | 60.97 |
| 4345 | O | HOH | 15 | 21.388 | 1.839 | 41.400 | 1.00 | 61.41 |
| 4346 | O | HOH | 16 | 47.452 - | 16.061 | 63.707 | 1.00 | 60.73 |
| 4347 | O | HOH | 17 | 52.653 | 15.955 | 63.901 | 1.00 | 64.75 |
| 4348 | O | HOH | 18 | 62.913 | 1.964 | 67.923 | 1.00 | 64.33 |
| 4349 | O | HOH | 19 | 62.507 | 3.936 | 69.792 | 1.00 | 60.95 |
| 4350 | O | HOH | 20 | 11.730 | 26.749 | 44.436 | 1.00 | 60.79 |
| 4351 | O | HOH | 21 | 48.735 | 13.308 | 64.587 | 1.00 | 62.06 |
| 4352 | O | HOH | 22 | 32.377 | 39.863 | 58.144 | 1.00 | 63.51 |
| 4353 | O | HOH | 23 | 58.924 | 9.831 | 70.947 | 1.00 | 61.40 |
| 4354 | O | HOH | 24 | 39.278 | 17.448 | 64.290 | 1.00 | 62.12 |
| 4355 | O | HOH | 25 | 40.573 | 48.042 | 36.816 | 1.00 | 60.96 |
| 4356 | O | HOH | 26 | 40.494 | 35.299 | 48.387 | 1.00 | 59.93 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4357 | O | HOH | 27 | 61.454 | 1.678 | 61.901 | 1.00 | 60.51 |
| 4358 | O | HOH | 28 | 9.075 | 22.638 | 42.296 | 1.00 | 61.65 |
| 4359 | O | HOH | 29 | 51.369 | 13.900 | 63.592 | 1.00 | 64.00 |
| 4360 | O | HOH | 30 | 61.184 | -0.481 | 44.937 | 1.00 | 61.95 |
| 4361 | O | HOH | 31 | 19.041 | 16.035 | 52.737 | 1.00 | 60.85 |
| 4362 | O | HOH | 32 | 37.487 | 3.963 | 49.092 | 1.00 | 60.40 |
| 4363 | O | HOH | 33 | 31.183 | 34.399 | 55.395 | 1.00 | 61.32 |
| 4364 | O | HOH | 34 | 25.672 | 33.490 | 53.795 | 1.00 | 61.76 |
| 4365 | O | HOH | 35 | 24.467 | 27.177 | 45.107 | 1.00 | 62.37 |
| 4366 | O | HOH | 36 | 47.899 | 30.685 | 35.691 | 1.00 | 60.62 |
| 4367 | O | HOH | 37 | 31.250 | 45.014 | 24.427 | 1.00 | 63.26 |
| 4368 | O | HOH | 38 | 60.719 | -0.340 | 49.987 | 1.00 | 60.94 |
| 4369 | O | HOH | 39 | 48.761 | 14.305 | 46.147 | 1.00 | 59.45 |
| 4370 | O | HOH | 40 | 52.252 | 11.824 | 45.533 | 1.00 | 59.86 |
| 4371 | O | HOH | 41 | 40.704 | 30.604 | 47.765 | 1.00 | 62.04 |
| 4372 | O | HOH | 42 | 34.599 | 19.541 | 73.265 | 1.00 | 61.69 |
| 4373 | O | HOH | 43 | 44.135 | 32.951 | 48.092 | 1.00 | 60.11 |
| 4374 | O | HOH | 44 | 16.447 | 16.136 | 55.224 | 1.00 | 58.77 |
| 4375 | O | HOH | 45 | 37.470 | 21.079 | 29.057 | 1.00 | 61.47 |
| 4376 | O | HOH | 46 | 14.411 | 15.785 | 52.085 | 1.00 | 58.97 |
| 4377 | O | HOH | 47 | 27.199 | 25.588 | 51.919 | 1.00 | 58.58 |
| 4378 | O | HOH | 48 | 32.466 | 25.097 | 53.254 | 1.00 | 60.88 |
| 4379 | O | HOH | 49 | 17.927 | 39.612 | 49.972 | 1.00 | 61.48 |
| 4380 | O | HOH | 50 | 17.243 | 38.022 | 52.339 | 1.00 | 61.61 |
| 4381 | O | HOH | 51 | 65.714 | 6.374 | 72.458 | 1.00 | 61.45 |
| 4382 | O | HOH | 52 | 25.540 | 34.686 | 57.601 | 1.00 | 59.81 |
| 4383 | O | HOH | 53 | 22.812 | 3.452 | 38.767 | 1.00 | 62.42 |
| 4384 | C1 | DEX | 1 | 31.791 | 3.330 | 56.615 | 1.00 | 59.00 |
| 4385 | H1 | DEX | 1 | 30.892 | 2.719 | 56.626 | 1.00 | 59.00 |
| 4386 | C2 | DEX | 1 | 32.066 | 4.057 | 55.552 | 1.00 | 59.00 |
| 4387 | H2 | DEX | 1 | 31.418 | 4.016 | 54.717 | 1.00 | 59.00 |
| 4388 | C3 | DEX | 1 | 33.314 | 4.929 | 55.514 | 1.00 | 59.00 |
| 4389 | C4 | DEX | 1 | 34.176 | 5.061 | 56.733 | 1.00 | 59.00 |
| 4390 | H4 | DEX | 1 | 35.013 | 5.729 | 56.720 | 1.00 | 59.00 |
| 4391 | C5 | DEX | 1 | 33.915 | 4.329 | 57.855 | 1.00 | 59.00 |
| 4392 | C6 | DEX | 1 | 34.782 | 4.456 | 59.133 | 1.00 | 59.00 |
| 4393 | H61 | DEX | 1 | 35.558 | 5.172 | 59.015 | 1.00 | 59.00 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4394 | H62 | DEX | 1 | 35.262 | 3.483 | 59.339 | 1.00 | 59.00 |
| 4395 | C7 | DEX | 1 | 33.905 | 4.834 | 60.331 | 1.00 | 59.00 |
| 4396 | H71 | DEX | 1 | 33.520 | 5.861 | 60.202 | 1.00 | 59.00 |
| 4397 | H72 | DEX | 1 | 34.515 | 4.837 | 61.236 | 1.00 | 59.00 |
| 4398 | C8 | DEX | 1 | 32.690 | 3.903 | 60.544 | 1.00 | 59.00 |
| 4399 | H8 | DEX | 1 | 33.063 | 2.878 | 60.787 | 1.00 | 59.00 |
| 4400 | C9 | DEX | 1 | 31.759 | 3.803 | 59.162 | 1.00 | 59.00 |
| 4401 | C10 | DEX | 1 | 32.677 | 3.304 | 57.900 | 1.00 | 59.00 |
| 4402 | C11 | DEX | 1 | 30.360 | 2.986 | 59.327 | 1.00 | 59.00 |
| 4403 | H11 | DEX | 1 | 29.743 | 3.203 | 58.478 | 1.00 | 59.00 |
| 4404 | C12 | DEX | 1 | 29.599 | 3.415 | 60.596 | 1.00 | 59.00 |
| 4405 | H121 | DEX | 1 | 28.744 | 2.788 | 60.729 | 1.00 | 59.00 |
| 4406 | H122 | DEX | 1 | 29.221 | 4.448 | 60.436 | 1.00 | 59.00 |
| 4407 | C13 | DEX | 1 | 30.518 | 3.414 | 61.924 | 1.00 | 59.00 |
| 4408 | C14 | DEX | 1 | 31.758 | 4.387 | 61.726 | 1.00 | 59.00 |
| 4409 | H14 | DEX | 1 | 31.359 | 5.403 | 61.401 | 1.00 | 59.00 |
| 4410 | C15 | DEX | 1 | 32.374 | 4.589 | 63.095 | 1.00 | 59.00 |
| 4411 | H151 | DEX | 1 | 32.893 | 5.547 | 63.111 | 1.00 | 59.00 |
| 4412 | H152 | DEX | 1 | 33.119 | 3.796 | 63.281 | 1.00 | 59.00 |
| 4413 | C16 | DEX | 1 | 31.175 | 4.486 | 64.093 | 1.00 | 59.00 |
| 4414 | H16 | DEX | 1 | 31.391 | 3.605 | 64.743 | 1.00 | 59.00 |
| 4415 | C17 | DEX | 1 | 29.863 | 4.144 | 63.168 | 1.00 | 59.00 |
| 4416 | C18 | DEX | 1 | 30.929 | 1.834 | 62.325 | 1.00 | 59.00 |
| 4417 | H181 | DEX | 1 | 31.535 | 1.833 | 63.241 | 1.00 | 59.00 |
| 4418 | H182 | DEX | 1 | 30.050 | 1.248 | 62.496 | 1.00 | 59.00 |
| 4419 | H183 | DEX | 1 | 31.537 | 1.374 | 61.558 | 1.00 | 59.00 |
| 4420 | C19 | DEX | 1 | 33.270 | 1.833 | 58.015 | 1.00 | 59.00 |
| 4421 | H191 | DEX | 1 | 33.916 | 1.724 | 58.905 | 1.00 | 59.00 |
| 4422 | H192 | DEX | 1 | 32.485 | 1.095 | 58.112 | 1.00 | 59.00 |
| 4423 | H193 | DEX | 1 | 33.870 | 1.605 | 57.134 | 1.00 | 59.00 |
| 4424 | C20 | DEX | 1 | 28.759 | 3.270 | 63.873 | 1.00 | 59.00 |
| 4425 | C21 | DEX | 1 | 27.338 | 3.348 | 63.353 | 1.00 | 59.00 |
| 4426 | H211 | DEX | 1 | 27.350 | 3.637 | 62.283 | 1.00 | 59.00 |
| 4427 | H212 | DEX | 1 | 26.827 | 4.148 | 63.876 | 1.00 | 59.00 |
| 4428 | C22 | DEX | 1 | 31.008 | 5.693 | 64.947 | 1.00 | 59.00 |
| 4429 | H221 | DEX | 1 | 30.160 | 5.560 | 65.619 | 1.00 | 59.00 |
| 4430 | H222 | DEX | 1 | 31.912 | 5.877 | 65.542 | 1.00 | 59.00 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4431 | H223 | DEX | 1 | 30.811 | 6.588 | 64.313 | 1.00 | 59.00 |
| 4432 | F1 | DEX | 1 | 31.331 | 5.130 | 58.833 | 1.00 | 59.00 |
| 4433 | 01 | DEX | 1 | 33.617 | 5.512 | 54.507 | 1.00 | 59.00 |
| 4434 | 02 | DEX | 1 | 30.601 | 1.580 | 59.361 | 1.00 | 59.00 |
| 4435 | HO2 | DEX | 1 | 29.784 | 1.163 | 59.706 | 1.00 | 59.00 |
| 4436 | 03 | DEX | 1 | 29.236 | 5.409 | 62.711 | 1.00 | 59.00 |
| 4437 | H3 | DEX | 1 | 28.816 | 5.780 | 63.475 | 1.00 | 59.00 |
| 4438 | 04 | DEX | 1 | 29.058 | 2.511 | 64.818 | 1.00 | 59.00 |
| 4439 | 05 | DEX | 1 | 26.689 | 2.117 | 63.492 | 1.00 | 59.00 |
| 4440 | H5 | DEX | 1 | 25.816 | 2.344 | 63.756 | 1.00 | 59.00 |
| 4441 | C1 | DEX | 1 | 21.344 | 23.582 | 37.624 | 1.00 | 59.00 |
| 4442 | H1 | DEX | 1 | 20.325 | 23.208 | 37.634 | 1.00 | 59.00 |
| 4443 | C2 | DEX | 1 | 22.105 | 23.392 | 38.670 | 1.00 | 59.00 |
| 4444 | H2 | DEX | 1 | 21.710 | 22.910 | 39.509 | 1.00 | 59.00 |
| 4445 | C3 | DEX | 1 | 23.539 | 23.892 | 38.687 | 1.00 | 59.00 |
| 4446 | C4 | DEX | 1 | 24.137 | 24.501 | 37.450 | 1.00 | 59.00 |
| 4447 | H4 | DEX | 1 | 25.173 | 24.791 | 37.441 | 1.00 | 59.00 |
| 4448 | C5 | DEX | 1 | 23.372 | 24.700 | 36.346 | 1.00 | 59.00 |
| 4449 | C6 | DEX | 1 | 23.965 | 25.312 | 35.061 | 1.00 | 59.00 |
| 4450 | H61 | DEX | 1 | 24.996 | 25.542 | 35.157 | 1.00 | 59.00 |
| 4451 | H62 | DEX | 1 | 23.444 | 26.267 | 34.853 | 1.00 | 59.00 |
| 4452 | C7 | DEX | 1 | 23.752 | 24.345 | 33.877 | 1.00 | 59.00 |
| 4453 | H71 | DEX | 1 | 24.370 | 23.444 | 34.001 | 1.00 | 59.00 |
| 4454 | H72 | DEX | 1 | 24.092 | 24.829 | 32.956 | 1.00 | 59.00 |
| 4455 | C8 | DEX | 1 | 22.275 | 23.885 | 33.692 | 1.00 | 59.00 |
| 4456 | H8 | DEX | 1 | 21.638 | 24.764 | 33.460 | 1.00 | 59.00 |
| 4457 | C9 | DEX | 1 | 21.676 | 23.232 | 35.081 | 1.00 | 59.00 |
| 4458 | C10 | DEX | 1 | 21.819 | 24.294 | 36.329 | 1.00 | 59.00 |
| 4459 | C11 | DEX | 1 | 20.197 | 22.585 | 34.938 | 1.00 | 59.00 |
| 4460 | H11 | DEX | 1 | 20.028 | 21.974 | 35.784 | 1.00 | 59.00 |
| 4461 | C12 | DEX | 1 | 20.107 | 21.699 | 33.700 | 1.00 | 59.00 |
| 4462 | H121 | DEX | 1 | 19.130 | 21.365 | 33.602 | 1.00 | 59.00 |
| 4463 | H122 | DEX | 1 | 20.720 | 20.795 | 33.859 | 1.00 | 59.00 |
| 4464 | C13 | DEX | 1 | 20.600 | 22.429 | 32.344 | 1.00 | 59.00 |
| 4465 | C14 | DEX | 1 | 22.105 | 22.863 | 32.515 | 1.00 | 59.00 |
| 4466 | H14 | DEX | 1 | 22.701 | 21.953 | 32.834 | 1.00 | 59.00 |
| 4467 | C15 | DEX | 1 | 22.602 | 23.242 | 31.129 | 1.00 | 59.00 |

TABLE 3 (continued)

| ATOMIC COORDINATES FOR THE GR/TIF2/DEX MODEL USED IN MOLECULAR REPLACEMENT | | | | | | | | |
|---|---|---|---|---|---|---|---|---|
| ATOM | ATOM TYPE | RESIDUE | # | X | Y | Z | B | ATOM |
| 4468 | H151 | DEX | 1 | 23.685 | 23.110 | 31.097 | 1.00 | 59.00 |
| 4469 | H152 | DEX | 1 | 22.383 | 24.310 | 30.934 | 1.00 | 59.00 |
| 4470 | C16 | DEX | 1 | 21.806 | 22.306 | 30.152 | 1.00 | 59.00 |
| 4471 | H16 | DEX | 1 | 21.207 | 22.984 | 29.504 | 1.00 | 59.00 |
| 4472 | C17 | DEX | 1 | 20.783 | 21.450 | 31.097 | 1.00 | 59.00 |
| 4473 | C18 | DEX | 1 | 19.540 | 23.677 | 31.944 | 1.00 | 59.00 |
| 4474 | H181 | DEX | 1 | 19.873 | 24.157 | 31.015 | 1.00 | 59.00 |
| 4475 | H182 | DEX | 1 | 18.547 | 23.297 | 31.792 | 1.00 | 59.00 |
| 4476 | H183 | DEX | 1 | 19.525 | 24.449 | 32.700 | 1.00 | 59.00 |
| 4477 | C19 | DEX | 1 | 20.959 | 25.638 | 36.205 | 1.00 | 59.00 |
| 4478 | H191 | DEX | 1 | 21.232 | 26.215 | 35.303 | 1.00 | 59.00 |
| 4479 | H192 | DEX | 1 | 19.899 | 25.426 | 36.127 | 1.00 | 59.00 |
| 4480 | H193 | DEX | 1 | 21.132 | 26.270 | 37.072 | 1.00 | 59.00 |
| 4481 | C20 | DEX | 1 | 19.417 | 21.067 | 30.421 | 1.00 | 59.00 |
| 4482 | C21 | DEX | 1 | 18.443 | 20.176 | 31.204 | 1.00 | 59.00 |
| 4483 | H211 | DEX | 1 | 17.932 | 20.800 | 31.959 | 1.00 | 59.00 |
| 4484 | H212 | DEX | 1 | 19.031 | 19.423 | 31.779 | 1.00 | 59.00 |
| 4485 | C22 | DEX | 1 | 22.671 | 21.454 | 29.301 | 1.00 | 59.00 |
| 4486 | H221 | DEX | 1 | 22.061 | 20.835 | 28.644 | 1.00 | 59.00 |
| 4487 | H222 | DEX | 1 | 23.334 | 22.077 | 28.688 | 1.00 | 59.00 |
| 4488 | H223 | DEX | 1 | 23.300 | 20.785 | 29.933 | 1.00 | 59.00 |
| 4489 | F1 | DEX | 1 | 22.519 | 22.128 | 35.397 | 1.00 | 59.00 |
| 4490 | O | DEX | 1 | 24.201 | 23.808 | 39.692 | 1.00 | 59.00 |
| 4491 | 02 | DEX | 1 | 19.179 | 23.598 | 34.905 | 1.00 | 59.00 |
| 4492 | HO2 | DEX | 1 | 18.367 | 23.168 | 34.580 | 1.00 | 59.00 |
| 4493 | 03 | DEX | 1 | 21.444 | 20.210 | 31.554 | 1.00 | 59.00 |
| 4494 | H3 | DEX | 1 | 21.502 | 19.648 | 30.802 | 1.00 | 59.00 |
| 4495 | 04 | DEX | 1 | 19.127 | 21.505 | 29.299 | 1.00 | 59.00 |
| 4496 | 05 | DEX | 1 | 17.530 | 19.572 | 30.381 | 1.00 | 59.00 |
| 4497 | H5 | DEX | 1 | 17.435 | 18.711 | 30.744 | 1.00 | 59.00 |

TABLE 4

ATOMIC COORDINATES OF AR IN COMPLEX WITH BICALUTAMIDE OBTAINED FROM HOMOLOGY MODELING OF THE CRYSTAL STRUCTURE COORDINATES OF GRα IN COMPLEX WITH FP

| Atom Number | Atom Type | Residue Name | Residue Number | X Coordinate | Y Coordinate | Z Coordinate |
|---|---|---|---|---|---|---|
| 1 | N | ILE | 672 | -7.802 | 44.333 | 21.810 |
| 2 | HN1 | ILE | 672 | -8.615 | 43.811 | 21.453 |
| 3 | HN2 | ILE | 672 | -8.089 | 44.905 | 22.617 |
| 4 | HN3 | ILE | 672 | -7.435 | 44.946 | 21.068 |
| 5 | CA | ILE | 672 | -6.772 | 43.402 | 22.220 |
| 6 | C | ILE | 672 | -5.691 | 44.331 | 22.701 |
| 7 | O | ILE | 672 | -5.692 | 45.515 | 22.363 |
| 8 | CB | ILE | 672 | -6.276 | 42.491 | 21.024 |
| 9 | CG1 | ILE | 672 | -5.637 | 43.272 | 19.831 |
| 10 | CG2 | ILE | 672 | -7.403 | 41.575 | 20.455 |
| 11 | CD1 | ILE | 672 | -4.861 | 42.419 | 18.807 |
| 12 | N | PHE | 673 | -4.769 | 43.820 | 23.494 |
| 13 | CA | PHE | 673 | -3.723 | 44.678 | 24.008 |
| 14 | HN | PHE | 673 | -4.793 | 42.850 | 23.738 |
| 15 | C | PHE | 673 | -2.862 | 45.295 | 22.900 |
| 16 | O | PHE | 673 | -2.512 | 46.478 | 22.951 |
| 17 | CB | PHE | 673 | -2.800 | 43.874 | 24.973 |
| 18 | CG | PHE | 673 | -1.802 | 44.693 | 25.806 |
| 19 | CD1 | PHE | 673 | -2.082 | 46.032 | 26.100 |
| 20 | CE1 | PHE | 673 | -1.154 | 46.810 | 26.784 |
| 21 | CZ | PHE | 673 | 0.054 | 46.249 | 27.194 |
| 22 | CE2 | PHE | 673 | 0.335 | 44.913 | 26.915 |
| 23 | CD2 | PHE | 673 | -0.590 | 44.136 | 26.221 |
| 24 | N | LEU | 674 | -2.522 | 44.494 | 21.898 |
| 25 | CA | LEU | 674 | -1.722 | 44.978 | 20.786 |
| 26 | HN | LEU | 674 | -2.821 | 43.540 | 21.909 |
| 27 | C | LEU | 674 | -2.622 | 45.837 | 19.898 |
| 28 | O | LEU | 674 | -2.149 | 46.600 | 19.056 |
| 29 | CB | LEU | 674 | -1.153 | 43.767 | 19.994 |
| 30 | CG | LEU | 674 | -2.157 | 42.828 | 19.272 |
| 31 | CD1 | LEU | 674 | -1.413 | 41.640 | 18.646 |
| 32 | CD2 | LEU | 674 | -3.264 | 42.312 | 20.205 |
| 33 | N | ASN | 675 | -3.931 | 45.693 | 20.088 |
| 34 | CA | ASN | 675 | -4.913 | 46.442 | 19.306 |
| 35 | HN | ASN | 675 | -4.252 | 45.054 | 20.786 |
| 36 | C | ASN | 675 | -4.860 | 47.910 | 19.668 |
| 37 | O | ASN | 675 | -4.889 | 48.806 | 18.829 |
| 38 | CB | ASN | 675 | -6.326 | 45.855 | 19.591 |
| 39 | CG | ASN | 675 | -7.519 | 46.577 | 18.957 |
| 40 | OD1 | ASN | 675 | -8.064 | 46.168 | 17.942 |
| 41 | ND2 | ASN | 675 | -7.952 | 47.679 | 19.507 |
| 42 | 1HD2 | ASN | 675 | -8.644 | 48.159 | 18.925 |
| 43 | 2HD2 | ASN | 675 | -7.420 | 48.042 | 20.298 |
| 44 | N | VAL | 676 | -4.799 | 48.126 | 20.963 |
| 45 | CA | VAL | 676 | -4.752 | 49.439 | 21.528 |
| 46 | HN | VAL | 676 | -4.785 | 47.339 | 21.580 |
| 47 | C | VAL | 676 | -3.362 | 50.002 | 21.308 |

| | | | | | | |
|---|---|---|---|---|---|---|
| 48 | O | VAL | 676 | -3.176 | 51.208 | 21.154 |
| 49 | CB | VAL | 676 | -5.057 | 49.282 | 23.071 |
| 50 | CG1 | VAL | 676 | -5.366 | 50.591 | 23.849 |
| 51 | CG2 | VAL | 676 | -6.239 | 48.333 | 23.383 |
| 52 | N | LEU | 677 | -2.374 | 49.119 | 21.292 |
| 53 | CA | LEU | 677 | -1.001 | 49.552 | 21.090 |
| 54 | HN | LEU | 677 | -2.574 | 48.147 | 21.419 |
| 55 | C | LEU | 677 | -0.869 | 50.119 | 19.665 |
| 56 | O | LEU | 677 | -0.131 | 51.073 | 19.421 |
| 57 | CB | LEU | 677 | -0.068 | 48.363 | 21.316 |
| 58 | CG | LEU | 677 | 1.249 | 48.527 | 22.075 |
| 59 | CD1 | LEU | 677 | 1.094 | 49.268 | 23.390 |
| 60 | CD2 | LEU | 677 | 1.764 | 47.137 | 22.328 |
| 61 | N | GLU | 678 | -1.606 | 49.544 | 18.727 |
| 62 | CA | GLU | 678 | -1.548 | 50.045 | 17.376 |
| 63 | HN | GLU | 678 | -2.197 | 48.770 | 18.954 |
| 64 | C | GLU | 678 | -2.330 | 51.346 | 17.200 |
| 65 | O | GLU | 678 | -1.845 | 52.246 | 16.524 |
| 66 | CB | GLU | 678 | -2.055 | 49.004 | 16.399 |
| 67 | CG | GLU | 678 | -2.187 | 49.525 | 14.999 |
| 68 | CD | GLU | 678 | -2.559 | 48.425 | 14.030 |
| 69 | OE1 | GLU | 678 | -2.750 | 48.734 | 12.830 |
| 70 | OE2 | GLU | 678 | -2.654 | 47.252 | 14.473 |
| 71 | HE2 | GLU | 678 | -2.890 | 46.667 | 13.769 |
| 72 | N | ALA | 679 | -3.518 | 51.484 | 17.797 |
| 73 | CA | ALA | 679 | -4.251 | 52.738 | 17.584 |
| 74 | HN | ALA | 679 | -3.895 | 50.756 | 18.370 |
| 75 | C | ALA | 679 | -3.645 | 53.964 | 18.253 |
| 76 | O | ALA | 679 | -4.043 | 55.082 | 17.931 |
| 77 | CB | ALA | 679 | -5.746 | 52.562 | 17.903 |
| 78 | N | ILE | 680 | -2.694 | 53.786 | 19.173 |
| 79 | CA | ILE | 680 | -2.079 | 54.944 | 19.831 |
| 80 | HN | ILE | 680 | -2.401 | 52.861 | 19.414 |
| 81 | C | ILE | 680 | -0.672 | 55.231 | 19.325 |
| 82 | O | ILE | 680 | 0.069 | 56.001 | 19.939 |
| 83 | CB | ILE | 680 | -1.980 | 54.783 | 21.367 |
| 84 | CG2 | ILE | 680 | -3.269 | 54.216 | 21.925 |
| 85 | CG1 | ILE | 680 | -0.801 | 53.879 | 21.725 |
| 86 | CD1 | ILE | 680 | -0.571 | 53.751 | 23.208 |
| 87 | N | GLU | 681 | -0.301 | 54.606 | 18.214 |
| 88 | CA | GLU | 681 | 1.020 | 54.801 | 17.640 |
| 89 | HN | GLU | 681 | -0.944 | 53.988 | 17.762 |
| 90 | C | GLU | 681 | 1.093 | 56.227 | 17.144 |
| 91 | O | GLU | 681 | 0.147 | 56.718 | 16.543 |
| 92 | CB | GLU | 681 | 1.237 | 53.825 | 16.483 |
| 93 | CG | GLU | 681 | 2.643 | 53.807 | 15.879 |
| 94 | CD | GLU | 681 | 3.760 | 53.831 | 16.920 |
| 95 | OE1 | GLU | 681 | 3.570 | 53.258 | 18.018 |
| 96 | OE2 | GLU | 681 | 4.832 | 54.412 | 16.631 |
| 97 | HE2 | GLU | 681 | 5.431 | 54.351 | 17.360 |
| 98 | N | PRO | 682 | 2.175 | 56.936 | 17.458 |
| 99 | CA | PRO | 682 | 2.103 | 58.288 | 16.900 |
| 100 | CD | PRO | 682 | 2.934 | 56.915 | 18.715 |
| 101 | C | PRO | 682 | 2.205 | 58.244 | 15.379 |
| 102 | O | PRO | 682 | 2.532 | 57.209 | 14.789 |

| | | | | | | |
|---|---|---|---|---|---|---|
| 103 | CB | PRO | 682 | 3.278 | 59.025 | 17.552 |
| 104 | CG | PRO | 682 | 3.904 | 58.034 | 18.527 |
| 105 | N | GLY | 683 | 1.908 | 59.358 | 14.734 |
| 106 | CA | GLY | 683 | 1.978 | 59.373 | 13.291 |
| 107 | HN | GLY | 683 | 1.640 | 60.179 | 15.237 |
| 108 | C | GLY | 683 | 3.198 | 60.165 | 12.862 |
| 109 | O | GLY | 683 | 3.578 | 61.122 | 13.536 |
| 110 | N | VAL | 684 | 3.807 | 59.769 | 11.747 |
| 111 | CA | VAL | 684 | 5.011 | 60.440 | 11.267 |
| 112 | HN | VAL | 684 | 3.433 | 58.999 | 11.229 |
| 113 | C | VAL | 684 | 4.883 | 61.937 | 11.156 |
| 114 | O | VAL | 684 | 3.915 | 62.458 | 10.610 |
| 115 | CB | VAL | 684 | 5.466 | 59.926 | 9.887 |
| 116 | CG1 | VAL | 684 | 5.969 | 58.504 | 10.001 |
| 117 | CG2 | VAL | 684 | 4.322 | 60.031 | 8.885 |
| 118 | N | VAL | 685 | 5.876 | 62.639 | 11.673 |
| 119 | CA | VAL | 685 | 5.853 | 64.080 | 11.590 |
| 120 | HN | VAL | 685 | 6.640 | 62.174 | 12.120 |
| 121 | C | VAL | 685 | 7.097 | 64.568 | 10.856 |
| 122 | O | VAL | 685 | 8.225 | 64.160 | 11.169 |
| 123 | CB | VAL | 685 | 5.760 | 64.727 | 13.029 |
| 124 | CG1 | VAL | 685 | 6.902 | 64.371 | 14.020 |
| 125 | CG2 | VAL | 685 | 5.694 | 66.273 | 13.019 |
| 126 | N | CYS | 686 | 6.859 | 65.427 | 9.863 |
| 127 | CA | CYS | 686 | 7.899 | 65.995 | 9.007 |
| 128 | HN | CYS | 686 | 5.911 | 65.698 | 9.695 |
| 129 | C | CYS | 686 | 8.844 | 66.938 | 9.690 |
| 130 | O | CYS | 686 | 8.559 | 67.480 | 10.759 |
| 131 | CB | CYS | 686 | 7.230 | 66.625 | 7.770 |
| 132 | SG | CYS | 686 | 6.474 | 65.353 | 6.733 |
| 133 | HG | CYS | 686 | 7.391 | 65.368 | 5.769 |
| 134 | N | ALA | 687 | 9.970 | 67.152 | 9.023 |
| 135 | CA | ALA | 687 | 11.028 | 67.995 | 9.539 |
| 136 | HN | ALA | 687 | 10.094 | 66.714 | 8.133 |
| 137 | C | ALA | 687 | 10.956 | 69.476 | 9.185 |
| 138 | O | ALA | 687 | 11.644 | 70.281 | 9.806 |
| 139 | CB | ALA | 687 | 12.359 | 67.437 | 9.100 |
| 140 | N | GLY | 688 | 10.141 | 69.846 | 8.202 |
| 141 | CA | GLY | 688 | 10.070 | 71.249 | 7.828 |
| 142 | HN | GLY | 688 | 9.583 | 69.167 | 7.725 |
| 143 | C | GLY | 688 | 11.444 | 71.762 | 7.423 |
| 144 | O | GLY | 688 | 11.889 | 72.816 | 7.879 |
| 145 | N | HIS | 689 | 12.131 | 71.003 | 6.571 |
| 146 | CA | HIS | 689 | 13.464 | 71.376 | 6.102 |
| 147 | HN | HIS | 689 | 11.722 | 70.152 | 6.241 |
| 148 | C | HIS | 689 | 13.342 | 72.135 | 4.797 |
| 149 | O | HIS | 689 | 12.377 | 71.977 | 4.040 |
| 150 | CB | HIS | 689 | 14.316 | 70.110 | 5.908 |
| 151 | CG | HIS | 689 | 15.770 | 70.426 | 5.713 |
| 152 | ND1 | HIS | 689 | 16.648 | 70.861 | 6.702 |
| 153 | CE1 | HIS | 689 | 17.810 | 70.790 | 6.025 |
| 154 | NE2 | HIS | 689 | 17.774 | 70.371 | 4.731 |
| 155 | CD2 | HIS | 689 | 16.438 | 70.133 | 4.533 |
| 156 | HE2 | HIS | 689 | 18.550 | 70.256 | 4.064 |
| 157 | N | ASP | 690 | 14.333 | 72.973 | 4.559 |

| | | | | | | |
|---|---|---|---|---|---|---|
| 158 | CA | ASP | 690 | 14.410 | 73.739 | 3.341 |
| 159 | HN | ASP | 690 | 15.053 | 73.080 | 5.244 |
| 160 | C | ASP | 690 | 15.539 | 73.046 | 2.609 |
| 161 | O | ASP | 690 | 16.711 | 73.253 | 2.928 |
| 162 | CB | ASP | 690 | 14.800 | 75.178 | 3.628 |
| 163 | CG | ASP | 690 | 14.786 | 76.022 | 2.386 |
| 164 | OD1 | ASP | 690 | 14.680 | 75.431 | 1.294 |
| 165 | OD2 | ASP | 690 | 14.880 | 77.259 | 2.497 |
| 166 | HD2 | ASP | 690 | 14.857 | 77.655 | 1.640 |
| 167 | N | ASN | 691 | 15.183 | 72.196 | 1.658 |
| 168 | CA | ASN | 691 | 16.168 | 71.457 | 0.886 |
| 169 | HN | ASN | 691 | 14.211 | 72.058 | 1.467 |
| 170 | C | ASN | 691 | 16.414 | 72.208 | -0.411 |
| 171 | O | ASN | 691 | 17.014 | 71.684 | -1.351 |
| 172 | CB | ASN | 691 | 15.643 | 70.020 | 0.600 |
| 173 | CG | ASN | 691 | 14.332 | 69.904 | -0.185 |
| 174 | OD1 | ASN | 691 | 14.308 | 69.649 | -1.380 |
| 175 | ND2 | ASN | 691 | 13.204 | 70.107 | 0.441 |
| 176 | 1HD2 | ASN | 691 | 12.406 | 70.157 | -0.199 |
| 177 | 2HD2 | ASN | 691 | 13.262 | 70.406 | 1.415 |
| 178 | N | ASN | 692 | 15.935 | 73.449 | -0.435 |
| 179 | CA | ASN | 692 | 16.057 | 74.325 | -1.592 |
| 180 | HN | ASN | 692 | 15.470 | 73.797 | 0.379 |
| 181 | C | ASN | 692 | 17.406 | 74.995 | -1.581 |
| 182 | O | ASN | 692 | 17.708 | 75.846 | -2.418 |
| 183 | CB | ASN | 692 | 14.916 | 75.383 | -1.546 |
| 184 | CG | ASN | 692 | 15.214 | 76.694 | -0.812 |
| 185 | OD1 | ASN | 692 | 15.510 | 77.721 | -1.406 |
| 186 | ND2 | ASN | 692 | 15.173 | 76.709 | 0.493 |
| 187 | 1HD2 | ASN | 692 | 15.534 | 77.583 | 0.885 |
| 188 | 2HD2 | ASN | 692 | 15.013 | 75.817 | 0.961 |
| 189 | N | GLN | 693 | 18.223 | 74.651 | -0.605 |
| 190 | CA | GLN | 693 | 19.516 | 75.267 | -0.548 |
| 191 | HN | GLN | 693 | 17.947 | 73.976 | 0.079 |
| 192 | C | GLN | 693 | 20.441 | 74.218 | 0.032 |
| 193 | O | GLN | 693 | 20.051 | 73.480 | 0.945 |
| 194 | CB | GLN | 693 | 19.445 | 76.508 | 0.368 |
| 195 | CG | GLN | 693 | 18.110 | 77.323 | 0.324 |
| 196 | CD | GLN | 693 | 17.987 | 78.603 | 1.161 |
| 197 | OE1 | GLN | 693 | 16.977 | 79.287 | 1.123 |
| 198 | NE2 | GLN | 693 | 18.986 | 78.991 | 1.913 |
| 199 | 2HE2 | GLN | 693 | 19.825 | 78.413 | 1.846 |
| 200 | 1HE2 | GLN | 693 | 18.847 | 79.886 | 2.386 |
| 201 | N | PRO | 694 | 21.688 | 74.161 | -0.473 |
| 202 | CA | PRO | 694 | 22.654 | 73.163 | -0.016 |
| 203 | CD | PRO | 694 | 22.365 | 75.246 | -1.206 |
| 204 | C | PRO | 694 | 22.661 | 72.720 | 1.398 |
| 205 | O | PRO | 694 | 22.222 | 73.405 | 2.316 |
| 206 | CB | PRO | 694 | 24.018 | 73.697 | -0.465 |
| 207 | CG | PRO | 694 | 23.811 | 75.102 | -0.777 |
| 208 | N | ASP | 695 | 23.175 | 71.516 | 1.550 |
| 209 | CA | ASP | 695 | 23.249 | 70.949 | 2.849 |
| 210 | HN | ASP | 695 | 23.510 | 71.006 | 0.757 |
| 211 | C | ASP | 695 | 24.549 | 71.260 | 3.463 |
| 212 | O | ASP | 695 | 25.441 | 71.872 | 2.877 |

| | | | | | | |
|---|---|---|---|---|---|---|
| 213 | CB | ASP | 695 | 23.090 | 69.450 | 2.784 |
| 214 | CG | ASP | 695 | 21.834 | 69.063 | 2.108 |
| 215 | OD1 | ASP | 695 | 20.895 | 69.889 | 2.139 |
| 216 | OD2 | ASP | 695 | 21.777 | 67.951 | 1.553 |
| 217 | HD2 | ASP | 695 | 20.924 | 67.836 | 1.165 |
| 218 | N | SER | 696 | 24.636 | 70.819 | 4.690 |
| 219 | CA | SER | 696 | 25.822 | 70.972 | 5.447 |
| 220 | HN | SER | 696 | 23.847 | 70.365 | 5.104 |
| 221 | C | SER | 696 | 25.383 | 70.258 | 6.675 |
| 222 | O | SER | 696 | 24.203 | 70.267 | 7.041 |
| 223 | CB | SER | 696 | 26.139 | 72.446 | 5.692 |
| 224 | OG | SER | 696 | 25.437 | 73.057 | 6.764 |
| 225 | HG | SER | 696 | 25.733 | 73.975 | 6.792 |
| 226 | N | PHE | 697 | 26.530 | 69.629 | 7.299 |
| 227 | CA | PHE | 697 | 26.464 | 68.629 | 8.376 |
| 228 | HN | PHE | 697 | 27.449 | 69.713 | 6.888 |
| 229 | C | PHE | 697 | 25.787 | 69.190 | 9.644 |
| 230 | O | PHE | 697 | 24.983 | 68.458 | 10.181 |
| 231 | CB | PHE | 697 | 27.792 | 67.916 | 8.744 |
| 232 | CG | PHE | 697 | 28.885 | 68.879 | 9.171 |
| 233 | CD1 | PHE | 697 | 29.085 | 69.233 | 10.558 |
| 234 | CE1 | PHE | 697 | 30.116 | 70.166 | 10.939 |
| 235 | CZ | PHE | 697 | 30.974 | 70.747 | 9.940 |
| 236 | CE2 | PHE | 697 | 30.793 | 70.386 | 8.561 |
| 237 | CD2 | PHE | 697 | 29.762 | 69.457 | 8.182 |
| 238 | N | ALA | 698 | 26.114 | 70.494 | 10.112 |
| 239 | CA | ALA | 698 | 25.527 | 71.392 | 11.074 |
| 240 | HN | ALA | 698 | 27.103 | 70.500 | 9.969 |
| 241 | C | ALA | 698 | 24.166 | 71.500 | 10.449 |
| 242 | O | ALA | 698 | 23.510 | 70.508 | 10.391 |
| 243 | CB | ALA | 698 | 26.288 | 72.729 | 11.082 |
| 244 | N | ALA | 699 | 23.678 | 72.598 | 9.944 |
| 245 | CA | ALA | 699 | 22.339 | 72.472 | 9.371 |
| 246 | HN | ALA | 699 | 24.182 | 73.462 | 9.948 |
| 247 | C | ALA | 699 | 21.405 | 71.259 | 9.586 |
| 248 | O | ALA | 699 | 20.255 | 71.418 | 9.959 |
| 249 | CB | ALA | 699 | 22.524 | 72.827 | 7.885 |
| 250 | N | LEU | 700 | 21.874 | 70.055 | 9.313 |
| 251 | CA | LEU | 700 | 21.016 | 68.898 | 9.427 |
| 252 | HN | LEU | 700 | 22.825 | 69.943 | 9.027 |
| 253 | C | LEU | 700 | 20.937 | 68.217 | 10.741 |
| 254 | O | LEU | 700 | 19.955 | 67.556 | 11.097 |
| 255 | CB | LEU | 700 | 21.502 | 67.918 | 8.322 |
| 256 | CG | LEU | 700 | 21.248 | 68.304 | 6.839 |
| 257 | CD1 | LEU | 700 | 21.362 | 67.062 | 5.944 |
| 258 | CD2 | LEU | 700 | 19.881 | 68.973 | 6.626 |
| 259 | N | LEU | 701 | 22.241 | 67.945 | 11.274 |
| 260 | CA | LEU | 701 | 22.393 | 67.526 | 12.650 |
| 261 | HN | LEU | 701 | 23.099 | 68.182 | 10.776 |
| 262 | C | LEU | 701 | 21.407 | 68.489 | 13.358 |
| 263 | O | LEU | 701 | 20.678 | 68.015 | 14.213 |
| 264 | CB | LEU | 701 | 23.817 | 67.670 | 13.228 |
| 265 | CG | LEU | 701 | 24.838 | 66.645 | 12.675 |
| 266 | CD1 | LEU | 701 | 26.284 | 67.148 | 12.829 |
| 267 | CD2 | LEU | 701 | 24.711 | 65.277 | 13.353 |

| | | | | | | |
|---|---|---|---|---|---|---|
| 268 | N | SER | 702 | 21.348 | 69.874 | 12.942 |
| 269 | CA | SER | 702 | 20.571 | 70.736 | 13.707 |
| 270 | HN | SER | 702 | 21.934 | 70.156 | 12.182 |
| 271 | C | SER | 702 | 19.114 | 70.479 | 13.542 |
| 272 | O | SER | 702 | 18.437 | 70.227 | 14.534 |
| 273 | CB | SER | 702 | 20.916 | 72.229 | 13.479 |
| 274 | OG | SER | 702 | 21.723 | 72.780 | 14.526 |
| 275 | HG | SER | 702 | 21.208 | 72.741 | 15.337 |
| 276 | N | SER | 703 | 18.549 | 70.230 | 12.261 |
| 277 | CA | SER | 703 | 17.171 | 69.657 | 12.150 |
| 278 | HN | SER | 703 | 19.093 | 70.385 | 11.423 |
| 279 | C | SER | 703 | 16.944 | 68.360 | 13.073 |
| 280 | O | SER | 703 | 15.874 | 68.265 | 13.654 |
| 281 | CB | SER | 703 | 16.690 | 69.307 | 10.728 |
| 282 | OG | SER | 703 | 17.300 | 68.126 | 10.230 |
| 283 | HG | SER | 703 | 18.260 | 68.280 | 10.219 |
| 284 | N | LEU | 704 | 17.917 | 67.301 | 13.105 |
| 285 | CA | LEU | 704 | 17.740 | 65.962 | 13.703 |
| 286 | HN | LEU | 704 | 18.682 | 67.368 | 12.438 |
| 287 | C | LEU | 704 | 17.494 | 66.130 | 15.217 |
| 288 | O | LEU | 704 | 16.800 | 65.338 | 15.832 |
| 289 | CB | LEU | 704 | 18.981 | 65.050 | 13.574 |
| 290 | CG | LEU | 704 | 18.981 | 63.995 | 12.452 |
| 291 | CD1 | LEU | 704 | 18.888 | 64.522 | 11.006 |
| 292 | CD2 | LEU | 704 | 20.249 | 63.146 | 12.628 |
| 293 | N | ASN | 705 | 18.197 | 67.197 | 15.831 |
| 294 | CA | ASN | 705 | 18.013 | 67.600 | 17.218 |
| 295 | HN | ASN | 705 | 18.768 | 67.838 | 15.279 |
| 296 | C | ASN | 705 | 16.526 | 68.043 | 17.385 |
| 297 | O | ASN | 705 | 15.910 | 67.607 | 18.336 |
| 298 | CB | ASN | 705 | 18.960 | 68.709 | 17.715 |
| 299 | CG | ASN | 705 | 20.404 | 68.204 | 17.811 |
| 300 | OD1 | ASN | 705 | 20.926 | 67.453 | 17.012 |
| 301 | ND2 | ASN | 705 | 21.132 | 68.648 | 18.913 |
| 302 | 1HD2 | ASN | 705 | 21.930 | 68.109 | 19.248 |
| 303 | 2HD2 | ASN | 705 | 20.900 | 69.547 | 19.352 |
| 304 | N | GLU | 706 | 15.954 | 68.965 | 16.451 |
| 305 | CA | GLU | 706 | 14.614 | 69.578 | 16.579 |
| 306 | HN | GLU | 706 | 16.550 | 69.275 | 15.686 |
| 307 | C | GLU | 706 | 13.565 | 68.456 | 16.435 |
| 308 | O | GLU | 706 | 12.642 | 68.415 | 17.230 |
| 309 | CB | GLU | 706 | 14.253 | 70.731 | 15.602 |
| 310 | CG | GLU | 706 | 15.227 | 71.930 | 15.629 |
| 311 | CD | GLU | 706 | 15.722 | 72.275 | 17.032 |
| 312 | OE1 | GLU | 706 | 15.056 | 72.441 | 18.043 |
| 313 | OE2 | GLU | 706 | 17.073 | 72.369 | 17.008 |
| 314 | HE2 | GLU | 706 | 17.366 | 72.541 | 17.911 |
| 315 | N | LEU | 707 | 13.710 | 67.522 | 15.388 |
| 316 | CA | LEU | 707 | 12.797 | 66.378 | 15.245 |
| 317 | HN | LEU | 707 | 14.627 | 67.512 | 14.939 |
| 318 | C | LEU | 707 | 12.929 | 65.492 | 16.540 |
| 319 | O | LEU | 707 | 11.940 | 65.187 | 17.173 |
| 320 | CB | LEU | 707 | 12.868 | 65.572 | 13.923 |
| 321 | CG | LEU | 707 | 11.772 | 64.471 | 13.807 |
| 322 | CD1 | LEU | 707 | 10.351 | 64.964 | 14.105 |

| | | | | | | |
|---|---|---|---|---|---|---|
| 323 | CD2 | LEU | 707 | 11.741 | 63.819 | 12.412 |
| 324 | N | GLY | 708 | 14.203 | 65.213 | 17.065 |
| 325 | CA | GLY | 708 | 14.409 | 64.586 | 18.375 |
| 326 | HN | GLY | 708 | 15.077 | 65.405 | 16.550 |
| 327 | C | GLY | 708 | 13.607 | 65.286 | 19.523 |
| 328 | O | GLY | 708 | 13.223 | 64.551 | 20.422 |
| 329 | N | GLU | 709 | 13.368 | 66.711 | 19.424 |
| 330 | CA | GLU | 709 | 12.430 | 67.291 | 20.455 |
| 331 | HN | GLU | 709 | 13.232 | 66.870 | 18.416 |
| 332 | C | GLU | 709 | 11.025 | 66.813 | 20.116 |
| 333 | O | GLU | 709 | 10.470 | 66.147 | 20.971 |
| 334 | CB | GLU | 709 | 12.087 | 68.724 | 20.989 |
| 335 | CG | GLU | 709 | 11.457 | 69.883 | 20.147 |
| 336 | CD | GLU | 709 | 10.300 | 70.500 | 20.944 |
| 337 | OE1 | GLU | 709 | 9.121 | 70.204 | 20.833 |
| 338 | OE2 | GLU | 709 | 10.727 | 71.422 | 21.860 |
| 339 | HE2 | GLU | 709 | 10.002 | 71.760 | 22.412 |
| 340 | N | ARG | 710 | 10.411 | 67.381 | 18.930 |
| 341 | CA | ARG | 710 | 9.015 | 67.165 | 18.680 |
| 342 | HN | ARG | 710 | 11.030 | 67.728 | 18.226 |
| 343 | C | ARG | 710 | 8.576 | 65.706 | 18.863 |
| 344 | O | ARG | 710 | 7.454 | 65.462 | 19.302 |
| 345 | CB | ARG | 710 | 8.675 | 67.723 | 17.311 |
| 346 | CG | ARG | 710 | 9.112 | 69.185 | 17.218 |
| 347 | CD | ARG | 710 | 8.843 | 69.723 | 15.849 |
| 348 | NE | ARG | 710 | 7.501 | 69.334 | 15.445 |
| 349 | CZ | ARG | 710 | 7.222 | 68.779 | 14.275 |
| 350 | NH1 | ARG | 710 | 8.172 | 68.495 | 13.340 |
| 351 | 1HH1 | ARG | 710 | 7.915 | 68.079 | 12.463 |
| 352 | 2HH1 | ARG | 710 | 9.132 | 68.701 | 13.519 |
| 353 | NH2 | ARG | 710 | 5.921 | 68.492 | 13.989 |
| 354 | 1HH2 | ARG | 710 | 5.691 | 68.079 | 13.109 |
| 355 | 2HH2 | ARG | 710 | 5.210 | 68.697 | 14.659 |
| 356 | HE | ARG | 710 | 6.749 | 69.494 | 16.085 |
| 357 | N | GLN | 711 | 9.493 | 64.717 | 18.415 |
| 358 | CA | GLN | 711 | 9.092 | 63.326 | 18.536 |
| 359 | HN | GLN | 711 | 10.448 | 64.947 | 18.164 |
| 360 | C | GLN | 711 | 8.965 | 62.993 | 20.054 |
| 361 | O | GLN | 711 | 7.965 | 62.385 | 20.362 |
| 362 | CB | GLN | 711 | 9.955 | 62.253 | 17.866 |
| 363 | CG | GLN | 711 | 9.197 | 60.900 | 17.986 |
| 364 | CD | GLN | 711 | 9.873 | 59.707 | 17.350 |
| 365 | OE1 | GLN | 711 | 9.305 | 58.632 | 17.263 |
| 366 | NE2 | GLN | 711 | 11.203 | 59.917 | 16.945 |
| 367 | 1HE2 | GLN | 711 | 11.810 | 59.096 | 17.046 |
| 368 | 2HE2 | GLN | 711 | 11.540 | 60.821 | 17.305 |
| 369 | N | LEU | 712 | 10.009 | 63.314 | 20.993 |
| 370 | CA | LEU | 712 | 10.114 | 62.841 | 22.369 |
| 371 | HN | LEU | 712 | 10.567 | 64.088 | 20.697 |
| 372 | C | LEU | 712 | 8.899 | 63.352 | 23.142 |
| 373 | O | LEU | 712 | 8.473 | 62.756 | 24.139 |
| 374 | CB | LEU | 712 | 11.437 | 63.327 | 23.028 |
| 375 | CG | LEU | 712 | 11.585 | 63.178 | 24.566 |
| 376 | CD1 | LEU | 712 | 12.902 | 63.812 | 25.033 |
| 377 | CD2 | LEU | 712 | 10.409 | 63.797 | 25.339 |

| | | | | | | |
|---|---|---|---|---|---|---|
| 378 | N | VAL | 713 | 8.336 | 64.448 | 22.642 |
| 379 | CA | VAL | 713 | 7.145 | 65.049 | 23.218 |
| 380 | HN | VAL | 713 | 8.748 | 64.876 | 21.837 |
| 381 | C | VAL | 713 | 5.974 | 64.150 | 22.835 |
| 382 | O | VAL | 713 | 5.085 | 63.912 | 23.641 |
| 383 | CB | VAL | 713 | 6.926 | 66.518 | 22.679 |
| 384 | CG1 | VAL | 713 | 7.690 | 67.647 | 23.424 |
| 385 | CG2 | VAL | 713 | 7.287 | 66.698 | 21.185 |
| 386 | N | HIS | 714 | 5.987 | 63.628 | 21.616 |
| 387 | CA | HIS | 714 | 4.923 | 62.724 | 21.198 |
| 388 | HN | HIS | 714 | 6.728 | 63.855 | 20.983 |
| 389 | C | HIS | 714 | 5.107 | 61.377 | 21.913 |
| 390 | O | HIS | 714 | 4.164 | 60.573 | 22.006 |
| 391 | CB | HIS | 714 | 4.961 | 62.530 | 19.673 |
| 392 | CG | HIS | 714 | 3.692 | 62.979 | 19.010 |
| 393 | ND1 | HIS | 714 | 2.568 | 62.188 | 18.780 |
| 394 | CE1 | HIS | 714 | 1.770 | 63.092 | 18.181 |
| 395 | NE2 | HIS | 714 | 2.251 | 64.351 | 17.998 |
| 396 | CD2 | HIS | 714 | 3.507 | 64.273 | 18.542 |
| 397 | HE2 | HIS | 714 | 1.793 | 65.165 | 17.563 |
| 398 | N | VAL | 715 | 6.328 | 61.159 | 22.422 |
| 399 | CA | VAL | 715 | 6.725 | 59.930 | 23.143 |
| 400 | HN | VAL | 715 | 7.016 | 61.876 | 22.307 |
| 401 | C | VAL | 715 | 6.098 | 59.865 | 24.535 |
| 402 | O | VAL | 715 | 5.860 | 58.789 | 25.096 |
| 403 | CB | VAL | 715 | 8.302 | 59.868 | 23.233 |
| 404 | CG1 | VAL | 715 | 8.899 | 58.734 | 24.112 |
| 405 | CG2 | VAL | 715 | 9.004 | 59.733 | 21.861 |
| 406 | N | VAL | 716 | 5.853 | 61.042 | 25.092 |
| 407 | CA | VAL | 716 | 5.240 | 61.166 | 26.387 |
| 408 | HN | VAL | 716 | 6.102 | 61.873 | 24.594 |
| 409 | C | VAL | 716 | 3.724 | 60.950 | 26.214 |
| 410 | O | VAL | 716 | 3.127 | 60.223 | 27.013 |
| 411 | CB | VAL | 716 | 5.570 | 62.543 | 26.984 |
| 412 | CG1 | VAL | 716 | 4.977 | 62.676 | 28.383 |
| 413 | CG2 | VAL | 716 | 7.094 | 62.724 | 27.016 |
| 414 | N | LYS | 717 | 3.089 | 61.546 | 25.190 |
| 415 | CA | LYS | 717 | 1.644 | 61.294 | 25.014 |
| 416 | HN | LYS | 717 | 3.583 | 62.147 | 24.561 |
| 417 | C | LYS | 717 | 1.536 | 59.802 | 24.651 |
| 418 | O | LYS | 717 | 0.566 | 59.132 | 25.009 |
| 419 | CB | LYS | 717 | 0.962 | 62.138 | 23.883 |
| 420 | CG | LYS | 717 | 0.856 | 63.702 | 24.059 |
| 421 | CD | LYS | 717 | -0.247 | 64.447 | 23.158 |
| 422 | CE | LYS | 717 | -0.072 | 64.305 | 21.606 |
| 423 | NZ | LYS | 717 | -0.903 | 65.223 | 20.724 |
| 424 | HZ1 | LYS | 717 | -0.504 | 66.172 | 20.745 |
| 425 | HZ2 | LYS | 717 | -0.896 | 64.869 | 19.757 |
| 426 | HZ3 | LYS | 717 | -1.844 | 65.248 | 21.062 |
| 427 | N | TRP | 718 | 2.523 | 59.265 | 23.943 |
| 428 | CA | TRP | 718 | 2.427 | 57.855 | 23.605 |
| 429 | HN | TRP | 718 | 3.307 | 59.813 | 23.652 |
| 430 | C | TRP | 718 | 2.530 | 57.001 | 24.873 |
| 431 | O | TRP | 718 | 1.646 | 56.190 | 25.144 |
| 432 | CB | TRP | 718 | 3.514 | 57.461 | 22.599 |

| | | | | | | |
|---|---|---|---|---|---|---|
| 433 | CG | TRP | 718 | 3.692 | 55.982 | 22.458 |
| 434 | CD1 | TRP | 718 | 2.864 | 55.102 | 21.832 |
| 435 | NE1 | TRP | 718 | 3.387 | 53.831 | 21.906 |
| 436 | CE2 | TRP | 718 | 4.576 | 53.879 | 22.585 |
| 437 | CD2 | TRP | 718 | 4.777 | 55.221 | 22.976 |
| 438 | HE1 | TRP | 718 | 2.967 | 53.007 | 21.526 |
| 439 | CE3 | TRP | 718 | 5.938 | 55.547 | 23.688 |
| 440 | CZ3 | TRP | 718 | 6.813 | 54.537 | 24.037 |
| 441 | CH2 | TRP | 718 | 6.583 | 53.207 | 23.640 |
| 442 | CZ2 | TRP | 718 | 5.458 | 52.858 | 22.937 |
| 443 | N | ALA | 719 | 3.593 | 57.202 | 25.646 |
| 444 | CA | ALA | 719 | 3.823 | 56.439 | 26.880 |
| 445 | HN | ALA | 719 | 4.260 | 57.897 | 25.377 |
| 446 | C | ALA | 719 | 2.603 | 56.421 | 27.797 |
| 447 | O | ALA | 719 | 2.173 | 55.353 | 28.240 |
| 448 | CB | ALA | 719 | 5.039 | 57.001 | 27.626 |
| 449 | N | LYS | 720 | 2.056 | 57.602 | 28.074 |
| 450 | CA | LYS | 720 | 0.877 | 57.749 | 28.915 |
| 451 | HN | LYS | 720 | 2.473 | 58.425 | 27.688 |
| 452 | C | LYS | 720 | -0.310 | 56.892 | 28.435 |
| 453 | O | LYS | 720 | -1.087 | 56.394 | 29.246 |
| 454 | CB | LYS | 720 | 0.486 | 59.228 | 28.972 |
| 455 | CG | LYS | 720 | 1.490 | 60.104 | 29.739 |
| 456 | CD | LYS | 720 | 1.324 | 61.613 | 29.489 |
| 457 | CE | LYS | 720 | -0.138 | 62.049 | 29.374 |
| 458 | NZ | LYS | 720 | -0.392 | 63.445 | 29.911 |
| 459 | HZ1 | LYS | 720 | 0.444 | 64.028 | 29.762 |
| 460 | HZ2 | LYS | 720 | -1.194 | 63.862 | 29.418 |
| 461 | HZ3 | LYS | 720 | -0.593 | 63.395 | 30.889 |
| 462 | N | ALA | 721 | -0.447 | 56.695 | 27.130 |
| 463 | CA | ALA | 721 | -1.556 | 55.887 | 26.617 |
| 464 | HN | ALA | 721 | 0.210 | 57.099 | 26.494 |
| 465 | C | ALA | 721 | -1.321 | 54.381 | 26.706 |
| 466 | O | ALA | 721 | -2.236 | 53.584 | 26.468 |
| 467 | CB | ALA | 721 | -1.864 | 56.274 | 25.170 |
| 468 | N | LEU | 722 | -0.098 | 53.982 | 27.036 |
| 469 | CA | LEU | 722 | 0.200 | 52.564 | 27.138 |
| 470 | HN | LEU | 722 | 0.617 | 54.658 | 27.214 |
| 471 | C | LEU | 722 | -0.492 | 51.986 | 28.360 |
| 472 | O | LEU | 722 | -0.279 | 52.425 | 29.492 |
| 473 | CB | LEU | 722 | 1.737 | 52.336 | 27.210 |
| 474 | CG | LEU | 722 | 2.576 | 52.603 | 25.931 |
| 475 | CD1 | LEU | 722 | 4.062 | 52.733 | 26.293 |
| 476 | CD2 | LEU | 722 | 2.397 | 51.510 | 24.865 |
| 477 | N | PRO | 723 | -1.364 | 51.004 | 28.133 |
| 478 | CA | PRO | 723 | -2.117 | 50.324 | 29.181 |
| 479 | CD | PRO | 723 | -1.813 | 50.558 | 26.805 |
| 480 | C | PRO | 723 | -1.207 | 49.971 | 30.340 |
| 481 | O | PRO | 723 | -0.257 | 49.205 | 30.179 |
| 482 | CB | PRO | 723 | -2.635 | 49.093 | 28.460 |
| 483 | CG | PRO | 723 | -2.975 | 49.632 | 27.153 |
| 484 | N | GLY | 724 | -1.488 | 50.541 | 31.504 |
| 485 | CA | GLY | 724 | -0.686 | 50.263 | 32.676 |
| 486 | HN | GLY | 724 | -2.263 | 51.169 | 31.573 |
| 487 | C | GLY | 724 | 0.523 | 51.152 | 32.913 |

| | | | | | | |
|---|---|---|---|---|---|---|
| 488 | O | GLY | 724 | 1.221 | 50.970 | 33.900 |
| 489 | N | PHE | 725 | 0.796 | 52.120 | 32.039 |
| 490 | CA | PHE | 725 | 1.967 | 52.963 | 32.256 |
| 491 | HN | PHE | 725 | 0.206 | 52.267 | 31.245 |
| 492 | C | PHE | 725 | 1.713 | 54.100 | 33.242 |
| 493 | O | PHE | 725 | 2.584 | 54.419 | 34.053 |
| 494 | CB | PHE | 725 | 2.474 | 53.554 | 30.937 |
| 495 | CG | PHE | 725 | 3.713 | 54.394 | 31.096 |
| 496 | CD1 | PHE | 725 | 4.959 | 53.797 | 31.287 |
| 497 | CE1 | PHE | 725 | 6.098 | 54.575 | 31.517 |
| 498 | CZ | PHE | 725 | 5.991 | 55.961 | 31.555 |
| 499 | CE2 | PHE | 725 | 4.751 | 56.569 | 31.359 |
| 500 | CD2 | PHE | 725 | 3.622 | 55.785 | 31.127 |
| 501 | N | ARG | 726 | 0.528 | 54.705 | 33.164 |
| 502 | CA | ARG | 726 | 0.150 | 55.833 | 34.029 |
| 503 | HN | ARG | 726 | -0.133 | 54.378 | 32.488 |
| 504 | C | ARG | 726 | 0.050 | 55.468 | 35.498 |
| 505 | O | ARG | 726 | 0.346 | 56.277 | 36.381 |
| 506 | CB | ARG | 726 | -1.197 | 56.436 | 33.600 |
| 507 | CG | ARG | 726 | -1.155 | 57.242 | 32.317 |
| 508 | CD | ARG | 726 | -2.138 | 58.410 | 32.336 |
| 509 | NE | ARG | 726 | -1.902 | 59.315 | 33.460 |
| 510 | CZ | ARG | 726 | -0.717 | 59.828 | 33.797 |
| 511 | NH1 | ARG | 726 | 0.438 | 59.673 | 33.109 |
| 512 | 1HH1 | ARG | 726 | 1.279 | 60.106 | 33.442 |
| 513 | 2HH1 | ARG | 726 | 0.452 | 59.128 | 32.276 |
| 514 | NH2 | ARG | 726 | -0.677 | 60.598 | 34.918 |
| 515 | 1HH2 | ARG | 726 | 0.189 | 61.015 | 35.197 |
| 516 | 2HH2 | ARG | 726 | -1.510 | 60.744 | 35.449 |
| 517 | HE | ARG | 726 | -2.690 | 59.569 | 34.020 |
| 518 | N | ASN | 727 | -0.380 | 54.242 | 35.752 |
| 519 | CA | ASN | 727 | -0.544 | 53.777 | 37.110 |
| 520 | HN | ASN | 727 | -0.595 | 53.628 | 34.993 |
| 521 | C | ASN | 727 | 0.765 | 53.516 | 37.822 |
| 522 | O | ASN | 727 | 0.778 | 53.044 | 38.950 |
| 523 | CB | ASN | 727 | -1.436 | 52.541 | 37.106 |
| 524 | CG | ASN | 727 | -2.805 | 52.837 | 36.517 |
| 525 | OD1 | ASN | 727 | -3.261 | 52.166 | 35.586 |
| 526 | ND2 | ASN | 727 | -3.556 | 53.863 | 37.133 |
| 527 | 1HD2 | ASN | 727 | -4.491 | 54.048 | 36.802 |
| 528 | 2HD2 | ASN | 727 | -3.175 | 54.391 | 37.903 |
| 529 | N | LEU | 728 | 1.882 | 53.801 | 37.176 |
| 530 | CA | LEU | 728 | 3.131 | 53.615 | 37.881 |
| 531 | HN | LEU | 728 | 1.864 | 54.134 | 36.233 |
| 532 | C | LEU | 728 | 3.372 | 54.966 | 38.511 |
| 533 | O | LEU | 728 | 2.730 | 55.960 | 38.163 |
| 534 | CB | LEU | 728 | 4.285 | 53.273 | 36.943 |
| 535 | CG | LEU | 728 | 4.343 | 51.909 | 36.258 |
| 536 | CD1 | LEU | 728 | 4.962 | 52.123 | 34.901 |
| 537 | CD2 | LEU | 728 | 5.151 | 50.896 | 37.058 |
| 538 | N | HIS | 729 | 4.308 | 55.000 | 39.441 |
| 539 | CA | HIS | 729 | 4.627 | 56.232 | 40.110 |
| 540 | HN | HIS | 729 | 4.799 | 54.162 | 39.683 |
| 541 | C | HIS | 729 | 5.054 | 57.283 | 39.094 |
| 542 | O | HIS | 729 | 5.887 | 57.008 | 38.239 |

| | | | | | | |
|---|---|---|---|---|---|---|
| 543 | CB | HIS | 729 | 5.742 | 55.981 | 41.108 |
| 544 | CG | HIS | 729 | 5.813 | 57.008 | 42.187 |
| 545 | ND1 | HIS | 729 | 6.381 | 58.249 | 42.000 |
| 546 | CE1 | HIS | 729 | 6.253 | 58.959 | 43.105 |
| 547 | NE2 | HIS | 729 | 5.620 | 58.221 | 44.003 |
| 548 | CD2 | HIS | 729 | 5.333 | 56.999 | 43.455 |
| 549 | HE2 | HIS | 729 | 5.389 | 58.510 | 44.932 |
| 550 | N | VAL | 730 | 4.481 | 58.476 | 39.192 |
| 551 | CA | VAL | 730 | 4.812 | 59.579 | 38.294 |
| 552 | HN | VAL | 730 | 3.798 | 58.625 | 39.907 |
| 553 | C | VAL | 730 | 6.313 | 59.875 | 38.306 |
| 554 | O | VAL | 730 | 6.791 | 60.726 | 37.559 |
| 555 | CB | VAL | 730 | 3.984 | 60.854 | 38.725 |
| 556 | CG1 | VAL | 730 | 3.709 | 61.905 | 37.614 |
| 557 | CG2 | VAL | 730 | 2.598 | 60.527 | 39.330 |
| 558 | N | ASP | 731 | 7.044 | 59.181 | 39.173 |
| 559 | CA | ASP | 731 | 8.487 | 59.358 | 39.290 |
| 560 | HN | ASP | 731 | 6.589 | 58.516 | 39.764 |
| 561 | C | ASP | 731 | 9.214 | 58.319 | 38.448 |
| 562 | O | ASP | 731 | 10.326 | 58.562 | 37.974 |
| 563 | CB | ASP | 731 | 8.915 | 59.232 | 40.757 |
| 564 | CG | ASP | 731 | 9.276 | 60.572 | 41.382 |
| 565 | OD1 | ASP | 731 | 8.645 | 61.590 | 41.021 |
| 566 | OD2 | ASP | 731 | 10.184 | 60.599 | 42.245 |
| 567 | HD2 | ASP | 731 | 10.306 | 61.486 | 42.548 |
| 568 | N | ASP | 732 | 8.597 | 57.155 | 38.283 |
| 569 | CA | ASP | 732 | 9.174 | 56.089 | 37.469 |
| 570 | HN | ASP | 732 | 7.714 | 57.004 | 38.728 |
| 571 | C | ASP | 732 | 8.833 | 56.439 | 36.039 |
| 572 | O | ASP | 732 | 9.643 | 56.265 | 35.127 |
| 573 | CB | ASP | 732 | 8.558 | 54.740 | 37.817 |
| 574 | CG | ASP | 732 | 8.720 | 54.394 | 39.271 |
| 575 | OD1 | ASP | 732 | 9.723 | 54.839 | 39.871 |
| 576 | OD2 | ASP | 732 | 7.853 | 53.670 | 39.809 |
| 577 | HD2 | ASP | 732 | 8.076 | 53.532 | 40.717 |
| 578 | N | GLN | 733 | 7.603 | 56.922 | 35.872 |
| 579 | CA | GLN | 733 | 7.091 | 57.359 | 34.588 |
| 580 | HN | GLN | 733 | 7.005 | 56.988 | 36.671 |
| 581 | C | GLN | 733 | 8.131 | 58.302 | 34.013 |
| 582 | O | GLN | 733 | 8.317 | 58.385 | 32.802 |
| 583 | CB | GLN | 733 | 5.756 | 58.103 | 34.767 |
| 584 | CG | GLN | 733 | 4.525 | 57.204 | 34.669 |
| 585 | CD | GLN | 733 | 3.203 | 57.950 | 34.838 |
| 586 | OE1 | GLN | 733 | 2.880 | 58.872 | 34.083 |
| 587 | NE2 | GLN | 733 | 2.575 | 57.795 | 36.072 |
| 588 | 1HE2 | GLN | 733 | 1.680 | 58.234 | 36.220 |
| 589 | 2HE2 | GLN | 733 | 2.839 | 57.042 | 36.695 |
| 590 | N | MET | 734 | 8.829 | 59.003 | 34.899 |
| 591 | CA | MET | 734 | 9.852 | 59.943 | 34.473 |
| 592 | HN | MET | 734 | 8.647 | 58.882 | 35.875 |
| 593 | C | MET | 734 | 11.220 | 59.333 | 34.340 |
| 594 | O | MET | 734 | 12.079 | 59.902 | 33.666 |
| 595 | CB | MET | 734 | 9.951 | 61.118 | 35.433 |
| 596 | CG | MET | 734 | 9.164 | 62.319 | 34.998 |
| 597 | SD | MET | 734 | 9.654 | 63.746 | 35.952 |

| | | | | | | |
|---|---|---|---|---|---|---|
| 598 | CE | MET | 734 | 8.455 | 63.667 | 37.244 |
| 599 | N | ALA | 735 | 11.455 | 58.199 | 34.992 |
| 600 | CA | ALA | 735 | 12.771 | 57.598 | 34.869 |
| 601 | HN | ALA | 735 | 10.745 | 57.772 | 35.552 |
| 602 | C | ALA | 735 | 12.803 | 56.821 | 33.566 |
| 603 | O | ALA | 735 | 13.748 | 56.931 | 32.777 |
| 604 | CB | ALA | 735 | 13.083 | 56.721 | 36.094 |
| 605 | N | VAL | 736 | 11.744 | 56.053 | 33.341 |
| 606 | CA | VAL | 736 | 11.638 | 55.250 | 32.143 |
| 607 | HN | VAL | 736 | 11.004 | 56.030 | 34.013 |
| 608 | C | VAL | 736 | 11.833 | 56.098 | 30.863 |
| 609 | O | VAL | 736 | 12.645 | 55.741 | 30.008 |
| 610 | CB | VAL | 736 | 10.251 | 54.493 | 32.110 |
| 611 | CG1 | VAL | 736 | 10.007 | 53.452 | 33.238 |
| 612 | CG2 | VAL | 736 | 9.022 | 55.434 | 32.150 |
| 613 | N | ILE | 737 | 11.142 | 57.233 | 30.745 |
| 614 | CA | ILE | 737 | 11.297 | 58.090 | 29.570 |
| 615 | HN | ILE | 737 | 10.509 | 57.502 | 31.470 |
| 616 | C | ILE | 737 | 12.635 | 58.751 | 29.471 |
| 617 | O | ILE | 737 | 13.027 | 59.243 | 28.416 |
| 618 | CB | ILE | 737 | 10.118 | 59.146 | 29.580 |
| 619 | CG1 | ILE | 737 | 8.725 | 58.571 | 29.167 |
| 620 | CG2 | ILE | 737 | 10.413 | 60.390 | 28.685 |
| 621 | CD1 | ILE | 737 | 7.530 | 59.536 | 29.307 |
| 622 | N | GLN | 738 | 13.337 | 58.801 | 30.584 |
| 623 | CA | GLN | 738 | 14.626 | 59.444 | 30.567 |
| 624 | HN | GLN | 738 | 12.981 | 58.399 | 31.427 |
| 625 | C | GLN | 738 | 15.719 | 58.459 | 30.267 |
| 626 | O | GLN | 738 | 16.740 | 58.801 | 29.672 |
| 627 | CB | GLN | 738 | 14.864 | 60.136 | 31.890 |
| 628 | CG | GLN | 738 | 14.349 | 61.550 | 31.885 |
| 629 | CD | GLN | 738 | 14.079 | 62.051 | 33.272 |
| 630 | OE1 | GLN | 738 | 14.762 | 61.667 | 34.231 |
| 631 | NE2 | GLN | 738 | 12.982 | 62.863 | 33.554 |
| 632 | 1HE2 | GLN | 738 | 12.838 | 63.166 | 34.506 |
| 633 | 2HE2 | GLN | 738 | 12.311 | 63.088 | 32.846 |
| 634 | N | TYR | 739 | 15.499 | 57.221 | 30.677 |
| 635 | CA | TYR | 739 | 16.473 | 56.184 | 30.422 |
| 636 | HN | TYR | 739 | 14.654 | 57.002 | 31.165 |
| 637 | C | TYR | 739 | 16.332 | 55.637 | 29.012 |
| 638 | O | TYR | 739 | 17.282 | 55.096 | 28.463 |
| 639 | CB | TYR | 739 | 16.270 | 55.032 | 31.377 |
| 640 | CG | TYR | 739 | 16.890 | 55.184 | 32.734 |
| 641 | CD1 | TYR | 739 | 16.859 | 56.394 | 33.424 |
| 642 | CE1 | TYR | 739 | 17.360 | 56.485 | 34.721 |
| 643 | CZ | TYR | 739 | 17.890 | 55.346 | 35.321 |
| 644 | OH | TYR | 739 | 18.388 | 55.465 | 36.601 |
| 645 | HH | TYR | 739 | 19.129 | 54.856 | 36.698 |
| 646 | CE2 | TYR | 739 | 17.929 | 54.150 | 34.650 |
| 647 | CD2 | TYR | 739 | 17.437 | 54.076 | 33.369 |
| 648 | N | SER | 740 | 15.157 | 55.784 | 28.406 |
| 649 | CA | SER | 740 | 14.983 | 55.203 | 27.088 |
| 650 | HN | SER | 740 | 14.411 | 56.282 | 28.849 |
| 651 | C | SER | 740 | 14.524 | 56.053 | 25.911 |
| 652 | O | SER | 740 | 14.159 | 55.506 | 24.878 |

| | | | | | | |
|---|---|---|---|---|---|---|
| 653 | CB | SER | 740 | 14.066 | 53.978 | 27.206 |
| 654 | OG | SER | 740 | 12.749 | 54.286 | 27.635 |
| 655 | HG | SER | 740 | 12.366 | 53.459 | 27.955 |
| 656 | N | TRP | 741 | 14.519 | 57.465 | 26.099 |
| 657 | CA | TRP | 741 | 14.133 | 58.264 | 24.924 |
| 658 | HN | TRP | 741 | 14.497 | 57.891 | 27.017 |
| 659 | C | TRP | 741 | 15.078 | 57.774 | 23.764 |
| 660 | O | TRP | 741 | 14.533 | 57.416 | 22.734 |
| 661 | CB | TRP | 741 | 14.133 | 59.797 | 25.069 |
| 662 | CG | TRP | 741 | 15.531 | 60.335 | 24.953 |
| 663 | CD1 | TRP | 741 | 16.564 | 60.105 | 25.810 |
| 664 | NE1 | TRP | 741 | 17.782 | 60.643 | 25.364 |
| 665 | CE2 | TRP | 741 | 17.427 | 61.323 | 24.191 |
| 666 | CD2 | TRP | 741 | 16.085 | 61.156 | 23.847 |
| 667 | HE1 | TRP | 741 | 18.695 | 60.585 | 25.806 |
| 668 | CE3 | TRP | 741 | 15.531 | 61.689 | 22.656 |
| 669 | CZ3 | TRP | 741 | 16.425 | 62.386 | 21.792 |
| 670 | CH2 | TRP | 741 | 17.785 | 62.601 | 22.177 |
| 671 | CZ2 | TRP | 741 | 18.294 | 62.098 | 23.404 |
| 672 | N | MET | 742 | 16.511 | 57.700 | 23.949 |
| 673 | CA | MET | 742 | 17.460 | 57.375 | 22.864 |
| 674 | HN | MET | 742 | 16.918 | 58.325 | 24.642 |
| 675 | C | MET | 742 | 17.074 | 56.103 | 22.055 |
| 676 | O | MET | 742 | 17.258 | 56.121 | 20.863 |
| 677 | CB | MET | 742 | 18.909 | 57.247 | 23.380 |
| 678 | CG | MET | 742 | 19.989 | 56.973 | 22.306 |
| 679 | SD | MET | 742 | 20.191 | 58.185 | 20.942 |
| 680 | CE | MET | 742 | 19.820 | 59.767 | 21.764 |
| 681 | N | GLY | 743 | 16.525 | 54.998 | 22.724 |
| 682 | CA | GLY | 743 | 16.219 | 53.640 | 22.332 |
| 683 | HN | GLY | 743 | 16.635 | 55.206 | 23.696 |
| 684 | C | GLY | 743 | 14.879 | 53.727 | 21.599 |
| 685 | O | GLY | 743 | 14.703 | 53.142 | 20.535 |
| 686 | N | LEU | 744 | 13.800 | 54.496 | 22.119 |
| 687 | CA | LEU | 744 | 12.465 | 54.599 | 21.489 |
| 688 | HN | LEU | 744 | 13.971 | 55.061 | 22.943 |
| 689 | C | LEU | 744 | 12.576 | 55.382 | 20.155 |
| 690 | O | LEU | 744 | 12.106 | 54.862 | 19.151 |
| 691 | CB | LEU | 744 | 11.443 | 55.298 | 22.402 |
| 692 | CG | LEU | 744 | 11.169 | 54.461 | 23.672 |
| 693 | CD1 | LEU | 744 | 10.761 | 55.334 | 24.862 |
| 694 | CD2 | LEU | 744 | 10.137 | 53.369 | 23.354 |
| 695 | N | MET | 745 | 13.215 | 56.673 | 20.210 |
| 696 | CA | MET | 745 | 13.794 | 57.402 | 19.077 |
| 697 | HN | MET | 745 | 13.550 | 56.997 | 21.124 |
| 698 | C | MET | 745 | 14.432 | 56.310 | 18.190 |
| 699 | O | MET | 745 | 13.896 | 55.988 | 17.139 |
| 700 | CB | MET | 745 | 14.920 | 58.444 | 19.353 |
| 701 | CG | MET | 745 | 14.584 | 59.758 | 20.055 |
| 702 | SD | MET | 745 | 13.271 | 60.680 | 19.205 |
| 703 | CE | MET | 745 | 11.881 | 60.003 | 20.165 |
| 704 | N | VAL | 746 | 15.687 | 55.784 | 18.607 |
| 705 | CA | VAL | 746 | 16.555 | 55.323 | 17.526 |
| 706 | HN | VAL | 746 | 16.146 | 56.021 | 19.498 |
| 707 | C | VAL | 746 | 15.868 | 54.054 | 16.896 |

| | | | | | | |
|---|---|---|---|---|---|---|
| 708 | O | VAL | 746 | 15.708 | 54.010 | 15.695 |
| 709 | CB | VAL | 746 | 18.020 | 55.201 | 18.011 |
| 710 | CG1 | VAL | 746 | 18.940 | 54.565 | 16.974 |
| 711 | CG2 | VAL | 746 | 18.652 | 56.574 | 18.389 |
| 712 | N | PHE | 747 | 15.451 | 52.984 | 17.734 |
| 713 | CA | PHE | 747 | 14.928 | 51.646 | 17.382 |
| 714 | HN | PHE | 747 | 15.517 | 53.130 | 18.736 |
| 715 | C | PHE | 747 | 14.070 | 51.788 | 16.122 |
| 716 | O | PHE | 747 | 14.382 | 51.106 | 15.168 |
| 717 | CB | PHE | 747 | 14.101 | 51.009 | 18.516 |
| 718 | CG | PHE | 747 | 13.615 | 49.602 | 18.246 |
| 719 | CD1 | PHE | 747 | 14.545 | 48.503 | 18.348 |
| 720 | CE1 | PHE | 747 | 14.085 | 47.149 | 18.314 |
| 721 | CZ | PHE | 747 | 12.691 | 46.873 | 18.137 |
| 722 | CE2 | PHE | 747 | 11.744 | 47.952 | 18.031 |
| 723 | CD2 | PHE | 747 | 12.206 | 49.310 | 18.105 |
| 724 | N | ALA | 748 | 12.988 | 52.734 | 16.197 |
| 725 | CA | ALA | 748 | 11.929 | 53.166 | 15.258 |
| 726 | HN | ALA | 748 | 12.980 | 53.238 | 17.081 |
| 727 | C | ALA | 748 | 12.452 | 53.970 | 14.009 |
| 728 | O | ALA | 748 | 11.827 | 53.896 | 12.962 |
| 729 | CB | ALA | 748 | 10.866 | 54.042 | 15.955 |
| 730 | N | MET | 749 | 13.577 | 54.829 | 14.173 |
| 731 | CA | MET | 749 | 14.460 | 55.045 | 13.006 |
| 732 | HN | MET | 749 | 14.041 | 54.673 | 15.058 |
| 733 | C | MET | 749 | 14.556 | 53.699 | 12.250 |
| 734 | O | MET | 749 | 14.153 | 53.723 | 11.093 |
| 735 | CB | MET | 749 | 15.938 | 55.490 | 13.154 |
| 736 | CG | MET | 749 | 16.676 | 55.496 | 11.809 |
| 737 | SD | MET | 749 | 15.908 | 56.663 | 10.662 |
| 738 | CE | MET | 749 | 16.406 | 58.131 | 11.579 |
| 739 | N | GLY | 750 | 15.125 | 52.555 | 12.918 |
| 740 | CA | GLY | 750 | 15.233 | 51.315 | 12.206 |
| 741 | HN | GLY | 750 | 15.461 | 52.500 | 13.881 |
| 742 | C | GLY | 750 | 13.913 | 51.116 | 11.449 |
| 743 | O | GLY | 750 | 13.991 | 50.745 | 10.291 |
| 744 | N | TRP | 751 | 12.676 | 51.353 | 12.134 |
| 745 | CA | TRP | 751 | 11.460 | 50.940 | 11.462 |
| 746 | HN | TRP | 751 | 12.636 | 51.565 | 13.110 |
| 747 | C | TRP | 751 | 11.085 | 51.734 | 10.187 |
| 748 | O | TRP | 751 | 10.868 | 51.159 | 9.134 |
| 749 | CB | TRP | 751 | 10.334 | 50.995 | 12.476 |
| 750 | CG | TRP | 751 | 9.056 | 50.606 | 11.930 |
| 751 | CD1 | TRP | 751 | 8.036 | 51.435 | 11.565 |
| 752 | NE1 | TRP | 751 | 6.971 | 50.695 | 11.112 |
| 753 | CE2 | TRP | 751 | 7.298 | 49.364 | 11.173 |
| 754 | CD2 | TRP | 751 | 8.609 | 49.275 | 11.699 |
| 755 | HE1 | TRP | 751 | 6.100 | 51.066 | 10.791 |
| 756 | CE3 | TRP | 751 | 9.194 | 48.008 | 11.852 |
| 757 | CZ3 | TRP | 751 | 8.448 | 46.879 | 11.513 |
| 758 | CH2 | TRP | 751 | 7.139 | 47.004 | 11.001 |
| 759 | CZ2 | TRP | 751 | 6.547 | 48.234 | 10.834 |
| 760 | N | ARG | 752 | 10.828 | 53.134 | 10.236 |
| 761 | CA | ARG | 752 | 10.481 | 53.916 | 9.023 |
| 762 | HN | ARG | 752 | 11.064 | 53.682 | 11.044 |

| | | | | | | |
|---|---|---|---|---|---|---|
| 763 | C | ARG | 752 | 11.467 | 53.500 | 7.878 |
| 764 | O | ARG | 752 | 11.091 | 53.384 | 6.727 |
| 765 | CB | ARG | 752 | 10.508 | 55.465 | 9.244 |
| 766 | CG | ARG | 752 | 9.234 | 55.992 | 9.981 |
| 767 | CD | ARG | 752 | 9.081 | 57.491 | 10.410 |
| 768 | NE | ARG | 752 | 10.218 | 58.276 | 10.932 |
| 769 | CZ | ARG | 752 | 11.005 | 57.728 | 11.925 |
| 770 | NH1 | ARG | 752 | 10.676 | 56.604 | 12.600 |
| 771 | 1HH1 | ARG | 752 | 11.297 | 56.253 | 13.312 |
| 772 | 2HH1 | ARG | 752 | 9.810 | 56.136 | 12.400 |
| 773 | NH2 | ARG | 752 | 12.193 | 58.225 | 12.333 |
| 774 | 1HH2 | ARG | 752 | 12.725 | 57.805 | 13.083 |
| 775 | 2HH2 | ARG | 752 | 12.508 | 59.058 | 11.877 |
| 776 | HE | ARG | 752 | 10.438 | 59.158 | 10.490 |
| 777 | N | SER | 753 | 12.832 | 53.352 | 8.234 |
| 778 | CA | SER | 753 | 13.858 | 53.113 | 7.230 |
| 779 | HN | SER | 753 | 13.094 | 53.305 | 9.198 |
| 780 | C | SER | 753 | 13.671 | 51.814 | 6.491 |
| 781 | O | SER | 753 | 13.787 | 51.732 | 5.269 |
| 782 | CB | SER | 753 | 15.220 | 53.082 | 7.901 |
| 783 | OG | SER | 753 | 15.598 | 54.311 | 8.521 |
| 784 | HG | SER | 753 | 16.377 | 54.104 | 9.055 |
| 785 | N | PHE | 754 | 13.406 | 50.789 | 7.273 |
| 786 | CA | PHE | 754 | 13.217 | 49.465 | 6.764 |
| 787 | HN | PHE | 754 | 13.334 | 50.941 | 8.259 |
| 788 | C | PHE | 754 | 11.985 | 49.344 | 5.896 |
| 789 | O | PHE | 754 | 11.951 | 48.557 | 4.958 |
| 790 | CB | PHE | 754 | 13.080 | 48.488 | 7.971 |
| 791 | CG | PHE | 754 | 12.144 | 47.285 | 7.775 |
| 792 | CD1 | PHE | 754 | 12.430 | 46.341 | 6.782 |
| 793 | CE1 | PHE | 754 | 11.651 | 45.196 | 6.657 |
| 794 | CZ | PHE | 754 | 10.592 | 44.977 | 7.535 |
| 795 | CE2 | PHE | 754 | 10.309 | 45.908 | 8.534 |
| 796 | CD2 | PHE | 754 | 11.081 | 47.060 | 8.653 |
| 797 | N | THR | 755 | 10.970 | 50.138 | 6.171 |
| 798 | CA | THR | 755 | 9.755 | 49.992 | 5.397 |
| 799 | HN | THR | 755 | 11.036 | 50.823 | 6.897 |
| 800 | C | THR | 755 | 9.638 | 50.815 | 4.144 |
| 801 | O | THR | 755 | 8.932 | 50.439 | 3.209 |
| 802 | CB | THR | 755 | 8.553 | 50.275 | 6.360 |
| 803 | OG1 | THR | 755 | 8.606 | 49.415 | 7.490 |
| 804 | HG1 | THR | 755 | 7.888 | 49.688 | 8.068 |
| 805 | CG2 | THR | 755 | 7.141 | 50.057 | 5.774 |
| 806 | N | ASN | 756 | 10.347 | 51.932 | 4.147 |
| 807 | CA | ASN | 756 | 10.323 | 52.902 | 3.073 |
| 808 | HN | ASN | 756 | 10.933 | 52.116 | 4.936 |
| 809 | C | ASN | 756 | 11.405 | 52.732 | 2.021 |
| 810 | O | ASN | 756 | 11.350 | 53.354 | 0.962 |
| 811 | CB | ASN | 756 | 10.413 | 54.308 | 3.734 |
| 812 | CG | ASN | 756 | 9.814 | 55.482 | 2.952 |
| 813 | OD1 | ASN | 756 | 10.054 | 56.646 | 3.238 |
| 814 | ND2 | ASN | 756 | 9.001 | 55.229 | 1.962 |
| 815 | 1HD2 | ASN | 756 | 8.541 | 56.073 | 1.610 |
| 816 | 2HD2 | ASN | 756 | 8.750 | 54.253 | 1.807 |
| 817 | N | VAL | 757 | 12.404 | 51.906 | 2.311 |

| | | | | | | |
|---|---|---|---|---|---|---|
| 818 | CA | VAL | 757 | 13.501 | 51.711 | 1.378 |
| 819 | HN | VAL | 757 | 12.401 | 51.413 | 3.181 |
| 820 | C | VAL | 757 | 14.375 | 50.537 | 1.784 |
| 821 | O | VAL | 757 | 15.610 | 50.602 | 1.730 |
| 822 | CB | VAL | 757 | 14.355 | 53.036 | 1.271 |
| 823 | CG1 | VAL | 757 | 14.528 | 53.853 | 2.581 |
| 824 | CG2 | VAL | 757 | 15.790 | 52.817 | 0.734 |
| 825 | N | ASN | 758 | 13.702 | 49.482 | 2.233 |
| 826 | CA | ASN | 758 | 14.314 | 48.218 | 2.620 |
| 827 | HN | ASN | 758 | 12.708 | 49.561 | 2.310 |
| 828 | C | ASN | 758 | 15.612 | 48.239 | 3.458 |
| 829 | O | ASN | 758 | 16.410 | 47.298 | 3.378 |
| 830 | CB | ASN | 758 | 14.517 | 47.400 | 1.311 |
| 831 | CG | ASN | 758 | 13.894 | 47.971 | 0.033 |
| 832 | OD1 | ASN | 758 | 14.553 | 48.581 | -0.797 |
| 833 | ND2 | ASN | 758 | 12.612 | 47.821 | -0.165 |
| 834 | 1HD2 | ASN | 758 | 12.273 | 48.366 | -0.962 |
| 835 | 2HD2 | ASN | 758 | 12.076 | 47.388 | 0.588 |
| 836 | N | SER | 759 | 15.798 | 49.283 | 4.266 |
| 837 | CA | SER | 759 | 16.988 | 49.450 | 5.119 |
| 838 | HN | SER | 759 | 15.092 | 49.990 | 4.295 |
| 839 | C | SER | 759 | 18.206 | 49.933 | 4.318 |
| 840 | O | SER | 759 | 19.349 | 49.548 | 4.604 |
| 841 | CB | SER | 759 | 17.291 | 48.133 | 5.878 |
| 842 | OG | SER | 759 | 17.771 | 47.094 | 5.019 |
| 843 | HG | SER | 759 | 17.071 | 46.891 | 4.393 |
| 844 | N | ARG | 760 | 17.949 | 50.786 | 3.330 |
| 845 | CA | ARG | 760 | 18.985 | 51.346 | 2.466 |
| 846 | HN | ARG | 760 | 16.999 | 51.058 | 3.171 |
| 847 | C | ARG | 760 | 19.341 | 52.784 | 2.844 |
| 848 | O | ARG | 760 | 20.516 | 53.158 | 2.902 |
| 849 | CB | ARG | 760 | 18.480 | 51.254 | 1.000 |
| 850 | CG | ARG | 760 | 18.391 | 49.814 | 0.428 |
| 851 | CD | ARG | 760 | 19.600 | 49.453 | -0.445 |
| 852 | NE | ARG | 760 | 19.406 | 48.074 | -0.963 |
| 853 | CZ | ARG | 760 | 20.247 | 47.425 | -1.757 |
| 854 | NH1 | ARG | 760 | 21.372 | 47.913 | -2.190 |
| 855 | 1HH1 | ARG | 760 | 21.553 | 48.860 | -1.855 |
| 856 | 2HH1 | ARG | 760 | 21.933 | 47.318 | -2.796 |
| 857 | NH2 | ARG | 760 | 19.923 | 46.235 | -2.118 |
| 858 | 1HH2 | ARG | 760 | 19.025 | 45.948 | -1.724 |
| 859 | 2HH2 | ARG | 760 | 20.563 | 45.732 | -2.730 |
| 860 | HE | ARG | 760 | 18.572 | 47.596 | -0.690 |
| 861 | N | MET | 761 | 18.293 | 53.567 | 3.089 |
| 862 | CA | MET | 761 | 18.370 | 54.983 | 3.462 |
| 863 | HN | MET | 761 | 17.384 | 53.158 | 3.015 |
| 864 | C | MET | 761 | 17.939 | 55.180 | 4.938 |
| 865 | O | MET | 761 | 17.228 | 54.341 | 5.487 |
| 866 | CB | MET | 761 | 17.443 | 55.779 | 2.503 |
| 867 | CG | MET | 761 | 18.096 | 56.281 | 1.198 |
| 868 | SD | MET | 761 | 19.267 | 55.055 | 0.591 |
| 869 | CE | MET | 761 | 20.100 | 56.069 | -0.638 |
| 870 | N | LEU | 762 | 18.646 | 56.133 | 5.719 |
| 871 | CA | LEU | 762 | 18.008 | 56.502 | 6.977 |
| 872 | HN | LEU | 762 | 19.291 | 56.801 | 5.349 |

| | | | | | | |
|---|---|---|---|---|---|---|
| 873 | C | LEU | 762 | 16.874 | 57.403 | 6.518 |
| 874 | O | LEU | 762 | 17.022 | 58.280 | 5.682 |
| 875 | CB | LEU | 762 | 18.807 | 57.249 | 8.045 |
| 876 | CG | LEU | 762 | 19.912 | 56.413 | 8.714 |
| 877 | CD1 | LEU | 762 | 20.320 | 57.178 | 9.974 |
| 878 | CD2 | LEU | 762 | 19.487 | 54.969 | 9.048 |
| 879 | N | TYR | 763 | 15.670 | 57.231 | 7.238 |
| 880 | CA | TYR | 763 | 14.513 | 58.088 | 6.999 |
| 881 | HN | TYR | 763 | 15.561 | 56.388 | 7.766 |
| 882 | C | TYR | 763 | 13.906 | 58.809 | 8.186 |
| 883 | O | TYR | 763 | 12.846 | 58.426 | 8.682 |
| 884 | CB | TYR | 763 | 13.390 | 57.253 | 6.318 |
| 885 | CG | TYR | 763 | 13.796 | 56.416 | 5.099 |
| 886 | CD1 | TYR | 763 | 13.972 | 57.016 | 3.849 |
| 887 | CD2 | TYR | 763 | 14.003 | 55.040 | 5.240 |
| 888 | CE1 | TYR | 763 | 14.356 | 56.247 | 2.753 |
| 889 | CE2 | TYR | 763 | 14.386 | 54.274 | 4.144 |
| 890 | CZ | TYR | 763 | 14.563 | 54.878 | 2.901 |
| 891 | OH | TYR | 763 | 14.942 | 54.127 | 1.825 |
| 892 | HH | TYR | 763 | 15.040 | 53.217 | 2.109 |
| 893 | N | PHE | 764 | 14.785 | 59.734 | 8.835 |
| 894 | CA | PHE | 764 | 14.442 | 60.479 | 10.083 |
| 895 | HN | PHE | 764 | 15.772 | 59.742 | 8.578 |
| 896 | C | PHE | 764 | 12.936 | 60.840 | 10.052 |
| 897 | O | PHE | 764 | 12.200 | 60.760 | 11.025 |
| 898 | CB | PHE | 764 | 15.272 | 61.780 | 10.272 |
| 899 | CG | PHE | 764 | 16.672 | 61.362 | 10.642 |
| 900 | CD1 | PHE | 764 | 17.646 | 60.998 | 9.635 |
| 901 | CE1 | PHE | 764 | 18.897 | 60.394 | 10.028 |
| 902 | CZ | PHE | 764 | 19.179 | 60.161 | 11.424 |
| 903 | CE2 | PHE | 764 | 18.228 | 60.565 | 12.429 |
| 904 | CD2 | PHE | 764 | 16.976 | 61.155 | 12.037 |
| 905 | N | ALA | 765 | 12.573 | 61.312 | 8.769 |
| 906 | CA | ALA | 765 | 11.243 | 61.719 | 8.333 |
| 907 | HN | ALA | 765 | 13.221 | 61.061 | 8.011 |
| 908 | C | ALA | 765 | 11.117 | 61.299 | 6.822 |
| 909 | O | ALA | 765 | 12.084 | 61.094 | 6.089 |
| 910 | CB | ALA | 765 | 11.042 | 63.241 | 8.506 |
| 911 | N | PRO | 766 | 9.756 | 61.236 | 6.359 |
| 912 | CA | PRO | 766 | 9.578 | 61.215 | 4.895 |
| 913 | CD | PRO | 766 | 8.902 | 60.205 | 6.984 |
| 914 | C | PRO | 766 | 9.892 | 62.462 | 4.031 |
| 915 | O | PRO | 766 | 10.011 | 62.338 | 2.812 |
| 916 | CB | PRO | 766 | 8.151 | 60.692 | 4.748 |
| 917 | CG | PRO | 766 | 8.154 | 59.579 | 5.772 |
| 918 | N | ASP | 767 | 10.023 | 63.635 | 4.659 |
| 919 | CA | ASP | 767 | 10.402 | 64.883 | 3.987 |
| 920 | HN | ASP | 767 | 9.853 | 63.663 | 5.644 |
| 921 | C | ASP | 767 | 11.908 | 65.110 | 4.248 |
| 922 | O | ASP | 767 | 12.416 | 66.219 | 4.071 |
| 923 | CB | ASP | 767 | 9.546 | 66.079 | 4.513 |
| 924 | CG | ASP | 767 | 10.320 | 67.074 | 5.442 |
| 925 | OD1 | ASP | 767 | 11.386 | 66.743 | 6.007 |
| 926 | OD2 | ASP | 767 | 9.830 | 68.214 | 5.632 |
| 927 | HD2 | ASP | 767 | 10.394 | 68.708 | 6.207 |

| | | | | | | |
|---|---|---|---|---|---|---|
| 928 | N | LEU | 768 | 12.622 | 64.067 | 4.686 |
| 929 | CA | LEU | 768 | 14.066 | 64.183 | 4.963 |
| 930 | HN | LEU | 768 | 12.166 | 63.188 | 4.829 |
| 931 | C | LEU | 768 | 14.766 | 62.853 | 5.221 |
| 932 | O | LEU | 768 | 14.844 | 62.374 | 6.362 |
| 933 | CB | LEU | 768 | 14.356 | 65.126 | 6.149 |
| 934 | CG | LEU | 768 | 15.833 | 65.518 | 6.400 |
| 935 | CD1 | LEU | 768 | 15.922 | 66.481 | 7.567 |
| 936 | CD2 | LEU | 768 | 16.695 | 64.304 | 6.705 |
| 937 | N | VAL | 769 | 15.303 | 62.307 | 4.134 |
| 938 | CA | VAL | 769 | 16.033 | 61.049 | 4.107 |
| 939 | HN | VAL | 769 | 15.198 | 62.798 | 3.270 |
| 940 | C | VAL | 769 | 17.515 | 61.321 | 3.918 |
| 941 | O | VAL | 769 | 17.910 | 62.236 | 3.195 |
| 942 | CB | VAL | 769 | 15.470 | 60.162 | 2.927 |
| 943 | CG1 | VAL | 769 | 13.936 | 59.910 | 2.925 |
| 944 | CG2 | VAL | 769 | 15.794 | 60.713 | 1.518 |
| 945 | N | PHE | 770 | 18.501 | 60.585 | 4.675 |
| 946 | CA | PHE | 770 | 19.970 | 60.640 | 4.547 |
| 947 | HN | PHE | 770 | 18.186 | 59.784 | 5.216 |
| 948 | C | PHE | 770 | 20.261 | 59.601 | 3.429 |
| 949 | O | PHE | 770 | 20.554 | 58.427 | 3.649 |
| 950 | CB | PHE | 770 | 20.836 | 60.221 | 5.758 |
| 951 | CG | PHE | 770 | 21.233 | 61.246 | 6.795 |
| 952 | CD1 | PHE | 770 | 22.499 | 61.037 | 7.466 |
| 953 | CE1 | PHE | 770 | 22.940 | 61.897 | 8.521 |
| 954 | CZ | PHE | 770 | 22.151 | 63.037 | 8.872 |
| 955 | CE2 | PHE | 770 | 20.900 | 63.270 | 8.203 |
| 956 | CD2 | PHE | 770 | 20.416 | 62.367 | 7.193 |
| 957 | N | ASN | 771 | 20.172 | 60.171 | 2.136 |
| 958 | CA | ASN | 771 | 20.583 | 59.440 | 0.952 |
| 959 | HN | ASN | 771 | 19.831 | 61.101 | 1.999 |
| 960 | C | ASN | 771 | 22.104 | 59.406 | 0.993 |
| 961 | O | ASN | 771 | 22.740 | 59.804 | 1.982 |
| 962 | CB | ASN | 771 | 20.157 | 60.228 | -0.266 |
| 963 | CG | ASN | 771 | 20.462 | 61.686 | -0.088 |
| 964 | OD1 | ASN | 771 | 21.245 | 62.041 | 0.802 |
| 965 | ND2 | ASN | 771 | 19.834 | 62.588 | -0.969 |
| 966 | 1HD2 | ASN | 771 | 20.070 | 63.569 | -0.935 |
| 967 | 2HD2 | ASN | 771 | 19.233 | 62.251 | -1.704 |
| 968 | N | GLU | 772 | 22.663 | 58.979 | -0.126 |
| 969 | CA | GLU | 772 | 24.082 | 58.822 | -0.276 |
| 970 | HN | GLU | 772 | 22.072 | 58.756 | -0.901 |
| 971 | C | GLU | 772 | 24.616 | 60.138 | -0.412 |
| 972 | O | GLU | 772 | 25.341 | 60.550 | 0.437 |
| 973 | CB | GLU | 772 | 24.391 | 58.080 | -1.511 |
| 974 | CG | GLU | 772 | 25.666 | 57.375 | -1.442 |
| 975 | CD | GLU | 772 | 26.030 | 56.969 | -2.820 |
| 976 | OE1 | GLU | 772 | 25.501 | 55.939 | -3.297 |
| 977 | OE2 | GLU | 772 | 26.811 | 57.715 | -3.446 |
| 978 | HE2 | GLU | 772 | 26.969 | 57.356 | -4.306 |
| 979 | N | TYR | 773 | 24.351 | 60.774 | -1.523 |
| 980 | CA | TYR | 773 | 24.793 | 62.102 | -1.541 |
| 981 | HN | TYR | 773 | 23.873 | 60.349 | -2.292 |
| 982 | C | TYR | 773 | 25.042 | 62.437 | -0.030 |

| | | | | | | |
|---|---|---|---|---|---|---|
| 983 | O | TYR | 773 | 26.119 | 62.188 | 0.452 |
| 984 | CB | TYR | 773 | 23.579 | 62.947 | -2.027 |
| 985 | CG | TYR | 773 | 23.890 | 64.339 | -2.587 |
| 986 | CD1 | TYR | 773 | 22.957 | 65.375 | -2.482 |
| 987 | CD2 | TYR | 773 | 25.123 | 64.581 | -3.202 |
| 988 | CE1 | TYR | 773 | 23.258 | 66.640 | -2.982 |
| 989 | CE2 | TYR | 773 | 25.421 | 65.845 | -3.701 |
| 990 | CZ | TYR | 773 | 24.488 | 66.874 | -3.590 |
| 991 | OH | TYR | 773 | 24.780 | 68.116 | -4.077 |
| 992 | HH | TYR | 773 | 25.661 | 68.094 | -4.455 |
| 993 | N | ARG | 774 | 24.048 | 62.861 | 0.759 |
| 994 | CA | ARG | 774 | 24.284 | 63.236 | 2.187 |
| 995 | HN | ARG | 774 | 23.123 | 62.929 | 0.385 |
| 996 | C | ARG | 774 | 25.212 | 62.467 | 3.112 |
| 997 | O | ARG | 774 | 26.035 | 63.039 | 3.822 |
| 998 | CB | ARG | 774 | 22.962 | 63.385 | 2.896 |
| 999 | CG | ARG | 774 | 22.235 | 64.527 | 2.333 |
| 1000 | CD | ARG | 774 | 20.817 | 64.471 | 2.706 |
| 1001 | NE | ARG | 774 | 20.155 | 65.689 | 2.277 |
| 1002 | CZ | ARG | 774 | 18.843 | 65.836 | 2.288 |
| 1003 | NH1 | ARG | 774 | 17.944 | 64.906 | 2.689 |
| 1004 | 1HH1 | ARG | 774 | 16.968 | 65.106 | 2.663 |
| 1005 | 2HH1 | ARG | 774 | 18.268 | 64.016 | 3.011 |
| 1006 | NH2 | ARG | 774 | 18.393 | 67.046 | 1.851 |
| 1007 | 1HH2 | ARG | 774 | 17.416 | 67.245 | 1.824 |
| 1008 | 2HH2 | ARG | 774 | 19.071 | 67.726 | 1.561 |
| 1009 | HE | ARG | 774 | 20.718 | 66.451 | 1.959 |
| 1010 | N | MET | 775 | 24.911 | 61.105 | 3.376 |
| 1011 | CA | MET | 775 | 25.779 | 60.583 | 4.448 |
| 1012 | HN | MET | 775 | 24.830 | 60.517 | 2.549 |
| 1013 | C | MET | 775 | 27.305 | 60.651 | 4.013 |
| 1014 | O | MET | 775 | 28.147 | 60.293 | 4.826 |
| 1015 | CB | MET | 775 | 25.318 | 59.204 | 4.870 |
| 1016 | CG | MET | 775 | 25.815 | 58.802 | 6.270 |
| 1017 | SD | MET | 775 | 24.477 | 58.163 | 7.351 |
| 1018 | CE | MET | 775 | 25.415 | 57.094 | 8.481 |
| 1019 | N | HIS | 776 | 27.640 | 61.215 | 2.696 |
| 1020 | CA | HIS | 776 | 29.031 | 61.282 | 2.344 |
| 1021 | HN | HIS | 776 | 26.964 | 61.792 | 2.238 |
| 1022 | C | HIS | 776 | 29.746 | 62.384 | 3.101 |
| 1023 | O | HIS | 776 | 30.975 | 62.481 | 3.039 |
| 1024 | CB | HIS | 776 | 29.130 | 61.523 | 0.828 |
| 1025 | CG | HIS | 776 | 27.956 | 62.293 | 0.296 |
| 1026 | ND1 | HIS | 776 | 26.766 | 61.743 | -0.172 |
| 1027 | CE1 | HIS | 776 | 26.107 | 62.869 | -0.503 |
| 1028 | NE2 | HIS | 776 | 26.735 | 64.060 | -0.309 |
| 1029 | CD2 | HIS | 776 | 27.943 | 63.678 | 0.215 |
| 1030 | HE2 | HIS | 776 | 26.395 | 65.013 | -0.501 |
| 1031 | N | LYS | 777 | 28.943 | 63.122 | 3.884 |
| 1032 | CA | LYS | 777 | 29.320 | 64.309 | 4.668 |
| 1033 | HN | LYS | 777 | 27.987 | 62.835 | 3.941 |
| 1034 | C | LYS | 777 | 30.189 | 64.269 | 5.882 |
| 1035 | O | LYS | 777 | 29.801 | 63.817 | 6.953 |
| 1036 | CB | LYS | 777 | 28.017 | 65.109 | 4.943 |
| 1037 | CG | LYS | 777 | 27.271 | 65.533 | 3.654 |

| | | | | | | |
|---|---|---|---|---|---|---|
| 1038 | CD | LYS | 777 | 27.913 | 66.694 | 2.890 |
| 1039 | CE | LYS | 777 | 27.075 | 67.006 | 1.644 |
| 1040 | NZ | LYS | 777 | 27.690 | 68.123 | 0.906 |
| 1041 | HZ2 | LYS | 777 | 28.714 | 68.062 | 0.944 |
| 1042 | HZ1 | LYS | 777 | 27.439 | 68.097 | -0.088 |
| 1043 | HZ3 | LYS | 777 | 27.389 | 68.991 | 1.303 |
| 1044 | N | SER | 778 | 31.289 | 64.992 | 5.774 |
| 1045 | HN | SER | 778 | 31.459 | 65.526 | 4.910 |
| 1046 | CA | SER | 778 | 32.264 | 65.049 | 6.850 |
| 1047 | C | SER | 778 | 32.066 | 64.179 | 8.078 |
| 1048 | O | SER | 778 | 32.971 | 63.485 | 8.496 |
| 1049 | CB | SER | 778 | 32.289 | 66.554 | 7.216 |
| 1050 | OG | SER | 778 | 32.013 | 67.404 | 6.097 |
| 1051 | HG | SER | 778 | 32.714 | 67.269 | 5.454 |
| 1052 | N | ARG | 779 | 31.042 | 64.493 | 9.064 |
| 1053 | CA | ARG | 779 | 31.023 | 63.897 | 10.434 |
| 1054 | HN | ARG | 779 | 30.246 | 65.047 | 8.755 |
| 1055 | C | ARG | 779 | 29.590 | 63.361 | 10.800 |
| 1056 | O | ARG | 779 | 28.841 | 63.946 | 11.571 |
| 1057 | CB | ARG | 779 | 31.557 | 64.839 | 11.534 |
| 1058 | CG | ARG | 779 | 33.073 | 65.106 | 11.405 |
| 1059 | CD | ARG | 779 | 33.744 | 65.498 | 12.728 |
| 1060 | NE | ARG | 779 | 33.977 | 64.443 | 13.758 |
| 1061 | CZ | ARG | 779 | 34.629 | 64.605 | 14.960 |
| 1062 | NH1 | ARG | 779 | 35.116 | 65.817 | 15.379 |
| 1063 | 1HH1 | ARG | 779 | 34.981 | 66.593 | 14.763 |
| 1064 | 2HH1 | ARG | 779 | 35.591 | 65.928 | 16.255 |
| 1065 | NH2 | ARG | 779 | 34.793 | 63.521 | 15.765 |
| 1066 | 1HH2 | ARG | 779 | 34.408 | 62.676 | 15.376 |
| 1067 | 2HH2 | ARG | 779 | 35.262 | 63.562 | 16.652 |
| 1068 | HE | ARG | 779 | 33.656 | 63.520 | 13.542 |
| 1069 | N | MET | 780 | 29.272 | 62.128 | 10.169 |
| 1070 | CA | MET | 780 | 27.864 | 61.726 | 9.970 |
| 1071 | HN | MET | 780 | 29.848 | 61.854 | 9.387 |
| 1072 | C | MET | 780 | 27.908 | 60.242 | 9.574 |
| 1073 | O | MET | 780 | 27.272 | 59.464 | 10.278 |
| 1074 | CB | MET | 780 | 27.041 | 62.330 | 8.814 |
| 1075 | CG | MET | 780 | 26.567 | 63.776 | 8.902 |
| 1076 | SD | MET | 780 | 25.378 | 64.019 | 7.543 |
| 1077 | CE | MET | 780 | 24.600 | 65.573 | 8.095 |
| 1078 | N | TYR | 781 | 28.649 | 59.894 | 8.359 |
| 1079 | CA | TYR | 781 | 28.868 | 58.454 | 8.021 |
| 1080 | HN | TYR | 781 | 29.099 | 60.596 | 7.760 |
| 1081 | C | TYR | 781 | 28.899 | 57.569 | 9.335 |
| 1082 | O | TYR | 781 | 28.171 | 56.608 | 9.513 |
| 1083 | CB | TYR | 781 | 30.119 | 58.165 | 7.148 |
| 1084 | CG | TYR | 781 | 30.298 | 56.667 | 7.018 |
| 1085 | CD1 | TYR | 781 | 29.562 | 55.920 | 6.025 |
| 1086 | CE1 | TYR | 781 | 29.644 | 54.479 | 6.002 |
| 1087 | CZ | TYR | 781 | 30.477 | 53.764 | 6.943 |
| 1088 | OH | TYR | 781 | 30.565 | 52.391 | 6.924 |
| 1089 | HH | TYR | 781 | 29.809 | 52.055 | 6.426 |
| 1090 | CE2 | TYR | 781 | 31.236 | 54.523 | 7.898 |
| 1091 | CD2 | TYR | 781 | 31.157 | 55.955 | 7.937 |
| 1092 | N | SER | 782 | 29.890 | 58.001 | 10.221 |

| | | | | | | |
|---|---|---|---|---|---|---|
| 1093 | CA | SER | 782 | 30.342 | 57.544 | 11.482 |
| 1094 | HN | SER | 782 | 30.414 | 58.669 | 9.692 |
| 1095 | C | SER | 782 | 29.351 | 57.385 | 12.601 |
| 1096 | O | SER | 782 | 29.510 | 56.535 | 13.475 |
| 1097 | CB | SER | 782 | 31.546 | 58.422 | 11.907 |
| 1098 | OG | SER | 782 | 32.608 | 57.663 | 12.495 |
| 1099 | HG | SER | 782 | 32.266 | 57.264 | 13.300 |
| 1100 | N | GLN | 783 | 28.232 | 58.202 | 12.588 |
| 1101 | CA | GLN | 783 | 27.423 | 58.261 | 13.809 |
| 1102 | HN | GLN | 783 | 28.195 | 58.944 | 11.888 |
| 1103 | C | GLN | 783 | 26.191 | 57.347 | 13.631 |
| 1104 | O | GLN | 783 | 25.946 | 56.384 | 14.349 |
| 1105 | CB | GLN | 783 | 27.169 | 59.738 | 14.096 |
| 1106 | CG | GLN | 783 | 28.525 | 60.430 | 14.416 |
| 1107 | CD | GLN | 783 | 29.225 | 59.669 | 15.557 |
| 1108 | OE1 | GLN | 783 | 28.571 | 59.239 | 16.479 |
| 1109 | NE2 | GLN | 783 | 30.618 | 59.475 | 15.570 |
| 1110 | 1HE2 | GLN | 783 | 30.948 | 59.552 | 16.532 |
| 1111 | 2HE2 | GLN | 783 | 31.148 | 59.941 | 14.847 |
| 1112 | N | CYS | 784 | 25.446 | 57.695 | 12.493 |
| 1113 | CA | CYS | 784 | 24.205 | 57.100 | 12.023 |
| 1114 | HN | CYS | 784 | 25.686 | 58.565 | 12.035 |
| 1115 | C | CYS | 784 | 24.455 | 55.669 | 11.442 |
| 1116 | O | CYS | 784 | 23.491 | 55.056 | 10.994 |
| 1117 | CB | CYS | 784 | 23.599 | 57.996 | 10.914 |
| 1118 | SG | CYS | 784 | 22.363 | 59.176 | 11.504 |
| 1119 | HG | CYS | 784 | 22.061 | 59.709 | 10.322 |
| 1120 | N | VAL | 785 | 25.779 | 55.124 | 11.438 |
| 1121 | CA | VAL | 785 | 25.912 | 53.807 | 11.104 |
| 1122 | HN | VAL | 785 | 26.470 | 55.639 | 11.944 |
| 1123 | C | VAL | 785 | 25.261 | 52.926 | 12.098 |
| 1124 | O | VAL | 785 | 24.784 | 51.860 | 11.743 |
| 1125 | CB | VAL | 785 | 27.461 | 53.505 | 11.015 |
| 1126 | CG1 | VAL | 785 | 28.124 | 52.919 | 12.292 |
| 1127 | CG2 | VAL | 785 | 27.846 | 52.534 | 9.872 |
| 1128 | N | ARG | 786 | 25.210 | 53.364 | 13.345 |
| 1129 | CA | ARG | 786 | 24.613 | 52.485 | 14.303 |
| 1130 | HN | ARG | 786 | 25.569 | 54.261 | 13.604 |
| 1131 | C | ARG | 786 | 23.134 | 52.462 | 14.182 |
| 1132 | O | ARG | 786 | 22.488 | 51.556 | 14.682 |
| 1133 | CB | ARG | 786 | 25.058 | 52.862 | 15.743 |
| 1134 | CG | ARG | 786 | 26.537 | 52.533 | 16.077 |
| 1135 | CD | ARG | 786 | 27.404 | 53.791 | 16.214 |
| 1136 | NE | ARG | 786 | 28.793 | 53.370 | 16.529 |
| 1137 | CZ | ARG | 786 | 29.821 | 54.186 | 16.714 |
| 1138 | NH1 | ARG | 786 | 29.750 | 55.483 | 16.649 |
| 1139 | 1HH1 | ARG | 786 | 28.811 | 55.822 | 16.442 |
| 1140 | 2HH1 | ARG | 786 | 30.610 | 56.004 | 16.810 |
| 1141 | NH2 | ARG | 786 | 30.962 | 53.653 | 16.975 |
| 1142 | 1HH2 | ARG | 786 | 30.910 | 52.633 | 17.003 |
| 1143 | 2HH2 | ARG | 786 | 31.757 | 54.274 | 17.115 |
| 1144 | HE | ARG | 786 | 28.967 | 52.388 | 16.607 |
| 1145 | N | MET | 787 | 22.362 | 53.565 | 13.664 |
| 1146 | CA | MET | 787 | 20.914 | 53.644 | 13.317 |
| 1147 | HN | MET | 787 | 22.895 | 54.316 | 13.257 |

| | | | | | | |
|---|---|---|---|---|---|---|
| 1148 | C | MET | 787 | 20.615 | 52.774 | 12.060 |
| 1149 | O | MET | 787 | 19.523 | 52.225 | 11.947 |
| 1150 | CB | MET | 787 | 20.545 | 55.080 | 12.895 |
| 1151 | CG | MET | 787 | 20.214 | 56.022 | 14.047 |
| 1152 | SD | MET | 787 | 19.941 | 57.729 | 13.485 |
| 1153 | CE | MET | 787 | 20.679 | 58.638 | 14.873 |
| 1154 | N | ARG | 788 | 21.643 | 52.808 | 11.071 |
| 1155 | CA | ARG | 788 | 21.545 | 52.016 | 9.879 |
| 1156 | HN | ARG | 788 | 22.321 | 53.542 | 11.110 |
| 1157 | C | ARG | 788 | 21.468 | 50.589 | 10.305 |
| 1158 | O | ARG | 788 | 20.835 | 49.738 | 9.690 |
| 1159 | CB | ARG | 788 | 22.833 | 52.293 | 9.056 |
| 1160 | CG | ARG | 788 | 22.708 | 53.433 | 8.011 |
| 1161 | CD | ARG | 788 | 24.063 | 53.832 | 7.411 |
| 1162 | NE | ARG | 788 | 23.839 | 54.942 | 6.450 |
| 1163 | CZ | ARG | 788 | 24.782 | 55.549 | 5.742 |
| 1164 | NH1 | ARG | 788 | 26.046 | 55.246 | 5.791 |
| 1165 | 1HH1 | ARG | 788 | 26.249 | 54.481 | 6.435 |
| 1166 | 2HH1 | ARG | 788 | 26.675 | 55.784 | 5.199 |
| 1167 | NH2 | ARG | 788 | 24.416 | 56.499 | 4.957 |
| 1168 | 1HH2 | ARG | 788 | 23.407 | 56.657 | 4.993 |
| 1169 | 2HH2 | ARG | 788 | 25.134 | 56.971 | 4.412 |
| 1170 | HE | ARG | 788 | 22.899 | 55.259 | 6.327 |
| 1171 | N | HIS | 789 | 22.153 | 50.339 | 11.396 |
| 1172 | CA | HIS | 789 | 22.243 | 49.017 | 11.904 |
| 1173 | HN | HIS | 789 | 22.616 | 51.086 | 11.873 |
| 1174 | C | HIS | 789 | 20.950 | 48.353 | 12.340 |
| 1175 | O | HIS | 789 | 20.775 | 47.145 | 12.174 |
| 1176 | CB | HIS | 789 | 23.254 | 49.033 | 13.063 |
| 1177 | CG | HIS | 789 | 23.245 | 47.752 | 13.845 |
| 1178 | ND1 | HIS | 789 | 23.758 | 46.531 | 13.415 |
| 1179 | CE1 | HIS | 789 | 23.365 | 45.744 | 14.434 |
| 1180 | NE2 | HIS | 789 | 22.661 | 46.314 | 15.450 |
| 1181 | CD2 | HIS | 789 | 22.586 | 47.627 | 15.060 |
| 1182 | HE2 | HIS | 789 | 22.279 | 45.877 | 16.301 |
| 1183 | N | LEU | 790 | 20.049 | 49.131 | 12.906 |
| 1184 | CA | LEU | 790 | 18.817 | 48.561 | 13.415 |
| 1185 | HN | LEU | 790 | 20.216 | 50.114 | 12.985 |
| 1186 | C | LEU | 790 | 17.807 | 48.322 | 12.301 |
| 1187 | O | LEU | 790 | 17.107 | 47.307 | 12.280 |
| 1188 | CB | LEU | 790 | 18.223 | 49.485 | 14.515 |
| 1189 | CG | LEU | 790 | 19.132 | 50.595 | 15.108 |
| 1190 | CD1 | LEU | 790 | 18.405 | 51.321 | 16.248 |
| 1191 | CD2 | LEU | 790 | 20.478 | 50.052 | 15.615 |
| 1192 | N | SER | 791 | 17.761 | 49.278 | 11.384 |
| 1193 | CA | SER | 791 | 16.882 | 49.259 | 10.220 |
| 1194 | HN | SER | 791 | 18.369 | 50.064 | 11.497 |
| 1195 | C | SER | 791 | 17.248 | 48.056 | 9.390 |
| 1196 | O | SER | 791 | 16.448 | 47.474 | 8.650 |
| 1197 | CB | SER | 791 | 17.148 | 50.497 | 9.388 |
| 1198 | OG | SER | 791 | 17.297 | 50.290 | 8.012 |
| 1199 | HG | SER | 791 | 17.517 | 51.144 | 7.619 |
| 1200 | N | GLN | 792 | 18.510 | 47.706 | 9.525 |
| 1201 | CA | GLN | 792 | 19.063 | 46.614 | 8.791 |
| 1202 | HN | GLN | 792 | 19.094 | 48.216 | 10.156 |

| | | | | | | |
|---|---|---|---|---|---|---|
| 1203 | C | GLN | 792 | 18.670 | 45.295 | 9.410 |
| 1204 | O | GLN | 792 | 18.528 | 44.284 | 8.737 |
| 1205 | CB | GLN | 792 | 20.598 | 46.786 | 8.783 |
| 1206 | CG | GLN | 792 | 21.441 | 45.470 | 8.858 |
| 1207 | CD | GLN | 792 | 22.971 | 45.564 | 8.916 |
| 1208 | OE1 | GLN | 792 | 23.661 | 44.562 | 9.015 |
| 1209 | NE2 | GLN | 792 | 23.555 | 46.735 | 8.884 |
| 1210 | 2HE2 | GLN | 792 | 22.920 | 47.534 | 8.871 |
| 1211 | 1HE2 | GLN | 792 | 24.571 | 46.713 | 8.993 |
| 1212 | N | GLU | 793 | 18.482 | 45.308 | 10.714 |
| 1213 | CA | GLU | 793 | 18.132 | 44.091 | 11.393 |
| 1214 | HN | GLU | 793 | 18.582 | 46.159 | 11.228 |
| 1215 | C | GLU | 793 | 16.668 | 43.824 | 11.239 |
| 1216 | O | GLU | 793 | 16.230 | 42.679 | 11.129 |
| 1217 | CB | GLU | 793 | 18.518 | 44.229 | 12.845 |
| 1218 | CG | GLU | 793 | 19.997 | 44.150 | 12.996 |
| 1219 | CD | GLU | 793 | 20.469 | 42.740 | 12.723 |
| 1220 | OE1 | GLU | 793 | 20.202 | 41.865 | 13.569 |
| 1221 | OE2 | GLU | 793 | 21.081 | 42.493 | 11.663 |
| 1222 | HE2 | GLU | 793 | 21.309 | 41.576 | 11.637 |
| 1223 | N | PHE | 794 | 15.909 | 44.900 | 11.228 |
| 1224 | CA | PHE | 794 | 14.477 | 44.796 | 11.066 |
| 1225 | HN | PHE | 794 | 16.330 | 45.802 | 11.332 |
| 1226 | C | PHE | 794 | 14.232 | 44.138 | 9.744 |
| 1227 | O | PHE | 794 | 13.408 | 43.246 | 9.581 |
| 1228 | CB | PHE | 794 | 13.862 | 46.228 | 11.037 |
| 1229 | CG | PHE | 794 | 13.638 | 46.904 | 12.399 |
| 1230 | CD1 | PHE | 794 | 13.231 | 48.242 | 12.448 |
| 1231 | CE1 | PHE | 794 | 13.102 | 48.891 | 13.671 |
| 1232 | CZ | PHE | 794 | 13.362 | 48.203 | 14.854 |
| 1233 | CE2 | PHE | 794 | 13.757 | 46.867 | 14.813 |
| 1234 | CD2 | PHE | 794 | 13.897 | 46.218 | 13.588 |
| 1235 | N | GLY | 795 | 14.976 | 44.617 | 8.782 |
| 1236 | CA | GLY | 795 | 14.838 | 44.102 | 7.476 |
| 1237 | HN | GLY | 795 | 15.639 | 45.342 | 8.971 |
| 1238 | C | GLY | 795 | 15.235 | 42.653 | 7.417 |
| 1239 | O | GLY | 795 | 14.511 | 41.796 | 6.912 |
| 1240 | N | TRP | 796 | 16.426 | 42.387 | 7.907 |
| 1241 | CA | TRP | 796 | 16.928 | 41.041 | 7.870 |
| 1242 | HN | TRP | 796 | 16.981 | 43.119 | 8.304 |
| 1243 | C | TRP | 796 | 16.024 | 40.070 | 8.594 |
| 1244 | O | TRP | 796 | 15.810 | 38.937 | 8.166 |
| 1245 | CB | TRP | 796 | 18.344 | 41.020 | 8.510 |
| 1246 | CG | TRP | 796 | 18.823 | 39.637 | 8.963 |
| 1247 | CD1 | TRP | 796 | 20.079 | 39.349 | 9.535 |
| 1248 | NE1 | TRP | 796 | 20.240 | 37.968 | 9.764 |
| 1249 | CE2 | TRP | 796 | 19.052 | 37.416 | 9.313 |
| 1250 | CD2 | TRP | 796 | 18.184 | 38.423 | 8.823 |
| 1251 | HE1 | TRP | 796 | 21.063 | 37.477 | 10.131 |
| 1252 | CE3 | TRP | 796 | 16.916 | 38.084 | 8.283 |
| 1253 | CZ3 | TRP | 796 | 16.542 | 36.740 | 8.264 |
| 1254 | CH2 | TRP | 796 | 17.397 | 35.743 | 8.755 |
| 1255 | CZ2 | TRP | 796 | 18.653 | 36.062 | 9.276 |
| 1256 | N | LEU | 797 | 15.495 | 40.520 | 9.712 |
| 1257 | CA | LEU | 797 | 14.655 | 39.668 | 10.510 |

| | | | | | | |
|---|---|---|---|---|---|---|
| 1258 | HN | LEU | 797 | 15.677 | 41.458 | 10.007 |
| 1259 | C | LEU | 797 | 13.233 | 39.665 | 10.035 |
| 1260 | O | LEU | 797 | 12.396 | 38.918 | 10.547 |
| 1261 | CB | LEU | 797 | 14.713 | 40.140 | 11.936 |
| 1262 | CG | LEU | 797 | 15.052 | 39.039 | 12.925 |
| 1263 | CD1 | LEU | 797 | 16.041 | 38.065 | 12.339 |
| 1264 | CD2 | LEU | 797 | 15.612 | 39.703 | 14.158 |
| 1265 | N | GLN | 798 | 12.952 | 40.532 | 9.071 |
| 1266 | CA | GLN | 798 | 11.615 | 40.583 | 8.549 |
| 1267 | HN | GLN | 798 | 13.660 | 41.140 | 8.713 |
| 1268 | C | GLN | 798 | 10.695 | 40.731 | 9.721 |
| 1269 | O | GLN | 798 | 9.911 | 39.842 | 10.019 |
| 1270 | CB | GLN | 798 | 11.347 | 39.285 | 7.826 |
| 1271 | CG | GLN | 798 | 12.088 | 39.230 | 6.534 |
| 1272 | CD | GLN | 798 | 11.426 | 40.137 | 5.538 |
| 1273 | OE1 | GLN | 798 | 12.016 | 41.104 | 5.053 |
| 1274 | NE2 | GLN | 798 | 10.143 | 39.757 | 5.076 |
| 1275 | 1HE2 | GLN | 798 | 9.717 | 40.296 | 4.336 |
| 1276 | 2HE2 | GLN | 798 | 9.714 | 38.902 | 5.392 |
| 1277 | N | ILE | 799 | 10.802 | 41.849 | 10.410 |
| 1278 | CA | ILE | 799 | 9.957 | 42.060 | 11.554 |
| 1279 | HN | ILE | 799 | 11.465 | 42.546 | 10.139 |
| 1280 | C | ILE | 799 | 8.636 | 42.654 | 11.111 |
| 1281 | O | ILE | 799 | 8.601 | 43.514 | 10.245 |
| 1282 | CB | ILE | 799 | 10.695 | 42.984 | 12.606 |
| 1283 | CG1 | ILE | 799 | 9.752 | 43.671 | 13.645 |
| 1284 | CG2 | ILE | 799 | 11.563 | 44.092 | 11.934 |
| 1285 | CD1 | ILE | 799 | 10.446 | 44.309 | 14.866 |
| 1286 | N | THR | 800 | 7.549 | 42.174 | 11.707 |
| 1287 | CA | THR | 800 | 6.206 | 42.646 | 11.374 |
| 1288 | HN | THR | 800 | 7.653 | 41.466 | 12.405 |
| 1289 | C | THR | 800 | 5.740 | 43.761 | 12.307 |
| 1290 | O | THR | 800 | 6.142 | 43.810 | 13.468 |
| 1291 | CB | THR | 800 | 5.226 | 41.426 | 11.437 |
| 1292 | OG1 | THR | 800 | 4.904 | 41.115 | 12.786 |
| 1293 | HG1 | THR | 800 | 4.250 | 40.410 | 12.749 |
| 1294 | CG2 | THR | 800 | 5.748 | 40.098 | 10.847 |
| 1295 | N | PRO | 801 | 4.866 | 44.635 | 11.811 |
| 1296 | CA | PRO | 801 | 4.351 | 45.754 | 12.613 |
| 1297 | C | PRO | 801 | 3.904 | 45.330 | 13.998 |
| 1298 | O | PRO | 801 | 4.118 | 46.035 | 14.988 |
| 1299 | CB | PRO | 801 | 3.235 | 46.428 | 11.802 |
| 1300 | CG | PRO | 801 | 2.565 | 45.259 | 11.065 |
| 1301 | CD | PRO | 801 | 3.742 | 44.349 | 10.705 |
| 1302 | N | GLN | 802 | 3.249 | 44.186 | 14.073 |
| 1303 | CA | GLN | 802 | 2.791 | 43.772 | 15.369 |
| 1304 | HN | GLN | 802 | 3.079 | 43.631 | 13.259 |
| 1305 | C | GLN | 802 | 3.939 | 43.440 | 16.298 |
| 1306 | O | GLN | 802 | 3.917 | 43.859 | 17.456 |
| 1307 | CB | GLN | 802 | 1.795 | 42.595 | 15.279 |
| 1308 | CG | GLN | 802 | 0.278 | 42.976 | 15.236 |
| 1309 | CD | GLN | 802 | -0.774 | 41.860 | 15.274 |
| 1310 | OE1 | GLN | 802 | -0.681 | 40.932 | 16.061 |
| 1311 | NE2 | GLN | 802 | -1.779 | 41.881 | 14.435 |
| 1312 | 2HE2 | GLN | 802 | -1.761 | 42.646 | 13.759 |

| | | | | | | |
|---|---|---|---|---|---|---|
| 1313 | 1HE2 | GLN | 802 | -2.397 | 41.069 | 14.483 |
| 1314 | N | GLU | 803 | 4.954 | 42.726 | 15.820 |
| 1315 | CA | GLU | 803 | 6.095 | 42.447 | 16.690 |
| 1316 | HN | GLU | 803 | 4.937 | 42.385 | 14.881 |
| 1317 | C | GLU | 803 | 6.767 | 43.775 | 17.046 |
| 1318 | O | GLU | 803 | 7.105 | 44.024 | 18.194 |
| 1319 | CB | GLU | 803 | 7.107 | 41.550 | 15.999 |
| 1320 | CG | GLU | 803 | 6.522 | 40.254 | 15.553 |
| 1321 | CD | GLU | 803 | 7.380 | 39.567 | 14.525 |
| 1322 | OE1 | GLU | 803 | 7.888 | 40.268 | 13.614 |
| 1323 | OE2 | GLU | 803 | 7.534 | 38.332 | 14.621 |
| 1324 | HE2 | GLU | 803 | 8.090 | 38.028 | 13.920 |
| 1325 | N | PHE | 804 | 6.961 | 44.631 | 16.052 |
| 1326 | CA | PHE | 804 | 7.599 | 45.926 | 16.270 |
| 1327 | HN | PHE | 804 | 6.663 | 44.383 | 15.130 |
| 1328 | C | PHE | 804 | 6.929 | 46.789 | 17.341 |
| 1329 | O | PHE | 804 | 7.599 | 47.422 | 18.157 |
| 1330 | CB | PHE | 804 | 7.619 | 46.716 | 14.927 |
| 1331 | CG | PHE | 804 | 7.823 | 48.236 | 15.031 |
| 1332 | CD1 | PHE | 804 | 9.111 | 48.750 | 15.221 |
| 1333 | CE1 | PHE | 804 | 9.326 | 50.123 | 15.217 |
| 1334 | CZ | PHE | 804 | 8.259 | 50.993 | 15.006 |
| 1335 | CE2 | PHE | 804 | 6.975 | 50.488 | 14.803 |
| 1336 | CD2 | PHE | 804 | 6.757 | 49.113 | 14.818 |
| 1337 | N | LEU | 805 | 5.607 | 46.853 | 17.289 |
| 1338 | CA | LEU | 805 | 4.846 | 47.635 | 18.238 |
| 1339 | HN | LEU | 805 | 5.123 | 46.347 | 16.575 |
| 1340 | C | LEU | 805 | 5.157 | 47.193 | 19.666 |
| 1341 | O | LEU | 805 | 5.472 | 48.013 | 20.534 |
| 1342 | CB | LEU | 805 | 3.348 | 47.483 | 17.961 |
| 1343 | CG | LEU | 805 | 2.776 | 48.377 | 16.869 |
| 1344 | CD1 | LEU | 805 | 1.284 | 48.208 | 16.796 |
| 1345 | CD2 | LEU | 805 | 3.116 | 49.813 | 17.180 |
| 1346 | N | CYS | 806 | 5.049 | 45.885 | 19.882 |
| 1347 | CA | CYS | 806 | 5.314 | 45.273 | 21.168 |
| 1348 | HN | CYS | 806 | 4.771 | 45.298 | 19.122 |
| 1349 | C | CYS | 806 | 6.753 | 45.478 | 21.587 |
| 1350 | O | CYS | 806 | 7.049 | 45.668 | 22.759 |
| 1351 | CB | CYS | 806 | 5.047 | 43.781 | 21.103 |
| 1352 | SG | CYS | 806 | 3.280 | 43.364 | 20.905 |
| 1353 | HG | CYS | 806 | 3.138 | 42.037 | 20.857 |
| 1354 | N | MET | 807 | 7.657 | 45.430 | 20.633 |
| 1355 | CA | MET | 807 | 9.054 | 45.604 | 20.957 |
| 1356 | HN | MET | 807 | 7.378 | 45.273 | 19.686 |
| 1357 | C | MET | 807 | 9.406 | 47.017 | 21.406 |
| 1358 | O | MET | 807 | 10.286 | 47.205 | 22.252 |
| 1359 | CB | MET | 807 | 9.920 | 45.227 | 19.760 |
| 1360 | CG | MET | 807 | 10.038 | 43.744 | 19.538 |
| 1361 | SD | MET | 807 | 11.148 | 43.450 | 18.194 |
| 1362 | CE | MET | 807 | 10.046 | 43.456 | 16.968 |
| 1363 | N | LYS | 808 | 8.737 | 48.013 | 20.833 |
| 1364 | CA | LYS | 808 | 9.020 | 49.393 | 21.192 |
| 1365 | HN | LYS | 808 | 8.035 | 47.811 | 20.150 |
| 1366 | C | LYS | 808 | 8.475 | 49.690 | 22.582 |
| 1367 | O | LYS | 808 | 9.008 | 50.541 | 23.304 |

| | | | | | | |
|---|---|---|---|---|---|---|
| 1368 | CB | LYS | 808 | 8.393 | 50.324 | 20.170 |
| 1369 | CG | LYS | 808 | 8.973 | 51.714 | 20.152 |
| 1370 | CD | LYS | 808 | 8.430 | 52.481 | 18.948 |
| 1371 | CE | LYS | 808 | 6.976 | 52.947 | 19.146 |
| 1372 | NZ | LYS | 808 | 5.878 | 51.911 | 19.313 |
| 1373 | HZ1 | LYS | 808 | 4.967 | 52.385 | 19.393 |
| 1374 | HZ2 | LYS | 808 | 5.868 | 51.285 | 18.495 |
| 1375 | HZ3 | LYS | 808 | 6.049 | 51.374 | 20.139 |
| 1376 | N | ALA | 809 | 7.405 | 48.990 | 22.947 |
| 1377 | CA | ALA | 809 | 6.794 | 49.152 | 24.249 |
| 1378 | HN | ALA | 809 | 7.011 | 48.333 | 22.304 |
| 1379 | C | ALA | 809 | 7.792 | 48.552 | 25.214 |
| 1380 | O | ALA | 809 | 8.098 | 49.138 | 26.238 |
| 1381 | CB | ALA | 809 | 5.433 | 48.434 | 24.276 |
| 1382 | N | LEU | 810 | 8.316 | 47.381 | 24.860 |
| 1383 | CA | LEU | 810 | 9.317 | 46.724 | 25.680 |
| 1384 | HN | LEU | 810 | 8.013 | 46.945 | 24.013 |
| 1385 | C | LEU | 810 | 10.541 | 47.619 | 25.856 |
| 1386 | O | LEU | 810 | 11.125 | 47.645 | 26.920 |
| 1387 | CB | LEU | 810 | 9.732 | 45.399 | 25.057 |
| 1388 | CG | LEU | 810 | 9.203 | 44.140 | 25.744 |
| 1389 | CD1 | LEU | 810 | 8.142 | 44.484 | 26.747 |
| 1390 | CD2 | LEU | 810 | 8.655 | 43.185 | 24.714 |
| 1391 | N | LEU | 811 | 10.939 | 48.369 | 24.839 |
| 1392 | CA | LEU | 811 | 12.106 | 49.226 | 25.037 |
| 1393 | HN | LEU | 811 | 10.456 | 48.350 | 23.963 |
| 1394 | C | LEU | 811 | 11.882 | 50.322 | 26.062 |
| 1395 | O | LEU | 811 | 12.836 | 50.825 | 26.638 |
| 1396 | CB | LEU | 811 | 12.541 | 49.892 | 23.740 |
| 1397 | CG | LEU | 811 | 13.182 | 49.049 | 22.650 |
| 1398 | CD1 | LEU | 811 | 13.307 | 49.968 | 21.459 |
| 1399 | CD2 | LEU | 811 | 14.549 | 48.490 | 23.053 |
| 1400 | N | LEU | 812 | 10.630 | 50.712 | 26.265 |
| 1401 | CA | LEU | 812 | 10.312 | 51.764 | 27.230 |
| 1402 | HN | LEU | 812 | 9.890 | 50.278 | 25.750 |
| 1403 | C | LEU | 812 | 10.574 | 51.184 | 28.611 |
| 1404 | O | LEU | 812 | 10.954 | 51.899 | 29.541 |
| 1405 | CB | LEU | 812 | 8.839 | 52.169 | 27.109 |
| 1406 | CG | LEU | 812 | 8.322 | 53.116 | 28.189 |
| 1407 | CD1 | LEU | 812 | 9.120 | 54.420 | 28.142 |
| 1408 | CD2 | LEU | 812 | 6.830 | 53.361 | 27.988 |
| 1409 | N | PHE | 813 | 10.381 | 49.872 | 28.710 |
| 1410 | CA | PHE | 813 | 10.577 | 49.134 | 29.944 |
| 1411 | HN | PHE | 813 | 10.089 | 49.370 | 27.896 |
| 1412 | C | PHE | 813 | 11.923 | 48.441 | 29.920 |
| 1413 | O | PHE | 813 | 12.131 | 47.479 | 30.657 |
| 1414 | CB | PHE | 813 | 9.458 | 48.055 | 30.068 |
| 1415 | CG | PHE | 813 | 8.019 | 48.533 | 29.822 |
| 1416 | CD1 | PHE | 813 | 6.960 | 47.624 | 29.937 |
| 1417 | CE1 | PHE | 813 | 5.660 | 48.020 | 29.640 |
| 1418 | CZ | PHE | 813 | 5.407 | 49.331 | 29.243 |
| 1419 | CE2 | PHE | 813 | 6.455 | 50.245 | 29.139 |
| 1420 | CD2 | PHE | 813 | 7.758 | 49.847 | 29.426 |
| 1421 | N | SER | 814 | 12.836 | 48.936 | 29.093 |
| 1422 | CA | SER | 814 | 14.140 | 48.299 | 28.921 |

| | | | | | | |
|---|---|---|---|---|---|---|
| 1423 | HN | SER | 814 | 12.627 | 49.766 | 28.575 |
| 1424 | C | SER | 814 | 15.279 | 48.552 | 29.904 |
| 1425 | O | SER | 814 | 16.359 | 47.972 | 29.754 |
| 1426 | CB | SER | 814 | 14.661 | 48.552 | 27.506 |
| 1427 | OG | SER | 814 | 15.276 | 49.824 | 27.308 |
| 1428 | HG | SER | 814 | 14.565 | 50.423 | 27.041 |
| 1429 | N | ILE | 815 | 15.084 | 49.428 | 30.877 |
| 1430 | CA | ILE | 815 | 16.134 | 49.624 | 31.870 |
| 1431 | HN | ILE | 815 | 14.231 | 49.947 | 30.930 |
| 1432 | C | ILE | 815 | 15.597 | 50.417 | 33.047 |
| 1433 | O | ILE | 815 | 14.769 | 51.313 | 32.899 |
| 1434 | CB | ILE | 815 | 17.441 | 50.281 | 31.265 |
| 1435 | CG1 | ILE | 815 | 17.293 | 51.786 | 30.874 |
| 1436 | CG2 | ILE | 815 | 17.986 | 49.509 | 30.023 |
| 1437 | CD1 | ILE | 815 | 18.608 | 52.540 | 30.588 |
| 1438 | N | ILE | 816 | 16.033 | 50.023 | 34.233 |
| 1439 | CA | ILE | 816 | 15.585 | 50.666 | 35.447 |
| 1440 | HN | ILE | 816 | 16.684 | 49.266 | 34.291 |
| 1441 | C | ILE | 816 | 16.791 | 51.117 | 36.256 |
| 1442 | O | ILE | 816 | 17.930 | 50.741 | 35.961 |
| 1443 | CB | ILE | 816 | 14.666 | 49.671 | 36.266 |
| 1444 | CG1 | ILE | 816 | 13.280 | 49.373 | 35.608 |
| 1445 | CG2 | ILE | 816 | 14.415 | 50.145 | 37.730 |
| 1446 | CD1 | ILE | 816 | 12.498 | 48.180 | 36.195 |
| 1447 | N | PRO | 817 | 16.563 | 51.958 | 37.275 |
| 1448 | CA | PRO | 817 | 17.668 | 52.431 | 38.099 |
| 1449 | CD | PRO | 817 | 15.276 | 52.418 | 37.819 |
| 1450 | C | PRO | 817 | 18.185 | 51.221 | 38.868 |
| 1451 | O | PRO | 817 | 17.402 | 50.334 | 39.217 |
| 1452 | CB | PRO | 817 | 16.993 | 53.465 | 38.993 |
| 1453 | CG | PRO | 817 | 15.648 | 52.866 | 39.206 |
| 1454 | N | VAL | 818 | 19.493 | 51.150 | 39.095 |
| 1455 | CA | VAL | 818 | 20.051 | 50.018 | 39.834 |
| 1456 | HN | VAL | 818 | 20.098 | 51.873 | 38.760 |
| 1457 | C | VAL | 818 | 19.707 | 50.210 | 41.288 |
| 1458 | O | VAL | 818 | 20.462 | 50.805 | 42.041 |
| 1459 | CB | VAL | 818 | 21.616 | 49.930 | 39.634 |
| 1460 | CG1 | VAL | 818 | 22.412 | 49.182 | 40.739 |
| 1461 | CG2 | VAL | 818 | 22.042 | 49.265 | 38.302 |
| 1462 | N | ASP | 819 | 18.554 | 49.692 | 41.671 |
| 1463 | CA | ASP | 819 | 18.050 | 49.804 | 43.023 |
| 1464 | HN | ASP | 819 | 18.004 | 49.200 | 40.995 |
| 1465 | C | ASP | 819 | 16.579 | 49.551 | 42.796 |
| 1466 | O | ASP | 819 | 15.893 | 48.957 | 43.627 |
| 1467 | CB | ASP | 819 | 18.259 | 51.217 | 43.592 |
| 1468 | CG | ASP | 819 | 19.591 | 51.361 | 44.337 |
| 1469 | OD1 | ASP | 819 | 20.076 | 50.357 | 44.906 |
| 1470 | OD2 | ASP | 819 | 20.141 | 52.485 | 44.364 |
| 1471 | HD2 | ASP | 819 | 20.948 | 52.430 | 44.853 |
| 1472 | N | GLY | 820 | 16.109 | 50.000 | 41.634 |
| 1473 | CA | GLY | 820 | 14.719 | 49.790 | 41.279 |
| 1474 | HN | GLY | 820 | 16.717 | 50.483 | 41.004 |
| 1475 | C | GLY | 820 | 13.825 | 50.996 | 41.429 |
| 1476 | O | GLY | 820 | 14.203 | 52.016 | 42.013 |
| 1477 | N | LEU | 821 | 12.610 | 50.858 | 40.909 |

| | | | | | | |
|---|---|---|---|---|---|---|
| 1478 | CA | LEU | 821 | 11.630 | 51.928 | 40.943 |
| 1479 | HN | LEU | 821 | 12.363 | 49.989 | 40.480 |
| 1480 | C | LEU | 821 | 10.728 | 51.875 | 42.168 |
| 1481 | O | LEU | 821 | 10.668 | 50.869 | 42.873 |
| 1482 | CB | LEU | 821 | 10.747 | 51.874 | 39.691 |
| 1483 | CG | LEU | 821 | 11.282 | 51.133 | 38.465 |
| 1484 | CD1 | LEU | 821 | 10.234 | 51.086 | 37.376 |
| 1485 | CD2 | LEU | 821 | 12.499 | 51.831 | 37.961 |
| 1486 | N | LYS | 822 | 10.023 | 52.977 | 42.394 |
| 1487 | CA | LYS | 822 | 9.092 | 53.097 | 43.507 |
| 1488 | HN | LYS | 822 | 10.136 | 53.755 | 41.776 |
| 1489 | C | LYS | 822 | 7.993 | 52.055 | 43.370 |
| 1490 | O | LYS | 822 | 7.441 | 51.575 | 44.364 |
| 1491 | CB | LYS | 822 | 8.456 | 54.488 | 43.516 |
| 1492 | CG | LYS | 822 | 9.436 | 55.628 | 43.706 |
| 1493 | CD | LYS | 822 | 8.712 | 56.915 | 44.064 |
| 1494 | CE | LYS | 822 | 9.633 | 58.114 | 43.934 |
| 1495 | NZ | LYS | 822 | 9.044 | 59.459 | 44.297 |
| 1496 | HZ1 | LYS | 822 | 9.774 | 60.183 | 44.231 |
| 1497 | HZ2 | LYS | 822 | 8.277 | 59.685 | 43.648 |
| 1498 | HZ3 | LYS | 822 | 8.690 | 59.427 | 45.232 |
| 1499 | N | ASN | 823 | 7.685 | 51.710 | 42.124 |
| 1500 | CA | ASN | 823 | 6.638 | 50.742 | 41.831 |
| 1501 | HN | ASN | 823 | 8.186 | 52.127 | 41.367 |
| 1502 | C | ASN | 823 | 7.218 | 49.524 | 41.124 |
| 1503 | O | ASN | 823 | 6.548 | 48.918 | 40.287 |
| 1504 | CB | ASN | 823 | 5.554 | 51.426 | 40.949 |
| 1505 | CG | ASN | 823 | 5.219 | 52.887 | 41.269 |
| 1506 | OD1 | ASN | 823 | 5.671 | 53.817 | 40.618 |
| 1507 | ND2 | ASN | 823 | 4.443 | 53.147 | 42.287 |
| 1508 | 1HD2 | ASN | 823 | 4.407 | 54.146 | 42.505 |
| 1509 | 2HD2 | ASN | 823 | 4.156 | 52.356 | 42.864 |
| 1510 | N | GLN | 824 | 8.456 | 49.178 | 41.466 |
| 1511 | CA | GLN | 824 | 9.149 | 48.037 | 40.861 |
| 1512 | HN | GLN | 824 | 8.931 | 49.716 | 42.162 |
| 1513 | C | GLN | 824 | 8.192 | 46.878 | 40.678 |
| 1514 | O | GLN | 824 | 8.146 | 46.244 | 39.621 |
| 1515 | CB | GLN | 824 | 10.317 | 47.597 | 41.749 |
| 1516 | CG | GLN | 824 | 11.125 | 46.435 | 41.192 |
| 1517 | CD | GLN | 824 | 11.753 | 46.736 | 39.838 |
| 1518 | OE1 | GLN | 824 | 12.593 | 47.637 | 39.708 |
| 1519 | NE2 | GLN | 824 | 11.300 | 46.051 | 38.710 |
| 1520 | 1HE2 | GLN | 824 | 11.713 | 46.227 | 37.809 |
| 1521 | 2HE2 | GLN | 824 | 10.698 | 45.239 | 38.819 |
| 1522 | N | LYS | 825 | 7.428 | 46.617 | 41.721 |
| 1523 | CA | LYS | 825 | 6.437 | 45.559 | 41.743 |
| 1524 | HN | LYS | 825 | 7.538 | 47.180 | 42.541 |
| 1525 | C | LYS | 825 | 5.617 | 45.521 | 40.432 |
| 1526 | O | LYS | 825 | 5.665 | 44.542 | 39.674 |
| 1527 | CB | LYS | 825 | 5.509 | 45.753 | 42.974 |
| 1528 | CG | LYS | 825 | 5.192 | 47.239 | 43.277 |
| 1529 | CD | LYS | 825 | 6.046 | 47.867 | 44.381 |
| 1530 | CE | LYS | 825 | 7.479 | 47.326 | 44.282 |
| 1531 | NZ | LYS | 825 | 8.344 | 48.054 | 45.227 |
| 1532 | HZ2 | LYS | 825 | 7.837 | 48.262 | 46.095 |

| | | | | | | |
|---|---|---|---|---|---|---|
| 1533 | HZ1 | LYS | 825 | 9.156 | 47.490 | 45.500 |
| 1534 | HZ3 | LYS | 825 | 8.656 | 48.906 | 44.807 |
| 1535 | N | PHE | 826 | 4.911 | 46.618 | 40.171 |
| 1536 | CA | PHE | 826 | 4.043 | 46.821 | 39.000 |
| 1537 | HN | PHE | 826 | 4.976 | 47.370 | 40.826 |
| 1538 | C | PHE | 826 | 4.787 | 46.827 | 37.655 |
| 1539 | O | PHE | 826 | 4.284 | 46.363 | 36.626 |
| 1540 | CB | PHE | 826 | 3.299 | 48.180 | 39.169 |
| 1541 | CG | PHE | 826 | 1.789 | 48.172 | 38.885 |
| 1542 | CD1 | PHE | 826 | 1.182 | 49.308 | 38.335 |
| 1543 | CE1 | PHE | 826 | -0.195 | 49.346 | 38.146 |
| 1544 | CZ | PHE | 826 | -0.974 | 48.244 | 38.490 |
| 1545 | CE2 | PHE | 826 | -0.375 | 47.105 | 39.026 |
| 1546 | CD2 | PHE | 826 | 1.002 | 47.069 | 39.226 |
| 1547 | N | PHE | 827 | 5.974 | 47.402 | 37.672 |
| 1548 | CA | PHE | 827 | 6.803 | 47.465 | 36.494 |
| 1549 | HN | PHE | 827 | 6.307 | 47.805 | 38.525 |
| 1550 | C | PHE | 827 | 6.951 | 46.051 | 35.974 |
| 1551 | O | PHE | 827 | 6.585 | 45.728 | 34.845 |
| 1552 | CB | PHE | 827 | 8.162 | 47.986 | 36.894 |
| 1553 | CG | PHE | 827 | 9.121 | 48.038 | 35.779 |
| 1554 | CD1 | PHE | 827 | 9.173 | 49.152 | 34.962 |
| 1555 | CE1 | PHE | 827 | 10.054 | 49.210 | 33.908 |
| 1556 | CZ | PHE | 827 | 10.896 | 48.134 | 33.652 |
| 1557 | CE2 | PHE | 827 | 10.851 | 47.007 | 34.469 |
| 1558 | CD2 | PHE | 827 | 9.958 | 46.962 | 35.523 |
| 1559 | N | ASP | 828 | 7.494 | 45.200 | 36.835 |
| 1560 | CA | ASP | 828 | 7.725 | 43.824 | 36.478 |
| 1561 | HN | ASP | 828 | 7.746 | 45.520 | 37.748 |
| 1562 | C | ASP | 828 | 6.457 | 43.114 | 36.085 |
| 1563 | O | ASP | 828 | 6.497 | 42.067 | 35.439 |
| 1564 | CB | ASP | 828 | 8.428 | 43.100 | 37.621 |
| 1565 | CG | ASP | 828 | 9.875 | 43.534 | 37.760 |
| 1566 | OD1 | ASP | 828 | 10.488 | 43.887 | 36.727 |
| 1567 | OD2 | ASP | 828 | 10.406 | 43.513 | 38.890 |
| 1568 | HD2 | ASP | 828 | 11.302 | 43.807 | 38.823 |
| 1569 | N | GLU | 829 | 5.322 | 43.685 | 36.452 |
| 1570 | CA | GLU | 829 | 4.073 | 43.059 | 36.084 |
| 1571 | HN | GLU | 829 | 5.329 | 44.536 | 36.976 |
| 1572 | C | GLU | 829 | 3.716 | 43.494 | 34.675 |
| 1573 | O | GLU | 829 | 3.333 | 42.673 | 33.831 |
| 1574 | CB | GLU | 829 | 2.972 | 43.475 | 37.028 |
| 1575 | CG | GLU | 829 | 1.739 | 42.656 | 36.823 |
| 1576 | CD | GLU | 829 | 0.549 | 43.287 | 37.463 |
| 1577 | OE1 | GLU | 829 | 0.751 | 44.056 | 38.432 |
| 1578 | OE2 | GLU | 829 | -0.580 | 43.009 | 37.001 |
| 1579 | HE2 | GLU | 829 | -1.243 | 43.470 | 37.491 |
| 1580 | N | LEU | 830 | 3.845 | 44.792 | 34.429 |
| 1581 | CA | LEU | 830 | 3.554 | 45.353 | 33.128 |
| 1582 | HN | LEU | 830 | 4.151 | 45.398 | 35.162 |
| 1583 | C | LEU | 830 | 4.558 | 44.788 | 32.155 |
| 1584 | O | LEU | 830 | 4.190 | 44.248 | 31.119 |
| 1585 | CB | LEU | 830 | 3.648 | 46.904 | 33.186 |
| 1586 | CG | LEU | 830 | 2.597 | 47.670 | 34.034 |
| 1587 | CD1 | LEU | 830 | 2.769 | 49.183 | 33.847 |

| | | | | | | |
|---|---|---|---|---|---|---|
| 1588 | CD2 | LEU | 830 | 1.153 | 47.266 | 33.696 |
| 1589 | N | ARG | 831 | 5.831 | 44.879 | 32.504 |
| 1590 | CA | ARG | 831 | 6.857 | 44.348 | 31.632 |
| 1591 | HN | ARG | 831 | 6.083 | 45.314 | 33.368 |
| 1592 | C | ARG | 831 | 6.663 | 42.886 | 31.230 |
| 1593 | O | ARG | 831 | 7.031 | 42.498 | 30.125 |
| 1594 | CB | ARG | 831 | 8.222 | 44.487 | 32.276 |
| 1595 | CG | ARG | 831 | 9.338 | 43.990 | 31.382 |
| 1596 | CD | ARG | 831 | 10.592 | 44.687 | 31.817 |
| 1597 | NE | ARG | 831 | 11.788 | 44.423 | 31.025 |
| 1598 | CZ | ARG | 831 | 12.240 | 43.212 | 30.720 |
| 1599 | NH1 | ARG | 831 | 11.722 | 42.067 | 31.237 |
| 1600 | 1HH1 | ARG | 831 | 12.110 | 41.185 | 30.975 |
| 1601 | 2HH1 | ARG | 831 | 10.954 | 42.117 | 31.874 |
| 1602 | NH2 | ARG | 831 | 13.301 | 43.102 | 29.867 |
| 1603 | 1HH2 | ARG | 831 | 13.666 | 42.201 | 29.627 |
| 1604 | 2HH2 | ARG | 831 | 13.711 | 43.925 | 29.480 |
| 1605 | HE | ARG | 831 | 12.305 | 45.210 | 30.688 |
| 1606 | N | MET | 832 | 6.107 | 42.067 | 32.117 |
| 1607 | CA | MET | 832 | 5.894 | 40.660 | 31.804 |
| 1608 | HN | MET | 832 | 5.830 | 42.421 | 33.011 |
| 1609 | C | MET | 832 | 4.759 | 40.503 | 30.792 |
| 1610 | O | MET | 832 | 4.808 | 39.640 | 29.913 |
| 1611 | CB | MET | 832 | 5.565 | 39.887 | 33.086 |
| 1612 | CG | MET | 832 | 5.557 | 38.364 | 32.952 |
| 1613 | SD | MET | 832 | 7.172 | 37.729 | 32.409 |
| 1614 | CE | MET | 832 | 6.700 | 37.034 | 30.872 |
| 1615 | N | ASN | 833 | 3.740 | 41.353 | 30.925 |
| 1616 | CA | ASN | 833 | 2.576 | 41.314 | 30.040 |
| 1617 | HN | ASN | 833 | 3.773 | 42.038 | 31.653 |
| 1618 | C | ASN | 833 | 2.939 | 41.645 | 28.607 |
| 1619 | O | ASN | 833 | 2.409 | 41.038 | 27.664 |
| 1620 | CB | ASN | 833 | 1.472 | 42.277 | 30.566 |
| 1621 | CG | ASN | 833 | 1.143 | 42.206 | 32.060 |
| 1622 | OD1 | ASN | 833 | 1.580 | 43.017 | 32.863 |
| 1623 | ND2 | ASN | 833 | 0.388 | 41.232 | 32.493 |
| 1624 | 1HD2 | ASN | 833 | 0.356 | 41.188 | 33.515 |
| 1625 | 2HD2 | ASN | 833 | 0.115 | 40.521 | 31.814 |
| 1626 | N | TYR | 834 | 3.812 | 42.633 | 28.428 |
| 1627 | CA | TYR | 834 | 4.212 | 42.934 | 27.078 |
| 1628 | HN | TYR | 834 | 4.177 | 43.149 | 29.204 |
| 1629 | C | TYR | 834 | 5.231 | 41.906 | 26.606 |
| 1630 | O | TYR | 834 | 5.482 | 41.819 | 25.421 |
| 1631 | CB | TYR | 834 | 4.742 | 44.368 | 26.937 |
| 1632 | CG | TYR | 834 | 3.624 | 45.390 | 27.002 |
| 1633 | CD1 | TYR | 834 | 2.598 | 45.389 | 26.052 |
| 1634 | CE1 | TYR | 834 | 1.490 | 46.230 | 26.193 |
| 1635 | CZ | TYR | 834 | 1.410 | 47.080 | 27.284 |
| 1636 | OH | TYR | 834 | 0.360 | 47.950 | 27.408 |
| 1637 | HH | TYR | 834 | 0.446 | 48.416 | 28.251 |
| 1638 | CE2 | TYR | 834 | 2.427 | 47.107 | 28.224 |
| 1639 | CD2 | TYR | 834 | 3.523 | 46.270 | 28.079 |
| 1640 | N | ILE | 835 | 5.828 | 41.106 | 27.485 |
| 1641 | CA | ILE | 835 | 6.748 | 40.123 | 26.919 |
| 1642 | HN | ILE | 835 | 5.659 | 41.174 | 28.468 |

| | | | | | | |
|---|---|---|---|---|---|---|
| 1643 | C | ILE | 835 | 5.924 | 39.010 | 26.292 |
| 1644 | O | ILE | 835 | 6.310 | 38.445 | 25.274 |
| 1645 | CB | ILE | 835 | 7.733 | 39.512 | 27.935 |
| 1646 | CG2 | ILE | 835 | 8.465 | 38.324 | 27.285 |
| 1647 | CG1 | ILE | 835 | 8.770 | 40.563 | 28.335 |
| 1648 | CD1 | ILE | 835 | 9.397 | 40.319 | 29.678 |
| 1649 | N | LYS | 836 | 4.782 | 38.702 | 26.894 |
| 1650 | CA | LYS | 836 | 3.921 | 37.672 | 26.337 |
| 1651 | HN | LYS | 836 | 4.514 | 39.179 | 27.732 |
| 1652 | C | LYS | 836 | 3.173 | 38.282 | 25.150 |
| 1653 | O | LYS | 836 | 2.781 | 37.583 | 24.217 |
| 1654 | CB | LYS | 836 | 2.939 | 37.173 | 27.394 |
| 1655 | CG | LYS | 836 | 3.582 | 36.404 | 28.542 |
| 1656 | CD | LYS | 836 | 2.575 | 36.312 | 29.676 |
| 1657 | CE | LYS | 836 | 2.909 | 35.261 | 30.726 |
| 1658 | NZ | LYS | 836 | 1.798 | 35.031 | 31.736 |
| 1659 | HZ1 | LYS | 836 | 1.400 | 35.937 | 32.021 |
| 1660 | HZ2 | LYS | 836 | 1.061 | 34.451 | 31.310 |
| 1661 | HZ3 | LYS | 836 | 2.168 | 34.562 | 32.538 |
| 1662 | N | GLU | 837 | 2.985 | 39.594 | 25.194 |
| 1663 | CA | GLU | 837 | 2.314 | 40.291 | 24.115 |
| 1664 | HN | GLU | 837 | 3.312 | 40.111 | 25.985 |
| 1665 | C | GLU | 837 | 3.224 | 40.145 | 22.892 |
| 1666 | O | GLU | 837 | 2.747 | 40.020 | 21.757 |
| 1667 | CB | GLU | 837 | 2.129 | 41.756 | 24.506 |
| 1668 | CG | GLU | 837 | 0.878 | 42.449 | 23.947 |
| 1669 | CD | GLU | 837 | -0.363 | 41.556 | 23.874 |
| 1670 | OE1 | GLU | 837 | -0.768 | 40.962 | 24.903 |
| 1671 | OE2 | GLU | 837 | -0.936 | 41.463 | 22.763 |
| 1672 | HE2 | GLU | 837 | -1.683 | 40.889 | 22.841 |
| 1673 | N | LEU | 838 | 4.535 | 40.143 | 23.135 |
| 1674 | CA | LEU | 838 | 5.502 | 39.980 | 22.058 |
| 1675 | HN | LEU | 838 | 4.861 | 40.255 | 24.074 |
| 1676 | C | LEU | 838 | 5.325 | 38.560 | 21.578 |
| 1677 | O | LEU | 838 | 5.338 | 38.275 | 20.383 |
| 1678 | CB | LEU | 838 | 6.945 | 40.169 | 22.549 |
| 1679 | CG | LEU | 838 | 7.938 | 39.886 | 21.417 |
| 1680 | CD1 | LEU | 838 | 7.511 | 40.699 | 20.219 |
| 1681 | CD2 | LEU | 838 | 9.362 | 40.214 | 21.805 |
| 1682 | N | ASP | 839 | 5.140 | 37.669 | 22.539 |
| 1683 | CA | ASP | 839 | 4.962 | 36.262 | 22.235 |
| 1684 | HN | ASP | 839 | 5.124 | 37.971 | 23.492 |
| 1685 | C | ASP | 839 | 3.814 | 35.945 | 21.293 |
| 1686 | O | ASP | 839 | 3.991 | 35.178 | 20.350 |
| 1687 | CB | ASP | 839 | 4.836 | 35.497 | 23.579 |
| 1688 | CG | ASP | 839 | 4.904 | 33.965 | 23.509 |
| 1689 | OD1 | ASP | 839 | 4.193 | 33.235 | 24.184 |
| 1690 | OD2 | ASP | 839 | 5.821 | 33.516 | 22.599 |
| 1691 | HD2 | ASP | 839 | 5.824 | 32.571 | 22.595 |
| 1692 | N | ARG | 840 | 2.640 | 36.517 | 21.558 |
| 1693 | CA | ARG | 840 | 1.457 | 36.297 | 20.722 |
| 1694 | HN | ARG | 840 | 2.563 | 37.116 | 22.355 |
| 1695 | C | ARG | 840 | 1.676 | 36.840 | 19.320 |
| 1696 | O | ARG | 840 | 1.331 | 36.197 | 18.326 |
| 1697 | CB | ARG | 840 | 0.244 | 36.979 | 21.411 |

| | | | | | | |
|---|---|---|---|---|---|---|
| 1698 | CG | ARG | 840 | -0.080 | 36.454 | 22.835 |
| 1699 | CD | ARG | 840 | -1.170 | 37.279 | 23.532 |
| 1700 | NE | ARG | 840 | -1.819 | 36.423 | 24.557 |
| 1701 | CZ | ARG | 840 | -2.807 | 36.797 | 25.359 |
| 1702 | NH1 | ARG | 840 | -3.350 | 37.978 | 25.353 |
| 1703 | 1HH1 | ARG | 840 | -2.940 | 38.603 | 24.658 |
| 1704 | 2HH1 | ARG | 840 | -4.102 | 38.147 | 26.018 |
| 1705 | NH2 | ARG | 840 | -3.253 | 35.929 | 26.197 |
| 1706 | 1HH2 | ARG | 840 | -2.761 | 35.036 | 26.120 |
| 1707 | 2HH2 | ARG | 840 | -4.009 | 36.210 | 26.817 |
| 1708 | HE | ARG | 840 | -1.484 | 35.485 | 24.650 |
| 1709 | N | ILE | 841 | 2.213 | 38.053 | 19.254 |
| 1710 | CA | ILE | 841 | 2.476 | 38.694 | 17.985 |
| 1711 | HN | ILE | 841 | 2.440 | 38.534 | 20.101 |
| 1712 | C | ILE | 841 | 3.331 | 37.722 | 17.193 |
| 1713 | O | ILE | 841 | 3.092 | 37.505 | 16.007 |
| 1714 | CB | ILE | 841 | 3.218 | 40.072 | 18.222 |
| 1715 | CG1 | ILE | 841 | 2.425 | 41.096 | 19.097 |
| 1716 | CG2 | ILE | 841 | 3.618 | 40.780 | 16.891 |
| 1717 | CD1 | ILE | 841 | 3.231 | 42.305 | 19.615 |
| 1718 | N | ILE | 842 | 4.316 | 37.130 | 17.862 |
| 1719 | CA | ILE | 842 | 5.197 | 36.165 | 17.212 |
| 1720 | HN | ILE | 842 | 4.456 | 37.350 | 18.827 |
| 1721 | C | ILE | 842 | 4.440 | 34.939 | 16.681 |
| 1722 | O | ILE | 842 | 4.598 | 34.576 | 15.516 |
| 1723 | CB | ILE | 842 | 6.304 | 35.673 | 18.167 |
| 1724 | CG2 | ILE | 842 | 7.024 | 34.482 | 17.547 |
| 1725 | CG1 | ILE | 842 | 7.295 | 36.803 | 18.451 |
| 1726 | CD1 | ILE | 842 | 8.262 | 36.508 | 19.591 |
| 1727 | N | ALA | 843 | 3.624 | 34.300 | 17.528 |
| 1728 | CA | ALA | 843 | 2.864 | 33.114 | 17.116 |
| 1729 | HN | ALA | 843 | 3.529 | 34.639 | 18.464 |
| 1730 | C | ALA | 843 | 1.852 | 33.364 | 16.013 |
| 1731 | O | ALA | 843 | 1.584 | 32.470 | 15.219 |
| 1732 | CB | ALA | 843 | 2.243 | 32.446 | 18.355 |
| 1733 | N | CYS | 844 | 1.272 | 34.560 | 15.966 |
| 1734 | CA | CYS | 844 | 0.265 | 34.833 | 14.952 |
| 1735 | HN | CYS | 844 | 1.526 | 35.269 | 16.625 |
| 1736 | C | CYS | 844 | 0.809 | 35.417 | 13.651 |
| 1737 | O | CYS | 844 | 0.066 | 36.025 | 12.892 |
| 1738 | CB | CYS | 844 | -0.847 | 35.704 | 15.569 |
| 1739 | SG | CYS | 844 | -0.792 | 37.381 | 14.899 |
| 1740 | HG | CYS | 844 | -0.268 | 37.965 | 15.973 |
| 1741 | N | LYS | 845 | 2.092 | 35.211 | 13.370 |
| 1742 | CA | LYS | 845 | 2.681 | 35.753 | 12.142 |
| 1743 | HN | LYS | 845 | 2.660 | 34.683 | 14.001 |
| 1744 | C | LYS | 845 | 3.342 | 34.641 | 11.356 |
| 1745 | O | LYS | 845 | 3.279 | 34.579 | 10.126 |
| 1746 | CB | LYS | 845 | 3.711 | 36.852 | 12.523 |
| 1747 | CG | LYS | 845 | 4.426 | 37.477 | 11.299 |
| 1748 | CD | LYS | 845 | 5.761 | 38.158 | 11.612 |
| 1749 | CE | LYS | 845 | 6.910 | 37.264 | 11.126 |
| 1750 | NZ | LYS | 845 | 7.238 | 37.604 | 9.730 |
| 1751 | HZ2 | LYS | 845 | 7.153 | 38.616 | 9.574 |
| 1752 | HZ1 | LYS | 845 | 8.209 | 37.362 | 9.506 |

| | | | | | | |
|---|---|---|---|---|---|---|
| 1753 | HZ3 | LYS | 845 | 6.621 | 37.115 | 9.113 |
| 1754 | N | ARG | 846 | 3.975 | 33.764 | 12.123 |
| 1755 | CA | ARG | 846 | 4.743 | 32.640 | 11.628 |
| 1756 | HN | ARG | 846 | 3.917 | 33.887 | 13.114 |
| 1757 | C | ARG | 846 | 4.039 | 31.621 | 10.739 |
| 1758 | O | ARG | 846 | 3.938 | 31.802 | 9.526 |
| 1759 | CB | ARG | 846 | 5.417 | 31.956 | 12.850 |
| 1760 | CG | ARG | 846 | 6.967 | 31.902 | 12.795 |
| 1761 | CD | ARG | 846 | 7.587 | 33.235 | 12.356 |
| 1762 | NE | ARG | 846 | 7.062 | 34.308 | 13.238 |
| 1763 | CZ | ARG | 846 | 7.378 | 35.593 | 13.160 |
| 1764 | NH1 | ARG | 846 | 8.199 | 36.095 | 12.285 |
| 1765 | 1HH1 | ARG | 846 | 8.584 | 35.395 | 11.650 |
| 1766 | 2HH1 | ARG | 846 | 8.364 | 37.099 | 12.325 |
| 1767 | NH2 | ARG | 846 | 6.834 | 36.391 | 14.008 |
| 1768 | 1HH2 | ARG | 846 | 6.208 | 35.899 | 14.649 |
| 1769 | 2HH2 | ARG | 846 | 7.074 | 37.379 | 13.956 |
| 1770 | HE | ARG | 846 | 6.415 | 34.038 | 13.952 |
| 1771 | N | LYS | 847 | 3.544 | 30.551 | 11.345 |
| 1772 | CA | LYS | 847 | 2.923 | 29.508 | 10.561 |
| 1773 | HN | LYS | 847 | 3.601 | 30.466 | 12.339 |
| 1774 | C | LYS | 847 | 3.910 | 28.361 | 10.659 |
| 1775 | O | LYS | 847 | 4.792 | 28.190 | 9.811 |
| 1776 | CB | LYS | 847 | 2.682 | 29.909 | 9.080 |
| 1777 | CG | LYS | 847 | 2.106 | 28.759 | 8.216 |
| 1778 | CD | LYS | 847 | 1.917 | 29.098 | 6.735 |
| 1779 | CE | LYS | 847 | 1.412 | 27.855 | 5.992 |
| 1780 | NZ | LYS | 847 | 1.065 | 28.219 | 4.607 |
| 1781 | HZ2 | LYS | 847 | 1.724 | 28.915 | 4.239 |
| 1782 | HZ1 | LYS | 847 | 1.127 | 27.409 | 3.982 |
| 1783 | HZ3 | LYS | 847 | 0.136 | 28.589 | 4.582 |
| 1784 | N | ASN | 848 | 3.755 | 27.613 | 11.746 |
| 1785 | CA | ASN | 848 | 4.563 | 26.452 | 12.119 |
| 1786 | HN | ASN | 848 | 3.014 | 27.868 | 12.368 |
| 1787 | C | ASN | 848 | 4.597 | 26.516 | 13.654 |
| 1788 | O | ASN | 848 | 3.550 | 26.710 | 14.274 |
| 1789 | CB | ASN | 848 | 5.977 | 26.531 | 11.523 |
| 1790 | CG | ASN | 848 | 6.207 | 25.526 | 10.393 |
| 1791 | OD1 | ASN | 848 | 7.251 | 24.867 | 10.338 |
| 1792 | ND2 | ASN | 848 | 5.198 | 25.367 | 9.416 |
| 1793 | 1HD2 | ASN | 848 | 5.373 | 24.776 | 8.619 |
| 1794 | 2HD2 | ASN | 848 | 4.431 | 26.024 | 9.406 |
| 1795 | N | PRO | 849 | 5.763 | 26.372 | 14.275 |
| 1796 | CA | PRO | 849 | 5.831 | 26.436 | 15.736 |
| 1797 | C | PRO | 849 | 7.207 | 26.150 | 16.311 |
| 1798 | O | PRO | 849 | 7.451 | 26.398 | 17.495 |
| 1799 | CB | PRO | 849 | 4.738 | 25.524 | 16.313 |
| 1800 | CG | PRO | 849 | 4.725 | 24.323 | 15.354 |
| 1801 | CD | PRO | 849 | 4.966 | 24.963 | 13.985 |
| 1802 | N | THR | 850 | 8.105 | 25.605 | 15.502 |
| 1803 | CA | THR | 850 | 9.441 | 25.356 | 16.017 |
| 1804 | HN | THR | 850 | 7.868 | 25.372 | 14.559 |
| 1805 | C | THR | 850 | 10.214 | 26.642 | 15.778 |
| 1806 | O | THR | 850 | 11.167 | 26.958 | 16.493 |
| 1807 | CB | THR | 850 | 10.126 | 24.154 | 15.283 |

| | | | | | | |
|---|---|---|---|---|---|---|
| 1808 | OG1 | THR | 850 | 10.482 | 24.519 | 13.955 |
| 1809 | HG1 | THR | 850 | 10.956 | 23.767 | 13.590 |
| 1810 | CG2 | THR | 850 | 9.273 | 22.877 | 15.118 |
| 1811 | N | SER | 851 | 9.787 | 27.400 | 14.773 |
| 1812 | CA | SER | 851 | 10.446 | 28.655 | 14.481 |
| 1813 | HN | SER | 851 | 9.010 | 27.103 | 14.218 |
| 1814 | C | SER | 851 | 9.822 | 29.736 | 15.357 |
| 1815 | O | SER | 851 | 10.123 | 30.918 | 15.207 |
| 1816 | CB | SER | 851 | 10.338 | 29.008 | 12.977 |
| 1817 | OG | SER | 851 | 8.993 | 29.270 | 12.561 |
| 1818 | HG | SER | 851 | 8.491 | 28.460 | 12.686 |
| 1819 | N | CYS | 852 | 8.965 | 29.326 | 16.289 |
| 1820 | CA | CYS | 852 | 8.348 | 30.300 | 17.178 |
| 1821 | HN | CYS | 852 | 8.746 | 28.354 | 16.378 |
| 1822 | C | CYS | 852 | 9.238 | 30.709 | 18.333 |
| 1823 | O | CYS | 852 | 9.409 | 31.901 | 18.572 |
| 1824 | CB | CYS | 852 | 6.965 | 29.806 | 17.647 |
| 1825 | SG | CYS | 852 | 5.666 | 30.398 | 16.540 |
| 1826 | HG | CYS | 852 | 5.180 | 31.334 | 17.351 |
| 1827 | N | SER | 853 | 9.809 | 29.754 | 19.059 |
| 1828 | CA | SER | 853 | 10.721 | 30.160 | 20.111 |
| 1829 | HN | SER | 853 | 9.617 | 28.788 | 18.886 |
| 1830 | C | SER | 853 | 11.947 | 30.684 | 19.428 |
| 1831 | O | SER | 853 | 12.640 | 31.546 | 19.946 |
| 1832 | CB | SER | 853 | 11.052 | 28.960 | 21.034 |
| 1833 | OG | SER | 853 | 9.993 | 28.652 | 21.947 |
| 1834 | HG | SER | 853 | 9.878 | 29.413 | 22.522 |
| 1835 | N | ARG | 854 | 12.253 | 30.159 | 18.260 |
| 1836 | CA | ARG | 854 | 13.429 | 30.673 | 17.628 |
| 1837 | HN | ARG | 854 | 11.696 | 29.443 | 17.839 |
| 1838 | C | ARG | 854 | 13.229 | 32.139 | 17.268 |
| 1839 | O | ARG | 854 | 14.183 | 32.906 | 17.281 |
| 1840 | CB | ARG | 854 | 13.792 | 29.826 | 16.377 |
| 1841 | CG | ARG | 854 | 15.162 | 30.165 | 15.733 |
| 1842 | CD | ARG | 854 | 15.371 | 29.457 | 14.388 |
| 1843 | NE | ARG | 854 | 16.798 | 29.601 | 14.002 |
| 1844 | CZ | ARG | 854 | 17.349 | 29.113 | 12.899 |
| 1845 | NH1 | ARG | 854 | 16.705 | 28.438 | 11.993 |
| 1846 | 1HH1 | ARG | 854 | 15.716 | 28.318 | 12.211 |
| 1847 | 2HH1 | ARG | 854 | 17.239 | 28.114 | 11.189 |
| 1848 | NH2 | ARG | 854 | 18.605 | 29.324 | 12.720 |
| 1849 | 1HH2 | ARG | 854 | 19.015 | 29.862 | 13.486 |
| 1850 | 2HH2 | ARG | 854 | 19.036 | 28.950 | 11.876 |
| 1851 | HE | ARG | 854 | 17.393 | 30.108 | 14.626 |
| 1852 | N | ARG | 855 | 11.988 | 32.524 | 16.962 |
| 1853 | CA | ARG | 855 | 11.655 | 33.911 | 16.585 |
| 1854 | HN | ARG | 855 | 11.254 | 31.846 | 16.990 |
| 1855 | C | ARG | 855 | 11.888 | 34.813 | 17.795 |
| 1856 | O | ARG | 855 | 12.635 | 35.796 | 17.748 |
| 1857 | CB | ARG | 855 | 10.184 | 33.966 | 16.157 |
| 1858 | CG | ARG | 855 | 9.698 | 35.261 | 15.556 |
| 1859 | CD | ARG | 855 | 10.351 | 35.566 | 14.227 |
| 1860 | NE | ARG | 855 | 9.768 | 36.777 | 13.671 |
| 1861 | CZ | ARG | 855 | 10.332 | 37.527 | 12.738 |
| 1862 | NH1 | ARG | 855 | 11.508 | 37.098 | 12.207 |

| | | | | | | |
|---|---|---|---|---|---|---|
| 1863 | 1HH1 | ARG | 855 | 11.953 | 37.643 | 11.499 |
| 1864 | 2HH1 | ARG | 855 | 11.913 | 36.244 | 12.526 |
| 1865 | NH2 | ARG | 855 | 9.731 | 38.655 | 12.360 |
| 1866 | 1HH2 | ARG | 855 | 10.147 | 39.221 | 11.648 |
| 1867 | 2HH2 | ARG | 855 | 8.869 | 38.930 | 12.797 |
| 1868 | HE | ARG | 855 | 8.876 | 37.064 | 14.019 |
| 1869 | N | PHE | 856 | 11.215 | 34.449 | 18.875 |
| 1870 | CA | PHE | 856 | 11.305 | 35.136 | 20.138 |
| 1871 | HN | PHE | 856 | 10.612 | 33.655 | 18.811 |
| 1872 | C | PHE | 856 | 12.763 | 35.347 | 20.485 |
| 1873 | O | PHE | 856 | 13.213 | 36.471 | 20.731 |
| 1874 | CB | PHE | 856 | 10.664 | 34.278 | 21.208 |
| 1875 | CG | PHE | 856 | 10.553 | 34.946 | 22.519 |
| 1876 | CD1 | PHE | 856 | 9.941 | 36.188 | 22.618 |
| 1877 | CE1 | PHE | 856 | 9.787 | 36.809 | 23.842 |
| 1878 | CZ | PHE | 856 | 10.246 | 36.185 | 24.997 |
| 1879 | CE2 | PHE | 856 | 10.863 | 34.944 | 24.911 |
| 1880 | CD2 | PHE | 856 | 11.013 | 34.328 | 23.668 |
| 1881 | N | TYR | 857 | 13.497 | 34.247 | 20.513 |
| 1882 | CA | TYR | 857 | 14.903 | 34.289 | 20.828 |
| 1883 | HN | TYR | 857 | 13.067 | 33.367 | 20.311 |
| 1884 | C | TYR | 857 | 15.591 | 35.350 | 19.966 |
| 1885 | O | TYR | 857 | 16.340 | 36.174 | 20.483 |
| 1886 | CB | TYR | 857 | 15.511 | 32.908 | 20.612 |
| 1887 | CG | TYR | 857 | 16.991 | 32.859 | 20.832 |
| 1888 | CD1 | TYR | 857 | 17.532 | 32.721 | 22.110 |
| 1889 | CE1 | TYR | 857 | 18.909 | 32.720 | 22.307 |
| 1890 | CZ | TYR | 857 | 19.746 | 32.857 | 21.210 |
| 1891 | OH | TYR | 857 | 21.112 | 32.882 | 21.399 |
| 1892 | HH | TYR | 857 | 21.529 | 32.623 | 20.569 |
| 1893 | CE2 | TYR | 857 | 19.221 | 32.991 | 19.936 |
| 1894 | CD2 | TYR | 857 | 17.857 | 32.988 | 19.756 |
| 1895 | N | GLN | 858 | 15.322 | 35.361 | 18.668 |
| 1896 | CA | GLN | 858 | 15.949 | 36.355 | 17.800 |
| 1897 | HN | GLN | 858 | 14.689 | 34.689 | 18.283 |
| 1898 | C | GLN | 858 | 15.404 | 37.765 | 18.019 |
| 1899 | O | GLN | 858 | 16.168 | 38.739 | 18.044 |
| 1900 | CB | GLN | 858 | 15.752 | 36.015 | 16.334 |
| 1901 | CG | GLN | 858 | 16.085 | 34.612 | 15.954 |
| 1902 | CD | GLN | 858 | 15.869 | 34.395 | 14.479 |
| 1903 | OE1 | GLN | 858 | 14.743 | 34.153 | 14.025 |
| 1904 | NE2 | GLN | 858 | 17.014 | 34.493 | 13.651 |
| 1905 | 1HE2 | GLN | 858 | 16.916 | 34.308 | 12.664 |
| 1906 | 2HE2 | GLN | 858 | 17.927 | 34.643 | 14.051 |
| 1907 | N | LEU | 859 | 14.087 | 37.895 | 18.144 |
| 1908 | CA | LEU | 859 | 13.550 | 39.228 | 18.350 |
| 1909 | HN | LEU | 859 | 13.484 | 37.099 | 18.097 |
| 1910 | C | LEU | 859 | 14.160 | 39.797 | 19.635 |
| 1911 | O | LEU | 859 | 14.722 | 40.892 | 19.626 |
| 1912 | CB | LEU | 859 | 12.014 | 39.207 | 18.390 |
| 1913 | CG | LEU | 859 | 11.357 | 38.916 | 17.031 |
| 1914 | CD1 | LEU | 859 | 9.853 | 39.037 | 17.129 |
| 1915 | CD2 | LEU | 859 | 11.876 | 39.897 | 15.993 |
| 1916 | N | THR | 860 | 14.098 | 39.033 | 20.723 |
| 1917 | CA | THR | 860 | 14.656 | 39.500 | 21.990 |

| | | | | | | |
|---|---|---|---|---|---|---|
| 1918 | HN | THR | 860 | 13.667 | 38.132 | 20.673 |
| 1919 | C | THR | 860 | 16.176 | 39.693 | 21.962 |
| 1920 | O | THR | 860 | 16.754 | 40.268 | 22.890 |
| 1921 | CB | THR | 860 | 14.288 | 38.558 | 23.132 |
| 1922 | OG1 | THR | 860 | 14.757 | 37.228 | 22.968 |
| 1923 | HG1 | THR | 860 | 14.601 | 36.794 | 23.817 |
| 1924 | CG2 | THR | 860 | 12.779 | 38.552 | 23.335 |
| 1925 | N | LYS | 861 | 16.830 | 39.216 | 20.907 |
| 1926 | CA | LYS | 861 | 18.270 | 39.397 | 20.817 |
| 1927 | HN | LYS | 861 | 16.336 | 38.737 | 20.182 |
| 1928 | C | LYS | 861 | 18.456 | 40.826 | 20.335 |
| 1929 | O | LYS | 861 | 19.325 | 41.553 | 20.810 |
| 1930 | CB | LYS | 861 | 18.892 | 38.425 | 19.817 |
| 1931 | CG | LYS | 861 | 20.364 | 38.109 | 20.110 |
| 1932 | CD | LYS | 861 | 20.502 | 37.119 | 21.279 |
| 1933 | CE | LYS | 861 | 21.959 | 36.933 | 21.747 |
| 1934 | NZ | LYS | 861 | 22.156 | 35.824 | 22.770 |
| 1935 | HZ1 | LYS | 861 | 21.493 | 35.952 | 23.548 |
| 1936 | HZ2 | LYS | 861 | 21.989 | 34.911 | 22.324 |
| 1937 | HZ3 | LYS | 861 | 23.092 | 35.856 | 23.121 |
| 1938 | N | LEU | 862 | 17.603 | 41.223 | 19.396 |
| 1939 | CA | LEU | 862 | 17.635 | 42.565 | 18.843 |
| 1940 | HN | LEU | 862 | 16.918 | 40.577 | 19.059 |
| 1941 | C | LEU | 862 | 17.397 | 43.581 | 19.962 |
| 1942 | O | LEU | 862 | 18.051 | 44.628 | 20.021 |
| 1943 | CB | LEU | 862 | 16.553 | 42.714 | 17.782 |
| 1944 | CG | LEU | 862 | 16.934 | 43.629 | 16.630 |
| 1945 | CD1 | LEU | 862 | 15.660 | 44.226 | 16.085 |
| 1946 | CD2 | LEU | 862 | 17.893 | 44.732 | 17.085 |
| 1947 | N | LEU | 863 | 16.452 | 43.267 | 20.842 |
| 1948 | CA | LEU | 863 | 16.140 | 44.140 | 21.963 |
| 1949 | HN | LEU | 863 | 15.944 | 42.412 | 20.731 |
| 1950 | C | LEU | 863 | 17.379 | 44.403 | 22.790 |
| 1951 | O | LEU | 863 | 17.776 | 45.549 | 22.950 |
| 1952 | CB | LEU | 863 | 15.043 | 43.515 | 22.829 |
| 1953 | CG | LEU | 863 | 13.714 | 43.547 | 22.073 |
| 1954 | CD1 | LEU | 863 | 12.587 | 43.029 | 22.926 |
| 1955 | CD2 | LEU | 863 | 13.447 | 44.988 | 21.639 |
| 1956 | N | ASP | 864 | 17.999 | 43.343 | 23.298 |
| 1957 | CA | ASP | 864 | 19.198 | 43.467 | 24.125 |
| 1958 | HN | ASP | 864 | 17.634 | 42.432 | 23.108 |
| 1959 | C | ASP | 864 | 20.327 | 44.239 | 23.470 |
| 1960 | O | ASP | 864 | 21.122 | 44.895 | 24.145 |
| 1961 | CB | ASP | 864 | 19.733 | 42.092 | 24.495 |
| 1962 | CG | ASP | 864 | 18.831 | 41.367 | 25.445 |
| 1963 | OD1 | ASP | 864 | 17.986 | 42.034 | 26.080 |
| 1964 | OD2 | ASP | 864 | 18.971 | 40.138 | 25.571 |
| 1965 | HD2 | ASP | 864 | 18.343 | 39.811 | 26.197 |
| 1966 | N | SER | 865 | 20.400 | 44.148 | 22.152 |
| 1967 | CA | SER | 865 | 21.458 | 44.813 | 21.411 |
| 1968 | HN | SER | 865 | 19.714 | 43.614 | 21.657 |
| 1969 | C | SER | 865 | 21.136 | 46.275 | 21.164 |
| 1970 | O | SER | 865 | 21.914 | 47.011 | 20.551 |
| 1971 | CB | SER | 865 | 21.676 | 44.093 | 20.087 |
| 1972 | OG | SER | 865 | 21.198 | 44.768 | 18.923 |

| | | | | | | |
|---|---|---|---|---|---|---|
| 1973 | HG | SER | 865 | 20.247 | 44.893 | 19.052 |
| 1974 | N | VAL | 866 | 19.975 | 46.683 | 21.653 |
| 1975 | CA | VAL | 866 | 19.504 | 48.049 | 21.499 |
| 1976 | HN | VAL | 866 | 19.402 | 46.028 | 22.145 |
| 1977 | C | VAL | 866 | 20.218 | 48.908 | 22.537 |
| 1978 | O | VAL | 866 | 20.382 | 50.115 | 22.363 |
| 1979 | CB | VAL | 866 | 17.942 | 48.064 | 21.735 |
| 1980 | CG1 | VAL | 866 | 17.131 | 49.109 | 20.919 |
| 1981 | CG2 | VAL | 866 | 17.252 | 46.706 | 21.460 |
| 1982 | N | GLN | 867 | 20.636 | 48.265 | 23.626 |
| 1983 | CA | GLN | 867 | 21.366 | 48.965 | 24.663 |
| 1984 | HN | GLN | 867 | 20.445 | 47.289 | 23.728 |
| 1985 | C | GLN | 867 | 22.676 | 49.372 | 24.018 |
| 1986 | O | GLN | 867 | 23.069 | 50.525 | 24.094 |
| 1987 | CB | GLN | 867 | 21.611 | 48.048 | 25.882 |
| 1988 | CG | GLN | 867 | 20.397 | 47.844 | 26.848 |
| 1989 | CD | GLN | 867 | 19.936 | 46.419 | 27.177 |
| 1990 | OE1 | GLN | 867 | 18.987 | 46.219 | 27.919 |
| 1991 | NE2 | GLN | 867 | 20.578 | 45.392 | 26.680 |
| 1992 | 2HE2 | GLN | 867 | 21.402 | 45.627 | 26.124 |
| 1993 | 1HE2 | GLN | 867 | 20.246 | 44.480 | 27.000 |
| 1994 | N | PRO | 868 | 23.343 | 48.454 | 23.333 |
| 1995 | CA | PRO | 868 | 24.594 | 48.867 | 22.735 |
| 1996 | C | PRO | 868 | 24.485 | 49.904 | 21.618 |
| 1997 | O | PRO | 868 | 25.405 | 50.714 | 21.471 |
| 1998 | CB | PRO | 868 | 25.417 | 47.634 | 22.332 |
| 1999 | CG | PRO | 868 | 24.369 | 46.674 | 21.747 |
| 2000 | CD | PRO | 868 | 23.150 | 46.894 | 22.646 |
| 2001 | N | ILE | 869 | 23.417 | 49.918 | 20.817 |
| 2002 | CA | ILE | 869 | 23.396 | 50.978 | 19.804 |
| 2003 | HN | ILE | 869 | 22.679 | 49.248 | 20.903 |
| 2004 | C | ILE | 869 | 23.025 | 52.308 | 20.464 |
| 2005 | O | ILE | 869 | 23.534 | 53.352 | 20.062 |
| 2006 | CB | ILE | 869 | 22.493 | 50.676 | 18.539 |
| 2007 | CG1 | ILE | 869 | 21.636 | 49.374 | 18.647 |
| 2008 | CG2 | ILE | 869 | 23.313 | 50.609 | 17.214 |
| 2009 | CD1 | ILE | 869 | 20.519 | 49.215 | 17.595 |
| 2010 | N | ALA | 870 | 22.176 | 52.274 | 21.495 |
| 2011 | CA | ALA | 870 | 21.805 | 53.511 | 22.196 |
| 2012 | HN | ALA | 870 | 21.790 | 51.400 | 21.791 |
| 2013 | C | ALA | 870 | 23.037 | 54.118 | 22.876 |
| 2014 | O | ALA | 870 | 23.216 | 55.331 | 22.884 |
| 2015 | CB | ALA | 870 | 20.651 | 53.247 | 23.179 |
| 2016 | N | ARG | 871 | 23.895 | 53.278 | 23.440 |
| 2017 | CA | ARG | 871 | 25.100 | 53.794 | 24.070 |
| 2018 | HN | ARG | 871 | 23.715 | 52.294 | 23.431 |
| 2019 | C | ARG | 871 | 25.856 | 54.646 | 23.041 |
| 2020 | O | ARG | 871 | 26.083 | 55.831 | 23.258 |
| 2021 | CB | ARG | 871 | 25.980 | 52.625 | 24.594 |
| 2022 | CG | ARG | 871 | 27.191 | 53.058 | 25.463 |
| 2023 | CD | ARG | 871 | 27.897 | 51.866 | 26.122 |
| 2024 | NE | ARG | 871 | 26.863 | 50.904 | 26.580 |
| 2025 | CZ | ARG | 871 | 27.100 | 49.752 | 27.193 |
| 2026 | NH1 | ARG | 871 | 28.286 | 49.306 | 27.482 |
| 2027 | 1HH1 | ARG | 871 | 29.033 | 49.937 | 27.192 |

| | | | | | | |
|---|---|---|---|---|---|---|
| 2028 | 2HH1 | ARG | 871 | 28.340 | 48.405 | 27.955 |
| 2029 | NH2 | ARG | 871 | 26.084 | 49.034 | 27.519 |
| 2030 | 1HH2 | ARG | 871 | 25.203 | 49.473 | 27.247 |
| 2031 | 2HH2 | ARG | 871 | 26.259 | 48.147 | 27.987 |
| 2032 | HE | ARG | 871 | 25.907 | 51.146 | 26.412 |
| 2033 | N | GLU | 872 | 26.408 | 53.950 | 21.931 |
| 2034 | CA | GLU | 872 | 27.385 | 54.484 | 20.981 |
| 2035 | HN | GLU | 872 | 26.260 | 52.944 | 21.910 |
| 2036 | C | GLU | 872 | 26.849 | 55.815 | 20.337 |
| 2037 | O | GLU | 872 | 27.667 | 56.632 | 19.950 |
| 2038 | CB | GLU | 872 | 27.726 | 53.433 | 19.901 |
| 2039 | CG | GLU | 872 | 28.328 | 52.104 | 20.449 |
| 2040 | CD | GLU | 872 | 28.409 | 51.032 | 19.362 |
| 2041 | OE1 | GLU | 872 | 29.430 | 50.652 | 18.829 |
| 2042 | OE2 | GLU | 872 | 27.203 | 50.544 | 18.978 |
| 2043 | HE2 | GLU | 872 | 27.293 | 50.078 | 18.137 |
| 2044 | N | LEU | 873 | 25.436 | 56.022 | 20.276 |
| 2045 | CA | LEU | 873 | 24.724 | 57.200 | 19.760 |
| 2046 | HN | LEU | 873 | 24.861 | 55.285 | 20.690 |
| 2047 | C | LEU | 873 | 24.595 | 58.270 | 20.894 |
| 2048 | O | LEU | 873 | 25.019 | 59.379 | 20.636 |
| 2049 | CB | LEU | 873 | 23.337 | 56.855 | 19.192 |
| 2050 | CG | LEU | 873 | 23.378 | 55.882 | 17.985 |
| 2051 | CD1 | LEU | 873 | 22.002 | 55.231 | 17.841 |
| 2052 | CD2 | LEU | 873 | 23.780 | 56.573 | 16.663 |
| 2053 | N | HIS | 874 | 23.965 | 57.966 | 22.145 |
| 2054 | CA | HIS | 874 | 24.158 | 58.786 | 23.367 |
| 2055 | HN | HIS | 874 | 23.560 | 57.051 | 22.324 |
| 2056 | C | HIS | 874 | 25.542 | 59.472 | 23.379 |
| 2057 | O | HIS | 874 | 25.571 | 60.658 | 23.626 |
| 2058 | CB | HIS | 874 | 24.234 | 58.102 | 24.741 |
| 2059 | CG | HIS | 874 | 22.936 | 57.702 | 25.317 |
| 2060 | ND1 | HIS | 874 | 22.813 | 56.503 | 25.961 |
| 2061 | CE1 | HIS | 874 | 21.722 | 56.586 | 26.613 |
| 2062 | NE2 | HIS | 874 | 20.972 | 57.795 | 26.378 |
| 2063 | CD2 | HIS | 874 | 21.766 | 58.465 | 25.455 |
| 2064 | HE2 | HIS | 874 | 20.081 | 58.130 | 26.741 |
| 2065 | N | GLN | 875 | 26.721 | 58.681 | 23.357 |
| 2066 | CA | GLN | 875 | 28.044 | 59.302 | 23.396 |
| 2067 | HN | GLN | 875 | 26.630 | 57.688 | 23.284 |
| 2068 | C | GLN | 875 | 28.165 | 60.488 | 22.389 |
| 2069 | O | GLN | 875 | 28.635 | 61.533 | 22.815 |
| 2070 | CB | GLN | 875 | 29.172 | 58.270 | 23.174 |
| 2071 | CG | GLN | 875 | 29.403 | 57.239 | 24.328 |
| 2072 | CD | GLN | 875 | 28.824 | 57.531 | 25.718 |
| 2073 | OE1 | GLN | 875 | 28.948 | 56.733 | 26.633 |
| 2074 | NE2 | GLN | 875 | 28.161 | 58.640 | 25.931 |
| 2075 | 2HE2 | GLN | 875 | 28.033 | 59.231 | 25.108 |
| 2076 | 1HE2 | GLN | 875 | 27.754 | 58.726 | 26.864 |
| 2077 | N | PHE | 876 | 27.718 | 60.280 | 21.057 |
| 2078 | CA | PHE | 876 | 27.841 | 61.462 | 20.165 |
| 2079 | HN | PHE | 876 | 26.885 | 59.685 | 20.995 |
| 2080 | C | PHE | 876 | 26.792 | 62.593 | 20.503 |
| 2081 | O | PHE | 876 | 27.169 | 63.758 | 20.556 |
| 2082 | CB | PHE | 876 | 27.834 | 61.063 | 18.696 |

| | | | | | | |
|---|---|---|---|---|---|---|
| 2083 | CG | PHE | 876 | 28.247 | 62.231 | 17.831 |
| 2084 | CD1 | PHE | 876 | 27.246 | 63.136 | 17.335 |
| 2085 | CE1 | PHE | 876 | 27.575 | 64.131 | 16.353 |
| 2086 | CZ | PHE | 876 | 28.931 | 64.264 | 15.911 |
| 2087 | CE2 | PHE | 876 | 29.959 | 63.443 | 16.484 |
| 2088 | CD2 | PHE | 876 | 29.624 | 62.433 | 17.447 |
| 2089 | N | THR | 877 | 25.428 | 62.231 | 20.747 |
| 2090 | CA | THR | 877 | 24.359 | 63.113 | 21.226 |
| 2091 | HN | THR | 877 | 25.102 | 61.272 | 20.752 |
| 2092 | C | THR | 877 | 24.977 | 64.063 | 22.321 |
| 2093 | O | THR | 877 | 24.824 | 65.272 | 22.274 |
| 2094 | CB | THR | 877 | 23.155 | 62.343 | 21.893 |
| 2095 | OG1 | THR | 877 | 22.571 | 61.260 | 21.154 |
| 2096 | HG1 | THR | 877 | 21.647 | 61.493 | 20.956 |
| 2097 | CG2 | THR | 877 | 22.055 | 63.305 | 22.371 |
| 2098 | N | PHE | 878 | 25.576 | 63.410 | 23.433 |
| 2099 | CA | PHE | 878 | 25.950 | 64.048 | 24.711 |
| 2100 | HN | PHE | 878 | 25.863 | 62.435 | 23.355 |
| 2101 | C | PHE | 878 | 27.225 | 64.946 | 24.507 |
| 2102 | O | PHE | 878 | 27.176 | 66.082 | 24.911 |
| 2103 | CB | PHE | 878 | 26.189 | 63.055 | 25.877 |
| 2104 | CG | PHE | 878 | 24.941 | 62.370 | 26.424 |
| 2105 | CD1 | PHE | 878 | 23.710 | 62.202 | 25.690 |
| 2106 | CE1 | PHE | 878 | 22.579 | 61.526 | 26.255 |
| 2107 | CZ | PHE | 878 | 22.652 | 60.972 | 27.569 |
| 2108 | CE2 | PHE | 878 | 23.846 | 61.156 | 28.333 |
| 2109 | CD2 | PHE | 878 | 24.980 | 61.840 | 27.762 |
| 2110 | N | ASP | 879 | 28.404 | 64.415 | 23.941 |
| 2111 | CA | ASP | 879 | 29.580 | 65.087 | 23.411 |
| 2112 | HN | ASP | 879 | 28.357 | 63.418 | 23.880 |
| 2113 | C | ASP | 879 | 29.097 | 66.302 | 22.642 |
| 2114 | O | ASP | 879 | 29.026 | 67.386 | 23.195 |
| 2115 | CB | ASP | 879 | 30.337 | 64.088 | 22.498 |
| 2116 | CG | ASP | 879 | 31.786 | 64.445 | 22.138 |
| 2117 | OD1 | ASP | 879 | 32.533 | 63.682 | 21.542 |
| 2118 | OD2 | ASP | 879 | 32.135 | 65.707 | 22.532 |
| 2119 | HD2 | ASP | 879 | 33.032 | 65.877 | 22.288 |
| 2120 | N | LEU | 880 | 28.746 | 66.177 | 21.254 |
| 2121 | CA | LEU | 880 | 28.095 | 67.195 | 20.392 |
| 2122 | HN | LEU | 880 | 28.531 | 65.227 | 20.984 |
| 2123 | C | LEU | 880 | 27.516 | 68.324 | 21.297 |
| 2124 | O | LEU | 880 | 27.833 | 69.492 | 21.123 |
| 2125 | CB | LEU | 880 | 26.987 | 66.542 | 19.521 |
| 2126 | CG | LEU | 880 | 26.285 | 67.339 | 18.404 |
| 2127 | CD1 | LEU | 880 | 27.309 | 67.731 | 17.314 |
| 2128 | CD2 | LEU | 880 | 25.111 | 66.489 | 17.844 |
| 2129 | N | LEU | 881 | 26.617 | 67.870 | 22.315 |
| 2130 | CA | LEU | 881 | 25.882 | 68.810 | 23.169 |
| 2131 | HN | LEU | 881 | 26.432 | 66.890 | 22.389 |
| 2132 | C | LEU | 881 | 26.754 | 69.636 | 24.119 |
| 2133 | O | LEU | 881 | 26.385 | 70.749 | 24.493 |
| 2134 | CB | LEU | 881 | 24.799 | 68.018 | 23.956 |
| 2135 | CG | LEU | 881 | 23.904 | 68.799 | 24.955 |
| 2136 | CD1 | LEU | 881 | 22.656 | 69.333 | 24.239 |
| 2137 | CD2 | LEU | 881 | 23.483 | 67.949 | 26.165 |

| | | | | | | |
|---|---|---|---|---|---|---|
| 2138 | N | ILE | 882 | 27.893 | 69.086 | 24.527 |
| 2139 | CA | ILE | 882 | 28.793 | 69.797 | 25.426 |
| 2140 | HN | ILE | 882 | 28.136 | 68.169 | 24.212 |
| 2141 | C | ILE | 882 | 29.999 | 70.348 | 24.666 |
| 2142 | O | ILE | 882 | 31.141 | 70.250 | 25.129 |
| 2143 | CB | ILE | 882 | 29.229 | 68.835 | 26.606 |
| 2144 | CG1 | ILE | 882 | 28.050 | 68.094 | 27.312 |
| 2145 | CG2 | ILE | 882 | 30.069 | 69.563 | 27.700 |
| 2146 | CD1 | ILE | 882 | 28.437 | 67.190 | 28.501 |
| 2147 | N | LYS | 883 | 29.736 | 70.910 | 23.488 |
| 2148 | CA | LYS | 883 | 30.771 | 71.515 | 22.651 |
| 2149 | HN | LYS | 883 | 28.791 | 70.918 | 23.162 |
| 2150 | C | LYS | 883 | 30.117 | 72.518 | 21.717 |
| 2151 | O | LYS | 883 | 29.586 | 72.137 | 20.671 |
| 2152 | CB | LYS | 883 | 31.531 | 70.422 | 21.850 |
| 2153 | CG | LYS | 883 | 32.746 | 70.970 | 21.061 |
| 2154 | CD | LYS | 883 | 33.247 | 70.060 | 19.935 |
| 2155 | CE | LYS | 883 | 34.398 | 70.755 | 19.197 |
| 2156 | NZ | LYS | 883 | 34.996 | 69.818 | 18.229 |
| 2157 | HZ2 | LYS | 883 | 34.272 | 69.230 | 17.800 |
| 2158 | HZ1 | LYS | 883 | 35.451 | 70.319 | 17.459 |
| 2159 | HZ3 | LYS | 883 | 35.667 | 69.241 | 18.695 |
| 2160 | N | SER | 884 | 28.420 | 72.393 | 21.414 |
| 2161 | CA | SER | 884 | 27.396 | 72.888 | 20.489 |
| 2162 | HN | SER | 884 | 28.135 | 72.067 | 22.315 |
| 2163 | C | SER | 884 | 27.610 | 74.331 | 20.042 |
| 2164 | O | SER | 884 | 27.438 | 74.658 | 18.872 |
| 2165 | CB | SER | 884 | 26.017 | 72.756 | 21.129 |
| 2166 | OG | SER | 884 | 25.870 | 73.467 | 22.358 |
| 2167 | HG | SER | 884 | 24.943 | 73.377 | 22.616 |
| 2168 | N | HIS | 885 | 27.987 | 75.188 | 20.985 |
| 2169 | CA | HIS | 885 | 28.234 | 76.608 | 20.719 |
| 2170 | HN | HIS | 885 | 28.107 | 74.850 | 21.918 |
| 2171 | C | HIS | 885 | 29.414 | 76.858 | 19.764 |
| 2172 | O | HIS | 885 | 29.555 | 77.954 | 19.228 |
| 2173 | CB | HIS | 885 | 28.426 | 77.340 | 22.060 |
| 2174 | CG | HIS | 885 | 29.180 | 78.638 | 21.969 |
| 2175 | ND1 | HIS | 885 | 28.563 | 79.849 | 21.732 |
| 2176 | CE1 | HIS | 885 | 29.462 | 80.820 | 21.769 |
| 2177 | NE2 | HIS | 885 | 30.643 | 80.282 | 22.016 |
| 2178 | CD2 | HIS | 885 | 30.496 | 78.918 | 22.141 |
| 2179 | HE2 | HIS | 885 | 31.505 | 80.783 | 22.098 |
| 2180 | N | MET | 886 | 31.650 | 74.889 | 20.070 |
| 2181 | CA | MET | 886 | 32.565 | 75.329 | 19.028 |
| 2182 | HN | MET | 886 | 31.962 | 74.238 | 20.761 |
| 2183 | C | MET | 886 | 31.840 | 74.738 | 17.822 |
| 2184 | O | MET | 886 | 31.805 | 75.321 | 16.735 |
| 2185 | CB | MET | 886 | 33.963 | 74.675 | 19.210 |
| 2186 | CG | MET | 886 | 34.261 | 74.102 | 20.611 |
| 2187 | SD | MET | 886 | 36.001 | 73.655 | 20.730 |
| 2188 | CE | MET | 886 | 35.927 | 72.697 | 22.250 |
| 2189 | N | VAL | 887 | 31.241 | 73.571 | 18.051 |
| 2190 | CA | VAL | 887 | 30.508 | 72.855 | 17.019 |
| 2191 | HN | VAL | 887 | 31.297 | 73.172 | 18.966 |
| 2192 | C | VAL | 887 | 29.274 | 73.631 | 16.592 |

| | | | | | | |
|---|---|---|---|---|---|---|
| 2193 | O | VAL | 887 | 28.923 | 73.650 | 15.414 |
| 2194 | CB | VAL | 887 | 30.115 | 71.415 | 17.537 |
| 2195 | CG1 | VAL | 887 | 30.240 | 70.255 | 16.511 |
| 2196 | CG2 | VAL | 887 | 30.919 | 70.953 | 18.776 |
| 2197 | N | SER | 888 | 28.604 | 74.242 | 17.558 |
| 2198 | CA | SER | 888 | 27.445 | 75.073 | 17.258 |
| 2199 | HN | SER | 888 | 28.899 | 74.133 | 18.507 |
| 2200 | C | SER | 888 | 26.109 | 74.426 | 16.874 |
| 2201 | O | SER | 888 | 25.280 | 75.109 | 16.273 |
| 2202 | CB | SER | 888 | 27.808 | 76.071 | 16.151 |
| 2203 | OG | SER | 888 | 28.887 | 76.954 | 16.451 |
| 2204 | HG | SER | 888 | 29.184 | 77.309 | 15.605 |
| 2205 | N | VAL | 889 | 25.838 | 73.039 | 17.163 |
| 2206 | CA | VAL | 889 | 24.556 | 72.431 | 16.670 |
| 2207 | HN | VAL | 889 | 26.322 | 72.541 | 17.906 |
| 2208 | C | VAL | 889 | 23.593 | 72.412 | 17.909 |
| 2209 | O | VAL | 889 | 23.913 | 71.900 | 18.962 |
| 2210 | CB | VAL | 889 | 24.621 | 71.056 | 15.955 |
| 2211 | CG1 | VAL | 889 | 25.677 | 71.023 | 14.826 |
| 2212 | CG2 | VAL | 889 | 24.864 | 69.889 | 16.914 |
| 2213 | N | ASP | 890 | 22.347 | 73.037 | 17.745 |
| 2214 | CA | ASP | 890 | 21.379 | 73.348 | 18.812 |
| 2215 | HN | ASP | 890 | 22.029 | 72.987 | 16.798 |
| 2216 | C | ASP | 890 | 20.246 | 72.324 | 19.082 |
| 2217 | O | ASP | 890 | 19.542 | 71.921 | 18.159 |
| 2218 | CB | ASP | 890 | 20.820 | 74.764 | 18.514 |
| 2219 | CG | ASP | 890 | 21.278 | 75.896 | 19.445 |
| 2220 | OD1 | ASP | 890 | 22.383 | 76.413 | 19.372 |
| 2221 | OD2 | ASP | 890 | 20.313 | 76.270 | 20.338 |
| 2222 | HD2 | ASP | 890 | 20.643 | 76.966 | 20.885 |
| 2223 | N | PHE | 891 | 20.127 | 72.032 | 20.451 |
| 2224 | CA | PHE | 891 | 19.184 | 71.088 | 21.009 |
| 2225 | HN | PHE | 891 | 20.797 | 72.434 | 21.092 |
| 2226 | C | PHE | 891 | 17.936 | 71.983 | 21.374 |
| 2227 | O | PHE | 891 | 18.086 | 73.118 | 21.814 |
| 2228 | CB | PHE | 891 | 19.814 | 70.282 | 22.175 |
| 2229 | CG | PHE | 891 | 21.021 | 69.477 | 21.682 |
| 2230 | CD1 | PHE | 891 | 20.987 | 68.025 | 21.698 |
| 2231 | CE1 | PHE | 891 | 22.129 | 67.235 | 21.275 |
| 2232 | CZ | PHE | 891 | 23.323 | 67.884 | 20.817 |
| 2233 | CE2 | PHE | 891 | 23.373 | 69.314 | 20.772 |
| 2234 | CD2 | PHE | 891 | 22.247 | 70.111 | 21.206 |
| 2235 | N | PRO | 892 | 16.645 | 71.447 | 21.085 |
| 2236 | CA | PRO | 892 | 15.369 | 72.021 | 21.537 |
| 2237 | CD | PRO | 892 | 16.431 | 70.128 | 20.505 |
| 2238 | C | PRO | 892 | 15.213 | 71.856 | 23.072 |
| 2239 | O | PRO | 892 | 15.924 | 71.125 | 23.735 |
| 2240 | CB | PRO | 892 | 14.305 | 71.131 | 20.920 |
| 2241 | CG | PRO | 892 | 14.995 | 69.761 | 20.823 |
| 2242 | N | GLU | 893 | 14.098 | 72.520 | 23.629 |
| 2243 | CA | GLU | 893 | 13.795 | 72.507 | 25.059 |
| 2244 | HN | GLU | 893 | 13.417 | 72.885 | 22.994 |
| 2245 | C | GLU | 893 | 13.842 | 71.099 | 25.612 |
| 2246 | O | GLU | 893 | 14.651 | 70.779 | 26.492 |
| 2247 | CB | GLU | 893 | 12.399 | 73.087 | 25.305 |

| | | | | | | |
|---|---|---|---|---|---|---|
| 2248 | CG | GLU | 893 | 12.359 | 74.589 | 25.235 |
| 2249 | CD | GLU | 893 | 13.300 | 75.215 | 26.243 |
| 2250 | OE1 | GLU | 893 | 14.507 | 74.896 | 26.220 |
| 2251 | OE2 | GLU | 893 | 12.832 | 76.021 | 27.067 |
| 2252 | HE2 | GLU | 893 | 13.523 | 76.332 | 27.631 |
| 2253 | N | MET | 894 | 12.947 | 70.271 | 25.087 |
| 2254 | CA | MET | 894 | 12.861 | 68.895 | 25.507 |
| 2255 | HN | MET | 894 | 12.320 | 70.609 | 24.386 |
| 2256 | C | MET | 894 | 14.249 | 68.252 | 25.562 |
| 2257 | O | MET | 894 | 14.870 | 68.271 | 26.618 |
| 2258 | CB | MET | 894 | 11.912 | 68.116 | 24.584 |
| 2259 | CG | MET | 894 | 10.604 | 67.677 | 25.273 |
| 2260 | SD | MET | 894 | 10.728 | 67.416 | 27.096 |
| 2261 | CE | MET | 894 | 11.156 | 65.740 | 27.227 |
| 2262 | N | MET | 895 | 14.844 | 67.682 | 24.391 |
| 2263 | CA | MET | 895 | 16.108 | 66.917 | 24.435 |
| 2264 | HN | MET | 895 | 14.463 | 67.895 | 23.478 |
| 2265 | C | MET | 895 | 17.168 | 67.650 | 25.267 |
| 2266 | O | MET | 895 | 17.989 | 66.999 | 25.886 |
| 2267 | CB | MET | 895 | 16.757 | 66.650 | 23.073 |
| 2268 | CG | MET | 895 | 15.965 | 65.699 | 22.172 |
| 2269 | SD | MET | 895 | 16.747 | 65.346 | 20.554 |
| 2270 | CE | MET | 895 | 18.397 | 66.112 | 20.684 |
| 2271 | N | ALA | 896 | 17.158 | 69.058 | 25.252 |
| 2272 | CA | ALA | 896 | 18.176 | 69.798 | 25.976 |
| 2273 | HN | ALA | 896 | 16.441 | 69.553 | 24.759 |
| 2274 | C | ALA | 896 | 18.197 | 69.481 | 27.480 |
| 2275 | O | ALA | 896 | 19.277 | 69.320 | 28.049 |
| 2276 | CB | ALA | 896 | 17.986 | 71.297 | 25.751 |
| 2277 | N | GLU | 897 | 17.013 | 69.378 | 28.106 |
| 2278 | CA | GLU | 897 | 16.852 | 69.103 | 29.554 |
| 2279 | HN | GLU | 897 | 16.185 | 69.496 | 27.559 |
| 2280 | C | GLU | 897 | 17.169 | 67.641 | 29.921 |
| 2281 | O | GLU | 897 | 17.828 | 67.362 | 30.929 |
| 2282 | CB | GLU | 897 | 15.399 | 69.460 | 30.000 |
| 2283 | CG | GLU | 897 | 15.155 | 69.773 | 31.529 |
| 2284 | CD | GLU | 897 | 14.687 | 71.232 | 31.813 |
| 2285 | OE1 | GLU | 897 | 15.306 | 72.174 | 31.267 |
| 2286 | OE2 | GLU | 897 | 13.718 | 71.441 | 32.590 |
| 2287 | HE2 | GLU | 897 | 13.561 | 72.371 | 32.656 |
| 2288 | N | ILE | 898 | 16.693 | 66.718 | 29.088 |
| 2289 | CA | ILE | 898 | 16.906 | 65.293 | 29.296 |
| 2290 | HN | ILE | 898 | 16.168 | 67.015 | 28.290 |
| 2291 | C | ILE | 898 | 18.372 | 64.971 | 29.082 |
| 2292 | O | ILE | 898 | 18.963 | 64.230 | 29.866 |
| 2293 | CB | ILE | 898 | 16.065 | 64.484 | 28.325 |
| 2294 | CG2 | ILE | 898 | 16.032 | 63.034 | 28.734 |
| 2295 | CG1 | ILE | 898 | 14.641 | 65.010 | 28.340 |
| 2296 | CD1 | ILE | 898 | 13.785 | 64.322 | 27.349 |
| 2297 | N | ILE | 899 | 19.013 | 65.460 | 27.911 |
| 2298 | CA | ILE | 899 | 20.391 | 65.025 | 27.815 |
| 2299 | HN | ILE | 899 | 18.613 | 66.102 | 27.232 |
| 2300 | C | ILE | 899 | 21.065 | 65.586 | 29.109 |
| 2301 | O | ILE | 899 | 21.557 | 64.777 | 29.867 |
| 2302 | CB | ILE | 899 | 21.118 | 65.405 | 26.543 |

| | | | | | | |
|---|---|---|---|---|---|---|
| 2303 | CG2 | ILE | 899 | 22.558 | 64.893 | 26.717 |
| 2304 | CG1 | ILE | 899 | 20.440 | 64.810 | 25.285 |
| 2305 | CD1 | ILE | 899 | 20.622 | 65.767 | 24.106 |
| 2306 | N | SER | 900 | 21.002 | 67.001 | 29.398 |
| 2307 | CA | SER | 900 | 21.566 | 67.613 | 30.614 |
| 2308 | HN | SER | 900 | 20.553 | 67.602 | 28.736 |
| 2309 | C | SER | 900 | 21.374 | 66.795 | 31.884 |
| 2310 | O | SER | 900 | 22.286 | 66.672 | 32.706 |
| 2311 | CB | SER | 900 | 20.953 | 69.029 | 30.750 |
| 2312 | OG | SER | 900 | 19.545 | 69.053 | 30.493 |
| 2313 | HG | SER | 900 | 19.415 | 68.789 | 29.578 |
| 2314 | N | VAL | 901 | 20.169 | 66.272 | 32.061 |
| 2315 | CA | VAL | 901 | 19.888 | 65.470 | 33.231 |
| 2316 | HN | VAL | 901 | 19.451 | 66.433 | 31.384 |
| 2317 | C | VAL | 901 | 20.678 | 64.169 | 33.202 |
| 2318 | O | VAL | 901 | 21.380 | 63.839 | 34.153 |
| 2319 | CB | VAL | 901 | 18.336 | 65.184 | 33.317 |
| 2320 | CG1 | VAL | 901 | 17.897 | 64.050 | 34.284 |
| 2321 | CG2 | VAL | 901 | 17.492 | 66.416 | 33.722 |
| 2322 | N | GLN | 902 | 20.582 | 63.445 | 32.093 |
| 2323 | CA | GLN | 902 | 21.263 | 62.164 | 31.955 |
| 2324 | HN | GLN | 902 | 20.028 | 63.787 | 31.334 |
| 2325 | C | GLN | 902 | 22.784 | 62.163 | 31.856 |
| 2326 | O | GLN | 902 | 23.421 | 61.208 | 32.299 |
| 2327 | CB | GLN | 902 | 20.717 | 61.415 | 30.737 |
| 2328 | CG | GLN | 902 | 19.252 | 61.040 | 30.828 |
| 2329 | CD | GLN | 902 | 18.940 | 60.208 | 32.056 |
| 2330 | OE1 | GLN | 902 | 19.638 | 59.237 | 32.361 |
| 2331 | NE2 | GLN | 902 | 18.007 | 60.708 | 32.969 |
| 2332 | 1HE2 | GLN | 902 | 17.720 | 60.104 | 33.720 |
| 2333 | 2HE2 | GLN | 902 | 17.531 | 61.588 | 32.810 |
| 2334 | N | VAL | 903 | 23.371 | 63.214 | 31.288 |
| 2335 | CA | VAL | 903 | 24.819 | 63.244 | 31.093 |
| 2336 | HN | VAL | 903 | 22.815 | 63.991 | 30.991 |
| 2337 | C | VAL | 903 | 25.674 | 62.568 | 32.174 |
| 2338 | O | VAL | 903 | 26.442 | 61.655 | 31.866 |
| 2339 | CB | VAL | 903 | 25.318 | 64.719 | 30.822 |
| 2340 | CG1 | VAL | 903 | 24.613 | 65.484 | 29.668 |
| 2341 | CG2 | VAL | 903 | 25.217 | 65.652 | 32.053 |
| 2342 | N | PRO | 904 | 25.556 | 62.991 | 33.449 |
| 2343 | CA | PRO | 904 | 26.383 | 62.331 | 34.468 |
| 2344 | CD | PRO | 904 | 24.668 | 63.995 | 34.066 |
| 2345 | C | PRO | 904 | 26.046 | 60.853 | 34.659 |
| 2346 | O | PRO | 904 | 26.772 | 59.978 | 34.194 |
| 2347 | CB | PRO | 904 | 26.109 | 63.164 | 35.722 |
| 2348 | CG | PRO | 904 | 24.691 | 63.591 | 35.528 |
| 2349 | N | LYS | 905 | 24.927 | 60.589 | 35.323 |
| 2350 | CA | LYS | 905 | 24.462 | 59.233 | 35.620 |
| 2351 | HN | LYS | 905 | 24.371 | 61.358 | 35.638 |
| 2352 | C | LYS | 905 | 24.605 | 58.128 | 34.550 |
| 2353 | O | LYS | 905 | 24.720 | 56.946 | 34.900 |
| 2354 | CB | LYS | 905 | 22.996 | 59.291 | 36.064 |
| 2355 | CG | LYS | 905 | 22.030 | 59.560 | 34.928 |
| 2356 | CD | LYS | 905 | 20.583 | 59.336 | 35.345 |
| 2357 | CE | LYS | 905 | 20.083 | 60.442 | 36.262 |

| | | | | | | |
|---|---|---|---|---|---|---|
| 2358 | NZ | LYS | 905 | 18.618 | 60.349 | 36.653 |
| 2359 | HZ1 | LYS | 905 | 18.227 | 61.297 | 36.749 |
| 2360 | HZ2 | LYS | 905 | 18.100 | 59.837 | 35.925 |
| 2361 | HZ3 | LYS | 905 | 18.535 | 59.864 | 37.523 |
| 2362 | N | ILE | 906 | 24.590 | 58.493 | 33.265 |
| 2363 | CA | ILE | 906 | 24.699 | 57.512 | 32.172 |
| 2364 | HN | ILE | 906 | 24.502 | 59.463 | 33.038 |
| 2365 | C | ILE | 906 | 26.087 | 56.939 | 31.960 |
| 2366 | O | ILE | 906 | 26.376 | 55.800 | 32.329 |
| 2367 | CB | ILE | 906 | 24.127 | 58.174 | 30.852 |
| 2368 | CG1 | ILE | 906 | 22.814 | 57.521 | 30.312 |
| 2369 | CG2 | ILE | 906 | 25.168 | 58.202 | 29.690 |
| 2370 | CD1 | ILE | 906 | 21.527 | 58.350 | 30.491 |
| 2371 | N | LEU | 907 | 26.923 | 57.761 | 31.330 |
| 2372 | CA | LEU | 907 | 28.298 | 57.423 | 30.985 |
| 2373 | HN | LEU | 907 | 26.586 | 58.668 | 31.076 |
| 2374 | C | LEU | 907 | 28.968 | 56.365 | 31.867 |
| 2375 | O | LEU | 907 | 29.557 | 55.412 | 31.351 |
| 2376 | CB | LEU | 907 | 29.155 | 58.720 | 30.925 |
| 2377 | CG | LEU | 907 | 29.319 | 59.549 | 32.227 |
| 2378 | CD1 | LEU | 907 | 30.099 | 60.838 | 31.937 |
| 2379 | CD2 | LEU | 907 | 27.973 | 59.894 | 32.885 |
| 2380 | N | SER | 908 | 28.866 | 56.524 | 33.185 |
| 2381 | CA | SER | 908 | 29.473 | 55.585 | 34.132 |
| 2382 | HN | SER | 908 | 28.358 | 57.309 | 33.540 |
| 2383 | C | SER | 908 | 28.974 | 54.145 | 33.984 |
| 2384 | O | SER | 908 | 29.703 | 53.261 | 33.522 |
| 2385 | CB | SER | 908 | 29.242 | 56.123 | 35.566 |
| 2386 | OG | SER | 908 | 28.881 | 57.509 | 35.589 |
| 2387 | HG | SER | 908 | 29.621 | 58.005 | 35.229 |
| 2388 | N | GLY | 909 | 27.727 | 53.925 | 34.405 |
| 2389 | CA | GLY | 909 | 27.115 | 52.605 | 34.341 |
| 2390 | HN | GLY | 909 | 27.198 | 54.688 | 34.776 |
| 2391 | C | GLY | 909 | 26.426 | 52.237 | 35.647 |
| 2392 | O | GLY | 909 | 26.387 | 51.071 | 36.044 |
| 2393 | N | LYS | 910 | 25.865 | 53.251 | 36.308 |
| 2394 | CA | LYS | 910 | 25.182 | 53.106 | 37.593 |
| 2395 | HN | LYS | 910 | 25.915 | 54.163 | 35.901 |
| 2396 | C | LYS | 910 | 23.655 | 52.955 | 37.392 |
| 2397 | O | LYS | 910 | 22.850 | 53.294 | 38.269 |
| 2398 | CB | LYS | 910 | 25.508 | 54.360 | 38.450 |
| 2399 | CG | LYS | 910 | 26.794 | 55.097 | 37.998 |
| 2400 | CD | LYS | 910 | 26.636 | 56.607 | 37.803 |
| 2401 | CE | LYS | 910 | 27.995 | 57.214 | 37.429 |
| 2402 | NZ | LYS | 910 | 27.817 | 58.633 | 37.073 |
| 2403 | HZ2 | LYS | 910 | 27.105 | 59.070 | 37.670 |
| 2404 | HZ1 | LYS | 910 | 28.680 | 59.166 | 37.222 |
| 2405 | HZ3 | LYS | 910 | 27.542 | 58.704 | 36.114 |
| 2406 | N | VAL | 911 | 23.302 | 52.427 | 36.214 |
| 2407 | CA | VAL | 911 | 21.929 | 52.172 | 35.734 |
| 2408 | HN | VAL | 911 | 24.046 | 52.179 | 35.594 |
| 2409 | C | VAL | 911 | 21.828 | 50.671 | 35.391 |
| 2410 | O | VAL | 911 | 22.809 | 50.084 | 34.932 |
| 2411 | CB | VAL | 911 | 21.666 | 53.060 | 34.454 |
| 2412 | CG1 | VAL | 911 | 21.087 | 54.480 | 34.706 |

| | | | | | | |
|---|---|---|---|---|---|---|
| 2413 | CG2 | VAL | 911 | 22.921 | 53.281 | 33.575 |
| 2414 | N | LYS | 912 | 20.666 | 50.053 | 35.581 |
| 2415 | CA | LYS | 912 | 20.552 | 48.628 | 35.274 |
| 2416 | HN | LYS | 912 | 19.877 | 50.558 | 35.931 |
| 2417 | C | LYS | 912 | 19.777 | 48.267 | 34.014 |
| 2418 | O | LYS | 912 | 18.544 | 48.228 | 34.010 |
| 2419 | CB | LYS | 912 | 19.948 | 47.873 | 36.448 |
| 2420 | CG | LYS | 912 | 19.772 | 46.377 | 36.216 |
| 2421 | CD | LYS | 912 | 18.912 | 45.841 | 37.339 |
| 2422 | CE | LYS | 912 | 18.472 | 44.409 | 37.153 |
| 2423 | NZ | LYS | 912 | 17.517 | 43.918 | 38.231 |
| 2424 | HZ1 | LYS | 912 | 16.988 | 44.716 | 38.611 |
| 2425 | HZ2 | LYS | 912 | 16.862 | 43.235 | 37.825 |
| 2426 | HZ3 | LYS | 912 | 18.038 | 43.483 | 38.965 |
| 2427 | N | PRO | 913 | 20.528 | 47.980 | 32.960 |
| 2428 | CA | PRO | 913 | 19.971 | 47.592 | 31.674 |
| 2429 | C | PRO | 913 | 19.437 | 46.168 | 31.795 |
| 2430 | O | PRO | 913 | 20.208 | 45.247 | 32.038 |
| 2431 | CB | PRO | 913 | 21.081 | 47.729 | 30.622 |
| 2432 | CG | PRO | 913 | 22.370 | 47.474 | 31.418 |
| 2433 | CD | PRO | 913 | 22.090 | 48.144 | 32.766 |
| 2434 | N | ILE | 914 | 18.132 | 45.976 | 31.641 |
| 2435 | CA | ILE | 914 | 17.573 | 44.628 | 31.718 |
| 2436 | HN | ILE | 914 | 17.529 | 46.756 | 31.472 |
| 2437 | C | ILE | 914 | 17.956 | 43.896 | 30.438 |
| 2438 | O | ILE | 914 | 17.995 | 44.499 | 29.370 |
| 2439 | CB | ILE | 914 | 16.003 | 44.733 | 31.889 |
| 2440 | CG1 | ILE | 914 | 15.530 | 45.812 | 32.915 |
| 2441 | CG2 | ILE | 914 | 15.345 | 43.373 | 32.279 |
| 2442 | CD1 | ILE | 914 | 14.007 | 45.901 | 33.144 |
| 2443 | N | TYR | 915 | 18.239 | 42.605 | 30.527 |
| 2444 | CA | TYR | 915 | 18.602 | 41.856 | 29.326 |
| 2445 | HN | TYR | 915 | 18.204 | 42.144 | 31.413 |
| 2446 | C | TYR | 915 | 17.790 | 40.570 | 29.208 |
| 2447 | O | TYR | 915 | 17.364 | 40.025 | 30.218 |
| 2448 | CB | TYR | 915 | 20.110 | 41.472 | 29.342 |
| 2449 | CG | TYR | 915 | 21.114 | 42.616 | 29.160 |
| 2450 | CD1 | TYR | 915 | 22.470 | 42.349 | 28.948 |
| 2451 | CD2 | TYR | 915 | 20.670 | 43.942 | 29.198 |
| 2452 | CE1 | TYR | 915 | 23.368 | 43.398 | 28.768 |
| 2453 | CE2 | TYR | 915 | 21.570 | 44.988 | 29.019 |
| 2454 | CZ | TYR | 915 | 22.919 | 44.715 | 28.804 |
| 2455 | OH | TYR | 915 | 23.803 | 45.741 | 28.625 |
| 2456 | HH | TYR | 915 | 23.322 | 46.569 | 28.679 |
| 2457 | N | PHE | 916 | 17.547 | 40.099 | 27.984 |
| 2458 | CA | PHE | 916 | 16.801 | 38.847 | 27.812 |
| 2459 | HN | PHE | 916 | 17.873 | 40.599 | 27.182 |
| 2460 | C | PHE | 916 | 17.772 | 37.692 | 27.803 |
| 2461 | O | PHE | 916 | 17.483 | 36.618 | 28.320 |
| 2462 | CB | PHE | 916 | 16.012 | 38.831 | 26.509 |
| 2463 | CG | PHE | 916 | 14.782 | 39.658 | 26.558 |
| 2464 | CD1 | PHE | 916 | 13.647 | 39.198 | 27.206 |
| 2465 | CE1 | PHE | 916 | 12.534 | 40.005 | 27.331 |
| 2466 | CZ | PHE | 916 | 12.552 | 41.283 | 26.807 |
| 2467 | CE2 | PHE | 916 | 13.680 | 41.743 | 26.155 |

| | | | | | | |
|---|---|---|---|---|---|---|
| 2468 | CD2 | PHE | 916 | 14.787 | 40.933 | 26.035 |
| 2469 | N | HIS | 917 | 18.929 | 37.930 | 27.198 |
| 2470 | CA | HIS | 917 | 19.985 | 36.938 | 27.104 |
| 2471 | HN | HIS | 917 | 19.079 | 38.830 | 26.789 |
| 2472 | C | HIS | 917 | 21.218 | 37.571 | 27.755 |
| 2473 | O | HIS | 917 | 21.857 | 38.430 | 27.144 |
| 2474 | OXT | HIS | 917 | 22.578 | 37.226 | 28.030 |
| 2475 | CB | HIS | 917 | 20.300 | 36.628 | 25.631 |
| 2476 | CG | HIS | 917 | 19.093 | 36.359 | 24.792 |
| 2477 | ND1 | HIS | 917 | 18.208 | 35.328 | 25.058 |
| 2478 | CE1 | HIS | 917 | 17.249 | 35.325 | 24.154 |
| 2479 | NE2 | HIS | 917 | 17.471 | 36.322 | 23.306 |
| 2480 | CD2 | HIS | 917 | 18.612 | 36.976 | 23.686 |
| 2481 | HE2 | HIS | 917 | 16.900 | 36.556 | 22.520 |
| 2482 | N1 | BIC | 1 | 13.946 | 58.376 | 15.059 |
| 2483 | C2 | BIC | 1 | 14.799 | 59.044 | 15.463 |
| 2484 | C3 | BIC | 1 | 15.841 | 59.881 | 15.953 |
| 2485 | C4 | BIC | 1 | 15.755 | 61.284 | 15.720 |
| 2486 | C5 | BIC | 1 | 14.563 | 61.950 | 15.018 |
| 2487 | F6 | BIC | 1 | 13.473 | 61.888 | 15.833 |
| 2488 | F7 | BIC | 1 | 14.284 | 61.324 | 13.839 |
| 2489 | F8 | BIC | 1 | 14.827 | 63.259 | 14.743 |
| 2490 | C9 | BIC | 1 | 16.847 | 62.050 | 16.204 |
| 2491 | C10 | BIC | 1 | 17.942 | 61.475 | 16.889 |
| 2492 | N11 | BIC | 1 | 18.989 | 62.358 | 17.280 |
| 2493 | C12 | BIC | 1 | 20.087 | 62.115 | 18.161 |
| 2494 | O13 | BIC | 1 | 20.203 | 61.135 | 18.877 |
| 2495 | C14 | BIC | 1 | 21.226 | 63.177 | 18.098 |
| 2496 | C15 | BIC | 1 | 20.663 | 64.593 | 18.310 |
| 2497 | O16 | BIC | 1 | 22.231 | 62.983 | 19.086 |
| 2498 | C17 | BIC | 1 | 21.912 | 63.056 | 16.727 |
| 2499 | S18 | BIC | 1 | 22.550 | 61.414 | 16.461 |
| 2500 | O19 | BIC | 1 | 23.652 | 61.024 | 17.469 |
| 2501 | O20 | BIC | 1 | 21.445 | 60.344 | 16.394 |
| 2502 | C21 | BIC | 1 | 23.155 | 61.723 | 14.866 |
| 2503 | C22 | BIC | 1 | 22.265 | 61.621 | 13.774 |
| 2504 | C23 | BIC | 1 | 22.764 | 61.780 | 12.465 |
| 2505 | C24 | BIC | 1 | 24.122 | 62.129 | 12.302 |
| 2506 | F25 | BIC | 1 | 24.627 | 62.319 | 11.037 |
| 2507 | C26 | BIC | 1 | 24.991 | 62.301 | 13.395 |
| 2508 | C27 | BIC | 1 | 24.507 | 62.085 | 14.702 |
| 2509 | C28 | BIC | 1 | 17.982 | 60.076 | 17.127 |
| 2510 | C29 | BIC | 1 | 16.918 | 59.274 | 16.650 |
| 2511 | H33 | BIC | 1 | 18.992 | 63.281 | 16.873 |
| 2512 | H39 | BIC | 1 | 22.378 | 62.027 | 19.128 |

TABLE 5

ATOMIC COORDINATES OF PR IN COMPLEX WITH RWJ-60130 OBTAINED FROM HOMOLOGY MODELING OF THE CRYSTAL STRUCTURE COORDINATES OF GRα IN COMPLEX WITH FP

| Atom Number | Atom Type | Residue Name | Residue Number | X Coordinate | Y Coordinate | Z Coordinate |
|---|---|---|---|---|---|---|

| | | | | | | |
|---|---|---|---|---|---|---|
| 1 | N | GLN | 682 | -15.010 | 41.258 | 15.638 |
| 2 | HN1 | GLN | 682 | -15.045 | 42.257 | 15.389 |
| 3 | HN2 | GLN | 682 | -14.426 | 40.755 | 14.955 |
| 4 | CA | GLN | 682 | -14.439 | 41.109 | 16.970 |
| 5 | C | GLN | 682 | -14.873 | 42.160 | 18.025 |
| 6 | O | GLN | 682 | -15.047 | 41.805 | 19.197 |
| 7 | CB | GLN | 682 | -12.914 | 41.005 | 16.866 |
| 8 | CG | GLN | 682 | -12.305 | 41.836 | 15.765 |
| 9 | CD | GLN | 682 | -12.397 | 43.302 | 16.071 |
| 10 | OE1 | GLN | 682 | -12.505 | 43.688 | 17.231 |
| 11 | NE2 | GLN | 682 | -12.345 | 44.181 | 14.969 |
| 12 | 1HE2 | GLN | 682 | -12.352 | 45.177 | 15.141 |
| 13 | 2HE2 | GLN | 682 | -12.214 | 43.835 | 14.031 |
| 14 | HN3 | GLN | 682 | -15.936 | 40.879 | 15.627 |
| 15 | N | LEU | 683 | -15.080 | 43.419 | 17.608 |
| 16 | CA | LEU | 683 | -15.510 | 44.539 | 18.490 |
| 17 | HN | LEU | 683 | -14.935 | 43.618 | 16.639 |
| 18 | C | LEU | 683 | -15.065 | 44.431 | 19.944 |
| 19 | O | LEU | 683 | -15.839 | 44.716 | 20.859 |
| 20 | CB | LEU | 683 | -17.038 | 44.695 | 18.484 |
| 21 | CG | LEU | 683 | -17.799 | 45.363 | 17.338 |
| 22 | CD1 | LEU | 683 | -17.383 | 44.762 | 16.011 |
| 23 | CD2 | LEU | 683 | -19.300 | 45.185 | 17.568 |
| 24 | N | ILE | 684 | -13.816 | 44.047 | 20.146 |
| 25 | CA | ILE | 684 | -13.270 | 43.859 | 21.478 |
| 26 | HN | ILE | 684 | -13.227 | 43.881 | 19.355 |
| 27 | C | ILE | 684 | -11.755 | 44.117 | 21.381 |
| 28 | O | ILE | 684 | -11.026 | 43.391 | 20.698 |
| 29 | CB | ILE | 684 | -13.587 | 42.385 | 21.960 |
| 30 | CG1 | ILE | 684 | -13.414 | 42.151 | 23.496 |
| 31 | CG2 | ILE | 684 | -12.746 | 41.308 | 21.208 |
| 32 | CD1 | ILE | 684 | -14.014 | 40.842 | 24.049 |
| 33 | N | PRO | 685 | -11.269 | 45.167 | 22.062 |
| 34 | CA | PRO | 685 | -9.856 | 45.563 | 22.059 |
| 35 | CD | PRO | 685 | -11.987 | 45.849 | 23.154 |
| 36 | C | PRO | 685 | -8.881 | 44.516 | 22.556 |
| 37 | O | PRO | 685 | -9.106 | 43.879 | 23.581 |
| 38 | CB | PRO | 685 | -9.853 | 46.799 | 22.947 |
| 39 | CG | PRO | 685 | -10.854 | 46.430 | 23.987 |
| 40 | N | PRO | 686 | -7.801 | 44.333 | 21.810 |
| 41 | CA | PRO | 686 | -6.772 | 43.402 | 22.220 |
| 42 | C | PRO | 686 | -5.691 | 44.331 | 22.701 |
| 43 | O | PRO | 686 | -5.692 | 45.515 | 22.363 |
| 44 | CB | PRO | 686 | -6.319 | 42.505 | 21.059 |
| 45 | CG | PRO | 686 | -6.674 | 43.324 | 19.808 |
| 46 | CD | PRO | 686 | -8.000 | 43.988 | 20.187 |
| 47 | N | LEU | 687 | -4.769 | 43.820 | 23.494 |
| 48 | CA | LEU | 687 | -3.723 | 44.678 | 24.008 |
| 49 | HN | LEU | 687 | -4.792 | 42.851 | 23.738 |
| 50 | C | LEU | 687 | -2.861 | 45.295 | 22.900 |
| 51 | O | LEU | 687 | -2.512 | 46.478 | 22.951 |
| 52 | CB | LEU | 687 | -2.879 | 43.883 | 24.978 |
| 53 | CG | LEU | 687 | -1.894 | 44.651 | 25.842 |
| 54 | CD1 | LEU | 687 | -2.286 | 46.117 | 26.083 |
| 55 | CD2 | LEU | 687 | -1.842 | 43.877 | 27.129 |

| | | | | | | |
|---|---|---|---|---|---|---|
| 56 | N | ILE | 688 | -2.521 | 44.494 | 21.898 |
| 57 | CA | ILE | 688 | -1.721 | 44.978 | 20.786 |
| 58 | HN | ILE | 688 | -2.821 | 43.540 | 21.909 |
| 59 | C | ILE | 688 | -2.621 | 45.837 | 19.898 |
| 60 | O | ILE | 688 | -2.148 | 46.601 | 19.056 |
| 61 | CB | ILE | 688 | -1.130 | 43.737 | 20.000 |
| 62 | CG1 | ILE | 688 | -2.201 | 42.834 | 19.307 |
| 63 | CG2 | ILE | 688 | -0.234 | 42.826 | 20.895 |
| 64 | CD1 | ILE | 688 | -1.662 | 41.818 | 18.279 |
| 65 | N | ASN | 689 | -3.931 | 45.693 | 20.088 |
| 66 | CA | ASN | 689 | -4.913 | 46.442 | 19.306 |
| 67 | HN | ASN | 689 | -4.252 | 45.054 | 20.786 |
| 68 | C | ASN | 689 | -4.859 | 47.910 | 19.668 |
| 69 | O | ASN | 689 | -4.889 | 48.806 | 18.829 |
| 70 | CB | ASN | 689 | -6.326 | 45.855 | 19.591 |
| 71 | CG | ASN | 689 | -7.519 | 46.578 | 18.956 |
| 72 | OD1 | ASN | 689 | -8.064 | 46.169 | 17.942 |
| 73 | ND2 | ASN | 689 | -7.952 | 47.680 | 19.507 |
| 74 | 1HD2 | ASN | 689 | -8.644 | 48.160 | 18.925 |
| 75 | 2HD2 | ASN | 689 | -7.420 | 48.042 | 20.298 |
| 76 | N | LEU | 690 | -4.799 | 48.126 | 20.963 |
| 77 | CA | LEU | 690 | -4.752 | 49.439 | 21.528 |
| 78 | HN | LEU | 690 | -4.784 | 47.339 | 21.580 |
| 79 | C | LEU | 690 | -3.362 | 50.002 | 21.308 |
| 80 | O | LEU | 690 | -3.176 | 51.209 | 21.154 |
| 81 | CB | LEU | 690 | -5.047 | 49.288 | 22.995 |
| 82 | CG | LEU | 690 | -5.349 | 50.530 | 23.767 |
| 83 | CD1 | LEU | 690 | -6.690 | 50.364 | 24.404 |
| 84 | CD2 | LEU | 690 | -4.307 | 50.719 | 24.805 |
| 85 | N | LEU | 691 | -2.374 | 49.119 | 21.292 |
| 86 | CA | LEU | 691 | -1.001 | 49.552 | 21.090 |
| 87 | HN | LEU | 691 | -2.574 | 48.147 | 21.419 |
| 88 | C | LEU | 691 | -0.869 | 50.120 | 19.665 |
| 89 | O | LEU | 691 | -0.131 | 51.073 | 19.421 |
| 90 | CB | LEU | 691 | -0.068 | 48.363 | 21.316 |
| 91 | CG | LEU | 691 | 1.250 | 48.527 | 22.075 |
| 92 | CD1 | LEU | 691 | 1.095 | 49.268 | 23.390 |
| 93 | CD2 | LEU | 691 | 1.764 | 47.137 | 22.328 |
| 94 | N | MET | 692 | -1.606 | 49.544 | 18.727 |
| 95 | CA | MET | 692 | -1.547 | 50.045 | 17.376 |
| 96 | HN | MET | 692 | -2.197 | 48.770 | 18.954 |
| 97 | C | MET | 692 | -2.330 | 51.347 | 17.200 |
| 98 | O | MET | 692 | -1.845 | 52.246 | 16.524 |
| 99 | CB | MET | 692 | -2.049 | 48.959 | 16.384 |
| 100 | CG | MET | 692 | -2.206 | 49.409 | 14.916 |
| 101 | SD | MET | 692 | -2.628 | 47.993 | 13.887 |
| 102 | CE | MET | 692 | -2.810 | 48.850 | 12.317 |
| 103 | N | SER | 693 | -3.518 | 51.484 | 17.797 |
| 104 | CA | SER | 693 | -4.251 | 52.738 | 17.584 |
| 105 | HN | SER | 693 | -3.895 | 50.756 | 18.370 |
| 106 | C | SER | 693 | -3.645 | 53.964 | 18.253 |
| 107 | O | SER | 693 | -4.043 | 55.082 | 17.931 |
| 108 | CB | SER | 693 | -5.755 | 52.588 | 17.923 |
| 109 | OG | SER | 693 | -6.254 | 51.270 | 17.670 |
| 110 | HG | SER | 693 | -6.172 | 51.109 | 16.726 |

| | | | | | | |
|---|---|---|---|---|---|---|
| 111 | N | ILE | 694 | -2.694 | 53.786 | 19.173 |
| 112 | CA | ILE | 694 | -2.079 | 54.944 | 19.831 |
| 113 | HN | ILE | 694 | -2.400 | 52.861 | 19.414 |
| 114 | C | ILE | 694 | -0.672 | 55.231 | 19.325 |
| 115 | O | ILE | 694 | 0.069 | 56.001 | 19.939 |
| 116 | CB | ILE | 694 | -1.980 | 54.783 | 21.367 |
| 117 | CG2 | ILE | 694 | -3.269 | 54.216 | 21.925 |
| 118 | CG1 | ILE | 694 | -0.801 | 53.879 | 21.725 |
| 119 | CD1 | ILE | 694 | -0.570 | 53.751 | 23.208 |
| 120 | N | GLU | 695 | -0.301 | 54.606 | 18.214 |
| 121 | CA | GLU | 695 | 1.020 | 54.801 | 17.640 |
| 122 | HN | GLU | 695 | -0.944 | 53.988 | 17.762 |
| 123 | C | GLU | 695 | 1.093 | 56.227 | 17.144 |
| 124 | O | GLU | 695 | 0.148 | 56.718 | 16.543 |
| 125 | CB | GLU | 695 | 1.238 | 53.825 | 16.483 |
| 126 | CG | GLU | 695 | 2.643 | 53.807 | 15.879 |
| 127 | CD | GLU | 695 | 3.760 | 53.831 | 16.920 |
| 128 | OE1 | GLU | 695 | 3.570 | 53.258 | 18.018 |
| 129 | OE2 | GLU | 695 | 4.832 | 54.413 | 16.631 |
| 130 | HE2 | GLU | 695 | 5.431 | 54.352 | 17.360 |
| 131 | N | PRO | 696 | 2.175 | 56.936 | 17.458 |
| 132 | CA | PRO | 696 | 2.104 | 58.288 | 16.900 |
| 133 | CD | PRO | 696 | 2.935 | 56.915 | 18.715 |
| 134 | C | PRO | 696 | 2.205 | 58.244 | 15.379 |
| 135 | O | PRO | 696 | 2.532 | 57.209 | 14.789 |
| 136 | CB | PRO | 696 | 3.278 | 59.025 | 17.552 |
| 137 | CG | PRO | 696 | 3.904 | 58.034 | 18.527 |
| 138 | N | ASP | 697 | 1.909 | 59.358 | 14.734 |
| 139 | CA | ASP | 697 | 1.979 | 59.373 | 13.291 |
| 140 | HN | ASP | 697 | 1.640 | 60.179 | 15.237 |
| 141 | C | ASP | 697 | 3.198 | 60.165 | 12.862 |
| 142 | O | ASP | 697 | 3.578 | 61.123 | 13.536 |
| 143 | CB | ASP | 697 | 0.666 | 59.986 | 12.735 |
| 144 | CG | ASP | 697 | 0.355 | 61.433 | 13.143 |
| 145 | OD1 | ASP | 697 | -0.395 | 62.158 | 12.506 |
| 146 | OD2 | ASP | 697 | 0.984 | 61.806 | 14.298 |
| 147 | HD2 | ASP | 697 | 0.761 | 62.700 | 14.504 |
| 148 | N | VAL | 698 | 3.807 | 59.769 | 11.747 |
| 149 | CA | VAL | 698 | 5.012 | 60.440 | 11.267 |
| 150 | HN | VAL | 698 | 3.433 | 58.999 | 11.229 |
| 151 | C | VAL | 698 | 4.883 | 61.937 | 11.156 |
| 152 | O | VAL | 698 | 3.915 | 62.458 | 10.611 |
| 153 | CB | VAL | 698 | 5.466 | 59.926 | 9.887 |
| 154 | CG1 | VAL | 698 | 5.970 | 58.504 | 10.001 |
| 155 | CG2 | VAL | 698 | 4.322 | 60.031 | 8.885 |
| 156 | N | ILE | 699 | 5.876 | 62.639 | 11.673 |
| 157 | CA | ILE | 699 | 5.854 | 64.080 | 11.590 |
| 158 | HN | ILE | 699 | 6.640 | 62.174 | 12.120 |
| 159 | C | ILE | 699 | 7.097 | 64.568 | 10.856 |
| 160 | O | ILE | 699 | 8.225 | 64.160 | 11.169 |
| 161 | CB | ILE | 699 | 5.736 | 64.714 | 13.036 |
| 162 | CG1 | ILE | 699 | 6.895 | 64.330 | 14.012 |
| 163 | CG2 | ILE | 699 | 4.383 | 64.379 | 13.735 |
| 164 | CD1 | ILE | 699 | 6.826 | 64.960 | 15.418 |
| 165 | N | TYR | 700 | 6.859 | 65.427 | 9.863 |

| | | | | | | |
|---|---|---|---|---|---|---|
| 166 | CA | TYR | 700 | 7.899 | 65.995 | 9.007 |
| 167 | HN | TYR | 700 | 5.912 | 65.698 | 9.695 |
| 168 | C | TYR | 700 | 8.844 | 66.938 | 9.690 |
| 169 | O | TYR | 700 | 8.559 | 67.480 | 10.759 |
| 170 | CB | TYR | 700 | 7.285 | 66.703 | 7.797 |
| 171 | CG | TYR | 700 | 6.627 | 65.742 | 6.847 |
| 172 | CD1 | TYR | 700 | 5.329 | 65.966 | 6.401 |
| 173 | CE1 | TYR | 700 | 4.665 | 65.026 | 5.623 |
| 174 | CZ | TYR | 700 | 5.300 | 63.843 | 5.279 |
| 175 | OH | TYR | 700 | 4.635 | 62.900 | 4.526 |
| 176 | HH | TYR | 700 | 5.069 | 62.049 | 4.659 |
| 177 | CE2 | TYR | 700 | 6.608 | 63.600 | 5.697 |
| 178 | CD2 | TYR | 700 | 7.262 | 64.551 | 6.478 |
| 179 | N | ALA | 701 | 9.971 | 67.152 | 9.023 |
| 180 | CA | ALA | 701 | 11.029 | 67.995 | 9.539 |
| 181 | HN | ALA | 701 | 10.094 | 66.714 | 8.133 |
| 182 | C | ALA | 701 | 10.957 | 69.476 | 9.185 |
| 183 | O | ALA | 701 | 11.645 | 70.282 | 9.806 |
| 184 | CB | ALA | 701 | 12.359 | 67.437 | 9.100 |
| 185 | N | GLY | 702 | 10.142 | 69.846 | 8.202 |
| 186 | CA | GLY | 702 | 10.071 | 71.249 | 7.828 |
| 187 | HN | GLY | 702 | 9.584 | 69.167 | 7.725 |
| 188 | C | GLY | 702 | 11.445 | 71.762 | 7.423 |
| 189 | O | GLY | 702 | 11.889 | 72.816 | 7.879 |
| 190 | N | HIS | 703 | 12.131 | 71.003 | 6.571 |
| 191 | CA | HIS | 703 | 13.464 | 71.376 | 6.102 |
| 192 | HN | HIS | 703 | 11.722 | 70.153 | 6.241 |
| 193 | C | HIS | 703 | 13.342 | 72.135 | 4.797 |
| 194 | O | HIS | 703 | 12.377 | 71.977 | 4.040 |
| 195 | CB | HIS | 703 | 14.316 | 70.111 | 5.907 |
| 196 | CG | HIS | 703 | 15.770 | 70.427 | 5.713 |
| 197 | ND1 | HIS | 703 | 16.648 | 70.863 | 6.702 |
| 198 | CE1 | HIS | 703 | 17.810 | 70.793 | 6.025 |
| 199 | NE2 | HIS | 703 | 17.774 | 70.374 | 4.731 |
| 200 | CD2 | HIS | 703 | 16.439 | 70.135 | 4.532 |
| 201 | HE2 | HIS | 703 | 18.550 | 70.259 | 4.064 |
| 202 | N | ASP | 704 | 14.333 | 72.973 | 4.559 |
| 203 | CA | ASP | 704 | 14.410 | 73.739 | 3.341 |
| 204 | HN | ASP | 704 | 15.053 | 73.080 | 5.244 |
| 205 | C | ASP | 704 | 15.539 | 73.046 | 2.609 |
| 206 | O | ASP | 704 | 16.712 | 73.253 | 2.928 |
| 207 | CB | ASP | 704 | 14.801 | 75.179 | 3.628 |
| 208 | CG | ASP | 704 | 14.786 | 76.022 | 2.386 |
| 209 | OD1 | ASP | 704 | 14.680 | 75.431 | 1.294 |
| 210 | OD2 | ASP | 704 | 14.881 | 77.259 | 2.497 |
| 211 | HD2 | ASP | 704 | 14.858 | 77.655 | 1.639 |
| 212 | N | ASN | 705 | 15.184 | 72.196 | 1.658 |
| 213 | CA | ASN | 705 | 16.169 | 71.457 | 0.886 |
| 214 | HN | ASN | 705 | 14.212 | 72.058 | 1.467 |
| 215 | C | ASN | 705 | 16.414 | 72.208 | -0.411 |
| 216 | O | ASN | 705 | 17.014 | 71.684 | -1.351 |
| 217 | CB | ASN | 705 | 15.643 | 70.020 | 0.600 |
| 218 | CG | ASN | 705 | 14.332 | 69.904 | -0.185 |
| 219 | OD1 | ASN | 705 | 14.308 | 69.649 | -1.380 |
| 220 | ND2 | ASN | 705 | 13.204 | 70.107 | 0.440 |

| | | | | | | |
|---|---|---|---|---|---|---|
| 221 | 1HD2 | ASN | 705 | 12.407 | 70.157 | -0.200 |
| 222 | 2HD2 | ASN | 705 | 13.262 | 70.406 | 1.414 |
| 223 | N | THR | 706 | 15.935 | 73.449 | -0.435 |
| 224 | CA | THR | 706 | 16.057 | 74.325 | -1.592 |
| 225 | HN | THR | 706 | 15.470 | 73.797 | 0.379 |
| 226 | C | THR | 706 | 17.406 | 74.995 | -1.581 |
| 227 | O | THR | 706 | 17.708 | 75.846 | -2.418 |
| 228 | CB | THR | 706 | 14.910 | 75.390 | -1.546 |
| 229 | OG1 | THR | 706 | 15.333 | 76.547 | -0.837 |
| 230 | HG1 | THR | 706 | 14.613 | 77.181 | -0.907 |
| 231 | CG2 | THR | 706 | 13.605 | 74.960 | -0.840 |
| 232 | N | LYS | 707 | 18.223 | 74.651 | -0.605 |
| 233 | CA | LYS | 707 | 19.517 | 75.267 | -0.548 |
| 234 | HN | LYS | 707 | 17.947 | 73.976 | 0.079 |
| 235 | C | LYS | 707 | 20.441 | 74.218 | 0.032 |
| 236 | O | LYS | 707 | 20.052 | 73.480 | 0.945 |
| 237 | CB | LYS | 707 | 19.445 | 76.516 | 0.374 |
| 238 | CG | LYS | 707 | 18.097 | 77.274 | 0.280 |
| 239 | CD | LYS | 707 | 18.015 | 78.548 | 1.126 |
| 240 | CE | LYS | 707 | 16.663 | 79.230 | 0.883 |
| 241 | NZ | LYS | 707 | 16.507 | 80.357 | 1.819 |
| 242 | HZ2 | LYS | 707 | 17.405 | 80.833 | 1.966 |
| 243 | HZ1 | LYS | 707 | 15.867 | 81.066 | 1.446 |
| 244 | HZ3 | LYS | 707 | 16.158 | 80.019 | 2.693 |
| 245 | N | PRO | 708 | 21.688 | 74.161 | -0.473 |
| 246 | CA | PRO | 708 | 22.654 | 73.163 | -0.016 |
| 247 | CD | PRO | 708 | 22.365 | 75.246 | -1.206 |
| 248 | C | PRO | 708 | 22.661 | 72.720 | 1.398 |
| 249 | O | PRO | 708 | 22.222 | 73.405 | 2.316 |
| 250 | CB | PRO | 708 | 24.018 | 73.697 | -0.465 |
| 251 | CG | PRO | 708 | 23.812 | 75.102 | -0.777 |
| 252 | N | ASP | 709 | 23.175 | 71.516 | 1.550 |
| 253 | CA | ASP | 709 | 23.249 | 70.949 | 2.849 |
| 254 | HN | ASP | 709 | 23.511 | 71.006 | 0.757 |
| 255 | C | ASP | 709 | 24.550 | 71.260 | 3.463 |
| 256 | O | ASP | 709 | 25.441 | 71.872 | 2.877 |
| 257 | CB | ASP | 709 | 23.091 | 69.450 | 2.784 |
| 258 | CG | ASP | 709 | 21.834 | 69.063 | 2.108 |
| 259 | OD1 | ASP | 709 | 20.895 | 69.889 | 2.139 |
| 260 | OD2 | ASP | 709 | 21.777 | 67.951 | 1.553 |
| 261 | HD2 | ASP | 709 | 20.924 | 67.836 | 1.164 |
| 262 | N | THR | 710 | 24.636 | 70.819 | 4.690 |
| 263 | CA | THR | 710 | 25.823 | 70.973 | 5.447 |
| 264 | HN | THR | 710 | 23.847 | 70.365 | 5.104 |
| 265 | C | THR | 710 | 25.383 | 70.258 | 6.675 |
| 266 | O | THR | 710 | 24.204 | 70.267 | 7.041 |
| 267 | CB | THR | 710 | 26.132 | 72.475 | 5.761 |
| 268 | OG1 | THR | 710 | 25.356 | 72.923 | 6.865 |
| 269 | HG1 | THR | 710 | 25.645 | 73.822 | 7.048 |
| 270 | CG2 | THR | 710 | 25.824 | 73.487 | 4.637 |
| 271 | N | SER | 711 | 26.329 | 69.578 | 7.289 |
| 272 | CA | SER | 711 | 25.985 | 68.820 | 8.444 |
| 273 | HN | SER | 711 | 27.270 | 69.593 | 6.950 |
| 274 | C | SER | 711 | 25.348 | 69.652 | 9.408 |
| 275 | O | SER | 711 | 24.169 | 69.484 | 9.541 |

| | | | | | | |
|---|---|---|---|---|---|---|
| 276 | CB | SER | 711 | 27.252 | 68.144 | 9.023 |
| 277 | OG | SER | 711 | 28.080 | 67.556 | 8.013 |
| 278 | HG | SER | 711 | 27.569 | 66.859 | 7.593 |
| 279 | N | SER | 712 | 26.114 | 70.494 | 10.112 |
| 280 | CA | SER | 712 | 25.527 | 71.392 | 11.074 |
| 281 | HN | SER | 712 | 27.104 | 70.500 | 9.969 |
| 282 | C | SER | 712 | 24.166 | 71.500 | 10.449 |
| 283 | O | SER | 712 | 23.511 | 70.508 | 10.391 |
| 284 | CB | SER | 712 | 26.267 | 72.753 | 11.066 |
| 285 | OG | SER | 712 | 27.583 | 72.674 | 11.625 |
| 286 | HG | SER | 712 | 27.489 | 72.421 | 12.547 |
| 287 | N | SER | 713 | 23.678 | 72.598 | 9.944 |
| 288 | CA | SER | 713 | 22.339 | 72.472 | 9.371 |
| 289 | HN | SER | 713 | 24.182 | 73.462 | 9.948 |
| 290 | C | SER | 713 | 21.406 | 71.259 | 9.586 |
| 291 | O | SER | 713 | 20.256 | 71.418 | 9.959 |
| 292 | CB | SER | 713 | 22.505 | 72.804 | 7.867 |
| 293 | OG | SER | 713 | 21.363 | 73.468 | 7.314 |
| 294 | HG | SER | 713 | 20.620 | 72.862 | 7.377 |
| 295 | N | LEU | 714 | 21.874 | 70.055 | 9.313 |
| 296 | CA | LEU | 714 | 21.017 | 68.898 | 9.427 |
| 297 | HN | LEU | 714 | 22.826 | 69.943 | 9.027 |
| 298 | C | LEU | 714 | 20.938 | 68.217 | 10.741 |
| 299 | O | LEU | 714 | 19.956 | 67.556 | 11.097 |
| 300 | CB | LEU | 714 | 21.502 | 67.918 | 8.321 |
| 301 | CG | LEU | 714 | 21.248 | 68.304 | 6.839 |
| 302 | CD1 | LEU | 714 | 21.363 | 67.062 | 5.943 |
| 303 | CD2 | LEU | 714 | 19.880 | 68.972 | 6.625 |
| 304 | N | LEU | 715 | 22.058 | 68.261 | 11.408 |
| 305 | CA | LEU | 715 | 22.089 | 67.687 | 12.697 |
| 306 | HN | LEU | 715 | 22.873 | 68.690 | 11.016 |
| 307 | C | LEU | 715 | 21.204 | 68.642 | 13.356 |
| 308 | O | LEU | 715 | 20.386 | 68.325 | 14.216 |
| 309 | CB | LEU | 715 | 23.540 | 67.807 | 13.243 |
| 310 | CG | LEU | 715 | 24.610 | 66.823 | 12.697 |
| 311 | CD1 | LEU | 715 | 25.926 | 66.993 | 13.467 |
| 312 | CD2 | LEU | 715 | 24.157 | 65.355 | 12.764 |
| 313 | N | THR | 716 | 21.348 | 69.874 | 12.942 |
| 314 | CA | THR | 716 | 20.571 | 70.736 | 13.707 |
| 315 | HN | THR | 716 | 21.934 | 70.157 | 12.182 |
| 316 | C | THR | 716 | 19.114 | 70.479 | 13.542 |
| 317 | O | THR | 716 | 18.437 | 70.227 | 14.534 |
| 318 | CB | THR | 716 | 20.967 | 72.126 | 13.506 |
| 319 | OG1 | THR | 716 | 21.793 | 72.724 | 14.509 |
| 320 | HG1 | THR | 716 | 22.011 | 73.607 | 14.181 |
| 321 | CG2 | THR | 716 | 19.768 | 72.979 | 13.458 |
| 322 | N | SER | 717 | 18.638 | 70.483 | 12.305 |
| 323 | CA | SER | 717 | 17.243 | 70.208 | 12.067 |
| 324 | HN | SER | 717 | 19.248 | 70.677 | 11.536 |
| 325 | C | SER | 717 | 16.872 | 68.910 | 12.817 |
| 326 | O | SER | 717 | 15.856 | 68.912 | 13.493 |
| 327 | CB | SER | 717 | 16.926 | 70.103 | 10.554 |
| 328 | OG | SER | 717 | 17.601 | 69.011 | 9.919 |
| 329 | HG | SER | 717 | 18.545 | 69.178 | 9.987 |
| 330 | N | LEU | 718 | 17.691 | 67.844 | 12.756 |

| | | | | | | |
|---|---|---|---|---|---|---|
| 331 | CA | LEU | 718 | 17.371 | 66.544 | 13.424 |
| 332 | HN | LEU | 718 | 18.546 | 67.924 | 12.244 |
| 333 | C | LEU | 718 | 17.026 | 66.582 | 14.903 |
| 334 | O | LEU | 718 | 16.107 | 65.898 | 15.360 |
| 335 | CB | LEU | 718 | 18.454 | 65.464 | 13.111 |
| 336 | CG | LEU | 718 | 17.916 | 64.771 | 11.843 |
| 337 | CD1 | LEU | 718 | 16.946 | 65.767 | 11.273 |
| 338 | CD2 | LEU | 718 | 18.930 | 64.423 | 10.767 |
| 339 | N | ASN | 719 | 17.745 | 67.412 | 15.634 |
| 340 | CA | ASN | 719 | 17.523 | 67.628 | 17.058 |
| 341 | HN | ASN | 719 | 18.481 | 67.921 | 15.188 |
| 342 | C | ASN | 719 | 16.166 | 68.279 | 17.320 |
| 343 | O | ASN | 719 | 15.458 | 67.918 | 18.260 |
| 344 | CB | ASN | 719 | 18.614 | 68.525 | 17.580 |
| 345 | CG | ASN | 719 | 19.895 | 67.790 | 17.829 |
| 346 | OD1 | ASN | 719 | 20.167 | 66.736 | 17.265 |
| 347 | ND2 | ASN | 719 | 20.913 | 68.674 | 18.216 |
| 348 | 1HD2 | ASN | 719 | 21.861 | 68.327 | 18.261 |
| 349 | 2HD2 | ASN | 719 | 20.709 | 69.592 | 18.580 |
| 350 | N | GLN | 720 | 15.824 | 69.277 | 16.515 |
| 351 | CA | GLN | 720 | 14.531 | 69.918 | 16.671 |
| 352 | HN | GLN | 720 | 16.455 | 69.586 | 15.803 |
| 353 | C | GLN | 720 | 13.553 | 68.764 | 16.493 |
| 354 | O | GLN | 720 | 12.674 | 68.521 | 17.327 |
| 355 | CB | GLN | 720 | 14.336 | 70.984 | 15.571 |
| 356 | CG | GLN | 720 | 14.185 | 72.462 | 16.060 |
| 357 | CD | GLN | 720 | 15.330 | 73.109 | 16.849 |
| 358 | OE1 | GLN | 720 | 15.113 | 73.748 | 17.867 |
| 359 | NE2 | GLN | 720 | 16.562 | 73.003 | 16.420 |
| 360 | 2HE2 | GLN | 720 | 16.667 | 72.516 | 15.528 |
| 361 | 1HE2 | GLN | 720 | 17.255 | 73.517 | 16.966 |
| 362 | N | LEU | 721 | 13.738 | 68.029 | 15.402 |
| 363 | CA | LEU | 721 | 12.865 | 66.914 | 15.121 |
| 364 | HN | LEU | 721 | 14.483 | 68.250 | 14.773 |
| 365 | C | LEU | 721 | 12.721 | 65.969 | 16.297 |
| 366 | O | LEU | 721 | 11.628 | 65.467 | 16.561 |
| 367 | CB | LEU | 721 | 13.353 | 66.111 | 13.921 |
| 368 | CG | LEU | 721 | 12.281 | 65.040 | 13.743 |
| 369 | CD1 | LEU | 721 | 10.943 | 65.750 | 13.669 |
| 370 | CD2 | LEU | 721 | 12.512 | 64.192 | 12.523 |
| 371 | N | GLY | 722 | 13.828 | 65.713 | 16.988 |
| 372 | CA | GLY | 722 | 13.811 | 64.809 | 18.123 |
| 373 | HN | GLY | 722 | 14.687 | 66.151 | 16.721 |
| 374 | C | GLY | 722 | 12.951 | 65.353 | 19.242 |
| 375 | O | GLY | 722 | 12.220 | 64.609 | 19.904 |
| 376 | N | GLU | 723 | 13.057 | 66.663 | 19.443 |
| 377 | CA | GLU | 723 | 12.278 | 67.325 | 20.462 |
| 378 | HN | GLU | 723 | 13.687 | 67.198 | 18.879 |
| 379 | C | GLU | 723 | 10.824 | 67.093 | 20.146 |
| 380 | O | GLU | 723 | 10.057 | 66.625 | 20.979 |
| 381 | CB | GLU | 723 | 12.612 | 68.843 | 20.496 |
| 382 | CG | GLU | 723 | 12.287 | 69.677 | 19.213 |
| 383 | CD | GLU | 723 | 12.698 | 71.151 | 19.181 |
| 384 | OE1 | GLU | 723 | 12.514 | 71.873 | 18.210 |
| 385 | OE2 | GLU | 723 | 13.285 | 71.582 | 20.332 |

| | | | | | | |
|---|---|---|---|---|---|---|
| 386 | HE2 | GLU | 723 | 13.514 | 72.495 | 20.248 |
| 387 | N | ARG | 724 | 10.411 | 67.381 | 18.930 |
| 388 | CA | ARG | 724 | 9.016 | 67.165 | 18.680 |
| 389 | HN | ARG | 724 | 11.031 | 67.728 | 18.226 |
| 390 | C | ARG | 724 | 8.576 | 65.706 | 18.864 |
| 391 | O | ARG | 724 | 7.454 | 65.462 | 19.302 |
| 392 | CB | ARG | 724 | 8.675 | 67.723 | 17.311 |
| 393 | CG | ARG | 724 | 9.113 | 69.186 | 17.218 |
| 394 | CD | ARG | 724 | 8.843 | 69.724 | 15.849 |
| 395 | NE | ARG | 724 | 7.501 | 69.334 | 15.445 |
| 396 | CZ | ARG | 724 | 7.222 | 68.779 | 14.275 |
| 397 | NH1 | ARG | 724 | 8.173 | 68.495 | 13.340 |
| 398 | 1HH1 | ARG | 724 | 7.915 | 68.079 | 12.463 |
| 399 | 2HH1 | ARG | 724 | 9.132 | 68.701 | 13.519 |
| 400 | NH2 | ARG | 724 | 5.922 | 68.492 | 13.989 |
| 401 | 1HH2 | ARG | 724 | 5.691 | 68.079 | 13.109 |
| 402 | 2HH2 | ARG | 724 | 5.210 | 68.697 | 14.659 |
| 403 | HE | ARG | 724 | 6.750 | 69.494 | 16.085 |
| 404 | N | GLN | 725 | 9.468 | 64.745 | 18.588 |
| 405 | CA | GLN | 725 | 9.152 | 63.311 | 18.716 |
| 406 | HN | GLN | 725 | 10.383 | 65.011 | 18.284 |
| 407 | C | GLN | 725 | 9.183 | 62.733 | 20.124 |
| 408 | O | GLN | 725 | 8.499 | 61.746 | 20.406 |
| 409 | CB | GLN | 725 | 10.104 | 62.471 | 17.867 |
| 410 | CG | GLN | 725 | 9.763 | 62.367 | 16.396 |
| 411 | CD | GLN | 725 | 10.923 | 61.811 | 15.594 |
| 412 | OE1 | GLN | 725 | 10.753 | 61.361 | 14.460 |
| 413 | NE2 | GLN | 725 | 12.140 | 61.680 | 16.282 |
| 414 | 1HE2 | GLN | 725 | 12.859 | 61.134 | 15.843 |
| 415 | 2HE2 | GLN | 725 | 12.241 | 61.966 | 17.241 |
| 416 | N | LEU | 726 | 10.009 | 63.314 | 20.993 |
| 417 | CA | LEU | 726 | 10.114 | 62.841 | 22.369 |
| 418 | HN | LEU | 726 | 10.568 | 64.088 | 20.697 |
| 419 | C | LEU | 726 | 8.899 | 63.352 | 23.142 |
| 420 | O | LEU | 726 | 8.473 | 62.756 | 24.139 |
| 421 | CB | LEU | 726 | 11.436 | 63.328 | 23.029 |
| 422 | CG | LEU | 726 | 11.755 | 62.846 | 24.470 |
| 423 | CD1 | LEU | 726 | 11.921 | 61.320 | 24.492 |
| 424 | CD2 | LEU | 726 | 13.011 | 63.512 | 25.055 |
| 425 | N | LEU | 727 | 8.336 | 64.448 | 22.642 |
| 426 | CA | LEU | 727 | 7.145 | 65.049 | 23.218 |
| 427 | HN | LEU | 727 | 8.749 | 64.876 | 21.837 |
| 428 | C | LEU | 727 | 5.975 | 64.151 | 22.835 |
| 429 | O | LEU | 727 | 5.085 | 63.912 | 23.641 |
| 430 | CB | LEU | 727 | 6.932 | 66.496 | 22.689 |
| 431 | CG | LEU | 727 | 7.678 | 67.654 | 23.405 |
| 432 | CD1 | LEU | 727 | 7.808 | 68.860 | 22.465 |
| 433 | CD2 | LEU | 727 | 6.990 | 68.086 | 24.711 |
| 434 | N | SER | 728 | 5.988 | 63.628 | 21.616 |
| 435 | CA | SER | 728 | 4.923 | 62.724 | 21.198 |
| 436 | HN | SER | 728 | 6.728 | 63.855 | 20.983 |
| 437 | C | SER | 728 | 5.107 | 61.377 | 21.913 |
| 438 | O | SER | 728 | 4.165 | 60.573 | 22.006 |
| 439 | CB | SER | 728 | 4.957 | 62.561 | 19.658 |
| 440 | OG | SER | 728 | 6.142 | 61.904 | 19.194 |

| | | | | | | |
|---|---|---|---|---|---|---|
| 441 | HG | SER | 728 | 6.889 | 62.462 | 19.424 |
| 442 | N | VAL | 729 | 6.328 | 61.159 | 22.422 |
| 443 | CA | VAL | 729 | 6.726 | 59.930 | 23.143 |
| 444 | HN | VAL | 729 | 7.016 | 61.876 | 22.307 |
| 445 | C | VAL | 729 | 6.098 | 59.865 | 24.535 |
| 446 | O | VAL | 729 | 5.860 | 58.789 | 25.096 |
| 447 | CB | VAL | 729 | 8.302 | 59.869 | 23.233 |
| 448 | CG1 | VAL | 729 | 8.899 | 58.734 | 24.111 |
| 449 | CG2 | VAL | 729 | 9.004 | 59.734 | 21.860 |
| 450 | N | VAL | 730 | 5.853 | 61.042 | 25.092 |
| 451 | CA | VAL | 730 | 5.240 | 61.166 | 26.387 |
| 452 | HN | VAL | 730 | 6.103 | 61.873 | 24.594 |
| 453 | C | VAL | 730 | 3.724 | 60.950 | 26.214 |
| 454 | O | VAL | 730 | 3.127 | 60.223 | 27.013 |
| 455 | CB | VAL | 730 | 5.571 | 62.543 | 26.984 |
| 456 | CG1 | VAL | 730 | 4.977 | 62.676 | 28.383 |
| 457 | CG2 | VAL | 730 | 7.094 | 62.724 | 27.016 |
| 458 | N | LYS | 731 | 3.089 | 61.547 | 25.190 |
| 459 | CA | LYS | 731 | 1.644 | 61.294 | 25.014 |
| 460 | HN | LYS | 731 | 3.584 | 62.147 | 24.561 |
| 461 | C | LYS | 731 | 1.536 | 59.802 | 24.651 |
| 462 | O | LYS | 731 | 0.566 | 59.132 | 25.009 |
| 463 | CB | LYS | 731 | 0.963 | 62.138 | 23.883 |
| 464 | CG | LYS | 731 | 0.856 | 63.702 | 24.059 |
| 465 | CD | LYS | 731 | -0.247 | 64.447 | 23.158 |
| 466 | CE | LYS | 731 | -0.072 | 64.305 | 21.606 |
| 467 | NZ | LYS | 731 | -0.903 | 65.223 | 20.724 |
| 468 | HZ1 | LYS | 731 | -0.504 | 66.173 | 20.744 |
| 469 | HZ2 | LYS | 731 | -0.896 | 64.870 | 19.757 |
| 470 | HZ3 | LYS | 731 | -1.844 | 65.249 | 21.061 |
| 471 | N | TRP | 732 | 2.523 | 59.265 | 23.943 |
| 472 | CA | TRP | 732 | 2.427 | 57.855 | 23.605 |
| 473 | HN | TRP | 732 | 3.307 | 59.813 | 23.652 |
| 474 | C | TRP | 732 | 2.531 | 57.001 | 24.873 |
| 475 | O | TRP | 732 | 1.647 | 56.190 | 25.144 |
| 476 | CB | TRP | 732 | 3.514 | 57.461 | 22.599 |
| 477 | CG | TRP | 732 | 3.692 | 55.982 | 22.458 |
| 478 | CD1 | TRP | 732 | 2.865 | 55.102 | 21.832 |
| 479 | NE1 | TRP | 732 | 3.387 | 53.831 | 21.906 |
| 480 | CE2 | TRP | 732 | 4.576 | 53.879 | 22.585 |
| 481 | CD2 | TRP | 732 | 4.778 | 55.221 | 22.976 |
| 482 | HE1 | TRP | 732 | 2.967 | 53.007 | 21.526 |
| 483 | CE3 | TRP | 732 | 5.938 | 55.547 | 23.688 |
| 484 | CZ3 | TRP | 732 | 6.813 | 54.537 | 24.037 |
| 485 | CH2 | TRP | 732 | 6.583 | 53.207 | 23.640 |
| 486 | CZ2 | TRP | 732 | 5.459 | 52.858 | 22.937 |
| 487 | N | SER | 733 | 3.594 | 57.202 | 25.646 |
| 488 | CA | SER | 733 | 3.823 | 56.439 | 26.880 |
| 489 | HN | SER | 733 | 4.261 | 57.897 | 25.377 |
| 490 | C | SER | 733 | 2.603 | 56.421 | 27.797 |
| 491 | O | SER | 733 | 2.173 | 55.353 | 28.240 |
| 492 | CB | SER | 733 | 5.072 | 57.002 | 27.604 |
| 493 | OG | SER | 733 | 4.850 | 58.298 | 28.171 |
| 494 | HG | SER | 733 | 4.662 | 58.901 | 27.446 |
| 495 | N | LYS | 734 | 2.056 | 57.602 | 28.074 |

| | | | | | | |
|---|---|---|---|---|---|---|
| 496 | CA | LYS | 734 | 0.877 | 57.749 | 28.915 |
| 497 | HN | LYS | 734 | 2.473 | 58.425 | 27.688 |
| 498 | C | LYS | 734 | -0.310 | 56.892 | 28.435 |
| 499 | O | LYS | 734 | -1.087 | 56.394 | 29.246 |
| 500 | CB | LYS | 734 | 0.487 | 59.228 | 28.972 |
| 501 | CG | LYS | 734 | 1.491 | 60.104 | 29.739 |
| 502 | CD | LYS | 734 | 1.325 | 61.613 | 29.489 |
| 503 | CE | LYS | 734 | -0.138 | 62.049 | 29.374 |
| 504 | NZ | LYS | 734 | -0.392 | 63.445 | 29.911 |
| 505 | HZ1 | LYS | 734 | 0.444 | 64.028 | 29.762 |
| 506 | HZ2 | LYS | 734 | -1.194 | 63.863 | 29.418 |
| 507 | HZ3 | LYS | 734 | -0.593 | 63.395 | 30.890 |
| 508 | N | SER | 735 | -0.446 | 56.695 | 27.130 |
| 509 | CA | SER | 735 | -1.555 | 55.887 | 26.617 |
| 510 | HN | SER | 735 | 0.211 | 57.099 | 26.494 |
| 511 | C | SER | 735 | -1.320 | 54.381 | 26.706 |
| 512 | O | SER | 735 | -2.236 | 53.585 | 26.468 |
| 513 | CB | SER | 735 | -1.872 | 56.330 | 25.167 |
| 514 | OG | SER | 735 | -0.870 | 55.921 | 24.229 |
| 515 | HG | SER | 735 | -0.051 | 56.360 | 24.473 |
| 516 | N | LEU | 736 | -0.098 | 53.982 | 27.036 |
| 517 | CA | LEU | 736 | 0.201 | 52.564 | 27.138 |
| 518 | HN | LEU | 736 | 0.618 | 54.658 | 27.214 |
| 519 | C | LEU | 736 | -0.492 | 51.986 | 28.360 |
| 520 | O | LEU | 736 | -0.279 | 52.425 | 29.492 |
| 521 | CB | LEU | 736 | 1.737 | 52.337 | 27.211 |
| 522 | CG | LEU | 736 | 2.577 | 52.603 | 25.932 |
| 523 | CD1 | LEU | 736 | 4.063 | 52.733 | 26.294 |
| 524 | CD2 | LEU | 736 | 2.398 | 51.510 | 24.866 |
| 525 | N | PRO | 737 | -1.364 | 51.004 | 28.133 |
| 526 | CA | PRO | 737 | -2.117 | 50.324 | 29.181 |
| 527 | CD | PRO | 737 | -1.813 | 50.558 | 26.805 |
| 528 | C | PRO | 737 | -1.207 | 49.971 | 30.340 |
| 529 | O | PRO | 737 | -0.257 | 49.205 | 30.179 |
| 530 | CB | PRO | 737 | -2.634 | 49.094 | 28.460 |
| 531 | CG | PRO | 737 | -2.974 | 49.632 | 27.153 |
| 532 | N | GLY | 738 | -1.488 | 50.541 | 31.504 |
| 533 | CA | GLY | 738 | -0.686 | 50.263 | 32.676 |
| 534 | HN | GLY | 738 | -2.263 | 51.169 | 31.573 |
| 535 | C | GLY | 738 | 0.524 | 51.152 | 32.913 |
| 536 | O | GLY | 738 | 1.221 | 50.970 | 33.900 |
| 537 | N | PHE | 739 | 0.796 | 52.120 | 32.039 |
| 538 | CA | PHE | 739 | 1.968 | 52.963 | 32.256 |
| 539 | HN | PHE | 739 | 0.206 | 52.267 | 31.245 |
| 540 | C | PHE | 739 | 1.713 | 54.100 | 33.242 |
| 541 | O | PHE | 739 | 2.584 | 54.419 | 34.053 |
| 542 | CB | PHE | 739 | 2.475 | 53.554 | 30.937 |
| 543 | CG | PHE | 739 | 3.713 | 54.395 | 31.096 |
| 544 | CD1 | PHE | 739 | 4.959 | 53.797 | 31.287 |
| 545 | CE1 | PHE | 739 | 6.098 | 54.575 | 31.517 |
| 546 | CZ | PHE | 739 | 5.992 | 55.961 | 31.555 |
| 547 | CE2 | PHE | 739 | 4.751 | 56.569 | 31.359 |
| 548 | CD2 | PHE | 739 | 3.623 | 55.785 | 31.127 |
| 549 | N | ARG | 740 | 0.528 | 54.705 | 33.164 |
| 550 | CA | ARG | 740 | 0.151 | 55.833 | 34.029 |

| | | | | | | |
|---|---|---|---|---|---|---|
| 551 | HN | ARG | 740 | -0.132 | 54.378 | 32.488 |
| 552 | C | ARG | 740 | 0.051 | 55.468 | 35.498 |
| 553 | O | ARG | 740 | 0.347 | 56.277 | 36.381 |
| 554 | CB | ARG | 740 | -1.196 | 56.436 | 33.600 |
| 555 | CG | ARG | 740 | -1.600 | 57.692 | 34.347 |
| 556 | CD | ARG | 740 | -2.959 | 58.220 | 33.899 |
| 557 | NE | ARG | 740 | -3.349 | 59.432 | 34.619 |
| 558 | CZ | ARG | 740 | -4.486 | 60.105 | 34.429 |
| 559 | NH1 | ARG | 740 | -5.500 | 59.734 | 33.612 |
| 560 | 1HH1 | ARG | 740 | -6.319 | 60.308 | 33.542 |
| 561 | 2HH1 | ARG | 740 | -5.429 | 58.896 | 33.081 |
| 562 | NH2 | ARG | 740 | -4.647 | 61.253 | 35.141 |
| 563 | 1HH2 | ARG | 740 | -5.492 | 61.780 | 35.040 |
| 564 | 2HH2 | ARG | 740 | -3.926 | 61.556 | 35.761 |
| 565 | HE | ARG | 740 | -2.715 | 59.784 | 35.307 |
| 566 | N | ASN | 741 | -0.380 | 54.242 | 35.752 |
| 567 | CA | ASN | 741 | -0.544 | 53.777 | 37.110 |
| 568 | HN | ASN | 741 | -0.595 | 53.628 | 34.993 |
| 569 | C | ASN | 741 | 0.765 | 53.516 | 37.822 |
| 570 | O | ASN | 741 | 0.778 | 53.044 | 38.950 |
| 571 | CB | ASN | 741 | -1.436 | 52.542 | 37.106 |
| 572 | CG | ASN | 741 | -2.805 | 52.837 | 36.517 |
| 573 | OD1 | ASN | 741 | -3.261 | 52.166 | 35.586 |
| 574 | ND2 | ASN | 741 | -3.556 | 53.863 | 37.133 |
| 575 | 1HD2 | ASN | 741 | -4.490 | 54.048 | 36.802 |
| 576 | 2HD2 | ASN | 741 | -3.174 | 54.391 | 37.903 |
| 577 | N | LEU | 742 | 1.882 | 53.801 | 37.176 |
| 578 | CA | LEU | 742 | 3.131 | 53.615 | 37.881 |
| 579 | HN | LEU | 742 | 1.864 | 54.134 | 36.233 |
| 580 | C | LEU | 742 | 3.372 | 54.966 | 38.511 |
| 581 | O | LEU | 742 | 2.731 | 55.960 | 38.163 |
| 582 | CB | LEU | 742 | 4.285 | 53.273 | 36.943 |
| 583 | CG | LEU | 742 | 4.343 | 51.909 | 36.258 |
| 584 | CD1 | LEU | 742 | 4.962 | 52.123 | 34.901 |
| 585 | CD2 | LEU | 742 | 5.151 | 50.896 | 37.058 |
| 586 | N | HIS | 743 | 4.309 | 55.000 | 39.441 |
| 587 | CA | HIS | 743 | 4.627 | 56.232 | 40.110 |
| 588 | HN | HIS | 743 | 4.799 | 54.162 | 39.683 |
| 589 | C | HIS | 743 | 5.054 | 57.283 | 39.094 |
| 590 | O | HIS | 743 | 5.888 | 57.008 | 38.239 |
| 591 | CB | HIS | 743 | 5.742 | 55.981 | 41.108 |
| 592 | CG | HIS | 743 | 5.813 | 57.008 | 42.187 |
| 593 | ND1 | HIS | 743 | 6.381 | 58.249 | 42.000 |
| 594 | CE1 | HIS | 743 | 6.253 | 58.959 | 43.105 |
| 595 | NE2 | HIS | 743 | 5.621 | 58.221 | 44.003 |
| 596 | HE2 | HIS | 743 | 5.383 | 58.518 | 44.960 |
| 597 | CD2 | HIS | 743 | 5.333 | 56.999 | 43.455 |
| 598 | N | ILE | 744 | 4.482 | 58.476 | 39.192 |
| 599 | CA | ILE | 744 | 4.813 | 59.579 | 38.294 |
| 600 | HN | ILE | 744 | 3.799 | 58.625 | 39.907 |
| 601 | C | ILE | 744 | 6.313 | 59.875 | 38.306 |
| 602 | O | ILE | 744 | 6.792 | 60.726 | 37.559 |
| 603 | CB | ILE | 744 | 3.959 | 60.842 | 38.722 |
| 604 | CG1 | ILE | 744 | 3.695 | 61.869 | 37.574 |
| 605 | CG2 | ILE | 744 | 4.582 | 61.612 | 39.927 |

| | | | | | | |
|---|---|---|---|---|---|---|
| 606 | CD1 | ILE | 744 | 2.243 | 61.944 | 37.059 |
| 607 | N | ASP | 745 | 7.044 | 59.181 | 39.173 |
| 608 | CA | ASP | 745 | 8.488 | 59.358 | 39.290 |
| 609 | HN | ASP | 745 | 6.590 | 58.516 | 39.764 |
| 610 | C | ASP | 745 | 9.214 | 58.319 | 38.448 |
| 611 | O | ASP | 745 | 10.326 | 58.563 | 37.974 |
| 612 | CB | ASP | 745 | 8.916 | 59.232 | 40.757 |
| 613 | CG | ASP | 745 | 9.276 | 60.572 | 41.382 |
| 614 | OD1 | ASP | 745 | 8.646 | 61.590 | 41.021 |
| 615 | OD2 | ASP | 745 | 10.184 | 60.599 | 42.245 |
| 616 | HD2 | ASP | 745 | 10.305 | 61.486 | 42.548 |
| 617 | N | ASP | 746 | 8.597 | 57.155 | 38.283 |
| 618 | CA | ASP | 746 | 9.175 | 56.089 | 37.469 |
| 619 | HN | ASP | 746 | 7.714 | 57.004 | 38.728 |
| 620 | C | ASP | 746 | 8.833 | 56.439 | 36.039 |
| 621 | O | ASP | 746 | 9.643 | 56.265 | 35.127 |
| 622 | CB | ASP | 746 | 8.558 | 54.740 | 37.817 |
| 623 | CG | ASP | 746 | 8.720 | 54.394 | 39.271 |
| 624 | OD1 | ASP | 746 | 9.723 | 54.839 | 39.871 |
| 625 | OD2 | ASP | 746 | 7.853 | 53.670 | 39.809 |
| 626 | HD2 | ASP | 746 | 8.076 | 53.533 | 40.717 |
| 627 | N | GLN | 747 | 7.603 | 56.923 | 35.872 |
| 628 | CA | GLN | 747 | 7.092 | 57.359 | 34.588 |
| 629 | HN | GLN | 747 | 7.005 | 56.988 | 36.671 |
| 630 | C | GLN | 747 | 8.132 | 58.302 | 34.013 |
| 631 | O | GLN | 747 | 8.317 | 58.385 | 32.802 |
| 632 | CB | GLN | 747 | 5.757 | 58.103 | 34.767 |
| 633 | CG | GLN | 747 | 4.525 | 57.205 | 34.669 |
| 634 | CD | GLN | 747 | 3.203 | 57.950 | 34.838 |
| 635 | OE1 | GLN | 747 | 2.880 | 58.872 | 34.083 |
| 636 | NE2 | GLN | 747 | 2.575 | 57.795 | 36.072 |
| 637 | 1HE2 | GLN | 747 | 1.680 | 58.234 | 36.220 |
| 638 | 2HE2 | GLN | 747 | 2.839 | 57.042 | 36.695 |
| 639 | N | ILE | 748 | 8.829 | 59.003 | 34.899 |
| 640 | CA | ILE | 748 | 9.852 | 59.943 | 34.473 |
| 641 | HN | ILE | 748 | 8.647 | 58.882 | 35.875 |
| 642 | C | ILE | 748 | 11.220 | 59.333 | 34.340 |
| 643 | O | ILE | 748 | 12.079 | 59.902 | 33.666 |
| 644 | CB | ILE | 748 | 9.852 | 61.182 | 35.458 |
| 645 | CG1 | ILE | 748 | 8.996 | 62.397 | 34.977 |
| 646 | CG2 | ILE | 748 | 11.288 | 61.698 | 35.781 |
| 647 | CD1 | ILE | 748 | 9.230 | 63.724 | 35.728 |
| 648 | N | THR | 749 | 11.455 | 58.200 | 34.992 |
| 649 | CA | THR | 749 | 12.771 | 57.598 | 34.869 |
| 650 | HN | THR | 749 | 10.745 | 57.772 | 35.552 |
| 651 | C | THR | 749 | 12.803 | 56.821 | 33.566 |
| 652 | O | THR | 749 | 13.748 | 56.931 | 32.777 |
| 653 | CB | THR | 749 | 13.094 | 56.634 | 36.027 |
| 654 | OG1 | THR | 749 | 12.430 | 56.896 | 37.254 |
| 655 | HG1 | THR | 749 | 12.611 | 56.151 | 37.839 |
| 656 | CG2 | THR | 749 | 14.573 | 56.755 | 36.400 |
| 657 | N | LEU | 750 | 11.745 | 56.053 | 33.341 |
| 658 | CA | LEU | 750 | 11.639 | 55.250 | 32.143 |
| 659 | HN | LEU | 750 | 11.005 | 56.030 | 34.013 |
| 660 | C | LEU | 750 | 11.834 | 56.098 | 30.863 |

| | | | | | | |
|---|---|---|---|---|---|---|
| 661 | O | LEU | 750 | 12.645 | 55.741 | 30.008 |
| 662 | CB | LEU | 750 | 10.300 | 54.486 | 32.171 |
| 663 | CG | LEU | 750 | 10.163 | 53.478 | 33.338 |
| 664 | CD1 | LEU | 750 | 8.820 | 52.765 | 33.259 |
| 665 | CD2 | LEU | 750 | 11.295 | 52.451 | 33.306 |
| 666 | N | ILE | 751 | 11.143 | 57.233 | 30.745 |
| 667 | CA | ILE | 751 | 11.298 | 58.090 | 29.570 |
| 668 | HN | ILE | 751 | 10.510 | 57.502 | 31.470 |
| 669 | C | ILE | 751 | 12.635 | 58.751 | 29.471 |
| 670 | O | ILE | 751 | 13.027 | 59.243 | 28.416 |
| 671 | CB | ILE | 751 | 10.118 | 59.145 | 29.580 |
| 672 | CG1 | ILE | 751 | 8.725 | 58.569 | 29.167 |
| 673 | CG2 | ILE | 751 | 10.412 | 60.390 | 28.686 |
| 674 | CD1 | ILE | 751 | 7.530 | 59.534 | 29.306 |
| 675 | N | GLN | 752 | 13.337 | 58.801 | 30.584 |
| 676 | CA | GLN | 752 | 14.626 | 59.444 | 30.567 |
| 677 | HN | GLN | 752 | 12.981 | 58.399 | 31.428 |
| 678 | C | GLN | 752 | 15.720 | 58.460 | 30.267 |
| 679 | O | GLN | 752 | 16.740 | 58.801 | 29.672 |
| 680 | CB | GLN | 752 | 14.864 | 60.136 | 31.890 |
| 681 | CG | GLN | 752 | 14.349 | 61.550 | 31.885 |
| 682 | CD | GLN | 752 | 14.080 | 62.051 | 33.272 |
| 683 | OE1 | GLN | 752 | 14.762 | 61.667 | 34.231 |
| 684 | NE2 | GLN | 752 | 12.982 | 62.864 | 33.554 |
| 685 | 1HE2 | GLN | 752 | 12.838 | 63.166 | 34.506 |
| 686 | 2HE2 | GLN | 752 | 12.311 | 63.088 | 32.846 |
| 687 | N | TYR | 753 | 15.499 | 57.221 | 30.677 |
| 688 | CA | TYR | 753 | 16.473 | 56.184 | 30.422 |
| 689 | HN | TYR | 753 | 14.654 | 57.002 | 31.165 |
| 690 | C | TYR | 753 | 16.332 | 55.637 | 29.012 |
| 691 | O | TYR | 753 | 17.282 | 55.096 | 28.463 |
| 692 | CB | TYR | 753 | 16.270 | 55.032 | 31.377 |
| 693 | CG | TYR | 753 | 16.891 | 55.184 | 32.734 |
| 694 | CD1 | TYR | 753 | 16.859 | 56.394 | 33.424 |
| 695 | CE1 | TYR | 753 | 17.361 | 56.485 | 34.721 |
| 696 | CZ | TYR | 753 | 17.891 | 55.346 | 35.321 |
| 697 | OH | TYR | 753 | 18.389 | 55.465 | 36.601 |
| 698 | HH | TYR | 753 | 19.130 | 54.856 | 36.698 |
| 699 | CE2 | TYR | 753 | 17.930 | 54.150 | 34.650 |
| 700 | CD2 | TYR | 753 | 17.437 | 54.076 | 33.369 |
| 701 | N | SER | 754 | 15.157 | 55.784 | 28.406 |
| 702 | CA | SER | 754 | 14.983 | 55.203 | 27.088 |
| 703 | HN | SER | 754 | 14.412 | 56.282 | 28.849 |
| 704 | C | SER | 754 | 14.524 | 56.053 | 25.911 |
| 705 | O | SER | 754 | 14.159 | 55.506 | 24.878 |
| 706 | CB | SER | 754 | 14.067 | 53.978 | 27.206 |
| 707 | OG | SER | 754 | 12.749 | 54.286 | 27.635 |
| 708 | HG | SER | 754 | 12.366 | 53.459 | 27.955 |
| 709 | N | TRP | 755 | 14.556 | 57.371 | 26.019 |
| 710 | CA | TRP | 755 | 14.089 | 58.170 | 24.891 |
| 711 | HN | TRP | 755 | 14.891 | 57.809 | 26.853 |
| 712 | C | TRP | 755 | 14.860 | 57.907 | 23.575 |
| 713 | O | TRP | 755 | 14.270 | 57.970 | 22.492 |
| 714 | CB | TRP | 755 | 14.082 | 59.679 | 25.243 |
| 715 | CG | TRP | 755 | 15.425 | 60.318 | 25.318 |

| | | | | | | |
|---|---|---|---|---|---|---|
| 716 | CD1 | TRP | 755 | 16.307 | 60.264 | 26.362 |
| 717 | NE1 | TRP | 755 | 17.499 | 60.846 | 26.005 |
| 718 | CE2 | TRP | 755 | 17.406 | 61.298 | 24.713 |
| 719 | CD2 | TRP | 755 | 16.107 | 60.984 | 24.251 |
| 720 | HE1 | TRP | 755 | 18.305 | 60.928 | 26.592 |
| 721 | CE3 | TRP | 755 | 15.747 | 61.347 | 22.945 |
| 722 | CZ3 | TRP | 755 | 16.687 | 61.997 | 22.145 |
| 723 | CH2 | TRP | 755 | 17.976 | 62.289 | 22.635 |
| 724 | CZ2 | TRP | 755 | 18.350 | 61.949 | 23.912 |
| 725 | N | MET | 756 | 16.154 | 57.583 | 23.653 |
| 726 | CA | MET | 756 | 16.939 | 57.351 | 22.436 |
| 727 | HN | MET | 756 | 16.590 | 57.497 | 24.549 |
| 728 | C | MET | 756 | 16.665 | 55.975 | 21.840 |
| 729 | O | MET | 756 | 16.599 | 55.804 | 20.620 |
| 730 | CB | MET | 756 | 18.437 | 57.489 | 22.727 |
| 731 | CG | MET | 756 | 19.322 | 57.374 | 21.487 |
| 732 | SD | MET | 756 | 19.295 | 58.819 | 20.443 |
| 733 | CE | MET | 756 | 20.458 | 59.745 | 21.299 |
| 734 | N | SER | 757 | 16.525 | 54.998 | 22.724 |
| 735 | CA | SER | 757 | 16.219 | 53.640 | 22.332 |
| 736 | HN | SER | 757 | 16.636 | 55.206 | 23.696 |
| 737 | C | SER | 757 | 14.879 | 53.727 | 21.599 |
| 738 | O | SER | 757 | 14.704 | 53.143 | 20.535 |
| 739 | CB | SER | 757 | 16.131 | 52.775 | 23.583 |
| 740 | OG | SER | 757 | 17.395 | 52.602 | 24.175 |
| 741 | HG | SER | 757 | 17.229 | 52.241 | 25.055 |
| 742 | N | LEU | 758 | 13.941 | 54.491 | 22.162 |
| 743 | CA | LEU | 758 | 12.628 | 54.685 | 21.544 |
| 744 | HN | LEU | 758 | 14.141 | 54.944 | 23.031 |
| 745 | C | LEU | 758 | 12.713 | 55.412 | 20.187 |
| 746 | O | LEU | 758 | 12.055 | 55.016 | 19.208 |
| 747 | CB | LEU | 758 | 11.729 | 55.508 | 22.468 |
| 748 | CG | LEU | 758 | 11.119 | 54.808 | 23.674 |
| 749 | CD1 | LEU | 758 | 10.281 | 55.784 | 24.452 |
| 750 | CD2 | LEU | 758 | 10.279 | 53.654 | 23.205 |
| 751 | N | MET | 759 | 13.511 | 56.479 | 20.156 |
| 752 | CA | MET | 759 | 13.702 | 57.297 | 18.966 |
| 753 | HN | MET | 759 | 14.001 | 56.731 | 20.990 |
| 754 | C | MET | 759 | 14.433 | 56.546 | 17.852 |
| 755 | O | MET | 759 | 14.021 | 56.589 | 16.690 |
| 756 | CB | MET | 759 | 14.447 | 58.591 | 19.331 |
| 757 | CG | MET | 759 | 13.553 | 59.646 | 19.984 |
| 758 | SD | MET | 759 | 14.425 | 61.220 | 20.263 |
| 759 | CE | MET | 759 | 13.139 | 62.315 | 21.053 |
| 760 | N | VAL | 760 | 15.514 | 55.854 | 18.209 |
| 761 | CA | VAL | 760 | 16.279 | 55.089 | 17.224 |
| 762 | HN | VAL | 760 | 15.807 | 55.857 | 19.165 |
| 763 | C | VAL | 760 | 15.451 | 53.923 | 16.702 |
| 764 | O | VAL | 760 | 15.347 | 53.742 | 15.490 |
| 765 | CB | VAL | 760 | 17.632 | 54.577 | 17.860 |
| 766 | CG1 | VAL | 760 | 17.508 | 53.476 | 18.950 |
| 767 | CG2 | VAL | 760 | 18.639 | 54.017 | 16.827 |
| 768 | N | PHE | 761 | 14.847 | 53.138 | 17.595 |
| 769 | CA | PHE | 761 | 14.062 | 52.008 | 17.125 |
| 770 | HN | PHE | 761 | 14.933 | 53.324 | 18.573 |

| | | | | | | |
|---|---|---|---|---|---|---|
| 771 | C | PHE | 761 | 13.040 | 52.442 | 16.091 |
| 772 | O | PHE | 761 | 12.930 | 51.819 | 15.041 |
| 773 | CB | PHE | 761 | 13.341 | 51.305 | 18.259 |
| 774 | CG | PHE | 761 | 12.974 | 49.889 | 17.946 |
| 775 | CD1 | PHE | 761 | 13.965 | 48.983 | 17.586 |
| 776 | CE1 | PHE | 761 | 13.653 | 47.672 | 17.293 |
| 777 | CZ | PHE | 761 | 12.321 | 47.252 | 17.340 |
| 778 | CE2 | PHE | 761 | 11.312 | 48.151 | 17.694 |
| 779 | CD2 | PHE | 761 | 11.644 | 49.464 | 17.986 |
| 780 | N | GLY | 762 | 12.287 | 53.501 | 16.375 |
| 781 | CA | GLY | 762 | 11.305 | 53.994 | 15.417 |
| 782 | HN | GLY | 762 | 12.396 | 53.964 | 17.254 |
| 783 | C | GLY | 762 | 11.969 | 54.319 | 14.068 |
| 784 | O | GLY | 762 | 11.510 | 53.828 | 13.038 |
| 785 | N | LEU | 763 | 13.028 | 55.129 | 14.052 |
| 786 | CA | LEU | 763 | 13.708 | 55.432 | 12.787 |
| 787 | HN | LEU | 763 | 13.359 | 55.533 | 14.905 |
| 788 | C | LEU | 763 | 14.084 | 54.093 | 12.138 |
| 789 | O | LEU | 763 | 13.863 | 53.883 | 10.944 |
| 790 | CB | LEU | 763 | 14.968 | 56.282 | 13.031 |
| 791 | CG | LEU | 763 | 16.092 | 56.607 | 12.014 |
| 792 | CD1 | LEU | 763 | 15.854 | 57.869 | 11.161 |
| 793 | CD2 | LEU | 763 | 17.329 | 56.845 | 12.863 |
| 794 | N | GLY | 764 | 14.643 | 53.181 | 12.926 |
| 795 | CA | GLY | 764 | 14.999 | 51.900 | 12.361 |
| 796 | HN | GLY | 764 | 14.813 | 53.377 | 13.892 |
| 797 | C | GLY | 764 | 13.834 | 51.459 | 11.509 |
| 798 | O | GLY | 764 | 13.961 | 51.256 | 10.308 |
| 799 | N | TRP | 765 | 12.676 | 51.353 | 12.134 |
| 800 | CA | TRP | 765 | 11.460 | 50.940 | 11.462 |
| 801 | HN | TRP | 765 | 12.637 | 51.565 | 13.110 |
| 802 | C | TRP | 765 | 11.085 | 51.734 | 10.187 |
| 803 | O | TRP | 765 | 10.868 | 51.159 | 9.134 |
| 804 | CB | TRP | 765 | 10.334 | 50.995 | 12.476 |
| 805 | CG | TRP | 765 | 9.056 | 50.606 | 11.930 |
| 806 | CD1 | TRP | 765 | 8.037 | 51.435 | 11.565 |
| 807 | NE1 | TRP | 765 | 6.972 | 50.695 | 11.112 |
| 808 | CE2 | TRP | 765 | 7.299 | 49.365 | 11.173 |
| 809 | CD2 | TRP | 765 | 8.609 | 49.276 | 11.699 |
| 810 | HE1 | TRP | 765 | 6.100 | 51.066 | 10.791 |
| 811 | CE3 | TRP | 765 | 9.194 | 48.009 | 11.852 |
| 812 | CZ3 | TRP | 765 | 8.448 | 46.879 | 11.513 |
| 813 | CH2 | TRP | 765 | 7.139 | 47.004 | 11.001 |
| 814 | CZ2 | TRP | 765 | 6.547 | 48.234 | 10.834 |
| 815 | N | ARG | 766 | 10.993 | 53.047 | 10.288 |
| 816 | CA | ARG | 766 | 10.647 | 53.869 | 9.138 |
| 817 | HN | ARG | 766 | 11.164 | 53.485 | 11.171 |
| 818 | C | ARG | 766 | 11.567 | 53.630 | 7.941 |
| 819 | O | ARG | 766 | 11.138 | 53.702 | 6.787 |
| 820 | CB | ARG | 766 | 10.709 | 55.349 | 9.519 |
| 821 | CG | ARG | 766 | 9.605 | 55.799 | 10.434 |
| 822 | CD | ARG | 766 | 9.469 | 57.293 | 10.363 |
| 823 | NE | ARG | 766 | 10.615 | 57.981 | 10.950 |
| 824 | CZ | ARG | 766 | 10.942 | 57.921 | 12.243 |
| 825 | NH1 | ARG | 766 | 10.223 | 57.241 | 13.179 |

| | | | | | | |
|---|---|---|---|---|---|---|
| 826 | 1HH1 | ARG | 766 | 10.522 | 57.249 | 14.133 |
| 827 | 2HH1 | ARG | 766 | 9.402 | 56.748 | 12.898 |
| 828 | NH2 | ARG | 766 | 12.050 | 58.599 | 12.645 |
| 829 | 1HH2 | ARG | 766 | 12.330 | 58.592 | 13.604 |
| 830 | 2HH2 | ARG | 766 | 12.572 | 59.116 | 11.966 |
| 831 | HE | ARG | 766 | 11.190 | 58.532 | 10.346 |
| 832 | N | SER | 767 | 12.833 | 53.352 | 8.234 |
| 833 | CA | SER | 767 | 13.858 | 53.113 | 7.230 |
| 834 | HN | SER | 767 | 13.095 | 53.305 | 9.198 |
| 835 | C | SER | 767 | 13.671 | 51.814 | 6.491 |
| 836 | O | SER | 767 | 13.787 | 51.732 | 5.269 |
| 837 | CB | SER | 767 | 15.220 | 53.082 | 7.901 |
| 838 | OG | SER | 767 | 15.599 | 54.311 | 8.521 |
| 839 | HG | SER | 767 | 16.377 | 54.104 | 9.055 |
| 840 | N | TYR | 768 | 13.406 | 50.789 | 7.273 |
| 841 | CA | TYR | 768 | 13.218 | 49.465 | 6.764 |
| 842 | HN | TYR | 768 | 13.335 | 50.941 | 8.259 |
| 843 | C | TYR | 768 | 11.985 | 49.344 | 5.896 |
| 844 | O | TYR | 768 | 11.951 | 48.557 | 4.958 |
| 845 | CB | TYR | 768 | 13.133 | 48.529 | 7.960 |
| 846 | CG | TYR | 768 | 12.233 | 47.343 | 7.789 |
| 847 | CD1 | TYR | 768 | 12.484 | 46.385 | 6.810 |
| 848 | CE1 | TYR | 768 | 11.714 | 45.239 | 6.715 |
| 849 | CZ | TYR | 768 | 10.673 | 45.040 | 7.611 |
| 850 | OH | TYR | 768 | 9.912 | 43.894 | 7.529 |
| 851 | HH | TYR | 768 | 9.463 | 43.774 | 8.376 |
| 852 | CE2 | TYR | 768 | 10.399 | 45.989 | 8.590 |
| 853 | CD2 | TYR | 768 | 11.177 | 47.133 | 8.670 |
| 854 | N | LYS | 769 | 10.970 | 50.138 | 6.171 |
| 855 | CA | LYS | 769 | 9.756 | 49.993 | 5.397 |
| 856 | HN | LYS | 769 | 11.037 | 50.823 | 6.897 |
| 857 | C | LYS | 769 | 9.638 | 50.815 | 4.144 |
| 858 | O | LYS | 769 | 8.932 | 50.439 | 3.209 |
| 859 | CB | LYS | 769 | 8.566 | 50.268 | 6.357 |
| 860 | CG | LYS | 769 | 8.565 | 49.361 | 7.613 |
| 861 | CD | LYS | 769 | 7.176 | 48.937 | 8.100 |
| 862 | CE | LYS | 769 | 7.046 | 47.412 | 7.991 |
| 863 | NZ | LYS | 769 | 5.686 | 47.067 | 7.542 |
| 864 | HZ2 | LYS | 769 | 5.346 | 47.752 | 6.857 |
| 865 | HZ1 | LYS | 769 | 5.669 | 46.157 | 7.069 |
| 866 | HZ3 | LYS | 769 | 5.070 | 47.045 | 8.330 |
| 867 | N | HIS | 770 | 10.348 | 51.932 | 4.147 |
| 868 | CA | HIS | 770 | 10.323 | 52.902 | 3.073 |
| 869 | HN | HIS | 770 | 10.934 | 52.116 | 4.936 |
| 870 | C | HIS | 770 | 11.406 | 52.732 | 2.021 |
| 871 | O | HIS | 770 | 11.350 | 53.354 | 0.962 |
| 872 | CB | HIS | 770 | 10.420 | 54.294 | 3.720 |
| 873 | CG | HIS | 770 | 9.805 | 55.363 | 2.866 |
| 874 | ND1 | HIS | 770 | 9.981 | 56.736 | 3.026 |
| 875 | CE1 | HIS | 770 | 9.222 | 57.185 | 2.008 |
| 876 | NE2 | HIS | 770 | 8.584 | 56.268 | 1.231 |
| 877 | CD2 | HIS | 770 | 8.969 | 55.080 | 1.795 |
| 878 | HE2 | HIS | 770 | 7.968 | 56.421 | 0.419 |
| 879 | N | VAL | 771 | 12.404 | 51.906 | 2.311 |
| 880 | CA | VAL | 771 | 13.502 | 51.711 | 1.378 |

| | | | | | | |
|---|---|---|---|---|---|---|
| 881 | HN | VAL | 771 | 12.401 | 51.414 | 3.181 |
| 882 | C | VAL | 771 | 14.375 | 50.537 | 1.784 |
| 883 | O | VAL | 771 | 15.610 | 50.602 | 1.730 |
| 884 | CB | VAL | 771 | 14.356 | 53.036 | 1.270 |
| 885 | CG1 | VAL | 771 | 14.529 | 53.853 | 2.580 |
| 886 | CG2 | VAL | 771 | 15.790 | 52.816 | 0.733 |
| 887 | N | SER | 772 | 13.702 | 49.482 | 2.233 |
| 888 | CA | SER | 772 | 14.314 | 48.218 | 2.620 |
| 889 | HN | SER | 772 | 12.708 | 49.561 | 2.310 |
| 890 | C | SER | 772 | 15.612 | 48.239 | 3.458 |
| 891 | O | SER | 772 | 16.410 | 47.298 | 3.378 |
| 892 | CB | SER | 772 | 14.528 | 47.410 | 1.335 |
| 893 | OG | SER | 772 | 13.975 | 47.893 | 0.103 |
| 894 | HG | SER | 772 | 14.283 | 47.293 | -0.588 |
| 895 | N | GLY | 773 | 15.798 | 49.283 | 4.266 |
| 896 | CA | GLY | 773 | 16.989 | 49.450 | 5.119 |
| 897 | HN | GLY | 773 | 15.092 | 49.990 | 4.295 |
| 898 | C | GLY | 773 | 18.206 | 49.933 | 4.318 |
| 899 | O | GLY | 773 | 19.350 | 49.548 | 4.604 |
| 900 | N | GLN | 774 | 17.949 | 50.786 | 3.330 |
| 901 | CA | GLN | 774 | 18.986 | 51.346 | 2.466 |
| 902 | HN | GLN | 774 | 17.000 | 51.058 | 3.171 |
| 903 | C | GLN | 774 | 19.341 | 52.784 | 2.844 |
| 904 | O | GLN | 774 | 20.516 | 53.158 | 2.902 |
| 905 | CB | GLN | 774 | 18.484 | 51.265 | 1.008 |
| 906 | CG | GLN | 774 | 18.381 | 49.831 | 0.391 |
| 907 | CD | GLN | 774 | 19.507 | 49.326 | -0.520 |
| 908 | OE1 | GLN | 774 | 19.482 | 48.199 | -0.988 |
| 909 | NE2 | GLN | 774 | 20.528 | 50.100 | -0.787 |
| 910 | 2HE2 | GLN | 774 | 20.522 | 51.002 | -0.309 |
| 911 | 1HE2 | GLN | 774 | 21.264 | 49.665 | -1.347 |
| 912 | N | MET | 775 | 18.294 | 53.567 | 3.089 |
| 913 | CA | MET | 775 | 18.370 | 54.983 | 3.462 |
| 914 | HN | MET | 775 | 17.384 | 53.158 | 3.015 |
| 915 | C | MET | 775 | 17.939 | 55.180 | 4.938 |
| 916 | O | MET | 775 | 17.228 | 54.341 | 5.487 |
| 917 | CB | MET | 775 | 17.442 | 55.779 | 2.503 |
| 918 | CG | MET | 775 | 18.095 | 56.282 | 1.197 |
| 919 | SD | MET | 775 | 19.266 | 55.057 | 0.589 |
| 920 | CE | MET | 775 | 20.099 | 56.071 | -0.639 |
| 921 | N | LEU | 776 | 18.401 | 56.252 | 5.592 |
| 922 | CA | LEU | 776 | 17.987 | 56.552 | 6.973 |
| 923 | HN | LEU | 776 | 19.044 | 56.864 | 5.132 |
| 924 | C | LEU | 776 | 16.867 | 57.558 | 6.762 |
| 925 | O | LEU | 776 | 17.090 | 58.616 | 6.182 |
| 926 | CB | LEU | 776 | 19.116 | 57.191 | 7.807 |
| 927 | CG | LEU | 776 | 20.065 | 56.240 | 8.555 |
| 928 | CD1 | LEU | 776 | 20.964 | 57.020 | 9.506 |
| 929 | CD2 | LEU | 776 | 19.243 | 55.216 | 9.333 |
| 930 | N | TYR | 777 | 15.670 | 57.231 | 7.238 |
| 931 | CA | TYR | 777 | 14.513 | 58.088 | 6.999 |
| 932 | HN | TYR | 777 | 15.561 | 56.388 | 7.766 |
| 933 | C | TYR | 777 | 13.906 | 58.809 | 8.186 |
| 934 | O | TYR | 777 | 12.846 | 58.426 | 8.682 |
| 935 | CB | TYR | 777 | 13.390 | 57.253 | 6.318 |

| | | | | | | |
|---|---|---|---|---|---|---|
| 936 | CG | TYR | 777 | 13.796 | 56.417 | 5.100 |
| 937 | CD1 | TYR | 777 | 13.972 | 57.016 | 3.849 |
| 938 | CD2 | TYR | 777 | 14.003 | 55.041 | 5.240 |
| 939 | CE1 | TYR | 777 | 14.357 | 56.247 | 2.753 |
| 940 | CE2 | TYR | 777 | 14.387 | 54.274 | 4.144 |
| 941 | CZ | TYR | 777 | 14.563 | 54.878 | 2.901 |
| 942 | OH | TYR | 777 | 14.943 | 54.128 | 1.825 |
| 943 | HH | TYR | 777 | 15.041 | 53.217 | 2.109 |
| 944 | N | PHE | 778 | 14.541 | 59.887 | 8.604 |
| 945 | CA | PHE | 778 | 14.068 | 60.646 | 9.741 |
| 946 | HN | PHE | 778 | 15.367 | 60.186 | 8.125 |
| 947 | C | PHE | 778 | 12.603 | 61.090 | 9.692 |
| 948 | O | PHE | 778 | 11.859 | 60.829 | 10.642 |
| 949 | CB | PHE | 778 | 15.020 | 61.807 | 9.972 |
| 950 | CG | PHE | 778 | 16.386 | 61.318 | 10.325 |
| 951 | CD1 | PHE | 778 | 17.224 | 60.778 | 9.351 |
| 952 | CE1 | PHE | 778 | 18.446 | 60.198 | 9.699 |
| 953 | CZ | PHE | 778 | 18.886 | 60.261 | 11.009 |
| 954 | CE2 | PHE | 778 | 18.061 | 60.811 | 11.987 |
| 955 | CD2 | PHE | 778 | 16.845 | 61.390 | 11.630 |
| 956 | N | ALA | 779 | 12.191 | 61.735 | 8.605 |
| 957 | CA | ALA | 779 | 10.803 | 62.195 | 8.413 |
| 958 | HN | ALA | 779 | 12.856 | 61.919 | 7.881 |
| 959 | C | ALA | 779 | 10.591 | 62.160 | 6.899 |
| 960 | O | ALA | 779 | 11.186 | 62.970 | 6.216 |
| 961 | CB | ALA | 779 | 10.644 | 63.625 | 8.933 |
| 962 | N | PRO | 780 | 9.756 | 61.236 | 6.359 |
| 963 | CA | PRO | 780 | 9.579 | 61.215 | 4.895 |
| 964 | CD | PRO | 780 | 8.903 | 60.205 | 6.984 |
| 965 | C | PRO | 780 | 9.892 | 62.462 | 4.031 |
| 966 | O | PRO | 780 | 10.012 | 62.338 | 2.812 |
| 967 | CB | PRO | 780 | 8.151 | 60.692 | 4.748 |
| 968 | CG | PRO | 780 | 8.154 | 59.579 | 5.772 |
| 969 | N | ASP | 781 | 10.023 | 63.635 | 4.660 |
| 970 | CA | ASP | 781 | 10.403 | 64.883 | 3.987 |
| 971 | HN | ASP | 781 | 9.853 | 63.663 | 5.645 |
| 972 | C | ASP | 781 | 11.908 | 65.110 | 4.248 |
| 973 | O | ASP | 781 | 12.416 | 66.219 | 4.071 |
| 974 | CB | ASP | 781 | 9.547 | 66.079 | 4.513 |
| 975 | CG | ASP | 781 | 10.321 | 67.074 | 5.442 |
| 976 | OD1 | ASP | 781 | 11.387 | 66.743 | 6.007 |
| 977 | OD2 | ASP | 781 | 9.831 | 68.214 | 5.632 |
| 978 | HD2 | ASP | 781 | 10.395 | 68.707 | 6.208 |
| 979 | N | LEU | 782 | 12.622 | 64.067 | 4.686 |
| 980 | CA | LEU | 782 | 14.066 | 64.183 | 4.963 |
| 981 | HN | LEU | 782 | 12.166 | 63.188 | 4.829 |
| 982 | C | LEU | 782 | 14.766 | 62.853 | 5.221 |
| 983 | O | LEU | 782 | 14.845 | 62.374 | 6.362 |
| 984 | CB | LEU | 782 | 14.356 | 65.126 | 6.149 |
| 985 | CG | LEU | 782 | 15.833 | 65.518 | 6.400 |
| 986 | CD1 | LEU | 782 | 15.922 | 66.482 | 7.567 |
| 987 | CD2 | LEU | 782 | 16.696 | 64.304 | 6.705 |
| 988 | N | ILE | 783 | 15.304 | 62.307 | 4.134 |
| 989 | CA | ILE | 783 | 16.033 | 61.049 | 4.107 |
| 990 | HN | ILE | 783 | 15.199 | 62.799 | 3.270 |

| | | | | | | |
|---|---|---|---|---|---|---|
| 991 | C | ILE | 783 | 17.515 | 61.321 | 3.918 |
| 992 | O | ILE | 783 | 17.910 | 62.236 | 3.195 |
| 993 | CB | ILE | 783 | 15.594 | 60.191 | 2.921 |
| 994 | CG2 | ILE | 783 | 16.348 | 58.883 | 2.912 |
| 995 | CG1 | ILE | 783 | 14.084 | 59.984 | 2.973 |
| 996 | CD1 | ILE | 783 | 13.580 | 58.911 | 2.035 |
| 997 | N | LEU | 784 | 18.352 | 60.533 | 4.567 |
| 998 | CA | LEU | 784 | 19.752 | 60.743 | 4.372 |
| 999 | HN | LEU | 784 | 18.017 | 59.814 | 5.176 |
| 1000 | C | LEU | 784 | 20.207 | 59.721 | 3.381 |
| 1001 | O | LEU | 784 | 20.504 | 58.575 | 3.718 |
| 1002 | CB | LEU | 784 | 20.567 | 60.640 | 5.692 |
| 1003 | CG | LEU | 784 | 20.745 | 61.925 | 6.544 |
| 1004 | CD1 | LEU | 784 | 21.240 | 61.561 | 7.951 |
| 1005 | CD2 | LEU | 784 | 21.709 | 62.936 | 5.901 |
| 1006 | N | ASN | 785 | 20.172 | 60.171 | 2.136 |
| 1007 | CA | ASN | 785 | 20.583 | 59.440 | 0.952 |
| 1008 | HN | ASN | 785 | 19.831 | 61.101 | 1.999 |
| 1009 | C | ASN | 785 | 22.105 | 59.406 | 0.993 |
| 1010 | O | ASN | 785 | 22.741 | 59.804 | 1.982 |
| 1011 | CB | ASN | 785 | 20.158 | 60.228 | -0.266 |
| 1012 | CG | ASN | 785 | 20.463 | 61.686 | -0.088 |
| 1013 | OD1 | ASN | 785 | 21.246 | 62.041 | 0.802 |
| 1014 | ND2 | ASN | 785 | 19.835 | 62.588 | -0.969 |
| 1015 | 1HD2 | ASN | 785 | 20.070 | 63.569 | -0.935 |
| 1016 | 2HD2 | ASN | 785 | 19.233 | 62.251 | -1.704 |
| 1017 | N | GLU | 786 | 22.663 | 58.979 | -0.126 |
| 1018 | CA | GLU | 786 | 24.082 | 58.822 | -0.276 |
| 1019 | HN | GLU | 786 | 22.072 | 58.756 | -0.901 |
| 1020 | C | GLU | 786 | 24.617 | 60.138 | -0.412 |
| 1021 | O | GLU | 786 | 25.341 | 60.550 | 0.437 |
| 1022 | CB | GLU | 786 | 24.391 | 58.080 | -1.511 |
| 1023 | CG | GLU | 786 | 25.667 | 57.375 | -1.441 |
| 1024 | CD | GLU | 786 | 26.031 | 56.969 | -2.820 |
| 1025 | OE1 | GLU | 786 | 25.501 | 55.940 | -3.297 |
| 1026 | OE2 | GLU | 786 | 26.811 | 57.715 | -3.446 |
| 1027 | HE2 | GLU | 786 | 26.969 | 57.356 | -4.306 |
| 1028 | N | GLN | 787 | 24.351 | 60.774 | -1.523 |
| 1029 | CA | GLN | 787 | 24.794 | 62.102 | -1.541 |
| 1030 | HN | GLN | 787 | 23.873 | 60.349 | -2.292 |
| 1031 | C | GLN | 787 | 25.042 | 62.437 | -0.030 |
| 1032 | O | GLN | 787 | 26.119 | 62.188 | 0.452 |
| 1033 | CB | GLN | 787 | 23.667 | 62.852 | -2.085 |
| 1034 | CG | GLN | 787 | 24.038 | 64.078 | -2.608 |
| 1035 | CD | GLN | 787 | 22.876 | 64.912 | -2.433 |
| 1036 | OE1 | GLN | 787 | 21.774 | 64.528 | -2.827 |
| 1037 | NE2 | GLN | 787 | 23.064 | 66.159 | -1.785 |
| 1038 | 1HE2 | GLN | 787 | 22.280 | 66.783 | -1.655 |
| 1039 | 2HE2 | GLN | 787 | 23.984 | 66.437 | -1.479 |
| 1040 | N | ARG | 788 | 24.048 | 62.861 | 0.759 |
| 1041 | CA | ARG | 788 | 24.284 | 63.236 | 2.187 |
| 1042 | HN | ARG | 788 | 23.124 | 62.930 | 0.385 |
| 1043 | C | ARG | 788 | 25.212 | 62.467 | 3.112 |
| 1044 | O | ARG | 788 | 26.035 | 63.039 | 3.822 |
| 1045 | CB | ARG | 788 | 22.962 | 63.385 | 2.896 |

| | | | | | | |
|---|---|---|---|---|---|---|
| 1046 | CG | ARG | 788 | 22.235 | 64.527 | 2.333 |
| 1047 | CD | ARG | 788 | 20.817 | 64.471 | 2.706 |
| 1048 | NE | ARG | 788 | 20.155 | 65.689 | 2.277 |
| 1049 | CZ | ARG | 788 | 18.843 | 65.836 | 2.288 |
| 1050 | NH1 | ARG | 788 | 17.944 | 64.906 | 2.689 |
| 1051 | 1HH1 | ARG | 788 | 16.968 | 65.106 | 2.663 |
| 1052 | 2HH1 | ARG | 788 | 18.268 | 64.016 | 3.011 |
| 1053 | NH2 | ARG | 788 | 18.393 | 67.046 | 1.851 |
| 1054 | 1HH2 | ARG | 788 | 17.416 | 67.245 | 1.824 |
| 1055 | 2HH2 | ARG | 788 | 19.071 | 67.726 | 1.561 |
| 1056 | HE | ARG | 788 | 20.719 | 66.452 | 1.960 |
| 1057 | N | MET | 789 | 25.022 | 61.164 | 3.130 |
| 1058 | CA | MET | 789 | 25.809 | 60.250 | 3.920 |
| 1059 | HN | MET | 789 | 24.290 | 60.787 | 2.563 |
| 1060 | C | MET | 789 | 27.284 | 60.373 | 3.637 |
| 1061 | O | MET | 789 | 28.101 | 59.697 | 4.243 |
| 1062 | CB | MET | 789 | 25.429 | 58.865 | 3.514 |
| 1063 | CG | MET | 789 | 24.167 | 58.419 | 4.097 |
| 1064 | SD | MET | 789 | 23.912 | 56.709 | 3.659 |
| 1065 | CE | MET | 789 | 22.418 | 56.454 | 4.443 |
| 1066 | N | LYS | 790 | 27.641 | 61.215 | 2.696 |
| 1067 | CA | LYS | 790 | 29.031 | 61.282 | 2.344 |
| 1068 | HN | LYS | 790 | 26.965 | 61.793 | 2.238 |
| 1069 | C | LYS | 790 | 29.746 | 62.384 | 3.101 |
| 1070 | O | LYS | 790 | 30.975 | 62.481 | 3.039 |
| 1071 | CB | LYS | 790 | 29.122 | 61.512 | 0.810 |
| 1072 | CG | LYS | 790 | 27.919 | 62.298 | 0.233 |
| 1073 | CD | LYS | 790 | 27.941 | 62.487 | -1.286 |
| 1074 | CE | LYS | 790 | 26.663 | 63.214 | -1.725 |
| 1075 | NZ | LYS | 790 | 26.758 | 63.551 | -3.157 |
| 1076 | HZ2 | LYS | 790 | 27.235 | 62.803 | -3.674 |
| 1077 | HZ1 | LYS | 790 | 25.829 | 63.643 | -3.580 |
| 1078 | HZ3 | LYS | 790 | 27.261 | 64.408 | -3.265 |
| 1079 | N | GLU | 791 | 28.943 | 63.122 | 3.884 |
| 1080 | CA | GLU | 791 | 29.321 | 64.309 | 4.668 |
| 1081 | HN | GLU | 791 | 27.987 | 62.835 | 3.941 |
| 1082 | C | GLU | 791 | 30.190 | 64.269 | 5.882 |
| 1083 | O | GLU | 791 | 29.802 | 63.817 | 6.953 |
| 1084 | CB | GLU | 791 | 28.014 | 65.107 | 4.935 |
| 1085 | CG | GLU | 791 | 27.277 | 65.712 | 3.694 |
| 1086 | CD | GLU | 791 | 27.943 | 66.869 | 2.946 |
| 1087 | OE1 | GLU | 791 | 28.629 | 67.715 | 3.503 |
| 1088 | OE2 | GLU | 791 | 27.700 | 66.866 | 1.606 |
| 1089 | HE2 | GLU | 791 | 28.137 | 67.602 | 1.205 |
| 1090 | N | SER | 792 | 31.290 | 64.992 | 5.774 |
| 1091 | HN | SER | 792 | 31.460 | 65.527 | 4.911 |
| 1092 | CA | SER | 792 | 32.264 | 65.049 | 6.850 |
| 1093 | C | SER | 792 | 32.067 | 64.179 | 8.078 |
| 1094 | O | SER | 792 | 32.972 | 63.485 | 8.496 |
| 1095 | CB | SER | 792 | 32.290 | 66.554 | 7.217 |
| 1096 | OG | SER | 792 | 32.013 | 67.404 | 6.098 |
| 1097 | HG | SER | 792 | 32.714 | 67.269 | 5.455 |
| 1098 | N | SER | 793 | 30.907 | 64.132 | 8.672 |
| 1099 | CA | SER | 793 | 30.965 | 63.399 | 9.897 |
| 1100 | HN | SER | 793 | 30.077 | 64.558 | 8.313 |

| | | | | | | |
|---|---|---|---|---|---|---|
| 1101 | C | SER | 793 | 29.791 | 62.534 | 10.105 |
| 1102 | O | SER | 793 | 29.540 | 62.065 | 11.206 |
| 1103 | CB | SER | 793 | 31.071 | 64.489 | 10.994 |
| 1104 | OG | SER | 793 | 32.304 | 64.430 | 11.719 |
| 1105 | HG | SER | 793 | 32.330 | 63.584 | 12.174 |
| 1106 | N | PHE | 794 | 29.108 | 62.259 | 9.021 |
| 1107 | CA | PHE | 794 | 27.866 | 61.582 | 9.147 |
| 1108 | HN | PHE | 794 | 29.455 | 62.522 | 8.121 |
| 1109 | C | PHE | 794 | 27.801 | 60.103 | 9.326 |
| 1110 | O | PHE | 794 | 27.198 | 59.595 | 10.283 |
| 1111 | CB | PHE | 794 | 26.966 | 61.982 | 7.939 |
| 1112 | CG | PHE | 794 | 26.406 | 63.413 | 7.948 |
| 1113 | CD1 | PHE | 794 | 26.710 | 64.269 | 9.013 |
| 1114 | CE1 | PHE | 794 | 26.147 | 65.538 | 9.076 |
| 1115 | CZ | PHE | 794 | 25.289 | 65.968 | 8.067 |
| 1116 | CE2 | PHE | 794 | 24.990 | 65.126 | 6.997 |
| 1117 | CD2 | PHE | 794 | 25.545 | 63.850 | 6.938 |
| 1118 | N | TYR | 795 | 28.424 | 59.411 | 8.394 |
| 1119 | CA | TYR | 795 | 28.382 | 57.988 | 8.407 |
| 1120 | HN | TYR | 795 | 28.928 | 59.887 | 7.673 |
| 1121 | C | TYR | 795 | 28.762 | 57.515 | 9.775 |
| 1122 | O | TYR | 795 | 28.033 | 56.784 | 10.449 |
| 1123 | CB | TYR | 795 | 29.348 | 57.438 | 7.376 |
| 1124 | CG | TYR | 795 | 29.176 | 55.974 | 7.269 |
| 1125 | CD1 | TYR | 795 | 28.002 | 55.450 | 6.752 |
| 1126 | CE1 | TYR | 795 | 27.745 | 54.108 | 6.812 |
| 1127 | CZ | TYR | 795 | 28.671 | 53.268 | 7.389 |
| 1128 | OH | TYR | 795 | 28.414 | 51.916 | 7.449 |
| 1129 | HH | TYR | 795 | 29.242 | 51.461 | 7.639 |
| 1130 | CE2 | TYR | 795 | 29.855 | 53.763 | 7.897 |
| 1131 | CD2 | TYR | 795 | 30.101 | 55.111 | 7.832 |
| 1132 | N | SER | 796 | 29.891 | 58.001 | 10.221 |
| 1133 | CA | SER | 796 | 30.342 | 57.544 | 11.482 |
| 1134 | HN | SER | 796 | 30.414 | 58.669 | 9.692 |
| 1135 | C | SER | 796 | 29.351 | 57.385 | 12.601 |
| 1136 | O | SER | 796 | 29.510 | 56.535 | 13.475 |
| 1137 | CB | SER | 796 | 31.546 | 58.421 | 11.908 |
| 1138 | OG | SER | 796 | 32.608 | 57.662 | 12.496 |
| 1139 | HG | SER | 796 | 32.265 | 57.264 | 13.300 |
| 1140 | N | LEU | 797 | 28.310 | 58.183 | 12.555 |
| 1141 | CA | LEU | 797 | 27.317 | 58.140 | 13.581 |
| 1142 | HN | LEU | 797 | 28.212 | 58.827 | 11.796 |
| 1143 | C | LEU | 797 | 26.033 | 57.625 | 13.033 |
| 1144 | O | LEU | 797 | 25.110 | 57.369 | 13.780 |
| 1145 | CB | LEU | 797 | 27.158 | 59.601 | 14.089 |
| 1146 | CG | LEU | 797 | 28.428 | 60.347 | 14.580 |
| 1147 | CD1 | LEU | 797 | 28.597 | 60.160 | 16.094 |
| 1148 | CD2 | LEU | 797 | 29.704 | 59.888 | 13.856 |
| 1149 | N | CYS | 798 | 25.928 | 57.473 | 11.730 |
| 1150 | CA | CYS | 798 | 24.626 | 57.048 | 11.241 |
| 1151 | HN | CYS | 798 | 26.698 | 57.640 | 11.114 |
| 1152 | C | CYS | 798 | 24.672 | 55.630 | 11.032 |
| 1153 | O | CYS | 798 | 23.734 | 54.964 | 10.598 |
| 1154 | CB | CYS | 798 | 24.304 | 57.699 | 9.924 |
| 1155 | SG | CYS | 798 | 25.429 | 57.249 | 8.557 |

| | | | | | | |
|---|---|---|---|---|---|---|
| 1156 | HG | CYS | 798 | 25.052 | 57.880 | 7.443 |
| 1157 | N | LEU | 799 | 25.779 | 55.124 | 11.438 |
| 1158 | CA | LEU | 799 | 25.912 | 53.807 | 11.104 |
| 1159 | HN | LEU | 799 | 26.470 | 55.639 | 11.944 |
| 1160 | C | LEU | 799 | 25.261 | 52.926 | 12.098 |
| 1161 | O | LEU | 799 | 24.784 | 51.861 | 11.743 |
| 1162 | CB | LEU | 799 | 27.437 | 53.512 | 11.014 |
| 1163 | CG | LEU | 799 | 27.888 | 52.198 | 10.320 |
| 1164 | CD1 | LEU | 799 | 29.402 | 52.227 | 10.070 |
| 1165 | CD2 | LEU | 799 | 27.519 | 50.943 | 11.127 |
| 1166 | N | THR | 800 | 25.210 | 53.364 | 13.345 |
| 1167 | CA | THR | 800 | 24.614 | 52.485 | 14.303 |
| 1168 | HN | THR | 800 | 25.569 | 54.261 | 13.604 |
| 1169 | C | THR | 800 | 23.134 | 52.463 | 14.182 |
| 1170 | O | THR | 800 | 22.489 | 51.556 | 14.682 |
| 1171 | CB | THR | 800 | 25.044 | 52.867 | 15.760 |
| 1172 | OG1 | THR | 800 | 26.408 | 52.530 | 15.980 |
| 1173 | HG1 | THR | 800 | 26.624 | 52.853 | 16.860 |
| 1174 | CG2 | THR | 800 | 24.282 | 52.165 | 16.905 |
| 1175 | N | MET | 801 | 22.602 | 53.452 | 13.488 |
| 1176 | CA | MET | 801 | 21.170 | 53.504 | 13.266 |
| 1177 | HN | MET | 801 | 23.189 | 54.169 | 13.114 |
| 1178 | C | MET | 801 | 20.868 | 52.600 | 12.120 |
| 1179 | O | MET | 801 | 19.942 | 51.793 | 12.150 |
| 1180 | CB | MET | 801 | 20.762 | 54.886 | 12.872 |
| 1181 | CG | MET | 801 | 20.691 | 55.778 | 14.039 |
| 1182 | SD | MET | 801 | 20.060 | 57.362 | 13.567 |
| 1183 | CE | MET | 801 | 21.271 | 57.817 | 12.406 |
| 1184 | N | TRP | 802 | 21.643 | 52.808 | 11.071 |
| 1185 | CA | TRP | 802 | 21.545 | 52.016 | 9.879 |
| 1186 | HN | TRP | 802 | 22.321 | 53.542 | 11.110 |
| 1187 | C | TRP | 802 | 21.468 | 50.589 | 10.305 |
| 1188 | O | TRP | 802 | 20.836 | 49.738 | 9.690 |
| 1189 | CB | TRP | 802 | 22.844 | 52.252 | 9.060 |
| 1190 | CG | TRP | 802 | 22.891 | 51.531 | 7.709 |
| 1191 | CD1 | TRP | 802 | 23.488 | 50.276 | 7.467 |
| 1192 | NE1 | TRP | 802 | 23.432 | 49.930 | 6.103 |
| 1193 | CE2 | TRP | 802 | 22.790 | 51.007 | 5.513 |
| 1194 | CD2 | TRP | 802 | 22.457 | 51.986 | 6.482 |
| 1195 | HE1 | TRP | 802 | 23.821 | 49.099 | 5.643 |
| 1196 | CE3 | TRP | 802 | 21.816 | 53.192 | 6.099 |
| 1197 | CZ3 | TRP | 802 | 21.507 | 53.379 | 4.752 |
| 1198 | CH2 | TRP | 802 | 21.828 | 52.408 | 3.793 |
| 1199 | CZ2 | TRP | 802 | 22.472 | 51.222 | 4.154 |
| 1200 | N | GLN | 803 | 22.153 | 50.339 | 11.396 |
| 1201 | CA | GLN | 803 | 22.243 | 49.017 | 11.904 |
| 1202 | HN | GLN | 803 | 22.616 | 51.086 | 11.873 |
| 1203 | C | GLN | 803 | 20.950 | 48.353 | 12.340 |
| 1204 | O | GLN | 803 | 20.775 | 47.145 | 12.174 |
| 1205 | CB | GLN | 803 | 23.270 | 49.040 | 13.058 |
| 1206 | CG | GLN | 803 | 23.347 | 47.750 | 13.940 |
| 1207 | CD | GLN | 803 | 23.993 | 46.486 | 13.360 |
| 1208 | OE1 | GLN | 803 | 23.982 | 45.432 | 13.976 |
| 1209 | NE2 | GLN | 803 | 24.591 | 46.532 | 12.197 |
| 1210 | 2HE2 | GLN | 803 | 24.626 | 47.457 | 11.766 |

| | | | | | | |
|---|---|---|---|---|---|---|
| 1211 | 1HE2 | GLN | 803 | 25.051 | 45.665 | 11.913 |
| 1212 | N | ILE | 804 | 20.050 | 49.131 | 12.906 |
| 1213 | CA | ILE | 804 | 18.817 | 48.561 | 13.415 |
| 1214 | HN | ILE | 804 | 20.217 | 50.114 | 12.985 |
| 1215 | C | ILE | 804 | 17.808 | 48.322 | 12.301 |
| 1216 | O | ILE | 804 | 17.107 | 47.307 | 12.280 |
| 1217 | CB | ILE | 804 | 18.233 | 49.500 | 14.547 |
| 1218 | CG1 | ILE | 804 | 19.231 | 50.578 | 15.080 |
| 1219 | CG2 | ILE | 804 | 17.683 | 48.703 | 15.770 |
| 1220 | CD1 | ILE | 804 | 18.615 | 51.703 | 15.936 |
| 1221 | N | PRO | 805 | 17.761 | 49.278 | 11.384 |
| 1222 | CA | PRO | 805 | 16.882 | 49.259 | 10.220 |
| 1223 | C | PRO | 805 | 17.249 | 48.056 | 9.390 |
| 1224 | O | PRO | 805 | 16.449 | 47.474 | 8.650 |
| 1225 | CB | PRO | 805 | 17.108 | 50.588 | 9.484 |
| 1226 | CG | PRO | 805 | 18.553 | 50.963 | 9.848 |
| 1227 | CD | PRO | 805 | 18.675 | 50.520 | 11.308 |
| 1228 | N | GLN | 806 | 18.511 | 47.706 | 9.525 |
| 1229 | CA | GLN | 806 | 19.063 | 46.614 | 8.791 |
| 1230 | HN | GLN | 806 | 19.095 | 48.217 | 10.156 |
| 1231 | C | GLN | 806 | 18.670 | 45.295 | 9.410 |
| 1232 | O | GLN | 806 | 18.528 | 44.284 | 8.737 |
| 1233 | CB | GLN | 806 | 20.598 | 46.786 | 8.783 |
| 1234 | CG | GLN | 806 | 21.441 | 45.470 | 8.858 |
| 1235 | CD | GLN | 806 | 22.971 | 45.564 | 8.916 |
| 1236 | OE1 | GLN | 806 | 23.661 | 44.563 | 9.015 |
| 1237 | NE2 | GLN | 806 | 23.555 | 46.735 | 8.884 |
| 1238 | 2HE2 | GLN | 806 | 22.920 | 47.534 | 8.870 |
| 1239 | 1HE2 | GLN | 806 | 24.571 | 46.713 | 8.992 |
| 1240 | N | GLU | 807 | 18.482 | 45.308 | 10.714 |
| 1241 | CA | GLU | 807 | 18.132 | 44.091 | 11.393 |
| 1242 | HN | GLU | 807 | 18.583 | 46.160 | 11.228 |
| 1243 | C | GLU | 807 | 16.669 | 43.824 | 11.239 |
| 1244 | O | GLU | 807 | 16.231 | 42.679 | 11.130 |
| 1245 | CB | GLU | 807 | 18.518 | 44.229 | 12.845 |
| 1246 | CG | GLU | 807 | 19.997 | 44.150 | 12.996 |
| 1247 | CD | GLU | 807 | 20.469 | 42.740 | 12.723 |
| 1248 | OE1 | GLU | 807 | 20.203 | 41.865 | 13.569 |
| 1249 | OE2 | GLU | 807 | 21.082 | 42.493 | 11.663 |
| 1250 | HE2 | GLU | 807 | 21.309 | 41.576 | 11.637 |
| 1251 | N | PHE | 808 | 15.910 | 44.900 | 11.228 |
| 1252 | CA | PHE | 808 | 14.478 | 44.796 | 11.066 |
| 1253 | HN | PHE | 808 | 16.330 | 45.802 | 11.332 |
| 1254 | C | PHE | 808 | 14.232 | 44.138 | 9.744 |
| 1255 | O | PHE | 808 | 13.408 | 43.246 | 9.581 |
| 1256 | CB | PHE | 808 | 13.861 | 46.228 | 11.038 |
| 1257 | CG | PHE | 808 | 13.638 | 46.903 | 12.400 |
| 1258 | CD1 | PHE | 808 | 13.232 | 48.242 | 12.450 |
| 1259 | CE1 | PHE | 808 | 13.103 | 48.891 | 13.673 |
| 1260 | CZ | PHE | 808 | 13.363 | 48.202 | 14.855 |
| 1261 | CE2 | PHE | 808 | 13.757 | 46.866 | 14.814 |
| 1262 | CD2 | PHE | 808 | 13.897 | 46.218 | 13.589 |
| 1263 | N | VAL | 809 | 14.977 | 44.617 | 8.782 |
| 1264 | CA | VAL | 809 | 14.838 | 44.102 | 7.476 |
| 1265 | HN | VAL | 809 | 15.639 | 45.342 | 8.971 |

| | | | | | | |
|---|---|---|---|---|---|---|
| 1266 | C | VAL | 809 | 15.236 | 42.654 | 7.417 |
| 1267 | O | VAL | 809 | 14.511 | 41.796 | 6.912 |
| 1268 | CB | VAL | 809 | 15.760 | 44.976 | 6.536 |
| 1269 | CG1 | VAL | 809 | 15.758 | 44.603 | 5.028 |
| 1270 | CG2 | VAL | 809 | 15.445 | 46.491 | 6.582 |
| 1271 | N | LYS | 810 | 16.426 | 42.387 | 7.907 |
| 1272 | CA | LYS | 810 | 16.928 | 41.042 | 7.870 |
| 1273 | HN | LYS | 810 | 16.981 | 43.119 | 8.304 |
| 1274 | C | LYS | 810 | 16.024 | 40.070 | 8.594 |
| 1275 | O | LYS | 810 | 15.810 | 38.937 | 8.166 |
| 1276 | CB | LYS | 810 | 18.353 | 41.041 | 8.489 |
| 1277 | CG | LYS | 810 | 18.830 | 39.637 | 8.937 |
| 1278 | CD | LYS | 810 | 20.248 | 39.591 | 9.511 |
| 1279 | CE | LYS | 810 | 20.176 | 39.645 | 11.043 |
| 1280 | NZ | LYS | 810 | 19.606 | 38.385 | 11.551 |
| 1281 | HZ2 | LYS | 810 | 19.931 | 37.588 | 10.990 |
| 1282 | HZ1 | LYS | 810 | 19.911 | 38.199 | 12.512 |
| 1283 | HZ3 | LYS | 810 | 18.608 | 38.433 | 11.520 |
| 1284 | N | LEU | 811 | 15.495 | 40.520 | 9.712 |
| 1285 | CA | LEU | 811 | 14.655 | 39.668 | 10.510 |
| 1286 | HN | LEU | 811 | 15.678 | 41.458 | 10.007 |
| 1287 | C | LEU | 811 | 13.234 | 39.665 | 10.035 |
| 1288 | O | LEU | 811 | 12.397 | 38.918 | 10.547 |
| 1289 | CB | LEU | 811 | 14.713 | 40.140 | 11.936 |
| 1290 | CG | LEU | 811 | 15.052 | 39.039 | 12.925 |
| 1291 | CD1 | LEU | 811 | 16.041 | 38.065 | 12.339 |
| 1292 | CD2 | LEU | 811 | 15.613 | 39.703 | 14.158 |
| 1293 | N | GLN | 812 | 12.952 | 40.532 | 9.071 |
| 1294 | CA | GLN | 812 | 11.615 | 40.583 | 8.549 |
| 1295 | HN | GLN | 812 | 13.660 | 41.140 | 8.713 |
| 1296 | C | GLN | 812 | 10.696 | 40.731 | 9.721 |
| 1297 | O | GLN | 812 | 9.911 | 39.842 | 10.019 |
| 1298 | CB | GLN | 812 | 11.347 | 39.285 | 7.826 |
| 1299 | CG | GLN | 812 | 12.088 | 39.230 | 6.534 |
| 1300 | CD | GLN | 812 | 11.426 | 40.137 | 5.538 |
| 1301 | OE1 | GLN | 812 | 12.016 | 41.104 | 5.053 |
| 1302 | NE2 | GLN | 812 | 10.143 | 39.757 | 5.076 |
| 1303 | 1HE2 | GLN | 812 | 9.717 | 40.296 | 4.336 |
| 1304 | 2HE2 | GLN | 812 | 9.714 | 38.902 | 5.392 |
| 1305 | N | VAL | 813 | 10.802 | 41.849 | 10.410 |
| 1306 | CA | VAL | 813 | 9.957 | 42.060 | 11.554 |
| 1307 | HN | VAL | 813 | 11.466 | 42.546 | 10.139 |
| 1308 | C | VAL | 813 | 8.637 | 42.654 | 11.111 |
| 1309 | O | VAL | 813 | 8.601 | 43.514 | 10.245 |
| 1310 | CB | VAL | 813 | 10.678 | 42.954 | 12.576 |
| 1311 | CG1 | VAL | 813 | 9.686 | 43.569 | 13.534 |
| 1312 | CG2 | VAL | 813 | 11.707 | 42.109 | 13.330 |
| 1313 | N | SER | 814 | 7.550 | 42.174 | 11.707 |
| 1314 | CA | SER | 814 | 6.206 | 42.646 | 11.374 |
| 1315 | HN | SER | 814 | 7.654 | 41.466 | 12.405 |
| 1316 | C | SER | 814 | 5.740 | 43.761 | 12.307 |
| 1317 | O | SER | 814 | 6.142 | 43.810 | 13.468 |
| 1318 | CB | SER | 814 | 5.222 | 41.499 | 11.479 |
| 1319 | OG | SER | 814 | 4.854 | 41.159 | 12.814 |
| 1320 | HG | SER | 814 | 4.170 | 40.479 | 12.754 |

| | | | | | | |
|---|---|---|---|---|---|---|
| 1321 | N | GLN | 815 | 4.866 | 44.635 | 11.811 |
| 1322 | CA | GLN | 815 | 4.351 | 45.754 | 12.613 |
| 1323 | HN | GLN | 815 | 4.552 | 44.527 | 10.867 |
| 1324 | C | GLN | 815 | 3.904 | 45.330 | 13.998 |
| 1325 | O | GLN | 815 | 4.118 | 46.035 | 14.988 |
| 1326 | CB | GLN | 815 | 3.195 | 46.426 | 11.840 |
| 1327 | CG | GLN | 815 | 2.824 | 47.881 | 12.278 |
| 1328 | CD | GLN | 815 | 3.920 | 48.953 | 12.315 |
| 1329 | OE1 | GLN | 815 | 3.652 | 50.122 | 12.543 |
| 1330 | NE2 | GLN | 815 | 5.164 | 48.624 | 12.073 |
| 1331 | 2HE2 | GLN | 815 | 5.311 | 47.646 | 11.821 |
| 1332 | 1HE2 | GLN | 815 | 5.821 | 49.407 | 12.053 |
| 1333 | N | GLU | 816 | 3.249 | 44.187 | 14.073 |
| 1334 | CA | GLU | 816 | 2.791 | 43.772 | 15.369 |
| 1335 | HN | GLU | 816 | 3.079 | 43.631 | 13.259 |
| 1336 | C | GLU | 816 | 3.939 | 43.440 | 16.298 |
| 1337 | O | GLU | 816 | 3.918 | 43.859 | 17.456 |
| 1338 | CB | GLU | 816 | 1.775 | 42.645 | 15.241 |
| 1339 | CG | GLU | 816 | 0.366 | 43.221 | 15.199 |
| 1340 | CD | GLU | 816 | -0.716 | 42.166 | 15.204 |
| 1341 | OE1 | GLU | 816 | -0.619 | 41.208 | 16.010 |
| 1342 | OE2 | GLU | 816 | -1.673 | 42.306 | 14.407 |
| 1343 | HE2 | GLU | 816 | -2.276 | 41.586 | 14.509 |
| 1344 | N | GLU | 817 | 4.954 | 42.726 | 15.821 |
| 1345 | CA | GLU | 817 | 6.095 | 42.447 | 16.690 |
| 1346 | HN | GLU | 817 | 4.937 | 42.386 | 14.881 |
| 1347 | C | GLU | 817 | 6.767 | 43.775 | 17.046 |
| 1348 | O | GLU | 817 | 7.106 | 44.024 | 18.195 |
| 1349 | CB | GLU | 817 | 7.107 | 41.550 | 15.999 |
| 1350 | CG | GLU | 817 | 6.523 | 40.254 | 15.554 |
| 1351 | CD | GLU | 817 | 7.381 | 39.567 | 14.525 |
| 1352 | OE1 | GLU | 817 | 7.888 | 40.268 | 13.614 |
| 1353 | OE2 | GLU | 817 | 7.534 | 38.332 | 14.621 |
| 1354 | HE2 | GLU | 817 | 8.090 | 38.028 | 13.919 |
| 1355 | N | PHE | 818 | 6.961 | 44.631 | 16.052 |
| 1356 | CA | PHE | 818 | 7.599 | 45.926 | 16.270 |
| 1357 | HN | PHE | 818 | 6.663 | 44.383 | 15.130 |
| 1358 | C | PHE | 818 | 6.930 | 46.789 | 17.341 |
| 1359 | O | PHE | 818 | 7.599 | 47.422 | 18.157 |
| 1360 | CB | PHE | 818 | 7.619 | 46.716 | 14.927 |
| 1361 | CG | PHE | 818 | 7.823 | 48.236 | 15.031 |
| 1362 | CD1 | PHE | 818 | 9.110 | 48.750 | 15.221 |
| 1363 | CE1 | PHE | 818 | 9.326 | 50.123 | 15.217 |
| 1364 | CZ | PHE | 818 | 8.258 | 50.993 | 15.006 |
| 1365 | CE2 | PHE | 818 | 6.975 | 50.488 | 14.804 |
| 1366 | CD2 | PHE | 818 | 6.756 | 49.113 | 14.818 |
| 1367 | N | LEU | 819 | 5.608 | 46.853 | 17.289 |
| 1368 | CA | LEU | 819 | 4.847 | 47.635 | 18.238 |
| 1369 | HN | LEU | 819 | 5.123 | 46.347 | 16.575 |
| 1370 | C | LEU | 819 | 5.158 | 47.193 | 19.666 |
| 1371 | O | LEU | 819 | 5.472 | 48.013 | 20.534 |
| 1372 | CB | LEU | 819 | 3.348 | 47.483 | 17.961 |
| 1373 | CG | LEU | 819 | 2.776 | 48.377 | 16.869 |
| 1374 | CD1 | LEU | 819 | 1.284 | 48.208 | 16.796 |
| 1375 | CD2 | LEU | 819 | 3.116 | 49.813 | 17.180 |

| | | | | | | |
|---|---|---|---|---|---|---|
| 1376 | N | CYS | 820 | 5.049 | 45.886 | 19.882 |
| 1377 | CA | CYS | 820 | 5.315 | 45.273 | 21.168 |
| 1378 | HN | CYS | 820 | 4.771 | 45.298 | 19.122 |
| 1379 | C | CYS | 820 | 6.753 | 45.478 | 21.587 |
| 1380 | O | CYS | 820 | 7.049 | 45.668 | 22.759 |
| 1381 | CB | CYS | 820 | 5.047 | 43.781 | 21.103 |
| 1382 | SG | CYS | 820 | 3.280 | 43.364 | 20.905 |
| 1383 | HG | CYS | 820 | 3.138 | 42.038 | 20.857 |
| 1384 | N | MET | 821 | 7.657 | 45.430 | 20.633 |
| 1385 | CA | MET | 821 | 9.054 | 45.604 | 20.957 |
| 1386 | HN | MET | 821 | 7.378 | 45.273 | 19.686 |
| 1387 | C | MET | 821 | 9.407 | 47.017 | 21.406 |
| 1388 | O | MET | 821 | 10.286 | 47.205 | 22.252 |
| 1389 | CB | MET | 821 | 9.920 | 45.227 | 19.760 |
| 1390 | CG | MET | 821 | 10.038 | 43.744 | 19.538 |
| 1391 | SD | MET | 821 | 11.149 | 43.450 | 18.194 |
| 1392 | CE | MET | 821 | 10.046 | 43.457 | 16.968 |
| 1393 | N | LYS | 822 | 8.738 | 48.013 | 20.833 |
| 1394 | CA | LYS | 822 | 9.021 | 49.393 | 21.192 |
| 1395 | HN | LYS | 822 | 8.035 | 47.811 | 20.150 |
| 1396 | C | LYS | 822 | 8.476 | 49.690 | 22.582 |
| 1397 | O | LYS | 822 | 9.008 | 50.541 | 23.304 |
| 1398 | CB | LYS | 822 | 8.393 | 50.324 | 20.170 |
| 1399 | CG | LYS | 822 | 8.973 | 51.714 | 20.152 |
| 1400 | CD | LYS | 822 | 8.431 | 52.481 | 18.948 |
| 1401 | CE | LYS | 822 | 6.976 | 52.947 | 19.146 |
| 1402 | NZ | LYS | 822 | 5.879 | 51.911 | 19.313 |
| 1403 | HZ1 | LYS | 822 | 4.968 | 52.385 | 19.393 |
| 1404 | HZ2 | LYS | 822 | 5.869 | 51.286 | 18.495 |
| 1405 | HZ3 | LYS | 822 | 6.049 | 51.374 | 20.139 |
| 1406 | N | VAL | 823 | 7.405 | 48.990 | 22.947 |
| 1407 | CA | VAL | 823 | 6.794 | 49.152 | 24.249 |
| 1408 | HN | VAL | 823 | 7.012 | 48.333 | 22.304 |
| 1409 | C | VAL | 823 | 7.792 | 48.552 | 25.214 |
| 1410 | O | VAL | 823 | 8.098 | 49.138 | 26.238 |
| 1411 | CB | VAL | 823 | 5.416 | 48.382 | 24.314 |
| 1412 | CG1 | VAL | 823 | 4.716 | 48.337 | 25.701 |
| 1413 | CG2 | VAL | 823 | 4.347 | 48.924 | 23.335 |
| 1414 | N | LEU | 824 | 8.316 | 47.381 | 24.860 |
| 1415 | CA | LEU | 824 | 9.318 | 46.724 | 25.680 |
| 1416 | HN | LEU | 824 | 8.014 | 46.945 | 24.013 |
| 1417 | C | LEU | 824 | 10.542 | 47.619 | 25.856 |
| 1418 | O | LEU | 824 | 11.126 | 47.645 | 26.920 |
| 1419 | CB | LEU | 824 | 9.732 | 45.399 | 25.057 |
| 1420 | CG | LEU | 824 | 9.203 | 44.140 | 25.744 |
| 1421 | CD1 | LEU | 824 | 8.142 | 44.484 | 26.747 |
| 1422 | CD2 | LEU | 824 | 8.656 | 43.185 | 24.714 |
| 1423 | N | LEU | 825 | 10.939 | 48.369 | 24.839 |
| 1424 | CA | LEU | 825 | 12.107 | 49.226 | 25.037 |
| 1425 | HN | LEU | 825 | 10.456 | 48.350 | 23.963 |
| 1426 | C | LEU | 825 | 11.882 | 50.322 | 26.062 |
| 1427 | O | LEU | 825 | 12.837 | 50.825 | 26.638 |
| 1428 | CB | LEU | 825 | 12.541 | 49.892 | 23.740 |
| 1429 | CG | LEU | 825 | 13.182 | 49.049 | 22.650 |
| 1430 | CD1 | LEU | 825 | 13.307 | 49.968 | 21.459 |

| | | | | | | |
|---|---|---|---|---|---|---|
| 1431 | CD2 | LEU | 825 | 14.549 | 48.490 | 23.053 |
| 1432 | N | LEU | 826 | 10.630 | 50.712 | 26.265 |
| 1433 | CA | LEU | 826 | 10.312 | 51.764 | 27.230 |
| 1434 | HN | LEU | 826 | 9.890 | 50.278 | 25.750 |
| 1435 | C | LEU | 826 | 10.574 | 51.184 | 28.611 |
| 1436 | O | LEU | 826 | 10.954 | 51.899 | 29.541 |
| 1437 | CB | LEU | 826 | 8.840 | 52.169 | 27.109 |
| 1438 | CG | LEU | 826 | 8.322 | 53.116 | 28.189 |
| 1439 | CD1 | LEU | 826 | 9.120 | 54.420 | 28.142 |
| 1440 | CD2 | LEU | 826 | 6.831 | 53.361 | 27.988 |
| 1441 | N | LEU | 827 | 10.382 | 49.872 | 28.710 |
| 1442 | CA | LEU | 827 | 10.577 | 49.134 | 29.944 |
| 1443 | HN | LEU | 827 | 10.089 | 49.370 | 27.896 |
| 1444 | C | LEU | 827 | 11.923 | 48.441 | 29.920 |
| 1445 | O | LEU | 827 | 12.131 | 47.479 | 30.657 |
| 1446 | CB | LEU | 827 | 9.495 | 48.072 | 30.065 |
| 1447 | CG | LEU | 827 | 8.104 | 48.624 | 29.807 |
| 1448 | CD1 | LEU | 827 | 7.060 | 47.528 | 29.964 |
| 1449 | CD2 | LEU | 827 | 7.865 | 49.767 | 30.773 |
| 1450 | N | ASN | 828 | 12.837 | 48.936 | 29.093 |
| 1451 | CA | ASN | 828 | 14.140 | 48.299 | 28.921 |
| 1452 | HN | ASN | 828 | 12.627 | 49.766 | 28.575 |
| 1453 | C | ASN | 828 | 15.279 | 48.552 | 29.904 |
| 1454 | O | ASN | 828 | 16.359 | 47.972 | 29.754 |
| 1455 | CB | ASN | 828 | 14.611 | 48.542 | 27.457 |
| 1456 | CG | ASN | 828 | 15.909 | 47.853 | 27.023 |
| 1457 | OD1 | ASN | 828 | 16.588 | 48.268 | 26.095 |
| 1458 | ND2 | ASN | 828 | 16.289 | 46.773 | 27.651 |
| 1459 | 1HD2 | ASN | 828 | 17.076 | 46.309 | 27.188 |
| 1460 | 2HD2 | ASN | 828 | 15.655 | 46.401 | 28.358 |
| 1461 | N | THR | 829 | 15.084 | 49.428 | 30.877 |
| 1462 | CA | THR | 829 | 16.135 | 49.624 | 31.870 |
| 1463 | HN | THR | 829 | 14.231 | 49.947 | 30.930 |
| 1464 | C | THR | 829 | 15.597 | 50.417 | 33.047 |
| 1465 | O | THR | 829 | 14.769 | 51.313 | 32.899 |
| 1466 | CB | THR | 829 | 17.426 | 50.297 | 31.293 |
| 1467 | OG1 | THR | 829 | 17.180 | 51.662 | 30.983 |
| 1468 | HG1 | THR | 829 | 18.024 | 52.029 | 30.704 |
| 1469 | CG2 | THR | 829 | 17.984 | 49.697 | 29.985 |
| 1470 | N | ILE | 830 | 16.033 | 50.023 | 34.233 |
| 1471 | CA | ILE | 830 | 15.585 | 50.666 | 35.447 |
| 1472 | HN | ILE | 830 | 16.684 | 49.267 | 34.291 |
| 1473 | C | ILE | 830 | 16.791 | 51.117 | 36.256 |
| 1474 | O | ILE | 830 | 17.930 | 50.741 | 35.961 |
| 1475 | CB | ILE | 830 | 14.665 | 49.670 | 36.265 |
| 1476 | CG1 | ILE | 830 | 13.280 | 49.373 | 35.607 |
| 1477 | CG2 | ILE | 830 | 14.414 | 50.145 | 37.730 |
| 1478 | CD1 | ILE | 830 | 12.498 | 48.179 | 36.193 |
| 1479 | N | PRO | 831 | 16.563 | 51.958 | 37.275 |
| 1480 | CA | PRO | 831 | 17.668 | 52.431 | 38.099 |
| 1481 | CD | PRO | 831 | 15.277 | 52.419 | 37.819 |
| 1482 | C | PRO | 831 | 18.186 | 51.221 | 38.868 |
| 1483 | O | PRO | 831 | 17.402 | 50.334 | 39.217 |
| 1484 | CB | PRO | 831 | 16.993 | 53.465 | 38.994 |
| 1485 | CG | PRO | 831 | 15.648 | 52.866 | 39.206 |

| | | | | | | |
|---|---|---|---|---|---|---|
| 1486 | N | LEU | 832 | 19.494 | 51.150 | 39.095 |
| 1487 | CA | LEU | 832 | 20.052 | 50.018 | 39.834 |
| 1488 | HN | LEU | 832 | 20.098 | 51.873 | 38.760 |
| 1489 | C | LEU | 832 | 19.707 | 50.210 | 41.288 |
| 1490 | O | LEU | 832 | 20.462 | 50.805 | 42.041 |
| 1491 | CB | LEU | 832 | 21.592 | 49.932 | 39.634 |
| 1492 | CG | LEU | 832 | 22.428 | 49.192 | 40.713 |
| 1493 | CD1 | LEU | 832 | 21.838 | 47.800 | 40.976 |
| 1494 | CD2 | LEU | 832 | 23.911 | 49.063 | 40.329 |
| 1495 | N | GLU | 833 | 18.554 | 49.692 | 41.671 |
| 1496 | CA | GLU | 833 | 18.050 | 49.804 | 43.023 |
| 1497 | HN | GLU | 833 | 18.004 | 49.201 | 40.995 |
| 1498 | C | GLU | 833 | 16.579 | 49.552 | 42.796 |
| 1499 | O | GLU | 833 | 15.893 | 48.957 | 43.627 |
| 1500 | CB | GLU | 833 | 18.296 | 51.227 | 43.599 |
| 1501 | CG | GLU | 833 | 19.635 | 51.466 | 44.371 |
| 1502 | CD | GLU | 833 | 20.266 | 50.286 | 45.114 |
| 1503 | OE1 | GLU | 833 | 21.328 | 50.371 | 45.718 |
| 1504 | OE2 | GLU | 833 | 19.537 | 49.139 | 45.040 |
| 1505 | HE2 | GLU | 833 | 19.980 | 48.453 | 45.517 |
| 1506 | N | GLY | 834 | 16.109 | 50.000 | 41.634 |
| 1507 | CA | GLY | 834 | 14.720 | 49.791 | 41.279 |
| 1508 | HN | GLY | 834 | 16.717 | 50.483 | 41.004 |
| 1509 | C | GLY | 834 | 13.826 | 50.996 | 41.429 |
| 1510 | O | GLY | 834 | 14.203 | 52.016 | 42.013 |
| 1511 | N | LEU | 835 | 12.610 | 50.858 | 40.909 |
| 1512 | CA | LEU | 835 | 11.630 | 51.928 | 40.943 |
| 1513 | HN | LEU | 835 | 12.364 | 49.989 | 40.480 |
| 1514 | C | LEU | 835 | 10.728 | 51.875 | 42.168 |
| 1515 | O | LEU | 835 | 10.668 | 50.869 | 42.873 |
| 1516 | CB | LEU | 835 | 10.747 | 51.874 | 39.691 |
| 1517 | CG | LEU | 835 | 11.282 | 51.133 | 38.465 |
| 1518 | CD1 | LEU | 835 | 10.235 | 51.086 | 37.376 |
| 1519 | CD2 | LEU | 835 | 12.499 | 51.831 | 37.961 |
| 1520 | N | ARG | 836 | 10.023 | 52.977 | 42.394 |
| 1521 | CA | ARG | 836 | 9.092 | 53.097 | 43.507 |
| 1522 | HN | ARG | 836 | 10.136 | 53.755 | 41.776 |
| 1523 | C | ARG | 836 | 7.993 | 52.055 | 43.370 |
| 1524 | O | ARG | 836 | 7.441 | 51.575 | 44.364 |
| 1525 | CB | ARG | 836 | 8.509 | 54.537 | 43.522 |
| 1526 | CG | ARG | 836 | 9.557 | 55.664 | 43.719 |
| 1527 | CD | ARG | 836 | 8.913 | 57.007 | 44.087 |
| 1528 | NE | ARG | 836 | 9.936 | 58.073 | 43.933 |
| 1529 | CZ | ARG | 836 | 9.751 | 59.361 | 44.190 |
| 1530 | NH1 | ARG | 836 | 8.635 | 59.870 | 44.622 |
| 1531 | 1HH1 | ARG | 836 | 7.901 | 59.173 | 44.750 |
| 1532 | 2HH1 | ARG | 836 | 8.615 | 60.875 | 44.783 |
| 1533 | NH2 | ARG | 836 | 10.745 | 60.153 | 43.998 |
| 1534 | 1HH2 | ARG | 836 | 11.571 | 59.655 | 43.659 |
| 1535 | 2HH2 | ARG | 836 | 10.609 | 61.143 | 44.190 |
| 1536 | HE | ARG | 836 | 10.841 | 57.796 | 43.608 |
| 1537 | N | SER | 837 | 7.685 | 51.710 | 42.124 |
| 1538 | CA | SER | 837 | 6.638 | 50.742 | 41.831 |
| 1539 | HN | SER | 837 | 8.187 | 52.127 | 41.367 |
| 1540 | C | SER | 837 | 7.219 | 49.524 | 41.124 |

| | | | | | | |
|---|---|---|---|---|---|---|
| 1541 | O | SER | 837 | 6.549 | 48.918 | 40.287 |
| 1542 | CB | SER | 837 | 5.578 | 51.392 | 40.941 |
| 1543 | OG | SER | 837 | 5.265 | 52.742 | 41.226 |
| 1544 | HG | SER | 837 | 6.121 | 53.196 | 41.183 |
| 1545 | N | GLN | 838 | 8.456 | 49.178 | 41.466 |
| 1546 | CA | GLN | 838 | 9.150 | 48.037 | 40.861 |
| 1547 | HN | GLN | 838 | 8.931 | 49.716 | 42.162 |
| 1548 | C | GLN | 838 | 8.192 | 46.878 | 40.678 |
| 1549 | O | GLN | 838 | 8.146 | 46.245 | 39.621 |
| 1550 | CB | GLN | 838 | 10.317 | 47.597 | 41.749 |
| 1551 | CG | GLN | 838 | 11.126 | 46.435 | 41.192 |
| 1552 | CD | GLN | 838 | 11.754 | 46.736 | 39.838 |
| 1553 | OE1 | GLN | 838 | 12.593 | 47.637 | 39.708 |
| 1554 | NE2 | GLN | 838 | 11.300 | 46.051 | 38.710 |
| 1555 | 1HE2 | GLN | 838 | 11.713 | 46.227 | 37.810 |
| 1556 | 2HE2 | GLN | 838 | 10.698 | 45.239 | 38.819 |
| 1557 | N | THR | 839 | 7.428 | 46.617 | 41.721 |
| 1558 | CA | THR | 839 | 6.438 | 45.559 | 41.743 |
| 1559 | HN | THR | 839 | 7.538 | 47.180 | 42.541 |
| 1560 | C | THR | 839 | 5.617 | 45.521 | 40.432 |
| 1561 | O | THR | 839 | 5.665 | 44.542 | 39.674 |
| 1562 | CB | THR | 839 | 5.497 | 45.756 | 42.980 |
| 1563 | OG1 | THR | 839 | 5.245 | 47.137 | 43.199 |
| 1564 | HG1 | THR | 839 | 4.610 | 47.182 | 43.920 |
| 1565 | CG2 | THR | 839 | 6.029 | 45.240 | 44.334 |
| 1566 | N | GLN | 840 | 4.911 | 46.618 | 40.171 |
| 1567 | CA | GLN | 840 | 4.043 | 46.821 | 39.000 |
| 1568 | HN | GLN | 840 | 4.977 | 47.370 | 40.826 |
| 1569 | C | GLN | 840 | 4.787 | 46.827 | 37.655 |
| 1570 | O | GLN | 840 | 4.284 | 46.363 | 36.626 |
| 1571 | CB | GLN | 840 | 3.285 | 48.149 | 39.220 |
| 1572 | CG | GLN | 840 | 1.744 | 48.117 | 38.951 |
| 1573 | CD | GLN | 840 | 1.057 | 49.370 | 38.395 |
| 1574 | OE1 | GLN | 840 | -0.136 | 49.373 | 38.136 |
| 1575 | NE2 | GLN | 840 | 1.756 | 50.454 | 38.169 |
| 1576 | 2HE2 | GLN | 840 | 2.758 | 50.364 | 38.342 |
| 1577 | 1HE2 | GLN | 840 | 1.235 | 51.221 | 37.739 |
| 1578 | N | PHE | 841 | 5.974 | 47.402 | 37.672 |
| 1579 | CA | PHE | 841 | 6.803 | 47.466 | 36.494 |
| 1580 | HN | PHE | 841 | 6.308 | 47.805 | 38.525 |
| 1581 | C | PHE | 841 | 6.951 | 46.051 | 35.974 |
| 1582 | O | PHE | 841 | 6.586 | 45.728 | 34.845 |
| 1583 | CB | PHE | 841 | 8.162 | 47.986 | 36.894 |
| 1584 | CG | PHE | 841 | 9.121 | 48.038 | 35.779 |
| 1585 | CD1 | PHE | 841 | 9.173 | 49.152 | 34.962 |
| 1586 | CE1 | PHE | 841 | 10.054 | 49.210 | 33.908 |
| 1587 | CZ | PHE | 841 | 10.896 | 48.134 | 33.652 |
| 1588 | CE2 | PHE | 841 | 10.852 | 47.007 | 34.469 |
| 1589 | CD2 | PHE | 841 | 9.958 | 46.962 | 35.523 |
| 1590 | N | GLU | 842 | 7.494 | 45.200 | 36.835 |
| 1591 | CA | GLU | 842 | 7.725 | 43.824 | 36.478 |
| 1592 | HN | GLU | 842 | 7.747 | 45.520 | 37.748 |
| 1593 | C | GLU | 842 | 6.457 | 43.114 | 36.085 |
| 1594 | O | GLU | 842 | 6.497 | 42.067 | 35.439 |
| 1595 | CB | GLU | 842 | 8.446 | 43.090 | 37.643 |

| | | | | | | |
|---|---|---|---|---|---|---|
| 1596 | CG | GLU | 842 | 9.948 | 43.446 | 37.896 |
| 1597 | CD | GLU | 842 | 10.807 | 43.863 | 36.699 |
| 1598 | OE1 | GLU | 842 | 11.982 | 44.190 | 36.805 |
| 1599 | OE2 | GLU | 842 | 10.143 | 43.835 | 35.511 |
| 1600 | HE2 | GLU | 842 | 10.726 | 44.102 | 34.817 |
| 1601 | N | GLU | 843 | 5.323 | 43.685 | 36.452 |
| 1602 | CA | GLU | 843 | 4.073 | 43.059 | 36.084 |
| 1603 | HN | GLU | 843 | 5.329 | 44.536 | 36.976 |
| 1604 | C | GLU | 843 | 3.717 | 43.494 | 34.675 |
| 1605 | O | GLU | 843 | 3.334 | 42.673 | 33.831 |
| 1606 | CB | GLU | 843 | 2.973 | 43.475 | 37.028 |
| 1607 | CG | GLU | 843 | 1.740 | 42.656 | 36.823 |
| 1608 | CD | GLU | 843 | 0.550 | 43.287 | 37.463 |
| 1609 | OE1 | GLU | 843 | 0.751 | 44.057 | 38.432 |
| 1610 | OE2 | GLU | 843 | -0.579 | 43.009 | 37.001 |
| 1611 | HE2 | GLU | 843 | -1.243 | 43.470 | 37.492 |
| 1612 | N | MET | 844 | 3.845 | 44.792 | 34.429 |
| 1613 | CA | MET | 844 | 3.554 | 45.353 | 33.128 |
| 1614 | HN | MET | 844 | 4.151 | 45.398 | 35.162 |
| 1615 | C | MET | 844 | 4.558 | 44.789 | 32.155 |
| 1616 | O | MET | 844 | 4.191 | 44.248 | 31.119 |
| 1617 | CB | MET | 844 | 3.648 | 46.902 | 33.186 |
| 1618 | CG | MET | 844 | 2.579 | 47.610 | 34.044 |
| 1619 | SD | MET | 844 | 2.732 | 49.395 | 33.860 |
| 1620 | CE | MET | 844 | 1.675 | 49.895 | 35.225 |
| 1621 | N | ARG | 845 | 5.831 | 44.879 | 32.504 |
| 1622 | CA | ARG | 845 | 6.858 | 44.348 | 31.632 |
| 1623 | HN | ARG | 845 | 6.083 | 45.314 | 33.368 |
| 1624 | C | ARG | 845 | 6.663 | 42.886 | 31.230 |
| 1625 | O | ARG | 845 | 7.031 | 42.498 | 30.125 |
| 1626 | CB | ARG | 845 | 8.222 | 44.487 | 32.276 |
| 1627 | CG | ARG | 845 | 9.338 | 43.990 | 31.382 |
| 1628 | CD | ARG | 845 | 10.592 | 44.687 | 31.817 |
| 1629 | NE | ARG | 845 | 11.788 | 44.423 | 31.025 |
| 1630 | CZ | ARG | 845 | 12.240 | 43.213 | 30.720 |
| 1631 | NH1 | ARG | 845 | 11.722 | 42.067 | 31.237 |
| 1632 | 1HH1 | ARG | 845 | 12.111 | 41.185 | 30.975 |
| 1633 | 2HH1 | ARG | 845 | 10.954 | 42.117 | 31.874 |
| 1634 | NH2 | ARG | 845 | 13.301 | 43.102 | 29.867 |
| 1635 | 1HH2 | ARG | 845 | 13.666 | 42.202 | 29.627 |
| 1636 | 2HH2 | ARG | 845 | 13.711 | 43.925 | 29.480 |
| 1637 | HE | ARG | 845 | 12.304 | 45.210 | 30.686 |
| 1638 | N | SER | 846 | 6.107 | 42.067 | 32.117 |
| 1639 | CA | SER | 846 | 5.895 | 40.660 | 31.804 |
| 1640 | HN | SER | 846 | 5.830 | 42.421 | 33.011 |
| 1641 | C | SER | 846 | 4.760 | 40.504 | 30.792 |
| 1642 | O | SER | 846 | 4.808 | 39.640 | 29.913 |
| 1643 | CB | SER | 846 | 5.598 | 39.892 | 33.116 |
| 1644 | OG | SER | 846 | 5.617 | 38.470 | 32.945 |
| 1645 | HG | SER | 846 | 4.910 | 38.245 | 32.334 |
| 1646 | N | SER | 847 | 3.741 | 41.353 | 30.925 |
| 1647 | CA | SER | 847 | 2.576 | 41.314 | 30.040 |
| 1648 | HN | SER | 847 | 3.773 | 42.038 | 31.653 |
| 1649 | C | SER | 847 | 2.939 | 41.645 | 28.607 |
| 1650 | O | SER | 847 | 2.409 | 41.039 | 27.664 |

| | | | | | | |
|---|---|---|---|---|---|---|
| 1651 | CB | SER | 847 | 1.478 | 42.262 | 30.584 |
| 1652 | OG | SER | 847 | 1.254 | 42.106 | 31.989 |
| 1653 | HG | SER | 847 | 0.935 | 41.211 | 32.132 |
| 1654 | N | TYR | 848 | 3.812 | 42.633 | 28.428 |
| 1655 | CA | TYR | 848 | 4.213 | 42.934 | 27.078 |
| 1656 | HN | TYR | 848 | 4.177 | 43.149 | 29.204 |
| 1657 | C | TYR | 848 | 5.231 | 41.906 | 26.606 |
| 1658 | O | TYR | 848 | 5.482 | 41.819 | 25.421 |
| 1659 | CB | TYR | 848 | 4.743 | 44.368 | 26.937 |
| 1660 | CG | TYR | 848 | 3.625 | 45.390 | 27.002 |
| 1661 | CD1 | TYR | 848 | 2.598 | 45.389 | 26.052 |
| 1662 | CE1 | TYR | 848 | 1.491 | 46.230 | 26.193 |
| 1663 | CZ | TYR | 848 | 1.411 | 47.080 | 27.284 |
| 1664 | OH | TYR | 848 | 0.360 | 47.950 | 27.408 |
| 1665 | HH | TYR | 848 | 0.446 | 48.416 | 28.251 |
| 1666 | CE2 | TYR | 848 | 2.427 | 47.107 | 28.224 |
| 1667 | CD2 | TYR | 848 | 3.523 | 46.270 | 28.079 |
| 1668 | N | ILE | 849 | 5.829 | 41.107 | 27.485 |
| 1669 | CA | ILE | 849 | 6.748 | 40.123 | 26.919 |
| 1670 | HN | ILE | 849 | 5.659 | 41.174 | 28.468 |
| 1671 | C | ILE | 849 | 5.925 | 39.010 | 26.292 |
| 1672 | O | ILE | 849 | 6.310 | 38.445 | 25.274 |
| 1673 | CB | ILE | 849 | 7.733 | 39.512 | 27.935 |
| 1674 | CG2 | ILE | 849 | 8.465 | 38.324 | 27.285 |
| 1675 | CG1 | ILE | 849 | 8.770 | 40.563 | 28.335 |
| 1676 | CD1 | ILE | 849 | 9.397 | 40.319 | 29.678 |
| 1677 | N | ARG | 850 | 4.782 | 38.702 | 26.894 |
| 1678 | CA | ARG | 850 | 3.921 | 37.672 | 26.337 |
| 1679 | HN | ARG | 850 | 4.514 | 39.179 | 27.732 |
| 1680 | C | ARG | 850 | 3.174 | 38.282 | 25.150 |
| 1681 | O | ARG | 850 | 2.782 | 37.583 | 24.217 |
| 1682 | CB | ARG | 850 | 2.936 | 37.158 | 27.424 |
| 1683 | CG | ARG | 850 | 3.598 | 36.378 | 28.590 |
| 1684 | CD | ARG | 850 | 2.667 | 36.225 | 29.799 |
| 1685 | NE | ARG | 850 | 3.213 | 35.160 | 30.678 |
| 1686 | CZ | ARG | 850 | 2.596 | 34.636 | 31.729 |
| 1687 | NH1 | ARG | 850 | 1.411 | 34.988 | 32.134 |
| 1688 | 1HH1 | ARG | 850 | 0.990 | 35.715 | 31.555 |
| 1689 | 2HH1 | ARG | 850 | 1.042 | 34.513 | 32.955 |
| 1690 | NH2 | ARG | 850 | 3.215 | 33.722 | 32.386 |
| 1691 | 1HH2 | ARG | 850 | 4.134 | 33.521 | 31.987 |
| 1692 | 2HH2 | ARG | 850 | 2.749 | 33.318 | 33.196 |
| 1693 | HE | ARG | 850 | 4.123 | 34.807 | 30.459 |
| 1694 | N | GLU | 851 | 2.986 | 39.594 | 25.194 |
| 1695 | CA | GLU | 851 | 2.315 | 40.291 | 24.115 |
| 1696 | HN | GLU | 851 | 3.313 | 40.111 | 25.985 |
| 1697 | C | GLU | 851 | 3.224 | 40.145 | 22.892 |
| 1698 | O | GLU | 851 | 2.748 | 40.020 | 21.757 |
| 1699 | CB | GLU | 851 | 2.129 | 41.756 | 24.506 |
| 1700 | CG | GLU | 851 | 0.878 | 42.449 | 23.947 |
| 1701 | CD | GLU | 851 | -0.362 | 41.556 | 23.874 |
| 1702 | OE1 | GLU | 851 | -0.767 | 40.962 | 24.903 |
| 1703 | OE2 | GLU | 851 | -0.936 | 41.463 | 22.763 |
| 1704 | HE2 | GLU | 851 | -1.682 | 40.889 | 22.840 |
| 1705 | N | LEU | 852 | 4.536 | 40.143 | 23.135 |

| | | | | | | |
|---|---|---|---|---|---|---|
| 1706 | CA | LEU | 852 | 5.503 | 39.980 | 22.058 |
| 1707 | HN | LEU | 852 | 4.861 | 40.255 | 24.074 |
| 1708 | C | LEU | 852 | 5.325 | 38.560 | 21.578 |
| 1709 | O | LEU | 852 | 5.338 | 38.275 | 20.384 |
| 1710 | CB | LEU | 852 | 6.946 | 40.169 | 22.549 |
| 1711 | CG | LEU | 852 | 7.938 | 39.886 | 21.417 |
| 1712 | CD1 | LEU | 852 | 7.511 | 40.699 | 20.219 |
| 1713 | CD2 | LEU | 852 | 9.362 | 40.214 | 21.805 |
| 1714 | N | ILE | 853 | 5.141 | 37.669 | 22.539 |
| 1715 | CA | ILE | 853 | 4.962 | 36.263 | 22.235 |
| 1716 | HN | ILE | 853 | 5.124 | 37.971 | 23.492 |
| 1717 | C | ILE | 853 | 3.814 | 35.945 | 21.293 |
| 1718 | O | ILE | 853 | 3.991 | 35.179 | 20.350 |
| 1719 | CB | ILE | 853 | 4.851 | 35.456 | 23.593 |
| 1720 | CG1 | ILE | 853 | 5.959 | 35.789 | 24.642 |
| 1721 | CG2 | ILE | 853 | 4.837 | 33.910 | 23.378 |
| 1722 | CD1 | ILE | 853 | 5.707 | 35.276 | 26.075 |
| 1723 | N | LYS | 854 | 2.640 | 36.517 | 21.558 |
| 1724 | CA | LYS | 854 | 1.457 | 36.297 | 20.722 |
| 1725 | HN | LYS | 854 | 2.564 | 37.116 | 22.355 |
| 1726 | C | LYS | 854 | 1.677 | 36.840 | 19.320 |
| 1727 | O | LYS | 854 | 1.331 | 36.197 | 18.326 |
| 1728 | CB | LYS | 854 | 0.262 | 37.026 | 21.293 |
| 1729 | CG | LYS | 854 | -0.140 | 36.604 | 22.653 |
| 1730 | CD | LYS | 854 | -1.219 | 37.528 | 23.151 |
| 1731 | CE | LYS | 854 | -2.034 | 36.824 | 24.193 |
| 1732 | NZ | LYS | 854 | -3.153 | 37.678 | 24.762 |
| 1733 | HZ1 | LYS | 854 | -2.861 | 38.666 | 24.773 |
| 1734 | HZ2 | LYS | 854 | -3.995 | 37.577 | 24.176 |
| 1735 | HZ3 | LYS | 854 | -3.360 | 37.381 | 25.694 |
| 1736 | N | ALA | 855 | 2.213 | 38.053 | 19.254 |
| 1737 | CA | ALA | 855 | 2.476 | 38.694 | 17.985 |
| 1738 | HN | ALA | 855 | 2.441 | 38.534 | 20.101 |
| 1739 | C | ALA | 855 | 3.331 | 37.722 | 17.193 |
| 1740 | O | ALA | 855 | 3.093 | 37.505 | 16.007 |
| 1741 | CB | ALA | 855 | 3.210 | 40.003 | 18.197 |
| 1742 | N | ILE | 856 | 4.317 | 37.130 | 17.862 |
| 1743 | CA | ILE | 856 | 5.197 | 36.165 | 17.212 |
| 1744 | HN | ILE | 856 | 4.457 | 37.350 | 18.827 |
| 1745 | C | ILE | 856 | 4.440 | 34.939 | 16.681 |
| 1746 | O | ILE | 856 | 4.599 | 34.576 | 15.516 |
| 1747 | CB | ILE | 856 | 6.305 | 35.673 | 18.167 |
| 1748 | CG2 | ILE | 856 | 7.024 | 34.482 | 17.547 |
| 1749 | CG1 | ILE | 856 | 7.295 | 36.803 | 18.451 |
| 1750 | CD1 | ILE | 856 | 8.262 | 36.508 | 19.591 |
| 1751 | N | GLY | 857 | 3.624 | 34.300 | 17.528 |
| 1752 | CA | GLY | 857 | 2.865 | 33.114 | 17.116 |
| 1753 | HN | GLY | 857 | 3.529 | 34.639 | 18.464 |
| 1754 | C | GLY | 857 | 1.853 | 33.364 | 16.013 |
| 1755 | O | GLY | 857 | 1.584 | 32.470 | 15.219 |
| 1756 | N | LEU | 858 | 1.272 | 34.561 | 15.966 |
| 1757 | CA | LEU | 858 | 0.266 | 34.833 | 14.952 |
| 1758 | HN | LEU | 858 | 1.526 | 35.269 | 16.625 |
| 1759 | C | LEU | 858 | 0.809 | 35.417 | 13.651 |
| 1760 | O | LEU | 858 | 0.067 | 36.025 | 12.892 |

| | | | | | | |
|---|---|---|---|---|---|---|
| 1761 | CB | LEU | 858 | -0.852 | 35.738 | 15.543 |
| 1762 | CG | LEU | 858 | -0.935 | 37.210 | 15.057 |
| 1763 | CD1 | LEU | 858 | -2.282 | 37.825 | 15.463 |
| 1764 | CD2 | LEU | 858 | 0.214 | 38.078 | 15.593 |
| 1765 | N | ARG | 859 | 2.093 | 35.212 | 13.370 |
| 1766 | CA | ARG | 859 | 2.681 | 35.753 | 12.142 |
| 1767 | HN | ARG | 859 | 2.660 | 34.683 | 14.001 |
| 1768 | C | ARG | 859 | 3.342 | 34.641 | 11.356 |
| 1769 | O | ARG | 859 | 3.279 | 34.579 | 10.126 |
| 1770 | CB | ARG | 859 | 3.756 | 36.799 | 12.482 |
| 1771 | CG | ARG | 859 | 4.482 | 37.395 | 11.260 |
| 1772 | CD | ARG | 859 | 5.850 | 38.007 | 11.619 |
| 1773 | NE | ARG | 859 | 6.988 | 37.182 | 11.200 |
| 1774 | CZ | ARG | 859 | 7.594 | 37.268 | 10.015 |
| 1775 | NH1 | ARG | 859 | 7.225 | 38.168 | 9.054 |
| 1776 | 1HH1 | ARG | 859 | 7.713 | 38.187 | 8.184 |
| 1777 | 2HH1 | ARG | 859 | 6.471 | 38.798 | 9.231 |
| 1778 | NH2 | ARG | 859 | 8.627 | 36.413 | 9.759 |
| 1779 | 1HH2 | ARG | 859 | 9.108 | 36.443 | 8.885 |
| 1780 | 2HH2 | ARG | 859 | 8.889 | 35.748 | 10.461 |
| 1781 | HE | ARG | 859 | 7.335 | 36.506 | 11.850 |
| 1782 | N | GLN | 860 | 3.975 | 33.764 | 12.123 |
| 1783 | CA | GLN | 860 | 4.743 | 32.640 | 11.628 |
| 1784 | HN | GLN | 860 | 3.918 | 33.887 | 13.114 |
| 1785 | C | GLN | 860 | 4.039 | 31.621 | 10.739 |
| 1786 | O | GLN | 860 | 3.938 | 31.802 | 9.526 |
| 1787 | CB | GLN | 860 | 5.410 | 31.950 | 12.839 |
| 1788 | CG | GLN | 860 | 6.972 | 31.872 | 12.816 |
| 1789 | CD | GLN | 860 | 7.777 | 33.116 | 12.418 |
| 1790 | OE1 | GLN | 860 | 7.604 | 34.186 | 12.980 |
| 1791 | NE2 | GLN | 860 | 8.648 | 33.046 | 11.444 |
| 1792 | 2HE2 | GLN | 860 | 8.695 | 32.143 | 10.970 |
| 1793 | 1HE2 | GLN | 860 | 9.093 | 33.933 | 11.199 |
| 1794 | N | LYS | 861 | 3.545 | 30.551 | 11.345 |
| 1795 | CA | LYS | 861 | 2.923 | 29.508 | 10.561 |
| 1796 | HN | LYS | 861 | 3.601 | 30.466 | 12.339 |
| 1797 | C | LYS | 861 | 3.910 | 28.361 | 10.659 |
| 1798 | O | LYS | 861 | 4.792 | 28.191 | 9.811 |
| 1799 | CB | LYS | 861 | 2.682 | 29.908 | 9.079 |
| 1800 | CG | LYS | 861 | 2.106 | 28.758 | 8.216 |
| 1801 | CD | LYS | 861 | 1.917 | 29.096 | 6.735 |
| 1802 | CE | LYS | 861 | 1.412 | 27.852 | 5.992 |
| 1803 | NZ | LYS | 861 | 1.065 | 28.215 | 4.607 |
| 1804 | HZ2 | LYS | 861 | 1.724 | 28.911 | 4.239 |
| 1805 | HZ1 | LYS | 861 | 1.128 | 27.405 | 3.982 |
| 1806 | HZ3 | LYS | 861 | 0.136 | 28.585 | 4.582 |
| 1807 | N | GLY | 862 | 3.755 | 27.613 | 11.746 |
| 1808 | CA | GLY | 862 | 4.564 | 26.452 | 12.119 |
| 1809 | HN | GLY | 862 | 3.015 | 27.868 | 12.368 |
| 1810 | C | GLY | 862 | 4.598 | 26.516 | 13.654 |
| 1811 | O | GLY | 862 | 3.551 | 26.710 | 14.274 |
| 1812 | N | VAL | 863 | 5.763 | 26.372 | 14.275 |
| 1813 | CA | VAL | 863 | 5.831 | 26.436 | 15.736 |
| 1814 | HN | VAL | 863 | 6.596 | 26.219 | 13.743 |
| 1815 | C | VAL | 863 | 7.207 | 26.150 | 16.311 |

| | | | | | | |
|---|---|---|---|---|---|---|
| 1816 | O | VAL | 863 | 7.451 | 26.398 | 17.495 |
| 1817 | CB | VAL | 863 | 4.749 | 25.470 | 16.363 |
| 1818 | CG1 | VAL | 863 | 3.432 | 26.135 | 16.853 |
| 1819 | CG2 | VAL | 863 | 4.303 | 24.329 | 15.418 |
| 1820 | N | VAL | 864 | 8.105 | 25.606 | 15.502 |
| 1821 | CA | VAL | 864 | 9.441 | 25.356 | 16.017 |
| 1822 | HN | VAL | 864 | 7.868 | 25.372 | 14.559 |
| 1823 | C | VAL | 864 | 10.214 | 26.642 | 15.778 |
| 1824 | O | VAL | 864 | 11.168 | 26.958 | 16.493 |
| 1825 | CB | VAL | 864 | 10.130 | 24.141 | 15.277 |
| 1826 | CG1 | VAL | 864 | 10.576 | 24.390 | 13.809 |
| 1827 | CG2 | VAL | 864 | 11.387 | 23.598 | 15.999 |
| 1828 | N | SER | 865 | 9.787 | 27.400 | 14.773 |
| 1829 | CA | SER | 865 | 10.446 | 28.656 | 14.481 |
| 1830 | HN | SER | 865 | 9.010 | 27.103 | 14.218 |
| 1831 | C | SER | 865 | 9.822 | 29.736 | 15.357 |
| 1832 | O | SER | 865 | 10.123 | 30.918 | 15.207 |
| 1833 | CB | SER | 865 | 10.338 | 29.008 | 12.977 |
| 1834 | OG | SER | 865 | 8.993 | 29.271 | 12.561 |
| 1835 | HG | SER | 865 | 8.491 | 28.461 | 12.687 |
| 1836 | N | SER | 866 | 8.965 | 29.326 | 16.289 |
| 1837 | CA | SER | 866 | 8.349 | 30.300 | 17.178 |
| 1838 | HN | SER | 866 | 8.747 | 28.354 | 16.378 |
| 1839 | C | SER | 866 | 9.238 | 30.709 | 18.333 |
| 1840 | O | SER | 866 | 9.409 | 31.901 | 18.572 |
| 1841 | CB | SER | 866 | 6.966 | 29.807 | 17.672 |
| 1842 | OG | SER | 866 | 5.888 | 30.247 | 16.838 |
| 1843 | HG | SER | 866 | 5.865 | 31.207 | 16.881 |
| 1844 | N | SER | 867 | 9.809 | 29.754 | 19.059 |
| 1845 | CA | SER | 867 | 10.722 | 30.160 | 20.111 |
| 1846 | HN | SER | 867 | 9.617 | 28.788 | 18.886 |
| 1847 | C | SER | 867 | 11.947 | 30.685 | 19.428 |
| 1848 | O | SER | 867 | 12.641 | 31.546 | 19.946 |
| 1849 | CB | SER | 867 | 11.053 | 28.960 | 21.034 |
| 1850 | OG | SER | 867 | 9.995 | 28.652 | 21.947 |
| 1851 | HG | SER | 867 | 9.881 | 29.413 | 22.523 |
| 1852 | N | GLN | 868 | 12.253 | 30.159 | 18.260 |
| 1853 | CA | GLN | 868 | 13.429 | 30.674 | 17.628 |
| 1854 | HN | GLN | 868 | 11.696 | 29.443 | 17.839 |
| 1855 | C | GLN | 868 | 13.229 | 32.139 | 17.268 |
| 1856 | O | GLN | 868 | 14.184 | 32.906 | 17.281 |
| 1857 | CB | GLN | 868 | 13.782 | 29.864 | 16.398 |
| 1858 | CG | GLN | 868 | 15.120 | 30.259 | 15.832 |
| 1859 | CD | GLN | 868 | 15.367 | 29.609 | 14.501 |
| 1860 | OE1 | GLN | 868 | 16.487 | 29.631 | 13.990 |
| 1861 | NE2 | GLN | 868 | 14.293 | 29.030 | 13.788 |
| 1862 | 1HE2 | GLN | 868 | 14.473 | 28.624 | 12.884 |
| 1863 | 2HE2 | GLN | 868 | 13.386 | 28.925 | 14.210 |
| 1864 | N | ARG | 869 | 11.988 | 32.524 | 16.962 |
| 1865 | CA | ARG | 869 | 11.656 | 33.911 | 16.585 |
| 1866 | HN | ARG | 869 | 11.254 | 31.846 | 16.990 |
| 1867 | C | ARG | 869 | 11.888 | 34.813 | 17.795 |
| 1868 | O | ARG | 869 | 12.635 | 35.796 | 17.748 |
| 1869 | CB | ARG | 869 | 10.184 | 33.966 | 16.157 |
| 1870 | CG | ARG | 869 | 9.698 | 35.261 | 15.556 |

| | | | | | | |
|---|---|---|---|---|---|---|
| 1871 | CD | ARG | 869 | 10.351 | 35.566 | 14.227 |
| 1872 | NE | ARG | 869 | 9.769 | 36.777 | 13.671 |
| 1873 | CZ | ARG | 869 | 10.332 | 37.527 | 12.738 |
| 1874 | NH1 | ARG | 869 | 11.508 | 37.098 | 12.207 |
| 1875 | 1HH1 | ARG | 869 | 11.954 | 37.643 | 11.499 |
| 1876 | 2HH1 | ARG | 869 | 11.914 | 36.244 | 12.526 |
| 1877 | NH2 | ARG | 869 | 9.731 | 38.655 | 12.360 |
| 1878 | 1HH2 | ARG | 869 | 10.147 | 39.221 | 11.648 |
| 1879 | 2HH2 | ARG | 869 | 8.870 | 38.930 | 12.797 |
| 1880 | HE | ARG | 869 | 8.876 | 37.064 | 14.019 |
| 1881 | N | PHE | 870 | 11.216 | 34.450 | 18.875 |
| 1882 | CA | PHE | 870 | 11.306 | 35.136 | 20.138 |
| 1883 | HN | PHE | 870 | 10.612 | 33.655 | 18.811 |
| 1884 | C | PHE | 870 | 12.763 | 35.347 | 20.485 |
| 1885 | O | PHE | 870 | 13.213 | 36.472 | 20.731 |
| 1886 | CB | PHE | 870 | 10.664 | 34.278 | 21.208 |
| 1887 | CG | PHE | 870 | 10.554 | 34.946 | 22.519 |
| 1888 | CD1 | PHE | 870 | 9.942 | 36.188 | 22.618 |
| 1889 | CE1 | PHE | 870 | 9.787 | 36.809 | 23.842 |
| 1890 | CZ | PHE | 870 | 10.246 | 36.185 | 24.997 |
| 1891 | CE2 | PHE | 870 | 10.864 | 34.944 | 24.911 |
| 1892 | CD2 | PHE | 870 | 11.014 | 34.328 | 23.668 |
| 1893 | N | TYR | 871 | 13.497 | 34.247 | 20.513 |
| 1894 | CA | TYR | 871 | 14.903 | 34.289 | 20.828 |
| 1895 | HN | TYR | 871 | 13.067 | 33.367 | 20.311 |
| 1896 | C | TYR | 871 | 15.591 | 35.350 | 19.966 |
| 1897 | O | TYR | 871 | 16.340 | 36.174 | 20.483 |
| 1898 | CB | TYR | 871 | 15.511 | 32.908 | 20.612 |
| 1899 | CG | TYR | 871 | 16.992 | 32.859 | 20.832 |
| 1900 | CD1 | TYR | 871 | 17.532 | 32.722 | 22.110 |
| 1901 | CE1 | TYR | 871 | 18.910 | 32.720 | 22.307 |
| 1902 | CZ | TYR | 871 | 19.747 | 32.857 | 21.210 |
| 1903 | OH | TYR | 871 | 21.112 | 32.882 | 21.399 |
| 1904 | HH | TYR | 871 | 21.530 | 32.623 | 20.569 |
| 1905 | CE2 | TYR | 871 | 19.221 | 32.991 | 19.936 |
| 1906 | CD2 | TYR | 871 | 17.858 | 32.988 | 19.756 |
| 1907 | N | GLN | 872 | 15.322 | 35.361 | 18.668 |
| 1908 | CA | GLN | 872 | 15.950 | 36.355 | 17.800 |
| 1909 | HN | GLN | 872 | 14.689 | 34.689 | 18.283 |
| 1910 | C | GLN | 872 | 15.405 | 37.765 | 18.019 |
| 1911 | O | GLN | 872 | 16.168 | 38.739 | 18.044 |
| 1912 | CB | GLN | 872 | 15.752 | 36.015 | 16.334 |
| 1913 | CG | GLN | 872 | 16.086 | 34.612 | 15.954 |
| 1914 | CD | GLN | 872 | 15.869 | 34.395 | 14.479 |
| 1915 | OE1 | GLN | 872 | 14.744 | 34.153 | 14.025 |
| 1916 | NE2 | GLN | 872 | 17.014 | 34.493 | 13.651 |
| 1917 | 1HE2 | GLN | 872 | 16.916 | 34.308 | 12.664 |
| 1918 | 2HE2 | GLN | 872 | 17.927 | 34.643 | 14.051 |
| 1919 | N | LEU | 873 | 14.087 | 37.895 | 18.144 |
| 1920 | CA | LEU | 873 | 13.550 | 39.228 | 18.350 |
| 1921 | HN | LEU | 873 | 13.484 | 37.099 | 18.097 |
| 1922 | C | LEU | 873 | 14.160 | 39.797 | 19.635 |
| 1923 | O | LEU | 873 | 14.722 | 40.892 | 19.626 |
| 1924 | CB | LEU | 873 | 12.014 | 39.207 | 18.390 |
| 1925 | CG | LEU | 873 | 11.357 | 38.916 | 17.031 |

| | | | | | | |
|---|---|---|---|---|---|---|
| 1926 | CD1 | LEU | 873 | 9.854 | 39.038 | 17.129 |
| 1927 | CD2 | LEU | 873 | 11.877 | 39.897 | 15.993 |
| 1928 | N | THR | 874 | 14.099 | 39.033 | 20.723 |
| 1929 | CA | THR | 874 | 14.656 | 39.500 | 21.990 |
| 1930 | HN | THR | 874 | 13.668 | 38.132 | 20.673 |
| 1931 | C | THR | 874 | 16.176 | 39.693 | 21.962 |
| 1932 | O | THR | 874 | 16.755 | 40.269 | 22.890 |
| 1933 | CB | THR | 874 | 14.289 | 38.558 | 23.132 |
| 1934 | OG1 | THR | 874 | 14.757 | 37.228 | 22.968 |
| 1935 | HG1 | THR | 874 | 14.602 | 36.794 | 23.817 |
| 1936 | CG2 | THR | 874 | 12.779 | 38.552 | 23.335 |
| 1937 | N | LYS | 875 | 16.830 | 39.216 | 20.908 |
| 1938 | CA | LYS | 875 | 18.270 | 39.397 | 20.817 |
| 1939 | HN | LYS | 875 | 16.336 | 38.737 | 20.182 |
| 1940 | C | LYS | 875 | 18.456 | 40.826 | 20.335 |
| 1941 | O | LYS | 875 | 19.326 | 41.553 | 20.810 |
| 1942 | CB | LYS | 875 | 18.892 | 38.425 | 19.817 |
| 1943 | CG | LYS | 875 | 20.365 | 38.109 | 20.110 |
| 1944 | CD | LYS | 875 | 20.502 | 37.119 | 21.279 |
| 1945 | CE | LYS | 875 | 21.960 | 36.934 | 21.747 |
| 1946 | NZ | LYS | 875 | 22.157 | 35.824 | 22.770 |
| 1947 | HZ1 | LYS | 875 | 21.494 | 35.952 | 23.548 |
| 1948 | HZ2 | LYS | 875 | 21.990 | 34.911 | 22.324 |
| 1949 | HZ3 | LYS | 875 | 23.093 | 35.856 | 23.121 |
| 1950 | N | LEU | 876 | 17.603 | 41.223 | 19.396 |
| 1951 | CA | LEU | 876 | 17.636 | 42.566 | 18.843 |
| 1952 | HN | LEU | 876 | 16.918 | 40.577 | 19.059 |
| 1953 | C | LEU | 876 | 17.397 | 43.581 | 19.962 |
| 1954 | O | LEU | 876 | 18.051 | 44.628 | 20.021 |
| 1955 | CB | LEU | 876 | 16.554 | 42.714 | 17.782 |
| 1956 | CG | LEU | 876 | 16.934 | 43.629 | 16.630 |
| 1957 | CD1 | LEU | 876 | 15.660 | 44.226 | 16.085 |
| 1958 | CD2 | LEU | 876 | 17.893 | 44.732 | 17.085 |
| 1959 | N | LEU | 877 | 16.452 | 43.267 | 20.842 |
| 1960 | CA | LEU | 877 | 16.140 | 44.140 | 21.963 |
| 1961 | HN | LEU | 877 | 15.944 | 42.412 | 20.731 |
| 1962 | C | LEU | 877 | 17.380 | 44.403 | 22.790 |
| 1963 | O | LEU | 877 | 17.777 | 45.549 | 22.950 |
| 1964 | CB | LEU | 877 | 15.043 | 43.515 | 22.829 |
| 1965 | CG | LEU | 877 | 13.715 | 43.547 | 22.073 |
| 1966 | CD1 | LEU | 877 | 12.588 | 43.029 | 22.926 |
| 1967 | CD2 | LEU | 877 | 13.447 | 44.988 | 21.639 |
| 1968 | N | ASP | 878 | 18.000 | 43.343 | 23.298 |
| 1969 | CA | ASP | 878 | 19.198 | 43.467 | 24.125 |
| 1970 | HN | ASP | 878 | 17.635 | 42.432 | 23.108 |
| 1971 | C | ASP | 878 | 20.328 | 44.239 | 23.470 |
| 1972 | O | ASP | 878 | 21.123 | 44.895 | 24.145 |
| 1973 | CB | ASP | 878 | 19.733 | 42.092 | 24.495 |
| 1974 | CG | ASP | 878 | 18.831 | 41.367 | 25.445 |
| 1975 | OD1 | ASP | 878 | 17.986 | 42.034 | 26.080 |
| 1976 | OD2 | ASP | 878 | 18.972 | 40.138 | 25.571 |
| 1977 | HD2 | ASP | 878 | 18.343 | 39.811 | 26.197 |
| 1978 | N | ASN | 879 | 20.400 | 44.149 | 22.152 |
| 1979 | CA | ASN | 879 | 21.459 | 44.813 | 21.411 |
| 1980 | HN | ASN | 879 | 19.715 | 43.614 | 21.657 |

| | | | | | | |
|---|---|---|---|---|---|---|
| 1981 | C | ASN | 879 | 21.137 | 46.275 | 21.164 |
| 1982 | O | ASN | 879 | 21.914 | 47.011 | 20.551 |
| 1983 | CB | ASN | 879 | 21.683 | 44.063 | 20.066 |
| 1984 | CG | ASN | 879 | 21.183 | 44.755 | 18.793 |
| 1985 | OD1 | ASN | 879 | 21.933 | 45.363 | 18.044 |
| 1986 | ND2 | ASN | 879 | 19.908 | 44.712 | 18.513 |
| 1987 | 1HD2 | ASN | 879 | 19.659 | 45.328 | 17.734 |
| 1988 | 2HD2 | ASN | 879 | 19.297 | 44.276 | 19.204 |
| 1989 | N | LEU | 880 | 19.975 | 46.683 | 21.653 |
| 1990 | CA | LEU | 880 | 19.505 | 48.049 | 21.499 |
| 1991 | HN | LEU | 880 | 19.402 | 46.028 | 22.145 |
| 1992 | C | LEU | 880 | 20.218 | 48.908 | 22.537 |
| 1993 | O | LEU | 880 | 20.382 | 50.115 | 22.363 |
| 1994 | CB | LEU | 880 | 17.967 | 48.063 | 21.730 |
| 1995 | CG | LEU | 880 | 17.118 | 49.090 | 20.933 |
| 1996 | CD1 | LEU | 880 | 17.370 | 48.929 | 19.428 |
| 1997 | CD2 | LEU | 880 | 15.612 | 48.968 | 21.222 |
| 1998 | N | HIS | 881 | 20.637 | 48.265 | 23.626 |
| 1999 | CA | HIS | 881 | 21.366 | 48.965 | 24.663 |
| 2000 | HN | HIS | 881 | 20.445 | 47.289 | 23.728 |
| 2001 | C | HIS | 881 | 22.677 | 49.372 | 24.018 |
| 2002 | O | HIS | 881 | 23.069 | 50.525 | 24.094 |
| 2003 | CB | HIS | 881 | 21.625 | 48.058 | 25.868 |
| 2004 | CG | HIS | 881 | 20.434 | 47.892 | 26.767 |
| 2005 | ND1 | HIS | 881 | 19.932 | 46.661 | 27.114 |
| 2006 | CE1 | HIS | 881 | 18.881 | 46.823 | 27.903 |
| 2007 | NE2 | HIS | 881 | 18.691 | 48.114 | 28.080 |
| 2008 | HE2 | HIS | 881 | 17.945 | 48.535 | 28.652 |
| 2009 | CD2 | HIS | 881 | 19.648 | 48.811 | 27.381 |
| 2010 | N | ASP | 882 | 23.343 | 48.454 | 23.333 |
| 2011 | CA | ASP | 882 | 24.594 | 48.867 | 22.735 |
| 2012 | HN | ASP | 882 | 22.998 | 47.520 | 23.234 |
| 2013 | C | ASP | 882 | 24.485 | 49.904 | 21.618 |
| 2014 | O | ASP | 882 | 25.405 | 50.714 | 21.471 |
| 2015 | CB | ASP | 882 | 25.429 | 47.634 | 22.300 |
| 2016 | CG | ASP | 882 | 26.949 | 47.831 | 22.199 |
| 2017 | OD1 | ASP | 882 | 27.668 | 47.123 | 21.510 |
| 2018 | OD2 | ASP | 882 | 27.396 | 48.890 | 22.939 |
| 2019 | HD2 | ASP | 882 | 28.333 | 48.968 | 22.844 |
| 2020 | N | LEU | 883 | 23.418 | 49.918 | 20.817 |
| 2021 | CA | LEU | 883 | 23.396 | 50.978 | 19.804 |
| 2022 | HN | LEU | 883 | 22.680 | 49.249 | 20.903 |
| 2023 | C | LEU | 883 | 23.025 | 52.309 | 20.464 |
| 2024 | O | LEU | 883 | 23.535 | 53.352 | 20.062 |
| 2025 | CB | LEU | 883 | 22.497 | 50.703 | 18.565 |
| 2026 | CG | LEU | 883 | 21.601 | 49.435 | 18.580 |
| 2027 | CD1 | LEU | 883 | 20.880 | 49.283 | 17.233 |
| 2028 | CD2 | LEU | 883 | 22.390 | 48.153 | 18.893 |
| 2029 | N | VAL | 884 | 22.176 | 52.274 | 21.495 |
| 2030 | CA | VAL | 884 | 21.806 | 53.511 | 22.196 |
| 2031 | HN | VAL | 884 | 21.791 | 51.400 | 21.791 |
| 2032 | C | VAL | 884 | 23.037 | 54.118 | 22.876 |
| 2033 | O | VAL | 884 | 23.216 | 55.331 | 22.884 |
| 2034 | CB | VAL | 884 | 20.666 | 53.265 | 23.239 |
| 2035 | CG1 | VAL | 884 | 20.883 | 54.102 | 24.501 |

| | | | | | | |
|---|---|---|---|---|---|---|
| 2036 | CG2 | VAL | 884 | 19.337 | 53.649 | 22.622 |
| 2037 | N | LYS | 885 | 23.896 | 53.278 | 23.440 |
| 2038 | CA | LYS | 885 | 25.100 | 53.795 | 24.070 |
| 2039 | HN | LYS | 885 | 23.716 | 52.295 | 23.431 |
| 2040 | C | LYS | 885 | 25.856 | 54.646 | 23.041 |
| 2041 | O | LYS | 885 | 26.084 | 55.831 | 23.258 |
| 2042 | CB | LYS | 885 | 25.988 | 52.630 | 24.587 |
| 2043 | CG | LYS | 885 | 27.187 | 53.104 | 25.445 |
| 2044 | CD | LYS | 885 | 27.964 | 51.982 | 26.141 |
| 2045 | CE | LYS | 885 | 26.976 | 50.933 | 26.667 |
| 2046 | NZ | LYS | 885 | 27.723 | 49.772 | 27.183 |
| 2047 | HZ2 | LYS | 885 | 28.586 | 50.074 | 27.649 |
| 2048 | HZ1 | LYS | 885 | 27.181 | 49.264 | 27.889 |
| 2049 | HZ3 | LYS | 885 | 27.946 | 49.156 | 26.427 |
| 2050 | N | GLN | 886 | 26.216 | 54.045 | 21.909 |
| 2051 | CA | GLN | 886 | 26.945 | 54.767 | 20.866 |
| 2052 | HN | GLN | 886 | 25.985 | 53.083 | 21.768 |
| 2053 | C | GLN | 886 | 26.165 | 55.928 | 20.329 |
| 2054 | O | GLN | 886 | 26.734 | 56.902 | 19.834 |
| 2055 | CB | GLN | 886 | 27.349 | 53.800 | 19.732 |
| 2056 | CG | GLN | 886 | 28.246 | 52.587 | 20.145 |
| 2057 | CD | GLN | 886 | 28.640 | 51.552 | 19.083 |
| 2058 | OE1 | GLN | 886 | 29.370 | 50.614 | 19.358 |
| 2059 | NE2 | GLN | 886 | 28.212 | 51.682 | 17.853 |
| 2060 | 2HE2 | GLN | 886 | 27.663 | 52.525 | 17.674 |
| 2061 | 1HE2 | GLN | 886 | 28.572 | 50.989 | 17.195 |
| 2062 | N | LEU | 887 | 24.856 | 55.851 | 20.484 |
| 2063 | CA | LEU | 887 | 24.046 | 56.947 | 20.040 |
| 2064 | HN | LEU | 887 | 24.439 | 55.045 | 20.903 |
| 2065 | C | LEU | 887 | 24.106 | 58.077 | 21.040 |
| 2066 | O | LEU | 887 | 24.103 | 59.242 | 20.645 |
| 2067 | CB | LEU | 887 | 22.623 | 56.496 | 19.816 |
| 2068 | CG | LEU | 887 | 22.595 | 56.012 | 18.381 |
| 2069 | CD1 | LEU | 887 | 21.174 | 55.720 | 18.026 |
| 2070 | CD2 | LEU | 887 | 23.193 | 57.071 | 17.439 |
| 2071 | N | HIS | 888 | 24.181 | 57.749 | 22.328 |
| 2072 | CA | HIS | 888 | 24.263 | 58.786 | 23.357 |
| 2073 | HN | HIS | 888 | 24.182 | 56.786 | 22.595 |
| 2074 | C | HIS | 888 | 25.637 | 59.455 | 23.422 |
| 2075 | O | HIS | 888 | 25.708 | 60.682 | 23.529 |
| 2076 | CB | HIS | 888 | 23.881 | 58.215 | 24.732 |
| 2077 | CG | HIS | 888 | 22.409 | 57.945 | 24.848 |
| 2078 | ND1 | HIS | 888 | 21.777 | 57.315 | 25.917 |
| 2079 | CE1 | HIS | 888 | 20.502 | 57.354 | 25.486 |
| 2080 | NE2 | HIS | 888 | 20.251 | 57.923 | 24.276 |
| 2081 | CD2 | HIS | 888 | 21.500 | 58.309 | 23.864 |
| 2082 | HE2 | HIS | 888 | 19.352 | 58.041 | 23.788 |
| 2083 | N | LEU | 889 | 26.721 | 58.682 | 23.357 |
| 2084 | CA | LEU | 889 | 28.044 | 59.302 | 23.396 |
| 2085 | HN | LEU | 889 | 26.630 | 57.688 | 23.284 |
| 2086 | C | LEU | 889 | 28.165 | 60.488 | 22.389 |
| 2087 | O | LEU | 889 | 28.636 | 61.533 | 22.815 |
| 2088 | CB | LEU | 889 | 29.181 | 58.264 | 23.171 |
| 2089 | CG | LEU | 889 | 29.449 | 57.220 | 24.288 |
| 2090 | CD1 | LEU | 889 | 28.805 | 57.678 | 25.604 |

| | | | | | | |
|---|---|---|---|---|---|---|
| 2091 | CD2 | LEU | 889 | 28.940 | 55.816 | 23.924 |
| 2092 | N | TYR | 890 | 27.761 | 60.362 | 21.102 |
| 2093 | CA | TYR | 890 | 27.894 | 61.500 | 20.123 |
| 2094 | HN | TYR | 890 | 27.367 | 59.496 | 20.795 |
| 2095 | C | TYR | 890 | 27.006 | 62.611 | 20.566 |
| 2096 | O | TYR | 890 | 27.299 | 63.777 | 20.356 |
| 2097 | CB | TYR | 890 | 27.462 | 61.227 | 18.625 |
| 2098 | CG | TYR | 890 | 28.164 | 62.223 | 17.650 |
| 2099 | CD1 | TYR | 890 | 27.744 | 62.487 | 16.309 |
| 2100 | CE1 | TYR | 890 | 28.494 | 63.375 | 15.472 |
| 2101 | CZ | TYR | 890 | 29.624 | 63.996 | 16.007 |
| 2102 | OH | TYR | 890 | 30.353 | 64.863 | 15.221 |
| 2103 | HH | TYR | 890 | 31.162 | 65.093 | 15.692 |
| 2104 | CE2 | TYR | 890 | 30.014 | 63.735 | 17.301 |
| 2105 | CD2 | TYR | 890 | 29.297 | 62.872 | 18.095 |
| 2106 | N | CYS | 891 | 25.909 | 62.247 | 21.191 |
| 2107 | CA | CYS | 891 | 24.971 | 63.257 | 21.570 |
| 2108 | HN | CYS | 891 | 25.735 | 61.284 | 21.397 |
| 2109 | C | CYS | 891 | 25.411 | 64.088 | 22.743 |
| 2110 | O | CYS | 891 | 25.410 | 65.316 | 22.669 |
| 2111 | CB | CYS | 891 | 23.625 | 62.607 | 21.821 |
| 2112 | SG | CYS | 891 | 22.292 | 63.762 | 22.287 |
| 2113 | HG | CYS | 891 | 21.160 | 63.081 | 22.481 |
| 2114 | N | LEU | 892 | 25.787 | 63.431 | 23.828 |
| 2115 | CA | LEU | 892 | 26.231 | 64.187 | 24.975 |
| 2116 | HN | LEU | 892 | 25.765 | 62.431 | 23.853 |
| 2117 | C | LEU | 892 | 27.389 | 65.064 | 24.502 |
| 2118 | O | LEU | 892 | 27.345 | 66.291 | 24.619 |
| 2119 | CB | LEU | 892 | 26.689 | 63.226 | 26.109 |
| 2120 | CG | LEU | 892 | 25.609 | 62.364 | 26.818 |
| 2121 | CD1 | LEU | 892 | 24.209 | 62.911 | 26.506 |
| 2122 | CD2 | LEU | 892 | 25.683 | 60.879 | 26.427 |
| 2123 | N | ASN | 893 | 28.404 | 64.415 | 23.941 |
| 2124 | CA | ASN | 893 | 29.581 | 65.087 | 23.411 |
| 2125 | HN | ASN | 893 | 28.358 | 63.418 | 23.880 |
| 2126 | C | ASN | 893 | 29.097 | 66.302 | 22.642 |
| 2127 | O | ASN | 893 | 29.026 | 67.386 | 23.195 |
| 2128 | CB | ASN | 893 | 30.335 | 64.103 | 22.470 |
| 2129 | CG | ASN | 893 | 31.777 | 64.465 | 22.099 |
| 2130 | OD1 | ASN | 893 | 32.504 | 63.700 | 21.483 |
| 2131 | ND2 | ASN | 893 | 32.252 | 65.622 | 22.475 |
| 2132 | 1HD2 | ASN | 893 | 33.260 | 65.701 | 22.314 |
| 2133 | 2HD2 | ASN | 893 | 31.652 | 66.199 | 23.065 |
| 2134 | N | THR | 894 | 28.741 | 66.123 | 21.377 |
| 2135 | CA | THR | 894 | 28.254 | 67.246 | 20.590 |
| 2136 | HN | THR | 894 | 28.807 | 65.215 | 20.963 |
| 2137 | C | THR | 894 | 27.525 | 68.300 | 21.446 |
| 2138 | O | THR | 894 | 27.814 | 69.494 | 21.339 |
| 2139 | CB | THR | 894 | 27.345 | 66.758 | 19.467 |
| 2140 | OG1 | THR | 894 | 26.339 | 65.975 | 20.047 |
| 2141 | HG1 | THR | 894 | 26.534 | 65.064 | 19.806 |
| 2142 | CG2 | THR | 894 | 28.154 | 66.447 | 18.220 |
| 2143 | N | PHE | 895 | 26.618 | 67.870 | 22.315 |
| 2144 | CA | PHE | 895 | 25.882 | 68.810 | 23.169 |
| 2145 | HN | PHE | 895 | 26.433 | 66.890 | 22.389 |

| | | | | | | |
|---|---|---|---|---|---|---|
| 2146 | C | PHE | 895 | 26.754 | 69.636 | 24.119 |
| 2147 | O | PHE | 895 | 26.385 | 70.750 | 24.493 |
| 2148 | CB | PHE | 895 | 24.841 | 68.056 | 23.988 |
| 2149 | CG | PHE | 895 | 24.061 | 68.928 | 24.928 |
| 2150 | CD1 | PHE | 895 | 22.887 | 69.546 | 24.518 |
| 2151 | CE1 | PHE | 895 | 22.143 | 70.328 | 25.391 |
| 2152 | CZ | PHE | 895 | 22.575 | 70.502 | 26.697 |
| 2153 | CE2 | PHE | 895 | 23.753 | 69.892 | 27.118 |
| 2154 | CD2 | PHE | 895 | 24.488 | 69.112 | 26.237 |
| 2155 | N | ILE | 896 | 27.893 | 69.086 | 24.527 |
| 2156 | CA | ILE | 896 | 28.794 | 69.797 | 25.426 |
| 2157 | HN | ILE | 896 | 28.136 | 68.169 | 24.212 |
| 2158 | C | ILE | 896 | 30.000 | 70.348 | 24.666 |
| 2159 | O | ILE | 896 | 31.141 | 70.250 | 25.129 |
| 2160 | CB | ILE | 896 | 29.230 | 68.835 | 26.605 |
| 2161 | CG1 | ILE | 896 | 28.050 | 68.095 | 27.313 |
| 2162 | CG2 | ILE | 896 | 30.071 | 69.563 | 27.700 |
| 2163 | CD1 | ILE | 896 | 28.438 | 67.191 | 28.501 |
| 2164 | N | GLN | 897 | 29.736 | 70.910 | 23.488 |
| 2165 | CA | GLN | 897 | 30.771 | 71.515 | 22.651 |
| 2166 | HN | GLN | 897 | 28.791 | 70.918 | 23.162 |
| 2167 | C | GLN | 897 | 30.118 | 72.518 | 21.717 |
| 2168 | O | GLN | 897 | 29.586 | 72.138 | 20.671 |
| 2169 | CB | GLN | 897 | 31.527 | 70.428 | 21.855 |
| 2170 | CG | GLN | 897 | 32.767 | 70.911 | 21.033 |
| 2171 | CD | GLN | 897 | 33.307 | 70.024 | 19.904 |
| 2172 | OE1 | GLN | 897 | 34.248 | 70.384 | 19.214 |
| 2173 | NE2 | GLN | 897 | 32.742 | 68.872 | 19.651 |
| 2174 | 2HE2 | GLN | 897 | 31.918 | 68.661 | 20.217 |
| 2175 | 1HE2 | GLN | 897 | 33.120 | 68.374 | 18.843 |
| 2176 | N | SER | 898 | 27.960 | 71.999 | 21.407 |
| 2177 | CA | SER | 898 | 26.923 | 72.646 | 20.604 |
| 2178 | HN | SER | 898 | 27.704 | 71.536 | 22.256 |
| 2179 | C | SER | 898 | 27.240 | 74.059 | 20.113 |
| 2180 | O | SER | 898 | 27.020 | 74.369 | 18.946 |
| 2181 | CB | SER | 898 | 25.579 | 72.638 | 21.346 |
| 2182 | OG | SER | 898 | 25.563 | 73.390 | 22.558 |
| 2183 | HG | SER | 898 | 24.693 | 73.242 | 22.952 |
| 2184 | N | ARG | 899 | 27.753 | 74.924 | 20.980 |
| 2185 | CA | ARG | 899 | 28.055 | 76.278 | 20.534 |
| 2186 | HN | ARG | 899 | 27.929 | 74.649 | 21.925 |
| 2187 | C | ARG | 899 | 29.194 | 76.268 | 19.523 |
| 2188 | O | ARG | 899 | 29.169 | 77.014 | 18.539 |
| 2189 | CB | ARG | 899 | 28.395 | 77.183 | 21.725 |
| 2190 | CG | ARG | 899 | 27.341 | 77.160 | 22.830 |
| 2191 | CD | ARG | 899 | 25.920 | 77.400 | 22.300 |
| 2192 | NE | ARG | 899 | 25.679 | 78.756 | 21.802 |
| 2193 | CZ | ARG | 899 | 25.625 | 79.845 | 22.569 |
| 2194 | NH1 | ARG | 899 | 25.713 | 79.793 | 23.932 |
| 2195 | 1HH1 | ARG | 899 | 25.662 | 80.613 | 24.501 |
| 2196 | 2HH1 | ARG | 899 | 25.777 | 78.923 | 24.416 |
| 2197 | NH2 | ARG | 899 | 25.471 | 81.061 | 21.972 |
| 2198 | 1HH2 | ARG | 899 | 25.413 | 81.909 | 22.499 |
| 2199 | 2HH2 | ARG | 899 | 25.387 | 81.153 | 20.981 |
| 2200 | HE | ARG | 899 | 25.546 | 78.874 | 20.818 |

| | | | | | | |
|---|---|---|---|---|---|---|
| 2201 | N | ALA | 900 | 31.651 | 74.889 | 20.070 |
| 2202 | CA | ALA | 900 | 32.565 | 75.329 | 19.028 |
| 2203 | HN | ALA | 900 | 31.963 | 74.238 | 20.761 |
| 2204 | C | ALA | 900 | 31.840 | 74.738 | 17.822 |
| 2205 | O | ALA | 900 | 31.805 | 75.321 | 16.735 |
| 2206 | CB | ALA | 900 | 33.957 | 74.712 | 19.249 |
| 2207 | N | LEU | 901 | 31.241 | 73.571 | 18.051 |
| 2208 | CA | LEU | 901 | 30.508 | 72.855 | 17.019 |
| 2209 | HN | LEU | 901 | 31.297 | 73.172 | 18.966 |
| 2210 | C | LEU | 901 | 29.275 | 73.631 | 16.592 |
| 2211 | O | LEU | 901 | 28.923 | 73.650 | 15.414 |
| 2212 | CB | LEU | 901 | 30.124 | 71.437 | 17.530 |
| 2213 | CG | LEU | 901 | 30.236 | 70.247 | 16.538 |
| 2214 | CD1 | LEU | 901 | 30.568 | 68.956 | 17.298 |
| 2215 | CD2 | LEU | 901 | 28.958 | 70.045 | 15.706 |
| 2216 | N | SER | 902 | 28.605 | 74.242 | 17.558 |
| 2217 | CA | SER | 902 | 27.445 | 75.073 | 17.258 |
| 2218 | HN | SER | 902 | 28.899 | 74.133 | 18.507 |
| 2219 | C | SER | 902 | 26.110 | 74.426 | 16.874 |
| 2220 | O | SER | 902 | 25.281 | 75.109 | 16.273 |
| 2221 | CB | SER | 902 | 27.808 | 76.071 | 16.151 |
| 2222 | OG | SER | 902 | 28.887 | 76.954 | 16.451 |
| 2223 | HG | SER | 902 | 29.185 | 77.309 | 15.605 |
| 2224 | N | VAL | 903 | 25.875 | 73.154 | 17.179 |
| 2225 | CA | VAL | 903 | 24.579 | 72.558 | 16.829 |
| 2226 | HN | VAL | 903 | 26.574 | 72.608 | 17.642 |
| 2227 | C | VAL | 903 | 23.634 | 72.822 | 18.011 |
| 2228 | O | VAL | 903 | 24.083 | 72.826 | 19.154 |
| 2229 | CB | VAL | 903 | 24.709 | 71.008 | 16.548 |
| 2230 | CG1 | VAL | 903 | 25.798 | 70.582 | 15.525 |
| 2231 | CG2 | VAL | 903 | 24.985 | 70.162 | 17.815 |
| 2232 | N | GLU | 904 | 22.347 | 73.037 | 17.745 |
| 2233 | CA | GLU | 904 | 21.379 | 73.348 | 18.812 |
| 2234 | HN | GLU | 904 | 22.030 | 72.987 | 16.798 |
| 2235 | C | GLU | 904 | 20.246 | 72.324 | 19.082 |
| 2236 | O | GLU | 904 | 19.542 | 71.921 | 18.159 |
| 2237 | CB | GLU | 904 | 20.743 | 74.726 | 18.525 |
| 2238 | CG | GLU | 904 | 21.151 | 75.892 | 19.461 |
| 2239 | CD | GLU | 904 | 22.541 | 76.479 | 19.185 |
| 2240 | OE1 | GLU | 904 | 22.754 | 77.073 | 18.098 |
| 2241 | OE2 | GLU | 904 | 23.424 | 76.353 | 20.069 |
| 2242 | HE2 | GLU | 904 | 24.226 | 76.756 | 19.773 |
| 2243 | N | PHE | 905 | 20.068 | 71.932 | 20.351 |
| 2244 | CA | PHE | 905 | 19.009 | 70.985 | 20.761 |
| 2245 | HN | PHE | 905 | 20.680 | 72.297 | 21.053 |
| 2246 | C | PHE | 905 | 17.830 | 71.786 | 21.373 |
| 2247 | O | PHE | 905 | 18.056 | 72.736 | 22.126 |
| 2248 | CB | PHE | 905 | 19.546 | 69.991 | 21.809 |
| 2249 | CG | PHE | 905 | 20.908 | 69.434 | 21.491 |
| 2250 | CD1 | PHE | 905 | 21.053 | 68.195 | 20.896 |
| 2251 | CE1 | PHE | 905 | 22.302 | 67.717 | 20.598 |
| 2252 | CZ | PHE | 905 | 23.450 | 68.464 | 20.853 |
| 2253 | CE2 | PHE | 905 | 23.313 | 69.690 | 21.437 |
| 2254 | CD2 | PHE | 905 | 22.044 | 70.175 | 21.759 |
| 2255 | N | PRO | 906 | 16.568 | 71.401 | 21.088 |

| | | | | | | |
|---|---|---|---|---|---|---|
| 2256 | CA | PRO | 906 | 15.415 | 72.135 | 21.630 |
| 2257 | CD | PRO | 906 | 16.114 | 70.141 | 20.461 |
| 2258 | C | PRO | 906 | 15.251 | 72.062 | 23.145 |
| 2259 | O | PRO | 906 | 16.142 | 71.609 | 23.854 |
| 2260 | CB | PRO | 906 | 14.241 | 71.504 | 20.899 |
| 2261 | CG | PRO | 906 | 14.638 | 70.083 | 20.855 |
| 2262 | N | GLU | 907 | 14.098 | 72.520 | 23.629 |
| 2263 | CA | GLU | 907 | 13.795 | 72.507 | 25.059 |
| 2264 | HN | GLU | 907 | 13.418 | 72.885 | 22.994 |
| 2265 | C | GLU | 907 | 13.842 | 71.099 | 25.612 |
| 2266 | O | GLU | 907 | 14.652 | 70.779 | 26.492 |
| 2267 | CB | GLU | 907 | 12.399 | 73.087 | 25.305 |
| 2268 | CG | GLU | 907 | 12.359 | 74.589 | 25.235 |
| 2269 | CD | GLU | 907 | 13.301 | 75.215 | 26.243 |
| 2270 | OE1 | GLU | 907 | 14.508 | 74.896 | 26.220 |
| 2271 | OE2 | GLU | 907 | 12.832 | 76.021 | 27.067 |
| 2272 | HE2 | GLU | 907 | 13.523 | 76.333 | 27.631 |
| 2273 | N | MET | 908 | 12.948 | 70.271 | 25.087 |
| 2274 | CA | MET | 908 | 12.861 | 68.895 | 25.507 |
| 2275 | HN | MET | 908 | 12.320 | 70.609 | 24.386 |
| 2276 | C | MET | 908 | 14.249 | 68.252 | 25.562 |
| 2277 | O | MET | 908 | 14.870 | 68.271 | 26.618 |
| 2278 | CB | MET | 908 | 11.912 | 68.116 | 24.584 |
| 2279 | CG | MET | 908 | 10.604 | 67.677 | 25.273 |
| 2280 | SD | MET | 908 | 10.728 | 67.416 | 27.096 |
| 2281 | CE | MET | 908 | 11.156 | 65.740 | 27.227 |
| 2282 | N | MET | 909 | 14.758 | 67.722 | 24.452 |
| 2283 | CA | MET | 909 | 16.077 | 67.061 | 24.448 |
| 2284 | HN | MET | 909 | 14.235 | 67.774 | 23.601 |
| 2285 | C | MET | 909 | 17.170 | 67.737 | 25.237 |
| 2286 | O | MET | 909 | 18.035 | 67.069 | 25.802 |
| 2287 | CB | MET | 909 | 16.519 | 66.828 | 22.977 |
| 2288 | CG | MET | 909 | 17.899 | 66.165 | 22.784 |
| 2289 | SD | MET | 909 | 18.232 | 65.948 | 21.029 |
| 2290 | CE | MET | 909 | 19.853 | 65.180 | 21.158 |
| 2291 | N | SER | 910 | 17.158 | 69.058 | 25.252 |
| 2292 | CA | SER | 910 | 18.176 | 69.798 | 25.976 |
| 2293 | HN | SER | 910 | 16.441 | 69.553 | 24.759 |
| 2294 | C | SER | 910 | 18.197 | 69.481 | 27.480 |
| 2295 | O | SER | 910 | 19.277 | 69.320 | 28.049 |
| 2296 | CB | SER | 910 | 17.984 | 71.313 | 25.716 |
| 2297 | OG | SER | 910 | 16.821 | 71.846 | 26.359 |
| 2298 | HG | SER | 910 | 16.055 | 71.406 | 25.978 |
| 2299 | N | GLU | 911 | 17.014 | 69.378 | 28.106 |
| 2300 | CA | GLU | 911 | 16.852 | 69.103 | 29.554 |
| 2301 | HN | GLU | 911 | 16.185 | 69.496 | 27.559 |
| 2302 | C | GLU | 911 | 17.169 | 67.641 | 29.921 |
| 2303 | O | GLU | 911 | 17.828 | 67.362 | 30.929 |
| 2304 | CB | GLU | 911 | 15.399 | 69.460 | 30.000 |
| 2305 | CG | GLU | 911 | 15.156 | 69.773 | 31.529 |
| 2306 | CD | GLU | 911 | 14.687 | 71.232 | 31.813 |
| 2307 | OE1 | GLU | 911 | 15.306 | 72.174 | 31.267 |
| 2308 | OE2 | GLU | 911 | 13.718 | 71.441 | 32.590 |
| 2309 | HE2 | GLU | 911 | 13.562 | 72.371 | 32.656 |
| 2310 | N | VAL | 912 | 16.693 | 66.718 | 29.088 |

| | | | | | | |
|---|---|---|---|---|---|---|
| 2311 | CA | VAL | 912 | 16.907 | 65.293 | 29.296 |
| 2312 | HN | VAL | 912 | 16.168 | 67.015 | 28.290 |
| 2313 | C | VAL | 912 | 18.373 | 64.971 | 29.082 |
| 2314 | O | VAL | 912 | 18.964 | 64.230 | 29.866 |
| 2315 | CB | VAL | 912 | 15.999 | 64.466 | 28.300 |
| 2316 | CG1 | VAL | 912 | 14.508 | 64.893 | 28.211 |
| 2317 | CG2 | VAL | 912 | 16.511 | 64.465 | 26.839 |
| 2318 | N | ILE | 913 | 18.973 | 65.514 | 28.026 |
| 2319 | CA | ILE | 913 | 20.389 | 65.246 | 27.825 |
| 2320 | HN | ILE | 913 | 18.465 | 66.091 | 27.387 |
| 2321 | C | ILE | 913 | 21.054 | 65.695 | 29.123 |
| 2322 | O | ILE | 913 | 21.556 | 64.862 | 29.876 |
| 2323 | CB | ILE | 913 | 20.963 | 66.006 | 26.611 |
| 2324 | CG2 | ILE | 913 | 22.463 | 65.834 | 26.555 |
| 2325 | CG1 | ILE | 913 | 20.385 | 65.418 | 25.322 |
| 2326 | CD1 | ILE | 913 | 20.604 | 66.257 | 24.089 |
| 2327 | N | ALA | 914 | 21.002 | 67.001 | 29.398 |
| 2328 | CA | ALA | 914 | 21.566 | 67.613 | 30.615 |
| 2329 | HN | ALA | 914 | 20.554 | 67.602 | 28.736 |
| 2330 | C | ALA | 914 | 21.374 | 66.795 | 31.884 |
| 2331 | O | ALA | 914 | 22.286 | 66.672 | 32.706 |
| 2332 | CB | ALA | 914 | 20.967 | 69.026 | 30.719 |
| 2333 | N | ALA | 915 | 20.169 | 66.272 | 32.061 |
| 2334 | CA | ALA | 915 | 19.888 | 65.470 | 33.231 |
| 2335 | HN | ALA | 915 | 19.452 | 66.433 | 31.384 |
| 2336 | C | ALA | 915 | 20.679 | 64.169 | 33.202 |
| 2337 | O | ALA | 915 | 21.380 | 63.839 | 34.153 |
| 2338 | CB | ALA | 915 | 18.369 | 65.242 | 33.315 |
| 2339 | N | GLN | 916 | 20.582 | 63.445 | 32.093 |
| 2340 | CA | GLN | 916 | 21.263 | 62.164 | 31.955 |
| 2341 | HN | GLN | 916 | 20.028 | 63.787 | 31.334 |
| 2342 | C | GLN | 916 | 22.784 | 62.163 | 31.856 |
| 2343 | O | GLN | 916 | 23.422 | 61.208 | 32.299 |
| 2344 | CB | GLN | 916 | 20.717 | 61.415 | 30.737 |
| 2345 | CG | GLN | 916 | 19.253 | 61.040 | 30.828 |
| 2346 | CD | GLN | 916 | 18.940 | 60.208 | 32.056 |
| 2347 | OE1 | GLN | 916 | 19.638 | 59.237 | 32.361 |
| 2348 | NE2 | GLN | 916 | 18.007 | 60.708 | 32.969 |
| 2349 | 1HE2 | GLN | 916 | 17.720 | 60.104 | 33.720 |
| 2350 | 2HE2 | GLN | 916 | 17.531 | 61.588 | 32.810 |
| 2351 | N | LEU | 917 | 23.371 | 63.214 | 31.288 |
| 2352 | CA | LEU | 917 | 24.819 | 63.244 | 31.093 |
| 2353 | HN | LEU | 917 | 22.816 | 63.991 | 30.991 |
| 2354 | C | LEU | 917 | 25.674 | 62.568 | 32.174 |
| 2355 | O | LEU | 917 | 26.442 | 61.655 | 31.866 |
| 2356 | CB | LEU | 917 | 25.308 | 64.696 | 30.824 |
| 2357 | CG | LEU | 917 | 24.629 | 65.496 | 29.680 |
| 2358 | CD1 | LEU | 917 | 25.512 | 65.475 | 28.425 |
| 2359 | CD2 | LEU | 917 | 23.227 | 64.967 | 29.336 |
| 2360 | N | PRO | 918 | 25.556 | 62.991 | 33.449 |
| 2361 | CA | PRO | 918 | 26.384 | 62.331 | 34.468 |
| 2362 | CD | PRO | 918 | 24.669 | 63.995 | 34.066 |
| 2363 | C | PRO | 918 | 26.046 | 60.853 | 34.659 |
| 2364 | O | PRO | 918 | 26.772 | 59.978 | 34.194 |
| 2365 | CB | PRO | 918 | 26.109 | 63.164 | 35.722 |

| | | | | | | |
|---|---|---|---|---|---|---|
| 2366 | CG | PRO | 918 | 24.691 | 63.591 | 35.528 |
| 2367 | N | LYS | 919 | 24.927 | 60.589 | 35.323 |
| 2368 | CA | LYS | 919 | 24.463 | 59.233 | 35.620 |
| 2369 | HN | LYS | 919 | 24.371 | 61.358 | 35.638 |
| 2370 | C | LYS | 919 | 24.606 | 58.128 | 34.550 |
| 2371 | O | LYS | 919 | 24.720 | 56.946 | 34.900 |
| 2372 | CB | LYS | 919 | 22.997 | 59.291 | 36.064 |
| 2373 | CG | LYS | 919 | 22.030 | 59.560 | 34.928 |
| 2374 | CD | LYS | 919 | 20.583 | 59.336 | 35.345 |
| 2375 | CE | LYS | 919 | 20.083 | 60.442 | 36.262 |
| 2376 | NZ | LYS | 919 | 18.618 | 60.349 | 36.653 |
| 2377 | HZ1 | LYS | 919 | 18.227 | 61.297 | 36.750 |
| 2378 | HZ2 | LYS | 919 | 18.100 | 59.837 | 35.926 |
| 2379 | HZ3 | LYS | 919 | 18.536 | 59.864 | 37.524 |
| 2380 | N | ILE | 920 | 24.591 | 58.493 | 33.265 |
| 2381 | CA | ILE | 920 | 24.699 | 57.512 | 32.172 |
| 2382 | HN | ILE | 920 | 24.503 | 59.463 | 33.038 |
| 2383 | C | ILE | 920 | 26.087 | 56.939 | 31.960 |
| 2384 | O | ILE | 920 | 26.377 | 55.800 | 32.329 |
| 2385 | CB | ILE | 920 | 24.127 | 58.173 | 30.852 |
| 2386 | CG1 | ILE | 920 | 22.815 | 57.520 | 30.312 |
| 2387 | CG2 | ILE | 920 | 25.168 | 58.202 | 29.690 |
| 2388 | CD1 | ILE | 920 | 21.527 | 58.350 | 30.490 |
| 2389 | N | LEU | 921 | 26.923 | 57.761 | 31.330 |
| 2390 | CA | LEU | 921 | 28.299 | 57.423 | 30.985 |
| 2391 | HN | LEU | 921 | 26.586 | 58.668 | 31.076 |
| 2392 | C | LEU | 921 | 28.969 | 56.365 | 31.867 |
| 2393 | O | LEU | 921 | 29.557 | 55.412 | 31.351 |
| 2394 | CB | LEU | 921 | 29.155 | 58.720 | 30.925 |
| 2395 | CG | LEU | 921 | 29.319 | 59.549 | 32.228 |
| 2396 | CD1 | LEU | 921 | 30.099 | 60.839 | 31.938 |
| 2397 | CD2 | LEU | 921 | 27.973 | 59.894 | 32.886 |
| 2398 | N | ALA | 922 | 28.866 | 56.524 | 33.185 |
| 2399 | CA | ALA | 922 | 29.474 | 55.585 | 34.132 |
| 2400 | HN | ALA | 922 | 28.358 | 57.309 | 33.540 |
| 2401 | C | ALA | 922 | 28.974 | 54.145 | 33.984 |
| 2402 | O | ALA | 922 | 29.703 | 53.261 | 33.522 |
| 2403 | CB | ALA | 922 | 29.258 | 56.148 | 35.547 |
| 2404 | N | GLY | 923 | 27.727 | 53.925 | 34.405 |
| 2405 | CA | GLY | 923 | 27.115 | 52.605 | 34.341 |
| 2406 | HN | GLY | 923 | 27.198 | 54.688 | 34.776 |
| 2407 | C | GLY | 923 | 26.426 | 52.237 | 35.647 |
| 2408 | O | GLY | 923 | 26.387 | 51.071 | 36.044 |
| 2409 | N | MET | 924 | 25.866 | 53.251 | 36.308 |
| 2410 | CA | MET | 924 | 25.182 | 53.106 | 37.593 |
| 2411 | HN | MET | 924 | 25.916 | 54.163 | 35.901 |
| 2412 | C | MET | 924 | 23.656 | 52.955 | 37.392 |
| 2413 | O | MET | 924 | 22.851 | 53.294 | 38.269 |
| 2414 | CB | MET | 924 | 25.507 | 54.360 | 38.450 |
| 2415 | CG | MET | 924 | 26.778 | 55.139 | 38.049 |
| 2416 | SD | MET | 924 | 26.396 | 56.892 | 37.892 |
| 2417 | CE | MET | 924 | 27.901 | 57.420 | 37.064 |
| 2418 | N | VAL | 925 | 23.302 | 52.427 | 36.214 |
| 2419 | CA | VAL | 925 | 21.930 | 52.172 | 35.734 |
| 2420 | HN | VAL | 925 | 24.046 | 52.179 | 35.594 |

| | | | | | | |
|---|---|---|---|---|---|---|
| 2421 | C | VAL | 925 | 21.829 | 50.671 | 35.391 |
| 2422 | O | VAL | 925 | 22.810 | 50.084 | 34.932 |
| 2423 | CB | VAL | 925 | 21.666 | 53.060 | 34.453 |
| 2424 | CG1 | VAL | 925 | 21.087 | 54.480 | 34.706 |
| 2425 | CG2 | VAL | 925 | 22.920 | 53.281 | 33.574 |
| 2426 | N | LYS | 926 | 20.666 | 50.053 | 35.581 |
| 2427 | CA | LYS | 926 | 20.552 | 48.628 | 35.274 |
| 2428 | HN | LYS | 926 | 19.878 | 50.558 | 35.931 |
| 2429 | C | LYS | 926 | 19.777 | 48.267 | 34.014 |
| 2430 | O | LYS | 926 | 18.544 | 48.228 | 34.010 |
| 2431 | CB | LYS | 926 | 19.948 | 47.873 | 36.448 |
| 2432 | CG | LYS | 926 | 19.773 | 46.377 | 36.216 |
| 2433 | CD | LYS | 926 | 18.912 | 45.841 | 37.339 |
| 2434 | CE | LYS | 926 | 18.473 | 44.409 | 37.153 |
| 2435 | NZ | LYS | 926 | 17.517 | 43.918 | 38.231 |
| 2436 | HZ1 | LYS | 926 | 16.988 | 44.716 | 38.611 |
| 2437 | HZ2 | LYS | 926 | 16.862 | 43.235 | 37.825 |
| 2438 | HZ3 | LYS | 926 | 18.038 | 43.483 | 38.966 |
| 2439 | N | PRO | 927 | 20.529 | 47.980 | 32.960 |
| 2440 | CA | PRO | 927 | 19.971 | 47.592 | 31.674 |
| 2441 | C | PRO | 927 | 19.437 | 46.168 | 31.795 |
| 2442 | O | PRO | 927 | 20.208 | 45.247 | 32.038 |
| 2443 | CB | PRO | 927 | 21.082 | 47.730 | 30.622 |
| 2444 | CG | PRO | 927 | 22.370 | 47.475 | 31.420 |
| 2445 | CD | PRO | 927 | 22.089 | 48.145 | 32.767 |
| 2446 | N | LEU | 928 | 18.132 | 45.976 | 31.641 |
| 2447 | CA | LEU | 928 | 17.573 | 44.628 | 31.718 |
| 2448 | HN | LEU | 928 | 17.530 | 46.756 | 31.472 |
| 2449 | C | LEU | 928 | 17.956 | 43.896 | 30.438 |
| 2450 | O | LEU | 928 | 17.995 | 44.499 | 29.370 |
| 2451 | CB | LEU | 928 | 16.059 | 44.688 | 31.811 |
| 2452 | CG | LEU | 928 | 15.563 | 45.735 | 32.794 |
| 2453 | CD1 | LEU | 928 | 14.058 | 45.677 | 32.854 |
| 2454 | CD2 | LEU | 928 | 16.163 | 45.484 | 34.161 |
| 2455 | N | LEU | 929 | 18.239 | 42.605 | 30.527 |
| 2456 | CA | LEU | 929 | 18.602 | 41.856 | 29.326 |
| 2457 | HN | LEU | 929 | 18.205 | 42.144 | 31.413 |
| 2458 | C | LEU | 929 | 17.790 | 40.570 | 29.208 |
| 2459 | O | LEU | 929 | 17.364 | 40.025 | 30.218 |
| 2460 | CB | LEU | 929 | 20.098 | 41.528 | 29.346 |
| 2461 | CG | LEU | 929 | 21.046 | 42.715 | 29.164 |
| 2462 | CD1 | LEU | 929 | 22.449 | 42.196 | 28.955 |
| 2463 | CD2 | LEU | 929 | 20.639 | 43.537 | 27.954 |
| 2464 | N | PHE | 930 | 17.547 | 40.099 | 27.984 |
| 2465 | CA | PHE | 930 | 16.801 | 38.847 | 27.812 |
| 2466 | HN | PHE | 930 | 17.874 | 40.599 | 27.182 |
| 2467 | C | PHE | 930 | 17.772 | 37.692 | 27.803 |
| 2468 | O | PHE | 930 | 17.483 | 36.618 | 28.320 |
| 2469 | CB | PHE | 930 | 16.012 | 38.831 | 26.509 |
| 2470 | CG | PHE | 930 | 14.783 | 39.658 | 26.558 |
| 2471 | CD1 | PHE | 930 | 13.647 | 39.198 | 27.206 |
| 2472 | CE1 | PHE | 930 | 12.534 | 40.005 | 27.331 |
| 2473 | CZ | PHE | 930 | 12.552 | 41.283 | 26.807 |
| 2474 | CE2 | PHE | 930 | 13.680 | 41.743 | 26.155 |
| 2475 | CD2 | PHE | 930 | 14.787 | 40.933 | 26.035 |

| | | | | | | |
|---|---|---|---|---|---|---|
| 2476 | N | HIS | 931 | 18.929 | 37.930 | 27.198 |
| 2477 | CA | HIS | 931 | 19.985 | 36.938 | 27.104 |
| 2478 | HN | HIS | 931 | 19.079 | 38.830 | 26.789 |
| 2479 | C | HIS | 931 | 21.218 | 37.571 | 27.755 |
| 2480 | O | HIS | 931 | 21.562 | 38.710 | 27.432 |
| 2481 | CB | HIS | 931 | 20.300 | 36.628 | 25.631 |
| 2482 | CG | HIS | 931 | 19.093 | 36.359 | 24.792 |
| 2483 | ND1 | HIS | 931 | 18.209 | 35.328 | 25.058 |
| 2484 | CE1 | HIS | 931 | 17.249 | 35.325 | 24.154 |
| 2485 | NE2 | HIS | 931 | 17.472 | 36.322 | 23.306 |
| 2486 | HE2 | HIS | 931 | 16.883 | 36.563 | 22.496 |
| 2487 | CD2 | HIS | 931 | 18.612 | 36.976 | 23.686 |
| 2488 | N | LYS | 932 | 21.870 | 36.851 | 28.668 |
| 2489 | CA | LYS | 932 | 23.078 | 37.366 | 29.332 |
| 2490 | HN | LYS | 932 | 21.531 | 35.941 | 28.907 |
| 2491 | C | LYS | 932 | 24.313 | 36.931 | 28.518 |
| 2492 | OXT | LYS | 932 | 25.424 | 37.309 | 29.006 |
| 2493 | O | LYS | 932 | 24.485 | 35.702 | 28.245 |
| 2494 | CB | LYS | 932 | 23.155 | 36.832 | 30.789 |
| 2495 | CG | LYS | 932 | 23.857 | 35.456 | 30.904 |
| 2496 | CD | LYS | 932 | 25.374 | 35.521 | 31.097 |
| 2497 | CE | LYS | 932 | 25.987 | 34.158 | 30.750 |
| 2498 | NZ | LYS | 932 | 27.457 | 34.263 | 30.767 |
| 2499 | HZ2 | LYS | 932 | 27.766 | 34.889 | 31.519 |
| 2500 | HZ1 | LYS | 932 | 27.893 | 33.354 | 30.955 |
| 2501 | HZ3 | LYS | 932 | 27.777 | 34.611 | 29.886 |
| 2502 | CL1 | RWJ | 1 | 14.371 | 60.020 | 15.893 |
| 2503 | C2 | RWJ | 1 | 15.944 | 60.715 | 16.157 |
| 2504 | C3 | RWJ | 1 | 17.076 | 59.893 | 16.188 |
| 2505 | C4 | RWJ | 1 | 16.933 | 58.402 | 15.993 |
| 2506 | F5 | RWJ | 1 | 16.659 | 57.827 | 17.160 |
| 2507 | F6 | RWJ | 1 | 18.068 | 57.907 | 15.510 |
| 2508 | F7 | RWJ | 1 | 15.944 | 58.160 | 15.137 |
| 2509 | C8 | RWJ | 1 | 18.342 | 60.452 | 16.400 |
| 2510 | C9 | RWJ | 1 | 18.475 | 61.834 | 16.581 |
| 2511 | C10 | RWJ | 1 | 19.813 | 62.425 | 16.805 |
| 2512 | N11 | RWJ | 1 | 20.828 | 61.627 | 17.080 |
| 2513 | N12 | RWJ | 1 | 22.183 | 62.087 | 17.319 |
| 2514 | S13 | RWJ | 1 | 23.312 | 60.945 | 16.624 |
| 2515 | O14 | RWJ | 1 | 22.844 | 59.613 | 16.851 |
| 2516 | O15 | RWJ | 1 | 24.594 | 61.111 | 17.236 |
| 2517 | C16 | RWJ | 1 | 23.453 | 61.246 | 14.814 |
| 2518 | C17 | RWJ | 1 | 24.421 | 62.130 | 14.325 |
| 2519 | C18 | RWJ | 1 | 24.528 | 62.358 | 12.948 |
| 2520 | C19 | RWJ | 1 | 23.667 | 61.702 | 12.060 |
| 2521 | I20 | RWJ | 1 | 23.827 | 62.045 | 9.995 |
| 2522 | C21 | RWJ | 1 | 22.698 | 60.818 | 12.550 |
| 2523 | C22 | RWJ | 1 | 22.591 | 60.589 | 13.927 |
| 2524 | C23 | RWJ | 1 | 22.448 | 63.426 | 16.774 |
| 2525 | C24 | RWJ | 1 | 23.812 | 63.921 | 17.247 |
| 2526 | C25 | RWJ | 1 | 21.352 | 64.423 | 17.195 |
| 2527 | C26 | RWJ | 1 | 19.977 | 63.923 | 16.713 |
| 2528 | C27 | RWJ | 1 | 17.343 | 62.656 | 16.550 |
| 2529 | C28 | RWJ | 1 | 16.077 | 62.097 | 16.337 |

TABLE 6

SUBSET OF ATOMIC COORDINATES OF GRα IN COMPLEX WITH THE BENZOXAZIN-1-ONE OBTAINED FROM MODELING OF THE CRYSTAL STRUCTURE OF GRα IN COMPLEX WITH FP

| Atom Number | Atom Type | Residue Name | Residue Number | X Coordinate | Y Coordinate | Z Coordinate |
|---|---|---|---|---|---|---|
| 1 | N | LEU | 544 | 5.940 | 62.649 | 11.764 |
| 2 | CA | LEU | 544 | 5.974 | 64.102 | 11.777 |
| 3 | CB | LEU | 544 | 5.952 | 64.855 | 13.135 |
| 4 | CG | LEU | 544 | 6.701 | 64.350 | 14.406 |
| 5 | CD1 | LEU | 544 | 6.322 | 65.241 | 15.616 |
| 6 | CD2 | LEU | 544 | 6.415 | 62.880 | 14.786 |
| 7 | C | LEU | 544 | 7.164 | 64.437 | 10.826 |
| 8 | O | LEU | 544 | 8.239 | 63.851 | 10.879 |
| 9 | HN | LEU | 544 | 6.575 | 62.118 | 12.352 |
| 10 | N | THR | 556 | 26.585 | 69.651 | 7.255 |
| 11 | CA | THR | 556 | 26.781 | 68.735 | 8.382 |
| 12 | CB | THR | 556 | 28.265 | 68.599 | 8.879 |
| 13 | OG1 | THR | 556 | 29.183 | 68.249 | 7.833 |
| 14 | CG2 | THR | 556 | 28.366 | 67.548 | 10.003 |
| 15 | C | THR | 556 | 25.830 | 69.214 | 9.501 |
| 16 | O | THR | 556 | 24.880 | 68.495 | 9.743 |
| 17 | HN | THR | 556 | 27.395 | 69.596 | 6.643 |
| 18 | HG1 | THR | 556 | 30.058 | 68.161 | 8.250 |
| 19 | N | ILE | 559 | 21.633 | 70.003 | 8.878 |
| 20 | CA | ILE | 559 | 20.880 | 68.737 | 8.871 |
| 21 | CB | ILE | 559 | 21.416 | 67.541 | 8.014 |
| 22 | CG2 | ILE | 559 | 20.550 | 66.283 | 8.280 |
| 23 | CG1 | ILE | 559 | 21.498 | 67.829 | 6.495 |
| 24 | CD1 | ILE | 559 | 21.957 | 66.639 | 5.616 |
| 25 | C | ILE | 559 | 20.891 | 68.309 | 10.355 |
| 26 | O | ILE | 559 | 19.818 | 68.267 | 10.917 |
| 27 | HN | ILE | 559 | 22.559 | 69.900 | 8.462 |
| 28 | N | MET | 560 | 22.116 | 67.951 | 11.005 |
| 29 | CA | MET | 560 | 22.238 | 67.530 | 12.398 |
| 30 | CB | MET | 560 | 23.650 | 67.542 | 13.010 |
| 31 | CG | MET | 560 | 24.560 | 66.453 | 12.433 |
| 32 | SD | MET | 560 | 26.049 | 66.144 | 13.442 |
| 33 | CE | MET | 560 | 26.836 | 64.975 | 12.282 |
| 34 | C | MET | 560 | 21.376 | 68.480 | 13.244 |
| 35 | O | MET | 560 | 20.739 | 67.976 | 14.146 |
| 36 | HN | MET | 560 | 22.988 | 67.997 | 10.493 |
| 37 | N | THR | 562 | 18.442 | 70.366 | 12.296 |
| 38 | CA | THR | 562 | 16.979 | 69.979 | 12.264 |
| 39 | CB | THR | 562 | 16.011 | 70.077 | 11.038 |
| 40 | OG1 | THR | 562 | 16.263 | 69.083 | 10.049 |
| 41 | CG2 | THR | 562 | 15.882 | 71.488 | 10.437 |
| 42 | C | THR | 562 | 16.792 | 68.531 | 12.863 |
| 43 | O | THR | 562 | 15.662 | 68.207 | 13.197 |
| 44 | HN | THR | 562 | 18.984 | 70.366 | 11.451 |
| 45 | HG1 | THR | 562 | 15.410 | 68.898 | 9.600 |

| | | | | | | |
|---|---|---|---|---|---|---|
| 46 | N | LEU | 563 | 17.916 | 67.661 | 12.989 |
| 47 | CA | LEU | 563 | 17.667 | 66.302 | 13.464 |
| 48 | CB | LEU | 563 | 18.721 | 65.226 | 13.171 |
| 49 | CG | LEU | 563 | 18.460 | 64.497 | 11.832 |
| 50 | CD1 | LEU | 563 | 17.838 | 65.349 | 10.696 |
| 51 | CD2 | LEU | 563 | 19.764 | 63.838 | 11.363 |
| 52 | C | LEU | 563 | 17.434 | 66.528 | 14.954 |
| 53 | O | LEU | 563 | 16.437 | 66.052 | 15.458 |
| 54 | HN | LEU | 563 | 18.676 | 67.802 | 12.334 |
| 55 | N | ASN | 564 | 18.322 | 67.363 | 15.671 |
| 56 | CA | ASN | 564 | 17.960 | 67.789 | 17.027 |
| 57 | CB | ASN | 564 | 18.861 | 68.818 | 17.707 |
| 58 | CG | ASN | 564 | 20.332 | 68.431 | 17.754 |
| 59 | OD1 | ASN | 564 | 20.859 | 67.537 | 17.111 |
| 60 | ND2 | ASN | 564 | 21.098 | 69.270 | 18.563 |
| 61 | C | ASN | 564 | 16.512 | 68.379 | 17.099 |
| 62 | O | ASN | 564 | 15.949 | 68.320 | 18.183 |
| 63 | HN | ASN | 564 | 19.184 | 67.712 | 15.267 |
| 64 | 1HD2 | ASN | 564 | 22.013 | 69.437 | 18.178 |
| 65 | 2HD2 | ASN | 564 | 20.718 | 70.064 | 19.073 |
| 66 | N | MET | 565 | 15.876 | 68.982 | 15.969 |
| 67 | CA | MET | 565 | 14.505 | 69.517 | 16.086 |
| 68 | CB | MET | 565 | 13.941 | 70.565 | 15.096 |
| 69 | CG | MET | 565 | 13.884 | 71.995 | 15.667 |
| 70 | SD | MET | 565 | 15.501 | 72.811 | 15.848 |
| 71 | CE | MET | 565 | 14.936 | 74.510 | 16.210 |
| 72 | C | MET | 565 | 13.523 | 68.316 | 16.120 |
| 73 | O | MET | 565 | 12.755 | 68.248 | 17.069 |
| 74 | HN | MET | 565 | 16.356 | 69.053 | 15.074 |
| 75 | N | LEU | 566 | 13.511 | 67.417 | 15.005 |
| 76 | CA | LEU | 566 | 12.680 | 66.204 | 14.995 |
| 77 | CB | LEU | 566 | 12.958 | 65.320 | 13.758 |
| 78 | CG | LEU | 566 | 11.899 | 64.218 | 13.505 |
| 79 | CD1 | LEU | 566 | 10.472 | 64.760 | 13.312 |
| 80 | CD2 | LEU | 566 | 12.276 | 63.386 | 12.274 |
| 81 | C | LEU | 566 | 12.864 | 65.372 | 16.314 |
| 82 | O | LEU | 566 | 11.991 | 64.589 | 16.666 |
| 83 | HN | LEU | 566 | 14.194 | 67.502 | 14.256 |
| 84 | N | GLY | 567 | 14.065 | 65.634 | 17.074 |
| 85 | CA | GLY | 567 | 14.368 | 65.122 | 18.410 |
| 86 | C | GLY | 567 | 13.395 | 65.818 | 19.373 |
| 87 | O | GLY | 567 | 12.470 | 65.164 | 19.815 |
| 88 | HN | GLY | 567 | 14.755 | 66.315 | 16.727 |
| 89 | N | GLY | 568 | 13.610 | 67.204 | 19.643 |
| 90 | CA | GLY | 568 | 12.543 | 67.993 | 20.319 |
| 91 | C | GLY | 568 | 11.085 | 67.356 | 20.210 |
| 92 | O | GLY | 568 | 10.571 | 66.926 | 21.229 |
| 93 | HN | GLY | 568 | 14.527 | 67.648 | 19.464 |
| 94 | N | GLN | 570 | 9.435 | 64.691 | 18.381 |
| 95 | CA | GLN | 570 | 9.047 | 63.293 | 18.600 |
| 96 | CB | GLN | 570 | 9.949 | 62.264 | 17.954 |
| 97 | CG | GLN | 570 | 9.720 | 61.923 | 16.466 |
| 98 | CD | GLN | 570 | 10.263 | 60.509 | 16.247 |
| 99 | OE1 | GLN | 570 | 9.774 | 59.737 | 15.440 |
| 100 | NE2 | GLN | 570 | 11.375 | 60.181 | 17.074 |

| | | | | | | |
|---|---|---|---|---|---|---|
| 101 | C | GLN | 570 | 9.122 | 62.923 | 20.112 |
| 102 | O | GLN | 570 | 8.291 | 62.153 | 20.562 |
| 103 | HN | GLN | 570 | 10.403 | 64.886 | 18.148 |
| 104 | 1HE2 | GLN | 570 | 11.793 | 59.248 | 17.045 |
| 105 | 2HE2 | GLN | 570 | 11.654 | 60.884 | 17.791 |
| 106 | N | VAL | 571 | 10.216 | 63.398 | 20.902 |
| 107 | CA | VAL | 571 | 10.426 | 62.911 | 22.293 |
| 108 | CB | VAL | 571 | 11.858 | 63.181 | 22.856 |
| 109 | CG1 | VAL | 571 | 12.086 | 62.848 | 24.356 |
| 110 | CG2 | VAL | 571 | 12.908 | 62.362 | 22.069 |
| 111 | C | VAL | 571 | 9.247 | 63.480 | 23.196 |
| 112 | O | VAL | 571 | 9.117 | 63.034 | 24.321 |
| 113 | HN | VAL | 571 | 10.991 | 63.862 | 20.446 |
| 114 | N | TRP | 600 | 14.536 | 57.459 | 26.142 |
| 115 | CA | TRP | 600 | 14.286 | 58.373 | 25.033 |
| 116 | CB | TRP | 600 | 14.536 | 59.885 | 25.241 |
| 117 | CG | TRP | 600 | 15.942 | 60.382 | 25.547 |
| 118 | CD2 | TRP | 600 | 16.813 | 61.177 | 24.647 |
| 119 | CE2 | TRP | 600 | 17.938 | 61.507 | 25.406 |
| 120 | CE3 | TRP | 600 | 16.684 | 61.587 | 23.302 |
| 121 | CD1 | TRP | 600 | 16.677 | 60.278 | 26.692 |
| 122 | NE1 | TRP | 600 | 17.971 | 60.868 | 26.641 |
| 123 | CZ2 | TRP | 600 | 18.944 | 62.371 | 24.931 |
| 124 | CZ3 | TRP | 600 | 17.722 | 62.405 | 22.786 |
| 125 | CH2 | TRP | 600 | 18.804 | 62.836 | 23.600 |
| 126 | C | TRP | 600 | 15.102 | 57.802 | 23.846 |
| 127 | O | TRP | 600 | 14.500 | 57.522 | 22.826 |
| 128 | HN | TRP | 600 | 14.723 | 57.833 | 27.064 |
| 129 | HE1 | TRP | 600 | 18.815 | 60.295 | 26.697 |
| 130 | N | MET | 601 | 16.503 | 57.579 | 23.980 |
| 131 | CA | MET | 601 | 17.340 | 57.410 | 22.781 |
| 132 | CB | MET | 601 | 18.846 | 57.418 | 23.119 |
| 133 | CG | MET | 601 | 19.743 | 57.697 | 21.905 |
| 134 | SD | MET | 601 | 19.495 | 59.370 | 21.193 |
| 135 | CE | MET | 601 | 20.712 | 60.293 | 22.193 |
| 136 | C | MET | 601 | 16.991 | 56.112 | 21.981 |
| 137 | O | MET | 601 | 17.166 | 56.100 | 20.777 |
| 138 | HN | MET | 601 | 16.954 | 58.108 | 24.708 |
| 139 | N | MET | 604 | 13.789 | 56.337 | 20.103 |
| 140 | CA | MET | 604 | 14.221 | 56.912 | 18.813 |
| 141 | CB | MET | 604 | 15.241 | 58.084 | 18.878 |
| 142 | CG | MET | 604 | 14.883 | 59.305 | 19.753 |
| 143 | SD | MET | 604 | 13.383 | 60.194 | 19.224 |
| 144 | CE | MET | 604 | 12.124 | 59.378 | 20.277 |
| 145 | C | MET | 604 | 14.836 | 55.772 | 17.917 |
| 146 | O | MET | 604 | 14.376 | 55.567 | 16.809 |
| 147 | HN | MET | 604 | 14.289 | 56.598 | 20.957 |
| 148 | N | ALA | 605 | 16.001 | 55.112 | 18.403 |
| 149 | CA | ALA | 605 | 16.978 | 54.442 | 17.507 |
| 150 | CB | ALA | 605 | 18.203 | 53.924 | 18.267 |
| 151 | C | ALA | 605 | 16.325 | 53.250 | 16.752 |
| 152 | O | ALA | 605 | 16.626 | 52.982 | 15.592 |
| 153 | HN | ALA | 605 | 16.380 | 55.356 | 19.311 |
| 154 | N | PHE | 606 | 15.361 | 52.586 | 17.574 |
| 155 | CA | PHE | 606 | 14.666 | 51.352 | 17.214 |

| | | | | | | |
|---|---|---|---|---|---|---|
| 156 | CB | PHE | 606 | 13.830 | 50.825 | 18.391 |
| 157 | CG | PHE | 606 | 13.363 | 49.414 | 18.133 |
| 158 | CD1 | PHE | 606 | 14.303 | 48.329 | 18.243 |
| 159 | CD2 | PHE | 606 | 11.979 | 49.117 | 17.871 |
| 160 | CE1 | PHE | 606 | 13.894 | 46.982 | 18.032 |
| 161 | CE2 | PHE | 606 | 11.568 | 47.761 | 17.629 |
| 162 | CZ | PHE | 606 | 12.541 | 46.700 | 17.660 |
| 163 | C | PHE | 606 | 13.748 | 51.686 | 16.029 |
| 164 | O | PHE | 606 | 13.790 | 50.980 | 15.040 |
| 165 | HN | PHE | 606 | 15.215 | 52.914 | 18.530 |
| 166 | N | ALA | 607 | 12.903 | 52.830 | 16.191 |
| 167 | CA | ALA | 607 | 11.863 | 53.392 | 15.284 |
| 168 | CB | ALA | 607 | 10.919 | 54.387 | 15.994 |
| 169 | C | ALA | 607 | 12.445 | 54.123 | 13.996 |
| 170 | O | ALA | 607 | 11.819 | 54.073 | 12.944 |
| 171 | HN | ALA | 607 | 12.937 | 53.245 | 17.125 |
| 172 | N | LEU | 608 | 13.643 | 54.902 | 14.103 |
| 173 | CA | LEU | 608 | 14.415 | 55.258 | 12.889 |
| 174 | CB | LEU | 608 | 15.872 | 55.766 | 13.015 |
| 175 | CG | LEU | 608 | 16.535 | 56.170 | 11.672 |
| 176 | CD1 | LEU | 608 | 15.564 | 56.947 | 10.783 |
| 177 | CD2 | LEU | 608 | 17.744 | 57.096 | 11.876 |
| 178 | C | LEU | 608 | 14.527 | 53.974 | 12.051 |
| 179 | O | LEU | 608 | 14.127 | 53.955 | 10.899 |
| 180 | HN | LEU | 608 | 14.169 | 54.816 | 14.968 |
| 181 | N | GLY | 609 | 15.072 | 52.871 | 12.762 |
| 182 | CA | GLY | 609 | 15.130 | 51.566 | 12.135 |
| 183 | C | GLY | 609 | 13.878 | 51.185 | 11.368 |
| 184 | O | GLY | 609 | 13.970 | 50.700 | 10.245 |
| 185 | HN | GLY | 609 | 15.265 | 52.896 | 13.770 |
| 186 | N | ARG | 611 | 10.880 | 53.198 | 10.162 |
| 187 | CA | ARG | 611 | 10.368 | 53.648 | 8.778 |
| 188 | CB | ARG | 611 | 9.869 | 55.007 | 8.239 |
| 189 | CG | ARG | 611 | 8.729 | 55.665 | 9.011 |
| 190 | CD | ARG | 611 | 9.283 | 56.945 | 9.610 |
| 191 | NE | ARG | 611 | 10.457 | 56.745 | 10.480 |
| 192 | CZ | ARG | 611 | 10.610 | 57.593 | 11.582 |
| 193 | NH1 | ARG | 611 | 9.812 | 58.685 | 11.785 |
| 194 | NH2 | ARG | 611 | 11.554 | 57.401 | 12.563 |
| 195 | C | ARG | 611 | 11.487 | 53.314 | 7.767 |
| 196 | O | ARG | 611 | 11.211 | 53.119 | 6.594 |
| 197 | HN | ARG | 611 | 11.128 | 53.875 | 10.893 |
| 198 | HE | ARG | 611 | 11.063 | 55.964 | 10.255 |
| 199 | 1HH1 | ARG | 611 | 9.920 | 59.306 | 12.572 |
| 200 | 2HH1 | ARG | 611 | 9.092 | 58.880 | 11.120 |
| 201 | 1HH2 | ARG | 611 | 11.603 | 58.057 | 13.338 |
| 202 | 2HH2 | ARG | 611 | 12.199 | 56.633 | 12.574 |
| 203 | N | LEU | 621 | 18.327 | 56.358 | 5.645 |
| 204 | CA | LEU | 621 | 17.892 | 56.466 | 7.047 |
| 205 | CB | LEU | 621 | 18.922 | 57.150 | 7.968 |
| 206 | CG | LEU | 621 | 20.091 | 56.281 | 8.447 |
| 207 | CD1 | LEU | 621 | 20.935 | 57.119 | 9.419 |
| 208 | CD2 | LEU | 621 | 19.593 | 54.989 | 9.109 |
| 209 | C | LEU | 621 | 16.629 | 57.328 | 6.938 |
| 210 | O | LEU | 621 | 16.839 | 58.529 | 6.960 |

| | | | | | | |
|---|---|---|---|---|---|---|
| 211 | HN | LEU | 621 | 19.234 | 56.744 | 5.456 |
| 212 | N | CYS | 622 | 15.306 | 56.815 | 6.729 |
| 213 | CA | CYS | 622 | 14.234 | 57.828 | 6.639 |
| 214 | CB | CYS | 622 | 12.926 | 57.233 | 6.114 |
| 215 | SG | CYS | 622 | 13.160 | 56.480 | 4.471 |
| 216 | C | CYS | 622 | 14.056 | 58.457 | 8.070 |
| 217 | O | CYS | 622 | 13.430 | 57.849 | 8.930 |
| 218 | HN | CYS | 622 | 15.072 | 55.855 | 7.001 |
| 219 | HG | CYS | 622 | 13.936 | 55.462 | 4.807 |
| 220 | N | PHE | 623 | 14.654 | 59.748 | 8.325 |
| 221 | CA | PHE | 623 | 14.539 | 60.297 | 9.730 |
| 222 | CB | PHE | 623 | 15.401 | 61.531 | 10.118 |
| 223 | CG | PHE | 623 | 16.792 | 61.075 | 10.519 |
| 224 | CD1 | PHE | 623 | 17.717 | 60.632 | 9.510 |
| 225 | CD2 | PHE | 623 | 17.211 | 61.046 | 11.907 |
| 226 | CE1 | PHE | 623 | 19.023 | 60.154 | 9.875 |
| 227 | CE2 | PHE | 623 | 18.543 | 60.603 | 12.269 |
| 228 | CZ | PHE | 623 | 19.450 | 60.148 | 11.243 |
| 229 | C | PHE | 623 | 13.037 | 60.614 | 10.025 |
| 230 | O | PHE | 623 | 12.538 | 60.486 | 11.136 |
| 231 | HN | PHE | 623 | 15.054 | 60.333 | 7.580 |
| 232 | N | ALA | 624 | 12.406 | 61.114 | 8.853 |
| 233 | CA | ALA | 624 | 11.113 | 61.720 | 8.500 |
| 234 | CB | ALA | 624 | 11.040 | 63.230 | 8.800 |
| 235 | C | ALA | 624 | 11.011 | 61.564 | 6.937 |
| 236 | O | ALA | 624 | 11.997 | 61.723 | 6.236 |
| 237 | HN | ALA | 624 | 13.007 | 60.967 | 8.040 |
| 238 | N | ILE | 629 | 18.455 | 60.562 | 4.653 |
| 239 | CA | ILE | 629 | 19.897 | 60.753 | 4.531 |
| 240 | CB | ILE | 629 | 20.661 | 60.701 | 5.882 |
| 241 | CG2 | ILE | 629 | 22.180 | 60.700 | 5.633 |
| 242 | CG1 | ILE | 629 | 20.202 | 61.914 | 6.757 |
| 243 | CD1 | ILE | 629 | 21.100 | 62.288 | 7.948 |
| 244 | C | ILE | 629 | 20.234 | 59.677 | 3.467 |
| 245 | O | ILE | 629 | 20.440 | 58.503 | 3.743 |
| 246 | HN | ILE | 629 | 18.175 | 59.971 | 5.421 |
| 247 | N | CYS | 638 | 31.034 | 64.418 | 9.081 |
| 248 | CA | CYS | 638 | 30.921 | 63.606 | 10.354 |
| 249 | CB | CYS | 638 | 30.871 | 64.542 | 11.581 |
| 250 | SG | CYS | 638 | 32.117 | 65.871 | 11.538 |
| 251 | C | CYS | 638 | 29.661 | 62.632 | 10.415 |
| 252 | O | CYS | 638 | 29.460 | 62.015 | 11.451 |
| 253 | HN | CYS | 638 | 30.213 | 64.921 | 8.792 |
| 254 | HG | CYS | 638 | 33.063 | 65.261 | 12.246 |
| 255 | N | MET | 639 | 28.777 | 62.501 | 9.293 |
| 256 | CA | MET | 639 | 27.531 | 61.705 | 9.308 |
| 257 | CB | MET | 639 | 26.619 | 61.890 | 8.061 |
| 258 | CG | MET | 639 | 25.304 | 62.647 | 8.306 |
| 259 | SD | MET | 639 | 25.528 | 64.439 | 8.562 |
| 260 | CE | MET | 639 | 23.886 | 64.822 | 9.258 |
| 261 | C | MET | 639 | 27.781 | 60.167 | 9.463 |
| 262 | O | MET | 639 | 27.214 | 59.637 | 10.405 |
| 263 | HN | MET | 639 | 28.926 | 63.047 | 8.457 |
| 264 | N | GLN | 642 | 28.314 | 58.322 | 12.657 |
| 265 | CA | GLN | 642 | 27.386 | 58.403 | 13.783 |

| | | | | | | |
|---|---|---|---|---|---|---|
| 266 | CB | GLN | 642 | 27.149 | 59.910 | 13.979 |
| 267 | CG | GLN | 642 | 28.513 | 60.516 | 14.419 |
| 268 | CD | GLN | 642 | 28.943 | 59.776 | 15.690 |
| 269 | OE1 | GLN | 642 | 28.125 | 59.689 | 16.582 |
| 270 | NE2 | GLN | 642 | 30.231 | 59.214 | 15.889 |
| 271 | C | GLN | 642 | 26.125 | 57.570 | 13.438 |
| 272 | O | GLN | 642 | 25.600 | 56.815 | 14.249 |
| 273 | HN | GLN | 642 | 28.450 | 59.189 | 12.137 |
| 274 | 1HE2 | GLN | 642 | 30.419 | 58.883 | 16.846 |
| 275 | 2HE2 | GLN | 642 | 30.820 | 59.014 | 15.082 |
| 276 | N | CYS | 643 | 25.630 | 57.792 | 12.127 |
| 277 | CA | CYS | 643 | 24.401 | 57.297 | 11.541 |
| 278 | CB | CYS | 643 | 24.216 | 58.034 | 10.196 |
| 279 | SG | CYS | 643 | 23.694 | 59.790 | 10.338 |
| 280 | C | CYS | 643 | 24.479 | 55.742 | 11.373 |
| 281 | O | CYS | 643 | 23.439 | 55.131 | 11.153 |
| 282 | HN | CYS | 643 | 26.203 | 58.317 | 11.470 |
| 283 | HG | CYS | 643 | 23.042 | 59.851 | 9.179 |
| 284 | N | MET | 646 | 22.352 | 53.505 | 13.602 |
| 285 | CA | MET | 646 | 20.895 | 53.587 | 13.333 |
| 286 | CB | MET | 646 | 20.467 | 55.015 | 12.990 |
| 287 | CG | MET | 646 | 20.910 | 56.120 | 13.959 |
| 288 | SD | MET | 646 | 20.663 | 57.723 | 13.144 |
| 289 | CE | MET | 646 | 21.900 | 58.682 | 14.059 |
| 290 | C | MET | 646 | 20.579 | 52.798 | 12.031 |
| 291 | O | MET | 646 | 19.508 | 52.233 | 11.852 |
| 292 | HN | MET | 646 | 22.870 | 54.262 | 13.186 |
| 293 | N | VAL | 729 | 22.065 | 52.181 | 21.522 |
| 294 | CA | VAL | 729 | 21.631 | 53.332 | 22.332 |
| 295 | CB | VAL | 729 | 20.716 | 52.951 | 23.514 |
| 296 | CG1 | VAL | 729 | 20.710 | 53.987 | 24.657 |
| 297 | CG2 | VAL | 729 | 19.304 | 52.701 | 22.983 |
| 298 | C | VAL | 729 | 22.872 | 54.072 | 22.877 |
| 299 | O | VAL | 729 | 22.863 | 55.289 | 22.841 |
| 300 | HN | VAL | 729 | 21.984 | 51.226 | 21.920 |
| 301 | N | ASN | 731 | 26.329 | 54.014 | 21.825 |
| 302 | CA | ASN | 731 | 27.138 | 54.820 | 20.870 |
| 303 | CB | ASN | 731 | 27.387 | 54.229 | 19.463 |
| 304 | CG | ASN | 731 | 28.845 | 53.776 | 19.317 |
| 305 | OD1 | ASN | 731 | 29.653 | 54.382 | 18.627 |
| 306 | ND2 | ASN | 731 | 29.150 | 52.557 | 19.959 |
| 307 | C | ASN | 731 | 26.443 | 56.202 | 20.692 |
| 308 | O | ASN | 731 | 27.108 | 57.230 | 20.731 |
| 309 | HN | ASN | 731 | 26.219 | 53.014 | 21.649 |
| 310 | 1HD2 | ASN | 731 | 30.063 | 52.119 | 20.012 |
| 311 | 2HD2 | ASN | 731 | 28.472 | 52.206 | 20.626 |
| 312 | N | LEU | 732 | 25.048 | 56.174 | 20.372 |
| 313 | CA | LEU | 732 | 24.290 | 57.406 | 20.172 |
| 314 | CB | LEU | 732 | 22.800 | 57.211 | 19.824 |
| 315 | CG | LEU | 732 | 22.433 | 56.300 | 18.630 |
| 316 | CD1 | LEU | 732 | 20.909 | 56.239 | 18.525 |
| 317 | CD2 | LEU | 732 | 23.040 | 56.667 | 17.267 |
| 318 | C | LEU | 732 | 24.382 | 58.269 | 21.468 |
| 319 | O | LEU | 732 | 24.564 | 59.463 | 21.309 |
| 320 | HN | LEU | 732 | 24.504 | 55.309 | 20.391 |

| | | | | | | |
|---|---|---|---|---|---|---|
| 321 | N | LEU | 733 | 24.161 | 57.703 | 22.765 |
| 322 | CA | LEU | 733 | 24.202 | 58.620 | 23.921 |
| 323 | CB | LEU | 733 | 24.049 | 57.968 | 25.301 |
| 324 | CG | LEU | 733 | 22.612 | 57.462 | 25.553 |
| 325 | CD1 | LEU | 733 | 22.698 | 56.176 | 26.382 |
| 326 | CD2 | LEU | 733 | 21.724 | 58.557 | 26.193 |
| 327 | C | LEU | 733 | 25.552 | 59.366 | 23.917 |
| 328 | O | LEU | 733 | 25.570 | 60.542 | 24.226 |
| 329 | HN | LEU | 733 | 24.452 | 56.716 | 22.873 |
| 330 | N | ASN | 734 | 26.726 | 58.640 | 23.601 |
| 331 | CA | ASN | 734 | 28.055 | 59.253 | 23.617 |
| 332 | CB | ASN | 734 | 29.217 | 58.329 | 23.269 |
| 333 | CG | ASN | 734 | 29.132 | 57.030 | 24.049 |
| 334 | OD1 | ASN | 734 | 28.749 | 56.886 | 25.196 |
| 335 | ND2 | ASN | 734 | 29.613 | 55.946 | 23.311 |
| 336 | C | ASN | 734 | 28.100 | 60.449 | 22.627 |
| 337 | O | ASN | 734 | 28.481 | 61.503 | 23.108 |
| 338 | HN | ASN | 734 | 26.659 | 57.646 | 23.351 |
| 339 | 1HD2 | ASN | 734 | 29.561 | 55.063 | 23.808 |
| 340 | 2HD2 | ASN | 734 | 29.782 | 55.936 | 22.305 |
| 341 | N | TYR | 735 | 27.804 | 60.285 | 21.227 |
| 342 | CA | TYR | 735 | 28.040 | 61.488 | 20.385 |
| 343 | CB | TYR | 735 | 27.995 | 61.224 | 18.877 |
| 344 | CG | TYR | 735 | 28.410 | 62.390 | 17.981 |
| 345 | CD1 | TYR | 735 | 27.587 | 62.809 | 16.877 |
| 346 | CE1 | TYR | 735 | 28.050 | 63.814 | 15.945 |
| 347 | CD2 | TYR | 735 | 29.695 | 63.022 | 18.119 |
| 348 | CE2 | TYR | 735 | 30.157 | 64.025 | 17.194 |
| 349 | CZ | TYR | 735 | 29.347 | 64.433 | 16.073 |
| 350 | OH | TYR | 735 | 29.775 | 65.405 | 15.187 |
| 351 | C | TYR | 735 | 27.034 | 62.613 | 20.790 |
| 352 | O | TYR | 735 | 27.369 | 63.784 | 20.800 |
| 353 | HN | TYR | 735 | 27.340 | 59.452 | 20.845 |
| 354 | HH | TYR | 735 | 30.552 | 65.841 | 15.572 |
| 355 | N | CYS | 736 | 25.709 | 62.223 | 21.091 |
| 356 | CA | CYS | 736 | 24.669 | 63.236 | 21.406 |
| 357 | CB | CYS | 736 | 23.284 | 62.741 | 21.834 |
| 358 | SG | CYS | 736 | 22.170 | 64.172 | 22.068 |
| 359 | C | CYS | 736 | 25.148 | 64.111 | 22.584 |
| 360 | O | CYS | 736 | 25.085 | 65.331 | 22.572 |
| 361 | HN | CYS | 736 | 25.569 | 61.233 | 21.285 |
| 362 | HG | CYS | 736 | 21.724 | 63.818 | 23.261 |
| 363 | N | PHE | 737 | 25.582 | 63.357 | 23.708 |
| 364 | CA | PHE | 737 | 26.089 | 64.021 | 24.917 |
| 365 | CB | PHE | 737 | 26.664 | 63.063 | 25.983 |
| 366 | CG | PHE | 737 | 25.636 | 62.240 | 26.724 |
| 367 | CD1 | PHE | 737 | 24.221 | 62.550 | 26.756 |
| 368 | CD2 | PHE | 737 | 26.128 | 61.152 | 27.526 |
| 369 | CE1 | PHE | 737 | 23.340 | 61.790 | 27.593 |
| 370 | CE2 | PHE | 737 | 25.242 | 60.365 | 28.321 |
| 371 | CZ | PHE | 737 | 23.847 | 60.690 | 28.361 |
| 372 | C | PHE | 737 | 27.229 | 65.005 | 24.499 |
| 373 | O | PHE | 737 | 27.067 | 66.187 | 24.739 |
| 374 | HN | PHE | 737 | 25.846 | 62.376 | 23.585 |
| 375 | N | THR | 739 | 28.681 | 66.189 | 21.280 |

| | | | | | | |
|---|---|---|---|---|---|---|
| 376 | CA | THR | 739 | 28.309 | 67.384 | 20.463 |
| 377 | CB | THR | 739 | 27.440 | 67.126 | 19.217 |
| 378 | OG1 | THR | 739 | 26.320 | 66.342 | 19.589 |
| 379 | CG2 | THR | 739 | 28.179 | 66.402 | 18.097 |
| 380 | C | THR | 739 | 27.566 | 68.474 | 21.305 |
| 381 | O | THR | 739 | 27.905 | 69.636 | 21.151 |
| 382 | HN | THR | 739 | 28.581 | 65.281 | 20.851 |
| 383 | HG1 | THR | 739 | 26.729 | 65.628 | 20.104 |
| 384 | N | PHE | 740 | 26.513 | 68.090 | 22.188 |
| 385 | CA | PHE | 740 | 25.779 | 69.026 | 23.045 |
| 386 | CB | PHE | 740 | 24.652 | 68.371 | 23.870 |
| 387 | CG | PHE | 740 | 24.048 | 69.312 | 24.883 |
| 388 | CD1 | PHE | 740 | 23.350 | 70.515 | 24.507 |
| 389 | CD2 | PHE | 740 | 24.123 | 68.970 | 26.273 |
| 390 | CE1 | PHE | 740 | 22.645 | 71.311 | 25.474 |
| 391 | CE2 | PHE | 740 | 23.445 | 69.769 | 27.246 |
| 392 | CZ | PHE | 740 | 22.678 | 70.918 | 26.852 |
| 393 | C | PHE | 740 | 26.785 | 69.777 | 23.972 |
| 394 | O | PHE | 740 | 26.564 | 70.958 | 24.193 |
| 395 | HN | PHE | 740 | 26.497 | 67.112 | 22.462 |
| 396 | N | ILE | 747 | 25.929 | 73.116 | 17.326 |
| 397 | CA | ILE | 747 | 24.692 | 72.401 | 16.976 |
| 398 | CB | ILE | 747 | 24.972 | 70.887 | 16.734 |
| 399 | CG2 | ILE | 747 | 23.710 | 70.032 | 16.954 |
| 400 | CG1 | ILE | 747 | 25.544 | 70.693 | 15.298 |
| 401 | CD1 | ILE | 747 | 26.024 | 69.275 | 14.951 |
| 402 | C | ILE | 747 | 23.693 | 72.719 | 18.146 |
| 403 | O | ILE | 747 | 24.061 | 72.665 | 19.315 |
| 404 | HN | ILE | 747 | 26.567 | 72.631 | 17.950 |
| 405 | N | PHE | 749 | 20.020 | 71.968 | 20.436 |
| 406 | CA | PHE | 749 | 19.113 | 70.994 | 21.038 |
| 407 | CB | PHE | 749 | 19.825 | 70.178 | 22.170 |
| 408 | CG | PHE | 749 | 21.123 | 69.551 | 21.674 |
| 409 | CD1 | PHE | 749 | 21.249 | 68.133 | 21.434 |
| 410 | CD2 | PHE | 749 | 22.271 | 70.378 | 21.389 |
| 411 | CE1 | PHE | 749 | 22.491 | 67.570 | 20.946 |
| 412 | CE2 | PHE | 749 | 23.466 | 69.834 | 20.808 |
| 413 | CZ | PHE | 749 | 23.576 | 68.425 | 20.575 |
| 414 | C | PHE | 749 | 17.922 | 71.872 | 21.559 |
| 415 | O | PHE | 749 | 18.158 | 72.657 | 22.462 |
| 416 | HN | PHE | 749 | 20.737 | 72.269 | 21.075 |
| 417 | N | PRO | 750 | 16.607 | 71.806 | 20.962 |
| 418 | CD | PRO | 750 | 16.137 | 70.841 | 19.982 |
| 419 | CA | PRO | 750 | 15.444 | 72.491 | 21.552 |
| 420 | CB | PRO | 750 | 14.275 | 72.210 | 20.593 |
| 421 | CG | PRO | 750 | 14.608 | 70.797 | 20.114 |
| 422 | C | PRO | 750 | 15.157 | 71.868 | 22.959 |
| 423 | O | PRO | 750 | 15.795 | 70.949 | 23.448 |
| 424 | N | LEU | 753 | 14.849 | 67.699 | 24.405 |
| 425 | CA | LEU | 753 | 16.084 | 66.906 | 24.480 |
| 426 | CB | LEU | 753 | 16.673 | 66.578 | 23.107 |
| 427 | CG | LEU | 753 | 16.010 | 65.374 | 22.410 |
| 428 | CD1 | LEU | 753 | 14.477 | 65.428 | 22.363 |
| 429 | CD2 | LEU | 753 | 16.598 | 65.289 | 20.994 |
| 430 | C | LEU | 753 | 17.160 | 67.649 | 25.276 |

| | | | | | | |
|---|---|---|---|---|---|---|
| 431 | O | LEU | 753 | 18.007 | 66.994 | 25.852 |
| 432 | HN | LEU | 753 | 14.524 | 68.016 | 23.508 |
| 433 | N | ILE | 757 | 18.993 | 65.466 | 27.892 |
| 434 | CA | ILE | 757 | 20.424 | 65.244 | 27.718 |
| 435 | CB | ILE | 757 | 20.977 | 66.101 | 26.557 |
| 436 | CG2 | ILE | 757 | 22.499 | 66.188 | 26.626 |
| 437 | CG1 | ILE | 757 | 20.534 | 65.490 | 25.217 |
| 438 | CD1 | ILE | 757 | 20.687 | 66.440 | 24.033 |
| 439 | C | ILE | 757 | 21.047 | 65.623 | 29.086 |
| 440 | O | ILE | 757 | 21.490 | 64.780 | 29.839 |
| 441 | HN | ILE | 757 | 18.565 | 66.197 | 27.326 |
| 442 | C1 | SCH | 1 | 17.190 | 58.143 | 15.541 |
| 443 | C2 | SCH | 1 | 16.015 | 59.057 | 15.282 |
| 444 | N3 | SCH | 1 | 14.994 | 58.476 | 14.773 |
| 445 | O4 | SCH | 1 | 13.834 | 59.242 | 14.487 |
| 446 | C5 | SCH | 1 | 13.788 | 60.544 | 14.792 |
| 447 | O6 | SCH | 1 | 12.758 | 61.154 | 14.555 |
| 448 | C7 | SCH | 1 | 14.949 | 61.278 | 15.410 |
| 449 | C8 | SCH | 1 | 14.902 | 62.627 | 15.770 |
| 450 | C9 | SCH | 1 | 16.030 | 63.231 | 16.334 |
| 451 | C10 | SCH | 1 | 17.235 | 62.523 | 16.444 |
| 452 | N11 | SCH | 1 | 18.388 | 63.110 | 16.871 |
| 453 | 1HN1 | SCH | 1 | 18.712 | 63.960 | 16.387 |
| 454 | C12 | SCH | 1 | 19.107 | 62.649 | 17.866 |
| 455 | C13 | SCH | 1 | 20.354 | 63.413 | 18.299 |
| 456 | O14 | SCH | 1 | 20.934 | 64.111 | 17.181 |
| 457 | C15 | SCH | 1 | 21.410 | 62.438 | 18.917 |
| 458 | C16 | SCH | 1 | 22.124 | 61.455 | 17.927 |
| 459 | C17 | SCH | 1 | 21.132 | 60.304 | 17.566 |
| 460 | C18 | SCH | 1 | 23.317 | 60.761 | 18.661 |
| 461 | C19 | SCH | 1 | 22.706 | 62.192 | 16.676 |
| 462 | C20 | SCH | 1 | 22.302 | 61.853 | 15.372 |
| 463 | C21 | SCH | 1 | 22.877 | 62.439 | 14.242 |
| 464 | F22 | SCH | 1 | 22.460 | 62.092 | 13.025 |
| 465 | C23 | SCH | 1 | 23.891 | 63.384 | 14.387 |
| 466 | C24 | SCH | 1 | 24.279 | 63.770 | 15.670 |
| 467 | C25 | SCH | 1 | 23.673 | 63.207 | 16.803 |
| 468 | O26 | SCH | 1 | 24.040 | 63.668 | 18.070 |
| 469 | C27 | SCH | 1 | 19.955 | 64.495 | 19.340 |
| 470 | F28 | SCH | 1 | 19.524 | 63.898 | 20.483 |
| 471 | F29 | SCH | 1 | 21.020 | 65.291 | 19.628 |
| 472 | F30 | SCH | 1 | 18.949 | 65.264 | 18.840 |
| 473 | O1 | SCH | 1 | 18.826 | 61.631 | 18.480 |
| 474 | C31 | SCH | 1 | 17.249 | 61.161 | 16.108 |
| 475 | C32 | SCH | 1 | 16.099 | 60.529 | 15.616 |
| 476 | H14 | SCH | 1 | 21.051 | 65.042 | 17.344 |
| 477 | H26 | SCH | 1 | 24.314 | 64.579 | 18.102 |

TABLE 7

SUBSET OF ATOMIC COORDINATES OF GRα IN COMPLEX WITH A-222977 OBTAINED FROM MODELING OF THE CRYSTAL STRUCTURE OF GRα IN COMPLEX WITH FP

| Atom Number | Atom Type | Residue Name | Residue Number | X Coordinate | Y Coordinate | Z Coordinate |
|---|---|---|---|---|---|---|
| 1 | N | LEU | 544 | 5.940 | 62.649 | 11.764 |
| 2 | CA | LEU | 544 | 5.974 | 64.102 | 11.777 |
| 3 | CB | LEU | 544 | 5.952 | 64.855 | 13.135 |
| 4 | CG | LEU | 544 | 6.701 | 64.350 | 14.406 |
| 5 | CD1 | LEU | 544 | 6.322 | 65.241 | 15.616 |
| 6 | CD2 | LEU | 544 | 6.415 | 62.880 | 14.786 |
| 7 | C | LEU | 544 | 7.164 | 64.437 | 10.826 |
| 8 | O | LEU | 544 | 8.239 | 63.851 | 10.879 |
| 9 | HN | LEU | 544 | 6.575 | 62.118 | 12.352 |
| 10 | N | ALA | 546 | 10.133 | 67.032 | 9.049 |
| 11 | CA | ALA | 546 | 11.077 | 67.973 | 9.665 |
| 12 | CB | ALA | 546 | 12.511 | 67.894 | 9.127 |
| 13 | C | ALA | 546 | 10.510 | 69.441 | 9.516 |
| 14 | O | ALA | 546 | 10.418 | 70.171 | 10.499 |
| 15 | HN | ALA | 546 | 10.336 | 66.691 | 8.101 |
| 16 | N | THR | 556 | 26.536 | 69.607 | 7.321 |
| 17 | CA | THR | 556 | 26.715 | 68.666 | 8.452 |
| 18 | CB | THR | 556 | 28.220 | 68.488 | 8.917 |
| 19 | OG1 | THR | 556 | 29.146 | 68.162 | 7.870 |
| 20 | CG2 | THR | 556 | 28.374 | 67.408 | 10.003 |
| 21 | C | THR | 556 | 25.830 | 69.166 | 9.633 |
| 22 | O | THR | 556 | 24.941 | 68.451 | 10.058 |
| 23 | HN | THR | 556 | 27.377 | 69.610 | 6.760 |
| 24 | HG1 | THR | 556 | 30.034 | 68.403 | 8.201 |
| 25 | N | ILE | 559 | 21.645 | 70.029 | 8.828 |
| 26 | CA | ILE | 559 | 20.905 | 68.760 | 8.713 |
| 27 | CB | ILE | 559 | 21.548 | 67.582 | 7.907 |
| 28 | CG2 | ILE | 559 | 20.807 | 66.252 | 8.203 |
| 29 | CG1 | ILE | 559 | 21.607 | 67.839 | 6.379 |
| 30 | CD1 | ILE | 559 | 22.044 | 66.636 | 5.512 |
| 31 | C | ILE | 559 | 20.752 | 68.306 | 10.169 |
| 32 | O | ILE | 559 | 19.621 | 68.351 | 10.607 |
| 33 | HN | ILE | 559 | 22.571 | 69.965 | 8.403 |
| 34 | N | MET | 560 | 21.905 | 67.857 | 10.889 |
| 35 | CA | MET | 560 | 21.941 | 67.369 | 12.260 |
| 36 | CB | MET | 560 | 23.351 | 67.439 | 12.875 |
| 37 | CG | MET | 560 | 24.324 | 66.388 | 12.320 |
| 38 | SD | MET | 560 | 26.019 | 66.718 | 12.895 |
| 39 | CE | MET | 560 | 26.828 | 65.234 | 12.229 |
| 40 | C | MET | 560 | 21.018 | 68.237 | 13.150 |
| 41 | O | MET | 560 | 20.533 | 67.696 | 14.121 |
| 42 | HN | MET | 560 | 22.823 | 67.966 | 10.473 |
| 43 | N | THR | 561 | 20.851 | 69.631 | 12.866 |
| 44 | CA | THR | 561 | 20.194 | 70.586 | 13.815 |
| 45 | CB | THR | 561 | 20.656 | 72.069 | 13.721 |

| | | | | | | |
|---|---|---|---|---|---|---|
| 46 | OG1 | THR | 561 | 21.982 | 72.165 | 14.217 |
| 47 | CG2 | THR | 561 | 19.786 | 73.052 | 14.525 |
| 48 | C | THR | 561 | 18.619 | 70.520 | 13.680 |
| 49 | O | THR | 561 | 17.937 | 70.552 | 14.690 |
| 50 | HN | THR | 561 | 21.449 | 70.022 | 12.137 |
| 51 | HG1 | THR | 561 | 22.203 | 73.109 | 14.307 |
| 52 | N | THR | 562 | 18.041 | 70.553 | 12.371 |
| 53 | CA | THR | 562 | 16.553 | 70.280 | 12.158 |
| 54 | CB | THR | 562 | 15.755 | 70.434 | 10.825 |
| 55 | OG1 | THR | 562 | 16.062 | 69.374 | 9.913 |
| 56 | CG2 | THR | 562 | 15.809 | 71.834 | 10.188 |
| 57 | C | THR | 562 | 16.262 | 68.792 | 12.499 |
| 58 | O | THR | 562 | 15.144 | 68.430 | 12.862 |
| 59 | HN | THR | 562 | 18.664 | 70.520 | 11.569 |
| 60 | HG1 | THR | 562 | 15.225 | 69.107 | 9.479 |
| 61 | N | LEU | 563 | 17.370 | 67.906 | 12.333 |
| 62 | CA | LEU | 563 | 17.285 | 66.577 | 12.933 |
| 63 | CB | LEU | 563 | 18.420 | 65.579 | 12.652 |
| 64 | CG | LEU | 563 | 18.147 | 64.632 | 11.465 |
| 65 | CD1 | LEU | 563 | 17.912 | 65.376 | 10.149 |
| 66 | CD2 | LEU | 563 | 19.291 | 63.626 | 11.351 |
| 67 | C | LEU | 563 | 17.087 | 66.791 | 14.472 |
| 68 | O | LEU | 563 | 16.099 | 66.310 | 14.993 |
| 69 | HN | LEU | 563 | 18.282 | 68.220 | 12.006 |
| 70 | N | ASN | 564 | 17.998 | 67.561 | 15.243 |
| 71 | CA | ASN | 564 | 17.762 | 67.781 | 16.699 |
| 72 | CB | ASN | 564 | 18.569 | 68.843 | 17.472 |
| 73 | CG | ASN | 564 | 20.056 | 68.607 | 17.508 |
| 74 | OD1 | ASN | 564 | 20.629 | 67.652 | 17.018 |
| 75 | ND2 | ASN | 564 | 20.778 | 69.585 | 18.188 |
| 76 | C | ASN | 564 | 16.319 | 68.294 | 16.953 |
| 77 | O | ASN | 564 | 15.795 | 68.053 | 18.031 |
| 78 | HN | ASN | 564 | 18.920 | 67.829 | 14.913 |
| 79 | 1HD2 | ASN | 564 | 21.765 | 69.634 | 17.976 |
| 80 | 2HD2 | ASN | 564 | 20.395 | 70.383 | 18.697 |
| 81 | N | MET | 565 | 15.684 | 69.107 | 15.971 |
| 82 | CA | MET | 565 | 14.312 | 69.583 | 16.188 |
| 83 | CB | MET | 565 | 13.648 | 70.584 | 15.211 |
| 84 | CG | MET | 565 | 13.656 | 72.043 | 15.707 |
| 85 | SD | MET | 565 | 15.315 | 72.785 | 15.771 |
| 86 | CE | MET | 565 | 14.869 | 74.511 | 16.170 |
| 87 | C | MET | 565 | 13.443 | 68.305 | 16.265 |
| 88 | O | MET | 565 | 12.678 | 68.164 | 17.205 |
| 89 | HN | MET | 565 | 16.160 | 69.310 | 15.099 |
| 90 | N | LEU | 566 | 13.547 | 67.404 | 15.171 |
| 91 | CA | LEU | 566 | 12.768 | 66.143 | 15.153 |
| 92 | CB | LEU | 566 | 12.945 | 65.257 | 13.893 |
| 93 | CG | LEU | 566 | 11.822 | 64.204 | 13.676 |
| 94 | CD1 | LEU | 566 | 10.450 | 64.837 | 13.381 |
| 95 | CD2 | LEU | 566 | 12.147 | 63.224 | 12.538 |
| 96 | C | LEU | 566 | 13.005 | 65.328 | 16.485 |
| 97 | O | LEU | 566 | 12.081 | 64.676 | 16.948 |
| 98 | HN | LEU | 566 | 14.426 | 67.455 | 14.649 |
| 99 | N | GLY | 567 | 14.263 | 65.502 | 17.171 |
| 100 | CA | GLY | 567 | 14.521 | 65.160 | 18.589 |

| | | | | | | |
|---|---|---|---|---|---|---|
| 101 | C | GLY | 567 | 13.465 | 65.833 | 19.538 |
| 102 | O | GLY | 567 | 12.547 | 65.132 | 19.919 |
| 103 | HN | GLY | 567 | 15.052 | 66.005 | 16.742 |
| 104 | N | GLY | 568 | 13.544 | 67.218 | 19.887 |
| 105 | CA | GLY | 568 | 12.411 | 67.926 | 20.537 |
| 106 | C | GLY | 568 | 10.982 | 67.303 | 20.254 |
| 107 | O | GLY | 568 | 10.368 | 66.848 | 21.205 |
| 108 | HN | GLY | 568 | 14.433 | 67.732 | 19.807 |
| 109 | N | GLN | 570 | 9.428 | 64.685 | 18.396 |
| 110 | CA | GLN | 570 | 9.049 | 63.281 | 18.604 |
| 111 | CB | GLN | 570 | 9.956 | 62.258 | 17.949 |
| 112 | CG | GLN | 570 | 9.753 | 61.961 | 16.450 |
| 113 | CD | GLN | 570 | 10.308 | 60.556 | 16.193 |
| 114 | OE1 | GLN | 570 | 9.806 | 59.796 | 15.383 |
| 115 | NE2 | GLN | 570 | 11.431 | 60.213 | 17.001 |
| 116 | C | GLN | 570 | 9.137 | 62.898 | 20.110 |
| 117 | O | GLN | 570 | 8.332 | 62.097 | 20.551 |
| 118 | HN | GLN | 570 | 10.392 | 64.875 | 18.141 |
| 119 | 1HE2 | GLN | 570 | 11.718 | 59.230 | 17.040 |
| 120 | 2HE2 | GLN | 570 | 11.633 | 60.875 | 17.778 |
| 121 | N | VAL | 571 | 10.216 | 63.398 | 20.902 |
| 122 | CA | VAL | 571 | 10.426 | 62.911 | 22.293 |
| 123 | CB | VAL | 571 | 11.858 | 63.181 | 22.856 |
| 124 | CG1 | VAL | 571 | 12.086 | 62.848 | 24.356 |
| 125 | CG2 | VAL | 571 | 12.908 | 62.362 | 22.069 |
| 126 | C | VAL | 571 | 9.247 | 63.480 | 23.196 |
| 127 | O | VAL | 571 | 9.117 | 63.034 | 24.321 |
| 128 | HN | VAL | 571 | 10.991 | 63.862 | 20.446 |
| 129 | N | TRP | 600 | 14.561 | 57.460 | 26.114 |
| 130 | CA | TRP | 600 | 14.324 | 58.379 | 25.003 |
| 131 | CB | TRP | 600 | 14.614 | 59.891 | 25.199 |
| 132 | CG | TRP | 600 | 16.028 | 60.382 | 25.501 |
| 133 | CD2 | TRP | 600 | 16.930 | 61.120 | 24.578 |
| 134 | CE2 | TRP | 600 | 18.065 | 61.447 | 25.325 |
| 135 | CE3 | TRP | 600 | 16.817 | 61.490 | 23.221 |
| 136 | CD1 | TRP | 600 | 16.747 | 60.323 | 26.661 |
| 137 | NE1 | TRP | 600 | 18.055 | 60.884 | 26.595 |
| 138 | CZ2 | TRP | 600 | 19.117 | 62.236 | 24.814 |
| 139 | CZ3 | TRP | 600 | 17.893 | 62.235 | 22.669 |
| 140 | CH2 | TRP | 600 | 19.012 | 62.627 | 23.455 |
| 141 | C | TRP | 600 | 15.100 | 57.770 | 23.809 |
| 142 | O | TRP | 600 | 14.466 | 57.424 | 22.825 |
| 143 | HN | TRP | 600 | 14.787 | 57.825 | 27.028 |
| 144 | HE1 | TRP | 600 | 18.880 | 60.300 | 26.729 |
| 145 | N | MET | 601 | 16.514 | 57.610 | 23.896 |
| 146 | CA | MET | 601 | 17.323 | 57.405 | 22.681 |
| 147 | CB | MET | 601 | 18.834 | 57.412 | 22.988 |
| 148 | CG | MET | 601 | 19.686 | 57.683 | 21.742 |
| 149 | SD | MET | 601 | 19.546 | 59.415 | 21.169 |
| 150 | CE | MET | 601 | 20.901 | 60.143 | 22.156 |
| 151 | C | MET | 601 | 16.967 | 56.077 | 21.930 |
| 152 | O | MET | 601 | 17.102 | 56.001 | 20.724 |
| 153 | HN | MET | 601 | 16.971 | 58.124 | 24.636 |
| 154 | N | LEU | 603 | 13.772 | 54.432 | 22.178 |
| 155 | CA | LEU | 603 | 12.454 | 54.451 | 21.540 |

| | | | | | | |
|---|---|---|---|---|---|---|
| 156 | CB | LEU | 603 | 11.434 | 55.244 | 22.366 |
| 157 | CG | LEU | 603 | 11.150 | 54.507 | 23.695 |
| 158 | CD1 | LEU | 603 | 10.728 | 55.452 | 24.824 |
| 159 | CD2 | LEU | 603 | 10.128 | 53.394 | 23.427 |
| 160 | C | LEU | 603 | 12.673 | 55.106 | 20.169 |
| 161 | O | LEU | 603 | 12.125 | 54.614 | 19.199 |
| 162 | HN | LEU | 603 | 13.876 | 54.975 | 23.034 |
| 163 | N | MET | 604 | 13.566 | 56.217 | 20.107 |
| 164 | CA | MET | 604 | 13.981 | 56.821 | 18.842 |
| 165 | CB | MET | 604 | 14.970 | 58.010 | 18.972 |
| 166 | CG | MET | 604 | 14.581 | 59.213 | 19.859 |
| 167 | SD | MET | 604 | 13.063 | 60.070 | 19.348 |
| 168 | CE | MET | 604 | 11.823 | 59.245 | 20.415 |
| 169 | C | MET | 604 | 14.636 | 55.715 | 17.935 |
| 170 | O | MET | 604 | 14.173 | 55.510 | 16.829 |
| 171 | HN | MET | 604 | 13.925 | 56.631 | 20.966 |
| 172 | N | ALA | 605 | 15.861 | 55.133 | 18.370 |
| 173 | CA | ALA | 605 | 16.781 | 54.474 | 17.414 |
| 174 | CB | ALA | 605 | 18.140 | 54.054 | 18.003 |
| 175 | C | ALA | 605 | 16.114 | 53.230 | 16.751 |
| 176 | O | ALA | 605 | 16.374 | 52.946 | 15.589 |
| 177 | HN | ALA | 605 | 16.240 | 55.310 | 19.293 |
| 178 | N | PHE | 606 | 15.195 | 52.543 | 17.598 |
| 179 | CA | PHE | 606 | 14.513 | 51.298 | 17.216 |
| 180 | CB | PHE | 606 | 13.747 | 50.755 | 18.425 |
| 181 | CG | PHE | 606 | 13.304 | 49.339 | 18.170 |
| 182 | CD1 | PHE | 606 | 14.259 | 48.269 | 18.295 |
| 183 | CD2 | PHE | 606 | 11.931 | 49.029 | 17.874 |
| 184 | CE1 | PHE | 606 | 13.870 | 46.921 | 18.067 |
| 185 | CE2 | PHE | 606 | 11.543 | 47.672 | 17.614 |
| 186 | CZ | PHE | 606 | 12.529 | 46.627 | 17.662 |
| 187 | C | PHE | 606 | 13.506 | 51.591 | 16.073 |
| 188 | O | PHE | 606 | 13.333 | 50.781 | 15.180 |
| 189 | HN | PHE | 606 | 15.064 | 52.882 | 18.553 |
| 190 | N | ALA | 607 | 12.820 | 52.837 | 16.170 |
| 191 | CA | ALA | 607 | 11.742 | 53.395 | 15.301 |
| 192 | CB | ALA | 607 | 10.836 | 54.405 | 16.040 |
| 193 | C | ALA | 607 | 12.289 | 54.110 | 13.992 |
| 194 | O | ALA | 607 | 11.664 | 54.064 | 12.939 |
| 195 | HN | ALA | 607 | 13.125 | 53.441 | 16.939 |
| 196 | N | LEU | 608 | 13.478 | 54.884 | 14.096 |
| 197 | CA | LEU | 608 | 14.239 | 55.248 | 12.891 |
| 198 | CB | LEU | 608 | 15.611 | 55.891 | 13.143 |
| 199 | CG | LEU | 608 | 16.409 | 56.104 | 11.838 |
| 200 | CD1 | LEU | 608 | 15.637 | 57.041 | 10.912 |
| 201 | CD2 | LEU | 608 | 17.811 | 56.651 | 12.114 |
| 202 | C | LEU | 608 | 14.475 | 53.960 | 12.058 |
| 203 | O | LEU | 608 | 14.118 | 53.927 | 10.891 |
| 204 | HN | LEU | 608 | 14.044 | 54.730 | 14.929 |
| 205 | N | GLY | 609 | 15.072 | 52.871 | 12.762 |
| 206 | CA | GLY | 609 | 15.130 | 51.566 | 12.135 |
| 207 | C | GLY | 609 | 13.878 | 51.185 | 11.368 |
| 208 | O | GLY | 609 | 13.970 | 50.700 | 10.245 |
| 209 | HN | GLY | 609 | 15.265 | 52.896 | 13.770 |
| 210 | N | ARG | 611 | 10.857 | 53.193 | 10.139 |

| | | | | | | |
|---|---|---|---|---|---|---|
| 211 | CA | ARG | 611 | 10.355 | 53.632 | 8.752 |
| 212 | CB | ARG | 611 | 9.843 | 54.982 | 8.210 |
| 213 | CG | ARG | 611 | 8.689 | 55.607 | 8.985 |
| 214 | CD | ARG | 611 | 9.225 | 56.890 | 9.590 |
| 215 | NE | ARG | 611 | 10.412 | 56.723 | 10.461 |
| 216 | CZ | ARG | 611 | 10.551 | 57.579 | 11.562 |
| 217 | NH1 | ARG | 611 | 9.672 | 58.613 | 11.781 |
| 218 | NH2 | ARG | 611 | 11.542 | 57.447 | 12.517 |
| 219 | C | ARG | 611 | 11.489 | 53.310 | 7.755 |
| 220 | O | ARG | 611 | 11.221 | 53.115 | 6.580 |
| 221 | HN | ARG | 611 | 11.052 | 53.878 | 10.873 |
| 222 | HE | ARG | 611 | 11.054 | 55.975 | 10.220 |
| 223 | 1HH1 | ARG | 611 | 9.774 | 59.243 | 12.566 |
| 224 | 2HH1 | ARG | 611 | 8.902 | 58.754 | 11.149 |
| 225 | 1HH2 | ARG | 611 | 11.573 | 58.106 | 13.291 |
| 226 | 2HH2 | ARG | 611 | 12.233 | 56.718 | 12.509 |
| 227 | N | LEU | 621 | 18.340 | 56.346 | 5.666 |
| 228 | CA | LEU | 621 | 17.897 | 56.453 | 7.068 |
| 229 | CB | LEU | 621 | 18.926 | 57.115 | 8.017 |
| 230 | CG | LEU | 621 | 20.170 | 56.278 | 8.350 |
| 231 | CD1 | LEU | 621 | 21.020 | 57.019 | 9.409 |
| 232 | CD2 | LEU | 621 | 19.779 | 54.857 | 8.782 |
| 233 | C | LEU | 621 | 16.632 | 57.319 | 6.960 |
| 234 | O | LEU | 621 | 16.849 | 58.518 | 6.998 |
| 235 | HN | LEU | 621 | 19.245 | 56.741 | 5.477 |
| 236 | N | CYS | 622 | 15.306 | 56.815 | 6.729 |
| 237 | CA | CYS | 622 | 14.234 | 57.828 | 6.639 |
| 238 | CB | CYS | 622 | 12.926 | 57.233 | 6.114 |
| 239 | SG | CYS | 622 | 13.160 | 56.480 | 4.471 |
| 240 | C | CYS | 622 | 14.056 | 58.457 | 8.070 |
| 241 | O | CYS | 622 | 13.430 | 57.849 | 8.930 |
| 242 | HN | CYS | 622 | 15.072 | 55.855 | 7.001 |
| 243 | HG | CYS | 622 | 13.936 | 55.462 | 4.807 |
| 244 | N | PHE | 623 | 14.601 | 59.763 | 8.308 |
| 245 | CA | PHE | 623 | 14.425 | 60.309 | 9.705 |
| 246 | CB | PHE | 623 | 15.244 | 61.569 | 10.081 |
| 247 | CG | PHE | 623 | 16.556 | 61.042 | 10.590 |
| 248 | CD1 | PHE | 623 | 17.579 | 60.635 | 9.669 |
| 249 | CD2 | PHE | 623 | 16.726 | 60.787 | 11.999 |
| 250 | CE1 | PHE | 623 | 18.761 | 59.986 | 10.164 |
| 251 | CE2 | PHE | 623 | 17.921 | 60.170 | 12.497 |
| 252 | CZ | PHE | 623 | 18.933 | 59.751 | 11.571 |
| 253 | C | PHE | 623 | 12.921 | 60.569 | 10.011 |
| 254 | O | PHE | 623 | 12.429 | 60.437 | 11.124 |
| 255 | HN | PHE | 623 | 15.017 | 60.332 | 7.556 |
| 256 | N | ALA | 624 | 12.283 | 61.028 | 8.839 |
| 257 | CA | ALA | 624 | 11.034 | 61.723 | 8.528 |
| 258 | CB | ALA | 624 | 11.045 | 63.224 | 8.877 |
| 259 | C | ALA | 624 | 10.967 | 61.630 | 6.967 |
| 260 | O | ALA | 624 | 11.954 | 61.868 | 6.293 |
| 261 | HN | ALA | 624 | 12.896 | 60.941 | 8.021 |
| 262 | N | LEU | 627 | 12.747 | 64.097 | 4.793 |
| 263 | CA | LEU | 627 | 14.163 | 64.327 | 5.140 |
| 264 | CB | LEU | 627 | 14.482 | 65.265 | 6.339 |
| 265 | CG | LEU | 627 | 15.970 | 65.710 | 6.422 |

| | | | | | | |
|---|---|---|---|---|---|---|
| 266 | CD1 | LEU | 627 | 16.199 | 66.758 | 7.534 |
| 267 | CD2 | LEU | 627 | 16.940 | 64.529 | 6.608 |
| 268 | C | LEU | 627 | 14.760 | 62.885 | 5.331 |
| 269 | O | LEU | 627 | 14.829 | 62.358 | 6.436 |
| 270 | HN | LEU | 627 | 12.277 | 63.333 | 5.282 |
| 271 | N | ILE | 629 | 18.460 | 60.556 | 4.655 |
| 272 | CA | ILE | 629 | 19.905 | 60.734 | 4.555 |
| 273 | CB | ILE | 629 | 20.662 | 60.638 | 5.912 |
| 274 | CG2 | ILE | 629 | 22.181 | 60.686 | 5.662 |
| 275 | CG1 | ILE | 629 | 20.189 | 61.794 | 6.853 |
| 276 | CD1 | ILE | 629 | 21.052 | 62.074 | 8.102 |
| 277 | C | ILE | 629 | 20.244 | 59.674 | 3.469 |
| 278 | O | ILE | 629 | 20.464 | 58.500 | 3.734 |
| 279 | HN | ILE | 629 | 18.154 | 59.992 | 5.437 |
| 280 | N | MET | 634 | 24.928 | 61.075 | 3.277 |
| 281 | CA | MET | 634 | 25.781 | 60.213 | 4.131 |
| 282 | CB | MET | 634 | 25.575 | 58.727 | 3.734 |
| 283 | CG | MET | 634 | 26.417 | 57.674 | 4.474 |
| 284 | SD | MET | 634 | 26.018 | 57.458 | 6.251 |
| 285 | CE | MET | 634 | 25.117 | 55.867 | 6.189 |
| 286 | C | MET | 634 | 27.288 | 60.594 | 3.951 |
| 287 | O | MET | 634 | 28.087 | 60.349 | 4.850 |
| 288 | HN | MET | 634 | 24.727 | 60.641 | 2.372 |
| 289 | N | CYS | 638 | 31.098 | 64.594 | 9.077 |
| 290 | CA | CYS | 638 | 31.055 | 64.015 | 10.454 |
| 291 | CB | CYS | 638 | 31.239 | 65.080 | 11.558 |
| 292 | SG | CYS | 638 | 32.568 | 66.285 | 11.216 |
| 293 | C | CYS | 638 | 29.709 | 63.226 | 10.664 |
| 294 | O | CYS | 638 | 29.190 | 63.165 | 11.770 |
| 295 | HN | CYS | 638 | 30.527 | 65.420 | 8.911 |
| 296 | HG | CYS | 638 | 33.011 | 66.409 | 12.463 |
| 297 | N | MET | 639 | 29.177 | 62.603 | 9.493 |
| 298 | CA | MET | 639 | 28.118 | 61.606 | 9.391 |
| 299 | CB | MET | 639 | 27.276 | 61.590 | 8.083 |
| 300 | CG | MET | 639 | 25.994 | 62.438 | 8.084 |
| 301 | SD | MET | 639 | 26.258 | 64.229 | 8.311 |
| 302 | CE | MET | 639 | 24.506 | 64.724 | 8.423 |
| 303 | C | MET | 639 | 28.818 | 60.222 | 9.463 |
| 304 | O | MET | 639 | 28.321 | 59.438 | 10.253 |
| 305 | HN | MET | 639 | 29.536 | 62.905 | 8.595 |
| 306 | N | TYR | 640 | 29.848 | 59.854 | 8.495 |
| 307 | CA | TYR | 640 | 29.739 | 58.468 | 7.927 |
| 308 | CB | TYR | 640 | 30.816 | 57.958 | 6.919 |
| 309 | CG | TYR | 640 | 30.450 | 56.539 | 6.491 |
| 310 | CD1 | TYR | 640 | 29.652 | 56.268 | 5.315 |
| 311 | CE1 | TYR | 640 | 29.053 | 54.968 | 5.129 |
| 312 | CD2 | TYR | 640 | 30.769 | 55.432 | 7.359 |
| 313 | CE2 | TYR | 640 | 30.153 | 54.145 | 7.178 |
| 314 | CZ | TYR | 640 | 29.243 | 53.913 | 6.093 |
| 315 | OH | TYR | 640 | 28.569 | 52.720 | 5.978 |
| 316 | C | TYR | 640 | 29.564 | 57.430 | 9.097 |
| 317 | O | TYR | 640 | 28.562 | 56.733 | 9.138 |
| 318 | HN | TYR | 640 | 30.292 | 60.563 | 7.909 |
| 319 | HH | TYR | 640 | 28.269 | 52.670 | 5.058 |
| 320 | N | ASP | 641 | 30.620 | 57.298 | 10.037 |

| | | | | | | |
|---|---|---|---|---|---|---|
| 321 | CA | ASP | 641 | 30.649 | 56.562 | 11.304 |
| 322 | CB | ASP | 641 | 31.628 | 57.260 | 12.263 |
| 323 | CG | ASP | 641 | 33.078 | 56.758 | 12.235 |
| 324 | OD1 | ASP | 641 | 33.794 | 57.386 | 13.008 |
| 325 | OD2 | ASP | 641 | 33.377 | 55.816 | 11.498 |
| 326 | C | ASP | 641 | 29.326 | 56.543 | 12.107 |
| 327 | O | ASP | 641 | 28.909 | 55.513 | 12.616 |
| 328 | HN | ASP | 641 | 31.416 | 57.909 | 9.941 |
| 329 | N | GLN | 642 | 28.792 | 57.830 | 12.393 |
| 330 | CA | GLN | 642 | 27.838 | 58.002 | 13.496 |
| 331 | CB | GLN | 642 | 27.603 | 59.502 | 13.734 |
| 332 | CG | GLN | 642 | 28.908 | 60.138 | 14.274 |
| 333 | CD | GLN | 642 | 29.236 | 59.505 | 15.630 |
| 334 | OE1 | GLN | 642 | 28.374 | 59.494 | 16.485 |
| 335 | NE2 | GLN | 642 | 30.522 | 58.980 | 15.921 |
| 336 | C | GLN | 642 | 26.552 | 57.241 | 13.119 |
| 337 | O | GLN | 642 | 25.935 | 56.582 | 13.949 |
| 338 | HN | GLN | 642 | 29.140 | 58.691 | 11.949 |
| 339 | 1HE2 | GLN | 642 | 30.718 | 58.815 | 16.912 |
| 340 | 2HE2 | GLN | 642 | 31.248 | 59.012 | 15.207 |
| 341 | N | CYS | 643 | 26.169 | 57.411 | 11.744 |
| 342 | CA | CYS | 643 | 25.074 | 56.740 | 11.046 |
| 343 | CB | CYS | 643 | 24.892 | 57.322 | 9.616 |
| 344 | SG | CYS | 643 | 24.484 | 59.105 | 9.519 |
| 345 | C | CYS | 643 | 25.277 | 55.166 | 10.943 |
| 346 | O | CYS | 643 | 24.394 | 54.575 | 10.340 |
| 347 | HN | CYS | 643 | 26.908 | 57.738 | 11.111 |
| 348 | HG | CYS | 643 | 24.214 | 59.154 | 8.216 |
| 349 | N | LYS | 644 | 26.392 | 54.438 | 11.500 |
| 350 | CA | LYS | 644 | 26.484 | 52.967 | 11.428 |
| 351 | CB | LYS | 644 | 27.925 | 52.366 | 11.385 |
| 352 | CG | LYS | 644 | 28.724 | 52.240 | 12.710 |
| 353 | CD | LYS | 644 | 30.263 | 52.025 | 12.578 |
| 354 | CE | LYS | 644 | 31.028 | 53.247 | 12.010 |
| 355 | NZ | LYS | 644 | 32.537 | 53.112 | 11.853 |
| 356 | C | LYS | 644 | 25.566 | 52.392 | 12.568 |
| 357 | O | LYS | 644 | 24.972 | 51.347 | 12.357 |
| 358 | HN | LYS | 644 | 27.136 | 54.909 | 12.007 |
| 359 | HZ1 | LYS | 644 | 32.915 | 53.999 | 11.523 |
| 360 | HZ2 | LYS | 644 | 33.013 | 52.905 | 12.724 |
| 361 | HZ3 | LYS | 644 | 32.798 | 52.408 | 11.176 |
| 362 | N | HIS | 645 | 25.428 | 53.102 | 13.810 |
| 363 | CA | HIS | 645 | 24.598 | 52.550 | 14.900 |
| 364 | CB | HIS | 645 | 24.916 | 53.293 | 16.219 |
| 365 | CG | HIS | 645 | 26.374 | 52.978 | 16.418 |
| 366 | CD2 | HIS | 645 | 26.819 | 51.696 | 16.836 |
| 367 | ND1 | HIS | 645 | 27.439 | 53.703 | 15.978 |
| 368 | CE1 | HIS | 645 | 28.488 | 52.840 | 16.035 |
| 369 | NE2 | HIS | 645 | 28.175 | 51.559 | 16.562 |
| 370 | C | HIS | 645 | 23.061 | 52.510 | 14.519 |
| 371 | O | HIS | 645 | 22.320 | 51.794 | 15.168 |
| 372 | HN | HIS | 645 | 25.935 | 53.960 | 14.039 |
| 373 | HE2 | HIS | 645 | 28.735 | 50.741 | 16.753 |
| 374 | N | MET | 646 | 22.577 | 53.278 | 13.414 |
| 375 | CA | MET | 646 | 21.229 | 53.597 | 12.908 |

| | | | | | | |
|---|---|---|---|---|---|---|
| 376 | CB | MET | 646 | 21.067 | 55.106 | 12.659 |
| 377 | CG | MET | 646 | 20.921 | 55.934 | 13.936 |
| 378 | SD | MET | 646 | 19.699 | 55.213 | 15.093 |
| 379 | CE | MET | 646 | 18.744 | 56.709 | 15.497 |
| 380 | C | MET | 646 | 20.954 | 52.953 | 11.515 |
| 381 | O | MET | 646 | 19.819 | 52.553 | 11.295 |
| 382 | HN | MET | 646 | 23.266 | 53.804 | 12.902 |
| 383 | N | LEU | 647 | 22.000 | 52.868 | 10.535 |
| 384 | CA | LEU | 647 | 21.770 | 51.903 | 9.439 |
| 385 | CB | LEU | 647 | 22.923 | 51.593 | 8.448 |
| 386 | CG | LEU | 647 | 23.029 | 52.476 | 7.171 |
| 387 | CD1 | LEU | 647 | 24.271 | 52.062 | 6.344 |
| 388 | CD2 | LEU | 647 | 21.765 | 52.405 | 6.279 |
| 389 | C | LEU | 647 | 21.445 | 50.582 | 10.169 |
| 390 | O | LEU | 647 | 20.690 | 49.782 | 9.652 |
| 391 | HN | LEU | 647 | 22.934 | 53.259 | 10.626 |
| 392 | CD1 | TYR | 716 | 17.532 | 32.722 | 22.110 |
| 393 | N | VAL | 728 | 23.354 | 49.846 | 20.762 |
| 394 | CA | VAL | 728 | 23.256 | 50.881 | 19.702 |
| 395 | CB | VAL | 728 | 22.271 | 50.756 | 18.493 |
| 396 | CG1 | VAL | 728 | 21.851 | 49.313 | 18.125 |
| 397 | CG2 | VAL | 728 | 21.008 | 51.638 | 18.612 |
| 398 | C | VAL | 728 | 23.001 | 52.237 | 20.432 |
| 399 | O | VAL | 728 | 23.533 | 53.260 | 20.022 |
| 400 | HN | VAL | 728 | 22.601 | 49.222 | 21.012 |
| 401 | N | VAL | 729 | 22.065 | 52.181 | 21.522 |
| 402 | CA | VAL | 729 | 21.631 | 53.332 | 22.332 |
| 403 | CB | VAL | 729 | 20.716 | 52.951 | 23.514 |
| 404 | CG1 | VAL | 729 | 20.710 | 53.987 | 24.657 |
| 405 | CG2 | VAL | 729 | 19.304 | 52.701 | 22.983 |
| 406 | C | VAL | 729 | 22.872 | 54.072 | 22.877 |
| 407 | O | VAL | 729 | 22.863 | 55.289 | 22.841 |
| 408 | HN | VAL | 729 | 21.984 | 51.226 | 21.920 |
| 409 | N | ASN | 731 | 26.233 | 53.877 | 21.871 |
| 410 | CA | ASN | 731 | 27.126 | 54.475 | 20.877 |
| 411 | CB | ASN | 731 | 27.404 | 53.649 | 19.613 |
| 412 | CG | ASN | 731 | 28.901 | 53.366 | 19.468 |
| 413 | OD1 | ASN | 731 | 29.507 | 53.633 | 18.446 |
| 414 | ND2 | ASN | 731 | 29.519 | 52.667 | 20.519 |
| 415 | C | ASN | 731 | 26.551 | 55.844 | 20.464 |
| 416 | O | ASN | 731 | 27.349 | 56.736 | 20.226 |
| 417 | HN | ASN | 731 | 26.048 | 52.864 | 21.816 |
| 418 | 1HD2 | ASN | 731 | 30.318 | 52.062 | 20.364 |
| 419 | 2HD2 | ASN | 731 | 28.955 | 52.425 | 21.331 |
| 420 | N | LEU | 732 | 25.140 | 56.051 | 20.345 |
| 421 | CA | LEU | 732 | 24.539 | 57.365 | 20.106 |
| 422 | CB | LEU | 732 | 23.036 | 57.361 | 19.768 |
| 423 | CG | LEU | 732 | 22.579 | 56.463 | 18.601 |
| 424 | CD1 | LEU | 732 | 21.077 | 56.679 | 18.381 |
| 425 | CD2 | LEU | 732 | 23.394 | 56.658 | 17.311 |
| 426 | C | LEU | 732 | 24.681 | 58.243 | 21.385 |
| 427 | O | LEU | 732 | 25.033 | 59.396 | 21.210 |
| 428 | HN | LEU | 732 | 24.475 | 55.281 | 20.309 |
| 429 | N | LEU | 733 | 24.261 | 57.745 | 22.668 |
| 430 | CA | LEU | 733 | 24.302 | 58.675 | 23.822 |

| | | | | | | |
|---|---|---|---|---|---|---|
| 431 | CB | LEU | 733 | 24.099 | 58.051 | 25.213 |
| 432 | CG | LEU | 733 | 22.649 | 57.569 | 25.463 |
| 433 | CD1 | LEU | 733 | 22.699 | 56.282 | 26.294 |
| 434 | CD2 | LEU | 733 | 21.767 | 58.663 | 26.114 |
| 435 | C | LEU | 733 | 25.667 | 59.408 | 23.841 |
| 436 | O | LEU | 733 | 25.694 | 60.583 | 24.149 |
| 437 | HN | LEU | 733 | 24.463 | 56.738 | 22.837 |
| 438 | N | ASN | 734 | 26.833 | 58.668 | 23.526 |
| 439 | CA | ASN | 734 | 28.174 | 59.245 | 23.493 |
| 440 | CB | ASN | 734 | 29.260 | 58.246 | 23.120 |
| 441 | CG | ASN | 734 | 29.158 | 56.986 | 23.977 |
| 442 | OD1 | ASN | 734 | 28.751 | 56.880 | 25.119 |
| 443 | ND2 | ASN | 734 | 29.648 | 55.880 | 23.295 |
| 444 | C | ASN | 734 | 28.234 | 60.472 | 22.527 |
| 445 | O | ASN | 734 | 28.553 | 61.514 | 23.068 |
| 446 | HN | ASN | 734 | 26.738 | 57.686 | 23.252 |
| 447 | 1HD2 | ASN | 734 | 29.554 | 55.005 | 23.798 |
| 448 | 2HD2 | ASN | 734 | 29.844 | 55.853 | 22.299 |
| 449 | N | TYR | 735 | 28.000 | 60.392 | 21.104 |
| 450 | CA | TYR | 735 | 28.221 | 61.581 | 20.238 |
| 451 | CB | TYR | 735 | 28.239 | 61.209 | 18.744 |
| 452 | CG | TYR | 735 | 28.732 | 62.317 | 17.822 |
| 453 | CD1 | TYR | 735 | 27.956 | 62.829 | 16.719 |
| 454 | CE1 | TYR | 735 | 28.534 | 63.787 | 15.799 |
| 455 | CD2 | TYR | 735 | 30.072 | 62.805 | 17.969 |
| 456 | CE2 | TYR | 735 | 30.634 | 63.779 | 17.082 |
| 457 | CZ | TYR | 735 | 29.889 | 64.260 | 15.953 |
| 458 | OH | TYR | 735 | 30.467 | 65.122 | 15.045 |
| 459 | C | TYR | 735 | 27.159 | 62.705 | 20.506 |
| 460 | O | TYR | 735 | 27.440 | 63.877 | 20.302 |
| 461 | HN | TYR | 735 | 27.788 | 59.498 | 20.650 |
| 462 | HH | TYR | 735 | 31.333 | 65.397 | 15.397 |
| 463 | N | CYS | 736 | 25.856 | 62.320 | 20.919 |
| 464 | CA | CYS | 736 | 24.872 | 63.368 | 21.255 |
| 465 | CB | CYS | 736 | 23.401 | 62.968 | 21.477 |
| 466 | SG | CYS | 736 | 22.331 | 64.411 | 21.872 |
| 467 | C | CYS | 736 | 25.370 | 64.119 | 22.524 |
| 468 | O | CYS | 736 | 25.411 | 65.337 | 22.520 |
| 469 | HN | CYS | 736 | 25.703 | 61.369 | 21.255 |
| 470 | HG | CYS | 736 | 22.888 | 64.730 | 23.040 |
| 471 | N | PHE | 737 | 25.665 | 63.340 | 23.679 |
| 472 | CA | PHE | 737 | 26.128 | 63.994 | 24.917 |
| 473 | CB | PHE | 737 | 26.698 | 63.040 | 25.989 |
| 474 | CG | PHE | 737 | 25.668 | 62.221 | 26.731 |
| 475 | CD1 | PHE | 737 | 24.255 | 62.536 | 26.760 |
| 476 | CD2 | PHE | 737 | 26.155 | 61.135 | 27.540 |
| 477 | CE1 | PHE | 737 | 23.365 | 61.776 | 27.586 |
| 478 | CE2 | PHE | 737 | 25.260 | 60.346 | 28.320 |
| 479 | CZ | PHE | 737 | 23.864 | 60.669 | 28.348 |
| 480 | C | PHE | 737 | 27.258 | 65.000 | 24.547 |
| 481 | O | PHE | 737 | 27.115 | 66.171 | 24.847 |
| 482 | HN | PHE | 737 | 25.857 | 62.344 | 23.573 |
| 483 | N | THR | 739 | 28.592 | 66.234 | 21.279 |
| 484 | CA | THR | 739 | 28.373 | 67.521 | 20.510 |
| 485 | CB | THR | 739 | 27.663 | 67.618 | 19.123 |

| | | | | | | |
|---|---|---|---|---|---|---|
| 486 | OG1 | THR | 739 | 26.244 | 67.699 | 19.219 |
| 487 | CG2 | THR | 739 | 28.080 | 66.555 | 18.092 |
| 488 | C | THR | 739 | 27.622 | 68.587 | 21.389 |
| 489 | O | THR | 739 | 27.985 | 69.751 | 21.344 |
| 490 | HN | THR | 739 | 28.445 | 65.361 | 20.782 |
| 491 | HG1 | THR | 739 | 25.917 | 66.790 | 19.295 |
| 492 | N | PHE | 740 | 26.510 | 68.154 | 22.175 |
| 493 | CA | PHE | 740 | 25.749 | 69.052 | 23.051 |
| 494 | CB | PHE | 740 | 24.650 | 68.360 | 23.884 |
| 495 | CG | PHE | 740 | 24.007 | 69.302 | 24.879 |
| 496 | CD1 | PHE | 740 | 23.277 | 70.477 | 24.473 |
| 497 | CD2 | PHE | 740 | 24.083 | 69.004 | 26.281 |
| 498 | CE1 | PHE | 740 | 22.548 | 71.279 | 25.421 |
| 499 | CE2 | PHE | 740 | 23.397 | 69.818 | 27.237 |
| 500 | CZ | PHE | 740 | 22.594 | 70.931 | 26.810 |
| 501 | C | PHE | 740 | 26.769 | 69.784 | 23.994 |
| 502 | O | PHE | 740 | 26.562 | 70.964 | 24.247 |
| 503 | HN | PHE | 740 | 26.262 | 67.166 | 22.125 |
| 504 | N | ILE | 747 | 25.923 | 73.096 | 17.350 |
| 505 | CA | ILE | 747 | 24.670 | 72.412 | 16.983 |
| 506 | CB | ILE | 747 | 24.877 | 70.891 | 16.705 |
| 507 | CG2 | ILE | 747 | 23.621 | 70.043 | 16.988 |
| 508 | CG1 | ILE | 747 | 25.331 | 70.694 | 15.231 |
| 509 | CD1 | ILE | 747 | 25.758 | 69.266 | 14.865 |
| 510 | C | ILE | 747 | 23.678 | 72.718 | 18.153 |
| 511 | O | ILE | 747 | 24.023 | 72.615 | 19.325 |
| 512 | HN | ILE | 747 | 26.560 | 72.584 | 17.960 |
| 513 | N | PHE | 749 | 20.021 | 72.104 | 20.457 |
| 514 | CA | PHE | 749 | 19.101 | 71.157 | 21.062 |
| 515 | CB | PHE | 749 | 19.807 | 70.398 | 22.215 |
| 516 | CG | PHE | 749 | 20.760 | 69.403 | 21.580 |
| 517 | CD1 | PHE | 749 | 20.217 | 68.150 | 21.142 |
| 518 | CD2 | PHE | 749 | 22.150 | 69.683 | 21.298 |
| 519 | CE1 | PHE | 749 | 21.043 | 67.158 | 20.511 |
| 520 | CE2 | PHE | 749 | 22.991 | 68.682 | 20.687 |
| 521 | CZ | PHE | 749 | 22.449 | 67.399 | 20.329 |
| 522 | C | PHE | 749 | 17.913 | 72.042 | 21.523 |
| 523 | O | PHE | 749 | 18.113 | 72.930 | 22.338 |
| 524 | HN | PHE | 749 | 20.752 | 72.442 | 21.072 |
| 525 | N | PRO | 750 | 16.607 | 71.806 | 20.962 |
| 526 | CD | PRO | 750 | 16.137 | 70.841 | 19.982 |
| 527 | CA | PRO | 750 | 15.444 | 72.491 | 21.552 |
| 528 | CB | PRO | 750 | 14.275 | 72.210 | 20.593 |
| 529 | CG | PRO | 750 | 14.608 | 70.797 | 20.114 |
| 530 | C | PRO | 750 | 15.157 | 71.868 | 22.959 |
| 531 | O | PRO | 750 | 15.795 | 70.949 | 23.448 |
| 532 | N | LEU | 753 | 14.852 | 67.716 | 24.377 |
| 533 | CA | LEU | 753 | 16.127 | 66.985 | 24.320 |
| 534 | CB | LEU | 753 | 16.739 | 66.915 | 22.902 |
| 535 | CG | LEU | 753 | 16.942 | 65.478 | 22.381 |
| 536 | CD1 | LEU | 753 | 15.611 | 64.700 | 22.396 |
| 537 | CD2 | LEU | 753 | 17.562 | 65.504 | 20.966 |
| 538 | C | LEU | 753 | 17.154 | 67.647 | 25.242 |
| 539 | O | LEU | 753 | 17.936 | 66.959 | 25.870 |
| 540 | HN | LEU | 753 | 14.507 | 68.086 | 23.510 |

| | | | | | | |
|---|---|---|---|---|---|---|
| 541 | N | ILE | 757 | 18.978 | 65.448 | 27.869 |
| 542 | CA | ILE | 757 | 20.414 | 65.238 | 27.688 |
| 543 | CB | ILE | 757 | 20.964 | 66.077 | 26.505 |
| 544 | CG2 | ILE | 757 | 22.488 | 66.162 | 26.576 |
| 545 | CG1 | ILE | 757 | 20.521 | 65.482 | 25.146 |
| 546 | CD1 | ILE | 757 | 20.619 | 66.465 | 23.973 |
| 547 | C | ILE | 757 | 21.045 | 65.622 | 29.064 |
| 548 | O | ILE | 757 | 21.490 | 64.771 | 29.808 |
| 549 | HN | ILE | 757 | 18.553 | 66.213 | 27.348 |
| 550 | O | TYR | 764 | 26.376 | 55.800 | 32.328 |
| 551 | C1 | A22 | 1 | 16.117 | 63.392 | 13.900 |
| 552 | O2 | A22 | 1 | 16.211 | 63.357 | 15.322 |
| 553 | C3 | A22 | 1 | 16.477 | 62.074 | 15.719 |
| 554 | C4 | A22 | 1 | 17.785 | 61.634 | 16.122 |
| 555 | C5 | A22 | 1 | 18.965 | 62.451 | 16.278 |
| 556 | C6 | A22 | 1 | 18.958 | 63.859 | 16.090 |
| 557 | C7 | A22 | 1 | 20.161 | 64.582 | 16.050 |
| 558 | C8 | A22 | 1 | 21.372 | 63.974 | 16.417 |
| 559 | N9 | A22 | 1 | 22.523 | 64.796 | 16.383 |
| 560 | C10 | A22 | 1 | 23.878 | 64.333 | 16.639 |
| 561 | C11 | A22 | 1 | 24.507 | 65.152 | 17.787 |
| 562 | C12 | A22 | 1 | 24.750 | 64.514 | 15.379 |
| 563 | C13 | A22 | 1 | 23.850 | 62.882 | 17.067 |
| 564 | C14 | A22 | 1 | 22.777 | 62.061 | 17.215 |
| 565 | C15 | A22 | 1 | 23.067 | 60.715 | 17.854 |
| 566 | C16 | A22 | 1 | 21.427 | 62.597 | 16.761 |
| 567 | C17 | A22 | 1 | 20.222 | 61.826 | 16.565 |
| 568 | C18 | A22 | 1 | 20.280 | 60.320 | 16.495 |
| 569 | C19 | A22 | 1 | 20.998 | 59.992 | 15.189 |
| 570 | C20 | A22 | 1 | 21.867 | 58.886 | 15.109 |
| 571 | C21 | A22 | 1 | 22.765 | 58.715 | 14.027 |
| 572 | C22 | A22 | 1 | 22.741 | 59.608 | 12.933 |
| 573 | C23 | A22 | 1 | 21.800 | 60.663 | 12.950 |
| 574 | O24 | A22 | 1 | 21.777 | 61.595 | 11.938 |
| 575 | C25 | A22 | 1 | 22.404 | 62.797 | 12.393 |
| 576 | S26 | A22 | 1 | 24.075 | 62.411 | 13.015 |
| 577 | C27 | A22 | 1 | 24.904 | 62.110 | 11.436 |
| 578 | C28 | A22 | 1 | 20.908 | 60.815 | 14.035 |
| 579 | O29 | A22 | 1 | 19.063 | 59.614 | 16.702 |
| 580 | C30 | A22 | 1 | 17.888 | 60.225 | 16.341 |
| 581 | C31 | A22 | 1 | 16.782 | 59.348 | 16.192 |
| 582 | C32 | A22 | 1 | 15.519 | 59.827 | 15.797 |
| 583 | C33 | A22 | 1 | 15.360 | 61.209 | 15.618 |
| 584 | H37 | A22 | 1 | 22.462 | 65.630 | 15.808 |

TABLE 8

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1 | N | ILE | A | 672 | 13.004 | 25.282 | 22.008 | 1.00 | 24.75 |
| 2 | CA | ILE | A | 672 | 14.475 | 25.215 | 22.242 | 1.00 | 25.11 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 3 | C | ILE | A | 672 | 15.115 | 23.900 | 21.789 | 1.00 | 25.32 |
| 4 | O | ILE | A | 672 | 16.189 | 23.926 | 21.195 | 1.00 | 24.67 |
| 5 | CB | ILE | A | 672 | 14.846 | 25.527 | 23.734 | 1.00 | 25.78 |
| 6 | CG1 | ILE | A | 672 | 14.842 | 27.035 | 23.978 | 1.00 | 25.60 |
| 7 | CG2 | ILE | A | 672 | 16.247 | 25.008 | 24.099 | 1.00 | 25.56 |
| 8 | CD1 | ILE | A | 672 | 15.312 | 27.404 | 25.360 | 1.00 | 25.81 |
| 9 | N | PHE | A | 673 | 14.448 | 22.768 | 22.030 | 1.00 | 25.89 |
| 10 | CA | PHE | A | 673 | 14.980 | 21.446 | 21.635 | 1.00 | 25.86 |
| 11 | C | PHE | A | 673 | 15.213 | 21.374 | 20.147 | 1.00 | 25.25 |
| 12 | O | PHE | A | 673 | 16.260 | 20.926 | 19.680 | 1.00 | 24.38 |
| 13 | CB | PHE | A | 673 | 14.020 | 20.306 | 22.029 | 1.00 | 26.22 |
| 14 | CG | PHE | A | 673 | 14.557 | 18.923 | 21.722 | 1.00 | 25.12 |
| 15 | CD1 | PHE | A | 673 | 15.765 | 18.501 | 22.251 | 1.00 | 25.16 |
| 16 | CD2 | PHE | A | 673 | 13.877 | 18.066 | 20.874 | 1.00 | 25.81 |
| 17 | CE1 | PHE | A | 673 | 16.286 | 17.255 | 21.946 | 1.00 | 23.42 |
| 18 | CE2 | PHE | A | 673 | 14.403 | 16.809 | 20.567 | 1.00 | 25.08 |
| 19 | CZ | PHE | A | 673 | 15.609 | 16.417 | 21.107 | 1.00 | 23.85 |
| 20 | N | LEU | A | 674 | 14.193 | 21.792 | 19.412 | 1.00 | 25.01 |
| 21 | CA | LEU | A | 674 | 14.237 | 21.802 | 17.969 | 1.00 | 25.58 |
| 22 | C | LEU | A | 674 | 15.199 | 22.801 | 17.357 | 1.00 | 25.10 |
| 23 | O | LEU | A | 674 | 15.743 | 22.518 | 16.294 | 1.00 | 26.08 |
| 24 | CB | LEU | A | 674 | 12.833 | 21.974 | 17.391 | 1.00 | 26.05 |
| 25 | CG | LEU | A | 674 | 12.067 | 20.653 | 17.317 | 1.00 | 26.55 |
| 26 | CD1 | LEU | A | 674 | 10.617 | 20.887 | 16.935 | 1.00 | 26.35 |
| 27 | CD2 | LEU | A | 674 | 12.762 | 19.758 | 16.304 | 1.00 | 26.09 |
| 28 | N | ASN | A | 675 | 15.440 | 23.939 | 18.019 | 1.00 | 24.63 |
| 29 | CA | ASN | A | 675 | 16.356 | 24.964 | 17.484 | 1.00 | 23.19 |
| 30 | C | ASN | A | 675 | 17.726 | 24.338 | 17.397 | 1.00 | 21.66 |
| 31 | O | ASN | A | 675 | 18.435 | 24.524 | 16.417 | 1.00 | 21.43 |
| 32 | CB | ASN | A | 675 | 16.478 | 26.215 | 18.393 | 1.00 | 24.20 |
| 33 | CG | ASN | A | 675 | 15.206 | 27.067 | 18.452 | 1.00 | 24.32 |
| 34 | OD1 | ASN | A | 675 | 14.368 | 27.062 | 17.547 | 1.00 | 24.82 |
| 35 | ND2 | ASN | A | 675 | 15.076 | 27.817 | 19.539 | 1.00 | 24.74 |
| 36 | N | VAL | A | 676 | 18.095 | 23.612 | 18.448 | 1.00 | 21.17 |
| 37 | CA | VAL | A | 676 | 19.394 | 22.952 | 18.507 | 1.00 | 20.92 |
| 38 | C | VAL | A | 676 | 19.501 | 21.830 | 17.473 | 1.00 | 19.78 |
| 39 | O | VAL | A | 676 | 20.421 | 21.827 | 16.646 | 1.00 | 19.99 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 40 | CB | VAL | A | 676 | 19.718 | 22.442 | 19.934 | 1.00 | 21.33 |
| 41 | CG1 | VAL | A | 676 | 18.899 | 21.237 | 20.247 | 1.00 | 24.09 |
| 42 | CG2 | VAL | A | 676 | 21.192 | 22.095 | 20.065 | 1.00 | 21.88 |
| 43 | N | LEU | A | 677 | 18.530 | 20.923 | 17.434 | 1.00 | 19.08 |
| 44 | CA | LEU | A | 677 | 18.601 | 19.848 | 16.453 | 1.00 | 17.91 |
| 45 | C | LEU | A | 677 | 18.768 | 20.427 | 15.068 | 1.00 | 16.96 |
| 46 | O | LEU | A | 677 | 19.640 | 20.008 | 14.347 | 1.00 | 14.94 |
| 47 | CB | LEU | A | 677 | 17.383 | 18.921 | 16.518 | 1.00 | 17.50 |
| 48 | CG | LEU | A | 677 | 17.267 | 18.083 | 17.798 | 1.00 | 16.78 |
| 49 | CD1 | LEU | A | 677 | 16.355 | 16.934 | 17.541 | 1.00 | 17.01 |
| 50 | CD2 | LEU | A | 677 | 18.615 | 17.555 | 18.225 | 1.00 | 17.10 |
| 51 | N | GLU | A | 678 | 17.980 | 21.445 | 14.736 | 1.00 | 19.12 |
| 52 | CA | GLU | A | 678 | 18.058 | 22.121 | 13.437 | 1.00 | 20.06 |
| 53 | C | GLU | A | 678 | 19.410 | 22.783 | 13.243 | 1.00 | 19.33 |
| 54 | O | GLU | A | 678 | 19.966 | 22.737 | 12.152 | 1.00 | 18.20 |
| 55 | CB | GLU | A | 678 | 16.972 | 23.188 | 13.317 | 1.00 | 23.33 |
| 56 | CG | GLU | A | 678 | 15.532 | 22.646 | 13.381 | 1.00 | 28.64 |
| 57 | CD | GLU | A | 678 | 14.459 | 23.736 | 13.387 | 1.00 | 32.31 |
| 58 | OE1 | GLU | A | 678 | 14.811 | 24.943 | 13.374 | 1.00 | 34.41 |
| 59 | OE2 | GLU | A | 678 | 13.253 | 23.384 | 13.410 | 1.00 | 34.91 |
| 60 | N | ALA | A | 679 | 19.966 | 23.324 | 14.329 | 1.00 | 19.45 |
| 61 | CA | ALA | A | 679 | 21.257 | 24.018 | 14.303 | 1.00 | 18.84 |
| 62 | C | ALA | A | 679 | 22.472 | 23.094 | 14.195 | 1.00 | 19.25 |
| 63 | O | ALA | A | 679 | 23.479 | 23.436 | 13.558 | 1.00 | 19.27 |
| 64 | CB | ALA | A | 679 | 21.388 | 24.919 | 15.517 | 1.00 | 17.67 |
| 65 | N | ILE | A | 680 | 22.395 | 21.914 | 14.802 | 1.00 | 18.82 |
| 66 | CA | ILE | A | 680 | 23.518 | 20.984 | 14.742 | 1.00 | 17.49 |
| 67 | C | ILE | A | 680 | 23.516 | 19.984 | 13.593 | 1.00 | 16.89 |
| 68 | O | ILE | A | 680 | 24.518 | 19.303 | 13.370 | 1.00 | 17.12 |
| 69 | CB | ILE | A | 680 | 23.674 | 20.231 | 16.056 | 1.00 | 17.05 |
| 70 | CG1 | ILE | A | 680 | 22.393 | 19.467 | 16.391 | 1.00 | 15.55 |
| 71 | CG2 | ILE | A | 680 | 24.022 | 21.213 | 17.158 | 1.00 | 16.83 |
| 72 | CD1 | ILE | A | 680 | 22.558 | 18.575 | 17.552 | 1.00 | 13.80 |
| 73 | N | GLU | A | 681 | 22.415 | 19.922 | 12.847 | 1.00 | 16.79 |
| 74 | CA | GLU | A | 681 | 22.265 | 19.002 | 11.719 | 1.00 | 17.26 |
| 75 | C | GLU | A | 681 | 23.370 | 19.128 | 10.673 | 1.00 | 18.79 |
| 76 | O | GLU | A | 681 | 23.517 | 20.173 | 10.043 | 1.00 | 20.09 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 77 | CB | GLU | A | 681 | 20.902 | 19.227 | 11.094 | 1.00 | 16.72 |
| 78 | CG | GLU | A | 681 | 20.579 | 18.300 | 9.952 | 1.00 | 18.48 |
| 79 | CD | GLU | A | 681 | 20.473 | 16.823 | 10.348 | 1.00 | 17.51 |
| 80 | OE1 | GLU | A | 681 | 20.659 | 16.502 | 11.524 | 1.00 | 17.58 |
| 81 | OE2 | GLU | A | 681 | 20.214 | 15.981 | 9.467 | 1.00 | 18.59 |
| 82 | N | PRO | A | 682 | 24.145 | 18.044 | 10.437 | 1.00 | 19.02 |
| 83 | CA | PRO | A | 682 | 25.252 | 18.021 | 9.472 | 1.00 | 19.11 |
| 84 | C | PRO | A | 682 | 24.912 | 18.475 | 8.057 | 1.00 | 19.76 |
| 85 | O | PRO | A | 682 | 23.771 | 18.382 | 7.625 | 1.00 | 21.13 |
| 86 | CB | PRO | A | 682 | 25.681 | 16.546 | 9.493 | 1.00 | 18.30 |
| 87 | CG | PRO | A | 682 | 25.338 | 16.109 | 10.846 | 1.00 | 17.08 |
| 88 | CD | PRO | A | 682 | 23.969 | 16.704 | 11.019 | 1.00 | 18.22 |
| 89 | N | GLY | A | 683 | 25.901 | 18.995 | 7.339 | 1.00 | 20.64 |
| 90 | CA | GLY | A | 683 | 25.665 | 19.422 | 5.972 | 1.00 | 21.67 |
| 91 | C | GLY | A | 683 | 25.809 | 18.260 | 4.990 | 1.00 | 23.13 |
| 92 | O | GLY | A | 683 | 25.595 | 17.108 | 5.355 | 1.00 | 23.47 |
| 93 | N | VAL | A | 684 | 26.190 | 18.567 | 3.748 | 1.00 | 23.58 |
| 94 | CA | VAL | A | 684 | 26.365 | 17.573 | 2.685 | 1.00 | 22.44 |
| 95 | C | VAL | A | 684 | 27.725 | 16.934 | 2.811 | 1.00 | 20.64 |
| 96 | O | VAL | A | 684 | 28.708 | 17.614 | 3.042 | 1.00 | 19.82 |
| 97 | CB | VAL | A | 684 | 26.320 | 18.216 | 1.259 | 1.00 | 24.93 |
| 98 | CG1 | VAL | A | 684 | 26.217 | 17.130 | 0.183 | 1.00 | 24.57 |
| 99 | CG2 | VAL | A | 684 | 25.153 | 19.228 | 1.131 | 1.00 | 24.89 |
| 100 | N | VAL | A | 685 | 27.778 | 15.631 | 2.585 | 1.00 | 19.05 |
| 101 | CA | VAL | A | 685 | 29.012 | 14.878 | 2.665 | 1.00 | 17.89 |
| 102 | C | VAL | A | 685 | 29.143 | 14.112 | 1.345 | 1.00 | 17.88 |
| 103 | O | VAL | A | 685 | 28.238 | 13.367 | 0.969 | 1.00 | 18.33 |
| 104 | CB | VAL | A | 685 | 28.955 | 13.857 | 3.867 | 1.00 | 17.81 |
| 105 | CG1 | VAL | A | 685 | 30.303 | 13.189 | 4.086 | 1.00 | 15.58 |
| 106 | CG2 | VAL | A | 685 | 28.527 | 14.556 | 5.147 | 1.00 | 16.27 |
| 107 | N | CYS | A | 686 | 30.224 | 14.339 | 0.609 | 1.00 | 17.00 |
| 108 | CA | CYS | A | 686 | 30.451 | 13.628 | -0.650 | 1.00 | 17.52 |
| 109 | C | CYS | A | 686 | 31.354 | 12.447 | -0.327 | 1.00 | 16.97 |
| 110 | O | CYS | A | 686 | 32.141 | 12.496 | 0.615 | 1.00 | 17.15 |
| 111 | CB | CYS | A | 686 | 31.101 | 14.534 | -1.706 | 1.00 | 17.76 |
| 112 | SG | CYS | A | 686 | 30.166 | 16.031 | -2.147 | 1.00 | 21.38 |
| 113 | N | ALA | A | 687 | 31.183 | 11.360 | -1.065 | 1.00 | 17.74 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 114 | CA | ALA | A | 687 | 31.949 | 10.132 | -0.836 | 1.00 | 17.57 |
| 115 | C | ALA | A | 687 | 33.277 | 10.161 | -1.526 | 1.00 | 18.06 |
| 116 | O | ALA | A | 687 | 34.185 | 9.431 | -1.139 | 1.00 | 17.98 |
| 117 | CB | ALA | A | 687 | 31.161 | 8.929 | -1.295 | 1.00 | 16.91 |
| 118 | N | GLY | A | 688 | 33.370 | 11.023 | -2.539 | 1.00 | 18.50 |
| 119 | CA | GLY | A | 688 | 34.580 | 11.167 | -3.326 | 1.00 | 19.16 |
| 120 | C | GLY | A | 688 | 34.705 | 10.099 | -4.388 | 1.00 | 19.90 |
| 121 | O | GLY | A | 688 | 35.802 | 9.730 | -4.771 | 1.00 | 20.86 |
| 122 | N | HIS | A | 689 | 33.582 | 9.630 | -4.907 | 1.00 | 20.92 |
| 123 | CA | HIS | A | 689 | 33.577 | 8.576 | -5.912 | 1.00 | 22.43 |
| 124 | C | HIS | A | 689 | 33.923 | 9.063 | -7.328 | 1.00 | 24.19 |
| 125 | O | HIS | A | 689 | 33.511 | 10.145 | -7.731 | 1.00 | 24.06 |
| 126 | CB | HIS | A | 689 | 32.195 | 7.917 | -5.900 | 1.00 | 22.00 |
| 127 | CG | HIS | A | 689 | 32.046 | 6.775 | -6.857 | 1.00 | 22.28 |
| 128 | ND1 | HIS | A | 689 | 31.040 | 6.724 | -7.796 | 1.00 | 22.44 |
| 129 | CD2 | HIS | A | 689 | 32.782 | 5.656 | -7.033 | 1.00 | 22.64 |
| 130 | CE1 | HIS | A | 689 | 31.166 | 5.627 | -8.516 | 1.00 | 23.43 |
| 131 | NE2 | HIS | A | 689 | 32.219 | 4.960 | -8.074 | 1.00 | 23.78 |
| 132 | N | ASP | A | 690 | 34.719 | 8.296 | -8.073 | 1.00 | 26.27 |
| 133 | CA | ASP | A | 690 | 35.017 | 8.691 | -9.447 | 1.00 | 28.86 |
| 134 | C | ASP | A | 690 | 33.872 | 8.164 | -10.286 | 1.00 | 30.15 |
| 135 | O | ASP | A | 690 | 33.701 | 6.952 | -10.409 | 1.00 | 30.46 |
| 136 | CB | ASP | A | 690 | 36.330 | 8.096 | -9.963 | 1.00 | 28.93 |
| 137 | CG | ASP | A | 690 | 36.696 | 8.618 | -11.361 | 1.00 | 30.03 |
| 138 | OD1 | ASP | A | 690 | 37.868 | 8.497 | -11.764 | 1.00 | 31.23 |
| 139 | OD2 | ASP | A | 690 | 35.819 | 9.170 | -12.061 | 1.00 | 29.72 |
| 140 | N | ASN | A | 691 | 33.065 | 9.067 | -10.832 | 1.00 | 32.35 |
| 141 | CA | ASN | A | 691 | 31.933 | 8.660 | -11.655 | 1.00 | 33.60 |
| 142 | C | ASN | A | 691 | 32.284 | 8.608 | -13.136 | 1.00 | 35.13 |
| 143 | O | ASN | A | 691 | 31.419 | 8.733 | -13.999 | 1.00 | 36.66 |
| 144 | CB | ASN | A | 691 | 30.725 | 9.562 | -11.416 | 1.00 | 32.74 |
| 145 | CG | ASN | A | 691 | 30.079 | 9.313 | -10.074 | 1.00 | 32.95 |
| 146 | OD1 | ASN | A | 691 | 29.187 | 8.474 | -9.930 | 1.00 | 32.13 |
| 147 | ND2 | ASN | A | 691 | 30.547 | 10.024 | -9.069 | 1.00 | 33.57 |
| 148 | N | ASN | A | 692 | 33.565 | 8.471 | -13.434 | 1.00 | 36.15 |
| 149 | CA | ASN | A | 692 | 33.995 | 8.365 | -14.819 | 1.00 | 37.44 |
| 150 | C | ASN | A | 692 | 34.425 | 6.913 | -15.027 | 1.00 | 38.39 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 151 | O | ASN | A | 692 | 34.414 | 6.406 | -16.139 | 1.00 | 39.10 |
| 152 | CB | ASN | A | 692 | 35.148 | 9.328 | -15.103 | 1.00 | 36.71 |
| 153 | N | GLN | A | 693 | 34.757 | 6.241 | -13.928 | 1.00 | 39.29 |
| 154 | CA | GLN | A | 693 | 35.200 | 4.849 | -13.942 | 1.00 | 40.00 |
| 155 | C | GLN | A | 693 | 33.988 | 3.939 | -13.854 | 1.00 | 39.48 |
| 156 | O | GLN | A | 693 | 32.997 | 4.298 | -13.217 | 1.00 | 40.11 |
| 157 | CB | GLN | A | 693 | 36.131 | 4.577 | -12.745 | 1.00 | 41.81 |
| 158 | CG | GLN | A | 693 | 37.538 | 4.029 | -13.110 | 1.00 | 44.34 |
| 159 | CD | GLN | A | 693 | 38.420 | 5.017 | -13.902 | 1.00 | 45.44 |
| 160 | OE1 | GLN | A | 693 | 39.378 | 5.587 | -13.363 | 1.00 | 45.95 |
| 161 | NE2 | GLN | A | 693 | 38.115 | 5.193 | -15.186 | 1.00 | 45.47 |
| 162 | N | PRO | A | 694 | 34.055 | 2.743 | -14.485 | 1.00 | 38.78 |
| 163 | CA | PRO | A | 694 | 32.970 | 1.762 | -14.489 | 1.00 | 36.98 |
| 164 | C | PRO | A | 694 | 32.575 | 1.304 | -13.109 | 1.00 | 35.56 |
| 165 | O | PRO | A | 694 | 33.411 | 1.198 | -12.204 | 1.00 | 35.44 |
| 166 | CB | PRO | A | 694 | 33.571 | 0.601 | -15.265 | 1.00 | 37.17 |
| 167 | CG | PRO | A | 694 | 34.432 | 1.271 | -16.234 | 1.00 | 38.48 |
| 168 | CD | PRO | A | 694 | 35.138 | 2.286 | -15.375 | 1.00 | 38.88 |
| 169 | N | ASP | A | 695 | 31.289 | 1.022 | -12.958 | 1.00 | 34.27 |
| 170 | CA | ASP | A | 695 | 30.776 | 0.534 | -11.698 | 1.00 | 32.38 |
| 171 | C | ASP | A | 695 | 31.318 | -0.868 | -11.524 | 1.00 | 32.55 |
| 172 | O | ASP | A | 695 | 31.237 | -1.707 | -12.429 | 1.00 | 33.50 |
| 173 | CB | ASP | A | 695 | 29.251 | 0.518 | -11.694 | 1.00 | 29.77 |
| 174 | CG | ASP | A | 695 | 28.660 | 1.901 | -11.608 | 1.00 | 28.80 |
| 175 | OD1 | ASP | A | 695 | 27.532 | 2.100 | -12.089 | 1.00 | 27.09 |
| 176 | OD2 | ASP | A | 695 | 29.329 | 2.794 | -11.057 | 1.00 | 28.72 |
| 177 | N | SER | A | 696 | 32.025 | -1.052 | -10.424 | 1.00 | 31.71 |
| 178 | CA | SER | A | 696 | 32.577 | -2.333 | -10.077 | 1.00 | 30.42 |
| 179 | C | SER | A | 696 | 32.340 | -2.445 | -8.577 | 1.00 | 30.15 |
| 180 | O | SER | A | 696 | 32.275 | -1.418 | -7.885 | 1.00 | 30.10 |
| 181 | CB | SER | A | 696 | 34.064 | -2.383 | -10.425 | 1.00 | 30.43 |
| 182 | OG | SER | A | 696 | 34.854 | -1.589 | -9.567 | 1.00 | 31.47 |
| 183 | N | PHE | A | 697 | 32.104 | -3.669 | -8.099 | 1.00 | 28.48 |
| 184 | CA | PHE | A | 697 | 31.890 | -3.933 | -6.679 | 1.00 | 26.74 |
| 185 | C | PHE | A | 697 | 32.956 | -3.205 | -5.846 | 1.00 | 26.76 |
| 186 | O | PHE | A | 697 | 32.641 | -2.393 | -4.972 | 1.00 | 27.12 |
| 187 | CB | PHE | A | 697 | 31.982 | -5.442 | -6.423 | 1.00 | 25.46 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 188 | CG | PHE | A | 697 | 31.781 | -5.827 | -4.989 | 1.00 | 24.31 |
| 189 | CD1 | PHE | A | 697 | 30.536 | -5.722 | -4.398 | 1.00 | 24.48 |
| 190 | CD2 | PHE | A | 697 | 32.845 | -6.281 | -4.220 | 1.00 | 24.78 |
| 191 | CE1 | PHE | A | 697 | 30.344 | -6.063 | -3.071 | 1.00 | 24.65 |
| 192 | CE2 | PHE | A | 697 | 32.659 | -6.626 | -2.886 | 1.00 | 24.77 |
| 193 | CZ | PHE | A | 697 | 31.406 | -6.512 | -2.315 | 1.00 | 24.30 |
| 194 | N | ALA | A | 698 | 34.219 | -3.495 | -6.140 | 1.00 | 25.86 |
| 195 | CA | ALA | A | 698 | 35.351 | -2.911 | -5.436 | 1.00 | 24.97 |
| 196 | C | ALA | A | 698 | 35.323 | -1.402 | -5.300 | 1.00 | 25.24 |
| 197 | O | ALA | A | 698 | 35.559 | -0.852 | -4.216 | 1.00 | 24.99 |
| 198 | CB | ALA | A | 698 | 36.596 | -3.305 | -6.131 | 1.00 | 25.52 |
| 199 | N | ALA | A | 699 | 35.029 | -0.737 | -6.414 | 1.00 | 25.11 |
| 200 | CA | ALA | A | 699 | 35.001 | 0.717 | -6.490 | 1.00 | 23.76 |
| 201 | C | ALA | A | 699 | 33.873 | 1.281 | -5.668 | 1.00 | 22.91 |
| 202 | O | ALA | A | 699 | 34.084 | 2.133 | -4.795 | 1.00 | 22.51 |
| 203 | CB | ALA | A | 699 | 34.845 | 1.156 | -7.943 | 1.00 | 24.31 |
| 204 | N | LEU | A | 700 | 32.682 | 0.770 | -5.957 | 1.00 | 21.56 |
| 205 | CA | LEU | A | 700 | 31.440 | 1.185 | -5.314 | 1.00 | 20.84 |
| 206 | C | LEU | A | 700 | 31.456 | 0.977 | -3.793 | 1.00 | 20.77 |
| 207 | O | LEU | A | 700 | 30.891 | 1.765 | -3.035 | 1.00 | 19.67 |
| 208 | CB | LEU | A | 700 | 30.274 | 0.397 | -5.937 | 1.00 | 19.59 |
| 209 | CG | LEU | A | 700 | 29.249 | 0.984 | -6.911 | 1.00 | 18.78 |
| 210 | CD1 | LEU | A | 700 | 29.727 | 2.269 | -7.529 | 1.00 | 18.69 |
| 211 | CD2 | LEU | A | 700 | 28.952 | -0.015 | -7.957 | 1.00 | 17.10 |
| 212 | N | LEU | A | 701 | 32.103 | -0.093 | -3.350 | 1.00 | 20.77 |
| 213 | CA | LEU | A | 701 | 32.147 | -0.367 | -1.941 | 1.00 | 20.58 |
| 214 | C | LEU | A | 701 | 33.261 | 0.365 | -1.241 | 1.00 | 20.86 |
| 215 | O | LEU | A | 701 | 33.126 | 0.734 | -0.088 | 1.00 | 21.69 |
| 216 | CB | LEU | A | 701 | 32.099 | -1.871 | -1.670 | 1.00 | 19.52 |
| 217 | CG | LEU | A | 701 | 30.582 | -2.050 | -1.567 | 1.00 | 19.40 |
| 218 | CD1 | LEU | A | 701 | 30.046 | -2.911 | -2.642 | 1.00 | 17.73 |
| 219 | CD2 | LEU | A | 701 | 30.173 | -2.510 | -0.217 | 1.00 | 17.25 |
| 220 | N | SER | A | 702 | 34.356 | 0.615 | -1.937 | 1.00 | 21.06 |
| 221 | CA | SER | A | 702 | 35.406 | 1.378 | -1.316 | 1.00 | 20.96 |
| 222 | C | SER | A | 702 | 34.874 | 2.791 | -1.105 | 1.00 | 20.81 |
| 223 | O | SER | A | 702 | 35.187 | 3.423 | -0.103 | 1.00 | 20.82 |
| 224 | CB | SER | A | 702 | 36.632 | 1.400 | -2.190 | 1.00 | 21.52 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 225 | OG | SER | A | 702 | 37.204 | 0.120 | -2.175 | 1.00 | 23.71 |
| 226 | N | SER | A | 703 | 34.023 | 3.250 | -2.028 | 1.00 | 20.55 |
| 227 | CA | SER | A | 703 | 33.443 | 4.585 | -1.934 | 1.00 | 18.91 |
| 228 | C | SER | A | 703 | 32.470 | 4.678 | -0.793 | 1.00 | 18.35 |
| 229 | O | SER | A | 703 | 32.520 | 5.625 | -0.025 | 1.00 | 18.89 |
| 230 | CB | SER | A | 703 | 32.755 | 4.966 | -3.224 | 1.00 | 18.44 |
| 231 | OG | SER | A | 703 | 33.748 | 5.182 | -4.194 | 1.00 | 20.63 |
| 232 | N | LEU | A | 704 | 31.596 | 3.684 | -0.662 | 1.00 | 17.26 |
| 233 | CA | LEU | A | 704 | 30.639 | 3.687 | 0.432 | 1.00 | 16.37 |
| 234 | C | LEU | A | 704 | 31.366 | 3.678 | 1.761 | 1.00 | 16.10 |
| 235 | O | LEU | A | 704 | 30.925 | 4.340 | 2.696 | 1.00 | 16.81 |
| 236 | CB | LEU | A | 704 | 29.691 | 2.497 | 0.342 | 1.00 | 15.19 |
| 237 | CG | LEU | A | 704 | 28.558 | 2.583 | -0.660 | 1.00 | 14.03 |
| 238 | CD1 | LEU | A | 704 | 27.882 | 1.259 | -0.748 | 1.00 | 12.28 |
| 239 | CD2 | LEU | A | 704 | 27.582 | 3.681 | -0.235 | 1.00 | 14.48 |
| 240 | N | ASN | A | 705 | 32.495 | 2.961 | 1.829 | 1.00 | 16.70 |
| 241 | CA | ASN | A | 705 | 33.307 | 2.863 | 3.049 | 1.00 | 16.66 |
| 242 | C | ASN | A | 705 | 33.955 | 4.201 | 3.410 | 1.00 | 17.17 |
| 243 | O | ASN | A | 705 | 33.970 | 4.587 | 4.570 | 1.00 | 17.46 |
| 244 | CB | ASN | A | 705 | 34.398 | 1.794 | 2.924 | 1.00 | 15.46 |
| 245 | CG | ASN | A | 705 | 33.850 | 0.384 | 2.941 | 1.00 | 16.24 |
| 246 | OD1 | ASN | A | 705 | 34.448 | -0.512 | 2.385 | 1.00 | 16.82 |
| 247 | ND2 | ASN | A | 705 | 32.726 | 0.180 | 3.592 | 1.00 | 16.07 |
| 248 | N | GLU | A | 706 | 34.512 | 4.882 | 2.415 | 1.00 | 17.04 |
| 249 | CA | GLU | A | 706 | 35.151 | 6.193 | 2.598 | 1.00 | 17.55 |
| 250 | C | GLU | A | 706 | 34.089 | 7.180 | 3.069 | 1.00 | 16.10 |
| 251 | O | GLU | A | 706 | 34.313 | 8.023 | 3.950 | 1.00 | 16.62 |
| 252 | CB | GLU | A | 706 | 35.739 | 6.668 | 1.258 | 1.00 | 18.93 |
| 253 | CG | GLU | A | 706 | 36.394 | 8.029 | 1.282 | 1.00 | 21.19 |
| 254 | CD | GLU | A | 706 | 37.488 | 8.146 | 2.347 | 1.00 | 23.68 |
| 255 | OE1 | GLU | A | 706 | 37.586 | 9.225 | 2.978 | 1.00 | 25.14 |
| 256 | OE2 | GLU | A | 706 | 38.246 | 7.175 | 2.569 | 1.00 | 24.37 |
| 257 | N | LEU | A | 707 | 32.927 | 7.076 | 2.445 | 1.00 | 15.13 |
| 258 | CA | LEU | A | 707 | 31.803 | 7.916 | 2.792 | 1.00 | 14.21 |
| 259 | C | LEU | A | 707 | 31.461 | 7.634 | 4.228 | 1.00 | 14.91 |
| 260 | O | LEU | A | 707 | 31.121 | 8.557 | 4.980 | 1.00 | 15.85 |
| 261 | CB | LEU | A | 707 | 30.604 | 7.579 | 1.925 | 1.00 | 12.85 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 262 | CG | LEU | A | 707 | 29.318 | 8.262 | 2.328 | 1.00 | 12.03 |
| 263 | CD1 | LEU | A | 707 | 29.537 | 9.745 | 2.280 | 1.00 | 13.09 |
| 264 | CD2 | LEU | A | 707 | 28.252 | 7.889 | 1.374 | 1.00 | 12.74 |
| 265 | N | GLY | A | 708 | 31.532 | 6.358 | 4.602 | 1.00 | 13.93 |
| 266 | CA | GLY | A | 708 | 31.230 | 5.976 | 5.965 | 1.00 | 12.96 |
| 267 | C | GLY | A | 708 | 32.213 | 6.620 | 6.917 | 1.00 | 13.13 |
| 268 | O | GLY | A | 708 | 31.849 | 7.061 | 7.987 | 1.00 | 13.55 |
| 269 | N | GLU | A | 709 | 33.468 | 6.687 | 6.514 | 1.00 | 14.14 |
| 270 | CA | GLU | A | 709 | 34.525 | 7.279 | 7.322 | 1.00 | 15.83 |
| 271 | C | GLU | A | 709 | 34.334 | 8.775 | 7.486 | 1.00 | 16.65 |
| 272 | O | GLU | A | 709 | 34.628 | 9.317 | 8.563 | 1.00 | 17.59 |
| 273 | CB | GLU | A | 709 | 35.874 | 7.046 | 6.658 | 1.00 | 16.73 |
| 274 | CG | GLU | A | 709 | 37.051 | 7.547 | 7.446 | 1.00 | 18.68 |
| 275 | CD | GLU | A | 709 | 37.573 | 6.514 | 8.401 | 1.00 | 21.63 |
| 276 | OE1 | GLU | A | 709 | 36.766 | 5.660 | 8.826 | 1.00 | 23.39 |
| 277 | OE2 | GLU | A | 709 | 38.784 | 6.544 | 8.723 | 1.00 | 23.17 |
| 278 | N | ARG | A | 710 | 33.845 | 9.427 | 6.428 | 1.00 | 16.70 |
| 279 | CA | ARG | A | 710 | 33.616 | 10.869 | 6.418 | 1.00 | 17.32 |
| 280 | C | ARG | A | 710 | 32.376 | 11.230 | 7.218 | 1.00 | 17.85 |
| 281 | O | ARG | A | 710 | 32.379 | 12.156 | 8.034 | 1.00 | 17.75 |
| 282 | CB | ARG | A | 710 | 33.459 | 11.346 | 4.990 | 1.00 | 16.07 |
| 283 | CG | ARG | A | 710 | 34.659 | 11.098 | 4.137 | 1.00 | 16.18 |
| 284 | CD | ARG | A | 710 | 34.329 | 11.498 | 2.706 | 1.00 | 16.39 |
| 285 | NE | ARG | A | 710 | 35.512 | 11.535 | 1.850 | 1.00 | 15.28 |
| 286 | CZ | ARG | A | 710 | 35.587 | 12.246 | 0.733 | 1.00 | 15.30 |
| 287 | NH1 | ARG | A | 710 | 34.550 | 12.975 | 0.357 | 1.00 | 14.96 |
| 288 | NH2 | ARG | A | 710 | 36.691 | 12.242 | 0.001 | 1.00 | 14.89 |
| 289 | N | GLN | A | 711 | 31.291 | 10.516 | 6.955 | 1.00 | 18.71 |
| 290 | CA | GLN | A | 711 | 30.067 | 10.745 | 7.697 | 1.00 | 19.38 |
| 291 | C | GLN | A | 711 | 30.209 | 10.494 | 9.188 | 1.00 | 19.48 |
| 292 | O | GLN | A | 711 | 29.564 | 11.183 | 9.985 | 1.00 | 19.57 |
| 293 | CB | GLN | A | 711 | 28.908 | 9.938 | 7.127 | 1.00 | 19.79 |
| 294 | CG | GLN | A | 711 | 28.377 | 10.566 | 5.878 | 1.00 | 22.36 |
| 295 | CD | GLN | A | 711 | 27.058 | 10.010 | 5.446 | 1.00 | 23.37 |
| 296 | OE1 | GLN | A | 711 | 26.758 | 9.932 | 4.244 | 1.00 | 25.35 |
| 297 | NE2 | GLN | A | 711 | 26.228 | 9.677 | 6.410 | 1.00 | 24.52 |
| 298 | N | LEU | A | 712 | 31.043 | 9.529 | 9.571 | 1.00 | 18.76 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 299 | CA | LEU | A | 712 | 31.259 | 9.227 | 10.984 | 1.00 | 19.20 |
| 300 | C | LEU | A | 712 | 31.876 | 10.428 | 11.704 | 1.00 | 19.23 |
| 301 | O | LEU | A | 712 | 31.507 | 10.743 | 12.834 | 1.00 | 17.65 |
| 302 | CB | LEU | A | 712 | 32.163 | 8.008 | 11.157 | 1.00 | 20.55 |
| 303 | CG | LEU | A | 712 | 32.522 | 7.607 | 12.590 | 1.00 | 21.95 |
| 304 | CD1 | LEU | A | 712 | 31.288 | 7.641 | 13.484 | 1.00 | 23.43 |
| 305 | CD2 | LEU | A | 712 | 33.132 | 6.223 | 12.586 | 1.00 | 22.57 |
| 306 | N | VAL | A | 713 | 32.809 | 11.099 | 11.039 | 1.00 | 19.70 |
| 307 | CA | VAL | A | 713 | 33.427 | 12.270 | 11.619 | 1.00 | 19.68 |
| 308 | C | VAL | A | 713 | 32.328 | 13.277 | 11.990 | 1.00 | 19.53 |
| 309 | O | VAL | A | 713 | 32.325 | 13.802 | 13.086 | 1.00 | 20.00 |
| 310 | CB | VAL | A | 713 | 34.453 | 12.859 | 10.658 | 1.00 | 20.01 |
| 311 | CG1 | VAL | A | 713 | 34.722 | 14.292 | 11.001 | 1.00 | 21.16 |
| 312 | CG2 | VAL | A | 713 | 35.750 | 12.069 | 10.750 | 1.00 | 20.06 |
| 313 | N | HIS | A | 714 | 31.330 | 13.434 | 11.128 | 1.00 | 19.20 |
| 314 | CA | HIS | A | 714 | 30.215 | 14.356 | 11.358 | 1.00 | 19.47 |
| 315 | C | HIS | A | 714 | 29.183 | 13.885 | 12.383 | 1.00 | 18.83 |
| 316 | O | HIS | A | 714 | 28.497 | 14.701 | 13.005 | 1.00 | 18.73 |
| 317 | CB | HIS | A | 714 | 29.498 | 14.658 | 10.038 | 1.00 | 20.77 |
| 318 | CG | HIS | A | 714 | 30.331 | 15.410 | 9.058 | 1.00 | 21.60 |
| 319 | ND1 | HIS | A | 714 | 30.131 | 16.744 | 8.784 | 1.00 | 22.32 |
| 320 | CD2 | HIS | A | 714 | 31.369 | 15.016 | 8.283 | 1.00 | 22.31 |
| 321 | CE1 | HIS | A | 714 | 31.005 | 17.139 | 7.876 | 1.00 | 23.41 |
| 322 | NE2 | HIS | A | 714 | 31.768 | 16.113 | 7.557 | 1.00 | 23.22 |
| 323 | N | VAL | A | 715 | 29.006 | 12.572 | 12.485 | 1.00 | 18.39 |
| 324 | CA | VAL | A | 715 | 28.063 | 11.972 | 13.434 | 1.00 | 16.86 |
| 325 | C | VAL | A | 715 | 28.667 | 12.166 | 14.817 | 1.00 | 15.60 |
| 326 | O | VAL | A | 715 | 27.958 | 12.422 | 15.788 | 1.00 | 15.49 |
| 327 | CB | VAL | A | 715 | 27.869 | 10.435 | 13.134 | 1.00 | 16.78 |
| 328 | CG1 | VAL | A | 715 | 27.037 | 9.756 | 14.197 | 1.00 | 17.10 |
| 329 | CG2 | VAL | A | 715 | 27.183 | 10.259 | 11.817 | 1.00 | 17.34 |
| 330 | N | VAL | A | 716 | 29.986 | 12.077 | 14.913 | 1.00 | 15.13 |
| 331 | CA | VAL | A | 716 | 30.622 | 12.250 | 16.205 | 1.00 | 15.01 |
| 332 | C | VAL | A | 716 | 30.419 | 13.681 | 16.708 | 1.00 | 15.83 |
| 333 | O | VAL | A | 716 | 30.129 | 13.883 | 17.887 | 1.00 | 16.61 |
| 334 | CB | VAL | A | 716 | 32.136 | 11.885 | 16.158 | 1.00 | 14.93 |
| 335 | CG1 | VAL | A | 716 | 32.825 | 12.233 | 17.481 | 1.00 | 13.26 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 336 | CG2 | VAL | A | 716 | 32.310 | 10.373 | 15.870 | 1.00 | 14.26 |
| 337 | N | LYS | A | 717 | 30.544 | 14.665 | 15.816 | 1.00 | 16.59 |
| 338 | CA | LYS | A | 717 | 30.390 | 16.082 | 16.183 | 1.00 | 17.20 |
| 339 | C | LYS | A | 717 | 28.951 | 16.387 | 16.534 | 1.00 | 16.77 |
| 340 | O | LYS | A | 717 | 28.658 | 16.931 | 17.593 | 1.00 | 18.49 |
| 341 | CB | LYS | A | 717 | 30.884 | 16.974 | 15.041 | 1.00 | 18.94 |
| 342 | CG | LYS | A | 717 | 32.361 | 16.747 | 14.698 | 1.00 | 22.56 |
| 343 | CD | LYS | A | 717 | 33.245 | 16.752 | 15.978 | 1.00 | 25.34 |
| 344 | CE | LYS | A | 717 | 34.294 | 15.609 | 16.007 | 1.00 | 27.06 |
| 345 | NZ | LYS | A | 717 | 34.709 | 15.195 | 17.410 | 1.00 | 27.21 |
| 346 | N | TRP | A | 718 | 28.049 | 15.976 | 15.659 | 1.00 | 15.68 |
| 347 | CA | TRP | A | 718 | 26.618 | 16.143 | 15.868 | 1.00 | 14.61 |
| 348 | C | TRP | A | 718 | 26.200 | 15.562 | 17.261 | 1.00 | 15.34 |
| 349 | O | TRP | A | 718 | 25.659 | 16.269 | 18.124 | 1.00 | 14.55 |
| 350 | CB | TRP | A | 718 | 25.889 | 15.442 | 14.689 | 1.00 | 11.97 |
| 351 | CG | TRP | A | 718 | 24.433 | 15.266 | 14.841 | 1.00 | 9.66 |
| 352 | CD1 | TRP | A | 718 | 23.472 | 16.199 | 14.645 | 1.00 | 9.89 |
| 353 | CD2 | TRP | A | 718 | 23.757 | 14.069 | 15.254 | 1.00 | 10.28 |
| 354 | NE1 | TRP | A | 718 | 22.228 | 15.688 | 14.918 | 1.00 | 8.38 |
| 355 | CE2 | TRP | A | 718 | 22.373 | 14.371 | 15.293 | 1.00 | 9.98 |
| 356 | CE3 | TRP | A | 718 | 24.176 | 12.778 | 15.612 | 1.00 | 10.09 |
| 357 | CZ2 | TRP | A | 718 | 21.394 | 13.419 | 15.663 | 1.00 | 9.00 |
| 358 | CZ3 | TRP | A | 718 | 23.201 | 11.835 | 15.980 | 1.00 | 8.20 |
| 359 | CH2 | TRP | A | 718 | 21.835 | 12.171 | 16.004 | 1.00 | 7.32 |
| 360 | N | ALA | A | 719 | 26.468 | 14.272 | 17.464 | 1.00 | 16.10 |
| 361 | CA | ALA | A | 719 | 26.143 | 13.559 | 18.683 | 1.00 | 15.03 |
| 362 | C | ALA | A | 719 | 26.623 | 14.346 | 19.881 | 1.00 | 15.62 |
| 363 | O | ALA | A | 719 | 25.857 | 14.646 | 20.785 | 1.00 | 15.85 |
| 364 | CB | ALA | A | 719 | 26.796 | 12.184 | 18.657 | 1.00 | 13.59 |
| 365 | N | LYS | A | 720 | 27.870 | 14.781 | 19.828 | 1.00 | 17.45 |
| 366 | CA | LYS | A | 720 | 28.463 | 15.516 | 20.924 | 1.00 | 18.63 |
| 367 | C | LYS | A | 720 | 27.822 | 16.860 | 21.204 | 1.00 | 18.98 |
| 368 | O | LYS | A | 720 | 27.921 | 17.377 | 22.321 | 1.00 | 19.86 |
| 369 | CB | LYS | A | 720 | 29.970 | 15.625 | 20.715 | 1.00 | 19.81 |
| 370 | CG | LYS | A | 720 | 30.644 | 14.292 | 21.012 | 1.00 | 21.18 |
| 371 | CD | LYS | A | 720 | 32.136 | 14.334 | 20.860 | 1.00 | 23.81 |
| 372 | CE | LYS | A | 720 | 32.762 | 12.975 | 21.244 | 1.00 | 25.84 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 373 | NZ | LYS | A | 720 | 32.729 | 12.661 | 22.708 | 1.00 | 26.70 |
| 374 | N | ALA | A | 721 | 27.070 | 17.369 | 20.238 | 1.00 | 18.21 |
| 375 | CA | ALA | A | 721 | 26.406 | 18.651 | 20.382 | 1.00 | 18.10 |
| 376 | C | ALA | A | 721 | 24.941 | 18.492 | 20.675 | 1.00 | 18.80 |
| 377 | O | ALA | A | 721 | 24.192 | 19.485 | 20.660 | 1.00 | 19.16 |
| 378 | CB | ALA | A | 721 | 26.584 | 19.461 | 19.146 | 1.00 | 17.43 |
| 379 | N | LEU | A | 722 | 24.518 | 17.247 | 20.904 | 1.00 | 19.00 |
| 380 | CA | LEU | A | 722 | 23.119 | 16.912 | 21.207 | 1.00 | 19.60 |
| 381 | C | LEU | A | 722 | 22.754 | 17.362 | 22.616 | 1.00 | 20.27 |
| 382 | O | LEU | A | 722 | 23.521 | 17.125 | 23.549 | 1.00 | 21.72 |
| 383 | CB | LEU | A | 722 | 22.955 | 15.395 | 21.119 | 1.00 | 19.45 |
| 384 | CG | LEU | A | 722 | 21.855 | 14.771 | 20.271 | 1.00 | 19.62 |
| 385 | CD1 | LEU | A | 722 | 21.540 | 15.657 | 19.099 | 1.00 | 17.02 |
| 386 | CD2 | LEU | A | 722 | 22.298 | 13.382 | 19.815 | 1.00 | 17.38 |
| 387 | N | PRO | A | 723 | 21.574 | 17.992 | 22.811 | 1.00 | 20.69 |
| 388 | CA | PRO | A | 723 | 21.211 | 18.428 | 24.167 | 1.00 | 21.24 |
| 389 | C | PRO | A | 723 | 21.266 | 17.287 | 25.195 | 1.00 | 21.66 |
| 390 | O | PRO | A | 723 | 20.821 | 16.165 | 24.935 | 1.00 | 21.14 |
| 391 | CB | PRO | A | 723 | 19.767 | 18.917 | 23.997 | 1.00 | 20.40 |
| 392 | CG | PRO | A | 723 | 19.706 | 19.349 | 22.624 | 1.00 | 20.05 |
| 393 | CD | PRO | A | 723 | 20.500 | 18.317 | 21.861 | 1.00 | 20.29 |
| 394 | N | GLY | A | 724 | 21.800 | 17.588 | 26.369 | 1.00 | 22.02 |
| 395 | CA | GLY | A | 724 | 21.874 | 16.598 | 27.416 | 1.00 | 22.29 |
| 396 | C | GLY | A | 724 | 22.838 | 15.478 | 27.132 | 1.00 | 23.13 |
| 397 | O | GLY | A | 724 | 23.076 | 14.658 | 28.004 | 1.00 | 23.78 |
| 398 | N | PHE | A | 725 | 23.434 | 15.446 | 25.946 | 1.00 | 24.14 |
| 399 | CA | PHE | A | 725 | 24.360 | 14.368 | 25.610 | 1.00 | 24.24 |
| 400 | C | PHE | A | 725 | 25.505 | 14.170 | 26.582 | 1.00 | 24.85 |
| 401 | O | PHE | A | 725 | 25.873 | 13.028 | 26.863 | 1.00 | 23.79 |
| 402 | CB | PHE | A | 725 | 24.915 | 14.554 | 24.214 | 1.00 | 23.59 |
| 403 | CG | PHE | A | 725 | 25.648 | 13.353 | 23.703 | 1.00 | 23.80 |
| 404 | CD1 | PHE | A | 725 | 24.944 | 12.239 | 23.260 | 1.00 | 22.83 |
| 405 | CD2 | PHE | A | 725 | 27.046 | 13.328 | 23.675 | 1.00 | 22.40 |
| 406 | CE1 | PHE | A | 725 | 25.623 | 11.130 | 22.804 | 1.00 | 22.77 |
| 407 | CE2 | PHE | A | 725 | 27.731 | 12.226 | 23.221 | 1.00 | 21.05 |
| 408 | CZ | PHE | A | 725 | 27.025 | 11.123 | 22.784 | 1.00 | 22.31 |
| 409 | N | ARG | A | 726 | 26.083 | 15.270 | 27.070 | 1.00 | 25.97 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 410 | CA | ARG | A | 726 | 27.207 | 15.229 | 28.033 | 1.00 | 27.63 |
| 411 | C | ARG | A | 726 | 26.902 | 14.615 | 29.423 | 1.00 | 27.24 |
| 412 | O | ARG | A | 726 | 27.797 | 14.449 | 30.253 | 1.00 | 27.08 |
| 413 | CB | ARG | A | 726 | 27.831 | 16.620 | 28.204 | 1.00 | 29.27 |
| 414 | CG | ARG | A | 726 | 28.622 | 17.087 | 26.995 | 1.00 | 31.68 |
| 415 | CD | ARG | A | 726 | 29.759 | 16.141 | 26.727 | 1.00 | 34.22 |
| 416 | NE | ARG | A | 726 | 30.657 | 16.595 | 25.670 | 1.00 | 37.18 |
| 417 | CZ | ARG | A | 726 | 31.872 | 16.090 | 25.464 | 1.00 | 38.28 |
| 418 | NH1 | ARG | A | 726 | 32.635 | 16.558 | 24.486 | 1.00 | 39.44 |
| 419 | NH2 | ARG | A | 726 | 32.329 | 15.109 | 26.232 | 1.00 | 38.78 |
| 420 | N | ASN | A | 727 | 25.632 | 14.316 | 29.683 | 1.00 | 27.15 |
| 421 | CA | ASN | A | 727 | 25.244 | 13.695 | 30.938 | 1.00 | 26.29 |
| 422 | C | ASN | A | 727 | 25.750 | 12.261 | 30.934 | 1.00 | 26.17 |
| 423 | O | ASN | A | 727 | 25.992 | 11.689 | 31.994 | 1.00 | 27.30 |
| 424 | CB | ASN | A | 727 | 23.717 | 13.687 | 31.115 | 1.00 | 25.46 |
| 425 | CG | ASN | A | 727 | 23.118 | 15.085 | 31.195 | 1.00 | 24.95 |
| 426 | OD1 | ASN | A | 727 | 21.947 | 15.277 | 30.893 | 1.00 | 24.88 |
| 427 | ND2 | ASN | A | 727 | 23.909 | 16.055 | 31.628 | 1.00 | 24.54 |
| 428 | N | LEU | A | 728 | 25.895 | 11.673 | 29.749 | 1.00 | 25.64 |
| 429 | CA | LEU | A | 728 | 26.362 | 10.289 | 29.625 | 1.00 | 25.16 |
| 430 | C | LEU | A | 728 | 27.833 | 10.208 | 29.974 | 1.00 | 25.79 |
| 431 | O | LEU | A | 728 | 28.571 | 11.157 | 29.739 | 1.00 | 25.15 |
| 432 | CB | LEU | A | 728 | 26.191 | 9.759 | 28.190 | 1.00 | 22.98 |
| 433 | CG | LEU | A | 728 | 24.859 | 9.711 | 27.448 | 1.00 | 20.68 |
| 434 | CD1 | LEU | A | 728 | 25.084 | 9.133 | 26.076 | 1.00 | 19.66 |
| 435 | CD2 | LEU | A | 728 | 23.856 | 8.883 | 28.203 | 1.00 | 19.79 |
| 436 | N | HIS | A | 729 | 28.247 | 9.064 | 30.516 | 1.00 | 27.05 |
| 437 | CA | HIS | A | 729 | 29.642 | 8.808 | 30.871 | 1.00 | 28.68 |
| 438 | C | HIS | A | 729 | 30.450 | 8.772 | 29.577 | 1.00 | 29.25 |
| 439 | O | HIS | A | 729 | 30.003 | 8.182 | 28.594 | 1.00 | 29.28 |
| 440 | CB | HIS | A | 729 | 29.737 | 7.455 | 31.570 | 1.00 | 30.72 |
| 441 | CG | HIS | A | 729 | 31.132 | 7.042 | 31.943 | 1.00 | 33.13 |
| 442 | ND1 | HIS | A | 729 | 31.460 | 6.603 | 33.209 | 1.00 | 34.21 |
| 443 | CD2 | HIS | A | 729 | 32.276 | 6.978 | 31.218 | 1.00 | 33.50 |
| 444 | CE1 | HIS | A | 729 | 32.744 | 6.293 | 33.247 | 1.00 | 34.64 |
| 445 | NE2 | HIS | A | 729 | 33.263 | 6.510 | 32.049 | 1.00 | 34.52 |
| 446 | N | VAL | A | 730 | 31.681 | 9.295 | 29.625 | 1.00 | 30.21 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 447 | CA | VAL | A | 730 | 32.592 | 9.365 | 28.465 | 1.00 | 31.02 |
| 448 | C | VAL | A | 730 | 32.662 | 8.108 | 27.600 | 1.00 | 30.89 |
| 449 | O | VAL | A | 730 | 32.704 | 8.192 | 26.371 | 1.00 | 30.40 |
| 450 | CB | VAL | A | 730 | 34.036 | 9.793 | 28.898 | 1.00 | 32.08 |
| 451 | CG1 | VAL | A | 730 | 35.077 | 9.446 | 27.811 | 1.00 | 32.91 |
| 452 | CG2 | VAL | A | 730 | 34.074 | 11.284 | 29.176 | 1.00 | 31.86 |
| 453 | N | ASP | A | 731 | 32.770 | 6.956 | 28.244 | 1.00 | 30.81 |
| 454 | CA | ASP | A | 731 | 32.819 | 5.709 | 27.509 | 1.00 | 31.55 |
| 455 | C | ASP | A | 731 | 31.474 | 5.425 | 26.889 | 1.00 | 28.94 |
| 456 | O | ASP | A | 731 | 31.408 | 4.912 | 25.789 | 1.00 | 29.47 |
| 457 | CB | ASP | A | 731 | 33.244 | 4.536 | 28.410 | 1.00 | 36.23 |
| 458 | CG | ASP | A | 731 | 32.966 | 3.152 | 27.771 | 1.00 | 40.32 |
| 459 | OD1 | ASP | A | 731 | 31.837 | 2.619 | 27.974 | 1.00 | 42.21 |
| 460 | OD2 | ASP | A | 731 | 33.867 | 2.599 | 27.075 | 1.00 | 42.23 |
| 461 | N | ASP | A | 732 | 30.403 | 5.760 | 27.587 | 1.00 | 26.17 |
| 462 | CA | ASP | A | 732 | 29.079 | 5.510 | 27.057 | 1.00 | 24.53 |
| 463 | C | ASP | A | 732 | 28.770 | 6.387 | 25.875 | 1.00 | 23.96 |
| 464 | O | ASP | A | 732 | 28.030 | 5.982 | 24.981 | 1.00 | 22.56 |
| 465 | CB | ASP | A | 732 | 28.024 | 5.711 | 28.119 | 1.00 | 24.04 |
| 466 | CG | ASP | A | 732 | 28.073 | 4.654 | 29.186 | 1.00 | 23.64 |
| 467 | OD1 | ASP | A | 732 | 28.728 | 3.592 | 28.984 | 1.00 | 22.31 |
| 468 | OD2 | ASP | A | 732 | 27.444 | 4.904 | 30.231 | 1.00 | 23.89 |
| 469 | N | GLN | A | 733 | 29.288 | 7.612 | 25.920 | 1.00 | 23.56 |
| 470 | CA | GLN | A | 733 | 29.121 | 8.591 | 24.855 | 1.00 | 22.94 |
| 471 | C | GLN | A | 733 | 29.636 | 7.997 | 23.557 | 1.00 | 23.20 |
| 472 | O | GLN | A | 733 | 28.979 | 8.075 | 22.522 | 1.00 | 23.08 |
| 473 | CB | GLN | A | 733 | 29.942 | 9.847 | 25.166 | 1.00 | 22.73 |
| 474 | CG | GLN | A | 733 | 29.359 | 10.776 | 26.225 | 1.00 | 23.24 |
| 475 | CD | GLN | A | 733 | 30.208 | 12.013 | 26.480 | 1.00 | 23.27 |
| 476 | OE1 | GLN | A | 733 | 30.018 | 12.696 | 27.477 | 1.00 | 24.33 |
| 477 | NE2 | GLN | A | 733 | 31.130 | 12.316 | 25.577 | 1.00 | 23.47 |
| 478 | N | MET | A | 734 | 30.853 | 7.459 | 23.625 | 1.00 | 23.37 |
| 479 | CA | MET | A | 734 | 31.545 | 6.832 | 22.508 | 1.00 | 24.31 |
| 480 | C | MET | A | 734 | 30.902 | 5.510 | 22.077 | 1.00 | 23.31 |
| 481 | O | MET | A | 734 | 30.732 | 5.247 | 20.884 | 1.00 | 23.35 |
| 482 | CB | MET | A | 734 | 33.003 | 6.596 | 22.906 | 1.00 | 27.26 |
| 483 | CG | MET | A | 734 | 33.749 | 5.604 | 22.047 | 1.00 | 31.61 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 484 | SD | MET | A | 734 | 35.293 | 5.121 | 22.821 | 1.00 | 39.54 |
| 485 | CE | MET | A | 734 | 34.884 | 3.401 | 23.387 | 1.00 | 37.92 |
| 486 | N | ALA | A | 735 | 30.571 | 4.671 | 23.052 | 1.00 | 21.64 |
| 487 | CA | ALA | A | 735 | 29.939 | 3.390 | 22.788 | 1.00 | 20.44 |
| 488 | C | ALA | A | 735 | 28.644 | 3.570 | 22.013 | 1.00 | 19.23 |
| 489 | O | ALA | A | 735 | 28.398 | 2.905 | 21.015 | 1.00 | 19.59 |
| 490 | CB | ALA | A | 735 | 29.650 | 2.683 | 24.110 | 1.00 | 20.71 |
| 491 | N | VAL | A | 736 | 27.799 | 4.460 | 22.501 | 1.00 | 18.69 |
| 492 | CA | VAL | A | 736 | 26.516 | 4.734 | 21.877 | 1.00 | 17.76 |
| 493 | C | VAL | A | 736 | 26.673 | 5.133 | 20.389 | 1.00 | 18.16 |
| 494 | O | VAL | A | 736 | 25.962 | 4.614 | 19.512 | 1.00 | 17.42 |
| 495 | CB | VAL | A | 736 | 25.742 | 5.771 | 22.760 | 1.00 | 18.30 |
| 496 | CG1 | VAL | A | 736 | 25.373 | 6.998 | 22.011 | 1.00 | 17.05 |
| 497 | CG2 | VAL | A | 736 | 24.544 | 5.118 | 23.420 | 1.00 | 17.25 |
| 498 | N | ILE | A | 737 | 27.658 | 5.985 | 20.096 | 1.00 | 17.60 |
| 499 | CA | ILE | A | 737 | 27.914 | 6.429 | 18.724 | 1.00 | 16.31 |
| 500 | C | ILE | A | 737 | 28.352 | 5.216 | 17.904 | 1.00 | 16.74 |
| 501 | O | ILE | A | 737 | 27.853 | 4.982 | 16.812 | 1.00 | 16.09 |
| 502 | CB | ILE | A | 737 | 29.046 | 7.497 | 18.683 | 1.00 | 14.71 |
| 503 | CG1 | ILE | A | 737 | 28.602 | 8.819 | 19.325 | 1.00 | 15.11 |
| 504 | CG2 | ILE | A | 737 | 29.476 | 7.772 | 17.272 | 1.00 | 14.05 |
| 505 | CD1 | ILE | A | 737 | 29.769 | 9.804 | 19.618 | 1.00 | 12.32 |
| 506 | N | GLN | A | 738 | 29.281 | 4.451 | 18.468 | 1.00 | 18.15 |
| 507 | CA | GLN | A | 738 | 29.850 | 3.260 | 17.845 | 1.00 | 17.97 |
| 508 | C | GLN | A | 738 | 28.904 | 2.111 | 17.548 | 1.00 | 16.76 |
| 509 | O | GLN | A | 738 | 29.249 | 1.226 | 16.788 | 1.00 | 16.47 |
| 510 | CB | GLN | A | 738 | 30.960 | 2.715 | 18.713 | 1.00 | 20.53 |
| 511 | CG | GLN | A | 738 | 32.278 | 3.394 | 18.568 | 1.00 | 23.88 |
| 512 | CD | GLN | A | 738 | 33.306 | 2.726 | 19.439 | 1.00 | 26.69 |
| 513 | OE1 | GLN | A | 738 | 33.027 | 2.390 | 20.593 | 1.00 | 29.30 |
| 514 | NE2 | GLN | A | 738 | 34.483 | 2.475 | 18.887 | 1.00 | 28.53 |
| 515 | N | TYR | A | 739 | 27.792 | 2.029 | 18.260 | 1.00 | 16.39 |
| 516 | CA | TYR | A | 739 | 26.819 | 0.983 | 17.995 | 1.00 | 16.41 |
| 517 | C | TYR | A | 739 | 25.721 | 1.527 | 17.100 | 1.00 | 16.45 |
| 518 | O | TYR | A | 739 | 25.138 | 0.793 | 16.295 | 1.00 | 17.63 |
| 519 | CB | TYR | A | 739 | 26.174 | 0.448 | 19.285 | 1.00 | 15.99 |
| 520 | CG | TYR | A | 739 | 27.130 | -0.115 | 20.313 | 1.00 | 15.68 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 521 | CD1 | TYR | A | 739 | 28.251 | -0.852 | 19.950 | 1.00 | 15.41 |
| 522 | CD2 | TYR | A | 739 | 26.925 | 0.131 | 21.656 | 1.00 | 16.49 |
| 523 | CE1 | TYR | A | 739 | 29.151 | -1.317 | 20.915 | 1.00 | 16.17 |
| 524 | CE2 | TYR | A | 739 | 27.817 | -0.321 | 22.624 | 1.00 | 17.09 |
| 525 | CZ | TYR | A | 739 | 28.921 | -1.040 | 22.253 | 1.00 | 16.83 |
| 526 | OH | TYR | A | 739 | 29.787 | -1.435 | 23.256 | 1.00 | 18.24 |
| 527 | N | SER | A | 740 | 25.453 | 2.822 | 17.195 | 1.00 | 15.61 |
| 528 | CA | SER | A | 740 | 24.384 | 3.404 | 16.403 | 1.00 | 15.61 |
| 529 | C | SER | A | 740 | 24.697 | 4.054 | 15.060 | 1.00 | 14.87 |
| 530 | O | SER | A | 740 | 23.778 | 4.451 | 14.376 | 1.00 | 15.75 |
| 531 | CB | SER | A | 740 | 23.619 | 4.403 | 17.252 | 1.00 | 15.49 |
| 532 | OG | SER | A | 740 | 24.512 | 5.421 | 17.682 | 1.00 | 18.39 |
| 533 | N | TRP | A | 741 | 25.948 | 4.188 | 14.659 | 1.00 | 14.33 |
| 534 | CA | TRP | A | 741 | 26.202 | 4.835 | 13.382 | 1.00 | 14.83 |
| 535 | C | TRP | A | 741 | 25.471 | 4.246 | 12.166 | 1.00 | 15.12 |
| 536 | O | TRP | A | 741 | 24.902 | 4.995 | 11.376 | 1.00 | 15.56 |
| 537 | CB | TRP | A | 741 | 27.706 | 4.997 | 13.113 | 1.00 | 15.35 |
| 538 | CG | TRP | A | 741 | 28.465 | 3.720 | 13.000 | 1.00 | 17.05 |
| 539 | CD1 | TRP | A | 741 | 28.995 | 3.016 | 14.020 | 1.00 | 17.12 |
| 540 | CD2 | TRP | A | 741 | 28.765 | 2.987 | 11.800 | 1.00 | 17.91 |
| 541 | NE1 | TRP | A | 741 | 29.592 | 1.878 | 13.551 | 1.00 | 19.60 |
| 542 | CE2 | TRP | A | 741 | 29.467 | 1.834 | 12.190 | 1.00 | 18.98 |
| 543 | CE3 | TRP | A | 741 | 28.505 | 3.193 | 10.434 | 1.00 | 18.66 |
| 544 | CZ2 | TRP | A | 741 | 29.915 | 0.876 | 11.266 | 1.00 | 18.79 |
| 545 | CZ3 | TRP | A | 741 | 28.949 | 2.240 | 9.509 | 1.00 | 17.91 |
| 546 | CH2 | TRP | A | 741 | 29.644 | 1.098 | 9.934 | 1.00 | 18.35 |
| 547 | N | MET | A | 742 | 25.391 | 2.920 | 12.034 | 1.00 | 14.55 |
| 548 | CA | MET | A | 742 | 24.723 | 2.339 | 10.870 | 1.00 | 12.90 |
| 549 | C | MET | A | 742 | 23.290 | 2.763 | 10.699 | 1.00 | 12.47 |
| 550 | O | MET | A | 742 | 22.886 | 3.153 | 9.610 | 1.00 | 13.31 |
| 551 | CB | MET | A | 742 | 24.785 | 0.815 | 10.866 | 1.00 | 13.16 |
| 552 | CG | MET | A | 742 | 24.219 | 0.185 | 9.597 | 1.00 | 12.17 |
| 553 | SD | MET | A | 742 | 25.353 | 0.336 | 8.263 | 1.00 | 15.00 |
| 554 | CE | MET | A | 742 | 26.462 | -0.994 | 8.639 | 1.00 | 13.52 |
| 555 | N | GLY | A | 743 | 22.497 | 2.656 | 11.748 | 1.00 | 12.12 |
| 556 | CA | GLY | A | 743 | 21.102 | 3.057 | 11.663 | 1.00 | 11.81 |
| 557 | C | GLY | A | 743 | 20.947 | 4.558 | 11.452 | 1.00 | 12.08 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 558 | O | GLY | A | 743 | 20.022 | 5.009 | 10.768 | 1.00 | 11.88 |
| 559 | N | LEU | A | 744 | 21.835 | 5.336 | 12.070 | 1.00 | 12.77 |
| 560 | CA | LEU | A | 744 | 21.817 | 6.797 | 11.972 | 1.00 | 12.22 |
| 561 | C | LEU | A | 744 | 22.087 | 7.258 | 10.563 | 1.00 | 12.20 |
| 562 | O | LEU | A | 744 | 21.424 | 8.173 | 10.080 | 1.00 | 14.22 |
| 563 | CB | LEU | A | 744 | 22.884 | 7.418 | 12.888 | 1.00 | 11.70 |
| 564 | CG | LEU | A | 744 | 22.702 | 7.481 | 14.399 | 1.00 | 9.95 |
| 565 | CD1 | LEU | A | 744 | 23.967 | 8.075 | 14.954 | 1.00 | 9.77 |
| 566 | CD2 | LEU | A | 744 | 21.516 | 8.341 | 14.799 | 1.00 | 9.16 |
| 567 | N | MET | A | 745 | 23.083 | 6.651 | 9.921 | 1.00 | 12.34 |
| 568 | CA | MET | A | 745 | 23.466 | 6.991 | 8.541 | 1.00 | 11.39 |
| 569 | C | MET | A | 745 | 22.462 | 6.498 | 7.508 | 1.00 | 12.29 |
| 570 | O | MET | A | 745 | 22.234 | 7.155 | 6.495 | 1.00 | 10.62 |
| 571 | CB | MET | A | 745 | 24.839 | 6.427 | 8.191 | 1.00 | 10.75 |
| 572 | CG | MET | A | 745 | 25.961 | 6.948 | 9.076 | 1.00 | 8.86 |
| 573 | SD | MET | A | 745 | 27.509 | 6.429 | 8.487 | 1.00 | 11.97 |
| 574 | CE | MET | A | 745 | 28.579 | 6.939 | 9.717 | 1.00 | 9.84 |
| 575 | N | VAL | A | 746 | 21.855 | 5.342 | 7.793 | 1.00 | 12.05 |
| 576 | CA | VAL | A | 746 | 20.874 | 4.733 | 6.934 | 1.00 | 11.50 |
| 577 | C | VAL | A | 746 | 19.605 | 5.565 | 6.942 | 1.00 | 12.13 |
| 578 | O | VAL | A | 746 | 19.000 | 5.792 | 5.907 | 1.00 | 12.72 |
| 579 | CB | VAL | A | 746 | 20.524 | 3.315 | 7.426 | 1.00 | 11.19 |
| 580 | CG1 | VAL | A | 746 | 19.245 | 2.852 | 6.811 | 1.00 | 10.17 |
| 581 | CG2 | VAL | A | 746 | 21.615 | 2.355 | 7.095 | 1.00 | 9.64 |
| 582 | N | PHE | A | 747 | 19.227 | 6.051 | 8.117 | 1.00 | 12.64 |
| 583 | CA | PHE | A | 747 | 18.014 | 6.857 | 8.304 | 1.00 | 12.63 |
| 584 | C | PHE | A | 747 | 18.137 | 8.241 | 7.621 | 1.00 | 13.26 |
| 585 | O | PHE | A | 747 | 17.178 | 8.751 | 7.042 | 1.00 | 13.81 |
| 586 | CB | PHE | A | 747 | 17.763 | 7.031 | 9.800 | 1.00 | 11.19 |
| 587 | CG | PHE | A | 747 | 16.411 | 7.542 | 10.126 | 1.00 | 10.00 |
| 588 | CD1 | PHE | A | 747 | 15.286 | 6.780 | 9.847 | 1.00 | 9.30 |
| 589 | CD2 | PHE | A | 747 | 16.253 | 8.798 | 10.700 | 1.00 | 7.79 |
| 590 | CE1 | PHE | A | 747 | 14.008 | 7.260 | 10.136 | 1.00 | 8.30 |
| 591 | CE2 | PHE | A | 747 | 14.996 | 9.293 | 10.993 | 1.00 | 6.75 |
| 592 | CZ | PHE | A | 747 | 13.867 | 8.524 | 10.707 | 1.00 | 8.21 |
| 593 | N | ALA | A | 748 | 19.298 | 8.873 | 7.740 | 1.00 | 12.46 |
| 594 | CA | ALA | A | 748 | 19.513 | 10.172 | 7.119 | 1.00 | 12.97 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 595 | C | ALA | A | 748 | 19.640 | 9.988 | 5.635 | 1.00 | 13.78 |
| 596 | O | ALA | A | 748 | 19.226 | 10.850 | 4.882 | 1.00 | 14.44 |
| 597 | CB | ALA | A | 748 | 20.749 | 10.808 | 7.648 | 1.00 | 11.50 |
| 598 | N | MET | A | 749 | 20.209 | 8.864 | 5.204 | 1.00 | 14.54 |
| 599 | CA | MET | A | 749 | 20.381 | 8.578 | 3.782 | 1.00 | 14.78 |
| 600 | C | MET | A | 749 | 19.023 | 8.390 | 3.142 | 1.00 | 15.85 |
| 601 | O | MET | A | 749 | 18.808 | 8.780 | 1.990 | 1.00 | 17.51 |
| 602 | CB | MET | A | 749 | 21.241 | 7.331 | 3.607 | 1.00 | 15.28 |
| 603 | CG | MET | A | 749 | 21.622 | 6.945 | 2.199 | 1.00 | 15.33 |
| 604 | SD | MET | A | 749 | 20.315 | 6.246 | 1.193 | 1.00 | 18.79 |
| 605 | CE | MET | A | 749 | 20.226 | 4.627 | 1.835 | 1.00 | 18.82 |
| 606 | N | GLY | A | 750 | 18.088 | 7.829 | 3.895 | 1.00 | 16.02 |
| 607 | CA | GLY | A | 750 | 16.748 | 7.618 | 3.384 | 1.00 | 16.34 |
| 608 | C | GLY | A | 750 | 16.057 | 8.956 | 3.225 | 1.00 | 17.79 |
| 609 | O | GLY | A | 750 | 15.263 | 9.135 | 2.289 | 1.00 | 19.00 |
| 610 | N | TRP | A | 751 | 16.361 | 9.897 | 4.121 | 1.00 | 17.36 |
| 611 | CA | TRP | A | 751 | 15.778 | 11.241 | 4.091 | 1.00 | 17.99 |
| 612 | C | TRP | A | 751 | 16.266 | 11.995 | 2.857 | 1.00 | 18.41 |
| 613 | O | TRP | A | 751 | 15.457 | 12.558 | 2.124 | 1.00 | 20.13 |
| 614 | CB | TRP | A | 751 | 16.108 | 12.026 | 5.366 | 1.00 | 16.08 |
| 615 | CG | TRP | A | 751 | 15.528 | 13.458 | 5.416 | 1.00 | 14.99 |
| 616 | CD1 | TRP | A | 751 | 16.225 | 14.636 | 5.364 | 1.00 | 13.27 |
| 617 | CD2 | TRP | A | 751 | 14.151 | 13.821 | 5.617 | 1.00 | 13.68 |
| 618 | NE1 | TRP | A | 751 | 15.375 | 15.705 | 5.538 | 1.00 | 12.27 |
| 619 | CE2 | TRP | A | 751 | 14.099 | 15.230 | 5.697 | 1.00 | 12.98 |
| 620 | CE3 | TRP | A | 751 | 12.967 | 13.090 | 5.743 | 1.00 | 14.14 |
| 621 | CZ2 | TRP | A | 751 | 12.907 | 15.926 | 5.899 | 1.00 | 14.74 |
| 622 | CZ3 | TRP | A | 751 | 11.775 | 13.780 | 5.942 | 1.00 | 14.63 |
| 623 | CH2 | TRP | A | 751 | 11.756 | 15.188 | 6.020 | 1.00 | 14.82 |
| 624 | N | ARG | A | 752 | 17.569 | 11.971 | 2.607 | 1.00 | 19.13 |
| 625 | CA | ARG | A | 752 | 18.150 | 12.616 | 1.431 | 1.00 | 19.06 |
| 626 | C | ARG | A | 752 | 17.572 | 12.077 | 0.138 | 1.00 | 20.27 |
| 627 | O | ARG | A | 752 | 17.392 | 12.815 | -0.828 | 1.00 | 20.66 |
| 628 | CB | ARG | A | 752 | 19.644 | 12.380 | 1.389 | 1.00 | 18.53 |
| 629 | CG | ARG | A | 752 | 20.370 | 12.908 | 2.567 | 1.00 | 18.25 |
| 630 | CD | ARG | A | 752 | 21.870 | 12.901 | 2.317 | 1.00 | 17.24 |
| 631 | NE | ARG | A | 752 | 22.467 | 11.573 | 2.298 | 1.00 | 14.94 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 632 | CZ | ARG | A | 752 | 22.976 | 10.973 | 3.370 | 1.00 | 14.90 |
| 633 | NH1 | ARG | A | 752 | 22.928 | 11.561 | 4.554 | 1.00 | 14.75 |
| 634 | NH2 | ARG | A | 752 | 23.684 | 9.864 | 3.240 | 1.00 | 13.87 |
| 635 | N | SER | A | 753 | 17.391 | 10.761 | 0.083 | 1.00 | 22.00 |
| 636 | CA | SER | A | 753 | 16.823 | 10.099 | -1.093 | 1.00 | 22.25 |
| 637 | C | SER | A | 753 | 15.434 | 10.635 | -1.289 | 1.00 | 23.31 |
| 638 | O | SER | A | 753 | 14.978 | 10.803 | -2.409 | 1.00 | 23.88 |
| 639 | CB | SER | A | 753 | 16.716 | 8.590 | -0.879 | 1.00 | 20.25 |
| 640 | OG | SER | A | 753 | 17.988 | 8.027 | -0.687 | 1.00 | 19.78 |
| 641 | N | PHE | A | 754 | 14.762 | 10.870 | -0.173 | 1.00 | 24.76 |
| 642 | CA | PHE | A | 754 | 13.405 | 11.375 | -0.156 | 1.00 | 26.45 |
| 643 | C | PHE | A | 754 | 13.239 | 12.818 | -0.630 | 1.00 | 27.47 |
| 644 | O | PHE | A | 754 | 12.543 | 13.100 | -1.614 | 1.00 | 26.87 |
| 645 | CB | PHE | A | 754 | 12.835 | 11.243 | 1.245 | 1.00 | 26.43 |
| 646 | CG | PHE | A | 754 | 11.447 | 11.765 | 1.364 | 1.00 | 28.06 |
| 647 | CD1 | PHE | A | 754 | 10.407 | 11.168 | 0.654 | 1.00 | 28.69 |
| 648 | CD2 | PHE | A | 754 | 11.184 | 12.895 | 2.118 | 1.00 | 27.96 |
| 649 | CE1 | PHE | A | 754 | 9.126 | 11.703 | 0.687 | 1.00 | 29.47 |
| 650 | CE2 | PHE | A | 754 | 9.901 | 13.442 | 2.160 | 1.00 | 28.93 |
| 651 | CZ | PHE | A | 754 | 8.876 | 12.849 | 1.445 | 1.00 | 29.47 |
| 652 | N | THR | A | 755 | 13.823 | 13.732 | 0.125 | 1.00 | 29.01 |
| 653 | CA | THR | A | 755 | 13.725 | 15.134 | -0.190 | 1.00 | 30.83 |
| 654 | C | THR | A | 755 | 14.317 | 15.460 | -1.552 | 1.00 | 32.57 |
| 655 | O | THR | A | 755 | 13.841 | 16.358 | -2.234 | 1.00 | 33.24 |
| 656 | CB | THR | A | 755 | 14.345 | 15.972 | 0.918 | 1.00 | 29.71 |
| 657 | OG1 | THR | A | 755 | 15.669 | 15.524 | 1.183 | 1.00 | 28.99 |
| 658 | CG2 | THR | A | 755 | 13.553 | 15.796 | 2.164 | 1.00 | 29.63 |
| 659 | N | ASN | A | 756 | 15.262 | 14.639 | -1.991 | 1.00 | 34.71 |
| 660 | CA | ASN | A | 756 | 15.920 | 14.842 | -3.273 | 1.00 | 36.48 |
| 661 | C | ASN | A | 756 | 15.360 | 14.065 | -4.457 | 1.00 | 37.88 |
| 662 | O | ASN | A | 756 | 14.684 | 14.628 | -5.313 | 1.00 | 39.57 |
| 663 | CB | ASN | A | 756 | 17.417 | 14.562 | -3.149 | 1.00 | 36.89 |
| 664 | CG | ASN | A | 756 | 18.137 | 15.616 | -2.344 | 1.00 | 37.02 |
| 665 | OD1 | ASN | A | 756 | 17.563 | 16.237 | -1.456 | 1.00 | 39.11 |
| 666 | ND2 | ASN | A | 756 | 19.392 | 15.844 | -2.668 | 1.00 | 37.24 |
| 667 | N | VAL | A | 757 | 15.654 | 12.773 | -4.518 | 1.00 | 38.99 |
| 668 | CA | VAL | A | 757 | 15.210 | 11.948 | -5.633 | 1.00 | 39.74 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 669 | C | VAL | A | 757 | 13.835 | 11.308 | -5.456 | 1.00 | 39.95 |
| 670 | O | VAL | A | 757 | 13.501 | 10.335 | -6.134 | 1.00 | 40.19 |
| 671 | CB | VAL | A | 757 | 16.274 | 10.869 | -5.971 | 1.00 | 39.96 |
| 672 | CG1 | VAL | A | 757 | 17.639 | 11.540 | -6.170 | 1.00 | 40.00 |
| 673 | CG2 | VAL | A | 757 | 16.354 | 9.819 | -4.871 | 1.00 | 39.28 |
| 674 | N | ASN | A | 758 | 13.037 | 11.874 | -4.559 | 1.00 | 40.46 |
| 675 | CA | ASN | A | 758 | 11.699 | 11.374 | -4.265 | 1.00 | 41.28 |
| 676 | C | ASN | A | 758 | 11.622 | 9.858 | -4.100 | 1.00 | 40.91 |
| 677 | O | ASN | A | 758 | 10.592 | 9.229 | -4.404 | 1.00 | 40.73 |
| 678 | CB | ASN | A | 758 | 10.678 | 11.894 | -5.288 | 1.00 | 43.82 |
| 679 | CG | ASN | A | 758 | 10.257 | 13.331 | -5.005 | 1.00 | 44.84 |
| 680 | OD1 | ASN | A | 758 | 11.097 | 14.199 | -4.764 | 1.00 | 46.40 |
| 681 | ND2 | ASN | A | 758 | 8.953 | 13.576 | -4.987 | 1.00 | 45.71 |
| 682 | N | SER | A | 759 | 12.733 | 9.298 | -3.612 | 1.00 | 40.04 |
| 683 | CA | SER | A | 759 | 12.891 | 7.877 | -3.326 | 1.00 | 38.71 |
| 684 | C | SER | A | 759 | 13.027 | 6.921 | -4.532 | 1.00 | 39.24 |
| 685 | O | SER | A | 759 | 12.833 | 5.711 | -4.382 | 1.00 | 39.20 |
| 686 | CB | SER | A | 759 | 11.763 | 7.415 | -2.395 | 1.00 | 37.53 |
| 687 | OG | SER | A | 759 | 11.496 | 8.369 | -1.378 | 1.00 | 34.26 |
| 688 | N | ARG | A | 760 | 13.409 | 7.438 | -5.704 | 1.00 | 39.12 |
| 689 | CA | ARG | A | 760 | 13.564 | 6.589 | -6.892 | 1.00 | 38.62 |
| 690 | C | ARG | A | 760 | 14.897 | 5.840 | -6.876 | 1.00 | 36.88 |
| 691 | O | ARG | A | 760 | 15.024 | 4.741 | -7.426 | 1.00 | 37.48 |
| 692 | CB | ARG | A | 760 | 13.451 | 7.422 | -8.171 | 1.00 | 40.63 |
| 693 | CG | ARG | A | 760 | 13.598 | 6.577 | -9.444 | 1.00 | 44.41 |
| 694 | CD | ARG | A | 760 | 13.903 | 7.394 | -10.715 | 1.00 | 46.97 |
| 695 | NE | ARG | A | 760 | 14.534 | 6.544 | -11.729 | 1.00 | 48.86 |
| 696 | CZ | ARG | A | 760 | 13.875 | 5.797 | -12.614 | 1.00 | 49.74 |
| 697 | NH1 | ARG | A | 760 | 12.542 | 5.795 | -12.649 | 1.00 | 50.04 |
| 698 | NH2 | ARG | A | 760 | 14.553 | 4.969 | -13.398 | 1.00 | 49.46 |
| 699 | N | MET | A | 761 | 15.902 | 6.466 | -6.275 | 1.00 | 34.87 |
| 700 | CA | MET | A | 761 | 17.238 | 5.890 | -6.159 | 1.00 | 32.21 |
| 701 | C | MET | A | 761 | 17.738 | 6.242 | -4.751 | 1.00 | 29.46 |
| 702 | O | MET | A | 761 | 17.144 | 7.080 | -4.075 | 1.00 | 28.57 |
| 703 | CB | MET | A | 761 | 18.171 | 6.510 | -7.194 | 1.00 | 33.77 |
| 704 | CG | MET | A | 761 | 17.588 | 6.682 | -8.571 | 1.00 | 36.10 |
| 705 | SD | MET | A | 761 | 18.859 | 7.115 | -9.788 | 1.00 | 40.36 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 706 | CE | MET | A | 761 | 18.737 | 8.904 | -9.809 | 1.00 | 38.10 |
| 707 | N | LEU | A | 762 | 18.837 | 5.635 | -4.319 | 1.00 | 26.78 |
| 708 | CA | LEU | A | 762 | 19.382 | 5.905 | -2.992 | 1.00 | 24.13 |
| 709 | C | LEU | A | 762 | 20.458 | 6.968 | -3.040 | 1.00 | 23.01 |
| 710 | O | LEU | A | 762 | 21.537 | 6.726 | -3.548 | 1.00 | 22.65 |
| 711 | CB | LEU | A | 762 | 19.956 | 4.637 | -2.393 | 1.00 | 24.05 |
| 712 | CG | LEU | A | 762 | 18.957 | 3.502 | -2.272 | 1.00 | 23.69 |
| 713 | CD1 | LEU | A | 762 | 19.615 | 2.272 | -1.632 | 1.00 | 23.99 |
| 714 | CD2 | LEU | A | 762 | 17.788 | 4.011 | -1.439 | 1.00 | 24.34 |
| 715 | N | TYR | A | 763 | 20.162 | 8.132 | -2.475 | 1.00 | 22.03 |
| 716 | CA | TYR | A | 763 | 21.066 | 9.273 | -2.450 | 1.00 | 20.69 |
| 717 | C | TYR | A | 763 | 22.068 | 9.173 | -1.323 | 1.00 | 18.78 |
| 718 | O | TYR | A | 763 | 21.910 | 9.828 | -0.304 | 1.00 | 17.73 |
| 719 | CB | TYR | A | 763 | 20.250 | 10.540 | -2.266 | 1.00 | 23.12 |
| 720 | CG | TYR | A | 763 | 20.946 | 11.782 | -2.730 | 1.00 | 25.58 |
| 721 | CD1 | TYR | A | 763 | 20.841 | 12.187 | -4.052 | 1.00 | 26.87 |
| 722 | CD2 | TYR | A | 763 | 21.662 | 12.590 | -1.841 | 1.00 | 26.77 |
| 723 | CE1 | TYR | A | 763 | 21.416 | 13.373 | -4.492 | 1.00 | 28.03 |
| 724 | CE2 | TYR | A | 763 | 22.247 | 13.789 | -2.272 | 1.00 | 28.35 |
| 725 | CZ | TYR | A | 763 | 22.107 | 14.172 | -3.604 | 1.00 | 28.85 |
| 726 | OH | TYR | A | 763 | 22.595 | 15.379 | -4.047 | 1.00 | 30.59 |
| 727 | N | PHE | A | 764 | 23.128 | 8.401 | -1.538 | 1.00 | 17.33 |
| 728 | CA | PHE | A | 764 | 24.152 | 8.191 | -0.533 | 1.00 | 16.94 |
| 729 | C | PHE | A | 764 | 24.964 | 9.441 | -0.375 | 1.00 | 17.39 |
| 730 | O | PHE | A | 764 | 25.379 | 9.797 | 0.734 | 1.00 | 17.28 |
| 731 | CB | PHE | A | 764 | 25.086 | 7.078 | -0.956 | 1.00 | 15.91 |
| 732 | CG | PHE | A | 764 | 24.505 | 5.724 | -0.807 | 1.00 | 16.79 |
| 733 | CD1 | PHE | A | 764 | 24.211 | 4.961 | -1.908 | 1.00 | 16.06 |
| 734 | CD2 | PHE | A | 764 | 24.267 | 5.205 | 0.450 | 1.00 | 16.83 |
| 735 | CE1 | PHE | A | 764 | 23.691 | 3.692 | -1.756 | 1.00 | 18.06 |
| 736 | CE2 | PHE | A | 764 | 23.748 | 3.941 | 0.606 | 1.00 | 18.27 |
| 737 | CZ | PHE | A | 764 | 23.458 | 3.176 | -0.496 | 1.00 | 17.80 |
| 738 | N | ALA | A | 765 | 25.224 | 10.084 | -1.503 | 1.00 | 17.00 |
| 739 | CA | ALA | A | 765 | 26.013 | 11.292 | -1.525 | 1.00 | 16.32 |
| 740 | C | ALA | A | 765 | 25.674 | 11.913 | -2.841 | 1.00 | 16.66 |
| 741 | O | ALA | A | 765 | 25.051 | 11.267 | -3.675 | 1.00 | 16.71 |
| 742 | CB | ALA | A | 765 | 27.479 | 10.957 | -1.460 | 1.00 | 16.17 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 743 | N | PRO | A | 766 | 26.016 | 13.196 | -3.032 | 1.00 | 17.18 |
| 744 | CA | PRO | A | 766 | 25.703 | 13.846 | -4.311 | 1.00 | 17.49 |
| 745 | C | PRO | A | 766 | 26.429 | 13.161 | -5.481 | 1.00 | 17.65 |
| 746 | O | PRO | A | 766 | 25.923 | 13.099 | -6.598 | 1.00 | 17.73 |
| 747 | CB | PRO | A | 766 | 26.183 | 15.277 | -4.077 | 1.00 | 17.30 |
| 748 | CG | PRO | A | 766 | 26.002 | 15.451 | -2.608 | 1.00 | 17.07 |
| 749 | CD | PRO | A | 766 | 26.544 | 14.169 | -2.064 | 1.00 | 15.41 |
| 750 | N | ASP | A | 767 | 27.578 | 12.569 | -5.166 | 1.00 | 18.27 |
| 751 | CA | ASP | A | 767 | 28.416 | 11.850 | -6.115 | 1.00 | 18.49 |
| 752 | C | ASP | A | 767 | 28.330 | 10.317 | -5.981 | 1.00 | 17.79 |
| 753 | O | ASP | A | 767 | 29.191 | 9.594 | -6.476 | 1.00 | 18.69 |
| 754 | CB | ASP | A | 767 | 29.877 | 12.312 | -5.955 | 1.00 | 18.71 |
| 755 | CG | ASP | A | 767 | 30.413 | 12.135 | -4.525 | 1.00 | 19.47 |
| 756 | OD1 | ASP | A | 767 | 29.611 | 12.038 | -3.569 | 1.00 | 20.31 |
| 757 | OD2 | ASP | A | 767 | 31.650 | 12.102 | -4.348 | 1.00 | 19.04 |
| 758 | N | LEU | A | 768 | 27.334 | 9.820 | -5.267 | 1.00 | 18.04 |
| 759 | CA | LEU | A | 768 | 27.164 | 8.379 | -5.110 | 1.00 | 18.20 |
| 760 | C | LEU | A | 768 | 25.690 | 8.129 | -4.930 | 1.00 | 18.58 |
| 761 | O | LEU | A | 768 | 25.184 | 8.068 | -3.812 | 1.00 | 17.79 |
| 762 | CB | LEU | A | 768 | 27.955 | 7.809 | -3.914 | 1.00 | 17.47 |
| 763 | CG | LEU | A | 768 | 28.032 | 6.263 | -3.786 | 1.00 | 16.12 |
| 764 | CD1 | LEU | A | 768 | 28.641 | 5.671 | -5.047 | 1.00 | 14.30 |
| 765 | CD2 | LEU | A | 768 | 28.850 | 5.846 | -2.563 | 1.00 | 15.17 |
| 766 | N | VAL | A | 769 | 24.979 | 8.156 | -6.048 | 1.00 | 19.79 |
| 767 | CA | VAL | A | 769 | 23.553 | 7.895 | -6.035 | 1.00 | 20.42 |
| 768 | C | VAL | A | 769 | 23.373 | 6.609 | -6.852 | 1.00 | 20.73 |
| 769 | O | VAL | A | 769 | 23.873 | 6.467 | -7.961 | 1.00 | 22.43 |
| 770 | CB | VAL | A | 769 | 22.709 | 9.142 | -6.447 | 1.00 | 19.95 |
| 771 | CG1 | VAL | A | 769 | 23.571 | 10.190 | -7.096 | 1.00 | 20.70 |
| 772 | CG2 | VAL | A | 769 | 21.537 | 8.757 | -7.277 | 1.00 | 19.19 |
| 773 | N | PHE | A | 770 | 22.871 | 5.604 | -6.157 | 1.00 | 19.70 |
| 774 | CA | PHE | A | 770 | 22.683 | 4.277 | -6.681 | 1.00 | 19.29 |
| 775 | C | PHE | A | 770 | 21.425 | 4.134 | -7.473 | 1.00 | 19.41 |
| 776 | O | PHE | A | 770 | 20.367 | 4.583 | -7.054 | 1.00 | 19.74 |
| 777 | CB | PHE | A | 770 | 22.596 | 3.263 | -5.503 | 1.00 | 18.44 |
| 778 | CG | PHE | A | 770 | 23.930 | 2.757 | -4.996 | 1.00 | 16.41 |
| 779 | CD1 | PHE | A | 770 | 25.079 | 3.546 | -5.053 | 1.00 | 14.52 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 780 | CD2 | PHE | A | 770 | 24.025 | 1.468 | -4.459 | 1.00 | 14.96 |
| 781 | CE1 | PHE | A | 770 | 26.291 | 3.070 | -4.588 | 1.00 | 13.39 |
| 782 | CE2 | PHE | A | 770 | 25.243 | 0.979 | -3.983 | 1.00 | 13.90 |
| 783 | CZ | PHE | A | 770 | 26.383 | 1.786 | -4.050 | 1.00 | 13.96 |
| 784 | N | ASN | A | 771 | 21.534 | 3.474 | -8.611 | 1.00 | 20.33 |
| 785 | CA | ASN | A | 771 | 20.363 | 3.157 | -9.410 | 1.00 | 20.23 |
| 786 | C | ASN | A | 771 | 20.278 | 1.636 | -9.292 | 1.00 | 21.01 |
| 787 | O | ASN | A | 771 | 21.129 | 1.013 | -8.648 | 1.00 | 20.52 |
| 788 | CB | ASN | A | 771 | 20.524 | 3.593 | -10.864 | 1.00 | 19.33 |
| 789 | CG | ASN | A | 771 | 21.883 | 3.304 | -11.403 | 1.00 | 18.89 |
| 790 | OD1 | ASN | A | 771 | 22.574 | 2.408 | -10.942 | 1.00 | 19.51 |
| 791 | ND2 | ASN | A | 771 | 22.289 | 4.069 | -12.382 | 1.00 | 19.02 |
| 792 | N | GLU | A | 772 | 19.258 | 1.043 | -9.898 | 1.00 | 22.23 |
| 793 | CA | GLU | A | 772 | 19.056 | -0.393 | -9.841 | 1.00 | 22.51 |
| 794 | C | GLU | A | 772 | 20.282 | -1.102 | -10.303 | 1.00 | 22.96 |
| 795 | O | GLU | A | 772 | 20.631 | -2.148 | -9.785 | 1.00 | 23.89 |
| 796 | CB | GLU | A | 772 | 17.888 | -0.799 | -10.711 | 1.00 | 23.17 |
| 797 | CG | GLU | A | 772 | 16.562 | -0.455 | -10.099 | 1.00 | 24.81 |
| 798 | CD | GLU | A | 772 | 15.761 | -1.672 | -9.724 | 1.00 | 25.41 |
| 799 | OE1 | GLU | A | 772 | 14.624 | -1.488 | -9.252 | 1.00 | 25.33 |
| 800 | OE2 | GLU | A | 772 | 16.265 | -2.803 | -9.913 | 1.00 | 26.23 |
| 801 | N | TYR | A | 773 | 20.961 | -0.531 | -11.276 | 1.00 | 22.51 |
| 802 | CA | TYR | A | 773 | 22.158 | -1.164 | -11.748 | 1.00 | 22.55 |
| 803 | C | TYR | A | 773 | 23.254 | -1.167 | -10.680 | 1.00 | 23.17 |
| 804 | O | TYR | A | 773 | 23.969 | -2.170 | -10.523 | 1.00 | 24.08 |
| 805 | CB | TYR | A | 773 | 22.640 | -0.492 | -13.018 | 1.00 | 22.84 |
| 806 | CG | TYR | A | 773 | 23.825 | -1.191 | -13.593 | 1.00 | 22.80 |
| 807 | CD1 | TYR | A | 773 | 23.680 | -2.384 | -14.304 | 1.00 | 23.02 |
| 808 | CD2 | TYR | A | 773 | 25.095 | -0.671 | -13.418 | 1.00 | 22.59 |
| 809 | CE1 | TYR | A | 773 | 24.791 | -3.041 | -14.837 | 1.00 | 23.77 |
| 810 | CE2 | TYR | A | 773 | 26.198 | -1.309 | -13.938 | 1.00 | 24.43 |
| 811 | CZ | TYR | A | 773 | 26.047 | -2.491 | -14.643 | 1.00 | 24.22 |
| 812 | OH | TYR | A | 773 | 27.172 | -3.094 | -15.155 | 1.00 | 25.98 |
| 813 | N | ARG | A | 774 | 23.432 | -0.044 | -9.982 | 1.00 | 22.85 |
| 814 | CA | ARG | A | 774 | 24.427 | 0.047 | -8.922 | 1.00 | 21.74 |
| 815 | C | ARG | A | 774 | 23.976 | -0.796 | -7.743 | 1.00 | 21.36 |
| 816 | O | ARG | A | 774 | 24.791 | -1.386 | -7.052 | 1.00 | 20.25 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 817 | CB | ARG | A | 774 | 24.623 | 1.487 | -8.494 | 1.00 | 22.23 |
| 818 | CG | ARG | A | 774 | 26.026 | 1.952 | -8.735 | 1.00 | 22.58 |
| 819 | CD | ARG | A | 774 | 26.073 | 3.066 | -9.756 | 1.00 | 23.92 |
| 820 | NE | ARG | A | 774 | 26.048 | 4.383 | -9.146 | 1.00 | 24.69 |
| 821 | CZ | ARG | A | 774 | 26.961 | 5.328 | -9.365 | 1.00 | 25.97 |
| 822 | NH1 | ARG | A | 774 | 27.982 | 5.111 | -10.171 | 1.00 | 25.01 |
| 823 | NH2 | ARG | A | 774 | 26.837 | 6.509 | -8.783 | 1.00 | 26.70 |
| 824 | N | MET | A | 775 | 22.669 | -0.854 | -7.512 | 1.00 | 21.93 |
| 825 | CA | MET | A | 775 | 22.136 | -1.681 | -6.439 | 1.00 | 23.85 |
| 826 | C | MET | A | 775 | 22.550 | -3.136 | -6.666 | 1.00 | 25.38 |
| 827 | O | MET | A | 775 | 22.897 | -3.832 | -5.733 | 1.00 | 25.75 |
| 828 | CB | MET | A | 775 | 20.614 | -1.582 | -6.380 | 1.00 | 23.42 |
| 829 | CG | MET | A | 775 | 20.121 | -0.241 | -5.955 | 1.00 | 23.46 |
| 830 | SD | MET | A | 775 | 18.333 | -0.199 | -5.865 | 1.00 | 26.50 |
| 831 | CE | MET | A | 775 | 17.909 | 1.086 | -7.064 | 1.00 | 27.26 |
| 832 | N | HIS | A | 776 | 22.507 | -3.593 | -7.912 | 1.00 | 27.39 |
| 833 | CA | HIS | A | 776 | 22.891 | -4.954 | -8.262 | 1.00 | 28.41 |
| 834 | C | HIS | A | 776 | 24.403 | -5.065 | -8.178 | 1.00 | 29.55 |
| 835 | O | HIS | A | 776 | 24.923 | -5.865 | -7.414 | 1.00 | 30.12 |
| 836 | CB | HIS | A | 776 | 22.418 | -5.302 | -9.684 | 1.00 | 29.01 |
| 837 | CG | HIS | A | 776 | 22.639 | -6.738 | -10.067 | 1.00 | 30.57 |
| 838 | ND1 | HIS | A | 776 | 23.764 | -7.168 | -10.739 | 1.00 | 30.81 |
| 839 | CD2 | HIS | A | 776 | 21.877 | -7.843 | -9.864 | 1.00 | 30.73 |
| 840 | CE1 | HIS | A | 776 | 23.685 | -8.475 | -10.932 | 1.00 | 29.87 |
| 841 | NE2 | HIS | A | 776 | 22.551 | -8.907 | -10.411 | 1.00 | 29.53 |
| 842 | N | LYS | A | 777 | 25.109 | -4.283 | -8.989 | 1.00 | 31.13 |
| 843 | CA | LYS | A | 777 | 26.570 | -4.290 | -8.980 | 1.00 | 32.73 |
| 844 | C | LYS | A | 777 | 27.032 | -3.655 | -7.660 | 1.00 | 34.07 |
| 845 | O | LYS | A | 777 | 27.382 | -2.478 | -7.611 | 1.00 | 36.43 |
| 846 | CB | LYS | A | 777 | 27.130 | -3.481 | -10.161 | 1.00 | 31.29 |
| 847 | CG | LYS | A | 777 | 26.678 | -3.948 | -11.525 | 1.00 | 30.55 |
| 848 | CD | LYS | A | 777 | 27.443 | -5.163 | -12.003 | 1.00 | 29.83 |
| 849 | CE | LYS | A | 777 | 28.928 | -4.856 | -12.116 | 1.00 | 30.35 |
| 850 | NZ | LYS | A | 777 | 29.631 | -5.860 | -12.983 | 1.00 | 30.53 |
| 851 | N | SER | A | 778 | 26.995 | -4.437 | -6.596 | 1.00 | 33.74 |
| 852 | CA | SER | A | 778 | 27.387 | -4.013 | -5.250 | 1.00 | 33.75 |
| 853 | C | SER | A | 778 | 27.065 | -5.204 | -4.366 | 1.00 | 32.95 |

TABLE 8 (continued)

| THREE-DIMENSIONAL COORDINATES OF AR IN COMPLEX WITH DHT | | | | | | | | | | |
|---|---|---|---|---|---|---|---|---|---|---|
| ATOM | ATOM TYPE | RESIDUE | # | X | Y | Z | OCC | B | ATOM | |
| 854 | O | SER | A | 778 | 27.447 | -5.260 | -3.194 | 1.00 | 31.80 | |
| 855 | CB | SER | A | 778 | 26.593 | -2.789 | -4.769 | 1.00 | 33.69 | |
| 856 | OG | SER | A | 778 | 25.254 | -3.122 | -4.452 | 1.00 | 33.41 | |
| 857 | N | ARG | A | 779 | 26.344 | -6.149 | -4.974 | 1.00 | 32.09 | |
| 858 | CA | ARG | A | 779 | 25.926 | -7.386 | -4.347 | 1.00 | 31.15 | |
| 859 | C | ARG | A | 779 | 25.128 | -7.063 | -3.084 | 1.00 | 30.75 | |
| 860 | O | ARG | A | 779 | 25.027 | -7.880 | -2.163 | 1.00 | 31.03 | |
| 861 | CB | ARG | A | 779 | 27.161 | -8.256 | -4.071 | 1.00 | 30.74 | |
| 862 | CG | ARG | A | 779 | 28.065 | -8.415 | -5.299 | 1.00 | 28.19 | |
| 863 | CD | ARG | A | 779 | 29.338 | -9.182 | -4.997 | 1.00 | 26.90 | |
| 864 | NE | ARG | A | 779 | 30.284 | -9.129 | -6.117 | 1.00 | 26.55 | |
| 865 | CZ | ARG | A | 779 | 31.583 | -9.401 | -6.014 | 1.00 | 26.64 | |
| 866 | NH1 | ARG | A | 779 | 32.091 | -9.753 | -4.846 | 1.00 | 27.87 | |
| 867 | NH2 | ARG | A | 779 | 32.398 | -9.234 | -7.050 | 1.00 | 26.64 | |
| 868 | N | MET | A | 780 | 24.521 | -5.875 | -3.097 | 1.00 | 29.82 | |
| 869 | CA | MET | A | 780 | 23.721 | -5.381 | -1.990 | 1.00 | 29.36 | |
| 870 | C | MET | A | 780 | 22.262 | -5.165 | -2.331 | 1.00 | 29.21 | |
| 871 | O | MET | A | 780 | 21.542 | -4.505 | -1.566 | 1.00 | 29.43 | |
| 872 | CB | MET | A | 780 | 24.295 | -4.068 | -1.473 | 1.00 | 30.17 | |
| 873 | CG | MET | A | 780 | 25.194 | -4.191 | -0.277 | 1.00 | 30.12 | |
| 874 | SD | MET | A | 780 | 25.835 | -2.592 | 0.168 | 1.00 | 31.24 | |
| 875 | CE | MET | A | 780 | 24.525 | -1.995 | 1.114 | 1.00 | 31.13 | |
| 876 | N | TYR | A | 781 | 21.831 | -5.638 | -3.497 | 1.00 | 28.58 | |
| 877 | CA | TYR | A | 781 | 20.433 | -5.498 | -3.897 | 1.00 | 28.82 | |
| 878 | C | TYR | A | 781 | 19.701 | -6.393 | -2.929 | 1.00 | 30.06 | |
| 879 | O | TYR | A | 781 | 19.984 | -7.589 | -2.856 | 1.00 | 32.08 | |
| 880 | CB | TYR | A | 781 | 20.229 | -5.985 | -5.338 | 1.00 | 27.73 | |
| 881 | CG | TYR | A | 781 | 18.896 | -5.604 | -5.964 | 1.00 | 26.05 | |
| 882 | CD1 | TYR | A | 781 | 18.847 | -4.861 | -7.140 | 1.00 | 25.95 | |
| 883 | CD2 | TYR | A | 781 | 17.686 | -5.984 | -5.382 | 1.00 | 25.08 | |
| 884 | CE1 | TYR | A | 781 | 17.624 | -4.510 | -7.718 | 1.00 | 25.58 | |
| 885 | CE2 | TYR | A | 781 | 16.471 | -5.643 | -5.955 | 1.00 | 24.57 | |
| 886 | CZ | TYR | A | 781 | 16.446 | -4.904 | -7.115 | 1.00 | 24.77 | |
| 887 | OH | TYR | A | 781 | 15.238 | -4.572 | -7.668 | 1.00 | 24.60 | |
| 888 | N | SER | A | 782 | 18.730 | -5.821 | -2.235 | 1.00 | 30.74 | |
| 889 | CA | SER | A | 782 | 17.935 | -6.500 | -1.198 | 1.00 | 31.41 | |
| 890 | C | SER | A | 782 | 18.027 | -5.483 | -0.070 | 1.00 | 30.50 | |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 891 | O | SER | A | 782 | 17.044 | -4.807 | 0.230 | 1.00 | 30.31 |
| 892 | CB | SER | A | 782 | 18.551 | -7.836 | -0.726 | 1.00 | 32.82 |
| 893 | OG | SER | A | 782 | 17.785 | -8.438 | 0.308 | 1.00 | 35.91 |
| 894 | N | GLN | A | 783 | 19.242 | -5.287 | 0.459 | 1.00 | 29.34 |
| 895 | CA | GLN | A | 783 | 19.455 | -4.314 | 1.522 | 1.00 | 28.01 |
| 896 | C | GLN | A | 783 | 19.089 | -2.963 | 0.935 | 1.00 | 26.36 |
| 897 | O | GLN | A | 783 | 18.342 | -2.211 | 1.544 | 1.00 | 26.08 |
| 898 | CB | GLN | A | 783 | 20.900 | -4.309 | 2.020 | 1.00 | 28.50 |
| 899 | CG | GLN | A | 783 | 21.327 | -5.532 | 2.805 | 1.00 | 29.62 |
| 900 | CD | GLN | A | 783 | 21.790 | -6.634 | 1.900 | 1.00 | 32.01 |
| 901 | OE1 | GLN | A | 783 | 21.486 | -6.621 | 0.714 | 1.00 | 33.24 |
| 902 | NE2 | GLN | A | 783 | 22.547 | -7.587 | 2.436 | 1.00 | 32.18 |
| 903 | N | CYS | A | 784 | 19.538 | -2.698 | -0.290 | 1.00 | 25.49 |
| 904 | CA | CYS | A | 784 | 19.212 | -1.439 | -0.956 | 1.00 | 24.45 |
| 905 | C | CYS | A | 784 | 17.698 | -1.290 | -1.146 | 1.00 | 25.03 |
| 906 | O | CYS | A | 784 | 17.155 | -0.183 | -1.044 | 1.00 | 25.67 |
| 907 | CB | CYS | A | 784 | 19.951 | -1.312 | -2.294 | 1.00 | 22.82 |
| 908 | SG | CYS | A | 784 | 21.746 | -0.989 | -2.120 | 1.00 | 18.24 |
| 909 | N | VAL | A | 785 | 17.003 | -2.406 | -1.360 | 1.00 | 25.25 |
| 910 | CA | VAL | A | 785 | 15.538 | -2.399 | -1.547 | 1.00 | 25.02 |
| 911 | C | VAL | A | 785 | 14.864 | -1.979 | -0.257 | 1.00 | 23.91 |
| 912 | O | VAL | A | 785 | 13.881 | -1.259 | -0.260 | 1.00 | 24.19 |
| 913 | CB | VAL | A | 785 | 14.987 | -3.826 | -1.903 | 1.00 | 25.94 |
| 914 | CG1 | VAL | A | 785 | 13.457 | -3.901 | -1.710 | 1.00 | 26.21 |
| 915 | CG2 | VAL | A | 785 | 15.349 | -4.195 | -3.324 | 1.00 | 26.31 |
| 916 | N | ARG | A | 786 | 15.402 | -2.455 | 0.853 | 1.00 | 25.02 |
| 917 | CA | ARG | A | 786 | 14.855 | -2.158 | 2.165 | 1.00 | 25.39 |
| 918 | C | ARG | A | 786 | 15.083 | -0.679 | 2.520 | 1.00 | 24.71 |
| 919 | O | ARG | A | 786 | 14.180 | -0.001 | 3.030 | 1.00 | 25.52 |
| 920 | CB | ARG | A | 786 | 15.468 | -3.114 | 3.198 | 1.00 | 26.15 |
| 921 | CG | ARG | A | 786 | 15.392 | -4.591 | 2.748 | 1.00 | 28.30 |
| 922 | CD | ARG | A | 786 | 15.314 | -5.583 | 3.900 | 1.00 | 29.76 |
| 923 | NE | ARG | A | 786 | 14.269 | -5.206 | 4.851 | 1.00 | 32.39 |
| 924 | CZ | ARG | A | 786 | 14.292 | -5.475 | 6.157 | 1.00 | 32.41 |
| 925 | NH1 | ARG | A | 786 | 15.301 | -6.153 | 6.701 | 1.00 | 32.09 |
| 926 | NH2 | ARG | A | 786 | 13.326 | -5.001 | 6.932 | 1.00 | 33.31 |
| 927 | N | MET | A | 787 | 16.246 | -0.146 | 2.160 | 1.00 | 23.53 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 928 | CA | MET | A | 787 | 16.548 | 1.252 | 2.463 | 1.00 | 22.11 |
| 929 | C | MET | A | 787 | 15.736 | 2.173 | 1.588 | 1.00 | 23.39 |
| 930 | O | MET | A | 787 | 15.387 | 3.281 | 1.997 | 1.00 | 24.11 |
| 931 | CB | MET | A | 787 | 18.018 | 1.528 | 2.261 | 1.00 | 20.46 |
| 932 | CG | MET | A | 787 | 18.883 | 0.925 | 3.314 | 1.00 | 17.04 |
| 933 | SD | MET | A | 787 | 20.578 | 0.861 | 2.788 | 1.00 | 20.46 |
| 934 | CE | MET | A | 787 | 21.285 | 1.969 | 3.729 | 1.00 | 20.07 |
| 935 | N | ARG | A | 788 | 15.521 | 1.752 | 0.348 | 1.00 | 24.89 |
| 936 | CA | ARG | A | 788 | 14.738 | 2.499 | -0.625 | 1.00 | 26.29 |
| 937 | C | ARG | A | 788 | 13.312 | 2.475 | -0.090 | 1.00 | 26.13 |
| 938 | O | ARG | A | 788 | 12.596 | 3.473 | -0.146 | 1.00 | 26.50 |
| 939 | CB | ARG | A | 788 | 14.833 | 1.790 | -1.980 | 1.00 | 28.55 |
| 940 | CG | ARG | A | 788 | 14.166 | 2.474 | -3.174 | 1.00 | 32.52 |
| 941 | CD | ARG | A | 788 | 14.217 | 1.541 | -4.395 | 1.00 | 35.44 |
| 942 | NE | ARG | A | 788 | 13.426 | 1.996 | -5.540 | 1.00 | 39.11 |
| 943 | CZ | ARG | A | 788 | 13.899 | 2.177 | -6.783 | 1.00 | 41.32 |
| 944 | NH1 | ARG | A | 788 | 15.182 | 1.960 | -7.081 | 1.00 | 41.94 |
| 945 | NH2 | ARG | A | 788 | 13.079 | 2.567 | -7.754 | 1.00 | 41.48 |
| 946 | N | HIS | A | 789 | 12.920 | 1.339 | 0.483 | 1.00 | 26.36 |
| 947 | CA | HIS | A | 789 | 11.587 | 1.173 | 1.052 | 1.00 | 26.76 |
| 948 | C | HIS | A | 789 | 11.369 | 2.133 | 2.231 | 1.00 | 26.08 |
| 949 | O | HIS | A | 789 | 10.275 | 2.671 | 2.394 | 1.00 | 25.72 |
| 950 | CB | HIS | A | 789 | 11.377 | -0.287 | 1.479 | 1.00 | 29.07 |
| 951 | CG | HIS | A | 789 | 9.970 | -0.609 | 1.879 | 1.00 | 30.42 |
| 952 | ND1 | HIS | A | 789 | 9.538 | -0.567 | 3.188 | 1.00 | 32.05 |
| 953 | CD2 | HIS | A | 789 | 8.890 | -0.944 | 1.137 | 1.00 | 31.35 |
| 954 | CE1 | HIS | A | 789 | 8.249 | -0.856 | 3.235 | 1.00 | 32.56 |
| 955 | NE2 | HIS | A | 789 | 7.831 | -1.087 | 2.001 | 1.00 | 32.55 |
| 956 | N | LEU | A | 790 | 12.413 | 2.318 | 3.048 | 1.00 | 25.92 |
| 957 | CA | LEU | A | 790 | 12.433 | 3.234 | 4.218 | 1.00 | 25.41 |
| 958 | C | LEU | A | 790 | 12.218 | 4.654 | 3.720 | 1.00 | 25.47 |
| 959 | O | LEU | A | 790 | 11.359 | 5.380 | 4.216 | 1.00 | 25.06 |
| 960 | CB | LEU | A | 790 | 13.811 | 3.216 | 4.887 | 1.00 | 23.94 |
| 961 | CG | LEU | A | 790 | 14.039 | 3.400 | 6.383 | 1.00 | 23.32 |
| 962 | CD1 | LEU | A | 790 | 15.444 | 3.930 | 6.570 | 1.00 | 22.41 |
| 963 | CD2 | LEU | A | 790 | 13.047 | 4.324 | 7.014 | 1.00 | 23.17 |
| 964 | N | SER | A | 791 | 13.040 | 5.056 | 2.757 | 1.00 | 25.60 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 965 | CA | SER | A | 791 | 12.942 | 6.375 | 2.177 | 1.00 | 26.51 |
| 966 | C | SER | A | 791 | 11.521 | 6.561 | 1.716 | 1.00 | 26.02 |
| 967 | O | SER | A | 791 | 10.950 | 7.632 | 1.885 | 1.00 | 26.00 |
| 968 | CB | SER | A | 791 | 13.851 | 6.446 | 0.973 | 1.00 | 28.35 |
| 969 | OG | SER | A | 791 | 14.936 | 5.559 | 1.179 | 1.00 | 32.32 |
| 970 | N | GLN | A | 792 | 10.964 | 5.505 | 1.122 | 1.00 | 26.31 |
| 971 | CA | GLN | A | 792 | 9.600 | 5.526 | 0.610 | 1.00 | 26.32 |
| 972 | C | GLN | A | 792 | 8.629 | 5.836 | 1.721 | 1.00 | 24.88 |
| 973 | O | GLN | A | 792 | 7.702 | 6.610 | 1.528 | 1.00 | 24.96 |
| 974 | CB | GLN | A | 792 | 9.237 | 4.200 | -0.112 | 1.00 | 28.65 |
| 975 | CG | GLN | A | 792 | 9.700 | 4.109 | -1.603 | 1.00 | 30.43 |
| 976 | CD | GLN | A | 792 | 9.421 | 2.749 | -2.277 | 1.00 | 31.95 |
| 977 | OE1 | GLN | A | 792 | 8.479 | 2.607 | -3.062 | 1.00 | 33.53 |
| 978 | NE2 | GLN | A | 792 | 10.273 | 1.764 | -2.007 | 1.00 | 32.31 |
| 979 | N | GLU | A | 793 | 8.886 | 5.301 | 2.907 | 1.00 | 23.72 |
| 980 | CA | GLU | A | 793 | 8.014 | 5.550 | 4.051 | 1.00 | 22.89 |
| 981 | C | GLU | A | 793 | 7.949 | 7.041 | 4.400 | 1.00 | 21.58 |
| 982 | O | GLU | A | 793 | 6.903 | 7.530 | 4.764 | 1.00 | 21.52 |
| 983 | CB | GLU | A | 793 | 8.460 | 4.728 | 5.273 | 1.00 | 23.65 |
| 984 | CG | GLU | A | 793 | 8.555 | 3.199 | 5.055 | 1.00 | 25.18 |
| 985 | CD | GLU | A | 793 | 7.383 | 2.406 | 5.651 | 1.00 | 27.08 |
| 986 | OE1 | GLU | A | 793 | 6.207 | 2.735 | 5.351 | 1.00 | 25.97 |
| 987 | OE2 | GLU | A | 793 | 7.648 | 1.450 | 6.433 | 1.00 | 28.69 |
| 988 | N | PHE | A | 794 | 9.042 | 7.784 | 4.274 | 1.00 | 21.26 |
| 989 | CA | PHE | A | 794 | 8.999 | 9.208 | 4.598 | 1.00 | 20.65 |
| 990 | C | PHE | A | 794 | 7.929 | 9.863 | 3.759 | 1.00 | 22.26 |
| 991 | O | PHE | A | 794 | 7.387 | 10.906 | 4.138 | 1.00 | 22.19 |
| 992 | CB | PHE | A | 794 | 10.334 | 9.890 | 4.323 | 1.00 | 19.81 |
| 993 | CG | PHE | A | 794 | 11.413 | 9.541 | 5.304 | 1.00 | 19.96 |
| 994 | CD1 | PHE | A | 794 | 11.226 | 9.728 | 6.662 | 1.00 | 20.01 |
| 995 | CD2 | PHE | A | 794 | 12.599 | 8.974 | 4.878 | 1.00 | 19.43 |
| 996 | CE1 | PHE | A | 794 | 12.206 | 9.347 | 7.566 | 1.00 | 19.86 |
| 997 | CE2 | PHE | A | 794 | 13.570 | 8.593 | 5.787 | 1.00 | 18.95 |
| 998 | CZ | PHE | A | 794 | 13.374 | 8.777 | 7.118 | 1.00 | 19.37 |
| 999 | N | GLY | A | 795 | 7.688 | 9.270 | 2.585 | 1.00 | 23.81 |
| 1000 | CA | GLY | A | 795 | 6.676 | 9.750 | 1.662 | 1.00 | 25.46 |
| 1001 | C | GLY | A | 795 | 5.309 | 9.232 | 2.037 | 1.00 | 26.19 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1002 | O | GLY | A | 795 | 4.414 | 10.002 | 2.345 | 1.00 | 27.46 |
| 1003 | N | TRP | A | 796 | 5.181 | 7.912 | 2.081 | 1.00 | 27.45 |
| 1004 | CA | TRP | A | 796 | 3.931 | 7.239 | 2.428 | 1.00 | 28.24 |
| 1005 | C | TRP | A | 796 | 3.345 | 7.826 | 3.706 | 1.00 | 29.13 |
| 1006 | O | TRP | A | 796 | 2.132 | 8.026 | 3.801 | 1.00 | 29.87 |
| 1007 | CB | TRP | A | 796 | 4.135 | 5.697 | 2.542 | 1.00 | 27.71 |
| 1008 | CG | TRP | A | 796 | 4.478 | 4.998 | 1.187 | 1.00 | 27.50 |
| 1009 | CD1 | TRP | A | 796 | 4.177 | 5.460 | -0.079 | 1.00 | 27.17 |
| 1010 | CD2 | TRP | A | 796 | 5.208 | 3.763 | 0.985 | 1.00 | 26.97 |
| 1011 | NE1 | TRP | A | 796 | 4.676 | 4.601 | -1.035 | 1.00 | 27.59 |
| 1012 | CE2 | TRP | A | 796 | 5.312 | 3.556 | -0.417 | 1.00 | 26.72 |
| 1013 | CE3 | TRP | A | 796 | 5.777 | 2.816 | 1.845 | 1.00 | 25.52 |
| 1014 | CZ2 | TRP | A | 796 | 5.967 | 2.448 | -0.970 | 1.00 | 25.70 |
| 1015 | CZ3 | TRP | A | 796 | 6.427 | 1.714 | 1.290 | 1.00 | 25.51 |
| 1016 | CH2 | TRP | A | 796 | 6.514 | 1.543 | -0.106 | 1.00 | 25.42 |
| 1017 | N | LEU | A | 797 | 4.223 | 8.212 | 4.632 | 1.00 | 29.96 |
| 1018 | CA | LEU | A | 797 | 3.816 | 8.768 | 5.923 | 1.00 | 29.80 |
| 1019 | C | LEU | A | 797 | 3.864 | 10.260 | 5.991 | 1.00 | 30.39 |
| 1020 | O | LEU | A | 797 | 3.447 | 10.827 | 6.983 | 1.00 | 32.25 |
| 1021 | CB | LEU | A | 797 | 4.692 | 8.223 | 7.061 | 1.00 | 28.43 |
| 1022 | CG | LEU | A | 797 | 4.552 | 6.736 | 7.383 | 1.00 | 27.68 |
| 1023 | CD1 | LEU | A | 797 | 5.709 | 6.269 | 8.228 | 1.00 | 27.20 |
| 1024 | CD2 | LEU | A | 797 | 3.216 | 6.470 | 8.058 | 1.00 | 26.62 |
| 1025 | N | GLN | A | 798 | 4.415 | 10.908 | 4.978 | 1.00 | 31.03 |
| 1026 | CA | GLN | A | 798 | 4.518 | 12.360 | 5.005 | 1.00 | 30.93 |
| 1027 | C | GLN | A | 798 | 5.267 | 12.764 | 6.294 | 1.00 | 30.02 |
| 1028 | O | GLN | A | 798 | 4.716 | 13.460 | 7.147 | 1.00 | 30.51 |
| 1029 | CB | GLN | A | 798 | 3.117 | 13.030 | 4.964 | 1.00 | 31.58 |
| 1030 | CG | GLN | A | 798 | 2.253 | 12.757 | 3.701 | 1.00 | 32.86 |
| 1031 | CD | GLN | A | 798 | 0.944 | 13.580 | 3.633 | 1.00 | 32.89 |
| 1032 | OE1 | GLN | A | 798 | 0.342 | 13.933 | 4.648 | 1.00 | 33.16 |
| 1033 | NE2 | GLN | A | 798 | 0.521 | 13.892 | 2.421 | 1.00 | 33.46 |
| 1034 | N | ILE | A | 799 | 6.497 | 12.283 | 6.462 | 1.00 | 28.60 |
| 1035 | CA | ILE | A | 799 | 7.277 | 12.634 | 7.648 | 1.00 | 27.76 |
| 1036 | C | ILE | A | 799 | 7.729 | 14.094 | 7.552 | 1.00 | 28.59 |
| 1037 | O | ILE | A | 799 | 8.181 | 14.552 | 6.496 | 1.00 | 29.81 |
| 1038 | CB | ILE | A | 799 | 8.546 | 11.747 | 7.829 | 1.00 | 26.34 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1039 | CG1 | ILE | A | 799 | 8.168 | 10.286 | 8.046 | 1.00 | 25.57 |
| 1040 | CG2 | ILE | A | 799 | 9.382 | 12.246 | 9.007 | 1.00 | 25.78 |
| 1041 | CD1 | ILE | A | 799 | 7.271 | 10.063 | 9.211 | 1.00 | 25.52 |
| 1042 | N | THR | A | 800 | 7.610 | 14.790 | 8.678 | 1.00 | 28.71 |
| 1043 | CA | THR | A | 800 | 7.967 | 16.197 | 8.874 | 1.00 | 28.68 |
| 1044 | C | THR | A | 800 | 9.475 | 16.347 | 9.116 | 1.00 | 28.69 |
| 1045 | O | THR | A | 800 | 10.069 | 15.510 | 9.796 | 1.00 | 29.15 |
| 1046 | CB | THR | A | 800 | 7.202 | 16.718 | 10.142 | 1.00 | 29.24 |
| 1047 | OG1 | THR | A | 800 | 5.839 | 17.003 | 9.815 | 1.00 | 31.49 |
| 1048 | CG2 | THR | A | 800 | 7.824 | 17.925 | 10.746 | 1.00 | 29.69 |
| 1049 | N | PRO | A | 801 | 10.116 | 17.407 | 8.565 | 1.00 | 28.18 |
| 1050 | CA | PRO | A | 801 | 11.555 | 17.600 | 8.780 | 1.00 | 27.74 |
| 1051 | C | PRO | A | 801 | 11.907 | 17.570 | 10.271 | 1.00 | 27.38 |
| 1052 | O | PRO | A | 801 | 12.981 | 17.101 | 10.666 | 1.00 | 27.75 |
| 1053 | CB | PRO | A | 801 | 11.797 | 18.983 | 8.178 | 1.00 | 27.08 |
| 1054 | CG | PRO | A | 801 | 10.908 | 18.956 | 7.002 | 1.00 | 26.58 |
| 1055 | CD | PRO | A | 801 | 9.618 | 18.376 | 7.569 | 1.00 | 27.62 |
| 1056 | N | GLN | A | 802 | 10.982 | 18.045 | 11.095 | 1.00 | 27.01 |
| 1057 | CA | GLN | A | 802 | 11.189 | 18.079 | 12.542 | 1.00 | 26.73 |
| 1058 | C | GLN | A | 802 | 10.968 | 16.715 | 13.219 | 1.00 | 24.81 |
| 1059 | O | GLN | A | 802 | 11.599 | 16.415 | 14.222 | 1.00 | 24.41 |
| 1060 | CB | GLN | A | 802 | 10.316 | 19.162 | 13.192 | 1.00 | 28.09 |
| 1061 | CG | GLN | A | 802 | 10.582 | 20.596 | 12.692 | 1.00 | 29.79 |
| 1062 | CD | GLN | A | 802 | 9.997 | 20.900 | 11.303 | 1.00 | 30.48 |
| 1063 | OE1 | GLN | A | 802 | 8.948 | 20.381 | 10.918 | 1.00 | 30.36 |
| 1064 | NE2 | GLN | A | 802 | 10.660 | 21.782 | 10.571 | 1.00 | 30.57 |
| 1065 | N | GLU | A | 803 | 10.064 | 15.904 | 12.669 | 1.00 | 23.52 |
| 1066 | CA | GLU | A | 803 | 9.797 | 14.558 | 13.196 | 1.00 | 21.64 |
| 1067 | C | GLU | A | 803 | 11.067 | 13.784 | 12.923 | 1.00 | 21.04 |
| 1068 | O | GLU | A | 803 | 11.537 | 13.042 | 13.777 | 1.00 | 20.89 |
| 1069 | CB | GLU | A | 803 | 8.632 | 13.897 | 12.459 | 1.00 | 20.29 |
| 1070 | CG | GLU | A | 803 | 7.277 | 14.434 | 12.848 | 1.00 | 18.44 |
| 1071 | CD | GLU | A | 803 | 6.147 | 13.786 | 12.119 | 1.00 | 17.84 |
| 1072 | OE1 | GLU | A | 803 | 6.308 | 13.392 | 10.958 | 1.00 | 18.19 |
| 1073 | OE2 | GLU | A | 803 | 5.065 | 13.680 | 12.704 | 1.00 | 19.88 |
| 1074 | N | PHE | A | 804 | 11.612 | 14.001 | 11.722 | 1.00 | 19.87 |
| 1075 | CA | PHE | A | 804 | 12.863 | 13.418 | 11.254 | 1.00 | 19.24 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1076 | C | PHE | A | 804 | 14.034 | 13.802 | 12.157 | 1.00 | 20.31 |
| 1077 | O | PHE | A | 804 | 14.807 | 12.939 | 12.564 | 1.00 | 21.30 |
| 1078 | CB | PHE | A | 804 | 13.144 | 13.867 | 9.822 | 1.00 | 17.23 |
| 1079 | CG | PHE | A | 804 | 14.557 | 13.645 | 9.384 | 1.00 | 14.85 |
| 1080 | CD1 | PHE | A | 804 | 15.012 | 12.380 | 9.095 | 1.00 | 13.76 |
| 1081 | CD2 | PHE | A | 804 | 15.440 | 14.706 | 9.301 | 1.00 | 13.69 |
| 1082 | CE1 | PHE | A | 804 | 16.335 | 12.160 | 8.729 | 1.00 | 13.79 |
| 1083 | CE2 | PHE | A | 804 | 16.765 | 14.496 | 8.936 | 1.00 | 13.36 |
| 1084 | CZ | PHE | A | 804 | 17.214 | 13.217 | 8.647 | 1.00 | 12.84 |
| 1085 | N | LEU | A | 805 | 14.187 | 15.086 | 12.463 | 1.00 | 20.30 |
| 1086 | CA | LEU | A | 805 | 15.271 | 15.503 | 13.339 | 1.00 | 20.09 |
| 1087 | C | LEU | A | 805 | 15.172 | 14.767 | 14.651 | 1.00 | 19.83 |
| 1088 | O | LEU | A | 805 | 16.142 | 14.205 | 15.106 | 1.00 | 20.77 |
| 1089 | CB | LEU | A | 805 | 15.250 | 17.008 | 13.582 | 1.00 | 19.58 |
| 1090 | CG | LEU | A | 805 | 15.552 | 17.834 | 12.330 | 1.00 | 20.47 |
| 1091 | CD1 | LEU | A | 805 | 15.704 | 19.281 | 12.707 | 1.00 | 19.84 |
| 1092 | CD2 | LEU | A | 805 | 16.816 | 17.343 | 11.670 | 1.00 | 19.41 |
| 1093 | N | CYS | A | 806 | 13.980 | 14.719 | 15.223 | 1.00 | 20.17 |
| 1094 | CA | CYS | A | 806 | 13.765 | 14.026 | 16.494 | 1.00 | 21.27 |
| 1095 | C | CYS | A | 806 | 13.938 | 12.515 | 16.378 | 1.00 | 20.36 |
| 1096 | O | CYS | A | 806 | 14.575 | 11.904 | 17.241 | 1.00 | 20.30 |
| 1097 | CB | CYS | A | 806 | 12.372 | 14.332 | 17.078 | 1.00 | 22.13 |
| 1098 | SG | CYS | A | 806 | 12.142 | 16.017 | 17.706 | 1.00 | 27.50 |
| 1099 | N | MET | A | 807 | 13.348 | 11.903 | 15.350 | 1.00 | 19.67 |
| 1100 | CA | MET | A | 807 | 13.491 | 10.458 | 15.160 | 1.00 | 18.10 |
| 1101 | C | MET | A | 807 | 14.947 | 10.062 | 14.979 | 1.00 | 17.75 |
| 1102 | O | MET | A | 807 | 15.371 | 9.038 | 15.490 | 1.00 | 18.41 |
| 1103 | CB | MET | A | 807 | 12.668 | 9.944 | 13.989 | 1.00 | 17.25 |
| 1104 | CG | MET | A | 807 | 11.195 | 9.877 | 14.279 | 1.00 | 16.70 |
| 1105 | SD | MET | A | 807 | 10.377 | 9.142 | 12.911 | 1.00 | 19.42 |
| 1106 | CE | MET | A | 807 | 10.144 | 10.560 | 11.908 | 1.00 | 16.21 |
| 1107 | N | LYS | A | 808 | 15.712 | 10.871 | 14.257 | 1.00 | 17.31 |
| 1108 | CA | LYS | A | 808 | 17.116 | 10.592 | 14.054 | 1.00 | 16.27 |
| 1109 | C | LYS | A | 808 | 17.857 | 10.726 | 15.376 | 1.00 | 16.89 |
| 1110 | O | LYS | A | 808 | 18.731 | 9.908 | 15.677 | 1.00 | 16.07 |
| 1111 | CB | LYS | A | 808 | 17.729 | 11.514 | 12.994 | 1.00 | 15.01 |
| 1112 | CG | LYS | A | 808 | 19.171 | 11.154 | 12.733 | 1.00 | 14.63 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1113 | CD | LYS | A | 808 | 19.679 | 11.569 | 11.371 | 1.00 | 15.42 |
| 1114 | CE | LYS | A | 808 | 19.422 | 13.053 | 11.092 | 1.00 | 15.64 |
| 1115 | NZ | LYS | A | 808 | 20.232 | 13.940 | 11.928 | 1.00 | 14.15 |
| 1116 | N | ALA | A | 809 | 17.522 | 11.747 | 16.166 | 1.00 | 16.53 |
| 1117 | CA | ALA | A | 809 | 18.175 | 11.931 | 17.461 | 1.00 | 17.68 |
| 1118 | C | ALA | A | 809 | 17.989 | 10.691 | 18.348 | 1.00 | 19.03 |
| 1119 | O | ALA | A | 809 | 18.932 | 10.207 | 18.996 | 1.00 | 20.50 |
| 1120 | CB | ALA | A | 809 | 17.628 | 13.139 | 18.155 | 1.00 | 16.91 |
| 1121 | N | LEU | A | 810 | 16.766 | 10.184 | 18.392 | 1.00 | 19.36 |
| 1122 | CA | LEU | A | 810 | 16.459 | 9.011 | 19.186 | 1.00 | 18.99 |
| 1123 | C | LEU | A | 810 | 17.116 | 7.716 | 18.722 | 1.00 | 18.88 |
| 1124 | O | LEU | A | 810 | 17.213 | 6.780 | 19.509 | 1.00 | 20.78 |
| 1125 | CB | LEU | A | 810 | 14.966 | 8.811 | 19.263 | 1.00 | 19.09 |
| 1126 | CG | LEU | A | 810 | 14.406 | 9.020 | 20.651 | 1.00 | 20.20 |
| 1127 | CD1 | LEU | A | 810 | 12.954 | 8.606 | 20.594 | 1.00 | 21.29 |
| 1128 | CD2 | LEU | A | 810 | 15.176 | 8.199 | 21.674 | 1.00 | 18.75 |
| 1129 | N | LEU | A | 811 | 17.537 | 7.636 | 17.456 | 1.00 | 17.63 |
| 1130 | CA | LEU | A | 811 | 18.215 | 6.447 | 16.959 | 1.00 | 15.58 |
| 1131 | C | LEU | A | 811 | 19.598 | 6.328 | 17.582 | 1.00 | 15.42 |
| 1132 | O | LEU | A | 811 | 20.189 | 5.252 | 17.554 | 1.00 | 17.27 |
| 1133 | CB | LEU | A | 811 | 18.346 | 6.456 | 15.438 | 1.00 | 14.70 |
| 1134 | CG | LEU | A | 811 | 17.148 | 6.107 | 14.574 | 1.00 | 14.14 |
| 1135 | CD1 | LEU | A | 811 | 17.511 | 6.408 | 13.164 | 1.00 | 13.66 |
| 1136 | CD2 | LEU | A | 811 | 16.744 | 4.632 | 14.746 | 1.00 | 13.62 |
| 1137 | N | LEU | A | 812 | 20.153 | 7.429 | 18.084 | 1.00 | 13.97 |
| 1138 | CA | LEU | A | 812 | 21.455 | 7.373 | 18.734 | 1.00 | 12.94 |
| 1139 | C | LEU | A | 812 | 21.330 | 6.658 | 20.098 | 1.00 | 12.97 |
| 1140 | O | LEU | A | 812 | 22.282 | 6.118 | 20.629 | 1.00 | 12.99 |
| 1141 | CB | LEU | A | 812 | 22.004 | 8.790 | 18.937 | 1.00 | 12.69 |
| 1142 | CG | LEU | A | 812 | 23.342 | 8.893 | 19.670 | 1.00 | 12.03 |
| 1143 | CD1 | LEU | A | 812 | 24.488 | 8.422 | 18.802 | 1.00 | 12.16 |
| 1144 | CD2 | LEU | A | 812 | 23.559 | 10.325 | 20.037 | 1.00 | 13.12 |
| 1145 | N | PHE | A | 813 | 20.136 | 6.681 | 20.662 | 1.00 | 13.55 |
| 1146 | CA | PHE | A | 813 | 19.859 | 6.064 | 21.950 | 1.00 | 14.19 |
| 1147 | C | PHE | A | 813 | 18.971 | 4.856 | 21.753 | 1.00 | 14.71 |
| 1148 | O | PHE | A | 813 | 18.058 | 4.618 | 22.530 | 1.00 | 14.79 |
| 1149 | CB | PHE | A | 813 | 19.137 | 7.088 | 22.821 | 1.00 | 15.20 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1150 | CG | PHE | A | 813 | 19.818 | 8.435 | 22.841 | 1.00 | 16.11 |
| 1151 | CD1 | PHE | A | 813 | 20.946 | 8.640 | 23.624 | 1.00 | 15.97 |
| 1152 | CD2 | PHE | A | 813 | 19.349 | 9.472 | 22.036 | 1.00 | 16.07 |
| 1153 | CE1 | PHE | A | 813 | 21.604 | 9.845 | 23.615 | 1.00 | 18.37 |
| 1154 | CE2 | PHE | A | 813 | 19.991 | 10.687 | 22.014 | 1.00 | 17.54 |
| 1155 | CZ | PHE | A | 813 | 21.126 | 10.883 | 22.801 | 1.00 | 17.99 |
| 1156 | N | SER | A | 814 | 19.255 | 4.082 | 20.709 | 1.00 | 16.09 |
| 1157 | CA | SER | A | 814 | 18.453 | 2.917 | 20.369 | 1.00 | 15.96 |
| 1158 | C | SER | A | 814 | 19.169 | 1.581 | 20.294 | 1.00 | 16.05 |
| 1159 | O | SER | A | 814 | 18.610 | 0.620 | 19.779 | 1.00 | 17.02 |
| 1160 | CB | SER | A | 814 | 17.697 | 3.172 | 19.062 | 1.00 | 15.79 |
| 1161 | OG | SER | A | 814 | 16.640 | 4.087 | 19.274 | 1.00 | 15.51 |
| 1162 | N | ILE | A | 815 | 20.395 | 1.498 | 20.779 | 1.00 | 16.07 |
| 1163 | CA | ILE | A | 815 | 21.099 | 0.226 | 20.747 | 1.00 | 17.04 |
| 1164 | C | ILE | A | 815 | 22.172 | 0.187 | 21.826 | 1.00 | 17.86 |
| 1165 | O | ILE | A | 815 | 23.111 | 0.981 | 21.802 | 1.00 | 18.49 |
| 1166 | CB | ILE | A | 815 | 21.620 | -0.086 | 19.325 | 1.00 | 16.75 |
| 1167 | CG1 | ILE | A | 815 | 22.600 | -1.245 | 19.341 | 1.00 | 17.01 |
| 1168 | CG2 | ILE | A | 815 | 22.222 | 1.113 | 18.706 | 1.00 | 17.43 |
| 1169 | CD1 | ILE | A | 815 | 22.915 | -1.753 | 17.953 | 1.00 | 17.98 |
| 1170 | N | ILE | A | 816 | 21.994 | -0.700 | 22.809 | 1.00 | 18.25 |
| 1171 | CA | ILE | A | 816 | 22.913 | -0.804 | 23.947 | 1.00 | 18.91 |
| 1172 | C | ILE | A | 816 | 23.302 | -2.226 | 24.385 | 1.00 | 20.13 |
| 1173 | O | ILE | A | 816 | 22.615 | -3.184 | 24.040 | 1.00 | 20.43 |
| 1174 | CB | ILE | A | 816 | 22.298 | -0.099 | 25.178 | 1.00 | 19.07 |
| 1175 | CG1 | ILE | A | 816 | 20.939 | -0.692 | 25.537 | 1.00 | 18.04 |
| 1176 | CG2 | ILE | A | 816 | 22.175 | 1.378 | 24.921 | 1.00 | 17.73 |
| 1177 | CD1 | ILE | A | 816 | 20.516 | -0.346 | 26.933 | 1.00 | 17.73 |
| 1178 | N | PRO | A | 817 | 24.392 | -2.385 | 25.180 | 1.00 | 20.85 |
| 1179 | CA | PRO | A | 817 | 24.805 | -3.720 | 25.631 | 1.00 | 22.05 |
| 1180 | C | PRO | A | 817 | 23.706 | -4.320 | 26.458 | 1.00 | 22.95 |
| 1181 | O | PRO | A | 817 | 22.988 | -3.594 | 27.151 | 1.00 | 23.12 |
| 1182 | CB | PRO | A | 817 | 26.016 | -3.444 | 26.523 | 1.00 | 21.47 |
| 1183 | CG | PRO | A | 817 | 26.554 | -2.197 | 26.001 | 1.00 | 21.80 |
| 1184 | CD | PRO | A | 817 | 25.303 | -1.373 | 25.730 | 1.00 | 21.00 |
| 1185 | N | VAL | A | 818 | 23.585 | -5.640 | 26.418 | 1.00 | 24.79 |
| 1186 | CA | VAL | A | 818 | 22.544 | -6.316 | 27.195 | 1.00 | 26.35 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1187 | C | VAL | A | 818 | 22.742 | -6.047 | 28.692 | 1.00 | 26.79 |
| 1188 | O | VAL | A | 818 | 21.777 | -5.849 | 29.421 | 1.00 | 26.79 |
| 1189 | CB | VAL | A | 818 | 22.513 | -7.860 | 26.916 | 1.00 | 27.19 |
| 1190 | CG1 | VAL | A | 818 | 23.864 | -8.515 | 27.282 | 1.00 | 27.82 |
| 1191 | CG2 | VAL | A | 818 | 21.362 | -8.524 | 27.676 | 1.00 | 27.72 |
| 1192 | N | ASP | A | 819 | 23.992 | -5.963 | 29.136 | 1.00 | 27.83 |
| 1193 | CA | ASP | A | 819 | 24.240 | -5.732 | 30.550 | 1.00 | 29.78 |
| 1194 | C | ASP | A | 819 | 24.377 | -4.266 | 30.937 | 1.00 | 29.73 |
| 1195 | O | ASP | A | 819 | 24.899 | -3.930 | 32.007 | 1.00 | 30.00 |
| 1196 | CB | ASP | A | 819 | 25.406 | -6.593 | 31.063 | 1.00 | 32.59 |
| 1197 | CG | ASP | A | 819 | 26.747 | -5.908 | 30.959 | 1.00 | 35.35 |
| 1198 | OD1 | ASP | A | 819 | 27.117 | -5.518 | 29.825 | 1.00 | 38.62 |
| 1199 | OD2 | ASP | A | 819 | 27.431 | -5.776 | 32.011 | 1.00 | 36.18 |
| 1200 | N | GLY | A | 820 | 23.839 | -3.403 | 30.085 | 1.00 | 29.43 |
| 1201 | CA | GLY | A | 820 | 23.878 | -1.974 | 30.342 | 1.00 | 28.69 |
| 1202 | C | GLY | A | 820 | 25.216 | -1.317 | 30.125 | 1.00 | 27.42 |
| 1203 | O | GLY | A | 820 | 26.221 | -1.982 | 29.938 | 1.00 | 26.73 |
| 1204 | N | LEU | A | 821 | 25.208 | 0.010 | 30.135 | 1.00 | 28.29 |
| 1205 | CA | LEU | A | 821 | 26.410 | 0.831 | 29.947 | 1.00 | 28.64 |
| 1206 | C | LEU | A | 821 | 26.948 | 1.164 | 31.349 | 1.00 | 28.62 |
| 1207 | O | LEU | A | 821 | 26.341 | 0.747 | 32.342 | 1.00 | 28.84 |
| 1208 | CB | LEU | A | 821 | 26.023 | 2.110 | 29.195 | 1.00 | 28.29 |
| 1209 | CG | LEU | A | 821 | 25.083 | 1.940 | 27.991 | 1.00 | 28.32 |
| 1210 | CD1 | LEU | A | 821 | 24.046 | 3.022 | 28.031 | 1.00 | 27.27 |
| 1211 | CD2 | LEU | A | 821 | 25.831 | 1.953 | 26.653 | 1.00 | 27.18 |
| 1212 | N | LYS | A | 822 | 28.060 | 1.897 | 31.441 | 1.00 | 28.49 |
| 1213 | CA | LYS | A | 822 | 28.642 | 2.268 | 32.741 | 1.00 | 29.80 |
| 1214 | C | LYS | A | 822 | 27.621 | 3.016 | 33.587 | 1.00 | 30.25 |
| 1215 | O | LYS | A | 822 | 27.353 | 2.655 | 34.731 | 1.00 | 31.02 |
| 1216 | CB | LYS | A | 822 | 29.865 | 3.169 | 32.576 | 1.00 | 30.45 |
| 1217 | CG | LYS | A | 822 | 30.924 | 2.626 | 31.666 | 1.00 | 32.84 |
| 1218 | CD | LYS | A | 822 | 31.517 | 1.345 | 32.194 | 1.00 | 35.27 |
| 1219 | CE | LYS | A | 822 | 32.433 | 0.688 | 31.161 | 1.00 | 36.20 |
| 1220 | NZ | LYS | A | 822 | 33.498 | 1.623 | 30.710 | 1.00 | 37.22 |
| 1221 | N | ASN | A | 823 | 27.065 | 4.080 | 33.029 | 1.00 | 29.98 |
| 1222 | CA | ASN | A | 823 | 26.070 | 4.852 | 33.735 | 1.00 | 29.55 |
| 1223 | C | ASN | A | 823 | 24.807 | 4.665 | 32.943 | 1.00 | 28.73 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1224 | O | ASN | A | 823 | 24.476 | 5.473 | 32.091 | 1.00 | 29.00 |
| 1225 | CB | ASN | A | 823 | 26.458 | 6.323 | 33.774 | 1.00 | 31.17 |
| 1226 | CG | ASN | A | 823 | 27.832 | 6.544 | 34.350 | 1.00 | 32.55 |
| 1227 | OD1 | ASN | A | 823 | 28.787 | 5.856 | 33.985 | 1.00 | 33.56 |
| 1228 | ND2 | ASN | A | 823 | 27.952 | 7.520 | 35.246 | 1.00 | 34.42 |
| 1229 | N | GLN | A | 824 | 24.127 | 3.562 | 33.199 | 1.00 | 27.99 |
| 1230 | CA | GLN | A | 824 | 22.893 | 3.227 | 32.514 | 1.00 | 27.77 |
| 1231 | C | GLN | A | 824 | 21.723 | 4.115 | 32.960 | 1.00 | 27.61 |
| 1232 | O | GLN | A | 824 | 20.747 | 4.275 | 32.226 | 1.00 | 27.58 |
| 1233 | CB | GLN | A | 824 | 22.590 | 1.731 | 32.738 | 1.00 | 28.13 |
| 1234 | CG | GLN | A | 824 | 21.343 | 1.158 | 32.077 | 1.00 | 28.93 |
| 1235 | CD | GLN | A | 824 | 21.331 | 1.302 | 30.551 | 1.00 | 30.20 |
| 1236 | OE1 | GLN | A | 824 | 22.300 | 0.976 | 29.855 | 1.00 | 30.02 |
| 1237 | NE2 | GLN | A | 824 | 20.211 | 1.775 | 30.028 | 1.00 | 29.72 |
| 1238 | N | LYS | A | 825 | 21.833 | 4.752 | 34.122 | 1.00 | 27.13 |
| 1239 | CA | LYS | A | 825 | 20.742 | 5.590 | 34.595 | 1.00 | 26.37 |
| 1240 | C | LYS | A | 825 | 20.679 | 6.917 | 33.876 | 1.00 | 24.74 |
| 1241 | O | LYS | A | 825 | 19.625 | 7.518 | 33.799 | 1.00 | 25.30 |
| 1242 | CB | LYS | A | 825 | 20.815 | 5.802 | 36.113 | 1.00 | 28.08 |
| 1243 | CG | LYS | A | 825 | 19.430 | 5.823 | 36.792 | 1.00 | 31.02 |
| 1244 | CD | LYS | A | 825 | 19.493 | 5.693 | 38.335 | 1.00 | 33.41 |
| 1245 | CE | LYS | A | 825 | 18.086 | 5.725 | 39.002 | 1.00 | 34.57 |
| 1246 | NZ | LYS | A | 825 | 17.196 | 4.516 | 38.739 | 1.00 | 35.55 |
| 1247 | N | PHE | A | 826 | 21.794 | 7.375 | 33.330 | 1.00 | 24.06 |
| 1248 | CA | PHE | A | 826 | 21.830 | 8.646 | 32.597 | 1.00 | 23.44 |
| 1249 | C | PHE | A | 826 | 21.344 | 8.463 | 31.178 | 1.00 | 21.78 |
| 1250 | O | PHE | A | 826 | 20.808 | 9.380 | 30.568 | 1.00 | 21.42 |
| 1251 | CB | PHE | A | 826 | 23.247 | 9.191 | 32.573 | 1.00 | 25.61 |
| 1252 | CG | PHE | A | 826 | 23.768 | 9.527 | 33.930 | 1.00 | 28.86 |
| 1253 | CD1 | PHE | A | 826 | 22.916 | 10.067 | 34.890 | 1.00 | 29.49 |
| 1254 | CD2 | PHE | A | 826 | 25.091 | 9.284 | 34.268 | 1.00 | 29.08 |
| 1255 | CE1 | PHE | A | 826 | 23.373 | 10.356 | 36.156 | 1.00 | 29.57 |
| 1256 | CE2 | PHE | A | 826 | 25.551 | 9.571 | 35.533 | 1.00 | 29.80 |
| 1257 | CZ | PHE | A | 826 | 24.688 | 10.108 | 36.479 | 1.00 | 30.14 |
| 1258 | N | PHE | A | 827 | 21.581 | 7.277 | 30.636 | 1.00 | 20.42 |
| 1259 | CA | PHE | A | 827 | 21.145 | 6.937 | 29.299 | 1.00 | 18.74 |
| 1260 | C | PHE | A | 827 | 19.627 | 6.778 | 29.277 | 1.00 | 19.19 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1261 | O | PHE | A | 827 | 18.962 | 7.183 | 28.331 | 1.00 | 18.79 |
| 1262 | CB | PHE | A | 827 | 21.814 | 5.644 | 28.857 | 1.00 | 17.62 |
| 1263 | CG | PHE | A | 827 | 21.238 | 5.083 | 27.610 | 1.00 | 16.77 |
| 1264 | CD1 | PHE | A | 827 | 21.780 | 5.412 | 26.380 | 1.00 | 16.74 |
| 1265 | CD2 | PHE | A | 827 | 20.123 | 4.261 | 27.656 | 1.00 | 16.46 |
| 1266 | CE1 | PHE | A | 827 | 21.225 | 4.939 | 25.212 | 1.00 | 16.50 |
| 1267 | CE2 | PHE | A | 827 | 19.555 | 3.782 | 26.491 | 1.00 | 17.41 |
| 1268 | CZ | PHE | A | 827 | 20.105 | 4.120 | 25.266 | 1.00 | 16.35 |
| 1269 | N | ASP | A | 828 | 19.079 | 6.150 | 30.312 | 1.00 | 20.42 |
| 1270 | CA | ASP | A | 828 | 17.638 | 5.943 | 30.421 | 1.00 | 21.69 |
| 1271 | C | ASP | A | 828 | 16.931 | 7.287 | 30.572 | 1.00 | 21.41 |
| 1272 | O | ASP | A | 828 | 15.835 | 7.487 | 30.070 | 1.00 | 21.87 |
| 1273 | CB | ASP | A | 828 | 17.325 | 5.045 | 31.633 | 1.00 | 23.37 |
| 1274 | CG | ASP | A | 828 | 17.885 | 3.627 | 31.487 | 1.00 | 24.46 |
| 1275 | OD1 | ASP | A | 828 | 17.900 | 3.095 | 30.365 | 1.00 | 26.34 |
| 1276 | OD2 | ASP | A | 828 | 18.296 | 3.023 | 32.501 | 1.00 | 26.77 |
| 1277 | N | GLU | A | 829 | 17.552 | 8.187 | 31.313 | 1.00 | 22.11 |
| 1278 | CA | GLU | A | 829 | 17.005 | 9.510 | 31.533 | 1.00 | 23.92 |
| 1279 | C | GLU | A | 829 | 17.011 | 10.166 | 30.174 | 1.00 | 22.78 |
| 1280 | O | GLU | A | 829 | 15.963 | 10.539 | 29.656 | 1.00 | 22.06 |
| 1281 | CB | GLU | A | 829 | 17.910 | 10.309 | 32.499 | 1.00 | 27.77 |
| 1282 | CG | GLU | A | 829 | 18.168 | 11.823 | 32.130 | 1.00 | 32.20 |
| 1283 | CD | GLU | A | 829 | 19.650 | 12.266 | 32.334 | 1.00 | 35.29 |
| 1284 | OE1 | GLU | A | 829 | 20.005 | 12.655 | 33.482 | 1.00 | 37.06 |
| 1285 | OE2 | GLU | A | 829 | 20.463 | 12.217 | 31.360 | 1.00 | 34.89 |
| 1286 | N | LEU | A | 830 | 18.201 | 10.200 | 29.575 | 1.00 | 22.21 |
| 1287 | CA | LEU | A | 830 | 18.437 | 10.812 | 28.272 | 1.00 | 22.14 |
| 1288 | C | LEU | A | 830 | 17.499 | 10.318 | 27.191 | 1.00 | 22.74 |
| 1289 | O | LEU | A | 830 | 16.874 | 11.114 | 26.481 | 1.00 | 23.35 |
| 1290 | CB | LEU | A | 830 | 19.885 | 10.575 | 27.852 | 1.00 | 21.24 |
| 1291 | CG | LEU | A | 830 | 20.415 | 11.572 | 26.833 | 1.00 | 21.76 |
| 1292 | CD1 | LEU | A | 830 | 20.037 | 13.004 | 27.215 | 1.00 | 21.40 |
| 1293 | CD2 | LEU | A | 830 | 21.895 | 11.429 | 26.752 | 1.00 | 22.34 |
| 1294 | N | ARG | A | 831 | 17.400 | 9.002 | 27.079 | 1.00 | 22.23 |
| 1295 | CA | ARG | A | 831 | 16.559 | 8.352 | 26.097 | 1.00 | 22.37 |
| 1296 | C | ARG | A | 831 | 15.086 | 8.667 | 26.302 | 1.00 | 22.46 |
| 1297 | O | ARG | A | 831 | 14.354 | 8.964 | 25.351 | 1.00 | 22.89 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1298 | CB | ARG | A | 831 | 16.780 | 6.849 | 26.186 | 1.00 | 22.50 |
| 1299 | CG | ARG | A | 831 | 15.957 | 6.087 | 25.219 | 1.00 | 22.59 |
| 1300 | CD | ARG | A | 831 | 16.130 | 4.600 | 25.375 | 1.00 | 23.29 |
| 1301 | NE | ARG | A | 831 | 15.921 | 3.972 | 24.074 | 1.00 | 25.50 |
| 1302 | CZ | ARG | A | 831 | 14.738 | 3.800 | 23.491 | 1.00 | 24.36 |
| 1303 | NH1 | ARG | A | 831 | 13.632 | 4.173 | 24.096 | 1.00 | 25.37 |
| 1304 | NH2 | ARG | A | 831 | 14.676 | 3.366 | 22.250 | 1.00 | 24.31 |
| 1305 | N | MET | A | 832 | 14.655 | 8.593 | 27.550 | 1.00 | 22.76 |
| 1306 | CA | MET | A | 832 | 13.276 | 8.859 | 27.923 | 1.00 | 22.91 |
| 1307 | C | MET | A | 832 | 12.879 | 10.262 | 27.513 | 1.00 | 23.47 |
| 1308 | O | MET | A | 832 | 11.740 | 10.512 | 27.097 | 1.00 | 23.79 |
| 1309 | CB | MET | A | 832 | 13.126 | 8.762 | 29.429 | 1.00 | 23.88 |
| 1310 | CG | MET | A | 832 | 11.739 | 9.050 | 29.870 | 1.00 | 24.65 |
| 1311 | SD | MET | A | 832 | 11.693 | 9.332 | 31.596 | 1.00 | 29.43 |
| 1312 | CE | MET | A | 832 | 10.059 | 10.026 | 31.651 | 1.00 | 29.67 |
| 1313 | N | ASN | A | 833 | 13.782 | 11.198 | 27.768 | 1.00 | 23.63 |
| 1314 | CA | ASN | A | 833 | 13.562 | 12.599 | 27.423 | 1.00 | 23.80 |
| 1315 | C | ASN | A | 833 | 13.403 | 12.761 | 25.905 | 1.00 | 23.94 |
| 1316 | O | ASN | A | 833 | 12.463 | 13.397 | 25.445 | 1.00 | 24.48 |
| 1317 | CB | ASN | A | 833 | 14.676 | 13.482 | 28.013 | 1.00 | 23.64 |
| 1318 | CG | ASN | A | 833 | 14.532 | 13.679 | 29.544 | 1.00 | 23.87 |
| 1319 | OD1 | ASN | A | 833 | 15.519 | 13.864 | 30.270 | 1.00 | 23.24 |
| 1320 | ND2 | ASN | A | 833 | 13.293 | 13.628 | 30.030 | 1.00 | 24.57 |
| 1321 | N | TYR | A | 834 | 14.240 | 12.093 | 25.123 | 1.00 | 23.69 |
| 1322 | CA | TYR | A | 834 | 14.121 | 12.165 | 23.673 | 1.00 | 24.70 |
| 1323 | C | TYR | A | 834 | 12.809 | 11.574 | 23.128 | 1.00 | 24.43 |
| 1324 | O | TYR | A | 834 | 12.297 | 12.006 | 22.082 | 1.00 | 24.26 |
| 1325 | CB | TYR | A | 834 | 15.340 | 11.532 | 23.007 | 1.00 | 25.39 |
| 1326 | CG | TYR | A | 834 | 16.491 | 12.489 | 22.872 | 1.00 | 25.49 |
| 1327 | CD1 | TYR | A | 834 | 16.802 | 13.051 | 21.635 | 1.00 | 26.75 |
| 1328 | CD2 | TYR | A | 834 | 17.239 | 12.869 | 23.986 | 1.00 | 26.00 |
| 1329 | CE1 | TYR | A | 834 | 17.828 | 13.975 | 21.502 | 1.00 | 27.67 |
| 1330 | CE2 | TYR | A | 834 | 18.268 | 13.791 | 23.873 | 1.00 | 26.69 |
| 1331 | CZ | TYR | A | 834 | 18.558 | 14.341 | 22.624 | 1.00 | 28.15 |
| 1332 | OH | TYR | A | 834 | 19.571 | 15.263 | 22.497 | 1.00 | 28.86 |
| 1333 | N | ILE | A | 835 | 12.260 | 10.599 | 23.843 | 1.00 | 24.33 |
| 1334 | CA | ILE | A | 835 | 11.004 | 9.991 | 23.450 | 1.00 | 23.48 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1335 | C | ILE | A | 835 | 9.893 | 10.977 | 23.687 | 1.00 | 25.05 |
| 1336 | O | ILE | A | 835 | 8.972 | 11.061 | 22.889 | 1.00 | 26.24 |
| 1337 | CB | ILE | A | 835 | 10.724 | 8.727 | 24.235 | 1.00 | 21.33 |
| 1338 | CG1 | ILE | A | 835 | 11.657 | 7.624 | 23.756 | 1.00 | 20.40 |
| 1339 | CG2 | ILE | A | 835 | 9.297 | 8.308 | 24.054 | 1.00 | 19.87 |
| 1340 | CD1 | ILE | A | 835 | 11.684 | 6.400 | 24.655 | 1.00 | 21.05 |
| 1341 | N | LYS | A | 836 | 9.998 | 11.738 | 24.771 | 1.00 | 26.82 |
| 1342 | CA | LYS | A | 836 | 9.006 | 12.747 | 25.137 | 1.00 | 28.28 |
| 1343 | C | LYS | A | 836 | 9.030 | 13.919 | 24.164 | 1.00 | 28.39 |
| 1344 | O | LYS | A | 836 | 7.997 | 14.545 | 23.946 | 1.00 | 29.09 |
| 1345 | CB | LYS | A | 836 | 9.245 | 13.281 | 26.556 | 1.00 | 29.95 |
| 1346 | CG | LYS | A | 836 | 9.115 | 12.252 | 27.712 | 1.00 | 32.62 |
| 1347 | CD | LYS | A | 836 | 7.690 | 11.672 | 27.901 | 1.00 | 33.04 |
| 1348 | CE | LYS | A | 836 | 7.575 | 10.238 | 27.344 | 1.00 | 34.32 |
| 1349 | NZ | LYS | A | 836 | 8.559 | 9.259 | 27.942 | 1.00 | 32.90 |
| 1350 | N | GLU | A | 837 | 10.194 | 14.249 | 23.606 | 1.00 | 28.61 |
| 1351 | CA | GLU | A | 837 | 10.276 | 15.351 | 22.643 | 1.00 | 28.70 |
| 1352 | C | GLU | A | 837 | 9.666 | 14.907 | 21.321 | 1.00 | 28.92 |
| 1353 | O | GLU | A | 837 | 9.041 | 15.699 | 20.626 | 1.00 | 28.40 |
| 1354 | CB | GLU | A | 837 | 11.715 | 15.824 | 22.439 | 1.00 | 29.34 |
| 1355 | CG | GLU | A | 837 | 12.305 | 16.584 | 23.627 | 1.00 | 32.13 |
| 1356 | CD | GLU | A | 837 | 11.553 | 17.887 | 23.971 | 1.00 | 34.30 |
| 1357 | OE1 | GLU | A | 837 | 11.612 | 18.303 | 25.157 | 1.00 | 34.18 |
| 1358 | OE2 | GLU | A | 837 | 10.925 | 18.503 | 23.063 | 1.00 | 35.58 |
| 1359 | N | LEU | A | 838 | 9.826 | 13.631 | 20.991 | 1.00 | 29.32 |
| 1360 | CA | LEU | A | 838 | 9.250 | 13.092 | 19.774 | 1.00 | 30.90 |
| 1361 | C | LEU | A | 838 | 7.740 | 13.211 | 19.884 | 1.00 | 32.66 |
| 1362 | O | LEU | A | 838 | 7.076 | 13.706 | 18.983 | 1.00 | 32.60 |
| 1363 | CB | LEU | A | 838 | 9.614 | 11.622 | 19.592 | 1.00 | 30.44 |
| 1364 | CG | LEU | A | 838 | 8.810 | 10.983 | 18.460 | 1.00 | 30.56 |
| 1365 | CD1 | LEU | A | 838 | 9.077 | 11.728 | 17.151 | 1.00 | 30.31 |
| 1366 | CD2 | LEU | A | 838 | 9.166 | 9.533 | 18.330 | 1.00 | 30.00 |
| 1367 | N | ASP | A | 839 | 7.186 | 12.724 | 20.979 | 1.00 | 34.82 |
| 1368 | CA | ASP | A | 839 | 5.755 | 12.823 | 21.162 | 1.00 | 37.61 |
| 1369 | C | ASP | A | 839 | 5.338 | 14.293 | 21.187 | 1.00 | 38.83 |
| 1370 | O | ASP | A | 839 | 4.285 | 14.645 | 20.672 | 1.00 | 39.02 |
| 1371 | CB | ASP | A | 839 | 5.331 | 12.117 | 22.449 | 1.00 | 39.22 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1372 | CG | ASP | A | 839 | 3.816 | 11.952 | 22.557 | 1.00 | 41.54 |
| 1373 | OD1 | ASP | A | 839 | 3.249 | 12.375 | 23.592 | 1.00 | 43.10 |
| 1374 | OD2 | ASP | A | 839 | 3.192 | 11.400 | 21.613 | 1.00 | 42.60 |
| 1375 | N | ARG | A | 840 | 6.195 | 15.151 | 21.731 | 1.00 | 40.51 |
| 1376 | CA | ARG | A | 840 | 5.916 | 16.580 | 21.828 | 1.00 | 42.20 |
| 1377 | C | ARG | A | 840 | 5.776 | 17.220 | 20.457 | 1.00 | 43.27 |
| 1378 | O | ARG | A | 840 | 4.860 | 18.004 | 20.232 | 1.00 | 43.50 |
| 1379 | CB | ARG | A | 840 | 7.032 | 17.289 | 22.610 | 1.00 | 43.32 |
| 1380 | CG | ARG | A | 840 | 6.657 | 18.639 | 23.261 | 1.00 | 45.47 |
| 1381 | CD | ARG | A | 840 | 6.945 | 19.881 | 22.401 | 1.00 | 46.95 |
| 1382 | NE | ARG | A | 840 | 8.319 | 20.371 | 22.542 | 1.00 | 48.57 |
| 1383 | CZ | ARG | A | 840 | 9.066 | 20.823 | 21.533 | 1.00 | 49.57 |
| 1384 | NH1 | ARG | A | 840 | 8.580 | 20.860 | 20.294 | 1.00 | 49.89 |
| 1385 | NH2 | ARG | A | 840 | 10.314 | 21.220 | 21.755 | 1.00 | 49.90 |
| 1386 | N | ILE | A | 841 | 6.663 | 16.876 | 19.528 | 1.00 | 44.53 |
| 1387 | CA | ILE | A | 841 | 6.600 | 17.483 | 18.211 | 1.00 | 46.22 |
| 1388 | C | ILE | A | 841 | 5.534 | 16.913 | 17.286 | 1.00 | 48.04 |
| 1389 | O | ILE | A | 841 | 5.472 | 17.272 | 16.109 | 1.00 | 48.90 |
| 1390 | CB | ILE | A | 841 | 7.983 | 17.572 | 17.510 | 1.00 | 45.82 |
| 1391 | CG1 | ILE | A | 841 | 8.383 | 16.237 | 16.918 | 1.00 | 46.46 |
| 1392 | CG2 | ILE | A | 841 | 9.044 | 18.078 | 18.463 | 1.00 | 46.40 |
| 1393 | CD1 | ILE | A | 841 | 8.064 | 16.150 | 15.463 | 1.00 | 45.92 |
| 1394 | N | ILE | A | 842 | 4.737 | 15.976 | 17.786 | 1.00 | 49.91 |
| 1395 | CA | ILE | A | 842 | 3.632 | 15.446 | 16.990 | 1.00 | 51.38 |
| 1396 | C | ILE | A | 842 | 2.384 | 16.003 | 17.659 | 1.00 | 52.63 |
| 1397 | O | ILE | A | 842 | 1.509 | 16.551 | 16.999 | 1.00 | 52.38 |
| 1398 | CB | ILE | A | 842 | 3.577 | 13.878 | 16.889 | 1.00 | 51.11 |
| 1399 | CG1 | ILE | A | 842 | 4.523 | 13.192 | 17.870 | 1.00 | 50.67 |
| 1400 | CG2 | ILE | A | 842 | 3.917 | 13.445 | 15.482 | 1.00 | 51.75 |
| 1401 | CD1 | ILE | A | 842 | 4.691 | 11.701 | 17.619 | 1.00 | 49.36 |
| 1402 | N | ALA | A | 843 | 2.356 | 15.939 | 18.986 | 1.00 | 54.81 |
| 1403 | CA | ALA | A | 843 | 1.242 | 16.456 | 19.761 | 1.00 | 57.24 |
| 1404 | C | ALA | A | 843 | 1.215 | 17.962 | 19.557 | 1.00 | 59.28 |
| 1405 | O | ALA | A | 843 | 1.847 | 18.704 | 20.304 | 1.00 | 59.69 |
| 1406 | CB | ALA | A | 843 | 1.424 | 16.129 | 21.247 | 1.00 | 56.69 |
| 1407 | N | CYS | A | 844 | 0.560 | 18.391 | 18.481 | 1.00 | 61.60 |
| 1408 | CA | CYS | A | 844 | 0.402 | 19.810 | 18.130 | 1.00 | 63.67 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1409 | C | CYS | A | 844 | -0.441 | 19.854 | 16.848 | 1.00 | 64.63 |
| 1410 | O | CYS | A | 844 | -1.618 | 19.471 | 16.889 | 1.00 | 64.70 |
| 1411 | CB | CYS | A | 844 | 1.766 | 20.536 | 17.979 | 1.00 | 64.05 |
| 1412 | SG | CYS | A | 844 | 2.751 | 20.268 | 16.470 | 1.00 | 65.30 |
| 1413 | N | LYS | A | 845 | 0.136 | 20.332 | 15.738 | 1.00 | 65.65 |
| 1414 | CA | LYS | A | 845 | -0.545 | 20.374 | 14.439 | 1.00 | 65.96 |
| 1415 | C | LYS | A | 845 | -0.079 | 19.165 | 13.644 | 1.00 | 66.49 |
| 1416 | O | LYS | A | 845 | -0.675 | 18.829 | 12.620 | 1.00 | 66.85 |
| 1417 | CB | LYS | A | 845 | -0.195 | 21.639 | 13.684 | 1.00 | 65.80 |
| 1418 | N | ARG | A | 846 | 0.998 | 18.533 | 14.127 | 1.00 | 66.74 |
| 1419 | CA | ARG | A | 846 | 1.601 | 17.343 | 13.511 | 1.00 | 66.90 |
| 1420 | C | ARG | A | 846 | 0.984 | 16.074 | 14.086 | 1.00 | 66.88 |
| 1421 | O | ARG | A | 846 | 1.675 | 15.092 | 14.345 | 1.00 | 66.35 |
| 1422 | CB | ARG | A | 846 | 3.110 | 17.337 | 13.730 | 1.00 | 66.74 |
| 1423 | N | LYS | A | 847 | -0.325 | 16.141 | 14.291 | 1.00 | 67.27 |
| 1424 | CA | LYS | A | 847 | -1.162 | 15.076 | 14.826 | 1.00 | 67.87 |
| 1425 | C | LYS | A | 847 | -2.420 | 15.816 | 15.251 | 1.00 | 68.35 |
| 1426 | O | LYS | A | 847 | -2.432 | 17.046 | 15.278 | 1.00 | 68.33 |
| 1427 | CB | LYS | A | 847 | -0.515 | 14.407 | 16.033 | 1.00 | 68.14 |
| 1428 | N | ASN | A | 848 | -3.468 | 15.079 | 15.597 | 1.00 | 69.11 |
| 1429 | CA | ASN | A | 848 | -4.728 | 15.685 | 16.016 | 1.00 | 69.71 |
| 1430 | C | ASN | A | 848 | -5.737 | 14.586 | 16.340 | 1.00 | 70.20 |
| 1431 | O | ASN | A | 848 | -5.342 | 13.474 | 16.720 | 1.00 | 70.21 |
| 1432 | CB | ASN | A | 848 | -5.272 | 16.598 | 14.907 | 1.00 | 69.68 |
| 1433 | N | PRO | A | 849 | -7.021 | 14.914 | 16.146 | 1.00 | 70.54 |
| 1434 | CA | PRO | A | 849 | -8.185 | 14.043 | 16.374 | 1.00 | 70.31 |
| 1435 | C | PRO | A | 849 | -7.856 | 12.661 | 16.920 | 1.00 | 69.92 |
| 1436 | O | PRO | A | 849 | -7.665 | 12.492 | 18.130 | 1.00 | 70.12 |
| 1437 | CB | PRO | A | 849 | -9.014 | 13.929 | 15.079 | 1.00 | 70.65 |
| 1438 | N | THR | A | 850 | -7.808 | 11.680 | 16.020 | 1.00 | 69.47 |
| 1439 | CA | THR | A | 850 | -7.473 | 10.304 | 16.377 | 1.00 | 68.74 |
| 1440 | C | THR | A | 850 | -6.061 | 10.015 | 15.845 | 1.00 | 67.76 |
| 1441 | O | THR | A | 850 | -5.590 | 8.867 | 15.864 | 1.00 | 67.27 |
| 1442 | CB | THR | A | 850 | -8.494 | 9.331 | 15.774 | 1.00 | 68.95 |
| 1443 | N | SER | A | 851 | -5.391 | 11.077 | 15.388 | 1.00 | 66.46 |
| 1444 | CA | SER | A | 851 | -4.046 | 10.962 | 14.846 | 1.00 | 65.28 |
| 1445 | C | SER | A | 851 | -3.023 | 10.710 | 15.944 | 1.00 | 64.16 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1446 | O | SER | A | 851 | -2.426 | 9.639 | 15.969 | 1.00 | 64.16 |
| 1447 | CB | SER | A | 851 | -3.664 | 12.200 | 14.018 | 1.00 | 65.24 |
| 1448 | OG | SER | A | 851 | -2.405 | 12.037 | 13.369 | 1.00 | 65.05 |
| 1449 | N | CYS | A | 852 | -2.873 | 11.651 | 16.879 | 1.00 | 62.68 |
| 1450 | CA | CYS | A | 852 | -1.901 | 11.526 | 17.974 | 1.00 | 60.96 |
| 1451 | C | CYS | A | 852 | -1.480 | 10.102 | 18.388 | 1.00 | 59.71 |
| 1452 | O | CYS | A | 852 | -0.282 | 9.800 | 18.422 | 1.00 | 59.67 |
| 1453 | CB | CYS | A | 852 | -2.353 | 12.333 | 19.191 | 1.00 | 61.34 |
| 1454 | SG | CYS | A | 852 | -1.712 | 14.024 | 19.216 | 1.00 | 62.06 |
| 1455 | N | SER | A | 853 | -2.440 | 9.223 | 18.678 | 1.00 | 57.85 |
| 1456 | CA | SER | A | 853 | -2.109 | 7.847 | 19.053 | 1.00 | 55.45 |
| 1457 | C | SER | A | 853 | -1.610 | 7.052 | 17.847 | 1.00 | 53.09 |
| 1458 | O | SER | A | 853 | -0.601 | 6.346 | 17.942 | 1.00 | 53.22 |
| 1459 | CB | SER | A | 853 | -3.308 | 7.133 | 19.686 | 1.00 | 56.27 |
| 1460 | OG | SER | A | 853 | -3.480 | 7.522 | 21.038 | 1.00 | 57.65 |
| 1461 | N | ARG | A | 854 | -2.304 | 7.172 | 16.719 | 1.00 | 49.75 |
| 1462 | CA | ARG | A | 854 | -1.924 | 6.462 | 15.500 | 1.00 | 46.39 |
| 1463 | C | ARG | A | 854 | -0.564 | 6.962 | 14.967 | 1.00 | 43.72 |
| 1464 | O | ARG | A | 854 | 0.294 | 6.164 | 14.564 | 1.00 | 43.80 |
| 1465 | CB | ARG | A | 854 | -3.021 | 6.634 | 14.443 | 1.00 | 47.23 |
| 1466 | CG | ARG | A | 854 | -2.747 | 5.970 | 13.105 | 1.00 | 48.09 |
| 1467 | CD | ARG | A | 854 | -2.985 | 6.943 | 11.940 | 1.00 | 49.85 |
| 1468 | NE | ARG | A | 854 | -4.340 | 7.500 | 11.936 | 1.00 | 51.11 |
| 1469 | CZ | ARG | A | 854 | -4.911 | 8.122 | 10.904 | 1.00 | 51.20 |
| 1470 | NH1 | ARG | A | 854 | -4.261 | 8.290 | 9.755 | 1.00 | 51.32 |
| 1471 | NH2 | ARG | A | 854 | -6.153 | 8.567 | 11.021 | 1.00 | 51.21 |
| 1472 | N | ARG | A | 855 | -0.360 | 8.273 | 15.019 | 1.00 | 39.68 |
| 1473 | CA | ARG | A | 855 | 0.860 | 8.926 | 14.558 | 1.00 | 36.58 |
| 1474 | C | ARG | A | 855 | 2.106 | 8.454 | 15.306 | 1.00 | 35.02 |
| 1475 | O | ARG | A | 855 | 3.180 | 8.326 | 14.723 | 1.00 | 34.97 |
| 1476 | CB | ARG | A | 855 | 0.713 | 10.439 | 14.734 | 1.00 | 36.42 |
| 1477 | CG | ARG | A | 855 | 1.801 | 11.264 | 14.082 | 1.00 | 34.79 |
| 1478 | CD | ARG | A | 855 | 1.724 | 11.109 | 12.594 | 1.00 | 32.86 |
| 1479 | NE | ARG | A | 855 | 2.869 | 11.699 | 11.907 | 1.00 | 30.54 |
| 1480 | CZ | ARG | A | 855 | 3.030 | 11.655 | 10.590 | 1.00 | 29.61 |
| 1481 | NH1 | ARG | A | 855 | 2.130 | 11.051 | 9.831 | 1.00 | 28.88 |
| 1482 | NH2 | ARG | A | 855 | 4.085 | 12.219 | 10.028 | 1.00 | 29.20 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1483 | N | PHE | A | 856 | 1.973 | 8.239 | 16.609 | 1.00 | 33.30 |
| 1484 | CA | PHE | A | 856 | 3.086 | 7.786 | 17.428 | 1.00 | 31.21 |
| 1485 | C | PHE | A | 856 | 3.308 | 6.290 | 17.254 | 1.00 | 30.61 |
| 1486 | O | PHE | A | 856 | 4.420 | 5.803 | 17.424 | 1.00 | 30.75 |
| 1487 | CB | PHE | A | 856 | 2.846 | 8.128 | 18.895 | 1.00 | 30.24 |
| 1488 | CG | PHE | A | 856 | 4.058 | 7.977 | 19.755 | 1.00 | 30.06 |
| 1489 | CD1 | PHE | A | 856 | 5.022 | 8.972 | 19.787 | 1.00 | 29.34 |
| 1490 | CD2 | PHE | A | 856 | 4.241 | 6.829 | 20.536 | 1.00 | 29.33 |
| 1491 | CE1 | PHE | A | 856 | 6.150 | 8.832 | 20.580 | 1.00 | 29.36 |
| 1492 | CE2 | PHE | A | 856 | 5.364 | 6.676 | 21.335 | 1.00 | 28.86 |
| 1493 | CZ | PHE | A | 856 | 6.325 | 7.680 | 21.357 | 1.00 | 29.62 |
| 1494 | N | TYR | A | 857 | 2.258 | 5.543 | 16.943 | 1.00 | 29.79 |
| 1495 | CA | TYR | A | 857 | 2.446 | 4.118 | 16.725 | 1.00 | 29.79 |
| 1496 | C | TYR | A | 857 | 3.206 | 3.929 | 15.419 | 1.00 | 29.44 |
| 1497 | O | TYR | A | 857 | 4.135 | 3.125 | 15.371 | 1.00 | 29.44 |
| 1498 | CB | TYR | A | 857 | 1.116 | 3.365 | 16.660 | 1.00 | 30.78 |
| 1499 | CG | TYR | A | 857 | 1.254 | 1.871 | 16.396 | 1.00 | 32.21 |
| 1500 | CD1 | TYR | A | 857 | 1.425 | 0.972 | 17.442 | 1.00 | 32.75 |
| 1501 | CD2 | TYR | A | 857 | 1.208 | 1.362 | 15.098 | 1.00 | 33.36 |
| 1502 | CE1 | TYR | A | 857 | 1.548 | -0.401 | 17.215 | 1.00 | 34.60 |
| 1503 | CE2 | TYR | A | 857 | 1.331 | -0.011 | 14.854 | 1.00 | 34.67 |
| 1504 | CZ | TYR | A | 857 | 1.503 | -0.887 | 15.918 | 1.00 | 35.61 |
| 1505 | OH | TYR | A | 857 | 1.652 | -2.244 | 15.697 | 1.00 | 36.96 |
| 1506 | N | GLN | A | 858 | 2.847 | 4.685 | 14.376 | 1.00 | 28.32 |
| 1507 | CA | GLN | A | 858 | 3.533 | 4.537 | 13.087 | 1.00 | 28.06 |
| 1508 | C | GLN | A | 858 | 4.967 | 5.077 | 13.037 | 1.00 | 26.56 |
| 1509 | O | GLN | A | 858 | 5.820 | 4.525 | 12.332 | 1.00 | 26.58 |
| 1510 | CB | GLN | A | 858 | 2.675 | 5.020 | 11.890 | 1.00 | 28.98 |
| 1511 | CG | GLN | A | 858 | 1.970 | 6.384 | 12.029 | 1.00 | 31.65 |
| 1512 | CD | GLN | A | 858 | 0.781 | 6.569 | 11.059 | 1.00 | 32.26 |
| 1513 | OE1 | GLN | A | 858 | 0.385 | 7.700 | 10.724 | 1.00 | 32.11 |
| 1514 | NE2 | GLN | A | 858 | 0.210 | 5.458 | 10.617 | 1.00 | 32.79 |
| 1515 | N | LEU | A | 859 | 5.266 | 6.101 | 13.825 | 1.00 | 24.64 |
| 1516 | CA | LEU | A | 859 | 6.622 | 6.632 | 13.832 | 1.00 | 22.82 |
| 1517 | C | LEU | A | 859 | 7.545 | 5.705 | 14.613 | 1.00 | 20.92 |
| 1518 | O | LEU | A | 859 | 8.694 | 5.486 | 14.222 | 1.00 | 20.35 |
| 1519 | CB | LEU | A | 859 | 6.675 | 8.067 | 14.395 | 1.00 | 23.93 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1520 | CG | LEU | A | 859 | 6.054 | 9.243 | 13.617 | 1.00 | 23.18 |
| 1521 | CD1 | LEU | A | 859 | 6.616 | 10.541 | 14.156 | 1.00 | 23.42 |
| 1522 | CD2 | LEU | A | 859 | 6.363 | 9.137 | 12.173 | 1.00 | 22.30 |
| 1523 | N | THR | A | 860 | 7.030 | 5.124 | 15.691 | 1.00 | 20.30 |
| 1524 | CA | THR | A | 860 | 7.821 | 4.195 | 16.505 | 1.00 | 20.14 |
| 1525 | C | THR | A | 860 | 7.969 | 2.855 | 15.765 | 1.00 | 20.30 |
| 1526 | O | THR | A | 860 | 8.922 | 2.108 | 15.985 | 1.00 | 20.28 |
| 1527 | CB | THR | A | 860 | 7.215 | 3.957 | 17.905 | 1.00 | 18.65 |
| 1528 | OG1 | THR | A | 860 | 5.849 | 3.551 | 17.797 | 1.00 | 18.55 |
| 1529 | CG2 | THR | A | 860 | 7.314 | 5.196 | 18.734 | 1.00 | 18.12 |
| 1530 | N | LYS | A | 861 | 7.040 | 2.600 | 14.851 | 1.00 | 21.02 |
| 1531 | CA | LYS | A | 861 | 7.046 | 1.411 | 14.034 | 1.00 | 21.82 |
| 1532 | C | LYS | A | 861 | 8.040 | 1.642 | 12.895 | 1.00 | 21.28 |
| 1533 | O | LYS | A | 861 | 8.781 | 0.750 | 12.510 | 1.00 | 21.01 |
| 1534 | CB | LYS | A | 861 | 5.649 | 1.178 | 13.475 | 1.00 | 23.82 |
| 1535 | CG | LYS | A | 861 | 5.375 | -0.268 | 13.110 | 1.00 | 26.71 |
| 1536 | CD | LYS | A | 861 | 5.015 | -1.106 | 14.321 | 1.00 | 28.40 |
| 1537 | CE | LYS | A | 861 | 4.924 | -2.587 | 13.922 | 1.00 | 30.14 |
| 1538 | NZ | LYS | A | 861 | 4.542 | -3.514 | 15.050 | 1.00 | 31.53 |
| 1539 | N | LEU | A | 862 | 8.093 | 2.863 | 12.389 | 1.00 | 21.49 |
| 1540 | CA | LEU | A | 862 | 9.021 | 3.192 | 11.311 | 1.00 | 21.32 |
| 1541 | C | LEU | A | 862 | 10.438 | 3.181 | 11.854 | 1.00 | 21.15 |
| 1542 | O | LEU | A | 862 | 11.386 | 2.884 | 11.133 | 1.00 | 22.30 |
| 1543 | CB | LEU | A | 862 | 8.713 | 4.577 | 10.731 | 1.00 | 22.14 |
| 1544 | CG | LEU | A | 862 | 9.816 | 5.281 | 9.921 | 1.00 | 21.98 |
| 1545 | CD1 | LEU | A | 862 | 10.022 | 4.563 | 8.622 | 1.00 | 22.72 |
| 1546 | CD2 | LEU | A | 862 | 9.456 | 6.726 | 9.670 | 1.00 | 22.58 |
| 1547 | N | LEU | A | 863 | 10.596 | 3.602 | 13.105 | 1.00 | 20.71 |
| 1548 | CA | LEU | A | 863 | 11.906 | 3.625 | 13.749 | 1.00 | 18.90 |
| 1549 | C | LEU | A | 863 | 12.462 | 2.218 | 14.004 | 1.00 | 18.08 |
| 1550 | O | LEU | A | 863 | 13.676 | 1.984 | 13.895 | 1.00 | 17.06 |
| 1551 | CB | LEU | A | 863 | 11.827 | 4.423 | 15.040 | 1.00 | 18.78 |
| 1552 | CG | LEU | A | 863 | 11.890 | 5.931 | 14.863 | 1.00 | 18.02 |
| 1553 | CD1 | LEU | A | 863 | 12.103 | 6.545 | 16.230 | 1.00 | 19.67 |
| 1554 | CD2 | LEU | A | 863 | 13.049 | 6.291 | 13.944 | 1.00 | 16.59 |
| 1555 | N | ASP | A | 864 | 11.592 | 1.307 | 14.436 | 1.00 | 18.33 |
| 1556 | CA | ASP | A | 864 | 11.985 | -0.088 | 14.642 | 1.00 | 19.28 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1557 | C | ASP | A | 864 | 12.467 | -0.692 | 13.321 | 1.00 | 19.06 |
| 1558 | O | ASP | A | 864 | 13.377 | -1.519 | 13.298 | 1.00 | 18.82 |
| 1559 | CB | ASP | A | 864 | 10.797 | -0.917 | 15.143 | 1.00 | 19.27 |
| 1560 | CG | ASP | A | 864 | 10.525 | -0.727 | 16.620 | 1.00 | 19.92 |
| 1561 | OD1 | ASP | A | 864 | 11.256 | 0.045 | 17.271 | 1.00 | 20.94 |
| 1562 | OD2 | ASP | A | 864 | 9.577 | -1.364 | 17.116 | 1.00 | 19.39 |
| 1563 | N | SER | A | 865 | 11.847 | -0.263 | 12.222 | 1.00 | 19.90 |
| 1564 | CA | SER | A | 865 | 12.202 | -0.764 | 10.894 | 1.00 | 19.17 |
| 1565 | C | SER | A | 865 | 13.634 | -0.507 | 10.489 | 1.00 | 18.01 |
| 1566 | O | SER | A | 865 | 14.213 | -1.294 | 9.765 | 1.00 | 18.93 |
| 1567 | CB | SER | A | 865 | 11.226 | -0.289 | 9.798 | 1.00 | 18.59 |
| 1568 | OG | SER | A | 865 | 11.167 | 1.123 | 9.613 | 1.00 | 19.91 |
| 1569 | N | VAL | A | 866 | 14.257 | 0.535 | 11.004 | 1.00 | 17.08 |
| 1570 | CA | VAL | A | 866 | 15.619 | 0.747 | 10.589 | 1.00 | 15.20 |
| 1571 | C | VAL | A | 866 | 16.564 | -0.260 | 11.194 | 1.00 | 14.83 |
| 1572 | O | VAL | A | 866 | 17.625 | -0.518 | 10.641 | 1.00 | 14.66 |
| 1573 | CB | VAL | A | 866 | 16.093 | 2.211 | 10.783 | 1.00 | 15.01 |
| 1574 | CG1 | VAL | A | 866 | 14.982 | 3.081 | 11.320 | 1.00 | 13.81 |
| 1575 | CG2 | VAL | A | 866 | 17.344 | 2.280 | 11.574 | 1.00 | 13.41 |
| 1576 | N | GLN | A | 867 | 16.168 | -0.873 | 12.302 | 1.00 | 15.12 |
| 1577 | CA | GLN | A | 867 | 17.031 | -1.849 | 12.977 | 1.00 | 15.64 |
| 1578 | C | GLN | A | 867 | 17.358 | -3.143 | 12.233 | 1.00 | 14.54 |
| 1579 | O | GLN | A | 867 | 18.487 | -3.594 | 12.271 | 1.00 | 15.92 |
| 1580 | CB | GLN | A | 867 | 16.508 | -2.155 | 14.374 | 1.00 | 16.10 |
| 1581 | CG | GLN | A | 867 | 16.526 | -0.968 | 15.315 | 1.00 | 16.35 |
| 1582 | CD | GLN | A | 867 | 17.910 | -0.474 | 15.672 | 1.00 | 17.91 |
| 1583 | OE1 | GLN | A | 867 | 18.924 | -1.175 | 15.510 | 1.00 | 17.76 |
| 1584 | NE2 | GLN | A | 867 | 17.958 | 0.750 | 16.201 | 1.00 | 17.53 |
| 1585 | N | PRO | A | 868 | 16.364 | -3.809 | 11.634 | 1.00 | 14.35 |
| 1586 | CA | PRO | A | 868 | 16.630 | -5.040 | 10.886 | 1.00 | 13.73 |
| 1587 | C | PRO | A | 868 | 17.500 | -4.704 | 9.674 | 1.00 | 13.45 |
| 1588 | O | PRO | A | 868 | 18.341 | -5.497 | 9.254 | 1.00 | 14.77 |
| 1589 | CB | PRO | A | 868 | 15.232 | -5.465 | 10.415 | 1.00 | 14.35 |
| 1590 | CG | PRO | A | 868 | 14.331 | -4.928 | 11.438 | 1.00 | 14.42 |
| 1591 | CD | PRO | A | 868 | 14.914 | -3.555 | 11.696 | 1.00 | 15.17 |
| 1592 | N | ILE | A | 869 | 17.289 | -3.514 | 9.113 | 1.00 | 13.49 |
| 1593 | CA | ILE | A | 869 | 18.043 | -3.044 | 7.970 | 1.00 | 12.06 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1594 | C | ILE | A | 869 | 19.458 | -2.818 | 8.411 | 1.00 | 11.70 |
| 1595 | O | ILE | A | 869 | 20.356 | -3.302 | 7.755 | 1.00 | 13.01 |
| 1596 | CB | ILE | A | 869 | 17.447 | -1.740 | 7.358 | 1.00 | 12.53 |
| 1597 | CG1 | ILE | A | 869 | 15.998 | -1.973 | 6.928 | 1.00 | 12.25 |
| 1598 | CG2 | ILE | A | 869 | 18.272 | -1.307 | 6.175 | 1.00 | 12.44 |
| 1599 | CD1 | ILE | A | 869 | 15.258 | -0.746 | 6.432 | 1.00 | 11.91 |
| 1600 | N | ALA | A | 870 | 19.655 | -2.254 | 9.610 | 1.00 | 12.19 |
| 1601 | CA | ALA | A | 870 | 21.007 | -1.993 | 10.110 | 1.00 | 11.52 |
| 1602 | C | ALA | A | 870 | 21.758 | -3.287 | 10.350 | 1.00 | 12.90 |
| 1603 | O | ALA | A | 870 | 22.955 | -3.374 | 10.074 | 1.00 | 13.99 |
| 1604 | CB | ALA | A | 870 | 20.971 | -1.189 | 11.375 | 1.00 | 10.62 |
| 1605 | N | ARG | A | 871 | 21.082 | -4.262 | 10.962 | 1.00 | 14.67 |
| 1606 | CA | ARG | A | 871 | 21.659 | -5.577 | 11.226 | 1.00 | 15.30 |
| 1607 | C | ARG | A | 871 | 22.119 | -6.287 | 9.939 | 1.00 | 16.18 |
| 1608 | O | ARG | A | 871 | 23.216 | -6.846 | 9.897 | 1.00 | 16.90 |
| 1609 | CB | ARG | A | 871 | 20.668 | -6.465 | 11.970 | 1.00 | 16.99 |
| 1610 | CG | ARG | A | 871 | 21.317 | -7.789 | 12.304 | 1.00 | 20.44 |
| 1611 | CD | ARG | A | 871 | 20.552 | -8.755 | 13.190 | 1.00 | 22.19 |
| 1612 | NE | ARG | A | 871 | 21.529 | -9.736 | 13.678 | 1.00 | 25.05 |
| 1613 | CZ | ARG | A | 871 | 22.248 | -9.581 | 14.785 | 1.00 | 24.87 |
| 1614 | NH1 | ARG | A | 871 | 22.085 | -8.513 | 15.553 | 1.00 | 26.86 |
| 1615 | NH2 | ARG | A | 871 | 23.221 | -10.425 | 15.059 | 1.00 | 27.12 |
| 1616 | N | GLU | A | 872 | 21.300 | -6.256 | 8.886 | 1.00 | 17.08 |
| 1617 | CA | GLU | A | 872 | 21.669 | -6.874 | 7.595 | 1.00 | 17.70 |
| 1618 | C | GLU | A | 872 | 22.961 | -6.229 | 7.064 | 1.00 | 16.22 |
| 1619 | O | GLU | A | 872 | 23.826 | -6.892 | 6.504 | 1.00 | 16.64 |
| 1620 | CB | GLU | A | 872 | 20.546 | -6.670 | 6.578 | 1.00 | 20.21 |
| 1621 | CG | GLU | A | 872 | 20.070 | -7.920 | 5.827 | 1.00 | 27.32 |
| 1622 | CD | GLU | A | 872 | 19.041 | -7.600 | 4.715 | 1.00 | 31.24 |
| 1623 | OE1 | GLU | A | 872 | 19.199 | -8.069 | 3.544 | 1.00 | 32.65 |
| 1624 | OE2 | GLU | A | 872 | 18.068 | -6.867 | 5.018 | 1.00 | 33.14 |
| 1625 | N | LEU | A | 873 | 23.109 | -4.927 | 7.254 | 1.00 | 15.48 |
| 1626 | CA | LEU | A | 873 | 24.304 | -4.230 | 6.781 | 1.00 | 13.64 |
| 1627 | C | LEU | A | 873 | 25.489 | -4.510 | 7.662 | 1.00 | 13.26 |
| 1628 | O | LEU | A | 873 | 26.621 | -4.541 | 7.185 | 1.00 | 12.91 |
| 1629 | CB | LEU | A | 873 | 24.040 | -2.718 | 6.664 | 1.00 | 13.09 |
| 1630 | CG | LEU | A | 873 | 22.957 | -2.359 | 5.640 | 1.00 | 12.60 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1631 | CD1 | LEU | A | 873 | 22.396 | -0.985 | 5.856 | 1.00 | 13.12 |
| 1632 | CD2 | LEU | A | 873 | 23.511 | -2.529 | 4.229 | 1.00 | 12.29 |
| 1633 | N | HIS | A | 874 | 25.237 | -4.688 | 8.960 | 1.00 | 14.96 |
| 1634 | CA | HIS | A | 874 | 26.297 | -5.011 | 9.935 | 1.00 | 15.73 |
| 1635 | C | HIS | A | 874 | 26.945 | -6.342 | 9.549 | 1.00 | 16.66 |
| 1636 | O | HIS | A | 874 | 28.171 | -6.454 | 9.539 | 1.00 | 16.68 |
| 1637 | CB | HIS | A | 874 | 25.735 | -5.154 | 11.351 | 1.00 | 14.09 |
| 1638 | CG | HIS | A | 874 | 25.513 | -3.860 | 12.062 | 1.00 | 13.53 |
| 1639 | ND1 | HIS | A | 874 | 24.365 | -3.588 | 12.771 | 1.00 | 12.74 |
| 1640 | CD2 | HIS | A | 874 | 26.303 | -2.769 | 12.204 | 1.00 | 12.74 |
| 1641 | CE1 | HIS | A | 874 | 24.451 | -2.397 | 13.313 | 1.00 | 11.45 |
| 1642 | NE2 | HIS | A | 874 | 25.616 | -1.878 | 12.990 | 1.00 | 10.87 |
| 1643 | N | GLN | A | 875 | 26.122 | -7.356 | 9.268 | 1.00 | 18.67 |
| 1644 | CA | GLN | A | 875 | 26.635 | -8.674 | 8.853 | 1.00 | 19.45 |
| 1645 | C | GLN | A | 875 | 27.324 | -8.521 | 7.491 | 1.00 | 18.37 |
| 1646 | O | GLN | A | 875 | 28.428 | -9.022 | 7.294 | 1.00 | 18.65 |
| 1647 | CB | GLN | A | 875 | 25.507 | -9.726 | 8.779 | 1.00 | 21.56 |
| 1648 | CG | GLN | A | 875 | 25.566 | -10.875 | 9.864 | 1.00 | 25.76 |
| 1649 | CD | GLN | A | 875 | 26.681 | -11.938 | 9.671 | 1.00 | 26.74 |
| 1650 | OE1 | GLN | A | 875 | 27.871 | -11.624 | 9.654 | 1.00 | 27.36 |
| 1651 | NE2 | GLN | A | 875 | 26.285 | -13.204 | 9.589 | 1.00 | 27.93 |
| 1652 | N | PHE | A | 876 | 26.737 | -7.724 | 6.597 | 1.00 | 18.47 |
| 1653 | CA | PHE | A | 876 | 27.338 | -7.515 | 5.280 | 1.00 | 18.22 |
| 1654 | C | PHE | A | 876 | 28.689 | -6.871 | 5.403 | 1.00 | 17.76 |
| 1655 | O | PHE | A | 876 | 29.687 | -7.412 | 4.920 | 1.00 | 17.95 |
| 1656 | CB | PHE | A | 876 | 26.453 | -6.641 | 4.377 | 1.00 | 19.25 |
| 1657 | CG | PHE | A | 876 | 26.966 | -6.506 | 2.954 | 1.00 | 19.63 |
| 1658 | CD1 | PHE | A | 876 | 28.038 | -5.675 | 2.657 | 1.00 | 18.97 |
| 1659 | CD2 | PHE | A | 876 | 26.380 | -7.226 | 1.917 | 1.00 | 19.90 |
| 1660 | CE1 | PHE | A | 876 | 28.519 | -5.558 | 1.343 | 1.00 | 20.30 |
| 1661 | CE2 | PHE | A | 876 | 26.857 | -7.113 | 0.597 | 1.00 | 20.70 |
| 1662 | CZ | PHE | A | 876 | 27.926 | -6.281 | 0.310 | 1.00 | 18.82 |
| 1663 | N | THR | A | 877 | 28.741 | -5.732 | 6.086 | 1.00 | 17.85 |
| 1664 | CA | THR | A | 877 | 30.002 | -5.024 | 6.215 | 1.00 | 17.77 |
| 1665 | C | THR | A | 877 | 31.040 | -5.884 | 6.900 | 1.00 | 17.52 |
| 1666 | O | THR | A | 877 | 32.208 | -5.849 | 6.514 | 1.00 | 16.51 |
| 1667 | CB | THR | A | 877 | 29.855 | -3.641 | 6.915 | 1.00 | 18.24 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1668 | OG1 | THR | A | 877 | 30.954 | -2.808 | 6.525 | 1.00 | 19.13 |
| 1669 | CG2 | THR | A | 877 | 29.868 | -3.765 | 8.444 | 1.00 | 17.92 |
| 1670 | N | PHE | A | 878 | 30.634 | -6.610 | 7.943 | 1.00 | 18.06 |
| 1671 | CA | PHE | A | 878 | 31.559 | -7.501 | 8.651 | 1.00 | 19.20 |
| 1672 | C | PHE | A | 878 | 32.176 | -8.567 | 7.725 | 1.00 | 18.91 |
| 1673 | O | PHE | A | 878 | 33.400 | -8.724 | 7.670 | 1.00 | 17.63 |
| 1674 | CB | PHE | A | 878 | 30.863 | -8.201 | 9.805 | 1.00 | 19.53 |
| 1675 | CG | PHE | A | 878 | 31.731 | -9.220 | 10.484 | 1.00 | 20.60 |
| 1676 | CD1 | PHE | A | 878 | 32.681 | -8.829 | 11.414 | 1.00 | 19.88 |
| 1677 | CD2 | PHE | A | 878 | 31.623 | -10.575 | 10.150 | 1.00 | 20.59 |
| 1678 | CE1 | PHE | A | 878 | 33.518 | -9.774 | 12.008 | 1.00 | 22.10 |
| 1679 | CE2 | PHE | A | 878 | 32.454 | -11.532 | 10.733 | 1.00 | 20.21 |
| 1680 | CZ | PHE | A | 878 | 33.403 | -11.138 | 11.660 | 1.00 | 20.82 |
| 1681 | N | ASP | A | 879 | 31.326 | -9.268 | 6.973 | 1.00 | 19.57 |
| 1682 | CA | ASP | A | 879 | 31.800 | -10.301 | 6.054 | 1.00 | 20.02 |
| 1683 | C | ASP | A | 879 | 32.723 | -9.654 | 5.044 | 1.00 | 20.35 |
| 1684 | O | ASP | A | 879 | 33.802 | -10.171 | 4.737 | 1.00 | 20.51 |
| 1685 | CB | ASP | A | 879 | 30.622 | -10.972 | 5.342 | 1.00 | 20.24 |
| 1686 | CG | ASP | A | 879 | 29.693 | -11.724 | 6.307 | 1.00 | 22.04 |
| 1687 | OD1 | ASP | A | 879 | 30.122 | -12.072 | 7.443 | 1.00 | 23.16 |
| 1688 | OD2 | ASP | A | 879 | 28.520 | -11.968 | 5.937 | 1.00 | 21.98 |
| 1689 | N | LEU | A | 880 | 32.342 | -8.472 | 4.580 | 1.00 | 20.77 |
| 1690 | CA | LEU | A | 880 | 33.149 | -7.775 | 3.596 | 1.00 | 20.33 |
| 1691 | C | LEU | A | 880 | 34.529 | -7.496 | 4.136 | 1.00 | 20.40 |
| 1692 | O | LEU | A | 880 | 35.513 | -7.723 | 3.453 | 1.00 | 21.41 |
| 1693 | CB | LEU | A | 880 | 32.484 | -6.471 | 3.180 | 1.00 | 20.23 |
| 1694 | CG | LEU | A | 880 | 33.089 | -5.838 | 1.939 | 1.00 | 18.50 |
| 1695 | CD1 | LEU | A | 880 | 33.310 | -6.886 | 0.855 | 1.00 | 19.38 |
| 1696 | CD2 | LEU | A | 880 | 32.159 | -4.762 | 1.477 | 1.00 | 18.07 |
| 1697 | N | LEU | A | 881 | 34.602 | -7.040 | 5.376 | 1.00 | 20.90 |
| 1698 | CA | LEU | A | 881 | 35.882 | -6.723 | 6.011 | 1.00 | 20.84 |
| 1699 | C | LEU | A | 881 | 36.818 | -7.923 | 6.188 | 1.00 | 21.55 |
| 1700 | O | LEU | A | 881 | 38.055 | -7.806 | 6.107 | 1.00 | 21.03 |
| 1701 | CB | LEU | A | 881 | 35.651 | -6.055 | 7.364 | 1.00 | 19.23 |
| 1702 | CG | LEU | A | 881 | 36.989 | -5.773 | 8.031 | 1.00 | 19.26 |
| 1703 | CD1 | LEU | A | 881 | 37.662 | -4.593 | 7.350 | 1.00 | 19.67 |
| 1704 | CD2 | LEU | A | 881 | 36.810 | -5.514 | 9.500 | 1.00 | 18.92 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1705 | N | ILE | A | 882 | 36.230 | -9.063 | 6.492 | 1.00 | 22.39 |
| 1706 | CA | ILE | A | 882 | 37.013 | -10.265 | 6.671 | 1.00 | 23.63 |
| 1707 | C | ILE | A | 882 | 37.668 | -10.643 | 5.340 | 1.00 | 24.48 |
| 1708 | O | ILE | A | 882 | 38.859 | -10.953 | 5.290 | 1.00 | 24.23 |
| 1709 | CB | ILE | A | 882 | 36.136 | -11.390 | 7.248 | 1.00 | 23.00 |
| 1710 | CG1 | ILE | A | 882 | 35.749 | -11.006 | 8.675 | 1.00 | 22.91 |
| 1711 1 | CG2 | ILE | A | 882 | 36.855 | -12.729 | 7.185 | 1.00 | 22.75 |
| 1712 | CD1 | ILE | A | 882 | 36.922 | -10.412 | 9.491 | 1.00 | 22.94 |
| 1713 | N | LYS | A | 883 | 36.908 | -10.541 | 4.256 | 1.00 | 25.73 |
| 1714 | CA | LYS | A | 883 | 37.441 | -10.868 | 2.945 | 1.00 | 28.25 |
| 1715 | C | LYS | A | 883 | 37.749 | -9.657 | 2.061 | 1.00 | 30.08 |
| 1716 | O | LYS | A | 883 | 37.823 | -9.790 | 0.841 | 1.00 | 30.81 |
| 1717 | CB | LYS | A | 883 | 36.492 | -11.820 | 2.211 | 1.00 | 27.47 |
| 1718 | CG | LYS | A | 883 | 35.140 | -11.240 | 1.932 | 1.00 | 27.17 |
| 1719 | CD | LYS | A | 883 | 34.293 | -12.163 | 1.109 | 1.00 | 27.60 |
| 1720 | CE | LYS | A | 883 | 32.926 | -11.544 | 0.899 | 1.00 | 28.94 |
| 1721 | NZ | LYS | A | 883 | 32.036 | -12.319 | -0.003 | 1.00 | 29.99 |
| 1722 | N | SER | A | 884 | 37.976 | -8.495 | 2.672 | 1.00 | 32.24 |
| 1723 | CA | SER | A | 884 | 38.268 | -7.260 | 1.938 | 1.00 | 33.66 |
| 1724 | C | SER | A | 884 | 39.500 | -7.349 | 1.042 | 1.00 | 35.48 |
| 1725 | O | SER | A | 884 | 39.491 | -6.867 | -0.087 | 1.00 | 35.08 |
| 1726 | CB | SER | A | 884 | 38.440 | -6.106 | 2.921 | 1.00 | 32.96 |
| 1727 | OG | SER | A | 884 | 39.466 | -6.384 | 3.856 | 1.00 | 32.02 |
| 1728 | N | HIS | A | 885 | 40.557 | -7.969 | 1.556 | 1.00 | 38.15 |
| 1729 | CA | HIS | A | 885 | 41.815 | -8.138 | 0.824 | 1.00 | 40.84 |
| 1730 | C | HIS | A | 885 | 41.682 | -9.017 | -0.432 | 1.00 | 41.12 |
| 1731 | O | HIS | A | 885 | 42.563 | -9.010 | -1.288 | 1.00 | 41.51 |
| 1732 | CB | HIS | A | 885 | 42.882 | -8.688 | 1.789 | 1.00 | 43.70 |
| 1733 | CG | HIS | A | 885 | 44.032 | -9.392 | 1.124 | 1.00 | 47.44 |
| 1734 | ND1 | HIS | A | 885 | 45.172 | -8.737 | 0.704 | 1.00 | 49.20 |
| 1735 | CD2 | HIS | A | 885 | 44.240 | -10.707 | 0.860 | 1.00 | 49.14 |
| 1736 | CE1 | HIS | A | 885 | 46.034 | -9.615 | 0.217 | 1.00 | 49.74 |
| 1737 | NE2 | HIS | A | 885 | 45.493 | -10.818 | 0.300 | 1.00 | 50.17 |
| 1738 | N | MET | A | 886 | 40.586 | -9.762 | -0.544 | 1.00 | 41.14 |
| 1739 | CA | MET | A | 886 | 40.372 | -10.639 | -1.686 | 1.00 | 41.17 |
| 1740 | C | MET | A | 886 | 39.455 | -10.074 | -2.761 | 1.00 | 40.68 |
| 1741 | O | MET | A | 886 | 39.535 | -10.476 | -3.923 | 1.00 | 41.66 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1742 | CB | MET | A | 886 | 39.859 | -11.989 | -1.212 | 1.00 | 43.08 |
| 1743 | CG | MET | A | 886 | 40.928 | -12.860 | -0.584 | 1.00 | 45.59 |
| 1744 | SD | MET | A | 886 | 40.175 | -14.113 | 0.457 | 1.00 | 50.78 |
| 1745 | CE | MET | A | 886 | 39.069 | -14.971 | -0.725 | 1.00 | 48.51 |
| 1746 | N | VAL | A | 887 | 38.542 | -9.193 | -2.370 | 1.00 | 39.25 |
| 1747 | CA | VAL | A | 887 | 37.637 | -8.565 | -3.333 | 1.00 | 37.55 |
| 1748 | C | VAL | A | 887 | 38.145 | -7.168 | -3.702 | 1.00 | 37.08 |
| 1749 | O | VAL | A | 887 | 37.484 | -6.444 | -4.442 | 1.00 | 37.26 |
| 1750 | CB | VAL | A | 887 | 36.187 | -8.459 | -2.802 | 1.00 | 37.19 |
| 1751 | CG | VAL | A | 887 | 35.526 | -9.828 | -2.756 | 1.00 | 37.49 |
| 1752 | CG2 | VAL | A | 887 | 36.175 | -7.817 | -1.429 | 1.00 | 36.99 |
| 1753 | N | SER | A | 888 | 39.320 | -6.809 | -3.188 | 1.00 | 35.90 |
| 1754 | CA | SER | A | 888 | 39.955 | -5.515 | -3.437 | 1.00 | 35.05 |
| 1755 | C | SER | A | 888 | 39.216 | -4.290 | -2.898 | 1.00 | 34.27 |
| 1756 | O | SER | A | 888 | 39.402 | -3.179 | -3.396 | 1.00 | 34.78 |
| 1757 | CB | SER | A | 888 | 40.231 | -5.342 | -4.929 | 1.00 | 35.29 |
| 1758 | OG | SER | A | 888 | 41.335 | -6.133 | -5.326 | 1.00 | 36.74 |
| 1759 | N | VAL | A | 889 | 38.391 | -4.485 | -1.875 | 1.00 | 32.78 |
| 1760 | CA | VAL | A | 889 | 37.636 | -3.386 | -1.283 | 1.00 | 31.50 |
| 1761 | C | VAL | A | 889 | 38.410 | -3.002 | -0.064 | 1.00 | 31.36 |
| 1762 | O | VAL | A | 889 | 38.855 | -3.895 | 0.648 | 1.00 | 32.20 |
| 1763 | CB | VAL | A | 889 | 36.244 | -3.857 | -0.772 | 1.00 | 30.79 |
| 1764 | CG1 | VAL | A | 889 | 35.509 | -2.729 | -0.055 | 1.00 | 30.12 |
| 1765 | CG2 | VAL | A | 889 | 35.410 | -4.364 | -1.903 | 1.00 | 30.08 |
| 1766 | N | ASP | A | 890 | 38.692 | -1.724 | 0.156 | 1.00 | 31.10 |
| 1767 | CA | ASP | A | 890 | 39.364 | -1.428 | 1.414 | 1.00 | 30.80 |
| 1768 | C | ASP | A | 890 | 38.629 | -0.493 | 2.326 | 1.00 | 28.51 |
| 1769 | O | ASP | A | 890 | 37.889 | 0.379 | 1.889 | 1.00 | 27.96 |
| 1770 | CB | ASP | A | 890 | 40.849 | -1.093 | 1.296 | 1.00 | 33.89 |
| 1771 | CG | ASP | A | 890 | 41.720 | -1.949 | 2.261 | 1.00 | 35.96 |
| 1772 | OD1 | ASP | A | 890 | 41.248 | -2.314 | 3.373 | 1.00 | 35.86 |
| 1773 | OD2 | ASP | A | 890 | 42.882 | -2.260 | 1.901 | 1.00 | 37.33 |
| 1774 | N | PHE | A | 891 | 38.761 | -0.782 | 3.610 | 1.00 | 26.20 |
| 1775 | CA | PHE | A | 891 | 38.096 | -0.045 | 4.661 | 1.00 | 24.15 |
| 1776 | C | PHE | A | 891 | 39.036 | 0.942 | 5.305 | 1.00 | 24.58 |
| 1777 | O | PHE | A | 891 | 40.150 | 0.574 | 5.695 | 1.00 | 25.30 |
| 1778 | CB | PHE | A | 891 | 37.595 | -1.027 | 5.732 | 1.00 | 20.51 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1779 | CG | PHE | A | 891 | 36.501 | -1.937 | 5.259 | 1.00 | 16.33 |
| 1780 | CD1 | PHE | A | 891 | 36.741 | -2.892 | 4.288 | 1.00 | 15.42 |
| 1781 | CD2 | PHE | A | 891 | 35.230 | -1.826 | 5.773 | 1.00 | 14.37 |
| 1782 | CE1 | PHE | A | 891 | 35.720 | -3.730 | 3.832 | 1.00 | 13.60 |
| 1783 | CE2 | PHE | A | 891 | 34.220 | -2.648 | 5.335 | 1.00 | 14.04 |
| 1784 | CZ | PHE | A | 891 | 34.467 | -3.607 | 4.353 | 1.00 | 13.43 |
| 1785 | N | PRO | A | 892 | 38.603 | 2.209 | 5.437 | 1.00 | 23.93 |
| 1786 | CA | PRO | A | 892 | 39.441 | 3.234 | 6.060 | 1.00 | 23.41 |
| 1787 | C | PRO | A | 892 | 39.655 | 2.866 | 7.520 | 1.00 | 23.31 |
| 1788 | O | PRO | A | 892 | 38.887 | 2.090 | 8.078 | 1.00 | 22.72 |
| 1789 | CB | PRO | A | 892 | 38.582 | 4.485 | 5.940 | 1.00 | 23.21 |
| 1790 | CG | PRO | A | 892 | 37.796 | 4.241 | 4.748 | 1.00 | 23.19 |
| 1791 | CD | PRO | A | 892 | 37.376 | 2.811 | 4.909 | 1.00 | 22.44 |
| 1792 | N | GLU | A | 893 | 40.619 | 3.517 | 8.157 | 1.00 | 24.55 |
| 1793 | CA | GLU | A | 893 | 40.984 | 3.267 | 9.555 | 1.00 | 26.50 |
| 1794 | C | GLU | A | 893 | 39.859 | 3.054 | 10.563 | 1.00 | 26.24 |
| 1795 | O | GLU | A | 893 | 39.750 | 1.992 | 11.180 | 1.00 | 27.29 |
| 1796 | CB | GLU | A | 893 | 41.885 | 4.385 | 10.072 | 1.00 | 28.90 |
| 1797 | CG | GLU | A | 893 | 42.329 | 4.192 | 11.509 | 1.00 | 33.98 |
| 1798 | CD | GLU | A | 893 | 42.441 | 5.498 | 12.280 | 1.00 | 37.38 |
| 1799 | OE1 | GLU | A | 893 | 43.356 | 6.292 | 11.955 | 1.00 | 39.69 |
| 1800 | OE2 | GLU | A | 893 | 41.624 | 5.729 | 13.216 | 1.00 | 39.34 |
| 1801 | N | MET | A | 894 | 39.052 | 4.078 | 10.782 | 1.00 | 26.07 |
| 1802 | CA | MET | A | 894 | 37.968 | 3.974 | 11.744 | 1.00 | 26.28 |
| 1803 | C | MET | A | 894 | 36.927 | 2.918 | 11.393 | 1.00 | 24.69 |
| 1804 | O | MET | A 894 | | 36.337 | 2.311 | 12.287 | 1.00 | 24.64 |
| 1805 | CB | MET | A | 894 | 37.313 | 5.337 | 11.954 | 1.00 | 28.30 |
| 1806 | CG | MET | A | 894 | 38.256 | 6.389 | 12.509 | 1.00 | 32.56 |
| 1807 | SD | MET | A | 894 | 38.847 | 5.925 | 14.144 | 1.00 | 38.01 |
| 1808 | CE | MET | A | 894 | 37.260 | 5.830 | 15.037 | 1.00 | 35.95 |
| 1809 | N | MET | A 895 | | 36.662 | 2.743 | 10.102 | 1.00 | 23.64 |
| 1810 | CA | MET | A | 895 | 35.705 | 1.738 | 9.645 | 1.00 | 22.83 |
| 1811 | C | MET | A | 895 | 36.171 | 0.328 | 10.032 | 1.00 | 22.26 |
| 1812 | O | MET | A | 895 | 35.469 | -0.383 | 10.714 | 1.00 | 22.26 |
| 1813 | CB | MET | A | 895 | 35.487 | 1.824 | 8.135 | 1.00 | 21.32 |
| 1814 | CG | MET | A | 895 | 34.669 | 3.006 | 7.693 | 1.00 | 21.17 |
| 1815 | SD | MET | A | 895 | 33.044 | 3.064 | 8.432 | 1.00 | 20.56 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1816 | CE | MET | A | 895 | 32.088 | 2.305 | 7.205 | 1.00 | 22.81 |
| 1817 | N | ALA | A | 896 | 37.362 | -0.066 | 9.616 | 1.00 | 22.06 |
| 1818 | CA | ALA | A | 896 | 37.867 | -1.378 | 9.953 | 1.00 | 22.36 |
| 1819 | C | ALA | A | 896 | 37.914 | -1.581 | 11.460 | 1.00 | 22.96 |
| 1820 | O | ALA | A | 896 | 37.520 | -2.630 | 11.947 | 1.00 | 23.87 |
| 1821 | CB | ALA | A | 896 | 39.243 | -1.588 | 9.350 | 1.00 | 22.56 |
| 1822 | N | GLU | A | 897 | 38.377 | -0.586 | 12.212 | 1.00 | 23.92 |
| 1823 | CA | GLU | A | 897 | 38.455 | -0.724 | 13.666 | 1.00 | 24.05 |
| 1824 | C | GLU | A | 897 | 37.076 | -0.901 | 14.276 | 1.00 | 22.80 |
| 1825 | O | GLU | A | 897 | 36.873 | -1.774 | 15.094 | 1.00 | 22.95 |
| 1826 | CB | GLU | A | 897 | 39.128 | 0.502 | 14.313 | 1.00 | 25.98 |
| 1827 | CG | GLU | A | 897 | 39.288 | 0.390 | 15.841 | 1.00 | 27.50 |
| 1828 | CD | GLU | A | 897 | 39.150 | 1.718 | 16.555 | 1.00 | 27.88 |
| 1829 | OE1 | GLU | A | 897 | 40.150 | 2.453 | 16.674 | 1.00 | 29.49 |
| 1830 | OE2 | GLU | A | 897 | 38.036 | 2.018 | 17.013 | 1.00 | 29.22 |
| 1831 | N | ILE | A | 898 | 36.129 | -0.071 | 13.884 | 1.00 | 22.19 |
| 1832 | CA | ILE | A | 898 | 34.801 | -0.178 | 14.459 | 1.00 | 21.88 |
| 1833 | C | ILE | A | 898 | 34.080 | -1.398 | 13.968 | 1.00 | 20.49 |
| 1834 | O | ILE | A | 898 | 33.228 | -1.917 | 14.656 | 1.00 | 21.90 |
| 1835 | CB | ILE | A | 898 | 33.940 | 1.077 | 14.196 | 1.00 | 21.85 |
| 1836 | CG1 | ILE | A | 898 | 34.438 | 2.233 | 15.043 | 1.00 | 22.82 |
| 1837 | CG2 | ILE | A | 898 | 32.478 | 0.836 | 14.587 | 1.00 | 22.66 |
| 1838 | CD1 | ILE | A | 898 | 33.490 | 3.390 | 15.019 | 1.00 | 23.11 |
| 1839 | N | ILE | A | 899 | 34.410 | -1.860 | 12.781 | 1.00 | 19.59 |
| 1840 | CA | ILE | A | 899 | 33.747 | -3.027 | 12.248 | 1.00 | 19.42 |
| 1841 | C | ILE | A | 899 | 34.305 | -4.338 | 12.832 | 1.00 | 19.02 |
| 1842 | O | ILE | A | 899 | 33.571 | -5.300 | 12.982 | 1.00 | 19.98 |
| 1843 | CB | ILE | A | 899 | 33.758 | -3.014 | 10.706 | 1.00 | 19.12 |
| 1844 | CG1 | ILE | A | 899 | 32.987 | -1.786 | 10.187 | 1.00 | 18.56 |
| 1845 | CG2 | ILE | A | 899 | 33.095 | -4.285 | 10.157 | 1.00 | 18.71 |
| 1846 | CD1 | ILE | A | 899 | 33.054 | -1.588 | 8.683 | 1.00 | 15.05 |
| 1847 | N | SER | A | 900 | 35.565 | -4.344 | 13.233 | 1.00 | 19.03 |
| 1848 | CA | SER | A | 900 | 36.177 | -5.518 | 13.822 | 1.00 | 19.74 |
| 1849 | C | SER | A | 900 | 36.135 | -5.502 | 15.355 | 1.00 | 20.48 |
| 1850 | O | SER | A | 900 | 36.352 | -6.521 | 16.010 | 1.00 | 21.19 |
| 1851 | CB | SER | A | 900 | 37.614 | -5.631 | 13.340 | 1.00 | 19.62 |
| 1852 | OG | SER | A | 900 | 38.368 | -4.478 | 13.683 | 1.00 | 22.08 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1853 | N | VAL | A | 901 | 35.866 | -4.346 | 15.939 | 1.00 | 20.99 |
| 1854 | CA | VAL | A | 901 | 35.808 | -4.235 | 17.396 | 1.00 | 20.43 |
| 1855 | C | VAL | A | 901 | 34.397 | -4.087 | 17.935 | 1.00 | 20.42 |
| 1856 | O | VAL | A | 901 | 33.999 | -4.823 | 18.841 | 1.00 | 21.68 |
| 1857 | CB | VAL | A | 901 | 36.705 | -3.074 | 17.927 | 1.00 | 20.22 |
| 1858 | CG1 | VAL | A | 901 | 36.407 | -2.785 | 19.382 | 1.00 | 20.37 |
| 1859 | CG2 | VAL | A | 901 | 38.168 | -3.436 | 17.782 | 1.00 | 18.81 |
| 1860 | N | GLN | A | 902 | 33.614 | -3.187 | 17.350 | 1.00 | 19.34 |
| 1861 | CA | GLN | A | 902 | 32.264 | -2.957 | 17.828 | 1.00 | 17.55 |
| 1862 | C | GLN | A | 902 | 31.145 | -3.766 | 17.207 | 1.00 | 16.24 |
| 1863 | O | GLN | A | 902 | 30.337 | -4.326 | 17.938 | 1.00 | 15.40 |
| 1864 | CB | GLN | A | 902 | 31.929 | -1.476 | 17.735 | 1.00 | 19.32 |
| 1865 | CG | GLN | A | 902 | 32.952 | -0.579 | 18.371 | 1.00 | 20.82 |
| 1866 | CD | GLN | A | 902 | 33.089 | -0.776 | 19.861 | 1.00 | 23.15 |
| 1867 | OE1 | GLN | A | 902 | 32.211 1 | -1.336 | 20.528 | 1.00 | 23.22 |
| 1868 | NE2 | GLN | A | 902 | 34.197 | -0.288 | 20.404 | 1.00 | 25.36 |
| 1869 | N | VAL | A | 903 | 31.075 | -3.810 | 15.872 | 1.00 | 15.79 |
| 1870 | CA | VAL | A | 903 | 30.025 | -4.552 | 15.144 | 1.00 | 15.22 |
| 1871 | C | VAL | A | 903 | 29.860 | -6.010 | 15.605 | 1.00 | 14.74 |
| 1872 | O | VAL | A | 903 | 28.732 | -6.489 | 15.693 | 1.00 | 14.48 |
| 1873 | CB | VAL | A | 903 | 30.195 | -4.461 | 13.594 | 1.00 | 14.30 |
| 1874 | CG1 | VAL | A | 903 | 29.159 | -5.314 | 12.883 | 1.00 | 13.20 |
| 1875 | CG2 | VAL | A | 903 | 30.012 | -3.005 | 13.147 | 1.00 | 14.90 |
| 1876 | N | PRO | A | 904 | 30.976 | -6.729 | 15.893 | 1.00 | 14.65 |
| 1877 | CA | PRO | A | 904 | 30.884 | -8.122 | 16.356 | 1.00 | 15.80 |
| 1878 | C | PRO | A | 904 | 30.053 | -8.206 | 17.632 | 1.00 | 16.77 |
| 1879 | O | PRO | A | 904 | 29.151 | -9.039 | 17.713 | 1.00 | 18.38 |
| 1880 | CB | PRO | A | 904 | 32.350 | -8.481 | 16.602 | 1.00 | 15.45 |
| 1881 | CG | PRO | A | 904 | 33.014 | -7.830 | 15.512 | 1.00 | 14.60 |
| 1882 | CD | PRO | A | 904 | 32.377 | -6.425 | 15.571 | 1.00 | 13.72 |
| 1883 | N | LYS | A | 905 | 30.286 | -7.295 | 18.589 | 1.00 | 17.00 |
| 1884 | CA | LYS | A | 905 | 29.525 | -7.292 | 19.830 | 1.00 | 16.34 |
| 1885 | C | LYS | A | 905 | 28.039 | -7.273 | 19.546 | 1.00 | 15.58 |
| 1886 | O | LYS | A | 905 | 27.251 | -7.817 | 20.297 | 1.00 | 15.43 |
| 1887 | CB | LYS | A | 905 | 29.866 | -6.085 | 20.668 | 1.00 | 18.17 |
| 1888 | CG | LYS | A | 905 | 31.293 | -6.007 | 21.132 | 1.00 | 19.96 |
| 1889 | CD | LYS | A | 905 | 31.464 | -4.733 | 21.947 | 1.00 | 22.09 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1890 | CE | LYS | A | 905 | 32.911 | -4.429 | 22.276 | 1.00 | 23.59 |
| 1891 | NZ | LYS | A | 905 | 33.003 | -3.173 | 23.083 | 1.00 | 27.13 |
| 1892 | N | ILE | A | 906 | 27.647 | -6.620 | 18.466 | 1.00 | 15.71 |
| 1893 | CA | ILE | A | 906 | 26.239 | -6.554 | 18.086 | 1.00 | 15.74 |
| 1894 | C | ILE | A | 906 | 25.800 | -7.899 | 17.478 | 1.00 | 17.05 |
| 1895 | O | ILE | A | 906 | 24.759 | -8.471 | 17.834 | 1.00 | 16.35 |
| 1896 | CB | ILE | A | 906 | 25.991 | -5.423 | 17.030 | 1.00 | 14.76 |
| 1897 | CG1 | ILE | A | 906 | 26.358 | -4.051 | 17.611 | 1.00 | 13.40 |
| 1898 | CG2 | ILE | A | 906 | 24.527 | -5.427 | 16.565 | 1.00 | 13.47 |
| 1899 | CD1 | ILE | A | 906 | 26.021 | -2.876 | 16.686 | 1.00 | 13.18 |
| 1900 | N | LEU | A | 907 | 26.609 | -8.385 | 16.539 | 1.00 | 17.95 |
| 1901 | CA | LEU | A | 907 | 26.348 | -9.631 | 15.827 | 1.00 | 17.64 |
| 1902 | C | LEU | A | 907 | 26.386 | -10.858 | 16.747 | 1.00 | 18.93 |
| 1903 | O | LEU | A | 907 | 25.756 | -11.860 | 16.437 | 1.00 | 20.36 |
| 1904 | CB | LEU | A | 907 | 27.331 | -9.787 | 14.659 | 1.00 | 15.32 |
| 1905 | CG | LEU | A | 907 | 27.338 | -8.653 | 13.632 | 1.00 | 14.36 |
| 1906 | CD1 | LEU | A | 907 | 28.382 | -8.885 | 12.557 | 1.00 | 12.28 |
| 1907 | CD2 | LEU | A | 907 | 25.947 | -8.531 | 13.029 | 1.00 | 13.60 |
| 1908 | N | SER | A | 908 | 27.097 | -10.805 | 17.868 | 1.00 | 19.02 |
| 1909 | CA | SER | A | 908 | 27.103 | -11.947 | 18.772 | 1.00 | 19.60 |
| 1910 | C | SER | A | 908 | 26.027 | -11.844 | 19.867 | 1.00 | 20.23 |
| 1911 | O | SER | A | 908 | 25.946 | -12.709 | 20.752 | 1.00 | 20.86 |
| 1912 | CB | SER | A | 908 | 28.469 | -12.099 | 19.407 | 1.00 | 19.21 |
| 1913 | OG | SER | A | 908 | 28.811 | -10.944 | 20.135 | 1.00 | 19.20 |
| 1914 | N | GLY | A | 909 | 25.208 | -10.791 | 19.812 | 1.00 | 19.30 |
| 1915 | CA | GLY | A | 909 | 24.169 | -10.603 | 20.817 | 1.00 | 18.22 |
| 1916 | C | GLY | A | 909 | 24.533 | -9.859 | 22.102 | 1.00 | 16.28 |
| 1917 | O | GLY | A | 909 | 23.711 | -9.712 | 22.987 | 1.00 | 16.09 |
| 1918 | N | LYS | A | 910 | 25.773 | -9.422 | 22.236 | 1.00 | 16.37 |
| 1919 | CA | LYS | A | 910 | 26.166 | -8.670 | 23.411 | 1.00 | 17.37 |
| 1920 | C | LYS | A | 910 | 25.472 | -7.296 | 23.532 | 1.00 | 18.68 |
| 1921 | O | LYS | A | 910 | 25.250 | -6.797 | 24.640 | 1.00 | 19.51 |
| 1922 | CB | LYS | A | 910 | 27.665 | -8.464 | 23.403 | 1.00 | 17.13 |
| 1923 | CG | LYS | A | 910 | 28.418 | -9.703 | 23.684 | 1.00 | 16.32 |
| 1924 | CD | LYS | A | 910 | 29.860 | -9.370 | 23.896 | 1.00 | 17.48 |
| 1925 | CE | LYS | A | 910 | 30.577 | -10.534 | 24.482 | 1.00 | 17.90 |
| 1926 | NZ | LYS | A | 910 | 32.055 | -10.284 | 24.502 | 1.00 | 19.93 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1927 | N | VAL | A | 911 | 25.219 | -6.641 | 22.397 | 1.00 | 18.99 |
| 1928 | CA | VAL | A | 911 | 24.545 | -5.341 | 22.396 | 1.00 | 17.49 |
| 1929 | C | VAL | A | 911 | 23.379 | -5.475 | 21.458 | 1.00 | 17.48 |
| 1930 | O | VAL | A | 911 | 23.504 | -6.015 | 20.358 | 1.00 | 17.40 |
| 1931 | CB | VAL | A | 911 | 25.501 | -4.130 | 22.041 | 1.00 | 17.52 |
| 1932 | CG1 | VAL | A | 911 | 26.928 | -4.550 | 22.019 | 1.00 | 15.48 |
| 1933 | CG2 | VAL | A | 911 | 25.094 | -3.412 | 20.788 | 1.00 | 15.48 |
| 1934 | N | LYS | A | 912 | 22.219 | -5.032 | 21.896 | 1.00 | 17.96 |
| 1935 | CA | LYS | A | 912 | 21.057 | -5.210 | 21.072 | 1.00 | 19.32 |
| 1936 | C | LYS | A | 912 | 20.262 | -3.943 | 20.903 | 1.00 | 19.72 |
| 1937 | O | LYS | A | 912 | 20.437 | -2.985 | 21.651 | 1.00 | 19.46 |
| 1938 | CB | LYS | A | 912 | 20.189 | -6.325 | 21.672 | 1.00 | 21.17 |
| 1939 | CG | LYS | A | 912 | 19.261 | -5.889 | 22.811 | 1.00 | 25.24 |
| 1940 | CD | LYS | A | 912 | 19.998 | -5.297 | 24.030 | 1.00 | 26.63 |
| 1941 | CE | LYS | A | 912 | 19.509 | -3.871 | 24.370 | 1.00 | 26.56 |
| 1942 | NZ | LYS | A | 912 | 18.028 | -3.782 | 24.457 | 1.00 | 27.08 |
| 1943 | N | PRO | A | 913 | 19.463 | -3.877 | 19.841 | 1.00 | 20.13 |
| 1944 | CA | PRO | A | 913 | 18.693 | -2.665 | 19.683 | 1.00 | 20.09 |
| 1945 | C | PRO | A | 913 | 17.555 | -2.665 | 20.658 | 1.00 | 20.77 |
| 1946 | O | PRO | A | 913 | 17.108 | -3.719 | 21.120 | 1.00 | 20.82 |
| 1947 | CB | PRO | A | 913 | 18.174 | -2.780 | 18.259 | 1.00 | 20.97 |
| 1948 | CG | PRO | A | 913 | 18.127 | -4.240 | 18.017 | 1.00 | 21.02 |
| 1949 | CD | PRO | A | 913 | 19.437 | -4.660 | 18.599 | 1.00 | 20.38 |
| 1950 | N | ILE | A | 914 | 17.094 | -1.460 | 20.972 | 1.00 | 20.62 |
| 1951 | CA | ILE | A | 914 | 15.965 | -1.262 | 21.846 | 1.00 | 18.90 |
| 1952 | C | ILE | A | 914 | 14.823 | -1.093 | 20.858 | 1.00 | 19.73 |
| 1953 | O | ILE | A | 914 | 14.946 | -0.313 | 19.909 | 1.00 | 20.71 |
| 1954 | CB | ILE | A | 914 | 16.119 | 0.012 | 22.659 | 1.00 | 17.34 |
| 1955 | CG1 | ILE | A | 914 | 17.445 | -0.022 | 23.418 | 1.00 | 16.40 |
| 1956 | CG2 | ILE | A | 914 | 14.953 | 0.149 | 23.589 | 1.00 | 15.42 |
| 1957 | CD1 | ILE | A | 914 | 17.794 | 1.261 | 24.098 | 1.00 | 15.82 |
| 1958 | N | TYR | A | 915 | 13.774 | -1.908 | 20.995 | 1.00 | 19.80 |
| 1959 | CA | TYR | A | 915 | 12.622 | -1.823 | 20.105 | 1.00 | 19.03 |
| 1960 | C | TYR | A | 915 | 11.468 | -1.273 | 20.882 | 1.00 | 18.68 |
| 1961 | O | TYR | A | 915 | 11.340 | -1.494 | 22.080 | 1.00 | 18.72 |
| 1962 | CB | TYR | A | 915 | 12.194 | -3.193 | 19.566 | 1.00 | 18.88 |
| 1963 | CG | TYR | A | 915 | 13.072 | -3.773 | 18.505 | 1.00 | 18.76 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 1964 | CD1 | TYR | A | 915 | 14.096 | -4.640 | 18.832 | 1.00 | 19.63 |
| 1965 | CD2 | TYR | A | 915 | 12.881 | -3.457 | 17.173 | 1.00 | 19.64 |
| 1966 | CE1 | TYR | A | 915 | 14.923 | -5.170 | 17.853 | 1.00 | 21.76 |
| 1967 | CE2 | TYR | A | 915 | 13.698 | -3.989 | 16.177 | 1.00 | 20.92 |
| 1968 | CZ | TYR | A | 915 | 14.721 | -4.839 | 16.531 | 1.00 | 21.98 |
| 1969 | OH | TYR | A | 915 | 15.592 | -5.314 | 15.577 | 1.00 | 25.00 |
| 1970 | N | PHE | A | 916 | 10.621 | -0.543 | 20.194 | 1.00 | 18.68 |
| 1971 | CA | PHE | A | 916 | 9.456 | -0.019 | 20.836 | 1.00 | 19.66 |
| 1972 | C | PHE | A | 916 | 8.451 | -1.148 | 20.868 | 1.00 | 21.60 |
| 1973 | O | PHE | A | 916 | 7.862 | -1.434 | 21.910 | 1.00 | 22.04 |
| 1974 | CB | PHE | A | 916 | 8.898 | 1.145 | 20.042 | 1.00 | 17.07 |
| 1975 | CG | PHE | A | 916 | 9.567 | 2.411 | 20.335 | 1.00 | 14.89 |
| 1976 | CD1 | PHE | A | 916 | 9.377 | 3.034 | 21.561 | 1.00 | 16.16 |
| 1977 | CD2 | PHE | A | 916 | 10.393 | 2.992 | 19.407 | 1.00 | 16.16 |
| 1978 | CE1 | PHE | A | 916 | 10.010 | 4.225 | 21.854 | 1.00 | 14.78 |
| 1979 | CE2 | PHE | A | 916 | 11.028 | 4.183 | 19.689 | 1.00 | 16.01 |
| 1980 | CZ | PHE | A | 916 | 10.836 | 4.800 | 20.916 | 1.00 | 15.28 |
| 1981 | N | HIS | A | 917 | 8.300 | -1.804 | 19.718 | 1.00 | 22.86 |
| 1982 | CA | HIS | A | 917 | 7.354 | -2.899 | 19.543 | 1.00 | 24.45 |
| 1983 | C | HIS | A | 917 | 8.077 | -4.225 | 19.477 | 1.00 | 25.83 |
| 1984 | O | HIS | A | 917 | 9.185 | -4.257 | 18.908 | 1.00 | 27.53 |
| 1985 | CB | HIS | A | 917 | 6.549 | -2.696 | 18.258 | 1.00 | 23.60 |
| 1986 | CG | HIS | A | 917 | 5.921 | -1.347 | 18.153 | 1.00 | 21.90 |
| 1987 | ND1 | HIS | A | 917 | 4.614 | -1.109 | 18.504 | 1.00 | 21.41 |
| 1988 | CD2 | HIS | A | 917 | 6.440 | -0.153 | 17.787 | 1.00 | 21.97 |
| 1989 | CE1 | HIS | A | 917 | 4.350 | 0.178 | 18.360 | 1.00 | 22.05 |
| 1990 | NE2 | HIS | A | 917 | 5.446 | 0.783 | 17.929 | 1.00 | 21.26 |
| 1991 | | HIS | A | 917 | | | | | |
| 1992 | C1 | DHT | | 201 | 27.685 | 5.199 | 4.565 | 1.00 | 13.59 |
| 1993 | C2 | DHT | | 201 | 26.814 | 6.485 | 4.636 | 1.00 | 12.55 |
| 1994 | C3 | DHT | | 201 | 25.484 | 6.280 | 3.944 | 1.00 | 12.58 |
| 1995 | 03 | DHT | | 201 | 24.904 | 7.249 | 3.448 | 1.00 | 11.99 |
| 1996 | C4 | DHT | | 201 | 24.887 | 4.964 | 3.857 | 1.00 | 13.18 |
| 1997 | C5 | DHT | | 201 | 25.464 | 3.903 | 4.357 | 1.00 | 13.98 |
| 1998 | C6 | DHT | | 201 | 24.727 | 2.560 | 4.241 | 1.00 | 14.79 |
| 1999 | C7 | DHT | | 201 | 25.613 | 1.454 | 3.609 | 1.00 | 14.79 |
| 2000 | C8 | DHT | | 201 | 26.955 | 1.303 | 4.359 | 1.00 | 15.54 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 2001 | C9 | DHT | | 201 | 27.708 | 2.656 | 4.279 | 1.00 | 14.37 |
| 2002 | C10 | DHT | | 201 | 26.943 | 3.876 | 4.949 | 1.00 | 14.56 |
| 2003 | C11 | DHT | | 201 | 29.161 | 2.525 | 4.830 | 1.00 | 14.73 |
| 2004 | C12 | DHT | | 201 | 29.951 | 1.344 | 4.192 | 1.00 | 14.11 |
| 2005 | C13 | DHT | | 201 | 29.194 | -0.010 | 4.339 | 1.00 | 15.34 |
| 2006 | C14 | DHT | | 201 | 27.784 | 0.212 | 3.680 | 1.00 | 15.67 |
| 2007 | C15 | DHT | | 201 | 27.178 | -1.232 | 3.647 | 1.00 | 15.64 |
| 2008 | C16 | DHT | | 201 | 28.435 | -2.118 | 3.310 | 1.00 | 15.37 |
| 2009 | C17 | DHT | | 201 | 29.679 | -1.189 | 3.426 | 1.00 | 14.87 |
| 2010 | 017 | DHT | | 201 | 30.910 | -1.918 | 3.981 | 1.00 | 16.20 |
| 2011 | C18 | DHT | | 201 | 29.107 | -0.450 | 5.847 | 1.00 | 14.67 |
| 2012 | C19 | DHT | | 201 | 26.781 | 3.770 | 6.524 | 1.00 | 13.94 |
| 2013 | O | HOH | | 1 | 16.187 | 17.463 | 26.217 | 1.00 | 26.98 |
| 2014 | O | HOH | | 2 | 19.878 | 17.183 | 14.290 | 1.00 | 13.49 |
| 2015 | O | HOH | | 3 | 18.473 | 14.908 | 14.407 | 1.00 | 6.52 |
| 2016 | O | HOH | | 4 | 29.144 | 18.703 | 11.673 | 1.00 | 37.40 |
| 2017 | O | HOH | | 5 | 27.076 | 19.321 | 12.893 | 1.00 | 18.76 |
| 2018 | O | HOH | | 6 | 23.789 | 12.817 | 9.649 | 1.00 | 33.78 |
| 2019 | O | HOH | | 7 | 25.400 | 14.577 | 5.432 | 1.00 | 19.79 |
| 2020 | O | HOH | | 8 | 23.015 | 12.473 | 12.245 | 1.00 | 14.03 |
| 2021 | O | HOH | | 9 | 25.209 | 14.445 | 2.442 | 1.00 | 19.95 |
| 2022 | O | HOH | | 10 | 34.235 | 16.490 | 0.235 | 1.00 | 41.09 |
| 2023 | O | HOH | | 11 | 31.687 | 16.720 | 1.143 | 1.00 | 22.88 |
| 2024 | O | HOH | | 12 | 26.451 | 12.094 | 2.237 | 1.00 | 8.25 |
| 2025 | O | HOH | | 13 | 11.606 | -0.191 | -7.963 | 1.00 | 46.13 |
| 2026 | O | HOH | | 14 | 13.798 | 0.894 | 17.657 | 1.00 | 15.30 |
| 2027 | O | HOH | | 15 | 15.475 | 2.114 | 16.386 | 1.00 | 12.01 |
| 2028 | O | HOH | | 16 | 8.514 | -2.110 | 12.665 | 1.00 | 21.79 |
| 2029 | O | HOH | | 17 | 23.094 | 0.783 | 14.094 | 1.00 | 10.94 |
| 2030 | O | HOH | | 18 | 23.758 | -13.306 | 5.541 | 1.00 | 40.43 |
| 2031 | O | HOH | | 19 | 22.933 | -11.472 | 10.611 | 1.00 | 31.03 |
| 2032 | O | HOH | | 20 | 26.094 | -11.914 | 5.354 | 1.00 | 51.71 |
| 2033 | O | HOH | | 21 | 10.995 | -6.843 | 16.294 | 1.00 | 29.91 |
| 2034 | O | HOH | | 22 | 23.088 | 7.362 | -10.811 | 1.00 | 30.10 |
| 2035 | O | HOH | | 23 | 26.671 | 9.139 | -8.686 | 1.00 | 38.12 |
| 2036 | O | HOH | | 24 | 35.410 | -8.438 | -7.084 | 1.00 | 42.68 |
| 2037 | O | HOH | | 25 | 10.842 | 24.253 | 21.391 | 1.00 | 43.09 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 2038 | O | HOH | | 26 | 40.376 | -11.785 | 7.548 | 1.00 | 54.35 |
| 2039 | O | HOH | | 27 | 1.671 | 16.382 | 5.866 | 1.00 | 24.50 |
| 2040 | O | HOH | | 28 | 8.009 | 20.744 | 8.572 | 1.00 | 36.16 |
| 2041 | O | HOH | | 29 | 29.490 | 17.190 | 30.961 | 1.00 | 56.26 |
| 2042 | O | HOH | | 30 | 23.829 | -12.134 | 25.596 | 1.00 | 39.41 |
| 2043 | O | HOH | | 31 | 42.457 | 5.523 | 7.132 | 1.00 | 28.93 |
| 2044 | O | HOH | | 32 | 41.318 | 2.323 | 2.406 | 1.00 | 38.22 |
| 2045 | O | HOH | | 33 | 25.857 | 7.152 | 30.722 | 1.00 | 18.97 |
| 2046 | O | HOH | | 34 | 18.191 | 16.505 | 27.701 | 1.00 | 29.01 |
| 2047 | O | HOH | | 35 | 14.018 | 2.408 | 20.246 | 1.00 | 18.75 |
| 2048 | O | HOH | | 36 | 14.651 | 4.006 | 17.873 | 1.00 | 21.70 |
| 2049 | O | HOH | | 37 | 5.786 | 11.770 | 25.499 | 1.00 | 35.58 |
| 2050 | O | HOH | | 38 | 30.734 | 13.383 | -9.834 | 1.00 | 25.35 |
| 2051 | O | HOH | | 39 | 0.334 | 6.151 | 20.624 | 1.00 | 27.66 |
| 2052 | O | HOH | | 40 | -2.677 | 2.639 | 17.420 | 1.00 | 35.67 |
| 2053 | O | HOH | | 41 | 0.868 | 8.543 | 25.138 | 1.00 | 43.49 |
| 2054 | O | HOH | | 42 | -8.085 | 7.667 | 23.358 | 1.00 | 40.82 |
| 2055 | O | HOH | | 43 | 6.749 | 1.200 | 9.766 | 1.00 | 24.57 |
| 2056 | O | HOH | | 44 | -0.636 | 8.734 | 6.585 | 1.00 | 40.09 |
| 2057 | O | HOH | | 45 | 22.487 | -4.734 | 14.335 | 1.00 | 28.04 |
| 2058 | O | HOH | | 46 | 18.615 | 17.070 | 7.167 | 1.00 | 23.83 |
| 2059 | O | HOH | | 47 | 38.089 | 13.268 | -2.716 | 1.00 | 28.02 |
| 2060 | O | HOH | | 48 | 29.251 | -11.850 | 12.519 | 1.00 | 25.40 |
| 2061 | O | HOH | | 49 | 23.684 | 9.361 | 5.898 | 1.00 | 24.06 |
| 2062 | O | HOH | | 50 | 23.124 | 15.837 | 0.189 | 1.00 | 29.07 |
| 2063 | O | HOH | | 51 | 34.079 | 8.287 | 19.446 | 1.00 | 34.35 |
| 2064 | O | HOH | | 52 | 37.522 | 2.898 | 1.092 | 1.00 | 22.39 |
| 2065 | O | HOH | | 53 | 21.838 | 14.392 | 5.445 | 1.00 | 20.42 |
| 2066 | O | HOH | | 54 | 16.106 | -10.859 | 0.784 | 1.00 | 48.09 |
| 2067 | O | HOH | | 55 | 11.295 | 27.231 | 20.742 | 1.00 | 24.50 |
| 2068 | O | HOH | | 56 | 21.562 | -7.923 | 18.100 | 1.00 | 34.94 |
| 2069 | O | HOH | | 57 | 41.647 | -2.962 | 5.907 | 1.00 | 41.33 |
| 2070 | O | HOH | | 58 | 12.897 | 22.682 | 24.938 | 1.00 | 44.10 |
| 2071 | O | HOH | | 59 | 33.709 | 13.619 | -5.931 | 1.00 | 26.84 |
| 2072 | O | HOH | | 60 | 0.019 | -4.834 | 14.164 | 1.00 | 36.91 |
| 2073 | O | HOH | | 61 | 39.563 | 3.365 | -2.334 | 1.00 | 36.56 |
| 2074 | O | HOH | | 62 | 16.244 | 18.091 | 7.952 | 1.00 | 25.52 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 2075 | O | HOH | | 63 | 13.038 | 13.790 | 19.688 | 1.00 | 21.93 |
| 2076 | O | HOH | | 64 | 22.095 | 3.621 | 21.834 | 1.00 | 19.27 |
| 2077 | O | HOH | | 65 | 25.524 | -2.235 | 39.160 | 1.00 | 30.91 |
| 2078 | O | HOH | | 66 | 25.090 | -1.064 | 36.971 | 1.00 | 31.16 |
| 2079 | O | HOH | | 67 | 5.186 | -1.082 | 5.207 | 1.00 | 26.66 |
| 2080 | O | HOH | | 68 | -0.310 | 15.229 | 24.529 | 1.00 | 28.42 |
| 2081 | O | HOH | | 69 | -6.181 | 9.210 | 18.935 | 1.00 | 37.84 |
| 2082 | O | HOH | | 70 | 17.508 | 26.662 | 14.814 | 1.00 | 30.32 |
| 2083 | O | HOH | | 71 | 17.401 | 31.211 | 13.007 | 1.00 | 30.57 |
| 2084 | O | HOH | | 72 | 21.268 | 22.961 | 10.009 | 1.00 | 33.92 |
| 2085 | O | HOH | | 73 | 26.335 | 12.379 | 6.567 | 1.00 | 36.58 |
| 2086 | O | HOH | | 74 | 33.730 | 15.077 | 4.345 | 1.00 | 24.42 |
| 2087 | O | HOH | | 75 | 28.576 | 2.290 | -15.305 | 1.00 | 30.54 |
| 2088 | O | HOH | | 76 | -5.886 | 5.402 | 21.276 | 1.00 | 48.01 |
| 2089 | O | HOH | | 77 | 31.878 | -6.250 | -10.283 | 1.00 | 38.45 |
| 2090 | O | HOH | | 78 | 30.673 | 18.487 | 18.256 | 1.00 | 34.16 |
| 2091 | O | HOH | | 79 | 35.035 | 20.192 | 15.084 | 1.00 | 37.70 |
| 2092 | O | HOH | | 80 | 32.791 | 17.836 | 19.423 | 1.00 | 35.34 |
| 2093 | O | HOH | | 81 | 22.587 | -14.097 | 7.907 | 1.00 | 30.71 |
| 2094 | O | HOH | | 82 | 29.778 | -9.620 | -0.255 | 1.00 | 24.68 |
| 2095 | O | HOH | | 83 | 25.904 | 17.949 | 24.176 | 1.00 | 16.80 |
| 2096 | O | HOH | | 84 | 33.066 | -13.092 | 7.455 | 1.00 | 20.84 |
| 2097 | O | HOH | | 85 | 31.787 | 15.265 | 28.988 | 1.00 | 32.80 |
| 2098 | O | HOH | | 86 | 27.029 | 0.835 | 13.994 | 1.00 | 20.01 |
| 2099 | O | HOH | | 87 | 20.499 | 2.720 | 16.384 | 1.00 | 31.66 |
| 2100 | O | HOH | | 88 | 10.991 | 16.858 | -1.085 | 1.00 | 30.58 |
| 2101 | O | HOH | | 89 | 7.904 | 10.344 | -5.081 | 1.00 | 41.55 |
| 2102 | O | HOH | | 90 | 12.570 | 3.398 | -10.099 | 1.00 | 26.95 |
| 2103 | O | HOH | | 91 | 17.128 | 3.214 | -10.962 | 1.00 | 22.24 |
| 2104 | O | HOH | | 92 | 17.056 | 1.547 | -4.553 | 1.00 | 26.98 |
| 2105 | O | HOH | | 93 | 11.020 | 0.892 | 6.595 | 1.00 | 25.24 |
| 2106 | O | HOH | | 94 | 24.948 | 1.135 | 35.230 | 1.00 | 27.44 |
| 2107 | O | HOH | | 95 | 24.006 | 5.653 | 35.765 | 1.00 | 34.54 |
| 2108 | O | HOH | | 96 | 29.738 | 0.950 | 27.680 | 1.00 | 26.47 |
| 2109 | O | HOH | | 97 | 1.507 | 8.706 | 22.315 | 1.00 | 36.26 |
| 2110 | O | HOH | | 98 | 10.755 | -4.751 | 9.776 | 1.00 | 27.77 |
| 2111 | O | HOH | | 99 | 20.223 | -3.560 | 14.440 | 1.00 | 25.10 |

TABLE 8 (continued)

| THREE-DIMENSIONAL COORDINATES OF AR IN COMPLEX WITH DHT | | | | | | | | | |
|---|---|---|---|---|---|---|---|---|---|
| ATOM | ATOM TYPE | RESIDUE | # | X | Y | Z | OCC | B | ATOM |
| 2112 | O | HOH | | 100 | 30.147 | -9.103 | 2.467 | 1.00 | 26.08 |
| 2113 | O | HOH | | 101 | 28.518 | -12.565 | -5.152 | 1.00 | 28.96 |
| 2114 | O | HOH | | 102 | 39.044 | 7.751 | 17.961 | 1.00 | 38.02 |
| 2115 | O | HOH | | 103 | 37.030 | 10.428 | 20.994 | 1.00 | 37.73 |
| 2116 | O | HOH | | 104 | 7.847 | -2.227 | 15.270 | 1.00 | 24.79 |
| 2117 | O | HOH | | 105 | 9.958 | -5.351 | 21.522 | 1.00 | 40.62 |
| 2118 | O | HOH | | 106 | 21.201 | 6.928 | -12.688 | 1.00 | 30.96 |

TABLE 9

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1 | N | CYS | A | 669 | 22.921 | 9.448 | 24.884 | 1.00 | 77.12 |
| 2 | CA | CYS | A | 669 | 22.859 | 9.723 | 23.414 | 1.00 | 86.87 |
| 3 | C | CYS | A | 669 | 21.892 | 8.793 | 22.633 | 1.00 | 86.11 |
| 4 | O | CYS | A | 669 | 22.074 | 8.565 | 21.427 | 1.00 | 77.81 |
| 5 | CB | CYS | A | 669 | 24.276 | 9.675 | 22.802 | 1.00 | 88.59 |
| 6 | SG | CYS | A | 669 | 24.838 | 11.217 | 21.992 | 1.00 | 94.18 |
| 7 | N | GLN | A | 670 | 20.835 | 8.318 | 23.308 | 1.00 | 87.01 |
| 8 | CA | GLN | A | 670 | 19.841 | 7.405 | 22.705 | 1.00 | 89.27 |
| 9 | C | GLN | A | 670 | 18.731 | 8.155 | 21.935 | 1.00 | 86.82 |
| 10 | O | GLN | A | 670 | 18.184 | 9.157 | 22.427 | 1.00 | 85.28 |
| 11 | CB | GLN | A | 670 | 19.222 | 6.498 | 23.792 | 1.00 | 93.96 |
| 12 | CG | GLN | A | 670 | 18.335 | 5.330 | 23.282 | 1.00 | 90.90 |
| 13 | CD | GLN | A | 670 | 18.946 | 3.972 | 23.548 | 1.00 | 89.73 |
| 14 | OE1 | GLN | A | 670 | 19.840 | 3.532 | 22.833 | 1.00 | 86.20 |
| 15 | NE2 | GLN | A | 670 | 18.456 | 3.297 | 24.575 | 1.00 | 91.02 |
| 16 | N | PRO | A | 671 | 18.312 | 7.612 | 20.764 | 1.00 | 82.62 |
| 17 | CA | PRO | A | 671 | 17.273 | 8.224 | 19.921 | 1.00 | 76.72 |
| 18 | C | PRO | A | 671 | 15.795 | 8.270 | 20.367 | 1.00 | 71.10 |
| 19 | O | PRO | A | 671 | 14.933 | 8.452 | 19.508 | 1.00 | 75.36 |
| 20 | CB | PRO | A | 671 | 17.414 | 7.449 | 18.599 | 1.00 | 73.17 |
| 21 | CG | PRO | A | 671 | 17.777 | 6.058 | 19.076 | 1.00 | 75.93 |
| 22 | CD | PRO | A | 671 | 18.835 | 6.381 | 20.127 | 1.00 | 80.30 |
| 23 | N | ILE | A | 672 | 15.473 | 8.187 | 21.661 | 1.00 | 59.44 |
| 24 | CA | ILE | A | 672 | 14.045 | 8.210 | 22.043 | 1.00 | 57.16 |
| 25 | C | ILE | A | 672 | 13.232 | 9.465 | 21.682 | 1.00 | 59.85 |
| 26 | O | ILE | A | 672 | 12.069 | 9.358 | 21.234 | 1.00 | 54.31 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 27 | CB | ILE | A | 672 | 13.792 | 7.852 | 23.525 | 1.00 | 54.74 |
| 28 | CG1 | ILE | A | 672 | 12.353 | 8.234 | 23.892 | 1.00 | 47.09 |
| 29 | CG2 | ILE | A | 672 | 14.835 | 8.496 | 24.426 | 1.00 | 64.17 |
| 30 | CD1 | ILE | A | 672 | 11.963 | 7.922 | 25.277 | 1.00 | 56.37 |
| 31 | N | PHE | A | 673 | 13.834 | 10.642 | 21.885 | 1.00 | 61.01 |
| 32 | CA | PHE | A | 673 | 13.167 | 11.909 | 21.583 | 1.00 | 53.54 |
| 33 | C | PHE | A | 673 | 12.960 | 12.039 | 20.068 | 1.00 | 44.47 |
| 34 | O | PHE | A | 673 | 11.858 | 12.376 | 19.585 | 1.00 | 38.12 |
| 35 | CB | PHE | A | 673 | 13.980 | 13.095 | 22.129 | 1.00 | 55.20 |
| 36 | CG | PHE | A | 673 | 13.247 | 14.403 | 22.055 | 1.00 | 54.87 |
| 37 | CD1 | PHE | A | 673 | 12.248 | 14.703 | 22.976 | 1.00 | 54.05 |
| 38 | CD2 | PHE | A | 673 | 13.491 | 15.292 | 21.017 | 1.00 | 47.32 |
| 39 | CE1 | PHE | A | 673 | 11.500 | 15.858 | 22.860 | 1.00 | 53.41 |
| 40 | CE2 | PHE | A | 673 | 12.749 | 16.448 | 20.897 | 1.00 | 49.17 |
| 41 | CZ | PHE | A | 673 | 11.749 | 16.730 | 21.816 | 1.00 | 53.29 |
| 42 | N | LEU | A | 674 | 14.023 | 11.743 | 19.327 | 1.00 | 36.26 |
| 43 | CA | LEU | A | 674 | 13.976 | 11.789 | 17.882 | 1.00 | 38.85 |
| 44 | C | LEU | A | 674 | 12.919 | 10.866 | 17.273 | 1.00 | 38.35 |
| 45 | O | LEU | A | 674 | 12.254 | 11.238 | 16.300 | 1.00 | 39.82 |
| 46 | CB | LEU | A | 674 | 15.358 | 11.487 | 17.303 | 1.00 | 45.56 |
| 47 | CG | LEU | A | 674 | 16.279 | 12.708 | 17.252 | 1.00 | 49.73 |
| 48 | CD1 | LEU | A | 674 | 17.687 | 12.339 | 16.760 | 1.00 | 48.00 |
| 49 | CD2 | LEU | A | 674 | 15.638 | 13.737 | 16.318 | 1.00 | 42.53 |
| 50 | N | ASN | A | 675 | 12.731 | 9.686 | 17.871 | 1.00 | 40.82 |
| 51 | CA | ASN | A | 675 | 11.744 | 8.705 | 17.370 | 1.00 | 41.39 |
| 52 | C | ASN | A | 675 | 10.374 | 9.297 | 17.456 | 1.00 | 39.61 |
| 53 | O | ASN | A | 675 | 9.527 | 9.092 | 16.568 | 1.00 | 33.60 |
| 54 | CB | ASN | A | 675 | 11.734 | 7.434 | 18.209 | 1.00 | 51.05 |
| 55 | CG | ASN | A | 675 | 13.008 | 6.648 | 18.091 | 1.00 | 55.02 |
| 56 | OD1 | ASN | A | 675 | 13.672 | 6.651 | 17.045 | 1.00 | 54.01 |
| 57 | ND2 | ASN | A | 675 | 13.370 | 5.971 | 19.166 | 1.00 | 60.08 |
| 58 | N | VAL | A | 676 | 10.159 | 10.022 | 18.551 | 1.00 | 37.88 |
| 59 | CA | VAL | A | 676 | 8.894 | 10.687 | 18.781 | 1.00 | 36.37 |
| 60 | C | VAL | A | 676 | 8.669 | 11.777 | 17.736 | 1.00 | 39.08 |
| 61 | O | VAL | A | 676 | 7.631 | 11.798 | 17.072 | 1.00 | 43.18 |
| 62 | CB | VAL | A | 676 | 8.821 | 11.336 | 20.153 | 1.00 | 36.30 |
| 63 | CG1 | VAL | A | 676 | 7.421 | 11.860 | 20.358 | 1.00 | 29.55 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 64 | CG2 | VAL | A | 676 | 9.187 | 10.341 | 21.236 | 1.00 | 30.15 |
| 65 | N | LEU | A | 677 | 9.637 | 12.667 | 17.547 | 1.00 | 38.08 |
| 66 | CA | LEU | A | 677 | 9.429 | 13.726 | 16.565 | 1.00 | 37.80 |
| 67 | C | LEU | A | 677 | 9.188 | 13.208 | 15.156 | 1.00 | 38.78 |
| 68 | O | LEU | A | 677 | 8.324 | 13.730 | 14.448 | 1.00 | 44.11 |
| 69 | CB | LEU | A | 677 | 10.571 | 14.726 | 16.574 | 1.00 | 34.97 |
| 70 | CG | LEU | A | 677 | 10.812 | 15.352 | 17.943 | 1.00 | 40.42 |
| 71 | CD1 | LEU | A | 677 | 11.862 | 16.404 | 17.760 | 1.00 | 35.87 |
| 72 | CD2 | LEU | A | 677 | 9.511 | 15.944 | 18.534 | 1.00 | 39.49 |
| 73 | N | GLU | A | 678 | 9.927 | 12.170 | 14.764 | 1.00 | 34.27 |
| 74 | CA | GLU | A | 678 | 9.788 | 11.576 | 13.433 | 1.00 | 33.68 |
| 75 | C | GLU | A | 678 | 8.502 | 10.791 | 13.361 | 1.00 | 31.24 |
| 76 | O | GLU | A | 678 | 7.837 | 10.730 | 12.318 | 1.00 | 29.04 |
| 77 | CB | GLU | A | 678 | 10.972 | 10.692 | 13.139 | 1.00 | 41.54 |
| 78 | CG | GLU | A | 678 | 12.250 | 11.475 | 13.231 | 1.00 | 62.50 |
| 79 | CD | GLU | A | 678 | 13.492 | 10.632 | 13.140 | 1.00 | 75.90 |
| 80 | OE1 | GLU | A | 678 | 13.382 | 9.393 | 13.275 | 1.00 | 81.73 |
| 81 | OE2 | GLU | A | 678 | 14.581 | 11.222 | 12.946 | 1.00 | 77.79 |
| 82 | N | ALA | A | 679 | 8.118 | 10.229 | 14.496 | 1.00 | 27.29 |
| 83 | CA | ALA | A | 679 | 6.878 | 9.486 | 14.561 | 1.00 | 31.51 |
| 84 | C | ALA | A | 679 | 5.658 | 10.400 | 14.416 | 1.00 | 37.88 |
| 85 | O | ALA | A | 679 | 4.657 | 10.013 | 13.784 | 1.00 | 39.80 |
| 86 | CB | ALA | A | 679 | 6.807 | 8.699 | 15.862 | 1.00 | 32.16 |
| 87 | N | ILE | A | 680 | 5.748 | 11.621 | 14.958 | 1.00 | 36.75 |
| 88 | CA | ILE | A | 680 | 4.623 | 12.567 | 14.893 | 1.00 | 33.51 |
| 89 | C | ILE | A | 680 | 4.603 | 13.553 | 13.732 | 1.00 | 29.78 |
| 90 | O | ILE | A | 680 | 3.560 | 14.137 | 13.425 | 1.00 | 35.01 |
| 91 | CB | ILE | A | 680 | 4.445 | 13.322 | 16.204 | 1.00 | 36.86 |
| 92 | CG1 | ILE | A | 680 | 5.672 | 14.178 | 16.493 | 1.00 | 39.01 |
| 93 | CG2 | ILE | A | 680 | 4.222 | 12.324 | 17.343 | 1.00 | 34.87 |
| 94 | CD1 | ILE | A | 680 | 5.503 | 15.046 | 17.719 | 1.00 | 38.54 |
| 95 | N | GLU | A | 681 | 5.732 | 13.677 | 13.044 | 1.00 | 31.29 |
| 96 | CA | GLU | A | 681 | 5.833 | 14.570 | 11.904 | 1.00 | 36.50 |
| 97 | C | GLU | A | 681 | 4.638 | 14.373 | 11.013 | 1.00 | 38.74 |
| 98 | O | GLU | A | 681 | 4.348 | 13.251 | 10.596 | 1.00 | 46.06 |
| 99 | CB | GLU | A | 681 | 7.101 | 14.285 | 11.106 | 1.00 | 33.49 |
| 100 | CG | GLU | A | 681 | 7.361 | 15.322 | 10.028 | 1.00 | 41.42 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 101 | CD | GLU | A | 681 | 7.500 | 16.742 | 10.581 | 1.00 | 49.46 |
| 102 | OE1 | GLU | A | 681 | 7.569 | 16.924 | 11.824 | 1.00 | 44.22 |
| 103 | OE2 | GLU | A | 681 | 7.527 | 17.687 | 9.759 | 1.00 | 52.12 |
| 104 | N | PRO | A | 682 | 3.892 | 15.446 | 10.751 | 1.00 | 41.06 |
| 105 | CA | PRO | A | 682 | 2.695 | 15.422 | 9.904 | 1.00 | 41.12 |
| 106 | C | PRO | A | 682 | 2.968 | 14.980 | 8.444 | 1.00 | 44.28 |
| 107 | O | PRO | A | 682 | 4.076 | 15.133 | 7.920 | 1.00 | 36.92 |
| 108 | CB | PRO | A | 682 | 2.214 | 16.870 | 9.965 | 1.00 | 43.30 |
| 109 | CG | PRO | A | 682 | 2.800 | 17.399 | 11.250 | 1.00 | 38.89 |
| 110 | CD | PRO | A | 682 | 4.159 | 16.800 | 11.261 | 1.00 | 39.59 |
| 111 | N | GLY | A | 683 | 1.943 | 14.446 | 7.788 | 1.00 | 48.21 |
| 112 | CA | GLY | A | 683 | 2.103 | 13.990 | 6.416 | 1.00 | 51.13 |
| 113 | C | GLY | A | 683 | 1.905 | 15.043 | 5.334 | 1.00 | 54.68 |
| 114 | O | GLY | A | 683 | 1.817 | 16.226 | 5.629 | 1.00 | 63.53 |
| 115 | N | VAL | A | 684 | 1.729 | 14.601 | 4.089 | 1.00 | 57.20 |
| 116 | CA | VAL | A | 684 | 1.544 | 15.505 | 2.959 | 1.00 | 54.91 |
| 117 | C | VAL | A | 684 | 0.123 | 16.048 | 2.952 | 1.00 | 54.45 |
| 118 | O | VAL | A | 684 | -0.828 | 15.287 | 2.775 | 1.00 | 57.51 |
| 119 | CB | VAL | A | 684 | 1.805 | 14.792 | 1.625 | 1.00 | 51.72 |
| 120 | CG1 | VAL | A | 684 | 1.618 | 15.769 | 0.487 | 1.00 | 53.17 |
| 121 | CG2 | VAL | A | 684 | 3.222 | 14.212 | 1.591 | 1.00 | 53.92 |
| 122 | N | VAL | A | 685 | -0.021 | 17.360 | 3.125 | 1.00 | 48.43 |
| 123 | CA | VAL | A | 685 | -1.341 | 17.974 | 3.163 | 1.00 | 44.96 |
| 124 | C | VAL | A | 685 | -1.603 | 18.758 | 1.887 | 1.00 | 46.69 |
| 125 | O | VAL | A | 685 | -0.934 | 19.742 | 1.644 | 1.00 | 54.09 |
| 126 | CB | VAL | A | 685 | -1.455 | 18.932 | 4.355 | 1.00 | 39.06 |
| 127 | CG1 | VAL | A | 685 | -2.888 | 19.062 | 4.764 | 1.00 | 41.14 |
| 128 | CG2 | VAL | A | 685 | -0.611 | 18.445 | 5.520 | 1.00 | 36.12 |
| 129 | N | CYS | A | 686 | -2.582 | 18.340 | 1.087 | 1.00 | 51.49 |
| 130 | CA | CYS | A | 686 | -2.921 | 19.015 | -0.177 | 1.00 | 55.13 |
| 131 | C | CYS | A | 686 | -3.830 | 20.266 | -0.010 | 1.00 | 56.12 |
| 132 | O | CYS | A | 686 | -4.856 | 20.208 | 0.681 | 1.00 | 54.29 |
| 133 | CB | CYS | A | 686 | -3.559 | 18.009 | -1.155 | 1.00 | 57.05 |
| 134 | SG | CYS | A | 686 | -2.391 | 16.910 | -2.025 | 1.00 | 65.52 |
| 135 | N | ALA | A | 687 | -3.484 | 21.374 | -0.681 | 1.00 | 54.36 |
| 136 | CA | ALA | A | 687 | -4.248 | 22.631 | -0.584 | 1.00 | 54.20 |
| 137 | C | ALA | A | 687 | -5.583 | 22.542 | -1.268 | 1.00 | 60.58 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 138 | O | ALA | A | 687 | -6.557 | 23.131 | -0.803 | 1.00 | 62.87 |
| 139 | CB | ALA | A | 687 | -3.463 | 23.815 | -1.168 | 1.00 | 49.21 |
| 140 | N | GLY | A | 688 | -5.623 | 21.791 | -2.366 | 1.00 | 67.12 |
| 141 | CA | GLY | A | 688 | -6.847 | 21.650 | -3.134 | 1.00 | 66.26 |
| 142 | C | GLY | A | 688 | -6.948 | 22.874 | -4.024 | 1.00 | 64.95 |
| 143 | O | GLY | A | 688 | -7.888 | 23.676 | -3.924 | 1.00 | 66.95 |
| 144 | N | HIS | A | 689 | -5.951 | 23.042 | -4.879 | 1.00 | 59.22 |
| 145 | CA | HIS | A | 689 | -5.925 | 24.179 | -5.769 | 1.00 | 54.36 |
| 146 | C | HIS | A | 689 | -6.022 | 23.716 | -7.220 | 1.00 | 59.75 |
| 147 | O | HIS | A | 689 | -5.393 | 22.717 | -7.602 | 1.00 | 62.11 |
| 148 | CB | HIS | A | 689 | -4.650 | 24.977 | -5.537 | 1.00 | 46.90 |
| 149 | CG | HIS | A | 689 | -4.364 | 25.964 | -6.617 | 1.00 | 52.16 |
| 150 | ND1 | HIS | A | 689 | -5.104 | 27.116 | -6.787 | 1.00 | 52.66 |
| 151 | CD2 | HIS | A | 689 | -3.474 | 25.931 | -7.633 | 1.00 | 58.89 |
| 152 | CE1 | HIS | A | 689 | -4.683 | 27.750 | -7.868 | 1.00 | 57.16 |
| 153 | NE2 | HIS | A | 689 | -3.695 | 27.052 | -8.402 | 1.00 | 60.29 |
| 154 | N | ASP | A | 690 | -6.838 | 24.424 | -8.011 | 1.00 | 60.46 |
| 155 | CA | ASP | A | 690 | -7.021 | 24.108 | -9.427 | 1.00 | 59.31 |
| 156 | C | ASP | A | 690 | -5.835 | 24.583 | -10.280 | 1.00 | 60.15 |
| 157 | O | ASP | A | 690 | -5.739 | 25.752 | -10.654 | 1.00 | 58.71 |
| 158 | CB | ASP | A | 690 | -8.334 | 24.701 | -9.949 | 1.00 | 60.31 |
| 159 | CG | ASP | A | 690 | -8.762 | 24.107 | -11.304 | 1.00 | 62.54 |
| 160 | OD1 | ASP | A | 690 | -7.904 | 23.586 | -12.054 | 1.00 | 54.14 |
| 161 | OD2 | ASP | A | 690 | -9.970 | 24.166 | -11.620 | 1.00 | 59.85 |
| 162 | N | ASN | A | 691 | -4.925 | 23.657 | -10.564 | 1.00 | 66.30 |
| 163 | CA | ASN | A | 691 | -3.736 | 23.940 | -11.369 | 1.00 | 71.61 |
| 164 | C | ASN | A | 691 | -4.037 | 23.857 | -12.868 | 1.00 | 73.70 |
| 165 | O | ASN | A | 691 | -3.121 | 23.715 | -13.682 | 1.00 | 81.27 |
| 166 | CB | ASN | A | 691 | -2.581 | 22.971 | -11.016 | 1.00 | 72.66 |
| 167 | CG | ASN | A | 691 | -1.672 | 23.506 | -9.923 | 1.00 | 72.16 |
| 168 | OD1 | ASN | A | 691 | -1.697 | 24.697 | -9.600 | 1.00 | 74.90 |
| 169 | ND2 | ASN | A | 691 | -0.845 | 22.633 | -9.365 | 1.00 | 72.55 |
| 170 | N | ASN | A | 692 | -5.317 | 23.908 | -13.227 | 1.00 | 70.75 |
| 171 | CA | ASN | A | 692 | -5.705 | 23.851 | -14.632 | 1.00 | 69.77 |
| 172 | C | ASN | A | 692 | -6.467 | 25.131 | -14.982 | 1.00 | 66.04 |
| 173 | O | ASN | A | 692 | -6.986 | 25.257 | -16.087 | 1.00 | 65.61 |
| 174 | CB | ASN | A | 692 | -6.551 | 22.600 | -14.958 | 1.00 | 68.43 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 175 | CG | ASN | A | 692 | -6.019 | 21.331 | -14.307 | 1.00 | 63.97 |
| 176 | OD1 | ASN | A | 692 | -5.501 | 20.438 | -14.972 | 0.00 | 65.30 |
| 177 | ND2 | ASN | A | 692 | -6.184 | 21.240 | -12.993 | 0.00 | 65.24 |
| 178 | N | GLN | A | 693 | -6.597 | 26.035 | -14.005 | 1.00 | 67.69 |
| 179 | CA | GLN | A | 693 | -7.255 | 27.340 | -14.196 | 1.00 | 71.99 |
| 180 | C | GLN | A | 693 | -6.171 | 28.429 | -14.199 | 1.00 | 74.61 |
| 181 | O | GLN | A | 693 | -5.230 | 28.359 | -13.413 | 1.00 | 73.58 |
| 182 | CB | GLN | A | 693 | -8.279 | 27.647 | -13.084 | 1.00 | 68.47 |
| 183 | CG | GLN | A | 693 | -9.575 | 26.819 | -13.134 | 1.00 | 72.04 |
| 184 | CD | GLN | A | 693 | -10.172 | 26.661 | -14.540 | 1.00 | 75.91 |
| 185 | OE1 | GLN | A | 693 | -9.955 | 27.497 | -15.423 | 1.00 | 85.37 |
| 186 | NE2 | GLN | A | 693 | -10.936 | 25.592 | -14.745 | 1.00 | 70.58 |
| 187 | N | PRO | A | 694 | -6.285 | 29.438 | -15.091 | 1.00 | 81.70 |
| 188 | CA | PRO | A | 694 | -5.302 | 30.531 | -15.181 | 1.00 | 80.62 |
| 189 | C | PRO | A | 694 | -5.118 | 31.249 | -13.856 | 1.00 | 77.25 |
| 190 | O | PRO | A | 694 | -6.106 | 31.640 | -13.206 | 1.00 | 74.33 |
| 191 | CB | PRO | A | 694 | -5.911 | 31.458 | -16.243 | 1.00 | 85.84 |
| 192 | CG | PRO | A | 694 | -7.421 | 31.170 | -16.151 | 1.00 | 89.04 |
| 193 | CD | PRO | A | 694 | -7.398 | 29.662 | -16.034 | 1.00 | 87.17 |
| 194 | N | ASP | A | 695 | -3.849 | 31.467 | -13.496 | 1.00 | 76.00 |
| 195 | CA | ASP | A | 695 | -3.494 | 32.117 | -12.237 | 1.00 | 63.22 |
| 196 | C | ASP | A | 695 | -4.294 | 33.356 | -11.942 | 1.00 | 56.02 |
| 197 | O | ASP | A | 695 | -4.859 | 33.997 | -12.842 | 1.00 | 56.35 |
| 198 | CB | ASP | A | 695 | -1.995 | 32.405 | -12.153 | 1.00 | 63.70 |
| 199 | CG | ASP | A | 695 | -1.190 | 31.192 | -11.684 | 1.00 | 66.50 |
| 200 | OD1 | ASP | A | 695 | -1.523 | 30.627 | -10.617 | 1.00 | 66.69 |
| 201 | OD2 | ASP | A | 695 | -0.217 | 30.802 | -12.377 | 1.00 | 71.13 |
| 202 | N | SER | A | 696 | -4.338 | 33.694 | -10.666 | 1.00 | 56.40 |
| 203 | CA | SER | A | 696 | -5.093 | 34.839 | -10.214 | 1.00 | 56.56 |
| 204 | C | SER | A | 696 | -4.690 | 35.191 | -8.788 | 1.00 | 61.68 |
| 205 | O | SER | A | 696 | -4.326 | 34.319 | -7.993 | 1.00 | 69.36 |
| 206 | CB | SER | A | 696 | -6.587 | 34.510 | -10.289 | 1.00 | 54.32 |
| 207 | OG | SER | A | 696 | -7.365 | 35.473 | -9.604 | 1.00 | 54.41 |
| 208 | N | PHE | A | 697 | -4.754 | 36.477 | -8.474 | 1.00 | 61.93 |
| 209 | CA | PHE | A | 697 | -4.412 | 36.974 | -7.153 | 1.00 | 61.05 |
| 210 | C | PHE | A | 697 | -5.344 | 36.448 | -6.037 | 1.00 | 61.34 |
| 211 | O | PHE | A | 697 | -4.881 | 36.053 | -4.971 | 1.00 | 62.55 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 212 | CB | PHE | A | 697 | -4.413 | 38.509 | -7.195 | 1.00 | 63.27 |
| 213 | CG | PHE | A | 697 | -4.961 | 39.156 | -5.951 | 1.00 | 60.53 |
| 214 | CD1 | PHE | A | 697 | -4.142 | 39.397 | -4.856 | 1.00 | 65.55 |
| 215 | CD2 | PHE | A | 697 | -6.306 | 39.484 | -5.864 | 1.00 | 61.74 |
| 216 | CE1 | PHE | A | 697 | -4.651 | 39.950 | -3.691 | 1.00 | 63.81 |
| 217 | CE2 | PHE | A | 697 | -6.825 | 40.034 | -4.710 | 1.00 | 66.91 |
| 218 | CZ | PHE | A | 697 | -5.994 | 40.267 | -3.617 | 1.00 | 69.41 |
| 219 | N | ALA | A | 698 | -6.653 | 36.464 | -6.261 | 1.00 | 58.96 |
| 220 | CA | ALA | A | 698 | -7.577 | 35.996 | -5.230 | 1.00 | 59.58 |
| 221 | C | ALA | A | 698 | -7.653 | 34.467 | -5.143 | 1.00 | 61.20 |
| 222 | O | ALA | A | 698 | -7.862 | 33.913 | -4.063 | 1.00 | 64.62 |
| 223 | CB | ALA | A | 698 | -8.965 | 36.589 | -5.448 | 1.00 | 56.65 |
| 224 | N | ALA | A | 699 | -7.492 | 33.783 | -6.272 | 1.00 | 58.65 |
| 225 | CA | ALA | A | 699 | -7.555 | 32.328 | -6.270 | 1.00 | 57.39 |
| 226 | C | ALA | A | 699 | -6.290 | 31.748 | -5.616 | 1.00 | 54.55 |
| 227 | O | ALA | A | 699 | -6.349 | 30.708 | -4.951 | 1.00 | 57.04 |
| 228 | CB | ALA | A | 699 | -7.749 | 31.791 | -7.694 | 1.00 | 58.30 |
| 229 | N | LEU | A | 700 | -5.155 | 32.423 | -5.786 | 1.00 | 44.77 |
| 230 | CA | LEU | A | 700 | -3.921 | 31.956 | -5.181 | 1.00 | 40.04 |
| 231 | C | LEU | A | 700 | -3.983 | 32.180 | -3.667 | 1.00 | 40.02 |
| 232 | O | LEU | A | 700 | -3.737 | 31.256 | -2.893 | 1.00 | 42.09 |
| 233 | CB | LEU | A | 700 | -2.709 | 32.661 | -5.800 | 1.00 | 40.84 |
| 234 | CG | LEU | A | 700 | -2.369 | 32.286 | -7.251 | 1.00 | 47.93 |
| 235 | CD1 | LEU | A | 700 | -1.195 | 33.092 | -7.751 | 1.00 | 52.49 |
| 236 | CD2 | LEU | A | 700 | -2.035 | 30.824 | -7.337 | 1.00 | 41.70 |
| 237 | N | LEU | A | 701 | -4.384 | 33.375 | -3.238 | 1.00 | 38.79 |
| 238 | CA | LEU | A | 701 | -4.468 | 33.679 | -1.808 | 1.00 | 40.37 |
| 239 | C | LEU | A | 701 | -5.548 | 32.917 | -1.024 | 1.00 | 45.30 |
| 240 | O | LEU | A | 701 | -5.305 | 32.557 | 0.128 | 1.00 | 45.13 |
| 241 | CB | LEU | A | 701 | -4.545 | 35.190 | -1.561 | 1.00 | 37.85 |
| 242 | CG | LEU | A | 701 | -3.253 | 35.971 | -1.868 | 1.00 | 41.30 |
| 243 | CD1 | LEU | A | 701 | -3.479 | 37.426 | -1.612 | 1.00 | 41.50 |
| 244 | CD2 | LEU | A | 701 | -2.070 | 35.506 | -1.025 | 1.00 | 35.31 |
| 245 | N | SER | A | 702 | -6.719 | 32.678 | -1.633 | 1.00 | 52.36 |
| 246 | CA | SER | A | 702 | -7.822 | 31.899 | -1.013 | 1.00 | 54.37 |
| 247 | C | SER | A | 702 | -7.388 | 30.433 | -0.783 | 1.00 | 51.62 |
| 248 | O | SER | A | 702 | -7.740 | 29.806 | 0.234 | 1.00 | 45.03 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 249 | CB | SER | A | 702 | -9.060 | 31.920 | -1.914 | 1.00 | 50.95 |
| 250 | OG | SER | A | 702 | -9.500 | 33.246 | -2.101 | 1.00 | 50.66 |
| 251 | N | SER | A | 703 | -6.649 | 29.890 | -1.756 | 1.00 | 49.24 |
| 252 | CA | SER | A | 703 | -6.119 | 28.536 | -1.660 | 1.00 | 46.51 |
| 253 | C | SER | A | 703 | -5.101 | 28.504 | -0.528 | 1.00 | 46.34 |
| 254 | O | SER | A | 703 | -5.141 | 27.597 | 0.293 | 1.00 | 52.79 |
| 255 | CB | SER | A | 703 | -5.442 | 28.115 | -2.956 | 1.00 | 37.23 |
| 256 | OG | SER | A | 703 | -6.388 | 28.080 | -4.005 | 1.00 | 45.32 |
| 257 | N | LEU | A | 704 | -4.215 | 29.501 | -0.459 | 1.00 | 40.74 |
| 258 | CA | LEU | A | 704 | -3.227 | 29.548 | 0.618 | 1.00 | 36.98 |
| 259 | C | LEU | A | 704 | -3.902 | 29.552 | 1.984 | 1.00 | 41.66 |
| 260 | O | LEU | A | 704 | -3.389 | 28.951 | 2.920 | 1.00 | 45.02 |
| 261 | CB | LEU | A | 704 | -2.302 | 30.751 | 0.485 | 1.00 | 30.44 |
| 262 | CG | LEU | A | 704 | -0.994 | 30.563 | -0.291 | 1.00 | 35.89 |
| 263 | CD1 | LEU | A | 704 | -0.235 | 31.859 | -0.308 | 1.00 | 36.89 |
| 264 | CD2 | LEU | A | 704 | -0.115 | 29.484 | 0.307 | 1.00 | 37.91 |
| 265 | N | ASN | A | 705 | -5.061 | 30.208 | 2.076 | 1.00 | 45.16 |
| 266 | CA | ASN | A | 705 | -5.849 | 30.289 | 3.311 | 1.00 | 43.35 |
| 267 | C | ASN | A | 705 | -6.521 | 28.962 | 3.618 | 1.00 | 42.54 |
| 268 | O | ASN | A | 705 | -6.677 | 28.594 | 4.779 | 1.00 | 40.34 |
| 269 | CB | ASN | A | 705 | -6.934 | 31.370 | 3.201 | 1.00 | 44.83 |
| 270 | CG | ASN | A | 705 | -6.362 | 32.771 | 3.137 | 1.00 | 43.71 |
| 271 | OD1 | ASN | A | 705 | -5.197 | 32.992 | 3.470 | 1.00 | 48.81 |
| 272 | ND2 | ASN | A | 705 | -7.176 | 33.726 | 2.694 | 1.00 | 38.59 |
| 273 | N | GLU | A | 706 | -6.991 | 28.269 | 2.591 | 1.00 | 42.62 |
| 274 | CA | GLU | A | 706 | -7.607 | 26.987 | 2.842 | 1.00 | 44.02 |
| 275 | C | GLU | A | 706 | -6.478 | 26.033 | 3.261 | 1.00 | 42.46 |
| 276 | O | GLU | A | 706 | -6.639 | 25.253 | 4.195 | 1.00 | 42.70 |
| 277 | CB | GLU | A | 706 | -8.352 | 26.472 | 1.621 | 1.00 | 45.19 |
| 278 | CG | GLU | A | 706 | -9.322 | 25.338 | 1.971 | 1.00 | 55.97 |
| 279 | CD | GLU | A | 706 | -10.417 | 25.735 | 2.986 | 1.00 | 58.77 |
| 280 | OE1 | GLU | A | 706 | -11.019 | 26.817 | 2.838 | 1.00 | 60.87 |
| 281 | OE2 | GLU | A | 706 | -10.707 | 24.945 | 3.918 | 1.00 | 60.16 |
| 282 | N | LEU | A | 707 | -5.307 | 26.167 | 2.636 | 1.00 | 38.31 |
| 283 | CA | LEU | A | 707 | -4.152 | 25.342 | 2.982 | 1.00 | 37.96 |
| 284 | C | LEU | A | 707 | -3.767 | 25.654 | 4.431 | 1.00 | 44.27 |
| 285 | O | LEU | A | 707 | -3.464 | 24.747 | 5.211 | 1.00 | 51.41 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 286 | CB | LEU | A | 707 | -2.958 | 25.608 | 2.046 | 1.00 | 35.41 |
| 287 | CG | LEU | A | 707 | -1.651 | 24.872 | 2.392 | 1.00 | 35.71 |
| 288 | CD1 | LEU | A | 707 | -1.895 | 23.385 | 2.326 | 1.00 | 38.82 |
| 289 | CD2 | LEU | A | 707 | -0.518 | 25.239 | 1.459 | 1.00 | 33.25 |
| 290 | N | GLY | A | 708 | -3.782 | 26.938 | 4.787 | 1.00 | 45.88 |
| 291 | CA | GLY | A | 708 | -3.463 | 27.344 | 6.144 | 1.00 | 40.92 |
| 292 | C | GLY | A | 708 | -4.386 | 26.618 | 7.096 | 1.00 | 39.05 |
| 293 | O | GLY | A | 708 | -3.937 | 25.851 | 7.924 | 1.00 | 45.81 |
| 294 | N | GLU | A | 709 | -5.685 | 26.790 | 6.913 | 1.00 | 46.04 |
| 295 | CA | GLU | A | 709 | -6.680 | 26.125 | 7.758 | 1.00 | 51.25 |
| 296 | C | GLU | A | 709 | -6.367 | 24.637 | 7.975 | 1.00 | 51.93 |
| 297 | O | GLU | A | 709 | -6.213 | 24.197 | 9.107 | 1.00 | 56.39 |
| 298 | CB | GLU | A | 709 | -8.079 | 26.273 | 7.151 | 1.00 | 56.56 |
| 299 | CG | GLU | A | 709 | -9.198 | 25.652 | 7.984 | 1.00 | 63.02 |
| 300 | CD | GLU | A | 709 | -9.766 | 26.593 | 9.042 | 1.00 | 64.61 |
| 301 | OE1 | GLU | A | 709 | -9.157 | 27.641 | 9.338 | 1.00 | 66.14 |
| 302 | OE2 | GLU | A | 709 | -10.855 | 26.293 | 9.573 | 1.00 | 65.41 |
| 303 | N | ARG | A | 710 | -6.235 | 23.864 | 6.901 | 1.00 | 53.43 |
| 304 | CA | ARG | A | 710 | -5.938 | 22.440 | 7.058 | 1.00 | 50.19 |
| 305 | C | ARG | A | 710 | -4.617 | 22.102 | 7.735 | 1.00 | 44.91 |
| 306 | O | ARG | A | 710 | -4.522 | 21.061 | 8.396 | 1.00 | 43.38 |
| 307 | CB | ARG | A | 710 | -6.032 | 21.652 | 5.747 | 1.00 | 56.69 |
| 308 | CG | ARG | A | 710 | -6.192 | 22.446 | 4.493 | 1.00 | 59.66 |
| 309 | CD | ARG | A | 710 | -7.462 | 22.035 | 3.786 | 1.00 | 59.37 |
| 310 | NE | ARG | A | 710 | -7.203 | 21.172 | 2.643 | 1.00 | 58.69 |
| 311 | CZ | ARG | A | 710 | -8.124 | 20.857 | 1.736 | 1.00 | 70.01 |
| 312 | NH1 | ARG | A | 710 | -9.364 | 21.349 | 1.854 | 1.00 | 75.20 |
| 313 | NH2 | ARG | A | 710 | -7.812 | 20.057 | 0.713 | 1.00 | 65.26 |
| 314 | N | GLN | A | 711 | -3.596 | 22.939 | 7.556 | 1.00 | 40.11 |
| 315 | CA | GLN | A | 711 | -2.310 | 22.685 | 8.189 | 1.00 | 34.69 |
| 316 | C | GLN | A | 711 | -2.355 | 23.007 | 9.653 | 1.00 | 36.60 |
| 317 | O | GLN | A | 711 | -1.501 | 22.557 | 10.408 | 1.00 | 40.79 |
| 318 | CB | GLN | A | 711 | -1.194 | 23.478 | 7.542 | 1.00 | 42.15 |
| 319 | CG | GLN | A | 711 | -0.753 | 22.877 | 6.244 | 1.00 | 43.03 |
| 320 | CD | GLN | A | 711 1 | 0.553 | 23.442 | 5.779 | 1.00 | 44.24 |
| 321 | OE1 | GLN | A | 711 | 1.321 | 23.988 | 6.567 | 1.00 | 54.32 |
| 322 | NE2 | GLN | A | 711 | 0.828 | 23.305 | 4.496 | 1.00 | 52.33 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 323 | N | LEU | A | 712 | -3.361 | 23.778 | 10.054 | 1.00 | 41.25 |
| 324 | CA | LEU | A | 712 | -3.561 | 24.163 | 11.457 | 1.00 | 43.47 |
| 325 | C | LEU | A | 712 | -4.061 | 22.938 | 12.222 | 1.00 | 45.20 |
| 326 | O | LEU | A | 712 | -3.595 | 22.628 | 13.320 | 1.00 | 46.51 |
| 327 | CB | LEU | A | 712 | -4.585 | 25.295 | 11.550 | 1.00 | 42.08 |
| 328 | CG | LEU | A | 712 | -4.829 | 25.943 | 12.905 | 1.00 | 45.04 |
| 329 | CD1 | LEU | A | 712 | -3.489 | 26.199 | 13.594 | 1.00 | 48.18 |
| 330 | CD2 | LEU | A | 712 | -5.610 | 27.248 | 12.711 | 1.00 | 44.32 |
| 331 | N | VAL | A | 713 | -5.014 | 22.240 | 11.623 | 1.00 | 42.76 |
| 332 | CA | VAL | A | 713 | -5.555 | 21.026 | 12.198 | 1.00 | 41.99 |
| 333 | C | VAL | A | 713 | -4.383 | 20.100 | 12.562 | 1.00 | 45.10 |
| 334 | O | VAL | A | 713 | -4.275 | 19.646 | 13.703 | 1.00 | 45.64 |
| 335 | CB | VAL | A | 713 | -6.480 | 20.348 | 11.170 | 1.00 | 43.85 |
| 336 | CG1 | VAL | A | 713 | -6.887 | 18.953 | 11.628 | 1.00 | 52.59 |
| 337 | CG2 | VAL | A | 713 | -7.708 | 21.203 | 10.966 | 1.00 | 42.38 |
| 338 | N | HIS | A | 714 | -3.471 | 19.905 | 11.604 | 1.00 | 46.35 |
| 339 | CA | HIS | A | 714 | -2.286 | 19.044 | 11.767 | 1.00 | 45.95 |
| 340 | C | HIS | A | 714 | -1.379 | 19.495 | 12.857 | 1.00 | 43.82 |
| 341 | O | HIS | A | 714 | -0.798 | 18.674 | 13.571 | 1.00 | 48.61 |
| 342 | CB | HIS | A | 714 | -1.458 | 18.971 | 10.487 | 1.00 | 49.61 |
| 343 | CG | HIS | A | 714 | -1.950 | 17.947 | 9.519 | 1.00 | 62.09 |
| 344 | ND1 | HIS | A | 714 | -3.157 | 18.058 | 8.873 | 1.00 | 63.02 |
| 345 | CD2 | HIS | A | 714 | -1.404 | 16.778 | 9.108 | 1.00 | 64.82 |
| 346 | CE1 | HIS | A | 714 | -3.340 | 17.005 | 8.100 | 1.00 | 70.96 |
| 347 | NE2 | HIS | A | 714 | -2.291 | 16.211 | 8.219 | 1.00 | 70.54 |
| 348 | N | VAL | A | 715 | -1.172 | 20.803 | 12.898 | 1.00 | 40.29 |
| 349 | CA | VAL | A | 715 | -0.326 | 21.415 | 13.908 | 1.00 | 39.63 |
| 350 | C | VAL | A | 715 | -0.962 | 21.201 | 15.273 | 1.00 | 36.62 |
| 351 | O | VAL | A | 715 | -0.266 | 20.874 | 16.244 | 1.00 | 30.18 |
| 352 | CB | VAL | A | 715 | -0.101 | 22.918 | 13.620 | 1.00 | 38.77 |
| 353 | CG1 | VAL | A | 715 | 0.500 | 23.617 | 14.820 | 1.00 | 30.17 |
| 354 | CG2 | VAL | A | 715 | 0.857 | 23.048 | 12.463 | 1.00 | 40.69 |
| 355 | N | VAL | A | 716 | -2.286 | 21.329 | 15.331 | 1.00 | 28.64 |
| 356 | CA | VAL | A | 716 | -2.994 | 21.113 | 16.570 | 1.00 | 28.84 |
| 357 | C | VAL | A | 716 | -2.687 | 19.683 | 17.037 | 1.00 | 36.83 |
| 358 | O | VAL | A | 716 | -2.078 | 19.485 | 18.092 | 1.00 | 36.70 |
| 359 | CB | VAL | A | 716 | -4.508 | 21.331 | 16.403 | 1.00 | 34.61 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 360 | CG1 | VAL | A | 716 | -5.239 | 20.839 | 17.647 | 1.00 | 29.84 |
| 361 | CG2 | VAL | A | 716 | -4.805 | 22.811 | 16.185 | 1.00 | 32.32 |
| 362 | N | LYS | A | 717 | -2.972 | 18.709 | 16.179 | 1.00 | 38.71 |
| 363 | CA | LYS | A | 717 | -2.737 | 17.313 | 16.505 | 1.00 | 32.14 |
| 364 | C | LYS | A | 717 | -1.263 | 16.990 | 16.699 | 1.00 | 32.82 |
| 365 | O | LYS | A | 717 | -0.920 | 16.262 | 17.631 | 1.00 | 34.86 |
| 366 | CB | LYS | A | 717 | -3.370 | 16.410 | 15.450 | 1.00 | 32.30 |
| 367 | CG | LYS | A | 717 | -4.890 | 16.352 | 15.569 | 1.00 | 38.88 |
| 368 | CD | LYS | A | 717 | -5.538 | 15.584 | 14.436 | 0.00 | 36.05 |
| 369 | CE | LYS | A | 717 | -7.009 | 15.353 | 14.736 | 0.00 | 36.14 |
| 370 | NZ | LYS | A | 717 | -7.739 | 14.704 | 13.619 | 0.00 | 35.32 |
| 371 | N | TRP | A | 718 | -0.383 | 17.589 | 15.893 | 1.00 | 31.69 |
| 372 | CA | TRP | A | 718 | 1.058 | 17.319 | 16.010 | 1.00 | 34.84 |
| 373 | C | TRP | A | 718 | 1.604 | 17.753 | 17.367 | 1.00 | 44.15 |
| 374 | O | TRP | A | 718 | 2.347 | 17.014 | 18.020 | 1.00 | 48.94 |
| 375 | CB | TRP | A | 718 | 1.850 | 17.995 | 14.883 | 1.00 | 25.87 |
| 376 | CG | TRP | A | 718 | 3.343 | 18.092 | 15.136 | 1.00 | 25.59 |
| 377 | CD1 | TRP | A | 718 | 4.279 | 17.133 | 14.909 | 1.00 | 35.87 |
| 378 | CD2 | TRP | A | 718 | 4.055 | 19.232 | 15.641 | 1.00 | 30.45 |
| 379 | NE1 | TRP | A | 718 | 5.533 | 17.598 | 15.237 | 1.00 | 32.13 |
| 380 | CE2 | TRP | A | 718 | 5.419 | 18.889 | 15.689 | 1.00 | 30.51 |
| 381 | CE3 | TRP | A | 718 | 3.672 | 20.519 | 16.046 | 1.00 | 32.20 |
| 382 | CZ2 | TRP | A | 718 | 6.403 | 19.782 | 16.119 | 1.00 | 32.90 |
| 383 | CZ3 | TRP | A | 718 | 4.650 | 21.408 | 16.468 | 1.00 | 25.41 |
| 384 | CH2 | TRP | A | 718 | 5.997 | 21.036 | 16.503 | 1.00 | 28.69 |
| 385 | N | ALA | A | 719 | 1.242 | 18.973 | 17.764 | 1.00 | 56.34 |
| 386 | CA | ALA | A | 719 | 1.654 | 19.580 | 19.037 | 1.00 | 52.43 |
| 387 | C | ALA | A | 719 | 1.176 | 18.846 | 20.305 | 1.00 | 47.73 |
| 388 | O | ALA | A | 719 | 1.968 | 18.662 | 21.246 | 1.00 | 46.60 |
| 389 | CB | ALA | A | 719 | 1.214 | 21.043 | 19.073 | 1.00 | 48.46 |
| 390 | N | LYS | A | 720 | -0.107 | 18.461 | 20.337 | 1.00 | 42.77 |
| 391 | CA | LYS | A | 720 | -0.697 | 17.744 | 21.478 | 1.00 | 45.06 |
| 392 | C | LYS | A | 720 | -0.019 | 16.386 | 21.772 | 1.00 | 47.46 |
| 393 | O | LYS | A | 720 | -0.227 | 15.789 | 22.835 | 1.00 | 50.10 |
| 394 | CB | LYS | A | 720 | -2.218 | 17.567 | 21.294 | 1.00 | 39.13 |
| 395 | CG | LYS | A | 720 | -3.057 | 18.632 | 21.979 | 1.00 | 45.90 |
| 396 | CD | LYS | A | 720 | -4.533 | 18.253 | 22.075 | 1.00 | 55.06 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 397 | CE | LYS | A | 720 | -5.236 | 19.088 | 23.161 | 1.00 | 63.65 |
| 398 | NZ | LYS | A | 720 | -6.668 | 18.719 | 23.402 | 1.00 | 62.72 |
| 399 | N | ALA | A | 721 | 0.810 | 15.926 | 20.842 | 1.00 | 40.80 |
| 400 | CA | ALA | A | 721 | 1.524 | 14.671 | 20.995 | 1.00 | 41.77 |
| 401 | C | ALA | A | 721 | 2.991 | 14.884 | 21.346 | 1.00 | 41.79 |
| 402 | O | ALA | A | 721 | 3.762 | 13.931 | 21.370 | 1.00 | 43.57 |
| 403 | CB | ALA | A | 721 | 1.410 | 13.840 | 19.710 | 1.00 | 45.25 |
| 404 | N | LEU | A | 722 | 3.382 | 16.131 | 21.585 | 1.00 | 40.71 |
| 405 | CA | LEU | A | 722 | 4.764 | 16.438 | 21.927 | 1.00 | 34.69 |
| 406 | C | LEU | A | 722 | 5.115 | 16.085 | 23.369 | 1.00 | 33.82 |
| 407 | O | LEU | A | 722 | 4.301 | 16.249 | 24.281 | 1.00 | 34.39 |
| 408 | CB | LEU | A | 722 | 5.058 | 17.911 | 21.665 | 1.00 | 32.26 |
| 409 | CG | LEU | A | 722 | 5.357 | 18.244 | 20.218 | 1.00 | 33.93 |
| 410 | CD1 | LEU | A | 722 | 5.226 | 19.728 | 20.021 | 1.00 | 43.98 |
| 411 1 | CD2 | LEU | A | 722 | 6.753 | 17.784 | 19.880 | 1.00 | 30.81 |
| 412 | N | PRO | A | 723 | 6.338 | 15.589 | 23.592 | 1.00 | 37.66 |
| 413 | CA | PRO | A | 723 | 6.820 | 15.209 | 24.917 | 1.00 | 35.69 |
| 414 | C | PRO | A | 723 | 6.724 | 16.368 | 25.893 | 1.00 | 39.06 |
| 415 | O | PRO | A | 723 | 7.512 | 17.304 | 25.833 | 1.00 | 39.26 |
| 416 | CB | PRO | A | 723 | 8.285 | 14.855 | 24.662 | 1.00 | 34.40 |
| 417 | CG | PRO | A | 723 | 8.272 | 14.339 | 23.283 | 1.00 | 36.44 |
| 418 | CD | PRO | A | 723 | 7.354 | 15.282 | 22.562 | 1.00 | 41.22 |
| 419 | N | GLY | A | 724 | 5.780 | 16.284 | 26.812 | 1.00 | 39.75 |
| 420 | CA | GLY | A | 724 | 5.652 | 17.336 | 27.794 | 1.00 | 34.24 |
| 421 | C | GLY | A | 724 | 4.544 | 18.311 | 27.480 | 1.00 | 36.36 |
| 422 | O | GLY | A | 724 | 3.911 | 18.837 | 28.398 | 1.00 | 37.90 |
| 423 | N | PHE | A | 725 | 4.212 | 18.465 | 26.201 | 1.00 | 32.86 |
| 424 | CA | PHE | A | 725 | 3.192 | 19.422 | 25.845 | 1.00 | 36.30 |
| 425 | C | PHE | A | 725 | 1.899 | 19.385 | 26.620 | 1.00 | 41.59 |
| 426 | O | PHE | A | 725 | 1.385 | 20.441 | 27.002 | 1.00 | 44.02 |
| 427 | CB | PHE | A | 725 | 2.842 | 19.365 | 24.383 | 1.00 | 32.46 |
| 428 | CG | PHE | A | 725 | 1.928 | 20.474 | 23.958 | 1.00 | 37.13 |
| 429 | CD1 | PHE | A | 725 | 2.453 | 21.687 | 23.542 | 1.00 | 37.70 |
| 430 | CD2 | PHE | A | 725 | 0.544 | 20.302 | 23.948 | 1.00 | 39.17 |
| 431 | CE1 | PHE | A | 725 | 1.618 | 22.717 | 23.107 | 1.00 | 36.54 |
| 432 | CE2 | PHE | A | 725 | -0.308 | 21.331 | 23.513 | 1.00 | 39.69 |
| 433 | CZ | PHE | A | 725 | 0.233 | 22.540 | 23.089 | 1.00 | 42.41 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 434 | N | ARG | A | 726 | 1.335 | 18.189 | 26.791 | 1.00 | 49.80 |
| 435 | CA | ARG | A | 726 | 0.050 | 18.042 | 27.489 | 1.00 | 49.46 |
| 436 | C | ARG | A | 726 | 0.057 | 18.304 | 28.995 | 1.00 | 52.43 |
| 437 | O | ARG | A | 726 | -0.978 | 18.139 | 29.644 | 1.00 | 52.56 |
| 438 | CB | ARG | A | 726 | -0.652 | 16.709 | 27.154 | 1.00 | 44.05 |
| 439 | CG | ARG | A | 726 | -1.692 | 16.821 | 26.023 | 1.00 | 44.55 |
| 440 | CD | ARG | A | 726 | -2.598 | 15.606 | 26.003 | 1.00 | 48.35 |
| 441 | NE | ARG | A | 726 | -3.771 | 15.752 | 25.130 | 1.00 | 56.42 |
| 442 | CZ | ARG | A | 726 | -5.040 | 15.569 | 25.526 | 1.00 | 62.91 |
| 443 | NH1 | ARG | A | 726 | -5.323 | 15.249 | 26.799 | 1.00 | 55.81 |
| 444 | NH2 | ARG | A | 726 | -6.028 | 15.649 | 24.632 | 1.00 | 55.35 |
| 445 | N | ASN | A | 727 | 1.213 | 18.685 | 29.551 | 1.00 | 53.84 |
| 446 | CA | ASN | A | 727 | 1.292 | 19.018 | 30.969 | 1.00 | 58.03 |
| 447 | C | ASN | A | 727 | 1.026 | 20.528 | 31.138 | 1.00 | 61.72 |
| 448 | O | ASN | A | 727 | 0.723 | 20.993 | 32.248 | 1.00 | 65.35 |
| 449 | CB | ASN | A | 727 | 2.636 | 18.589 | 31.604 | 1.00 | 63.66 |
| 450 | CG | ASN | A | 727 | 3.777 | 19.597 | 31.388 | 1.00 | 67.51 |
| 451 | OD1 | ASN | A | 727 | 4.837 | 19.234 | 30.881 | 1.00 | 68.14 |
| 452 | ND2 | ASN | A | 727 | 3.606 | 20.828 | 31.864 | 1.00 | 73.64 |
| 453 | N | LEU | A | 728 | 1.190 | 21.288 | 30.047 | 1.00 | 59.72 |
| 454 | CA | LEU | A | 728 | 0.957 | 22.739 | 30.035 | 1.00 | 50.93 |
| 455 | C | LEU | A | 728 | -0.520 | 23.033 | 30.227 | 1.00 | 47.17 |
| 456 | O | LEU | A | 728 | -1.365 | 22.247 | 29.822 | 1.00 | 45.55 |
| 457 | CB | LEU | A | 728 | 1.369 | 23.348 | 28.695 | 1.00 | 54.70 |
| 458 | CG | LEU | A | 728 | 2.822 | 23.408 | 28.228 | 1.00 | 52.07 |
| 459 | CD1 | LEU | A | 728 | 2.808 | 24.029 | 26.844 | 1.00 | 45.38 |
| 460 | CD2 | LEU | A | 728 | 3.700 | 24.223 | 29.181 | 1.00 | 47.21 |
| 461 | N | HIS | A | 729 | -0.841 | 24.201 | 30.763 | 1.00 | 45.54 |
| 462 | CA | HIS | A | 729 | -2.231 | 24.530 | 30.986 | 1.00 | 44.10 |
| 463 | C | HIS | A | 729 | -2.972 | 24.559 | 29.655 | 1.00 | 40.23 |
| 464 | O | HIS | A | 729 | -2.421 | 24.976 | 28.658 | 1.00 | 44.09 |
| 465 | CB | HIS | A | 729 | -2.328 | 25.851 | 31.716 | 1.00 | 54.55 |
| 466 | CG | HIS | A | 729 | -3.717 | 26.221 | 32.119 | 1.00 | 63.22 |
| 467 | ND1 | HIS | A | 729 | -4.016 | 26.777 | 33.340 | 1.00 | 63.71 |
| 468 | CD2 | HIS | A | 729 | -4.893 | 26.156 | 31.442 | 1.00 | 66.57 |
| 469 | CE1 | HIS | A | 729 | -5.308 | 27.046 | 33.401 | 1.00 | 68.21 |
| 470 | NE2 | HIS | A | 729 | -5.861 | 26.678 | 32.256 | 1.00 | 68.48 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 471 | N | VAL | A | 730 | -4.224 | 24.116 | 29.653 | 1.00 | 39.50 |
| 472 | CA | VAL | A | 730 | -5.024 | 24.042 | 28.433 | 1.00 | 42.50 |
| 473 | C | VAL | A | 730 | -5.016 | 25.262 | 27.563 | 1.00 | 45.07 |
| 474 | O | VAL | A | 730 | -4.893 | 25.178 | 26.339 | 1.00 | 50.12 |
| 475 | CB | VAL | A | 730 | -6.500 | 23.653 | 28.710 | 1,00 | 50.67 |
| 476 | CG1 | VAL | A | 730 | -7.044 | 24.387 | 29.936 | 1.00 | 48.50 |
| 477 | CG2 | VAL | A | 730 | -7.367 | 23.960 | 27.478 | 1.00 | 50.44 |
| 478 | N | ASP | A | 731 | -5.249 | 26.399 | 28.185 | 1.00 | 51.13 |
| 479 | CA | ASP | A | 731 | -5.269 | 27.646 | 27.453 | 1.00 | 51.89 |
| 480 | C | ASP | A | 731 | -3.856 | 27.909 | 26.971 | 1.00 | 46.10 |
| 481 | O | ASP | A | 731 | -3.663 | 28.213 | 25.810 | 1.00 | 51.50 |
| 482 | CB | ASP | A | 731 | -5.820 | 28.755 | 28.338 | 1.00 | 51.89 |
| 483 | CG | ASP | A | 731 | -7.194 | 28.415 | 28.872 | 1.00 | 59.37 |
| 484 | OD1 | ASP | A | 731 | -8.065 | 28.082 | 28.030 | 1.00 | 56.09 |
| 485 | OD2 | ASP | A | 731 | -7.373 | 28.414 | 30.122 | 1.00 | 61.65 |
| 486 | N | ASP | A | 732 | -2.862 | 27.675 | 27.814 | 1.00 | 37.19 |
| 487 | CA | ASP | A | 732 | -1.482 | 27.861 | 27.392 | 1.00 | 37.51 |
| 488 | C | ASP | A | 732 | -1.177 | 26.934 | 26.232 | 1.00 | 39.98 |
| 489 | O | ASP | A | 732 | -0.398 | 27.268 | 25.351 | 1.00 | 44.52 |
| 490 | CB | ASP | A | 732 | -0.516 | 27.597 | 28.536 | 1.00 | 40.29 |
| 491 | CG | ASP | A | 732 | -0.523 | 28.713 | 29.565 | 1.00 | 51.89 |
| 492 | OD1 | ASP | A | 732 | -1.171 | 29.755 | 29.313 | 1.00 | 56.09 |
| 493 | OD2 | ASP | A | 732 | 0.116 | 28.562 | 30.631 | 1.00 | 59.50 |
| 494 | N | GLN | A | 733 | -1.819 | 25.776 | 26.213 | 1.00 | 46.01 |
| 495 | CA | GLN | A | 733 | -1.621 | 24.832 | 25.131 | 1.00 | 48.12 |
| 496 | C | GLN | A | 733 | -2.183 | 25.485 | 23.884 | 1.00 | 48.61 |
| 497 | O | GLN | A | 733 | -1.531 | 25.558 | 22.854 | 1.00 | 51.31 |
| 498 | CB | GLN | A | 733 | -2.421 | 23.563 | 25.364 | 1.00 | 52.94 |
| 499 | CG | GLN | A | 733 | -1.791 | 22.511 | 26.234 | 1.00 | 52.25 |
| 500 | CD | GLN | A | 733 | -2.659 | 21.287 | 26.227 | 1.00 | 53.92 |
| 501 | OE1 | GLN | A | 733 | -3.252 | 20.925 | 27.238 | 1.00 | 59.82 |
| 502 | NE2 | GLN | A | 733 | -2.836 | 20.701 | 25.051 | 1.00 | 55.07 |
| 503 | N | MET | A | 734 | -3.416 | 25.952 | 23.993 | 1.00 | 48.02 |
| 504 | CA | MET | A | 734 | -4.107 | 26.588 | 22.887 | 1.00 | 50.74 |
| 505 | C | MET | A | 734 | -3.448 | 27.904 | 22.453 | 1.00 | 50.43 |
| 506 | O | MET | A | 734 | -3.495 | 28.269 | 21.286 | 1.00 | 53.47 |
| 507 | CB | MET | A | 734 | -5.545 | 26.832 | 23.297 | 1.00 | 56.63 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 508 | CG | MET | A | 734 | -6.530 | 26.884 | 22.158 | 1.00 | 71.95 |
| 509 | SD | MET | A | 734 | -8.226 | 26.806 | 22.797 | 1.00 | 94.18 |
| 510 | CE | MET | A | 734 | -7.929 | 26.442 | 24.662 | 1.00 | 83.04 |
| 511 1 | N | ALA | A | 735 | -2.779 | 28.577 | 23.384 | 1.00 | 49.75 |
| 512 | CA | ALA | A | 735 | -2.115 | 29.844 | 23.109 | 1.00 | 43.40 |
| 513 | C | ALA | A | 735 | -0.820 | 29.686 | 22.305 | 1.00 | 43.42 |
| 514 | O | ALA | A | 735 | -0.749 | 30.185 | 21.185 | 1.00 | 51.41 |
| 515 | CB | ALA | A | 735 | -1.845 | 30.602 | 24.406 | 1.00 | 36.08 |
| 516 | N | VAL | A | 736 | 0.198 | 29.002 | 22.832 | 1.00 | 38.22 |
| 517 | CA | VAL | A | 736 | 1.441 | 28.866 | 22.066 | 1.00 | 36.45 |
| 518 | C | VAL | A | 736 | 1.210 | 28.345 | 20.651 | 1.00 | 36.01 |
| 519 | O | VAL | A | 736 | 1.982 | 28.655 | 19.747 | 1.00 | 41.42 |
| 520 | CB | VAL | A | 736 | 2.502 | 28.000 | 22.764 | 1.00 | 35.87 |
| 521 | CG1 | VAL | A | 736 | 3.048 | 28.714 | 23.978 | 1.00 | 44.32 |
| 522 | CG2 | VAL | A | 736 | 1.924 | 26.673 | 23.156 | 1.00 | 36.64 |
| 523 | N | ILE | A | 737 | 0.132 | 27.585 | 20.465 | 1.00 | 30.08 |
| 524 | CA | ILE | A | 737 | -0.220 | 27.048 | 19.150 | 1.00 | 31.25 |
| 525 | C | ILE | A | 737 | -0.623 | 28.195 | 18.241 | 1.00 | 32.22 |
| 526 | O | ILE | A | 737 | 0.022 | 28.449 | 17.222 | 1.00 | 40.26 |
| 527 | CB | ILE | A | 737 | -1.361 | 25.992 | 19.250 | 1.00 | 32.49 |
| 528 | CG1 | ILE | A | 737 | -0.781 | 24.654 | 19.749 | 1.00 | 36.28 |
| 529 | CG2 | ILE | A | 737 | -2.061 | 25.803 | 17.907 | 1.00 | 22.38 |
| 530 | CD1 | ILE | A | 737 | -1.792 | 23.707 | 20.422 | 1.00 | 39.52 |
| 531 | N | GLN | A | 738 | -1.657 | 28.926 | 18.636 | 1.00 | 34.76 |
| 532 | CA | GLN | A | 738 | -2.127 | 30.064 | 17.857 | 1.00 | 36.96 |
| 533 | C | GLN | A | 738 | -0.999 | 31.078 | 17.575 | 1.00 | 38.78 |
| 534 | O | GLN | A | 738 | -0.780 | 31.434 | 16.422 | 1.00 | 47.01 |
| 535 | CB | GLN | A | 738 | -3.310 | 30.715 | 18.570 | 1.00 | 35.31 |
| 536 | CG | GLN | A | 738 | -4.548 | 29.817 | 18.615 | 1.00 | 36.30 |
| 537 | CD | GLN | A | 738 | -5.569 | 30.254 | 19.666 | 1.00 | 46.43 |
| 538 | OE1 | GLN | A | 738 | -5.196 | 30.752 | 20.728 | 1.00 | 51.09 |
| 539 | NE2 | GLN | A | 738 | -6.860 | 30.059 | 19.379 | 1.00 | 45.97 |
| 540 | N | TYR | A | 739 | -0.206 | 31.428 | 18.586 | 1.00 | 32.35 |
| 541 | CA | TYR | A | 739 | 0.867 | 32.387 | 18.402 | 1.00 | 30.36 |
| 542 | C | TYR | A | 739 | 1.975 | 31.867 | 17.526 | 1.00 | 34.27 |
| 543 | O | TYR | A | 739 | 2.502 | 32.609 | 16.712 | 1.00 | 35.19 |
| 544 | CB | TYR | A | 739 | 1.496 | 32.821 | 19.735 | 1.00 | 36.60 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 545 | CG | TYR | A | 739 | 0.565 | 33.487 | 20.754 | 1.00 | 41.72 |
| 546 | CD1 | TYR | A | 739 | -0.515 | 34.267 | 20.358 | 1.00 | 43.18 |
| 547 | CD2 | TYR | A | 739 | 0.736 | 33.272 | 22.126 | 1.00 | 44.87 |
| 548 | CE1 | TYR | A | 739 | -1.416 | 34.803 | 21.303 | 1.00 | 43.31 |
| 549 | CE2 | TYR | A | 739 | -0.163 | 33.808 | 23.073 | 1.00 | 39.98 |
| 550 | CZ | TYR | A | 739 | -1.239 | 34.561 | 22.649 | 1.00 | 38.00 |
| 551 | OH | TYR | A | 739 | -2.178 | 35.014 | 23.556 | 1.00 | 54.08 |
| 552 | N | SER | A | 740 | 2.351 | 30.606 | 17.674 | 1.00 | 36.52 |
| 553 | CA | SER | A | 740 | 3.459 | 30.114 | 16.875 | 1.00 | 38.17 |
| 554 | C | SER | A | 740 | 3.129 | 29.453 | 15.535 | 1.00 | 38.14 |
| 555 | O | SER | A | 740 | 4.024 | 29.259 | 14.706 | 1.00 | 41.67 |
| 556 | CB | SER | A | 740 | 4.390 | 29.231 | 17.727 | 1.00 | 42.37 |
| 557 | OG | SER | A | 740 | 3.756 | 28.053 | 18.200 | 1.00 | 39.05 |
| 558 | N | TRP | A | 741 | 1.851 | 29.268 | 15.236 | 1.00 | 32.00 |
| 559 | CA | TRP | A | 741 | 1.482 | 28.588 | 14.004 | 1.00 | 32.79 |
| 560 | C | TRP | A | 741 | 2.099 | 29.060 | 12.681 | 1.00 | 34.24 |
| 561 | O | TRP | A | 741 | 2.578 | 28.250 | 11.891 | 1.00 | 34.43 |
| 562 | CB | TRP | A | 741 | -0.034 | 28.446 | 13.918 | 1.00 | 44.21 |
| 563 | CG | TRP | A | 741 | -0.733 | 29.487 | 13.136 | 1.00 | 58.12 |
| 564 | CD1 | TRP | A | 741 | -0.889 | 30.806 | 13.458 | 1.00 | 64.16 |
| 565 | CD2 | TRP | A | 741 | -1.365 | 29.303 | 11.870 | 1.00 | 63.13 |
| 566 | NE1 | TRP | A | 741 | -1.574 | 31.462 | 12.462 | 1.00 | 67.31 |
| 567 | CE2 | TRP | A | 741 | -1.882 | 30.562 | 11.473 | 1.00 | 67.95 |
| 568 | CE3 | TRP | A | 741 | -1.558 | 28.194 | 11.031 | 1.00 | 57.71 |
| 569 | CZ2 | TRP | A | 741 | -2.561 | 30.747 | 10.260 | 1.00 | 70.02 |
| 570 | CZ3 | TRP | A | 741 | -2.232 | 28.373 | 9.831 | 1.00 | 59.16 |
| 571 | CH2 | TRP | A | 741 | -2.731 | 29.642 | 9.458 | 1.00 | 65.30 |
| 572 | N | MET | A | 742 | 2.184 | 30.370 | 12.489 | 1.00 | 41.58 |
| 573 | CA | MET | A | 742 | 2.749 | 30.945 | 11.265 | 1.00 | 39.13 |
| 574 | C | MET | A | 742 | 4.193 | 30.537 | 11.090 | 1.00 | 30.85 |
| 575 | O | MET | A | 742 | 4.602 | 30.115 | 10.017 | 1.00 | 34.78 |
| 576 | CB | MET | A | 742 | 2.689 | 32.476 | 11.309 | 1.00 | 42.39 |
| 577 | CG | MET | A | 742 | 3.147 | 33.177 | 10.032 | 1.00 | 43.70 |
| 578 | SD | MET | A | 742 | 1.988 | 32.993 | 8.658 | 1.00 | 45.17 |
| 579 | CE | MET | A | 742 | 0.678 | 34.132 | 9.133 | 1.00 | 22.14 |
| 580 | N | GLY | A | 743 | 4.954 | 30.648 | 12.165 | 1.00 | 24.94 |
| 581 | CA | GLY | A | 743 | 6.367 | 30.312 | 12.117 | 1.00 | 27.24 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 582 | C | GLY | A | 743 | 6.630 | 28.836 | 11.886 | 1.00 | 27.21 |
| 583 | O | GLY | A | 743 | 7.660 | 28.461 | 11.322 | 1.00 | 27.69 |
| 584 | N | LEU | A | 744 | 5.734 | 27.983 | 12.372 | 1.00 | 25.91 |
| 585 | CA | LEU | A | 744 | 5.895 | 26.550 | 12.172 | 1.00 | 26.90 |
| 586 | C | LEU | A | 744 | 5.632 | 26.287 | 10.708 | 1.00 | 27.04 |
| 587 | O | LEU | A | 744 | 6.375 | 25.574 | 10.048 | 1.00 | 31.01 |
| 588 | CB | LEU | A | 744 | 4.899 | 25.755 | 13.018 | 1.00 | 25.72 |
| 589 | CG | LEU | A | 744 | 5.234 | 25.626 | 14.514 | 1.00 | 29.11 |
| 590 | CD1 | LEU | A | 744 | 4.063 | 25.022 | 15.275 | 1.00 | 23.25 |
| 591 | CD2 | LEU | A | 744 | 6.484 | 24.771 | 14.689 | 1.00 | 24.15 |
| 592 | N | MET | A | 745 | 4.566 | 26.886 | 10.200 | 1.00 | 25.67 |
| 593 | CA | MET | A | 745 | 4.188 | 26.725 | 8.803 | 1.00 | 23.96 |
| 594 | C | MET | A | 745 | 5.254 | 27.179 | 7.822 | 1.00 | 29.61 |
| 595 | O | MET | A | 745 | 5.550 | 26.480 | 6.857 | 1.00 | 34.60 |
| 596 | CB | MET | A | 745 | 2.895 | 27.454 | 8.534 | 1.00 | 20.46 |
| 597 | CG | MET | A | 745 | 1.730 | 26.888 | 9.310 | 1.00 | 19.98 |
| 598 | SD | MET | A | 745 | 0.297 | 27.272 | 8.341 | 1.00 | 43.15 |
| 599 | CE | MET | A | 745 | 0.642 | 29.041 | 8.042 | 1.00 | 44.27 |
| 600 | N | VAL | A | 746 | 5.830 | 28.341 | 8.095 | 1.00 | 27.98 |
| 601 | CA | VAL | A | 746 | 6.876 | 28.924 | 7.288 | 1.00 | 24.84 |
| 602 | C | VAL | A | 746 | 8.107 | 28.051 | 7.345 | 1.00 | 28.42 |
| 603 | O | VAL | A | 746 | 8.749 | 27.786 | 6.333 | 1.00 | 37.05 |
| 604 | CB | VAL | A | 746 | 7.248 | 30.304 | 7.835 | 1.00 | 31.98 |
| 605 | CG1 | VAL | A | 746 | 8.423 | 30.888 | 7.073 | 1.00 | 29.03 |
| 606 | CG2 | VAL | A | 746 | 6.066 | 31.196 | 7.737 | 1.00 | 32.19 |
| 607 | N | PHE | A | 747 | 8.439 | 27.607 | 8.541 | 1.00 | 31.29 |
| 608 | CA | PHE | A | 747 | 9.605 | 26.765 | 8.736 | 1.00 | 32.19 |
| 609 | C | PHE | A | 747 | 9.468 | 25.401 | 8.030 | 1.00 | 35.99 |
| 610 | O | PHE | A | 747 | 10.398 | 24.916 | 7.384 | 1.00 | 34.95 |
| 611 | CB | PHE | A | 747 | 9.820 | 26.536 | 10.224 | 1.00 | 27.90 |
| 612 | CG | PHE | A | 747 | 11.209 | 26.082 | 10.573 | 1.00 | 26.00 |
| 613 | CD1 | PHE | A | 747 | 12.293 | 26.915 | 10.343 | 1.00 | 24.54 |
| 614 | CD2 | PHE | A | 747 | 11.428 | 24.846 | 11.166 | 1.00 | 27.23 |
| 615 | CE1 | PHE | A | 747 | 13.571 | 26.532 | 10.699 | 1.00 | 25.88 |
| 616 | CE2 | PHE | A | 747 | 12.711 | 24.451 | 11.528 | 1.00 | 25.61 |
| 617 | CZ | PHE | A | 747 | 13.785 | 25.297 | 11.293 | 1.00 | 28.75 |
| 618 | N | ALA | A | 748 | 8.309 | 24.774 | 8.171 | 1.00 | 35.11 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 619 | CA | ALA | A | 748 | 8.096 | 23.483 | 7.561 | 1.00 | 34.00 |
| 620 | C | ALA | A | 748 | 8.114 | 23.683 | 6.054 | 1.00 | 37.26 |
| 621 | O | ALA | A | 748 | 8.831 | 22.973 | 5.344 | 1.00 | 35.87 |
| 622 | CB | ALA | A | 748 | 6.773 | 22.896 | 8.022 | 1.00 | 29.48 |
| 623 | N | MET | A | 749 | 7.385 | 24.707 | 5.591 | 1.00 | 41.15 |
| 624 | CA | MET | A | 749 | 7.277 | 25.044 | 4.167 | 1.00 | 33.31 |
| 625 | C | MET | A | 749 | 8.647 | 25.225 | 3.563 | 1.00 | 35.12 |
| 626 | O | MET | A | 749 | 8.934 | 24.711 | 2.491 | 1.00 | 37.94 |
| 627 | CB | MET | A | 749 | 6.444 | 26.303 | 3.982 | 1.00 | 35.95 |
| 628 | CG | MET | A | 749 | 6.179 | 26.682 | 2.542 | 1.00 | 45.47 |
| 629 | SD | MET | A | 749 | 7.444 | 27.726 | 1.821 | 1.00 | 50.89 |
| 630 | CE | MET | A | 749 | 7.553 | 28.954 | 3.104 | 1.00 | 55.72 |
| 631 | N | GLY | A | 750 | 9.507 | 25.929 | 4.278 | 1.00 | 36.97 |
| 632 | CA | GLY | A | 750 | 10.863 | 26.138 | 3.810 | 1.00 | 42.04 |
| 633 | C | GLY | A | 750 | 11.628 | 24.828 | 3.715 | 1.00 | 43.22 |
| 634 | O | GLY | A | 750 | 12.530 | 24.678 | 2.889 | 1.00 | 43.48 |
| 635 | N | TRP | A | 751 | 11.304 | 23.876 | 4.581 | 1.00 | 45.43 |
| 636 | CA | TRP | A | 751 | 11.976 | 22.588 | 4.528 | 1.00 | 42.53 |
| 637 | C | TRP | A | 751 | 11.519 | 21.806 | 3.294 | 1.00 | 43.17 |
| 638 | O | TRP | A | 751 | 12.336 | 21.207 | 2.596 | 1.00 | 40.24 |
| 639 | CB | TRP | A | 751 | 11.717 | 21.776 | 5.787 | 1.00 | 39.21 |
| 640 | CG | TRP | A | 751 | 12.359 | 20.401 | 5.737 | 1.00 | 41.85 |
| 641 | CD1 | TRP | A | 751 | 11.736 | 19.213 | 5.461 | 1.00 | 39.44 |
| 642 | CD2 | TRP | A | 751 | 13.743 | 20.085 | 5.968 | 1.00 | 37.37 |
| 643 | NE1 | TRP | A | 751 | 12.645 | 18.186 | 5.516 | 1.00 | 42.23 |
| 644 | CE2 | TRP | A | 751 | 13.878 | 18.692 | 5.821 | 1.00 | 41.26 |
| 645 | CE3 | TRP | A | 751 | 14.877 | 20.841 | 6.275 | 1.00 | 41.35 |
| 646 | CZ2 | TRP | A | 751 | 15.110 | 18.046 | 5.978 | 1.00 | 48.39 |
| 647 | CZ3 | TRP | A | 751 | 16.104 | 20.195 | 6.431 | 1.00 | 39.62 |
| 648 | CH2 | TRP | A | 751 | 16.208 | 18.817 | 6.280 | 1.00 | 43.38 |
| 649 | N | ARG | A | 752 | 10.214 | 21.792 | 3.037 | 1.00 | 42.27 |
| 650 | CA | ARG | A | 752 | 9.683 | 21.100 | 1.862 | 1.00 | 41.53 |
| 651 | C | ARG | A | 752 | 10.257 | 21.740 | 0.602 | 1.00 | 44.18 |
| 652 | O | ARG | A | 752 | 10.522 | 21.048 | -0.380 | 1.00 | 43.20 |
| 653 | CB | ARG | A | 752 | 8.163 | 21.186 | 1.800 | 1.00 | 42.14 |
| 654 | CG | ARG | A | 752 | 7.441 | 20.465 | 2.920 | 1.00 | 49.76 |
| 655 | CD | ARG | A | 752 | 5.938 | 20.434 | 2.649 | 1.00 | 48.23 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 656 | NE | ARG | A | 752 | 5.382 | 21.773 | 2.483 | 1.00 | 45.23 |
| 657 | CZ | ARG | A | 752 | 5.013 | 22.572 | 3.490 | 1.00 | 52.17 |
| 658 | NH1 | ARG | A | 752 | 5.131 | 22.175 | 4.764 | 1.00 | 33.80 |
| 659 | NH2 | ARG | A | 752 | 4.536 | 23.785 | 3.223 | 1.00 | 49.84 |
| 660 | N | SER | A | 753 | 10.441 | 23.058 | 0.624 | 1.00 | 38.26 |
| 661 | CA | SER | A | 753 | 10.998 | 23.733 | -0.523 | 1.00 | 35.26 |
| 662 | C | SER | A | 753 | 12.453 | 23.400 | -0.707 | 1.00 | 35.66 |
| 663 | O | SER | A | 753 | 12.973 | 23.538 | -1.807 | 1.00 | 43.18 |
| 664 | CB | SER | A | 753 | 10.798 | 25.233 | -0.444 | 1.00 | 33.49 |
| 665 | OG | SER | A | 753 | 9.414 | 25.514 | -0.453 | 1.00 | 36.06 |
| 666 | N | PHE | A | 754 | 13.113 | 22.938 | 0.343 | 1.00 | 33.77 |
| 667 | CA | PHE | A | 754 | 14.523 | 22.575 | 0.224 | 1.00 | 41.59 |
| 668 | C | PHE | A | 754 | 14.709 | 21.153 | -0.316 | 1.00 | 49.14 |
| 669 | O | PHE | A | 754 | 15.524 | 20.919 | -1.211 | 1.00 | 48.97 |
| 670 | CB | PHE | A | 754 | 15.242 | 22.702 | 1.564 | 1.00 | 39.18 |
| 671 | CG | PHE | A | 754 | 16.668 | 22.204 | 1.540 | 1.00 | 40.02 |
| 672 | CD1 | PHE | A | 754 | 17.596 | 22.746 | 0.645 | 1.00 | 48.31 |
| 673 | CD2 | PHE | A | 754 | 17.086 | 21.202 | 2.423 | 1.00 | 36.75 |
| 674 | CE1 | PHE | A | 754 | 18.923 | 22.311 | 0.635 | 1.00 | 47.66 |
| 675 | CE2 | PHE | A | 754 | 18.411 | 20.757 | 2.432 | 1.00 | 41.02 |
| 676 | CZ | PHE | A | 754 | 19.333 | 21.313 | 1.532 | 1.00 | 50.13 |
| 677 | N | THR | A | 755 | 13.948 | 20.208 | 0.225 | 1.00 | 53.24 |
| 678 | CA | THR | A | 755 | 14.053 | 18.818 | -0.197 | 1.00 | 53.33 |
| 679 | C | THR | A | 755 | 13.287 | 18.474 | -1.478 | 1.00 | 54.87 |
| 680 | O | THR | A | 755 | 13.554 | 17.431 | -2.068 | 1.00 | 57.41 |
| 681 | CB | THR | A | 755 | 13.596 | 17.830 | 0.934 | 1.00 | 45.98 |
| 682 | OG1 | THR | A | 755 | 12.221 | 18.055 | 1.245 | 1.00 | 49.35 |
| 683 | CG2 | THR | A | 755 | 14.405 | 18.033 | 2.190 | 1.00 | 40.03 |
| 684 | N | ASN | A | 756 | 12.360 | 19.336 | -1.911 | 1.00 | 52.97 |
| 685 | CA | ASN | A | 756 | 11.539 | 19.044 | -3.097 | 1.00 | 56.15 |
| 686 | C | ASN | A | 756 | 11.821 | 19.826 | -4.394 | 1.00 | 55.33 |
| 687 | O | ASN | A | 756 | 11.705 | 19.257 | -5.481 | 1.00 | 54.81 |
| 688 | CB | ASN | A | 756 | 10.019 | 19.124 | -2.769 | 1.00 | 60.43 |
| 689 | CG | ASN | A | 756 | 9.504 | 17.959 | -1.869 | 1.00 | 57.82 |
| 690 | OD1 | ASN | A | 756 | 10.123 | 16.909 | -1.763 | 1.00 | 55.71 |
| 691 | ND2 | ASN | A | 756 | 8.354 | 18.169 | -1.234 | 1.00 | 56.46 |
| 692 | N | VAL | A | 757 | 12.155 | 21.115 | -4.293 | 1.00 | 54.53 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 693 | CA | VAL | A | 757 | 12.427 | 21.962 | -5.470 | 1.00 | 50.64 |
| 694 | C | VAL | A | 757 | 13.732 | 22.776 | -5.370 | 1.00 | 47.30 |
| 695 | O | VAL | A | 757 | 13.903 | 23.786 | -6.053 | 1.00 | 48.03 |
| 696 | CB | VAL | A | 757 | 11.239 | 22.943 | -5.756 | 1.00 | 50.94 |
| 697 | CG1 | VAL | A | 757 | 9.927 | 22.177 | -5.892 | 1.00 | 53.22 |
| 698 | CG2 | VAL | A | 757 | 11.125 | 23.998 | -4.660 | 1.00 | 54.28 |
| 699 | N | ASN | A | 758 | 14.676 | 22.274 | -4.582 | 1.00 | 53.28 |
| 700 | CA | ASN | A | 758 | 15.977 | 22.917 | -4.340 | 1.00 | 61.04 |
| 701 | C | ASN | A | 758 | 15.937 | 24.448 | -4.140 | 1.00 | 63.53 |
| 702 | O | ASN | A | 758 | 16.674 | 25.191 | -4.802 | 1.00 | 65.98 |
| 703 | CB | ASN | A | 758 | 17.026 | 22.499 | -5.388 | 1.00 | 63.98 |
| 704 | CG | ASN | A | 758 | 18.440 | 22.442 | -4.813 | 1.00 | 62.08 |
| 705 | OD1 | ASN | A | 758 | 18.681 | 22.852 | -3.679 | 0.00 | 62.62 |
| 706 | ND2 | ASN | A | 758 | 19.369 | 21.908 | -5.590 | 0.00 | 62.48 |
| 707 | N | SER | A | 759 | 14.999 | 24.879 | -3.281 | 1.00 | 63.72 |
| 708 | CA | SER | A | 759 | 14.763 | 26.267 | -2.849 | 1.00 | 61.29 |
| 709 | C | SER | A | 759 | 14.379 | 27.351 | -3.849 | 1.00 | 66.13 |
| 710 | O | SER | A | 759 | 14.067 | 28.472 | -3.443 | 1.00 | 71.37 |
| 711 | CB | SER | A | 759 | 15.939 | 26.770 | -1.995 | 1.00 | 60.64 |
| 712 | OG | SER | A | 759 | 16.080 | 26.018 | -0.799 | 1.00 | 55.92 |
| 713 | N | ARG | A | 760 | 14.388 | 27.045 | -5.140 | 1.00 | 64.35 |
| 714 | CA | ARG | A | 760 | 14.041 | 28.064 | -6.109 | 1.00 | 59.78 |
| 715 | C | ARG | A | 760 | 12.551 | 28.416 | -6.070 | 1.00 | 55.67 |
| 716 | O | ARG | A | 760 | 12.186 | 29.563 | -6.345 | 1.00 | 60.84 |
| 717 | CB | ARG | A | 760 | 14.492 | 27.658 | -7.507 | 1.00 | 69.19 |
| 718 | CG | ARG | A | 760 | 14.046 | 26.283 | -7.883 | 1.00 | 73.58 |
| 719 | CD | ARG | A | 760 | 13.925 | 26.094 | -9.381 | 1.00 | 78.27 |
| 720 | NE | ARG | A | 760 | 13.214 | 24.847 | -9.635 | 1.00 | 80.85 |
| 721 | CZ | ARG | A | 760 | 13.642 | 23.646 | -9.246 | 1.00 | 80.46 |
| 722 | NH1 | ARG | A | 760 | 14.801 | 23.528 | -8.597 | 1.00 | 79.19 |
| 723 | NH2 | ARG | A | 760 | 12.853 | 22.584 | -9.388 | 1.00 | 76.84 |
| 724 | N | MET | A | 761 | 11.701 | 27.453 | -5.707 | 1.00 | 46.97 |
| 725 | CA | MET | A | 761 | 10.256 | 27.695 | -5.622 | 1.00 | 41.01 |
| 726 | C | MET | A | 761 | 9.744 | 27.418 | -4.222 | 1.00 | 37.53 |
| 727 | O | MET | A | 761 | 10.451 | 26.824 | -3.412 | 1.00 | 38.47 |
| 728 | CB | MET | A | 761 | 9.496 | 26.827 | -6.612 | 1.00 | 38.49 |
| 729 | CG | MET | A | 761 | 9.926 | 27.040 | -8.034 | 1.00 | 42.52 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 730 | SD | MET | A | 761 | 8.981 | 25.994 | -9.104 | 1.00 | 50.12 |
| 731 | CE | MET | A | 761 | 10.000 | 24.558 | -9.111 | 1.00 | 43.61 |
| 732 | N | LEU | A | 762 | 8.526 | 27.860 | -3.930 | 1.00 | 35.00 |
| 733 | CA | LEU | A | 762 | 7.949 | 27.638 | -2.614 | 1.00 | 36.81 |
| 734 | C | LEU | A | 762 | 6.902 | 26.548 | -2.664 | 1.00 | 41.13 |
| 735 | O | LEU | A | 762 | 5.821 | 26.739 | -3.243 | 1.00 | 36.62 |
| 736 | CB | LEU | A | 762 | 7.344 | 28.912 | -2.048 | 1.00 | 39.16 |
| 737 | CG | LEU | A | 762 | 8.366 | 30.021 | -1.827 | 1.00 | 43.49 |
| 738 | CD1 | LEU | A | 762 | 7.670 | 31.178 | -1.169 | 1.00 | 45.89 |
| 739 | CD2 | LEU | A | 762 | 9.498 | 29.530 | -0.952 | 1.00 | 44.69 |
| 740 | N | TYR | A | 763 | 7.245 | 25.419 | -2.033 | 1.00 | 44.41 |
| 741 | CA | TYR | A | 763 | 6.422 | 24.209 | -1.963 | 1.00 | 39.75 |
| 742 | C | TYR | A | 763 | 5.412 | 24.241 | -0.839 | 1.00 | 36.53 |
| 743 | O | TYR | A | 763 | 5.504 | 23.487 | 0.129 | 1.00 | 36.31 |
| 744 | CB | TYR | A | 763 | 7.323 | 22.968 | -1.831 | 1.00 | 47.60 |
| 745 | CG | TYR | A | 763 | 6.713 | 21.644 | -2.309 | 1.00 | 61.61 |
| 746 | CD1 | TYR | A | 763 | 5.701 | 21.002 | -1.579 | 1.00 | 67.78 |
| 747 | CD2 | TYR | A | 763 | 7.174 | 21.015 | -3.476 | 1.00 | 62.67 |
| 748 | CE1 | TYR | A | 763 | 5.164 | 19.770 | -1.998 | 1.00 | 63.34 |
| 749 | CE2 | TYR | A | 763 | 6.639 | 19.778 | -3.897 | 1.00 | 59.80 |
| 750 | CZ | TYR | A | 763 | 5.634 | 19.173 | -3.149 | 1.00 | 62.05 |
| 751 | OH | TYR | A | 763 | 5.066 | 17.995 | -3.558 | 1.00 | 63.40 |
| 752 | N | PHE | A | 764 | 4.445 | 25.130 | -0.958 | 1.00 | 37.20 |
| 753 | CA | PHE | A | 764 | 3.409 | 25.221 | 0.042 | 1.00 | 38.87 |
| 754 | C | PHE | A | 764 | 2.681 | 23.864 | 0.164 | 1.00 | 47.03 |
| 755 | O | PHE | A | 764 | 2.506 | 23.333 | 1.263 | 1.00 | 48.96 |
| 756 | CB | PHE | A | 764 | 2.441 | 26.350 | -0.321 | 1.00 | 34.03 |
| 757 | CG | PHE | A | 764 | 3.048 | 27.695 | -0.225 | 1.00 | 29.36 |
| 758 | CD1 | PHE | A | 764 | 3.571 | 28.142 | 0.976 | 1.00 | 32.46 |
| 759 | CD2 | PHE | A | 764 | 3.129 | 28.510 | -1.328 | 1.00 | 37.55 |
| 760 | CE1 | PHE | A | 764 | 4.169 | 29.384 | 1.076 | 1.00 | 35.80 |
| 761 | CE2 | PHE | A | 764 | 3.727 | 29.759 | -1.236 | 1.00 | 39.68 |
| 762 | CZ | PHE | A | 764 | 4.247 | 30.196 | -0.033 | 1.00 | 29.54 |
| 763 | N | ALA | A | 765 | 2.314 | 23.286 | -0.978 | 1.00 | 56.26 |
| 764 | CA | ALA | A | 765 | 1.608 | 21.999 | -1.049 | 1.00 | 51.43 |
| 765 | C | ALA | A | 765 | 1.969 | 21.343 | -2.386 | 1.00 | 51.42 |
| 766 | O | ALA | A | 765 | 2.449 | 22.022 | -3.289 | 1.00 | 51.71 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 767 | CB | ALA | A | 765 | 0.105 | 22.232 | -0.959 | 1.00 | 47.80 |
| 768 | N | PRO | A | 766 | 1.766 | 20.021 | -2.533 | 1.00 | 52.86 |
| 769 | CA | PRO | A | 766 | 2.120 | 19.407 | -3.813 | 1.00 | 50.96 |
| 770 | C | PRO | A | 766 | 1.359 | 20.040 | -4.970 | 1.00 | 48.25 |
| 771 | O | PRO | A | 766 | 1.893 | 20.144 | -6.082 | 1.00 | 44.31 |
| 772 | CB | PRO | A | 766 | 1.721 | 17.949 | -3.604 | 1.00 | 57.00 |
| 773 | CG | PRO | A | 766 | 1.899 | 17.761 | -2.133 | 1.00 | 58.08 |
| 774 | CD | PRO | A | 766 | 1.237 | 19.002 | -1.617 | 1.00 | 57.23 |
| 775 | N | ASP | A | 767 | 0.128 | 20.479 | -4.707 | 1.00 | 41.59 |
| 776 | CA | ASP | A | 767 | -0.681 | 21.119 | -5.743 | 1.00 | 47.13 |
| 777 | C | ASP | A | 767 | -0.774 | 22.666 | -5.645 | 1.00 | 51.59 |
| 778 | O | ASP | A | 767 | -1.618 | 23.311 | -6.292 | 1.00 | 55.70 |
| 779 | CB | ASP | A | 767 | -2.073 | 20.485 | -5.785 | 1.00 | 48.65 |
| 780 | CG | ASP | A | 767 | -2.833 | 20.667 | -4.505 | 1.00 | 51.77 |
| 781 | OD1 | ASP | A | 767 | -2.205 | 20.619 | -3.431 | 1.00 | 54.76 |
| 782 | OD2 | ASP | A | 767 | -4.071 | 20.839 | -4.573 | 1.00 | 59.43 |
| 783 | N | LEU | A | 768 | 0.148 | 23.255 | -4.893 | 1.00 | 48.07 |
| 784 | CA | LEU | A | 768 | 0.196 | 24.697 | -4.719 | 1.00 | 47.04 |
| 785 | C | LEU | A | 768 | 1.658 | 25.080 | -4.564 | 1.00 | 49.99 |
| 786 | O | LEU | A | 768 | 2.141 | 25.212 | -3.436 | 1.00 | 53.64 |
| 787 | CB | LEU | A | 768 | -0.574 | 25.108 | -3.450 | 1.00 | 51.86 |
| 788 | CG | LEU | A | 768 | -0.631 | 26.580 | -3.002 | 1.00 | 50.53 |
| 789 | CD1 | LEU | A | 768 | -1.328 | 27.390 | -4.062 | 1.00 | 47.97 |
| 790 | CD2 | LEU | A | 768 | -1.376 | 26.701 | -1.702 | 1.00 | 46.42 |
| 791 | N | VAL | A | 769 | 2.387 | 25.176 | -5.676 | 1.00 | 48.70 |
| 792 | CA | VAL | A | 769 | 3.796 | 25.575 | -5.620 | 1.00 | 48.86 |
| 793 | C | VAL | A | 769 | 3.954 | 26.933 | -6.287 | 1.00 | 47.94 |
| 794 | O | VAL | A | 769 | 3.352 | 27.190 | -7.335 | 1.00 | 49.55 |
| 795 | CB | VAL | A | 769 | 4.728 | 24.572 | -6.321 | 1.00 | 50.85 |
| 796 | CG1 | VAL | A | 769 | 6.181 | 25.049 | -6.216 | 1.00 | 53.54 |
| 797 | CG2 | VAL | A | 769 | 4.581 | 23.208 | -5.687 | 1.00 | 53.42 |
| 798 | N | PHE | A | 770 | 4.734 | 27.808 | -5.668 | 1.00 | 43.61 |
| 799 | CA | PHE | A | 770 | 4.935 | 29.124 | -6.230 | 1.00 | 40.71 |
| 800 | C | PHE | A | 770 | 6.179 | 29.276 | -7.044 | 1.00 | 39.27 |
| 801 | O | PHE | A | 770 | 7.291 | 29.151 | -6.531 | 1.00 | 38.13 |
| 802 | CB | PHE | A | 770 | 4.939 | 30.197 | -5.147 | 1.00 | 41.24 |
| 803 | CG | PHE | A | 770 | 3.582 | 30.650 | -4.753 | 1.00 | 39.60 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 804 | CD1 | PHE | A | 770 | 2.489 | 29.803 | -4.896 | 1.00 | 40.94 |
| 805 | CD2 | PHE | A | 770 | 3.391 | 31.916 | -4.220 | 1.00 | 35.47 |
| 806 | CE1 | PHE | A | 770 | 1.216 | 30.208 | -4.515 | 1.00 | 39.80 |
| 807 | CE2 | PHE | A | 770 | 2.123 | 32.332 | -3.834 | 1.00 | 37.83 |
| 808 | CZ | PHE | A | 770 | 1.027 | 31.476 | -3.980 | 1.00 | 36.48 |
| 809 | N | ASN | A | 771 | 5.988 | 29.397 | -8.345 | 1.00 | 38.22 |
| 810 | CA | ASN | A | 771 | 7.113 | 29.673 | -9.219 | 1.00 | 39.10 |
| 811 | C | ASN | A | 771 | 7.181 | 31.196 | -9.066 | 1.00 | 43.17 |
| 812 | O | ASN | A | 771 | 6.341 | 31.785 | -8.345 | 1.00 | 39.83 |
| 813 | CB | ASN | A | 771 | 6.834 | 29.268 | -10.685 | 1.00 | 33.28 |
| 814 | CG | ASN | A | 771 | 5.384 | 29.492 | -11.110 | 1.00 | 35.37 |
| 815 | OD1 | ASN | A | 771 | 4.656 | 30.309 | -10.546 | 1.00 | 45.29 |
| 816 | ND2 | ASN | A | 771 | 4.956 | 28.737 | -12.094 | 1.00 | 41.79 |
| 817 | N | GLU | A | 772 | 8.166 | 31.836 | -9.694 | 1.00 | 43.13 |
| 818 | CA | GLU | A | 772 | 8.264 | 33.285 | -9.605 | 1.00 | 42.44 |
| 819 | C | GLU | A | 772 | 7.015 | 33.997 | -10.136 | 1.00 | 45.81 |
| 820 | O | GLU | A | 772 | 6.615 | 35.016 | -9.579 | 1.00 | 53.45 |
| 821 | CB | GLU | A | 772 | 9.541 | 33.815 | -10.274 | 1.00 | 52.58 |
| 822 | CG | GLU | A | 772 | 10.814 | 33.657 | -9.401 | 1.00 | 58.43 |
| 823 | CD | GLU | A | 772 | 11.791 | 34.850 | -9.483 | 1.00 | 56.79 |
| 824 | OE1 | GLU | A | 772 | 11.719 | 35.655 | -10.434 | 1.00 | 55.14 |
| 825 | OE2 | GLU | A | 772 | 12.654 | 34.974 | -8.583 | 1.00 | 61.19 |
| 826 | N | TYR | A | 773 | 6.354 | 33.454 | -11.157 | 1.00 | 39.45 |
| 827 | CA | TYR | A | 773 | 5.147 | 34.117 | -11.659 | 1.00 | 38.06 |
| 828 | C | TYR | A | 773 | 4.088 | 34.215 | -10.565 | 1.00 | 38.71 |
| 829 | O | TYR | A | 773 | 3.493 | 35.271 | -10.391 | 1.00 | 41.42 |
| 830 | CB | TYR | A | 773 | 4.563 | 33.425 | -12.907 | 1.00 | 37.63 |
| 831 | CG | TYR | A | 773 | 3.266 | 34.033 | -13.417 | 1.00 | 38.44 |
| 832 | CD1 | TYR | A | 773 | 3.248 | 35.277 | -14.059 | 1.00 | 36.94 |
| 833 | CD2 | TYR | A | 773 | 2.043 | 33.397 | -13.197 | 1.00 | 40.91 |
| 834 | CE1 | TYR | A | 773 | 2.038 | 35.883 | -14.459 | 1.00 | 34.34 |
| 835 | CE2 | TYR | A | 773 | 0.833 | 33.990 | -13.595 | 1.00 | 46.98 |
| 836 | CZ | TYR | A | 773 | 0.835 | 35.235 | -14.223 | 1.00 | 42.85 |
| 837 | OH | TYR | A | 773 | -0.361 | 35.824 | -14.599 | 1.00 | 42.16 |
| 838 | N | ARG | A | 774 | 3.864 | 33.137 | -9.817 | 1.00 | 43.58 |
| 839 | CA | ARG | A | 774 | 2.863 | 33.146 | -8.751 | 1.00 | 41.94 |
| 840 | C | ARG | A | 774 | 3.216 | 34.035 | -7.578 | 1.00 | 41.03 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 841 | O | ARG | A | 774 | 2.330 | 34.701 | -7.032 | 1.00 | 43.75 |
| 842 | CB | ARG | A | 774 | 2.580 | 31.748 | -8.252 | 1.00 | 46.64 |
| 843 | CG | ARG | A | 774 | 1.421 | 31.121 | -8.923 | 1.00 | 45.32 |
| 844 | CD | ARG | A | 774 | 1.735 | 29.667 | -9.102 | 1.00 | 47.16 |
| 845 | NE | ARG | A | 774 | 0.588 | 28.968 | -9.642 | 1.00 | 43.73 |
| 846 | CZ | ARG | A | 774 | 0.344 | 27.682 | -9.445 | 1.00 | 41.94 |
| 847 | NH1 | ARG | A | 774 | 1.183 | 26.954 | -8.717 | 1.00 | 42.65 |
| 848 | NH2 | ARG | A | 774 | -0.753 | 27.136 | -9.952 | 1.00 | 39.92 |
| 849 | N | MET | A | 775 | 4.486 | 34.023 | -7.168 | 1.00 | 34.68 |
| 850 | CA | MET | A | 775 | 4.955 | 34.877 | -6.069 | 1.00 | 38.36 |
| 851 | C | MET | A | 775 | 4.496 | 36.328 | -6.332 | 1.00 | 41.88 |
| 852 | O | MET | A | 775 | 4.119 | 37.065 | -5.416 | 1.00 | 41.28 |
| 853 | CB | MET | A | 775 | 6.485 | 34.839 | -5.975 | 1.00 | 33.18 |
| 854 | CG | MET | A | 775 | 7.046 | 33.622 | -5.276 | 1.00 | 24.32 |
| 855 | SD | MET | A | 775 | 8.813 | 33.416 | -5.539 | 1.00 | 40.19 |
| 856 | CE | MET | A | 775 | 9.443 | 34.965 | -4.970 | 1.00 | 48.34 |
| 857 | N | HIS | A | 776 | 4.484 | 36.695 | -7.608 | 1.00 | 46.87 |
| 858 | CA | HIS | A | 776 | 4.065 | 38.006 | -8.065 | 1.00 | 41.06 |
| 859 | C | HIS | A | 776 | 2.568 | 38.136 | -8.311 | 1.00 | 43.18 |
| 860 | O | HIS | A | 776 | 1.969 | 39.153 | -7.991 | 1.00 | 48.98 |
| 861 | CB | HIS | A | 776 | 4.804 | 38.351 | -9.348 | 1.00 | 45.79 |
| 862 | CG | HIS | A | 776 | 4.486 | 39.712 | -9.873 | 1.00 | 44.99 |
| 863 | ND1 | HIS | A | 776 | 3.327 | 39.992 | -10.560 | 1.00 | 46.34 |
| 864 | CD2 | HIS | A | 776 | 5.167 | 40.879 | -9.788 | 1.00 | 44.43 |
| 865 | CE1 | HIS | A | 776 | 3.300 | 41.270 | -10.877 | 1.00 | 44.55 |
| 866 | NE2 | HIS | A | 776 | 4.406 | 41.833 | -10.419 | 1.00 | 49.86 |
| 867 | N | LYS | A | 777 | 1.964 | 37.143 | -8.938 | 1.00 | 46.29 |
| 868 | CA | LYS | A | 777 | 0.542 | 37.214 | -9.220 | 1.00 | 48.46 |
| 869 | C | LYS | A | 777 | -0.276 | 37.241 | -7.920 | 1.00 | 49.12 |
| 870 | O | LYS | A | 777 | -1.356 | 37.835 | -7.869 | 1.00 | 53.96 |
| 871 | CB | LYS | A | 777 | 0.128 | 36.042 | -10.126 | 1.00 | 50.10 |
| 872 | CG | LYS | A | 777 | -1.193 | 36.233 | -10.872 | 1.00 | 58.08 |
| 873 | CD | LYS | A | 777 | -1.161 | 37.460 | -11.791 | 1.00 | 65.16 |
| 874 | CE | LYS | A | 777 | -2.090 | 38.590 | -11.301 | 1.00 | 75.26 |
| 875 | NZ | LYS | A | 777 | -3.524 | 38.363 | -11.635 | 1.00 | 77.33 |
| 876 | N | SER | A | 778 | 0.249 | 36.616 | -6.870 | 1.00 | 50.25 |
| 877 | CA | SER | A | 778 | -0.432 | 36.551 | -5.571 | 1.00 | 48.63 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 878 | C | SER | A | 778 | -0.193 | 37.808 | -4.756 | 1.00 | 53.35 |
| 879 | O | SER | A | 778 | -0.827 | 38.019 | -3.727 | 1.00 | 57.00 |
| 880 | CB | SER | A | 778 | 0.116 | 35.397 | -4.761 | 1.00 | 42.58 |
| 881 | OG | SER | A | 778 | 1.446 | 35.715 | -4.386 | 1.00 | 43.83 |
| 882 | N | ARG | A | 779 | 0.842 | 38.546 | -5.141 | 1.00 | 58.37 |
| 883 | CA | ARG | A | 779 | 1.242 | 39.798 | -4.491 | 1.00 | 56.74 |
| 884 | C | ARG | A | 779 | 1.901 | 39.668 | -3.113 | 1.00 | 54.76 |
| 885 | O | ARG | A | 779 | 1.801 | 40.580 | -2.276 | 1.00 | 51.96 |
| 886 | CB | ARG | A | 779 | 0.081 | 40.799 | -4.461 | 1.00 | 57.70 |
| 887 | CG | ARG | A | 779 | -0.472 | 41.124 | -5.841 | 1.00 | 56.33 |
| 888 | CD | ARG | A | 779 | -1.603 | 42.120 | -5.735 | 1.00 | 58.79 |
| 889 | NE | ARG | A | 779 | -2.471 | 42.088 | -6.907 | 1.00 | 62.79 |
| 890 | CZ | ARG | A | 779 | -3.095 | 43.150 | -7.397 | 1.00 | 61.40 |
| 891 | NH1 | ARG | A | 779 | -2.935 | 44.337 | -6.824 | 1.00 | 62.02 |
| 892 | NH2 | ARG | A | 779 | -3.912 | 43.007 | -8.426 | 1.00 | 65.13 |
| 893 | N | MET | A | 780 | 2.600 | 38.551 | -2.897 | 1.00 | 46.79 |
| 894 | CA | MET | A | 780 | 3.323 | 38.334 | -1.650 | 1.00 | 42.92 |
| 895 | C | MET | A | 780 | 4.773 | 38.214 | -2.049 | 1.00 | 41.04 |
| 896 | O | MET | A | 780 | 5.595 | 37.686 | -1.311 | 1.00 | 43.38 |
| 897 | CB | MET | A | 780 | 2.953 | 37.015 | -1.005 | 1.00 | 45.45 |
| 898 | CG | MET | A | 780 | 1.530 | 36.831 | -0.596 | 1.00 | 54.10 |
| 899 | SD | MET | A | 780 | 1.554 | 35.622 | 0.748 | 1.00 | 62.75 |
| 900 | CE | MET | A | 780 | 2.616 | 34.289 | 0.007 | 1.00 | 62.66 |
| 901 | N | TYR | A | 781 | 5.092 | 38.712 | -3.229 | 1.00 | 39.62 |
| 902 | CA | TYR | A | 781 | 6.429 | 38.610 | -3.744 | 1.00 | 33.06 |
| 903 | C | TYR | A | 781 | 7.532 | 38.923 | -2.753 | 1.00 | 33.20 |
| 904 | O | TYR | A | 781 | 8.499 | 38.166 | -2.682 | 1.00 | 40.06 |
| 905 | CB | TYR | A | 781 | 6.581 | 39.428 | -5.019 | 1.00 | 33.87 |
| 906 | CG | TYR | A | 781 | 7.782 | 38.995 | -5.819 | 1.00 | 46.69 |
| 907 | CD1 | TYR | A | 781 | 9.063 | 39.330 | -5.400 | 1.00 | 53.38 |
| 908 | CD2 | TYR | A | 781 | 7.651 | 38.203 | -6.952 | 1.00 | 48.14 |
| 909 | CE1 | TYR | A | 781 | 10.184 | 38.887 | -6.071 | 1.00 | 50.46 |
| 910 | CE2 | TYR | A | 781 | 8.778 | 37.756 | -7.644 | 1.00 | 46.86 |
| 911 | CZ | TYR | A | 781 | 10.051 | 38.105 | -7.190 | 1.00 | 51.34 |
| 912 | OH | TYR | A | 781 | 11.207 | 37.679 | -7.825 | 1.00 | 55.86 |
| 913 | N | SER | A | 782 | 7.419 | 40.009 | -1.986 | 1.00 | 39.72 |
| 914 | CA | SER | A | 782 | 8.492 | 40.346 | -1.009 | 1.00 | 46.33 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 915 | C | SER | A | 782 | 8.597 | 39.361 | 0.159 | 1.00 | 38.58 |
| 916 | O | SER | A | 782 | 9.697 | 39.007 | 0.604 | 1.00 | 34.19 |
| 917 | CB | SER | A | 782 | 8.364 | 41.780 | -0.472 | 1.00 | 44.39 |
| 918 | OG | SER | A | 782 | 7.042 | 42.049 | -0.044 | 1.00 | 52.83 |
| 919 | N | GLN | A | 783 | 7.453 | 38.904 | 0.650 | 1.00 | 36.42 |
| 920 | CA | GLN | A | 783 | 7.486 | 37.948 | 1.736 | 1.00 | 43.09 |
| 921 | C | GLN | A | 783 | 8.040 | 36.661 | 1.160 | 1.00 | 41.26 |
| 922 | O | GLN | A | 783 | 8.980 | 36.075 | 1.709 | 1.00 | 47.55 |
| 923 | CB | GLN | A | 783 | 6.102 | 37.717 | 2.321 | 1.00 | 42.73 |
| 924 | CG | GLN | A | 783 | 5.538 | 38.915 | 3.059 | 1.00 | 48.39 |
| 925 | CD | GLN | A | 783 | 5.113 | 40.045 | 2.137 | 1.00 | 50.96 |
| 926 | OE1 | GLN | A | 783 | 4.504 | 39.831 | 1.083 | 1.00 | 53.94 |
| 927 | NE2 | GLN | A | 783 | 5.430 | 41.260 | 2.538 | 1.00 | 52.89 |
| 928 | N | CYS | A | 784 | 7.532 | 36.300 | -0.015 | 1.00 | 36.76 |
| 929 | CA | CYS | A | 784 | 7.945 | 35.084 | -0.710 | 1.00 | 35.38 |
| 930 | C | CYS | A | 784 | 9.456 | 35.006 | -0.859 | 1.00 | 33.10 |
| 931 | O | CYS | A | 784 | 10.073 | 33.978 | -0.568 | 1.00 | 39.32 |
| 932 | CB | CYS | A | 784 | 7.244 | 34.989 | -2.066 | 1.00 | 31.26 |
| 933 | SG | CYS | A | 784 | 5.513 | 34.425 | -1.949 | 1.00 | 42.23 |
| 934 | N | VAL | A | 785 | 10.060 | 36.132 | -1.199 | 1.00 | 33.87 |
| 935 | CA | VAL | A | 785 | 11.491 | 36.173 | -1.363 | 1.00 | 31.80 |
| 936 | C | VAL | A | 785 | 12.149 | 35.937 | -0.019 | 1.00 | 33.12 |
| 937 | O | VAL | A | 785 | 13.164 | 35.267 | 0.051 | 1.00 | 35.91 |
| 938 | CB | VAL | A | 785 | 11.928 | 37.502 | -1.962 | 1.00 | 37.92 |
| 939 | CG1 | VAL | A | 785 | 13.417 | 37.484 | -2.236 | 1.00 | 35.87 |
| 940 | CG2 | VAL | A | 785 | 11.171 | 37.748 | -3.264 | 1.00 | 33.21 |
| 941 | N | ARG | A | 786 | 11.556 | 36.461 | 1.052 | 1.00 | 36.61 |
| 942 | CA | ARG | A | 786 | 12.091 | 36.278 | 2.406 | 1.00 | 38.66 |
| 943 | C | ARG | A | 786 | 12.037 | 34.801 | 2.831 | 1.00 | 36.30 |
| 944 | O | ARG | A | 786 | 13.008 | 34.249 | 3.362 | 1.00 | 38.29 |
| 945 | CB | ARG | A | 786 | 11.343 | 37.175 | 3.407 | 1.00 | 44.04 |
| 946 | CG | ARG | A | 786 | 11.732 | 38.650 | 3.320 | 1.00 | 47.85 |
| 947 | CD | ARG | A | 786 | 12.880 | 38.968 | 4.255 | 1.00 | 50.22 |
| 948 | NE | ARG | A | 786 | 12.397 | 39.082 | 5.635 | 1.00 | 62.62 |
| 949 | CZ | ARG | A | 786 | 13.172 | 39.167 | 6.719 | 1.00 | 58.03 |
| 950 | NH1 | ARG | A | 786 | 14.494 | 39.134 | 6.609 | 1.00 | 61.23 |
| 951 | NH2 | ARG | A | 786 | 12.621 | 39.385 | 7.908 | 1.00 | 54.77 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 952 | N | MET | A | 787 | 10.919 | 34.150 | 2.553 | 1.00 | 32.73 |
| 953 | CA | MET | A | 787 | 10.763 | 32.746 | 2.883 | 1.00 | 34.16 |
| 954 | C | MET | A | 787 | 11.733 | 31.878 | 2.066 | 1.00 | 38.91 |
| 955 | O | MET | A | 787 | 12.229 | 30.846 | 2.539 | 1.00 | 43.10 |
| 956 | CB | MET | A | 787 | 9.331 | 32.315 | 2.621 | 1.00 | 27.15 |
| 957 | CG | MET | A | 787 | 8.337 | 33.046 | 3.449 | 1.00 | 32.77 |
| 958 | SD | MET | A | 787 | 6.714 | 32.334 | 3.317 | 1.00 | 46.02 |
| 959 | CE | MET | A | 787 | 6.063 | 33.236 | 2.001 | 1.00 | 31.94 |
| 960 | N | ARG | A | 788 | 11.999 | 32.296 | 0.835 | 1.00 | 39.06 |
| 961 | CA | ARG | A | 788 | 12.902 | 31.570 | -0.035 | 1.00 | 34.24 |
| 962 | C | ARG | A | 788 | 14.323 | 31.664 | 0.502 | 1.00 | 34.59 |
| 963 | O | ARG | A | 788 | 15.119 | 30.742 | 0.332 | 1.00 | 37.27 |
| 964 | CB | ARG | A | 788 | 12.790 | 32.122 | -1.446 | 1.00 | 39.81 |
| 965 | CG | ARG | A | 788 | 13.263 | 31.196 | -2.528 | 1.00 | 45.01 |
| 966 | CD | ARG | A | 788 | 12.846 | 31.727 | -3.887 | 1.00 | 54.21 |
| 967 | NE | ARG | A | 788 | 13.605 | 32.913 | -4.290 | 1.00 | 63.42 |
| 968 | CZ | ARG | A | 788 | 13.340 | 33.620 | -5.386 | 1.00 | 68.54 |
| 969 | NH1 | ARG | A | 788 | 12.329 | 33.261 | -6.163 | 1.00 | 72.57 |
| 970 | NH2 | ARG | A | 788 | 14.126 | 34.630 | -5.752 | 1.00 | 72.25 |
| 971 | N | HIS | A | 789 | 14.636 | 32.759 | 1.191 | 1.00 | 40.92 |
| 972 | CA | HIS | A | 789 | 15.973 | 32.916 | 1.784 | 1.00 | 53.01 |
| 973 | C | HIS | A | 789 | 16.107 | 31.777 | 2.782 | 1.00 | 54.43 |
| 974 | O | HIS | A | 789 | 17.066 | 31.000 | 2.730 | 1.00 | 50.35 |
| 975 | CB | HIS | A | 789 | 16.114 | 34.232 | 2.575 | 1.00 | 64.44 |
| 976 | CG | HIS | A | 789 | 16.334 | 35.456 | 1.739 | 1.00 | 74.91 |
| 977 | ND1 | HIS | A | 789 | 16.916 | 35.419 | 0.490 | 1.00 | 80.32 |
| 978 | CD2 | HIS | A | 789 | 16.051 | 36.759 | 1.982 | 1.00 | 74.30 |
| 979 | CE1 | HIS | A | 789 | 16.982 | 36.648 | -0.001 | 1.00 | 78.33 |
| 980 | NE2 | HIS | A | 789 | 16.465 | 37.481 | 0.886 | 1.00 | 74.90 |
| 981 | N | LEU | A | 790 | 15.112 | 31.700 | 3.674 | 1.00 | 56.83 |
| 982 | CA | LEU | A | 790 | 15.018 | 30.684 | 4.731 | 1.00 | 50.54 |
| 983 | C | LEU | A | 790 | 15.194 | 29.297 | 4.140 | 1.00 | 45.95 |
| 984 | O | LEU | A | 790 | 16.076 | 28.551 | 4.573 | 1.00 | 43.16 |
| 985 | CB | LEU | A | 790 | 13.660 | 30.757 | 5.427 | 1.00 | 47.65 |
| 986 | CG | LEU | A | 790 | 13.455 | 29.853 | 6.634 | 1.00 | 49.09 |
| 987 | CD1 | LEU | A | 790 | 14.230 | 30.415 | 7.825 | 1.00 | 56.25 |
| 988 | CD2 | LEU | A | 790 | 11.970 | 29.760 | 6.962 | 1.00 | 46.26 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 989 | N | SER | A | 791 | 14.388 | 28.963 | 3.130 | 1.00 | 38.77 |
| 990 | CA | SER | A | 791 | 14.516 | 27.661 | 2.501 | 1.00 | 35.86 |
| 991 | C | SER | A | 791 | 15.951 | 27.507 | 2.010 | 1.00 | 37.61 |
| 992 | O | SER | A | 791 | 16.533 | 26.432 | 2.127 | 1.00 | 42.51 |
| 993 | CB | SER | A | 791 | 13.515 | 27.479 | 1.363 | 1.00 | 39.07 |
| 994 | OG | SER | A | 791 | 13.974 | 28.090 | 0.177 | 1.00 | 55.38 |
| 995 | N | GLN | A | 792 | 16.563 | 28.585 | 1.541 | 1.00 | 38.43 |
| 996 | CA | GLN | A | 792 | 17.938 | 28.481 | 1.086 | 1.00 | 43.65 |
| 997 | C | GLN | A | 792 | 18.978 | 28.304 | 2.201 | 1.00 | 46.43 |
| 998 | O | GLN | A | 792 | 20.049 | 27.761 | 1.951 | 1.00 | 53.03 |
| 999 | CB | GLN | A | 792 | 18.297 | 29.652 | 0.201 | 1.00 | 43.96 |
| 1000 | CG | GLN | A | 792 | 17.455 | 29.723 | -1.021 | 1.00 | 50.40 |
| 1001 | CD | GLN | A | 792 | 17.516 | 31.082 | -1.648 | 1.00 | 56.80 |
| 1002 | OE1 | GLN | A | 792 | 17.275 | 32.081 | -0.975 | 1.00 | 59.46 |
| 1003 | NE2 | GLN | A | 792 | 17.852 | 31.142 | -2.938 | 1.00 | 56.24 |
| 1004 | N | GLU | A | 793 | 18.684 | 28.734 | 3.424 | 1.00 | 46.80 |
| 1005 | CA | GLU | A | 793 | 19.658 | 28.561 | 4.510 | 1.00 | 46.06 |
| 1006 | C | GLU | A | 793 | 19.852 | 27.078 | 4.787 | 1.00 | 40.74 |
| 1007 | O | GLU | A | 793 | 20.972 | 26.632 | 5.000 | 1.00 | 41.28 |
| 1008 | CB | GLU | A | 793 | 19.214 | 29.248 | 5.814 | 1.00 | 51.06 |
| 1009 | CG | GLU | A | 793 | 18.949 | 30.772 | 5.734 | 1.00 | 67.15 |
| 1010 | CD | GLU | A | 793 | 20.212 | 31.644 | 5.762 | 1.00 | 74.35 |
| 1011 | OE1 | GLU | A | 793 | 21.328 | 31.098 | 5.703 | 1.00 | 72.15 |
| 1012 | OE2 | GLU | A | 793 | 20.083 | 32.894 | 5.837 | 1.00 | 83.11 |
| 1013 | N | PHE | A | 794 | 18.771 | 26.302 | 4.760 | 1.00 | 38.60 |
| 1014 | CA | PHE | A | 794 | 18.862 | 24.866 | 5.031 | 1.00 | 36.65 |
| 1015 | C | PHE | A | 794 | 19.960 | 24.277 | 4.206 | 1.00 | 40.69 |
| 1016 | O | PHE | A | 794 | 20.761 | 23.478 | 4.690 | 1.00 | 41.76 |
| 1017 | CB | PHE | A | 794 | 17.562 | 24.160 | 4.686 | 1.00 | 32.29 |
| 1018 | CG | PHE | A | 794 | 16.462 | 24.410 | 5.669 | 1.00 | 37.63 |
| 1019 | CD1 | PHE | A | 794 | 16.704 | 24.358 | 7.030 | 1.00 | 37.22 |
| 1020 | CD2 | PHE | A | 794 | 15.173 | 24.718 | 5.229 | 1.00 | 43.04 |
| 1021 | CE1 | PHE | A | 794 | 15.665 | 24.605 | 7.945 | 1.00 | 35.06 |
| 1022 | CE2 | PHE | A | 794 | 14.126 | 24.969 | 6.140 | 1.00 | 37.46 |
| 1023 | CZ | PHE | A | 794 | 14.376 | 24.913 | 7.494 | 1.00 | 27.46 |
| 1024 | N | GLY | A | 795 | 20.018 | 24.729 | 2.963 | 1.00 | 51.69 |
| 1025 | CA | GLY | A | 795 | 21.033 | 24.260 | 2.040 | 1.00 | 57.61 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1026 | C | GLY | A | 795 | 22.440 | 24.695 | 2.391 | 1.00 | 58.51 |
| 1027 | O | GLY | A | 795 | 23.338 | 23.850 | 2.481 | 1.00 | 54.63 |
| 1028 | N | TRP | A | 796 | 22.625 | 25.994 | 2.624 | 1.00 | 56.85 |
| 1029 | CA | TRP | A | 796 | 23.942 | 26.504 | 2.944 | 1.00 | 61.47 |
| 1030 | C | TRP | A | 796 | 24.433 | 25.986 | 4.301 | 1.00 | 59.21 |
| 1031 | O | TRP | A | 796 | 25.614 | 25.649 | 4.469 | 1.00 | 55.17 |
| 1032 | CB | TRP | A | 796 | 23.966 | 28.039 | 2.770 | 1.00 | 77.04 |
| 1033 | CG | TRP | A | 796 | 24.441 | 28.924 | 3.938 | 1.00 | 99.05 |
| 1034 | CD1 | TRP | A | 796 | 23.779 | 30.018 | 4.436 | 1.00 | 103.16 |
| 1035 | CD2 | TRP | A | 796 | 25.693 | 28.847 | 4.686 | 1.00 | 107.94 |
| 1036 | NE1 | TRP | A | 796 | 24.528 | 30.622 | 5.430 | 1.00 | 107.43 |
| 1037 | CE2 | TRP | A | 796 | 25.701 | 29.929 | 5.606 | 1.00 | 109.90 |
| 1038 | CE3 | TRP | A | 796 | 26.801 | 27.976 | 4.667 | 1.00 | 108.72 |
| 1039 | CZ2 | TRP | A | 796 | 26.778 | 30.157 | 6.503 | 1.00 | 110.69 |
| 1040 | CZ3 | TRP | A | 796 | 27.871 | 28.204 | 5.561 | 1.00 | 110.67 |
| 1041 | CH2 | TRP | A | 796 | 27.845 | 29.286 | 6.462 | 1.00 | 111.09 |
| 1042 | N | LEU | A | 797 | 23.500 | 25.779 | 5.221 | 1.00 | 56.75 |
| 1043 | CA | LEU | A | 797 | 23.851 | 25.286 | 6.546 | 1.00 | 54.04 |
| 1044 | C | LEU | A | 797 | 24.012 | 23.767 | 6.640 | 1.00 | 56.56 |
| 1045 | O | LEU | A | 797 | 24.629 | 23.277 | 7.587 | 1.00 | 62.03 |
| 1046 | CB | LEU | A | 797 | 22.839 | 25.782 | 7.583 | 1.00 | 52.49 |
| 1047 | CG | LEU | A | 797 | 23.080 | 27.184 | 8.151 | 1.00 | 42.33 |
| 1048 | CD1 | LEU | A | 797 | 21.835 | 27.737 | 8.827 | 1.00 | 39.02 |
| 1049 | CD2 | LEU | A | 797 | 24.229 | 27.123 | 9.128 | 1.00 | 43.89 |
| 1050 | N | GLN | A | 798 | 23.462 | 23.020 | 5.684 | 1.00 | 53.42 |
| 1051 | CA | GLN | A | 798 | 23.560 | 21.561 | 5.708 | 1.00 | 57.87 |
| 1052 | C | GLN | A | 798 | 22.788 | 20.960 | 6.890 | 1.00 | 54.98 |
| 1053 | O | GLN | A | 798 | 23.268 | 20.039 | 7.571 | 1.00 | 53.85 |
| 1054 | CB | GLN | A | 798 | 25.028 | 21.103 | 5.774 | 1.00 | 67.87 |
| 1055 | CG | GLN | A | 798 | 25.773 | 21.088 | 4.438 | 1.00 | 83.30 |
| 1056 | CD | GLN | A | 798 | 27.115 | 20.345 | 4.507 | 1.00 | 91.19 |
| 1057 | OE1 | GLN | A | 798 | 27.910 | 20.564 | 5.428 | 1.00 | 96.98 |
| 1058 | NE2 | GLN | A | 798 | 27.368 | 19.463 | 3.532 | 1.00 | 87.09 |
| 1059 | N | ILE | A | 799 | 21.597 | 21.494 | 7.131 | 1.00 | 49.17 |
| 1060 | CA | ILE | A | 799 | 20.728 | 21.040 | 8.219 | 1.00 | 45.76 |
| 1061 | C | ILE | A | 799 | 20.348 | 19.545 | 8.071 | 1.00 | 47.10 |
| 1062 | O | ILE | A | 799 | 19.766 | 19.135 | 7.059 | 1.00 | 40.12 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1063 | CB | ILE | A | 799 | 19.414 | 21.894 | 8.258 | 1.00 | 38.57 |
| 1064 | CG1 | ILE | A | 799 | 19.733 | 23.388 | 8.383 | 1.00 | 36.16 |
| 1065 | CG2 | ILE | A | 799 | 18.532 | 21.450 | 9.379 | 1.00 | 36.81 |
| 1066 | CD1 | ILE | A | 799 | 20.686 | 23.691 | 9.480 | 1.00 | 27.19 |
| 1067 | N | THR | A | 800 | 20.735 | 18.725 | 9.045 | 1.00 | 46.19 |
| 1068 | CA | THR | A | 800 | 20.386 | 17.310 | 9.026 | 1.00 | 47.55 |
| 1069 | C | THR | A | 800 | 18.883 | 17.205 | 9.354 | 1.00 | 48.95 |
| 1070 | O | THR | A | 800 | 18.345 | 18.013 | 10.125 | 1.00 | 55.00 |
| 1071 | CB | THR | A | 800 | 21.222 | 16.474 | 10.060 | 1.00 | 47.29 |
| 1072 | OG1 | THR | A | 800 | 20.773 | 16.729 | 11.396 | 1.00 | 53.15 |
| 1073 | CG2 | THR | A | 800 | 22.696 | 16.813 | 9.968 | 1.00 | 53.33 |
| 1074 | N | PRO | A | 801 | 18.170 | 16.238 | 8.740 | 1.00 | 47.30 |
| 1075 | CA | PRO | A | 801 | 16.733 | 16.068 | 9.000 | 1.00 | 42.31 |
| 1076 | C | PRO | A | 801 | 16.353 | 15.955 | 10.476 | 1.00 | 38.64 |
| 1077 | O | PRO | A | 801 | 15.193 | 16.130 | 10.835 | 1.00 | 40.23 |
| 1078 | CB | PRO | A | 801 | 16.404 | 14.799 | 8.221 | 1.00 | 47.59 |
| 1079 | CG | PRO | A | 801 | 17.285 | 14.951 | 7.007 | 1.00 | 46.66 |
| 1080 | CD | PRO | A | 801 | 18.603 | 15.381 | 7.615 | 1.00 | 45.27 |
| 1081 | N | GLN | A | 802 | 17.351 | 15.682 | 11.310 | 1.00 | 41.68 |
| 1082 | CA | GLN | A | 802 | 17.210 | 15.560 | 12.754 | 1.00 | 43.58 |
| 1083 | C | GLN | A | 802 | 17.275 | 16.959 | 13.403 | 1.00 | 48.44 |
| 1084 | O | GLN | A | 802 | 16.505 | 17.266 | 14.329 | 1.00 | 49.81 |
| 1085 | CB | GLN | A | 802 | 18.338 | 14.684 | 13.311 | 1.00 | 46.98 |
| 1086 | CG | GLN | A | 802 | 18.321 | 13.250 | 12.830 | 1.00 | 46.45 |
| 1087 | CD | GLN | A | 802 | 18.685 | 13.116 | 11.373 | 1.00 | 48.73 |
| 1088 | OE1 | GLN | A | 802 | 19.827 | 13.413 | 10.968 | 1.00 | 53.22 |
| 1089 | NE2 | GLN | A | 802 | 17.727 | 12.666 | 10.566 | 1.00 | 43.12 |
| 1090 | N | GLU | A | 803 | 18.218 | 17.790 | 12.950 | 1.00 | 41.48 |
| 1091 | CA | GLU | A | 803 | 18.322 | 19.142 | 13.483 | 1.00 | 32.56 |
| 1092 | C | GLU | A | 803 | 17.034 | 19.841 | 13.133 | 1.00 | 34.21 |
| 1093 | O | GLU | A | 803 | 16.456 | 20.543 | 13.951 | 1.00 | 39.30 |
| 1094 | CB | GLU | A | 803 | 19.485 | 19.869 | 12.879 | 1.00 | 27.56 |
| 1095 | CG | GLU | A | 803 | 20.796 | 19.325 | 13.342 | 1.00 | 33.55 |
| 1096 | CD | GLU | A | 803 | 21.903 | 19.778 | 12.436 | 1.00 | 40.34 |
| 1097 | OE1 | GLU | A | 803 | 21.618 | 20.023 | 11.249 | 1.00 | 39.58 |
| 1098 | OE2 | GLU | A | 803 | 23.057 | 19.915 | 12.882 | 1.00 | 46.91 |
| 1099 | N | PHE | A | 804 | 16.541 | 19.584 | 11.932 | 1.00 | 32.10 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1100 | CA | PHE | A | 804 | 15.286 | 20.166 | 11.519 | 1.00 | 26.58 |
| 1101 | C | PHE | A | 804 | 14.157 | 19.876 | 12.497 | 1.00 | 29.08 |
| 1102 | O | PHE | A | 804 | 13.528 | 20.793 | 13.024 | 1.00 | 38.23 |
| 1103 | CB | PHE | A | 804 | 14.872 | 19.663 | 10.142 | 1.00 | 24.46 |
| 1104 | CG | PHE | A | 804 | 13.445 | 20.032 | 9.767 | 1.00 | 35.13 |
| 1105 | CD1 | PHE | A | 804 | 13.091 | 21.361 | 9.540 | 1.00 | 36.25 |
| 1106 | CD2 | PHE | A | 804 | 12.468 | 19.048 | 9.617 | 1.00 | 38.14 |
| 1107 | CE1 | PHE | A | 804 | 11.795 | 21.712 | 9.164 | 1.00 | 27.83 |
| 1108 | CE2 | PHE | A | 804 | 11.163 | 19.385 | 9.238 | 1.00 | 39.32 |
| 1109 | CZ | PHE | A | 804 | 10.826 | 20.723 | 9.011 | 1.00 | 38.61 |
| 1110 | N | LEU | A | 805 | 13.887 | 18.600 | 12.728 | 1.00 | 30.73 |
| 1111 1 | CA | LEU | A | 805 | 12.784 | 18.215 | 13.586 | 1.00 | 28.43 |
| 1112 | C | LEU | A | 805 | 12.880 | 18.799 | 14.946 | 1.00 | 26.10 |
| 1113 | O | LEU | A | 805 | 11.881 | 19.243 | 15.493 | 1.00 | 34.14 |
| 1114 | CB | LEU | A | 805 | 12.648 | 16.702 | 13.661 | 1.00 | 34.10 |
| 1115 | CG | LEU | A | 805 | 12.000 | 16.079 | 12.423 | 1.00 | 43.20 |
| 1116 | CD1 | LEU | A | 805 | 12.046 | 14.617 | 12.600 | 1.00 | 36.94 |
| 1117 | CD2 | LEU | A | 805 | 10.549 | 16.523 | 12.252 | 1.00 | 44.97 |
| 1118 | N | CYS | A | 806 | 14.082 | 18.836 | 15.493 | 1.00 | 27.20 |
| 1119 | CA | CYS | A | 806 | 14.250 | 19.396 | 16.831 | 1.00 | 39.83 |
| 1120 | C | CYS | A | 806 | 13.904 | 20.894 | 16.843 | 1.00 | 41.37 |
| 1121 | O | CYS | A | 806 | 13.145 | 21.367 | 17.704 | 1.00 | 38.83 |
| 1122 | CB | CYS | A | 806 | 15.669 | 19.143 | 17.341 | 1.00 | 43.31 |
| 1123 | SG | CYS | A | 806 | 16.021 | 17.398 | 17.526 | 0.50 | 37.47 |
| 1124 | N | MET | A | 807 | 14.420 | 21.610 | 15.846 | 1.00 | 38.56 |
| 1125 | CA | MET | A | 807 | 14.161 | 23.027 | 15.692 | 1.00 | 33.72 |
| 1126 | C | MET | A | 807 | 12.662 | 23.301 | 15.488 | 1.00 | 31.70 |
| 1127 | O | MET | A | 807 | 12.093 | 24.168 | 16.148 | 1.00 | 37.83 |
| 1128 | CB | MET | A | 807 | 14.973 | 23.589 | 14.519 | 1.00 | 37.50 |
| 1129 | CG | MET | A | 807 | 16.474 | 23.539 | 14.740 | 1.00 | 38.94 |
| 1130 | SD | MET | A | 807 | 17.486 | 24.563 | 13.596 | 1.00 | 42.84 |
| 1131 | CE | MET | A | 807 | 17.165 | 23.799 | 12.065 | 1.00 | 49.58 |
| 1132 | N | LYS | A | 808 | 12.006 | 22.545 | 14.617 | 1.00 | 27.59 |
| 1133 | CA | LYS | A | 808 | 10.597 | 22.787 | 14.378 | 1.00 | 27.15 |
| 1134 | C | LYS | A | 808 | 9.841 | 22.681 | 15.686 | 1.00 | 29.83 |
| 1135 | O | LYS | A | 808 | 8.954 | 23.486 | 15.952 | 1.00 | 35.67 |
| 1136 | CB | LYS | A | 808 | 10.029 | 21.837 | 13.313 | 1.00 | 24.49 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1137 | CG | LYS | A | 808 | 8.598 | 22.158 | 12.811 1 | 1.00 | 23.62 |
| 1138 | CD | LYS | A | 808 | 8.148 | 21.143 | 11.740 | 1.00 | 24.64 |
| 1139 | CE | LYS | A | 808 | 7.157 | 20.101 | 12.280 | 1.00 | 29.39 |
| 1140 | NZ | LYS | A | 808 | 5.683 | 20.529 | 12.198 | 1.00 | 36.92 |
| 1141 | N | ALA | A | 809 | 10.220 | 21.727 | 16.530 | 1.00 | 32.53 |
| 1142 | CA | ALA | A | 809 | 9.553 | 21.557 | 17.826 | 1.00 | 38.51 |
| 1143 | C | ALA | A | 809 | 9.786 | 22.766 | 18.749 | 1.00 | 43.27 |
| 1144 | O | ALA | A | 809 | 8.844 | 23.305 | 19.346 | 1.00 | 43.55 |
| 1145 | CB | ALA | A | 809 | 10.029 | 20.295 | 18.501 | 1.00 | 25.72 |
| 1146 | N | LEU | A | 810 | 11.042 | 23.193 | 18.836 | 1.00 | 41.18 |
| 1147 | CA | LEU | A | 810 | 11.460 | 24.340 | 19.651 | 1.00 | 40.05 |
| 1148 | C | LEU | A | 810 | 10.732 | 25.626 | 19.269 | 1.00 | 38.85 |
| 1149 | O | LEU | A | 810 | 10.445 | 26.460 | 20.134 | 1.00 | 38.70 |
| 1150 | CB | LEU | A | 810 | 12.961 | 24.534 | 19.502 | 1.00 | 39.64 |
| 1151 | CG | LEU | A | 810 | 13.779 | 25.313 | 20.524 | 1.00 | 43.39 |
| 1152 | CD1 | LEU | A | 810 | 13.361 | 24.959 | 21.942 | 1.00 | 41.58 |
| 1153 | CD2 | LEU | A | 810 | 15.256 | 24.981 | 20.284 | 1.00 | 34.95 |
| 1154 | N | LEU | A | 811 | 10.409 | 25.763 | 17.982 | 1.00 | 31.82 |
| 1155 | CA | LEU | A | 811 | 9.683 | 26.923 | 17.489 | 1.00 | 34.47 |
| 1156 | C | LEU | A | 811 | 8.359 | 27.093 | 18.200 | 1.00 | 35.94 |
| 1157 | O | LEU | A | 811 | 7.918 | 28.219 | 18.435 | 1.00 | 47.05 |
| 1158 | CB | LEU | A | 811 | 9.384 | 26.811 | 15.988 | 1.00 | 33.79 |
| 1159 | CG | LEU | A | 811 | 10.309 | 27.474 | 14.973 | 1.00 | 28.95 |
| 1160 | CD1 | LEU | A | 811 | 9.523 | 27.595 | 13.709 | 1.00 | 30.30 |
| 1161 | CD2 | LEU | A | 811 | 10.767 | 28.875 | 15.444 | 1.00 | 20.35 |
| 1162 | N | LEU | A | 812 | 7.713 | 25.978 | 18.527 | 1.00 | 31.68 |
| 1163 | CA | LEU | A | 812 | 6.418 | 26.032 | 19.190 | 1.00 | 30.90 |
| 1164 | C | LEU | A | 812 | 6.459 | 26.850 | 20.469 | 1.00 | 29.57 |
| 1165 | O | LEU | A | 812 | 5.476 | 27.486 | 20.836 | 1.00 | 35.81 |
| 1166 | CB | LEU | A | 812 | 5.934 | 24.622 | 19.511 | 1.00 | 29.23 |
| 1167 | CG | LEU | A | 812 | 4.600 | 24.544 | 20.281 | 1.00 | 34.64 |
| 1168 | CD1 | LEU | A | 812 | 3.428 | 25.002 | 19.417 | 1.00 | 30.86 |
| 1169 | CD2 | LEU | A | 812 | 4.355 | 23.144 | 20.738 | 1.00 | 31.58 |
| 1170 | N | PHE | A | 813 | 7.604 | 26.788 | 21.144 | 1.00 | 32.36 |
| 1171 | CA | PHE | A | 813 | 7.862 | 27.480 | 22.405 | 1.00 | 36.84 |
| 1172 | C | PHE | A | 813 | 8.709 | 28.693 | 22.180 | 1.00 | 36.98 |
| 1173 | O | PHE | A | 813 | 9.584 | 28.983 | 22.996 | 1.00 | 34.78 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1174 | CB | PHE | A | 813 | 8.669 | 26.585 | 23.335 | 1.00 | 39.81 |
| 1175 | CG | PHE | A | 813 | 8.119 | 25.242 | 23.454 | 1.00 | 42.06 |
| 1176 | CD1 | PHE | A | 813 | 6.837 | 25.057 | 23.960 | 1.00 | 40.53 |
| 1177 | CD2 | PHE | A | 813 | 8.826 | 24.156 | 22.984 | 1.00 | 43.44 |
| 1178 | CE1 | PHE | A | 813 | 6.255 | 23.794 | 23.990 | 1.00 | 49.40 |
| 1179 | CE2 | PHE | A | 813 | 8.259 | 22.884 | 23.006 | 1.00 | 48.76 |
| 1180 | CZ | PHE | A | 813 | 6.966 | 22.698 | 23.510 | 1.00 | 49.26 |
| 1181 | N | SER | A | 814 | 8.488 | 29.380 | 21.071 | 1.00 | 39.85 |
| 1182 | CA | SER | A | 814 | 9.284 | 30.547 | 20.748 | 1.00 | 40.33 |
| 1183 | C | SER | A | 814 | 8.531 | 31.858 | 20.778 | 1.00 | 34.59 |
| 1184 | O | SER | A | 814 | 9.125 | 32.897 | 20.612 | 1.00 | 39.81 |
| 1185 | CB | SER | A | 814 | 9.938 | 30.364 | 19.374 | 1.00 | 45.33 |
| 1186 | OG | SER | A | 814 | 11.096 | 29.544 | 19.469 | 1.00 | 51.73 |
| 1187 | N | ILE | A | 815 | 7.242 | 31.838 | 21.040 | 1.00 | 38.17 |
| 1188 | CA | ILE | A | 815 | 6.508 | 33.083 | 21.033 | 1.00 | 46.03 |
| 1189 | C | ILE | A | 815 | 5.456 | 33.076 | 22.149 | 1.00 | 46.20 |
| 1190 | O | ILE | A | 815 | 4.526 | 32.260 | 22.112 | 1.00 | 45.64 |
| 1191 | CB | ILE | A | 815 | 5.937 | 33.329 | 19.597 | 1.00 | 49.44 |
| 1192 | CG1 | ILE | A | 815 | 4.943 | 34.477 | 19.593 | 1.00 | 50.62 |
| 1193 | CG2 | ILE | A | 815 | 5.401 | 32.041 | 18.971 | 1.00 | 50.44 |
| 1194 | CD1 | ILE | A | 815 | 5.406 | 35.592 | 18.748 | 1.00 | 55.65 |
| 1195 | N | ILE | A | 816 | 5.645 | 33.950 | 23.152 | 1.00 | 47.67 |
| 1196 | CA | ILE | A | 816 | 4.762 | 34.056 | 24.344 | 1.00 | 46.12 |
| 1197 | C | ILE | A | 816 | 4.302 | 35.482 | 24.731 | 1.00 | 42.20 |
| 1198 | O | ILE | A | 816 | 5.002 | 36.460 | 24.436 | 1.00 | 43.23 |
| 1199 | CB | ILE | A | 816 | 5.446 | 33.417 | 25.609 | 1.00 | 50.07 |
| 1200 | CG1 | ILE | A | 816 | 6.701 | 34.194 | 26.018 | 1.00 | 49.09 |
| 1201 | CG2 | ILE | A | 816 | 5.819 | 31.970 | 25.326 | 1.00 | 52.85 |
| 1202 | CD1 | ILE | A | 816 | 7.442 | 33.578 | 27.204 | 1.00 | 37.43 |
| 1203 | N | PRO | A | 817 | 3.123 | 35.618 | 25.402 | 1.00 | 37.07 |
| 1204 | CA | PRO | A | 817 | 2.619 | 36.933 | 25.811 | 1.00 | 40.94 |
| 1205 | C | PRO | A | 817 | 3.702 | 37.599 | 26.659 | 1.00 | 49.75 |
| 1206 | O | PRO | A | 817 | 4.595 | 36.891 | 27.163 | 1.00 | 47.60 |
| 1207 | CB | PRO | A | 817 | 1.396 | 36.581 | 26.662 | 1.00 | 27.66 |
| 1208 | CG | PRO | A | 817 | 0.902 | 35.368 | 26.063 | 1.00 | 28.99 |
| 1209 | CD | PRO | A | 817 | 2.186 | 34.579 | 25.855 | 1.00 | 33.43 |
| 1210 | N | VAL | A | 818 | 3.663 | 38.933 | 26.798 | 1.00 | 57.41 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1211 | CA | VAL | A | 818 | 4.690 | 39.631 | 27.592 | 1.00 | 58.97 |
| 1212 | C | VAL | A | 818 | 4.728 | 39.410 | 29.124 | 1.00 | 59.31 |
| 1213 | O | VAL | A | 818 | 5.801 | 39.574 | 29.720 | 1.00 | 63.79 |
| 1214 | CB | VAL | A | 818 | 4.893 | 41.127 | 27.206 | 1.00 | 54.46 |
| 1215 | CG1 | VAL | A | 818 | 5.738 | 41.211 | 25.950 | 1.00 | 49.94 |
| 1216 | CG2 | VAL | A | 818 | 3.575 | 41.824 | 27.009 | 1.00 | 53.66 |
| 1217 | N | ASP | A | 819 | 3.607 | 39.061 | 29.769 | 1.00 | 56.35 |
| 1218 | CA | ASP | A | 819 | 3.654 | 38.741 | 31.212 | 1.00 | 61.78 |
| 1219 | C | ASP | A | 819 | 4.167 | 37.302 | 31.212 | 1.00 | 63.93 |
| 1220 | O | ASP | A | 819 | 5.322 | 36.998 | 31.566 | 1.00 | 69.29 |
| 1221 | CB | ASP | A | 819 | 2.270 | 38.746 | 31.869 | 1.00 | 60.71 |
| 1222 | CG | ASP | A | 819 | 1.174 | 39.031 | 30.888 | 1.00 | 71.79 |
| 1223 | OD1 | ASP | A | 819 | 0.947 | 40.218 | 30.600 | 1.00 | 78.23 |
| 1224 | OD2 | ASP | A | 819 | 0.577 | 38.069 | 30.373 | 1.00 | 78.87 |
| 1225 | N | GLY | A | 820 | 3.296 | 36.419 | 30.758 | 1.00 | 57.92 |
| 1226 | CA | GLY | A | 820 | 3.652 | 35.034 | 30.672 | 1.00 | 46.58 |
| 1227 | C | GLY | A | 820 | 2.369 | 34.304 | 30.440 | 1.00 | 45.74 |
| 1228 | O | GLY | A | 820 | 1.293 | 34.894 | 30.200 | 1.00 | 40.14 |
| 1229 | N | LEU | A | 821 | 2.497 | 32.995 | 30.500 | 1.00 | 46.98 |
| 1230 | CA | LEU | A | 821 | 1.378 | 32.105 | 30.327 | 1.00 | 47.93 |
| 1231 | C | LEU | A | 821 | 0.787 | 31.902 | 31.733 | 1.00 | 47.05 |
| 1232 | O | LEU | A | 821 | 1.456 | 32.188 | 32.737 | 1.00 | 45.88 |
| 1233 | CB | LEU | A | 821 | 1.905 | 30.808 | 29.708 | 1.00 | 54.39 |
| 1234 | CG | LEU | A | 821 | 2.560 | 31.017 | 28.336 | 1.00 | 50.67 |
| 1235 | CD1 | LEU | A | 821 | 3.858 | 30.259 | 28.235 | 1.00 | 43.32 |
| 1236 | CD2 | LEU | A | 821 | 1.602 | 30.622 | 27.240 | 1.00 | 46.90 |
| 1237 | N | LYS | A | 822 | -0.437 | 31.393 | 31.820 | 1.00 | 48.17 |
| 1238 | CA | LYS | A | 822 | -1.103 | 31.181 | 33.105 | 1.00 | 52.88 |
| 1239 | C | LYS | A | 822 | -0.449 | 30.065 | 33.935 | 1.00 | 59.84 |
| 1240 | O | LYS | A | 822 | -1.111 | 29.361 | 34.709 | 1.00 | 62.81 |
| 1241 | CB | LYS | A | 822 | -2.609 | 30.951 | 32.880 | 1.00 | 56.87 |
| 1242 | CG | LYS | A | 822 | -3.321 | 32.193 | 32.288 | 1.00 | 65.23 |
| 1243 | CD | LYS | A | 822 | -4.729 | 31.923 | 31.719 | 1.00 | 63.38 |
| 1244 | CE | LYS | A | 822 | -5.371 | 33.194 | 31.088 | 1.00 | 65.28 |
| 1245 | NZ | LYS | A | 822 | -4.534 | 33.875 | 30.032 | 1.00 | 59.84 |
| 1246 | N | ASN | A | 823 | 0.862 | 29.930 | 33.744 | 1.00 | 62.47 |
| 1247 | CA | ASN | A | 823 | 1.739 | 28.988 | 34.419 | 1.00 | 60.37 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1248 | C | ASN | A | 823 | 3.025 | 28.904 | 33.616 | 1.00 | 58.03 |
| 1249 | O | ASN | A | 823 | 3.364 | 27.879 | 33.017 | 1.00 | 50.60 |
| 1250 | CB | ASN | A | 823 | 1.138 | 27.611 | 34.544 | 1.00 | 65.57 |
| 1251 | CG | ASN | A | 823 | 2.094 | 26.649 | 35.178 | 1.00 | 75.73 |
| 1252 | OD1 | ASN | A | 823 | 3.101 | 27.051 | 35.811 | 1.00 | 68.88 |
| 1253 | ND2 | ASN | A | 823 | 1.830 | 25.367 | 34.986 | 1.00 | 81.03 |
| 1254 | N | GLN | A | 824 | 3.743 | 30.012 | 33.631 | 1.00 | 59.10 |
| 1255 | CA | GLN | A | 824 | 4.993 | 30.158 | 32.914 | 1.00 | 56.38 |
| 1256 | C | GLN | A | 824 | 6.069 | 29.145 | 33.329 | 1.00 | 54.52 |
| 1257 | O | GLN | A | 824 | 6.826 | 28.677 | 32.483 | 1.00 | 53.91 |
| 1258 | CB | GLN | A | 824 | 5.485 | 31.592 | 33.129 | 1.00 | 56.68 |
| 1259 | CG | GLN | A | 824 | 6.761 | 31.972 | 32.412 | 1.00 | 54.92 |
| 1260 | CD | GLN | A | 824 | 6.532 | 32.329 | 30.976 | 1.00 | 59.81 |
| 1261 | OE1 | GLN | A | 824 | 5.462 | 32.833 | 30.602 | 1.00 | 57.91 |
| 1262 | NE2 | GLN | A | 824 | 7.533 | 32.078 | 30.150 | 1.00 | 62.36 |
| 1263 | N | LYS | A | 825 | 6.099 | 28.777 | 34.613 | 1.00 | 55.18 |
| 1264 | CA | LYS | A | 825 | 7.098 | 27.841 | 35.160 | 1.00 | 53.31 |
| 1265 | C | LYS | A | 825 | 7.351 | 26.550 | 34.357 | 1.00 | 46.62 |
| 1266 | O | LYS | A | 825 | 8.497 | 26.236 | 34.015 | 1.00 | 40.85 |
| 1267 | CB | LYS | A | 825 | 6.769 | 27.515 | 36.626 | 1.00 | 57.76 |
| 1268 | CG | LYS | A | 825 | 7.128 | 28.634 | 37.617 | 1.00 | 64.19 |
| 1269 | CD | LYS | A | 825 | 6.432 | 28.463 | 38.979 | 1.00 | 77.45 |
| 1270 | CE | LYS | A | 825 | 7.122 | 29.254 | 40.125 | 1.00 | 86.97 |
| 1271 | NZ | LYS | A | 825 | 7.186 | 30.763 | 40.023 | 1.00 | 89.42 |
| 1272 | N | PHE | A | 826 | 6.285 | 25.811 | 34.053 | 1.00 | 46.35 |
| 1273 | CA | PHE | A | 826 | 6.399 | 24.562 | 33.288 | 1.00 | 47.42 |
| 1274 | C | PHE | A | 826 | 6.892 | 24.863 | 31.853 | 1.00 | 48.67 |
| 1275 | O | PHE | A | 826 | 7.847 | 24.243 | 31.395 | 1.00 | 48.05 |
| 1276 | CB | PHE | A | 826 | 5.063 | 23.774 | 33.273 | 1.00 | 43.87 |
| 1277 | CG | PHE | A | 826 | 4.588 | 23.295 | 34.653 | 1.00 | 56.48 |
| 1278 | CD1 | PHE | A | 826 | 5.443 | 23.288 | 35.770 | 1.00 | 62.15 |
| 1279 | CD2 | PHE | A | 826 | 3.273 | 22.832 | 34.831 | 1.00 | 60.89 |
| 1280 | CE1 | PHE | A | 826 | 4.994 | 22.827 | 37.040 | 1.00 | 53.25 |
| 1281 | CE2 | PHE | A | 826 | 2.813 | 22.367 | 36.097 | 1.00 | 53.93 |
| 1282 | CZ | PHE | A | 826 | 3.681 | 22.368 | 37.193 | 1.00 | 51.47 |
| 1283 | N | PHE | A | 827 | 6.272 | 25.835 | 31.168 | 1.00 | 44.70 |
| 1284 | CA | PHE | A | 827 | 6.670 | 26.234 | 29.800 | 1.00 | 40.53 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1285 | C | PHE | A | 827 | 8.181 | 26.414 | 29.746 | 1.00 | 37.37 |
| 1286 | O | PHE | A | 827 | 8.848 | 25.934 | 28.835 | 1.00 | 35.40 |
| 1287 | CB | PHE | A | 827 | 5.964 | 27.558 | 29.375 | 1.00 | 35.40 |
| 1288 | CG | PHE | A | 827 | 6.497 | 28.177 | 28.070 | 1.00 | 31.04 |
| 1289 | CD1 | PHE | A | 827 | 7.750 | 28.793 | 28.018 | 1.00 | 32.73 |
| 1290 | CD2 | PHE | A | 827 | 5.742 | 28.125 | 26.900 | 1.00 | 34.40 |
| 1291 | CE1 | PHE | A | 827 | 8.239 | 29.331 | 26.834 | 1.00 | 34.03 |
| 1292 | CE2 | PHE | A | 827 | 6.225 | 28.664 | 25.705 | 1.00 | 26.27 |
| 1293 | CZ | PHE | A | 827 | 7.472 | 29.265 | 25.676 | 1.00 | 30.82 |
| 1294 | N | ASP | A | 828 | 8.705 | 27.105 | 30.745 | 1.00 | 42.94 |
| 1295 | CA | ASP | A | 828 | 10.120 | 27.388 | 30.829 | 1.00 | 49.10 |
| 1296 | C | ASP | A | 828 | 10.933 | 26.130 | 31.071 | 1.00 | 49.16 |
| 1297 | O | ASP | A | 828 | 12.107 | 26.056 | 30.681 | 1.00 | 48.64 |
| 1298 | CB | ASP | A | 828 | 10.371 | 28.459 | 31.899 | 1.00 | 53.78 |
| 1299 | CG | ASP | A | 828 | 9.730 | 29.810 | 31.539 | 1.00 | 65.91 |
| 1300 | OD1 | ASP | A | 828 | 9.610 | 30.121 | 30.329 | 1.00 | 69.82 |
| 1301 | OD2 | ASP | A | 828 | 9.357 | 30.576 | 32.461 | 1.00 | 63.94 |
| 1302 | N | GLU | A | 829 | 10.313 | 25.138 | 31.703 | 1.00 | 49.55 |
| 1303 | CA | GLU | A | 829 | 10.997 | 23.875 | 31.941 | 1.00 | 49.00 |
| 1304 | C | GLU | A | 829 | 10.952 | 23.103 | 30.622 | 1.00 | 37.58 |
| 1305 | O | GLU | A | 829 | 11.969 | 22.573 | 30.177 | 1.00 | 35.86 |
| 1306 | CB | GLU | A | 829 | 10.350 | 23.073 | 33.076 | 1.00 | 61.68 |
| 1307 | CG | GLU | A | 829 | 11.366 | 22.268 | 33.925 | 1.00 | 81.07 |
| 1308 | CD | GLU | A | 829 | 10.939 | 20.814 | 34.222 | 1.00 | 92.42 |
| 1309 | OE1 | GLU | A | 829 | 9.952 | 20.603 | 34.969 | 1.00 | 97.07 |
| 1310 | OE2 | GLU | A | 829 | 11.619 | 19.881 | 33.727 | 1.00 | 91.99 |
| 1311 | N | LEU | A | 830 | 9.794 | 23.102 | 29.962 | 1.00 | 33.30 |
| 1312 | CA | LEU | A | 830 | 9.626 | 22.436 | 28.661 | 1.00 | 37.63 |
| 1313 | C | LEU | A | 830 | 10.621 | 23.003 | 27.673 | 1.00 | 41.33 |
| 1314 | O | LEU | A | 830 | 11.366 | 22.261 | 27.031 | 1.00 | 46.84 |
| 1315 | CB | LEU | A | 830 | 8.240 | 22.701 | 28.083 | 1.00 | 30.37 |
| 1316 | CG | LEU | A | 830 | 7.257 | 21.551 | 28.118 | 1.00 | 39.95 |
| 1317 | CD1 | LEU | A | 830 | 6.108 | 21.877 | 27.212 | 1.00 | 34.14 |
| 1318 | CD2 | LEU | A | 830 | 7.951 | 20.268 | 27.671 | 1.00 | 49.69 |
| 1319 | N | ARG | A | 831 | 10.621 | 24.333 | 27.566 | 1.00 | 44.13 |
| 1320 | CA | ARG | A | 831 | 11.504 | 25.048 | 26.658 | 1.00 | 40.24 |
| 1321 | C | ARG | A | 831 | 12.977 | 24.673 | 26.862 | 1.00 | 37.21 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1322 | O | ARG | A | 831 | 13.690 | 24.354 | 25.901 | 1.00 | 37.75 |
| 1323 | CB | ARG | A | 831 | 11.285 | 26.566 | 26.765 | 1.00 | 34.87 |
| 1324 | CG | ARG | A | 831 | 12.179 | 27.322 | 25.816 | 1.00 | 34.44 |
| 1325 | CD | ARG | A | 831 | 11.915 | 28.804 | 25.731 | 1.00 | 38.71 |
| 1326 | NE | ARG | A | 831 | 13.020 | 29.423 | 25.006 | 1.00 | 39.34 |
| 1327 | CZ | ARG | A | 831 | 13.073 | 29.573 | 23.682 | 1.00 | 44.99 |
| 1328 | NH1 | ARG | A | 831 | 12.060 | 29.164 | 22.926 | 1.00 | 40.07 |
| 1329 | NH2 | ARG | A | 831 | 14.178 | 30.050 | 23.104 | 1.00 | 41.88 |
| 1330 | N | MET | A | 832 | 13.411 | 24.625 | 28.116 | 1.00 | 38.65 |
| 1331 | CA | MET | A | 832 | 14.799 | 24.281 | 28.440 | 1.00 | 43.09 |
| 1332 | C | MET | A | 832 | 15.239 | 22.880 | 27.996 | 1.00 | 44.84 |
| 1333 | O | MET | A | 832 | 16.400 | 22.662 | 27.602 | 1.00 | 43.33 |
| 1334 | CB | MET | A | 832 | 15.065 | 24.409 | 29.941 | 1.00 | 41.77 |
| 1335 | CG | MET | A | 832 | 16.486 | 23.967 | 30.274 | 1.00 | 50.47 |
| 1336 | SD | MET | A | 832 | 16.856 | 23.608 | 31.997 | 1.00 | 61.59 |
| 1337 | CE | MET | A | 832 | 15.233 | 23.187 | 32.715 | 1.00 | 51.34 |
| 1338 | N | ASN | A | 833 | 14.339 | 21.914 | 28.141 | 1.00 | 46.14 |
| 1339 | CA | ASN | A | 833 | 14.661 | 20.552 | 27.754 | 1.00 | 44.12 |
| 1340 | C | ASN | A | 833 | 14.756 | 20.387 | 26.255 | 1.00 | 40.57 |
| 1341 | O | ASN | A | 833 | 15.670 | 19.729 | 25.766 | 1.00 | 45.06 |
| 1342 | CB | ASN | A | 833 | 13.689 | 19.580 | 28.379 | 1.00 | 47.22 |
| 1343 | CG | ASN | A | 833 | 14.098 | 19.202 | 29.782 | 1.00 | 44.35 |
| 1344 | OD1 | ASN | A | 833 | 15.262 | 18.849 | 30.041 | 1.00 | 53.06 |
| 1345 | ND2 | ASN | A | 833 | 13.161 | 19.299 | 30.705 | 1.00 | 42.24 |
| 1346 | N | TYR | A | 834 | 13.866 | 21.048 | 25.523 | 1.00 | 35.70 |
| 1347 | CA | TYR | A | 834 | 13.923 | 21.006 | 24.074 | 1.00 | 36.60 |
| 1348 | C | TYR | A | 834 | 15.260 | 21.582 | 23.589 | 1.00 | 35.58 |
| 1349 | O | TYR | A | 834 | 15.891 | 21.044 | 22.670 | 1.00 | 39.23 |
| 1350 | CB | TYR | A | 834 | 12.723 | 21.729 | 23.473 | 1.00 | 33.03 |
| 1351 | CG | TYR | A | 834 | 11.480 | 20.849 | 23.446 | 1.00 | 39.21 |
| 1352 | CD1 | TYR | A | 834 | 10.724 | 20.630 | 24.597 | 1.00 | 41.70 |
| 1353 | CD2 | TYR | A | 834 | 11.085 | 20.196 | 22.274 | 1.00 | 38.14 |
| 1354 | CE1 | TYR | A | 834 | 9.598 | 19.774 | 24.587 | 1.00 | 44.38 |
| 1355 | CE2 | TYR | A | 834 | 9.959 | 19.337 | 22.255 | 1.00 | 42.60 |
| 1356 | CZ | TYR | A | 834 | 9.229 | 19.132 | 23.416 | 1.00 | 42.12 |
| 1357 | OH | TYR | A | 834 | 8.164 | 18.264 | 23.404 | 1.00 | 38.82 |
| 1358 | N | ILE | A | 835 | 15.754 | 22.607 | 24.275 | 1.00 | 37.68 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1359 | CA | ILE | A | 835 | 17.042 | 23.197 | 23.901 | 1.00 | 42.31 |
| 1360 | C | ILE | A | 835 | 18.103 | 22.133 | 24.060 | 1.00 | 42.75 |
| 1361 | O | ILE | A | 835 | 18.995 | 21.997 | 23.217 | 1.00 | 45.95 |
| 1362 | CB | ILE | A | 835 | 17.408 | 24.417 | 24.782 | 1.00 | 37.10 |
| 1363 | CG1 | ILE | A | 835 | 16.357 | 25.512 | 24.558 | 1.00 | 41.30 |
| 1364 | CG2 | ILE | A | 835 | 18.832 | 24.912 | 24.457 | 1.00 | 29.58 |
| 1365 | CD1 | ILE | A | 835 | 16.610 | 26.802 | 25.286 | 1.00 | 39.55 |
| 1366 | N | LYS | A | 836 | 17.980 | 21.373 | 25.144 | 1.00 | 44.22 |
| 1367 | CA | LYS | A | 836 | 18.925 | 20.313 | 25.442 | 1.00 | 44.77 |
| 1368 | C | LYS | A | 836 | 18.846 | 19.252 | 24.363 | 1.00 | 41.28 |
| 1369 | O | LYS | A | 836 | 19.866 | 18.780 | 23.914 | 1.00 | 46.84 |
| 1370 | CB | LYS | A | 836 | 18.655 | 19.694 | 26.813 | 1.00 | 46.26 |
| 1371 | CG | LYS | A | 836 | 19.116 | 20.512 | 28.011 | 1.00 | 49.87 |
| 1372 | CD | LYS | A | 836 | 18.609 | 19.834 | 29.274 | 0.00 | 50.55 |
| 1373 | CE | LYS | A | 836 | 19.311 | 20.321 | 30.524 | 0.00 | 51.77 |
| 1374 | NZ | LYS | A | 836 | 18.709 | 19.697 | 31.735 | 0.00 | 52.70 |
| 1375 | N | GLU | A | 837 | 17.655 | 18.928 | 23.883 | 1.00 | 40.62 |
| 1376 | CA | GLU | A | 837 | 17.543 | 17.899 | 22.851 | 1.00 | 44.21 |
| 1377 | C | GLU | A | 837 | 18.253 | 18.344 | 21.603 | 1.00 | 43.91 |
| 1378 | O | GLU | A | 837 | 18.902 | 17.549 | 20.922 | 1.00 | 49.11 |
| 1379 | CB | GLU | A | 837 | 16.077 | 17.573 | 22.516 | 1.00 | 43.88 |
| 1380 | CG | GLU | A | 837 | 15.226 | 17.032 | 23.675 | 1.00 | 44.03 |
| 1381 | CD | GLU | A | 837 | 15.937 | 15.978 | 24.534 | 1.00 | 50.34 |
| 1382 | OE1 | GLU | A | 837 | 16.915 | 15.332 | 24.076 | 1.00 | 45.21 |
| 1383 | OE2 | GLU | A | 837 | 15.512 | 15.804 | 25.696 | 1.00 | 56.07 |
| 1384 | N | LEU | A | 838 | 18.113 | 19.627 | 21.305 | 1.00 | 44.87 |
| 1385 | CA | LEU | A | 838 | 18.739 | 20.218 | 20.139 | 1.00 | 43.87 |
| 1386 | C | LEU | A | 838 | 20.244 | 20.120 | 20.285 | 1.00 | 42.53 |
| 1387 | O | LEU | A | 838 | 20.949 | 19.835 | 19.332 | 1.00 | 42.95 |
| 1388 | CB | LEU | A | 838 | 18.324 | 21.675 | 20.019 | 1.00 | 42.66 |
| 1389 | CG | LEU | A | 838 | 19.051 | 22.463 | 18.932 | 1.00 | 44.98 |
| 1390 | CD1 | LEU | A | 838 | 18.810 | 21.814 | 17.567 | 1.00 | 39.69 |
| 1391 | CD2 | LEU | A | 838 | 18.571 | 23.909 | 18.964 | 1.00 | 37.64 |
| 1392 | N | ASP | A | 839 | 20.713 | 20.346 | 21.501 | 1.00 | 44.58 |
| 1393 | CA | ASP | A | 839 | 22.129 | 20.286 | 21.844 | 1.00 | 52.02 |
| 1394 | C | ASP | A | 839 | 22.630 | 18.829 | 21.696 | 1.00 | 57.30 |
| 1395 | O | ASP | A | 839 | 23.750 | 18.569 | 21.224 | 1.00 | 54.16 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1396 | CB | ASP | A | 839 | 22.262 | 20.760 | 23.305 | 1.00 | 59.66 |
| 1397 | CG | ASP | A | 839 | 23.673 | 21.234 | 23.674 | 1.00 | 66.70 |
| 1398 | OD1 | ASP | A | 839 | 24.584 | 21.284 | 22.812 | 1.00 | 65.99 |
| 1399 | OD2 | ASP | A | 839 | 23.850 | 21.598 | 24.860 | 1.00 | 68.25 |
| 1400 | N | ARG | A | 840 | 21.768 | 17.884 | 22.081 | 1.00 | 62.29 |
| 1401 | CA | ARG | A | 840 | 22.065 | 16.453 | 22.022 | 1.00 | 62.05 |
| 1402 | C | ARG | A | 840 | 22.280 | 16.022 | 20.591 | 1.00 | 60.23 |
| 1403 | O | ARG | A | 840 | 23.331 | 15.468 | 20.268 | 1.00 | 57.37 |
| 1404 | CB | ARG | A | 840 | 20.927 | 15.623 | 22.643 | 1.00 | 61.97 |
| 1405 | CG | ARG | A | 840 | 21.035 | 14.114 | 22.407 | 1.00 | 62.90 |
| 1406 | CD | ARG | A | 840 | 20.219 | 13.320 | 23.422 | 1.00 | 62.88 |
| 1407 | NE | ARG | A | 840 | 20.676 | 13.542 | 24.799 | 1.00 | 67.96 |
| 1408 | CZ | ARG | A | 840 | 19.875 | 13.676 | 25.861 | 1.00 | 65.20 |
| 1409 | NH1 | ARG | A | 840 | 18.550 | 13.618 | 25.718 | 1.00 | 60.29 |
| 1410 | NH2 | ARG | A | 840 | 20.404 | 13.889 | 27.066 | 1.00 | 61.82 |
| 1411 | N | ILE | A | 841 | 21.298 | 16.311 | 19.739 | 1.00 | 57.25 |
| 1412 | CA | ILE | A | 841 | 21.344 | 15.956 | 18.325 | 1.00 | 64.29 |
| 1413 | C | ILE | A | 841 | 22.494 | 16.602 | 17.508 | 1.00 | 71.86 |
| 1414 | O | ILE | A | 841 | 22.706 | 16.276 | 16.327 | 1.00 | 78.18 |
| 1415 | CB | ILE | A | 841 | 19.998 | 16.261 | 17.674 | 1.00 | 66.05 |
| 1416 | CG1 | ILE | A | 841 | 19.813 | 17.765 | 17.505 | 1.00 | 60.52 |
| 1417 | CG2 | ILE | A | 841 | 18.887 | 15.730 | 18.567 | 1.00 | 69.45 |
| 1418 | CD1 | ILE | A | 841 | 18.835 | 18.115 | 16.439 | 1.00 | 55.24 |
| 1419 | N | ILE | A | 842 | 23.210 | 17.541 | 18.127 | 1.00 | 74.71 |
| 1420 | CA | ILE | A | 842 | 24.354 | 18.208 | 17.489 | 1.00 | 72.84 |
| 1421 | C | ILE | A | 842 | 25.643 | 17.430 | 17.845 | 1.00 | 76.76 |
| 1422 | O | ILE | A | 842 | 26.431 | 17.080 | 16.952 | 1.00 | 76.88 |
| 1423 | CB | ILE | A | 842 | 24.507 | 19.684 | 17.990 | 1.00 | 62.10 |
| 1424 | CG1 | ILE | A | 842 | 23.312 | 20.522 | 17.569 | 1.00 | 54.67 |
| 1425 | CG2 | ILE | A | 842 | 25.769 | 20.303 | 17.439 | 1.00 | 59.08 |
| 1426 | CD1 | ILE | A | 842 | 23.101 | 20.519 | 16.109 | 1.00 | 56.78 |
| 1427 | N | ALA | A | 843 | 25.819 | 17.166 | 19.152 | 1.00 | 82.38 |
| 1428 | CA | ALA | A | 843 | 26.979 | 16.461 | 19.733 | 1.00 | 81.09 |
| 1429 | C | ALA | A | 843 | 26.960 | 14.955 | 19.524 | 1.00 | 79.83 |
| 1430 | O | ALA | A | 843 | 27.924 | 14.260 | 19.838 | 1.00 | 79.34 |
| 1431 | CB | ALA | A | 843 | 27.076 | 16.768 | 21.247 | 1.00 | 73.81 |
| 1432 | N | CYS | A | 844 | 25.872 | 14.469 | 18.947 | 1.00 | 80.33 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1433 | CA | CYS | A | 844 | 25.657 | 13.052 | 18.721 | 1.00 | 80.34 |
| 1434 | C | CYS | A | 844 | 25.771 | 12.645 | 17.215 | 1.00 | 84.98 |
| 1435 | O | CYS | A | 844 | 25.056 | 11.750 | 16.759 | 1.00 | 90.65 |
| 1436 | CB | CYS | A | 844 | 24.269 | 12.717 | 19.315 | 1.00 | 80.98 |
| 1437 | SG | CYS | A | 844 | 24.002 | 11.110 | 20.120 | 1.00 | 93.38 |
| 1438 | N | LYS | A | 845 | 26.665 | 13.302 | 16.460 | 1.00 | 86.19 |
| 1439 | CA | LYS | A | 845 | 26.917 | 13.002 | 15.026 | 1.00 | 83.63 |
| 1440 | C | LYS | A | 845 | 28.420 | 13.101 | 14.620 | 1.00 | 90.32 |
| 1441 | O | LYS | A | 845 | 29.110 | 12.079 | 14.517 | 1.00 | 87.60 |
| 1442 | CB | LYS | A | 845 | 26.063 | 13.874 | 14.087 | 1.00 | 72.76 |
| 1443 | CG | LYS | A | 845 | 24.727 | 13.263 | 13.735 | 1.00 | 66.51 |
| 1444 | CD | LYS | A | 845 | 23.998 | 14.104 | 12.702 | 0.00 | 63.58 |
| 1445 | CE | LYS | A | 845 | 24.644 | 14.005 | 11.326 | 0.00 | 60.54 |
| 1446 | NZ | LYS | A | 845 | 23.795 | 13.251 | 10.351 | 0.00 | 57.84 |
| 1447 | N | ARG | A | 846 | 28.929 | 14.316 | 14.384 | 1.00 | 97.68 |
| 1448 | CA | ARG | A | 846 | 30.336 | 14.489 | 13.998 | 1.00 | 99.68 |
| 1449 | C | ARG | A | 846 | 31.289 | 15.076 | 15.097 | 1.00 | 104.16 |
| 1450 | O | ARG | A | 846 | 31.315 | 14.556 | 16.229 | 1.00 | 101.78 |
| 1451 | CB | ARG | A | 846 | 30.442 | 15.246 | 12.671 | 1.00 | 94.46 |
| 1452 | CG | ARG | A | 846 | 31.702 | 14.917 | 11.839 | 1.00 | 92.16 |
| 1453 | CD | ARG | A | 846 | 31.538 | 13.643 | 10.999 | 1.00 | 87.99 |
| 1454 | NE | ARG | A | 846 | 32.671 | 12.718 | 11.128 | 1.00 | 86.09 |
| 1455 | CZ | ARG | A | 846 | 32.667 | 11.463 | 10.675 | 1.00 | 85.82 |
| 1456 | NH1 | ARG | A | 846 | 31.583 | 10.985 | 10.058 | 1.00 | 79.74 |
| 1457 | NH2 | ARG | A | 846 | 33.720 | 10.666 | 10.891 | 1.00 | 82.90 |
| 1458 | N | LYS | A | 847 | 32.040 | 16.139 | 14.736 | 1.00 | 109.20 |
| 1459 | CA | LYS | A | 847 | 33.061 | 16.820 | 15.585 | 1.00 | 108.66 |
| 1460 | C | LYS | A | 847 | 32.769 | 17.424 | 16.961 | 1.00 | 113.81 |
| 1461 | O | LYS | A | 847 | 31.596 | 17.569 | 17.368 | 1.00 | 118.99 |
| 1462 | CB | LYS | A | 847 | 33.760 | 17.897 | 14.758 | 0.00 | 98.30 |
| 1463 | CG | LYS | A | 847 | 34.514 | 17.383 | 13.563 | 0.00 | 85.34 |
| 1464 | CD | LYS | A | 847 | 35.150 | 18.542 | 12.840 | 0.00 | 73.33 |
| 1465 | CE | LYS | A | 847 | 35.928 | 18.073 | 11.641 | 0.00 | 64.16 |
| 1466 | NZ | LYS | A | 847 | 36.604 | 19.219 | 10.996 | 0.00 | 56.55 |
| 1467 | N | ASN | A | 848 | 33.860 | 17.821 | 17.636 | 1.00 | 115.89 |
| 1468 | CA | ASN | A | 848 | 33.904 | 18.449 | 18.991 | 1.00 | 116.39 |
| 1469 | C | ASN | A | 848 | 35.379 | 18.906 | 19.266 | 1.00 | 119.41 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1470 | O | ASN | A | 848 | 36.305 | 18.286 | 18.735 | 1.00 | 122.42 |
| 1471 | CB | ASN | A | 848 | 33.392 | 17.448 | 20.089 | 1.00 | 106.55 |
| 1472 | CG | ASN | A | 848 | 33.984 | 16.032 | 19.955 | 1.00 | 99.70 |
| 1473 | OD1 | ASN | A | 848 | 35.078 | 15.825 | 19.426 | 1.00 | 96.25 |
| 1474 | ND2 | ASN | A | 848 | 33.240 | 15.046 | 20.457 | 1.00 | 89.90 |
| 1475 | N | PRO | A | 849 | 35.622 | 20.019 | 20.031 | 1.00 | 119.93 |
| 1476 | CA | PRO | A | 849 | 34.808 | 21.031 | 20.742 | 1.00 | 119.46 |
| 1477 | C | PRO | A | 849 | 34.292 | 22.298 | 20.008 | 1.00 | 118.74 |
| 1478 | O | PRO | A | 849 | 33.082 | 22.562 | 20.025 | 1.00 | 121.98 |
| 1479 | CB | PRO | A | 849 | 35.725 | 21.422 | 21.914 | 1.00 | 118.16 |
| 1480 | CG | PRO | A | 849 | 37.063 | 21.454 | 21.259 | 1.00 | 118.96 |
| 1481 | CD | PRO | A | 849 | 37.039 | 20.160 | 20.430 | 1.00 | 120.61 |
| 1482 | N | THR | A | 850 | 35.178 | 23.098 | 19.399 | 1.00 | 113.94 |
| 1483 | CA | THR | A | 850 | 34.738 | 24.330 | 18.701 | 1.00 | 107.06 |
| 1484 | C | THR | A | 850 | 33.693 | 24.100 | 17.584 | 1.00 | 103.74 |
| 1485 | O | THR | A | 850 | 32.820 | 24.944 | 17.372 | 1.00 | 104.75 |
| 1486 | CB | THR | A | 850 | 35.931 | 25.182 | 18.134 | 1.00 | 105.27 |
| 1487 | OG1 | THR | A | 850 | 36.538 | 24.500 | 17.025 | 1.00 | 104.75 |
| 1488 | CG2 | THR | A | 850 | 36.976 | 25.482 | 19.222 | 1.00 | 99.79 |
| 1489 | N | SER | A | 851 | 33.788 | 22.949 | 16.909 | 1.00 | 98.29 |
| 1490 | CA | SER | A | 851 | 32.885 | 22.562 | 15.810 | 1.00 | 93.63 |
| 1491 | C | SER | A | 851 | 31.374 | 22.559 | 16.173 | 1.00 | 89.97 |
| 1492 | O | SER | A | 851 | 30.553 | 23.123 | 15.429 | 1.00 | 85.73 |
| 1493 | CB | SER | A | 851 | 33.319 | 21.185 | 15.262 | 1.00 | 93.65 |
| 1494 | OG | SER | A | 851 | 32,729 | 20.888 | 14.012 | 1.00 | 96.10 |
| 1495 | N | CYS | A | 852 | 31.000 | 21.964 | 17.311 | 1.00 | 87.36 |
| 1496 | CA | CYS | A | 852 | 29.587 | 21.932 | 17.706 | 1.00 | 85.60 |
| 1497 | C | CYS | A | 852 | 29.128 | 23.167 | 18.541 | 1.00 | 85.71 |
| 1498 | O | CYS | A | 852 | 27.929 | 23.468 | 18.592 | 1.00 | 81.46 |
| 1499 | CB | CYS | A | 852 | 29.260 | 20.605 | 18.409 | 1.00 | 85.66 |
| 1500 | SG | CYS | A | 852 | 29.511 | 20.548 | 20.196 | 1.00 | 92.38 |
| 1501 | N | SER | A | 853 | 30.071 | 23.873 | 19.184 | 1.00 | 88.63 |
| 1502 | CA | SER | A | 853 | 29.760 | 25.085 | 19.970 | 1.00 | 85.42 |
| 1503 | C | SER | A | 853 | 29.261 | 26.147 | 18.970 | 1.00 | 81.80 |
| 1504 | O | SER | A | 853 | 28.320 | 26.914 | 19.260 | 1.00 | 75.88 |
| 1505 | CB | SER | A | 853 | 31.015 | 25.615 | 20.702 | 1.00 | 83.41 |
| 1506 | OG | SER | A | 853 | 31.524 | 24.733 | 21.692 | 1.00 | 75.95 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1507 | N | ARG | A | 854 | 29.929 | 26.190 | 17.806 | 1.00 | 77.44 |
| 1508 | CA | ARG | A | 854 | 29.586 | 27.108 | 16.721 | 1.00 | 75.23 |
| 1509 | C | ARG | A | 854 | 28.428 | 26.555 | 15.903 | 1.00 | 68.44 |
| 1510 | O | ARG | A | 854 | 27.614 | 27.325 | 15.381 | 1.00 | 64.21 |
| 1511 | CB | ARG | A | 854 | 30.803 | 27.423 | 15.831 | 1.00 | 83.31 |
| 1512 | CG | ARG | A | 854 | 31.492 | 26.211 | 15.215 | 1.00 | 95.75 |
| 1513 | CD | ARG | A | 854 | 32.915 | 26.540 | 14.706 | 1.00 | 105.49 |
| 1514 | NE | ARG | A | 854 | 32.927 | 27.070 | 13.344 | 1.00 | 111.61 |
| 1515 | CZ | ARG | A | 854 | 33.255 | 28.318 | 13.014 | 1.00 | 107.74 |
| 1516 | NH1 | ARG | A | 854 | 33.614 | 29.182 | 13.963 | 1.00 | 108.73 |
| 1517 | NH2 | ARG | A | 854 | 33.171 | 28.704 | 11.740 | 1.00 | 100.08 |
| 1518 | N | ARG | A | 855 | 28.341 | 25.228 | 15.801 | 1.00 | 62.47 |
| 1519 | CA | ARG | A | 855 | 27.229 | 24.606 | 15.087 | 1.00 | 56.87 |
| 1520 | C | ARG | A | 855 | 25.922 | 24.945 | 15.831 | 1.00 | 55.34 |
| 1521 | O | ARG | A | 855 | 24.861 | 25.054 | 15.206 | 1.00 | 61.05 |
| 1522 | CB | ARG | A | 855 | 27.423 | 23.085 | 14.974 | 1.00 | 53.03 |
| 1523 | CG | ARG | A | 855 | 26.247 | 22.321 | 14.341 | 1.00 | 51.39 |
| 1524 | CD | ARG | A | 855 | 25.983 | 22.707 | 12.891 | 1.00 | 55.10 |
| 1525 | NE | ARG | A | 855 | 24.943 | 21.899 | 12.233 | 1.00 | 54.36 |
| 1526 | CZ | ARG | A | 855 | 24.745 | 21.858 | 10.911 | 1.00 | 55.00 |
| 1527 | NH1 | ARG | A | 855 | 25.517 | 22.579 | 10.110 | 1.00 | 52.83 |
| 1528 | NH2 | ARG | A | 855 | 23.796 | 21.094 | 10.384 | 1.00 | 48.85 |
| 1529 | N | PHE | A | 856 | 26.003 | 25.152 | 17.152 | 1.00 | 52.54 |
| 1530 | CA | PHE | A | 856 | 24.817 | 25.508 | 17.962 | 1.00 | 49.53 |
| 1531 | C | PHE | A | 856 | 24.438 | 26.974 | 17.738 | 1.00 | 48.70 |
| 1532 | O | PHE | A | 856 | 23.254 | 27.326 | 17.597 | 1.00 | 45.42 |
| 1533 | CB | PHE | A | 856 | 25.066 | 25.270 | 19.457 | 1.00 | 46.16 |
| 1534 | CG | PHE | A | 856 | 23.852 | 25.527 | 20.319 | 1.00 | 46.93 |
| 1535 | CD1 | PHE | A | 856 | 22.699 | 24.751 | 20.170 | 1.00 | 47.04 |
| 1536 | CD2 | PHE | A | 856 | 23.845 | 26.549 | 21.259 | 1.00 | 41.32 |
| 1537 | CE1 | PHE | A | 856 | 21.547 | 24.986 | 20.949 | 1.00 | 46.91 |
| 1538 | CE2 | PHE | A | 856 | 22.711 | 26.785 | 22.033 | 1.00 | 45.75 |
| 1539 | CZ | PHE | A | 856 | 21.553 | 25.999 | 21.877 | 1.00 | 42.35 |
| 1540 | N | TYR | A | 857 | 25.461 | 27.822 | 17.725 | 1.00 | 47.67 |
| 1541 | CA | TYR | A | 857 | 25.283 | 29.238 | 17.487 | 1.00 | 47.32 |
| 1542 | C | TYR | A | 857 | 24.503 | 29.373 | 16.181 | 1.00 | 44.87 |
| 1543 | O | TYR | A | 857 | 23.404 | 29.945 | 16.159 | 1.00 | 42.27 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1544 | CB | TYR | A | 857 | 26.667 | 29.901 | 17.407 | 1.00 | 50.61 |
| 1545 | CG | TYR | A | 857 | 26.684 | 31.282 | 16.807 | 1.00 | 54.67 |
| 1546 | CD1 | TYR | A | 857 | 26.450 | 32.422 | 17.593 | 1.00 | 59.56 |
| 1547 | CD2 | TYR | A | 857 | 26.935 | 31.454 | 15.442 | 1.00 | 54.77 |
| 1548 | CE1 | TYR | A | 857 | 26.469 | 33.719 | 17.022 | 1.00 | 58.68 |
| 1549 | CE2 | TYR | A | 857 | 26.953 | 32.728 | 14.860 | 1.00 | 58.39 |
| 1550 | CZ | TYR | A | 857 | 26.720 | 33.855 | 15.647 | 1.00 | 61.97 |
| 1551 | OH | TYR | A | 857 | 26.725 | 35.091 | 15.035 | 1.00 | 56.70 |
| 1552 | N | GLN | A | 858 | 25.006 | 28.706 | 15.141 | 1.00 | 43.56 |
| 1553 | CA | GLN | A | 858 | 24.400 | 28.744 | 13.803 | 1.00 | 42.92 |
| 1554 | C | GLN | A | 858 | 22.927 | 28.400 | 13.731 | 1.00 | 43.62 |
| 1555 | O | GLN | A | 858 | 22.173 | 29.017 | 12.988 | 1.00 | 47.41 |
| 1556 | CB | GLN | A | 858 | 25.112 | 27.795 | 12.859 | 1.00 | 38.18 |
| 1557 | CG | GLN | A | 858 | 26.563 | 28.061 | 12.645 | 1.00 | 41.30 |
| 1558 | CD | GLN | A | 858 | 27.216 | 26.990 | 11.788 | 1.00 | 48.05 |
| 1559 | OE1 | GLN | A | 858 | 28.399 | 27.051 | 11.521 | 1.00 | 60.25 |
| 1560 | NE2 | GLN | A | 858 | 26.446 | 25.995 | 11.369 | 1.00 | 53.15 |
| 1561 | N | LEU | A | 859 | 22.523 | 27.372 | 14.452 | 1.00 | 46.85 |
| 1562 | CA | LEU | A | 859 | 21.136 | 26.956 | 14.407 | 1.00 | 44.50 |
| 1563 | C | LEU | A | 859 | 20.226 | 27.884 | 15.180 | 1.00 | 41.57 |
| 1564 | O | LEU | A | 859 | 19.091 | 28.129 | 14.764 | 1.00 | 41.55 |
| 1565 | CB | LEU | A | 859 | 20.998 | 25.529 | 14.919 | 1.00 | 49.03 |
| 1566 | CG | LEU | A | 859 | 21.571 | 24.412 | 14.044 | 1.00 | 51.91 |
| 1567 | CD1 | LEU | A | 859 | 21.563 | 23.090 | 14.809 | 1.00 | 49.78 |
| 1568 | CD2 | LEU | A | 859 | 20.736 | 24.301 | 12.793 | 1.00 | 54.19 |
| 1569 | N | THR | A | 860 | 20.712 | 28.393 | 16.307 | 1.00 | 36.22 |
| 1570 | CA | THR | A | 860 | 19.910 | 29.300 | 17.111 | 1.00 | 36.58 |
| 1571 | C | THR | A | 860 | 19.694 | 30.592 | 16.322 | 1.00 | 39.98 |
| 1572 | O | THR | A | 860 | 18.600 | 31.177 | 16.351 | 1.00 | 41.71 |
| 1573 | CB | THR | A | 860 | 20.557 | 29.532 | 18.474 | 1.00 | 35.81 |
| 1574 | OG1 | THR | A | 860 | 21.942 | 29.877 | 18.304 | 1.00 | 38.10 |
| 1575 | CG2 | THR | A | 860 | 20.445 | 28.256 | 19.302 | 1.00 | 33.36 |
| 1576 | N | LYS | A | 861 | 20.718 | 30.998 | 15.564 | 1.00 | 35.42 |
| 1577 | CA | LYS | A | 861 | 20.598 | 32.178 | 14.715 | 1.00 | 38.56 |
| 1578 | C | LYS | A | 861 | 19.556 | 31.871 | 13.639 | 1.00 | 44.29 |
| 1579 | O | LYS | A | 861 | 18.669 | 32.682 | 13.358 | 1.00 | 45.57 |
| 1580 | CB | LYS | A | 861 | 21.932 | 32.530 | 14.076 | 1.00 | 35.42 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1581 | CG | LYS | A | 861 | 22.807 | 33.422 | 14.941 | 1.00 | 48.32 |
| 1582 | CD | LYS | A | 861 | 22.394 | 34.906 | 14.850 | 1.00 | 56.04 |
| 1583 | CE | LYS | A | 861 | 23.270 | 35.776 | 15.755 | 1.00 | 60.56 |
| 1584 | NZ | LYS | A | 861 | 22.863 | 37.205 | 15.751 | 1.00 | 65.17 |
| 1585 | N | LEU | A | 862 | 19.644 | 30.667 | 13.075 | 1.00 | 46.55 |
| 1586 | CA | LEU | A | 862 | 18.690 | 30.228 | 12.069 | 1.00 | 39.38 |
| 1587 | C | LEU | A | 862 | 17.272 | 30.273 | 12.642 | 1.00 | 40.85 |
| 1588 | O | LEU | A | 862 | 16.353 | 30.720 | 11.954 | 1.00 | 45.44 |
| 1589 | CB | LEU | A | 862 | 19.009 | 28.808 | 11.607 | 1.00 | 41.98 |
| 1590 | CG | LEU | A | 862 | 17.987 | 28.298 | 10.587 | 1.00 | 49.48 |
| 1591 | CD1 | LEU | A | 862 | 18.036 | 29.176 | 9.346 | 1.00 | 46.04 |
| 1592 | CD2 | LEU | A | 862 | 18.245 | 26.832 | 10.228 | 1.00 | 47.86 |
| 1593 | N | LEU | A | 863 | 17.108 | 29.839 | 13.900 | 1.00 | 38.85 |
| 1594 | CA | LEU | A | 863 | 15.807 | 29.824 | 14.575 | 1.00 | 37.02 |
| 1595 | C | LEU | A | 863 | 15.265 | 31.223 | 14.689 | 1.00 | 35.94 |
| 1596 | O | LEU | A | 863 | 14.075 | 31.459 | 14.455 | 1.00 | 36.06 |
| 1597 | CB | LEU | A | 863 | 15.915 | 29.232 | 15.980 | 1.00 | 43.64 |
| 1598 | CG | LEU | A | 863 | 15.888 | 27.707 | 16.086 | 1.00 | 42.37 |
| 1599 | CD1 | LEU | A | 863 | 15.854 | 27.238 | 17.548 | 1.00 | 35.50 |
| 1600 | CD2 | LEU | A | 863 | 14.671 | 27.219 | 15.334 | 1.00 | 35.55 |
| 1601 | N | ASP | A | 864 | 16.161 | 32.142 | 15.041 | 1.00 | 35.57 |
| 1602 | CA | ASP | A | 864 | 15.845 | 33.564 | 15.196 | 1.00 | 37.53 |
| 1603 | C | ASP | A | 864 | 15.341 | 34.254 | 13.898 | 1.00 | 38.46 |
| 1604 | O | ASP | A | 864 | 14.455 | 35.123 | 13.959 | 1.00 | 36.51 |
| 1605 | CB | ASP | A | 864 | 17.051 | 34.299 | 15.824 | 1.00 | 29.27 |
| 1606 | CG | ASP | A | 864 | 17.214 | 34.005 | 17.334 | 1.00 | 39.10 |
| 1607 | OD1 | ASP | A | 864 | 16.388 | 33.270 | 17.927 | 1.00 | 42.54 |
| 1608 | OD2 | ASP | A | 864 | 18.174 | 34.526 | 17.947 | 1.00 | 37.49 |
| 1609 | N | SER | A | 865 | 15.843 | 33.796 | 12.740 | 1.00 | 40.46 |
| 1610 | CA | SER | A | 865 | 15.476 | 34.302 | 11.393 | 1.00 | 35.87 |
| 1611 | C | SER | A | 865 | 14.004 | 34.116 | 11.058 | 1.00 | 37.22 |
| 1612 | O | SER | A | 865 | 13.454 | 34.844 | 10.231 | 1.00 | 46.57 |
| 1613 | CB | SER | A | 865 | 16.235 | 33.551 | 10.284 | 1.00 | 34.07 |
| 1614 | OG | SER | A | 865 | 17.577 | 33.266 | 10.622 | 1.00 | 46.76 |
| 1615 | N | VAL | A | 866 | 13.398 | 33.095 | 11.650 | 1.00 | 35.77 |
| 1616 | CA | VAL | A | 866 | 12.013 | 32.752 | 11.399 | 1.00 | 35.13 |
| 1617 | C | VAL | A | 866 | 11.031 | 33.779 | 11.950 | 1.00 | 38.58 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1618 | O | VAL | A | 866 | 9.973 | 34.057 | 11.353 | 1.00 | 36.20 |
| 1619 | CB | VAL | A | 866 | 11.686 | 31.354 | 12.004 | 1.00 | 27.91 |
| 1620 | CG1 | VAL | A | 866 | 10.252 | 30.944 | 11.666 | 1.00 | 22.22 |
| 1621 | CG2 | VAL | A | 866 | 12.666 | 30.322 | 11.482 | 1.00 | 33.78 |
| 1622 | N | GLN | A | 867 | 11.395 | 34.366 | 13.081 | 1.00 | 39.58 |
| 1623 | CA | GLN | A | 867 | 10.525 | 35.342 | 13.735 | 1.00 | 43.96 |
| 1624 | C | GLN | A | 867 | 10.213 | 36.633 | 12.921 | 1.00 | 38.67 |
| 1625 | O | GLN | A | 867 | 9.030 | 37.022 | 12.789 | 1.00 | 34.00 |
| 1626 | CB | GLN | A | 867 | 11.039 | 35.603 | 15.163 | 1.00 | 47.82 |
| 1627 | CG | GLN | A | 867 | 11.248 | 34.307 | 15.987 | 1.00 | 37.53 |
| 1628 | CD | GLN | A | 867 | 9.951 | 33.535 | 16.271 | 1.00 | 40.95 |
| 1629 | OE1 | GLN | A | 867 | 8.841 | 33.972 | 15.943 | 1.00 | 40.47 |
| 1630 | NE2 | GLN | A | 867 | 10.097 | 32.375 | 16.883 | 1.00 | 44.07 |
| 1631 | N | PRO | A | 868 | 11.254 | 37.306 | 12.370 | 1.00 | 32.90 |
| 1632 | CA | PRO | A | 868 | 11.071 | 38.522 | 11.569 | 1.00 | 32.84 |
| 1633 | C | PRO | A | 868 | 10.156 | 38.173 | 10.407 | 1.00 | 35.58 |
| 1634 | O | PRO | A | 868 | 9.132 | 38.815 | 10.173 | 1.00 | 34.36 |
| 1635 | CB | PRO | A | 868 | 12.477 | 38.796 | 11.052 | 1.00 | 34.85 |
| 1636 | CG | PRO | A | 868 | 13.329 | 38.358 | 12.184 | 1.00 | 35.76 |
| 1637 | CD | PRO | A | 868 | 12.690 | 37.069 | 12.631 | 1.00 | 35.45 |
| 1638 | N | ILE | A | 869 | 10.494 | 37.075 | 9.740 | 1.00 | 35.25 |
| 1639 | CA | ILE | A | 869 | 9.730 | 36.578 | 8.616 | 1.00 | 31.95 |
| 1640 | C | ILE | A | 869 | 8.284 | 36.304 | 9.002 | 1.00 | 28.05 |
| 1641 | O | ILE | A | 869 | 7.359 | 36.758 | 8.332 | 1.00 | 33.69 |
| 1642 | CB | ILE | A | 869 | 10.389 | 35.318 | 8.072 | 1.00 | 34.50 |
| 1643 | CG1 | ILE | A | 869 | 11.841 | 35.639 | 7.697 | 1.00 | 31.97 |
| 1644 | CG2 | ILE | A | 869 | 9.630 | 34.813 | 6.870 | 1.00 | 25.52 |
| 1645 | CD1 | ILE | A | 869 | 12.604 | 34.477 | 7.120 | 1.00 | 36.91 |
| 1646 | N | ALA | A | 870 | 8.089 | 35.599 | 10.109 | 1.00 | 29.58 |
| 1647 | CA | ALA | A | 870 | 6.750 | 35.276 | 10.593 | 1.00 | 30.48 |
| 1648 | C | ALA | A | 870 | 6.010 | 36.567 | 10.913 | 1.00 | 34.81 |
| 1649 | O | ALA | A | 870 | 4.793 | 36.692 | 10.672 | 1.00 | 31.33 |
| 1650 | CB | ALA | A | 870 | 6.838 | 34.402 | 11.825 | 1.00 | 27.17 |
| 1651 | N | ARG | A | 871 | 6.754 | 37.534 | 11.441 | 1.00 | 35.08 |
| 1652 | CA | ARG | A | 871 | 6.201 | 38.845 | 11.766 | 1.00 | 38.73 |
| 1653 | C | ARG | A | 871 | 5.587 | 39.457 | 10.509 | 1.00 | 36.69 |
| 1654 | O | ARG | A | 871 | 4.411 | 39.824 | 10.496 | 1.00 | 33.58 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1655 | CB | ARG | A | 871 | 7.303 | 39.754 | 12.271 | 1.00 | 42.63 |
| 1656 | CG | ARG | A | 871 | 6.935 | 41.209 | 12.267 | 1.00 | 48.87 |
| 1657 | CD | ARG | A | 871 | 6.488 | 41.628 | 13.610 | 1.00 | 39.03 |
| 1658 | NE | ARG | A | 871 | 5.783 | 42.900 | 13.554 | 1.00 | 54.34 |
| 1659 | CZ | ARG | A | 871 | 5.420 | 43.599 | 14.629 | 1.00 | 58.85 |
| 1660 | NH1 | ARG | A | 871 | 5.717 | 43.140 | 15.841 | 1.00 | 58.33 |
| 1661 | NH2 | ARG | A | 871 | 4.700 | 44.713 | 14.497 | 1.00 | 67.98 |
| 1662 | N | GLU | A | 872 | 6.388 | 39.518 | 9.447 | 1.00 | 33.87 |
| 1663 | CA | GLU | A | 872 | 5.954 | 40.047 | 8.149 | 1.00 | 36.69 |
| 1664 | C | GLU | A | 872 | 4.721 | 39.346 | 7.610 | 1.00 | 35.07 |
| 1665 | O | GLU | A | 872 | 3.835 | 39.982 | 7.039 | 1.00 | 38.97 |
| 1666 | CB | GLU | A | 872 | 7.074 | 39.920 | 7.099 | 1.00 | 48.05 |
| 1667 | CG | GLU | A | 872 | 7.897 | 41.206 | 6.847 | 1.00 | 57.85 |
| 1668 | CD | GLU | A | 872 | 8.928 | 41.037 | 5.731 | 1.00 | 68.10 |
| 1669 | OE1 | GLU | A | 872 | 9.952 | 40.366 | 5.990 | 1.00 | 74.25 |
| 1670 | OE2 | GLU | A | 872 | 8.722 | 41.566 | 4.606 | 1.00 | 66.63 |
| 1671 | N | LEU | A | 873 | 4.671 | 38.027 | 7.747 | 1.00 | 37.62 |
| 1672 | CA | LEU | A | 873 | 3.528 | 37.281 | 7.244 | 1.00 | 35.57 |
| 1673 | C | LEU | A | 873 | 2.295 | 37.630 | 8.027 | 1.00 | 36.96 |
| 1674 | O | LEU | A | 873 | 1.187 | 37.664 | 7.475 | 1.00 | 37.28 |
| 1675 | CB | LEU | A | 873 | 3.794 | 35.790 | 7.331 | 1.00 | 46.12 |
| 1676 | CG | LEU | A | 873 | 5.003 | 35.377 | 6.498 | 1.00 | 44.82 |
| 1677 | CD1 | LEU | A | 873 | 5.185 | 33.921 | 6.706 | 1.00 | 46.86 |
| 1678 | CD2 | LEU | A | 873 | 4.807 | 35.680 | 5.024 | 1.00 | 41.83 |
| 1679 | N | HIS | A | 874 | 2.505 | 37.887 | 9.320 | 1.00 | 32.90 |
| 1680 | CA | HIS | A | 874 | 1.430 | 38.269 | 10.242 | 1.00 | 37.34 |
| 1681 | C | HIS | A | 874 | 0.775 | 39.603 | 9.828 | 1.00 | 38.25 |
| 1682 | O | HIS | A | 874 | -0.457 | 39.758 | 9.814 | 1.00 | 36.81 |
| 1683 | CB | HIS | A | 874 | 1.997 | 38.405 | 11.660 | 1.00 | 42.16 |
| 1684 | CG | HIS | A | 874 | 2.251 | 37.099 | 12.352 | 1.00 | 46.12 |
| 1685 | ND1 | HIS | A | 874 | 1.296 | 36.104 | 12.440 | 1.00 | 42.36 |
| 1686 | CD2 | HIS | A | 874 | 3.339 | 36.643 | 13.015 | 1.00 | 41.95 |
| 1687 | CE1 | HIS | A | 874 | 1.790 | 35.088 | 13.130 | 1.00 | 41.59 |
| 1688 | NE2 | HIS | A | 874 | 3.028 | 35.390 | 13.486 | 1.00 | 44.61 |
| 1689 | N | GLN | A | 875 | 1.628 | 40.572 | 9.520 | 1.00 | 38.39 |
| 1690 | CA | GLN | A | 875 | 1.217 | 41.906 | 9.090 | 1.00 | 36.36 |
| 1691 | C | GLN | A | 875 | 0.363 | 41.731 | 7.845 | 1.00 | 34.57 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1692 | O | GLN | A | 875 | -0.822 | 42.017 | 7.846 | 1.00 | 42.38 |
| 1693 | CB | GLN | A | 875 | 2.471 | 42.727 | 8.765 | 1.00 | 38.26 |
| 1694 | CG | GLN | A | 875 | 2.247 | 44.182 | 8.555 | 1.00 | 44.03 |
| 1695 | CD | GLN | A | 875 | 1.775 | 44.875 | 9.800 | 1.00 | 47.03 |
| 1696 | OE1 | GLN | A | 875 | 2.554 | 45.079 | 10.754 | 1.00 | 42.92 |
| 1697 | NE2 | GLN | A | 875 | 0.504 | 45.272 | 9.799 | 1.00 | 34.68 |
| 1698 | N | PHE | A | 876 | 0.943 | 41.081 | 6.849 | 1.00 | 33.26 |
| 1699 | CA | PHE | A | 876 | 0.284 | 40.845 | 5.582 | 1.00 | 33.07 |
| 1700 | C | PHE | A | 876 | -1.070 | 40.161 | 5.677 | 1.00 | 35.06 |
| 1701 | O | PHE | A | 876 | -2.036 | 40.573 | 5.032 | 1.00 | 34.48 |
| 1702 | CB | PHE | A | 876 | 1.212 | 40.017 | 4.692 | 1.00 | 39.86 |
| 1703 | CG | PHE | A | 876 | 0.676 | 39.782 | 3.313 | 1.00 | 39.61 |
| 1704 | CD1 | PHE | A | 876 | -0.320 | 38.846 | 3.085 | 1.00 | 41.67 |
| 1705 | CD2 | PHE | A | 876 | 1.163 | 40.507 | 2.248 | 1.00 | 39.57 |
| 1706 | CE1 | PHE | A | 876 | -0.823 | 38.650 | 1.838 | 1.00 | 44.63 |
| 1707 | CE2 | PHE | A | 876 | 0.665 | 40.318 | 0.991 | 1.00 | 40.20 |
| 1708 | CZ | PHE | A | 876 | -0.329 | 39.385 | 0.780 | 1.00 | 49.53 |
| 1709 | N | THR | A | 877 | -1.126 | 39.053 | 6.405 | 1.00 | 40.28 |
| 1710 | CA | THR | A | 877 | -2.380 | 38.320 | 6.505 | 1.00 | 38.84 |
| 1711 | C | THR | A | 877 | -3.450 | 39.157 | 7.208 | 1.00 | 41.69 |
| 1712 | O | THR | A | 877 | -4.594 | 39.193 | 6.767 | 1.00 | 41.63 |
| 1713 | CB | THR | A | 877 | -2.193 | 36,900 | 7.135 | 1.00 | 33.79 |
| 1714 | OG1 | THR | A | 877 | -3.364 | 36.117 | 6.886 | 1.00 | 38.07 |
| 1715 | CG2 | THR | A | 877 | -1.937 | 36.956 | 8.640 | 1.00 | 26.06 |
| 1716 | N | PHE | A | 878 | -3.062 | 39.889 | 8.251 | 1.00 | 39.96 |
| 1717 | CA | PHE | A | 878 | -3.997 | 40.737 | 8.984 | 1.00 | 38.02 |
| 1718 | C | PHE | A | 878 | -4.600 | 41.760 | 8.006 | 1.00 | 38.85 |
| 1719 | O | PHE | A | 878 | -5.821 | 41.922 | 7.916 | 1.00 | 33.84 |
| 1720 | CB | PHE | A | 878 | -3.248 | 41.450 | 10.109 | 1.00 | 41.25 |
| 1721 | CG | PHE | A | 878 | -4.035 | 42.540 | 10.764 | 1.00 | 45.72 |
| 1722 | CD1 | PHE | A | 878 | -5.318 | 42.298 | 11.237 | 1.00 | 47.19 |
| 1723 | CD2 | PHE | A | 878 | -3.490 | 43.810 | 10.910 | 1.00 | 47.82 |
| 1724 | CE1 | PHE | A | 878 | -6.045 | 43.296 | 11.842 | 1.00 | 52.43 |
| 1725 | CE2 | PHE | A | 878 | -4.208 | 44.813 | 11.515 | 1.00 | 44.62 |
| 1726 | CZ | PHE | A | 878 | -5.488 | 44.563 | 11.983 | 1.00 | 50.04 |
| 1727 | N | ASP | A | 879 | -3.717 | 42.398 | 7.243 | 1.00 | 41.64 |
| 1728 | CA | ASP | A | 879 | -4.078 | 43.395 | 6.242 | 1.00 | 44.39 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1729 | C | ASP | A | 879 | -4.975 | 42.733 | 5.201 | 1.00 | 41.74 |
| 1730 | O | ASP | A | 879 | -6.013 | 43.272 | 4.797 | 1.00 | 45.58 |
| 1731 | CB | ASP | A | 879 | -2.799 | 43.987 | 5.597 | 1.00 | 50.23 |
| 1732 | CG | ASP | A | 879 | -1.938 | 44.849 | 6.595 | 1.00 | 65.18 |
| 1733 | OD1 | ASP | A | 879 | -2.253 | 44.919 | 7.821 | 1.00 | 64.91 |
| 1734 | OD2 | ASP | A | 879 | -0.930 | 45.461 | 6.140 | 1.00 | 57.87 |
| 1735 | N | LEU | A | 880 | -4.581 | 41.542 | 4.784 | 1.00 | 39.59 |
| 1736 | CA | LEU | A | 880 | -5.349 | 40.778 | 3.824 | 1.00 | 41.16 |
| 1737 | C | LEU | A | 880 | -6.737 | 40.477 | 4.404 | 1.00 | 41.39 |
| 1738 | O | LEU | A | 880 | -7.720 | 40.472 | 3.682 | 1.00 | 46.61 |
| 1739 | CB | LEU | A | 880 | -4.594 | 39.483 | 3.484 | 1.00 | 41.26 |
| 1740 | CG | LEU | A | 880 | -5.117 | 38.492 | 2.429 | 1.00 | 46.22 |
| 1741 | CD1 | LEU | A | 880 | -5.910 | 39.174 | 1.299 | 1.00 | 39.53 |
| 1742 | CD2 | LEU | A | 880 | -3.925 | 37.702 | 1.873 | 1.00 | 45.58 |
| 1743 | N | LEU | A | 881 | -6.826 | 40.273 | 5.713 | 1.00 | 46.94 |
| 1744 | CA | LEU | A | 881 | -8.113 | 39.974 | 6.330 | 1.00 | 46.60 |
| 1745 | C | LEU | A | 881 | -9.028 | 41.185 | 6.310 | 1.00 | 50.61 |
| 1746 | O | LEU | A | 881 | -10.188 | 41.070 | 5.925 | 1.00 | 50.82 |
| 1747 | CB | LEU | A | 881 | -7.964 | 39.468 | 7.775 | 1.00 | 38.43 |
| 1748 | CG | LEU | A | 881 | -9.282 | 39.274 | 8.551 | 1.00 | 37.70 |
| 1749 | CD1 | LEU | A | 881 | -10.162 | 38.204 | 7.887 | 1.00 | 30.98 |
| 1750 | CD2 | LEU | A | 881 | -8.979 | 38.918 | 9.997 | 1.00 | 30.24 |
| 1751 | N | ILE | A | 882 | -8.531 | 42.341 | 6.746 | 1.00 | 48.70 |
| 1752 | CA | ILE | A | 882 | -9.390 | 43.517 | 6.752 | 1.00 | 55.61 |
| 1753 | C | ILE | A | 882 | -9.806 | 43.885 | 5.314 | 1.00 | 53.19 |
| 1754 | O | ILE | A | 882 | -10.966 | 44.227 | 5.076 | 1.00 | 56.55 |
| 1755 | CB | ILE | A | 882 | -8.797 | 44.720 | 7.603 | 1.00 | 54.73 |
| 1756 | CG1 | ILE | A | 882 | -7.489 | 45.252 | 7.008 | 1.00 | 58.00 |
| 1757 | CG2 | ILE | A | 882 | -8.566 | 44.271 | 9.054 | 1.00 | 42.17 |
| 1758 | CD1 | ILE | A | 882 | -6.825 | 46.382 | 7.811 | 1.00 | 61.98 |
| 1759 | N | LYS | A | 883 | -8.917 | 43.674 | 4.343 | 1.00 | 51.98 |
| 1760 | CA | LYS | A | 883 | -9.229 | 43.976 | 2.939 | 1.00 | 53.80 |
| 1761 | C | LYS | A | 883 | -10.126 | 42.935 | 2.296 | 1.00 | 56.69 |
| 1762 | O | LYS | A | 883 | -10.888 | 43.257 | 1.389 | 1.00 | 56.03 |
| 1763 | CB | LYS | A | 883 | -7.976 | 44.008 | 2.073 | 1.00 | 49.48 |
| 1764 | CG | LYS | A | 883 | -7.056 | 45.152 | 2.279 | 1.00 | 51.98 |
| 1765 | CD | LYS | A | 883 | -5.970 | 45.061 | 1.236 | 1.00 | 57.39 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1766 | CE | LYS | A | 883 | -4.752 | 45.885 | 1.601 | 1.00 | 65,28 |
| 1767 | NZ | LYS | A | 883 | -3.755 | 45.773 | 0.507 | 1.00 | 72.32 |
| 1768 | N | SER | A | 884 | -10.000 | 41.687 | 2.758 | 1.00 | 64.45 |
| 1769 | CA | SER | A | 884 | -10.721 | 40.515 | 2.228 | 1.00 | 60.51 |
| 1770 | C | SER | A | 884 | -12.055 | 40.765 | 1.543 | 1.00 | 57.37 |
| 1771 | O | SER | A | 884 | -12.167 | 40.549 | 0.334 | 1.00 | 55.96 |
| 1772 | CB | SER | A | 884 | -10.873 | 39.434 | 3.304 | 1.00 | 60.86 |
| 1773 | OG | SER | A | 884 | -11.559 | 39.933 | 4.437 | 1.00 | 69.00 |
| 1774 | N | HIS | A | 885 | -13.029 | 41.258 | 2.308 | 1.00 | 59.05 |
| 1775 | CA | HIS | A | 885 | -14.376 | 41.558 | 1.826 | 1.00 | 68.14 |
| 1776 | C | HIS | A | 885 | -14.388 | 42.167 | 0.424 | 1.00 | 74.11 |
| 1777 | O | HIS | A | 885 | -14.901 | 41.561 | -0.515 | 1.00 | 78.87 |
| 1778 | CB | HIS | A | 885 | -15.091 | 42.532 | 2.781 | 1.00 | 74.25 |
| 1779 | CG | HIS | A | 885 | -14.848 | 42.258 | 4.241 | 1.00 | 79.02 |
| 1780 | ND1 | HIS | A | 885 | -13.766 | 42.770 | 4.919 | 1.00 | 75.36 |
| 1781 | CD2 | HIS | A | 885 | -15.545 | 41.521 | 5.135 | 1.00 | 74.74 |
| 1782 | CE1 | HIS | A | 885 | -13.802 | 42.360 | 6.178 | 1.00 | 69.84 |
| 1783 | NE2 | HIS | A | 885 | -14.871 | 41.599 | 6.335 | 1.00 | 76.42 |
| 1784 | N | MET | A | 886 | -13.813 | 43.358 | 0.284 | 1.00 | 77.20 |
| 1785 | CA | MET | A | 886 | -13.780 | 44.065 | -1.003 | 1.00 | 75.99 |
| 1786 | C | MET | A | 886 | -12.804 | 43.540 | -2.083 | 1.00 | 66.55 |
| 1787 | O | MET | A | 886 | -12.844 | 44.000 | -3.225 | 1.00 | 63.97 |
| 1788 | CB | MET | A | 886 | -13.570 | 45.582 | -0.755 | 1.00 | 87.39 |
| 1789 | CG | MET | A | 886 | -12.135 | 46.127 | -0.979 | 1.00 | 94.48 |
| 1790 | SD | MET | A | 886 | -11.788 | 47.728 | -0.157 | 1.00 | 94.87 |
| 1791 | CE | MET | A | 886 | -10.212 | 48.246 | -1.001 | 1.00 | 94.14 |
| 1792 | N | VAL | A | 887 | -11.942 | 42.584 | -1.738 | 1.00 | 60.32 |
| 1793 | CA | VAL | A | 887 | -10.968 | 42.057 | -2.702 | 1.00 | 54.75 |
| 1794 | C | VAL | A | 887 | -11.146 | 40.564 | -3.026 | 1.00 | 56.80 |
| 1795 | O | VAL | A | 887 | -10.263 | 39.931 | -3.624 | 1.00 | 52.84 |
| 1796 | CB | VAL | A | 887 | -9.525 | 42.334 | -2.213 | 1.00 | 48.73 |
| 1797 | CG1 | VAL | A | 887 | -9.081 | 41.277 | -1.221 | 1.00 | 55.82 |
| 1798 | CG2 | VAL | A | 887 | -8.578 | 42.459 | -3.377 | 1.00 | 42.79 |
| 1799 | N | SER | A | 888 | -12.293 | 40.033 | -2.599 | 1.00 | 60.63 |
| 1800 | CA | SER | A | 888 | -12.752 | 38.643 | -2.786 | 1.00 | 60.83 |
| 1801 | C | SER | A | 888 | -11.912 | 37.401 | -2.406 | 1.00 | 65.27 |
| 1802 | O | SER | A | 888 | -12.196 | 36.292 | -2.885 | 1.00 | 62.05 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1803 | CB | SER | A | 888 | -13.368 | 38.463 | -4.169 | 1.00 | 51.52 |
| 1804 | OG | SER | A | 888 | -12.417 | 38.680 | -5.181 | 1.00 | 58.27 |
| 1805 | N | VAL | A | 889 | -10.918 | 37.579 | -1.529 | 1.00 | 70.51 |
| 1806 | CA | VAL | A | 889 | -10.079 | 36.475 | -1.036 | 1.00 | 69.77 |
| 1807 | C | VAL | A | 889 | -10.894 | 35.856 | 0.115 | 1.00 | 71.90 |
| 1808 | O | VAL | A | 889 | -11.475 | 36.609 | 0.910 | 1.00 | 70.16 |
| 1809 | CB | VAL | A | 889 | -8.740 | 37.003 | -0.461 | 1.00 | 68.58 |
| 1810 | CG1 | VAL | A | 889 | -7.880 | 35.843 | -0.016 | 1.00 | 73.27 |
| 1811 | CG2 | VAL | A | 889 | -8.006 | 37.859 | -1.489 | 1.00 | 65.97 |
| 1812 | N | ASP | A | 890 | -10.969 | 34.520 | 0.203 | 1.00 | 74.76 |
| 1813 | CA | ASP | A | 890 | -11.758 | 33.870 | 1.271 | 1.00 | 72.71 |
| 1814 | C | ASP | A | 890 | -10.920 | 33.314 | 2.457 | 1.00 | 69.55 |
| 1815 | O | ASP | A | 890 | -9.783 | 32.831 | 2.270 | 1.00 | 70.86 |
| 1816 | CB | ASP | A | 890 | -12.783 | 32.857 | 0.666 | 1.00 | 72.91 |
| 1817 | CG | ASP | A | 890 | -12.332 | 31.385 | 0.735 | 1.00 | 80.84 |
| 1818 | OD1 | ASP | A | 890 | -11.326 | 30.981 | 0.093 | 1.00 | 76.21 |
| 1819 | OD2 | ASP | A | 890 | -13.045 | 30.618 | 1.413 | 1.00 | 78.59 |
| 1820 | N | PHE | A | 891 | -11.431 | 33.508 | 3.680 | 1.00 | 59.70 |
| 1821 | CA | PHE | A | 891 | -10.746 | 33.068 | 4.902 | 1.00 | 52.91 |
| 1822 | C | PHE | A | 891 | -11.598 | 32.045 | 5.665 | 1.00 | 49.30 |
| 1823 | O | PHE | A | 891 | -12.740 | 32.326 | 6.012 | 1.00 | 50.65 |
| 1824 | CB | PHE | A | 891 | -10.474 | 34.274 | 5.848 | 1.00 | 55.80 |
| 1825 | CG | PHE | A | 891 | -9.223 | 35.088 | 5.518 | 1.00 | 47.41 |
| 1826 | CD1 | PHE | A | 891 | -9.250 | 36.101 | 4.554 | 1.00 | 40.14 |
| 1827 | CD2 | PHE | A | 891 | -8.027 | 34.837 | 6.171 | 1.00 | 45.78 |
| 1828 | CE1 | PHE | A | 891 | -8.107 | 36.842 | 4.246 | 1.00 | 34.67 |
| 1829 | CE2 | PHE | A | 891 | -6.876 | 35.585 | 5.862 | 1.00 | 46.05 |
| 1830 | CZ | PHE | A | 891 | -6.922 | 36.582 | 4.900 | 1.00 | 37.02 |
| 1831 | N | PRO | A | 892 | -11.052 | 30.845 | 5.926 | 1.00 | 44.99 |
| 1832 | CA | PRO | A | 892 | -11.782 | 29.806 | 6.660 | 1.00 | 39.76 |
| 1833 | C | PRO | A | 892 | -11.976 | 30.370 | 8.058 | 1.00 | 44.96 |
| 1834 | O | PRO | A | 892 | -11.052 | 30.952 | 8.621 | 1.00 | 46.87 |
| 1835 | CB | PRO | A | 892 | -10.792 | 28.648 | 6.669 | 1.00 | 45.27 |
| 1836 | CG | PRO | A | 892 | -9.970 | 28.878 | 5.420 | 1.00 | 46.05 |
| 1837 | CD | PRO | A | 892 | -9.737 | 30.353 | 5.480 | 1.00 | 46.20 |
| 1838 | N | GLU | A | 893 | -13.176 | 30.234 | 8.604 | 1.00 | 51.64 |
| 1839 | CA | GLU | A | 893 | -13.508 | 30.792 | 9.914 | 1.00 | 51.25 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1840 | C | GLU | A | 893 | -12.577 | 30.549 | 11.077 | 1.00 | 52.74 |
| 1841 | O | GLU | A | 893 | -12.321 | 31.462 | 11.858 | 1.00 | 58.39 |
| 1842 | CB | GLU | A | 893 | -14.937 | 30.437 | 10.304 | 1.00 | 59.05 |
| 1843 | CG | GLU | A | 893 | -15.987 | 31.401 | 9.724 | 1.00 | 75.65 |
| 1844 | CD | GLU | A | 893 | -15.800 | 31.699 | 8.224 | 1.00 | 82.95 |
| 1845 | OE1 | GLU | A | 893 | -15.706 | 30.745 | 7.418 | 1.00 | 84.06 |
| 1846 | OE2 | GLU | A | 893 | -15.762 | 32.891 | 7.845 | 1.00 | 86.37 |
| 1847 | N | MET | A | 894 | -12.043 | 29.348 | 11.205 | 1.00 | 52.94 |
| 1848 | CA | MET | A | 894 | -11.152 | 29.075 | 12.329 | 1.00 | 57.23 |
| 1849 | C | MET | A | 894 | -9.942 | 29.975 | 12.239 | 1.00 | 57.71 |
| 1850 | O | MET | A | 894 | -9.458 | 30.518 | 13.230 | 1.00 | 60.99 |
| 1851 | CB | MET | A | 894 | -10.720 | 27.607 | 12.332 | 1.00 | 59.88 |
| 1852 | CG | MET | A | 894 | -9.861 | 27.205 | 13.522 | 1.00 | 68.32 |
| 1853 | SD | MET | A | 894 | -9.861 | 25.428 | 13.821 | 1.00 | 73.67 |
| 1854 | CE | MET | A | 894 | -9.322 | 24.840 | 12.166 | 1.00 | 72.54 |
| 1855 | N | MET | A | 895 | -9.466 | 30.130 | 11.021 | 1.00 | 61.08 |
| 1856 | CA | MET | A | 895 | -8.311 | 30.948 | 10.744 | 1.00 | 58.05 |
| 1857 | C | MET | A | 895 | -8.679 | 32.431 | 10.789 | 1.00 | 56.79 |
| 1858 | O | MET | A | 895 | -7.979 | 33.226 | 11.422 | 1.00 | 56.34 |
| 1859 | CB | MET | A | 895 | -7.751 | 30.527 | 9.396 | 1.00 | 57.93 |
| 1860 | CG | MET | A | 895 | -6.936 | 31.548 | 8.720 | 1.00 | 67.27 |
| 1861 | SD | MET | A | 895 | -6.668 | 30.991 | 7.072 | 1.00 | 79.81 |
| 1862 | CE | MET | A | 895 | -4.870 | 31.161 | 6.982 | 1.00 | 78.99 |
| 1863 | N | ALA | A | 896 | -9.786 | 32.802 | 10.152 | 1.00 | 49.66 |
| 1864 | CA | ALA | A | 896 | -10.219 | 34.186 | 10.170 | 1.00 | 46.91 |
| 1865 | C | ALA | A | 896 | -10.211 | 34.621 | 11.627 | 1.00 | 50.19 |
| 1866 | O | ALA | A | 896 | -9.655 | 35.672 | 11.971 | 1.00 | 55.53 |
| 1867 | CB | ALA | A | 896 | -11.616 | 34.311 | 9.591 | 1.00 | 45.04 |
| 1868 | N | GLU | A | 897 | -10.737 | 33.759 | 12.496 | 1.00 | 52.29 |
| 1869 | CA | GLU | A | 897 | -10.805 | 34.053 | 13.928 | 1.00 | 52.77 |
| 1870 | C | GLU | A | 897 | -9.442 | 34.244 | 14.591 | 1.00 | 48.32 |
| 1871 | O | GLU | A | 897 | -9.253 | 35.144 | 15.402 | 1.00 | 49.52 |
| 1872 | CB | GLU | A | 897 | -11.612 | 32.990 | 14.664 | 1.00 | 54.94 |
| 1873 | CG | GLU | A | 897 | -11.927 | 33.355 | 16.111 | 1.00 | 65.44 |
| 1874 | CD | GLU | A | 897 | -13.217 | 32.725 | 16.600 | 1.00 | 75.27 |
| 1875 | OE1 | GLU | A | 897 | -14.284 | 33.102 | 16.074 | 1.00 | 78.93 |
| 1876 | OE2 | GLU | A | 897 | -13.167 | 31.868 | 17.511 1 | 1.00 | 79.28 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1877 | N | ILE | A | 898 | -8.477 | 33.417 | 14.241 | 1.00 | 45.91 |
| 1878 | CA | ILE | A | 898 | -7.160 | 33.561 | 14.836 | 1.00 | 43.63 |
| 1879 | C | ILE | A | 898 | -6.510 | 34.843 | 14.323 | 1.00 | 41.42 |
| 1880 | O | ILE | A | 898 | -5.891 | 35.578 | 15.087 | 1.00 | 41.91 |
| 1881 | CB | ILE | A | 898 | -6.275 | 32.288 | 14.567 | 1.00 | 45.82 |
| 1882 | CG1 | ILE | A | 898 | -6.797 | 31.125 | 15.430 | 1.00 | 48.14 |
| 1883 | CG2 | ILE | A | 898 | -4.776 | 32.537 | 14.847 | 1.00 | 34.18 |
| 1884 | CD1 | ILE | A | 898 | -5.911 | 29.894 | 15.425 | 1.00 | 60.11 |
| 1885 | N | ILE | A | 899 | -6.734 | 35.177 | 13.064 | 1.00 | 37.89 |
| 1886 | CA | ILE | A | 899 | -6.105 | 36.372 | 12.526 | 1.00 | 40.46 |
| 1887 | C | ILE | A | 899 | -6.640 | 37.624 | 13.202 | 1.00 | 39.91 |
| 1888 | O | ILE | A | 899 | -5.880 | 38.533 | 13.524 | 1.00 | 36.62 |
| 1889 | CB | ILE | A | 899 | -6.290 | 36.486 | 10.986 | 1.00 | 46.34 |
| 1890 | CG1 | ILE | A | 899 | -5.722 | 35.250 | 10.280 | 1.00 | 51.08 |
| 1891 | CG2 | ILE | A | 899 | -5.612 | 37.759 | 10.448 | 1.00 | 45.77 |
| 1892 | CD1 | ILE | A | 899 | -4.239 | 34.978 | 10.550 | 1.00 | 53.92 |
| 1893 | N | SER | A | 900 | -7.927 | 37.612 | 13.520 | 1.00 | 39.54 |
| 1894 | CA | SER | A | 900 | -8.576 | 38.764 | 14.129 | 1.00 | 39.63 |
| 1895 | C | SER | A | 900 | -8.474 | 38.925 | 15.652 | 1.00 | 41.36 |
| 1896 | O | SER | A | 900 | -8.821 | 39.970 | 16.188 | 1.00 | 46.90 |
| 1897 | CB | SER | A | 900 | -10.048 | 38.823 | 13.670 | 1.00 | 37.01 |
| 1898 | OG | SER | A | 900 | -10.963 | 38.377 | 14.652 | 1.00 | 40.29 |
| 1899 | N | VAL | A | 901 | -7.942 | 37.938 | 16.350 | 1.00 | 34.58 |
| 1900 | CA | VAL | A | 901 | -7.889 | 38.027 | 17.799 | 1.00 | 32.25 |
| 1901 | C | VAL | A | 901 | -6.516 | 37.812 | 18.388 | 1.00 | 33.93 |
| 1902 | O | VAL | A | 901 | -6.116 | 38.496 | 19.325 | 1.00 | 37.49 |
| 1903 | CB | VAL | A | 901 | -8.844 | 36.967 | 18.443 | 1.00 | 40.69 |
| 1904 | CG1 | VAL | A | 901 | -8.793 | 37.045 | 19.976 | 1.00 | 37.09 |
| 1905 | CG2 | VAL | A | 901 | -10.273 | 37.146 | 17.930 | 1.00 | 37.39 |
| 1906 | N | GLN | A | 902 | -5.813 | 36.820 | 17.865 | 1.00 | 38.32 |
| 1907 | CA | GLN | A | 902 | -4.504 | 36.475 | 18.376 | 1.00 | 36.30 |
| 1908 | C | GLN | A | 902 | -3.432 | 37.222 | 17.639 | 1.00 | 39.26 |
| 1909 | O | GLN | A | 902 | -2.419 | 37.566 | 18.234 | 1.00 | 41.22 |
| 1910 | CB | GLN | A | 902 | -4.247 | 34.958 | 18.276 | 1.00 | 36.78 |
| 1911 | CG | GLN | A | 902 | -5.309 | 34.053 | 18.926 | 1.00 | 41.09 |
| 1912 | CD | GLN | A | 902 | -5.529 | 34.320 | 20.418 | 1.00 | 47.88 |
| 1913 | OE1 | GLN | A | 902 | -6.664 | 34.277 | 20.899 | 1.00 | 56.15 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1914 | NE2 | GLN | A | 902 | -4.447 | 34.587 | 21.155 | 1.00 | 43.52 |
| 1915 | N | VAL | A | 903 | -3.621 | 37.449 | 16.340 | 1.00 | 40.86 |
| 1916 | CA | VAL | A | 903 | -2.611 | 38.167 | 15.569 | 1.00 | 38.57 |
| 1917 | C | VAL | A | 903 | -2.431 | 39.624 | 16.026 | 1.00 | 37.36 |
| 1918 | O | VAL | A | 903 | -1.294 | 40.077 | 16.171 | 1.00 | 39.48 |
| 1919 | CB | VAL | A | 903 | -2.841 | 38.035 | 14.028 | 1.00 | 42.29 |
| 1920 | CG1 | VAL | A | 903 | -1.947 | 38.994 | 13.270 | 1.00 | 39.28 |
| 1921 | CG2 | VAL | A | 903 | -2.503 | 36.622 | 13.574 | 1.00 | 39.72 |
| 1922 | N | PRO | A | 904 | -3.534 | 40.366 | 16.292 | 1.00 | 30.75 |
| 1923 | CA | PRO | A | 904 | -3.373 | 41.753 | 16.738 | 1.00 | 32.91 |
| 1924 | C | PRO | A | 904 | -2.436 | 41.878 | 17.945 | 1.00 | 36.72 |
| 1925 | O | PRO | A | 904 | -1.624 | 42.801 | 18.019 | 1.00 | 40.29 |
| 1926 | CB | PRO | A | 904 | -4.797 | 42.161 | 17.092 | 1.00 | 29.43 |
| 1927 | CG | PRO | A | 904 | -5.571 | 41.493 | 16.035 | 1.00 | 30.40 |
| 1928 | CD | PRO | A | 904 | -4.962 | 40.088 | 16.042 | 1.00 | 34.37 |
| 1929 | N | LYS | A | 905 | -2.489 | 40.909 | 18.851 | 1.00 | 36.01 |
| 1930 | CA | LYS | A | 905 | -1.632 | 40.937 | 20.031 | 1.00 | 32.07 |
| 1931 | C | LYS | A | 905 | -0.149 | 40.868 | 19.677 | 1.00 | 30.44 |
| 1932 | O | LYS | A | 905 | 0.688 | 41.365 | 20.422 | 1.00 | 33.55 |
| 1933 | CB | LYS | A | 905 | -1.995 | 39.807 | 21.022 | 1.00 | 34.01 |
| 1934 | CG | LYS | A | 905 | -3.485 | 39.698 | 21.377 | 1.00 | 37.81 |
| 1935 | CD | LYS | A | 905 | -3.751 | 38.653 | 22.476 | 1.00 | 51.29 |
| 1936 | CE | LYS | A | 905 | -5.233 | 38.624 | 22.886 | 1.00 | 51.15 |
| 1937 | NZ | LYS | A | 905 | -5.552 | 37.658 | 23.987 | 1.00 | 63.78 |
| 1938 | N | ILE | A | 906 | 0.185 | 40.258 | 18.545 | 1.00 | 38.99 |
| 1939 | CA | ILE | A | 906 | 1.588 | 40.103 | 18.142 | 1.00 | 43.43 |
| 1940 | C | ILE | A | 906 | 2.076 | 41.353 | 17.449 | 1.00 | 46.59 |
| 1941 | O | ILE | A | 906 | 3.203 | 41.817 | 17.688 | 1.00 | 46.25 |
| 1942 | CB | ILE | A | 906 | 1.818 | 38.828 | 17.217 | 1.00 | 41.03 |
| 1943 | CG1 | ILE | A | 906 | 1.453 | 37.545 | 17.993 | 1.00 | 41.68 |
| 1944 | CG2 | ILE | A | 906 | 3.289 | 38.764 | 16.745 | 1.00 | 35.00 |
| 1945 | CD1 | ILE | A | 906 | 1.386 | 36.270 | 17.189 | 1.00 | 36.91 |
| 1946 | N | LEU | A | 907 | 1.217 | 41.891 | 16.587 | 1.00 | 50.54 |
| 1947 | CA | LEU | A | 907 | 1.516 | 43.111 | 15.831 | 1.00 | 47.53 |
| 1948 | C | LEU | A | 907 | 1.639 | 44.317 | 16.784 | 1.00 | 42.06 |
| 1949 | O | LEU | A | 907 | 2.496 | 45.199 | 16.590 | 1.00 | 41.92 |
| 1950 | CB | LEU | A | 907 | 0.437 | 43.324 | 14.751 | 1.00 | 43.43 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1951 | CG | LEU | A | 907 | 0.323 | 42.131 | 13.782 | 1.00 | 31.27 |
| 1952 | CD1 | LEU | A | 907 | -0.857 | 42.290 | 12.901 | 1.00 | 29.15 |
| 1953 | CD2 | LEU | A | 907 | 1.580 | 41.967 | 12.953 | 1.00 | 29.66 |
| 1954 | N | SER | A | 908 | 0.851 | 44.282 | 17.858 | 1.00 | 36.02 |
| 1955 | CA | SER | A | 908 | 0.871 | 45.328 | 18.852 | 1.00 | 41.77 |
| 1956 | C | SER | A | 908 | 1.943 | 45.131 | 19.931 | 1.00 | 45.83 |
| 1957 | O | SER | A | 908 | 2.115 | 45.985 | 20.788 | 1.00 | 57.61 |
| 1958 | CB | SER | A | 908 | -0.519 | 45.531 | 19.479 | 1.00 | 44.15 |
| 1959 | OG | SER | A | 908 | -0.913 | 44.474 | 20.334 | 1.00 | 47.22 |
| 1960 | N | GLY | A | 909 | 2.684 | 44.030 | 19.886 | 1.00 | 40.63 |
| 1961 | CA | GLY | A | 909 | 3.726 | 43.813 | 20.877 | 1.00 | 31.68 |
| 1962 | C | GLY | A | 909 | 3.324 | 43.227 | 22.233 | 1.00 | 37.71 |
| 1963 | O | GLY | A | 909 | 4.173 | 43.163 | 23.129 | 1.00 | 42.65 |
| 1964 | N | LYS | A | 910 | 2.071 | 42.791 | 22.401 | 1.00 | 33.50 |
| 1965 | CA | LYS | A | 910 | 1.619 | 42.182 | 23.673 | 1.00 | 42.03 |
| 1966 | C | LYS | A | 910 | 2.064 | 40.702 | 23.839 | 1.00 | 41.10 |
| 1967 | O | LYS | A | 910 | 2.036 | 40.137 | 24.932 | 1.00 | 39.99 |
| 1968 | CB | LYS | A | 910 | 0.100 | 42.320 | 23.805 | 1.00 | 41.10 |
| 1969 | CG | LYS | A | 910 | -0.357 | 43.751 | 23.582 | 1.00 | 45.52 |
| 1970 | CD | LYS | A | 910 | -1.830 | 43.904 | 23.726 | 1.00 | 40.97 |
| 1971 | CE | LYS | A | 910 | -2.190 | 43.976 | 25.163 | 1.00 | 42.32 |
| 1972 | NZ | LYS | A | 910 | -3.651 | 43.819 | 25.260 | 1.00 | 53.43 |
| 1973 | N | VAL | A | 911 | 2.497 | 40.114 | 22.728 | 1.00 | 42.05 |
| 1974 | CA | VAL | A | 911 | 2.992 | 38.746 | 22.624 | 1.00 | 35.21 |
| 1975 | C | VAL | A | 911 | 4.267 | 38.912 | 21.806 | 1.00 | 36.16 |
| 1976 | O | VAL | A | 911 | 4.224 | 39.387 | 20.671 | 1.00 | 37.87 |
| 1977 | CB | VAL | A | 911 | 2.025 | 37.872 | 21.822 | 1.00 | 29.65 |
| 1978 | CG1 | VAL | A | 911 | 2.661 | 36.551 | 21.476 | 1.00 | 33.30 |
| 1979 | CG2 | VAL | A | 911 | 0.736 | 37.674 | 22.588 | 1.00 | 35.10 |
| 1980 | N | LYS | A | 912 | 5.396 | 38.504 | 22.358 | 1.00 | 35.80 |
| 1981 | CA | LYS | A | 912 | 6.638 | 38.677 | 21.653 | 1.00 | 36.47 |
| 1982 | C | LYS | A | 912 | 7.451 | 37.409 | 21.556 | 1.00 | 40.23 |
| 1983 | O | LYS | A | 912 | 7.293 | 36.472 | 22.334 | 1.00 | 39.68 |
| 1984 | CB | LYS | A | 912 | 7.483 | 39.752 | 22.347 | 1.00 | 37.13 |
| 1985 | CG | LYS | A | 912 | 7.952 | 39.353 | 23.729 | 0.00 | 38.33 |
| 1986 | CD | LYS | A | 912 | 8.846 | 40.405 | 24.332 | 0.00 | 39.07 |
| 1987 | CE | LYS | A | 912 | 9.124 | 40.089 | 25.784 | 0.00 | 39.77 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 1988 | NZ | LYS | A | 912 | 9.862 | 41.200 | 26.423 | 0.00 | 40.28 |
| 1989 | N | PRO | A | 913 | 8.266 | 37.333 | 20.517 | 1.00 | 39.61 |
| 1990 | CA | PRO | A | 913 | 9.146 | 36.208 | 20.249 | 1.00 | 40.14 |
| 1991 | C | PRO | A | 913 | 10.247 | 36.181 | 21.301 | 1.00 | 36.50 |
| 1992 | O | PRO | A | 913 | 10.565 | 37.200 | 21.893 | 1.00 | 38.67 |
| 1993 | CB | PRO | A | 913 | 9.711 | 36.564 | 18.875 | 1.00 | 41.93 |
| 1994 | CG | PRO | A | 913 | 8.534 | 37.200 | 18.210 | 1.00 | 44.72 |
| 1995 | CD | PRO | A | 913 | 8.060 | 38.134 | 19.296 | 1.00 | 42.22 |
| 1996 | N | ILE | A | 914 | 10.813 | 35.008 | 21.538 | 1.00 | 34.68 |
| 1997 | CA | ILE | A | 914 | 11.883 | 34.848 | 22.497 | 1.00 | 36.82 |
| 1998 | C | ILE | A | 914 | 13.085 | 34.508 | 21.654 | 1.00 | 40.11 |
| 1999 | O | ILE | A | 914 | 13.129 | 33.460 | 21.029 | 1.00 | 43.44 |
| 2000 | CB | ILE | A | 914 | 11.625 | 33.663 | 23.434 | 1.00 | 38.93 |
| 2001 | CG1 | ILE | A | 914 | 10.311 | 33.834 | 24.173 | 1.00 | 32.49 |
| 2002 | CG2 | ILE | A | 914 | 12.743 | 33.540 | 24.435 | 1.00 | 46.36 |
| 2003 | CD1 | ILE | A | 914 | 9.917 | 32.567 | 24.899 | 1.00 | 39.70 |
| 2004 | N | TYR | A | 915 | 14.047 | 35.409 | 21.603 | 1.00 | 45.62 |
| 2005 | CA | TYR | A | 915 | 15.235 | 35.191 | 20.798 | 1.00 | 45.53 |
| 2006 | C | TYR | A | 915 | 16.352 | 34.582 | 21.608 | 1.00 | 47.03 |
| 2007 | O | TYR | A | 915 | 16.359 | 34.665 | 22.833 | 1.00 | 51.25 |
| 2008 | CB | TYR | A | 915 | 15.717 | 36.510 | 20.165 | 1.00 | 38.58 |
| 2009 | CG | TYR | A | 915 | 14.778 | 37.052 | 19.122 | 1.00 | 36.78 |
| 2010 | CD1 | TYR | A | 915 | 13.600 | 37.695 | 19.484 | 1.00 | 38.75 |
| 2011 | CD2 | TYR | A | 915 | 15.042 | 36.875 | 17.767 | 1.00 | 47.40 |
| 2012 | CE1 | TYR | A | 915 | 12.696 | 38.141 | 18.527 | 1.00 | 49.22 |
| 2013 | CE2 | TYR | A | 915 | 14.142 | 37.317 | 16.786 | 1.00 | 52.13 |
| 2014 | CZ | TYR | A | 915 | 12.969 | 37.952 | 17.175 | 1.00 | 56.55 |
| 2015 | OH | TYR | A | 915 | 12.067 | 38.377 | 16.212 | 1.00 | 55.66 |
| 2016 | N | PHE | A | 916 | 17.298 | 33.964 | 20.915 | 1.00 | 46.26 |
| 2017 | CA | PHE | A | 916 | 18.439 | 33.376 | 21.580 | 1.00 | 44.65 |
| 2018 | C | PHE | A | 916 | 19.487 | 34.457 | 21.707 | 1.00 | 44.05 |
| 2019 | O | PHE | A | 916 | 20.132 | 34.568 | 22.738 | 1.00 | 47.61 |
| 2020 | CB | PHE | A | 916 | 18.993 | 32.180 | 20.787 | 1.00 | 42.46 |
| 2021 | CG | PHE | A | 916 | 18.213 | 30.915 | 20.991 | 1.00 | 44.06 |
| 2022 | CD1 | PHE | A | 916 | 17.006 | 30.705 | 20.326 | 1.00 | 46.38 |
| 2023 | CD2 | PHE | A | 916 | 18.670 | 29.948 | 21.875 | 1.00 | 47.18 |
| 2024 | CE1 | PHE | A | 916 | 16.263 | 29.553 | 20.540 | 1.00 | 45.51 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 2025 | CE2 | PHE | A | 916 | 17.939 | 28.789 | 22.098 | 1.00 | 46.95 |
| 2026 | CZ | PHE | A | 916 | 16.732 | 28.594 | 21.426 | 1.00 | 45.10 |
| 2027 | N | HIS | A | 917 | 19.603 | 35.303 | 20.689 | 1.00 | 46.97 |
| 2028 | CA | HIS | A | 917 | 20.611 | 36.352 | 20.694 | 1.00 | 47.06 |
| 2029 | C | HIS | A | 917 | 20.043 | 37.777 | 20.743 | 1.00 | 52.16 |
| 2030 | O | HIS | A | 917 | 20.353 | 38.603 | 19.896 | 1.00 | 57.53 |
| 2031 | CB | HIS | A | 917 | 21.524 | 36.170 | 19.471 | 1.00 | 49.03 |
| 2032 | CG | HIS | A | 917 | 21.994 | 34.756 | 19.246 | 1.00 | 43.80 |
| 2033 | ND1 | HIS | A | 917 | 23.287 | 34.340 | 19.494 | 1.00 | 47.63 |
| 2034 | CD2 | HIS | A | 917 | 21.343 | 33.670 | 18.762 | 1.00 | 48.20 |
| 2035 | CE1 | HIS | A | 917 | 23.414 | 33.065 | 19.173 | 1.00 | 51.22 |
| 2036 | NE2 | HIS | A | 917 | 22.248 | 32.631 | 18.726 | 1.00 | 47.78 |
| 2037 | N | THR | A | 918 | 19.206 | 38.052 | 21.735 | 1.00 | 62.03 |
| 2038 | CA | THR | A | 918 | 18.597 | 39.381 | 21.917 | 1.00 | 72.18 |
| 2039 | C | THR | A | 918 | 19.558 | 40.395 | 22.571 | 1.00 | 75.33 |
| 2040 | O | THR | A | 918 | 19.150 | 41.565 | 22.781 | 1.00 | 78.71 |
| 2041 | CB | THR | A | 918 | 17.378 | 39.309 | 22.847 | 1.00 | 73.07 |
| 2042 | OG1 | THR | A | 918 | 16.840 | 37.980 | 22.842 | 1.00 | 72.53 |
| 2043 | CG2 | THR | A | 918 | 16.301 | 40.339 | 22.419 | 1.00 | 76.16 |
| 2044 | OXT | THR | A | 918 | 20.674 | 39.997 | 22.964 | 1.00 | 76.97 |
| 2045 | | THR | A | 918 | | | | | |
| 2046 | C1 | R18 | A | 1000 | 0.414 | 28.070 | 4.103 | 1.00 | 47.66 |
| 2047 | C2 | R18 | A | 1000 | 1.195 | 26.999 | 4.832 | 1.00 | 49.34 |
| 2048 | C3 | R18 | A | 1000 | 2.661 | 27.140 | 4.532 | 1.00 | 53.90 |
| 2049 | C4 | R18 | A | 1000 | 3.174 | 28.457 | 4.794 | 1.00 | 55.05 |
| 2050 | C5 | R18 | A | 1000 | 2.367 | 29.553 | 4.780 | 1.00 | 50.29 |
| 2051 | C6 | R18 | A | 1000 | 2.973 | 30.906 | 5.116 | 1.00 | 47.48 |
| 2052 | C7 | R18 | A | 1000 | 2.207 | 32.030 | 4.457 | 1.00 | 46.11 |
| 2053 | C8 | R18 | A | 1000 | 0.733 | 31.962 | 4.898 | 1.00 | 45.61 |
| 2054 | C9 | R18 | A | 1000 | 0.124 | 30.597 | 4.514 | 1.00 | 49.94 |
| 2055 | C10 | R18 | A | 1000 | 0.912 | 29.480 | 4.476 | 1.00 | 49.84 |
| 2056 | C11 | R18 | A | 1000 | -1.316 | 30.583 | 4.251 | 1.00 | 47.61 |
| 2057 | C12 | R18 | A | 1000 | -2.102 | 31.675 | 4.310 | 1.00 | 47.26 |
| 2058 | C13 | R18 | A | 1000 | -1.535 | 33.039 | 4.664 | 1.00 | 44.26 |
| 2059 | C14 | R18 | A | 1000 | -0.056 | 33.066 | 4.261 | 1.00 | 42.93 |
| 2060 | C15 | R18 | A | 1000 | 0.387 | 34.509 | 4.572 | 1.00 | 43.22 |
| 2061 | C16 | R18 | A | 1000 | -0.899 | 35.299 | 4.311 | 1.00 | 41.50 |

TABLE 9 (continued)

| THREE-DIMENSIONAL COORDINATES OF AR IN COMPLEX WITH THE LIGAND R1881 | | | | | | | | | |
|---|---|---|---|---|---|---|---|---|---|
| ATOM | ATOM TYPE | RESIDUE | # | X | Y | Z | OCC | B | ATOM |
| 2062 | C17 | R18 | A | 1000 | -2.001 | 34.282 | 3.900 | 1.00 | 43.39 |
| 2063 | C18 | R18 | A | 1000 | -1.725 | 33.228 | 6.189 | 1.00 | 41.74 |
| 2064 | C27 | R18 | A | 1000 | -2.034 | 34.050 | 2.412 | 1.00 | 40.38 |
| 2065 | 083 | R18 | A | 1000 | 3.375 | 26.212 | 4.162 | 1.00 | 59.41 |
| 2066 | 097 | R18 | A | 1000 | -3.257 | 34.797 | 4.345 | 1.00 | 48.46 |
| 2067 | O | HOH | Z | 1 | 17.517 | 11.963 | 20.575 | 1.00 | 51.58 |
| 2068 | O | HOH | Z | 2 | 7.977 | 16.353 | 14.548 | 1.00 | 29.86 |
| 2069 | O | HOH | Z | 3 | 3.112 | 18.553 | 3.147 | 1.00 | 65.78 |
| 2070 | O | HOH | Z | 4 | 1.314 | 20.835 | 2.312 | 1.00 | 46.40 |
| 2071 | O | HOH | Z | 5 | 0.422 | 24.646 | -12.882 | 1.00 | 58.80 |
| 2072 | O | HOH | Z | 6 | -1.370 | 30.527 | -15.016 | 1.00 | 41.87 |
| 2073 | O | HOH | Z | 7 | -4.737 | 36.972 | -13.426 | 1.00 | 75.71 |
| 2074 | O | HOH | Z | 8 | 4.151 | 24.024 | 6.333 | 1.00 | 32.89 |
| 2075 | O | HOH | Z | 9 | -7.536 | 14.518 | 23.118 | 1.00 | 42.77 |
| 2076 | O | HOH | Z | 10 | 1.368 | 26.376 | 31.674 | 1.00 | 49.63 |
| 2077 | O | HOH | Z | 11 | 1.207 | 32.439 | 14.426 | 1.00 | 45.58 |
| 2078 | O | HOH | Z | 12 | 4.179 | 32.582 | 14.418 | 1.00 | 31.77 |
| 2079 | O | HOH | Z | 13 | -5.348 | 34.883 | 24.137 | 1.00 | 62.76 |
| 2080 | O | HOH | Z | 14 | 2.739 | 25.007 | -10.984 | 1.00 | 53.83 |
| 2081 | O | HOH | Z | 15 | 10.790 | 29.074 | 3.632 | 1.00 | 61.00 |
| 2082 | O | HOH | Z | 16 | 18.090 | 34.834 | 6.262 | 1.00 | 66.21 |
| 2083 | O | HOH | Z | 17 | 28.211 | 24.615 | 5.938 | 1.00 | 56.69 |
| 2084 | O | HOH | Z | 18 | 5.741 | 29.774 | 22.458 | 1.00 | 37.52 |
| 2085 | O | HOH | Z | 19 | 12.529 | 31.030 | 16.979 | 1.00 | 35.29 |
| 2086 | O | HOH | Z | 20 | 7.674 | 37.499 | 32.487 | 1.00 | 46.92 |
| 2087 | O | HOH | Z | 21 | 26.453 | 8.689 | 17.052 | 1.00 | 48.49 |
| 2088 | O | HOH | Z | 22 | 7.032 | 36.581 | 14.890 | 1.00 | 40.54 |
| 2089 | O | HOH | Z | 23 | 6.659 | 32.126 | 15.319 | 1.00 | 44.19 |
| 2090 | O | HOH | Z | 24 | 2.693 | 46.635 | 13.278 | 1.00 | 46.55 |
| 2091 | O | HOH | Z | 25 | 5.869 | 40.798 | 18.893 | 1.00 | 48.79 |
| 2092 | O | HOH | Z | 26 | 16.821 | 36.244 | 25.158 | 1.00 | 58.36 |

TABLE 10

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1 | N | GLN | A | 682 | 31.180 | -1.959 | 93.866 | 1.00 | 69.36 |
| 2 | CA | GLN | A | 682 | 32.157 | -2.958 | 94.388 | 1.00 | 66.54 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3 | C | GLN | A | 682 | 33.310 | -3.159 | 93.410 | 1.00 | 66.47 |
| 4 | O | GLN | A | 682 | 34.414 | -2.653 | 93.627 | 1.00 | 66.82 |
| 5 | CB | GLN | A | 682 | 31.453 | -4.285 | 94.646 | 1.00 | 68.47 |
| 6 | N | LEU | A | 683 | 33.053 | -3.900 | 92.336 | 1.00 | 63.30 |
| 7 | CA | LEU | A | 683 | 34.077 | -4.153 | 91.334 | 1.00 | 60.85 |
| 8 | C | LEU | A | 683 | 34.028 | -3.134 | 90.197 | 1.00 | 56.94 |
| 9 | O | LEU | A | 683 | 35.057 | -2.832 | 89.590 | 1.00 | 54.60 |
| 10 | CB | LEU | A | 683 | 33.941 | -5.573 | 90.767 | 1.00 | 61.13 |
| 11 | CG | LEU | A | 683 | 34.171 | -6.734 | 91.743 | 1.00 | 66.50 |
| 12 | CD1 | LEU | A | 683 | 34.044 | -8.065 | 91.003 | 1.00 | 60.00 |
| 13 | CD2 | LEU | A | 683 | 35.555 | -6.617 | 92.370 | 1.00 | 59.53 |
| 14 | N | ILE | A | 684 | 32.839 | -2.607 | 89.911 | 1.00 | 52.97 |
| 15 | CA | ILE | A | 684 | 32.696 | -1.624 | 88.844 | 1.00 | 48.69 |
| 16 | C | ILE | A | 684 | 33.249 | -0.282 | 89.320 | 1.00 | 46.25 |
| 17 | O | ILE | A | 684 | 32.771 | 0.276 | 90.302 | 1.00 | 41.04 |
| 18 | CB | ILE | A | 684 | 31.219 | -1.426 | 88.437 | 1.00 | 52.18 |
| 19 | CG1 | ILE | A | 684 | 30.594 | -2.764 | 88.029 | 1.00 | 50.50 |
| 20 | CG2 | ILE | A | 684 | 31.137 | -0.448 | 87.269 | 1.00 | 48.24 |
| 21 | CD1 | ILE | A | 684 | 31.258 | -3.423 | 86.839 | 1.00 | 51.57 |
| 22 | N | PRO | A | 685 | 34.270 | 0.250 | 88.628 | 1.00 | 40.66 |
| 23 | CA | PRO | A | 685 | 34.874 | 1.532 | 89.001 | 1.00 | 39.82 |
| 24 | C | PRO | A | 685 | 33.846 | 2.665 | 89.084 | 1.00 | 36.13 |
| 25 | O | PRO | A | 685 | 32.885 | 2.697 | 88.319 | 1.00 | 35.64 |
| 26 | CB | PRO | A | 685 | 35.913 | 1.743 | 87.901 | 1.00 | 34.22 |
| 27 | CG | PRO | A | 685 | 36.328 | 0.330 | 87.591 | 1.00 | 43.36 |
| 28 | CD | PRO | A | 685 | 34.951 | -0.290 | 87.441 | 1.00 | 40.38 |
| 29 | N | PRO | A | 686 | 34.049 | 3.610 | 90.013 | 1.00 | 35.29 |
| 30 | CA | PRO | A | 686 | 33.161 | 4.756 | 90.234 | 1.00 | 35.12 |
| 31 | C | PRO | A | 686 | 32.856 | 5.569 | 88.978 | 1.00 | 30.01 |
| 32 | O | PRO | A | 686 | 31.698 | 5.883 | 88.696 | 1.00 | 29.64 |
| 33 | CB | PRO | A | 686 | 33.923 | 5.573 | 91.275 | 1.00 | 36.34 |
| 34 | CG | PRO | A | 686 | 34.626 | 4.484 | 92.076 | 1.00 | 41.25 |
| 35 | CD | PRO | A | 686 | 35.210 | 3.700 | 90.919 | 1.00 | 39.57 |
| 36 | N | LEU | A | 687 | 33.901 | 5.907 | 88.231 | 1.00 | 29.04 |
| 37 | CA | LEU | A | 687 | 33.729 | 6.701 | 87.013 | 1.00 | 28.23 |
| 38 | C | LEU | A | 687 | 32.874 | 5.982 | 85.970 | 1.00 | 26.62 |
| 39 | O | LEU | A | 687 | 32.096 | 6.607 | 85.240 | 1.00 | 25.82 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 40 | CB | LEU | A | 687 | 35.086 | 7.048 | 86.422 | 1.00 | 25.68 |
| 41 | CG | LEU | A | 687 | 35.023 | 7.915 | 85.165 | 1.00 | 34.63 |
| 42 | CD1 | LEU | A | 687 | 34.104 | 9.118 | 85.408 | 1.00 | 30.55 |
| 43 | CD2 | LEU | A | 687 | 36.436 | 8.348 | 84.790 | 1.00 | 30.65 |
| 44 | N | ILE | A | 688 | 33.015 | 4.663 | 85.898 | 1.00 | 27.06 |
| 45 | CA | ILE | A | 688 | 32.226 | 3.891 | 84.951 | 1.00 | 23.18 |
| 46 | C | ILE | A | 688 | 30.762 | 3.860 | 85.377 | 1.00 | 24.05 |
| 47 | O | ILE | A | 688 | 29.859 | 3.990 | 84.545 | 1.00 | 26.77 |
| 48 | CB | ILE | A | 688 | 32.788 | 2.477 | 84.815 | 1.00 | 23.51 |
| 49 | CG1 | ILE | A | 688 | 34.173 | 2.557 | 84.174 | 1.00 | 26.92 |
| 50 | CG2 | ILE | A | 688 | 31.848 | 1.616 | 83.978 | 1.00 | 28.19 |
| 51 | CD1 | ILE | A | 688 | 34.884 | 1.237 | 84.088 | 1.00 | 31.87 |
| 52 | N | ASN | A | 689 | 30.505 | 3.704 | 86.674 | 1.00 | 25.92 |
| 53 | CA | ASN | A | 689 | 29.118 | 3.713 | 87.138 | 1.00 | 27.71 |
| 54 | C | ASN | A | 689 | 28.513 | 5.088 | 86.837 | 1.00 | 22.00 |
| 55 | O | ASN | A | 689 | 27.343 | 5.195 | 86.472 | 1.00 | 26.26 |
| 56 | CB | ASN | A | 689 | 29.050 | 3.436 | 88.645 | 1.00 | 31.49 |
| 57 | CG | ASN | A | 689 | 29.163 | 1.966 | 88.972 | 1.00 | 38.87 |
| 58 | OD1 | ASN | A | 689 | 29.561 | 1.593 | 90.081 | 1.00 | 46.19 |
| 59 | ND2 | ASN | A | 689 | 28.778 | 1.116 | 88.023 | 1.00 | 36.32 |
| 60 | N | LEU | A | 690 | 29.313 | 6.139 | 86.983 | 1.00 | 26.88 |
| 61 | CA | LEU | A | 690 | 28.811 | 7.491 | 86.715 | 1.00 | 25.43 |
| 62 | C | LEU | A | 690 | 28.445 | 7.621 | 85.236 | 1.00 | 27.05 |
| 63 | O | LEU | A | 690 | 27.384 | 8.139 | 84.895 | 1.00 | 26.07 |
| 64 | CB | LEU | A | 690 | 29.860 | 8.528 | 87.112 | 1.00 | 26.35 |
| 65 | CG | LEU | A | 690 | 29.488 | 10.012 | 86.976 | 1.00 | 35.60 |
| 66 | CD1 | LEU | A | 690 | 30.409 | 10.836 | 87.880 | 1.00 | 36.71 |
| 67 | CD2 | LEU | A | 690 | 29.595 | 10.475 | 85.522 | 1.00 | 30.46 |
| 68 | N | LEU | A | 691 | 29.309 | 7.123 | 84.360 | 1.00 | 25.73 |
| 69 | CA | LEU | A | 691 | 29.038 | 7.174 | 82.922 | 1.00 | 23.64 |
| 70 | C | LEU | A | 691 | 27.766 | 6.400 | 82.595 | 1.00 | 27.62 |
| 71 | O | LEU | A | 691 | 26.990 | 6.810 | 81.738 | 1.00 | 22.81 |
| 72 | CB | LEU | A | 691 | 30.209 | 6.586 | 82.128 | 1.00 | 24.64 |
| 73 | CG | LEU | A | 691 | 31.565 | 7.295 | 82.220 | 1.00 | 25.70 |
| 74 | CD1 | LEU | A | 691 | 32.610 | 6.538 | 81.381 | 1.00 | 21.63 |
| 75 | CD2 | LEU | A | 691 | 31.422 | 8.739 | 81.722 | 1.00 | 20.83 |
| 76 | N | MET | A | 692 | 27.562 | 5.259 | 83.253 | 1.00 | 24.62 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 77 | CA | MET | A | 692 | 26.351 | 4.476 | 83.019 | 1.00 | 27.60 |
| 78 | C | MET | A | 692 | 25.156 | 5.337 | 83.440 | 1.00 | 27.94 |
| 79 | O | MET | A | 692 | 24.145 | 5.416 | 82.745 | 1.00 | 25.86 |
| 80 | CB | MET | A | 692 | 26.385 | 3.195 | 83.860 | 1.00 | 27.45 |
| 81 | CG | MET | A | 692 | 25.197 | 2.289 | 83.686 | 1.00 | 39.52 |
| 82 | SD | MET | A | 692 | 25.017 | 1.642 | 82.004 | 1.00 | 51.06 |
| 83 | CE | MET | A | 692 | 24.268 | 3.029 | 81.134 | 1.00 | 52.36 |
| 84 | N | SER | A | 693 | 25.296 | 6.010 | 84.574 | 1.00 | 25.24 |
| 85 | CA | SER | A | 693 | 24.216 | 6.835 | 85.083 | 1.00 | 31.97 |
| 86 | C | SER | A | 693 | 23.878 | 8.044 | 84.219 | 1.00 | 29.88 |
| 87 | O | SER | A | 693 | 22.719 | 8.455 | 84.157 | 1.00 | 28.14 |
| 88 | CB | SER | A | 693 | 24.531 | 7.313 | 86.508 | 1.00 | 38.05 |
| 89 | OG | SER | A | 693 | 25.623 | 8.222 | 86.526 | 1.00 | 43.01 |
| 90 | N | ILE | A | 694 | 24.865 | 8.625 | 83.547 | 1.00 | 25.23 |
| 91 | CA | ILE | A | 694 | 24.553 | 9.808 | 82.741 | 1.00 | 26.22 |
| 92 | C | ILE | A | 694 | 24.257 | 9.520 | 81.279 | 1.00 | 23.06 |
| 93 | O | ILE | A | 694 | 24.031 | 10.442 | 80.504 | 1.00 | 24.41 |
| 94 | CB | ILE | A | 694 | 25.669 | 10.875 | 82.813 | 1.00 | 22.83 |
| 95 | CG1 | ILE | A | 694 | 26.984 | 10.307 | 82.265 | 1.00 | 22.20 |
| 96 | CG2 | ILE | A | 694 | 25.849 | 11.338 | 84.270 | 1.00 | 28.20 |
| 97 | CD1 | ILE | A | 694 | 28.060 | 11.373 | 82.014 | 1.00 | 22.62 |
| 98 | N | GLU | A | 695 | 24.257 | 8.242 | 80.899 | 1.00 | 26.89 |
| 99 | CA | GLU | A | 695 | 23.969 | 7.876 | 79.517 | 1.00 | 21.93 |
| 100 | C | GLU | A | 695 | 22.511 | 8.296 | 79.289 | 1.00 | 28.96 |
| 101 | O | GLU | A | 695 | 21.632 | 7.992 | 80.087 | 1.00 | 29.21 |
| 102 | CB | GLU | A | 695 | 24.150 | 6.362 | 79.338 | 1.00 | 34.17 |
| 103 | CG | GLU | A | 695 | 24.063 | 5.848 | 77.911 | 1.00 | 34.86 |
| 104 | CD | GLU | A | 695 | 25.240 | 6.232 | 77.021 | 1.00 | 45.46 |
| 105 | OE1 | GLU | A | 695 | 26.126 | 7.019 | 77.436 | 1.00 | 31.19 |
| 106 | OE2 | GLU | A | 695 | 25.275 | 5.730 | 75.873 | 1.00 | 49.30 |
| 107 | N | PRO | A | 696 | 22.242 | 9.037 | 78.215 | 1.00 | 32.33 |
| 108 | CA | PRO | A | 696 | 20.865 | 9.469 | 77.961 | 1.00 | 34.70 |
| 109 | C | PRO | A | 696 | 19.862 | 8.330 | 77.764 | 1.00 | 30.39 |
| 110 | O | PRO | A | 696 | 20.232 | 7.235 | 77.371 | 1.00 | 27.63 |
| 111 | CB | PRO | A | 696 | 21.020 | 10.333 | 76.710 | 1.00 | 38.27 |
| 112 | CG | PRO | A | 696 | 22.198 | 9.652 | 75.997 | 1.00 | 40.07 |
| 113 | CD | PRO | A | 696 | 23.141 | 9.561 | 77.173 | 1.00 | 34.72 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 114 | N | ASP | A | 697 | 18.588 | 8.613 | 78.037 | 1.00 | 38.27 |
| 115 | CA | ASP | A | 697 | 17.516 | 7.632 | 77.863 | 1.00 | 35.46 |
| 116 | C | ASP | A | 697 | 17.341 | 7.513 | 76.340 | 1.00 | 35.96 |
| 117 | O | ASP | A | 697 | 17.600 | 8.468 | 75.620 | 1.00 | 30.43 |
| 118 | CB | ASP | A | 697 | 16.238 | 8.147 | 78.523 | 1.00 | 44.25 |
| 119 | CG | ASP | A | 697 | 15.176 | 7.069 | 78.683 | 1.00 | 53.62 |
| 120 | OD1 | ASP | A | 697 | 15.420 | 5.901 | 78.302 | 1.00 | 57.04 |
| 121 | OD2 | ASP | A | 697 | 14.085 | 7.398 | 79.203 | 1.00 | 61.97 |
| 122 | N | VAL | A | 698 | 16.909 | 6.359 | 75.841 | 1.00 | 32.80 |
| 123 | CA | VAL | A | 698 | 16.766 | 6.195 | 74.393 | 1.00 | 34.66 |
| 124 | C | VAL | A | 698 | 15.941 | 7.312 | 73.736 | 1.00 | 28.44 |
| 125 | O | VAL | A | 698 | 14.937 | 7.775 | 74.266 | 1.00 | 30.11 |
| 126 | CB | VAL | A | 698 | 16.153 | 4.813 | 74.026 | 1.00 | 41.68 |
| 127 | CG1 | VAL | A | 698 | 14.649 | 4.830 | 74.237 | 1.00 | 38.06 |
| 128 | CG2 | VAL | A | 698 | 16.517 | 4.451 | 72.586 | 1.00 | 45.80 |
| 129 | N | ILE | A | 699 | 16.401 | 7.751 | 72.575 | 1.00 | 30.17 |
| 130 | CA | ILE | A | 699 | 15.749 | 8.817 | 71.837 | 1.00 | 26.23 |
| 131 | C | ILE | A | 699 | 14.912 | 8.250 | 70.696 | 1.00 | 28.40 |
| 132 | O | ILE | A | 699 | 15.381 | 7.393 | 69.954 | 1.00 | 22.17 |
| 133 | CB | ILE | A | 699 | 16.809 | 9.774 | 71.240 | 1.00 | 28.58 |
| 134 | CG1 | ILE | A | 699 | 17.715 | 10.336 | 72.348 | 1.00 | 29.46 |
| 135 | CG2 | ILE | A | 699 | 16.135 | 10.897 | 70.496 | 1.00 | 24.35 |
| 136 | CD1 | ILE | A | 699 | 16.979 | 11.118 | 73.395 | 1.00 | 37.93 |
| 137 | N | TYR | A | 700 | 13.678 | 8.730 | 70.566 | 1.00 | 23.87 |
| 138 | CA | TYR | A | 700 | 12.788 | 8.305 | 69.488 | 1.00 | 27.94 |
| 139 | C | TYR | A | 700 | 12.944 | 9.243 | 68.304 | 1.00 | 28.04 |
| 140 | O | TYR | A | 700 | 13.238 | 10.427 | 68.474 | 1.00 | 24.24 |
| 141 | CB | TYR | A | 700 | 11.342 | 8.316 | 69.963 | 1.00 | 27.40 |
| 142 | CG | TYR | A | 700 | 11.049 | 7.190 | 70.923 | 1.00 | 34.60 |
| 143 | CD1 | TYR | A | 700 | 11.300 | 7.331 | 72.284 | 1.00 | 37.81 |
| 144 | CD2 | TYR | A | 700 | 10.584 | 5.960 | 70.462 | 1.00 | 38.29 |
| 145 | CE1 | TYR | A | 700 | 11.096 | 6.276 | 73.165 | 1.00 | 47.26 |
| 146 | CE2 | TYR | A | 700 | 10.374 | 4.893 | 71.337 | 1.00 | 39.38 |
| 147 | CZ | TYR | A | 700 | 10.634 | 5.062 | 72.687 | 1.00 | 41.71 |
| 148 | OH | TYR | A | 700 | 10.434 | 4.024 | 73.569 | 1.00 | 52.51 |
| 149 | N | ALA | A | 701 | 12.755 | 8.724 | 67.096 | 1.00 | 23.60 |
| 150 | CA | ALA | A | 701 | 12.882 | 9.578 | 65.929 | 1.00 | 26.37 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 151 | C | ALA | A | 701 | 11.576 | 10.323 | 65.649 | 1.00 | 29.47 |
| 152 | O | ALA | A | 701 | 11.579 | 11.354 | 64.970 | 1.00 | 28.14 |
| 153 | CB | ALA | A | 701 | 13.272 | 8.762 | 64.709 | 1.00 | 27.79 |
| 154 | N | GLY | A | 702 | 10.474 | 9.811 | 66.187 | 1.00 | 29.99 |
| 155 | CA | GLY | A | 702 | 9.179 | 10.419 | 65.925 | 1.00 | 32.21 |
| 156 | C | GLY | A | 702 | 8.818 | 10.120 | 64.473 | 1.00 | 39.13 |
| 157 | O | GLY | A | 702 | 8.145 | 10.902 | 63.803 | 1.00 | 40.76 |
| 158 | N | HIS | A | 703 | 9.278 | 8.970 | 63.983 | 1.00 | 38.38 |
| 159 | CA | HIS | A | 703 | 9.042 | 8.558 | 62.602 | 1.00 | 46.41 |
| 160 | C | HIS | A | 703 | 7.638 | 7.980 | 62.347 | 1.00 | 47.68 |
| 161 | O | HIS | A | 703 | 7.091 | 7.256 | 63.177 | 1.00 | 49.96 |
| 162 | CB | HIS | A | 703 | 10.129 | 7.559 | 62.194 | 1.00 | 42.73 |
| 163 | CG | HIS | A | 703 | 9.978 | 7.034 | 60.803 | 1.00 | 48.58 |
| 164 | ND1 | HIS | A | 703 | 9.082 | 6.039 | 60.474 | 1.00 | 52.11 |
| 165 | CD2 | HIS | A | 703 | 10.589 | 7.388 | 59.649 | 1.00 | 46.18 |
| 166 | CE1 | HIS | A | 703 | 9.151 | 5.800 | 59.177 | 1.00 | 50.15 |
| 167 | NE2 | HIS | A | 703 | 10.057 | 6.606 | 58.653 | 1.00 | 50.30 |
| 168 | N | ASP | A | 704 | 7.079 | 8.306 | 61.181 | 1.00 | 51.90 |
| 169 | CA | ASP | A | 704 | 5.742 | 7.869 | 60.777 | 1.00 | 52.61 |
| 170 | C | ASP | A | 704 | 5.506 | 6.363 | 60.863 | 1.00 | 54.82 |
| 171 | O | ASP | A | 704 | 4.746 | 5.902 | 61.715 | 1.00 | 57.82 |
| 172 | CB | ASP | A | 704 | 5.453 | 8.356 | 59.359 | 1.00 | 52.84 |
| 173 | N | ASN | A | 705 | 6.147 | 5.616 | 59.965 | 1.00 | 55.70 |
| 174 | CA | ASN | A | 705 | 6.031 | 4.161 | 59.889 | 1.00 | 56.60 |
| 175 | C | ASN | A | 705 | 4.799 | 3.742 | 59.081 | 1.00 | 60.14 |
| 176 | O | ASN | A | 705 | 4.906 | 2.984 | 58.109 | 1.00 | 61.18 |
| 177 | CB | ASN | A | 705 | 5.979 | 3.551 | 61.295 | 1.00 | 57.02 |
| 178 | N | THR | A | 706 | 3.633 | 4.239 | 59.479 | 1.00 | 59.68 |
| 179 | CA | THR | A | 706 | 2.384 | 3.911 | 58.794 | 1.00 | 60.50 |
| 180 | C | THR | A | 706 | 2.420 | 4.213 | 57.289 | 1.00 | 61.78 |
| 181 | O | THR | A | 706 | 1.670 | 3.614 | 56.513 | 1.00 | 61.98 |
| 182 | CB | THR | A | 706 | 1.226 | 4.662 | 59.451 | 1.00 | 57.03 |
| 183 | N | LYS | A | 707 | 3.286 | 5.135 | 56.874 | 1.00 | 62.40 |
| 184 | CA | LYS | A | 707 | 3.386 | 5.488 | 55.459 | 1.00 | 64.42 |
| 185 | C | LYS | A | 707 | 4.616 | 4.854 | 54.812 | 1.00 | 66.61 |
| 186 | O | LYS | A | 707 | 5.571 | 4.477 | 55.502 | 1.00 | 64.83 |
| 187 | CB | LYS | A | 707 | 3.439 | 7.004 | 55.302 | 1.00 | 60.35 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 188 | N | PRO | A | 708 | 4.595 | 4.705 | 53.475 | 1.00 | 67.95 |
| 189 | CA | PRO | A | 708 | 5.704 | 4.119 | 52.713 | 1.00 | 69.09 |
| 190 | C | PRO | A | 708 | 6.924 | 5.047 | 52.748 | 1.00 | 68.98 |
| 191 | O | PRO | A | 708 | 6.891 | 6.153 | 52.205 | 1.00 | 70.54 |
| 192 | CB | PRO | A | 708 | 5.111 | 3.987 | 51.304 | 1.00 | 71.22 |
| 193 | CG | PRO | A | 708 | 3.600 | 3.904 | 51.570 | 1.00 | 69.09 |
| 194 | CD | PRO | A | 708 | 3.493 | 5.039 | 52.559 | 1.00 | 68.58 |
| 195 | N | ASP | A | 709 | 7.997 | 4.585 | 53.380 | 1.00 | 68.95 |
| 196 | CA | ASP | A | 709 | 9.220 | 5.371 | 53.510 | 1.00 | 68.41 |
| 197 | C | ASP | A | 709 | 9.961 | 5.612 | 52.197 | 1.00 | 65.11 |
| 198 | O | ASP | A | 709 | 10.416 | 4.666 | 51.556 | 1.00 | 67.29 |
| 199 | CB | ASP | A | 709 | 10.188 | 4.684 | 54.482 | 1.00 | 72.12 |
| 200 | CG | ASP | A | 709 | 9.584 | 4.452 | 55.861 | 1.00 | 74.27 |
| 201 | OD1 | ASP | A | 709 | 8.401 | 4.798 | 56.074 | 1.00 | 77.63 |
| 202 | OD2 | ASP | A | 709 | 10.302 | 3.918 | 56.733 | 1.00 | 75.63 |
| 203 | N | THR | A | 710 | 10.087 | 6.876 | 51.799 | 1.00 | 61.36 |
| 204 | CA | THR | A | 710 | 10.827 | 7.201 | 50.585 | 1.00 | 54.04 |
| 205 | C | THR | A | 710 | 12.256 | 7.499 | 51.026 | 1.00 | 53.38 |
| 206 | O | THR | A | 710 | 12.519 | 7.683 | 52.214 | 1.00 | 44.49 |
| 207 | CB | THR | A | 710 | 10.247 | 8.438 | 49.852 | 1.00 | 56.37 |
| 208 | OG1 | THR | A | 710 | 10.381 | 9.603 | 50.674 | 1.00 | 58.14 |
| 209 | CG2 | THR | A | 710 | 8.782 | 8.221 | 49.537 | 1.00 | 59.13 |
| 210 | N | SER | A | 711 | 13.182 | 7.535 | 50.079 | 1.00 | 47.04 |
| 211 | CA | SER | A | 711 | 14.566 | 7.807 | 50.412 | 1.00 | 48.03 |
| 212 | C | SER | A | 711 | 14.702 | 9.163 | 51.102 | 1.00 | 44.05 |
| 213 | O | SER | A | 711 | 15.368 | 9.278 | 52.129 | 1.00 | 41.23 |
| 214 | CB | SER | A | 711 | 15.415 | 7.760 | 49.146 | 1.00 | 50.15 |
| 215 | OG | SER | A | 711 | 15.267 | 6.499 | 48.508 | 1.00 | 63.32 |
| 216 | N | SER | A | 712 | 14.058 | 10.184 | 50.550 | 1.00 | 37.98 |
| 217 | CA | SER | A | 712 | 14.132 | 11.518 | 51.136 | 1.00 | 39.84 |
| 218 | C | SER | A | 712 | 13.389 | 11.627 | 52.472 | 1.00 | 37.46 |
| 219 | O | SER | A | 712 | 13.810 | 12.375 | 53.357 | 1.00 | 35.48 |
| 220 | CB | SER | A | 712 | 13.579 | 12.561 | 50.159 | 1.00 | 40.58 |
| 221 | OG | SER | A | 712 | 12.198 | 12.349 | 49.920 | 1.00 | 48.57 |
| 222 | N | SER | A | 713 | 12.285 | 10.900 | 52.630 | 1.00 | 36.47 |
| 223 | CA | SER | A | 713 | 11.548 | 10.979 | 53.890 | 1.00 | 34.65 |
| 224 | C | SER | A | 713 | 12.388 | 10.291 | 54.960 | 1.00 | 31.60 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 225 | O | SER | A | 713 | 12.459 | 10.737 | 56.102 | 1.00 | 28.24 |
| 226 | CB | SER | A | 713 | 10.163 | 10.308 | 53.782 | 1.00 | 35.58 |
| 227 | OG | SER | A | 713 | 10.266 | 8.900 | 53.711 1 | 1.00 | 43.95 |
| 228 | N | LEU | A | 714 | 13.040 | 9.207 | 54.574 | 1.00 | 27.90 |
| 229 | CA | LEU | A | 714 | 13.897 | 8.469 | 55.490 | 1.00 | 29.29 |
| 230 | C | LEU | A | 714 | 15.031 | 9.364 | 56.008 | 1.00 | 28.57 |
| 231 | O | LEU | A | 714 | 15.253 | 9.451 | 57.215 | 1.00 | 25.88 |
| 232 | CB | LEU | A | 714 | 14.488 | 7.255 | 54.781 | 1.00 | 27.95 |
| 233 | CG | LEU | A | 714 | 15.515 | 6.429 | 55.552 | 1.00 | 32.79 |
| 234 | CD1 | LEU | A | 714 | 15.004 | 6.197 | 56.951 | 1.00 | 31.65 |
| 235 | CD2 | LEU | A | 714 | 15.783 | 5.110 | 54.823 | 1.00 | 36.96 |
| 236 | N | LEU | A | 715 | 15.735 | 10.025 | 55.094 | 1.00 | 25.56 |
| 237 | CA | LEU | A | 715 | 16.832 | 10.915 | 55.482 | 1.00 | 24.24 |
| 238 | C | LEU | A | 715 | 16.340 | 12.042 | 56.345 | 1.00 | 22.49 |
| 239 | O | LEU | A | 715 | 16.992 | 12.398 | 57.317 | 1.00 | 21.35 |
| 240 | CB | LEU | A | 715 | 17.541 | 11.463 | 54.256 | 1.00 | 24.95 |
| 241 | CG | LEU | A | 715 | 18.210 | 10.360 | 53.431 | 1.00 | 27.77 |
| 242 | CD1 | LEU | A | 715 | 18.781 | 10.946 | 52.146 | 1.00 | 27.06 |
| 243 | CD2 | LEU | A | 715 | 19.300 | 9.695 | 54.259 | 1.00 | 32.37 |
| 244 | N | THR | A | 716 | 15.179 | 12.598 | 56.004 | 1.00 | 21.24 |
| 245 | CA | THR | A | 716 | 14.586 | 13.664 | 56.795 | 1.00 | 24.54 |
| 246 | C | THR | A | 716 | 14.306 | 13.159 | 58.207 | 1.00 | 24.54 |
| 247 | O | THR | A | 716 | 14.552 | 13.864 | 59.181 | 1.00 | 21.07 |
| 248 | CB | THR | A | 716 | 13.265 | 14.171 | 56.164 | 1.00 | 24.90 |
| 249 | OG1 | THR | A | 716 | 13.561 | 14.873 | 54.948 | 1.00 | 26.56 |
| 250 | CG2 | THR | A | 716 | 12.520 | 15.088 | 57.125 | 1.00 | 23.35 |
| 251 | N | SER | A | 717 | 13.776 | 11.945 | 58.322 | 1.00 | 19.11 |
| 252 | CA | SER | A | 717 | 13.508 | 11.381 | 59.635 | 1.00 | 21.51 |
| 253 | C | SER | A | 717 | 14.815 | 11.112 | 60.389 | 1.00 | 19.84 |
| 254 | O | SER | A | 717 | 14.860 | 11.270 | 61.619 | 1.00 | 21.66 |
| 255 | CB | SER | A | 717 | 12.706 | 10.077 | 59.527 | 1.00 | 24.02 |
| 256 | OG | SER | A | 717 | 11.397 | 10.321 | 59.029 | 1.00 | 36.60 |
| 257 | N | LEU | A | 718 | 15.866 | 10.687 | 59.683 | 1.00 | 18.47 |
| 258 | CA | LEU | A | 718 | 17.133 | 10.441 | 60.368 | 1.00 | 20.90 |
| 259 | C | LEU | A | 718 | 17.738 | 11.766 | 60.828 | 1.00 | 20.84 |
| 260 | O | LEU | A | 718 | 18.425 | 11.825 | 61.861 | 1.00 | 20.00 |
| 261 | CB | LEU | A | 718 | 18.113 | 9.706 | 59.464 | 1.00 | 17.55 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 262 | CG | LEU | A | 718 | 17.770 | 8.242 | 59.191 | 1.00 | 19.20 |
| 263 | CD1 | LEU | A | 718 | 18.704 | 7.721 | 58.127 | 1.00 | 20.15 |
| 264 | CD2 | LEU | A | 718 | 17.902 | 7.418 | 60.467 | 1.00 | 19.08 |
| 265 | N | ASN | A | 719 | 17.498 | 12.832 | 60.070 | 1.00 | 17.81 |
| 266 | CA | ASN | A | 719 | 18.018 | 14.148 | 60.498 | 1.00 | 19.99 |
| 267 | C | ASN | A | 719 | 17.274 | 14.651 | 61.732 | 1.00 | 18.99 |
| 268 | O | ASN | A | 719 | 17.862 | 15.300 | 62.591 | 1.00 | 20.08 |
| 269 | CB | ASN | A | 719 | 17.910 | 15.208 | 59.392 | 1.00 | 22.67 |
| 270 | CG | ASN | A | 719 | 18.942 | 15.026 | 58.296 | 1.00 | 27.38 |
| 271 | OD1 | ASN | A | 719 | 20.042 | 14.490 | 58.523 | 1.00 | 24.69 |
| 272 | ND2 | ASN | A | 719 | 18.622 | 15.530 | 57.106 | 1.00 | 23.33 |
| 273 | N | GLN | A | 720 | 15.975 | 14.380 | 61.808 | 1.00 | 18.44 |
| 274 | CA | GLN | A | 720 | 15.165 | 14.785 | 62.966 | 1.00 | 21.61 |
| 275 | C | GLN | A | 720 | 15.693 | 14.023 | 64.186 | 1.00 | 23.93 |
| 276 | O | GLN | A | 720 | 15.832 | 14.582 | 65.273 | 1.00 | 18.25 |
| 277 | CB | GLN | A | 720 | 13.683 | 14.456 | 62.730 | 1.00 | 22.65 |
| 278 | CG | GLN | A | 720 | 12.817 | 14.620 | 63.963 | 1.00 | 26.41 |
| 279 | CD | GLN | A | 720 | 12.882 | 16.019 | 64.530 | 1.00 | 26.67 |
| 280 | OE1 | GLN | A | 720 | 12.760 | 16.211 | 65.741 | 1.00 | 31.96 |
| 281 | NE2 | GLN | A | 720 | 13.052 | 17.013 | 63.658 | 1.00 | 28.01 |
| 282 | N | LEU | A | 721 | 16.006 | 12.741 | 63.995 | 1.00 | 19.58 |
| 283 | CA | LEU | A | 721 | 16.568 | 11.942 | 65.084 | 1.00 | 17.13 |
| 284 | C | LEU | A | 721 | 17.924 | 12.545 | 65.476 | 1.00 | 16.29 |
| 285 | O | LEU | A | 721 | 18.228 | 12.690 | 66.662 | 1.00 | 19.55 |
| 286 | CB | LEU | A | 721 | 16.742 | 10.479 | 64.638 | 1.00 | 17.00 |
| 287 | CG | LEU | A | 721 | 17.378 | 9.549 | 65.673 | 1.00 | 19.10 |
| 288 | CD1 | LEU | A | 721 | 16.508 | 9.497 | 66.942 | 1.00 | 18.65 |
| 289 | CD2 | LEU | A | 721 | 17.536 | 8.145 | 65.037 | 1.00 | 17.08 |
| 290 | N | GLY | A | 722 | 18.724 | 12.896 | 64.476 | 1.00 | 16.83 |
| 291 | CA | GLY | A | 722 | 20.036 | 13.486 | 64.710 | 1.00 | 17.42 |
| 292 | C | GLY | A | 722 | 19.929 | 14.774 | 65.516 | 1.00 | 24.76 |
| 293 | O | GLY | A | 722 | 20.749 | 15.038 | 66.402 | 1.00 | 19.56 |
| 294 | N | GLU | A | 723 | 18.923 | 15.586 | 65.210 | 1.00 | 20.84 |
| 295 | CA | GLU | A | 723 | 18.709 | 16.848 | 65.951 | 1.00 | 22.88 |
| 296 | C | GLU | A | 723 | 18.408 | 16.526 | 67.410 | 1.00 | 23.92 |
| 297 | O | GLU | A | 723 | 18.896 | 17.195 | 68.328 | 1.00 | 23.22 |
| 298 | CB | GLU | A | 723 | 17.521 | 17.623 | 65.372 | 1.00 | 30.25 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 299 | CG | GLU | A | 723 | 17.240 | 18.998 | 66.018 | 1.00 | 27.93 |
| 300 | CD | GLU | A | 723 | 18.083 | 20.112 | 65.417 | 1.00 | 37.81 |
| 301 | OE1 | GLU | A | 723 | 18.978 | 19.780 | 64.624 | 1.00 | 29.92 |
| 302 | OE2 | GLU | A | 723 | 17.847 | 21.311 | 65.726 | 1.00 | 32.81 |
| 303 | N | ARG | A | 724 | 17.597 | 15.496 | 67.640 | 1.00 | 17.75 |
| 304 | CA | ARG | A | 724 | 17.258 | 15.112 | 69.007 | 1.00 | 19.86 |
| 305 | C | ARG | A | 724 | 18.445 | 14.490 | 69.735 | 1.00 | 18.81 |
| 306 | O | ARG | A | 724 | 18.565 | 14.633 | 70.958 | 1.00 | 20.56 |
| 307 | CB | ARG | A | 724 | 16.085 | 14.135 | 69.015 | 1.00 | 18.63 |
| 308 | CG | ARG | A | 724 | 14.820 | 14.733 | 68.421 | 1.00 | 24.16 |
| 309 | CD | ARG | A | 724 | 13.713 | 13.709 | 68.362 | 1.00 | 28.88 |
| 310 | NE | ARG | A | 724 | 12.531 | 14.280 | 67.730 | 1.00 | 27.71 |
| 311 | CZ | ARG | A | 724 | 11.330 | 13.726 | 67.739 | 1.00 | 32.19 |
| 312 | NH1 | ARG | A | 724 | 11.131 | 12.566 | 68.352 | 1.00 | 28.19 |
| 313 | NH2 | ARG | A | 724 | 10.315 | 14.360 | 67.162 | 1.00 | 35.89 |
| 314 | N | GLN | A | 725 | 19.296 | 13.770 | 69.009 | 1.00 | 19.77 |
| 315 | CA | GLN | A | 725 | 20.474 | 13.188 | 69.634 | 1.00 | 19.30 |
| 316 | C | GLN | A | 725 | 21.545 | 14.239 | 69.906 | 1.00 | 21.49 |
| 317 | O | GLN | A | 725 | 22.309 | 14.113 | 70.855 | 1.00 | 21.43 |
| 318 | CB | GLN | A | 725 | 21.055 | 12.063 | 68.771 | 1.00 | 19.47 |
| 319 | CG | GLN | A | 725 | 20.135 | 10.843 | 68.774 | 1.00 | 22.41 |
| 320 | CD | GLN | A | 725 | 20.746 | 9.633 | 68.092 | 1.00 | 30.53 |
| 321 | OE1 | GLN | A | 725 | 20.104 | 8.578 | 67.970 | 1.00 | 32.93 |
| 322 | NE2 | GLN | A | 725 | 21.987 | 9.770 | 67.647 | 1.00 | 27.09 |
| 323 | N | LEU | A | 726 | 21.592 | 15.284 | 69.085 | 1.00 | 21.82 |
| 324 | CA | LEU | A | 726 | 22.573 | 16.347 | 69.288 | 1.00 | 22.79 |
| 325 | C | LEU | A | 726 | 22.284 | 17.027 | 70.624 | 1.00 | 21.40 |
| 326 | O | LEU | A | 726 | 23.207 | 17.293 | 71.406 | 1.00 | 23.41 |
| 327 | CB | LEU | A | 726 | 22.526 | 17.367 | 68.135 | 1.00 | 19.53 |
| 328 | CG | LEU | A | 726 | 23.533 | 18.531 | 68.138 | 1.00 | 24.90 |
| 329 | CD1 | LEU | A | 726 | 24.948 | 18.005 | 68.300 | 1.00 | 22.88 |
| 330 | CD2 | LEU | A | 726 | 23.421 | 19.325 | 66.812 | 1.00 | 20.28 |
| 331 | N | LEU | A | 727 | 21.007 | 17.301 | 70.890 | 1.00 | 18.00 |
| 332 | CA | LEU | A | 727 | 20.623 | 17.906 | 72.156 | 1.00 | 19.91 |
| 333 | C | LEU | A | 727 | 21.078 | 16.978 | 73.281 | 1.00 | 24.23 |
| 334 | O | LEU | A | 727 | 21.678 | 17.402 | 74.274 | 1.00 | 19.07 |
| 335 | CB | LEU | A | 727 | 19.105 | 18.065 | 72.247 | 1.00 | 21.64 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 336 | CG | LEU | A | 727 | 18.591 | 18.563 | 73.594 | 1.00 | 21.40 |
| 337 | CD1 | LEU | A | 727 | 19.256 | 19.906 | 73.912 | 1.00 | 26.36 |
| 338 | CD2 | LEU | A | 727 | 17.082 | 18.699 | 73.546 | 1.00 | 28.00 |
| 339 | N | SER | A | 728 | 20.783 | 15.695 | 73.106 | 1.00 | 21.94 |
| 340 | CA | SER | A | 728 | 21.131 | 14.690 | 74.097 | 1.00 | 21.38 |
| 341 | C | SER | A | 728 | 22.637 | 14.682 | 74.355 | 1.00 | 20.10 |
| 342 | O | SER | A | 728 | 23.066 | 14.611 | 75.512 | 1.00 | 22.32 |
| 343 | CB | SER | A | 728 | 20.645 | 13.310 | 73.630 | 1.00 | 24.08 |
| 344 | OG | SER | A | 728 | 20.719 | 12.380 | 74.689 | 1.00 | 30.43 |
| 345 | N | VAL | A | 729 | 23.433 | 14.741 | 73.297 | 1.00 | 18.72 |
| 346 | CA | VAL | A | 729 | 24.891 | 14.781 | 73.415 | 1.00 | 20.27 |
| 347 | C | VAL | A | 729 | 25.388 | 15.988 | 74.203 | 1.00 | 18.31 |
| 348 | O | VAL | A | 729 | 26.274 | 15.860 | 75.049 | 1.00 | 19.16 |
| 349 | CB | VAL | A | 729 | 25.574 | 14.796 | 72.034 | 1.00 | 17.15 |
| 350 | CG1 | VAL | A | 729 | 27.060 | 15.147 | 72.164 | 1.00 | 20.31 |
| 351 | CG2 | VAL | A | 729 | 25.453 | 13.395 | 71.407 | 1.00 | 21.83 |
| 352 | N | VAL | A | 730 | 24.830 | 17.159 | 73.937 | 1.00 | 17.43 |
| 353 | CA | VAL | A | 730 | 25.282 | 18.333 | 74.660 | 1.00 | 21.44 |
| 354 | C | VAL | A | 730 | 24.888 | 18.225 | 76.132 | 1.00 | 19.86 |
| 355 | O | VAL | A | 730 | 25.678 | 18.584 | 76.993 | 1.00 | 21.47 |
| 356 | CB | VAL | A | 730 | 24.725 | 19.630 | 74.038 | 1.00 | 20.22 |
| 357 | CG1 | VAL | A | 730 | 25.210 | 20.849 | 74.834 | 1.00 | 21.24 |
| 358 | CG2 | VAL | A | 730 | 25.178 | 19.734 | 72.596 | 1.00 | 19.32 |
| 359 | N | LYS | A | 731 | 23.686 | 17.727 | 76.427 | 1.00 | 17.73 |
| 360 | CA | LYS | A | 731 | 23.275 | 17.552 | 77.817 | 1.00 | 22.58 |
| 361 | C | LYS | A | 731 | 24.186 | 16.546 | 78.517 | 1.00 | 23.23 |
| 362 | O | LYS | A | 731 | 24.613 | 16.757 | 79.659 | 1.00 | 20.64 |
| 363 | CB | LYS | A | 731 | 21.808 | 17.121 | 77.911 | 1.00 | 23.07 |
| 364 | CG | LYS | A | 731 | 20.850 | 18.296 | 77.646 | 1.00 | 25.92 |
| 365 | CD | LYS | A | 731 | 19.388 | 18.009 | 78.016 | 1.00 | 37.08 |
| 366 | CE | LYS | A | 731 | 18.717 | 17.034 | 77.063 | 1.00 | 48.50 |
| 367 | NZ | LYS | A | 731 | 17.247 | 16.901 | 77.346 | 1.00 | 49.18 |
| 368 | N | TRP | A | 732 | 24.486 | 15.452 | 77.828 | 1.00 | 18.73 |
| 369 | CA | TRP | A | 732 | 25.383 | 14.437 | 78.364 | 1.00 | 21.37 |
| 370 | C | TRP | A | 732 | 26.743 | 15.038 | 78.703 | 1.00 | 22.20 |
| 371 | O | TRP | A | 732 | 27.293 | 14.772 | 79.770 | 1.00 | 23.93 |
| 372 | CB | TRP | A | 732 | 25.552 | 13.321 | 77.334 | 1.00 | 21.47 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 373 | CG | TRP | A | 732 | 26.674 | 12.347 | 77.582 | 1.00 | 17.78 |
| 374 | CD1 | TRP | A | 732 | 26.728 | 11.348 | 78.528 | 1.00 | 20.71 |
| 375 | CD2 | TRP | A | 732 | 27.861 | 12.225 | 76.806 | 1.00 | 17.23 |
| 376 | NE1 | TRP | A | 732 | 27.879 | 10.613 | 78.370 | 1.00 | 19.88 |
| 377 | CE2 | TRP | A | 732 | 28.593 | 11.130 | 77.318 | 1.00 | 19.41 |
| 378 | CE3 | TRP | A | 732 | 28.383 | 12.938 | 75.713 | 1.00 | 19.83 |
| 379 | CZ2 | TRP | A | 732 | 29.824 | 10.726 | 76.771 | 1.00 | 18.15 |
| 380 | CZ3 | TRP | A | 732 | 29.612 | 12.533 | 75.165 | 1.00 | 20.23 |
| 381 | CH2 | TRP | A | 732 | 30.314 | 11.435 | 75.701 | 1.00 | 22.27 |
| 382 | N | SER | A | 733 | 27.274 | 15.872 | 77.811 | 1.00 | 20.68 |
| 383 | CA | SER | A | 733 | 28.594 | 16.460 | 78.025 | 1.00 | 22.57 |
| 384 | C | SER | A | 733 | 28.650 | 17.371 | 79.250 | 1.00 | 20.83 |
| 385 | O | SER | A | 733 | 29.701 | 17.523 | 79.843 | 1.00 | 22.59 |
| 386 | CB | SER | A | 733 | 29.052 | 17.247 | 76.783 | 1.00 | 22.82 |
| 387 | OG | SER | A | 733 | 28.287 | 18.431 | 76.611 | 1.00 | 27.11 |
| 388 | N | LYS | A | 734 | 27.522 | 17.976 | 79.619 | 1.00 | 20.76 |
| 389 | CA | LYS | A | 734 | 27.495 | 18.878 | 80.769 | 1.00 | 25.82 |
| 390 | C | LYS | A | 734 | 27.610 | 18.084 | 82.069 | 1.00 | 26.99 |
| 391 | O | LYS | A | 734 | 27.952 | 18.643 | 83.117 | 1.00 | 24.98 |
| 392 | CB | LYS | A | 734 | 26.211 | 19.724 | 80.761 | 1.00 | 27.35 |
| 393 | CG | LYS | A | 734 | 26.007 | 20.439 | 79.443 | 1.00 | 34.69 |
| 394 | CD | LYS | A | 734 | 25.400 | 21.833 | 79.600 | 1.00 | 44.84 |
| 395 | CE | LYS | A | 734 | 24.047 | 21.812 | 80.278 | 1.00 | 49.64 |
| 396 | NZ | LYS | A | 734 | 23.525 | 23.208 | 80.476 | 1.00 | 49.55 |
| 397 | N | SER | A | 735 | 27.325 | 16.786 | 82.003 | 1.00 | 23.17 |
| 398 | CA | SER | A | 735 | 27.451 | 15.922 | 83.180 | 1.00 | 23.40 |
| 399 | C | SER | A | 735 | 28.698 | 15.045 | 83.135 | 1.00 | 24.42 |
| 400 | O | SER | A | 735 | 29.010 | 14.345 | 84.110 | 1.00 | 21.97 |
| 401 | CB | SER | A | 735 | 26.221 | 15.016 | 83.324 | 1.00 | 24.11 |
| 402 | OG | SER | A | 735 | 25.066 | 15.769 | 83.629 | 1.00 | 29.58 |
| 403 | N | LEU | A | 736 | 29.421 | 15.085 | 82.015 | 1.00 | 19.77 |
| 404 | CA | LEU | A | 736 | 30.613 | 14.246 | 81.835 | 1.00 | 21.02 |
| 405 | C | LEU | A | 736 | 31.774 | 14.753 | 82.669 | 1.00 | 21.28 |
| 406 | O | LEU | A | 736 | 32.247 | 15.858 | 82.451 | 1.00 | 22.53 |
| 407 | CB | LEU | A | 736 | 31.017 | 14.233 | 80.354 | 1.00 | 23.10 |
| 408 | CG | LEU | A | 736 | 32.215 | 13.395 | 79.912 | 1.00 | 22.82 |
| 409 | CD1 | LEU | A | 736 | 31.963 | 11.915 | 80.168 | 1.00 | 22.40 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 410 | CD2 | LEU | A | 736 | 32.443 | 13.639 | 78.412 | 1.00 | 20.94 |
| 411 | N | PRO | A | 737 | 32.285 | 13.930 | 83.595 | 1.00 | 22.85 |
| 412 | CA | PRO | A | 737 | 33.398 | 14.411 | 84.424 | 1.00 | 24.25 |
| 413 | C | PRO | A | 737 | 34.563 | 15.028 | 83.659 | 1.00 | 25.32 |
| 414 | O | PRO | A | 737 | 35.114 | 14.423 | 82.734 | 1.00 | 25.05 |
| 415 | CB | PRO | A | 737 | 33.790 | 13.163 | 85.219 | 1.00 | 24.36 |
| 416 | CG | PRO | A | 737 | 32.452 | 12.470 | 85.397 | 1.00 | 23.83 |
| 417 | CD | PRO | A | 737 | 31.958 | 12.535 | 83.947 | 1.00 | 20.46 |
| 418 | N | GLY | A | 738 | 34.911 | 16.253 | 84.053 | 1.00 | 25.15 |
| 419 | CA | GLY | A | 738 | 36.016 | 16.969 | 83.444 | 1.00 | 25.65 |
| 420 | C | GLY | A | 738 | 35.703 | 17.825 | 82.231 | 1.00 | 23.09 |
| 421 | O | GLY | A | 738 | 36.394 | 18.803 | 81.956 | 1.00 | 25.86 |
| 422 | N | PHE | A | 739 | 34.648 | 17.493 | 81.504 | 1.00 | 22.74 |
| 423 | CA | PHE | A | 739 | 34.363 | 18.252 | 80.296 | 1.00 | 23.63 |
| 424 | C | PHE | A | 739 | 34.018 | 19.717 | 80.536 | 1.00 | 22.42 |
| 425 | O | PHE | A | 739 | 34.537 | 20.591 | 79.856 | 1.00 | 21.78 |
| 426 | CB | PHE | A | 739 | 33.217 | 17.598 | 79.506 | 1.00 | 21.38 |
| 427 | CG | PHE | A | 739 | 33.129 | 18.065 | 78.077 | 1.00 | 20.80 |
| 428 | CD1 | PHE | A | 739 | 34.130 | 17.715 | 77.165 | 1.00 | 22.81 |
| 429 | CD2 | PHE | A | 739 | 32.063 | 18.851 | 77.642 | 1.00 | 24.10 |
| 430 | CE1 | PHE | A | 739 | 34.072 | 18.134 | 75.848 | 1.00 | 24.39 |
| 431 | CE2 | PHE | A | 739 | 31.998 | 19.278 | 76.313 | 1.00 | 19.38 |
| 432 | CZ | PHE | A | 739 | 33.001 | 18.918 | 75.423 | 1.00 | 23.48 |
| 433 | N | ARG | A | 740 | 33.147 | 19.979 | 81.508 | 1.00 | 22.15 |
| 434 | CA | ARG | A | 740 | 32.700 | 21.344 | 81.780 | 1.00 | 25.70 |
| 435 | C | ARG | A | 740 | 33.823 | 22.255 | 82.232 | 1.00 | 24.37 |
| 436 | O | ARG | A | 740 | 33.667 | 23.477 | 82.278 | 1.00 | 24.34 |
| 437 | CB | ARG | A | 740 | 31.615 | 21.352 | 82.851 | 1.00 | 23.04 |
| 438 | CG | ARG | A | 740 | 32.071 | 20.742 | 84.166 | 1.00 | 24.68 |
| 439 | CD | ARG | A | 740 | 31.094 | 21.087 | 85.281 | 1.00 | 25.40 |
| 440 | NE | ARG | A | 740 | 31.569 | 20.540 | 86.545 | 1.00 | 24.48 |
| 441 | CZ | ARG | A | 740 | 31.023 | 20.804 | 87.721 | 1.00 | 20.47 |
| 442 | NH1 | ARG | A | 740 | 29.979 | 21.612 | 87.791 | 1.00 | 23.59 |
| 443 | NH2 | ARG | A | 740 | 31.535 | 20.261 | 88.819 | 1.00 | 26.38 |
| 444 | N | ASN | A | 741 | 34.957 | 21.656 | 82.555 | 1.00 | 27.20 |
| 445 | CA | ASN | A | 741 | 36.100 | 22.409 | 83.029 | 1.00 | 23.55 |
| 446 | C | ASN | A | 741 | 37.024 | 22.889 | 81.918 | 1.00 | 26.53 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 447 | O | ASN | A | 741 | 37.922 | 23.694 | 82.170 | 1.00 | 27.16 |
| 448 | CB | ASN | A | 741 | 36.828 | 21.571 | 84.063 | 1.00 | 32.06 |
| 449 | CG | ASN | A | 741 | 35.926 | 21.211 | 85.230 | 1.00 | 33.85 |
| 450 | OD1 | ASN | A | 741 | 36.120 | 20.201 | 85.899 | 1.00 | 37.94 |
| 451 | ND2 | ASN | A | 741 | 34.928 | 22.054 | 85.481 | 1.00 | 28.27 |
| 452 | N | LEU | A | 742 | 36.806 | 22.402 | 80.698 | 1.00 | 22.63 |
| 453 | CA | LEU | A | 742 | 37.572 | 22.859 | 79.547 | 1.00 | 22.51 |
| 454 | C | LEU | A | 742 | 36.961 | 24.201 | 79.206 | 1.00 | 19.73 |
| 455 | O | LEU | A | 742 | 35.843 | 24.497 | 79.590 | 1.00 | 23.16 |
| 456 | CB | LEU | A | 742 | 37.388 | 21.936 | 78.336 | 1.00 | 21.95 |
| 457 | CG | LEU | A | 742 | 37.803 | 20.486 | 78.573 | 1.00 | 20.73 |
| 458 | CD1 | LEU | A | 742 | 37.333 | 19.611 | 77.383 | 1.00 | 23.47 |
| 459 | CD2 | LEU | A | 742 | 39.318 | 20.415 | 78.768 | 1.00 | 22.85 |
| 460 | N | HIS | A | 743 | 37.712 | 25.006 | 78.483 | 1.00 | 21.02 |
| 461 | CA | HIS | A | 743 | 37.248 | 26.319 | 78.056 | 1.00 | 25.86 |
| 462 | C | HIS | A | 743 | 35.972 | 26.079 | 77.252 | 1.00 | 26.17 |
| 463 | O | HIS | A | 743 | 35.873 | 25.074 | 76.559 | 1.00 | 23.09 |
| 464 | CB | HIS | A | 743 | 38.358 | 26.936 | 77.204 | 1.00 | 28.77 |
| 465 | CG | HIS | A | 743 | 38.130 | 28.363 | 76.836 | 1.00 | 36.70 |
| 466 | ND1 | HIS | A | 743 | 37.179 | 28.753 | 75.921 | 1.00 | 34.14 |
| 467 | CD2 | HIS | A | 743 | 38.742 | 29.497 | 77.251 | 1.00 | 38.95 |
| 468 | CE1 | HIS | A | 743 | 37.215 | 30.067 | 75.785 | 1.00 | 39.45 |
| 469 | NE2 | HIS | A | 743 | 38.155 | 30.542 | 76.582 | 1.00 | 39.37 |
| 470 | N | ILE | A | 744 | 34.996 | 26.980 | 77.355 | 1.00 | 20.73 |
| 471 | CA | ILE | A | 744 | 33.743 | 26.827 | 76.623 | 1.00 | 27.73 |
| 472 | C | ILE | A | 744 | 33.951 | 26.674 | 75.118 | 1.00 | 27.07 |
| 473 | O | ILE | A | 744 | 33.264 | 25.875 | 74.461 | 1.00 | 24.14 |
| 474 | CB | ILE | A | 744 | 32.790 | 28.013 | 76.890 | 1.00 | 26.09 |
| 475 | CG1 | ILE | A | 744 | 32.515 | 28.111 | 78.389 | 1.00 | 32.88 |
| 476 | CG2 | ILE | A | 744 | 31.453 | 27.810 | 76.148 | 1.00 | 29.39 |
| 477 | CD1 | ILE | A | 744 | 31.940 | 26.858 | 78.979 | 1.00 | 39.72 |
| 478 | N | ASP | A | 745 | 34.888 | 27.432 | 74.554 | 1.00 | 24.97 |
| 479 | CA | ASP | A | 745 | 35.140 | 27.317 | 73.126 | 1.00 | 27.70 |
| 480 | C | ASP | A | 745 | 35.633 | 25.917 | 72.773 | 1.00 | 25.79 |
| 481 | O | ASP | A | 745 | 35.334 | 25.406 | 71.696 | 1.00 | 26.38 |
| 482 | CB | ASP | A | 745 | 36.175 | 28.338 | 72.639 | 1.00 | 33.81 |
| 483 | CG | ASP | A | 745 | 35.692 | 29.769 | 72.762 | 1.00 | 46.44 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 484 | OD1 | ASP | A | 745 | 34.462 | 29.984 | 72.755 | 1.00 | 51.31 |
| 485 | OD2 | ASP | A | 745 | 36.548 | 30.683 | 72.829 | 1.00 | 50.96 |
| 486 | N | ASP | A | 746 | 36.406 | 25.312 | 73.667 | 1.00 | 23.70 |
| 487 | CA | ASP | A | 746 | 36.904 | 23.971 | 73.418 | 1.00 | 22.43 |
| 488 | C | ASP | A | 746 | 35.755 | 22.975 | 73.520 | 1.00 | 19.90 |
| 489 | O | ASP | A | 746 | 35.672 | 22.049 | 72.712 | 1.00 | 22.60 |
| 490 | CB | ASP | A | 746 | 37.992 | 23.577 | 74.406 | 1.00 | 21.92 |
| 491 | CG | ASP | A | 746 | 39.172 | 24.513 | 74.370 | 1.00 | 33.42 |
| 492 | OD1 | ASP | A | 746 | 39.421 | 25.130 | 73.300 | 1.00 | 34.56 |
| 493 | OD2 | ASP | A | 746 | 39.862 | 24.610 | 75.408 | 1.00 | 39.54 |
| 494 | N | GLN | A | 747 | 34.861 | 23.176 | 74.486 | 1.00 | 19.31 |
| 495 | CA | GLN | A | 747 | 33.739 | 22.252 | 74.657 | 1.00 | 17.71 |
| 496 | C | GLN | A | 747 | 32.919 | 22.281 | 73.385 | 1.00 | 23.99 |
| 497 | O | GLN | A | 747 | 32.563 | 21.238 | 72.852 | 1.00 | 19.91 |
| 498 | CB | GLN | A | 747 | 32.846 | 22.655 | 75.826 | 1.00 | 19.37 |
| 499 | CG | GLN | A | 747 | 33.563 | 22.742 | 77.152 | 1.00 | 22.18 |
| 500 | CD | GLN | A | 747 | 32.642 | 23.127 | 78.281 | 1.00 | 22.76 |
| 501 | OE1 | GLN | A | 747 | 33.052 | 23.798 | 79.230 | 1.00 | 25.01 |
| 502 | NE2 | GLN | A | 747 | 31.397 | 22.675 | 78.212 | 1.00 | 18.79 |
| 503 | N | ILE | A | 748 | 32.605 | 23.481 | 72.908 | 1.00 | 21.08 |
| 504 | CA | ILE | A | 748 | 31.835 | 23.604 | 71.680 | 1.00 | 21.96 |
| 505 | C | ILE | A | 748 | 32.556 | 22.969 | 70.493 | 1.00 | 24.64 |
| 506 | O | ILE | A | 748 | 31.942 | 22.218 | 69.732 | 1.00 | 23.28 |
| 507 | CB | ILE | A | 748 | 31.527 | 25.087 | 71.368 | 1.00 | 25.55 |
| 508 | CG1 | ILE | A | 748 | 30.603 | 25.642 | 72.445 | 1.00 | 22.21 |
| 509 | CG2 | ILE | A | 748 | 30.871 | 25.213 | 69.993 | 1.00 | 25.02 |
| 510 | CD1 | ILE | A | 748 | 30.292 | 27.138 | 72.308 | 1.00 | 26.31 |
| 511 | N | THR | A | 749 | 33.847 | 23.268 | 70.332 | 1.00 | 19.75 |
| 512 | CA | THR | A | 749 | 34.645 | 22.716 | 69.240 | 1.00 | 20.18 |
| 513 | C | THR | A | 749 | 34.678 | 21.177 | 69.271 | 1.00 | 19.01 |
| 514 | O | THR | A | 749 | 34.541 | 20.536 | 68.238 | 1.00 | 18.23 |
| 515 | CB | THR | A | 749 | 36.104 | 23.229 | 69.301 | 1.00 | 24.15 |
| 516 | OG1 | THR | A | 749 | 36.104 | 24.656 | 69.166 | 1.00 | 26.23 |
| 517 | CG2 | THR | A | 749 | 36.925 | 22.631 | 68.177 | 1.00 | 23.53 |
| 518 | N | LEU | A | 750 | 34.864 | 20.583 | 70.450 | 1.00 | 17.46 |
| 519 | CA | LEU | A | 750 | 34.899 | 19.117 | 70.541 | 1.00 | 19.07 |
| 520 | C | LEU | A | 750 | 33.570 | 18.457 | 70.141 | 1.00 | 17.18 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 521 | O | LEU | A | 750 | 33.569 | 17.386 | 69.522 | 1.00 | 17.28 |
| 522 | CB | LEU | A | 750 | 35.293 | 18.670 | 71.955 | 1.00 | 18.92 |
| 523 | CG | LEU | A | 750 | 36.741 | 19.094 | 72.288 | 1.00 | 22.74 |
| 524 | CD1 | LEU | A | 750 | 37.054 | 18.900 | 73.774 | 1.00 | 22.99 |
| 525 | CD2 | LEU | A | 750 | 37.674 | 18.281 | 71.444 | 1.00 | 20.48 |
| 526 | N | ILE | A | 751 | 32.457 | 19.073 | 70.524 | 1.00 | 16.29 |
| 527 | CA | ILE | A | 751 | 31.136 | 18.544 | 70.148 | 1.00 | 23.76 |
| 528 | C | ILE | A | 751 | 30.940 | 18.695 | 68.635 | 1.00 | 22.00 |
| 529 | O | ILE | A | 751 | 30.462 | 17.777 | 67.966 | 1.00 | 17.05 |
| 530 | CB | ILE | A | 751 | 29.991 | 19.264 | 70.913 | 1.00 | 18.60 |
| 531 | CG1 | ILE | A | 751 | 30.014 | 18.842 | 72.388 | 1.00 | 22.25 |
| 532 | CG2 | ILE | A | 751 | 28.614 | 18.908 | 70.322 | 1.00 | 22.79 |
| 533 | CD1 | ILE | A | 751 | 29.698 | 17.360 | 72.594 | 1.00 | 33.00 |
| 534 | N | GLN | A | 752 | 31.311 | 19.848 | 68.085 | 1.00 | 21.43 |
| 535 | CA | GLN | A | 752 | 31.144 | 20.031 | 66.643 | 1.00 | 23.26 |
| 536 | C | GLN | A | 752 | 32.038 | 19.105 | 65.824 | 1.00 | 22.82 |
| 537 | O | GLN | A | 752 | 31.702 | 18.771 | 64.700 | 1.00 | 22.26 |
| 538 | CB | GLN | A | 752 | 31.357 | 21.505 | 66.248 | 1.00 | 21.04 |
| 539 | CG | GLN | A | 752 | 30.346 | 22.408 | 66.965 | 1.00 | 26.41 |
| 540 | CD | GLN | A | 752 | 30.410 | 23.867 | 66.541 | 1.00 | 34.88 |
| 541 | OE1 | GLN | A | 752 | 31.485 | 24.427 | 66.339 | 1.00 | 28.80 |
| 542 | NE2 | GLN | A | 752 | 29.245 | 24.500 | 66.453 | 1.00 | 32.80 |
| 543 | N | TYR | A | 753 | 33.171 | 18.680 | 66.381 | 1.00 | 21.10 |
| 544 | CA | TYR | A | 753 | 34.060 | 17.765 | 65.657 | 1.00 | 23.73 |
| 545 | C | TYR | A | 753 | 33.590 | 16.333 | 65.756 | 1.00 | 21.41 |
| 546 | O | TYR | A | 753 | 33.692 | 15.561 | 64.806 | 1.00 | 24.76 |
| 547 | CB | TYR | A | 753 | 35.471 | 17.782 | 66.250 | 1.00 | 22.41 |
| 548 | CG | TYR | A | 753 | 36.339 | 18.964 | 65.885 | 1.00 | 21.14 |
| 549 | CD1 | TYR | A | 753 | 35.855 | 20.015 | 65.117 | 1.00 | 22.56 |
| 550 | CD2 | TYR | A | 753 | 37.666 | 19.006 | 66.295 | 1.00 | 25.54 |
| 551 | CE1 | TYR | A | 753 | 36.683 | 21.093 | 64.759 | 1.00 | 27.58 |
| 552 | CE2 | TYR | A | 753 | 38.496 | 20.067 | 65.947 | 1.00 | 23.54 |
| 553 | CZ | TYR | A | 753 | 38.002 | 21.104 | 65.181 | 1.00 | 22.36 |
| 554 | OH | TYR | A | 753 | 38.840 | 22.145 | 64.843 | 1.00 | 29.42 |
| 555 | N | SER | A | 754 | 33.038 | 15.988 | 66.904 | 1.00 | 19.69 |
| 556 | CA | SER | A | 754 | 32.699 | 14.594 | 67.147 | 1.00 | 21.05 |
| 557 | C | SER | A | 754 | 31.265 | 14.109 | 67.119 | 1.00 | 20.67 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 558 | O | SER | A | 754 | 31.042 | 12.893 | 67.259 | 1.00 | 17.40 |
| 559 | CB | SER | A | 754 | 33.288 | 14.196 | 68.498 | 1.00 | 24.21 |
| 560 | OG | SER | A | 754 | 32.556 | 14.827 | 69.535 | 1.00 | 27.35 |
| 561 | N | TRP | A | 755 | 30.300 | 14.998 | 66.911 | 1.00 | 17.55 |
| 562 | CA | TRP | A | 755 | 28.912 | 14.547 | 66.960 | 1.00 | 21.93 |
| 563 | C | TRP | A | 755 | 28.620 | 13.345 | 66.055 | 1.00 | 18.91 |
| 564 | O | TRP | A | 755 | 27.956 | 12.409 | 66.486 | 1.00 | 20.07 |
| 565 | CB | TRP | A | 755 | 27.925 | 15.684 | 66.647 | 1.00 | 22.52 |
| 566 | CG | TRP | A | 755 | 28.003 | 16.222 | 65.257 | 1.00 | 23.08 |
| 567 | CD1 | TRP | A | 755 | 28.859 | 17.175 | 64.791 | 1.00 | 28.21 |
| 568 | CD2 | TRP | A | 755 | 27.217 | 15.803 | 64.141 | 1.00 | 24.51 |
| 569 | NE1 | TRP | A | 755 | 28.655 | 17.379 | 63.445 | 1.00 | 25.19 |
| 570 | CE2 | TRP | A | 755 | 27.651 | 16.548 | 63.022 | 1.00 | 26.64 |
| 571 | CE3 | TRP | A | 755 | 26.189 | 14.869 | 63.979 | 1.00 | 25.75 |
| 572 | CZ2 | TRP | A | 755 | 27.089 | 16.388 | 61.743 | 1.00 | 29.86 |
| 573 | CZ3 | TRP | A | 755 | 25.630 | 14.707 | 62.707 | 1.00 | 32.59 |
| 574 | CH2 | TRP | A | 755 | 26.083 | 15.465 | 61.608 | 1.00 | 30.85 |
| 575 | N | MET | A | 756 | 29.114 | 13.357 | 64.820 | 1.00 | 21.38 |
| 576 | CA | MET | A | 756 | 28.848 | 12.243 | 63.896 | 1.00 | 18.69 |
| 577 | C | MET | A | 756 | 29.439 | 10.939 | 64.415 | 1.00 | 23.20 |
| 578 | O | MET | A | 756 | 28.794 | 9.878 | 64.350 | 1.00 | 21.19 |
| 579 | CB | MET | A | 756 | 29.432 | 12.529 | 62.511 | 1.00 | 21.49 |
| 580 | CG | MET | A | 756 | 29.112 | 11.430 | 61.496 | 1.00 | 22.22 |
| 581 | SD | MET | A | 756 | 27.367 | 11.449 | 60.920 | 1.00 | 27.03 |
| 582 | CE | MET | A | 756 | 27.451 | 12.902 | 59.772 | 1.00 | 26.64 |
| 583 | N | SER | A | 757 | 30.675 | 11.013 | 64.899 | 1.00 | 19.23 |
| 584 | CA | SER | A | 757 | 31.344 | 9.845 | 65.451 | 1.00 | 22.32 |
| 585 | C | SER | A | 757 | 30.575 | 9.283 | 66.631 | 1.00 | 20.68 |
| 586 | O | SER | A | 757 | 30.376 | 8.078 | 66.718 | 1.00 | 21.09 |
| 587 | CB | SER | A | 757 | 32.759 | 10.190 | 65.911 | 1.00 | 23.54 |
| 588 | OG | SER | A | 757 | 33.562 | 10.611 | 64.826 | 1.00 | 31.88 |
| 589 | N | LEU | A | 758 | 30.150 | 10.149 | 67.548 | 1.00 | 20.06 |
| 590 | CA | LEU | A | 758 | 29.430 | 9.698 | 68.735 | 1.00 | 16.39 |
| 591 | C | LEU | A | 758 | 28.105 | 9.061 | 68.355 | 1.00 | 18.68 |
| 592 | O | LEU | A | 758 | 27.709 | 8.038 | 68.918 | 1.00 | 18.94 |
| 593 | CB | LEU | A | 758 | 29.147 | 10.880 | 69.675 | 1.00 | 14,15 |
| 594 | CG | LEU | A | 758 | 30.373 | 11.599 | 70.232 | 1.00 | 20.53 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 595 | CD1 | LEU | A | 758 | 29.919 | 12.855 | 70.981 | 1.00 | 20.07 |
| 596 | CD2 | LEU | A | 758 | 31.121 | 10.656 | 71.186 | 1.00 | 24.11 |
| 597 | N | MET | A | 759 | 27.410 | 9.674 | 67.404 | 1.00 | 18.42 |
| 598 | CA | MET | A | 759 | 26.125 | 9.149 | 67.001 | 1.00 | 19.21 |
| 599 | C | MET | A | 759 | 26.209 | 7.828 | 66.242 | 1.00 | 19.93 |
| 600 | O | MET | A | 759 | 25.363 | 6.949 | 66.456 | 1.00 | 23.09 |
| 601 | CB | MET | A | 759 | 25.364 | 10.197 | 66.193 | 1.00 | 21.20 |
| 602 | CG | MET | A | 759 | 24.937 | 11.397 | 67.065 | 1.00 | 21.45 |
| 603 | SD | MET | A | 759 | 23.950 | 12.587 | 66.168 | 1.00 | 25.97 |
| 604 | CE | MET | A | 759 | 23.941 | 13.961 | 67.348 | 1.00 | 26.52 |
| 605 | N | VAL | A | 760 | 27.193 | 7.673 | 65.365 | 1.00 | 18.66 |
| 606 | CA | VAL | A | 760 | 27.300 | 6.397 | 64.638 | 1.00 | 19,71 |
| 607 | C | VAL | A | 760 | 27.779 | 5.292 | 65.596 | 1.00 | 22.38 |
| 608 | O | VAL | A | 760 | 27.409 | 4.127 | 65.448 | 1.00 | 18.63 |
| 609 | CB | VAL | A | 760 | 28.262 | 6.492 | 63.417 | 1.00 | 20.60 |
| 610 | CG1 | VAL | A | 760 | 29.708 | 6.659 | 63.860 | 1.00 | 22.90 |
| 611 | CG2 | VAL | A | 760 | 28.129 | 5.226 | 62.559 | 1.00 | 22.05 |
| 612 | N | PHE | A | 761 | 28.597 | 5.672 | 66.572 | 1.00 | 18.16 |
| 613 | CA | PHE | A | 761 | 29.107 | 4.729 | 67.579 | 1.00 | 20.65 |
| 614 | C | PHE | A | 761 | 27.907 | 4.256 | 68.419 | 1.00 | 21.25 |
| 615 | O | PHE | A | 761 | 27.773 | 3.058 | 68.717 | 1.00 | 23.39 |
| 616 | CB | PHE | A | 761 | 30.166 | 5.441 | 68.447 | 1.00 | 19.84 |
| 617 | CG | PHE | A | 761 | 31.100 | 4.502 | 69.206 | 1.00 | 22.22 |
| 618 | CD1 | PHE | A | 761 | 31.944 | 3.631 | 68.520 | 1.00 | 22.72 |
| 619 | CD2 | PHE | A | 761 | 31.158 | 4.529 | 70.597 | 1.00 | 23.08 |
| 620 | CE1 | PHE | A | 761 | 32.834 | 2.802 | 69.200 | 1.00 | 23.74 |
| 621 | CE2 | PHE | A | 761 | 32.044 | 3.706 | 71.297 | 1.00 | 24.95 |
| 622 | CZ | PHE | A | 761 | 32.880 | 2.842 | 70.602 | 1.00 | 22.67 |
| 623 | N | GLY | A | 762 | 27.041 | 5.196 | 68.803 | 1.00 | 18.00 |
| 624 | CA | GLY | A | 762 | 25.851 | 4.861 | 69.564 | 1.00 | 19.15 |
| 625 | C | GLY | A | 762 | 24.928 | 3.957 | 68.761 | 1.00 | 19.60 |
| 626 | O | GLY | A | 762 | 24.304 | 3.038 | 69.306 | 1.00 | 17.94 |
| 627 | N | LEU | A | 763 | 24.815 | 4.241 | 67.465 | 1.00 | 18.18 |
| 628 | CA | LEU | A | 763 | 24.008 | 3.416 | 66.575 | 1.00 | 18.82 |
| 629 | C | LEU | A | 763 | 24.562 | 1.994 | 66.611 | 1.00 | 20.32 |
| 630 | O | LEU | A | 763 | 23.795 | 1.011 | 66.652 | 1.00 | 21.06 |
| 631 | CB | LEU | A | 763 | 24.088 | 3.962 | 65.149 | 1.00 | 18.85 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 632 | CG | LEU | A | 763 | 23.668 | 3.022 | 64.007 | 1.00 | 15.12 |
| 633 | CD1 | LEU | A | 763 | 22.181 | 2.761 | 64.078 | 1.00 | 21.31 |
| 634 | CD2 | LEU | A | 763 | 24.048 | 3.657 | 62.680 | 1.00 | 22.01 |
| 635 | N | GLY | A | 764 | 25.889 | 1.892 | 66.591 | 1.00 | 19.32 |
| 636 | CA | GLY | A | 764 | 26.546 | 0.596 | 66.609 | 1.00 | 22.15 |
| 637 | C | GLY | A | 764 | 26.182 | -0.164 | 67.864 | 1.00 | 22.09 |
| 638 | O | GLY | A | 764 | 25.798 | -1.351 | 67.826 | 1.00 | 18.52 |
| 639 | N | TRP | A | 765 | 26.279 | 0.520 | 68.998 | 1.00 | 18.65 |
| 640 | CA | TRP | A | 765 | 25.954 | -0.118 | 70.265 | 1.00 | 21.70 |
| 641 | C | TRP | A | 765 | 24.485 | -0.582 | 70.330 | 1.00 | 20.69 |
| 642 | O | TRP | A | 765 | 24.202 | -1.710 | 70.730 | 1.00 | 20.73 |
| 643 | CB | TRP | A | 765 | 26.275 | 0.832 | 71.426 | 1.00 | 19.80 |
| 644 | CG | TRP | A | 765 | 25.985 | 0.232 | 72.766 | 1.00 | 20.60 |
| 645 | CD1 | TRP | A | 765 | 24.895 | 0.450 | 73.543 | 1.00 | 26.35 |
| 646 | CD2 | TRP | A | 765 | 26.765 | -0.770 | 73.435 | 1.00 | 22.75 |
| 647 | NE1 | TRP | A | 765 | 24.936 | -0.354 | 74.660 | 1.00 | 25.80 |
| 648 | CE2 | TRP | A | 765 | 26.076 | -1.114 | 74.618 | 1.00 | 27.72 |
| 649 | CE3 | TRP | A | 765 | 27.974 | -1.408 | 73.145 | 1.00 | 24.76 |
| 650 | CZ2 | TRP | A | 765 | 26.558 | -2.080 | 75.522 | 1.00 | 28.33 |
| 651 | CZ3 | TRP | A | 765 | 28.461 | -2.372 | 74.045 | 1.00 | 25.79 |
| 652 | CH2 | TRP | A | 765 | 27.747 | -2.692 | 75.217 | 1.00 | 23.99 |
| 653 | N | ARG | A | 766 | 23.544 | 0.273 | 69.936 | 1.00 | 20.81 |
| 654 | CA | ARG | A | 766 | 22.136 | -0.116 | 69.987 | 1.00 | 18.86 |
| 655 | C | ARG | A | 766 | 21.844 | -1.288 | 69.048 | 1.00 | 18.44 |
| 656 | O | ARG | A | 766 | 21.066 | -2.185 | 69.381 | 1.00 | 20.30 |
| 657 | CB | ARG | A | 766 | 21.223 | 1.061 | 69.624 | 1.00 | 18.28 |
| 658 | CG | ARG | A | 766 | 21.246 | 2.229 | 70.632 | 1.00 | 20.05 |
| 659 | CD | ARG | A | 766 | 20.179 | 3.260 | 70.256 | 1.00 | 25.08 |
| 660 | NE | ARG | A | 766 | 20.413 | 3.889 | 68.956 | 1.00 | 20.13 |
| 661 | CZ | ARG | A | 766 | 21.239 | 4.908 | 68.742 | 1.00 | 22.33 |
| 662 | NH1 | ARG | A | 766 | 21.909 | 5.442 | 69.754 | 1.00 | 22.73 |
| 663 | NH2 | ARG | A | 766 | 21.380 | 5.412 | 67.519 | 1.00 | 19.31 |
| 664 | N | SER | A | 767 | 22.469 | -1.275 | 67.885 | 1.00 | 20.91 |
| 665 | CA | SER | A | 767 | 22.271 | -2.340 | 66.899 | 1.00 | 18.59 |
| 666 | C | SER | A | 767 | 22.793 | -3.660 | 67.473 | 1.00 | 24.76 |
| 667 | O | SER | A | 767 | 22.130 | -4.692 | 67.406 | 1.00 | 19.96 |
| 668 | CB | SER | A | 767 | 23.002 | -1.975 | 65.607 | 1.00 | 19.66 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 669 | OG | SER | A | 767 | 22.444 | -0.786 | 65.077 | 1.00 | 23.01 |
| 670 | N | TYR | A | 768 | 23.977 | -3.595 | 68.067 | 1.00 | 20.64 |
| 671 | CA | TYR | A | 768 | 24.604 | -4.752 | 68.710 | 1.00 | 21.51 |
| 672 | C | TYR | A | 768 | 23.708 | -5.300 | 69.831 | 1.00 | 20.49 |
| 673 | O | TYR | A | 768 | 23.339 | -6.481 | 69.841 | 1.00 | 20.85 |
| 674 | CB | TYR | A | 768 | 25.963 | -4.291 | 69.260 | 1.00 | 20.88 |
| 675 | CG | TYR | A | 768 | 26.629 | -5.164 | 70.298 | 1.00 | 24.41 |
| 676 | CD1 | TYR | A | 768 | 26.882 | -6.520 | 70.072 | 1.00 | 24.71 |
| 677 | CD2 | TYR | A | 768 | 27.126 | -4.590 | 71.463 | 1.00 | 22.00 |
| 678 | CE1 | TYR | A | 768 | 27.642 | -7.273 | 70.993 | 1.00 | 21.58 |
| 679 | CE2 | TYR | A | 768 | 27.872 | -5.325 | 72.370 | 1.00 | 21.74 |
| 680 | CZ | TYR | A | 768 | 28.137 | -6.656 | 72.125 | 1.00 | 24.16 |
| 681 | OH | TYR | A | 768 | 28.969 | -7.309 | 73.000 | 1.00 | 22.86 |
| 682 | N | LYS | A | 769 | 23.313 | -4.427 | 70.751 | 1.00 | 19.61 |
| 683 | CA | LYS | A | 769 | 22.500 | -4.833 | 71.899 | 1.00 | 20.88 |
| 684 | C | LYS | A | 769 | 21.090 | -5.323 | 71.636 | 1.00 | 25.83 |
| 685 | O | LYS | A | 769 | 20.661 | -6.320 | 72.222 | 1.00 | 23.20 |
| 686 | CB | LYS | A | 769 | 22.402 | -3.682 | 72.904 | 1.00 | 26.26 |
| 687 | CG | LYS | A | 769 | 23.682 | -3.356 | 73.623 | 1.00 | 29.74 |
| 688 | CD | LYS | A | 769 | 23.998 | -4.345 | 74.756 | 1.00 | 34.33 |
| 689 | CE | LYS | A | 769 | 23.010 | -4.251 | 75.914 | 1.00 | 31.35 |
| 690 | NZ | LYS | A | 769 | 23.424 | -5.118 | 77.078 | 1.00 | 27.64 |
| 691 | N | HIS | A | 770 | 20.372 | -4.627 | 70.762 | 1.00 | 20.34 |
| 692 | CA | HIS | A | 770 | 18.968 | -4.935 | 70.496 | 1.00 | 25.32 |
| 693 | C | HIS | A | 770 | 18.652 | -5.887 | 69.353 | 1.00 | 24.30 |
| 694 | O | HIS | A | 770 | 17.631 | -6.572 | 69.382 | 1.00 | 23.64 |
| 695 | CB | HIS | A | 770 | 18.204 | -3.622 | 70.246 | 1.00 | 25.43 |
| 696 | CG | HIS | A | 770 | 18.239 | -2.672 | 71.397 | 1.00 | 32.32 |
| 697 | ND1 | HIS | A | 770 | 17.517 | -2.879 | 72.554 | 1.00 | 34.84 |
| 698 | CD2 | HIS | A | 770 | 18.920 | -1.516 | 71.581 | 1.00 | 28.95 |
| 699 | CE1 | HIS | A | 770 | 17.751 | -1.889 | 73.398 | 1.00 | 36.65 |
| 700 | NE2 | HIS | A | 770 | 18.598 | -1.049 | 72.833 | 1.00 | 35.04 |
| 701 | N | VAL | A | 771 | 19.509 | -5.934 | 68.341 | 1.00 | 24.39 |
| 702 | CA | VAL | A | 771 | 19.231 | -6.795 | 67.202 | 1.00 | 25.76 |
| 703 | C | VAL | A | 771 | 20.441 | -7.614 | 66.787 | 1.00 | 24.92 |
| 704 | O | VAL | A | 771 | 20.568 | -8.008 | 65.639 | 1.00 | 27.56 |
| 705 | CB | VAL | A | 771 | 18.687 | -5.952 | 65.995 | 1.00 | 27.85 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 706 | CG1 | VAL | A | 771 | 17.295 | -5.404 | 66.320 | 1.00 | 29.87 |
| 707 | CG2 | VAL | A | 771 | 19.606 | -4.767 | 65.718 | 1.00 | 28.77 |
| 708 | N | SER | A | 772 | 21.324 | -7.883 | 67.745 | 1.00 | 22.68 |
| 709 | CA | SER | A | 772 | 22.532 | -8.669 | 67.495 | 1.00 | 24.24 |
| 710 | C | SER | A | 772 | 23.352 | -8.197 | 66.311 | 1.00 | 22.19 |
| 711 | O | SER | A | 772 | 24.033 | -8.994 | 65.653 | 1.00 | 24.35 |
| 712 | CB | SER | A | 772 | 22.167 | -10.153 | 67.332 | 1.00 | 22.79 |
| 713 | OG | SER | A | 772 | 21.539 | -10.582 | 68.518 | 1.00 | 24.02 |
| 714 | N | GLY | A | 773 | 23.299 | -6.886 | 66.062 | 1.00 | 20.76 |
| 715 | CA | GLY | A | 773 | 24.066 | -6.290 | 64.982 | 1.00 | 21.80 |
| 716 | C | GLY | A | 773 | 23.555 | -6.616 | 63.595 | 1.00 | 20.80 |
| 717 | O | GLY | A | 773 | 24.199 | -6.274 | 62.604 | 1.00 | 25.34 |
| 718 | N | GLN | A | 774 | 22.386 | -7.234 | 63.518 | 1.00 | 23.07 |
| 719 | CA | GLN | A | 774 | 21.845 | -7.632 | 62.226 | 1.00 | 23.42 |
| 720 | C | GLN | A | 774 | 20.901 | -6.638 | 61.538 | 1.00 | 25.60 |
| 721 | O | GLN | A | 774 | 20.414 | -6.906 | 60.440 | 1.00 | 23.56 |
| 722 | CB | GLN | A | 774 | 21.174 | -9.000 | 62.365 | 1.00 | 25.32 |
| 723 | CG | GLN | A | 774 | 22.103 | -10.032 | 62.943 | 1.00 | 24.41 |
| 724 | CD | GLN | A | 774 | 23.443 | -10.066 | 62.236 | 1.00 | 34.16 |
| 725 | OE1 | GLN | A | 774 | 23.514 | -10.285 | 61.030 | 1.00 | 40.94 |
| 726 | NE2 | GLN | A | 774 | 24.517 | -9.844 | 62.987 | 1.00 | 33.82 |
| 727 | N | MET | A | 775 | 20.625 | -5.513 | 62.190 | 1.00 | 21.96 |
| 728 | CA | MET | A | 775 | 19.805 | -4.447 | 61.603 | 1.00 | 22.20 |
| 729 | C | MET | A | 775 | 20.398 | -3.172 | 62.206 | 1.00 | 24.08 |
| 730 | O | MET | A | 775 | 21.132 | -3.256 | 63.188 | 1.00 | 21.84 |
| 731 | CB | MET | A | 775 | 18.332 | -4.566 | 62.011 | 1.00 | 23.95 |
| 732 | CG | MET | A | 775 | 17.635 | -5.838 | 61.531 | 1.00 | 28.07 |
| 733 | SD | MET | A | 775 | 15.873 | -5.735 | 61.844 | 1.00 | 37.34 |
| 734 | CE | MET | A | 775 | 15.340 | -7.409 | 61.263 | 1.00 | 37.01 |
| 735 | N | LEU | A | 776 | 20.115 | -2.014 | 61.612 | 1.00 | 21.78 |
| 736 | CA | LEU | A | 776 | 20.621 | -0.743 | 62.158 | 1.00 | 22.28 |
| 737 | C | LEU | A | 776 | 19.518 | -0.144 | 63.016 | 1.00 | 20.07 |
| 738 | O | LEU | A | 776 | 18.489 | 0.318 | 62.520 | 1.00 | 21.05 |
| 739 | CB | LEU | A | 776 | 21.044 | 0.217 | 61.039 | 1.00 | 21.10 |
| 740 | CG | LEU | A | 776 | 22.256 | -0.267 | 60.223 | 1.00 | 24.71 |
| 741 | CD1 | LEU | A | 776 | 22.648 | 0.758 | 59.184 | 1.00 | 26.09 |
| 742 | CD2 | LEU | A | 776 | 23.429 | -0.534 | 61.159 | 1.00 | 23.43 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 743 | N | TYR | A | 777 | 19.733 | -0.180 | 64.322 | 1.00 | 20.52 |
| 744 | CA | TYR | A | 777 | 18.755 | 0.303 | 65.278 | 1.00 | 17.48 |
| 745 | C | TYR | A | 777 | 18.967 | 1.804 | 65.547 | 1.00 | 21.46 |
| 746 | O | TYR | A | 777 | 19.525 | 2.184 | 66.565 | 1.00 | 18.14 |
| 747 | CB | TYR | A | 777 | 18.902 | -0.521 | 66.566 | 1.00 | 20.38 |
| 748 | CG | TYR | A | 777 | 17.768 | -0.416 | 67.572 | 1.00 | 22.57 |
| 749 | CD1 | TYR | A | 777 | 17.608 | 0.717 | 68.373 | 1.00 | 22.04 |
| 750 | CD2 | TYR | A | 777 | 16.877 | -1.474 | 67.748 | 1.00 | 25.77 |
| 751 | CE1 | TYR | A | 777 | 16.580 | 0.782 | 69.335 | 1.00 | 22.56 |
| 752 | CE2 | TYR | A | 777 | 15.859 | -1.421 | 68.693 | 1.00 | 25.86 |
| 753 | CZ | TYR | A | 777 | 15.716 | -0.299 | 69.485 | 1.00 | 23.25 |
| 754 | OH | TYR | A | 777 | 14.721 | -0.279 | 70.440 | 1.00 | 24.58 |
| 755 | N | PHE | A | 778 | 18.527 | 2.649 | 64.616 | 1.00 | 20.19 |
| 756 | CA | PHE | A | 778 | 18.677 | 4.091 | 64.796 | 1.00 | 22.33 |
| 757 | C | PHE | A | 778 | 17.888 | 4.572 | 66.019 | 1.00 | 19.77 |
| 758 | O | PHE | A | 778 | 18.369 | 5.373 | 66.817 | 1.00 | 20.69 |
| 759 | CB | PHE | A | 778 | 18.233 | 4.843 | 63.526 | 1.00 | 17.81 |
| 760 | CG | PHE | A | 778 | 19.170 | 4.685 | 62.380 | 1.00 | 19.78 |
| 761 | CD1 | PHE | A | 778 | 19.038 | 3.633 | 61.485 | 1.00 | 26.89 |
| 762 | CD2 | PHE | A | 778 | 20.230 | 5.569 | 62.211 | 1.00 | 21.49 |
| 763 | CE1 | PHE | A | 778 | 19.963 | 3.474 | 60.432 | 1.00 | 24.89 |
| 764 | CE2 | PHE | A | 778 | 21.151 | 5.413 | 61.168 | 1.00 | 21.92 |
| 765 | CZ | PHE | A | 778 | 21.016 | 4.366 | 60.277 | 1.00 | 25.29 |
| 766 | N | ALA | A | 779 | 16.672 | 4.079 | 66.169 | 1.00 | 18.84 |
| 767 | CA | ALA | A | 779 | 15.836 | 4.438 | 67.307 | 1.00 | 18.94 |
| 768 | C | ALA | A | 779 | 14.808 | 3.318 | 67.390 | 1.00 | 23.24 |
| 769 | O | ALA | A | 779 | 14.714 | 2.503 | 66.471 | 1.00 | 21.88 |
| 770 | CB | ALA | A | 779 | 15.151 | 5.785 | 67.063 | 1.00 | 19.00 |
| 771 | N | PRO | A | 780 | 14.074 | 3.224 | 68.501 | 1.00 | 24.08 |
| 772 | CA | PRO | A | 780 | 13.061 | 2.176 | 68.645 | 1.00 | 22.75 |
| 773 | C | PRO | A | 780 | 11.985 | 2.260 | 67.551 | 1.00 | 31.17 |
| 774 | O | PRO | A | 780 | 11.405 | 1.242 | 67.163 | 1.00 | 27.19 |
| 775 | CB | PRO | A | 780 | 12.506 | 2.451 | 70.039 | 1.00 | 23.42 |
| 776 | CG | PRO | A | 780 | 13.723 | 3.011 | 70.760 | 1.00 | 29.68 |
| 777 | CD | PRO | A | 780 | 14.122 | 4.036 | 69.731 | 1.00 | 19.66 |
| 778 | N | ASP | A | 781 | 11.728 | 3.465 | 67.045 | 1.00 | 24.25 |
| 779 | CA | ASP | A | 781 | 10.722 | 3.643 | 65.995 | 1.00 | 30.35 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 780 | C | ASP | A | 781 | 11.345 | 3.803 | 64.608 | 1.00 | 28.45 |
| 781 | O | ASP | A | 781 | 10.666 | 4.154 | 63.631 | 1.00 | 31.54 |
| 782 | CB | ASP | A | 781 | 9.856 | 4.854 | 66.328 | 1.00 | 33.03 |
| 783 | CG | ASP | A | 781 | 10.648 | 6.149 | 66.354 | 1.00 | 42.61 |
| 784 | OD1 | ASP | A | 781 | 11.799 | 6.152 | 66.847 | 1.00 | 37.30 |
| 785 | OD2 | ASP | A | 781 | 10.105 | 7.175 | 65.899 | 1.00 | 39.66 |
| 786 | N | LEU | A | 782 | 12.640 | 3.528 | 64.518 | 1.00 | 25.42 |
| 787 | CA | LEU | A | 782 | 13.352 | 3.654 | 63.264 | 1.00 | 23.61 |
| 788 | C | LEU | A | 782 | 14.483 | 2.619 | 63.221 | 1.00 | 25.27 |
| 789 | O | LEU | A | 782 | 15.635 | 2.899 | 63.548 | 1.00 | 21.60 |
| 790 | CB | LEU | A | 782 | 13.907 | 5.072 | 63.134 | 1.00 | 25.81 |
| 791 | CG | LEU | A | 782 | 14.296 | 5.483 | 61.718 | 1.00 | 26.41 |
| 792 | CD1 | LEU | A | 782 | 13.086 | 5.260 | 60.823 | 1.00 | 37.20 |
| 793 | CD2 | LEU | A | 782 | 14.693 | 6.947 | 61.682 | 1.00 | 23.25 |
| 794 | N | ILE | A | 783 | 14.117 | 1.401 | 62.844 | 1.00 | 20.00 |
| 795 | CA | ILE | A | 783 | 15.048 | 0.298 | 62.742 | 1.00 | 21.82 |
| 796 | C | ILE | A | 783 | 15.144 | -0.054 | 61.258 | 1.00 | 26.37 |
| 797 | O | ILE | A | 783 | 14.125 | -0.343 | 60.640 | 1.00 | 27.10 |
| 798 | CB | ILE | A | 783 | 14.500 | -0.924 | 63.496 | 1.00 | 19.86 |
| 799 | CG1 | ILE | A | 783 | 14.240 | -0.558 | 64.957 | 1.00 | 23.93 |
| 800 | CG2 | ILE | A | 783 | 15.491 | -2.086 | 63.374 | 1.00 | 26.69 |
| 801 | CD1 | ILE | A | 783 | 13.358 | -1.543 | 65.718 | 1.00 | 22.62 |
| 802 | N | LEU | A | 784 | 16.346 | -0.041 | 60.690 | 1.00 | 24.00 |
| 803 | CA | LEU | A | 784 | 16.496 | -0.369 | 59.277 | 1.00 | 30.39 |
| 804 | C | LEU | A | 784 | 17.146 | -1.717 | 58.979 | 1.00 | 28.22 |
| 805 | O | LEU | A | 784 | 18.156 | -2.095 | 59.583 | 1.00 | 27.38 |
| 806 | CB | LEU | A | 784 | 17.296 | 0.715 | 58.547 | 1.00 | 27.74 |
| 807 | CG | LEU | A | 784 | 16.753 | 2.148 | 58.588 | 1.00 | 33.96 |
| 808 | CD1 | LEU | A | 784 | 17.619 | 3.030 | 57.678 | 1.00 | 30.69 |
| 809 | CD2 | LEU | A | 784 | 15.306 | 2.182 | 58.142 | 1.00 | 33.34 |
| 810 | N | ASN | A | 785 | 16.545 | -2.421 | 58.022 | 1.00 | 37.87 |
| 811 | CA | ASN | A | 785 | 17.041 | -3.708 | 57.537 | 1.00 | 34.28 |
| 812 | C | ASN | A | 785 | 17.310 | -3.491 | 56.050 | 1.00 | 40.43 |
| 813 | O | ASN | A | 785 | 16.947 | -2.442 | 55.492 | 1.00 | 32.88 |
| 814 | CB | ASN | A | 785 | 15.998 | -4.814 | 57.720 | 1.00 | 43.78 |
| 815 | CG | ASN | A | 785 | 14.679 | -4.482 | 57.058 | 1.00 | 39.05 |
| 816 | OD1 | ASN | A | 785 | 14.644 | -4.013 | 55.922 | 1.00 | 56.25 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 817 | ND2 | ASN | A | 785 | 13.584 | -4.740 | 57.758 | 1.00 | 55.74 |
| 818 | N | GLU | A | 786 | 17.945 | -4.475 | 55.419 | 1.00 | 37.34 |
| 819 | CA | GLU | A | 786 | 18.290 | -4.406 | 54.001 | 1.00 | 41.84 |
| 820 | C | GLU | A | 786 | 17.137 | -3.983 | 53.103 | 1.00 | 36.02 |
| 821 | O | GLU | A | 786 | 17.332 | -3.187 | 52.190 | 1.00 | 37.52 |
| 822 | CB | GLU | A | 786 | 18.829 | -5.753 | 53.508 | 1.00 | 43.26 |
| 823 | CG | GLU | A | 786 | 20.141 | -6.218 | 54.140 | 1.00 | 53.06 |
| 824 | CD | GLU | A | 786 | 19.987 | -6.714 | 55.569 | 1.00 | 52.18 |
| 825 | OE1 | GLU | A | 786 | 18.877 | -6.622 | 56.135 | 1.00 | 55.65 |
| 826 | OE2 | GLU | A | 786 | 20.990 | -7.206 | 56.128 | 1.00 | 59.59 |
| 827 | N | GLN | A | 787 | 15.945 | -4.515 | 53.360 | 1.00 | 37.37 |
| 828 | CA | GLN | A | 787 | 14.757 | -4.194 | 52.563 | 1.00 | 41.31 |
| 829 | C | GLN | A | 787 | 14.403 | -2.705 | 52.523 | 1.00 | 44.67 |
| 830 | O | GLN | A | 787 | 13.863 | -2.214 | 51.529 | 1.00 | 39.36 |
| 831 | CB | GLN | A | 787 | 13.541 | -4.954 | 53.094 | 1.00 | 39.63 |
| 832 | CG | GLN | A | 787 | 13.659 | -6.470 | 53.044 | 1.00 | 54.67 |
| 833 | CD | GLN | A | 787 | 12.516 | -7.161 | 53.765 | 1.00 | 54.04 |
| 834 | OE1 | GLN | A | 787 | 11.359 | -7.095 | 53.343 | 1.00 | 65.04 |
| 835 | NE2 | GLN | A | 787 | 12.835 | -7.819 | 54.871 | 1.00 | 58.37 |
| 836 | N | ARG | A | 788 | 14.709 | -1.989 | 53.600 | 1.00 | 42.38 |
| 837 | CA | ARG | A | 788 | 14.379 | -0.570 | 53.680 | 1.00 | 46.01 |
| 838 | C | ARG | A | 788 | 15.474 | 0.355 | 53.157 | 1.00 | 44.06 |
| 839 | O | ARG | A | 788 | 15.307 | 1.576 | 53.123 | 1.00 | 43.28 |
| 840 | CB | ARG | A | 788 | 14.020 | -0.219 | 55.129 | 1.00 | 45.62 |
| 841 | CG | ARG | A | 788 | 12.956 | -1.149 | 55.694 | 1.00 | 51.99 |
| 842 | CD | ARG | A | 788 | 12.554 | -0.827 | 57.121 | 1.00 | 54.00 |
| 843 | NE | ARG | A | 788 | 11.834 | 0.440 | 57.238 | 1.00 | 62.20 |
| 844 | CZ | ARG | A | 788 | 11.303 | 0.893 | 58.372 | 1.00 | 62.82 |
| 845 | NH1 | ARG | A | 788 | 11.411 | 0.184 | 59.490 | 1.00 | 63.08 |
| 846 | NH2 | ARG | A | 788 | 10.659 | 2.052 | 58.391 | 1.00 | 64.33 |
| 847 | N | MET | A | 789 | 16.589 | -0.226 | 52.738 | 1.00 | 42.60 |
| 848 | CA | MET | A | 789 | 17.690 | 0.563 | 52.207 | 1.00 | 44.82 |
| 849 | C | MET | A | 789 | 17.687 | 0.382 | 50.697 | 1.00 | 44.81 |
| 850 | O | MET | A | 789 | 18.545 | -0.303 | 50.143 | 1.00 | 41.79 |
| 851 | CB | MET | A | 789 | 19.013 | 0.072 | 52.791 | 1.00 | 47.11 |
| 852 | CG | MET | A | 789 | 19.047 | 0.097 | 54.307 | 1.00 | 38.54 |
| 853 | SD | MET | A | 789 | 20.519 | -0.711 | 54.954 | 1.00 | 40.15 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 854 | CE | MET | A | 789 | 20.192 | -0.603 | 56.713 | 1.00 | 40.02 |
| 855 | N | LYS | A | 790 | 16.706 | 0.990 | 50.040 | 1.00 | 45.90 |
| 856 | CA | LYS | A | 790 | 16.575 | 0.879 | 48.591 | 1.00 | 49.64 |
| 857 | C | LYS | A | 790 | 17.686 | 1.632 | 47.883 | 1.00 | 52.38 |
| 858 | O | LYS | A | 790 | 18.423 | 1.065 | 47.073 | 1.00 | 56.13 |
| 859 | CB | LYS | A | 790 | 15.225 | 1.435 | 48.148 | 1.00 | 52.46 |
| 860 | CG | LYS | A | 790 | 14.047 | 0.799 | 48.844 | 1.00 | 50.21 |
| 861 | CD | LYS | A | 790 | 12.752 | 1.440 | 48.405 | 1.00 | 55.50 |
| 862 | CE | LYS | A | 790 | 11.583 | 0.875 | 49.176 | 1.00 | 54.17 |
| 863 | NZ | LYS | A | 790 | 10.334 | 1.600 | 48.845 | 1.00 | 57.04 |
| 864 | N | GLU | A | 791 | 17.782 | 2.920 | 48.194 | 1.00 | 54.04 |
| 865 | CA | GLU | A | 791 | 18.780 | 3.817 | 47.621 | 1.00 | 54.00 |
| 866 | C | GLU | A | 791 | 20.162 | 3.174 | 47.656 | 1.00 | 52.73 |
| 867 | O | GLU | A | 791 | 20.689 | 2.887 | 48.728 | 1.00 | 52.98 |
| 868 | CB | GLU | A | 791 | 18.792 | 5.116 | 48.427 | 1.00 | 61.18 |
| 869 | CG | GLU | A | 791 | 19.609 | 6.246 | 47.844 | 1.00 | 67.64 |
| 870 | CD | GLU | A | 791 | 19.632 | 7.444 | 48.769 | 1.00 | 74.88 |
| 871 | OE1 | GLU | A | 791 | 18.547 | 7.856 | 49.241 | 1.00 | 78.04 |
| 872 | OE2 | GLU | A | 791 | 20.733 | 7.979 | 49.017 | 1.00 | 77.72 |
| 873 | N | SER | A | 792 | 20.755 | 2.947 | 46.490 | 1.00 | 47.20 |
| 874 | CA | SER | A | 792 | 22.074 | 2.326 | 46.439 | 1.00 | 47.93 |
| 875 | C | SER | A | 792 | 23.177 | 3.113 | 47.168 | 1.00 | 45.20 |
| 876 | O | SER | A | 792 | 24.041 | 2.507 | 47.813 | 1.00 | 41.96 |
| 877 | CB | SER | A | 792 | 22.475 | 2.064 | 44.979 | 1.00 | 47.55 |
| 878 | OG | SER | A | 792 | 22.355 | 3.235 | 44.193 | 1.00 | 54.54 |
| 879 | N | SER | A | 793 | 23.153 | 4.448 | 47.077 | 1.00 | 41.56 |
| 880 | CA | SER | A | 793 | 24.175 | 5.262 | 47.746 | 1.00 | 34.71 |
| 881 | C | SER | A | 793 | 24.018 | 5.190 | 49.268 | 1.00 | 33.35 |
| 882 | O | SER | A | 793 | 25.009 | 5.091 | 49.988 | 1.00 | 30.89 |
| 883 | CB | SER | A | 793 | 24.100 | 6.728 | 47.289 | 1.00 | 36.41 |
| 884 | OG | SER | A | 793 | 22.859 | 7.318 | 47.626 | 1.00 | 41.06 |
| 885 | N | PHE | A | 794 | 22.774 | 5.227 | 49.739 | 1.00 | 32.63 |
| 886 | CA | PHE | A | 794 | 22.492 | 5.165 | 51.170 | 1.00 | 35.71 |
| 887 | C | PHE | A | 794 | 22.762 | 3.762 | 51.725 | 1.00 | 35.32 |
| 888 | O | PHE | A | 794 | 23.256 | 3.613 | 52.839 | 1.00 | 33.54 |
| 889 | CB | PHE | A | 794 | 21.044 | 5.564 | 51.447 | 1.00 | 42.64 |
| 890 | CG | PHE | A | 794 | 20.716 | 5.627 | 52.906 | 1.00 | 46.25 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 891 | CD1 | PHE | A | 794 | 21.441 | 6.457 | 53.751 | 1.00 | 47.24 |
| 892 | CD2 | PHE | A | 794 | 19.714 | 4.832 | 53.442 | 1.00 | 50.84 |
| 893 | CE1 | PHE | A | 794 | 21.176 | 6.495 | 55.118 | 1.00 | 52.49 |
| 894 | CE2 | PHE | A | 794 | 19.441 | 4.862 | 54.805 | 1.00 | 52.13 |
| 895 | CZ | PHE | A | 794 | 20.177 | 5.696 | 55.643 | 1.00 | 44.92 |
| 896 | N | TYR | A | 795 | 22.435 | 2.738 | 50.942 | 1.00 | 31.90 |
| 897 | CA | TYR | A | 795 | 22.687 | 1.355 | 51.356 | 1.00 | 28.99 |
| 898 | C | TYR | A | 795 | 24.184 | 1.203 | 51.510 | 1.00 | 25.10 |
| 899 | O | TYR | A | 795 | 24.671 | 0.609 | 52.465 | 1.00 | 29.55 |
| 900 | CB | TYR | A | 795 | 22.183 | 0.378 | 50.287 | 1.00 | 37.34 |
| 901 | CG | TYR | A | 795 | 22.478 | -1.079 | 50.578 | 1.00 | 40.29 |
| 902 | CD1 | TYR | A | 795 | 21.891 | -1.730 | 51.666 | 1.00 | 40.54 |
| 903 | CD2 | TYR | A | 795 | 23.331 | -1.810 | 49.753 | 1.00 | 42.80 |
| 904 | CE1 | TYR | A | 795 | 22.145 | -3.068 | 51.923 | 1.00 | 42.21 |
| 905 | CE2 | TYR | A | 795 | 23.592 | -3.154 | 50.001 | 1.00 | 45.44 |
| 906 | CZ | TYR | A | 795 | 22.992 | -3.779 | 51.087 | 1.00 | 44.14 |
| 907 | OH | TYR | A | 795 | 23.225 | -5.116 | 51.323 | 1.00 | 48.25 |
| 908 | N | SER | A | 796 | 24.937 | 1.757 | 50.571 | 1.00 | 24.54 |
| 909 | CA | SER | A | 796 | 26.373 | 1.631 | 50.665 | 1.00 | 25.44 |
| 910 | C | SER | A | 796 | 26.866 | 2.323 | 51.932 | 1.00 | 28.08 |
| 911 | O | SER | A | 796 | 27.807 | 1.855 | 52.569 | 1.00 | 31.55 |
| 912 | CB | SER | A | 796 | 27.053 | 2.239 | 49.439 | 1.00 | 32.47 |
| 913 | OG | SER | A | 796 | 28.460 | 2.133 | 49.564 | 1.00 | 43.41 |
| 914 | N | LEU | A | 797 | 26.239 | 3.438 | 52.295 | 1.00 | 25.12 |
| 915 | CA | LEU | A | 797 | 26.665 | 4.141 | 53.503 | 1.00 | 25.95 |
| 916 | C | LEU | A | 797 | 26.302 | 3.314 | 54.736 | 1.00 | 27.16 |
| 917 | O | LEU | A | 797 | 27.044 | 3.292 | 55.716 | 1.00 | 29.13 |
| 918 | CB | LEU | A | 797 | 26.010 | 5.520 | 53.603 | 1.00 | 32.01 |
| 919 | CG | LEU | A | 797 | 26.631 | 6.398 | 54.704 | 1.00 | 30.49 |
| 920 | CD1 | LEU | A | 797 | 28.039 | 6.772 | 54.291 | 1.00 | 36.98 |
| 921 | CD2 | LEU | A | 797 | 25.822 | 7.646 | 54.917 | 1.00 | 32.88 |
| 922 | N | CYS | A | 798 | 25.152 | 2.650 | 54.695 | 1.00 | 26.93 |
| 923 | CA | CYS | A | 798 | 24.736 | 1.811 | 55.810 | 1.00 | 28.14 |
| 924 | C | CYS | A | 798 | 25.681 | 0.634 | 56.017 | 1.00 | 24.65 |
| 925 | O | CYS | A | 798 | 25.953 | 0.253 | 57.157 | 1.00 | 25.73 |
| 926 | CB | CYS | A | 798 | 23.306 | 1.308 | 55.607 | 1.00 | 24.73 |
| 927 | SG | CYS | A | 798 | 22.054 | 2.594 | 55.812 | 1.00 | 32.75 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 928 | N | LEU | A | 799 | 26.181 | 0.054 | 54.925 | 1.00 | 25.25 |
| 929 | CA | LEU | A | 799 | 27.124 | -1.059 | 55.034 | 1.00 | 26.29 |
| 930 | C | LEU | A | 799 | 28.387 | -0.603 | 55.732 | 1.00 | 27.01 |
| 931 | O | LEU | A | 799 | 29.047 | -1.374 | 56.441 | 1.00 | 27.73 |
| 932 | CB | LEU | A | 799 | 27.492 | -1.602 | 53.654 | 1.00 | 28.72 |
| 933 | CG | LEU | A | 799 | 26.395 | -2.303 | 52.861 | 1.00 | 35.76 |
| 934 | CD1 | LEU | A | 799 | 26.980 | -2.774 | 51.524 | 1.00 | 38.62 |
| 935 | CD2 | LEU | A | 799 | 25.859 | -3.485 | 53.660 | 1.00 | 34.72 |
| 936 | N | THR | A | 800 | 28.743 | 0.655 | 55.501 | 1.00 | 28.68 |
| 937 | CA | THR | A | 800 | 29.918 | 1.242 | 56.125 | 1.00 | 27.55 |
| 938 | C | THR | A | 800 | 29.636 | 1.394 | 57.612 | 1.00 | 20.19 |
| 939 | O | THR | A | 800 | 30.487 | 1.090 | 58.447 | 1.00 | 28.87 |
| 940 | CB | THR | A | 800 | 30.222 | 2.620 | 55.503 | 1.00 | 37.30 |
| 941 | OG1 | THR | A | 800 | 30.660 | 2.421 | 54.153 | 1.00 | 34.67 |
| 942 | CG2 | THR | A | 800 | 31.290 | 3.374 | 56.302 | 1.00 | 32.14 |
| 943 | N | MET | A | 801 | 28.442 | 1.871 | 57.935 | 1.00 | 20.28 |
| 944 | CA | MET | A | 801 | 28.063 | 2.042 | 59.333 | 1.00 | 23.80 |
| 945 | C | MET | A | 801 | 27.990 | 0.679 | 60.017 | 1.00 | 25.24 |
| 946 | O | MET | A | 801 | 28.380 | 0.535 | 61.173 | 1.00 | 23.42 |
| 947 | CB | MET | A | 801 | 26.705 | 2.750 | 59.439 | 1.00 | 23.19 |
| 948 | CG | MET | A | 801 | 26.745 | 4.207 | 58.953 | 1.00 | 25.74 |
| 949 | SD | MET | A | 801 | 25.204 | 5.075 | 59.342 | 1.00 | 29.64 |
| 950 | CE | MET | A | 801 | 24.118 | 4.431 | 58.116 | 1.00 | 40.66 |
| 951 | N | TRP | A | 802 | 27.512 | -0.318 | 59.280 | 1.00 | 27.17 |
| 952 | CA | TRP | A | 802 | 27.352 | -1.674 | 59.804 | 1.00 | 27.11 |
| 953 | C | TRP | A | 802 | 28.642 | -2.297 | 60.328 | 1.00 | 28.77 |
| 954 | O | TRP | A | 802 | 28.617 | -3.235 | 61.132 | 1.00 | 26.96 |
| 955 | CB | TRP | A | 802 | 26.764 | -2.588 | 58.733 | 1.00 | 27.64 |
| 956 | CG | TRP | A | 802 | 25.504 | -3.273 | 59.178 | 1.00 | 29.69 |
| 957 | CD1 | TRP | A | 802 | 25.309 | -3.977 | 60.341 | 1.00 | 24.68 |
| 958 | CD2 | TRP | A | 802 | 24.261 | -3.308 | 58.475 | 1.00 | 27.66 |
| 959 | NE1 | TRP | A | 802 | 24.019 | -4.441 | 60.398 | 1.00 | 31.31 |
| 960 | CE2 | TRP | A | 802 | 23.352 | -4.047 | 59.268 | 1.00 | 31.04 |
| 961 | CE3 | TRP | A | 802 | 23.822 | -2.785 | 57.248 | 1.00 | 37.29 |
| 962 | CZ2 | TRP | A | 802 | 22.028 | -4.278 | 58.874 | 1.00 | 31.47 |
| 963 | CZ3 | TRP | A | 802 | 22.503 | -3.012 | 56.855 | 1.00 | 25.61 |
| 964 | CH2 | TRP | A | 802 | 21.622 | -3.754 | 57.670 | 1.00 | 33.86 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 965 | N | GLN | A | 803 | 29.772 | -1.775 | 59.870 | 1.00 | 27.01 |
| 966 | CA | GLN | A | 803 | 31.066 | -2.280 | 60.304 | 1.00 | 29.15 |
| 967 | C | GLN | A | 803 | 31.242 | -2.199 | 61.810 | 1.00 | 27.44 |
| 968 | O | GLN | A | 803 | 31.895 | -3.052 | 62.408 | 1.00 | 26.00 |
| 969 | CB | GLN | A | 803 | 32.190 | -1.492 | 59.648 | 1.00 | 27.84 |
| 970 | CG | GLN | A | 803 | 32.293 | -1.680 | 58.156 | 1.00 | 27.53 |
| 971 | CD | GLN | A | 803 | 33.392 | -0.813 | 57.564 | 1.00 | 37.98 |
| 972 | OE1 | GLN | A | 803 | 33.273 | 0.414 | 57.508 | 1.00 | 41.49 |
| 973 | NE2 | GLN | A | 803 | 34.477 | -1.446 | 57.145 | 1.00 | 37.86 |
| 974 | N | ILE | A | 804 | 30.683 | -1.163 | 62.424 | 1.00 | 23.42 |
| 975 | CA | ILE | A | 804 | 30.825 | -1.016 | 63.861 | 1.00 | 25.19 |
| 976 | C | ILE | A | 804 | 30.106 | -2.116 | 64.658 | 1.00 | 22.21 |
| 977 | O | ILE | A | 804 | 30.737 | -2.795 | 65.467 | 1.00 | 28.86 |
| 978 | CB | ILE | A | 804 | 30.353 | 0.379 | 64.330 | 1.00 | 25.31 |
| 979 | CG1 | ILE | A | 804 | 31.085 | 1.467 | 63.531 | 1.00 | 27.19 |
| 980 | CG2 | ILE | A | 804 | 30.639 | 0.537 | 65.826 | 1.00 | 29.31 |
| 981 | CD1 | ILE | A | 804 | 30.774 | 2.893 | 63.988 | 1.00 | 29.28 |
| 982 | N | PRO | A | 805 | 28.790 | -2.309 | 64.455 | 1.00 | 22.89 |
| 983 | CA | PRO | A | 805 | 28.092 | -3.366 | 65.211 | 1.00 | 26.67 |
| 984 | C | PRO | A | 805 | 28.769 | -4.716 | 64.986 | 1.00 | 25.60 |
| 985 | O | PRO | A | 805 | 28.815 | -5.568 | 65.879 | 1.00 | 25.13 |
| 986 | CB | PRO | A | 805 | 26.686 | -3.353 | 64.602 | 1.00 | 22.17 |
| 987 | CG | PRO | A | 805 | 26.531 | -1.937 | 64.165 | 1.00 | 33.89 |
| 988 | CD | PRO | A | 805 | 27.852 | -1.659 | 63.522 | 1.00 | 25.28 |
| 989 | N | GLN | A | 806 | 29.287 | -4.903 | 63.773 | 1.00 | 29.12 |
| 990 | CA | GLN | A | 806 | 29.971 | -6.144 | 63.416 | 1.00 | 28.65 |
| 991 | C | GLN | A | 806 | 31.202 | -6.364 | 64.259 | 1.00 | 30.22 |
| 992 | O | GLN | A | 806 | 31.441 | -7.478 | 64.714 | 1.00 | 28.51 |
| 993 | CB | GLN | A | 806 | 30.348 | -6.156 | 61.928 | 1.00 | 33.89 |
| 994 | CG | GLN | A | 806 | 29.136 | -6.069 | 61.024 | 1.00 | 40.04 |
| 995 | CD | GLN | A | 806 | 28.130 | -7.161 | 61.297 | 1.00 | 51.23 |
| 996 | OE1 | GLN | A | 806 | 27.651 | -7.316 | 62.425 | 1.00 | 56.13 |
| 997 | NE2 | GLN | A | 806 | 27.787 | -7.923 | 60.260 | 1.00 | 58.14 |
| 998 | N | GLU | A | 807 | 31.986 | -5.312 | 64.477 | 1.00 | 25.11 |
| 999 | CA | GLU | A | 807 | 33.174 | -5.446 | 65.299 | 1.00 | 25.25 |
| 1000 | C | GLU | A | 807 | 32.775 | -5.606 | 66.742 | 1.00 | 21.49 |
| 1001 | O | GLU | A | 807 | 33.476 | -6.286 | 67.484 | 1.00 | 25.66 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1002 | CB | GLU | A | 807 | 34.120 | -4.249 | 65.122 | 1.00 | 28.40 |
| 1003 | CG | GLU | A | 807 | 34.779 | -4.252 | 63.762 | 1.00 | 41.59 |
| 1004 | CD | GLU | A | 807 | 35.673 | -5.480 | 63.565 | 1.00 | 49.95 |
| 1005 | OE1 | GLU | A | 807 | 36.146 | -6.046 | 64.576 | 1.00 | 44.78 |
| 1006 | OE2 | GLU | A | 807 | 35.920 | -5.871 | 62.401 | 1.00 | 51 .07 |
| 1007 | N | PHE | A | 808 | 31.651 | -4.992 | 67.131 | 1.00 | 24.33 |
| 1008 | CA | PHE | A | 808 | 31.143 | -5.090 | 68.500 | 1.00 | 26.06 |
| 1009 | C | PHE | A | 808 | 30.783 | -6.533 | 68.780 | 1.00 | 26.07 |
| 1010 | O | PHE | A | 808 | 31.086 | -7.052 | 69.845 | 1.00 | 22.03 |
| 1011 | CB | PHE | A | 808 | 29.905 | -4.197 | 68.722 | 1.00 | 22.64 |
| 1012 | CG | PHE | A | 808 | 30.241 | -2.741 | 68.942 | 1.00 | 21.70 |
| 1013 | CD1 | PHE | A | 808 | 31.554 | -2.306 | 68.890 | 1.00 | 24.72 |
| 1014 | CD2 | PHE | A | 808 | 29.239 | -1.814 | 69.223 | 1.00 | 24.00 |
| 1015 | CE1 | PHE | A | 808 | 31.881 | -0.961 | 69.116 | 1.00 | 28.45 |
| 1016 | CE2 | PHE | A | 808 | 29.546 | -0.471 | 69.451 | 1.00 | 23.16 |
| 1017 | CZ | PHE | A | 808 | 30.872 | -0.045 | 69.398 | 1.00 | 26.38 |
| 1018 | N | VAL | A | 809 | 30.146 | -7.176 | 67.806 | 1.00 | 26.08 |
| 1019 | CA | VAL | A | 809 | 29.762 | -8.588 | 67.937 | 1.00 | 24.64 |
| 1020 | C | VAL | A | 809 | 31.002 | -9.470 | 67.997 | 1.00 | 26.29 |
| 1021 | O | VAL | A | 809 | 31.119 | -10.337 | 68.866 | 1.00 | 28.56 |
| 1022 | CB | VAL | A | 809 | 28.893 | -9.023 | 66.744 | 1.00 | 28.11 |
| 1023 | CG1 | VAL | A | 809 | 28.782 | -10.541 | 66.691 | 1.00 | 30.55 |
| 1024 | CG2 | VAL | A | 809 | 27.514 | -8.415 | 66.877 | 1.00 | 29.17 |
| 1025 | N | LYS | A | 810 | 31.934 | -9.242 | 67.074 | 1.00 | 22.16 |
| 1026 | CA | LYS | A | 810 | 33.163 | -10.023 | 66.997 | 1.00 | 28.14 |
| 1027 | C | LYS | A | 810 | 34.005 | -9.923 | 68.275 | 1.00 | 29.30 |
| 1028 | O | LYS | A | 810 | 34.468 | -10.931 | 68.823 | 1.00 | 29.13 |
| 1029 | CB | LYS | A | 810 | 33.970 | -9.547 | 65.785 | 1.00 | 31.67 |
| 1030 | CG | LYS | A | 810 | 35.211 | -10.349 | 65.433 | 1.00 | 41.13 |
| 1031 | CD | LYS | A | 810 | 35.952 | -9.686 | 64.256 | 1.00 | 46.76 |
| 1032 | CE | LYS | A | 810 | 35.028 | -9.495 | 63.053 | 1.00 | 48.50 |
| 1033 | NZ | LYS | A | 810 | 35.669 | -8.774 | 61.916 | 1.00 | 53.84 |
| 1034 | N | LEU | A | 811 | 34.203 | -8.704 | 68.758 | 1.00 | 29.04 |
| 1035 | CA | LEU | A | 811 | 34.997 | -8.495 | 69.962 | 1.00 | 27.03 |
| 1036 | C | LEU | A | 811 | 34.227 | -8.671 | 71.271 | 1.00 | 24.56 |
| 1037 | O | LEU | A | 811 | 34.834 | -8.749 | 72.344 | 1.00 | 25.98 |
| 1038 | CB | LEU | A | 811 | 35.613 | -7.086 | 69.942 | 1.00 | 30.03 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1039 | CG | LEU | A | 811 | 36.682 | -6.756 | 68.897 | 1.00 | 36.08 |
| 1040 | CD1 | LEU | A | 811 | 37.157 | -5.327 | 69.075 | 1.00 | 28.22 |
| 1041 | CD2 | LEU | A | 811 | 37.849 | -7.707 | 69.059 | 1.00 | 33.46 |
| 1042 | N | GLN | A | 812 | 32.901 | -8.734 | 71.184 | 1.00 | 24.63 |
| 1043 | CA | GLN | A | 812 | 32.052 | -8.840 | 72.366 | 1.00 | 24.29 |
| 1044 | C | GLN | A | 812 | 32.351 | -7.707 | 73.363 | 1.00 | 28.28 |
| 1045 | O | GLN | A | 812 | 32.608 | -7.935 | 74.553 | 1.00 | 26.02 |
| 1046 | CB | GLN | A | 812 | 32.202 | -10.214 | 73.030 | 1.00 | 31.81 |
| 1047 | CG | GLN | A | 812 | 31.752 | -11.335 | 72.115 | 1.00 | 34.45 |
| 1048 | CD | GLN | A | 812 | 31.604 | -12.687 | 72.810 | 1.00 | 43.12 |
| 1049 | OE1 | GLN | A | 812 | 31.282 | -13.693 | 72.162 | 1.00 | 48.46 |
| 1050 | NE2 | GLN | A | 812 | 31.825 | -12.720 | 74.117 | 1.00 | 38.30 |
| 1051 | N | VAL | A | 813 | 32.314 | -6.479 | 72.851 | 1.00 | 23.00 |
| 1052 | CA | VAL | A | 813 | 32.547 | -5.274 | 73.659 | 1.00 | 23.27 |
| 1053 | C | VAL | A | 813 | 31.539 | -5.173 | 74.804 | 1.00 | 20.62 |
| 1054 | O | VAL | A | 813 | 30.342 | -5.422 | 74.631 | 1.00 | 24.25 |
| 1055 | CB | VAL | A | 813 | 32.452 | -4.002 | 72.776 | 1.00 | 22.34 |
| 1056 | CG1 | VAL | A | 813 | 32.527 | -2.735 | 73.626 | 1.00 | 22.21 |
| 1057 | CG2 | VAL | A | 813 | 33.564 | -4.017 | 71.777 | 1.00 | 20.16 |
| 1058 | N | SER | A | 814 | 32.033 | -4.814 | 75.984 | 1.00 | 21.62 |
| 1059 | CA | SER | A | 814 | 31.168 | -4.708 | 77.141 | 1.00 | 22.92 |
| 1060 | C | SER | A | 814 | 30.636 | -3.294 | 77.308 | 1.00 | 21.08 |
| 1061 | O | SER | A | 814 | 31.191 | -2.345 | 76.763 | 1.00 | 22.56 |
| 1062 | CB | SER | A | 814 | 31.931 | -5.094 | 78.406 | 1.00 | 23.84 |
| 1063 | OG | SER | A | 814 | 32.977 | -4.161 | 78.643 | 1.00 | 27.14 |
| 1064 | N | GLN | A | 815 | 29.549 | -3.174 | 78.057 | 1.00 | 22.79 |
| 1065 | CA | GLN | A | 815 | 28.938 | -1.882 | 78.332 | 1.00 | 26.53 |
| 1066 | C | GLN | A | 815 | 29.995 | -0.959 | 78.945 | 1.00 | 24.03 |
| 1067 | O | GLN | A | 815 | 30.083 | 0.209 | 78.590 | 1.00 | 23.00 |
| 1068 | CB | GLN | A | 815 | 27.757 | -2.077 | 79.304 | 1.00 | 26.89 |
| 1069 | CG | GLN | A | 815 | 27.053 | -0.812 | 79.802 | 1.00 | 37.27 |
| 1070 | CD | GLN | A | 815 | 26.399 | -0.002 | 78.700 | 1.00 | 46.40 |
| 1071 | OE1 | GLN | A | 815 | 27.072 | 0.686 | 77.927 | 1.00 | 49.69 |
| 1072 | NE2 | GLN | A | 815 | 25.073 | -0.085 | 78.618 | 1.00 | 48.97 |
| 1073 | N | GLU | A | 816 | 30.806 | -1.498 | 79.853 | 1.00 | 24.42 |
| 1074 | CA | GLU | A | 816 | 31.844 | -0.706 | 80.527 | 1.00 | 24.69 |
| 1075 | C | GLU | A | 816 | 32.909 | -0.216 | 79.555 | 1.00 | 21.73 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1076 | O | GLU | A | 816 | 33.384 | 0.908 | 79.674 | 1.00 | 23.19 |
| 1077 | CB | GLU | A | 816 | 32.522 | -1.517 | 81.634 | 1.00 | 27.58 |
| 1078 | CG | GLU | A | 816 | 31.621 | -2.014 | 82.759 | 1.00 | 32.90 |
| 1079 | CD | GLU | A | 816 | 30.595 | -3.052 | 82.308 | 1.00 | 38.95 |
| 1080 | OE1 | GLU | A | 816 | 30.910 | -3.887 | 81.435 | 1.00 | 36.30 |
| 1081 | OE2 | GLU | A | 816 | 29.475 | -3.052 | 82.861 | 1.00 | 49.03 |
| 1082 | N | GLU | A | 817 | 33.307 | -1.066 | 78.606 | 1.00 | 21.48 |
| 1083 | CA | GLU | A | 817 | 34.301 | -0.666 | 77.613 | 1.00 | 20.83 |
| 1084 | C | GLU | A | 817 | 33.701 | 0.360 | 76.660 | 1.00 | 20.85 |
| 1085 | O | GLU | A | 817 | 34.348 | 1.329 | 76.299 | 1.00 | 23.00 |
| 1086 | CB | GLU | A | 817 | 34.778 | -1.867 | 76.791 | 1.00 | 22.67 |
| 1087 | CG | GLU | A | 817 | 35.660 | -2.868 | 77.560 | 1.00 | 25.39 |
| 1088 | CD | GLU | A | 817 | 35.826 | -4.193 | 76.820 | 1.00 | 33.69 |
| 1089 | OE1 | GLU | A | 817 | 34.971 | -4.524 | 75.961 | 1.00 | 30.03 |
| 1090 | OE2 | GLU | A | 817 | 36.801 | -4.917 | 77.103 | 1.00 | 30.51 |
| 1091 | N | PHE | A | 818 | 32.469 | 0.119 | 76.224 | 1.00 | 21.31 |
| 1092 | CA | PHE | A | 818 | 31.805 | 1.043 | 75.304 | 1.00 | 22.08 |
| 1093 | C | PHE | A | 818 | 31.696 | 2.467 | 75.884 | 1.00 | 21.29 |
| 1094 | O | PHE | A | 818 | 31.920 | 3.460 | 75.180 | 1.00 | 20.03 |
| 1095 | CB | PHE | A | 818 | 30.406 | 0.528 | 75.002 | 1.00 | 23.06 |
| 1096 | CG | PHE | A | 818 | 29.513 | 1.549 | 74.373 | 1.00 | 22.01 |
| 1097 | CD1 | PHE | A | 818 | 29.678 | 1.914 | 73.040 | 1.00 | 22.32 |
| 1098 | CD2 | PHE | A | 818 | 28.514 | 2.156 | 75.124 | 1.00 | 23.62 |
| 1099 | CE1 | PHE | A | 818 | 28.852 | 2.869 | 72.467 | 1.00 | 27.65 |
| 1100 | CE2 | PHE | A | 818 | 27.681 | 3.116 | 74.558 | 1.00 | 28.88 |
| 1101 | CZ | PHE | A | 818 | 27.852 | 3.471 | 73.231 | 1.00 | 22.16 |
| 1102 | N | LEU | A | 819 | 31.323 | 2.556 | 77.154 | 1.00 | 21.31 |
| 1103 | CA | LEU | A | 819 | 31.164 | 3.857 | 77.812 | 1.00 | 23.49 |
| 1104 | C | LEU | A | 819 | 32.445 | 4.699 | 77.808 | 1.00 | 26.91 |
| 1105 | O | LEU | A | 819 | 32.394 | 5.907 | 77.557 | 1.00 | 20.81 |
| 1106 | CB | LEU | A | 819 | 30.640 | 3.655 | 79.238 | 1.00 | 24.50 |
| 1107 | CG | LEU | A | 819 | 29.199 | 3.116 | 79.294 | 1.00 | 23.57 |
| 1108 | CD1 | LEU | A | 819 | 28.780 | 2.812 | 80.728 | 1.00 | 22.54 |
| 1109 | CD2 | LEU | A | 819 | 28.256 | 4.174 | 78.693 | 1.00 | 27.51 |
| 1110 | N | CYS | A | 820 | 33.586 | 4.068 | 78.087 | 1.00 | 22.87 |
| 1111 | CA | CYS | A | 820 | 34.870 | 4.761 | 78.087 | 1.00 | 26.80 |
| 1112 | C | CYS | A | 820 | 35.306 | 5.082 | 76.656 | 1.00 | 25.96 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1113 | O | CYS | A | 820 | 35.858 | 6.149 | 76.379 | 1.00 | 20.10 |
| 1114 | CB | CYS | A | 820 | 35.940 | 3.895 | 78.748 | 1.00 | 27.46 |
| 1115 | SG | CYS | A | 820 | 35.550 | 3.486 | 80.454 | 1.00 | 33.20 |
| 1116 | N | MET | A | 821 | 35.079 | 4.151 | 75.739 | 1.00 | 18.27 |
| 1117 | CA | MET | A | 821 | 35.456 | 4.420 | 74.371 | 1.00 | 20.73 |
| 1118 | C | MET | A | 821 | 34.661 | 5.603 | 73.811 | 1.00 | 18.21 |
| 1119 | O | MET | A | 821 | 35.186 | 6.383 | 73.031 | 1.00 | 20.04 |
| 1120 | CB | MET | A | 821 | 35.238 | 3.178 | 73.520 | 1.00 | 23.28 |
| 1121 | CG | MET | A | 821 | 36.100 | 2.003 | 73.934 | 1.00 | 26.12 |
| 1122 | SD | MET | A | 821 | 35.578 | 0.506 | 73.031 | 1.00 | 30.71 |
| 1123 | CE | MET | A | 821 | 36.127 | 0.928 | 71.492 | 1.00 | 17.04 |
| 1124 | N | LYS | A | 822 | 33.403 | 5.748 | 74.210 | 1.00 | 22.26 |
| 1125 | CA | LYS | A | 822 | 32.595 | 6.856 | 73.684 | 1.00 | 21.99 |
| 1126 | C | LYS | A | 822 | 33.157 | 8.208 | 74.120 | 1.00 | 23.71 |
| 1127 | O | LYS | A | 822 | 33.125 | 9.182 | 73.359 | 1.00 | 20.06 |
| 1128 | CB | LYS | A | 822 | 31.123 | 6.713 | 74.115 | 1.00 | 24.02 |
| 1129 | CG | LYS | A | 822 | 30.164 | 7.608 | 73.337 | 1.00 | 31.07 |
| 1130 | CD | LYS | A | 822 | 28.727 | 7.077 | 73.410 | 1.00 | 38.28 |
| 1131 | CE | LYS | A | 822 | 28.155 | 7.091 | 74.822 | 1.00 | 39.48 |
| 1132 | NZ | LYS | A | 822 | 27.958 | 8.479 | 75.331 | 1.00 | 42.42 |
| 1133 | N | VAL | A | 823 | 33.686 | 8.269 | 75.339 | 1.00 | 20.18 |
| 1134 | CA | VAL | A | 823 | 34.272 | 9.515 | 75.815 | 1.00 | 19.22 |
| 1135 | C | VAL | A | 823 | 35.541 | 9.802 | 75.028 | 1.00 | 21.84 |
| 1136 | O | VAL | A | 823 | 35.806 | 10.939 | 74.644 | 1.00 | 21.05 |
| 1137 | CB | VAL | A | 823 | 34.643 | 9.439 | 77.304 | 1.00 | 19.35 |
| 1138 | CG1 | VAL | A | 823 | 35.288 | 10.747 | 77.725 | 1.00 | 18.41 |
| 1139 | CG2 | VAL | A | 823 | 33.400 | 9.182 | 78.141 | 1.00 | 23.25 |
| 1140 | N | LEU | A | 824 | 36.333 | 8.766 | 74.780 | 1.00 | 19.81 |
| 1141 | CA | LEU | A | 824 | 37.561 | 8.950 | 74.019 | 1.00 | 20.73 |
| 1142 | C | LEU | A | 824 | 37.252 | 9.452 | 72.614 | 1.00 | 24.51 |
| 1143 | O | LEU | A | 824 | 38.054 | 10.173 | 72.021 | 1.00 | 21.96 |
| 1144 | CB | LEU | A | 824 | 38.371 | 7.644 | 73.976 | 1.00 | 21.13 |
| 1145 | CG | LEU | A | 824 | 38.943 | 7.289 | 75.355 | 1.00 | 20.69 |
| 1146 | CD1 | LEU | A | 824 | 39.580 | 5.921 | 75.310 | 1.00 | 23.72 |
| 1147 | CD2 | LEU | A | 824 | 39.978 | 8.354 | 75.784 | 1.00 | 21.59 |
| 1148 | N | LEU | A | 825 | 36.087 | 9.098 | 72.080 | 1.00 | 22.43 |
| 1149 | CA | LEU | A | 825 | 35.712 | 9.582 | 70.758 | 1.00 | 24.57 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1150 | C | LEU | A | 825 | 35.421 | 11.068 | 70.793 | 1.00 | 22.02 |
| 1151 | O | LEU | A | 825 | 35.737 | 11.773 | 69.845 | 1.00 | 23.56 |
| 1152 | CB | LEU | A | 825 | 34.480 | 8.868 | 70.206 | 1.00 | 19.15 |
| 1153 | CG | LEU | A | 825 | 34.643 | 7.542 | 69.500 | 1.00 | 31.93 |
| 1154 | CD1 | LEU | A | 825 | 33.284 | 7.197 | 68.894 | 1.00 | 28.38 |
| 1155 | CD2 | LEU | A | 825 | 35.718 | 7.644 | 68.395 | 1.00 | 26.52 |
| 1156 | N | LEU | A | 826 | 34.802 | 11.529 | 71.875 | 1.00 | 18.07 |
| 1157 | CA | LEU | A | 826 | 34.507 | 12.948 | 72.046 | 1.00 | 20.16 |
| 1158 | C | LEU | A | 826 | 35.819 | 13.741 | 72.040 | 1.00 | 23.85 |
| 1159 | O | LEU | A | 826 | 35.886 | 14.882 | 71.572 | 1.00 | 23.95 |
| 1160 | CB | LEU | A | 826 | 33.805 | 13.171 | 73.384 | 1.00 | 21.07 |
| 1161 | CG | LEU | A | 826 | 33.603 | 14.620 | 73.851 | 1.00 | 22.15 |
| 1162 | CD1 | LEU | A | 826 | 32.731 | 15.381 | 72.850 | 1.00 | 21.62 |
| 1163 | CD2 | LEU | A | 826 | 32.966 | 14.629 | 75.234 | 1.00 | 22.53 |
| 1164 | N | LEU | A | 827 | 36.863 | 13.114 | 72.565 | 1.00 | 16.99 |
| 1165 | CA | LEU | A | 827 | 38.168 | 13.734 | 72.693 | 1.00 | 21.87 |
| 1166 | C | LEU | A | 827 | 39.134 | 13.226 | 71.637 | 1.00 | 24.10 |
| 1167 | O | LEU | A | 827 | 40.340 | 13.255 | 71.860 | 1.00 | 24.64 |
| 1168 | CB | LEU | A | 827 | 38.737 | 13.389 | 74.081 | 1.00 | 20.22 |
| 1169 | CG | LEU | A | 827 | 37.804 | 13.622 | 75.273 | 1.00 | 27.92 |
| 1170 | CD1 | LEU | A | 827 | 38.451 | 13.115 | 76.571 | 1.00 | 24.44 |
| 1171 | CD2 | LEU | A | 827 | 37.471 | 15.111 | 75.381 | 1.00 | 21.00 |
| 1172 | N | ASN | A | 828 | 38.638 | 12.797 | 70.477 | 1.00 | 21.55 |
| 1173 | CA | ASN | A | 828 | 39.547 | 12.205 | 69.492 | 1.00 | 26.34 |
| 1174 | C | ASN | A | 828 | 40.068 | 13.073 | 68.343 | 1.00 | 21.48 |
| 1175 | O | ASN | A | 828 | 40.831 | 12.604 | 67.501 | 1.00 | 25.22 |
| 1176 | CB | ASN | A | 828 | 38.896 | 10.940 | 68.938 | 1.00 | 24.75 |
| 1177 | CG | ASN | A | 828 | 39.862 | 9.775 | 68.868 | 1.00 | 39.47 |
| 1178 | OD1 | ASN | A | 828 | 40.751 | 9.655 | 69.711 | 1.00 | 33.27 |
| 1179 | ND2 | ASN | A | 828 | 39.676 | 8.891 | 67.881 | 1.00 | 37.42 |
| 1180 | N | THR | A | 829 | 39.675 | 14.336 | 68.330 | 1.00 | 20.94 |
| 1181 | CA | THR | A | 829 | 40.088 | 15.263 | 67.283 | 1.00 | 23.18 |
| 1182 | C | THR | A | 829 | 40.174 | 16.653 | 67.881 | 1.00 | 24.51 |
| 1183 | O | THR | A | 829 | 39.279 | 17.062 | 68.623 | 1.00 | 20.61 |
| 1184 | CB | THR | A | 829 | 39.047 | 15.287 | 66.125 | 1.00 | 27.14 |
| 1185 | OG1 | THR | A | 829 | 38.876 | 13.963 | 65.609 | 1.00 | 27.76 |
| 1186 | CG2 | THR | A | 829 | 39.512 | 16.196 | 64.987 | 1.00 | 27.52 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1187 | N | ILE | A | 830 | 41.255 | 17.368 | 67.578 | 1.00 | 23.20 |
| 1188 | CA | ILE | A | 830 | 41.416 | 18.736 | 68.072 | 1.00 | 22.29 |
| 1189 | C | ILE | A | 830 | 41.876 | 19.651 | 66.939 | 1.00 | 23.93 |
| 1190 | O | ILE | A | 830 | 42.271 | 19.176 | 65.874 | 1.00 | 23.93 |
| 1191 | CB | ILE | A | 830 | 42.428 | 18.799 | 69.226 | 1.00 | 24.86 |
| 1192 | CG1 | ILE | A | 830 | 43.805 | 18.332 | 68.744 | 1.00 | 22.58 |
| 1193 | CG2 | ILE | A | 830 | 41.914 | 17.962 | 70.402 | 1.00 | 25.47 |
| 1194 | CD1 | ILE | A | 830 | 44.856 | 18.368 | 69.827 | 1.00 | 28.86 |
| 1195 | N | PRO | A | 831 | 41.818 | 20.971 | 67.150 | 1.00 | 23.63 |
| 1196 | CA | PRO | A | 831 | 42.240 | 21.919 | 66.117 | 1.00 | 26.05 |
| 1197 | C | PRO | A | 831 | 43.727 | 21.796 | 65.831 | 1.00 | 30.91 |
| 1198 | O | PRO | A | 831 | 44.496 | 21.316 | 66.673 | 1.00 | 27.25 |
| 1199 | CB | PRO | A | 831 | 41.888 | 23.277 | 66.732 | 1.00 | 28.62 |
| 1200 | CG | PRO | A | 831 | 40.752 | 22.925 | 67.707 | 1.00 | 25.78 |
| 1201 | CD | PRO | A | 831 | 41.368 | 21.704 | 68.341 | 1.00 | 28.72 |
| 1202 | N | LEU | A | 832 | 44.126 | 22.225 | 64.636 | 1.00 | 31.80 |
| 1203 | CA | LEU | A | 832 | 45.528 | 22.180 | 64.238 | 1.00 | 34.58 |
| 1204 | C | LEU | A | 832 | 46.404 | 22.954 | 65.216 | 1.00 | 32.37 |
| 1205 | O | LEU | A | 832 | 47.558 | 22.591 | 65.444 | 1.00 | 38.50 |
| 1206 | CB | LEU | A | 832 | 45.699 | 22.760 | 62.827 | 1.00 | 34.68 |
| 1207 | CG | LEU | A | 832 | 45.108 | 21.920 | 61.691 | 1.00 | 34.02 |
| 1208 | CD1 | LEU | A | 832 | 45.298 | 22.624 | 60.342 | 1.00 | 34.39 |
| 1209 | CD2 | LEU | A | 832 | 45.787 | 20.563 | 61.686 | 1.00 | 36.07 |
| 1210 | N | GLU | A | 833 | 45.853 | 24.012 | 65.798 | 1.00 | 33.46 |
| 1211 | CA | GLU | A | 833 | 46.605 | 24.817 | 66.751 | 1.00 | 35.43 |
| 1212 | C | GLU | A | 833 | 46.379 | 24.348 | 68.188 | 1.00 | 37.02 |
| 1213 | O | GLU | A | 833 | 46.866 | 24.959 | 69.143 | 1.00 | 34.87 |
| 1214 | CB | GLU | A | 833 | 46.239 | 26.304 | 66.611 | 1.00 | 40.38 |
| 1215 | CG | GLU | A | 833 | 44.786 | 26.683 | 66.901 | 1.00 | 46.00 |
| 1216 | CD | GLU | A | 833 | 43.802 | 26.239 | 65.825 | 1.00 | 52.16 |
| 1217 | OE1 | GLU | A | 833 | 44.211 | 25.549 | 64.867 | 1.00 | 49.08 |
| 1218 | OE2 | GLU | A | 833 | 42.607 | 26.589 | 65.943 | 1.00 | 50.96 |
| 1219 | N | GLY | A | 834 | 45.650 | 23.249 | 68.344 | 1.00 | 32.49 |
| 1220 | CA | GLY | A | 834 | 45.392 | 22.741 | 69.675 | 1.00 | 28.49 |
| 1221 | C | GLY | A | 834 | 44.231 | 23.479 | 70.312 | 1.00 | 30.77 |
| 1222 | O | GLY | A | 834 | 43.649 | 24.372 | 69.705 | 1.00 | 29.05 |
| 1223 | N | LEU | A | 835 | 43.908 | 23.102 | 71.544 | 1.00 | 27.80 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1224 | CA | LEU | A | 835 | 42.812 | 23.691 | 72.297 | 1.00 | 31.88 |
| 1225 | C | LEU | A | 835 | 43.325 | 24.754 | 73.267 | 1.00 | 30.49 |
| 1226 | O | LEU | A | 835 | 44.491 | 24.737 | 73.647 | 1.00 | 28.71 |
| 1227 | CB | LEU | A | 835 | 42.119 | 22.595 | 73.102 | 1.00 | 28.43 |
| 1228 | CG | LEU | A | 835 | 41.438 | 21.461 | 72.335 | 1.00 | 31.10 |
| 1229 | CD1 | LEU | A | 835 | 41.071 | 20.363 | 73.310 | 1.00 | 29.92 |
| 1230 | CD2 | LEU | A | 835 | 40.200 | 21.995 | 71.598 | 1.00 | 27.76 |
| 1231 | N | ARG | A | 836 | 42.446 | 25.659 | 73.685 | 1.00 | 33.09 |
| 1232 | CA | ARG | A | 836 | 42.839 | 26.695 | 74.644 | 1.00 | 33.34 |
| 1233 | C | ARG | A | 836 | 43.209 | 26.019 | 75.973 | 1.00 | 37.41 |
| 1234 | O | ARG | A | 836 | 44.155 | 26.427 | 76.662 | 1.00 | 35.58 |
| 1235 | CB | ARG | A | 836 | 41.683 | 27.673 | 74.872 | 1.00 | 37.62 |
| 1236 | CG | ARG | A | 836 | 41.129 | 28.268 | 73.591 | 1.00 | 43.12 |
| 1237 | CD | ARG | A | 836 | 40.175 | 29.438 | 73.840 | 1.00 | 55.06 |
| 1238 | NE | ARG | A | 836 | 40.858 | 30.690 | 74.181 | 1.00 | 65.30 |
| 1239 | CZ | ARG | A | 836 | 41.461 | 30.952 | 75.339 | 1.00 | 65.60 |
| 1240 | NH1 | ARG | A | 836 | 41.467 | 30.059 | 76.319 | 1.00 | 69.19 |
| 1241 | NH2 | ARG | A | 836 | 42.048 | 32.127 | 75.525 | 1.00 | 71.09 |
| 1242 | N | SER | A | 837 | 42.439 | 24.994 | 76.337 | 1.00 | 28.39 |
| 1243 | CA | SER | A | 837 | 42.672 | 24.234 | 77.557 | 1.00 | 29.00 |
| 1244 | C | SER | A | 837 | 43.364 | 22.924 | 77.192 | 1.00 | 30.23 |
| 1245 | O | SER | A | 837 | 42.910 | 21.851 | 77.576 | 1.00 | 26.81 |
| 1246 | CB | SER | A | 837 | 41.339 | 23.933 | 78.249 | 1.00 | 23.84 |
| 1247 | OG | SER | A | 837 | 40.660 | 25.131 | 78.575 | 1.00 | 31.90 |
| 1248 | N | GLN | A | 838 | 44.467 | 23.007 | 76.452 | 1.00 | 29.41 |
| 1249 | CA | GLN | A | 838 | 45.162 | 21.796 | 76.031 | 1.00 | 28.12 |
| 1250 | C | GLN | A | 838 | 45.642 | 20.947 | 77.207 | 1.00 | 30.39 |
| 1251 | O | GLN | A | 838 | 45.503 | 19.730 | 77.190 | 1.00 | 26.03 |
| 1252 | CB | GLN | A | 838 | 46.341 | 22.154 | 75.125 | 1.00 | 25.64 |
| 1253 | CG | GLN | A | 838 | 46.954 | 20.986 | 74.371 | 1.00 | 32.23 |
| 1254 | CD | GLN | A | 838 | 45.976 | 20.338 | 73.401 | 1.00 | 37.10 |
| 1255 | OE1 | GLN | A | 838 | 45.199 | 21.021 | 72.730 | 1.00 | 37.64 |
| 1256 | NE2 | GLN | A | 838 | 46.028 | 19.017 | 73.306 | 1.00 | 42.95 |
| 1257 | N | THR | A | 839 | 46.224 | 21.578 | 78.224 | 1.00 | 27.88 |
| 1258 | CA | THR | A | 839 | 46.688 | 20.825 | 79.385 | 1.00 | 30.26 |
| 1259 | C | THR | A | 839 | 45.573 | 20.042 | 80.054 | 1.00 | 27.76 |
| 1260 | O | THR | A | 839 | 45.724 | 18.847 | 80.322 | 1.00 | 28.54 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1261 | CB | THR | A | 839 | 47.298 | 21.744 | 80.436 | 1.00 | 31.84 |
| 1262 | OG1 | THR | A | 839 | 48.408 | 22.426 | 79.857 | 1.00 | 38.66 |
| 1263 | CG2 | THR | A | 839 | 47.766 | 20.939 | 81.643 | 1.00 | 35.08 |
| 1264 | N | GLN | A | 840 | 44.466 | 20.725 | 80.337 | 1.00 | 27.40 |
| 1265 | CA | GLN | A | 840 | 43.314 | 20.095 | 80.964 | 1.00 | 27.76 |
| 1266 | C | GLN | A | 840 | 42.790 | 18.986 | 80.059 | 1.00 | 27.08 |
| 1267 | O | GLN | A | 840 | 42.382 | 17.926 | 80.534 | 1.00 | 24.43 |
| 1268 | CB | GLN | A | 840 | 42.199 | 21.106 | 81.205 | 1.00 | 29.57 |
| 1269 | CG | GLN | A | 840 | 42.471 | 22.127 | 82.286 | 1.00 | 46.45 |
| 1270 | CD | GLN | A | 840 | 41.237 | 22.957 | 82.640 | 1.00 | 53.97 |
| 1271 | OE1 | GLN | A | 840 | 40.747 | 23.754 | 81.829 | 1.00 | 56.14 |
| 1272 | NE2 | GLN | A | 840 | 40.723 | 22.763 | 83.852 | 1.00 | 51.09 |
| 1273 | N | PHE | A | 841 | 42.821 | 19.235 | 78.757 | 1.00 | 25.08 |
| 1274 | CA | PHE | A | 841 | 42.343 | 18.247 | 77.788 | 1.00 | 25.20 |
| 1275 | C | PHE | A | 841 | 43.192 | 16.985 | 77.814 | 1.00 | 22.17 |
| 1276 | O | PHE | A | 841 | 42.661 | 15.871 | 77.804 | 1.00 | 24.69 |
| 1277 | CB | PHE | A | 841 | 42.312 | 18.858 | 76.381 | 1.00 | 24.39 |
| 1278 | CG | PHE | A | 841 | 42.136 | 17.846 | 75.275 | 1.00 | 25.18 |
| 1279 | CD1 | PHE | A | 841 | 40.857 | 17.414 | 74.898 | 1.00 | 26.95 |
| 1280 | CD2 | PHE | A | 841 | 43.243 | 17.288 | 74.642 | 1.00 | 21.90 |
| 1281 | CE1 | PHE | A | 841 | 40.690 | 16.435 | 73.898 | 1.00 | 22.61 |
| 1282 | CE2 | PHE | A | 841 | 43.092 | 16.309 | 73.645 | 1.00 | 21.56 |
| 1283 | CZ | PHE | A | 841 | 41.813 | 15.880 | 73.274 | 1.00 | 23.75 |
| 1284 | N | GLU | A | 842 | 44.506 | 17.155 | 77.855 | 1.00 | 26.80 |
| 1285 | CA | GLU | A | 842 | 45.423 | 16.012 | 77.905 | 1.00 | 30.80 |
| 1286 | C | GLU | A | 842 | 45.156 | 15.160 | 79.144 | 1.00 | 27.28 |
| 1287 | O | GLU | A | 842 | 45.093 | 13.933 | 79.073 | 1.00 | 27.77 |
| 1288 | CB | GLU | A | 842 | 46.879 | 16.489 | 77.963 | 1.00 | 28.70 |
| 1289 | CG | GLU | A | 842 | 47.746 | 15.961 | 76.851 | 1.00 | 50.83 |
| 1290 | CD | GLU | A | 842 | 47.567 | 16.720 | 75.552 | 1.00 | 54.53 |
| 1291 | OE1 | GLU | A | 842 | 48.053 | 17.872 | 75.456 | 1.00 | 61.42 |
| 1292 | OE2 | GLU | A | 842 | 46.940 | 16.166 | 74.626 | 1.00 | 61.75 |
| 1293 | N | GLU | A | 843 | 45.039 | 15.820 | 80.289 | 1.00 | 29.50 |
| 1294 | CA | GLU | A | 843 | 44.776 | 15.123 | 81.539 | 1.00 | 27.52 |
| 1295 | C | GLU | A | 843 | 43.439 | 14.385 | 81.472 | 1.00 | 29.29 |
| 1296 | O | GLU | A | 843 | 43.311 | 13.263 | 81.942 | 1.00 | 29.26 |
| 1297 | CB | GLU | A | 843 | 44.767 | 16.110 | 82.713 | 1.00 | 32.59 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1298 | CG | GLU | A | 843 | 46.149 | 16.594 | 83.160 | 1.00 | 38.26 |
| 1299 | CD | GLU | A | 843 | 46.080 | 17.675 | 84.235 | 1.00 | 47.34 |
| 1300 | OE1 | GLU | A | 843 | 45.229 | 17.570 | 85.144 | 1.00 | 51.54 |
| 1301 | OE2 | GLU | A | 843 | 46.893 | 18.625 | 84.187 | 1.00 | 49.88 |
| 1302 | N | MET | A | 844 | 42.439 | 15.015 | 80.878 | 1.00 | 25.95 |
| 1303 | CA | MET | A | 844 | 41.141 | 14.377 | 80.788 | 1.00 | 26.70 |
| 1304 | C | MET | A | 844 | 41.233 | 13.125 | 79.929 | 1.00 | 26.41 |
| 1305 | O | MET | A | 844 | 40.770 | 12.050 | 80.314 | 1.00 | 25.61 |
| 1306 | CB | MET | A | 844 | 40.128 | 15.351 | 80.203 | 1.00 | 26.67 |
| 1307 | CG | MET | A | 844 | 38.737 | 14.761 | 80.116 | 1.00 | 30.25 |
| 1308 | SD | MET | A | 844 | 37.486 | 15.952 | 79.605 | 1.00 | 28.34 |
| 1309 | CE | MET | A | 844 | 36.074 | 14.840 | 79.459 | 1.00 | 23.92 |
| 1310 | N | ARG | A | 845 | 41.860 | 13.264 | 78.769 | 1.00 | 26.84 |
| 1311 | CA | ARG | A | 845 | 42.001 | 12.140 | 77.864 | 1.00 | 29.65 |
| 1312 | C | ARG | A | 845 | 42.753 | 10.981 | 78.546 | 1.00 | 31.46 |
| 1313 | O | ARG | A | 845 | 42.335 | 9.831 | 78.466 | 1.00 | 28.49 |
| 1314 | CB | ARG | A | 845 | 42.700 | 12.616 | 76.590 | 1.00 | 29.38 |
| 1315 | CG | ARG | A | 845 | 42.609 | 11.649 | 75.433 | 1.00 | 38.40 |
| 1316 | CD | ARG | A | 845 | 43.039 | 12.303 | 74.132 | 1.00 | 33.58 |
| 1317 | NE | ARG | A | 845 | 42.924 | 11.361 | 73.022 | 1.00 | 43.23 |
| 1318 | CZ | ARG | A | 845 | 43.763 | 10.357 | 72.807 | 1.00 | 45.03 |
| 1319 | NH1 | ARG | A | 845 | 44.806 | 10.180 | 73.610 | 1.00 | 58.21 |
| 1320 | NH2 | ARG | A | 845 | 43.578 | 9.545 | 71.772 | 1.00 | 52.78 |
| 1321 | N | SER | A | 846 | 43.844 | 11.284 | 79.241 | 1.00 | 29.00 |
| 1322 | CA | SER | A | 846 | 44.605 | 10.243 | 79.935 | 1.00 | 29.23 |
| 1323 | C | SER | A | 846 | 43.777 | 9.537 | 81.004 | 1.00 | 25.05 |
| 1324 | O | SER | A | 846 | 43.882 | 8.328 | 81.179 | 1.00 | 27.74 |
| 1325 | CB | SER | A | 846 | 45.863 | 10.840 | 80.567 | 1.00 | 29.17 |
| 1326 | OG | SER | A | 846 | 46.745 | 11.267 | 79.550 | 1.00 | 35.25 |
| 1327 | N | SER | A | 847 | 42.966 | 10.298 | 81.727 | 1.00 | 27.46 |
| 1328 | CA | SER | A | 847 | 42.116 | 9.736 | 82.769 | 1.00 | 26.94 |
| 1329 | C | SER | A | 847 | 41.134 | 8.736 | 82.170 | 1.00 | 28.69 |
| 1330 | O | SER | A | 847 | 40.870 | 7.692 | 82.753 | 1.00 | 24.01 |
| 1331 | CB | SER | A | 847 | 41.346 | 10.849 | 83.482 | 1.00 | 33.23 |
| 1332 | OG | SER | A | 847 | 42.249 | 11.709 | 84.158 | 1.00 | 39.50 |
| 1333 | N | TYR | A | 848 | 40.587 | 9.056 | 81.005 | 1.00 | 23.69 |
| 1334 | CA | TYR | A | 848 | 39.648 | 8.141 | 80.369 | 1.00 | 24.43 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1335 | C | TYR | A | 848 | 40.336 | 6.957 | 79.707 | 1.00 | 25.10 |
| 1336 | O | TYR | A | 848 | 39.747 | 5.888 | 79.586 | 1.00 | 24.91 |
| 1337 | CB | TYR | A | 848 | 38.762 | 8.913 | 79.402 | 1.00 | 22.98 |
| 1338 | CG | TYR | A | 848 | 37.683 | 9.651 | 80.152 | 1.00 | 21.21 |
| 1339 | CD1 | TYR | A | 848 | 36.543 | 8.975 | 80.595 | 1.00 | 21.26 |
| 1340 | CD2 | TYR | A | 848 | 37.816 | 11.003 | 80.459 | 1.00 | 21.12 |
| 1341 | CE1 | TYR | A | 848 | 35.551 | 9.624 | 81.324 | 1.00 | 22.44 |
| 1342 | CE2 | TYR | A | 848 | 36.828 | 11.665 | 81.191 | 1.00 | 23.86 |
| 1343 | CZ | TYR | A | 848 | 35.693 | 10.968 | 81.619 | 1.00 | 28.65 |
| 1344 | OH | TYR | A | 848 | 34.686 | 11.611 | 82.319 | 1.00 | 23.47 |
| 1345 | N | ILE | A | 849 | 41.581 | 7.133 | 79.289 | 1.00 | 23.21 |
| 1346 | CA | ILE | A | 849 | 42.314 | 6.019 | 78.714 | 1.00 | 29.14 |
| 1347 | C | ILE | A | 849 | 42.566 | 5.060 | 79.879 | 1.00 | 30.21 |
| 1348 | O | ILE | A | 849 | 42.446 | 3.846 | 79.744 | 1.00 | 26.87 |
| 1349 | CB | ILE | A | 849 | 43.654 | 6.488 | 78.069 | 1.00 | 29.35 |
| 1350 | CG1 | ILE | A | 849 | 43.356 | 7.281 | 76.790 | 1.00 | 23.92 |
| 1351 | CG2 | ILE | A | 849 | 44.547 | 5.291 | 77.729 | 1.00 | 27.00 |
| 1352 | CD1 | ILE | A | 849 | 44.583 | 7.816 | 76.109 | 1.00 | 22.76 |
| 1353 | N | ARG | A | 850 | 42.889 | 5.608 | 81.043 | 1.00 | 28.71 |
| 1354 | CA | ARG | A | 850 | 43.113 | 4.753 | 82.195 | 1.00 | 32.10 |
| 1355 | C | ARG | A | 850 | 41.816 | 4.078 | 82.611 | 1.00 | 29.94 |
| 1356 | O | ARG | A | 850 | 41.824 | 2.959 | 83.107 | 1.00 | 28.11 |
| 1357 | CB | ARG | A | 850 | 43.672 | 5.538 | 83.387 | 1.00 | 35.86 |
| 1358 | CG | ARG | A | 850 | 45.100 | 6.028 | 83.221 | 1.00 | 40.68 |
| 1359 | CD | ARG | A | 850 | 45.662 | 6.509 | 84.558 | 1.00 | 46.12 |
| 1360 | NE | ARG | A | 850 | 44.999 | 7.702 | 85.079 | 1.00 | 48.88 |
| 1361 | CZ | ARG | A | 850 | 45.160 | 8.924 | 84.581 | 1.00 | 47.54 |
| 1362 | NH1 | ARG | A | 850 | 45.964 | 9.121 | 83.546 | 1.00 | 47.38 |
| 1363 | NH2 | ARG | A | 850 | 44.515 | 9.952 | 85.115 | 1.00 | 52.75 |
| 1364 | N | GLU | A | 851 | 40.691 | 4.750 | 82.408 | 1.00 | 26.26 |
| 1365 | CA | GLU | A | 851 | 39.428 | 4.149 | 82.798 | 1.00 | 24.07 |
| 1366 | C | GLU | A | 851 | 39.058 | 3.010 | 81.843 | 1.00 | 25.19 |
| 1367 | O | GLU | A | 851 | 38.480 | 2.005 | 82.269 | 1.00 | 27.72 |
| 1368 | CB | GLU | A | 851 | 38.325 | 5.209 | 82.851 | 1.00 | 29.75 |
| 1369 | CG | GLU | A | 851 | 37.158 | 4.816 | 83.735 | 1.00 | 32.34 |
| 1370 | CD | GLU | A | 851 | 37.559 | 4.663 | 85.207 | 1.00 | 48.72 |
| 1371 | OE1 | GLU | A | 851 | 38.709 | 5.013 | 85.558 | 1.00 | 47.00 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1372 | OE2 | GLU | A | 851 | 36.723 | 4.202 | 86.018 | 1.00 | 46.97 |
| 1373 | N | LEU | A | 852 | 39.390 | 3.158 | 80.562 | 1.00 | 23.97 |
| 1374 | CA | LEU | A | 852 | 39.127 | 2.104 | 79.584 | 1.00 | 22.66 |
| 1375 | C | LEU | A | 852 | 39.945 | 0.872 | 79.992 | 1.00 | 30.81 |
| 1376 | O | LEU | A | 852 | 39.490 | -0.260 | 79.844 | 1.00 | 24.16 |
| 1377 | CB | LEU | A | 852 | 39.572 | 2.547 | 78.186 | 1.00 | 22.80 |
| 1378 | CG | LEU | A | 852 | 39.595 | 1.452 | 77.115 | 1.00 | 25.24 |
| 1379 | CD1 | LEU | A | 852 | 38.196 | 0.939 | 76.908 | 1.00 | 25.51 |
| 1380 | CD2 | LEU | A | 852 | 40.186 | 1.996 | 75.801 | 1.00 | 27.39 |
| 1381 | N | ILE | A | 853 | 41.167 | 1.096 | 80.474 | 1.00 | 24.04 |
| 1382 | CA | ILE | A | 853 | 42.008 | -0.021 | 80.897 | 1.00 | 29.31 |
| 1383 | C | ILE | A | 853 | 41.328 | -0.764 | 82.035 | 1.00 | 28.39 |
| 1384 | O | ILE | A | 853 | 41.275 | -1.994 | 82.025 | 1.00 | 28.22 |
| 1385 | CB | ILE | A | 853 | 43.418 | 0.461 | 81.316 | 1.00 | 32.13 |
| 1386 | CG1 | ILE | A | 853 | 44.150 | 0.976 | 80.077 | 1.00 | 26.53 |
| 1387 | CG2 | ILE | A | 853 | 44.212 | -0.686 | 81.980 | 1.00 | 26.27 |
| 1388 | CD1 | ILE | A | 853 | 45.496 | 1.620 | 80.364 | 1.00 | 29.29 |
| 1389 | N | LYS | A | 854 | 40.778 | -0.025 | 82.999 | 1.00 | 29.91 |
| 1390 | CA | LYS | A | 854 | 40.070 | -0.645 | 84.121 | 1.00 | 27.78 |
| 1391 | C | LYS | A | 854 | 38.861 | -1.428 | 83.616 | 1.00 | 28.75 |
| 1392 | O | LYS | A | 854 | 38.564 | -2.515 | 84.106 | 1.00 | 30.96 |
| 1393 | CB | LYS | A | 854 | 39.594 | 0.408 | 85.121 | 1.00 | 32.36 |
| 1394 | CG | LYS | A | 854 | 40.695 | 1.151 | 85.839 | 1.00 | 32.65 |
| 1395 | CD | LYS | A | 854 | 40.084 | 2.199 | 86.763 | 1.00 | 41.08 |
| 1396 | CE | LYS | A | 854 | 41.158 | 3.004 | 87.445 | 1.00 | 47.72 |
| 1397 | NZ | LYS | A | 854 | 42.079 | 3.620 | 86.445 | 1.00 | 54.97 |
| 1398 | N | ALA | A | 855 | 38.154 | -0.875 | 82.634 | 1.00 | 25.66 |
| 1399 | CA | ALA | A | 855 | 36.981 | -1.561 | 82.099 | 1.00 | 26.89 |
| 1400 | C | ALA | A | 855 | 37.422 | -2.887 | 81.490 | 1.00 | 25.18 |
| 1401 | O | ALA | A | 855 | 36.761 | -3.914 | 81.667 | 1.00 | 24.74 |
| 1402 | CB | ALA | A | 855 | 36.292 | -0.698 | 81.038 | 1.00 | 22.45 |
| 1403 | N | ILE | A | 856 | 38.543 | -2.853 | 80.780 | 1.00 | 25.69 |
| 1404 | CA | ILE | A | 856 | 39.079 | -4.051 | 80.132 | 1.00 | 28.42 |
| 1405 | C | ILE | A | 856 | 39.473 | -5.061 | 81.212 | 1.00 | 32.00 |
| 1406 | O | ILE | A | 856 | 39.232 | -6.258 | 81.066 | 1.00 | 33.59 |
| 1407 | CB | ILE | A | 856 | 40.297 | -3.690 | 79.258 | 1.00 | 31.03 |
| 1408 | CG1 | ILE | A | 856 | 39.817 | -2.913 | 78.024 | 1.00 | 25.53 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1409 | CG2 | ILE | A | 856 | 41.066 | -4.950 | 78.854 | 1.00 | 29.18 |
| 1410 | CD1 | ILE | A | 856 | 40.940 | -2.382 | 77.164 | 1.00 | 27.39 |
| 1411 | N | GLY | A | 857 | 40.060 | -4.559 | 82.294 | 1.00 | 33.82 |
| 1412 | CA | GLY | A | 857 | 40.475 | -5.410 | 83.397 | 1.00 | 34.58 |
| 1413 | C | GLY | A | 857 | 39.345 | -6.137 | 84.105 | 1.00 | 37.55 |
| 1414 | O | GLY | A | 857 | 39.566 | -7.165 | 84.749 | 1.00 | 37.64 |
| 1415 | N | LEU | A | 858 | 38.129 | -5.615 | 83.994 | 1.00 | 33.59 |
| 1416 | CA | LEU | A | 858 | 36.981 | -6.237 | 84.630 | 1.00 | 37.87 |
| 1417 | C | LEU | A | 858 | 36.730 | -7.652 | 84.110 | 1.00 | 42.05 |
| 1418 | O | LEU | A | 858 | 36.162 | -8.482 | 84.816 | 1.00 | 37.32 |
| 1419 | CB | LEU | A | 858 | 35.723 | -5.393 | 84.404 | 1.00 | 35.77 |
| 1420 | CG | LEU | A | 858 | 35.707 | -3.981 | 84.980 | 1.00 | 34.70 |
| 1421 | CD1 | LEU | A | 858 | 34.393 | -3.311 | 84.614 | 1.00 | 39.67 |
| 1422 | CD2 | LEU | A | 858 | 35.873 | -4.036 | 86.488 | 1.00 | 40.03 |
| 1423 | N | ARG | A | 859 | 37.161 | -7.921 | 82.880 | 1.00 | 42.57 |
| 1424 | CA | ARG | A | 859 | 36.947 | -9.221 | 82.256 | 1.00 | 52.08 |
| 1425 | C | ARG | A | 859 | 38.188 | -9.797 | 81.586 | 1.00 | 54.79 |
| 1426 | O | ARG | A | 859 | 38.201 | -10.972 | 81.215 | 1.00 | 58.54 |
| 1427 | CB | ARG | A | 859 | 35.820 | -9.121 | 81.226 | 1.00 | 52.12 |
| 1428 | CG | ARG | A | 859 | 34.472 | -8.758 | 81.830 | 1.00 | 58.32 |
| 1429 | CD | ARG | A | 859 | 33.381 | -8.622 | 80.776 | 1.00 | 61.04 |
| 1430 | NE | ARG | A | 859 | 32.082 | -8.385 | 81.399 | 1.00 | 72.95 |
| 1431 | CZ | ARG | A | 859 | 30.938 | -8.230 | 80.737 | 1.00 | 76.45 |
| 1432 | NH1 | ARG | A | 859 | 30.918 | -8.279 | 79.411 | 1.00 | 80.87 |
| 1433 | NH2 | ARG | A | 859 | 29.810 | -8.021 | 81.406 | 1.00 | 77.97 |
| 1434 | N | GLN | A | 860 | 39.222 | -8.976 | 81.416 | 1.00 | 60.36 |
| 1435 | CA | GLN | A | 860 | 40.460 | -9.442 | 80.800 | 1.00 | 65.05 |
| 1436 | C | GLN | A | 860 | 41.558 | -9.610 | 81.840 | 1.00 | 67.98 |
| 1437 | O | GLN | A | 860 | 42.356 | -8.704 | 82.096 | 1.00 | 68.79 |
| 1438 | CB | GLN | A | 860 | 40.910 | -8.493 | 79.688 | 1.00 | 64.71 |
| 1439 | CG | GLN | A | 860 | 39.943 | -8.453 | 78.519 | 1.00 | 68.26 |
| 1440 | CD | GLN | A | 860 | 39.754 | -9.810 | 77.861 | 1.00 | 71.18 |
| 1441 | OE1 | GLN | A | 860 | 38.897 | -9.980 | 76.991 | 1.00 | 72.78 |
| 1442 | NE2 | GLN | A | 860 | 40.561 | -10.784 | 78.268 | 1.00 | 72.46 |
| 1443 | N | LYS | A | 861 | 41.560 | -10.798 | 82.434 | 1.00 | 70.37 |
| 1444 | CA | LYS | A | 861 | 42.507 | -11.206 | 83.459 | 1.00 | 71.87 |
| 1445 | C | LYS | A | 861 | 43.962 | -11.197 | 82.978 | 1.00 | 72.34 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1446 | O | LYS | A | 861 | 44.825 | -10.575 | 83.603 | 1.00 | 71.39 |
| 1447 | CB | LYS | A | 861 | 42.111 | -12.604 | 83.946 | 1.00 | 74.09 |
| 1448 | CG | LYS | A | 861 | 41.860 | -13.587 | 82.797 | 1.00 | 76.68 |
| 1449 | CD | LYS | A | 861 | 41.293 | -14.925 | 83.273 | 1.00 | 77.75 |
| 1450 | CE | LYS | A | 861 | 40.977 | -15.836 | 82.086 | 1.00 | 77.67 |
| 1451 | NZ | LYS | A | 861 | 40.368 | -17.136 | 82.492 | 1.00 | 78.97 |
| 1452 | N | GLY | A | 862 | 44.230 | -11.885 | 81.870 | 1.00 | 72.03 |
| 1453 | CA | GLY | A | 862 | 45.583 | -11.943 | 81.340 | 1.00 | 72.71 |
| 1454 | C | GLY | A | 862 | 46.226 | -10.580 | 81.144 | 1.00 | 71.15 |
| 1455 | O | GLY | A | 862 | 45.589 | -9.655 | 80.644 | 1.00 | 71.21 |
| 1456 | N | VAL | A | 863 | 47.489 | -10.452 | 81.535 | 1.00 | 70.79 |
| 1457 | CA | VAL | A | 863 | 48.212 | -9.191 | 81.392 | 1.00 | 69.40 |
| 1458 | C | VAL | A | 863 | 48.467 | -8.888 | 79.918 | 1.00 | 68.93 |
| 1459 | O | VAL | A | 863 | 48.543 | -7.723 | 79.516 | 1.00 | 66.12 |
| 1460 | CB | VAL | A | 863 | 49.574 | -9.234 | 82.125 | 1.00 | 71.43 |
| 1461 | CG1 | VAL | A | 863 | 49.359 | -9.445 | 83.617 | 1.00 | 70.39 |
| 1462 | CG2 | VAL | A | 863 | 50.441 | -10.347 | 81.544 | 1.00 | 72.36 |
| 1463 | N | VAL | A | 864 | 48.605 | -9.943 | 79.120 | 1.00 | 65.17 |
| 1464 | CA | VAL | A | 864 | 48.848 | -9.782 | 77.693 | 1.00 | 64.59 |
| 1465 | C | VAL | A | 864 | 47.511 | -9.789 | 76.963 | 1.00 | 61.44 |
| 1466 | O | VAL | A | 864 | 47.331 | -9.088 | 75.971 | 1.00 | 61.24 |
| 1467 | CB | VAL | A | 864 | 49.744 | -10.916 | 77.139 | 1.00 | 65.52 |
| 1468 | CG1 | VAL | A | 864 | 49.034 | -12.258 | 77.263 | 1.00 | 67.34 |
| 1469 | CG2 | VAL | A | 864 | 50.117 | -10.624 | 75.691 | 1.00 | 65.68 |
| 1470 | N | SER | A | 865 | 46.574 | -10.588 | 77.464 | 1.00 | 58.29 |
| 1471 | CA | SER | A | 865 | 45.250 | -10.666 | 76.868 | 1.00 | 52.84 |
| 1472 | C | SER | A | 865 | 44.584 | -9.299 | 77.017 | 1.00 | 51.66 |
| 1473 | O | SER | A | 865 | 43.901 | -8.825 | 76.109 | 1.00 | 45.86 |
| 1474 | CB | SER | A | 865 | 44.410 | -11.733 | 77.572 | 1.00 | 55.37 |
| 1475 | OG | SER | A | 865 | 43.096 | -11.778 | 77.043 | 1.00 | 56.88 |
| 1476 | N | SER | A | 866 | 44.798 | -8.674 | 78.169 | 1.00 | 44.60 |
| 1477 | CA | SER | A | 866 | 44.234 | -7.361 | 78.442 | 1.00 | 45.64 |
| 1478 | C | SER | A | 866 | 44.961 | -6.340 | 77.581 | 1.00 | 44.47 |
| 1479 | O | SER | A | 866 | 44.375 | -5.360 | 77.123 | 1.00 | 39.29 |
| 1480 | CB | SER | A | 866 | 44.417 | -6.997 | 79.914 | 1.00 | 45.32 |
| 1481 | OG | SER | A | 866 | 45.788 | -6.829 | 80.218 | 1.00 | 48.12 |
| 1482 | N | SER | A | 867 | 46.247 | -6.587 | 77.367 | 1.00 | 43.23 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1483 | CA | SER | A | 867 | 47.077 | -5.707 | 76.565 | 1.00 | 42.20 |
| 1484 | C | SER | A | 867 | 46.642 | -5.804 | 75.109 | 1.00 | 41.29 |
| 1485 | O | SER | A | 867 | 46.510 | -4.791 | 74.420 | 1.00 | 42.13 |
| 1486 | CB | SER | A | 867 | 48.547 | -6.107 | 76.706 | 1.00 | 43.71 |
| 1487 | OG | SER | A | 867 | 49.383 | -5.173 | 76.055 | 1.00 | 50.34 |
| 1488 | N | GLN | A | 868 | 46.420 | -7.028 | 74.640 | 1.00 | 35.72 |
| 1489 | CA | GLN | A | 868 | 45.982 | -7.234 | 73.270 | 1.00 | 36.75 |
| 1490 | C | GLN | A | 868 | 44.575 | -6.663 | 73.101 | 1.00 | 31.61 |
| 1491 | O | GLN | A | 868 | 44.198 | -6.218 | 72.017 | 1.00 | 34.73 |
| 1492 | CB | GLN | A | 868 | 46.006 | -8.727 | 72.927 | 1.00 | 41.06 |
| 1493 | CG | GLN | A | 868 | 47.410 | -9.332 | 73.045 | 1.00 | 51.40 |
| 1494 | CD | GLN | A | 868 | 47.458 | -10.819 | 72.756 | 1.00 | 56.24 |
| 1495 | OE1 | GLN | A | 868 | 47.151 | -11.263 | 71.647 | 1.00 | 66.21 |
| 1496 | NE2 | GLN | A | 868 | 47.850 | -11.603 | 73.756 | 1.00 | 61.83 |
| 1497 | N | ARG | A | 869 | 43.815 | -6.667 | 74.188 | 1.00 | 30.91 |
| 1498 | CA | ARG | A | 869 | 42.443 | -6.153 | 74.193 | 1.00 | 33.76 |
| 1499 | C | ARG | A | 869 | 42.483 | -4.639 | 74.028 | 1.00 | 29.74 |
| 1500 | O | ARG | A | 869 | 41.703 | -4.053 | 73.274 | 1.00 | 28.46 |
| 1501 | CB | ARG | A | 869 | 41.781 | -6.493 | 75.521 | 1.00 | 31.23 |
| 1502 | CG | ARG | A | 869 | 40.335 | -6.051 | 75.662 | 1.00 | 40.84 |
| 1503 | CD | ARG | A | 869 | 39.462 | -6.760 | 74.664 | 1.00 | 36.78 |
| 1504 | NE | ARG | A | 869 | 38.039 | -6.531 | 74.910 | 1.00 | 41.13 |
| 1505 | CZ | ARG | A | 869 | 37.079 | -7.104 | 74.198 | 1.00 | 35.24 |
| 1506 | NH1 | ARG | A | 869 | 37.410 | -7.936 | 73.217 | 1.00 | 32.73 |
| 1507 | NH2 | ARG | A | 869 | 35.805 | -6.857 | 74.464 | 1.00 | 33.12 |
| 1508 | N | PHE | A | 870 | 43.405 | -4.017 | 74.748 | 1.00 | 27.87 |
| 1509 | CA | PHE | A | 870 | 43.561 | -2.578 | 74.691 | 1.00 | 29.28 |
| 1510 | C | PHE | A | 870 | 43.929 | -2.212 | 73.268 | 1.00 | 30.03 |
| 1511 | O | PHE | A | 870 | 43.410 | -1.244 | 72.707 | 1.00 | 29.75 |
| 1512 | CB | PHE | A | 870 | 44.655 | -2.123 | 75.644 | 1.00 | 30.24 |
| 1513 | CG | PHE | A | 870 | 44.801 | -0.631 | 75.724 | 1.00 | 32.22 |
| 1514 | CD1 | PHE | A | 870 | 43.875 | 0.136 | 76.426 | 1.00 | 30.59 |
| 1515 | CD2 | PHE | A | 870 | 45.859 | 0.008 | 75.089 | 1.00 | 33.43 |
| 1516 | CE1 | PHE | A | 870 | 44.004 | 1.522 | 76.496 | 1.00 | 29.82 |
| 1517 | CE2 | PHE | A | 870 | 45.996 | 1.389 | 75.152 | 1.00 | 33.14 |
| 1518 | CZ | PHE | A | 870 | 45.064 | 2.149 | 75.859 | 1.00 | 28.67 |
| 1519 | N | TYR | A | 871 | 44.805 | -3.010 | 72.667 | 1.00 | 28.46 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1520 | CA | TYR | A | 871 | 45.230 | -2.753 | 71.297 | 1.00 | 33.75 |
| 1521 | C | TYR | A | 871 | 44.060 | -2.815 | 70.326 | 1.00 | 30.34 |
| 1522 | O | TYR | A | 871 | 43.918 | -1.949 | 69.466 | 1.00 | 31.44 |
| 1523 | CB | TYR | A | 871 | 46.286 | -3.765 | 70.835 | 1.00 | 32.68 |
| 1524 | CG | TYR | A | 871 | 46.685 | -3.571 | 69.387 | 1.00 | 39.92 |
| 1525 | CD1 | TYR | A | 871 | 47.512 | -2.519 | 69.003 | 1.00 | 45.08 |
| 1526 | CD2 | TYR | A | 871 | 46.182 | -4.404 | 68.390 | 1.00 | 45.67 |
| 1527 | CE1 | TYR | A | 871 | 47.829 | -2.297 | 67.656 | 1.00 | 52.13 |
| 1528 | CE2 | TYR | A | 871 | 46.488 | -4.192 | 67.042 | 1.00 | 51.47 |
| 1529 | CZ | TYR | A | 871 | 47.310 | -3.139 | 66.682 | 1.00 | 51.04 |
| 1530 | OH | TYR | A | 871 | 47.608 | -2.930 | 65.351 | 1.00 | 54.66 |
| 1531 | N | GLN | A | 872 | 43.238 | -3.849 | 70.465 | 1.00 | 27.62 |
| 1532 | CA | GLN | A | 872 | 42.087 | -4.041 | 69.601 | 1.00 | 28.60 |
| 1533 | C | GLN | A | 872 | 41.069 | -2.908 | 69.710 | 1.00 | 30.83 |
| 1534 | O | GLN | A | 872 | 40.583 | -2.386 | 68.696 | 1.00 | 26.66 |
| 1535 | CB | GLN | A | 872 | 41.392 | -5.355 | 69.940 | 1.00 | 34.35 |
| 1536 | CG | GLN | A | 872 | 42.276 | -6.586 | 69.790 | 1.00 | 38.32 |
| 1537 | CD | GLN | A | 872 | 41.536 | -7.857 | 70.141 | 1.00 | 43.83 |
| 1538 | OE1 | GLN | A | 872 | 41.009 | -8.001 | 71.246 | 1.00 | 44.13 |
| 1539 | NE2 | GLN | A | 872 | 41.492 | -8.788 | 69.204 | 1.00 | 49.31 |
| 1540 | N | LEU | A | 873 | 40.744 | -2.537 | 70.941 | 1.00 | 26.84 |
| 1541 | CA | LEU | A | 873 | 39.768 | -1.477 | 71.159 | 1.00 | 25.39 |
| 1542 | C | LEU | A | 873 | 40.261 | -0.121 | 70.665 | 1.00 | 27.79 |
| 1543 | O | LEU | A | 873 | 39.494 | 0.632 | 70.052 | 1.00 | 27.31 |
| 1544 | CB | LEU | A | 873 | 39.391 | -1.399 | 72.642 | 1.00 | 24.65 |
| 1545 | CG | LEU | A | 873 | 38.754 | -2.687 | 73.190 | 1.00 | 26.51 |
| 1546 | CD1 | LEU | A | 873 | 38.334 | -2.516 | 74.626 | 1.00 | 29.15 |
| 1547 | CD2 | LEU | A | 873 | 37.565 | -3.064 | 72.327 | 1.00 | 24.41 |
| 1548 | N | THR | A | 874 | 41.528 | 0.195 | 70.920 | 1.00 | 26.19 |
| 1549 | CA | THR | A | 874 | 42.068 | 1.473 | 70.481 | 1.00 | 29.70 |
| 1550 | C | THR | A | 874 | 42.277 | 1.478 | 68.965 | 1.00 | 32.67 |
| 1551 | O | THR | A | 874 | 42.227 | 2.533 | 68.322 | 1.00 | 30.17 |
| 1552 | CB | THR | A | 874 | 43.385 | 1.837 | 71.234 | 1.00 | 29.72 |
| 1553 | OG1 | THR | A | 874 | 44.377 | 0.827 | 71.031 | 1.00 | 33.15 |
| 1554 | CG2 | THR | A | 874 | 43.117 | 1.965 | 72.715 | 1.00 | 32.70 |
| 1555 | N | LYS | A | 875 | 42.489 | 0.306 | 68.377 | 1.00 | 29.23 |
| 1556 | CA | LYS | A | 875 | 42.661 | 0.249 | 66.930 | 1.00 | 29.94 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1557 | C | LYS | A | 875 | 41.290 | 0.526 | 66.311 | 1.00 | 25.15 |
| 1558 | O | LYS | A | 875 | 41.184 | 1.171 | 65.284 | 1.00 | 30.87 |
| 1559 | CB | LYS | A | 875 | 43.173 | -1.135 | 66.495 | 1.00 | 32.66 |
| 1560 | CG | LYS | A | 875 | 43.612 | -1.210 | 65.033 | 1.00 | 41.23 |
| 1561 | CD | LYS | A | 875 | 44.781 | -0.267 | 64.756 | 1.00 | 45.10 |
| 1562 | CE | LYS | A | 875 | 45.193 | -0.300 | 63.295 | 1.00 | 45.88 |
| 1563 | NZ | LYS | A | 875 | 46.368 | 0.572 | 63.042 | 1.00 | 50.22 |
| 1564 | N | LEU | A | 876 | 40.232 | 0.047 | 66.954 | 1.00 | 28.84 |
| 1565 | CA | LEU | A | 876 | 38.875 | 0.282 | 66.460 | 1.00 | 30.79 |
| 1566 | C | LEU | A | 876 | 38.587 | 1.794 | 66.451 | 1.00 | 31.46 |
| 1567 | O | LEU | A | 876 | 38.047 | 2.329 | 65.479 | 1.00 | 28.53 |
| 1568 | CB | LEU | A | 876 | 37.864 | -0.458 | 67.333 | 1.00 | 29.92 |
| 1569 | CG | LEU | A | 876 | 36.369 | -0.362 | 66.999 | 1.00 | 36.08 |
| 1570 | CD1 | LEU | A | 876 | 35.623 | -1.521 | 67.641 | 1.00 | 37.87 |
| 1571 | CD2 | LEU | A | 876 | 35.817 | 0.960 | 67.493 | 1.00 | 34.23 |
| 1572 | N | LEU | A | 877 | 38.960 | 2.481 | 67.525 | 1.00 | 26.83 |
| 1573 | CA | LEU | A | 877 | 38.750 | 3.926 | 67.590 | 1.00 | 32.06 |
| 1574 | C | LEU | A | 877 | 39.517 | 4.622 | 66.465 | 1.00 | 31.54 |
| 1575 | O | LEU | A | 877 | 38.984 | 5.538 | 65.816 | 1.00 | 27.43 |
| 1576 | CB | LEU | A | 877 | 39.191 | 4.472 | 68.950 | 1.00 | 28.40 |
| 1577 | CG | LEU | A | 877 | 38.327 | 4.058 | 70.146 | 1.00 | 33.60 |
| 1578 | CD1 | LEU | A | 877 | 38.892 | 4.669 | 71.418 | 1.00 | 27.76 |
| 1579 | CD2 | LEU | A | 877 | 36.880 | 4.525 | 69.941 | 1.00 | 36.94 |
| 1580 | N | ASP | A | 878 | 40.765 | 4.203 | 66.238 | 1.00 | 30.89 |
| 1581 | CA | ASP | A | 878 | 41.568 | 4.782 | 65.157 | 1.00 | 31.45 |
| 1582 | C | ASP | A | 878 | 40.853 | 4.591 | 63.829 | 1.00 | 33.06 |
| 1583 | O | ASP | A | 878 | 40.771 | 5.522 | 63.026 | 1.00 | 27.21 |
| 1584 | CB | ASP | A | 878 | 42.946 | 4.113 | 65.033 | 1.00 | 32.85 |
| 1585 | CG | ASP | A | 878 | 43.925 | 4.541 | 66.118 | 1.00 | 41.09 |
| 1586 | OD1 | ASP | A | 878 | 43.584 | 5.414 | 66.944 | 1.00 | 34.77 |
| 1587 | OD2 | ASP | A | 878 | 45.055 | 3.994 | 66.134 | 1.00 | 35.56 |
| 1588 | N | ASN | A | 879 | 40.356 | 3.377 | 63.588 | 1.00 | 23.23 |
| 1589 | CA | ASN | A | 879 | 39.665 | 3.101 | 62.334 | 1.00 | 29.88 |
| 1590 | C | ASN | A | 879 | 38.387 | 3.907 | 62.137 | 1.00 | 27.44 |
| 1591 | O | ASN | A | 879 | 37.949 | 4.114 | 61.006 | 1.00 | 28.18 |
| 1592 | CB | ASN | A | 879 | 39.367 | 1.600 | 62.197 | 1.00 | 30.74 |
| 1593 | CG | ASN | A | 879 | 40.637 | 0.764 | 62.168 | 1.00 | 34.40 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1594 | OD1 | ASN | A | 879 | 41.648 | 1.178 | 61.596 | 1.00 | 41.39 |
| 1595 | ND2 | ASN | A | 879 | 40.584 | -0.423 | 62.760 | 1.00 | 42.87 |
| 1596 | N | LEU | A | 880 | 37.796 | 4.383 | 63.226 | 1.00 | 27.04 |
| 1597 | CA | LEU | A | 880 | 36.576 | 5.177 | 63.100 | 1.00 | 28.38 |
| 1598 | C | LEU | A | 880 | 36.792 | 6.459 | 62.283 | 1.00 | 29.38 |
| 1599 | O | LEU | A | 880 | 35.886 | 6.927 | 61.585 | 1.00 | 26.74 |
| 1600 | CB | LEU | A | 880 | 36.018 | 5.508 | 64.484 | 1.00 | 27.75 |
| 1601 | CG | LEU | A | 880 | 35.486 | 4.285 | 65.230 | 1.00 | 38.99 |
| 1602 | CD1 | LEU | A | 880 | 35.009 | 4.692 | 66.610 | 1.00 | 40.02 |
| 1603 | CD2 | LEU | A | 880 | 34.350 | 3.650 | 64.436 | 1.00 | 40.28 |
| 1604 | N | HIS | A | 881 | 37.991 | 7.021 | 62.354 | 1.00 | 25.82 |
| 1605 | CA | HIS | A | 881 | 38.286 | 8.241 | 61.605 | 1.00 | 26.55 |
| 1606 | C | HIS | A | 881 | 37.990 | 8.104 | 60.114 | 1.00 | 28.01 |
| 1607 | O | HIS | A | 881 | 37.313 | 8.950 | 59.539 | 1.00 | 26.18 |
| 1608 | CB | HIS | A | 881 | 39.749 | 8.666 | 61.806 | 1.00 | 27.98 |
| 1609 | CG | HIS | A | 881 | 40.008 | 9.366 | 63.105 | 1.00 | 31.07 |
| 1610 | ND1 | HIS | A | 881 | 40.931 | 8.916 | 64.026 | 1.00 | 34.25 |
| 1611 | CD2 | HIS | A | 881 | 39.492 | 10.509 | 63.619 | 1.00 | 25.45 |
| 1612 | CE1 | HIS | A | 881 | 40.974 | 9.753 | 65.049 | 1.00 | 30.14 |
| 1613 | NE2 | HIS | A | 881 | 40.110 | 10.727 | 64.826 | 1.00 | 32.22 |
| 1614 | N | ASP | A | 882 | 38.468 | 7.039 | 59.481 | 1.00 | 30.88 |
| 1615 | CA | ASP | A | 882 | 38.218 | 6.873 | 58.049 | 1.00 | 33.37 |
| 1616 | C | ASP | A | 882 | 36.746 | 6.575 | 57.802 | 1.00 | 32.18 |
| 1617 | O | ASP | A | 882 | 36.169 | 7.037 | 56.819 | 1.00 | 28.61 |
| 1618 | CB | ASP | A | 882 | 39.082 | 5.750 | 57.472 | 1.00 | 41.89 |
| 1619 | CG | ASP | A | 882 | 39.088 | 5.738 | 55.943 | 1.00 | 53.35 |
| 1620 | OD1 | ASP | A | 882 | 39.469 | 6.768 | 55.336 | 1.00 | 57.38 |
| 1621 | OD2 | ASP | A | 882 | 38.721 | 4.697 | 55.346 | 1.00 | 62.05 |
| 1622 | N | LEU | A | 883 | 36.139 | 5.796 | 58.692 | 1.00 | 25.95 |
| 1623 | CA | LEU | A | 883 | 34.728 | 5.467 | 58.558 | 1.00 | 24.45 |
| 1624 | C | LEU | A | 883 | 33.864 | 6.732 | 58.623 | 1.00 | 24.31 |
| 1625 | O | LEU | A | 883 | 33.015 | 6.966 | 57.764 | 1.00 | 24.16 |
| 1626 | CB | LEU | A | 883 | 34.306 | 4.481 | 59.660 | 1.00 | 25.35 |
| 1627 | CG | LEU | A | 883 | 32.882 | 3.908 | 59.636 | 1.00 | 28.35 |
| 1628 | CD1 | LEU | A | 883 | 32.814 | 2.745 | 60.621 | 1.00 | 31.42 |
| 1629 | CD2 | LEU | A | 883 | 31.847 | 4.957 | 59.983 | 1.00 | 26.70 |
| 1630 | N | VAL | A | 884 | 34.081 | 7.539 | 59.655 | 1.00 | 23.01 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1631 | CA | VAL | A | 884 | 33.314 | 8.771 | 59.837 | 1.00 | 25.27 |
| 1632 | C | VAL | A | 884 | 33.474 | 9.762 | 58.678 | 1.00 | 22.87 |
| 1633 | O | VAL | A | 884 | 32.551 | 10.522 | 58.359 | 1.00 | 21.03 |
| 1634 | CB | VAL | A | 884 | 33.715 | 9.458 | 61.162 | 1.00 | 28.67 |
| 1635 | CG1 | VAL | A | 884 | 33.064 | 10.827 | 61.263 | 1.00 | 30.03 |
| 1636 | CG2 | VAL | A | 884 | 33.274 | 8.588 | 62.338 | 1.00 | 28.50 |
| 1637 | N | LYS | A | 885 | 34.642 | 9.757 | 58.055 | 1.00 | 23.37 |
| 1638 | CA | LYS | A | 885 | 34.886 | 10.649 | 56.926 | 1.00 | 23.75 |
| 1639 | C | LYS | A | 885 | 33.895 | 10.401 | 55.788 | 1.00 | 24.31 |
| 1640 | O | LYS | A | 885 | 33.477 | 11.343 | 55.109 | 1.00 | 23.94 |
| 1641 | CB | LYS | A | 885 | 36.309 | 10.477 | 56.418 | 1.00 | 23.23 |
| 1642 | CG | LYS | A | 885 | 36.681 | 11.476 | 55.335 | 1.00 | 34.73 |
| 1643 | CD | LYS | A | 885 | 38.164 | 11.383 | 54.997 | 1.00 | 40.32 |
| 1644 | CE | LYS | A | 885 | 38.563 | 12.449 | 53.990 | 1.00 | 45.17 |
| 1645 | NZ | LYS | A | 885 | 40.031 | 12.437 | 53.725 | 1.00 | 50.18 |
| 1646 | N | GLN | A | 886 | 33.513 | 9.140 | 55.574 | 1.00 | 20.06 |
| 1647 | CA | GLN | A | 886 | 32.547 | 8.829 | 54.523 | 1.00 | 21.61 |
| 1648 | C | GLN | A | 886 | 31.185 | 9.358 | 54.940 | 1.00 | 20.55 |
| 1649 | O | GLN | A | 886 | 30.423 | 9.843 | 54.104 | 1.00 | 21.72 |
| 1650 | CB | GLN | A | 886 | 32.453 | 7.319 | 54.275 | 1.00 | 27.13 |
| 1651 | CG | GLN | A | 886 | 33.795 | 6.688 | 54.059 | 1.00 | 32.13 |
| 1652 | CD | GLN | A | 886 | 33.718 | 5.402 | 53.291 | 1.00 | 45.67 |
| 1653 | OE1 | GLN | A | 886 | 33.018 | 4.467 | 53.680 | 1.00 | 52.55 |
| 1654 | NE2 | GLN | A | 886 | 34.453 | 5.337 | 52.185 | 1.00 | 51.53 |
| 1655 | N | LEU | A | 887 | 30.859 | 9.241 | 56.226 | 1.00 | 18.60 |
| 1656 | CA | LEU | A | 887 | 29.591 | 9.768 | 56.707 | 1.00 | 19.89 |
| 1657 | C | LEU | A | 887 | 29.602 | 11.309 | 56.577 | 1.00 | 21.61 |
| 1658 | O | LEU | A | 887 | 28.607 | 11.911 | 56.172 | 1.00 | 18.84 |
| 1659 | CB | LEU | A | 887 | 29.363 | 9.362 | 58.163 | 1.00 | 24.80 |
| 1660 | CG | LEU | A | 887 | 29.337 | 7.867 | 58.499 | 1.00 | 32.83 |
| 1661 | CD1 | LEU | A | 887 | 28.908 | 7.716 | 59.961 | 1.00 | 30.42 |
| 1662 | CD2 | LEU | A | 887 | 28.362 | 7.129 | 57.600 | 1.00 | 34.48 |
| 1663 | N | HIS | A | 888 | 30.722 | 11.949 | 56.922 | 1.00 | 20.58 |
| 1664 | CA | HIS | A | 888 | 30.834 | 13.416 | 56.812 | 1.00 | 19.88 |
| 1665 | C | HIS | A | 888 | 30.633 | 13.886 | 55.368 | 1.00 | 23.80 |
| 1666 | O | HIS | A | 888 | 29.931 | 14.867 | 55.107 | 1.00 | 21.80 |
| 1667 | CB | HIS | A | 888 | 32.205 | 13.878 | 57.303 | 1.00 | 21.04 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1668 | CG | HIS | A | 888 | 32.338 | 13.932 | 58.792 | 1.00 | 19.68 |
| 1669 | ND1 | HIS | A | 888 | 33.561 | 13.960 | 59.424 | 1.00 | 21.40 |
| 1670 | CD2 | HIS | A | 888 | 31.407 | 14.036 | 59.768 | 1.00 | 20.80 |
| 1671 | CE1 | HIS | A | 888 | 33.378 | 14.080 | 60.728 | 1.00 | 24.56 |
| 1672 | NE2 | HIS | A | 888 | 32.081 | 14.129 | 60.964 | 1.00 | 26.11 |
| 1673 | N | LEU | A | 889 | 31.248 | 13.178 | 54.426 | 1.00 | 22.67 |
| 1674 | CA | LEU | A | 889 | 31.114 | 13.550 | 53.025 | 1.00 | 21.83 |
| 1675 | C | LEU | A | 889 | 29.679 | 13.377 | 52.530 | 1.00 | 21.54 |
| 1676 | O | LEU | A | 889 | 29.144 | 14.251 | 51.854 | 1.00 | 20.75 |
| 1677 | CB | LEU | A | 889 | 32.056 | 12.712 | 52.174 | 1.00 | 20.66 |
| 1678 | CG | LEU | A | 889 | 32.041 | 13.046 | 50.683 | 1.00 | 26.71 |
| 1679 | CD1 | LEU | A | 889 | 32.298 | 14.544 | 50.441 | 1.00 | 27.37 |
| 1680 | CD2 | LEU | A | 889 | 33.118 | 12.204 | 50.026 | 1.00 | 22.77 |
| 1681 | N | TYR | A | 890 | 29.048 | 12.255 | 52.874 | 1.00 | 19.92 |
| 1682 | CA | TYR | A | 890 | 27.668 | 12.011 | 52.439 | 1.00 | 19.47 |
| 1683 | C | TYR | A | 890 | 26.732 | 13.091 | 53.022 | 1.00 | 22.05 |
| 1684 | O | TYR | A | 890 | 25.853 | 13.637 | 52.339 | 1.00 | 20.67 |
| 1685 | CB | TYR | A | 890 | 27.237 | 10.601 | 52.890 | 1.00 | 19.20 |
| 1686 | CG | TYR | A | 890 | 25.944 | 10.124 | 52.272 | 1.00 | 27.69 |
| 1687 | CD1 | TYR | A | 890 | 24.713 | 10.641 | 52.685 | 1.00 | 26.53 |
| 1688 | CD2 | TYR | A | 890 | 25.954 | 9.174 | 51.250 | 1.00 | 24.52 |
| 1689 | CE1 | TYR | A | 890 | 23.522 | 10.221 | 52.095 | 1.00 | 30.01 |
| 1690 | CE2 | TYR | A | 890 | 24.773 | 8.751 | 50.654 | 1.00 | 26.75 |
| 1691 | CZ | TYR | A | 890 | 23.562 | 9.278 | 51.083 | 1.00 | 33.34 |
| 1692 | OH | TYR | A | 890 | 22.379 | 8.868 | 50.505 | 1.00 | 39.29 |
| 1693 | N | CYS | A | 891 | 26.938 | 13.417 | 54.291 | 1.00 | 19.66 |
| 1694 | CA | CYS | A | 891 | 26.129 | 14.427 | 54.968 | 1.00 | 21.68 |
| 1695 | C | CYS | A | 891 | 26.253 | 15.813 | 54.324 | 1.00 | 20.24 |
| 1696 | O | CYS | A | 891 | 25.256 | 16.468 | 54.016 | 1.00 | 22.45 |
| 1697 | CB | CYS | A | 891 | 26.547 | 14.505 | 56.440 | 1.00 | 22.87 |
| 1698 | SG | CYS | A | 891 | 25.632 | 15.719 | 57.423 | 1.00 | 25.25 |
| 1699 | N | LEU | A | 892 | 27.480 | 16.261 | 54.122 | 1.00 | 21.55 |
| 1700 | CA | LEU | A | 892 | 27.706 | 17.577 | 53.528 | 1.00 | 24.47 |
| 1701 | C | LEU | A | 892 | 27.148 | 17.649 | 52.110 | 1.00 | 25.40 |
| 1702 | O | LEU | A | 892 | 26.626 | 18.691 | 51.703 | 1.00 | 28.02 |
| 1703 | CB | LEU | A | 892 | 29.194 | 17.913 | 53.529 | 1.00 | 24.44 |
| 1704 | CG | LEU | A | 892 | 29.526 | 19.338 | 53.070 | 1.00 | 23.14 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1705 | CD1 | LEU | A | 892 | 28.775 | 20.349 | 53.943 | 1.00 | 29.95 |
| 1706 | CD2 | LEU | A | 892 | 31.029 | 19.551 | 53.163 | 1.00 | 28.07 |
| 1707 | N | ASN | A | 893 | 27.266 | 16.558 | 51.353 | 1.00 | 23.21 |
| 1708 | CA | ASN | A | 893 | 26.702 | 16.526 | 50.002 | 1.00 | 23.92 |
| 1709 | C | ASN | A | 893 | 25.180 | 16.641 | 50.038 | 1.00 | 25.99 |
| 1710 | O | ASN | A | 893 | 24.592 | 17.406 | 49.284 | 1.00 | 27.51 |
| 1711 | CB | ASN | A | 893 | 27.037 | 15.224 | 49.263 | 1.00 | 27.01 |
| 1712 | CG | ASN | A | 893 | 28.363 | 15.275 | 48.562 | 1.00 | 29.03 |
| 1713 | OD1 | ASN | A | 893 | 28.794 | 16.335 | 48.109 | 1.00 | 30.83 |
| 1714 | ND2 | ASN | A | 893 | 29.004 | 14.116 | 48.421 | 1.00 | 29.90 |
| 1715 | N | THR | A | 894 | 24.543 | 15.853 | 50.896 | 1.00 | 25.80 |
| 1716 | CA | THR | A | 894 | 23.089 | 15.871 | 51.014 | 1.00 | 25.04 |
| 1717 | C | THR | A | 894 | 22.594 | 17.211 | 51.529 | 1.00 | 24.51 |
| 1718 | O | THR | A | 894 | 21.518 | 17.670 | 51.147 | 1.00 | 28.41 |
| 1719 | CB | THR | A | 894 | 22.598 | 14.741 | 51.958 | 1.00 | 22.97 |
| 1720 | OG1 | THR | A | 894 | 23.051 | 13.488 | 51.441 | 1.00 | 23.34 |
| 1721 | CG2 | THR | A | 894 | 21.089 | 14.729 | 52.057 | 1.00 | 25.92 |
| 1722 | N | PHE | A | 895 | 23.395 | 17.832 | 52.390 | 1.00 | 23.12 |
| 1723 | CA | PHE | A | 895 | 23.072 | 19.126 | 52.983 | 1.00 | 23.28 |
| 1724 | C | PHE | A | 895 | 23.083 | 20.188 | 51.878 | 1.00 | 26.81 |
| 1725 | O | PHE | A | 895 | 22.185 | 21.022 | 51.808 | 1.00 | 27.75 |
| 1726 | CB | PHE | A | 895 | 24.113 | 19.477 | 54.049 | 1.00 | 22.97 |
| 1727 | CG | PHE | A | 895 | 23.848 | 20.775 | 54.766 | 1.00 | 20.82 |
| 1728 | CD1 | PHE | A | 895 | 22.805 | 20.892 | 55.673 | 1.00 | 21.56 |
| 1729 | CD2 | PHE | A | 895 | 24.632 | 21.892 | 54.497 | 1.00 | 25.72 |
| 1730 | CE1 | PHE | A | 895 | 22.536 | 22.104 | 56.310 | 1.00 | 26.99 |
| 1731 | CE2 | PHE | A | 895 | 24.374 | 23.109 | 55.126 | 1.00 | 28.83 |
| 1732 | CZ | PHE | A | 895 | 23.324 | 23.217 | 56.035 | 1.00 | 24.76 |
| 1733 | N | ILE | A | 896 | 24.104 | 20.157 | 51.024 | 1.00 | 26.30 |
| 1734 | CA | ILE | A | 896 | 24.189 | 21.112 | 49.915 | 1.00 | 27.51 |
| 1735 | C | ILE | A | 896 | 23.068 | 20.887 | 48.886 | 1.00 | 30.83 |
| 1736 | O | ILE | A | 896 | 22.585 | 21.834 | 48.256 | 1.00 | 31.33 |
| 1737 | CB | ILE | A | 896 | 25.561 | 21.018 | 49.213 | 1.00 | 30.94 |
| 1738 | CG1 | ILE | A | 896 | 26.643 | 21.588 | 50.133 | 1.00 | 31.10 |
| 1739 | CG2 | ILE | A | 896 | 25.527 | 21.750 | 47.876 | 1.00 | 33.25 |
| 1740 | CD1 | ILE | A | 896 | 28.031 | 21.529 | 49.540 | 1.00 | 42.81 |
| 1741 | N | GLN | A | 897 | 22.640 | 19.637 | 48.723 | 1.00 | 29.65 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1742 | CA | GLN | A | 897 | 21.590 | 19.326 | 47.752 | 1.00 | 30.69 |
| 1743 | C | GLN | A | 897 | 20.239 | 19.143 | 48.430 | 1.00 | 33.21 |
| 1744 | O | GLN | A | 897 | 19.288 | 18.673 | 47.800 | 1.00 | 36.42 |
| 1745 | CB | GLN | A | 897 | 21.951 | 18.040 | 47.003 | 1.00 | 34.22 |
| 1746 | CG | GLN | A | 897 | 23.385 | 18.025 | 46.508 | 1.00 | 33.58 |
| 1747 | CD | GLN | A | 897 | 23.814 | 16.698 | 45.900 | 1.00 | 46.71 |
| 1748 | OE1 | GLN | A | 897 | 25.010 | 16.444 | 45.732 | 1.00 | 45.14 |
| 1749 | NE2 | GLN | A | 897 | 22.847 | 15.857 | 45.547 | 1.00 | 39.95 |
| 1750 | N | SER | A | 898 | 20.145 | 19.519 | 49.705 | 1.00 | 31.45 |
| 1751 | CA | SER | A | 898 | 18.909 | 19.333 | 50.464 | 1.00 | 35.66 |
| 1752 | C | SER | A | 898 | 17.620 | 19.766 | 49.764 | 1.00 | 34.55 |
| 1753 | O | SER | A | 898 | 16.635 | 19.034 | 49.780 | 1.00 | 31.82 |
| 1754 | CB | SER | A | 898 | 19.011 | 20.008 | 51.839 | 1.00 | 32.53 |
| 1755 | OG | SER | A | 898 | 19.182 | 21.410 | 51.741 | 1.00 | 46.60 |
| 1756 | N | ARG | A | 899 | 17.606 | 20.942 | 49.149 | 1.00 | 37.58 |
| 1757 | CA | ARG | A | 899 | 16.385 | 21.377 | 48.482 | 1.00 | 43.27 |
| 1758 | C | ARG | A | 899 | 16.084 | 20.489 | 47.282 | 1.00 | 46.14 |
| 1759 | O | ARG | A | 899 | 14.925 | 20.152 | 47.023 | 1.00 | 46.04 |
| 1760 | CB | ARG | A | 899 | 16.488 | 22.847 | 48.057 | 1.00 | 45.98 |
| 1761 | CG | ARG | A | 899 | 16.891 | 23.785 | 49.194 | 1.00 | 58.17 |
| 1762 | CD | ARG | A | 899 | 16.030 | 23.598 | 50.451 | 1.00 | 63.03 |
| 1763 | NE | ARG | A | 899 | 14.637 | 24.028 | 50.302 | 1.00 | 72.16 |
| 1764 | CZ | ARG | A | 899 | 14.247 | 25.295 | 50.169 | 1.00 | 72.47 |
| 1765 | NH1 | ARG | A | 899 | 15.142 | 26.275 | 50.168 | 1.00 | 73.96 |
| 1766 | NH2 | ARG | A | 899 | 12.957 | 25.586 | 50.049 | 1.00 | 70.95 |
| 1767 | N | ALA | A | 900 | 17.127 | 20.076 | 46.571 | 1.00 | 43.89 |
| 1768 | CA | ALA | A | 900 | 16.960 | 19.227 | 45.400 | 1.00 | 44.86 |
| 1769 | C | ALA | A | 900 | 16.538 | 17.812 | 45.768 | 1.00 | 45.61 |
| 1770 | O | ALA | A | 900 | 15.768 | 17.174 | 45.053 | 1.00 | 44.57 |
| 1771 | CB | ALA | A | 900 | 18.260 | 19.181 | 44.601 | 1.00 | 49.14 |
| 1772 | N | LEU | A | 901 | 17.049 | 17.325 | 46.891 | 1.00 | 44.06 |
| 1773 | CA | LEU | A | 901 | 16.748 | 15.977 | 47.351 | 1.00 | 42.19 |
| 1774 | C | LEU | A | 901 | 15.509 | 15.927 | 48.227 | 1.00 | 40.67 |
| 1775 | O | LEU | A | 901 | 15.065 | 14.851 | 48.618 | 1.00 | 44.90 |
| 1776 | CB | LEU | A | 901 | 17.949 | 15.425 | 48.119 | 1.00 | 39.53 |
| 1777 | CG | LEU | A | 901 | 19.224 | 15.214 | 47.306 | 1.00 | 41.94 |
| 1778 | CD1 | LEU | A | 901 | 20.369 | 14.833 | 48.234 | 1.00 | 43.72 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1779 | CD2 | LEU | A | 901 | 18.984 | 14.128 | 46.266 | 1.00 | 41.21 |
| 1780 | N | SER | A | 902 | 14.944 | 17.092 | 48.516 | 1.00 | 38.21 |
| 1781 | CA | SER | A | 902 | 13.773 | 17.180 | 49.365 | 1.00 | 35.93 |
| 1782 | C | SER | A | 902 | 14.094 | 16.659 | 50.762 | 1.00 | 34.57 |
| 1783 | O | SER | A | 902 | 13.243 | 16.063 | 51.415 | 1.00 | 33.32 |
| 1784 | CB | SER | A | 902 | 12.603 | 16.382 | 48.773 | 1.00 | 42.40 |
| 1785 | OG | SER | A | 902 | 12.170 | 16.942 | 47.546 | 1.00 | 50.36 |
| 1786 | N | VAL | A | 903 | 15.325 | 16.884 | 51.219 | 1.00 | 32.92 |
| 1787 | CA | VAL | A | 903 | 15.734 | 16.436 | 52.552 | 1.00 | 30.26 |
| 1788 | C | VAL | A | 903 | 15.849 | 17.645 | 53.484 | 1.00 | 31.81 |
| 1789 | O | VAL | A | 903 | 16.582 | 18.586 | 53.198 | 1.00 | 34.09 |
| 1790 | CB | VAL | A | 903 | 17.087 | 15.700 | 52.500 | 1.00 | 30.61 |
| 1791 | CG1 | VAL | A | 903 | 17.527 | 15.298 | 53.919 | 1.00 | 30.31 |
| 1792 | CG2 | VAL | A | 903 | 16.967 | 14.449 | 51.608 | 1.00 | 26.90 |
| 1793 | N | GLU | A | 904 | 15.104 | 17.627 | 54.584 | 1.00 | 28.12 |
| 1794 | CA | GLU | A | 904 | 15.153 | 18.720 | 55.543 | 1.00 | 27.45 |
| 1795 | C | GLU | A | 904 | 16.163 | 18.489 | 56.665 | 1.00 | 27.94 |
| 1796 | O | GLU | A | 904 | 16.217 | 17.400 | 57.223 | 1.00 | 26.23 |
| 1797 | CB | GLU | A | 904 | 13.780 | 18.941 | 56.175 | 1.00 | 33.68 |
| 1798 | CG | GLU | A | 904 | 13.807 | 20.006 | 57.271 | 1.00 | 51.81 |
| 1799 | CD | GLU | A | 904 | 12.457 | 20.233 | 57.929 | 1.00 | 62.71 |
| 1800 | OE1 | GLU | A | 904 | 11.469 | 19.575 | 57.530 | 1.00 | 63.38 |
| 1801 | OE2 | GLU | A | 904 | 12.387 | 21.080 | 58.852 | 1.00 | 63.52 |
| 1802 | N | PHE | A | 905 | 16.957 | 19.516 | 56.982 | 1.00 | 25.35 |
| 1803 | CA | PHE | A | 905 | 17.935 | 19.447 | 58.071 | 1.00 | 24.39 |
| 1804 | C | PHE | A | 905 | 17.468 | 20.454 | 59.109 | 1.00 | 26.69 |
| 1805 | O | PHE | A | 905 | 17.219 | 21.610 | 58.770 | 1.00 | 27.33 |
| 1806 | CB | PHE | A | 905 | 19.359 | 19.856 | 57.629 | 1.00 | 25.14 |
| 1807 | CG | PHE | A | 905 | 20.072 | 18.833 | 56.786 | 1.00 | 21.63 |
| 1808 | CD1 | PHE | A | 905 | 19.735 | 18.642 | 55.457 | 1.00 | 22.11 |
| 1809 | CD2 | PHE | A | 905 | 21.068 | 18.043 | 57.343 | 1.00 | 23.38 |
| 1810 | CE1 | PHE | A | 905 | 20.383 | 17.678 | 54.691 | 1.00 | 19.55 |
| 1811 | CE2 | PHE | A | 905 | 21.726 | 17.072 | 56.592 | 1.00 | 24.14 |
| 1812 | CZ | PHE | A | 905 | 21.382 | 16.888 | 55.261 | 1.00 | 22.83 |
| 1813 | N | PRO | A | 906 | 17.333 | 20.037 | 60.379 | 1.00 | 23.73 |
| 1814 | CA | PRO | A | 906 | 16.891 | 20.955 | 61.432 | 1.00 | 22.74 |
| 1815 | C | PRO | A | 906 | 17.982 | 22.002 | 61.720 | 1.00 | 22.33 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1816 | O | PRO | A | 906 | 19.116 | 21.882 | 61.267 | 1.00 | 22.26 |
| 1817 | CB | PRO | A | 906 | 16.649 | 20.024 | 62.623 | 1.00 | 24.51 |
| 1818 | CG | PRO | A | 906 | 16.392 | 18.675 | 61.959 | 1.00 | 30.94 |
| 1819 | CD | PRO | A | 906 | 17.516 | 18.695 | 60.948 | 1.00 | 29.32 |
| 1820 | N | GLU | A | 907 | 17.627 | 23.016 | 62.495 | 1.00 | 25.11 |
| 1821 | CA | GLU | A | 907 | 18.543 | 24.119 | 62.809 | 1.00 | 22.58 |
| 1822 | C | GLU | A | 907 | 19.848 | 23.834 | 63.543 | 1.00 | 21.81 |
| 1823 | O | GLU | A | 907 | 20.896 | 24.342 | 63.162 | 1.00 | 23.58 |
| 1824 | CB | GLU | A | 907 | 17.782 | 25.193 | 63.589 | 1.00 | 23.00 |
| 1825 | CG | GLU | A | 907 | 16.592 | 25.833 | 62.848 | 1.00 | 25.08 |
| 1826 | CD | GLU | A | 907 | 16.951 | 26.530 | 61.539 | 1.00 | 31.26 |
| 1827 | OE1 | GLU | A | 907 | 18.064 | 27.086 | 61.413 | 1.00 | 31.56 |
| 1828 | OE2 | GLU | A | 907 | 16.089 | 26.561 | 60.631 | 1.00 | 31.17 |
| 1829 | N | MET | A | 908 | 19.803 | 23.052 | 64.618 | 1.00 | 20.50 |
| 1830 | CA | MET | A | 908 | 21.033 | 22.786 | 65.356 | 1.00 | 22.40 |
| 1831 | C | MET | A | 908 | 21.994 | 21.936 | 64.541 | 1.00 | 20.88 |
| 1832 | O | MET | A | 908 | 23.205 | 22.153 | 64.566 | 1.00 | 22.84 |
| 1833 | CB | MET | A | 908 | 20.706 | 22.112 | 66.694 | 1.00 | 28.13 |
| 1834 | CG | MET | A | 908 | 19.770 | 22.954 | 67.553 | 1.00 | 30.10 |
| 1835 | SD | MET | A | 908 | 19.342 | 22.226 | 69.147 | 1.00 | 36.75 |
| 1836 | CE | MET | A | 908 | 18.819 | 20.575 | 68.656 | 1.00 | 29.48 |
| 1837 | N | MET | A | 909 | 21.458 | 20.977 | 63.798 | 1.00 | 22.18 |
| 1838 | CA | MET | A | 909 | 22.303 | 20.120 | 62.982 | 1.00 | 20.40 |
| 1839 | C | MET | A | 909 | 22.866 | 20.915 | 61.825 | 1.00 | 20.24 |
| 1840 | O | MET | A | 909 | 24.018 | 20.734 | 61.442 | 1.00 | 20.23 |
| 1841 | CB | MET | A | 909 | 21.496 | 18.937 | 62.450 | 1.00 | 24.51 |
| 1842 | CG | MET | A | 909 | 22.335 | 17.920 | 61.747 | 1.00 | 36.88 |
| 1843 | SD | MET | A | 909 | 21.313 | 16.481 | 61.357 | 1.00 | 38.44 |
| 1844 | CE | MET | A | 909 | 22.491 | 15.495 | 60.507 | 1.00 | 42.73 |
| 1845 | N | SER | A | 910 | 22.052 | 21.801 | 61.263 | 1.00 | 22.17 |
| 1846 | CA | SER | A | 910 | 22.520 | 22.624 | 60.160 | 1.00 | 24.13 |
| 1847 | C | SER | A | 910 | 23.678 | 23.505 | 60.651 | 1.00 | 24.76 |
| 1848 | O | SER | A | 910 | 24.641 | 23.752 | 59.915 | 1.00 | 20.25 |
| 1849 | CB | SER | A | 910 | 21.381 | 23.509 | 59.630 | 1.00 | 24.96 |
| 1850 | OG | SER | A | 910 | 20.334 | 22.728 | 59.067 | 1.00 | 29.09 |
| 1851 | N | GLU | A | 911 | 23.577 | 23.985 | 61.888 | 1.00 | 21.90 |
| 1852 | CA | GLU | A | 911 | 24.637 | 24.831 | 62.430 | 1.00 | 25.07 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1853 | C | GLU | A | 911 | 25.965 | 24.103 | 62.594 | 1.00 | 25.56 |
| 1854 | O | GLU | A | 911 | 26.997 | 24.631 | 62.208 | 1.00 | 26.95 |
| 1855 | CB | GLU | A | 911 | 24.232 | 25.458 | 63.770 | 1.00 | 26.93 |
| 1856 | CG | GLU | A | 911 | 25.361 | 26.272 | 64.407 | 1.00 | 30.33 |
| 1857 | CD | GLU | A | 911 | 25.842 | 27.437 | 63.535 | 1.00 | 45.68 |
| 1858 | OE1 | GLU | A | 911 | 25.054 | 27.944 | 62.705 | 1.00 | 48.37 |
| 1859 | OE2 | GLU | A | 911 | 27.010 | 27.865 | 63.694 | 1.00 | 40.75 |
| 1860 | N | VAL | A | 912 | 25.959 | 22.901 | 63.163 | 1.00 | 23.66 |
| 1861 | CA | VAL | A | 912 | 27.215 | 22.197 | 63.335 | 1.00 | 24.09 |
| 1862 | C | VAL | A | 912 | 27.816 | 21.806 | 61.995 | 1.00 | 22.11 |
| 1863 | O | VAL | A | 912 | 29.035 | 21.848 | 61.830 | 1.00 | 23.47 |
| 1864 | CB | VAL | A | 912 | 27.070 | 20.931 | 64.259 | 1.00 | 25.40 |
| 1865 | CG1 | VAL | A | 912 | 26.617 | 21.357 | 65.635 | 1.00 | 27.52 |
| 1866 | CG2 | VAL | A | 912 | 26.066 | 19.926 | 63.673 | 1.00 | 28.29 |
| 1867 | N | ILE | A | 913 | 26.968 | 21.462 | 61.022 | 1.00 | 20.76 |
| 1868 | CA | ILE | A | 913 | 27.475 | 21.077 | 59.704 | 1.00 | 20.13 |
| 1869 | C | ILE | A | 913 | 28.167 | 22.255 | 59.050 | 1.00 | 23.37 |
| 1870 | O | ILE | A | 913 | 29.302 | 22.149 | 58.567 | 1.00 | 25.36 |
| 1871 | CB | ILE | A | 913 | 26.336 | 20.565 | 58.788 | 1.00 | 19.24 |
| 1872 | CG1 | ILE | A | 913 | 25.835 | 19.220 | 59.302 | 1.00 | 22.39 |
| 1873 | CG2 | ILE | A | 913 | 26.833 | 20.425 | 57.348 | 1.00 | 18.20 |
| 1874 | CD1 | ILE | A | 913 | 24.472 | 18.815 | 58.727 | 1.00 | 26.26 |
| 1875 | N | ALA | A | 914 | 27.491 | 23.392 | 59.051 | 1.00 | 23.60 |
| 1876 | CA | ALA | A | 914 | 28.041 | 24.600 | 58.451 | 1.00 | 30.05 |
| 1877 | C | ALA | A | 914 | 29.260 | 25.131 | 59.199 | 1.00 | 27.97 |
| 1878 | O | ALA | A | 914 | 30.189 | 25.666 | 58.595 | 1.00 | 31.89 |
| 1879 | CB | ALA | A | 914 | 26.953 | 25.694 | 58.381 | 1.00 | 24.81 |
| 1880 | N | ALA | A | 915 | 29.273 | 24.983 | 60.515 | 1.00 | 28.98 |
| 1881 | CA | ALA | A | 915 | 30.394 | 25.494 | 61.292 | 1.00 | 30.10 |
| 1882 | C | ALA | A | 915 | 31.698 | 24.711 | 61.143 | 1.00 | 29.70 |
| 1883 | O | ALA | A | 915 | 32.777 | 25.299 | 61.203 | 1.00 | 30.09 |
| 1884 | CB | ALA | A | 915 | 30.009 | 25.578 | 62.773 | 1.00 | 29.66 |
| 1885 | N | GLN | A | 916 | 31.622 | 23.401 | 60.923 | 1.00 | 26.34 |
| 1886 | CA | GLN | A | 916 | 32.848 | 22.627 | 60.843 | 1.00 | 23.29 |
| 1887 | C | GLN | A | 916 | 33.045 | 21.567 | 59.764 | 1.00 | 23.47 |
| 1888 | O | GLN | A | 916 | 34.190 | 21.219 | 59.480 | 1.00 | 24.44 |
| 1889 | CB | GLN | A | 916 | 33.101 | 21.914 | 62.180 | 1.00 | 29.83 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1890 | CG | GLN | A | 916 | 33.182 | 22.761 | 63.424 | 1.00 | 31.14 |
| 1891 | CD | GLN | A | 916 | 34.230 | 23.845 | 63.355 | 1.00 | 37.37 |
| 1892 | OE1 | GLN | A | 916 | 35.302 | 23.660 | 62.780 | 1.00 | 33.07 |
| 1893 | NE2 | GLN | A | 916 | 33.938 | 24.979 | 63.979 | 1.00 | 42.40 |
| 1894 | N | LEU | A | 917 | 31.980 | 21.005 | 59.193 | 1.00 | 24.92 |
| 1895 | CA | LEU | A | 917 | 32.200 | 19.927 | 58.218 | 1.00 | 27.91 |
| 1896 | C | LEU | A | 917 | 33.163 | 20.194 | 57.078 | 1.00 | 27.51 |
| 1897 | O | LEU | A | 917 | 34.019 | 19.362 | 56.790 | 1.00 | 30.01 |
| 1898 | CB | LEU | A | 917 | 30.885 | 19.372 | 57.646 | 1.00 | 28.45 |
| 1899 | CG | LEU | A | 917 | 30.195 | 18.339 | 58.540 | 1.00 | 34.41 |
| 1900 | CD1 | LEU | A | 917 | 29.179 | 17.522 | 57.722 | 1.00 | 28.16 |
| 1901 | CD2 | LEU | A | 917 | 31.244 | 17.388 | 59.093 | 1.00 | 34.26 |
| 1902 | N | PRO | A | 918 | 33.025 | 21.329 | 56.387 | 1.00 | 27.54 |
| 1903 | CA | PRO | A | 918 | 33.977 | 21.564 | 55.301 | 1.00 | 27.73 |
| 1904 | C | PRO | A | 918 | 35.434 | 21.568 | 55.809 | 1.00 | 24.86 |
| 1905 | O | PRO | A | 918 | 36.325 | 21.024 | 55.166 | 1.00 | 25.75 |
| 1906 | CB | PRO | A | 918 | 33.536 | 22.922 | 54.762 | 1.00 | 27.68 |
| 1907 | CG | PRO | A | 918 | 32.031 | 22.901 | 55.026 | 1.00 | 26.00 |
| 1908 | CD | PRO | A | 918 | 32.060 | 22.439 | 56.472 | 1.00 | 28.16 |
| 1909 | N | LYS | A | 919 | 35.672 | 22.184 | 56.962 | 1.00 | 23.19 |
| 1910 | CA | LYS | A | 919 | 37.023 | 22.238 | 57.529 | 1.00 | 24.99 |
| 1911 | C | LYS | A | 919 | 37.508 | 20.830 | 57.870 | 1.00 | 26.95 |
| 1912 | O | LYS | A | 919 | 38.632 | 20.444 | 57.537 | 1.00 | 26.77 |
| 1913 | CB | LYS | A | 919 | 37.023 | 23.105 | 58.794 | 1.00 | 31.71 |
| 1914 | CG | LYS | A | 919 | 38.382 | 23.291 | 59.446 | 1.00 | 36.39 |
| 1915 | CD | LYS | A | 919 | 38.233 | 24.038 | 60,774 | 1.00 | 41.59 |
| 1916 | CE | LYS | A | 919 | 37.608 | 25.418 | 60.579 | 1.00 | 46.46 |
| 1917 | NZ | LYS | A | 919 | 37.319 | 26.115 | 61.879 | 1.00 | 52.55 |
| 1918 | N | ILE | A | 920 | 36.655 | 20.057 | 58.533 | 1.00 | 25.62 |
| 1919 | CA | ILE | A | 920 | 37.013 | 18.683 | 58.898 | 1.00 | 25.44 |
| 1920 | C | ILE | A | 920 | 37.322 | 17.851 | 57.660 | 1.00 | 28.84 |
| 1921 | O | ILE | A | 920 | 38.336 | 17.156 | 57.607 | 1.00 | 23.14 |
| 1922 | CB | ILE | A | 920 | 35.871 | 18.019 | 59.665 | 1.00 | 25.43 |
| 1923 | CG1 | ILE | A | 920 | 35.618 | 18.810 | 60.951 | 1.00 | 27.84 |
| 1924 | CG2 | ILE | A | 920 | 36.195 | 16.555 | 59.947 | 1.00 | 26.23 |
| 1925 | CD1 | ILE | A | 920 | 34.396 | 18.353 | 61.713 | 1.00 | 30.88 |
| 1926 | N | LEU | A | 921 | 36.439 | 17.900 | 56.667 | 1.00 | 26.45 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1927 | CA | LEU | A | 921 | 36.666 | 17.144 | 55.427 | 1.00 | 29.61 |
| 1928 | C | LEU | A | 921 | 37.946 | 17.540 | 54.707 | 1.00 | 30.39 |
| 1929 | O | LEU | A | 921 | 38.630 | 16.695 | 54.116 | 1.00 | 32.46 |
| 1930 | CB | LEU | A | 921 | 35.480 | 17.314 | 54.469 | 1.00 | 27.19 |
| 1931 | CG | LEU | A | 921 | 34.263 | 16.472 | 54.845 | 1.00 | 30.49 |
| 1932 | CD1 | LEU | A | 921 | 33.102 | 16.772 | 53.931 | 1.00 | 31.06 |
| 1933 | CD2 | LEU | A | 921 | 34.648 | 14.981 | 54.750 | 1.00 | 32.39 |
| 1934 | N | ALA | A | 922 | 38.271 | 18.824 | 54.743 | 1.00 | 28.14 |
| 1935 | CA | ALA | A | 922 | 39.474 | 19.290 | 54.081 | 1.00 | 29.10 |
| 1936 | C | ALA | A | 922 | 40.713 | 18.854 | 54.858 | 1.00 | 29.03 |
| 1937 | O | ALA | A | 922 | 41.827 | 19.063 | 54.407 | 1.00 | 32.66 |
| 1938 | CB | ALA | A | 922 | 39.431 | 20.802 | 53.934 | 1.00 | 25.78 |
| 1939 | N | GLY | A | 923 | 40.517 | 18.239 | 56.025 | 1.00 | 30.49 |
| 1940 | CA | GLY | A | 923 | 41.654 | 17.799 | 56.821 | 1.00 | 30.14 |
| 1941 | C | GLY | A | 923 | 42.363 | 18.923 | 57.570 | 1.00 | 28.27 |
| 1942 | O | GLY | A | 923 | 43.565 | 18.844 | 57.827 | 1.00 | 35.23 |
| 1943 | N | MET | A | 924 | 41.634 | 19.975 | 57.926 | 1.00 | 27.77 |
| 1944 | CA | MET | A | 924 | 42.237 | 21.094 | 58.641 | 1.00 | 31.40 |
| 1945 | C | MET | A | 924 | 42.021 | 20.949 | 60.147 | 1.00 | 28.03 |
| 1946 | O | MET | A | 924 | 41.716 | 21.914 | 60.846 | 1.00 | 31.74 |
| 1947 | CB | MET | A | 924 | 41.656 | 22.412 | 58.126 | 1.00 | 32.66 |
| 1948 | CG | MET | A | 924 | 41.757 | 22.533 | 56.622 | 1.00 | 42.63 |
| 1949 | SD | MET | A | 924 | 43.454 | 22.403 | 56.010 | 1.00 | 42.80 |
| 1950 | CE | MET | A | 924 | 44.203 | 23.867 | 56.804 | 1.00 | 43.49 |
| 1951 | N | VAL | A | 925 | 42.167 | 19.715 | 60.616 | 1.00 | 29.55 |
| 1952 | CA | VAL | A | 925 | 42.028 | 19.354 | 62.024 | 1.00 | 28.30 |
| 1953 | C | VAL | A | 925 | 43.041 | 18.257 | 62.292 | 1.00 | 31.38 |
| 1954 | O | VAL | A | 925 | 43.547 | 17.632 | 61.360 | 1.00 | 28.00 |
| 1955 | CB | VAL | A | 925 | 40.620 | 18.814 | 62.362 | 1.00 | 27.98 |
| 1956 | CG1 | VAL | A | 925 | 39.587 | 19.919 | 62.174 | 1.00 | 25.65 |
| 1957 | CG2 | VAL | A | 925 | 40.310 | 17.599 | 61.504 | 1.00 | 28.25 |
| 1958 | N | LYS | A | 926 | 43.319 | 18.017 | 63.568 | 1.00 | 28.65 |
| 1959 | CA | LYS | A | 926 | 44.299 | 17.027 | 63.972 | 1.00 | 27.13 |
| 1960 | C | LYS | A | 926 | 43.667 | 15.795 | 64.609 | 1.00 | 27.33 |
| 1961 | O | LYS | A | 926 | 43.332 | 15.791 | 65.790 | 1.00 | 26.71 |
| 1962 | CB | LYS | A | 926 | 45.275 | 17.677 | 64.949 | 1.00 | 30.31 |
| 1963 | CG | LYS | A | 926 | 46.273 | 16.727 | 65.570 | 1.00 | 36.48 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 1964 | CD | LYS | A | 926 | 47.144 | 17.464 | 66.576 | 1.00 | 42.65 |
| 1965 | CE | LYS | A | 926 | 48.016 | 16.489 | 67.350 | 1.00 | 45.66 |
| 1966 | NZ | LYS | A | 926 | 48.834 | 15.673 | 66.424 | 1.00 | 41.46 |
| 1967 | N | PRO | A | 927 | 43.494 | 14.725 | 63.825 | 1.00 | 29.35 |
| 1968 | CA | PRO | A | 927 | 42.894 | 13.504 | 64.360 | 1.00 | 29.76 |
| 1969 | C | PRO | A | 927 | 43.890 | 12.891 | 65.342 | 1.00 | 32.65 |
| 1970 | O | PRO | A | 927 | 45.079 | 12.817 | 65.033 | 1.00 | 31.57 |
| 1971 | CB | PRO | A | 927 | 42.721 | 12.641 | 63.109 | 1.00 | 32.15 |
| 1972 | CG | PRO | A | 927 | 42.704 | 13.692 | 61.952 | 1.00 | 33.46 |
| 1973 | CD | PRO | A | 927 | 43.837 | 14.547 | 62.404 | 1.00 | 29.87 |
| 1974 | N | LEU | A | 928 | 43.442 | 12.483 | 66.528 | 1.00 | 26.08 |
| 1975 | CA | LEU | A | 928 | 44.376 | 11.871 | 67.467 | 1.00 | 28.80 |
| 1976 | C | LEU | A | 928 | 44.318 | 10.382 | 67.226 | 1.00 | 32.70 |
| 1977 | O | LEU | A | 928 | 43.235 | 9.805 | 67.126 | 1.00 | 32.54 |
| 1978 | CB | LEU | A | 928 | 44.005 | 12.198 | 68.920 | 1.00 | 28.21 |
| 1979 | CG | LEU | A | 928 | 43.999 | 13.703 | 69.214 | 1.00 | 27.44 |
| 1980 | CD1 | LEU | A | 928 | 43.689 | 13.946 | 70.690 | 1.00 | 25.95 |
| 1981 | CD2 | LEU | A | 928 | 45.361 | 14.305 | 68.852 | 1.00 | 28.60 |
| 1982 | N | LEU | A | 929 | 45.485 | 9.763 | 67.116 | 1.00 | 29.11 |
| 1983 | CA | LEU | A | 929 | 45.564 | 8.329 | 66.855 | 1.00 | 32.04 |
| 1984 | C | LEU | A | 929 | 46.301 | 7.595 | 67.966 | 1.00 | 33.98 |
| 1985 | O | LEU | A | 929 | 47.258 | 8.125 | 68.526 | 1.00 | 35.14 |
| 1986 | CB | LEU | A | 929 | 46.292 | 8.092 | 65.525 | 1.00 | 30.80 |
| 1987 | CG | LEU | A | 929 | 45.639 | 8.707 | 64.281 | 1.00 | 41.73 |
| 1988 | CD1 | LEU | A | 929 | 46.568 | 8.573 | 63.076 | 1.00 | 37.07 |
| 1989 | CD2 | LEU | A | 929 | 44.309 | 8.022 | 64.023 | 1.00 | 38.20 |
| 1990 | N | PHE | A | 930 | 45.844 | 6.387 | 68.293 | 1.00 | 30.93 |
| 1991 | CA | PHE | A | 930 | 46.505 | 5.580 | 69.316 | 1.00 | 36.94 |
| 1992 | C | PHE | A | 930 | 47.659 | 4.788 | 68.730 | 1.00 | 37.82 |
| 1993 | O | PHE | A | 930 | 48.595 | 4.419 | 69.441 | 1.00 | 40.73 |
| 1994 | CB | PHE | A | 930 | 45.546 | 4.589 | 69.962 | 1.00 | 33.98 |
| 1995 | CG | PHE | A | 930 | 44.615 | 5.212 | 70.942 | 1.00 | 31.86 |
| 1996 | CD1 | PHE | A | 930 | 45.035 | 5.440 | 72.248 | 1.00 | 27.98 |
| 1997 | CD2 | PHE | A | 930 | 43.346 | 5.613 | 70.562 | 1.00 | 29.84 |
| 1998 | CE1 | PHE | A | 930 | 44.197 | 6.063 | 73.157 | 1.00 | 34.01 |
| 1999 | CE2 | PHE | A | 930 | 42.496 | 6.238 | 71.470 | 1.00 | 31.12 |
| 2000 | CZ | PHE | A | 930 | 42.922 | 6.464 | 72.765 | 1.00 | 31.58 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2001 | N | HIS | A | 931 | 47.580 | 4.517 | 67.434 | 1.00 | 39.64 |
| 2002 | CA | HIS | A | 931 | 48.622 | 3.762 | 66.764 | 1.00 | 43.59 |
| 2003 | C | HIS | A | 931 | 49.174 | 4.515 | 65.573 | 1.00 | 45.74 |
| 2004 | O | HIS | A | 931 | 48.430 | 4.988 | 64.719 | 1.00 | 48.72 |
| 2005 | CB | HIS | A | 931 | 48.065 | 2.406 | 66.347 | 1.00 | 41.38 |
| 2006 | CG | HIS | A | 931 | 47.460 | 1.655 | 67.488 | 1.00 | 44.29 |
| 2007 | ND1 | HIS | A | 931 | 48.185 | 1.302 | 68.605 | 1.00 | 49.69 |
| 2008 | CD2 | HIS | A | 931 | 46.184 | 1.276 | 67.733 | 1.00 | 48.24 |
| 2009 | CE1 | HIS | A | 931 | 47.381 | 0.741 | 69.491 | 1.00 | 50.45 |
| 2010 | NE2 | HIS | A | 931 | 46.161 | 0.712 | 68.986 | 1.00 | 49.57 |
| 2011 1 | N | LYS | A | 932 | 50.496 | 4.635 | 65.550 | 1.00 | 50.39 |
| 2012 | CA | LYS | A | 932 | 51.221 | 5.314 | 64.486 | 1.00 | 56.63 |
| 2013 | C | LYS | A | 932 | 50.976 | 4.605 | 63.161 | 1.00 | 53.01 |
| 2014 | O | LYS | A | 932 | 50.889 | 3.363 | 63.224 | 1.00 | 56.94 |
| 2015 | CB | LYS | A | 932 | 52.711 | 5.303 | 64.831 | 1.00 | 57.98 |
| 2016 | CG | LYS | A | 932 | 53.169 | 3.943 | 65.342 | 1.00 | 61.12 |
| 2017 | CD | LYS | A | 932 | 54.581 | 3.972 | 65.892 | 1.00 | 65.23 |
| 2018 | CE | LYS | A | 932 | 54.895 | 2.685 | 66.651 | 1.00 | 65.01 |
| 2019 | NZ | LYS | A | 932 | 54.769 | 1.469 | 65.799 | 1.00 | 63.73 |
| 2020 | | LYS | A | 932 | | | | | |
| 2021 | C1 | STR | A | 1 | 21.206 | 9.935 | 63.081 | 1.00 | 24.82 |
| 2022 | C2 | STR | A | 1 | 21.241 | 9.446 | 64.551 | 1.00 | 23.75 |
| 2023 | C3 | STR | A | 1 | 22.000 | 8.125 | 64.630 | 1.00 | 25.95 |
| 2024 | 03 | STR | A | 1 | 21.701 | 7.301 | 65.512 | 1.00 | 31.79 |
| 2025 | C4 | STR | A | 1 | 23.118 | 7.872 | 63.734 | 1.00 | 21.13 |
| 2026 | C5 | STR | A | 1 | 23.453 | 8.727 | 62.785 | 1.00 | 20.44 |
| 2027 | C6 | STR | A | 1 | 24.697 | 8.443 | 61.951 | 1.00 | 23.35 |
| 2028 | C7 | STR | A | 1 | 24.449 | 8.637 | 60.443 | 1.00 | 28.93 |
| 2029 | C8 | STR | A | 1 | 23.789 | 9.997 | 60.098 | 1.00 | 21.68 |
| 2030 | C9 | STR | A | 1 | 22.434 | 10.095 | 60.872 | 1.00 | 20.21 |
| 2031 | C10 | STR | A | 1 | 22.614 | 10.023 | 62.434 | 1.00 | 19.15 |
| 2032 | C11 | STR | A | 1 | 21.633 | 11.354 | 60.450 | 1.00 | 20.97 |
| 2033 | C12 | STR | A | 1 | 21.432 | 11.434 | 58.911 | 1.00 | 21.57 |
| 2034 | C13 | STR | A | 1 | 22.786 | 11.404 | 58.169 | 1.00 | 21.81 |
| 2035 | C14 | STR | A | 1 | 23.483 | 10.060 | 58.598 | 1.00 | 21.81 |
| 2036 | C15 | STR | A | 1 | 24.674 | 9.918 | 57.618 | 1.00 | 26.96 |
| 2037 | C16 | STR | A | 1 | 24.072 | 10.450 | 56.267 | 1.00 | 31.06 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2038 | C17 | STR | A | 1 | 22.714 | 11.149 | 56.627 | 1.00 | 22.28 |
| 2039 | C18 | STR | A | 1 | 23.659 | 12.677 | 58.454 | 1.00 | 20.57 |
| 2040 | C19 | STR | A | 1 | 23.427 | 11.246 | 63.007 | 1.00 | 21.41 |
| 2041 | C20 | STR | A | 1 | 22.375 | 12.388 | 55.781 | 1.00 | 25.07 |
| 2042 | 020 | STR | A | 1 | 23.212 | 12.876 | 55.052 | 1.00 | 29.48 |
| 2043 | C21 | STR | A | 1 | 21.009 | 12.976 | 55.857 | 1.00 | 25.63 |
| 2044 | N | LEU | B | 683 | 60.447 | 28.744 | 14.730 | 1.00 | 61.24 |
| 2045 | CA | LEU | B | 683 | 59.070 | 29.295 | 14.876 | 1.00 | 59.60 |
| 2046 | C | LEU | B | 683 | 58.273 | 28.573 | 15.963 | 1.00 | 56.34 |
| 2047 | O | LEU | B | 683 | 58.751 | 27.612 | 16.566 | 1.00 | 58.01 |
| 2048 | CB | LEU | B | 683 | 58.346 | 29.241 | 13.523 | 1.00 | 59.47 |
| 2049 | CG | LEU | B | 683 | 58.334 | 27.934 | 12.719 | 1.00 | 63.19 |
| 2050 | CD1 | LEU | B | 683 | 57.530 | 26.865 | 13.447 | 1.00 | 61.93 |
| 2051 | CD2 | LEU | B | 683 | 57.728 | 28.201 | 11.343 | 1.00 | 61.94 |
| 2052 | N | ILE | B | 684 | 57.055 | 29.045 | 16.212 | 1.00 | 57.02 |
| 2053 | CA | ILE | B | 684 | 56.194 | 28.468 | 17.244 | 1.00 | 48.99 |
| 2054 | C | ILE | B | 684 | 55.519 | 27.152 | 16.828 | 1.00 | 48.76 |
| 2055 | O | ILE | B | 684 | 54.990 | 27.044 | 15.724 | 1.00 | 41.43 |
| 2056 | CB | ILE | B | 684 | 55.094 | 29.468 | 17.631 | 1.00 | 54.99 |
| 2057 | CG1 I | ILE | B | 684 | 55.718 | 30.827 | 17.971 | 1.00 | 54.78 |
| 2058 | CG2 | ILE | B | 684 | 54.296 | 28.933 | 18.815 | 1.00 | 49.74 |
| 2059 | CD1 | ILE | B | 684 | 56.677 | 30.804 | 19.143 | 1.00 | 54.94 |
| 2060 | N | PRO | B | 685 | 55.532 | 26.135 | 17.710 | 1.00 | 42.36 |
| 2061 | CA | PRO | B | 685 | 54.912 | 24.837 | 17.424 | 1.00 | 42.23 |
| 2062 | C | PRO | B | 685 | 53.443 | 25.058 | 17.084 | 1.00 | 41.20 |
| 2063 | O | PRO | B | 685 | 52.741 | 25.801 | 17.780 | 1.00 | 37.15 |
| 2064 | CB | PRO | B | 685 | 55.095 | 24.082 | 18.738 | 1.00 | 44.89 |
| 2065 | CG | PRO | B | 685 | 56.412 | 24.638 | 19.230 | 1.00 | 47.57 |
| 2066 | CD | PRO | B | 685 | 56.116 | 26.115 | 19.062 | 1.00 | 46.29 |
| 2067 | N | PRO | B | 686 | 52.948 | 24.395 | 16.029 | 1.00 | 37.46 |
| 2068 | CA | PRO | B | 686 | 51.549 | 24.575 | 15.644 | 1.00 | 35.10 |
| 2069 | C | PRO | B | 686 | 50.533 | 24.458 | 16.783 | 1.00 | 29.47 |
| 2070 | O | PRO | B | 686 | 49.675 | 25.317 | 16.919 | 1.00 | 28.34 |
| 2071 | CB | PRO | B | 686 | 51.364 | 23.520 | 14.549 | 1.00 | 37.20 |
| 2072 | CG | PRO | B | 686 | 52.417 | 22.466 | 14.906 | 1.00 | 39.30 |
| 2073 | CD | PRO | B | 686 | 53.579 | 23.402 | 15.146 | 1.00 | 39.17 |
| 2074 | N | LEU | B | 687 | 50.641 | 23.423 | 17.607 | 1.00 | 27.64 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2075 | CA | LEU | B | 687 | 49.680 | 23.254 | 18.698 | 1.00 | 29.33 |
| 2076 | C | LEU | B | 687 | 49.656 | 24.470 | 19.623 | 1.00 | 32.07 |
| 2077 | O | LEU | B | 687 | 48.603 | 24.851 | 20.154 | 1.00 | 29.30 |
| 2078 | CB | LEU | B | 687 | 49.991 | 21.982 | 19.489 | 1.00 | 29.35 |
| 2079 | CG | LEU | B | 687 | 49.030 | 21.634 | 20.640 | 1.00 | 28.23 |
| 2080 | CD1 | LEU | B | 687 | 47.590 | 21.521 | 20.135 | 1.00 | 34.54 |
| 2081 | CD2 | LEU | B | 687 | 49.476 | 20.327 | 21.279 | 1.00 | 37.01 |
| 2082 | N | ILE | B | 688 | 50.814 | 25.094 | 19.810 | 1.00 | 29.32 |
| 2083 | CA | ILE | B | 688 | 50.893 | 26.268 | 20.659 | 1.00 | 29.95 |
| 2084 | C | ILE | B | 688 | 50.225 | 27.466 | 19.976 | 1.00 | 29.12 |
| 2085 | O | ILE | B | 688 | 49.544 | 28.260 | 20.626 | 1.00 | 31.19 |
| 2086 | CB | ILE | B | 688 | 52.365 | 26.573 | 21.029 | 1.00 | 25.50 |
| 2087 | CG1 | ILE | B | 688 | 52.945 | 25.393 | 21.826 | 1.00 | 35.36 |
| 2088 | CG2 | ILE | B | 688 | 52.450 | 27.851 | 21.831 | 1.00 | 30.73 |
| 2089 | CD1 | ILE | B | 688 | 54.354 | 25.613 | 22.354 | 1.00 | 30.80 |
| 2090 | N | ASN | B | 689 | 50.396 | 27.607 | 18.663 | 1.00 | 32.90 |
| 2091 | CA | ASN | B | 689 | 49.744 | 28.720 | 17.970 | 1.00 | 26.76 |
| 2092 | C | ASN | B | 689 | 48.246 | 28.520 | 18.037 | 1.00 | 27.49 |
| 2093 | O | ASN | B | 689 | 47.490 | 29.473 | 18.168 | 1.00 | 29.38 |
| 2094 | CB | ASN | B | 689 | 50.163 | 28.802 | 16.497 | 1.00 | 37.63 |
| 2095 | CG | ASN | B | 689 | 51.544 | 29.392 | 16.315 | 1.00 | 42.51 |
| 2096 | OD1 | ASN | B | 689 | 51.843 | 30.467 | 16.844 | 1.00 | 50.26 |
| 2097 | ND2 | ASN | B | 689 | 52.391 | 28.706 | 15.550 | 1.00 | 49.44 |
| 2098 | N | LEU | B | 690 | 47.816 | 27.270 | 17.938 | 1.00 | 26.48 |
| 2099 | CA | LEU | B | 690 | 46.389 | 26.992 | 17.998 | 1.00 | 30.44 |
| 2100 | C | LEU | B | 690 | 45.879 | 27.386 | 19.379 | 1.00 | 29.46 |
| 2101 | O | LEU | B | 690 | 44.849 | 28.034 | 19.487 | 1.00 | 25.83 |
| 2102 | CB | LEU | B | 690 | 46.110 | 25.518 | 17.737 | 1.00 | 33.45 |
| 2103 | CG | LEU | B | 690 | 44.641 | 25.073 | 17.699 | 1.00 | 36.64 |
| 2104 | CD1 | LEU | B | 690 | 43.832 | 25.943 | 16.752 | 1.00 | 48.71 |
| 2105 | CD2 | LEU | B | 690 | 44.591 | 23.624 | 17.254 | 1.00 | 48.02 |
| 2106 | N | LEU | B | 691 | 46.607 | 26.999 | 20.426 | 1.00 | 24.64 |
| 2107 | CA | LEU | B | 691 | 46.203 | 27.342 | 21.788 | 1.00 | 25.80 |
| 2108 | C | LEU | B | 691 | 46.058 | 28.845 | 21.954 | 1.00 | 24.20 |
| 2109 | O | LEU | B | 691 | 45.139 | 29.311 | 22.620 | 1.00 | 27.02 |
| 2110 | CB | LEU | B | 691 | 47.195 | 26.774 | 22.821 | 1.00 | 23.63 |
| 2111 | CG | LEU | B | 691 | 47.230 | 25.246 | 23.002 | 1.00 | 27.81 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2112 | CD1 | LEU | B | 691 | 48.278 | 24.847 | 24.069 | 1.00 | 22.95 |
| 2113 | CD2 | LEU | B | 691 | 45.838 | 24.749 | 23.443 | 1.00 | 24.81 |
| 2114 | N | MET | B | 692 | 46.942 | 29.626 | 21.337 | 1.00 | 27.01 |
| 2115 | CA | MET | B | 692 | 46.833 | 31.077 | 21.464 | 1.00 | 28.55 |
| 2116 | C | MET | B | 692 | 45.605 | 31.584 | 20.723 | 1.00 | 28.03 |
| 2117 | O | MET | B | 692 | 44.955 | 32.535 | 21.154 | 1.00 | 29.01 |
| 2118 | CB | MET | B | 692 | 48.086 | 31.780 | 20.922 | 1.00 | 34.30 |
| 2119 | CG | MET | B | 692 | 48.009 | 33.297 | 21.034 | 1.00 | 43.26 |
| 2120 | SD | MET | B | 692 | 47.662 | 33.855 | 22.730 | 1.00 | 60.38 |
| 2121 | CE | MET | B | 692 | 47.493 | 35.639 | 22.492 | 1.00 | 57.28 |
| 2122 | N | SER | B | 693 | 45.300 | 30.936 | 19.609 | 1.00 | 30.34 |
| 2123 | CA | SER | B | 693 | 44.153 | 31.288 | 18.782 | 1.00 | 33.29 |
| 2124 | C | SER | B | 693 | 42.802 | 31.154 | 19.478 | 1.00 | 31.49 |
| 2125 | O | SER | B | 693 | 41.906 | 31.971 | 19.269 | 1.00 | 31.71 |
| 2126 | CB | SER | B | 693 | 44.140 | 30.404 | 17.534 | 1.00 | 35.00 |
| 2127 | OG | SER | B | 693 | 42.924 | 30.567 | 16.829 | 1.00 | 53.17 |
| 2128 | N | ILE | B | 694 | 42.652 | 30.124 | 20.308 | 1.00 | 30.50 |
| 2129 | CA | ILE | B | 694 | 41.380 | 29.891 | 20.983 | 1.00 | 29.73 |
| 2130 | C | ILE | B | 694 | 41.282 | 30.457 | 22.389 | 1.00 | 29.02 |
| 2131 | O | ILE | B | 694 | 40.279 | 30.258 | 23.066 | 1.00 | 27.58 |
| 2132 | CB | ILE | B | 694 | 41.043 | 28.375 | 21.043 | 1.00 | 28.72 |
| 2133 | CG1 | ILE | B | 694 | 42.051 | 27.648 | 21.929 | 1.00 | 26.83 |
| 2134 | CG2 | ILE | B | 694 | 41.070 | 27.780 | 19.626 | 1.00 | 26.41 |
| 2135 | CD1 | ILE | B | 694 | 41.753 | 26.158 | 22.140 | 1.00 | 25.75 |
| 2136 | N | GLU | B | 695 | 42.318 | 31.160 | 22.832 | 1.00 | 28.38 |
| 2137 | CA | GLU | B | 695 | 42.300 | 31.744 | 24.166 | 1.00 | 34.30 |
| 2138 | C | GLU | B | 695 | 41.148 | 32.745 | 24.219 | 1.00 | 32.52 |
| 2139 | O | GLU | B | 695 | 40.968 | 33.533 | 23.302 | 1.00 | 40.33 |
| 2140 | CB | GLU | B | 695 | 43.609 | 32.484 | 24.445 | 1.00 | 34.44 |
| 2141 | CG | GLU | B | 695 | 43.717 | 32.959 | 25.870 | 1.00 | 44.19 |
| 2142 | CD | GLU | B | 695 | 44.179 | 31.868 | 26.819 | 1.00 | 46.10 |
| 2143 | OE1 | GLU | B | 695 | 43.742 | 30.701 | 26.682 | 1.00 | 34.62 |
| 2144 | OE2 | GLU | B | 695 | 44.983 | 32.196 | 27.718 | 1.00 | 55.08 |
| 2145 | N | PRO | B | 696 | 40.353 | 32.729 | 25.298 | 1.00 | 35.67 |
| 2146 | CA | PRO | B | 696 | 39.216 | 33.642 | 25.460 | 1.00 | 33.01 |
| 2147 | C | PRO | B | 696 | 39.590 | 35.124 | 25.452 | 1.00 | 38.74 |
| 2148 | O | PRO | B | 696 | 40.695 | 35.502 | 25.834 | 1.00 | 31.49 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2149 | CB | PRO | B | 696 | 38.633 | 33.216 | 26.808 | 1.00 | 33.87 |
| 2150 | CG | PRO | B | 696 | 38.988 | 31.725 | 26.849 | 1.00 | 42.63 |
| 2151 | CD | PRO | B | 696 | 40.449 | 31.833 | 26.461 | 1.00 | 33.91 |
| 2152 | N | ASP | B | 697 | 38.660 | 35.961 | 25.016 | 1.00 | 37.09 |
| 2153 | CA | ASP | B | 697 | 38.896 | 37.392 | 24.995 | 1.00 | 40.83 |
| 2154 | C | ASP | B | 697 | 38.697 | 37.865 | 26.445 | 1.00 | 41.22 |
| 2155 | O | ASP | B | 697 | 38.208 | 37.108 | 27.281 | 1.00 | 31.86 |
| 2156 | CB | ASP | B | 697 | 37.881 | 38.064 | 24.070 | 1.00 | 51.77 |
| 2157 | CG | ASP | B | 697 | 38.345 | 39.419 | 23.581 | 1.00 | 50.70 |
| 2158 | OD1 | ASP | B | 697 | 38.620 | 40.306 | 24.416 | 1.00 | 65.14 |
| 2159 | OD2 | ASP | B | 697 | 38.437 | 39.595 | 22.352 | 1.00 | 58.30 |
| 2160 | N | VAL | B | 698 | 39.086 | 39.099 | 26.752 | 1.00 | 40.92 |
| 2161 | CA | VAL | B | 698 | 38.925 | 39.600 | 28.115 | 1.00 | 39.77 |
| 2162 | C | VAL | B | 698 | 37.462 | 39.569 | 28.523 | 1.00 | 36.77 |
| 2163 | O | VAL | B | 698 | 36.567 | 39.817 | 27.710 | 1.00 | 39.48 |
| 2164 | CB | VAL | B | 698 | 39.463 | 41.044 | 28.271 | 1.00 | 47.15 |
| 2165 | CG1 | VAL | B | 698 | 40.986 | 41.049 | 28.157 | 1.00 | 51.98 |
| 2166 | CG2 | VAL | B | 698 | 38.851 | 41.948 | 27.206 | 1.00 | 44.01 |
| 2167 | N | ILE | B | 699 | 37.216 | 39.238 | 29.784 | 1.00 | 34.12 |
| 2168 | CA | ILE | B | 699 | 35.857 | 39.178 | 30.305 | 1.00 | 25.82 |
| 2169 | C | ILE | B | 699 | 35.728 | 40.202 | 31.421 | 1.00 | 30.49 |
| 2170 | O | ILE | B | 699 | 36.616 | 40.315 | 32.264 | 1.00 | 26.57 |
| 2171 | CB | ILE | B | 699 | 35.550 | 37.758 | 30.841 | 1.00 | 28.35 |
| 2172 | CG1 | ILE | B | 699 | 35.595 | 36.770 | 29.677 | 1.00 | 28.39 |
| 2173 | CG2 | ILE | B | 699 | 34.198 | 37.731 | 31.553 | 1.00 | 25.63 |
| 2174 | CD1 | ILE | B | 699 | 35.451 | 35.322 | 30.072 | 1.00 | 35.91 |
| 2175 | N | TYR | B | 700 | 34.631 | 40.956 | 31.408 | 1.00 | 29.91 |
| 2176 | CA | TYR | B | 700 | 34.369 | 41.975 | 32.422 | 1.00 | 32.17 |
| 2177 | C | TYR | B | 700 | 33.522 | 41.412 | 33.561 | 1.00 | 32.19 |
| 2178 | O | TYR | B | 700 | 32.717 | 40.511 | 33.357 | 1.00 | 33.61 |
| 2179 | CB | TYR | B | 700 | 33.629 | 43.152 | 31.793 | 1.00 | 37.36 |
| 2180 | CG | TYR | B | 700 | 34.433 | 43.900 | 30.760 | 1.00 | 44.51 |
| 2181 | CD1 | TYR | B | 700 | 35.387 | 44.848 | 31.134 | 1.00 | 48.38 |
| 2182 | CD2 | TYR | B | 700 | 34.267 | 43.635 | 29.402 | 1.00 | 49.71 |
| 2183 | CE1 | TYR | B | 700 | 36.154 | 45.515 | 30.170 | 1.00 | 52.90 |
| 2184 | CE2 | TYR | B | 700 | 35.025 | 44.290 | 28.438 | 1.00 | 52.02 |
| 2185 | CZ | TYR | B | 700 | 35.966 | 45.227 | 28.823 | 1.00 | 54.03 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2186 | OH | TYR | B | 700 | 36.721 | 45.862 | 27.854 | 1.00 | 59.25 |
| 2187 | N | ALA | B | 701 | 33.695 | 41.962 | 34.757 | 1.00 | 30.94 |
| 2188 | CA | ALA | B | 701 | 32.938 | 41.509 | 35.919 | 1.00 | 33.53 |
| 2189 | C | ALA | B | 701 | 31.571 | 42.184 | 36.006 | 1.00 | 36.38 |
| 2190 | O | ALA | B | 701 | 30.657 | 41.664 | 36.657 | 1.00 | 33.68 |
| 2191 | CB | ALA | B | 701 | 33.730 | 41.787 | 37.204 | 1.00 | 30.56 |
| 2192 | N | GLY | B | 702 | 31.435 | 43.333 | 35.344 | 1.00 | 34.92 |
| 2193 | CA | GLY | B | 702 | 30.192 | 44.082 | 35.396 | 1.00 | 36.02 |
| 2194 | C | GLY | B | 702 | 30.048 | 44.717 | 36.766 | 1.00 | 36.08 |
| 2195 | O | GLY | B | 702 | 28.944 | 44.994 | 37.224 | 1.00 | 35.67 |
| 2196 | N | HIS | B | 703 | 31.178 | 44.967 | 37.419 | 1.00 | 36.94 |
| 2197 | CA | HIS | B | 703 | 31.181 | 45.536 | 38.759 | 1.00 | 39.32 |
| 2198 | C | HIS | B | 703 | 30.955 | 47.049 | 38.842 | 1.00 | 43.22 |
| 2199 | O | HIS | B | 703 | 31.440 | 47.806 | 38.010 | 1.00 | 37.01 |
| 2200 | CB | HIS | B | 703 | 32.486 | 45.158 | 39.460 | 1.00 | 38.71 |
| 2201 | CG | HIS | B | 703 | 32.561 | 45.614 | 40.881 | 1.00 | 36.27 |
| 2202 | ND1 | HIS | B | 703 | 32.950 | 46.888 | 41.232 | 1.00 | 43.46 |
| 2203 | CD2 | HIS | B | 703 | 32.243 | 44.985 | 42.035 | 1.00 | 36.91 |
| 2204 | CE1 | HIS | B | 703 | 32.870 | 47.024 | 42.543 | 1.00 | 38.85 |
| 2205 | NE2 | HIS | B | 703 | 32.442 | 45.882 | 43.054 | 1.00 | 41.40 |
| 2206 | N | ASP | B | 704 | 30.221 | 47.471 | 39.871 | 1.00 | 46.40 |
| 2207 | CA | ASP | B | 704 | 29.898 | 48.880 | 40.097 | 1.00 | 53.26 |
| 2208 | C | ASP | B | 704 | 31.066 | 49.860 | 39.983 | 1.00 | 53.35 |
| 2209 | O | ASP | B | 704 | 31.280 | 50.462 | 38.932 | 1.00 | 58.50 |
| 2210 | CB | ASP | B | 704 | 29.229 | 49.051 | 41.470 | 1.00 | 61.65 |
| 2211 | CG | ASP | B | 704 | 30.001 | 48.370 | 42.592 | 1.00 | 66.85 |
| 2212 | OD1 | ASP | B | 704 | 30.031 | 47.119 | 42.619 | 1.00 | 72.50 |
| 2213 | OD2 | ASP | B | 704 | 30.580 | 49.082 | 43.441 | 1.00 | 69.65 |
| 2214 | N | ASN | B | 705 | 31.809 | 50.011 | 41.074 | 1.00 | 55.13 |
| 2215 | CA | ASN | B | 705 | 32.951 | 50.922 | 41.157 | 1.00 | 50.42 |
| 2216 | C | ASN | B | 705 | 32.525 | 52.345 | 41.457 | 1.00 | 54.10 |
| 2217 | O | ASN | B | 705 | 33.359 | 53.174 | 41.812 | 1.00 | 52.31 |
| 2218 | CB | ASN | B | 705 | 33.805 | 50.915 | 39.882 | 1.00 | 52.40 |
| 2219 | CG | ASN | B | 705 | 34.614 | 49.646 | 39.729 | 1.00 | 47.64 |
| 2220 | OD1 | ASN | B | 705 | 35.296 | 49.218 | 40.661 | 1.00 | 49.26 |
| 2221 | ND2 | ASN | B | 705 | 34.555 | 49.043 | 38.546 | 1.00 | 47.16 |
| 2222 | N | THR | B | 706 | 31.236 | 52.637 | 41.308 | 1.00 | 52.08 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2223 | CA | THR | B | 706 | 30.749 | 53.976 | 41.618 | 1.00 | 55.23 |
| 2224 | C | THR | B | 706 | 30.386 | 53.941 | 43.101 | 1.00 | 55.70 |
| 2225 | O | THR | B | 706 | 30.307 | 54.975 | 43.765 | 1.00 | 59.81 |
| 2226 | CB | THR | B | 706 | 29.487 | 54.348 | 40.815 | 1.00 | 52.39 |
| 2227 | OG1 | THR | B | 706 | 28.365 | 53.616 | 41.319 | 1.00 | 50.26 |
| 2228 | CG2 | THR | B | 706 | 29.683 | 54.026 | 39.332 | 1.00 | 55.79 |
| 2229 | N | LYS | B | 707 | 30.151 | 52.731 | 43.604 | 1.00 | 55.87 |
| 2230 | CA | LYS | B | 707 | 29.821 | 52.525 | 45.009 | 1.00 | 54.80 |
| 2231 | C | LYS | B | 707 | 31.003 | 51.934 | 45.758 | 1.00 | 53.32 |
| 2232 | O | LYS | B | 707 | 31.826 | 51.235 | 45.168 | 1.00 | 54.94 |
| 2233 | CB | LYS | B | 707 | 28.633 | 51.577 | 45.159 | 1.00 | 53.68 |
| 2234 | CG | LYS | B | 707 | 27.293 | 52.225 | 44.945 | 1.00 | 55.52 |
| 2235 | CD | LYS | B | 707 | 26.235 | 51.395 | 45.645 | 1.00 | 56.98 |
| 2236 | CE | LYS | B | 707 | 25.006 | 52.230 | 45.965 | 1.00 | 57.78 |
| 2237 | NZ | LYS | B | 707 | 24.065 | 51.506 | 46.869 | 1.00 | 59.46 |
| 2238 | N | PRO | B | 708 | 31.107 | 52.214 | 47.067 | 1.00 | 50.91 |
| 2239 | CA | PRO | B | 708 | 32.195 | 51.699 | 47.906 | 1.00 | 49.84 |
| 2240 | C | PRO | B | 708 | 32.080 | 50.185 | 48.100 | 1.00 | 46.87 |
| 2241 | O | PRO | B | 708 | 30.975 | 49.653 | 48.229 | 1.00 | 48.38 |
| 2242 | CB | PRO | B | 708 | 31.995 | 52.459 | 49.219 | 1.00 | 52.26 |
| 2243 | CG | PRO | B | 708 | 31.253 | 53.731 | 48.769 | 1.00 | 52.21 |
| 2244 | CD | PRO | B | 708 | 30.238 | 53.087 | 47.870 | 1.00 | 51.56 |
| 2245 | N | ASP | B | 709 | 33.219 | 49.498 | 48.123 | 1.00 | 46.70 |
| 2246 | CA | ASP | B | 709 | 33.248 | 48.044 | 48.306 | 1.00 | 41.74 |
| 2247 | C | ASP | B | 709 | 32.628 | 47.602 | 49.618 | 1.00 | 41.06 |
| 2248 | O | ASP | B | 709 | 32.739 | 48.285 | 50.628 | 1.00 | 36.26 |
| 2249 | CB | ASP | B | 709 | 34.684 | 47.505 | 48.321 | 1.00 | 45.78 |
| 2250 | CG | ASP | B | 709 | 35.362 | 47.559 | 46.974 | 1.00 | 49.80 |
| 2251 | OD1 | ASP | B | 709 | 34.697 | 47.848 | 45.957 | 1.00 | 50.41 |
| 2252 | OD2 | ASP | B | 709 | 36.582 | 47.291 | 46.946 | 1.00 | 47.34 |
| 2253 | N | THR | B | 710 | 31.984 | 46.441 | 49.588 | 1.00 | 38.12 |
| 2254 | CA | THR | B | 710 | 31.413 | 45.831 | 50.780 | 1.00 | 38.56 |
| 2255 | C | THR | B | 710 | 31.761 | 44.366 | 50.535 | 1.00 | 40.19 |
| 2256 | O | THR | B | 710 | 31.870 | 43.941 | 49.383 | 1.00 | 34.38 |
| 2257 | CB | THR | B | 710 | 29.874 | 45.995 | 50.881 | 1.00 | 41.95 |
| 2258 | OG1 | THR | B | 710 | 29.228 | 45.193 | 49.887 | 1.00 | 44.34 |
| 2259 | CG2 | THR | B | 710 | 29.481 | 47.454 | 50.679 | 1.00 | 48.02 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2260 | N | SER | B | 711 | 31.971 | 43.605 | 51.598 | 1.00 | 36.89 |
| 2261 | CA | SER | B | 711 | 32.320 | 42.200 | 51.449 | 1.00 | 43.49 |
| 2262 | C | SER | B | 711 | 31.361 | 41.476 | 50.522 | 1.00 | 41.27 |
| 2263 | O | SER | B | 711 | 31.780 | 40.718 | 49.647 | 1.00 | 43.17 |
| 2264 | CB | SER | B | 711 | 32.304 | 41.500 | 52.807 | 1.00 | 40.05 |
| 2265 | OG | SER | B | 711 | 32.541 | 40.115 | 52.642 | 1.00 | 53.25 |
| 2266 | N | SER | B | 712 | 30.069 | 41.717 | 50.709 | 1.00 | 40.73 |
| 2267 | CA | SER | B | 712 | 29.069 | 41.041 | 49.897 | 1.00 | 38.79 |
| 2268 | C | SER | B | 712 | 29.018 | 41.457 | 48.434 | 1.00 | 37.75 |
| 2269 | O | SER | B | 712 | 28.812 | 40.601 | 47.578 | 1.00 | 32.93 |
| 2270 | CB | SER | B | 712 | 27.680 | 41.185 | 50.528 | 1.00 | 40.76 |
| 2271 | OG | SER | B | 712 | 27.288 | 42.541 | 50.611 | 1.00 | 52.08 |
| 2272 | N | SER | B | 713 | 29.207 | 42.744 | 48.136 | 1.00 | 31.42 |
| 2273 | CA | SER | B | 713 | 29.151 | 43.196 | 46.744 | 1.00 | 30.39 |
| 2274 | C | SER | B | 713 | 30.384 | 42.769 | 45.962 | 1.00 | 26.79 |
| 2275 | O | SER | B | 713 | 30.287 | 42.444 | 44.787 | 1.00 | 27.38 |
| 2276 | CB | SER | B | 713 | 28.991 | 44.725 | 46.651 | 1.00 | 32.46 |
| 2277 | OG | SER | B | 713 | 30.115 | 45.404 | 47.190 | 1.00 | 37.41 |
| 2278 | N | LEU | B | 714 | 31.539 | 42.777 | 46.620 | 1.00 | 26.90 |
| 2279 | CA | LEU | B | 714 | 32.796 | 42.386 | 45.989 | 1.00 | 23.45 |
| 2280 | C | LEU | B | 714 | 32.815 | 40.878 | 45.691 | 1.00 | 25.66 |
| 2281 | O | LEU | B | 714 | 33.186 | 40.465 | 44.592 | 1.00 | 24.25 |
| 2282 | CB | LEU | B | 714 | 33.959 | 42.750 | 46.908 | 1.00 | 30.39 |
| 2283 | CG | LEU | B | 714 | 35.393 | 42.649 | 46.411 | 1.00 | 38.20 |
| 2284 | CD1 | LEU | B | 714 | 35.557 | 43.398 | 45.091 | 1.00 | 41.76 |
| 2285 | CD2 | LEU | B | 714 | 36.309 | 43.239 | 47.491 | 1.00 | 35.78 |
| 2286 | N | LEU | B | 715 | 32.436 | 40.055 | 46.666 | 1.00 | 20.32 |
| 2287 | CA | LEU | B | 715 | 32.409 | 38.607 | 46.435 | 1.00 | 24.51 |
| 2288 | C | LEU | B | 715 | 31.303 | 38.257 | 45.423 | 1.00 | 23.41 |
| 2289 | O | LEU | B | 715 | 31.449 | 37.350 | 44.615 | 1.00 | 19.77 |
| 2290 | CB | LEU | B | 715 | 32.219 | 37.855 | 47.757 | 1.00 | 23.31 |
| 2291 | CG | LEU | B | 715 | 33.439 | 37.895 | 48.691 | 1.00 | 25.25 |
| 2292 | CD1 | LEU | B | 715 | 33.075 | 37.397 | 50.065 | 1.00 | 26.50 |
| 2293 | CD2 | LEU | B | 715 | 34.583 | 37.067 | 48.089 | 1.00 | 22.19 |
| 2294 | N | THR | B | 716 | 30.204 | 38.998 | 45.447 | 1.00 | 22.64 |
| 2295 | CA | THR | B | 716 | 29.123 | 38.728 | 44.496 | 1.00 | 23.68 |
| 2296 | C | THR | B | 716 | 29.605 | 39.054 | 43.073 | 1.00 | 22.06 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2297 | O | THR | B | 716 | 29.319 | 38.314 | 42.134 | 1.00 | 22.59 |
| 2298 | CB | THR | B | 716 | 27.839 | 39.535 | 44.901 | 1.00 | 20.94 |
| 2299 | OG1 | THR | B | 716 | 27.329 | 39.003 | 46.134 | 1.00 | 25.72 |
| 2300 | CG2 | THR | B | 716 | 26.765 | 39.459 | 43.820 | 1.00 | 28.64 |
| 2301 | N | SER | B | 717 | 30.358 | 40.151 | 42.908 | 1.00 | 23.37 |
| 2302 | CA | SER | B | 717 | 30.884 | 40.496 | 41.596 | 1.00 | 25.88 |
| 2303 | C | SER | B | 717 | 31.927 | 39.474 | 41.153 | 1.00 | 21.32 |
| 2304 | O | SER | B | 717 | 32.008 | 39.144 | 39.976 | 1.00 | 21.40 |
| 2305 | CB | SER | B | 717 | 31.527 | 41.897 | 41.581 | 1.00 | 27.55 |
| 2306 | OG | SER | B | 717 | 30.537 | 42.909 | 41.675 | 1.00 | 37.69 |
| 2307 | N | LEU | B | 718 | 32.738 | 38.991 | 42.083 | 1.00 | 19.85 |
| 2308 | CA | LEU | B | 718 | 33.732 | 37.996 | 41.714 | 1.00 | 21.19 |
| 2309 | C | LEU | B | 718 | 33.004 | 36.746 | 41.234 | 1.00 | 21.11 |
| 2310 | O | LEU | B | 718 | 33.440 | 36.099 | 40.286 | 1.00 | 21.04 |
| 2311 | CB | LEU | B | 718 | 34.661 | 37.675 | 42.889 | 1.00 | 22.44 |
| 2312 | CG | LEU | B | 718 | 35.709 | 38.750 | 43.211 | 1.00 | 24.98 |
| 2313 | CD1 | LEU | B | 718 | 36.442 | 38.413 | 44.499 | 1.00 | 24.73 |
| 2314 | CD2 | LEU | B | 718 | 36.702 | 38.848 | 42.067 | 1.00 | 24.25 |
| 2315 | N | ASN | B | 719 | 31.887 | 36.407 | 41.879 | 1.00 | 22.37 |
| 2316 | CA | ASN | B | 719 | 31.129 | 35.242 | 41.445 | 1.00 | 17.98 |
| 2317 | C | ASN | B | 719 | 30.495 | 35.418 | 40.073 | 1.00 | 18.99 |
| 2318 | O | ASN | B | 719 | 30.393 | 34.461 | 39.328 | 1.00 | 20.78 |
| 2319 | CB | ASN | B | 719 | 30.032 | 34.862 | 42.446 | 1.00 | 18.41 |
| 2320 | CG | ASN | B | 719 | 30.586 | 34.171 | 43.658 | 1.00 | 24.20 |
| 2321 | OD1 | ASN | B | 719 | 31.629 | 33.518 | 43.574 | 1.00 | 28.51 |
| 2322 | ND2 | ASN | B | 719 | 29.885 | 34.262 | 44.778 | 1.00 | 22.40 |
| 2323 | N | GLN | B | 720 | 30.035 | 36.628 | 39.759 | 1.00 | 22.71 |
| 2324 | CA | GLN | B | 720 | 29.445 | 36.900 | 38.463 | 1.00 | 23.51 |
| 2325 | C | GLN | B | 720 | 30.557 | 36.762 | 37.417 | 1.00 | 23.71 |
| 2326 | O | GLN | B | 720 | 30.354 | 36.212 | 36.338 | 1.00 | 24.20 |
| 2327 | CB | GLN | B | 720 | 28.839 | 38.309 | 38.448 | 1.00 | 22.72 |
| 2328 | CG | GLN | B | 720 | 28.243 | 38.715 | 37.116 | 1.00 | 29.98 |
| 2329 | CD | GLN | B | 720 | 27.325 | 39.924 | 37.231 | 1.00 | 35.29 |
| 2330 | OE1 | GLN | B | 720 | 26.217 | 39.831 | 37.754 | 1.00 | 33.28 |
| 2331 | NE2 | GLN | B | 720 | 27.797 | 41.071 | 36.759 | 1.00 | 37.20 |
| 2332 | N | LEU | B | 721 | 31.744 | 37.249 | 37.764 | 1.00 | 22.80 |
| 2333 | CA | LEU | B | 721 | 32.891 | 37.143 | 36.874 | 1.00 | 18.97 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2334 | C | LEU | B | 721 | 33.197 | 35.652 | 36.683 | 1.00 | 23.82 |
| 2335 | O | LEU | B | 721 | 33.417 | 35.172 | 35.566 | 1.00 | 22.42 |
| 2336 | CB | LEU | B | 721 | 34.096 | 37.866 | 37.498 | 1.00 | 21.95 |
| 2337 | CG | LEU | B | 721 | 35.387 | 37.769 | 36.696 | 1.00 | 20.86 |
| 2338 | CD1 | LEU | B | 721 | 35.162 | 38.452 | 35.346 | 1.00 | 22.61 |
| 2339 | CD2 | LEU | B | 721 | 36.537 | 38.433 | 37.453 | 1.00 | 19.99 |
| 2340 | N | GLY | B | 722 | 33.219 | 34.925 | 37.793 | 1.00 | 18.60 |
| 2341 | CA | GLY | B | 722 | 33.464 | 33.495 | 37.739 | 1.00 | 21.51 |
| 2342 | C | GLY | B | 722 | 32.497 | 32.793 | 36.807 | 1.00 | 22.85 |
| 2343 | O | GLY | B | 722 | 32.888 | 31.928 | 36.019 | 1.00 | 21.10 |
| 2344 | N | GLU | B | 723 | 31.225 | 33.174 | 36.889 | 1.00 | 20.92 |
| 2345 | CA | GLU | B | 723 | 30.187 | 32.582 | 36.044 | 1.00 | 22.44 |
| 2346 | C | GLU | B | 723 | 30.514 | 32.814 | 34.570 | 1.00 | 22.88 |
| 2347 | O | GLU | B | 723 | 30.448 | 31.895 | 33.766 | 1.00 | 22.34 |
| 2348 | CB | GLU | B | 723 | 28.830 | 33.219 | 36.370 | 1.00 | 28.49 |
| 2349 | CG | GLU | B | 723 | 27.635 | 32.646 | 35.614 | 1.00 | 29.16 |
| 2350 | CD | GLU | B | 723 | 27.225 | 31.269 | 36.108 | 1.00 | 48.36 |
| 2351 | OE1 | GLU | B | 723 | 28.005 | 30.666 | 36.876 | 1.00 | 43.10 |
| 2352 | OE2 | GLU | B | 723 | 26.126 | 30.784 | 35.726 | 1.00 | 39.94 |
| 2353 | N | ARG | B | 724 | 30.858 | 34.054 | 34.225 | 1.00 | 20.90 |
| 2354 | CA | ARG | B | 724 | 31.185 | 34.389 | 32.846 | 1.00 | 24.02 |
| 2355 | C | ARG | B | 724 | 32.431 | 33.652 | 32.389 | 1.00 | 24.96 |
| 2356 | O | ARG | B | 724 | 32.512 | 33.179 | 31.260 | 1.00 | 21.65 |
| 2357 | CB | ARG | B | 724 | 31.417 | 35.884 | 32.712 | 1.00 | 27.26 |
| 2358 | CG | ARG | B | 724 | 30.227 | 36.735 | 33.040 | 1.00 | 26.22 |
| 2359 | CD | ARG | B | 724 | 30.661 | 38.187 | 33.084 | 1.00 | 29.11 |
| 2360 | NE | ARG | B | 724 | 29.636 | 39.091 | 33.592 | 1.00 | 39.88 |
| 2361 | CZ | ARG | B | 724 | 28.566 | 39.475 | 32.905 | 1.00 | 47.37 |
| 2362 | NH1 | ARG | B | 724 | 28.370 | 39.029 | 31.668 | 1.00 | 54.23 |
| 2363 | NH2 | ARG | B | 724 | 27.708 | 40.329 | 33.445 | 1.00 | 44.85 |
| 2364 | N | GLN | B | 725 | 33.419 | 33.573 | 33.267 | 1.00 | 20.98 |
| 2365 | CA | GLN | B | 725 | 34.631 | 32.866 | 32.910 | 1.00 | 24.59 |
| 2366 | C | GLN | B | 725 | 34.372 | 31.378 | 32.750 | 1.00 | 26.24 |
| 2367 | O | GLN | B | 725 | 35.002 | 30.735 | 31.912 | 1.00 | 23.92 |
| 2368 | CB | GLN | B | 725 | 35.712 | 33.097 | 33.958 | 1.00 | 22.57 |
| 2369 | CG | GLN | B | 725 | 36.411 | 34.427 | 33.771 | 1.00 | 29.09 |
| 2370 | CD | GLN | B | 725 | 37.471 | 34.675 | 34.813 | 1.00 | 34.68 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2371 | OE1 | GLN | B | 725 | 38.060 | 33.730 | 35.349 | 1.00 | 34.94 |
| 2372 | NE2 | GLN | B | 725 | 37.749 | 35.944 | 35.089 | 1.00 | 31.50 |
| 2373 | N | LEU | B | 726 | 33.452 | 30.834 | 33.544 | 1.00 | 22.27 |
| 2374 | CA | LEU | B | 726 | 33.141 | 29.411 | 33.450 | 1.00 | 22.12 |
| 2375 | C | LEU | B | 726 | 32.524 | 29.133 | 32.077 | 1.00 | 22.14 |
| 2376 | O | LEU | B | 726 | 32.838 | 28.124 | 31.433 | 1.00 | 19.87 |
| 2377 | CB | LEU | B | 726 | 32.197 | 28.991 | 34.585 | 1.00 | 21.36 |
| 2378 | CG | LEU | B | 726 | 31.870 | 27.503 | 34.717 | 1.00 | 28.83 |
| 2379 | CD1 | LEU | B | 726 | 33.166 | 26.696 | 34.750 | 1.00 | 26.29 |
| 2380 | CD2 | LEU | B | 726 | 31.042 | 27.265 | 35.999 | 1.00 | 27.99 |
| 2381 | N | LEU | B | 727 | 31.652 | 30.023 | 31.619 | 1.00 | 21.02 |
| 2382 | CA | LEU | B | 727 | 31.059 | 29.837 | 30.301 | 1.00 | 25.96 |
| 2383 | C | LEU | B | 727 | 32.150 | 29.841 | 29.230 | 1.00 | 25.51 |
| 2384 | O | LEU | B | 727 | 32.080 | 29.064 | 28.266 | 1.00 | 20.92 |
| 2385 | CB | LEU | B | 727 | 30.055 | 30.948 | 29.985 | 1.00 | 29.25 |
| 2386 | CG | LEU | B | 727 | 28.749 | 30.917 | 30.766 | 1.00 | 39.65 |
| 2387 | CD1 | LEU | B | 727 | 27.887 | 32.091 | 30.358 | 1.00 | 38.17 |
| 2388 | CD2 | LEU | B | 727 | 28.028 | 29.603 | 30.492 | 1.00 | 38.71 |
| 2389 | N | SER | B | 728 | 33.159 | 30.701 | 29.407 | 1.00 | 22.52 |
| 2390 | CA | SER | B | 728 | 34.247 | 30.790 | 28.442 | 1.00 | 25.41 |
| 2391 | C | SER | B | 728 | 35.132 | 29.550 | 28.475 | 1.00 | 22.52 |
| 2392 | O | SER | B | 728 | 35.628 | 29.129 | 27.438 | 1.00 | 19.75 |
| 2393 | CB | SER | B | 728 | 35.088 | 32.069 | 28.644 | 1.00 | 28.09 |
| 2394 | OG | SER | B | 728 | 35.801 | 32.055 | 29.860 | 1.00 | 44.44 |
| 2395 | N | VAL | B | 729 | 35.337 | 28.982 | 29.665 | 1.00 | 20.70 |
| 2396 | CA | VAL | B | 729 | 36.107 | 27.739 | 29.814 | 1.00 | 20.20 |
| 2397 | C | VAL | B | 729 | 35.413 | 26.610 | 29.050 | 1.00 | 16.88 |
| 2398 | O | VAL | B | 729 | 36.066 | 25.812 | 28.386 | 1.00 | 19.20 |
| 2399 | CB | VAL | B | 729 | 36.215 | 27.328 | 31.297 | 1.00 | 20.97 |
| 2400 | CG1 | VAL | B | 729 | 36.711 | 25.900 | 31.440 | 1.00 | 22.68 |
| 2401 | CG2 | VAL | B | 729 | 37.177 | 28.284 | 32.010 | 1.00 | 26.21 |
| 2402 | N | VAL | B | 730 | 34.092 | 26.523 | 29.154 | 1.00 | 18.77 |
| 2403 | CA | VAL | B | 730 | 33.400 | 25.461 | 28.434 | 1.00 | 20.28 |
| 2404 | C | VAL | B | 730 | 33.605 | 25.648 | 26.931 | 1.00 | 18.42 |
| 2405 | O | VAL | B | 730 | 33.973 | 24.706 | 26.239 | 1.00 | 21.52 |
| 2406 | CB | VAL | B | 730 | 31.901 | 25.420 | 28.772 | 1.00 | 21.49 |
| 2407 | CG1 | VAL | B | 730 | 31.228 | 24.300 | 27.947 | 1.00 | 25.40 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2408 | CG2 | VAL | B | 730 | 31.721 | 25.149 | 30.252 | 1.00 | 24.07 |
| 2409 | N | LYS | B | 731 | 33.397 | 26.870 | 26.442 | 1.00 | 22.29 |
| 2410 | CA | LYS | B | 731 | 33.591 | 27.195 | 25.024 | 1.00 | 22.57 |
| 2411 1 | C | LYS | B | 731 | 35.017 | 26.824 | 24.589 | 1.00 | 22.82 |
| 2412 | O | LYS | B | 731 | 35.231 | 26.168 | 23.567 | 1.00 | 20.83 |
| 2413 | CB | LYS | B | 731 | 33.358 | 28.695 | 24.816 | 1.00 | 27.41 |
| 2414 | CG | LYS | B | 731 | 33.541 | 29.215 | 23.377 | 1.00 | 33.69 |
| 2415 | CD | LYS | B | 731 | 32.513 | 28.628 | 22.415 | 1.00 | 42.24 |
| 2416 | CE | LYS | B | 731 | 32.628 | 29.239 | 21.010 | 1.00 | 43.85 |
| 2417 | NZ | LYS | B | 731 | 33.961 | 29.015 | 20.381 | 1.00 | 43.69 |
| 2418 | N | TRP | B | 732 | 35.999 | 27.266 | 25.372 | 1.00 | 19.99 |
| 2419 | CA | TRP | B | 732 | 37.401 | 26.981 | 25.106 | 1.00 | 20.03 |
| 2420 | C | TRP | B | 732 | 37.668 | 25.471 | 25.013 | 1.00 | 22.42 |
| 2421 | O | TRP | B | 732 | 38.343 | 25.003 | 24.098 | 1.00 | 21.10 |
| 2422 | CB | TRP | B | 732 | 38.242 | 27.603 | 26.233 | 1.00 | 21.24 |
| 2423 | CG | TRP | B | 732 | 39.702 | 27.242 | 26.283 | 1.00 | 23.70 |
| 2424 | CD1 | TRP | B | 732 | 40.696 | 27.670 | 25.444 | 1.00 | 24.42 |
| 2425 | CD2 | TRP | B | 732 | 40.336 | 26.404 | 27.260 | 1.00 | 25.32 |
| 2426 | NE1 | TRP | B | 732 | 41.906 | 27.154 | 25.843 | 1.00 | 24.62 |
| 2427 | CE2 | TRP | B | 732 | 41.715 | 26.373 | 26.954 | 1.00 | 23.58 |
| 2428 | CE3 | TRP | B | 732 | 39.869 | 25.677 | 28.368 | 1.00 | 23.75 |
| 2429 | CZ2 | TRP | B | 732 | 42.640 | 25.640 | 27.716 | 1.00 | 21.37 |
| 2430 | CZ3 | TRP | B | 732 | 40.794 | 24.946 | 29.131 | 1.00 | 25.72 |
| 2431 | CH2 | TRP | B | 732 | 42.160 | 24.935 | 28.797 | 1.00 | 25.44 |
| 2432 | N | SER | B | 733 | 37.122 | 24.710 | 25.953 | 1.00 | 20.29 |
| 2433 | CA | SER | B | 733 | 37.354 | 23.263 | 25.985 | 1.00 | 17.86 |
| 2434 | C | SER | B | 733 | 36.817 | 22.570 | 24.725 | 1.00 | 17.55 |
| 2435 | O | SER | B | 733 | 37.352 | 21.539 | 24.298 | 1.00 | 19.01 |
| 2436 | CB | SER | B | 733 | 36.718 | 22.637 | 27.246 | 1.00 | 21.68 |
| 2437 | OG | SER | B | 733 | 35.305 | 22.650 | 27.205 | 1.00 | 23.79 |
| 2438 | N | LYS | B | 734 | 35.777 | 23.160 | 24.133 | 1.00 | 21.12 |
| 2439 | CA | LYS | B | 734 | 35.179 | 22.608 | 22.917 | 1.00 | 19.68 |
| 2440 | C | LYS | B | 734 | 36.090 | 22.818 | 21.712 | 1.00 | 20.73 |
| 2441 | O | LYS | B | 734 | 35.969 | 22.107 | 20.715 | 1.00 | 22.12 |
| 2442 | CB | LYS | B | 734 | 33.825 | 23.256 | 22.623 | 1.00 | 24.55 |
| 2443 | CG | LYS | B | 734 | 32.716 | 22.963 | 23.615 | 1.00 | 32.49 |
| 2444 | CD | LYS | B | 734 | 31.394 | 23.451 | 23.040 | 1.00 | 42.26 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2445 | CE | LYS | B | 734 | 30.205 | 23.137 | 23.936 | 1.00 | 40.61 |
| 2446 | NZ | LYS | B | 734 | 30.231 | 23.960 | 25.168 | 1.00 | 48.42 |
| 2447 | N | SER | B | 735 | 37.000 | 23.788 | 21.806 | 1.00 | 19.46 |
| 2448 | CA | SER | B | 735 | 37.937 | 24.086 | 20.721 | 1.00 | 23.70 |
| 2449 | C | SER | B | 735 | 39.327 | 23.512 | 21.008 | 1.00 | 22.09 |
| 2450 | O | SER | B | 735 | 40.174 | 23.495 | 20.142 | 1.00 | 24.41 |
| 2451 | CB | SER | B | 735 | 38.086 | 25.597 | 20.521 | 1.00 | 27.76 |
| 2452 | OG | SER | B | 735 | 36.848 | 26.243 | 20.279 | 1.00 | 31.96 |
| 2453 | N | LEU | B | 736 | 39.535 | 23.048 | 22.232 | 1.00 | 23.63 |
| 2454 | CA | LEU | B | 736 | 40.817 | 22.494 | 22.665 | 1.00 | 20.47 |
| 2455 | C | LEU | B | 736 | 41.113 | 21.140 | 22.021 | 1.00 | 21.67 |
| 2456 | O | LEU | B | 736 | 40.393 | 20.170 | 22.231 | 1.00 | 22.30 |
| 2457 | CB | LEU | B | 736 | 40.801 | 22.353 | 24.184 | 1.00 | 22.13 |
| 2458 | CG | LEU | B | 736 | 42.046 | 21.828 | 24.877 | 1.00 | 25.58 |
| 2459 | CD1 | LEU | B | 736 | 43.226 | 22.778 | 24.641 | 1.00 | 24.77 |
| 2460 | CD2 | LEU | B | 736 | 41.735 | 21.702 | 26.376 | 1.00 | 24.96 |
| 2461 | N | PRO | B | 737 | 42.192 | 21.058 | 21.227 | 1.00 | 24.15 |
| 2462 | CA | PRO | B | 737 | 42.559 | 19.806 | 20.561 | 1.00 | 27.85 |
| 2463 | C | PRO | B | 737 | 42.607 | 18.605 | 21.497 | 1.00 | 29.75 |
| 2464 | O | PRO | B | 737 | 43.323 | 18.596 | 22.514 | 1.00 | 25.17 |
| 2465 | CB | PRO | B | 737 | 43.923 | 20.136 | 19.967 | 1.00 | 31.60 |
| 2466 | CG | PRO | B | 737 | 43.757 | 21.565 | 19.610 | 1.00 | 24.35 |
| 2467 | CD | PRO | B | 737 | 43.177 | 22.102 | 20.900 | 1.00 | 27.34 |
| 2468 | N | GLY | B | 738 | 41.809 | 17.605 | 21.149 | 1.00 | 25.39 |
| 2469 | CA | GLY | B | 738 | 41.747 | 16.381 | 21.916 | 1.00 | 26.62 |
| 2470 | C | GLY | B | 738 | 40.624 | 16.272 | 22.931 | 1.00 | 23.45 |
| 2471 | O | GLY | B | 738 | 40.090 | 15.194 | 23.141 | 1.00 | 26.46 |
| 2472 | N | PHE | B | 739 | 40.229 | 17.383 | 23.539 | 1.00 | 24.87 |
| 2473 | CA | PHE | B | 739 | 39.218 | 17.335 | 24.585 | 1.00 | 19.47 |
| 2474 | C | PHE | B | 739 | 37.860 | 16.782 | 24.178 | 1.00 | 25.30 |
| 2475 | O | PHE | B | 739 | 37.301 | 15.944 | 24.874 | 1.00 | 21.67 |
| 2476 | CB | PHE | B | 739 | 39.015 | 18.714 | 25.175 | 1.00 | 17.19 |
| 2477 | CG | PHE | B | 739 | 38.439 | 18.692 | 26.559 | 1.00 | 23.41 |
| 2478 | CD1 | PHE | B | 739 | 39.182 | 18.176 | 27.617 | 1.00 | 28.09 |
| 2479 | CD2 | PHE | B | 739 | 37.178 | 19.218 | 26.814 | 1.00 | 25.22 |
| 2480 | CE1 | PHE | B | 739 | 38.674 | 18.200 | 28.907 | 1.00 | 26.53 |
| 2481 | CE2 | PHE | B | 739 | 36.662 | 19.245 | 28.109 | 1.00 | 23.43 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2482 | CZ | PHE | B | 739 | 37.413 | 18.738 | 29.152 | 1.00 | 27.21 |
| 2483 | N | ARG | B | 740 | 37.329 | 17.252 | 23.055 | 1.00 | 23.16 |
| 2484 | CA | ARG | B | 740 | 36.008 | 16.801 | 22.599 | 1.00 | 24.65 |
| 2485 | C | ARG | B | 740 | 35.942 | 15.298 | 22.331 | 1.00 | 26.61 |
| 2486 | O | ARG | B | 740 | 34.854 | 14.739 | 22.213 | 1.00 | 32.12 |
| 2487 | CB | ARG | B | 740 | 35.607 | 17.534 | 21.318 | 1.00 | 22.44 |
| 2488 | CG | ARG | B | 740 | 36.578 | 17.308 | 20.173 | 1.00 | 25.67 |
| 2489 | CD | ARG | B | 740 | 36.001 | 17.852 | 18.873 | 1.00 | 25.78 |
| 2490 | NE | ARG | B | 740 | 36.906 | 17.610 | 17.757 | 1.00 | 22.52 |
| 2491 | CZ | ARG | B | 740 | 36.605 | 17.907 | 16.497 | 1.00 | 27.51 |
| 2492 | NH1 | ARG | B | 740 | 35.428 | 18.444 | 16.212 | 1.00 | 25.90 |
| 2493 | NH2 | ARG | B | 740 | 37.473 | 17.658 | 15.532 | 1.00 | 24.02 |
| 2494 | N | ASN | B | 741 | 37.102 | 14.654 | 22.236 | 1.00 | 24.93 |
| 2495 | CA | ASN | B | 741 | 37.147 | 13.229 | 21.956 | 1.00 | 27.05 |
| 2496 | C | ASN | B | 741 | 37.072 | 12.358 | 23.206 | 1.00 | 28.80 |
| 2497 | O | ASN | B | 741 | 37.020 | 11.136 | 23.109 | 1.00 | 27.35 |
| 2498 | CB | ASN | B | 741 | 38.392 | 12.925 | 21.134 | 1.00 | 29.54 |
| 2499 | CG | ASN | B | 741 | 38.408 | 13.689 | 19.821 | 1.00 | 28.39 |
| 2500 | OD1 | ASN | B | 741 | 39.465 | 14.059 | 19.312 | 1.00 | 29.43 |
| 2501 | ND2 | ASN | B | 741 | 37.226 | 13.916 | 19.263 | 1.00 | 25.86 |
| 2502 | N | LEU | B | 742 | 37.069 | 12.989 | 24.379 | 1.00 | 27.36 |
| 2503 | CA | LEU | B | 742 | 36.922 | 12.258 | 25.632 | 1.00 | 26.94 |
| 2504 | C | LEU | B | 742 | 35.414 | 12.108 | 25.808 | 1.00 | 25.47 |
| 2505 | O | LEU | B | 742 | 34.648 | 12.905 | 25.273 | 1.00 | 27.23 |
| 2506 | CB | LEU | B | 742 | 37.491 | 13.064 | 26.813 | 1.00 | 23.76 |
| 2507 | CG | LEU | B | 742 | 38.995 | 13.350 | 26.739 | 1.00 | 25.50 |
| 2508 | CD1 | LEU | B | 742 | 39.449 | 14.126 | 27.976 | 1.00 | 29.97 |
| 2509 | CD2 | LEU | B | 742 | 39.751 | 12.040 | 26.658 | 1.00 | 28.27 |
| 2510 | N | HIS | B | 743 | 34.987 | 11.088 | 26.542 | 1.00 | 22.87 |
| 2511 | CA | HIS | B | 743 | 33.565 | 10.873 | 26.786 | 1.00 | 26.83 |
| 2512 | C | HIS | B | 743 | 33.009 | 12.167 | 27.392 | 1.00 | 26.76 |
| 2513 | O | HIS | B | 743 | 33.701 | 12.835 | 28.171 | 1.00 | 24.67 |
| 2514 | CB | HIS | B | 743 | 33.402 | 9.691 | 27.741 | 1.00 | 30.89 |
| 2515 | CG | HIS | B | 743 | 31.996 | 9.201 | 27.874 | 1.00 | 35.28 |
| 2516 | ND1 | HIS | B | 743 | 31.008 | 9.918 | 28.515 | 1.00 | 36.31 |
| 2517 | CD2 | HIS | B | 743 | 31.410 | 8.057 | 27.443 | 1.00 | 38.01 |
| 2518 | CE1 | HIS | B | 743 | 29.875 | 9.238 | 28.473 | 1.00 | 41.45 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2519 | NE2 | HIS | B | 743 | 30.093 | 8.105 | 27.828 | 1.00 | 34.92 |
| 2520 | N | ILE | B | 744 | 31.784 | 12.541 | 27.018 | 1.00 | 25.83 |
| 2521 | CA | ILE | B | 744 | 31.172 | 13.769 | 27.536 | 1.00 | 28.41 |
| 2522 | C | ILE | B | 744 | 31.205 | 13.806 | 29.068 | 1.00 | 27.71 |
| 2523 | O | ILE | B | 744 | 31.410 | 14.868 | 29.669 | 1.00 | 25.71 |
| 2524 | CB | ILE | B | 744 | 29.695 | 13.919 | 27.053 | 1.00 | 29.59 |
| 2525 | CG1 | ILE | B | 744 | 29.082 | 15.205 | 27.610 | 1.00 | 37.61 |
| 2526 | CG2 | ILE | B | 744 | 28.869 | 12.710 | 27.499 | 1.00 | 32.16 |
| 2527 | CD1 | ILE | B | 744 | 29.747 | 16.482 | 27.116 | 1.00 | 40.77 |
| 2528 | N | ASP | B | 745 | 31.006 | 12.658 | 29.711 | 1.00 | 25.28 |
| 2529 | CA | ASP | B | 745 | 31.037 | 12.657 | 31.168 | 1.00 | 26.77 |
| 2530 | C | ASP | B | 745 | 32.424 | 13.001 | 31.733 | 1.00 | 29.51 |
| 2531 | O | ASP | B | 745 | 32.524 | 13.657 | 32.780 | 1.00 | 24.05 |
| 2532 | CB | ASP | B | 745 | 30.544 | 11.318 | 31.722 | 1.00 | 35.05 |
| 2533 | CG | ASP | B | 745 | 29.068 | 11.075 | 31.418 | 1.00 | 36.78 |
| 2534 | OD1 | ASP | B | 745 | 28.266 | 12.015 | 31.582 | 1.00 | 47.93 |
| 2535 | OD2 | ASP | B | 745 | 28.707 | 9.946 | 31.033 | 1.00 | 51.31 |
| 2536 | N | ASP | B | 746 | 33.492 | 12.557 | 31.062 | 1.00 | 24.59 |
| 2537 | CA | ASP | B | 746 | 34.836 | 12.900 | 31.536 | 1.00 | 25.82 |
| 2538 | C | ASP | B | 746 | 35.098 | 14.400 | 31.324 | 1.00 | 24.98 |
| 2539 | O | ASP | B | 746 | 35.762 | 15.049 | 32.130 | 1.00 | 23.59 |
| 2540 | CB | ASP | B | 746 | 35.935 | 12.125 | 30.804 | 1.00 | 27.37 |
| 2541 | CG | ASP | B | 746 | 35.774 | 10.616 | 30.908 | 1.00 | 35.48 |
| 2542 | OD1 | ASP | B | 746 | 35.307 | 10.109 | 31.957 | 1.00 | 33.88 |
| 2543 | OD2 | ASP | B | 746 | 36.155 | 9.937 | 29.933 | 1.00 | 34.19 |
| 2544 | N | GLN | B | 747 | 34.597 | 14.948 | 30.222 | 1.00 | 23.17 |
| 2545 | CA | GLN | B | 747 | 34.794 | 16.367 | 29.946 | 1.00 | 23.00 |
| 2546 | C | GLN | B | 747 | 34.183 | 17.197 | 31.056 | 1.00 | 19.52 |
| 2547 | O | GLN | B | 747 | 34.805 | 18.141 | 31.563 | 1.00 | 21.92 |
| 2548 | CB | GLN | B | 747 | 34.139 | 16.761 | 28.617 | 1.00 | 20.19 |
| 2549 | CG | GLN | B | 747 | 34.774 | 16.122 | 27.398 | 1.00 | 18.51 |
| 2550 | CD | GLN | B | 747 | 34.103 | 16.560 | 26.104 | 1.00 | 29.12 |
| 2551 | OE1 | GLN | B | 747 | 33.915 | 17.747 | 25.876 | 1.00 | 22.13 |
| 2552 | NE2 | GLN | B | 747 | 33.760 | 15.601 | 25.243 | 1.00 | 22.48 |
| 2553 | N | ILE | B | 748 | 32.948 | 16.855 | 31.415 | 1.00 | 20.67 |
| 2554 | CA | ILE | B | 748 | 32.247 | 17.575 | 32.454 | 1.00 | 24.05 |
| 2555 | C | ILE | B | 748 | 32.978 | 17.422 | 33.766 | 1.00 | 23.11 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2556 | O | ILE | B | 748 | 33.196 | 18.404 | 34.471 | 1.00 | 20.45 |
| 2557 | CB | ILE | B | 748 | 30.802 | 17.085 | 32.581 | 1.00 | 24.48 |
| 2558 | CG1 | ILE | B | 748 | 30.032 | 17.501 | 31.328 | 1.00 | 28.95 |
| 2559 | CG2 | ILE | B | 748 | 30.146 | 17.674 | 33.830 | 1.00 | 28.57 |
| 2560 | CD1 | ILE | B | 748 | 28.623 | 16.921 | 31.244 | 1.00 | 37.34 |
| 2561 | N | THR | B | 749 | 33.388 | 16.200 | 34.076 | 1.00 | 19.37 |
| 2562 | CA | THR | B | 749 | 34.108 | 15.961 | 35.320 | 1.00 | 22.97 |
| 2563 | C | THR | B | 749 | 35.404 | 16.768 | 35.376 | 1.00 | 19.50 |
| 2564 | O | THR | B | 749 | 35.698 | 17.391 | 36.390 | 1.00 | 20.73 |
| 2565 | CB | THR | B | 749 | 34.439 | 14.464 | 35.492 | 1.00 | 27.90 |
| 2566 | OG1 | THR | B | 749 | 33.218 | 13.724 | 35.603 | 1.00 | 30.62 |
| 2567 | CG2 | THR | B | 749 | 35.269 | 14.238 | 36.742 | 1.00 | 31.03 |
| 2568 | N | LEU | B | 750 | 36.170 | 16.780 | 34.286 | 1.00 | 19.81 |
| 2569 | CA | LEU | B | 750 | 37.433 | 17.526 | 34.295 | 1.00 | 20.44 |
| 2570 | C | LEU | B | 750 | 37.232 | 19.026 | 34.495 | 1.00 | 23.24 |
| 2571 | O | LEU | B | 750 | 38.015 | 19.676 | 35.186 | 1.00 | 17.97 |
| 2572 | CB | LEU | B | 750 | 38.223 | 17.259 | 33.002 | 1.00 | 23.58 |
| 2573 | CG | LEU | B | 750 | 38.682 | 15.795 | 32.882 | 1.00 | 22.85 |
| 2574 | CD1 | LEU | B | 750 | 39.218 | 15.509 | 31.477 | 1.00 | 22.39 |
| 2575 | CD2 | LEU | B | 750 | 39.776 | 15.524 | 33.922 | 1.00 | 26.05 |
| 2576 | N | ILE | B | 751 | 36.193 | 19.587 | 33.881 | 1.00 | 16.58 |
| 2577 | CA | ILE | B | 751 | 35.930 | 21.000 | 34.061 | 1.00 | 19.30 |
| 2578 | C | ILE | B | 751 | 35.486 | 21.264 | 35.503 | 1.00 | 19.20 |
| 2579 | O | ILE | B | 751 | 35.894 | 22.258 | 36.112 | 1.00 | 20.53 |
| 2580 | CB | ILE | B | 751 | 34.862 | 21.477 | 33.064 | 1.00 | 19.73 |
| 2581 | CG1 | ILE | B | 751 | 35.477 | 21.535 | 31.655 | 1.00 | 17.39 |
| 2582 | CG2 | ILE | B | 751 | 34.333 | 22.842 | 33.474 | 1.00 | 19.69 |
| 2583 | CD1 | ILE | B | 751 | 34.453 | 21.888 | 30.553 | 1.00 | 25.86 |
| 2584 | N | GLN | B | 752 | 34.649 | 20.377 | 36.039 | 1.00 | 17.90 |
| 2585 | CA | GLN | B | 752 | 34.167 | 20.524 | 37.407 | 1.00 | 19.84 |
| 2586 | C | GLN | B | 752 | 35.290 | 20.395 | 38.424 | 1.00 | 22.37 |
| 2587 | O | GLN | B | 752 | 35.241 | 21.000 | 39.485 | 1.00 | 20.97 |
| 2588 | CB | GLN | B | 752 | 33.067 | 19.502 | 37.702 | 1.00 | 23.55 |
| 2589 | CG | GLN | B | 752 | 31.771 | 19.857 | 36.983 | 1.00 | 23.27 |
| 2590 | CD | GLN | B | 752 | 30.646 | 18.872 | 37.223 | 1.00 | 27.86 |
| 2591 | OE1 | GLN | B | 752 | 29.481 | 19.154 | 36.920 | 1.00 | 30.08 |
| 2592 | NE2 | GLN | B | 752 | 30.980 | 17.714 | 37.735 | 1.00 | 21.11 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2593 | N | TYR | B | 753 | 36.299 | 19.588 | 38.102 | 1.00 | 16.66 |
| 2594 | CA | TYR | B | 753 | 37.444 | 19.437 | 39.013 | 1.00 | 21.25 |
| 2595 | C | TYR | B | 753 | 38.451 | 20.586 | 38.939 | 1.00 | 21.49 |
| 2596 | O | TYR | B | 753 | 39.049 | 20.985 | 39.945 | 1.00 | 28.69 |
| 2597 | CB | TYR | B | 753 | 38.256 | 18.186 | 38.682 | 1.00 | 19.38 |
| 2598 | CG | TYR | B | 753 | 37.659 | 16.849 | 39.059 | 1.00 | 26.62 |
| 2599 | CD1 | TYR | B | 753 | 36.491 | 16.747 | 39.813 | 1.00 | 24.99 |
| 2600 | CD2 | TYR | B | 753 | 38.323 | 15.668 | 38.710 | 1.00 | 27.30 |
| 2601 | CE1 | TYR | B | 753 | 36.007 | 15.502 | 40.215 | 1.00 | 28.65 |
| 2602 | CE2 | TYR | B | 753 | 37.850 | 14.432 | 39.106 | 1.00 | 26.34 |
| 2603 | CZ | TYR | B | 753 | 36.707 | 14.347 | 39.853 | 1.00 | 29.07 |
| 2604 | OH | TYR | B | 753 | 36.282 | 13.103 | 40.254 | 1.00 | 34.63 |
| 2605 | N | SER | B | 754 | 38.639 | 21.126 | 37.744 | 1.00 | 20.94 |
| 2606 | CA | SER | B | 754 | 39.679 | 22.122 | 37.540 | 1.00 | 20.30 |
| 2607 | C | SER | B | 754 | 39.318 | 23.587 | 37.387 | 1.00 | 20.01 |
| 2608 | O | SER | B | 754 | 40.218 | 24.404 | 37.314 | 1.00 | 20.79 |
| 2609 | CB | SER | B | 754 | 40.476 | 21.734 | 36.297 | 1.00 | 26.89 |
| 2610 | OG | SER | B | 754 | 39.650 | 21.929 | 35.155 | 1.00 | 24.56 |
| 2611 | N | TRP | B | 755 | 38.040 | 23.943 | 37.347 | 1.00 | 18.81 |
| 2612 | CA | TRP | B | 755 | 37.710 | 25.352 | 37.136 | 1.00 | 21.09 |
| 2613 | C | TRP | B | 755 | 38.414 | 26.366 | 38.060 | 1.00 | 22.06 |
| 2614 | O | TRP | B | 755 | 38.864 | 27.419 | 37.598 | 1.00 | 21.85 |
| 2615 | CB | TRP | B | 755 | 36.188 | 25.585 | 37.199 | 1.00 | 19.53 |
| 2616 | CG | TRP | B | 755 | 35.576 | 25.368 | 38.537 | 1.00 | 19.91 |
| 2617 | CD1 | TRP | B | 755 | 35.105 | 24.196 | 39.045 | 1.00 | 22.90 |
| 2618 | CD2 | TRP | B | 755 | 35.393 | 26.354 | 39.557 | 1.00 | 24.20 |
| 2619 | NE1 | TRP | B | 755 | 34.636 | 24.389 | 40.323 | 1.00 | 25.70 |
| 2620 | CE2 | TRP | B | 755 | 34.804 | 25.707 | 40.661 | 1.00 | 19.09 |
| 2621 | CE3 | TRP | B | 755 | 35.672 | 27.728 | 39.643 | 1.00 | 23.21 |
| 2622 | CZ2 | TRP | B | 755 | 34.486 | 26.379 | 41.842 | 1.00 | 22.66 |
| 2623 | CZ3 | TRP | B | 755 | 35.356 | 28.394 | 40.811 | 1.00 | 25.02 |
| 2624 | CH2 | TRP | B | 755 | 34.767 | 27.720 | 41.900 | 1.00 | 26.92 |
| 2625 | N | MET | B | 756 | 38.512 | 26.084 | 39.353 | 1.00 | 20.39 |
| 2626 | CA | MET | B | 756 | 39.169 | 27.042 | 40.249 | 1.00 | 22.21 |
| 2627 | C | MET | B | 756 | 40.641 | 27.207 | 39.885 | 1.00 | 18.94 |
| 2628 | O | MET | B | 756 | 41.183 | 28.334 | 39.891 | 1.00 | 21.30 |
| 2629 | CB | MET | B | 756 | 39.051 | 26.592 | 41.720 | 1.00 | 24.34 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2630 | CG | MET | B | 756 | 39.665 | 27.592 | 42.706 | 1.00 | 22.35 |
| 2631 | SD | MET | B | 756 | 38.654 | 29.079 | 42.939 | 1.00 | 27.19 |
| 2632 | CE | MET | B | 756 | 37.316 | 28.325 | 43.999 | 1.00 | 23.83 |
| 2633 | N | SER | B | 757 | 41.289 | 26.098 | 39.556 | 1.00 | 20.58 |
| 2634 | CA | SER | B | 757 | 42.700 | 26.133 | 39.208 | 1.00 | 21.19 |
| 2635 | C | SER | B | 757 | 42.875 | 26.932 | 37.917 | 1.00 | 20.60 |
| 2636 | O | SER | B | 757 | 43.734 | 27.793 | 37.820 | 1.00 | 19.30 |
| 2637 | CB | SER | B | 757 | 43.254 | 24.702 | 39.054 | 1.00 | 25.76 |
| 2638 | OG | SER | B | 757 | 42.837 | 24.114 | 37.832 | 1.00 | 34.55 |
| 2639 | N | LEU | B | 758 | 42.050 | 26.665 | 36.915 | 1.00 | 18.54 |
| 2640 | CA | LEU | B | 758 | 42.149 | 27.419 | 35.678 | 1.00 | 20.03 |
| 2641 | C | LEU | B | 758 | 41.929 | 28.915 | 35.894 | 1.00 | 19.30 |
| 2642 | O | LEU | B | 758 | 42.652 | 29.749 | 35.323 | 1.00 | 21.43 |
| 2643 | CB | LEU | B | 758 | 41.122 | 26.896 | 34.667 | 1.00 | 20.21 |
| 2644 | CG | LEU | B | 758 | 41.373 | 25.461 | 34.198 | 1.00 | 25.47 |
| 2645 | CD1 | LEU | B | 758 | 40.154 | 24.953 | 33.419 | 1.00 | 26.99 |
| 2646 | CD2 | LEU | B | 758 | 42.649 | 25.424 | 33.351 | 1.00 | 22.02 |
| 2647 | N | MET | B | 759 | 40.949 | 29.282 | 36.721 | 1.00 | 19.96 |
| 2648 | CA | MET | B | 759 | 40.679 | 30.702 | 36.916 | 1.00 | 17.34 |
| 2649 | C | MET | B | 759 | 41.713 | 31.441 | 37.742 | 1.00 | 19.85 |
| 2650 | O | MET | B | 759 | 42.016 | 32.600 | 37.455 | 1.00 | 21.26 |
| 2651 | CB | MET | B | 759 | 39.268 | 30.914 | 37.491 | 1.00 | 21.30 |
| 2652 | CG | MET | B | 759 | 38.202 | 30.434 | 36.522 | 1.00 | 25.18 |
| 2653 | SD | MET | B | 759 | 36.495 | 30.703 | 37.019 | 1.00 | 32.88 |
| 2654 | CE | MET | B | 759 | 35.638 | 29.677 | 35.765 | 1.00 | 35.46 |
| 2655 | N | VAL | B | 760 | 42.282 | 30.788 | 38.748 | 1.00 | 19.44 |
| 2656 | CA | VAL | B | 760 | 43.288 | 31.476 | 39.548 | 1.00 | 22.37 |
| 2657 | C | VAL | B | 760 | 44.582 | 31.585 | 38.723 | 1.00 | 23.17 |
| 2658 | O | VAL | B | 760 | 45.346 | 32.547 | 38.842 | 1.00 | 22.82 |
| 2659 | CB | VAL | B | 760 | 43.511 | 30.753 | 40.921 | 1.00 | 22.03 |
| 2660 | CG1 | VAL | B | 760 | 44.296 | 29.450 | 40.762 | 1.00 | 22.24 |
| 2661 | CG2 | VAL | B | 760 | 44.161 | 31.725 | 41.908 | 1.00 | 26.70 |
| 2662 | N | PHE | B | 761 | 44.810 | 30.611 | 37.847 | 1.00 | 23.09 |
| 2663 | CA | PHE | B | 761 | 45.989 | 30.643 | 36.989 | 1.00 | 24.16 |
| 2664 | C | PHE | B | 761 | 45.832 | 31.784 | 35.978 | 1.00 | 23.45 |
| 2665 | O | PHE | B | 761 | 46.794 | 32.493 | 35.674 | 1.00 | 24.87 |
| 2666 | CB | PHE | B | 761 | 46.128 | 29.298 | 36.266 | 1.00 | 25.81 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2667 | CG | PHE | B | 761 | 47.512 | 29.020 | 35.741 | 1.00 | 25.64 |
| 2668 | CD1 | PHE | B | 761 | 48.586 | 28.889 | 36.616 | 1.00 | 25.18 |
| 2669 | CD2 | PHE | B | 761 | 47.719 | 28.794 | 34.383 | 1.00 | 19.58 |
| 2670 | CE1 | PHE | B | 761 | 49.836 | 28.528 | 36.135 | 1.00 | 27.13 |
| 2671 | CE2 | PHE | B | 761 | 48.964 | 28.430 | 33.895 | 1.00 | 25.89 |
| 2672 | CZ | PHE | B | 761 | 50.026 | 28.294 | 34.770 | 1.00 | 26.79 |
| 2673 | N | GLY | B | 762 | 44.619 | 31.932 | 35.445 | 1.00 | 26.41 |
| 2674 | CA | GLY | B | 762 | 44.315 | 32.988 | 34.493 | 1.00 | 22.56 |
| 2675 | C | GLY | B | 762 | 44.459 | 34.341 | 35.170 | 1.00 | 24.22 |
| 2676 | 0 | GLY | B | 762 | 45.005 | 35.281 | 34.588 | 1.00 | 22.86 |
| 2677 | N | LEU | B | 763 | 43.966 | 34.441 | 36.406 | 1.00 | 22.38 |
| 2678 | CA | LEU | B | 763 | 44.083 | 35.674 | 37.186 | 1.00 | 20.84 |
| 2679 | C | LEU | B | 763 | 45.567 | 36.032 | 37.319 | 1.00 | 23.50 |
| 2680 | 0 | LEU | B | 763 | 45.961 | 37.202 | 37.183 | 1.00 | 23.47 |
| 2681 | CB | LEU | B | 763 | 43.490 | 35.476 | 38.586 | 1.00 | 25.56 |
| 2682 | CG | LEU | B | 763 | 43.743 | 36.570 | 39.641 | 1.00 | 19.73 |
| 2683 | CD1 | LEU | B | 763 | 43.178 | 37.897 | 39.172 | 1.00 | 20.52 |
| 2684 | CD2 | LEU | B | 763 | 43.081 | 36.166 | 40.948 | 1.00 | 21.82 |
| 2685 | N | GLY | B | 764 | 46.379 | 35.015 | 37.588 | 1.00 | 23.30 |
| 2686 | CA | GLY | B | 764 | 47.805 | 35.230 | 37.747 | 1.00 | 26.93 |
| 2687 | C | GLY | B | 764 | 48.398 | 35.805 | 36.481 | 1.00 | 28.28 |
| 2688 | O | GLY | B | 764 | 49.167 | 36.768 | 36.511 | 1.00 | 31.36 |
| 2689 | N | TRP | B | 765 | 48.025 | 35.220 | 35.351 | 1.00 | 23.45 |
| 2690 | CA | TRP | B | 765 | 48.539 | 35.681 | 34.073 | 1.00 | 27.13 |
| 2691 | C | TRP | B | 765 | 48.133 | 37.117 | 33.746 | 1.00 | 26.40 |
| 2692 | O | TRP | B | 765 | 48.970 | 37.924 | 33.333 | 1.00 | 29.82 |
| 2693 | CB | TRP | B | 765 | 48.091 | 34.726 | 32.961 | 1.00 | 26.89 |
| 2694 | CG | TRP | B | 765 | 48.662 | 35.089 | 31.648 | 1.00 | 26.96 |
| 2695 | CD1 | TRP | B | 765 | 48.053 | 35.775 | 30.641 | 1.00 | 29.28 |
| 2696 | CD2 | TRP | B | 765 | 50.019 | 34.893 | 31.241 | 1.00 | 26.36 |
| 2697 | NE1 | TRP | B | 765 | 48.951 | 36.022 | 29.630 | 1.00 | 29.91 |
| 2698 | CE2 | TRP | B | 765 | 50.167 | 35.493 | 29.975 | 1.00 | 31.07 |
| 2699 | CE3 | TRP | B | 765 | 51.127 | 34.270 | 31.832 | 1.00 | 29.13 |
| 2700 | CZ2 | TRP | B | 765 | 51.388 | 35.489 | 29.279 | 1.00 | 33.25 |
| 2701 | CZ3 | TRP | B | 765 | 52.342 | 34.264 | 31.140 | 1.00 | 32.77 |
| 2702 | CH2 | TRP | B | 765 | 52.456 | 34.868 | 29.881 | 1.00 | 26.10 |
| 2703 | N | ARG | B | 766 | 46.855 | 37.451 | 33.924 | 1.00 | 25.83 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2704 | CA | ARG | B | 766 | 46.406 | 38.808 | 33.639 | 1.00 | 24.82 |
| 2705 | C | ARG | B | 766 | 47.084 | 39.831 | 34.538 | 1.00 | 24.76 |
| 2706 | O | ARG | B | 766 | 47.389 | 40.943 | 34.104 | 1.00 | 28.34 |
| 2707 | CB | ARG | B | 766 | 44.883 | 38.939 | 33.808 | 1.00 | 26.93 |
| 2708 | CG | ARG | B | 766 | 44.047 | 38.200 | 32.774 | 1.00 | 24.24 |
| 2709 | CD | ARG | B | 766 | 42.589 | 38.568 | 32.948 | 1.00 | 26.44 |
| 2710 | NE | ARG | B | 766 | 42.078 | 38.160 | 34.256 | 1.00 | 26.20 |
| 2711 | CZ | ARG | B | 766 | 41.655 | 36.932 | 34.559 | 1.00 | 23.20 |
| 2712 | NH1 | ARG | B | 766 | 41.673 | 35.975 | 33.656 | 1.00 | 26.38 |
| 2713 | NH2 | ARG | B | 766 | 41.210 | 36.665 | 35.784 | 1.00 | 23.30 |
| 2714 | N | SER | B | 767 | 47.322 | 39.465 | 35.793 | 1.00 | 21.83 |
| 2715 | CA | SER | B | 767 | 47.954 | 40.405 | 36.721 | 1.00 | 24.98 |
| 2716 | C | SER | B | 767 | 49.388 | 40.617 | 36.273 | 1.00 | 26.41 |
| 2717 | O | SER | B | 767 | 49.878 | 41.740 | 36.209 | 1.00 | 26.61 |
| 2718 | CB | SER | B | 767 | 47.961 | 39.857 | 38.147 | 1.00 | 24.94 |
| 2719 | OG | SER | B | 767 | 46.653 | 39.669 | 38.646 | 1.00 | 30.28 |
| 2720 | N | TYR | B | 768 | 50.050 | 39.512 | 35.967 | 1.00 | 29.18 |
| 2721 | CA | TYR | B | 768 | 51.436 | 39.532 | 35.514 | 1.00 | 30.71 |
| 2722 | C | TYR | B | 768 | 51.631 | 40.356 | 34.241 | 1.00 | 34.29 |
| 2723 | O | TYR | B | 768 | 52.572 | 41.143 | 34.137 | 1.00 | 37.28 |
| 2724 | CB | TYR | B | 768 | 51.890 | 38.082 | 35.319 | 1.00 | 31.45 |
| 2725 | CG | TYR | B | 768 | 53.208 | 37.878 | 34.597 | 1.00 | 36.24 |
| 2726 | CD1 | TYR | B | 768 | 54.365 | 38.560 | 34.984 | 1.00 | 38.98 |
| 2727 | CD2 | TYR | B | 768 | 53.308 | 36.950 | 33.560 | 1.00 | 38.42 |
| 2728 | CE1 | TYR | B | 768 | 55.590 | 38.317 | 34.356 | 1.00 | 40.04 |
| 2729 | CE2 | TYR | B | 768 | 54.525 | 36.700 | 32.929 | 1.00 | 40.42 |
| 2730 | CZ | TYR | B | 768 | 55.661 | 37.386 | 33.331 | 1.00 | 43.23 |
| 2731 | OH | TYR | B | 768 | 56.865 | 37.125 | 32.706 | 1.00 | 51.72 |
| 2732 | N | LYS | B | 769 | 50.707 | 40.223 | 33.300 | 1.00 | 33.52 |
| 2733 | CA | LYS | B | 769 | 50.828 | 40.907 | 32.018 | 1.00 | 37.03 |
| 2734 | C | LYS | B | 769 | 50.290 | 42.338 | 31.934 | 1.00 | 36.01 |
| 2735 | O | LYS | B | 769 | 50.755 | 43.115 | 31.103 | 1.00 | 33.96 |
| 2736 | CB | LYS | B | 769 | 50.172 | 40.031 | 30.943 | 1.00 | 41.58 |
| 2737 | CG | LYS | B | 769 | 50.422 | 40.435 | 29.497 | 1.00 | 50.59 |
| 2738 | CD | LYS | B | 769 | 49.872 | 39.351 | 28.577 | 1.00 | 52.94 |
| 2739 | CE | LYS | B | 769 | 50.140 | 39.630 | 27.113 | 1.00 | 55.40 |
| 2740 | NZ | LYS | B | 769 | 49.610 | 38.525 | 26.263 | 1.00 | 56.70 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2741 | N | HIS | B | 770 | 49.329 | 42.700 | 32.780 | 1.00 | 35.24 |
| 2742 | CA | HIS | B | 770 | 48.758 | 44.047 | 32.723 | 1.00 | 34.60 |
| 2743 | C | HIS | B | 770 | 49.204 | 45.022 | 33.790 | 1.00 | 36.43 |
| 2744 | O | HIS | B | 770 | 49.272 | 46.229 | 33.541 | 1.00 | 34.37 |
| 2745 | CB | HIS | B | 770 | 47.236 | 43.983 | 32.747 | 1.00 | 35.70 |
| 2746 | CG | HIS | B | 770 | 46.649 | 43.272 | 31.573 | 1.00 | 46.81 |
| 2747 | ND1 | HIS | B | 770 | 46.827 | 43.706 | 30.277 | 1.00 | 50.52 |
| 2748 | CD2 | HIS | B | 770 | 45.892 | 42.152 | 31.496 | 1.00 | 49.20 |
| 2749 | CE1 | HIS | B | 770 | 46.205 | 42.883 | 29.452 | 1.00 | 49.27 |
| 2750 | NE2 | HIS | B | 770 | 45.630 | 41.932 | 30.165 | 1.00 | 51.77 |
| 2751 | N | VAL | B | 771 | 49.487 | 44.520 | 34.985 | 1.00 | 33.76 |
| 2752 | CA | VAL | B | 771 | 49.907 | 45.409 | 36.054 | 1.00 | 32.20 |
| 2753 | C | VAL | B | 771 | 51.229 | 44.988 | 36.679 | 1.00 | 30.98 |
| 2754 | O | VAL | B | 771 | 51.479 | 45.235 | 37.852 | 1.00 | 34.33 |
| 2755 | CB | VAL | B | 771 | 48.795 | 45.520 | 37.136 | 1.00 | 34.95 |
| 2756 | CG1 | VAL | B | 771 | 47.603 | 46.297 | 36.576 | 1.00 | 33.50 |
| 2757 | CG2 | VAL | B | 771 | 48.332 | 44.140 | 37.557 | 1.00 | 29.74 |
| 2758 | N | SER | B | 772 | 52.075 | 44.350 | 35.878 | 1.00 | 30.62 |
| 2759 | CA | SER | B | 772 | 53.386 | 43.907 | 36.338 | 1.00 | 31.95 |
| 2760 | C | SER | B | 772 | 53.284 | 43.015 | 37.561 | 1.00 | 33.92 |
| 2761 | O | SER | B | 772 | 54.226 | 42.930 | 38.350 | 1.00 | 30.00 |
| 2762 | CB | SER | B | 772 | 54.256 | 45.117 | 36.674 | 1.00 | 34.85 |
| 2763 | OG | SER | B | 772 | 54.357 | 45.981 | 35.558 | 1.00 | 43.83 |
| 2764 | N | GLY | B | 773 | 52.128 | 42.371 | 37.724 | 1.00 | 28.37 |
| 2765 | CA | GLY | B | 773 | 51.917 | 41.473 | 38.844 | 1.00 | 29.00 |
| 2766 | C | GLY | B | 773 | 51.706 | 42.154 | 40.186 | 1.00 | 26.33 |
| 2767 | O | GLY | B | 773 | 51.644 | 41.482 | 41.224 | 1.00 | 26.94 |
| 2768 | N | GLN | B | 774 | 51.554 | 43.474 | 40.177 | 1.00 | 24.89 |
| 2769 | CA | GLN | B | 774 | 51.411 | 44.204 | 41.429 | 1.00 | 26.11 |
| 2770 | C | GLN | B | 774 | 49.994 | 44.597 | 41.871 | 1.00 | 28.67 |
| 2771 | O | GLN | B | 774 | 49.818 | 45.376 | 42.811 | 1.00 | 27.16 |
| 2772 | CB | GLN | B | 774 | 52.345 | 45.425 | 41.417 | 1.00 | 25.33 |
| 2773 | CG | GLN | B | 774 | 53.803 | 45.022 | 41.149 | 1.00 | 29.08 |
| 2774 | CD | GLN | B | 774 | 54.233 | 43.816 | 41.973 | 1.00 | 27.66 |
| 2775 | OE1 | GLN | B | 774 | 54.227 | 43.848 | 43.216 | 1.00 | 30.12 |
| 2776 | NE2 | GLN | B | 774 | 54.600 | 42.738 | 41.289 | 1.00 | 30.15 |
| 2777 | N | MET | B | 775 | 48.993 | 44.041 | 41.196 | 1.00 | 24.77 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2778 | CA | MET | B | 775 | 47.589 | 44.238 | 41.560 | 1.00 | 26.82 |
| 2779 | C | MET | B | 775 | 46.920 | 42.961 | 41.091 | 1.00 | 24.83 |
| 2780 | O | MET | B | 775 | 47.494 | 42.244 | 40.288 | 1.00 | 26,99 |
| 2781 | CB | MET | B | 775 | 46.952 | 45.419 | 40.827 | 1.00 | 28.09 |
| 2782 | CG | MET | B | 775 | 47.653 | 46.741 | 41.024 | 1.00 | 26.44 |
| 2783 | SD | MET | B | 775 | 46.706 | 48.019 | 40.194 | 1.00 | 37.11 |
| 2784 | CE | MET | B | 775 | 47.958 | 49.275 | 39.887 | 1.00 | 41.20 |
| 2785 | N | LEU | B | 776 | 45.739 | 42.655 | 41.611 | 1.00 | 26.95 |
| 2786 | CA | LEU | B | 776 | 45.017 | 41.454 | 41.176 | 1.00 | 23.64 |
| 2787 | C | LEU | B | 776 | 44.072 | 41.936 | 40.097 | 1.00 | 25.94 |
| 2788 | O | LEU | B | 776 | 43.125 | 42.672 | 40.372 | 1.00 | 26.27 |
| 2789 | CB | LEU | B | 776 | 44.235 | 40.826 | 42.330 | 1.00 | 26.64 |
| 2790 | CG | LEU | B | 776 | 45.070 | 40.094 | 43.390 | 1.00 | 27.80 |
| 2791 | CD1 | LEU | B | 776 | 44.161 | 39.532 | 44.462 | 1.00 | 26.56 |
| 2792 | CD2 | LEU | B | 776 | 45.855 | 38.956 | 42.735 | 1.00 | 29.50 |
| 2793 | N | TYR | B | 777 | 44.357 | 41.521 | 38.868 | 1.00 | 23.88 |
| 2794 | CA | TYR | B | 777 | 43.590 | 41.934 | 37.705 | 1.00 | 23.44 |
| 2795 | C | TYR | B | 777 | 42.525 | 40.892 | 37.399 | 1.00 | 25.02 |
| 2796 | O | TYR | B | 777 | 42.665 | 40.086 | 36.477 | 1.00 | 22.33 |
| 2797 | CB | TYR | B | 777 | 44.565 | 42.113 | 36.533 | 1.00 | 27.34 |
| 2798 | CG | TYR | B | 777 | 44.011 | 42.846 | 35.342 | 1.00 | 29.61 |
| 2799 | CD1 | TYR | B | 777 | 43.122 | 42.230 | 34.463 | 1.00 | 32.26 |
| 2800 | CD2 | TYR | B | 777 | 44.397 | 44.155 | 35.078 | 1.00 | 30.45 |
| 2801 | CE1 | TYR | B | 777 | 42.644 | 42.899 | 33.350 | 1.00 | 32.66 |
| 2802 | CE2 | TYR | B | 777 | 43.926 | 44.834 | 33.971 | 1.00 | 33.13 |
| 2803 | CZ | TYR | B | 777 | 43.054 | 44.205 | 33.108 | 1.00 | 34.40 |
| 2804 | OH | TYR | B | 777 | 42.624 | 44.877 | 31.982 | 1.00 | 34.07 |
| 2805 | N | PHE | B | 778 | 41.463 | 40.899 | 38.204 | 1.00 | 22.02 |
| 2806 | CA | PHE | B | 778 | 40.359 | 39.952 | 38.004 | 1.00 | 24.28 |
| 2807 | C | PHE | B | 778 | 39.700 | 40.235 | 36.663 | 1.00 | 24.40 |
| 2808 | O | PHE | B | 778 | 39.355 | 39.313 | 35.916 | 1.00 | 23.54 |
| 2809 | CB | PHE | B | 778 | 39.329 | 40.097 | 39.126 | 1.00 | 20.00 |
| 2810 | CG | PHE | B | 778 | 39.805 | 39.594 | 40.444 | 1.00 | 21.83 |
| 2811 | CD1 | PHE | B | 778 | 39.841 | 38.227 | 40.709 | 1.00 | 21.24 |
| 2812 | CD2 | PHE | B | 778 | 40.238 | 40.482 | 41.426 | 1.00 | 25.82 |
| 2813 | CE1 | PHE | B | 778 | 40.299 | 37.762 | 41.930 | 1.00 | 24.17 |
| 2814 | CE2 | PHE | B | 778 | 40.702 | 40.013 | 42.656 | 1.00 | 21.13 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2815 | CZ | PHE | B | 778 | 40.729 | 38.650 | 42.901 | 1.00 | 21.77 |
| 2816 | N | ALA | B | 779 | 39.530 | 41.522 | 36.367 | 1.00 | 22.16 |
| 2817 | CA | ALA | B | 779 | 38.930 | 41.976 | 35.113 | 1.00 | 24.84 |
| 2818 | C | ALA | B | 779 | 39.289 | 43.453 | 34.917 | 1.00 | 25.04 |
| 2819 | O | ALA | B | 779 | 39.724 | 44.114 | 35.850 | 1.00 | 25.47 |
| 2820 | CB | ALA | B | 779 | 37.407 | 41.814 | 35.155 | 1.00 | 20.33 |
| 2821 | N | PRO | B | 780 | 39.075 | 43.995 | 33.708 | 1.00 | 29.20 |
| 2822 | CA | PRO | B | 780 | 39.412 | 45.403 | 33.488 | 1.00 | 31.77 |
| 2823 | C | PRO | B | 780 | 38.656 | 46.367 | 34.407 | 1.00 | 33.65 |
| 2824 | O | PRO | B | 780 | 39.149 | 47.463 | 34.704 | 1.00 | 31,21 |
| 2825 | CB | PRO | B | 780 | 39.066 | 45.603 | 32.004 | 1.00 | 33.06 |
| 2826 | CG | PRO | B | 780 | 39.260 | 44.203 | 31.433 | 1.00 | 37.79 |
| 2827 | CD | PRO | B | 780 | 38.515 | 43.414 | 32.480 | 1.00 | 28.89 |
| 2828 | N | ASP | B | 781 | 37.465 | 45.951 | 34.851 | 1.00 | 30.03 |
| 2829 | CA | ASP | B | 781 | 36.603 | 46.758 | 35.719 | 1.00 | 27.54 |
| 2830 | C | ASP | B | 781 | 36.606 | 46.262 | 37.171 | 1.00 | 30.56 |
| 2831 | O | ASP | B | 781 | 35.774 | 46.680 | 37.987 | 1.00 | 30.31 |
| 2832 | CB | ASP | B | 781 | 35.163 | 46.739 | 35.172 | 1.00 | 30.77 |
| 2833 | CG | ASP | B | 781 | 34.536 | 45.344 | 35.210 | 1.00 | 32.93 |
| 2834 | OD1 | ASP | B | 781 | 35.251 | 44.350 | 34.942 | 1.00 | 30.51 |
| 2835 | OD2 | ASP | B | 781 | 33.317 | 45.240 | 35.482 | 1.00 | 38.32 |
| 2836 | N | LEU | B | 782 | 37.539 | 45.368 | 37.485 | 1.00 | 28.57 |
| 2837 | CA | LEU | B | 782 | 37.667 | 44.820 | 38.833 | 1.00 | 27.89 |
| 2838 | C | LEU | B | 782 | 39.135 | 44.522 | 39.090 | 1.00 | 27.07 |
| 2839 | O | LEU | B | 782 | 39.611 | 43.382 | 38.993 | 1.00 | 25.66 |
| 2840 | CB | LEU | B | 782 | 36.808 | 43.555 | 38.996 | 1.00 | 23.48 |
| 2841 | CG | LEU | B | 782 | 36.673 | 43.026 | 40.431 | 1.00 | 27.04 |
| 2842 | CD1 | LEU | B | 782 | 36.229 | 44.163 | 41.340 | 1.00 | 30.90 |
| 2843 | CD2 | LEU | B | 782 | 35.666 | 41.850 | 40.492 | 1.00 | 26.37 |
| 2844 | N | ILE | B | 783 | 39.860 | 45.584 | 39.399 | 1.00 | 26.64 |
| 2845 | CA | ILE | B | 783 | 41.279 | 45.491 | 39.675 | 1.00 | 28.26 |
| 2846 | C | ILE | B | 783 | 41.460 | 45.862 | 41.126 | 1.00 | 25.73 |
| 2847 | O | ILE | B | 783 | 40.948 | 46.879 | 41.587 | 1.00 | 29.13 |
| 2848 | CB | ILE | B | 783 | 42.070 | 46.471 | 38.778 | 1.00 | 28.66 |
| 2849 | CG1 | ILE | B | 783 | 41.847 | 46.101 | 37.314 | 1.00 | 28.24 |
| 2850 | CG2 | ILE | B | 783 | 43.552 | 46.422 | 39.108 | 1.00 | 30.46 |
| 2851 | CD1 | ILE | B | 783 | 42.448 | 47.084 | 36.325 | 1.00 | 33.70 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2852 | N | LEU | B | 784 | 42.175 | 45.022 | 41.855 | 1.00 | 30.10 |
| 2853 | CA | LEU | B | 784 | 42.400 | 45.278 | 43.263 | 1.00 | 27.98 |
| 2854 | C | LEU | B | 784 | 43.870 | 45.525 | 43.576 | 1.00 | 26.93 |
| 2855 | O | LEU | B | 784 | 44.663 | 44.587 | 43.507 | 1.00 | 24.54 |
| 2856 | CB | LEU | B | 784 | 41.969 | 44.081 | 44.099 | 1.00 | 30.52 |
| 2857 | CG | LEU | B | 784 | 40.599 | 43.447 | 43.895 | 1.00 | 35.83 |
| 2858 | CD1 | LEU | B | 784 | 40.345 | 42.533 | 45.087 | 1.00 | 36.58 |
| 2859 | CD2 | LEU | B | 784 | 39.523 | 44.498 | 43.810 | 1.00 | 36.04 |
| 2860 | N | ASN | B | 785 | 44.237 | 46.761 | 43.912 | 1.00 | 25.22 |
| 2861 | CA | ASN | B | 785 | 45.625 | 47.017 | 44.306 | 1.00 | 30.59 |
| 2862 | C | ASN | B | 785 | 45.715 | 46.566 | 45.763 | 1.00 | 29.47 |
| 2863 | O | ASN | B | 785 | 44.688 | 46.271 | 46.387 | 1.00 | 30.15 |
| 2864 | CB | ASN | B | 785 | 46.018 | 48.501 | 44.155 | 1.00 | 25.33 |
| 2865 | CG | ASN | B | 785 | 45.043 | 49.451 | 44.824 | 1.00 | 29.56 |
| 2866 | OD1 | ASN | B | 785 | 44.530 | 49.182 | 45.907 | 1.00 | 35.66 |
| 2867 | ND2 | ASN | B | 785 | 44.815 | 50.590 | 44.193 | 1.00 | 30.74 |
| 2868 | N | GLU | B | 786 | 46.918 | 46.509 | 46.321 | 1.00 | 30.15 |
| 2869 | CA | GLU | B | 786 | 47.051 | 46.007 | 47.684 | 1.00 | 29.94 |
| 2870 | C | GLU | B | 786 | 46.168 | 46.734 | 48.691 | 1.00 | 30.50 |
| 2871 | O | GLU | B | 786 | 45.591 | 46.100 | 49.572 | 1.00 | 30.06 |
| 2872 | CB | GLU | B | 786 | 48.527 | 46.011 | 48.132 | 1.00 | 34.17 |
| 2873 | CG | GLU | B | 786 | 48.744 | 45.444 | 49.540 | 1.00 | 33.86 |
| 2874 | CD | GLU | B | 786 | 50.188 | 45.061 | 49.836 | 1.00 | 41.39 |
| 2875 | OE1 | GLU | B | 786 | 51.113 | 45.780 | 49.403 | 1.00 | 34.83 |
| 2876 | OE2 | GLU | B | 786 | 50.399 | 44.042 | 50.530 | 1.00 | 40.62 |
| 2877 | N | GLN | B | 787 | 46.051 | 48.051 | 48.569 | 1.00 | 32.42 |
| 2878 | CA | GLN | B | 787 | 45.205 | 48.804 | 49.499 | 1.00 | 35.44 |
| 2879 | C | GLN | B | 787 | 43.748 | 48.337 | 49.417 | 1.00 | 34.61 |
| 2880 | O | GLN | B | 787 | 43.051 | 48.231 | 50.430 | 1.00 | 31.06 |
| 2881 | CB | GLN | B | 787 | 45.267 | 50.303 | 49.195 | 1.00 | 33.37 |
| 2882 | CG | GLN | B | 787 | 44.361 | 51.147 | 50.096 | 1.00 | 42.09 |
| 2883 | CD | GLN | B | 787 | 44.724 | 51.049 | 51.571 | 1.00 | 48.86 |
| 2884 | OE1 | GLN | B | 787 | 43.991 | 51.537 | 52.435 | 1.00 | 55.24 |
| 2885 | NE2 | GLN | B | 787 | 45.861 | 50.430 | 51.864 | 1.00 | 49.14 |
| 2886 | N | ARG | B | 788 | 43.283 | 48.065 | 48.206 | 1.00 | 31.38 |
| 2887 | CA | ARG | B | 788 | 41.909 | 47.625 | 48.043 | 1.00 | 34.58 |
| 2888 | C | ARG | B | 788 | 41.784 | 46.219 | 48.645 | 1.00 | 32.19 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2889 | O | ARG | B | 788 | 40.755 | 45.872 | 49.240 | 1.00 | 35.75 |
| 2890 | CB | ARG | B | 788 | 41.524 | 47.641 | 46.570 | 1.00 | 26.45 |
| 2891 | CG | ARG | B | 788 | 40.034 | 47.506 | 46.356 | 1.00 | 37.78 |
| 2892 | CD | ARG | B | 788 | 39.652 | 47.601 | 44.891 | 1.00 | 43.22 |
| 2893 | NE | ARG | B | 788 | 38.202 | 47.526 | 44.738 | 1.00 | 40.59 |
| 2894 | CZ | ARG | B | 788 | 37.560 | 47.561 | 43.577 | 1.00 | 46.17 |
| 2895 | NH1 | ARG | B | 788 | 38.236 | 47.671 | 42.438 | 1.00 | 45.32 |
| 2896 | NH2 | ARG | B | 788 | 36.233 | 47.491 | 43.563 | 1.00 | 47.99 |
| 2897 | N | MET | B | 789 | 42.841 | 45.420 | 48.505 | 1.00 | 34.27 |
| 2898 | CA | MET | B | 789 | 42.865 | 44.072 | 49.068 | 1.00 | 30.67 |
| 2899 | C | MET | B | 789 | 42.757 | 44.175 | 50.583 | 1.00 | 37.95 |
| 2900 | O | MET | B | 789 | 41.995 | 43.451 | 51.220 | 1.00 | 33.50 |
| 2901 | CB | MET | B | 789 | 44.184 | 43.352 | 48.774 | 1.00 | 31.27 |
| 2902 | CG | MET | B | 789 | 44.522 | 43.165 | 47.317 | 1.00 | 30.70 |
| 2903 | SD | MET | B | 789 | 46.045 | 42.194 | 47.093 | 1.00 | 32.85 |
| 2904 | CE | MET | B | 789 | 46.482 | 42.698 | 45.419 | 1.00 | 29.49 |
| 2905 | N | LYS | B | 790 | 43.545 | 45.076 | 51.157 | 1.00 | 35.89 |
| 2906 | CA | LYS | B | 790 | 43.574 | 45.250 | 52.605 | 1.00 | 40.34 |
| 2907 | C | LYS | B | 790 | 42.210 | 45.644 | 53.187 | 1.00 | 41.10 |
| 2908 | O | LYS | B | 790 | 41.873 | 45.255 | 54.309 | 1.00 | 41.11 |
| 2909 | CB | LYS | B | 790 | 44.642 | 46.290 | 52.948 | 1.00 | 38.07 |
| 2910 | CG | LYS | B | 790 | 45.016 | 46.402 | 54.411 | 1.00 | 46.09 |
| 2911 | CD | LYS | B | 790 | 46.202 | 47.357 | 54.572 | 1.00 | 50.19 |
| 2912 | CE | LYS | B | 790 | 47.419 | 46.859 | 53.795 | 1.00 | 53.06 |
| 2913 | NZ | LYS | B | 790 | 48.594 | 47.781 | 53.878 | 1.00 | 61.09 |
| 2914 | N | GLU | B | 791 | 41.425 | 46.397 | 52.418 | 1.00 | 42.50 |
| 2915 | CA | GLU | B | 791 | 40.097 | 46.848 | 52.848 | 1.00 | 45.42 |
| 2916 | C | GLU | B | 791 | 38.974 | 45.916 | 52.374 | 1.00 | 45.11 |
| 2917 | O | GLU | B | 791 | 37.797 | 46.271 | 52.456 | 1.00 | 46.15 |
| 2918 | CB | GLU | B | 791 | 39.812 | 48.248 | 52.281 | 1.00 | 45.54 |
| 2919 | CG | GLU | B | 791 | 40.788 | 49.343 | 52.711 | 1.00 | 51.32 |
| 2920 | CD | GLU | B | 791 | 40.477 | 50.691 | 52.075 | 1.00 | 53.09 |
| 2921 | OE1 | GLU | B | 791 | 39.310 | 51.129 | 52.147 | 1.00 | 59.60 |
| 2922 | OE2 | GLU | B | 791 | 41.400 | 51.326 | 51.520 | 1.00 | 53.82 |
| 2923 | N | SER | B | 792 | 39.324 | 44.726 | 51.897 | 1.00 | 43.25 |
| 2924 | CA | SER | B | 792 | 38.325 | 43.803 | 51.344 | 1.00 | 45.29 |
| 2925 | C | SER | B | 792 | 37.463 | 42.934 | 52.267 | 1.00 | 45.71 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2926 | O | SER | B | 792 | 36.291 | 42.696 | 51.966 | 1.00 | 48.47 |
| 2927 | CB | SER | B | 792 | 39.004 | 42.886 | 50.321 | 1.00 | 41.92 |
| 2928 | OG | SER | B | 792 | 39.956 | 42.038 | 50.946 | 1.00 | 37.83 |
| 2929 | N | SER | B | 793 | 38.060 | 42.456 | 53.359 | 1.00 | 47.99 |
| 2930 | CA | SER | B | 793 | 37.444 | 41.566 | 54.352 | 1.00 | 44.28 |
| 2931 | C | SER | B | 793 | 38.058 | 40.181 | 54.126 | 1.00 | 45.52 |
| 2932 | O | SER | B | 793 | 37.939 | 39.278 | 54.967 | 1.00 | 38.10 |
| 2933 | CB | SER | B | 793 | 35.909 | 41.484 | 54.229 | 1.00 | 52.05 |
| 2934 | OG | SER | B | 793 | 35.503 | 40.710 | 53.110 | 1.00 | 51.73 |
| 2935 | N | PHE | B | 794 | 38.709 | 40.011 | 52.975 | 1.00 | 36.75 |
| 2936 | CA | PHE | B | 794 | 39.383 | 38.752 | 52.674 | 1.00 | 34.46 |
| 2937 | C | PHE | B | 794 | 40.815 | 38.977 | 52.193 | 1.00 | 35.15 |
| 2938 | O | PHE | B | 794 | 41.285 | 38.344 | 51.245 | 1.00 | 29.12 |
| 2939 | CB | PHE | B | 794 | 38.597 | 37.897 | 51.659 | 1.00 | 34.09 |
| 2940 | CG | PHE | B | 794 | 38.136 | 38.638 | 50.435 | 1.00 | 31.82 |
| 2941 | CD1 | PHE | B | 794 | 37.005 | 39.454 | 50.480 | 1.00 | 34.38 |
| 2942 | CD2 | PHE | B | 794 | 38.807 | 38.487 | 49.227 | 1.00 | 33.70 |
| 2943 | CE1 | PHE | B | 794 | 36.546 | 40.108 | 49.336 | 1.00 | 32.42 |
| 2944 | CE2 | PHE | B | 794 | 38.358 | 39.141 | 48.070 | 1.00 | 35.10 |
| 2945 | CZ | PHE | B | 794 | 37.227 | 39.951 | 48.127 | 1.00 | 33.58 |
| 2946 | N | TYR | B | 795 | 41.503 | 39.884 | 52.884 | 1.00 | 35.15 |
| 2947 | CA | TYR | B | 795 | 42.895 | 40.227 | 52.584 | 1.00 | 36.03 |
| 2948 | C | TYR | B | 795 | 43.759 | 38.970 | 52.496 | 1.00 | 29.03 |
| 2949 | O | TYR | B | 795 | 44.546 | 38.810 | 51.558 | 1.00 | 30.92 |
| 2950 | CB | TYR | B | 795 | 43.446 | 41.148 | 53.682 | 1.00 | 33.87 |
| 2951 | CG | TYR | B | 795 | 44.860 | 41.666 | 53.462 | 1.00 | 42.87 |
| 2952 | CD1 | TYR | B | 795 | 45.405 | 41.771 | 52.179 | 1.00 | 45.74 |
| 2953 | CD2 | TYR | B | 795 | 45.636 | 42.091 | 54.540 | 1.00 | 43.44 |
| 2954 | CE1 | TYR | B | 795 | 46.689 | 42.287 | 51.979 | 1.00 | 46.16 |
| 2955 | CE2 | TYR | B | 795 | 46.914 | 42.605 | 54.353 | 1.00 | 49.66 |
| 2956 | CZ | TYR | B | 795 | 47.436 | 42.700 | 53.071 | 1.00 | 51.20 |
| 2957 | OH | TYR | B | 795 | 48.707 | 43.199 | 52.884 | 1.00 | 51.61 |
| 2958 | N | SER | B | 796 | 43.609 | 38.065 | 53.458 | 1.00 | 29.71 |
| 2959 | CA | SER | B | 796 | 44.432 | 36.855 | 53.463 | 1.00 | 33.41 |
| 2960 | C | SER | B | 796 | 44.262 | 36.003 | 52.210 | 1.00 | 34.99 |
| 2961 | O | SER | B | 796 | 45.228 | 35.433 | 51.697 | 1.00 | 30.81 |
| 2962 | CB | SER | B | 796 | 44.128 | 36.004 | 54.696 | 1.00 | 32.48 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 2963 | OG | SER | B | 796 | 42.764 | 35.641 | 54.718 | 1.00 | 46.57 |
| 2964 | N | LEU | B | 797 | 43.031 | 35.919 | 51.720 | 1.00 | 31.82 |
| 2965 | CA | LEU | B | 797 | 42.768 | 35.135 | 50.521 | 1.00 | 34.40 |
| 2966 | C | LEU | B | 797 | 43.399 | 35.854 | 49.342 | 1.00 | 27.87 |
| 2967 | O | LEU | B | 797 | 43.963 | 35.224 | 48.451 | 1.00 | 31.27 |
| 2968 | CB | LEU | B | 797 | 41.261 | 35.000 | 50.291 | 1.00 | 31.93 |
| 2969 | CG | LEU | B | 797 | 40.858 | 33.875 | 49.334 | 1.00 | 37.66 |
| 2970 | CD1 | LEU | B | 797 | 41.251 | 32.542 | 49.949 | 1.00 | 36.98 |
| 2971 | CD2 | LEU | B | 797 | 39.362 | 33.900 | 49.106 | 1.00 | 38.90 |
| 2972 | N | CYS | B | 798 | 43.292 | 37.179 | 49.337 | 1.00 | 31.24 |
| 2973 | CA | CYS | B | 798 | 43.885 | 37.980 | 48.271 | 1.00 | 30.70 |
| 2974 | C | CYS | B | 798 | 45.377 | 37.723 | 48.202 | 1.00 | 29.69 |
| 2975 | O | CYS | B | 798 | 45.912 | 37.508 | 47.120 | 1.00 | 28.98 |
| 2976 | CB | CYS | B | 798 | 43.620 | 39.468 | 48.492 | 1.00 | 30.81 |
| 2977 | SG | CYS | B | 798 | 41.896 | 39.959 | 48.184 | 1.00 | 34.74 |
| 2978 | N | LEU | B | 799 | 46.057 | 37.738 | 49.344 | 1.00 | 30.05 |
| 2979 | CA | LEU | B | 799 | 47.499 | 37.467 | 49.350 | 1.00 | 31.95 |
| 2980 | C | LEU | B | 799 | 47.804 | 36.066 | 48.826 | 1.00 | 34.28 |
| 2981 | O | LEU | B | 799 | 48.771 | 35.862 | 48.090 | 1.00 | 31.99 |
| 2982 | CB | LEU | B | 799 | 48.078 | 37.608 | 50.756 | 1.00 | 37.86 |
| 2983 | CG | LEU | B | 799 | 47.990 | 39.005 | 51.366 | 1.00 | 40.98 |
| 2984 | CD1 | LEU | B | 799 | 48.504 | 38.957 | 52.806 | 1.00 | 39.48 |
| 2985 | CD2 | LEU | B | 799 | 48.799 | 39.996 | 50.528 | 1.00 | 38.01 |
| 2986 | N | THR | B | 800 | 46.991 | 35.089 | 49.214 | 1.00 | 32.31 |
| 2987 | CA | THR | B | 800 | 47.200 | 33.722 | 48.749 | 1.00 | 31.22 |
| 2988 | C | THR | B | 800 | 47.153 | 33.664 | 47.230 | 1.00 | 28.73 |
| 2989 | O | THR | B | 800 | 47.970 | 32.990 | 46.587 | 1.00 | 31.25 |
| 2990 | CB | THR | B | 800 | 46.125 | 32.774 | 49.338 | 1.00 | 32.96 |
| 2991 | OG1 | THR | B | 800 | 46.392 | 32.603 | 50.729 | 1.00 | 37.48 |
| 2992 | CG2 | THR | B | 800 | 46.121 | 31.414 | 48.638 | 1.00 | 31.79 |
| 2993 | N | MET | B | 801 | 46.196 | 34.372 | 46.648 | 1.00 | 27.39 |
| 2994 | CA | MET | B | 801 | 46.078 | 34.376 | 45.206 | 1.00 | 25.92 |
| 2995 | C | MET | B | 801 | 47.190 | 35.190 | 44.553 | 1.00 | 28.37 |
| 2996 | O | MET | B | 801 | 47.715 | 34.804 | 43.516 | 1.00 | 26.82 |
| 2997 | CB | MET | B | 801 | 44.725 | 34.938 | 44.794 | 1.00 | 30.43 |
| 2998 | CG | MET | B | 801 | 43.567 | 33.998 | 45.053 | 1.00 | 25.00 |
| 2999 | SD | MET | B | 801 | 42.026 | 34.755 | 44.543 | 1.00 | 39.28 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3000 | CE | MET | B | 801 | 41.910 | 36.095 | 45.721 | 1.00 | 31.74 |
| 3001 | N | TRP | B | 802 | 47.555 | 36.301 | 45.180 | 1.00 | 27.61 |
| 3002 | CA | TRP | B | 802 | 48.574 | 37.204 | 44.656 | 1.00 | 29.01 |
| 3003 | C | TRP | B | 802 | 49.942 | 36.527 | 44.503 | 1.00 | 31.69 |
| 3004 | O | TRP | B | 802 | 50.812 | 37.016 | 43.773 | 1.00 | 31.46 |
| 3005 | CB | TRP | B | 802 | 48.640 | 38.429 | 45.574 | 1.00 | 28.86 |
| 3006 | CG | TRP | B | 802 | 49.311 | 39.628 | 45.007 | 1.00 | 29.63 |
| 3007 | CD1 | TRP | B | 802 | 49.528 | 39.914 | 43.686 | 1.00 | 30.48 |
| 3008 | CD2 | TRP | B | 802 | 49.716 | 40.781 | 45.738 | 1.00 | 27.02 |
| 3009 | NE1 | TRP | B | 802 | 50.040 | 41.184 | 43.554 | 1.00 | 26.74 |
| 3010 | CE2 | TRP | B | 802 | 50.163 | 41.738 | 44.800 | 1.00 | 30.15 |
| 3011 | CE3 | TRP | B | 802 | 49.740 | 41.102 | 47.100 | 1.00 | 27.81 |
| 3012 | CZ2 | TRP | B | 802 | 50.630 | 42.990 | 45.182 | 1.00 | 29.45 |
| 3013 | CZ3 | TRP | B | 802 | 50.207 | 42.356 | 47.479 | 1.00 | 31.99 |
| 3014 | CH2 | TRP | B | 802 | 50.643 | 43.282 | 46.521 | 1.00 | 30.34 |
| 3015 | N | GLN | B | 803 | 50.118 | 35.386 | 45.164 | 1.00 | 31.67 |
| 3016 | CA | GLN | B | 803 | 51.371 | 34.641 | 45.081 | 1.00 | 33.58 |
| 3017 | C | GLN | B | 803 | 51.638 | 34.097 | 43.676 | 1.00 | 37.24 |
| 3018 | O | GLN | B | 803 | 52.787 | 33.878 | 43.293 | 1.00 | 33.37 |
| 3019 | CB | GLN | B | 803 | 51.365 | 33.465 | 46.061 | 1.00 | 35.29 |
| 3020 | CG | GLN | B | 803 | 51.335 | 33.839 | 47.539 | 1.00 | 45.49 |
| 3021 | CD | GLN | B | 803 | 51.288 | 32.611 | 48.447 | 1.00 | 46.24 |
| 3022 | OE1 | GLN | B | 803 | 52.138 | 31.728 | 48.355 | 1.00 | 55.28 |
| 3023 | NE2 | GLN | B | 803 | 50.293 | 32.557 | 49.327 | 1.00 | 53.88 |
| 3024 | N | ILE | B | 804 | 50.587 | 33.867 | 42.900 | 1.00 | 27.44 |
| 3025 | CA | ILE | B | 804 | 50.789 | 33.320 | 41.567 | 1.00 | 28.71 |
| 3026 | C | ILE | B | 804 | 51.477 | 34.279 | 40.612 | 1.00 | 22.83 |
| 3027 | O | ILE | B | 804 | 52.439 | 33.906 | 39.951 | 1.00 | 28.57 |
| 3028 | CB | ILE | B | 804 | 49.454 | 32.854 | 40.947 | 1.00 | 24.30 |
| 3029 | CG1 | ILE | B | 804 | 48.854 | 31.748 | 41.819 | 1.00 | 30.72 |
| 3030 | CG2 | ILE | B | 804 | 49.692 | 32.334 | 39.531 | 1.00 | 32.39 |
| 3031 | CD1 | ILE | B | 804 | 47.479 | 31.293 | 41.391 | 1.00 | 28.78 |
| 3032 | N | PRO | B | 805 | 50.969 | 35.514 | 40.493 | 1.00 | 26.89 |
| 3033 | CA | PRO | B | 805 | 51.627 | 36.449 | 39.577 | 1.00 | 29.13 |
| 3034 | C | PRO | B | 805 | 53.055 | 36.778 | 40.018 | 1.00 | 33.32 |
| 3035 | O | PRO | B | 805 | 53.924 | 37.035 | 39.193 | 1.00 | 32.54 |
| 3036 | CB | PRO | B | 805 | 50.684 | 37.654 | 39.588 | 1.00 | 31.35 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3037 | CG | PRO | B | 805 | 50.099 | 37.602 | 40.969 | 1.00 | 34.40 |
| 3038 | CD | PRO | B | 805 | 49.762 | 36.125 | 41.070 | 1.00 | 27.20 |
| 3039 | N | GLN | B | 806 | 53.302 | 36.745 | 41.319 | 1.00 | 32.34 |
| 3040 | CA | GLN | B | 806 | 54.637 | 37.050 | 41.809 | 1.00 | 36.70 |
| 3041 | C | GLN | B | 806 | 55.569 | 35.922 | 41.367 | 1.00 | 39.30 |
| 3042 | O | GLN | B | 806 | 56.774 | 36.124 | 41.175 | 1.00 | 37.37 |
| 3043 | CB | GLN | B | 806 | 54.623 | 37.180 | 43.338 | 1.00 | 37.55 |
| 3044 | CG | GLN | B | 806 | 53.654 | 38.230 | 43.899 | 1.00 | 40.88 |
| 3045 | CD | GLN | B | 806 | 53.948 | 39.660 | 43.444 | 1.00 | 48.76 |
| 3046 | OE1 | GLN | B | 806 | 53.279 | 40.605 | 43.870 | 1.00 | 53.24 |
| 3047 | NE2 | GLN | B | 806 | 54.943 | 39.824 | 42.583 | 1.00 | 42.24 |
| 3048 | N | GLU | B | 807 | 54.999 | 34.735 | 41.178 | 1.00 | 32.14 |
| 3049 | CA | GLU | B | 807 | 55.791 | 33.593 | 40.764 | 1.00 | 35.89 |
| 3050 | C | GLU | B | 807 | 56.033 | 33.651 | 39.260 | 1.00 | 32.99 |
| 3051 | O | GLU | B | 807 | 57.071 | 33.198 | 38.781 | 1.00 | 34.50 |
| 3052 | CB | GLU | B | 807 | 55.086 | 32.293 | 41.150 | 1.00 | 40.93 |
| 3053 | CG | GLU | B | 807 | 55.989 | 31.087 | 41.147 | 1.00 | 44.91 |
| 3054 | CD | GLU | B | 807 | 57.191 | 31.280 | 42.051 | 1.00 | 55.41 |
| 3055 | OE1 | GLU | B | 807 | 57.002 | 31.627 | 43.239 | 1.00 | 58.81 |
| 3056 | OE2 | GLU | B | 807 | 58.327 | 31.082 | 41.575 | 1.00 | 62.55 |
| 3057 | N | PHE | B | 808 | 55.076 | 34.203 | 38.517 | 1.00 | 32.23 |
| 3058 | CA | PHE | B | 808 | 55.230 | 34.345 | 37.068 | 1.00 | 29.01 |
| 3059 | C | PHE | B | 808 | 56.333 | 35.382 | 36.832 | 1.00 | 31.50 |
| 3060 | O | PHE | B | 808 | 57.128 | 35.246 | 35.914 | 1.00 | 32.70 |
| 3061 | CB | PHE | B | 808 | 53.933 | 34.841 | 36.420 | 1.00 | 29.87 |
| 3062 | CG | PHE | B | 808 | 52.870 | 33.786 | 36.279 | 1.00 | 29.41 |
| 3063 | CD1 | PHE | B | 808 | 53.146 | 32.454 | 36.546 | 1.00 | 34.35 |
| 3064 | CD2 | PHE | B | 808 | 51.594 | 34.135 | 35.857 | 1.00 | 30.60 |
| 3065 | CE1 | PHE | B | 808 | 52.156 | 31.472 | 36.395 | 1.00 | 40.92 |
| 3066 | CE2 | PHE | B | 808 | 50.599 | 33.174 | 35.701 | 1.00 | 27.80 |
| 3067 | CZ | PHE | B | 808 | 50.877 | 31.842 | 35.970 | 1.00 | 29.99 |
| 3068 | N | VAL | B | 809 | 56.366 | 36.420 | 37.665 | 1.00 | 36.56 |
| 3069 | CA | VAL | B | 809 | 57.391 | 37.459 | 37.543 | 1.00 | 36.76 |
| 3070 | C | VAL | B | 809 | 58.743 | 36.800 | 37.770 | 1.00 | 40.16 |
| 3071 | O | VAL | B | 809 | 59.673 | 36.949 | 36.965 | 1.00 | 40.88 |
| 3072 | CB | VAL | B | 809 | 57.190 | 38.585 | 38.592 | 1.00 | 38.00 |
| 3073 | CG1 | VAL | B | 809 | 58.409 | 39.520 | 38.615 | 1.00 | 39.88 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3074 | CG2 | VAL | B | 809 | 55.944 | 39.390 | 38.251 | 1.00 | 34.92 |
| 3075 | N | LYS | B | 810 | 58.831 | 36.044 | 38.857 | 1.00 | 35.81 |
| 3076 | CA | LYS | B | 810 | 60.059 | 35.365 | 39.223 | 1.00 | 39.66 |
| 3077 | C | LYS | B | 810 | 60.589 | 34.395 | 38.176 | 1.00 | 40.69 |
| 3078 | O | LYS | B | 810 | 61.783 | 34.396 | 37.887 | 1.00 | 39.53 |
| 3079 | CB | LYS | B | 810 | 59.875 | 34.633 | 40.549 | 1.00 | 44.11 |
| 3080 | CG | LYS | B | 810 | 61.103 | 33.854 | 40.995 | 1.00 | 50.84 |
| 3081 | CD | LYS | B | 810 | 60.917 | 33.255 | 42.385 | 1.00 | 58.43 |
| 3082 | CE | LYS | B | 810 | 60.773 | 34.341 | 43.448 | 1.00 | 56.52 |
| 3083 | NZ | LYS | B | 810 | 60.552 | 33.768 | 44.812 | 1.00 | 61.40 |
| 3084 | N | LEU | B | 811 1 | 59.707 | 33.570 | 37.612 | 1.00 | 37.80 |
| 3085 | CA | LEU | B | 811 | 60.107 | 32.596 | 36.601 | 1.00 | 38.18 |
| 3086 | C | LEU | B | 811 | 60.127 | 33.145 | 35.183 | 1.00 | 37.73 |
| 3087 | O | LEU | B | 811 | 60.578 | 32.469 | 34.258 | 1.00 | 37.38 |
| 3088 | CB | LEU | B | 811 1 | 59.168 | 31.392 | 36.631 | 1.00 | 41.71 |
| 3089 | CG | LEU | B | 811 | 59.188 | 30.533 | 37.889 | 1.00 | 43.91 |
| 3090 | CD1 | LEU | B | 811 | 58.114 | 29.464 | 37.790 | 1.00 | 47.64 |
| 3091 | CD2 | LEU | B | 811 | 60.562 | 29.901 | 38.041 | 1.00 | 47.76 |
| 3092 | N | GLN | B | 812 | 59.627 | 34.362 | 35.012 | 1.00 | 36.65 |
| 3093 | CA | GLN | B | 812 | 59.564 | 34.979 | 33.693 | 1.00 | 38.46 |
| 3094 | C | GLN | B | 812 | 58.839 | 34.031 | 32.724 | 1.00 | 35.35 |
| 3095 | O | GLN | B | 812 | 59.291 | 33.786 | 31.607 | 1.00 | 33.66 |
| 3096 | CB | GLN | B | 812 | 60.983 | 35.281 | 33.206 | 1.00 | 46.92 |
| 3097 | CG | GLN | B | 812 | 61.763 | 36.139 | 34.196 | 1.00 | 52.42 |
| 3098 | CD | GLN | B | 812 | 63.241 | 36.277 | 33.852 | 1.00 | 62.75 |
| 3099 | OE1 | GLN | B | 812 | 63.995 | 36.928 | 34.579 | 1.00 | 62.90 |
| 3100 | NE2 | GLN | B | 812 | 63.661 | 35.662 | 32.746 | 1.00 | 62.19 |
| 3101 | N | VAL | B | 813 | 57.706 | 33.496 | 33.166 | 1.00 | 33.05 |
| 3102 | CA | VAL | B | 813 | 56.928 | 32.577 | 32.334 | 1.00 | 30.09 |
| 3103 | C | VAL | B | 813 | 56.559 | 33.219 | 31.005 | 1.00 | 26.61 |
| 3104 | O | VAL | B | 813 | 56.136 | 34.374 | 30.948 | 1.00 | 27.45 |
| 3105 | CB | VAL | B | 813 | 55.645 | 32.126 | 33.063 | 1.00 | 32.65 |
| 3106 | CG1 | VAL | B | 813 | 54.816 | 31.198 | 32.158 | 1.00 | 32.05 |
| 3107 | CG2 | VAL | B | 813 | 56.024 | 31.405 | 34.332 | 1.00 | 31.32 |
| 3108 | N | SER | B | 814 | 56.745 | 32.469 | 29.924 | 1.00 | 28.74 |
| 3109 | CA | SER | B | 814 | 56.415 | 32.982 | 28.599 | 1.00 | 26.63 |
| 3110 | C | SER | B | 814 | 54.972 | 32.609 | 28.307 | 1.00 | 25.32 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3111 | O | SER | B | 814 | 54.413 | 31.720 | 28.945 | 1.00 | 26.17 |
| 3112 | CB | SER | B | 814 | 57.291 | 32.338 | 27.537 | 1.00 | 20.76 |
| 3113 | OG | SER | B | 814 | 57.087 | 30.930 | 27.530 | 1.00 | 32.55 |
| 3114 | N | GLN | B | 815 | 54.387 | 33.290 | 27.332 | 1.00 | 29.06 |
| 3115 | CA | GLN | B | 815 | 53.014 | 33.031 | 26.931 | 1.00 | 30.84 |
| 3116 | C | GLN | B | 815 | 52.858 | 31.597 | 26.438 | 1.00 | 30.78 |
| 3117 | O | GLN | B | 815 | 51.800 | 30.983 | 26.614 | 1.00 | 29.76 |
| 3118 | CB | GLN | B | 815 | 52.613 | 34.010 | 25.825 | 1.00 | 33.76 |
| 3119 | CG | GLN | B | 815 | 51.168 | 33.883 | 25.367 | 1.00 | 48.56 |
| 3120 | CD | GLN | B | 815 | 50.791 | 34.935 | 24.337 | 1.00 | 53.59 |
| 3121 | OE1 | GLN | B | 815 | 50.880 | 36.139 | 24.595 | 1.00 | 61.73 |
| 3122 | NE2 | GLN | B | 815 | 50.370 | 34.487 | 23.167 | 1.00 | 53.05 |
| 3123 | N | GLU | B | 816 | 53.914 | 31.062 | 25.821 | 1.00 | 28.64 |
| 3124 | CA | GLU | B | 816 | 53.886 | 29.709 | 25.294 | 1.00 | 26.48 |
| 3125 | C | GLU | B | 816 | 53.873 | 28.657 | 26.400 | 1.00 | 28.37 |
| 3126 | O | GLU | B | 816 | 53.181 | 27.647 | 26.297 | 1.00 | 26.00 |
| 3127 | CB | GLU | B | 816 | 55.090 | 29.464 | 24.370 | 1.00 | 28.52 |
| 3128 | CG | GLU | B | 816 | 55.187 | 30.379 | 23.135 | 1.00 | 30.85 |
| 3129 | CD | GLU | B | 816 | 55.387 | 31.853 | 23.474 | 1.00 | 40.74 |
| 3130 | OE1 | GLU | B | 816 | 56.191 | 32.155 | 24.380 | 1.00 | 35.62 |
| 3131 | OE2 | GLU | B | 816 | 54.756 | 32.718 | 22.821 | 1.00 | 48.10 |
| 3132 | N | GLU | B | 817 | 54.646 | 28.875 | 27.458 | 1.00 | 28.18 |
| 3133 | CA | GLU | B | 817 | 54.679 | 27.908 | 28.561 | 1.00 | 22.67 |
| 3134 | C | GLU | B | 817 | 53.334 | 27.952 | 29.308 | 1.00 | 26.65 |
| 3135 | O | GLU | B | 817 | 52.798 | 26.912 | 29.718 | 1.00 | 27.82 |
| 3136 | CB | GLU | B | 817 | 55.827 | 28.253 | 29.508 | 1.00 | 31.49 |
| 3137 | CG | GLU | B | 817 | 57.197 | 28.222 | 28.815 | 1.00 | 30.28 |
| 3138 | CD | GLU | B | 817 | 58.301 | 28.832 | 29.664 | 1.00 | 41.20 |
| 3139 | OE1 | GLU | B | 817 | 58.072 | 29.918 | 30.242 | 1.00 | 33.70 |
| 3140 | OE2 | GLU | B | 817 | 59.403 | 28.243 | 29.730 | 1.00 | 35.22 |
| 3141 | N | PHE | B | 818 | 52.812 | 29.162 | 29.470 | 1.00 | 22.36 |
| 3142 | CA | PHE | B | 818 | 51.535 | 29.410 | 30.140 | 1.00 | 25.25 |
| 3143 | C | PHE | B | 818 | 50.392 | 28.641 | 29.484 | 1.00 | 24.77 |
| 3144 | O | PHE | B | 818 | 49.610 | 27.981 | 30.167 | 1.00 | 24.06 |
| 3145 | CB | PHE | B | 818 | 51.196 | 30.903 | 30.087 | 1.00 | 22.70 |
| 3146 | CG | PHE | B | 818 | 49.781 | 31.227 | 30.526 | 1.00 | 24.41 |
| 3147 | CD1 | PHE | B | 818 | 49.388 | 31.042 | 31.845 | 1.00 | 24.27 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3148 | CD2 | PHE | B | 818 | 48.846 | 31.703 | 29.612 | 1.00 | 29.17 |
| 3149 | CE1 | PHE | B | 818 | 48.077 | 31.321 | 32.256 | 1.00 | 27.63 |
| 3150 | CE2 | PHE | B | 818 | 47.530 | 31.985 | 30.006 | 1.00 | 31.24 |
| 3151 | CZ | PHE | B | 818 | 47.147 | 31.794 | 31.329 | 1.00 | 27.17 |
| 3152 | N | LEU | B | 819 | 50.299 | 28.728 | 28.158 | 1.00 | 26.67 |
| 3153 | CA | LEU | B | 819 | 49.220 | 28.053 | 27.434 | 1.00 | 21.35 |
| 3154 | C | LEU | B | 819 | 49.221 | 26.540 | 27.594 | 1.00 | 30.98 |
| 3155 | O | LEU | B | 819 | 48.158 | 25.926 | 27.736 | 1.00 | 23.62 |
| 3156 | CB | LEU | B | 819 | 49.259 | 28.437 | 25.953 | 1.00 | 29.60 |
| 3157 | CG | LEU | B | 819 | 48.936 | 29.911 1 | 25.692 | 1.00 | 22.84 |
| 3158 | CD1 | LEU | B | 819 | 49.334 | 30.306 | 24.285 | 1.00 | 30.19 |
| 3159 | CD2 | LEU | B | 819 | 47.459 | 30.156 | 25.931 | 1.00 | 31.22 |
| 3160 | N | CYS | B | 820 | 50.399 | 25.931 | 27.572 | 1.00 | 22.63 |
| 3161 | CA | CYS | B | 820 | 50.491 | 24.489 | 27.755 | 1.00 | 28.50 |
| 3162 | C | CYS | B | 820 | 50.171 | 24.127 | 29.200 | 1.00 | 26.00 |
| 3163 | O | CYS | B | 820 | 49.458 | 23.144 | 29.465 | 1.00 | 24.25 |
| 3164 | CB | CYS | B | 820 | 51.896 | 23.993 | 27.406 | 1.00 | 27.91 |
| 3165 | SG | CYS | B | 820 | 52.317 | 24.170 | 25.661 | 1.00 | 32.47 |
| 3166 | N | MET | B | 821 | 50.707 | 24.913 | 30.135 | 1.00 | 22.36 |
| 3167 | CA | MET | B | 821 | 50.483 | 24.654 | 31.556 | 1.00 | 21.33 |
| 3168 | C | MET | B | 821 | 49.015 | 24.778 | 31.898 | 1.00 | 26.26 |
| 3169 | O | MET | B | 821 | 48.503 | 24.021 | 32.715 | 1.00 | 25.87 |
| 3170 | CB | MET | B | 821 | 51.278 | 25.626 | 32.433 | 1.00 | 26.99 |
| 3171 | CG | MET | B | 821 | 52.782 | 25.421 | 32.382 | 1.00 | 28.60 |
| 3172 | SD | MET | B | 821 | 53.621 | 26.820 | 33.184 | 1.00 | 34.96 |
| 3173 | CE | MET | B | 821 | 53.294 | 26.479 | 34.906 | 1.00 | 34.63 |
| 3174 | N | LYS | B | 822 | 48.334 | 25.721 | 31.267 | 1.00 | 22.01 |
| 3175 | CA | LYS | B | 822 | 46.923 | 25.884 | 31.562 | 1.00 | 24.77 |
| 3176 | C | LYS | B | 822 | 46.135 | 24.647 | 31.126 | 1.00 | 24.24 |
| 3177 | O | LYS | B | 822 | 45.188 | 24.221 | 31.804 | 1.00 | 20.06 |
| 3178 | CB | LYS | B | 822 | 46.396 | 27.164 | 30.901 | 1.00 | 22.46 |
| 3179 | CG | LYS | B | 822 | 44.991 | 27.512 | 31.372 | 1.00 | 25.45 |
| 3180 | CD | LYS | B | 822 | 44.675 | 28.996 | 31.201 | 1.00 | 34.91 |
| 3181 | CE | LYS | B | 822 | 44.712 | 29.433 | 29.753 | 1.00 | 38.39 |
| 3182 | NZ | LYS | B | 822 | 43.654 | 28.750 | 28.981 | 1.00 | 37.51 |
| 3183 | N | VAL | B | 823 | 46.532 | 24.043 | 30.007 | 1.00 | 22.51 |
| 3184 | CA | VAL | B | 823 | 45.852 | 22.831 | 29.546 | 1.00 | 19.35 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3185 | C | VAL | B | 823 | 46.140 | 21.700 | 30.511 1 | 1.00 | 20.40 |
| 3186 | O | VAL | B | 823 | 45.266 | 20.879 | 30.822 | 1.00 | 18.38 |
| 3187 | CB | VAL | B | 823 | 46.316 | 22.387 | 28.147 | 1.00 | 17.40 |
| 3188 | CG1 | VAL | B | 823 | 45.663 | 21.037 | 27.785 | 1.00 | 23.17 |
| 3189 | CG2 | VAL | B | 823 | 45.910 | 23.426 | 27.129 | 1.00 | 25.04 |
| 3190 | N | LEU | B | 824 | 47.372 | 21.657 | 31.012 | 1.00 | 20.55 |
| 3191 | CA | LEU | B | 824 | 47.705 | 20.611 | 31.951 | 1.00 | 21.76 |
| 3192 | C | LEU | B | 824 | 46.874 | 20.734 | 33.230 | 1.00 | 19.28 |
| 3193 | O | LEU | B | 824 | 46.540 | 19.721 | 33.837 | 1.00 | 22.64 |
| 3194 | CB | LEU | B | 824 | 49.210 | 20.610 | 32.245 | 1.00 | 22.31 |
| 3195 | CG | LEU | B | 824 | 50.058 | 20.144 | 31.047 | 1.00 | 22.09 |
| 3196 | CD1 | LEU | B | 824 | 51.548 | 20.248 | 31.388 | 1.00 | 32.05 |
| 3197 | CD2 | LEU | B | 824 | 49.668 | 18.707 | 30.684 | 1.00 | 23.33 |
| 3198 | N | LEU | B | 825 | 46.511 | 21.956 | 33.619 | 1.00 | 21.54 |
| 3199 | CA | LEU | B | 825 | 45.684 | 22.134 | 34.814 | 1.00 | 21.16 |
| 3200 | C | LEU | B | 825 | 44.304 | 21.500 | 34.594 | 1.00 | 22.24 |
| 3201 | O | LEU | B | 825 | 43.757 | 20.858 | 35.483 | 1.00 | 20.01 |
| 3202 | CB | LEU | B | 825 | 45.547 | 23.614 | 35.164 | 1.00 | 19.34 |
| 3203 | CG | LEU | B | 825 | 46.814 | 24.203 | 35.786 | 1.00 | 22.57 |
| 3204 | CD1 | LEU | B | 825 | 46.629 | 25.698 | 36.063 | 1.00 | 22.91 |
| 3205 | CD2 | LEU | B | 825 | 47.101 | 23.450 | 37.096 | 1.00 | 22.94 |
| 3206 | N | LEU | B | 826 | 43.750 | 21.674 | 33.403 | 1.00 | 21.69 |
| 3207 | CA | LEU | B | 826 | 42.465 | 21.067 | 33.072 | 1.00 | 21.95 |
| 3208 | C | LEU | B | 826 | 42.544 | 19.533 | 33.192 | 1.00 | 24.10 |
| 3209 | O | LEU | B | 826 | 41.552 | 18.865 | 33.499 | 1.00 | 23.49 |
| 3210 | CB | LEU | B | 826 | 42.072 | 21.462 | 31.637 | 1.00 | 20.89 |
| 3211 1 | CG | LEU | B | 826 | 40.832 | 20.808 | 31.017 | 1.00 | 23.40 |
| 3212 | CD1 | LEU | B | 826 | 39.543 | 21.272 | 31.757 | 1.00 | 21.60 |
| 3213 | CD2 | LEU | B | 826 | 40.759 | 21.198 | 29.532 | 1.00 | 21.37 |
| 3214 | N | LEU | B | 827 | 43.727 | 18.973 | 32.953 | 1.00 | 21.74 |
| 3215 | CA | LEU | B | 827 | 43.902 | 17.531 | 32.999 | 1.00 | 26.22 |
| 3216 | C | LEU | B | 827 | 44.732 | 17.087 | 34.208 | 1.00 | 26.34 |
| 3217 | O | LEU | B | 827 | 45.374 | 16.041 | 34.157 | 1.00 | 27.08 |
| 3218 | CB | LEU | B | 827 | 44.620 | 17.081 | 31.715 | 1.00 | 23.15 |
| 3219 | CG | LEU | B | 827 | 44.092 | 17.626 | 30.388 | 1.00 | 25.05 |
| 3220 | CD1 | LEU | B | 827 | 45.027 | 17.213 | 29.229 | 1.00 | 23.87 |
| 3221 | CD2 | LEU | B | 827 | 42.672 | 17.097 | 30.180 | 1.00 | 24.70 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3222 | N | ASN | B | 828 | 44.702 | 17.855 | 35.295 | 1.00 | 26.63 |
| 3223 | CA | ASN | B | 828 | 45.531 | 17.537 | 36.466 | 1.00 | 23.58 |
| 3224 | C | ASN | B | 828 | 44.887 | 16.756 | 37.625 | 1.00 | 26.70 |
| 3225 | O | ASN | B | 828 | 45.534 | 16.519 | 38.639 | 1.00 | 25.23 |
| 3226 | CB | ASN | B | 828 | 46.130 | 18.838 | 37.025 | 1.00 | 28.11 |
| 3227 | CG | ASN | B | 828 | 47.592 | 18.685 | 37.418 | 1.00 | 37.40 |
| 3228 | OD1 | ASN | B | 828 | 48.159 | 19.529 | 38.113 | 1.00 | 38.18 |
| 3229 | ND2 | ASN | B | 828 | 48.213 | 17.617 | 36.947 | 1.00 | 33.20 |
| 3230 | N | THR | B | 829 | 43.621 | 16.377 | 37.499 | 1.00 | 23.98 |
| 3231 | CA | THR | B | 829 | 42.938 | 15.622 | 38.552 | 1.00 | 23.70 |
| 3232 | C | THR | B | 829 | 41.947 | 14.683 | 37.866 | 1.00 | 25.10 |
| 3233 | O | THR | B | 829 | 41.250 | 15.100 | 36.952 | 1.00 | 25.89 |
| 3234 | CB | THR | B | 829 | 42.133 | 16.555 | 39.496 | 1.00 | 24.60 |
| 3235 | OG1 | THR | B | 829 | 42.984 | 17.586 | 40.006 | 1.00 | 28.70 |
| 3236 | CG2 | THR | B | 829 | 41.557 | 15.760 | 40.661 | 1.00 | 28.67 |
| 3237 | N | ILE | B | 830 | 41.887 | 13.427 | 38.295 | 1.00 | 22.29 |
| 3238 | CA | ILE | B | 830 | 40.969 | 12.442 | 37.715 | 1.00 | 24.53 |
| 3239 | C | ILE | B | 830 | 40.210 | 11.721 | 38.848 | 1.00 | 24.95 |
| 3240 | O | ILE | B | 830 | 40.627 | 11.783 | 40.005 | 1.00 | 20.63 |
| 3241 | CB | ILE | B | 830 | 41.795 | 11.428 | 36.877 | 1.00 | 28.68 |
| 3242 | CG1 | ILE | B | 830 | 42.571 | 12.177 | 35.799 | 1.00 | 30.48 |
| 3243 | CG2 | ILE | B | 830 | 40.913 | 10.426 | 36.194 | 1.00 | 36.11 |
| 3244 | CD1 | ILE | B | 830 | 41.689 | 12.930 | 34.851 | 1.00 | 43.97 |
| 3245 | N | PRO | B | 831 | 39.067 | 11.070 | 38.544 | 1.00 | 23.25 |
| 3246 | CA | PRO | B | 831 | 38.351 | 10.377 | 39.616 | 1.00 | 24.91 |
| 3247 | C | PRO | B | 831 | 39.232 | 9.258 | 40.168 | 1.00 | 31.60 |
| 3248 | O | PRO | B | 831 | 40.238 | 8.897 | 39.558 | 1.00 | 30.37 |
| 3249 | CB | PRO | B | 831 | 37.122 | 9.822 | 38.900 | 1.00 | 29.20 |
| 3250 | CG | PRO | B | 831 | 36.909 | 10.831 | 37.777 | 1.00 | 27.01 |
| 3251 | CD | PRO | B | 831 | 38.324 | 10.924 | 37.277 | 1.00 | 22.94 |
| 3252 | N | LEU | B | 832 | 38.863 | 8.724 | 41.325 | 1.00 | 29.91 |
| 3253 | CA | LEU | B | 832 | 39.632 | 7.630 | 41.911 | 1.00 | 35.35 |
| 3254 | C | LEU | B | 832 | 39.541 | 6.396 | 41.017 | 1.00 | 34.24 |
| 3255 | O | LEU | B | 832 | 40.499 | 5.638 | 40.898 | 1.00 | 42.02 |
| 3256 | CB | LEU | B | 832 | 39.103 | 7.304 | 43.312 | 1.00 | 31.75 |
| 3257 | CG | LEU | B | 832 | 39.307 | 8.462 | 44.291 | 1.00 | 37.36 |
| 3258 | CD1 | LEU | B | 832 | 38.703 | 8.121 | 45.640 | 1.00 | 38.79 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3259 | CD2 | LEU | B | 832 | 40.793 | 8.762 | 44.419 | 1.00 | 38.51 |
| 3260 | N | GLU | B | 833 | 38.394 | 6.223 | 40.369 | 1.00 | 37.07 |
| 3261 | CA | GLU | B | 833 | 38.157 | 5.079 | 39.495 | 1.00 | 40.48 |
| 3262 | C | GLU | B | 833 | 38.648 | 5.346 | 38.078 | 1.00 | 36.29 |
| 3263 | O | GLU | B | 833 | 38.431 | 4.532 | 37.177 | 1.00 | 39.85 |
| 3264 | CB | GLU | B | 833 | 36.656 | 4.756 | 39.435 | 1.00 | 43.67 |
| 3265 | CG | GLU | B | 833 | 35.832 | 5.259 | 40.620 | 1.00 | 59.82 |
| 3266 | CD | GLU | B | 833 | 35.634 | 6.772 | 40.595 | 1.00 | 59.36 |
| 3267 | OE1 | GLU | B | 833 | 34.979 | 7.261 | 39.652 | 1.00 | 65.43 |
| 3268 | OE2 | GLU | B | 833 | 36.135 | 7.471 | 41.502 | 1.00 | 61.15 |
| 3269 | N | GLY | B | 834 | 39.307 | 6.481 | 37.880 | 1.00 | 33.66 |
| 3270 | CA | GLY | B | 834 | 39.783 | 6.828 | 36.558 | 1.00 | 32.35 |
| 3271 | C | GLY | B | 834 | 38.640 | 7.338 | 35.694 | 1.00 | 29.12 |
| 3272 | O | GLY | B | 834 | 37.489 | 7.379 | 36.128 | 1.00 | 28.18 |
| 3273 | N | LEU | B | 835 | 38.954 | 7.719 | 34.468 | 1.00 | 28.73 |
| 3274 | CA | LEU | B | 835 | 37.947 | 8.228 | 33.532 | 1.00 | 31.82 |
| 3275 | C | LEU | B | 835 | 37.397 | 7.131 | 32.613 | 1.00 | 30.35 |
| 3276 | O | LEU | B | 835 | 37.987 | 6.058 | 32.503 | 1.00 | 33.32 |
| 3277 | CB | LEU | B | 835 | 38.579 | 9.312 | 32.671 | 1.00 | 25.42 |
| 3278 | CG | LEU | B | 835 | 39.101 | 10.535 | 33.411 | 1.00 | 28.35 |
| 3279 | CD1 | LEU | B | 835 | 40.019 | 11.335 | 32.491 | 1.00 | 26.14 |
| 3280 | CD2 | LEU | B | 835 | 37.935 | 11.374 | 33.882 | 1.00 | 25.47 |
| 3281 | N | ARG | B | 836 | 36.283 | 7.405 | 31.938 | 1.00 | 32.23 |
| 3282 | CA | ARG | B | 836 | 35.721 | 6.422 | 31.012 | 1.00 | 33.49 |
| 3283 | C | ARG | B | 836 | 36.602 | 6.367 | 29.764 | 1.00 | 32.90 |
| 3284 | O | ARG | B | 836 | 36.700 | 5.328 | 29.102 | 1.00 | 35.77 |
| 3285 | CB | ARG | B | 836 | 34.290 | 6.787 | 30.629 | 1.00 | 32.58 |
| 3286 | CG | ARG | B | 836 | 33.302 | 6.699 | 31.780 | 1.00 | 45.22 |
| 3287 | CD | ARG | B | 836 | 31.880 | 6.883 | 31.280 | 1.00 | 50.46 |
| 3288 | NE | ARG | B | 836 | 31.554 | 5.891 | 30.254 | 1.00 | 63.72 |
| 3289 | CZ | ARG | B | 836 | 30.366 | 5.771 | 29.671 | 1.00 | 61.91 |
| 3290 | NH1 | ARG | B | 836 | 29.372 | 6.586 | 30.004 | 1.00 | 64.45 |
| 3291 | NH2 | ARG | B | 836 | 30.173 | 4.837 | 28.748 | 1.00 | 68.06 |
| 3292 | N | SER | B | 837 | 37.237 | 7.490 | 29.445 | 1.00 | 27.88 |
| 3293 | CA | SER | B | 837 | 38.130 | 7.577 | 28.297 | 1.00 | 30.32 |
| 3294 | C | SER | B | 837 | 39.564 | 7.712 | 28.814 | 1.00 | 29.09 |
| 3295 | O | SER | B | 837 | 40.341 | 8.538 | 28.353 | 1.00 | 25.31 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3296 | CB | SER | B | 837 | 37.758 | 8.785 | 27.431 | 1.00 | 33.53 |
| 3297 | OG | SER | B | 837 | 36.408 | 8.698 | 26.991 | 1.00 | 32.13 |
| 3298 | N | GLN | B | 838 | 39.912 | 6.867 | 29.772 | 1.00 | 32.66 |
| 3299 | CA | GLN | B | 838 | 41.242 | 6.907 | 30.355 | 1.00 | 30.97 |
| 3300 | C | GLN | B | 838 | 42.377 | 6.811 | 29.330 | 1.00 | 32.88 |
| 3301 | O | GLN | B | 838 | 43.378 | 7.515 | 29.448 | 1.00 | 29.00 |
| 3302 | CB | GLN | B | 838 | 41.392 | 5.798 | 31.402 | 1.00 | 37.64 |
| 3303 | CG | GLN | B | 838 | 42.645 | 5.953 | 32.253 | 1.00 | 34.77 |
| 3304 | CD | GLN | B | 838 | 42.619 | 7.213 | 33.117 | 1.00 | 42.70 |
| 3305 | OE1 | GLN | B | 838 | 43.665 | 7.713 | 33.533 | 1.00 | 43.05 |
| 3306 | NE2 | GLN | B | 838 | 41.424 | 7.718 | 33.404 | 1.00 | 40.24 |
| 3307 | N | THR | B | 839 | 42.236 | 5.957 | 28.320 | 1.00 | 32.29 |
| 3308 | CA | THR | B | 839 | 43.291 | 5.829 | 27.321 | 1.00 | 32.08 |
| 3309 | C | THR | B | 839 | 43.495 | 7.122 | 26.538 | 1.00 | 30.37 |
| 3310 | O | THR | B | 839 | 44.623 | 7.583 | 26.369 | 1.00 | 27.02 |
| 3311 1 | CB | THR | B | 839 | 42.994 | 4.685 | 26.316 | 1.00 | 36.84 |
| 3312 | OG1 | THR | B | 839 | 42.914 | 3.442 | 27.021 | 1.00 | 33.01 |
| 3313 | CG2 | THR | B | 839 | 44.106 | 4.593 | 25.253 | 1.00 | 34.47 |
| 3314 | N | GLN | B | 840 | 42.403 | 7.709 | 26.057 | 1.00 | 27.73 |
| 3315 | CA | GLN | B | 840 | 42.501 | 8.945 | 25.292 | 1.00 | 28.34 |
| 3316 | C | GLN | B | 840 | 43.027 | 10.070 | 26.168 | 1.00 | 28.06 |
| 3317 | O | GLN | B | 840 | 43.735 | 10.963 | 25.700 | 1.00 | 26.67 |
| 3318 | CB | GLN | B | 840 | 41.142 | 9.339 | 24.711 | 1.00 | 32.45 |
| 3319 | CG | GLN | B | 840 | 40.481 | 8.269 | 23.836 | 1.00 | 46.64 |
| 3320 | CD | GLN | B | 840 | 39.661 | 7.236 | 24.617 | 1.00 | 53.33 |
| 3321 | OE1 | GLN | B | 840 | 40.172 | 6.514 | 25.478 | 1.00 | 44.62 |
| 3322 | NE2 | GLN | B | 840 | 38.370 | 7.169 | 24.306 | 1.00 | 55.68 |
| 3323 | N | PHE | B | 841 | 42.666 | 10.021 | 27.444 | 1.00 | 24.94 |
| 3324 | CA | PHE | B | 841 | 43.108 | 11.018 | 28.397 | 1.00 | 27.01 |
| 3325 | C | PHE | B | 841 | 44.619 | 10.962 | 28.540 | 1.00 | 23.28 |
| 3326 | O | PHE | B | 841 | 45.293 | 11.981 | 28.443 | 1.00 | 22.03 |
| 3327 | CB | PHE | B | 841 | 42.480 | 10.769 | 29.767 | 1.00 | 25.37 |
| 3328 | CG | PHE | B | 841 | 43.038 | 11.651 | 30.844 | 1.00 | 22.03 |
| 3329 | CD1 | PHE | B | 841 | 42.632 | 12.965 | 30.962 | 1.00 | 26.46 |
| 3330 | CD2 | PHE | B | 841 | 44.005 | 11.166 | 31.719 | 1.00 | 26.10 |
| 3331 | CE1 | PHE | B | 841 | 43.174 | 13.799 | 31.937 | 1.00 | 25.57 |
| 3332 | CE2 | PHE | B | 841 | 44.561 | 11.995 | 32.703 | 1.00 | 25.36 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3333 | CZ | PHE | B | 841 | 44.141 | 13.313 | 32.808 | 1.00 | 25.63 |
| 3334 | N | GLU | B | 842 | 45.152 | 9.770 | 28.781 | 1.00 | 27.54 |
| 3335 | CA | GLU | B | 842 | 46.607 | 9.639 | 28.939 | 1.00 | 29.02 |
| 3336 | C | GLU | B | 842 | 47.347 | 10.143 | 27.717 | 1.00 | 25.06 |
| 3337 | O | GLU | B | 842 | 48.357 | 10.826 | 27.837 | 1.00 | 27.55 |
| 3338 | CB | GLU | B | 842 | 47.010 | 8.183 | 29.199 | 1.00 | 36.85 |
| 3339 | CG | GLU | B | 842 | 46.593 | 7.628 | 30.568 | 1.00 | 46.79 |
| 3340 | CD | GLU | B | 842 | 47.223 | 8.376 | 31.741 | 1.00 | 50.21 |
| 3341 | OE1 | GLU | B | 842 | 47.948 | 9.364 | 31.510 | 1.00 | 51.33 |
| 3342 | OE2 | GLU | B | 842 | 46.987 | 7.974 | 32.901 | 1.00 | 54.97 |
| 3343 | N | GLU | B | 843 | 46.849 | 9.795 | 26.535 | 1.00 | 27.99 |
| 3344 | CA | GLU | B | 843 | 47.484 | 10.235 | 25.302 | 1.00 | 29.91 |
| 3345 | C | GLU | B | 843 | 47.455 | 11.750 | 25.197 | 1.00 | 24.87 |
| 3346 | O | GLU | B | 843 | 48.445 | 12.375 | 24.829 | 1.00 | 25.77 |
| 3347 | CB | GLU | B | 843 | 46.788 | 9.619 | 24.084 | 1.00 | 33.71 |
| 3348 | CG | GLU | B | 843 | 46.809 | 8.087 | 24.081 | 1.00 | 43.17 |
| 3349 | CD | GLU | B | 843 | 46.362 | 7.487 | 22.761 | 1.00 | 47.10 |
| 3350 | OE1 | GLU | B | 843 | 46.082 | 8.258 | 21.822 | 1.00 | 53.30 |
| 3351 | OE2 | GLU | B | 843 | 46.304 | 6.241 | 22.656 | 1.00 | 50.03 |
| 3352 | N | MET | B | 844 | 46.319 | 12.345 | 25.542 | 1.00 | 25.91 |
| 3353 | CA | MET | B | 844 | 46.168 | 13.787 | 25.468 | 1.00 | 23.10 |
| 3354 | C | MET | B | 844 | 47.064 | 14.475 | 26.490 | 1.00 | 22.35 |
| 3355 | O | MET | B | 844 | 47.746 | 15.454 | 26.184 | 1.00 | 23.84 |
| 3356 | CB | MET | B | 844 | 44.711 | 14.182 | 25.722 | 1.00 | 24.04 |
| 3357 | CG | MET | B | 844 | 44.442 | 15.681 | 25.592 | 1.00 | 27.46 |
| 3358 | SD | MET | B | 844 | 42.709 | 16.055 | 26.067 | 1.00 | 29.20 |
| 3359 | CE | MET | B | 844 | 42.699 | 17.809 | 25.977 | 1.00 | 22.37 |
| 3360 | N | ARG | B | 845 | 47.046 | 13.975 | 27.716 | 1.00 | 25.23 |
| 3361 | CA | ARG | B | 845 | 47.887 | 14.574 | 28.740 | 1.00 | 29.00 |
| 3362 | C | ARG | B | 845 | 49.353 | 14.423 | 28.345 | 1.00 | 27.50 |
| 3363 | O | ARG | B | 845 | 50.121 | 15.372 | 28.446 | 1.00 | 25.08 |
| 3364 | CB | ARG | B | 845 | 47.613 | 13.934 | 30.101 | 1.00 | 27.08 |
| 3365 | CG | ARG | B | 845 | 48.367 | 14.594 | 31.240 | 1.00 | 37.67 |
| 3366 | CD | ARG | B | 845 | 47.781 | 14.196 | 32.586 | 1.00 | 36.90 |
| 3367 | NE | ARG | B | 845 | 48.525 | 14.771 | 33.705 | 1.00 | 51.09 |
| 3368 | CZ | ARG | B | 845 | 49.746 | 14.390 | 34.063 | 1.00 | 42.11 |
| 3369 | NH1 | ARG | B | 845 | 50.363 | 13.422 | 33.400 | 1.00 | 50.54 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3370 | NH2 | ARG | B | 845 | 50.345 | 14.969 | 35.091 | 1.00 | 47.30 |
| 3371 | N | SER | B | 846 | 49.742 | 13.240 | 27.871 | 1.00 | 26.92 |
| 3372 | CA | SER | B | 846 | 51.134 | 13.049 | 27.463 | 1.00 | 28.80 |
| 3373 | C | SER | B | 846 | 51.480 | 14.022 | 26.331 | 1.00 | 25.62 |
| 3374 | O | SER | B | 846 | 52.563 | 14.601 | 26.285 | 1.00 | 25.05 |
| 3375 | CB | SER | B | 846 | 51.348 | 11.595 | 27.021 | 1.00 | 27.15 |
| 3376 | OG | SER | B | 846 | 51.189 | 10.730 | 28.138 | 1.00 | 31.19 |
| 3377 | N | SER | B | 847 | 50.532 | 14.220 | 25.422 | 1.00 | 27.30 |
| 3378 | CA | SER | B | 847 | 50.727 | 15.117 | 24.294 | 1.00 | 22.26 |
| 3379 | C | SER | B | 847 | 51.003 | 16.551 | 24.715 | 1.00 | 28.59 |
| 3380 | O | SER | B | 847 | 51.886 | 17.207 | 24.154 | 1.00 | 25.88 |
| 3381 | CB | SER | B | 847 | 49.494 | 15.060 | 23.382 | 1.00 | 31.83 |
| 3382 | OG | SER | B | 847 | 49.605 | 15.975 | 22.305 | 1.00 | 37.65 |
| 3383 | N | TYR | B | 848 | 50.243 | 17.057 | 25.686 | 1.00 | 24.14 |
| 3384 | CA | TYR | B | 848 | 50.452 | 18.423 | 26.145 | 1.00 | 24.20 |
| 3385 | C | TYR | B | 848 | 51.718 | 18.555 | 26.995 | 1.00 | 21.09 |
| 3386 | O | TYR | B | 848 | 52.310 | 19.629 | 27.051 | 1.00 | 22.03 |
| 3387 | CB | TYR | B | 848 | 49.209 | 18.922 | 26.891 | 1.00 | 20.70 |
| 3388 | CG | TYR | B | 848 | 48.113 | 19.361 | 25.925 | 1.00 | 22.54 |
| 3389 | CD1 | TYR | B | 848 | 48.205 | 20.577 | 25.257 | 1.00 | 17.53 |
| 3390 | CD2 | TYR | B | 848 | 47.042 | 18.516 | 25.618 | 1.00 | 24.44 |
| 3391 | CE1 | TYR | B | 848 | 47.249 | 20.959 | 24.294 | 1.00 | 22.50 |
| 3392 | CE2 | TYR | B | 848 | 46.086 | 18.880 | 24.660 | 1.00 | 21.73 |
| 3393 | CZ | TYR | B | 848 | 46.196 | 20.097 | 24.007 | 1.00 | 23.06 |
| 3394 | OH | TYR | B | 848 | 45.247 | 20.476 | 23.077 | 1.00 | 27.42 |
| 3395 | N | ILE | B | 849 | 52.113 | 17.482 | 27.675 | 1.00 | 22.59 |
| 3396 | CA | ILE | B | 849 | 53.357 | 17.533 | 28.457 | 1.00 | 25.53 |
| 3397 | C | ILE | B | 849 | 54.491 | 17.695 | 27.436 | 1.00 | 26.66 |
| 3398 | O | ILE | B | 849 | 55.391 | 18.529 | 27.608 | 1.00 | 25.61 |
| 3399 | CB | ILE | B | 849 | 53.569 | 16.237 | 29.284 | 1.00 | 29.06 |
| 3400 | CG1 | ILE | B | 849 | 52.549 | 16.192 | 30.423 | 1.00 | 26.54 |
| 3401 | CG2 | ILE | B | 849 | 55.020 | 16.172 | 29.847 | 1.00 | 23.64 |
| 3402 | CD1 | ILE | B | 849 | 52.581 | 14.899 | 31.199 | 1.00 | 27.17 |
| 3403 | N | ARG | B | 850 | 54.436 | 16.910 | 26.363 | 1.00 | 24.89 |
| 3404 | CA | ARG | B | 850 | 55.453 | 17.016 | 25.319 | 1.00 | 25.68 |
| 3405 | C | ARG | B | 850 | 55.424 | 18.403 | 24.686 | 1.00 | 27.78 |
| 3406 | O | ARG | B | 850 | 56.464 | 18.918 | 24.266 | 1.00 | 27.55 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3407 | CB | ARG | B | 850 | 55.255 | 15.957 | 24.226 | 1.00 | 26.91 |
| 3408 | CG | ARG | B | 850 | 55.521 | 14.511 | 24.663 | 1.00 | 27.09 |
| 3409 | CD | ARG | B | 850 | 55.646 | 13.575 | 23.458 | 1.00 | 29.81 |
| 3410 | NE | ARG | B | 850 | 54.413 | 13.394 | 22.686 | 1.00 | 31.76 |
| 3411 | CZ | ARG | B | 850 | 53.402 | 12.598 | 23.037 | 1.00 | 31.98 |
| 3412 | NH1 | ARG | B | 850 | 53.448 | 11.880 | 24.160 | 1.00 | 35.55 |
| 3413 | NH2 | ARG | B | 850 | 52.340 | 12.504 | 22.254 | 1.00 | 34.47 |
| 3414 | N | GLU | B | 851 | 54.242 | 19.018 | 24.620 | 1.00 | 27.13 |
| 3415 | CA | GLU | B | 851 | 54.133 | 20.358 | 24.029 | 1.00 | 22.10 |
| 3416 | C | GLU | B | 851 | 54.765 | 21.378 | 24.987 | 1.00 | 27.46 |
| 3417 | O | GLU | B | 851 | 55.394 | 22.347 | 24.551 | 1.00 | 29.77 |
| 3418 | CB | GLU | B | 851 | 52.664 | 20.719 | 23.761 | 1.00 | 30.54 |
| 3419 | CG | GLU | B | 851 | 52.505 | 21.829 | 22.716 | 1.00 | 32.90 |
| 3420 | CD | GLU | B | 851 | 53.026 | 21.401 | 21.338 | 1.00 | 42.32 |
| 3421 | OE1 | GLU | B | 851 | 53.314 | 20.191 | 21.155 | 1.00 | 33.77 |
| 3422 | OE2 | GLU | B | 851 | 53.140 | 22.266 | 20.439 | 1.00 | 34.60 |
| 3423 | N | LEU | B | 852 | 54.587 | 21.168 | 26.293 | 1.00 | 23.76 |
| 3424 | CA | LEU | B | 852 | 55.204 | 22.064 | 27.265 | 1.00 | 26.78 |
| 3425 | C | LEU | B | 852 | 56.726 | 22.019 | 27.067 | 1.00 | 22.14 |
| 3426 | O | LEU | B | 852 | 57.399 | 23.046 | 27.101 | 1.00 | 26.98 |
| 3427 | CB | LEU | B | 852 | 54.879 | 21.625 | 28.687 | 1.00 | 22.84 |
| 3428 | CG | LEU | B | 852 | 55.560 | 22.451 | 29.782 | 1.00 | 26.06 |
| 3429 | CD1 | LEU | B | 852 | 55.271 | 23.923 | 29.577 | 1.00 | 23.63 |
| 3430 | CD2 | LEU | B | 852 | 55.080 | 21.993 | 31.145 | 1.00 | 26.99 |
| 3431 | N | ILE | B | 853 | 57.251 | 20.810 | 26.876 | 1.00 | 28.61 |
| 3432 | CA | ILE | B | 853 | 58.689 | 20.628 | 26.690 | 1.00 | 24.00 |
| 3433 | C | ILE | B | 853 | 59.166 | 21.389 | 25.458 | 1.00 | 27.59 |
| 3434 | O | ILE | B | 853 | 60.267 | 21.929 | 25.453 | 1.00 | 29.87 |
| 3435 | CB | ILE | B | 853 | 59.031 | 19.136 | 26.574 | 1.00 | 26.82 |
| 3436 | CG1 | ILE | B | 853 | 58.737 | 18.458 | 27.919 | 1.00 | 25.90 |
| 3437 | CG2 | ILE | B | 853 | 60.478 | 18.972 | 26.104 | 1.00 | 24.70 |
| 3438 | CD1 | ILE | B | 853 | 58.685 | 16.935 | 27.876 | 1.00 | 23.86 |
| 3439 | N | LYS | B | 854 | 58.343 | 21.442 | 24.411 | 1.00 | 24.04 |
| 3440 | CA | LYS | B | 854 | 58.725 | 22.192 | 23.215 | 1.00 | 28.78 |
| 3441 | C | LYS | B | 854 | 58.668 | 23.690 | 23.497 | 1.00 | 28.51 |
| 3442 | O | LYS | B | 854 | 59.508 | 24.457 | 23.020 | 1.00 | 29.04 |
| 3443 | CB | LYS | B | 854 | 57.788 | 21.893 | 22.039 | 1.00 | 29.18 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3444 | CG | LYS | B | 854 | 57.765 | 20.460 | 21.555 | 1.00 | 33.70 |
| 3445 | CD | LYS | B | 854 | 56.758 | 20.366 | 20.418 | 1.00 | 35.70 |
| 3446 | CE | LYS | B | 854 | 56.587 | 18.960 | 19.913 | 1.00 | 48.12 |
| 3447 | NZ | LYS | B | 854 | 55.515 | 18.932 | 18.876 | 1.00 | 43.84 |
| 3448 | N | ALA | B | 855 | 57.661 | 24.112 | 24.256 | 1.00 | 28.37 |
| 3449 | CA | ALA | B | 855 | 57.504 | 25.535 | 24.592 | 1.00 | 25.35 |
| 3450 | C | ALA | B | 855 | 58.719 | 26.035 | 25.379 | 1.00 | 26.32 |
| 3451 | O | ALA | B. | 855 | 59.203 | 27.156 | 25.185 | 1.00 | 25.77 |
| 3452 | CB | ALA | B | 855 | 56.218 | 25.745 | 25.417 | 1.00 | 29.46 |
| 3453 | N | ILE | B | 856 | 59.194 | 25.204 | 26.292 | 1.00 | 27.24 |
| 3454 | CA | ILE | B | 856 | 60.361 | 25.562 | 27.073 | 1.00 | 27.67 |
| 3455 | C | ILE | B | 856 | 61.543 | 25.659 | 26.100 | 1.00 | 34.93 |
| 3456 | O | ILE | B | 856 | 62.414 | 26.522 | 26.230 | 1.00 | 30.85 |
| 3457 | CB | ILE | B | 856 | 60.672 | 24.481 | 28.116 | 1.00 | 30.91 |
| 3458 | CG1 | ILE | B | 856 | 59.582 | 24.474 | 29.197 | 1.00 | 30.70 |
| 3459 | CG2 | ILE | B | 856 | 62.060 | 24.718 | 28.725 | 1.00 | 30.32 |
| 3460 | CD1 | ILE | B | 856 | 59.729 | 23.310 | 30.188 | 1.00 | 28.42 |
| 3461 | N | GLY | B | 857 | 61.540 | 24.771 | 25.113 | 1.00 | 32.68 |
| 3462 | CA | GLY | B | 857 | 62.618 | 24.739 | 24.141 | 1.00 | 37.55 |
| 3463 | C | GLY | B | 857 | 62.769 | 26.010 | 23.330 | 1.00 | 39.78 |
| 3464 | O | GLY | B | 857 | 63.836 | 26.269 | 22.777 | 1.00 | 38.63 |
| 3465 | N | LEU | B | 858 | 61.712 | 26.809 | 23.257 | 1.00 | 42.53 |
| 3466 | CA | LEU | B | 858 | 61.747 | 28.048 | 22.486 | 1.00 | 44.50 |
| 3467 | C | LEU | B | 858 | 62.711 | 29.110 | 23.012 | 1.00 | 49.32 |
| 3468 | O | LEU | B | 858 | 63.281 | 29.877 | 22.236 | 1.00 | 47.44 |
| 3469 | CB | LEU | B | 858 | 60.344 | 28.649 | 22.405 | 1.00 | 45.48 |
| 3470 | CG | LEU | B | 858 | 59.304 | 27.814 | 21.663 | 1.00 | 42.37 |
| 3471 | CD1 | LEU | B | 858 | 57.941 | 28.491 | 21.752 | 1.00 | 47.24 |
| 3472 | CD2 | LEU | B | 858 | 59.733 | 27.651 | 20.217 | 1.00 | 47.70 |
| 3473 | N | ARG | B | 859 | 62.887 | 29.171 | 24.323 | 1.00 | 51.14 |
| 3474 | CA | ARG | B | 859 | 63.774 | 30.170 | 24.899 | 1.00 | 60.57 |
| 3475 | C | ARG | B | 859 | 64.948 | 29.538 | 25.620 | 1.00 | 63.51 |
| 3476 | O | ARG | B | 859 | 66.088 | 29.648 | 25.172 | 1.00 | 66.86 |
| 3477 | CB | ARG | B | 859 | 62.994 | 31.080 | 25.852 | 1.00 | 60.03 |
| 3478 | CG | ARG | B | 859 | 61.946 | 31.931 | 25.147 | 1.00 | 66.28 |
| 3479 | CD | ARG | B | 859 | 61.112 | 32.750 | 26.120 | 1.00 | 65.17 |
| 3480 | NE | ARG | B | 859 | 60.150 | 33.601 | 25.422 | 1.00 | 72.22 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3481 | CZ | ARG | B | 859 | 59.228 | 33.155 | 24.573 | 1.00 | 75.01 |
| 3482 | NH1 | ARG | B | 859 | 59.134 | 31.857 | 24.305 | 1.00 | 74.54 |
| 3483 | NH2 | ARG | B | 859 | 58.394 | 34.006 | 23.990 | 1.00 | 77.06 |
| 3484 | N | GLN | B | 860 | 64.670 | 28.876 | 26.736 | 1.00 | 67.91 |
| 3485 | CA | GLN | B | 860 | 65.730 | 28.235 | 27.492 | 1.00 | 67.91 |
| 3486 | C | GLN | B | 860 | 66.514 | 27.268 | 26.623 | 1.00 | 70.16 |
| 3487 | O | GLN | B | 860 | 66.108 | 26.124 | 26.403 | 1.00 | 66.29 |
| 3488 | CB | GLN | B | 860 | 65.167 | 27.527 | 28.731 | 1.00 | 68.82 |
| 3489 | CG | GLN | B | 860 | 64.975 | 28.461 | 29.925 | 1.00 | 70.16 |
| 3490 | CD | GLN | B | 860 | 63.978 | 29.584 | 29.675 | 1.00 | 73.18 |
| 3491 | OE1 | GLN | B | 860 | 63.922 | 30.553 | 30.434 | 1.00 | 75.31 |
| 3492 | NE2 | GLN | B | 860 | 63.172 | 29.449 | 28.629 | 1.00 | 74.53 |
| 3493 | N | LYS | B | 861 | 67.636 | 27.766 | 26.110 | 1.00 | 70.28 |
| 3494 | CA | LYS | B | 861 | 68.527 | 26.982 | 25.274 | 1.00 | 70.98 |
| 3495 | C | LYS | B | 861 | 69.480 | 26.247 | 26.205 | 1.00 | 69.66 |
| 3496 | O | LYS | B | 861 | 70.017 | 26.833 | 27.149 | 1.00 | 69.56 |
| 3497 | CB | LYS | B | 861 | 69.334 | 27.889 | 24.342 | 1.00 | 73.98 |
| 3498 | CG | LYS | B | 861 | 68.512 | 28.696 | 23.352 | 1.00 | 76.75 |
| 3499 | CD | LYS | B | 861 | 67.728 | 27.806 | 22.403 | 1.00 | 79.85 |
| 3500 | CE | LYS | B | 861 | 67.077 | 28.623 | 21.296 | 1.00 | 82.15 |
| 3501 | NZ | LYS | B | 861 | 66.190 | 29.694 | 21.820 | 1.00 | 83.45 |
| 3502 | N | GLY | B | 862 | 69.687 | 24.964 | 25.942 | 1.00 | 65.51 |
| 3503 | CA | GLY | B | 862 | 70.581 | 24.187 | 26.777 | 1.00 | 62.36 |
| 3504 | C | GLY | B | 862 | 69.870 | 23.045 | 27.473 | 1.00 | 58.94 |
| 3505 | O | GLY | B | 862 | 68.786 | 23.219 | 28.027 | 1.00 | 55.70 |
| 3506 | N | VAL | B | 863 | 70.496 | 21.874 | 27.450 | 1.00 | 55.20 |
| 3507 | CA | VAL | B | 863 | 69.934 | 20.680 | 28.063 | 1.00 | 54.32 |
| 3508 | C | VAL | B | 863 | 69.691 | 20.830 | 29.567 | 1.00 | 52.94 |
| 3509 | O | VAL | B | 863 | 68.643 | 20.436 | 30.070 | 1.00 | 51.71 |
| 3510 | CB | VAL | B | 863 | 70.850 | 19.461 | 27.812 | 1.00 | 53.87 |
| 3511 | CG1 | VAL | B | 863 | 72.228 | 19.710 | 28.406 | 1.00 | 54.75 |
| 3512 | CG2 | VAL | B | 863 | 70.228 | 18.212 | 28.405 | 1.00 | 55.92 |
| 3513 | N | VAL | B | 864 | 70.651 | 21.404 | 30.282 | 1.00 | 51.96 |
| 3514 | CA | VAL | B | 864 | 70.513 | 21.578 | 31.725 | 1.00 | 50.09 |
| 3515 | C | VAL | B | 864 | 69.445 | 22.622 | 32.065 | 1.00 | 49.79 |
| 3516 | O | VAL | B | 864 | 68.577 | 22.383 | 32.903 | 1.00 | 46.15 |
| 3517 | CB | VAL | B | 864 | 71.864 | 22.001 | 32.372 | 1.00 | 50.90 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3518 | CG1 | VAL | B | 864 | 71.740 | 21.991 | 33.889 | 1.00 | 49.47 |
| 3519 | CG2 | VAL | B | 864 | 72.973 | 21.056 | 31.936 | 1.00 | 46.98 |
| 3520 | N | SER | B | 865 | 69.504 | 23.772 | 31.403 | 1.00 | 43.87 |
| 3521 | CA | SER | B | 865 | 68.539 | 24.830 | 31.653 | 1.00 | 46.16 |
| 3522 | C | SER | B | 865 | 67.124 | 24.366 | 31.314 | 1.00 | 43.21 |
| 3523 | O | SER | B | 865 | 66.178 | 24.660 | 32.045 | 1.00 | 41.52 |
| 3524 | CB | SER | B | 865 | 68.893 | 26.066 | 30.828 | 1.00 | 46.68 |
| 3525 | OG | SER | B | 865 | 68.014 | 27.136 | 31.116 | 1.00 | 59.14 |
| 3526 | N | SER | B | 866 | 66.988 | 23.628 | 30.215 | 1.00 | 40.92 |
| 3527 | CA | SER | B | 866 | 65.682 | 23.139 | 29.776 | 1.00 | 35.24 |
| 3528 | C | SER | B | 866 | 65.071 | 22.097 | 30.695 | 1.00 | 40.63 |
| 3529 | O | SER | B | 866 | 63.874 | 22.154 | 30.980 | 1.00 | 32.01 |
| 3530 | CB | SER | B | 866 | 65.772 | 22.565 | 28.362 | 1.00 | 37.02 |
| 3531 | OG | SER | B | 866 | 66.142 | 23.573 | 27.443 | 1.00 | 45.95 |
| 3532 | N | SER | B | 867 | 65.877 | 21.134 | 31.146 | 1.00 | 37.03 |
| 3533 | CA | SER | B | 867 | 65.372 | 20.099 | 32.040 | 1.00 | 36.93 |
| 3534 | C | SER | B | 867 | 65.077 | 20.710 | 33.407 | 1.00 | 40.17 |
| 3535 | O | SER | B | 867 | 64.149 | 20.282 | 34.099 | 1.00 | 35.95 |
| 3536 | CB | SER | B | 867 | 66.382 | 18.952 | 32.187 | 1.00 | 40.92 |
| 3537 | OG | SER | B | 867 | 67.556 | 19.387 | 32.850 | 1.00 | 48.34 |
| 3538 | N | GLN | B | 868 | 65.872 | 21.697 | 33.810 | 1.00 | 37.09 |
| 3539 | CA | GLN | B | 868 | 65.623 | 22.354 | 35.093 | 1.00 | 41.94 |
| 3540 | C | GLN | B | 868 | 64.340 | 23.189 | 35.025 | 1.00 | 34.55 |
| 3541 | O | GLN | B | 868 | 63.579 | 23.242 | 35.990 | 1.00 | 35.43 |
| 3542 | CB | GLN | B | 868 | 66.790 | 23.267 | 35.490 | 1.00 | 43.10 |
| 3543 | CG | GLN | B | 868 | 68.042 | 22.519 | 35.954 | 1.00 | 54.16 |
| 3544 | CD | GLN | B | 868 | 69.125 | 23.450 | 36.495 | 1.00 | 59.01 |
| 3545 | OE1 | GLN | B | 868 | 70.155 | 22.994 | 36.992 | 1.00 | 60.70 |
| 3546 | NE2 | GLN | B | 868 | 68.893 | 24.759 | 36.399 | 1.00 | 59.00 |
| 3547 | N | ARG | B | 869 | 64.112 | 23.847 | 33.893 | 1.00 | 34.25 |
| 3548 | CA | ARG | B | 869 | 62.918 | 24.666 | 33.746 | 1.00 | 32.37 |
| 3549 | C | ARG | B | 869 | 61.673 | 23.778 | 33.716 | 1.00 | 33.17 |
| 3550 | O | ARG | B | 869 | 60.633 | 24.147 | 34.262 | 1.00 | 32.22 |
| 3551 | CB | ARG | B | 869 | 63.001 | 25.541 | 32.500 | 1.00 | 34.11 |
| 3552 | CG | ARG | B | 869 | 61.908 | 26.619 | 32.493 | 1.00 | 39.35 |
| 3553 | CD | ARG | B | 869 | 62.127 | 27.633 | 31.406 | 1.00 | 42.79 |
| 3554 | NE | ARG | B | 869 | 61.101 | 28.672 | 31.368 | 1.00 | 40.15 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3555 | CZ | ARG | B | 869 | 60.828 | 29.522 | 32.350 | 1.00 | 39.72 |
| 3556 | NH1 | ARG | B | 869 | 61.502 | 29.478 | 33.492 | 1.00 | 43.65 |
| 3557 | NH2 | ARG | B | 869 | 59.867 | 30.425 | 32.185 | 1.00 | 36.33 |
| 3558 | N | PHE | B | 870 | 61.781 | 22.601 | 33.101 | 1.00 | 30.79 |
| 3559 | CA | PHE | B | 870 | 60.670 | 21.651 | 33.078 | 1.00 | 30.15 |
| 3560 | C | PHE | B | 870 | 60.299 | 21.259 | 34.516 | 1.00 | 35.55 |
| 3561 | O | PHE | B | 870 | 59.117 | 21.156 | 34.857 | 1.00 | 30.41 |
| 3562 | CB | PHE | B | 870 | 61.042 | 20.373 | 32.330 | 1.00 | 34.17 |
| 3563 | CG | PHE | B | 870 | 59.923 | 19.373 | 32.263 | 1.00 | 31.63 |
| 3564 | CD1 | PHE | B | 870 | 58.818 | 19.607 | 31.449 | 1.00 | 34.59 |
| 3565 | CD2 | PHE | B | 870 | 59.949 | 18.225 | 33.041 | 1.00 | 36.46 |
| 3566 | CE1 | PHE | B | 870 | 57.758 | 18.715 | 31.412 | 1.00 | 34.87 |
| 3567 | CE2 | PHE | B | 870 | 58.887 | 17.322 | 33.012 | 1.00 | 42.81 |
| 3568 | CZ | PHE | B | 870 | 57.789 | 17.569 | 32.195 | 1.00 | 35.48 |
| 3569 | N | TYR | B | 871 | 61.305 | 21.021 | 35.358 | 1.00 | 28.70 |
| 3570 | CA | TYR | B | 871 | 61.029 | 20.653 | 36.746 | 1.00 | 32.21 |
| 3571 | C | TYR | B | 871 | 60.381 | 21.809 | 37.513 | 1.00 | 31.93 |
| 3572 | O | TYR | B | 871 | 59.464 | 21.590 | 38.313 | 1.00 | 32.87 |
| 3573 | CB | TYR | B | 871 | 62.314 | 20.183 | 37.459 | 1.00 | 33.96 |
| 3574 | CG | TYR | B | 871 | 62.117 | 19.878 | 38.930 | 1.00 | 37.15 |
| 3575 | CD1 | TYR | B | 871 | 62.099 | 20.902 | 39.883 | 1.00 | 44.42 |
| 3576 | CD2 | TYR | B | 871 | 61.895 | 18.571 | 39.368 | 1.00 | 45.79 |
| 3577 | CE1 | TYR | B | 871 | 61.863 | 20.632 | 41.233 | 1.00 | 42.43 |
| 3578 | CE2 | TYR | B | 871 | 61.657 | 18.289 | 40.722 | 1.00 | 47.61 |
| 3579 | CZ | TYR | B | 871 | 61.641 | 19.326 | 41.646 | 1.00 | 50.61 |
| 3580 | OH | TYR | B | 871 | 61.393 | 19.061 | 42.978 | 1.00 | 55.59 |
| 3581 | N | GLN | B | 872 | 60.853 | 23.032 | 37.268 | 1.00 | 29.65 |
| 3582 | CA | GLN | B | 872 | 60.298 | 24.216 | 37.935 | 1.00 | 32.37 |
| 3583 | C | GLN | B | 872 | 58.832 | 24.460 | 37.566 | 1.00 | 31.80 |
| 3584 | O | GLN | B | 872 | 57.986 | 24.675 | 38.444 | 1.00 | 28.10 |
| 3585 | CB | GLN | B | 872 | 61.075 | 25.476 | 37.568 | 1.00 | 29.72 |
| 3586 | CG | GLN | B | 872 | 62.498 | 25.554 | 38.096 | 1.00 | 42.15 |
| 3587 | CD | GLN | B | 872 | 63.207 | 26.805 | 37.605 | 1.00 | 43.54 |
| 3588 | OE1 | GLN | B | 872 | 63.427 | 26.978 | 36.405 | 1.00 | 48.46 |
| 3589 | NE2 | GLN | B | 872 | 63.554 | 27.690 | 38.529 | 1.00 | 54.30 |
| 3590 | N | LEU | B | 873 | 58.541 | 24.449 | 36.267 | 1.00 | 30.13 |
| 3591 | CA | LEU | B | 873 | 57.176 | 24.686 | 35.806 | 1.00 | 30.27 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3592 | C | LEU | B | 873 | 56.205 | 23.598 | 36.234 | 1.00 | 35.12 |
| 3593 | O | LEU | B | 873 | 55.051 | 23.889 | 36.590 | 1.00 | 32.42 |
| 3594 | CB | LEU | B | 873 | 57.146 | 24.876 | 34.284 | 1.00 | 31.46 |
| 3595 | CG | LEU | B | 873 | 57.871 | 26.154 | 33.836 | 1.00 | 25.50 |
| 3596 | CD1 | LEU | B | 873 | 57.739 | 26.342 | 32.322 | 1.00 | 26.12 |
| 3597 | CD2 | LEU | B | 873 | 57.293 | 27.359 | 34.569 | 1.00 | 30.31 |
| 3598 | N | THR | B | 874 | 56.650 | 22.348 | 36.208 | 1.00 | 32.47 |
| 3599 | CA | THR | B | 874 | 55.777 | 21.274 | 36.631 | 1.00 | 31.43 |
| 3600 | C | THR | B | 874 | 55.664 | 21.315 | 38.158 | 1.00 | 33.30 |
| 3601 | O | THR | B | 874 | 54.610 | 21.009 | 38.713 | 1.00 | 32.00 |
| 3602 | CB | THR | B | 874 | 56.292 | 19.883 | 36.157 | 1.00 | 33.76 |
| 3603 | OG1 | THR | B | 874 | 57.628 | 19.683 | 36.610 | 1.00 | 36.17 |
| 3604 | CG2 | THR | B | 874 | 56.261 | 19.788 | 34.628 | 1.00 | 31.16 |
| 3605 | N | LYS | B | 875 | 56.736 | 21.710 | 38.842 | 1.00 | 31.72 |
| 3606 | CA | LYS | B | 875 | 56.677 | 21.780 | 40.303 | 1.00 | 35.19 |
| 3607 | C | LYS | B | 875 | 55.701 | 22.895 | 40.688 | 1.00 | 33.68 |
| 3608 | O | LYS | B | 875 | 54.983 | 22.794 | 41.686 | 1.00 | 33.61 |
| 3609 | CB | LYS | B | 875 | 58.062 | 22.067 | 40.907 | 1.00 | 37.62 |
| 3610 | CG | LYS | B | 875 | 58.091 | 21.945 | 42.429 | 1.00 | 40.41 |
| 3611 | CD | LYS | B | 875 | 57.761 | 20.509 | 42.843 | 1.00 | 43.28 |
| 3612 | CE | LYS | B | 875 | 57.618 | 20.357 | 44.354 | 1.00 | 41.31 |
| 3613 | NZ | LYS | B | 875 | 57.273 | 18.939 | 44.712 | 1.00 | 45.62 |
| 3614 | N | LEU | B | 876 | 55.682 | 23.958 | 39.890 | 1.00 | 30.46 |
| 3615 | CA | LEU | B | 876 | 54.782 | 25.081 | 40.129 | 1.00 | 34.18 |
| 3616 | C | LEU | B | 876 | 53.345 | 24.568 | 40.037 | 1.00 | 33.33 |
| 3617 | O | LEU | B | 876 | 52.510 | 24.892 | 40.881 | 1.00 | 32.77 |
| 3618 | CB | LEU | B | 876 | 55.026 | 26.179 | 39.093 | 1.00 | 35.40 |
| 3619 | CG | LEU | B | 876 | 54.302 | 27.520 | 39.218 | 1.00 | 35.72 |
| 3620 | CD1 | LEU | B | 876 | 54.848 | 28.485 | 38.179 | 1.00 | 42.08 |
| 3621 | CD2 | LEU | B | 876 | 52.806 | 27.336 | 39.039 | 1.00 | 42.68 |
| 3622 | N | LEU | B | 877 | 53.060 | 23.754 | 39.022 | 1.00 | 29.58 |
| 3623 | CA | LEU | B | 877 | 51.721 | 23.208 | 38.869 | 1.00 | 30.87 |
| 3624 | C | LEU | B | 877 | 51.345 | 22.311 | 40.053 | 1.00 | 34.72 |
| 3625 | O | LEU | B | 877 | 50.220 | 22.392 | 40.555 | 1.00 | 31.85 |
| 3626 | CB | LEU | B | 877 | 51.601 | 22.439 | 37.544 | 1.00 | 32.60 |
| 3627 | CG | LEU | B | 877 | 51.708 | 23.298 | 36.274 | 1.00 | 33.97 |
| 3628 | CD1 | LEU | B | 877 | 51.665 | 22.424 | 35.020 | 1.00 | 29.88 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3629 | CD2 | LEU | B | 877 | 50.565 | 24.314 | 36.268 | 1.00 | 34.90 |
| 3630 | N | ASP | B | 878 | 52.274 | 21.463 | 40.505 | 1.00 | 30.56 |
| 3631 | CA | ASP | B | 878 | 51.995 | 20.576 | 41.647 | 1.00 | 32.09 |
| 3632 | C | ASP | B | 878 | 51.659 | 21.415 | 42.872 | 1.00 | 26.56 |
| 3633 | O | ASP | B | 878 | 50.754 | 21.079 | 43.642 | 1.00 | 29.15 |
| 3634 | CB | ASP | B | 878 | 53.205 | 19.709 | 42.001 | 1.00 | 31.44 |
| 3635 | CG | ASP | B | 878 | 53.686 | 18.868 | 40.846 | 1.00 | 45.52 |
| 3636 | OD1 | ASP | B | 878 | 52.913 | 18.024 | 40.344 | 1.00 | 38.49 |
| 3637 | OD2 | ASP | B | 878 | 54.851 | 19.050 | 40.441 | 1.00 | 51.89 |
| 3638 | N | ASN | B | 879 | 52.411 | 22.491 | 43.061 | 1.00 | 26.43 |
| 3639 | CA | ASN | B | 879 | 52.187 | 23.393 | 44.185 | 1.00 | 33.73 |
| 3640 | C | ASN | B | 879 | 50.798 | 24.042 | 44.128 | 1.00 | 32.92 |
| 3641 | O | ASN | B | 879 | 50.233 | 24.391 | 45.163 | 1.00 | 33.36 |
| 3642 | CB | ASN | B | 879 | 53.255 | 24.490 | 44.222 | 1.00 | 31.44 |
| 3643 | CG | ASN | B | 879 | 54.604 | 23.989 | 44.705 | 1.00 | 41.99 |
| 3644 | OD1 | ASN | B | 879 | 55.580 | 24.742 | 44.727 | 1.00 | 46.26 |
| 3645 | ND2 | ASN | B | 879 | 54.666 | 22.719 | 45.102 | 1.00 | 38.65 |
| 3646 | N | LEU | B | 880 | 50.247 | 24.216 | 42.931 | 1.00 | 32.60 |
| 3647 | CA | LEU | B | 880 | 48.919 | 24.821 | 42.818 | 1.00 | 33.43 |
| 3648 | C | LEU | B | 880 | 47.832 | 24.063 | 43.566 | 1.00 | 33.86 |
| 3649 | O | LEU | B | 880 | 46.878 | 24.669 | 44.045 | 1.00 | 32.44 |
| 3650 | CB | LEU | B | 880 | 48.499 | 24.979 | 41.354 | 1.00 | 37.74 |
| 3651 | CG | LEU | B | 880 | 48.820 | 26.311 | 40.686 | 1.00 | 43.19 |
| 3652 | CD1 | LEU | B | 880 | 48.433 | 26.273 | 39.208 | 1.00 | 42.98 |
| 3653 | CD2 | LEU | B | 880 | 48.053 | 27.410 | 41.416 | 1.00 | 43.09 |
| 3654 | N | HIS | B | 881 | 47.960 | 22.745 | 43.659 | 1.00 | 34.37 |
| 3655 | CA | HIS | B | 881 | 46.971 | 21.944 | 44.377 | 1.00 | 36.86 |
| 3656 | C | HIS | B | 881 | 46.746 | 22.467 | 45.791 | 1.00 | 38.92 |
| 3657 | O | HIS | B | 881 | 45.613 | 22.620 | 46.232 | 1.00 | 38.03 |
| 3658 | CB | HIS | B | 881 | 47.424 | 20.486 | 44.438 | 1.00 | 38.32 |
| 3659 | CG | HIS | B | 881 | 47.321 | 19.770 | 43.129 | 1.00 | 46.94 |
| 3660 | ND1 | HIS | B | 881 | 48.098 | 18.675 | 42.814 | 1.00 | 54.16 |
| 3661 | CD2 | HIS | B | 881 | 46.513 | 19.975 | 42.062 | 1.00 | 49.76 |
| 3662 | CE1 | HIS | B | 881 | 47.775 | 18.237 | 41.611 | 1.00 | 49.51 |
| 3663 | NE2 | HIS | B | 881 | 46.814 | 19.008 | 41.132 | 1.00 | 56.41 |
| 3664 | N | ASP | B | 882 | 47.839 | 22.741 | 46.491 | 1.00 | 36.82 |
| 3665 | CA | ASP | B | 882 | 47.799 | 23.236 | 47.861 | 1.00 | 39.00 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3666 | C | ASP | B | 882 | 47.144 | 24.616 | 47.950 | 1.00 | 37.23 |
| 3667 | O | ASP | B | 882 | 46.325 | 24.876 | 48.833 | 1.00 | 38.12 |
| 3668 | CB | ASP | B | 882 | 49.229 | 23.324 | 48.397 | 1.00 | 48.17 |
| 3669 | CG | ASP | B | 882 | 50.038 | 22.075 | 48.099 | 1.00 | 57.26 |
| 3670 | OD1 | ASP | B | 882 | 50.294 | 21.799 | 46.900 | 1.00 | 57.71 |
| 3671 | OD2 | ASP | B | 882 | 50.409 | 21.362 | 49.058 | 1.00 | 65.58 |
| 3672 | N | LEU | B | 883 | 47.531 | 25.492 | 47.028 | 1.00 | 32.08 |
| 3673 | CA | LEU | B | 883 | 47.025 | 26.855 | 46.939 | 1.00 | 29.89 |
| 3674 | C | LEU | B | 883 | 45.527 | 26.833 | 46.637 | 1.00 | 31.31 |
| 3675 | O | LEU | B | 883 | 44.729 | 27.516 | 47.280 | 1.00 | 27.71 |
| 3676 | CB | LEU | B | 883 | 47.757 | 27.580 | 45.812 | 1.00 | 36.27 |
| 3677 | CG | LEU | B | 883 | 47.649 | 29.088 | 45.604 | 1.00 | 34.71 |
| 3678 | CD1 | LEU | B | 883 | 46.197 | 29.517 | 45.367 | 1.00 | 37.86 |
| 3679 | CD2 | LEU | B | 883 | 48.249 | 29.773 | 46.812 | 1.00 | 51.86 |
| 3680 | N | VAL | B | 884 | 45.154 | 26.047 | 45.639 | 1.00 | 26.92 |
| 3681 | CA | VAL | B | 884 | 43.757 | 25.940 | 45.248 | 1.00 | 24.81 |
| 3682 | C | VAL | B | 884 | 42.896 | 25.301 | 46.345 | 1.00 | 26.03 |
| 3683 | O | VAL | B | 884 | 41.712 | 25.600 | 46.473 | 1.00 | 22.11 |
| 3684 | CB | VAL | B | 884 | 43.640 | 25.139 | 43.938 | 1.00 | 29.47 |
| 3685 | CG1 | VAL | B | 884 | 42.196 | 24.853 | 43.614 | 1.00 | 30.72 |
| 3686 | CG2 | VAL | B | 884 | 44.278 | 25.944 | 42.805 | 1.00 | 30.08 |
| 3687 | N | LYS | B | 885 | 43.484 | 24.418 | 47.142 | 1.00 | 23.85 |
| 3688 | CA | LYS | B | 885 | 42.716 | 23.800 | 48.215 | 1.00 | 24.88 |
| 3689 | C | LYS | B | 885 | 42.189 | 24.905 | 49.145 | 1.00 | 25.51 |
| 3690 | O | LYS | B | 885 | 41.038 | 24.844 | 49.607 | 1.00 | 22.01 |
| 3691 | CB | LYS | B | 885 | 43.593 | 22.819 | 48.985 | 1.00 | 29.10 |
| 3692 | CG | LYS | B | 885 | 42.865 | 22.086 | 50.096 | 1.00 | 37.31 |
| 3693 | CD | LYS | B | 885 | 43.761 | 21.053 | 50.762 | 1.00 | 31.31 |
| 3694 | CE | LYS | B | 885 | 43.049 | 20.417 | 51.951 | 1.00 | 38.95 |
| 3695 | NZ | LYS | B | 885 | 43.916 | 19.437 | 52.668 | 1.00 | 41.49 |
| 3696 | N | GLN | B | 886 | 43.015 | 25.921 | 49.403 | 1.00 | 23.54 |
| 3697 | CA | GLN | B | 886 | 42.602 | 27.039 | 50.275 | 1.00 | 26.86 |
| 3698 | C | GLN | B | 886 | 41.428 | 27.822 | 49.684 | 1.00 | 29.25 |
| 3699 | O | GLN | B | 886 | 40.528 | 28.228 | 50.418 | 1.00 | 21.92 |
| 3700 | CB | GLN | B | 886 | 43.764 | 28.010 | 50.531 | 1.00 | 32.12 |
| 3701 | CG | GLN | B | 886 | 44.939 | 27.408 | 51.301 | 1.00 | 37.80 |
| 3702 | CD | GLN | B | 886 | 46.118 | 28.374 | 51.422 | 1.00 | 45.69 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3703 | OE1 | GLN | B | 886 | 46.041 | 29.400 | 52.103 | 1.00 | 44.67 |
| 3704 | NE2 | GLN | B | 886 | 47.212 | 28.051 | 50.743 | 1.00 | 49.75 |
| 3705 | N | LEU | B | 887 | 41.465 | 28.045 | 48.367 | 1.00 | 24.24 |
| 3706 | CA | LEU | B | 887 | 40.402 | 28.749 | 47.648 | 1.00 | 19.73 |
| 3707 | C | LEU | B | 887 | 39.127 | 27.894 | 47.648 | 1.00 | 17.60 |
| 3708 | O | LEU | B | 887 | 38.017 | 28.410 | 47.775 | 1.00 | 21.95 |
| 3709 | CB | LEU | B | 887 | 40.836 | 29.028 | 46.203 | 1.00 | 24.60 |
| 3710 | CG | LEU | B | 887 | 42.047 | 29.951 | 46.027 | 1.00 | 25.31 |
| 3711 1 | CD1 | LEU | B | 887 | 42.369 | 30.108 | 44.543 | 1.00 | 26.29 |
| 3712 | CD2 | LEU | B | 887 | 41.757 | 31.290 | 46.641 | 1.00 | 29.73 |
| 3713 | N | HIS | B | 888 | 39.292 | 26.580 | 47.502 | 1.00 | 18.82 |
| 3714 | CA | HIS | B | 888 | 38.165 | 25.649 | 47.513 | 1.00 | 18.69 |
| 3715 | C | HIS | B | 888 | 37.422 | 25.669 | 48.845 | 1.00 | 22.15 |
| 3716 | O | HIS | B | 888 | 36.194 | 25.684 | 48.874 | 1.00 | 19.70 |
| 3717 | CB | HIS | B | 888 | 38.654 | 24.228 | 47.247 | 1.00 | 20.05 |
| 3718 | CG | HIS | B | 888 | 38.877 | 23.923 | 45.796 | 1.00 | 22.81 |
| 3719 | ND1 | HIS | B | 888 | 39.526 | 22.784 | 45.370 | 1.00 | 26.59 |
| 3720 | CD2 | HIS | B | 888 | 38.459 | 24.559 | 44.674 | 1.00 | 23.24 |
| 3721 1 | CE1 | HIS | B | 888 | 39.495 | 22.726 | 44.050 | 1.00 | 27.50 |
| 3722 | NE2 | HIS | B | 888 | 38.854 | 23.791 | 43.603 | 1.00 | 29.04 |
| 3723 | N | LEU | B | 889 | 38.165 | 25.642 | 49.949 | 1.00 | 19.08 |
| 3724 | CA | LEU | B | 889 | 37.536 | 25.652 | 51.274 | 1.00 | 24.07 |
| 3725 | C | LEU | B | 889 | 36.812 | 26.977 | 51.535 | 1.00 | 23.35 |
| 3726 | O | LEU | B | 889 | 35.689 | 26.981 | 52.023 | 1.00 | 21.52 |
| 3727 | CB | LEU | B | 889 | 38.592 | 25.387 | 52.372 | 1.00 | 23.61 |
| 3728 | CG | LEU | B | 889 | 38.105 | 25.401 | 53.835 | 1.00 | 23.63 |
| 3729 | CD1 | LEU | B | 889 | 36.945 | 24.430 | 54.022 | 1.00 | 25.11 |
| 3730 | CD2 | LEU | B | 889 | 39.256 | 25.027 | 54.760 | 1.00 | 29.21 |
| 3731 | N | TYR | B | 890 | 37.445 | 28.098 | 51.193 | 1.00 | 21.91 |
| 3732 | CA | TYR | B | 890 | 36.828 | 29.408 | 51.404 | 1.00 | 23.79 |
| 3733 | C | TYR | B | 890 | 35.557 | 29.514 | 50.569 | 1.00 | 24.14 |
| 3734 | O | TYR | B | 890 | 34.534 | 30.029 | 51.035 | 1.00 | 23.58 |
| 3735 | CB | TYR | B | 890 | 37.813 | 30.531 | 51.026 | 1.00 | 25.81 |
| 3736 | CG | TYR | B | 890 | 37.301 | 31.930 | 51.332 | 1.00 | 26.23 |
| 3737 | CD1 | TYR | B | 890 | 36.308 | 32.528 | 50.547 | 1.00 | 23.51 |
| 3738 | CD2 | TYR | B | 890 | 37.781 | 32.636 | 52.432 | 1.00 | 29.47 |
| 3739 | CE1 | TYR | B | 890 | 35.813 | 33.793 | 50.861 | 1.00 | 26.90 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3740 | CE2 | TYR | B | 890 | 37.284 | 33.906 | 52.749 | 1.00 | 29.33 |
| 3741 | CZ | TYR | B | 890 | 36.303 | 34.471 | 51.957 | 1.00 | 27.90 |
| 3742 | OH | TYR | B | 890 | 35.814 | 35.716 | 52.265 | 1.00 | 28.09 |
| 3743 | N | CYS | B | 891 | 35.628 | 29.017 | 49.336 | 1.00 | 22.38 |
| 3744 | CA | CYS | B | 891 | 34.491 | 29.045 | 48.438 | 1.00 | 22.23 |
| 3745 | C | CYS | B | 891 | 33.338 | 28.199 | 48.976 | 1.00 | 19.96 |
| 3746 | O | CYS | B | 891 | 32.212 | 28.669 | 49.064 | 1.00 | 19.08 |
| 3747 | CB | CYS | B | 891 | 34.923 | 28.550 | 47.055 | 1.00 | 26.16 |
| 3748 | SG | CYS | B | 891 | 33.587 | 28.353 | 45.863 | 1.00 | 25.55 |
| 3749 | N | LEU | B | 892 | 33.618 | 26.958 | 49.366 | 1.00 | 20.12 |
| 3750 | CA | LEU | B | 892 | 32.574 | 26.101 | 49.889 | 1.00 | 19.88 |
| 3751 | C | LEU | B | 892 | 31.936 | 26.685 | 51.159 | 1.00 | 20.91 |
| 3752 | O | LEU | B | 892 | 30.718 | 26.605 | 51.329 | 1.00 | 20.98 |
| 3753 | CB | LEU | B | 892 | 33.126 | 24.689 | 50.166 | 1.00 | 22.12 |
| 3754 | CG | LEU | B | 892 | 32.067 | 23.682 | 50.640 | 1.00 | 28.56 |
| 3755 | CD1 | LEU | B | 892 | 30.955 | 23.585 | 49.580 | 1.00 | 23.62 |
| 3756 | CD2 | LEU | B | 892 | 32.719 | 22.316 | 50.887 | 1.00 | 24.56 |
| 3757 | N | ASN | B | 893 | 32.744 | 27.262 | 52.052 | 1.00 | 19.36 |
| 3758 | CA | ASN | B | 893 | 32.199 | 27.855 | 53.275 | 1.00 | 21.31 |
| 3759 | C | ASN | B | 893 | 31.329 | 29.044 | 52.897 | 1.00 | 23.26 |
| 3760 | O | ASN | B | 893 | 30.288 | 29.269 | 53.501 | 1.00 | 22.28 |
| 3761 | CB | ASN | B | 893 | 33.308 | 28.363 | 54.200 | 1.00 | 22.90 |
| 3762 | CG | ASN | B | 893 | 33.988 | 27.262 | 54.986 | 1.00 | 27.81 |
| 3763 | OD1 | ASN | B | 893 | 35.187 | 27.349 | 55.280 | 1.00 | 32.06 |
| 3764 | ND2 | ASN | B | 893 | 33.230 | 26.245 | 55.370 | 1.00 | 26.08 |
| 3765 | N | THR | B | 894 | 31.762 | 29.818 | 51.901 | 1.00 | 20.27 |
| 3766 | CA | THR | B | 894 | 30.993 | 30.989 | 51.511 | 1.00 | 21.51 |
| 3767 | C | THR | B | 894 | 29.705 | 30.546 | 50.822 | 1.00 | 20.80 |
| 3768 | O | THR | B | 894 | 28.660 | 31.175 | 50.977 | 1.00 | 21.06 |
| 3769 | CB | THR | B | 894 | 31.818 | 31.925 | 50.593 | 1.00 | 20.63 |
| 3770 | OG1 | THR | B | 894 | 32.987 | 32.359 | 51.301 | 1.00 | 25.68 |
| 3771 | CG2 | THR | B | 894 | 31.015 | 33.171 | 50.218 | 1.00 | 23.83 |
| 3772 | N | PHE | B | 895 | 29.779 | 29.436 | 50.096 | 1.00 | 19.69 |
| 3773 | CA | PHE | B | 895 | 28.614 | 28.901 | 49.381 | 1.00 | 21.30 |
| 3774 | C | PHE | B | 895 | 27.538 | 28.436 | 50.380 | 1.00 | 23.16 |
| 3775 | O | PHE | B | 895 | 26.345 | 28.723 | 50.231 | 1.00 | 22.53 |
| 3776 | CB | PHE | B | 895 | 29.059 | 27.715 | 48.517 | 1.00 | 20.99 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3777 | CG | PHE | B | 895 | 27.968 | 27.127 | 47.686 | 1.00 | 24.35 |
| 3778 | CD1 | PHE | B | 895 | 27.422 | 27.842 | 46.613 | 1.00 | 25.21 |
| 3779 | CD2 | PHE | B | 895 | 27.467 | 25.864 | 47.979 | 1.00 | 27.79 |
| 3780 | CE1 | PHE | B | 895 | 26.387 | 27.293 | 45.838 | 1.00 | 30.71 |
| 3781 | CE2 | PHE | B | 895 | 26.438 | 25.310 | 47.215 | 1.00 | 30.19 |
| 3782 | CZ | PHE | B | 895 | 25.897 | 26.024 | 46.146 | 1.00 | 29.55 |
| 3783 | N | ILE | B | 896 | 27.972 | 27.703 | 51.390 | 1.00 | 20.73 |
| 3784 | CA | ILE | B | 896 | 27.066 | 27.205 | 52.412 | 1.00 | 26.62 |
| 3785 | C | ILE | B | 896 | 26.416 | 28.363 | 53.166 | 1.00 | 24.12 |
| 3786 | O | ILE | B | 896 | 25.234 | 28.309 | 53.499 | 1.00 | 25.34 |
| 3787 | CB | ILE | B | 896 | 27.828 | 26.276 | 53.398 | 1.00 | 23.97 |
| 3788 | CG1 | ILE | B | 896 | 28.118 | 24.948 | 52.705 | 1.00 | 27.05 |
| 3789 | CG2 | ILE | B | 896 | 27.028 | 26.063 | 54.683 | 1.00 | 34.01 |
| 3790 | CD1 | ILE | B | 896 | 28.991 | 24.019 | 53.510 | 1.00 | 34.75 |
| 3791 | N | GLN | B | 897 | 27.173 | 29.427 | 53.403 | 1.00 | 19.74 |
| 3792 | CA | GLN | B | 897 | 26.645 | 30.573 | 54.145 | 1.00 | 23.53 |
| 3793 | C | GLN | B | 897 | 26.204 | 31.724 | 53.237 | 1.00 | 25.84 |
| 3794 | O | GLN | B | 897 | 26.002 | 32.848 | 53.713 | 1.00 | 26.80 |
| 3795 | CB | GLN | B | 897 | 27.722 | 31.081 | 55.111 | 1.00 | 26.51 |
| 3796 | CG | GLN | B | 897 | 28.384 | 29.972 | 55.924 | 1.00 | 26.00 |
| 3797 | CD | GLN | B | 897 | 29.571 | 30.449 | 56.734 | 1.00 | 34.46 |
| 3798 | OE1 | GLN | B | 897 | 30.451 | 29.655 | 57.089 | 1.00 | 37.93 |
| 3799 | NE2 | GLN | B | 897 | 29.601 | 31.741 | 57.051 | 1.00 | 32.90 |
| 3800 | N | SER | B | 898 | 26.014 | 31.448 | 51.950 | 1.00 | 24.26 |
| 3801 | CA | SER | B | 898 | 25.669 | 32.504 | 50.996 | 1.00 | 25.93 |
| 3802 | C | SER | B | 898 | 24.543 | 33.449 | 51.402 | 1.00 | 29.21 |
| 3803 | O | SER | B | 898 | 24.688 | 34.669 | 51.280 | 1.00 | 27.52 |
| 3804 | CB | SER | B | 898 | 25.364 | 31.917 | 49.613 | 1.00 | 23.86 |
| 3805 | OG | SER | B | 898 | 24.312 | 30.991 | 49.687 | 1.00 | 31.50 |
| 3806 | N | ARG | B | 899 | 23.433 | 32.904 | 51.896 | 1.00 | 24.74 |
| 3807 | CA | ARG | B | 899 | 22.307 | 33.752 | 52.292 | 1.00 | 32.34 |
| 3808 | C | ARG | B | 899 | 22.650 | 34.720 | 53.425 | 1.00 | 29.89 |
| 3809 | O | ARG | B | 899 | 22.342 | 35.910 | 53.355 | 1.00 | 33.92 |
| 3810 | CB | ARG | B | 899 | 21.103 | 32.902 | 52.733 | 1.00 | 31.79 |
| 3811 | CG | ARG | B | 899 | 20.414 | 32.070 | 51.652 | 1.00 | 45.87 |
| 3812 | CD | ARG | B | 899 | 19.077 | 31.532 | 52.204 | 1.00 | 50.81 |
| 3813 | NE | ARG | B | 899 | 18.248 | 30.819 | 51.231 | 1.00 | 61.64 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3814 | CZ | ARG | B | 899 | 17.821 | 31.324 | 50.075 | 1.00 | 63.01 |
| 3815 | NH1 | ARG | B | 899 | 18.143 | 32.564 | 49.717 | 1.00 | 64.60 |
| 3816 | NH2 | ARG | B | 899 | 17.052 | 30.589 | 49.281 | 1.00 | 64.49 |
| 3817 | N | ALA | B | 900 | 23.268 | 34.190 | 54.474 | 1.00 | 28.54 |
| 3818 | CA | ALA | B | 900 | 23.638 | 34.994 | 55.627 | 1.00 | 30.83 |
| 3819 | C | ALA | B | 900 | 24.669 | 36.051 | 55.269 | 1.00 | 35.27 |
| 3820 | O | ALA | B | 900 | 24.622 | 37.159 | 55.793 | 1.00 | 34.40 |
| 3821 | CB | ALA | B | 900 | 24.172 | 34.097 | 56.739 | 1.00 | 32.11 |
| 3822 | N | LEU | B | 901 | 25.580 | 35.720 | 54.354 | 1.00 | 30.18 |
| 3823 | CA | LEU | B | 901 | 26.628 | 36.645 | 53.939 | 1.00 | 32.20 |
| 3824 | C | LEU | B | 901 | 26.201 | 37.523 | 52.769 | 1.00 | 31.41 |
| 3825 | O | LEU | B | 901 | 26.950 | 38.401 | 52.343 | 1.00 | 34.81 |
| 3826 | CB | LEU | B | 901 | 27.873 | 35.861 | 53.525 | 1.00 | 32.10 |
| 3827 | CG | LEU | B | 901 | 28.546 | 34.937 | 54.535 | 1.00 | 35.51 |
| 3828 | CD1 | LEU | B | 901 | 29.567 | 34.057 | 53.797 | 1.00 | 33.35 |
| 3829 | CD2 | LEU | B | 901 | 29.207 | 35.746 | 55.635 | 1.00 | 35.37 |
| 3830 | N | SER | B | 902 | 25.008 | 37.271 | 52.236 | 1.00 | 29.08 |
| 3831 | CA | SER | B | 902 | 24.509 | 38.027 | 51.102 | 1.00 | 29.16 |
| 3832 | C | SER | B | 902 | 25.438 | 37.962 | 49.891 | 1.00 | 31.58 |
| 3833 | O | SER | B | 902 | 25.668 | 38.962 | 49.210 | 1.00 | 30.70 |
| 3834 | CB | SER | B | 902 | 24.278 | 39.488 | 51.505 | 1.00 | 35.73 |
| 3835 | OG | SER | B | 902 | 23.300 | 39.557 | 52.528 | 1.00 | 36.77 |
| 3836 | N | VAL | B | 903 | 25.991 | 36.779 | 49.641 | 1.00 | 28.81 |
| 3837 | CA | VAL | B | 903 | 26.857 | 36.572 | 48.486 | 1.00 | 28.11 |
| 3838 | C | VAL | B | 903 | 26.105 | 35.665 | 47.527 | 1.00 | 30.18 |
| 3839 | O | VAL | B | 903 | 25.799 | 34.521 | 47.851 | 1.00 | 37.08 |
| 3840 | CB | VAL | B | 903 | 28.183 | 35.891 | 48.870 | 1.00 | 28.45 |
| 3841 | CG1 | VAL | B | 903 | 28.995 | 35.583 | 47.604 | 1.00 | 24.13 |
| 3842 | CG2 | VAL | B | 903 | 28.998 | 36.809 | 49.759 | 1.00 | 23.64 |
| 3843 | N | GLU | B | 904 | 25.805 | 36.172 | 46.343 | 1.00 | 31.59 |
| 3844 | CA | GLU | B | 904 | 25.071 | 35.398 | 45.357 | 1.00 | 30.24 |
| 3845 | C | GLU | B | 904 | 25.993 | 34.542 | 44.497 | 1.00 | 33.23 |
| 3846 | O | GLU | B | 904 | 27.037 | 35.010 | 44.044 | 1.00 | 26.56 |
| 3847 | CB | GLU | B | 904 | 24.266 | 36.343 | 44.458 | 1.00 | 35.02 |
| 3848 | CG | GLU | B | 904 | 23.432 | 35.672 | 43.382 | 1.00 | 51.21 |
| 3849 | CD | GLU | B | 904 | 22.339 | 34.783 | 43.942 | 1.00 | 58.61 |
| 3850 | OE1 | GLU | B | 904 | 22.664 | 33.733 | 44.538 | 1.00 | 68.45 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3851 | OE2 | GLU | B | 904 | 21.149 | 35.143 | 43.794 | 1.00 | 71.56 |
| 3852 | N | PHE | B | 905 | 25.614 | 33.280 | 44.303 | 1.00 | 25.33 |
| 3853 | CA | PHE | B | 905 | 26.370 | 32.386 | 43.446 | 1.00 | 26.30 |
| 3854 | C | PHE | B | 905 | 25.461 | 32.046 | 42.280 | 1.00 | 25.19 |
| 3855 | O | PHE | B | 905 | 24.367 | 31.507 | 42.482 | 1.00 | 25.92 |
| 3856 | CB | PHE | B | 905 | 26.742 | 31.073 | 44.142 | 1.00 | 26.58 |
| 3857 | CG | PHE | B | 905 | 27.856 | 31.188 | 45.123 | 1.00 | 22.37 |
| 3858 | CD1 | PHE | B | 905 | 27.645 | 31.738 | 46.378 | 1.00 | 21.31 |
| 3859 | CD2 | PHE | B | 905 | 29.122 | 30.730 | 44.789 | 1.00 | 24.52 |
| 3860 | CE1 | PHE | B | 905 | 28.668 | 31.834 | 47.296 | 1.00 | 20.76 |
| 3861 | CE2 | PHE | B | 905 | 30.175 | 30.818 | 45.703 | 1.00 | 23.76 |
| 3862 | CZ | PHE | B | 905 | 29.946 | 31.370 | 46.958 | 1.00 | 25.94 |
| 3863 | N | PRO | B | 906 | 25.893 | 32.367 | 41.049 | 1.00 | 25.13 |
| 3864 | CA | PRO | B | 906 | 25.175 | 32.120 | 39.800 | 1.00 | 28.59 |
| 3865 | C | PRO | B | 906 | 24.942 | 30.642 | 39.577 | 1.00 | 25.62 |
| 3866 | O | PRO | B | 906 | 25.586 | 29.792 | 40.193 | 1.00 | 27.37 |
| 3867 | CB | PRO | B | 906 | 26.109 | 32.722 | 38.751 | 1.00 | 28.96 |
| 3868 | CG | PRO | B | 906 | 26.762 | 33.836 | 39.512 | 1.00 | 32.34 |
| 3869 | CD | PRO | B | 906 | 27.148 | 33.074 | 40.753 | 1.00 | 30.32 |
| 3870 | N | GLU | B | 907 | 24.030 | 30.353 | 38.663 | 1.00 | 29.57 |
| 3871 | CA | GLU | B | 907 | 23.615 | 28.999 | 38.312 | 1.00 | 30.56 |
| 3872 | C | GLU | B | 907 | 24.705 | 27.965 | 38.011 | 1.00 | 30.16 |
| 3873 | O | GLU | B | 907 | 24.777 | 26.925 | 38.671 | 1.00 | 28.20 |
| 3874 | CB | GLU | B | 907 | 22.673 | 29.097 | 37.111 | 1.00 | 39.44 |
| 3875 | CG | GLU | B | 907 | 21.926 | 27.827 | 36.761 | 1.00 | 50.34 |
| 3876 | CD | GLU | B | 907 | 21.408 | 27.863 | 35.333 | 1.00 | 55.65 |
| 3877 | OE1 | GLU | B | 907 | 20.837 | 28.902 | 34.929 | 1.00 | 53.82 |
| 3878 | OE2 | GLU | B | 907 | 21.573 | 26.850 | 34.620 | 1.00 | 56.79 |
| 3879 | N | MET | B | 908 | 25.537 | 28.224 | 37.005 | 1.00 | 27.50 |
| 3880 | CA | MET | B | 908 | 26.574 | 27.262 | 36.650 | 1.00 | 31.75 |
| 3881 | C | MET | B | 908 | 27.625 | 27.095 | 37.732 | 1.00 | 28.93 |
| 3882 | O | MET | B | 908 | 28.143 | 26.002 | 37.931 | 1.00 | 27.45 |
| 3883 | CB | MET | B | 908 | 27.259 | 27.633 | 35.326 | 1.00 | 36.79 |
| 3884 | CG | MET | B | 908 | 26.604 | 27.048 | 34.088 | 1.00 | 47.60 |
| 3885 | SD | MET | B | 908 | 27.569 | 27.345 | 32.563 | 1.00 | 70.60 |
| 3886 | CE | MET | B | 908 | 29.148 | 26.536 | 32.931 | 1.00 | 48.34 |
| 3887 | N | MET | B | 909 | 27.933 | 28.175 | 38.437 | 1.00 | 26.74 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3888 | CA | MET | B | 909 | 28.929 | 28.095 | 39.483 | 1.00 | 29.95 |
| 3889 | C | MET | B | 909 | 28.379 | 27.233 | 40.620 | 1.00 | 27.02 |
| 3890 | O | MET | B | 909 | 29.084 | 26.393 | 41.186 | 1.00 | 25.18 |
| 3891 | CB | MET | B | 909 | 29.277 | 29.500 | 39.966 | 1.00 | 29.74 |
| 3892 | CG | MET | B | 909 | 30.506 | 29.555 | 40.836 | 1.00 | 39.86 |
| 3893 | SD | MET | B | 909 | 31.104 | 31.254 | 40.979 | 1.00 | 43.66 |
| 3894 | CE | MET | B | 909 | 32.567 | 30.954 | 41.943 | 1.00 | 40.49 |
| 3895 | N | SER | B | 910 | 27.109 | 27.429 | 40.954 | 1.00 | 27.09 |
| 3896 | CA | SER | B | 910 | 26.511 | 26.632 | 42.015 | 1.00 | 25.16 |
| 3897 | C | SER | B | 910 | 26.487 | 25.178 | 41.595 | 1.00 | 25.86 |
| 3898 | O | SER | B | 910 | 26.669 | 24.286 | 42.416 | 1.00 | 23.23 |
| 3899 | CB | SER | B | 910 | 25.091 | 27.106 | 42.305 | 1.00 | 24.90 |
| 3900 | OG | SER | B | 910 | 25.101 | 28.461 | 42.699 | 1.00 | 26.02 |
| 3901 | N | GLU | B | 911 | 26.255 | 24.935 | 40.308 | 1.00 | 25.46 |
| 3902 | CA | GLU | B | 911 | 26.215 | 23.567 | 39.809 | 1.00 | 29.55 |
| 3903 | C | GLU | B | 911 | 27.564 | 22.863 | 39.976 | 1.00 | 29.76 |
| 3904 | O | GLU | B | 911 | 27.620 | 21.769 | 40.536 | 1.00 | 28.96 |
| 3905 | CB | GLU | B | 911 | 25.808 | 23.531 | 38.330 | 1.00 | 34.77 |
| 3906 | CG | GLU | B | 911 | 25.935 | 22.134 | 37.708 | 1.00 | 51.59 |
| 3907 | CD | GLU | B | 911 | 25.725 | 22.110 | 36.199 | 1.00 | 56.41 |
| 3908 | OE1 | GLU | B | 911 | 26.448 | 22.834 | 35.476 | 1.00 | 67.40 |
| 3909 | OE2 | GLU | B | 911 | 24.845 | 21.355 | 35.734 | 1.00 | 66.05 |
| 3910 | N | VAL | B | 912 | 28.644 | 23.474 | 39.494 | 1.00 | 25.36 |
| 3911 1 | CA | VAL | B | 912 | 29.959 | 22.850 | 39.619 | 1.00 | 29.35 |
| 3912 | C | VAL | B | 912 | 30.381 | 22.701 | 41.075 | 1.00 | 26.61 |
| 3913 | O | VAL | B | 912 | 31.067 | 21.740 | 41.429 | 1.00 | 29.13 |
| 3914 | CB | VAL | B | 912 | 31.097 | 23.635 | 38.865 | 1.00 | 29.04 |
| 3915 | CG1 | VAL | B | 912 | 30.780 | 23.735 | 37.383 | 1.00 | 29.38 |
| 3916 | CG2 | VAL | B | 912 | 31.285 | 25.015 | 39.470 | 1.00 | 27.40 |
| 3917 | N | ILE | B | 913 | 29.972 | 23.642 | 41.919 | 1.00 | 26.12 |
| 3918 | CA | ILE | B | 913 | 30.325 | 23.589 | 43.328 | 1.00 | 26.31 |
| 3919 | C | ILE | B | 913 | 29.612 | 22.421 | 44.012 | 1.00 | 27.59 |
| 3920 | O | ILE | B | 913 | 30.220 | 21.644 | 44.760 | 1.00 | 24.84 |
| 3921 | CB | ILE | B | 913 | 29.979 | 24.931 | 44.024 | 1.00 | 28.04 |
| 3922 | CG1 | ILE | B | 913 | 30.936 | 26.019 | 43.514 | 1.00 | 22.84 |
| 3923 | CG2 | ILE | B | 913 | 30.048 | 24.773 | 45.559 | 1.00 | 25.12 |
| 3924 | CD1 | ILE | B | 913 | 30.622 | 27.421 | 43.977 | 1.00 | 19.77 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3925 | N | ALA | B | 914 | 28.327 | 22.272 | 43.725 | 1.00 | 29.75 |
| 3926 | CA | ALA | B | 914 | 27.547 | 21.194 | 44.323 | 1.00 | 26.87 |
| 3927 | C | ALA | B | 914 | 27.931 | 19.836 | 43.770 | 1.00 | 29.99 |
| 3928 | O | ALA | B | 914 | 27.840 | 18.824 | 44.464 | 1.00 | 28.80 |
| 3929 | CB | ALA | B | 914 | 26.047 | 21.440 | 44.098 | 1.00 | 28.77 |
| 3930 | N | ALA | B | 915 | 28.372 | 19.794 | 42.521 | 1.00 | 27.03 |
| 3931 | CA | ALA | B | 915 | 28.718 | 18.514 | 41.917 | 1.00 | 27.59 |
| 3932 | C | ALA | B | 915 | 30.015 | 17.884 | 42.383 | 1.00 | 28.38 |
| 3933 | O | ALA | B | 915 | 30.111 | 16.664 | 42.427 | 1.00 | 34.38 |
| 3934 | CB | ALA | B | 915 | 28.733 | 18.633 | 40.389 | 1.00 | 24.76 |
| 3935 | N | GLN | B | 916 | 31.007 | 18.689 | 42.735 | 1.00 | 25.09 |
| 3936 | CA | GLN | B | 916 | 32.291 | 18.118 | 43.124 | 1.00 | 26.91 |
| 3937 | C | GLN | B | 916 | 33.073 | 18.705 | 44.293 | 1.00 | 25.33 |
| 3938 | O | GLN | B | 916 | 33.931 | 18.022 | 44.863 | 1.00 | 29.86 |
| 3939 | CB | GLN | B | 916 | 33.226 | 18.133 | 41.909 | 1.00 | 23.65 |
| 3940 | CG | GLN | B | 916 | 32.752 | 17.337 | 40.694 | 1.00 | 30.20 |
| 3941 | CD | GLN | B | 916 | 32.436 | 15.889 | 41.034 | 1.00 | 30.77 |
| 3942 | OE1 | GLN | B | 916 | 33.180 | 15.235 | 41.756 | 1.00 | 36.33 |
| 3943 | NE2 | GLN | B | 916 | 31.331 | 15.383 | 40.503 | 1.00 | 45.24 |
| 3944 | N | LEU | B | 917 | 32.810 | 19.951 | 44.665 | 1.00 | 27.46 |
| 3945 | CA | LEU | B | 917 | 33.629 | 20.558 | 45.700 | 1.00 | 23.85 |
| 3946 | C | LEU | B | 917 | 33.803 | 19.765 | 46.998 | 1.00 | 26.12 |
| 3947 | O | LEU | B | 917 | 34.930 | 19.611 | 47.484 | 1.00 | 23.20 |
| 3948 | CB | LEU | B | 917 | 33.148 | 21.987 | 45.970 | 1.00 | 32.48 |
| 3949 | CG | LEU | B | 917 | 34.234 | 22.886 | 46.551 | 1.00 | 27.86 |
| 3950 | CD1 | LEU | B | 917 | 35.492 | 22.792 | 45.701 | 1.00 | 32.98 |
| 3951 | CD2 | LEU | B | 917 | 33.750 | 24.346 | 46.572 | 1.00 | 37.82 |
| 3952 | N | PRO | B | 918 | 32.711 | 19.247 | 47.585 | 1.00 | 21.61 |
| 3953 | CA | PRO | B | 918 | 32.919 | 18.490 | 48.822 | 1.00 | 26.49 |
| 3954 | C | PRO | B | 918 | 33.838 | 17.292 | 48.586 | 1.00 | 24.53 |
| 3955 | O | PRO | B | 918 | 34.769 | 17.037 | 49.354 | 1.00 | 24.08 |
| 3956 | CB | PRO | B | 918 | 31.498 | 18.053 | 49.194 | 1.00 | 25.82 |
| 3957 | CG | PRO | B | 918 | 30.659 | 19.178 | 48.629 | 1.00 | 27.24 |
| 3958 | CD | PRO | B | 918 | 31.276 | 19.281 | 47.251 | 1.00 | 24.22 |
| 3959 | N | LYS | B | 919 | 33.567 | 16.567 | 47.509 | 1.00 | 23.53 |
| 3960 | CA | LYS | B | 919 | 34.339 | 15.381 | 47.159 | 1.00 | 26.07 |
| 3961 | C | LYS | B | 919 | 35.811 | 15.729 | 46.938 | 1.00 | 23.29 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3962 | O | LYS | B | 919 | 36.712 | 14.988 | 47.352 | 1.00 | 24.01 |
| 3963 | CB | LYS | B | 919 | 33.756 | 14.738 | 45.898 | 1.00 | 29.21 |
| 3964 | CG | LYS | B | 919 | 34.393 | 13.393 | 45.544 | 1.00 | 35.47 |
| 3965 | CD | LYS | B | 919 | 33.972 | 12.944 | 44.145 | 1.00 | 43.92 |
| 3966 | CE | LYS | B | 919 | 32.471 | 12.899 | 43.995 | 1.00 | 47.04 |
| 3967 | NZ | LYS | B | 919 | 32.081 | 12.653 | 42.573 | 1.00 | 55.56 |
| 3968 | N | ILE | B | 920 | 36.058 | 16.857 | 46.281 | 1.00 | 21.17 |
| 3969 | CA | ILE | B | 920 | 37.423 | 17.312 | 46.022 | 1.00 | 24.61 |
| 3970 | C | ILE | B | 920 | 38.167 | 17.663 | 47.320 | 1.00 | 26.60 |
| 3971 | O | ILE | B | 920 | 39.315 | 17.264 | 47.506 | 1.00 | 30.19 |
| 3972 | CB | ILE | B | 920 | 37.430 | 18.561 | 45.091 | 1.00 | 22.46 |
| 3973 | CG1 | ILE | B | 920 | 37.000 | 18.162 | 43.681 | 1.00 | 26.76 |
| 3974 | CG2 | ILE | B | 920 | 38.824 | 19.186 | 45.042 | 1.00 | 28.46 |
| 3975 | CD1 | ILE | B | 920 | 36.707 | 19.354 | 42.795 | 1.00 | 29.32 |
| 3976 | N | LEU | B | 921 | 37.515 | 18.396 | 48.214 | 1.00 | 26.87 |
| 3977 | CA | LEU | B | 921 | 38.147 | 18.790 | 49.473 | 1.00 | 31.04 |
| 3978 | C | LEU | B | 921 | 38.414 | 17.603 | 50.375 | 1.00 | 30.07 |
| 3979 | O | LEU | B | 921 | 39.377 | 17.604 | 51.141 | 1.00 | 33.85 |
| 3980 | CB | LEU | B | 921 | 37.273 | 19.809 | 50.223 | 1.00 | 28.75 |
| 3981 | CG | LEU | B | 921 | 37.402 | 21.287 | 49.868 | 1.00 | 31.61 |
| 3982 | CD1 | LEU | B | 921 | 36.346 | 22.078 | 50.621 | 1.00 | 31.82 |
| 3983 | CD2 | LEU | B | 921 | 38.809 | 21.784 | 50.244 | 1.00 | 28.69 |
| 3984 | N | ALA | B | 922 | 37.570 | 16.579 | 50.280 | 1.00 | 28.13 |
| 3985 | CA | ALA | B | 922 | 37.735 | 15.389 | 51.108 | 1.00 | 28.58 |
| 3986 | C | ALA | B | 922 | 38.824 | 14.481 | 50.571 | 1.00 | 30.58 |
| 3987 | O | ALA | B | 922 | 39.070 | 13.418 | 51.125 | 1.00 | 32.70 |
| 3988 | CB | ALA | B | 922 | 36.433 | 14.626 | 51.185 | 1.00 | 28.32 |
| 3989 | N | GLY | B | 923 | 39.450 | 14.896 | 49.477 | 1.00 | 28.46 |
| 3990 | CA | GLY | B | 923 | 40.503 | 14.101 | 48.863 | 1.00 | 30.11 |
| 3991 | C | GLY | B | 923 | 39.990 | 12.843 | 48.197 | 1.00 | 29.47 |
| 3992 | O | GLY | B | 923 | 40.736 | 11.881 | 48.014 | 1.00 | 33.11 |
| 3993 | N | MET | B | 924 | 38.719 | 12.827 | 47.825 | 1.00 | 27.25 |
| 3994 | CA | MET | B | 924 | 38.156 | 11.635 | 47.202 | 1.00 | 28.82 |
| 3995 | C | MET | B | 924 | 38.222 | 11.681 | 45.680 | 1.00 | 30.78 |
| 3996 | O | MET | B | 924 | 37.290 | 11.270 | 44.993 | 1.00 | 27.46 |
| 3997 | CB | MET | B | 924 | 36.720 | 11.416 | 47.688 | 1.00 | 30.45 |
| 3998 | CG | MET | B | 924 | 36.664 | 11.091 | 49.176 | 1.00 | 35.29 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 3999 | SD | MET | B | 924 | 37.558 | 9.556 | 49.557 | 1.00 | 43.72 |
| 4000 | CE | MET | B | 924 | 37.438 | 9.477 | 51.374 | 1.00 | 47.71 |
| 4001 | N | VAL | B | 925 | 39.358 | 12.173 | 45.186 | 1.00 | 29.26 |
| 4002 | CA | VAL | B | 925 | 39.670 | 12.280 | 43.763 | 1.00 | 28.51 |
| 4003 | C | VAL | B | 925 | 41.164 | 11.985 | 43.652 | 1.00 | 30.21 |
| 4004 | O | VAL | B | 925 | 41.843 | 11.802 | 44.661 | 1.00 | 29.32 |
| 4005 | CB | VAL | B | 925 | 39.407 | 13.709 | 43.208 | 1.00 | 23.55 |
| 4006 | CG1 | VAL | B | 925 | 37.928 | 14.070 | 43.363 | 1.00 | 29.41 |
| 4007 | CG2 | VAL | B | 925 | 40.300 | 14.720 | 43.912 | 1.00 | 27.96 |
| 4008 | N | LYS | B | 926 | 41.687 | 11.946 | 42.437 | 1.00 | 29.24 |
| 4009 | CA | LYS | B | 926 | 43.102 | 11.665 | 42.270 | 1.00 | 31.24 |
| 4010 | C | LYS | B | 926 | 43.886 | 12.774 | 41.590 | 1.00 | 31.61 |
| 4011 | O | LYS | B | 926 | 43.894 | 12.902 | 40.364 | 1.00 | 27.27 |
| 4012 | CB | LYS | B | 926 | 43.296 | 10.350 | 41.514 | 1.00 | 33.46 |
| 4013 | CG | LYS | B | 926 | 44.744 | 10.011 | 41.236 | 1.00 | 39.59 |
| 4014 | CD | LYS | B | 926 | 44.880 | 8.556 | 40.803 | 1.00 | 46.42 |
| 4015 | CE | LYS | B | 926 | 43.990 | 8.236 | 39.618 | 1.00 | 52.50 |
| 4016 | NZ | LYS | B | 926 | 44.036 | 6.792 | 39.270 | 1.00 | 54.88 |
| 4017 | N | PRO | B | 927 | 44.523 | 13.628 | 42.394 | 1.00 | 30.89 |
| 4018 | CA | PRO | B | 927 | 45.314 | 14.714 | 41.836 | 1.00 | 31.60 |
| 4019 | C | PRO | B | 927 | 46.506 | 14.074 | 41.149 | 1.00 | 36.21 |
| 4020 | O | PRO | B | 927 | 47.058 | 13.088 | 41.639 | 1.00 | 32.44 |
| 4021 | CB | PRO | B | 927 | 45,704 | 15.511 | 43.078 | 1.00 | 38.96 |
| 4022 | CG | PRO | B | 927 | 45.811 | 14.413 | 44.131 | 1.00 | 38.64 |
| 4023 | CD | PRO | B | 927 | 44.507 | 13.713 | 43.864 | 1.00 | 33.53 |
| 4024 | N | LEU | B | 928 | 46.886 | 14.609 | 40.000 | 1.00 | 33.51 |
| 4025 | CA | LEU | B | 928 | 48.022 | 14.056 | 39.280 | 1.00 | 36.50 |
| 4026 | C | LEU | B | 928 | 49.230 | 14.937 | 39.545 | 1.00 | 38.84 |
| 4027 | O | LEU | B | 928 | 49.217 | 16.135 | 39.276 | 1.00 | 41.78 |
| 4028 | CB | LEU | B | 928 | 47.716 | 13.980 | 37.786 | 1.00 | 29.72 |
| 4029 | CG | LEU | B | 928 | 46.444 | 13.187 | 37.476 | 1.00 | 29.00 |
| 4030 | CD1 | LEU | B | 928 | 46.220 | 13.130 | 35.965 | 1.00 | 29.19 |
| 4031 | CD2 | LEU | B | 928 | 46.554 | 11.788 | 38.059 | 1.00 | 32.16 |
| 4032 | N | LEU | B | 929 | 50.270 | 14.334 | 40.101 | 1.00 | 35.29 |
| 4033 | CA | LEU | B | 929 | 51.479 | 15.070 | 40.416 | 1.00 | 34.92 |
| 4034 | C | LEU | B | 929 | 52.582 | 14.697 | 39.446 | 1.00 | 32.09 |
| 4035 | O | LEU | B | 929 | 52.660 | 13.562 | 38.979 | 1.00 | 31.61 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 4036 | CB | LEU | B | 929 | 51.921 | 14.753 | 41.848 | 1.00 | 37.92 |
| 4037 | CG | LEU | B | 929 | 51.014 | 15.258 | 42.970 | 1.00 | 40.28 |
| 4038 | CD1 | LEU | B | 929 | 51.465 | 14.689 | 44.295 | 1.00 | 44.21 |
| 4039 | CD2 | LEU | B | 929 | 51.055 | 16.782 | 42.992 | 1.00 | 46.14 |
| 4040 | N | PHE | B | 930 | 53.425 | 15.666 | 39.134 | 1.00 | 33.50 |
| 4041 | CA | PHE | B | 930 | 54.534 | 15.426 | 38.235 | 1.00 | 37.57 |
| 4042 | C | PHE | B | 930 | 55.723 | 14.904 | 39.021 | 1.00 | 38.45 |
| 4043 | O | PHE | B | 930 | 56.527 | 14.133 | 38.499 | 1.00 | 37.59 |
| 4044 | CB | PHE | B | 930 | 54.929 | 16.710 | 37.515 | 1.00 | 36.92 |
| 4045 | CG | PHE | B | 930 | 53.861 | 17.252 | 36.624 | 1.00 | 36.88 |
| 4046 | CD1 | PHE | B | 930 | 52.880 | 18.103 | 37.123 | 1.00 | 34.19 |
| 4047 | CD2 | PHE | B | 930 | 53.817 | 16.887 | 35.281 | 1.00 | 41.45 |
| 4048 | CE1 | PHE | B | 930 | 51.869 | 18.587 | 36.286 | 1.00 | 44.09 |
| 4049 | CE2 | PHE | B | 930 | 52.810 | 17.364 | 34.440 | 1.00 | 42.91 |
| 4050 | CZ | PHE | B | 930 | 51.838 | 18.213 | 34.943 | 1.00 | 39.56 |
| 4051 | N | HIS | B | 931 | 55.803 | 15.311 | 40.285 | 1.00 | 36.07 |
| 4052 | CA | HIS | B | 931 | 56.903 | 14.917 | 41.149 | 1.00 | 41.47 |
| 4053 | C | HIS | B | 931 | 56.445 | 14.260 | 42.446 | 1.00 | 41.27 |
| 4054 | O | HIS | B | 931 | 55.401 | 14.697 | 42.972 | 1.00 | 45.00 |
| 4055 | CB | HIS | B | 931 | 57.750 | 16.151 | 41.448 | 1.00 | 40.17 |
| 4056 | CG | HIS | B | 931 | 58.212 | 16.864 | 40.215 | 1.00 | 48.97 |
| 4057 | ND1 | HIS | B | 931 | 58.951 | 16.244 | 39.230 | 1.00 | 44.03 |
| 4058 | CD2 | HIS | B | 931 | 58.025 | 18.140 | 39.798 | 1.00 | 47.89 |
| 4059 | CE1 | HIS | B | 931 | 59.199 | 17.106 | 38.260 | 1.00 | 49.04 |
| 4060 | NE2 | HIS | B | 931 | 58.650 | 18.264 | 38.581 | 1.00 | 50.90 |
| 4061 | | HIS | B | 931 | | | | | |
| 4062 | C1 | STR | B | 2 | 36.722 | 34.750 | 39.821 | 1.00 | 23.01 |
| 4063 | C2 | STR | B | 2 | 37.383 | 34.885 | 38.411 | 1.00 | 30.18 |
| 4064 | C3 | STR | B | 2 | 38.878 | 35.052 | 38.581 | 1.00 | 29.08 |
| 4065 | 03 | STR | B | 2 | 39.501 | 35.761 | 37.778 | 1.00 | 35.44 |
| 4066 | C4 | STR | B | 2 | 39.577 | 34.319 | 39.647 | 1.00 | 26.40 |
| 4067 | C5 | STR | B | 2 | 38.911 | 33.608 | 40.559 | 1.00 | 25.48 |
| 4068 | C6 | STR | B | 2 | 39.700 | 32.787 | 41.592 | 1.00 | 26.28 |
| 4069 | C7 | STR | B | 2 | 39.171 | 32.980 | 43.035 | 1.00 | 22.37 |
| 4070 | C8 | STR | B | 2 | 37.627 | 32.810 | 43.155 | 1.00 | 23.98 |
| 4071 | C9 | STR | B | 2 | 36.932 | 33.817 | 42.168 | 1.00 | 20.64 |
| 4072 | C10 | STR | B | 2 | 37.329 | 33.590 | 40.661 | 1.00 | 20.19 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 4073 | C11 | STR | B | 2 | 35.390 | 33.811 | 42.356 | 1.00 | 21.87 |
| 4074 | C12 | STR | B | 2 | 34.970 | 34.064 | 43.833 | 1.00 | 22.64 |
| 4075 | C13 | STR | B | 2 | 35.601 | 33.021 | 44.767 | 1.00 | 22.97 |
| 4076 | C14 | STR | B | 2 | 37.164 | 33.157 | 44.578 | 1.00 | 24.84 |
| 4077 | C15 | STR | B | 2 | 37.755 | 32.350 | 45.755 | 1.00 | 27.19 |
| 4078 | C16 | STR | B | 2 | 36.767 | 32.674 | 46.938 | 1.00 | 28.64 |
| 4079 | C17 | STR | B | 2 | 35.529 | 33.364 | 46.288 | 1.00 | 24.63 |
| 4080 | C18 | STR | B | 2 | 35.053 | 31.558 | 44.475 | 1.00 | 18.18 |
| 4081 | C19 | STR | B | 2 | 36.852 | 32.203 | 40.113 | 1.00 | 24.00 |
| 4082 | C20 | STR | B | 2 | 34.178 | 33.098 | 46.985 | 1.00 | 26.21 |
| 4083 | 020 | STR | B | 2 | 34.028 | 32.147 | 47.721 | 1.00 | 30.43 |
| 4084 | C21 | STR | B | 2 | 33.060 | 34.033 | 46.751 | 1.00 | 25.53 |
| 4085 | O | HOH | | 1000 | 33.666 | 17.404 | 87.251 | 1.00 | 22.19 |
| 4086 | O | HOH | | 1001 | 27.991 | 7.945 | 79.484 | 1.00 | 25.72 |
| 4087 | O | HOH | | 1002 | 16.741 | 15.065 | 72.689 | 1.00 | 29.58 |
| 4088 | O | HOH | | 1003 | 36.823 | 15.614 | 69.042 | 1.00 | 23.68 |
| 4089 | O | HOH | | 1004 | 23.313 | 7.474 | 68.146 | 1.00 | 21.10 |
| 4090 | O | HOH | | 1005 | 45.633 | 27.074 | 27.131 | 1.00 | 31.65 |
| 4091 | O | HOH | | 1006 | 37.862 | 19.992 | 22.137 | 1.00 | 22.34 |
| 4092 | O | HOH | | 1007 | 30.655 | 16.369 | 46.065 | 1.00 | 32.92 |
| 4093 | O | HOH | | 1008 | 27.171 | 36.648 | 41.698 | 1.00 | 25.15 |
| 4094 | O | HOH | | 1009 | 44.296 | 28.506 | 25.056 | 1.00 | 24.87 |
| 4095 | O | HOH | | 1010 | 17.917 | 6.774 | 69.155 | 1.00 | 22.62 |
| 4096 | O | HOH | | 1011 | 37.660 | 29.818 | 22.972 | 1.00 | 27.17 |
| 4097 | O | HOH | | 1012 | 37.688 | 23.686 | 40.855 | 1.00 | 27.87 |
| 4098 | O | HOH | | 1013 | 38.419 | 38.595 | 33.456 | 1.00 | 25.90 |
| 4099 | O | HOH | | 1014 | 30.427 | 9.090 | 51.398 | 1.00 | 29.15 |
| 4100 | O | HOH | | 1015 | 58.710 | 17.046 | 23.597 | 1.00 | 27.03 |
| 4101 | O | HOH | | 1016 | 33.659 | 21.949 | 41.759 | 1.00 | 25.64 |
| 4102 | O | HOH | | 1017 | 32.808 | 13.290 | 64.116 | 1.00 | 28.14 |
| 4103 | O | HOH | | 1018 | 10.078 | 12.182 | 57.163 | 1.00 | 32.35 |
| 4104 | O | HOH | | 1019 | 42.113 | 19.486 | 38.248 | 1.00 | 34.91 |
| 4105 | O | HOH | | 1020 | 41.094 | 18.002 | 36.209 | 1.00 | 26.81 |
| 4106 | O | HOH | | 1021 | 30.979 | 15.363 | 63.422 | 1.00 | 28.79 |
| 4107 | O | HOH | | 1022 | 36.936 | 29.095 | 20.508 | 1.00 | 35.94 |
| 4108 | O | HOH | | 1023 | 40.497 | 23.491 | 40.659 | 1.00 | 23.11 |
| 4109 | O | HOH | | 1024 | 33.929 | 24.678 | 57.710 | 1.00 | 38.13 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 4110 | O | HOH | | 1025 | 40.676 | 33.721 | 35.327 | 1.00 | 27.89 |
| 4111 | O | HOH | | 1026 | 40.680 | 28.650 | 53.024 | 1.00 | 33.04 |
| 4112 | O | HOH | | 1027 | 36.369 | 13.680 | 67.537 | 1.00 | 39.82 |
| 4113 | O | HOH | | 1028 | 34.722 | -5.251 | 80.392 | 1.00 | 32.81 |
| 4114 | O | HOH | | 1029 | 39.676 | -3.503 | 86.330 | 1.00 | 36.01 |
| 4115 | O | HOH | | 1030 | 27.842 | 22.939 | 85.773 | 1.00 | 44.76 |
| 4116 | O | HOH | | 1031 | 62.824 | 21.691 | 26.549 | 1.00 | 33.63 |
| 4117 | O | HOH | | 1032 | 16.427 | -9.397 | 69.013 | 1.00 | 54.60 |
| 4118 | O | HOH | | 1033 | 36.222 | 14.728 | 63.426 | 1.00 | 51.82 |
| 4119 | O | HOH | | 1034 | 43.556 | 21.247 | 45.108 | 1.00 | 29.39 |
| 4120 | O | HOH | | 1035 | 41.341 | 28.480 | 29.854 | 1.00 | 39.90 |
| 4121 | O | HOH | | 1036 | 51.585 | 48.214 | 48.363 | 1.00 | 30.49 |
| 4122 | O | HOH | | 1037 | 22.687 | 4.551 | 72.387 | 1.00 | 36.18 |
| 4123 | O | HOH | | 1038 | 59.098 | 29.600 | 26.109 | 1.00 | 35.27 |
| 4124 | O | HOH | | 1039 | 55.684 | 35.659 | 26.274 | 1.00 | 42.35 |
| 4125 | O | HOH | | 1040 | 31.061 | 18.050 | 82.280 | 1.00 | 21.99 |
| 4126 | O | HOH | | 1041 | 43.993 | 34.999 | 31.570 | 1.00 | 42.40 |
| 4127 | O | HOH | | 1042 | 12.626 | 17.393 | 61.026 | 1.00 | 33.69 |
| 4128 | O | HOH | | 1043 | 42.167 | 23.707 | 63.170 | 1.00 | 51.66 |
| 4129 | O | HOH | | 1044 | 30.801 | 12.191 | 46.877 | 1.00 | 44.64 |
| 4130 | O | HOH | | 1045 | 48.081 | 49.753 | 47.195 | 1.00 | 35.43 |
| 4131 | O | HOH | | 1046 | 30.363 | 26.610 | 56.223 | 1.00 | 32.27 |
| 4132 | O | HOH | | 1047 | 37.038 | 10.030 | 42.705 | 1.00 | 33.51 |
| 4133 | O | HOH | | 1048 | 23.093 | 13.225 | 81.215 | 1.00 | 42.96 |
| 4134 | O | HOH | | 1049 | 33.482 | 20.462 | 20.296 | 1.00 | 36.45 |
| 4135 | O | HOH | | 1050 | 14.055 | 13.721 | 72.306 | 1.00 | 45.30 |
| 4136 | O | HOH | | 1051 | 28.811 | 43.105 | 52.857 | 1.00 | 63.86 |
| 4137 | O | HOH | | 1052 | 47.646 | 34.627 | 52.909 | 1.00 | 36.00 |
| 4138 | O | HOH | | 1053 | 49.719 | 10.586 | 22.669 | 1.00 | 44.77 |
| 4139 | O | HOH | | 1054 | 43.185 | 10.462 | 60.207 | 1.00 | 48.26 |
| 4140 | O | HOH | | 1055 | 27.182 | 6.289 | 49.610 | 1.00 | 52.31 |
| 4141 | O | HOH | | 1056 | 70.880 | 15.984 | 30.217 | 1.00 | 46.28 |
| 4142 | O | HOH | | 1057 | 35.128 | 4.502 | 35.039 | 1.00 | 51.40 |
| 4143 | O | HOH | | 1058 | 14.812 | 16.700 | 59.385 | 1.00 | 33.36 |
| 4144 | O | HOH | | 1059 | 46.460 | 34.689 | 40.532 | 1.00 | 34.71 |
| 4145 | O | HOH | | 1060 | 49.051 | 47.265 | 44.651 | 1.00 | 28.92 |
| 4146 | O | HOH | | 1061 | 45.167 | 51.194 | 41.329 | 1.00 | 46.31 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 4147 | O | HOH | | 1062 | 29.592 | -4.047 | 56.348 | 1.00 | 37.89 |
| 4148 | O | HOH | | 1063 | 32.458 | 36.767 | 54.252 | 1.00 | 63.27 |
| 4149 | O | HOH | | 1064 | 30.664 | 20.362 | 27.986 | 1.00 | 53.78 |
| 4150 | O | HOH | | 1065 | 37.433 | -13.669 | 66.706 | 1.00 | 65.87 |
| 4151 | O | HOH | | 1066 | 36.201 | 30.984 | 24.993 | 1.00 | 38.56 |
| 4152 | O | HOH | | 1067 | 46.351 | 13.151 | 75.804 | 1.00 | 55.39 |
| 4153 | O | HOH | | 1068 | 33.456 | 12.303 | 40.428 | 1.00 | 58.95 |
| 4154 | O | HOH | | 1069 | 28.321 | 22.637 | 27.122 | 1.00 | 47.82 |
| 4155 | O | HOH | | 1070 | 40.552 | 14.140 | 55.956 | 1.00 | 53.39 |
| 4156 | O | HOH | | 1071 | 26.098 | 12.641 | 88.207 | 1.00 | 61.83 |
| 4157 | O | HOH | | 1072 | 48.503 | 17.493 | 34.436 | 1.00 | 48.80 |
| 4158 | O | HOH | | 1073 | 42.177 | 48.877 | 43.220 | 1.00 | 27.83 |
| 4159 | O | HOH | | 1074 | 38.062 | 47.031 | 49.044 | 1.00 | 44.21 |
| 4160 | O | HOH | | 1075 | 14.659 | 30.053 | 47.390 | 1.00 | 50.38 |
| 4161 | O | HOH | | 1076 | 23.348 | 29.500 | 45.003 | 1.00 | 31.93 |
| 4162 | O | HOH | | 1077 | 17.005 | 32.707 | 54.248 | 1.00 | 54.07 |
| 4163 | O | HOH | | 1078 | 13.257 | 10.576 | 47.601 | 1.00 | 41.84 |
| 4164 | O | HOH | | 1079 | 30.618 | -5.578 | 58.841 | 1.00 | 41.51 |
| 4165 | O | HOH | | 1080 | 36.612 | 5.182 | 88.679 | 1.00 | 30.35 |
| 4166 | O | HOH | | 1081 | 35.761 | 31.204 | 55.444 | 1.00 | 49.87 |
| 4167 | O | HOH | | 1082 | 32.381 | 16.046 | 22.635 | 1.00 | 37.44 |
| 4168 | O | HOH | | 1083 | 43.620 | 28.101 | 82.401 | 1.00 | 54.55 |
| 4169 | O | HOH | | 1084 | 8.058 | 8.892 | 73.013 | 1.00 | 55.15 |
| 4170 | O | HOH | | 1085 | 35.421 | -7.443 | 88.230 | 1.00 | 49.25 |
| 4171 | O | HOH | | 1086 | 27.342 | 7.972 | 45.641 | 1.00 | 50.17 |
| 4172 | O | HOH | | 1087 | 30.122 | 26.584 | 24.720 | 1.00 | 34.71 |
| 4173 | O | HOH | | 1088 | 26.449 | 11.806 | 48.387 | 1.00 | 51.75 |
| 4174 | O | HOH | | 1089 | 16.902 | 21.688 | 55.310 | 1.00 | 35.02 |
| 4175 | O | HOH | | 1090 | 39.032 | 18.772 | 82.856 | 1.00 | 38.44 |
| 4176 | O | HOH | | 1091 | 33.889 | -4.827 | 60.832 | 1.00 | 48.06 |
| 4177 | O | HOH | | 1092 | 52.975 | 40.371 | 51.817 | 1.00 | 61.64 |
| 4178 | O | HOH | | 1093 | 27.906 | 51.315 | 40.663 | 1.00 | 46.27 |
| 4179 | O | HOH | | 1094 | 42.167 | 18.770 | 42.670 | 1.00 | 58.14 |
| 4180 | O | HOH | | 1095 | 23.080 | 39.080 | 43.811 | 1.00 | 64.06 |
| 4181 | O | HOH | | 1096 | 34.247 | 25.343 | 19.682 | 1.00 | 42.03 |
| 4182 | O | HOH | | 1097 | 40.472 | 41.737 | 55.433 | 1.00 | 44.89 |
| 4183 | O | HOH | | 1098 | 56.942 | 34.297 | 20.120 | 1.00 | 58.67 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 4184 | O | HOH | | 1099 | 66.188 | 19.205 | 40.041 | 1.00 | 58.84 |
| 4185 | O | HOH | | 1100 | 36.630 | 11.860 | 62.910 | 1.00 | 37.72 |
| 4186 | O | HOH | | 1101 | 26.825 | 18.353 | 46.932 | 1.00 | 40.45 |
| 4187 | O | HOH | | 1102 | 22.250 | 32.330 | 37.131 | 1.00 | 53.43 |
| 4188 | O | HOH | | 1103 | 57.886 | 40.754 | 42.618 | 1.00 | 42.31 |
| 4189 | O | HOH | | 1104 | 46.731 | 16.884 | 21.013 | 1.00 | 59.18 |
| 4190 | O | HOH | | 1105 | 25.096 | 10.702 | 74.366 | 1.00 | 49.26 |
| 4191 | O | HOH | | 1106 | 41.831 | 26.244 | 69.694 | 1.00 | 50.01 |
| 4192 | O | HOH | | 1107 | 24.979 | 14.834 | 86.465 | 1.00 | 58.25 |
| 4193 | O | HOH | | 1108 | 42.044 | -18.293 | 88.405 | 1.00 | 53.84 |
| 4194 | O | HOH | | 1109 | 60.868 | 31.903 | 29.340 | 1.00 | 56.15 |
| 4195 | O | HOH | | 1110 | 47.828 | 8.529 | 78.005 | 1.00 | 57.38 |
| 4196 | O | HOH | | 1111 | 30.517 | 10.898 | 25.227 | 1.00 | 43.12 |
| 4197 | O | HOH | | 1112 | 26.673 | 45.793 | 49.522 | 1.00 | 42.53 |
| 4198 | O | HOH | | 1113 | 40.395 | 7.287 | 85.552 | 1.00 | 34.61 |
| 4199 | O | HOH | | 1114 | 36.002 | 50.942 | 48.176 | 1.00 | 55.29 |
| 4200 | O | HOH | | 1115 | 15.517 | 1.041 | 72.791 | 1.00 | 32.07 |
| 4201 | O | HOH | | 1116 | 24.858 | 15.134 | 26.431 | 1.00 | 49.39 |
| 4202 | O | HOH | | 1117 | 31.562 | 34.334 | 28.869 | 1.00 | 36.46 |
| 4203 | O | HOH | | 1118 | 35.859 | -12.062 | 72.482 | 1.00 | 54.03 |
| 4204 | O | HOH | | 1119 | 57.184 | 43.582 | 38.922 | 1.00 | 42.30 |
| 4205 | O | HOH | | 1120 | 20.561 | -8.563 | 70.577 | 1.00 | 60.48 |
| 4206 | O | HOH | | 1121 | 39.714 | -9.373 | 72.709 | 1.00 | 42.35 |
| 4207 | O | HOH | | 1122 | 29.084 | 21.194 | 77.543 | 1.00 | 55.53 |
| 4208 | O | HOH | | 1123 | 26.622 | 15.926 | 35.036 | 1.00 | 49.36 |
| 4209 | O | HOH | | 1124 | 21.471 | 9.364 | 71.434 | 1.00 | 41.52 |
| 4210 | O | HOH | | 1125 | 30.481 | 19.290 | 62.284 | 1.00 | 32.09 |
| 4211 | O | HOH | | 1126 | 35.637 | 11.560 | 67.233 | 1.00 | 46.34 |
| 4212 | O | HOH | | 1127 | 11.013 | 0.808 | 62.566 | 1.00 | 41.40 |
| 4213 | O | HOH | | 1128 | 33.112 | 34.801 | 52.664 | 1.00 | 42.33 |
| 4214 | O | HOH | | 1129 | 17.395 | 15.298 | 75.306 | 1.00 | 38.43 |
| 4215 | O | HOH | | 1130 | 12.781 | 11.039 | 72.132 | 1.00 | 36.36 |
| 4216 | O | HOH | | 1131 | 29.971 | 28.037 | 26.998 | 1.00 | 37.72 |
| 4217 | O | HOH | | 1132 | 18.569 | -10.048 | 69.689 | 1.00 | 49.58 |
| 4218 | O | HOH | | 1133 | 41.228 | 21.065 | 46.519 | 1.00 | 37.30 |
| 4219 | O | HOH | | 1134 | 37.135 | 24.743 | 63.591 | 1.00 | 49.04 |
| 4220 | O | HOH | | 1135 | 27.047 | 1.783 | 63.648 | 1.00 | 31.46 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 4221 | O | HOH | | 1136 | 7.627 | 13.172 | 67.948 | 1.00 | 47.51 |
| 4222 | O | HOH | | 1137 | 30.244 | 21.919 | 32.671 | 1.00 | 51.61 |
| 4223 | O | HOH | | 1138 | 23.072 | 31.139 | 55.222 | 1.00 | 40.35 |
| 4224 | O | HOH | | 1139 | 18.856 | 13.261 | 76.752 | 1.00 | 51.54 |
| 4225 | O | HOH | | 1140 | 14.629 | 17.453 | 71.521 | 1.00 | 44.61 |
| 4226 | O | HOH | | 1141 | 30.127 | 7.542 | 77.644 | 1.00 | 39.23 |
| 4227 | O | HOH | | 1142 | 29.410 | 10.319 | 49.169 | 1.00 | 47.59 |
| 4228 | O | HOH | | 1143 | 52.667 | 17.385 | 21.488 | 1.00 | 33.48 |
| 4229 | O | HOH | | 1144 | 24.791 | 30.379 | 57.499 | 1.00 | 50.29 |
| 4230 | O | HOH | | 1145 | 33.515 | 32.303 | 54.663 | 1.00 | 60.00 |
| 4231 | O | HOH | | 1146 | 43.670 | 11.568 | 23.068 | 1.00 | 42.60 |
| 4232 | O | HOH | | 1147 | 22.577 | -0.615 | 76.831 | 1.00 | 51.25 |
| 4233 | O | HOH | | 1148 | 32.288 | 40.493 | 29.506 | 1.00 | 44.45 |
| 4234 | O | HOH | | 1149 | 38.587 | 13.656 | 83.878 | 1.00 | 50.82 |
| 4235 | O | HOH | | 1150 | 49.160 | 49.467 | 49.792 | 1.00 | 43.40 |
| 4236 | O | HOH | | 1151 | 36.423 | 9.754 | 65.069 | 1.00 | 50.09 |
| 4237 | O | HOH | | 1152 | 41.806 | 7.306 | 67.548 | 1.00 | 31.99 |
| 4238 | O | HOH | | 1153 | 20.865 | 5.502 | 75.530 | 1.00 | 53.88 |
| 4239 | O | HOH | | 1154 | 26.670 | 47.493 | 47.589 | 1.00 | 51.95 |
| 4240 | O | HOH | | 1155 | 58.759 | 25.881 | 41.102 | 1.00 | 51.36 |
| 4241 | O | HOH | | 1156 | 22.627 | 35.482 | 48.933 | 1.00 | 49.41 |
| 4242 | O | HOH | | 1157 | 29.902 | 41.245 | 33.231 | 1.00 | 47.09 |
| 4243 | O | HOH | | 1158 | 42.020 | 38.368 | 56.303 | 1.00 | 54.13 |
| 4244 | O | HOH | | 1159 | 42.067 | 18.958 | 48.188 | 1.00 | 41.24 |
| 4245 | O | HOH | | 1160 | 50.498 | 28.461 | 49.894 | 1.00 | 58.63 |
| 4246 | O | HOH | | 1161 | 19.503 | 21.644 | 46.170 | 1.00 | 47.41 |
| 4247 | O | HOH | | 1162 | 23.864 | 30.453 | 31.232 | 1.00 | 50.37 |
| 4248 | O | HOH | | 1163 | 33.055 | 31.002 | 70.386 | 1.00 | 45.87 |
| 4249 | O | HOH | | 1164 | 69.219 | 22.617 | 25.319 | 1.00 | 54.13 |
| 4250 | O | HOH | | 1165 | 26.984 | 34.792 | 32.747 | 1.00 | 50.24 |
| 4251 | O | HOH | | 1166 | 18.904 | 22.434 | 40.238 | 1.00 | 55.76 |
| 4252 | O | HOH | | 1167 | 38.572 | 18.970 | 85.692 | 1.00 | 51.19 |
| 4253 | O | HOH | | 1168 | 55.921 | 7.586 | 67.043 | 1.00 | 56.10 |
| 4254 | O | HOH | | 1169 | 60.815 | 24.101 | 20.645 | 1.00 | 43.40 |
| 4255 | O | HOH | | 1170 | 36.032 | 13.461 | 58.415 | 1.00 | 36.46 |
| 4256 | O | HOH | | 1171 | 42.102 | 18.389 | 50.609 | 1.00 | 50.87 |
| 4257 | O | HOH | | 1172 | 61.353 | 15.073 | 41.877 | 1.00 | 55.08 |

TABLE 10 (continued)

| THREE-DIMENSIONAL COORDINATES OF PR IN COMPLEX WITH PG | | | | | | | | | |
|---|---|---|---|---|---|---|---|---|---|
| ATOM | ATOM TYPE | RESIDUE | # | X | Y | Z | OCC | B | ATOM |
| 4258 | O | HOH | | 1173 | 34.024 | 3.531 | 28.705 | 1.00 | 52.74 |
| 4259 | O | HOH | | 1174 | 41.542 | 7.988 | 49.104 | 1.00 | 55.00 |
| 4260 | O | HOH | | 1175 | 24.756 | 2.570 | 77.011 | 1.00 | 53.63 |
| 4261 | O | HOH | | 1176 | 41.140 | 5.822 | 60.200 | 1.00 | 39.74 |
| 4262 | O | HOH | | 1177 | 9.209 | 2.894 | 61.066 | 1.00 | 48.67 |
| 4263 | O | HOH | | 1178 | 37.046 | 11.589 | 60.375 | 1.00 | 40.88 |
| 4264 | O | HOH | | 1179 | 35.350 | 29.505 | 79.245 | 1.00 | 38.37 |

TABLE 11

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1 | CB | THR | 729 | -19.756 | 38.320 | 19.110 |
| 2 | C | THR | 729 | -18.114 | 38.275 | 17.211 |
| 3 | O | THR | 729 | -16.947 | 37.873 | 17.214 |
| 4 | N | THR | 729 | -18.598 | 36.174 | 18.360 |
| 5 | CA | THR | 729 | -19.213 | 37.506 | 17.945 |
| 6 | OG1 | THR | 729 | -20.818 | 37.625 | 19.751 |
| 7 | CG2 | THR | 729 | -20.352 | 39.701 | 18.762 |
| 8 | N | ILE | 730 | -18.503 | 39.394 | 16.608 |
| 9 | CA | ILE | 730 | -17.623 | 40.222 | 15.789 |
| 10 | CB | ILE | 730 | -18.297 | 41.571 | 15.469 |
| 11 | C | ILE | 730 | -16.196 | 40.504 | 16.195 |
| 12 | O | ILE | 730 | -15.740 | 40.220 | 17.294 |
| 13 | CG2 | ILE | 730 | -17.440 | 42.465 | 14.521 |
| 14 | CG1 | ILE | 730 | -19.731 | 41.454 | 14.861 |
| 15 | CD1 | ILE | 730 | -20.557 | 42.757 | 14.828 |
| 16 | N | SER | 731 | -15.505 | 41.060 | 15.220 |
| 17 | CA | SER | 731 | -14.134 | 41.446 | 15.326 |
| 18 | CB | SER | 731 | -13.299 | 40.445 | 16.166 |
| 19 | C | SER | 731 | -13.634 | 41.573 | 13.874 |
| 20 | O | SER | 731 | -14.280 | 41.111 I | 12.934 |
| 21 | OG | SER | 731 | -13.117 | 39.185 | 15.512 |
| 22 | N | ARG | 732 | -12.466 | 42.196 | 13.695 |
| 23 | CA | ARG | 732 | -11.890 | 42.630 | 14.953 |
| 24 | CB | ARG | 732 | -10.516 | 43.184 | 14.585 |
| 25 | C | ARG | 732 | -12.607 | 43.506 | 15.952 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 26 | O | ARG | 732 | -13.827 | 43.743 | 15.969 |
| 27 | CG | ARG | 732 | -10.508 | 44.628 | 14.011 |
| 28 | CD | ARG | 732 | -9.081 | 45.148 | 13.769 |
| 29 | NE | ARG | 732 | -9.137 | 46.534 | 13.219 |
| 30 | CZ | ARG | 732 | -8.082 | 47.277 | 12.899 |
| 31 | NH1 | ARG | 732 | -8.288 | 48.469 | 12.437 |
| 32 | NH2 | ARG | 732 | -6.849 | 46.871 | 13.023 |
| 33 | N | ALA | 733 | -11.742 | 43.858 | 16.871 |
| 34 | CA | ALA | 733 | -12.003 | 44.602 | 18.044 |
| 35 | CB | ALA | 733 | -10.933 | 45.657 | 18.148 |
| 36 | C | ALA | 733 | -13.363 | 45.135 | 18.293 |
| 37 | O | ALA | 733 | -13.652 | 46.285 | 18.018 |
| 38 | N | LEU | 734 | -14.245 | 44.261 | 18.739 |
| 39 | CA | LEU | 734 | -15.501 | 44.789 | 19.161 |
| 40 | CB | LEU | 734 | -16.704 | 44.008 | 18.666 |
| 41 | CG | LEU | 734 | -17.555 | 45.126 | 18.047 |
| 42 | CD1 | LEU | 734 | -18.950 | 44.631 | 17.803 |
| 43 | CD2 | LEU | 734 | -17.589 | 46.355 | 18.973 |
| 44 | C | LEU | 734 | -15.240 | 44.562 | 20.626 |
| 45 | O | LEU | 734 | -16.127 | 44.626 | 21.465 |
| 46 | N | THR | 735 | -13.962 | 44.302 | 20.897 |
| 47 | CA | THR | 735 | -13.428 | 44.048 | 22.224 |
| 48 | CB | THR | 735 | -13.995 | 42.763 | 22.803 |
| 49 | OG1 | THR | 735 | -14.494 | 41.807 | 21.860 |
| 50 | CG2 | THR | 735 | -15.075 | 43.091 | 23.820 |
| 51 | C | THR | 735 | -11.918 | 43.943 | 22.076 |
| 52 | O | THR | 735 | -11.364 | 42.855 | 21.905 |
| 53 | N | PRO | 736 | -11.241 | 45.088 | 22.203 |
| 54 | CD | PRO | 736 | -11.886 | 45.982 | 23.170 |
| 55 | CA | PRO | 736 | -9.820 | 45.423 | 22.111 |
| 56 | CB | PRO | 736 | -9.750 | 46.869 | 22.596 |
| 57 | CG | PRO | 736 | -11.131 | 47.234 | 22.999 |
| 58 | C | PRO | 736 | -8.819 | 44.559 | 22.827 |
| 59 | O | PRO | 736 | -9.060 | 44.008 | 23.899 |
| 60 | N | SER | 737 | -7.645 | 44.523 | 22.226 |
| 61 | CA | SER | 737 | -6.573 | 43.708 | 22.713 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 62 | CB | SER | 737 | -6.182 | 42.760 | 21.638 |
| 63 | C | SER | 737 | -5.375 | 44.525 | 23.067 |
| 64 | O | SER | 737 | -5.269 | 45.692 | 22.693 |
| 65 | OG | SER | 737 | -7.176 | 41.760 | 21.389 |
| 66 | N | PRO | 738 | -4.443 | 43.914 | 23.765 |
| 67 | CA | PRO | 738 | -3.306 | 44.709 | 24.076 |
| 68 | CB | PRO | 738 | -2.291 | 43.940 | 24.906 |
| 69 | C | PRO | 738 | -2.684 | 45.293 | 22.811 |
| 70 | O | PRO | 738 | -2.438 | 46.498 | 22.731 |
| 71 | CG | PRO | 738 | -2.473 | 42.521 | 24.341 |
| 72 | CD | PRO | 738 | -3.991 | 42.390 | 24.175 |
| 73 | N | VAL | 739 | -2.500 | 44.447 | 21.804 |
| 74 | CA | VAL | 739 | -1.889 | 44.833 | 20.541 |
| 75 | CB | VAL | 739 | -1.508 | 43.576 | 19.786 |
| 76 | CG1 | VAL | 739 | -2.772 | 42.871 | 19.326 |
| 77 | CG2 | VAL | 739 | -0.607 | 43.918 | 18.640 |
| 78 | C | VAL | 739 | -2.741 | 45.703 | 19.604 |
| 79 | O | VAL | 739 | -2.250 | 46.235 | 18.608 |
| 80 | N | MET | 740 | -4.019 | 45.833 | 19.923 |
| 81 | CA | MET | 740 | -4.941 | 46.603 | 19.100 |
| 82 | CB | MET | 740 | -6.349 | 46.084 | 19.336 |
| 83 | C | MET | 740 | -4.875 | 48.043 | 19.513 |
| 84 | O | MET | 740 | -4.947 | 48.972 | 18.706 |
| 85 | CG | MET | 740 | -6.600 | 44.615 | 18.934 |
| 86 | SD | MET | 740 | -8.364 | 44.257 | 19.004 |
| 87 | CE | MET | 740 | -8.277 | 42.465 | 18.887 |
| 88 | N | VAL | 741 | -4.766 | 48.181 | 20.818 |
| 89 | CA | VAL | 741 | -4.694 | 49.442 | 21.487 |
| 90 | CB | VAL | 741 | -4.802 | 49.170 | 22.971 |
| 91 | C | VAL | 741 | -3.357 | 50.057 | 21.149 |
| 92 | O | VAL | 741 | -3.223 | 51.269 | 20.958 |
| 93 | CG1 | VAL | 741 | -6.167 | 48.580 | 23.404 |
| 94 | CG2 | VAL | 741 | -4.562 | 50.382 | 23.914 |
| 95 | N | LEU | 742 | -2.362 | 49.190 | 21.059 |
| 96 | CA | LEU | 742 | -1.019 | 49.623 | 20.753 |
| 97 | CB | LEU | 742 | -0.084 | 48.427 | 20.896 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 98 | CG | LEU | 742 | 1.010 | 48.630 | 21.951 |
| 99 | CD1 | LEU | 742 | 0.433 | 49.160 | 23.240 |
| 100 | CD2 | LEU | 742 | 1.711 | 47.316 | 22.185 |
| 101 | C | LEU | 742 | -0.929 | 50.237 | 19.357 |
| 102 | O | LEU | 742 | -0.092 | 51.104 | 19.094 |
| 103 | N | GLU | 743 | -1.833 | 49.795 | 18.487 |
| 104 | CA | GLU | 743 | -1.904 | 50.219 | 17.097 |
| 105 | CB | GLU | 743 | -2.756 | 49.212 | 16.320 |
| 106 | CG | GLU | 743 | -2.136 | 48.796 | 15.007 |
| 107 | CD | GLU | 743 | -2.570 | 47.415 | 14.514 |
| 108 | OE1 | GLU | 743 | -2.934 | 46.529 | 15.329 |
| 109 | OE2 | GLU | 743 | -2.513 | 47.214 | 13.284 |
| 110 | C | GLU | 743 | -2.452 | 51.624 | 16.871 |
| 111 | O | GLU | 743 | -1.996 | 52.337 | 15.977 |
| 112 | N | ASN | 744 | -3.423 | 52.016 | 17.680 |
| 113 | CA | ASN | 744 | -4.044 | 53.316 | 17.530 |
| 114 | CB | ASN | 744 | -5.471 | 53.284 | 18.055 |
| 115 | C | ASN | 744 | -3.287 | 54.388 | 18.276 |
| 116 | O | ASN | 744 | -3.311 | 55.556 | 17.890 |
| 117 | CG | ASN | 744 | -6.417 | 52.253 | 17.431 |
| 118 | OD1 | ASN | 744 | -6.684 | 51.196 | 17.983 |
| 119 | ND2 | ASN | 744 | -6.937 | 52.503 | 16.259 |
| 120 | N | ILE | 745 | -2.614 | 54.006 | 19.356 |
| 121 | CA | ILE | 745 | -1.896 | 55.023 | 20.098 |
| 122 | CB | ILE | 745 | -1.765 | 54.673 | 21.610 |
| 123 | CG2 | ILE | 745 | -3.046 | 54.028 | 22.106 |
| 124 | CG1 | ILE | 745 | -0.582 | 53.744 | 21.859 |
| 125 | CD1 | ILE | 745 | -0.178 | 53.699 | 23.317 |
| 126 | C | ILE | 745 | -0.524 | 55.320 | 19.509 |
| 127 | O | ILE | 745 | 0.161 | 56.226 | 19.973 |
| 128 | N | GLU | 746 | -0.132 | 54.573 | 18.479 |
| 129 | CA | GLU | 746 | 1.167 | 54.785 | 17.826 |
| 130 | CB | GLU | 746 | 1.409 | 53.704 | 16.753 |
| 131 | CG | GLU | 746 | 2.741 | 53.793 | 15.984 |
| 132 | CD | GLU | 746 | 3.990 | 53.653 | 16.855 |
| 133 | OE1 | GLU | 746 | 4.303 | 52.528 | 17.298 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 134 | OE2 | GLU | 746 | 4.670 | 54.674 | 17.093 |
| 135 | C | GLU | 746 | 1.160 | 56.185 | 17.203 |
| 136 | O | GLU | 746 | 0.281 | 56.514 | 16.399 |
| 137 | N | PRO | 747 | 2.114 | 57.042 | 17.597 |
| 138 | CD | PRO | 747 | 3.001 | 57.025 | 18.773 |
| 139 | CA | PRO | 747 | 2.080 | 58.369 | 16.985 |
| 140 | CB | PRO | 747 | 3.240 | 59.122 | 17.655 |
| 141 | CG | PRO | 747 | 4.002 | 58.090 | 18.432 |
| 142 | C | PRO | 747 | 2.211 | 58.315 | 15.484 |
| 143 | O | PRO | 747 | 2.743 | 57.347 | 14.931 |
| 144 | N | GLU | 748 | 1.691 | 59.331 | 14.813 |
| 145 | CA | GLU | 748 | 1.833 | 59.328 | 13.380 |
| 146 | CB | GLU | 748 | 0.511 | 59.651 | 12.673 |
| 147 | CG | GLU | 748 | -0.106 | 61.009 | 12.712 |
| 148 | CD | GLU | 748 | -1.176 | 61.042 | 11.636 |
| 149 | OE1 | GLU | 748 | -2.016 | 60.110 | 11.627 |
| 150 | OE2 | GLU | 748 | -1.169 | 61.956 | 10.785 |
| 151 | C | GLU | 748 | 3.010 | 60.222 | 13.010 |
| 152 | O | GLU | 748 | 3.380 | 61.117 | 13.764 |
| 153 | N | ILE | 749 | 3.618 | 59.939 | 11.860 |
| 154 | CA | ILE | 749 | 4.850 | 60.602 | 11.416 |
| 155 | CB | ILE | 749 | 5.212 | 59.980 | 10.004 |
| 156 | C | ILE | 749 | 5.059 | 62.115 | 11.226 |
| 157 | O | ILE | 749 | 4.357 | 62.768 | 10.453 |
| 158 | CG2 | ILE | 749 | 5.462 | 58.447 | 10.077 |
| 159 | CG1 | ILE | 749 | 4.309 | 60.289 | 8.765 |
| 160 | CD1 | ILE | 749 | 2.804 | 59.971 | 8.865 |
| 161 | N | VAL | 750 | 6.091 | 62.639 | 11.893 |
| 162 | CA | VAL | 750 | 6.453 | 64.069 | 11.856 |
| 163 | CB | VAL | 750 | 6.926 | 64.489 | 13.295 |
| 164 | C | VAL | 750 | 7.513 | 64.559 | 10.849 |
| 165 | O | VAL | 750 | 8.707 | 64.280 | 11.013 |
| 166 | CG1 | VAL | 750 | 5.820 | 64.279 | 14.360 |
| 167 | CG2 | VAL | 750 | 7.395 | 65.961 | 13.399 |
| 168 | N | TYR | 751 | 7.070 | 65.321 | 9.841 |
| 169 | CA | TYR | 751 | 7.956 | 65.903 | 8.814 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 170 | CB | TYR | 751 | 7.131 | 66.540 | 7.688 |
| 171 | CG | TYR | 751 | 6.395 | 65.498 | 6.878 |
| 172 | CD1 | TYR | 751 | 5.170 | 64.989 | 7.305 |
| 173 | CE1 | TYR | 751 | 4.564 | 63.915 | 6.641 |
| 174 | CD2 | TYR | 751 | 6.988 | 64.921 | 5.756 |
| 175 | CE2 | TYR | 751 | 6.399 | 63.851 | 5.089 |
| 176 | CZ | TYR | 751 | 5.187 | 63.350 | 5.537 |
| 177 | OH | TYR | 751 | 4.593 | 62.295 | 4.880 |
| 178 | C | TYR | 751 | 8.851 | 66.939 | 9.489 |
| 179 | O | TYR | 751 | 8.486 | 67.464 | 10.534 |
| 180 | N | ALA | 752 | 9.999 | 67.259 | 8.893 |
| 181 | CA | ALA | 752 | 10.931 | 68.180 | 9.550 |
| 182 | CB | ALA | 752 | 12.356 | 67.636 | 9.405 |
| 183 | C | ALA | 752 | 10.942 | 69.673 | 9.246 |
| 184 | O | ALA | 752 | 11.638 | 70.420 | 9.935 |
| 185 | N | GLY | 753 | 10.195 | 70.128 | 8.246 |
| 186 | CA | GLY | 753 | 10.213 | 71.550 | 7.933 |
| 187 | C | GLY | 753 | 11.621 | 71.989 | 7.557 |
| 188 | O | GLY | 753 | 12.086 | 73.063 | 7.941 |
| 189 | N | TYR | 754 | 12.309 | 71.142 | 6.812 |
| 190 | CA | TYR | 754 | 13.662 | 71.437 | 6.386 |
| 191 | CB | TYR | 754 | 14.402 | 70.140 | 6.103 |
| 192 | CG | TYR | 754 | 15.874 | 70.279 | 5.811 |
| 193 | CD1 | TYR | 754 | 16.792 | 70.476 | 6.840 |
| 194 | CE1 | TYR | 754 | 18.162 | 70.466 | 6.595 |
| 195 | CD2 | TYR | 754 | 16.362 | 70.099 | 4.518 |
| 196 | CE2 | TYR | 754 | 17.724 | 70.090 | 4.263 |
| 197 | CZ | TYR | 754 | 18.618 | 70.268 | 5.309 |
| 198 | OH | TYR | 754 | 19.972 | 70.240 | 5.048 |
| 199 | C | TYR | 754 | 13.557 | 72.238 | 5.112 |
| 200 | O | TYR | 754 | 12.560 | 72.161 | 4.396 |
| 201 | N | ASP | 755 | 14.576 | 73.024 | 4.822 |
| 202 | CA | ASP | 755 | 14.548 | 73.803 | 3.609 |
| 203 | CB | ASP | 755 | 14.825 | 75.267 | 3.915 |
| 204 | CG | ASP | 755 | 14.947 | 76.086 | 2.669 |
| 205 | OD1 | ASP | 755 | 14.292 | 75.709 | 1.677 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 206 | OD2 | ASP | 755 | 15.684 | 77.091 | 2.677 |
| 207 | C | ASP | 755 | 15.600 | 73.242 | 2.673 |
| 208 | O | ASP | 755 | 16.777 | 73.546 | 2.795 |
| 209 | N | SER | 756 | 15.179 | 72.412 | 1.730 |
| 210 | CA | SER | 756 | 16.139 | 71.822 | 0.812 |
| 211 | CB | SER | 756 | 15.731 | 70.385 | 0.440 |
| 212 | OG | SER | 756 | 14.414 | 70.273 | -0.094 |
| 213 | C | SER | 756 | 16.338 | 72.672 | -0.423 |
| 214 | O | SER | 756 | 17.007 | 72.262 | -1.373 |
| 215 | N | SER | 757 | 15.754 | 73.858 | -0.427 |
| 216 | CA | SER | 757 | 15.981 | 74.718 | -1.564 |
| 217 | CB | SER | 757 | 14.974 | 75.867 | -1.613 |
| 218 | OG | SER | 757 | 14.787 | 76.751 | -0.529 |
| 219 | C | SER | 757 | 17.416 | 75.229 | -1.427 |
| 220 | O | SER | 757 | 17.984 | 75.718 | -2.385 |
| 221 | N | LYS | 758 | 18.018 | 75.104 | -0.245 |
| 222 | CA | LYS | 758 | 19.410 | 75.542 | -0.084 |
| 223 | CB | LYS | 758 | 19.569 | 76.631 | 1.022 |
| 224 | C | LYS | 758 | 20.317 | 74.345 | 0.233 |
| 225 | O | LYS | 758 | 19.879 | 73.368 | 0.846 |
| 226 | CG | LYS | 758 | 21.018 | 77.164 | 1.158 |
| 227 | CD | LYS | 758 | 21.225 | 78.187 | 2.279 |
| 228 | CE | LYS | 758 | 22.709 | 78.571 | 2.345 |
| 229 | NZ | LYS | 758 | 22.886 | 79.678 | 3.301 |
| 230 | N | PRO | 759 | 21.605 | 74.418 | -0.156 |
| 231 | CD | PRO | 759 | 22.272 | 75.497 | -0.900 |
| 232 | CA | PRO | 759 | 22.533 | 73.305 | 0.094 |
| 233 | CB | PRO | 759 | 23.747 | 73.665 | -0.752 |
| 234 | CG | PRO | 759 | 23.733 | 75.132 | -0.765 |
| 235 | C | PRO | 759 | 22.917 | 72.865 | 1.503 |
| 236 | O | PRO | 759 | 23.305 | 73.661 | 2.360 |
| 237 | N | ASP | 760 | 22.822 | 71.551 | 1.685 |
| 238 | CA | ASP | 760 | 23.121 | 70.877 | 2.939 |
| 239 | CB | ASP | 760 | 23.112 | 69.349 | 2.763 |
| 240 | CG | ASP | 760 | 21.888 | 68.816 | 2.030 |
| 241 | OD1 | ASP | 760 | 20.960 | 69.573 | 1.668 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 242 | OD2 | ASP | 760 | 21.879 | 67.589 | 1.818 |
| 243 | C | ASP | 760 | 24.480 | 71.222 | 3.533 |
| 244 | O | ASP | 760 | 25.312 | 71.900 | 2.930 |
| 245 | N | THR | 761 | 24.689 | 70.695 | 4.731 |
| 246 | CA | THR | 761 | 25.915 | 70.844 | 5.482 |
| 247 | CB | THR | 761 | 26.423 | 72.285 | 5.820 |
| 248 | C | THR | 761 | 25.635 | 70.009 | 6.710 |
| 249 | O | THR | 761 | 24.476 | 69.815 | 7.079 |
| 250 | OG1 | THR | 761 | 27.630 | 72.232 | 6.576 |
| 251 | CG2 | THR | 761 | 25.418 | 73.203 | 6.527 |
| 252 | N | ALA | 762 | 26.684 | 69.510 | 7.336 |
| 253 | CA | ALA | 762 | 26.498 | 68.676 | 8.506 |
| 254 | CB | ALA | 762 | 27.840 | 68.117 | 9.005 |
| 255 | C | ALA | 762 | 25.771 | 69.331 | 9.673 |
| 256 | O | ALA | 762 | 24.899 | 68.692 | 10.267 |
| 257 | N | GLU | 763 | 26.115 | 70.581 | 10.011 |
| 258 | CA | GLU | 763 | 25.476 | 71.272 | 11.144 |
| 259 | CB | GLU | 763 | 26.194 | 72.592 | 11.546 |
| 260 | C | GLU | 763 | 24.007 | 71.553 | 10.871 |
| 261 | O | GLU | 763 | 23.157 | 71.402 | 11.744 |
| 262 | CG | GLU | 763 | 26.307 | 73.719 | 10.479 |
| 263 | CD | GLU | 763 | 25.265 | 74.895 | 10.561 |
| 264 | OE1 | GLU | 763 | 24.497 | 74.906 | 11.528 |
| 265 | OE2 | GLU | 763 | 25.344 | 75.705 | 9.631 |
| 266 | N | ASN | 764 | 23.716 | 71.939 | 9.645 |
| 267 | CA | ASN | 764 | 22.360 | 72.239 | 9.245 |
| 268 | CB | ASN | 764 | 22.372 | 72.902 | 7.845 |
| 269 | C | ASN | 764 | 21.486 | 71.002 | 9.189 |
| 270 | O | ASN | 764 | 20.262 | 71.061 | 9.294 |
| 271 | CG | ASN | 764 | 20.995 | 73.387 | 7.376 |
| 272 | OD1 | ASN | 764 | 20.215 | 73.953 | 8.123 |
| 273 | ND2 | ASN | 764 | 20.735 | 73.180 | 6.002 |
| 274 | N | LEU | 765 | 22.127 | 69.872 | 8.974 |
| 275 | CA | LEU | 765 | 21.391 | 68.644 | 8.848 |
| 276 | CB | LEU | 765 | 22.223 | 67.683 | 7.963 |
| 277 | C | LEU | 765 | 21.024 | 68.015 | 10.158 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 278 | O | LEU | 765 | 20.024 | 67.298 | 10.275 |
| 279 | CG | LEU | 765 | 21.499 | 66.430 | 7.442 |
| 280 | CD1 | LEU | 765 | 20.143 | 66.766 | 6.772 |
| 281 | CD2 | LEU | 765 | 22.437 | 65.683 | 6.467 |
| 282 | N | LEU | 766 | 21.877 | 68.222 | 11.136 |
| 283 | CA | LEU | 766 | 21.567 | 67.619 | 12.386 |
| 284 | CB | LEU | 766 | 22.728 | 66.737 | 12.939 |
| 285 | C | LEU | 766 | 21.005 | 68.605 | 13.352 |
| 286 | O | LEU | 766 | 20.877 | 68.318 | 14.530 |
| 287 | CG | LEU | 766 | 23.913 | 67.344 | 13.722 |
| 288 | CD1 | LEU | 766 | 25.193 | 66.496 | 13.527 |
| 289 | CD2 | LEU | 766 | 24.184 | 68.811 | 13.399 |
| 290 | N | SER | 767 | 20.684 | 69.800 | 12.908 |
| 291 | CA | SER | 767 | 20.024 | 70.574 | 13.906 |
| 292 | CB | SER | 767 | 20.276 | 72.095 | 13.791 |
| 293 | C | SER | 767 | 18.550 | 70.269 | 13.691 |
| 294 | O | SER | 767 | 17.829 | 69.980 | 14.640 |
| 295 | OG | SER | 767 | 19.523 | 72.823 | 14.751 |
| 296 | N | THR | 768 | 18.110 | 70.288 | 12.437 |
| 297 | CA | THR | 768 | 16.720 | 70.012 | 12.116 |
| 298 | CB | THR | 768 | 16.570 | 69.964 | 10.593 |
| 299 | OG1 | THR | 768 | 17.092 | 71.157 | 9.999 |
| 300 | CG2 | THR | 768 | 15.112 | 69.830 | 10.188 |
| 301 | C | THR | 768 | 16.319 | 68.674 | 12.738 |
| 302 | O | THR | 768 | 15.155 | 68.460 | 13.145 |
| 303 | N | LEU | 769 | 17.315 | 67.800 | 12.839 |
| 304 | CA | LEU | 769 | 17.137 | 66.464 | 13.346 |
| 305 | CB | LEU | 769 | 18.395 | 65.722 | 12.955 |
| 306 | CG | LEU | 769 | 18.165 | 64.572 | 11.949 |
| 307 | CD1 | LEU | 769 | 17.010 | 64.884 | 10.998 |
| 308 | CD2 | LEU | 769 | 19.424 | 64.310 | 11.136 |
| 309 | C | LEU | 769 | 16.906 | 66.514 | 14.857 |
| 310 | O | LEU | 769 | 16.098 | 65.774 | 15.452 |
| 311 1 | N | ASN | 770 | 17.642 | 67.402 | 15.485 |
| 312 | CA | ASN | 770 | 17.460 | 67.562 | 16.888 |
| 313 | CB | ASN | 770 | 18.532 | 68.480 | 17.376 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 314 | CG | ASN | 770 | 19.775 | 67.729 | 17.672 |
| 315 | OD1 | ASN | 770 | 20.089 | 66.732 | 17.016 |
| 316 | ND2 | ASN | 770 | 20.607 | 68.445 | 18.542 |
| 317 | C | ASN | 770 | 16.073 | 68.135 | 17.052 |
| 318 | O | ASN | 770 | 15.238 | 67.569 | 17.732 |
| 319 | N | ARG | 771 | 15.825 | 69.267 | 16.420 |
| 320 | CA | ARG | 771 | 14.516 | 69.874 | 16.457 |
| 321 | CB | ARG | 771 | 14.469 | 70.956 | 15.411 |
| 322 | CG | ARG | 771 | 15.421 | 72.167 | 15.625 |
| 323 | C | ARG | 771 | 13.456 | 68.815 | 16.170 |
| 324 | O | ARG | 771 | 12.379 | 68.841 | 16.763 |
| 325 | CD | ARG | 771 | 15.508 | 73.114 | 14.406 |
| 326 | NE | ARG | 771 | 14.248 | 73.390 | 13.674 |
| 327 | CZ | ARG | 771 | 14.132 | 73.721 | 12.336 |
| 328 | NH1 | ARG | 771 | 12.884 | 73.956 | 11.815 |
| 329 | NH2 | ARG | 771 | 15.225 | 73.819 | 11.507 |
| 330 | N | LEU | 772 | 13.735 | 67.891 | 15.253 |
| 331 | CA | LEU | 772 | 12.746 | 66.848 | 15.013 |
| 332 | CB | LEU | 772 | 13.098 | 65.965 | 13.804 |
| 333 | CG | LEU | 772 | 11.863 | 65.253 | 13.219 |
| 334 | CD1 | LEU | 772 | 12.216 | 63.962 | 12.506 |
| 335 | CD2 | LEU | 772 | 10.921 | 64.937 | 14.353 |
| 336 | C | LEU | 772 | 12.792 | 66.025 | 16.305 |
| 337 | O | LEU | 772 | 11.797 | 65.443 | 16.737 |
| 338 | N | ALA | 773 | 13.968 | 66.008 | 16.927 |
| 339 | CA | ALA | 773 | 14.160 | 65.294 | 18.178 |
| 340 | C | ALA | 773 | 13.152 | 65.700 | 19.236 |
| 341 | O | ALA | 773 | 12.378 | 64.862 | 19.695 |
| 342 | CB | ALA | 773 | 15.616 | 65.518 | 18.624 |
| 343 | N | GLY | 774 | 13.146 | 66.976 | 19.621 |
| 344 | CA | GLY | 774 | 12.204 | 67.432 | 20.625 |
| 345 | C | GLY | 774 | 10.763 | 67.024 | 20.350 |
| 346 | O | GLY | 774 | 10.122 | 66.392 | 21.188 |
| 347 | N | LYS | 775 | 10.248 | 67.369 | 19.178 |
| 348 | CA | LYS | 775 | 8.868 | 67.045 | 18.855 |
| 349 | CB | LYS | 775 | 8.525 | 67.530 | 17.445 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 350 | CG | LYS | 775 | 8.419 | 69.054 | 17.353 |
| 351 | CD | LYS | 775 | 7.791 | 69.503 | 16.016 |
| 352 | C | LYS | 775 | 8.539 | 65.569 | 19.005 |
| 353 | O | LYS | 775 | 7.517 | 65.206 | 19.591 |
| 354 | CE | LYS | 775 | 7.787 | 71.028 | 15.818 |
| 355 | NZ | LYS | 775 | 7.242 | 71.426 | 14.456 |
| 356 | N | GLN | 776 | 9.412 | 64.719 | 18.477 |
| 357 | CA | GLN | 776 | 9.214 | 63.279 | 18.555 |
| 358 | CB | GLN | 776 | 10.318 | 62.566 | 17.788 |
| 359 | CG | GLN | 776 | 9.887 | 61.973 | 16.466 |
| 360 | CD | GLN | 776 | 11.078 | 61.625 | 15.631 |
| 361 | OE1 | GLN | 776 | 10.948 | 61.242 | 14.472 |
| 362 | NE2 | GLN | 776 | 12.331 | 61.695 | 16.275 |
| 363 | C | GLN | 776 | 9.218 | 62.800 | 19.996 |
| 364 | O | GLN | 776 | 8.373 | 61.998 | 20.393 |
| 365 | N | MET | 777 | 10.182 | 63.283 | 20.770 |
| 366 | CA | MET | 777 | 10.298 | 62.919 | 22.176 |
| 367 | CB | MET | 777 | 11.478 | 63.747 | 22.759 |
| 368 | C | MET | 777 | 9.015 | 63.253 | 22.923 |
| 369 | O | MET | 777 | 8.505 | 62.443 | 23.702 |
| 370 | CG | MET | 777 | 11.739 | 63.416 | 24.216 |
| 371 | SD | MET | 777 | 11.922 | 61.626 | 24.254 |
| 372 | CE | MET | 777 | 11.174 | 61.295 | 25.852 |
| 373 | N | ILE | 778 | 8.508 | 64.461 | 22.687 |
| 374 | CA | ILE | 778 | 7.271 | 64.908 | 23.318 |
| 375 | CB | ILE | 778 | 6.941 | 66.366 | 22.911 |
| 376 | CG2 | ILE | 778 | 5.432 | 66.624 | 22.997 |
| 377 | CG1 | ILE | 778 | 7.725 | 67.325 | 23.820 |
| 378 | CD1 | ILE | 778 | 8.335 | 68.525 | 23.116 |
| 379 | C | ILE | 778 | 6.161 | 63.965 | 22.902 |
| 380 | O | ILE | 778 | 5.392 | 63.509 | 23.735 |
| 381 | N | GLN | 779 | 6.088 | 63.655 | 21.621 |
| 382 | CA | GLN | 779 | 5.070 | 62.736 | 21.133 |
| 383 | CB | GLN | 779 | 5.125 | 62.293 | 19.652 |
| 384 | C | GLN | 779 | 5.123 | 61.425 | 21.925 |
| 385 | O | GLN | 779 | 4.083 | 60.818 | 22.219 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 386 | CG | GLN | 779 | 4.732 | 63.341 | 18.595 |
| 387 | CD | GLN | 779 | 4.569 | 62.623 | 17.248 |
| 388 | OE1 | GLN | 779 | 5.502 | 62.033 | 16.728 |
| 389 | NE2 | GLN | 779 | 3.274 | 62.678 | 16.676 |
| 390 | N | VAL | 780 | 6.345 | 61.004 | 22.264 |
| 391 | CA | VAL | 780 | 6.590 | 59.764 | 23.003 |
| 392 | CB | VAL | 780 | 8.113 | 59.380 | 23.122 |
| 393 | C | VAL | 780 | 5.932 | 59.780 | 24.373 |
| 394 | O | VAL | 780 | 5.427 | 58.760 | 24.842 |
| 395 | CG1 | VAL | 780 | 8.564 | 58.449 | 21.970 |
| 396 | CG2 | VAL | 780 | 8.538 | 58.770 | 24.479 |
| 397 | N | VAL | 781 | 5.951 | 60.945 | 25.014 |
| 398 | CA | VAL | 781 | 5.346 | 61.101 | 26.324 |
| 399 | CB | VAL | 781 | 5.625 | 62.498 | 26.891 |
| 400 | CG1 | VAL | 781 | 5.016 | 62.620 | 28.253 |
| 401 | CG2 | VAL | 781 | 7.111 | 62.740 | 26.963 |
| 402 | C | VAL | 781 | 3.825 | 60.855 | 26.267 |
| 403 | O | VAL | 781 | 3.306 | 60.031 | 27.019 |
| 404 | N | LYS | 782 | 3.118 | 61.548 | 25.370 |
| 405 | CA | LYS | 782 | 1.659 | 61.390 | 25.231 |
| 406 | CB | LYS | 782 | 1.118 | 62.386 | 24.204 |
| 407 | CG | LYS | 782 | -0.352 | 62.165 | 23.801 |
| 408 | CD | LYS | 782 | -0.801 | 63.198 | 22.762 |
| 409 | CE | LYS | 782 | -0.465 | 64.627 | 23.229 |
| 410 | NZ | LYS | 782 | -0.605 | 65.690 | 22.152 |
| 411 | C | LYS | 782 | 1.274 | 59.973 | 24.800 |
| 412 | O | LYS | 782 | 0.106 | 59.571 | 24.861 |
| 413 | N | TRP | 783 | 2.281 | 59.238 | 24.351 |
| 414 | CA | TRP | 783 | 2.152 | 57.862 | 23.881 |
| 415 | CB | TRP | 783 | 3.212 | 57.595 | 22.816 |
| 416 | CG | TRP | 783 | 3.470 | 56.155 | 22.564 |
| 417 | CD2 | TRP | 783 | 4.588 | 55.392 | 23.026 |
| 418 | CE2 | TRP | 783 | 4.399 | 54.069 | 22.586 |
| 419 | CE3 | TRP | 783 | 5.723 | 55.696 | 23.794 |
| 420 | CD1 | TRP | 783 | 2.677 | 55.293 | 21.869 |
| 421 | NE1 | TRP | 783 | 3.228 | 54.039 | 21.875 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 422 | CZ2 | TRP | 783 | 5.317 | 53.047 | 22.855 |
| 423 | CZ3 | TRP | 783 | 6.634 | 54.679 | 24.067 |
| 424 | CH2 | TRP | 783 | 6.421 | 53.370 | 23.606 |
| 425 | C | TRP | 783 | 2.377 | 56.910 | 25.039 |
| 426 | O | TRP | 783 | 1.644 | 55.939 | 25.215 |
| 427 | N | ALA | 784 | 3.430 | 57.186 | 25.804 |
| 428 | CA | ALA | 784 | 3.785 | 56.365 | 26.948 |
| 429 | CB | ALA | 784 | 4.985 | 56.962 | 27.673 |
| 430 | C | ALA | 784 | 2.593 | 56.305 | 27.881 |
| 431 | O | ALA | 784 | 2.217 | 55.241 | 28.357 |
| 432 | N | LYS | 785 | 1.992 | 57.466 | 28.114 |
| 433 | CA | LYS | 785 | 0.847 | 57.573 | 28.992 |
| 434 | CB | LYS | 785 | 0.509 | 59.053 | 29.222 |
| 435 | CG | LYS | 785 | 1.737 | 59.940 | 29.510 |
| 436 | CD | LYS | 785 | 1.413 | 61.389 | 29.990 |
| 437 | CE | LYS | 785 | 0.003 | 61.884 | 29.609 |
| 438 | NZ | LYS | 785 | -0.348 | 63.263 | 30.145 |
| 439 | C | LYS | 785 | -0.381 | 56.817 | 28.479 |
| 440 | O | LYS | 785 | -1.349 | 56.670 | 29.211 |
| 441 | N | VAL | 786 | -0.367 | 56.328 | 27.242 |
| 442 | CA | VAL | 786 | -1.532 | 55.592 | 26.746 |
| 443 | CB | VAL | 786 | -1.855 | 55.995 | 25.312 |
| 444 | C | VAL | 786 | -1.323 | 54.085 | 26.826 |
| 445 | O | VAL | 786 | -2.267 | 53.307 | 26.643 |
| 446 | CG1 | VAL | 786 | -2.296 | 57.471 | 25.159 |
| 447 | CG2 | VAL | 786 | -2.951 | 55.162 | 24.592 |
| 448 | N | LEU | 787 | -0.079 | 53.681 | 27.083 |
| 449 | CA | LEU | 787 | 0.257 | 52.268 | 27.214 |
| 450 | CB | LEU | 787 | 1.775 | 52.044 | 27.401 |
| 451 | C | LEU | 787 | -0.470 | 51.832 | 28.467 |
| 452 | O | LEU | 787 | -0.109 | 52.208 | 29.587 |
| 453 | CG | LEU | 787 | 2.734 | 52.584 | 26.306 |
| 454 | CD1 | LEU | 787 | 4.178 | 52.168 | 26.617 |
| 455 | CD2 | LEU | 787 | 2.350 | 52.109 | 24.895 |
| 456 | N | PRO | 788 | -1.543 | 51.063 | 28.293 |
| 457 | CD | PRO | 788 | -2.126 | 50.571 | 27.034 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 458 | CA | PRO | 788 | -2.287 | 50.611 | 29.461 |
| 459 | CB | PRO | 788 | -3.126 | 49.444 | 28.917 |
| 460 | CG | PRO | 788 | -2.658 | 49.248 | 27.445 |
| 461 | C | PRO | 788 | -1.346 | 50.207 | 30.603 |
| 462 | O | PRO | 788 | -0.498 | 49.331 | 30.431 |
| 463 | N | GLY | 789 | -1.469 | 50.871 | 31.751 |
| 464 | CA | GLY | 789 | -0.628 | 50.533 | 32.889 |
| 465 | C | GLY | 789 | 0.592 | 51.398 | 33.148 |
| 466 | O | GLY | 789 | 1.087 | 51.477 | 34.273 |
| 467 | N | PHE | 790 | 1.106 | 52.057 | 32.125 |
| 468 | CA | PHE | 790 | 2.271 | 52.877 | 32.357 |
| 469 | CB | PHE | 790 | 2.789 | 53.462 | 31.052 |
| 470 | CG | PHE | 790 | 3.949 | 54.379 | 31.239 |
| 471 | CD1 | PHE | 790 | 5.220 | 53.870 | 31.479 |
| 472 | CD2 | PHE | 790 | 3.768 | 55.750 | 31.196 |
| 473 | CE1 | PHE | 790 | 6.300 | 54.717 | 31.695 |
| 474 | CE2 | PHE | 790 | 4.840 | 56.612 | 31.410 |
| 475 | CZ | PHE | 790 | 6.112 | 56.096 | 31.653 |
| 476 | C | PHE | 790 | 1.972 | 54.015 | 33.328 |
| 477 | O | PHE | 790 | 2.849 | 54.414 | 34.108 |
| 478 | N | LYS | 791 | 0.752 | 54.548 | 33.304 |
| 479 | CA | LYS | 791 | 0.510 | 55.667 | 34.201 |
| 480 | CB | LYS | 791 | -0.334 | 56.759 | 33.530 |
| 481 | CG | LYS | 791 | -1.744 | 56.478 | 33.055 |
| 482 | CD | LYS | 791 | -2.230 | 57.816 | 32.484 |
| 483 | C | LYS | 791 | 0.026 | 55.391 | 35.608 |
| 484 | O | LYS | 791 | -0.404 | 56.302 | 36.312 |
| 485 | CE | LYS | 791 | -3.638 | 57.635 | 31.901 |
| 486 | NZ | LYS | 791 | -4.178 | 58.949 | 31.508 |
| 487 | N | ASN | 792 | 0.120 | 54.141 | 36.027 |
| 488 | CA | ASN | 792 | -0.240 | 53.788 | 37.383 |
| 489 | CB | ASN | 792 | -1.030 | 52.487 | 37.415 |
| 490 | CG | ASN | 792 | -2.422 | 52.652 | 36.853 |
| 491 | OD1 | ASN | 792 | -3.101 | 51.672 | 36.552 |
| 492 | ND2 | ASN | 792 | -2.974 | 53.949 | 36.710 |
| 493 | C | ASN | 792 | 1.095 | 53.627 | 38.071 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 494 | O | ASN | 792 | 1.180 | 53.287 | 39.244 |
| 495 | N | LEU | 793 | 2.153 | 53.875 | 37.312 |
| 496 | CA | LEU | 793 | 3.485 | 53.780 | 37.860 |
| 497 | CB | LEU | 793 | 4.519 | 53.587 | 36.745 |
| 498 | CG | LEU | 793 | 4.503 | 52.206 | 36.089 |
| 499 | CD1 | LEU | 793 | 5.496 | 52.158 | 34.949 |
| 500 | CD2 | LEU | 793 | 4.844 | 51.160 | 37.133 |
| 501 | C | LEU | 793 | 3.743 | 55.069 | 38.610 |
| 502 | O | LEU | 793 | 3.089 | 56.089 | 38.368 |
| 503 | N | PRO | 794 | 4.688 | 55.017 | 39.536 |
| 504 | CA | PRO | 794 | 5.007 | 56.194 | 40.313 |
| 505 | CB | PRO | 794 | 6.158 | 55.901 | 41.257 |
| 506 | C | PRO | 794 | 5.387 | 57.333 | 39.374 |
| 507 | O | PRO | 794 | 6.407 | 57.247 | 38.695 |
| 508 | CG | PRO | 794 | 6.975 | 54.906 | 40.416 |
| 509 | CD | PRO | 794 | 5.907 | 54.014 | 39.774 |
| 510 | N | LEU | 795 | 4.575 | 58.390 | 39.335 |
| 511 | CA | LEU | 795 | 4.854 | 59.521 | 38.455 |
| 512 | CB | LEU | 795 | 4.144 | 60.790 | 38.942 |
| 513 | CG | LEU | 795 | 4.450 | 62.072 | 38.145 |
| 514 | CD1 | LEU | 795 | 3.440 | 63.137 | 38.497 |
| 515 | CD2 | LEU | 795 | 5.857 | 62.592 | 38.441 |
| 516 | C | LEU | 795 | 6.348 | 59.801 | 38.317 |
| 517 | O | LEU | 795 | 6.769 | 60.390 | 37.324 |
| 518 | N | GLU | 796 | 7.144 | 59.401 | 39.303 |
| 519 | CA | GLU | 796 | 8.594 | 59.611 | 39.238 |
| 520 | CB | GLU | 796 | 9.257 | 59.516 | 40.604 |
| 521 | C | GLU | 796 | 9.251 | 58.572 | 38.367 |
| 522 | O | GLU | 796 | 10.315 | 58.816 | 37.809 |
| 523 | CG | GLU | 796 | 10.791 | 59.813 | 40.678 |
| 524 | CD | GLU | 796 | 11.462 | 59.829 | 42.054 |
| 525 | OE1 | GLU | 796 | 10.836 | 59.739 | 43.101 |
| 526 | OE2 | GLU | 796 | 12.818 | 59.952 | 42.001 |
| 527 | N | ASP | 797 | 8.635 | 57.391 | 38.316 |
| 528 | CA | ASP | 797 | 9.107 | 56.271 | 37.501 |
| 529 | CB | ASP | 797 | 8.383 | 54.968 | 37.863 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 530 | CG | ASP | 797 | 8.665 | 54.507 | 39.275 |
| 531 | OD1 | ASP | 797 | 9.833 | 54.606 | 39.707 |
| 532 | OD2 | ASP | 797 | 7.718 | 54.035 | 39.945 |
| 533 | C | ASP | 797 | 8.751 | 56.630 | 36.074 |
| 534 | O | ASP | 797 | 9.563 | 56.517 | 35.157 |
| 535 | N | GLN | 798 | 7.505 | 57.058 | 35.905 |
| 536 | CA | GLN | 798 | 7.024 | 57.464 | 34.610 |
| 537 | CB | GLN | 798 | 5.731 | 58.245 | 34.769 |
| 538 | CG | GLN | 798 | 4.565 | 57.314 | 34.706 |
| 539 | CD | GLN | 798 | 3.234 | 57.989 | 34.856 |
| 540 | OE1 | GLN | 798 | 2.991 | 59.060 | 34.288 |
| 541 | NE2 | GLN | 798 | 2.451 | 57.562 | 35.945 |
| 542 | C | GLN | 798 | 8.087 | 58.294 | 33.928 |
| 543 | O | GLN | 798 | 8.230 | 58.244 | 32.704 |
| 544 | N | ILE | 799 | 8.859 | 59.014 | 34.744 |
| 545 | CA | ILE | 799 | 9.945 | 59.891 | 34.300 |
| 546 | CB | ILE | 799 | 10.055 | 61.119 | 35.215 |
| 547 | C | ILE | 799 | 11.341 | 59.269 | 34.219 |
| 548 | O | ILE | 799 | 12.199 | 59.805 | 33.514 |
| 549 | CG2 | ILE | 799 | 11.139 | 62.134 | 34.736 |
| 550 | CG1 | ILE | 799 | 8.718 | 61.899 | 35.423 |
| 551 | CD1 | ILE | 799 | 8.711 | 62.930 | 36.571 |
| 552 | N | THR | 800 | 11.614 | 58.187 | 34.943 |
| 553 | CA | THR | 800 | 12.959 | 57.615 | 34.862 |
| 554 | CB | THR | 800 | 13.318 | 56.724 | 36.081 |
| 555 | OG1 | THR | 800 | 12.556 | 56.983 | 37.258 |
| 556 | CG2 | THR | 800 | 14.781 | 56.946 | 36.483 |
| 557 | C | THR | 800 | 13.011 | 56.767 | 33.604 |
| 558 | O | THR | 800 | 14.017 | 56.733 | 32.883 |
| 559 | N | LEU | 801 | 11.899 | 56.083 | 33.354 |
| 560 | CA | LEU | 801 | 11.769 | 55.242 | 32.183 |
| 561 | CB | LEU | 801 | 10.417 | 54.506 | 32.226 |
| 562 | CG | LEU | 801 | 10.393 | 53.076 | 32.791 |
| 563 | CD1 | LEU | 801 | 11.605 | 52.841 | 33.658 |
| 564 | CD2 | LEU | 801 | 9.110 | 52.836 | 33.568 |
| 565 | C | LEU | 801 | 11.914 | 56.102 | 30.918 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 566 | O | LEU | 801 | 12.701 | 55.763 | 30.037 |
| 567 | N | ILE | 802 | 11.208 | 57.228 | 30.833 |
| 568 | CA | ILE | 802 | 11.323 | 58.067 | 29.641 |
| 569 | CB | ILE | 802 | 10.281 | 59.172 | 29.675 |
| 570 | C | ILE | 802 | 12.703 | 58.668 | 29.396 |
| 571 | O | ILE | 802 | 13.146 | 58.779 | 28.251 |
| 572 | CG2 | ILE | 802 | 10.291 | 60.052 | 28.387 |
| 573 | CG1 | ILE | 802 | 8.817 | 58.681 | 29.916 |
| 574 | CD1 | ILE | 802 | 7.790 | 59.774 | 30.275 |
| 575 | N | GLN | 803 | 13.385 | 59.047 | 30.473 |
| 576 | CA | GLN | 803 | 14.713 | 59.645 | 30.380 |
| 577 | CB | GLN | 803 | 15.049 | 60.371 | 31.662 |
| 578 | CG | GLN | 803 | 14.189 | 61.560 | 31.931 |
| 579 | CD | GLN | 803 | 14.417 | 62.099 | 33.320 |
| 580 | OE1 | GLN | 803 | 14.007 | 63.217 | 33.631 |
| 581 | NE2 | GLN | 803 | 15.012 | 61.291 | 34.324 |
| 582 | C | GLN | 803 | 15.778 | 58.609 | 30.157 |
| 583 | O | GLN | 803 | 16.896 | 58.911 | 29.733 |
| 584 | N | TYR | 804 | 15.455 | 57.378 | 30.473 |
| 585 | CA | TYR | 804 | 16.440 | 56.352 | 30.278 |
| 586 | CB | TYR | 804 | 16.211 | 55.236 | 31.272 |
| 587 | CG | TYR | 804 | 16.847 | 55.463 | 32.612 |
| 588 | CD1 | TYR | 804 | 17.000 | 56.745 | 33.149 |
| 589 | CE1 | TYR | 804 | 17.576 | 56.924 | 34.403 |
| 590 | CD2 | TYR | 804 | 17.279 | 54.382 | 33.352 |
| 591 | CE2 | TYR | 804 | 17.842 | 54.543 | 34.590 |
| 592 | CZ | TYR | 804 | 17.993 | 55.802 | 35.112 |
| 593 | OH | TYR | 804 | 18.526 | 55.969 | 36.371 |
| 594 | C | TYR | 804 | 16.322 | 55.808 | 28.878 |
| 595 | O | TYR | 804 | 17.320 | 55.564 | 28.207 |
| 596 | N | SER | 805 | 15.085 | 55.671 | 28.427 |
| 597 | CA | SER | 805 | 14.819 | 55.079 | 27.130 |
| 598 | CB | SER | 805 | 13.733 | 54.018 | 27.296 |
| 599 | OG | SER | 805 | 12.460 | 54.582 | 27.593 |
| 600 | C | SER | 805 | 14.439 | 55.972 | 25.956 |
| 601 | O | SER | 805 | 14.044 | 55.457 | 24.912 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 602 | N | TRP | 806 | 14.563 | 57.284 | 26.086 |
| 603 | CA | TRP | 806 | 14.169 | 58.124 | 24.968 |
| 604 | CB | TRP | 806 | 14.217 | 59.619 | 25.378 |
| 605 | CG | TRP | 806 | 15.580 | 60.225 | 25.512 |
| 606 | CD2 | TRP | 806 | 16.283 | 60.932 | 24.488 |
| 607 | CE2 | TRP | 806 | 17.566 | 61.242 | 24.996 |
| 608 | CE3 | TRP | 806 | 15.941 | 61.369 | 23.202 |
| 609 | CD1 | TRP | 806 | 16.442 | 60.117 | 26.570 |
| 610 | NE1 | TRP | 806 | 17.644 | 60.720 | 26.265 |
| 611 1 | CZ2 | TRP | 806 | 18.530 | 61.913 | 24.243 |
| 612 | CZ3 | TRP | 806 | 16.888 | 62.037 | 22.454 |
| 613 | CH2 | TRP | 806 | 18.166 | 62.320 | 22.983 |
| 614 | C | TRP | 806 | 14.938 | 57.847 | 23.648 |
| 615 | O | TRP | 806 | 14.360 | 57.934 | 22.560 |
| 616 | N | MET | 807 | 16.213 | 57.477 | 23.720 |
| 617 | CA | MET | 807 | 16.948 | 57.218 | 22.482 |
| 618 | CB | MET | 807 | 18.447 | 57.385 | 22.706 |
| 619 | CG | MET | 807 | 19.273 | 57.315 | 21.430 |
| 620 | SD | MET | 807 | 19.210 | 58.799 | 20.405 |
| 621 | CE | MET | 807 | 20.498 | 59.740 | 21.131 |
| 622 | C | MET | 807 | 16.648 | 55.813 | 21.956 |
| 623 | O | MET | 807 | 16.531 | 55.594 | 20.746 |
| 624 | N | CYS | 808 | 16.532 | 54.861 | 22.870 |
| 625 | CA | CYS | 808 | 16.219 | 53.498 | 22.483 |
| 626 | CB | CYS | 808 | 15.888 | 52.536 | 23.651 |
| 627 | C | CYS | 808 | 14.977 | 53.612 | 21.599 |
| 628 | O | CYS | 808 | 14.928 | 53.066 | 20.498 |
| 629 | SG | CYS | 808 | 16.909 | 52.717 | 25.155 |
| 630 | N | LEU | 809 | 13.995 | 54.372 | 22.084 |
| 631 | CA | LEU | 809 | 12.724 | 54.608 | 21.393 |
| 632 | CB | LEU | 809 | 11.788 | 55.409 | 22.288 |
| 633 | CG | LEU | 809 | 11.389 | 54.821 | 23.629 |
| 634 | CD1 | LEU | 809 | 10.765 | 55.921 | 24.466 |
| 635 | CD2 | LEU | 809 | 10.440 | 53.655 | 23.429 |
| 636 | C | LEU | 809 | 12.844 | 55.364 | 20.070 |
| 637 | O | LEU | 809 | 12.258 | 54.966 | 19.068 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 638 | N | SER | 810 | 13.564 | 56.481 | 20.102 |
| 639 | CA | SER | 810 | 13.757 | 57.322 | 18.930 |
| 640 | CB | SER | 810 | 14.560 | 58.614 | 19.213 |
| 641 | C | SER | 810 | 14.486 | 56.544 | 17.826 |
| 642 | O | SER | 810 | 14.054 | 56.542 | 16.672 |
| 643 | OG | SER | 810 | 13.900 | 59.523 | 20.087 |
| 644 | N | SER | 811 | 15.570 | 55.852 | 18.181 |
| 645 | CA | SER | 811 | 16.311 | 55.075 | 17.189 |
| 646 | CB | SER | 811 | 17.662 | 54.604 | 17.786 |
| 647 | C | SER | 811 | 15.555 | 53.823 | 16.742 |
| 648 | O | SER | 811 | 15.607 | 53.465 | 15.569 |
| 649 | OG | SER | 811 | 18.474 | 53.915 | 16.843 |
| 650 | N | PHE | 812 | 14.853 | 53.150 | 17.644 |
| 651 | CA | PHE | 812 | 14.108 | 51.963 | 17.235 |
| 652 | CB | PHE | 812 | 13.521 | 51.232 | 18.454 |
| 653 | CG | PHE | 812 | 12.934 | 49.891 | 18.122 |
| 654 | CD1 | PHE | 812 | 13.739 | 48.872 | 17.624 |
| 655 | CD2 | PHE | 812 | 11.569 | 49.676 | 18.214 |
| 656 | CE1 | PHE | 812 | 13.195 | 47.657 | 17.222 |
| 657 | CE2 | PHE | 812 | 11.011 | 48.450 | 17.812 |
| 658 | CZ | PHE | 812 | 11.827 | 47.446 | 17.310 |
| 659 | C | PHE | 812 | 12.985 | 52.339 | 16.243 |
| 660 | O | PHE | 812 | 12.573 | 51.528 | 15.408 |
| 661 | N | ALA | 813 | 12.495 | 53.570 | 16.323 |
| 662 | CA | ALA | 813 | 11.446 | 54.012 | 15.416 |
| 663 | CB | ALA | 813 | 10.748 | 55.228 | 15.974 |
| 664 | C | ALA | 813 | 12.025 | 54.327 | 14.038 |
| 665 | O | ALA | 813 | 11.487 | 53.875 | 13.025 |
| 666 | N | LEU | 814 | 13.104 | 55.109 | 13.987 |
| 667 | CA | LEU | 814 | 13.724 | 55.420 | 12.700 |
| 668 | CB | LEU | 814 | 14.965 | 56.293 | 12.899 |
| 669 | CG | LEU | 814 | 15.989 | 56.573 | 11.792 |
| 670 | CD1 | LEU | 814 | 15.697 | 57.812 | 10.905 |
| 671 | CD2 | LEU | 814 | 17.278 | 56.803 | 12.556 |
| 672 | C | LEU | 814 | 14.079 | 54.080 | 12.050 |
| 673 | O | LEU | 814 | 13.740 | 53.851 | 10.888 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 674 | N | SER | 815 | 14.728 | 53.177 | 12.790 |
| 675 | CA | SER | 815 | 15.034 | 51.888 | 12.204 |
| 676 | C | SER | 815 | 13.831 | 51.476 | 11.361 |
| 677 | O | SER | 815 | 13.916 | 51.310 | 10.148 |
| 678 | CB | SER | 815 | 15.238 | 50.928 | 13.368 |
| 679 | OG | SER | 815 | 15.627 | 49.668 | 12.894 |
| 680 | N | TRP | 816 | 12.685 | 51.361 | 12.019 |
| 681 | CA | TRP | 816 | 11.434 | 50.978 | 11.379 |
| 682 | CB | TRP | 816 | 10.315 | 51.055 | 12.403 |
| 683 | CG | TRP | 816 | 9.047 | 50.515 | 11.895 |
| 684 | CD2 | TRP | 816 | 8.731 | 49.134 | 11.739 |
| 685 | CE2 | TRP | 816 | 7.411 | 49.065 | 11.243 |
| 686 | CE3 | TRP | 816 | 9.462 | 47.951 | 11.926 |
| 687 | CD1 | TRP | 816 | 7.930 | 51.215 | 11.522 |
| 688 | NE1 | TRP | 816 | 6.939 | 50.341 | 11.139 |
| 689 | CZ2 | TRP | 816 | 6.775 | 47.845 | 10.988 |
| 690 | CZ3 | TRP | 816 | 8.835 | 46.731 | 11.667 |
| 691 | CH2 | TRP | 816 | 7.504 | 46.690 | 11.180 |
| 692 | C | TRP | 816 | 11.010 | 51.780 | 10.145 |
| 693 | O | TRP | 816 | 10.683 | 51.213 | 9.110 |
| 694 | N | ARG | 817 | 10.969 | 53.099 | 10.266 |
| 695 | CA | ARG | 817 | 10.557 | 53.921 | 9.143 |
| 696 | CB | ARG | 817 | 10.573 | 55.403 | 9.531 |
| 697 | CG | ARG | 817 | 9.526 | 55.805 | 10.563 |
| 698 | CD | ARG | 817 | 9.295 | 57.307 | 10.536 |
| 699 | NE | ARG | 817 | 10.508 | 58.059 | 10.853 |
| 700 | CZ | ARG | 817 | 11.111 | 58.023 | 12.035 |
| 701 | NH1 | ARG | 817 | 10.639 | 57.318 | 13.120 |
| 702 | NH2 | ARG | 817 | 12.266 | 58.742 | 12.185 |
| 703 | C | ARG | 817 | 11.509 | 53.676 | 7.978 |
| 704 | O | ARG | 817 | 11.117 | 53.731 | 6.809 |
| 705 | N | SER | 818 | 12.756 | 53.381 | 8.314 |
| 706 | CA | SER | 818 | 13.786 | 53.127 | 7.321 |
| 707 | CB | SER | 818 | 15.145 | 53.168 | 7.986 |
| 708 | OG | SER | 818 | 15.502 | 54.445 | 8.498 |
| 709 | C | SER | 818 | 13.614 | 51.810 | 6.609 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 710 | O | SER | 818 | 13.688 | 51.727 | 5.385 |
| 711 | N | TYR | 819 | 13.409 | 50.767 | 7.383 |
| 712 | CA | TYR | 819 | 13.229 | 49.477 | 6.787 |
| 713 | CB | TYR | 819 | 13.071 | 48.486 | 7.923 |
| 714 | CG | TYR | 819 | 12.184 | 47.309 | 7.698 |
| 715 | CD1 | TYR | 819 | 12.514 | 46.302 | 6.796 |
| 716 | CE1 | TYR | 819 | 11.782 | 45.115 | 6.751 |
| 717 | CD2 | TYR | 819 | 11.087 | 47.115 | 8.532 |
| 718 | CE2 | TYR | 819 | 10.353 | 45.948 | 8.494 |
| 719 | CZ | TYR | 819 | 10.704 | 44.948 | 7.616 |
| 720 | OH | TYR | 819 | 9.962 | 43.786 | 7.582 |
| 721 | C | TYR | 819 | 12.008 | 49.556 | 5.874 |
| 722 | O | TYR | 819 | 12.083 | 49.263 | 4.685 |
| 723 | N | LYS | 820 | 10.905 | 50.032 | 6.412 |
| 724 | CA | LYS | 820 | 9.666 | 50.118 | 5.652 |
| 725 | CB | LYS | 820 | 8.555 | 50.467 | 6.636 |
| 726 | CG | LYS | 820 | 7.247 | 50.738 | 5.915 |
| 727 | CD | LYS | 820 | 6.567 | 49.442 | 5.644 |
| 728 | C | LYS | 820 | 9.616 | 51.061 | 4.440 |
| 729 | O | LYS | 820 | 8.699 | 50.967 | 3.621 |
| 730 | CE | LYS | 820 | 5.244 | 49.709 | 4.914 |
| 731 | NZ | LYS | 820 | 4.568 | 48.428 | 4.643 |
| 732 | N | HIS | 821 | 10.606 | 51.940 | 4.292 |
| 733 | CA | HIS | 821 | 10.583 | 52.921 | 3.208 |
| 734 | CB | HIS | 821 | 10.437 | 54.309 | 3.843 |
| 735 | C | HIS | 821 | 11.787 | 52.899 | 2.268 |
| 736 | O | HIS | 821 | 12.029 | 53.863 | 1.545 |
| 737 | CG | HIS | 821 | 9.186 | 55.009 | 3.400 |
| 738 | ND1 | HIS | 821 | 8.237 | 54.502 | 2.516 |
| 739 | CD2 | HIS | 821 | 8.840 | 56.279 | 3.843 |
| 740 | CE1 | HIS | 821 | 7.366 | 55.529 | 2.498 |
| 741 | NE2 | HIS | 821 | 7.651 | 56.623 | 3.254 |
| 742 | N | THR | 822 | 12.538 | 51.803 | 2.266 |
| 743 | CA | THR | 822 | 13.716 | 51.707 | 1.413 |
| 744 | CB | THR | 822 | 14.564 | 52.989 | 1.506 |
| 745 | C | THR | 822 | 14.547 | 50.512 | 1.846 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 746 | O | THR | 822 | 15.775 | 50.564 | 1.832 |
| 747 | OG1 | THR | 822 | 13.817 | 54.114 | 1.061 |
| 748 | CG2 | THR | 822 | 15.853 | 53.021 | 0.656 |
| 749 | N | ASN | 823 | 13.871 | 49.445 | 2.243 |
| 750 | CA | ASN | 823 | 14.534 | 48.220 | 2.692 |
| 751 | CB | ASN | 823 | 15.012 | 47.386 | 1.485 |
| 752 | C | ASN | 823 | 15.748 | 48.439 | 3.611 |
| 753 | O | ASN | 823 | 16.647 | 47.601 | 3.634 |
| 754 | CG | ASN | 823 | 13.948 | 46.974 | 0.462 |
| 755 | OD1 | ASN | 823 | 13.800 | 47.565 | -0.598 |
| 756 | ND2 | ASN | 823 | 13.160 | 45.972 | 0.742 |
| 757 | N | SER | 824 | 15.808 | 49.544 | 4.349 |
| 758 | CA | SER | 824 | 16.954 | 49.752 | 5.244 |
| 759 | CB | SER | 824 | 17.191 | 48.480 | 6.076 |
| 760 | C | SER | 824 | 18.265 | 50.165 | 4.543 |
| 761 | O | SER | 824 | 19.360 | 49.892 | 5.060 |
| 762 | OG | SER | 824 | 17.680 | 47.386 | 5.293 |
| 763 | N | GLN | 825 | 18.143 | 50.818 | 3.385 |
| 764 | CA | GLN | 825 | 19.282 | 51.297 | 2.587 |
| 765 | CB | GLN | 825 | 18.906 | 51.322 | 1.097 |
| 766 | C | GLN | 825 | 19.584 | 52.736 | 3.015 |
| 767 | O | GLN | 825 | 20.737 | 53.162 | 3.140 |
| 768 | CG | GLN | 825 | 20.033 | 51.767 | 0.108 |
| 769 | CD | GLN | 825 | 19.715 | 51.875 | -1.389 |
| 770 | OE1 | GLN | 825 | 20.556 | 52.257 | -2.187 |
| 771 | NE2 | GLN | 825 | 18.517 | 51.579 | -1.825 |
| 772 | N | PHE | 826 | 18.496 | 53.472 | 3.223 |
| 773 | CA | PHE | 826 | 18.522 | 54.867 | 3.621 |
| 774 | CB | PHE | 826 | 17.631 | 55.670 | 2.652 |
| 775 | C | PHE | 826 | 18.014 | 54.998 | 5.074 |
| 776 | O | PHE | 826 | 17.453 | 54.048 | 5.623 |
| 777 | CG | PHE | 826 | 18.000 | 55.595 | 1.162 |
| 778 | CD1 | PHE | 826 | 17.467 | 54.574 | 0.368 |
| 779 | CD2 | PHE | 826 | 18.910 | 56.497 | 0.605 |
| 780 | CE1 | PHE | 826 | 17.853 | 54.445 | -0.962 |
| 781 | CE2 | PHE | 826 | 19.288 | 56.374 | -0.729 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 782 | CZ | PHE | 826 | 18.762 | 55.347 | -1.511 |
| 783 | N | LEU | 827 | 18.251 | 56.154 | 5.696 |
| 784 | CA | LEU | 827 | 17.788 | 56.453 | 7.058 |
| 785 | CB | LEU | 827 | 18.886 | 57.119 | 7.902 |
| 786 | CG | LEU | 827 | 19.920 | 56.225 | 8.582 |
| 787 | CD1 | LEU | 827 | 20.807 | 57.060 | 9.505 |
| 788 | CD2 | LEU | 827 | 19.202 | 55.145 | 9.369 |
| 789 | C | LEU | 827 | 16.669 | 57.452 | 6.843 |
| 790 | O | LEU | 827 | 16.913 | 58.551 | 6.372 |
| 791 | N | TYR | 828 | 15.441 | 57.090 | 7.191 |
| 792 | CA | TYR | 828 | 14.331 | 58.004 | 6.948 |
| 793 | CB | TYR | 828 | 13.148 | 57.169 | 6.351 |
| 794 | C | TYR | 828 | 13.884 | 58.813 | 8.148 |
| 795 | O | TYR | 828 | 12.825 | 58.556 | 8.710 |
| 796 | CG | TYR | 828 | 13.150 | 57.149 | 4.832 |
| 797 | CD1 | TYR | 828 | 14.257 | 56.721 | 4.017 |
| 798 | CD2 | TYR | 828 | 11.970 | 57.613 | 4.164 |
| 799 | CE1 1 | TYR | 828 | 14.156 | 56.768 | 2.578 |
| 800 | CE2 | TYR | 828 | 11.858 | 57.630 | 2.734 |
| 801 | CZ | TYR | 828 | 12.951 | 57.195 | 1.913 |
| 802 | OH | TYR | 828 | 12.850 | 57.211 | 0.539 |
| 803 | N | PHE | 829 | 14.660 | 59.812 | 8.535 |
| 804 | CA | PHE | 829 | 14.262 | 60.597 | 9.684 |
| 805 | CB | PHE | 829 | 15.320 | 61.671 | 9.972 |
| 806 | CG | PHE | 829 | 16.625 | 61.104 | 10.525 |
| 807 | CD1 | PHE | 829 | 17.590 | 60.544 | 9.688 |
| 808 | CD2 | PHE | 829 | 16.893 | 61.149 | 11.891 |
| 809 | CE1 | PHE | 829 | 18.803 | 60.037 | 10.205 |
| 810 | CE2 | PHE | 829 | 18.093 | 60.642 | 12.411 |
| 811 | CZ | PHE | 829 | 19.049 | 60.094 | 11.567 |
| 812 | C | PHE | 829 | 12.815 | 61.141 | 9.542 |
| 813 | O | PHE | 829 | 12.088 | 61.226 | 10.530 |
| 814 | N | ALA | 830 | 12.389 | 61.465 | 8.323 |
| 815 | CA | ALA | 830 | 11.012 | 61.912 | 8.054 |
| 816 | CB | ALA | 830 | 10.922 | 63.421 | 8.091 |
| 817 | C | ALA | 830 | 10.751 | 61.380 | 6.641 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 818 | O | ALA | 830 | 11.690 | 61.306 | 5.852 |
| 819 | N | PRO | 831 | 9.507 | 60.970 | 6.295 |
| 820 | CD | PRO | 831 | 8.262 | 60.670 | 7.030 |
| 821 | CA | PRO | 831 | 9.417 | 60.478 | 4.911 |
| 822 | CB | PRO | 831 | 7.961 | 60.011 | 4.804 |
| 823 | CG | PRO | 831 | 7.665 | 59.539 | 6.198 |
| 824 | C | PRO | 831 | 9.790 | 61.542 | 3.867 |
| 825 | O | PRO | 831 | 9.926 | 61.245 | 2.674 |
| 826 | N | ASP | 832 | 9.977 | 62.772 | 4.352 |
| 827 | CA | ASP | 832 | 10.339 | 63.935 | 3.539 |
| 828 | CB | ASP | 832 | 9.543 | 65.143 | 3.974 |
| 829 | CG | ASP | 832 | 10.046 | 65.683 | 5.300 |
| 830 | OD1 | ASP | 832 | 10.054 | 64.891 | 6.255 |
| 831 | OD2 | ASP | 832 | 10.454 | 66.863 | 5.392 |
| 832 | C | ASP | 832 | 11.798 | 64.340 | 3.753 |
| 833 | O | ASP | 832 | 12.284 | 65.254 | 3.090 |
| 834 | N | LEU | 833 | 12.472 | 63.722 | 4.713 |
| 835 | CA | LEU | 833 | 13.861 | 64.082 | 4.992 |
| 836 | CB | LEU | 833 | 13.930 | 64.909 | 6.282 |
| 837 | CG | LEU | 833 | 15.281 | 65.514 | 6.662 |
| 838 | CD1 | LEU | 833 | 16.340 | 64.441 | 6.845 |
| 839 | CD2 | LEU | 833 | 15.696 | 66.464 | 5.573 |
| 840 | C | LEU | 833 | 14.716 | 62.823 | 5.121 |
| 841 | O | LEU | 833 | 14.824 | 62.254 | 6.207 |
| 842 | N | VAL | 834 | 15.334 | 62.407 | 4.019 |
| 843 | CA | VAL | 834 | 16.158 | 61.190 | 3.977 |
| 844 | CB | VAL | 834 | 15.808 | 60.368 | 2.693 |
| 845 | C | VAL | 834 | 17.674 | 61.416 | 3.967 |
| 846 | O | VAL | 834 | 18.170 | 62.415 | 3.448 |
| 847 | CG1 | VAL | 834 | 16.396 | 60.934 | 1.376 |
| 848 | CG2 | VAL | 834 | 14.282 | 60.184 | 2.512 |
| 849 | N | PHE | 835 | 18.416 | 60.488 | 4.560 |
| 850 | CA | PHE | 835 | 19.854 | 60.616 | 4.494 |
| 851 | CB | PHE | 835 | 20.630 | 60.228 | 5.745 |
| 852 | C | PHE | 835 | 20.280 | 59.596 | 3.485 |
| 853 | O | PHE | 835 | 20.286 | 58.387 | 3.725 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 854 | CG | PHE | 835 | 20.548 | 61.224 | 6.842 |
| 855 | CD1 | PHE | 835 | 19.878 | 62.496 | 6.797 |
| 856 | CD2 | PHE | 835 | 21.319 | 60.857 | 7.977 |
| 857 | CE1 | PHE | 835 | 20.101 | 63.432 | 7.850 |
| 858 | CE2 | PHE | 835 | 21.570 | 61.796 | 9.005 |
| 859 | CZ | PHE | 835 | 21.027 | 63.115 | 8.896 |
| 860 | N | ASN | 836 | 20.603 | 60.152 | 2.328 |
| 861 | CA | ASN | 836 | 21.032 | 59.451 | 1.146 |
| 862 | CB | ASN | 836 | 20.450 | 60.127 | -0.060 |
| 863 | CG | ASN | 836 | 20.793 | 61.596 | -0.078 |
| 864 | OD1 | ASN | 836 | 21.630 | 62.058 | 0.693 |
| 865 | ND2 | ASN | 836 | 20.115 | 62.368 | -1.049 |
| 866 | C | ASN | 836 | 22.534 | 59.501 | 0.992 |
| 867 | O | ASN | 836 | 23.249 | 60.240 | 1.687 |
| 868 | N | GLU | 837 | 22.949 | 58.760 | -0.032 |
| 869 | CA | GLU | 837 | 24.329 | 58.541 | -0.415 |
| 870 | CB | GLU | 837 | 24.396 | 57.808 | -1.742 |
| 871 | CG | GLU | 837 | 25.840 | 57.675 | -2.174 |
| 872 | CD | GLU | 837 | 26.031 | 57.213 | -3.602 |
| 873 | OE1 | GLU | 837 | 26.427 | 58.052 | -4.450 |
| 874 | OE2 | GLU | 837 | 25.794 | 56.012 | -3.869 |
| 875 | C | GLU | 837 | 25.227 | 59.722 | -0.535 |
| 876 | O | GLU | 837 | 26.416 | 59.668 | -0.228 |
| 877 | N | GLU | 838 | 24.647 | 60.782 | -1.040 |
| 878 | CA | GLU | 838 | 25.378 | 61.978 | -1.248 |
| 879 | CB | GLU | 838 | 24.642 | 62.790 | -2.263 |
| 880 | CG | GLU | 838 | 25.038 | 62.639 | -3.742 |
| 881 | CD | GLU | 838 | 26.168 | 61.663 | -4.134 |
| 882 | OE1 | GLU | 838 | 27.334 | 61.821 | -3.776 |
| 883 | C | GLU | 838 | 25.407 | 62.686 | 0.081 |
| 884 | O | GLU | 838 | 26.369 | 63.362 | 0.419 |
| 885 | OE2 | GLU | 838 | 25.730 | 60.547 | -4.780 |
| 886 | N | LYS | 839 | 24.345 | 62.521 | 0.845 |
| 887 | CA | LYS | 839 | 24.288 | 63.151 | 2.151 |
| 888 | CB | LYS | 839 | 22.863 | 63.198 | 2.639 |
| 889 | CG | LYS | 839 | 22.311 | 64.577 | 2.459 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 890 | CD | LYS | 839 | 20.763 | 64.663 | 2.497 |
| 891 | C | LYS | 839 | 25.096 | 62.361 | 3.137 |
| 892 | O | LYS | 839 | 25.659 | 62.856 | 4.103 |
| 893 | CE | LYS | 839 | 20.171 | 65.129 | 3.825 |
| 894 | NZ | LYS | 839 | 18.664 | 65.315 | 3.753 |
| 895 | N | MET | 840 | 25.122 | 61.087 | 2.878 |
| 896 | CA | MET | 840 | 25.789 | 60.159 | 3.731 |
| 897 | CB | MET | 840 | 25.603 | 58.818 | 3.159 |
| 898 | CG | MET | 840 | 25.733 | 57.886 | 4.225 |
| 899 | SD | MET | 840 | 25.047 | 58.641 | 5.697 |
| 900 | CE | MET | 840 | 24.686 | 57.251 | 6.529 |
| 901 | C | MET | 840 | 27.226 | 60.385 | 3.766 |
| 902 | O | MET | 840 | 28.007 | 59.936 | 4.606 |
| 903 | N | HIS | 841 | 27.573 | 61.081 | 2.751 |
| 904 | CA | HIS | 841 | 28.906 | 61.294 | 2.575 |
| 905 | CB | HIS | 841 | 29.072 | 61.097 | 1.163 |
| 906 | C | HIS | 841 | 29.329 | 62.646 | 3.089 |
| 907 | O | HIS | 841 | 30.409 | 63.072 | 2.743 |
| 908 | CG | HIS | 841 | 30.015 | 59.972 | 0.852 |
| 909 | ND1 | HIS | 841 | 30.678 | 59.182 | 1.788 |
| 910 | CD2 | HIS | 841 | 30.332 | 59.596 | -0.446 |
| 911 | CE1 | HIS | 841 | 31.356 | 58.365 | 0.960 |
| 912 | NE2 | HIS | 841 | 31.210 | 58.546 | -0.381 |
| 913 | N | GLN | 842 | 28.541 | 63.372 | 3.892 |
| 914 | CA | GLN | 842 | 29.156 | 64.615 | 4.349 |
| 915 | CB | GLN | 842 | 28.132 | 65.700 | 4.833 |
| 916 | C | GLN | 842 | 30.070 | 64.165 | 5.438 |
| 917 | O | GLN | 842 | 30.086 | 62.991 | 5.841 |
| 918 | CG | GLN | 842 | 27.078 | 65.394 | 5.944 |
| 919 | CD | GLN | 842 | 27.589 | 65.356 | 7.401 |
| 920 | OE1 | GLN | 842 | 28.755 | 65.223 | 7.682 |
| 921 | NE2 | GLN | 842 | 26.648 | 65.484 | 8.434 |
| 922 | N | SER | 843 | 30.830 | 65.108 | 5.957 |
| 923 | CA | SER | 843 | 31.759 | 64.768 | 7.003 |
| 924 | CB | SER | 843 | 32.336 | 66.099 | 7.608 |
| 925 | C | SER | 843 | 31.391 | 63.778 | 8.034 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 926 | O | SER | 843 | 31.420 | 62.553 | 7.872 |
| 927 | OG | SER | 843 | 33.011 | 66.917 | 6.653 |
| 928 | N | ALA | 844 | 30.999 | 64.345 | 9.132 |
| 929 | CA | ALA | 844 | 30.810 | 63.473 | 10.210 |
| 930 | CB | ALA | 844 | 31.009 | 64.296 | 11.460 |
| 931 | C | ALA | 844 | 29.573 | 62.635 | 10.269 |
| 932 | O | ALA | 844 | 29.221 | 62.183 | 11.333 |
| 933 | N | MET | 845 | 28.968 | 62.331 | 9.135 |
| 934 | CA | MET | 845 | 27.710 | 61.611 | 9.171 |
| 935 | CB | MET | 845 | 26.946 | 61.885 | 7.902 |
| 936 | CG | MET | 845 | 25.462 | 61.900 | 8.075 |
| 937 | SD | MET | 845 | 24.889 | 63.585 | 7.922 |
| 938 | CE | MET | 845 | 24.327 | 63.891 | 9.531 |
| 939 | C | MET | 845 | 27.725 | 60.135 | 9.325 |
| 940 | O | MET | 845 | 27.067 | 59.529 | 10.174 |
| 941 | N | TYR | 846 | 28.451 | 59.556 | 8.404 |
| 942 | CA | TYR | 846 | 28.495 | 58.155 | 8.351 |
| 943 | CB | TYR | 846 | 29.415 | 57.741 | 7.245 |
| 944 | CG | TYR | 846 | 29.192 | 56.313 | 7.082 |
| 945 | CD1 | TYR | 846 | 28.044 | 55.867 | 6.448 |
| 946 | CE1 | TYR | 846 | 27.676 | 54.551 | 6.503 |
| 947 | CD2 | TYR | 846 | 29.983 | 55.399 | 7.763 |
| 948 | CE2 | TYR | 846 | 29.627 | 54.082 | 7.832 |
| 949 | CZ | TYR | 846 | 28.470 | 53.663 | 7.203 |
| 950 | OH | TYR | 846 | 28.093 | 52.340 | 7.273 |
| 951 | C | TYR | 846 | 28.882 | 57.431 | 9.614 |
| 952 | O | TYR | 846 | 28.392 | 56.346 | 9.902 |
| 953 | N | GLU | 847 | 29.778 | 58.022 | 10.375 |
| 954 | CA | GLU | 847 | 30.229 | 57.375 | 11.588 |
| 955 | CB | GLU | 847 | 31.460 | 58.107 | 12.190 |
| 956 | C | GLU | 847 | 29.169 | 57.332 | 12.645 |
| 957 | O | GLU | 847 | 29.200 | 56.488 | 13.527 |
| 958 | CG | GLU | 847 | 32.714 | 58.107 | 11.274 |
| 959 | CD | GLU | 847 | 32.724 | 59.178 | 10.124 |
| 960 | OE1 | GLU | 847 | 31.953 | 60.136 | 10.244 |
| 961 | OE2 | GLU | 847 | 33.520 | 58.935 | 9.212 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 962 | N | LEU | 848 | 28.273 | 58.308 | 12.559 |
| 963 | CA | LEU | 848 | 27.154 | 58.520 | 13.459 |
| 964 | CB | LEU | 848 | 26.290 | 59.841 | 13.644 |
| 965 | C | LEU | 848 | 25.982 | 57.658 | 12.978 |
| 966 | O | LEU | 848 | 25.422 | 56.844 | 13.718 |
| 967 | CG | LEU | 848 | 26.887 | 61.083 | 14.323 |
| 968 | CD1 | LEU | 848 | 27.925 | 61.493 | 13.330 |
| 969 | CD2 | LEU | 848 | 26.178 | 62.437 | 14.532 |
| 970 | N | CYS | 849 | 25.650 | 57.839 | 11.701 |
| 971 | CA | CYS | 849 | 24.532 | 57.155 | 11.019 |
| 972 | CB | CYS | 849 | 24.405 | 57.671 | 9.579 |
| 973 | SG | CYS | 849 | 23.678 | 59.351 | 9.531 |
| 974 | C | CYS | 849 | 24.543 | 55.623 | 10.958 |
| 975 | O | CYS | 849 | 23.506 | 54.988 | 10.769 |
| 976 | N | GLN | 850 | 25.715 | 55.048 | 11.133 |
| 977 | CA | GLN | 850 | 25.944 | 53.608 | 11.062 |
| 978 | CB | GLN | 850 | 27.406 | 53.378 | 11.325 |
| 979 | CG | GLN | 850 | 27.924 | 51.916 | 11.314 |
| 980 | C | GLN | 850 | 25.184 | 52.763 | 12.043 |
| 981 | O | GLN | 850 | 24.733 | 51.634 | 11.793 |
| 982 | CD | GLN | 850 | 27.809 | 51.215 | 12.681 |
| 983 | OE1 | GLN | 850 | 27.149 | 50.200 | 12.827 |
| 984 | NE2 | GLN | 850 | 28.565 | 51.802 | 13.727 |
| 985 | N | GLY | 851 | 25.145 | 53.330 | 13.217 |
| 986 | CA | GLY | 851 | 24.522 | 52.700 | 14.298 |
| 987 | C | GLY | 851 | 23.106 | 52.528 | 14.083 |
| 988 | O | GLY | 851 | 22.547 | 51.454 | 14.255 |
| 989 | N | MET | 852 | 22.519 | 53.591 | 13.607 |
| 990 | CA | MET | 852 | 21.140 | 53.494 | 13.346 |
| 991 | CB | MET | 852 | 20.608 | 54.871 | 13.110 |
| 992 | CG | MET | 852 | 20.424 | 55.544 | 14.421 |
| 993 | SD | MET | 852 | 21.486 | 56.946 | 14.587 |
| 994 | CE | MET | 852 | 20.673 | 58.004 | 13.550 |
| 995 | C | MET | 852 | 20.810 | 52.578 | 12.197 |
| 996 | O | MET | 852 | 19.837 | 51.835 | 12.279 |
| 997 | N | HIS | 853 | 21.612 | 52.609 | 11.129 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 998 | CA | HIS | 853 | 21.304 | 51.767 | 9.970 |
| 999 | CB | HIS | 853 | 22.420 | 52.169 | 8.960 |
| 1000 | C | HIS | 853 | 21.379 | 50.345 | 10.421 |
| 1001 | O | HIS | 853 | 20.659 | 49.469 | 9.951 |
| 1002 | CG | HIS | 853 | 22.400 | 51.593 | 7.579 |
| 1003 | ND1 | HIS | 853 | 23.387 | 50.809 | 7.081 |
| 1004 | CD2 | HIS | 853 | 21.537 | 51.981 | 6.514 |
| 1005 | CE1 | HIS | 853 | 23.138 | 50.710 | 5.752 |
| 1006 | NE2 | HIS | 853 | 21.998 | 51.423 | 5.314 |
| 1007 | N | GLN | 854 | 22.262 | 50.110 | 11.355 |
| 1008 | CA | GLN | 854 | 22.339 | 48.801 | 11.876 |
| 1009 | CB | GLN | 854 | 23.440 | 48.628 | 12.964 |
| 1010 | C | GLN | 854 | 20.984 | 48.364 | 12.473 |
| 1011 | O | GLN | 854 | 20.608 | 47.196 | 12.391 |
| 1012 | CG | GLN | 854 | 24.048 | 47.211 | 13.119 |
| 1013 | CD | GLN | 854 | 23.110 | 46.185 | 13.774 |
| 1014 | OE1 | GLN | 854 | 22.675 | 45.231 | 13.152 |
| 1015 | NE2 | GLN | 854 | 22.838 | 46.395 | 15.146 |
| 1016 | N | ILE | 855 | 20.241 | 49.258 | 13.103 |
| 1017 | CA | ILE | 855 | 19.007 | 48.728 | 13.636 |
| 1018 | CB | ILE | 855 | 18.540 | 49.626 | 14.847 |
| 1019 | C | ILE | 855 | 18.008 | 48.491 | 12.500 |
| 1020 | O | ILE | 855 | 17.340 | 47.453 | 12.470 |
| 1021 | CG2 | ILE | 855 | 18.443 | 51.150 | 14.670 |
| 1022 | CG1 | ILE | 855 | 17.328 | 49.137 | 15.655 |
| 1023 | CD1 | ILE | 855 | 17.540 | 47.718 | 16.167 |
| 1024 | N | SER | 856 | 17.957 | 49.403 | 11.534 |
| 1025 | CA | SER | 856 | 17.062 | 49.251 | 10.377 |
| 1026 | CB | SER | 856 | 17.225 | 50.415 | 9.416 |
| 1027 | OG | SER | 856 | 16.618 | 50.174 | 8.183 |
| 1028 | C | SER | 856 | 17.331 | 47.979 | 9.573 |
| 1029 | O | SER | 856 | 16.412 | 47.323 | 9.066 |
| 1030 | N | LEU | 857 | 18.611 | 47.666 | 9.421 |
| 1031 | CA | LEU | 857 | 19.038 | 46.485 | 8.683 |
| 1032 | CB | LEU | 857 | 20.575 | 46.447 | 8.642 |
| 1033 | C | LEU | 857 | 18.502 | 45.237 | 9.374 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1034 | O | LEU | 857 | 17.965 | 44.322 | 8.748 |
| 1035 | CG | LEU | 857 | 21.320 | 47.661 | 8.023 |
| 1036 | CD1 | LEU | 857 | 22.829 | 47.385 | 7.974 |
| 1037 | CD2 | LEU | 857 | 20.812 | 48.015 | 6.616 |
| 1038 | N | GLN | 858 | 18.650 | 45.240 | 10.691 |
| 1039 | CA | GLN | 858 | 18.238 | 44.144 | 11.542 |
| 1040 | CB | GLN | 858 | 18.684 | 44.468 | 12.954 |
| 1041 | CG | GLN | 858 | 19.288 | 43.311 | 13.700 |
| 1042 | CD | GLN | 858 | 20.205 | 42.435 | 12.870 |
| 1043 | OE1 | GLN | 858 | 20.787 | 42.893 | 11.862 |
| 1044 | C | GLN | 858 | 16.746 | 43.849 | 11.479 |
| 1045 | O | GLN | 858 | 16.340 | 42.686 | 11.548 |
| 1046 | NE2 | GLN | 858 | 20.290 | 41.153 | 13.122 |
| 1047 | N | PHE | 859 | 15.939 | 44.899 | 11.363 |
| 1048 | CA | PHE | 859 | 14.487 | 44.761 | 11.249 |
| 1049 | CB | PHE | 859 | 13.657 | 46.030 | 11.128 |
| 1050 | C | PHE | 859 | 14.240 | 44.094 | 9.903 |
| 1051 | O | PHE | 859 | 13.519 | 43.102 | 9.792 |
| 1052 | CG | PHE | 859 | 13.725 | 46.803 | 12.385 |
| 1053 | CD1 | PHE | 859 | 14.553 | 46.478 | 13.511 |
| 1054 | CD2 | PHE | 859 | 12.875 | 47.932 | 12.422 |
| 1055 | CE1 | PHE | 859 | 14.586 | 47.350 | 14.620 |
| 1056 | CE2 | PHE | 859 | 12.808 | 48.720 | 13.599 |
| 1057 | CZ | PHE | 859 | 13.718 | 48.480 | 14.649 |
| 1058 | N | VAL | 860 | 14.871 | 44.645 | 8.879 |
| 1059 | CA | VAL | 860 | 14.758 | 44.135 | 7.528 |
| 1060 | CB | VAL | 860 | 15.677 | 44.943 | 6.610 |
| 1061 | C | VAL | 860 | 15.115 | 42.651 | 7.454 |
| 1062 | O | VAL | 860 | 14.315 | 41.819 | 7.012 |
| 1063 | CG1 | VAL | 860 | 15.273 | 46.430 | 6.469 |
| 1064 | CG2 | VAL | 860 | 15.831 | 44.420 | 5.154 |
| 1065 | N | ARG | 861 | 16.326 | 42.334 | 7.893 |
| 1066 | CA | ARG | 861 | 16.825 | 40.972 | 7.892 |
| 1067 | CB | ARG | 861 | 18.162 | 40.913 | 8.593 |
| 1068 | CG | ARG | 861 | 18.870 | 39.604 | 8.405 |
| 1069 | CD | ARG | 861 | 19.848 | 39.390 | 9.514 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1070 | NE | ARG | 861 | 19.228 | 38.616 | 10.571 |
| 1071 | CZ | ARG | 861 | 19.711 | 38.535 | 11.801 |
| 1072 | NH1 | ARG | 861 | 20.935 | 39.043 | 12.107 |
| 1073 | NH2 | ARG | 861 | 19.004 | 37.885 | 12.767 |
| 1074 | C | ARG | 861 | 15.898 | 40.026 | 8.614 |
| 1075 | O | ARG | 861 | 15.741 | 38.865 | 8.228 |
| 1076 | N | LEU | 862 | 15.297 | 40.508 | 9.689 |
| 1077 | CA | LEU | 862 | 14.413 | 39.666 | 10.468 |
| 1078 | CB | LEU | 862 | 14.535 | 40.041 | 11.944 |
| 1079 | CG | LEU | 862 | 15.646 | 39.262 | 12.636 |
| 1080 | CD1 | LEU | 862 | 15.630 | 39.590 | 14.115 |
| 1081 | CD2 | LEU | 862 | 15.417 | 37.773 | 12.426 |
| 1082 | C | LEU | 862 | 12.952 | 39.682 | 10.028 |
| 1083 | O | LEU | 862 | 12.119 | 38.979 | 10.620 |
| 1084 | N | GLN | 863 | 12.680 | 40.448 | 8.965 |
| 1085 | CA | GLN | 863 | 11.340 | 40.653 | 8.392 |
| 1086 | CB | GLN | 863 | 10.916 | 39.504 | 7.457 |
| 1087 | CG | GLN | 863 | 11.428 | 39.629 | 5.993 |
| 1088 | CD | GLN | 863 | 10.976 | 40.915 | 5.273 |
| 1089 | OE1 | GLN | 863 | 11.805 | 41.700 | 4.789 |
| 1090 | NE2 | GLN | 863 | 9.586 | 41.107 | 5.109 |
| 1091 | C | GLN | 863 | 10.351 | 40.827 | 9.514 |
| 1092 | O | GLN | 863 | 9.407 | 40.053 | 9.670 |
| 1093 | N | LEU | 864 | 10.596 | 41.870 | 10.296 |
| 1094 | CA | LEU | 864 | 9.779 | 42.199 | 11.447 |
| 1095 | CB | LEU | 864 | 10.528 | 43.160 | 12.410 |
| 1096 | C | LEU | 864 | 8.415 | 42.778 | 11.107 |
| 1097 | O | LEU | 864 | 8.271 | 43.751 | 10.368 |
| 1098 | CG | LEU | 864 | 11.894 | 42.701 | 12.985 |
| 1099 | CD1 | LEU | 864 | 12.416 | 43.734 | 13.994 |
| 1100 | CD2 | LEU | 864 | 11.824 | 41.317 | 13.651 |
| 1101 | N | THR | 865 | 7.429 | 42.112 | 11.694 |
| 1102 | CA | THR | 865 | 5.996 | 42.362 | 11.621 |
| 1103 | CB | THR | 865 | 5.338 | 41.183 | 12.351 |
| 1104 | C | THR | 865 | 5.650 | 43.704 | 12.299 |
| 1105 | O | THR | 865 | 6.383 | 44.157 | 13.177 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1106 | OG1 | THR | 865 | 5.645 | 39.958 | 11.697 |
| 1107 | CG2 | THR | 865 | 3.796 | 41.202 | 12.431 |
| 1108 | N | PHE | 866 | 4.549 | 44.344 | 11.902 |
| 1109 | CA | PHE | 866 | 4.179 | 45.602 | 12.542 |
| 1110 | CB | PHE | 866 | 2.971 | 46.256 | 11.885 |
| 1111 1 | CG | PHE | 866 | 2.764 | 47.665 | 12.387 |
| 1112 | CD1 | PHE | 866 | 3.849 | 48.527 | 12.499 |
| 1113 | CE1 | PHE | 866 | 3.698 | 49.840 | 12.880 |
| 1114 | CD2 | PHE | 866 | 1.491 | 48.163 | 12.686 |
| 1115 | CE2 | PHE | 866 | 1.325 | 49.499 | 13.076 |
| 1116 | CZ | PHE | 866 | 2.443 | 50.328 | 13.161 |
| 1117 | C | PHE | 866 | 3.810 | 45.338 | 13.983 |
| 1118 | O | PHE | 866 | 4.164 | 46.095 | 14.882 |
| 1119 | N | GLU | 867 | 3.068 | 44.261 | 14.198 |
| 1120 | CA | GLU | 867 | 2.654 | 43.934 | 15.538 |
| 1121 | CB | GLU | 867 | 1.665 | 42.778 | 15.514 |
| 1122 | CG | GLU | 867 | 0.327 | 43.271 | 15.008 |
| 1123 | CD | GLU | 867 | -0.811 | 42.319 | 15.281 |
| 1124 | OE1 | GLU | 867 | -0.557 | 41.199 | 15.789 |
| 1125 | OE2 | GLU | 867 | -1.968 | 42.697 | 14.982 |
| 1126 | C | GLU | 867 | 3.868 | 43.646 | 16.384 |
| 1127 | O | GLU | 867 | 4.059 | 44.288 | 17.417 |
| 1128 | N | GLU | 868 | 4.698 | 42.705 | 15.947 |
| 1129 | CA | GLU | 868 | 5.907 | 42.402 | 16.692 |
| 1130 | CB | GLU | 868 | 6.830 | 41.522 | 15.870 |
| 1131 | CG | GLU | 868 | 6.445 | 40.076 | 15.819 |
| 1132 | CD | GLU | 868 | 7.082 | 39.394 | 14.633 |
| 1133 | OE1 | GLU | 868 | 7.563 | 40.120 | 13.738 |
| 1134 | OE2 | GLU | 868 | 7.098 | 38.145 | 14.578 |
| 1135 | C | GLU | 868 | 6.639 | 43.703 | 17.021 |
| 1136 | O | GLU | 868 | 7.153 | 43.870 | 18.127 |
| 1137 | N | TYR | 869 | 6.682 | 44.620 | 16.057 |
| 1138 | CA | TYR | 869 | 7.362 | 45.912 | 16.220 |
| 1139 | CB | TYR | 869 | 7.334 | 46.717 | 14.917 |
| 1140 | CG | TYR | 869 | 7.566 | 48.214 | 15.110 |
| 1141 | CD1 | TYR | 869 | 8.841 | 48.707 | 15.377 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1142 | CE1 | TYR | 869 | 9.078 | 50.090 | 15.512 |
| 1143 | CD2 | TYR | 869 | 6.518 | 49.135 | 14.988 |
| 1144 | CE2 | TYR | 869 | 6.741 | 50.522 | 15.121 |
| 1145 | CZ | TYR | 869 | 8.031 | 50.995 | 15.377 |
| 1146 | OH | TYR | 869 | 8.278 | 52.347 | 15.435 |
| 1147 | C | TYR | 869 | 6.782 | 46.823 | 17.285 |
| 1148 | O | TYR | 869 | 7.505 | 47.585 | 17.942 |
| 1149 | N | THR | 870 | 5.459 | 46.790 | 17.394 |
| 1150 | CA | THR | 870 | 4.757 | 47.628 | 18.332 |
| 1151 | CB | THR | 870 | 3.267 | 47.598 | 18.011 |
| 1152 | C | THR | 870 | 5.034 | 47.166 | 19.744 |
| 1153 | O | THR | 870 | 5.166 | 47.973 | 20.677 |
| 1154 | OG1 | THR | 870 | 3.028 | 48.107 | 16.705 |
| 1155 | CG2 | THR | 870 | 2.352 | 48.438 | 18.927 |
| 1156 | N | ILE | 871 | 5.140 | 45.850 | 19.877 |
| 1157 | CA | ILE | 871 | 5.423 | 45.234 | 21.144 |
| 1158 | CB | ILE | 871 | 5.154 | 43.742 | 21.054 |
| 1159 | C | ILE | 871 | 6.861 | 45.490 | 21.549 |
| 1160 | O | ILE | 871 | 7.126 | 45.921 | 22.670 |
| 1161 | CG2 | ILE | 871 | 6.045 | 43.029 | 19.990 |
| 1162 | CG1 | ILE | 871 | 5.301 | 42.970 | 22.405 |
| 1163 | CD1 | ILE | 871 | 4.189 | 43.217 | 23.446 |
| 1164 | N | MET | 872 | 7.799 | 45.254 | 20.640 |
| 1165 | CA | MET | 872 | 9.204 | 45.461 | 20.964 |
| 1166 | CB | MET | 872 | 10.079 | 45.164 | 19.745 |
| 1167 | CG | MET | 872 | 10.038 | 43.703 | 19.358 |
| 1168 | SD | MET | 872 | 10.628 | 43.346 | 17.700 |
| 1169 | CE | MET | 872 | 12.453 | 43.439 | 17.963 |
| 1170 | C | MET | 872 | 9.497 | 46.859 | 21.456 |
| 1171 | O | MET | 872 | 10.363 | 47.055 | 22.306 |
| 1172 | N | LYS | 873 | 8.775 | 47.837 | 20.936 |
| 1173 | CA | LYS | 873 | 9.053 | 49.209 | 21.332 |
| 1174 | CB | LYS | 873 | 8.538 | 50.163 | 20.271 |
| 1175 | CG | LYS | 873 | 8.908 | 51.591 | 20.516 |
| 1176 | CD | LYS | 873 | 8.754 | 52.389 | 19.236 |
| 1177 | CE | LYS | 873 | 7.362 | 52.259 | 18.639 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1178 | NZ | LYS | 873 | 6.239 | 53.012 | 19.336 |
| 1179 | C | LYS | 873 | 8.491 | 49.569 | 22.700 |
| 1180 | O | LYS | 873 | 8.978 | 50.493 | 23.351 |
| 1181 | N | VAL | 874 | 7.463 | 48.847 | 23.128 |
| 1182 | CA | VAL | 874 | 6.866 | 49.089 | 24.430 |
| 1183 | CB | VAL | 874 | 5.492 | 48.376 | 24.544 |
| 1184 | C | VAL | 874 | 7.893 | 48.487 | 25.380 |
| 1185 | O | VAL | 874 | 8.224 | 49.083 | 26.404 |
| 1186 | CG1 | VAL | 874 | 4.419 | 48.922 | 23.572 |
| 1187 | CG2 | VAL | 874 | 4.821 | 48.392 | 25.946 |
| 1188 | N | LEU | 875 | 8.428 | 47.320 | 25.008 |
| 1189 | CA | LEU | 875 | 9.457 | 46.653 | 25.807 |
| 1190 | CB | LEU | 875 | 9.812 | 45.282 | 25.220 |
| 1191 | CG | LEU | 875 | 8.794 | 44.155 | 25.429 |
| 1192 | CD1 | LEU | 875 | 9.347 | 42.853 | 24.943 |
| 1193 | CD2 | LEU | 875 | 8.470 | 44.024 | 26.891 |
| 1194 | C | LEU | 875 | 10.730 | 47.502 | 25.899 |
| 1195 | O | LEU | 875 | 11.542 | 47.320 | 26.800 |
| 1196 | N | LEU | 876 | 10.913 | 48.436 | 24.982 |
| 1197 | CA | LEU | 876 | 12.115 | 49.251 | 25.029 |
| 1198 | CB | LEU | 876 | 12.420 | 49.780 | 23.628 |
| 1199 | CG | LEU | 876 | 13.716 | 49.452 | 22.883 |
| 1200 | CD1 | LEU | 876 | 14.049 | 47.987 | 22.801 |
| 1201 | CD2 | LEU | 876 | 13.505 | 49.985 | 21.502 |
| 1202 | C | LEU | 876 | 11.992 | 50.402 | 26.028 |
| 1203 | O | LEU | 876 | 12.992 | 50.971 | 26.459 |
| 1204 | N | LEU | 877 | 10.764 | 50.753 | 26.385 |
| 1205 | CA | LEU | 877 | 10.515 | 51.824 | 27.336 |
| 1206 | CB | LEU | 877 | 9.045 | 52.267 | 27.204 |
| 1207 | CG | LEU | 877 | 8.268 | 53.010 | 28.298 |
| 1208 | CD1 | LEU | 877 | 8.854 | 54.379 | 28.492 |
| 1209 | CD2 | LEU | 877 | 6.797 | 53.135 | 27.915 |
| 1210 | C | LEU | 877 | 10.809 | 51.215 | 28.712 |
| 1211 | O | LEU | 877 | 11.403 | 51.849 | 29.592 |
| 1212 | N | LEU | 878 | 10.432 | 49.948 | 28.853 |
| 1213 | CA | LEU | 878 | 10.611 | 49.201 | 30.091 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1214 | CB | LEU | 878 | 9.394 | 48.292 | 30.296 |
| 1215 | CG | LEU | 878 | 8.073 | 48.871 | 29.772 |
| 1216 | CD1 | LEU | 878 | 6.945 | 47.851 | 29.885 |
| 1217 | CD2 | LEU | 878 | 7.750 | 50.136 | 30.547 |
| 1218 | C | LEU | 878 | 11.887 | 48.349 | 30.075 |
| 1219 | O | LEU | 878 | 11.906 | 47.272 | 30.680 |
| 1220 | N | SER | 879 | 12.949 | 48.825 | 29.416 |
| 1221 | CA | SER | 879 | 14.183 | 48.040 | 29.311 |
| 1222 | CB | SER | 879 | 14.633 | 47.944 | 27.855 |
| 1223 | OG | SER | 879 | 15.010 | 49.185 | 27.276 |
| 1224 | C | SER | 879 | 15.377 | 48.434 | 30.177 |
| 1225 | O | SER | 879 | 16.435 | 47.801 | 30.115 |
| 1226 | N | THR | 880 | 15.241 | 49.506 | 30.945 |
| 1227 | CA | THR | 880 | 16.306 | 49.863 | 31.866 |
| 1228 | CB | THR | 880 | 17.441 | 50.658 | 31.193 |
| 1229 | C | THR | 880 | 15.672 | 50.612 | 33.024 |
| 1230 | O | THR | 880 | 14.854 | 51.521 | 32.838 |
| 1231 | OG1 | THR | 880 | 18.052 | 49.883 | 30.170 |
| 1232 | CG2 | THR | 880 | 18.611 | 51.086 | 32.105 |
| 1233 | N | ILE | 881 | 16.011 | 50.141 | 34.219 |
| 1234 | CA | ILE | 881 | 15.529 | 50.691 | 35.474 |
| 1235 | CB | ILE | 881 | 14.715 | 49.639 | 36.255 |
| 1236 | C | ILE | 881 | 16.750 | 51.096 | 36.301 |
| 1237 | O | ILE | 881 | 17.875 | 50.683 | 36.007 |
| 1238 | CG2 | ILE | 881 | 14.098 | 50.207 | 37.571 |
| 1239 | CG1 | ILE | 881 | 13.571 | 48.961 | 35.436 |
| 1240 | CD1 | ILE | 881 | 12.952 | 47.695 | 36.064 |
| 1241 | N | PRO | 882 | 16.541 | 51.911 | 37.353 |
| 1242 | CD | PRO | 882 | 15.213 | 52.253 | 37.874 |
| 1243 | CA | PRO | 882 | 17.583 | 52.405 | 38.259 |
| 1244 | CB | PRO | 882 | 16.793 | 53.230 | 39.274 |
| 1245 | CG | PRO | 882 | 15.491 | 53.531 | 38.574 |
| 1246 | C | PRO | 882 | 18.231 | 51.194 | 38.909 |
| 1247 | O | PRO | 882 | 17.620 | 50.126 | 38.947 |
| 1248 | N | LYS | 883 | 19.445 | 51.340 | 39.428 |
| 1249 | CA | LYS | 883 | 20.117 | 50.199 | 40.051 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1250 | CB | LYS | 883 | 21.558 | 50.551 | 40.430 |
| 1251 | CG | LYS | 883 | 22.417 | 49.338 | 40.803 |
| 1252 | CD | LYS | 883 | 23.224 | 49.590 | 42.079 |
| 1253 | CE | LYS | 883 | 22.299 | 49.706 | 43.280 |
| 1254 | NZ | LYS | 883 | 22.861 | 50.379 | 44.517 |
| 1255 | C | LYS | 883 | 19.355 | 49.753 | 41.292 |
| 1256 | O | LYS | 883 | 19.617 | 48.681 | 41.849 |
| 1257 | N | ASP | 884 | 18.408 | 50.581 | 41.721 |
| 1258 | CA | ASP | 884 | 17.606 | 50.272 | 42.889 |
| 1259 | CB | ASP | 884 | 17.762 | 51.369 | 43.940 |
| 1260 | CG | ASP | 884 | 19.210 | 51.672 | 44.247 |
| 1261 | OD1 | ASP | 884 | 20.041 | 50.750 | 44.122 |
| 1262 | OD2 | ASP | 884 | 19.512 | 52.825 | 44.627 |
| 1263 | C | ASP | 884 | 16.138 | 50.131 | 42.515 |
| 1264 | O | ASP | 884 | 15.251 | 50.464 | 43.303 |
| 1265 | N | GLY | 885 | 15.882 | 49.635 | 41.309 |
| 1266 | CA | GLY | 885 | 14.507 | 49.480 | 40.885 |
| 1267 | C | GLY | 885 | 13.772 | 50.793 | 41.061 |
| 1268 | O | GLY | 885 | 14.325 | 51.788 | 41.531 |
| 1269 | N | LEU | 886 | 12.497 | 50.792 | 40.704 |
| 1270 | CA | LEU | 886 | 11.704 | 52.001 | 40.798 |
| 1271 | CB | LEU | 886 | 10.794 | 52.067 | 39.584 |
| 1272 | CG | LEU | 886 | 11.543 | 51.498 | 38.387 |
| 1273 | CD1 | LEU | 886 | 10.575 | 50.833 | 37.457 |
| 1274 | CD2 | LEU | 886 | 12.319 | 52.589 | 37.698 |
| 1275 | C | LEU | 886 | 10.886 | 52.021 | 42.069 |
| 1276 | O | LEU | 886 | 11.072 | 51.198 | 42.965 |
| 1277 | N | LYS | 887 | 9.972 | 52.975 | 42.154 |
| 1278 | CA | LYS | 887 | 9.131 | 53.063 | 43.320 |
| 1279 | CB | LYS | 887 | 8.698 | 54.508 | 43.562 |
| 1280 | CG | LYS | 887 | 7.958 | 54.747 | 44.880 |
| 1281 | CD | LYS | 887 | 7.695 | 56.233 | 45.037 |
| 1282 | CE | LYS | 887 | 6.960 | 56.606 | 46.316 |
| 1283 | NZ | LYS | 887 | 6.728 | 58.098 | 46.415 |
| 1284 | C | LYS | 887 | 7.910 | 52.179 | 43.139 |
| 1285 | O | LYS | 887 | 7.102 | 52.048 | 44.051 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1286 | N | SER | 888 | 7.763 | 51.569 | 41.965 |
| 1287 | CA | SER | 888 | 6.615 | 50.688 | 41.729 |
| 1288 | CB | SER | 888 | 5.567 | 51.386 | 40.850 |
| 1289 | OG | SER | 888 | 4.960 | 52.527 | 41.454 |
| 1290 | C | SER | 888 | 7.027 | 49.362 | 41.087 |
| 1291 | O | SER | 888 | 6.225 | 48.699 | 40.426 |
| 1292 | N | GLN | 889 | 8.283 | 48.978 | 41.309 |
| 1293 | CA | GLN | 889 | 8.846 | 47.746 | 40.773 |
| 1294 | CB | GLN | 889 | 10.035 | 47.304 | 41.610 |
| 1295 | CG | GLN | 889 | 11.343 | 47.861 | 41.125 |
| 1296 | CD | GLN | 889 | 11.823 | 47.197 | 39.842 |
| 1297 | OE1 | GLN | 889 | 12.707 | 47.715 | 39.166 |
| 1298 | NE2 | GLN | 889 | 11.345 | 45.921 | 39.490 |
| 1299 | C | GLN | 889 | 7.904 | 46.574 | 40.640 |
| 1300 | O | GLN | 889 | 8.015 | 45.800 | 39.698 |
| 1301 | N | ALA | 890 | 6.994 | 46.410 | 41.583 |
| 1302 | CA | ALA | 890 | 6.090 | 45.281 | 41.470 |
| 1303 | CB | ALA | 890 | 5.206 | 45.180 | 42.714 |
| 1304 | C | ALA | 890 | 5.253 | 45.430 | 40.199 |
| 1305 | O | ALA | 890 | 5.190 | 44.502 | 39.394 |
| 1306 | N | ALA | 891 | 4.638 | 46.605 | 40.014 |
| 1307 | CA | ALA | 891 | 3.819 | 46.882 | 38.830 |
| 1308 | CB | ALA | 891 | 3.170 | 48.281 | 38.928 |
| 1309 | C | ALA | 891 | 4.707 | 46.811 | 37.580 |
| 1310 | O | ALA | 891 | 4.391 | 46.110 | 36.619 |
| 1311 | N | PHE | 892 | 5.821 | 47.543 | 37.604 |
| 1312 | CA | PHE | 892 | 6.767 | 47.547 | 36.502 |
| 1313 | CB | PHE | 892 | 8.095 | 48.151 | 36.942 |
| 1314 | CG | PHE | 892 | 9.110 | 48.217 | 35.852 |
| 1315 | CD1 | PHE | 892 | 9.105 | 49.277 | 34.959 |
| 1316 | CD2 | PHE | 892 | 10.025 | 47.184 | 35.668 |
| 1317 | CE1 | PHE | 892 | 10.000 | 49.328 | 33.900 |
| 1318 | CE2 | PHE | 892 | 10.928 | 47.218 | 34.606 |
| 1319 | CZ | PHE | 892 | 10.912 | 48.291 | 33.716 |
| 1320 | C | PHE | 892 | 7.045 | 46.128 | 36.016 |
| 1321 | O | PHE | 892 | 6.798 | 45.783 | 34.860 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1322 | N | GLU | 893 | 7.578 | 45.303 | 36.908 |
| 1323 | CA | GLU | 893 | 7.899 | 43.935 | 36.543 |
| 1324 | CB | GLU | 893 | 8.440 | 43.176 | 37.770 |
| 1325 | C | GLU | 893 | 6.684 | 43.238 | 35.929 |
| 1326 | O | GLU | 893 | 6.831 | 42.384 | 35.055 |
| 1327 | CG | GLU | 893 | 7.451 | 42.934 | 38.958 |
| 1328 | CD | GLU | 893 | 7.989 | 42.272 | 40.229 |
| 1329 | OE1 | GLU | 893 | 7.298 | 42.072 | 41.219 |
| 1330 | OE2 | GLU | 893 | 9.304 | 41.928 | 40.153 |
| 1331 | N | GLU | 894 | 5.491 | 43.637 | 36.359 |
| 1332 | CA | GLU | 894 | 4.232 | 43.066 | 35.869 |
| 1333 | CB | GLU | 894 | 3.100 | 43.444 | 36.828 |
| 1334 | CG | GLU | 894 | 2.259 | 42.274 | 37.299 |
| 1335 | CD | GLU | 894 | 0.876 | 42.263 | 36.679 |
| 1336 | OE1 | GLU | 894 | 0.116 | 43.238 | 36.905 |
| 1337 | OE2 | GLU | 894 | 0.552 | 41.278 | 35.966 |
| 1338 | C | GLU | 894 | 3.879 | 43.542 | 34.455 |
| 1339 | O | GLU | 894 | 3.375 | 42.779 | 33.628 |
| 1340 | N | MET | 895 | 4.121 | 44.821 | 34.197 |
| 1341 | CA | MET | 895 | 3.848 | 45.387 | 32.892 |
| 1342 | CB | MET | 895 | 4.040 | 46.900 | 32.930 |
| 1343 | C | MET | 895 | 4.847 | 44.714 | 31.975 |
| 1344 | O | MET | 895 | 4.471 | 43.967 | 31.082 |
| 1345 | CG | MET | 895 | 3.096 | 47.676 | 33.873 |
| 1346 | SD | MET | 895 | 3.257 | 49.445 | 33.578 |
| 1347 | CE | MET | 895 | 2.384 | 50.038 | 35.034 |
| 1348 | N | ARG | 896 | 6.134 | 44.938 | 32.213 |
| 1349 | CA | ARG | 896 | 7.114 | 44.290 | 31.373 |
| 1350 | CB | ARG | 896 | 8.462 | 44.273 | 32.055 |
| 1351 | CG | ARG | 896 | 9.570 | 43.824 | 31.153 |
| 1352 | CD | ARG | 896 | 10.755 | 44.671 | 31.489 |
| 1353 | NE | ARG | 896 | 11.915 | 44.395 | 30.662 |
| 1354 | CZ | ARG | 896 | 12.544 | 43.231 | 30.646 |
| 1355 | NH1 | ARG | 896 | 12.208 | 42.170 | 31.438 |
| 1356 | NH2 | ARG | 896 | 13.594 | 43.080 | 29.790 |
| 1357 | C | ARG | 896 | 6.724 | 42.853 | 31.006 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1358 | O | ARG | 896 | 6.778 | 42.484 | 29.843 |
| 1359 | N | THR | 897 | 6.321 | 42.040 | 31.982 |
| 1360 | CA | THR | 897 | 5.972 | 40.649 | 31.675 |
| 1361 | CB | THR | 897 | 5.836 | 39.832 | 32.966 |
| 1362 | C | THR | 897 | 4.713 | 40.500 | 30.812 |
| 1363 | O | THR | 897 | 4.537 | 39.495 | 30.117 |
| 1364 | OG1 | THR | 897 | 7.069 | 39.810 | 33.673 |
| 1365 | CG2 | THR | 897 | 5.460 | 38.344 | 32.797 |
| 1366 | N | ASN | 898 | 3.840 | 41.500 | 30.855 |
| 1367 | CA | ASN | 898 | 2.633 | 41.463 | 30.048 |
| 1368 | CB | ASN | 898 | 1.627 | 42.534 | 30.488 |
| 1369 | C | ASN | 898 | 3.027 | 41.690 | 28.597 |
| 1370 | O | ASN | 898 | 2.551 | 40.981 | 27.705 |
| 1371 | CG | ASN | 898 | 0.278 | 42.560 | 29.761 |
| 1372 | OD1 | ASN | 898 | -0.007 | 41.755 | 28.887 |
| 1373 | ND2 | ASN | 898 | -0.587 | 43.488 | 30.072 |
| 1374 | N | TYR | 899 | 3.901 | 42.668 | 28.358 |
| 1375 | CA | TYR | 899 | 4.357 | 42.916 | 26.993 |
| 1376 | CB | TYR | 899 | 4.958 | 44.319 | 26.837 |
| 1377 | CG | TYR | 899 | 3.898 | 45.370 | 26.990 |
| 1378 | CD1 | TYR | 899 | 2.761 | 45.351 | 26.171 |
| 1379 | CE1 | TYR | 899 | 1.691 | 46.186 | 26.418 |
| 1380 | CD2 | TYR | 899 | 3.939 | 46.276 | 28.047 |
| 1381 | CE2 | TYR | 899 | 2.873 | 47.116 | 28.304 |
| 1382 | CZ | TYR | 899 | 1.747 | 47.061 | 27.493 |
| 1383 | OH | TYR | 899 | 0.646 | 47.833 | 27.786 |
| 1384 | C | TYR | 899 | 5.342 | 41.871 | 26.485 |
| 1385 | O | TYR | 899 | 5.514 | 41.745 | 25.278 |
| 1386 | N | ILE | 900 | 6.002 | 41.120 | 27.362 |
| 1387 | CA | ILE | 900 | 6.888 | 40.094 | 26.830 |
| 1388 | CB | ILE | 900 | 7.818 | 39.464 | 27.891 |
| 1389 | CG2 | ILE | 900 | 8.529 | 38.247 | 27.308 |
| 1390 | CG1 | ILE | 900 | 8.885 | 40.475 | 28.305 |
| 1391 | CD1 | ILE | 900 | 9.789 | 39.977 | 29.395 |
| 1392 | C | ILE | 900 | 5.966 | 39.022 | 26.273 |
| 1393 | O | ILE | 900 | 6.308 | 38.318 | 25.332 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1394 | N | LYS | 901 | 4.774 | 38.920 | 26.847 |
| 1395 | CA | LYS | 901 | 3.820 | 37.936 | 26.375 |
| 1396 | CB | LYS | 901 | 2.807 | 37.615 | 27.481 |
| 1397 | CG | LYS | 901 | 3.401 | 36.899 | 28.709 |
| 1398 | CD | LYS | 901 | 2.301 | 36.662 | 29.743 |
| 1399 | CE | LYS | 901 | 2.768 | 35.909 | 30.996 |
| 1400 | NZ | LYS | 901 | 1.690 | 35.710 | 32.047 |
| 1401 | C | LYS | 901 | 3.116 | 38.438 | 25.106 |
| 1402 | O | LYS | 901 | 2.877 | 37.670 | 24.177 |
| 1403 | N | GLU | 902 | 2.798 | 39.726 | 25.065 |
| 1404 | CA | GLU | 902 | 2.149 | 40.312 | 23.902 |
| 1405 | CB | GLU | 902 | 2.008 | 41.822 | 24.130 |
| 1406 | CG | GLU | 902 | 0.593 | 42.462 | 24.051 |
| 1407 | CD | GLU | 902 | -0.577 | 41.510 | 23.728 |
| 1408 | OE1 | GLU | 902 | -0.991 | 40.733 | 24.621 |
| 1409 | OE2 | GLU | 902 | -1.096 | 41.558 | 22.584 |
| 1410 | C | GLU | 902 | 3.039 | 40.011 | 22.669 |
| 1411 | O | GLU | 902 | 2.537 | 39.667 | 21.596 |
| 1412 | N | LEU | 903 | 4.362 | 40.131 | 22.845 |
| 1413 | CA | LEU | 903 | 5.363 | 39.873 | 21.792 |
| 1414 | CB | LEU | 903 | 6.793 | 40.172 | 22.297 |
| 1415 | CG | LEU | 903 | 7.966 | 39.883 | 21.341 |
| 1416 | CD1 | LEU | 903 | 7.870 | 40.820 | 20.160 |
| 1417 | CD2 | LEU | 903 | 9.317 | 40.065 | 22.031 |
| 1418 | C | LEU | 903 | 5.302 | 38.420 | 21.334 |
| 1419 | O | LEU | 903 | 5.470 | 38.121 | 20.154 |
| 1420 | N | ARG | 904 | 5.081 | 37.516 | 22.276 |
| 1421 | CA | ARG | 904 | 4.987 | 36.115 | 21.925 |
| 1422 | C | ARG | 904 | 3.726 | 35.913 | 21.116 |
| 1423 | O | ARG | 904 | 3.677 | 35.090 | 20.209 |
| 1424 | CB | ARG | 904 | 4.966 | 35.268 | 23.229 |
| 1425 | CG | ARG | 904 | 5.012 | 33.727 | 23.027 |
| 1426 | CD | ARG | 904 | 4.952 | 32.966 | 24.363 |
| 1427 | NE | ARG | 904 | 5.000 | 31.497 | 24.107 |
| 1428 | CZ | ARG | 904 | 4.968 | 30.552 | 25.043 |
| 1429 | NH1 | ARG | 904 | 5.024 | 29.314 | 24.667 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1430 | NH2 | ARG | 904 | 4.882 | 30.803 | 26.319 |
| 1431 | N | LYS | 905 | 2.694 | 36.677 | 21.447 |
| 1432 | CA | LYS | 905 | 1.443 | 36.573 | 20.714 |
| 1433 | CB | LYS | 905 | 0.381 | 37.420 | 21.376 |
| 1434 | CG | LYS | 905 | -0.197 | 36.796 | 22.603 |
| 1435 | CD | LYS | 905 | -1.091 | 37.799 | 23.250 |
| 1436 | CE | LYS | 905 | -2.163 | 37.147 | 24.059 |
| 1437 | NZ | LYS | 905 | -3.222 | 38.155 | 24.471 |
| 1438 | C | LYS | 905 | 1.650 | 37.048 | 19.291 |
| 1439 | O | LYS | 905 | 1.153 | 36.432 | 18.342 |
| 1440 | N | MET | 906 | 2.383 | 38.151 | 19.153 |
| 1441 | CA | MET | 906 | 2.678 | 38.711 | 17.851 |
| 1442 | CB | MET | 906 | 3.582 | 39.912 | 18.000 |
| 1443 | C | MET | 906 | 3.358 | 37.635 | 17.025 |
| 1444 | O | MET | 906 | 2.876 | 37.279 | 15.958 |
| 1445 | CG | MET | 906 | 2.996 | 41.101 | 18.791 |
| 1446 | SD | MET | 906 | 4.072 | 42.535 | 18.622 |
| 1447 | CE | MET | 906 | 3.361 | 43.556 | 19.919 |
| 1448 | N | VAL | 907 | 4.476 | 37.114 | 17.539 |
| 1449 | CA | VAL | 907 | 5.262 | 36.053 | 16.882 |
| 1450 | CB | VAL | 907 | 6.249 | 35.411 | 17.901 |
| 1451 | C | VAL | 907 | 4.369 | 34.951 | 16.314 |
| 1452 | O | VAL | 907 | 4.371 | 34.664 | 15.112 |
| 1453 | CG1 | VAL | 907 | 7.338 | 36.382 | 18.418 |
| 1454 | CG2 | VAL | 907 | 7.019 | 34.152 | 17.415 |
| 1455 | N | THR | 908 | 3.616 | 34.336 | 17.209 |
| 1456 | CA | THR | 908 | 2.694 | 33.268 | 16.880 |
| 1457 | CB | THR | 908 | 1.867 | 32.900 | 18.119 |
| 1458 | C | THR | 908 | 1.735 | 33.600 | 15.736 |
| 1459 | O | THR | 908 | 1.647 | 32.855 | 14.760 |
| 1460 | OG1 | THR | 908 | 2.713 | 32.433 | 19.162 |
| 1461 | CG2 | THR | 908 | 0.822 | 31.778 | 17.937 |
| 1462 | N | LYS | 909 | 1.004 | 34.701 | 15.878 |
| 1463 | CA | LYS | 909 | 0.033 | 35.157 | 14.877 |
| 1464 | CB | LYS | 909 | -0.430 | 36.583 | 15.254 |
| 1465 | CG | LYS | 909 | -1.811 | 37.037 | 14.735 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1466 | CD | LYS | 909 | -2.258 | 38.400 | 15.341 |
| 1467 | CE | LYS | 909 | -3.726 | 38.753 | 14.996 |
| 1468 | NZ | LYS | 909 | -4.024 | 40.246 | 15.095 |
| 1469 | C | LYS | 909 | 0.691 | 35.136 | 13.480 |
| 1470 | O | LYS | 909 | 0.116 | 34.647 | 12.495 |
| 1471 | N | CYS | 910 | 1.915 | 35.660 | 13.446 |
| 1472 | CA | CYS | 910 | 2.776 | 35.795 | 12.272 |
| 1473 | CB | CYS | 910 | 4.202 | 36.113 | 12.754 |
| 1474 | C | CYS | 910 | 2.826 | 34.582 | 11.379 |
| 1475 | O | CYS | 910 | 2.789 | 34.651 | 10.145 |
| 1476 | SG | CYS | 910 | 5.286 | 36.472 | 11.353 |
| 1477 | N | PRO | 911 | 2.903 | 33.447 | 12.024 |
| 1478 | CA | PRO | 911 | 3.037 | 32.243 | 11.278 |
| 1479 | CB | PRO | 911 | 4.386 | 31.672 | 11.628 |
| 1480 | C | PRO | 911 | 1.964 | 31.280 | 11.628 |
| 1481 | O | PRO | 911 | 1.200 | 31.466 | 12.578 |
| 1482 | CG | PRO | 911 | 4.509 | 32.104 | 13.099 |
| 1483 | CD | PRO | 911 | 3.955 | 33.532 | 13.103 |
| 1484 | N | ASN | 912 | 1.895 | 30.246 | 10.817 |
| 1485 | CA | ASN | 912 | 0.954 | 29.196 | 11.077 |
| 1486 | C | ASN | 912 | 1.861 | 28.138 | 11.671 |
| 1487 | O | ASN | 912 | 1.440 | 27.332 | 12.495 |
| 1488 | CB | ASN | 912 | 0.341 | 28.704 | 9.733 |
| 1489 | CG | ASN | 912 | -0.357 | 29.755 | 8.863 |
| 1490 | OD1 | ASN | 912 | 0.187 | 30.262 | 7.893 |
| 1491 | ND2 | ASN | 912 | -1.563 | 30.137 | 9.183 |
| 1492 | N | ASN | 913 | 3.134 | 28.197 | 11.271 |
| 1493 | CA | ASN | 913 | 4.193 | 27.258 | 11.668 |
| 1494 | CB | ASN | 913 | 5.251 | 27.272 | 10.578 |
| 1495 | CG | ASN | 913 | 5.578 | 28.679 | 10.136 |
| 1496 | OD1 | ASN | 913 | 5.923 | 29.526 | 10.956 |
| 1497 | ND2 | ASN | 913 | 5.420 | 28.980 | 8.768 |
| 1498 | C | ASN | 913 | 4.881 | 27.454 | 13.028 |
| 1499 | O | ASN | 913 | 5.908 | 28.132 | 13.127 |
| 1500 | N | SER | 914 | 4.332 | 26.802 | 14.050 |
| 1501 | CA | SER | 914 | 4.830 | 26.870 | 15.426 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1502 | CB | SER | 914 | 3.756 | 26.337 | 16.382 |
| 1503 | OG | SER | 914 | 2.498 | 26.998 | 16.259 |
| 1504 | C | SER | 914 | 6.140 | 26.111 | 15.661 |
| 1505 | O | SER | 914 | 6.533 | 25.861 | 16.805 |
| 1506 | N | GLY | 915 | 6.809 | 25.755 | 14.570 |
| 1507 | CA | GLY | 915 | 8.074 | 25.033 | 14.645 |
| 1508 | C | GLY | 915 | 9.212 | 25.966 | 15.088 |
| 1509 | O | GLY | 915 | 10.036 | 25.590 | 15.933 |
| 1510 | N | GLN | 916 | 9.250 | 27.176 | 14.526 |
| 1511 | CA | GLN | 916 | 10.278 | 28.152 | 14.888 |
| 1512 | CB | GLN | 916 | 11.054 | 28.617 | 13.646 |
| 1513 | CG | GLN | 916 | 12.152 | 27.631 | 13.218 |
| 1514 | CD | GLN | 916 | 13.209 | 28.248 | 12.312 |
| 1515 | OE1 | GLN | 916 | 14.225 | 27.615 | 12.008 |
| 1516 | NE2 | GLN | 916 | 12.971 | 29.540 | 11.787 |
| 1517 | C | GLN | 916 | 9.721 | 29.356 | 15.658 |
| 1518 | O | GLN | 916 | 10.144 | 30.502 | 15.463 |
| 1519 | N | SER | 917 | 8.766 | 29.074 | 16.541 |
| 1520 | CA | SER | 917 | 8.176 | 30.109 | 17.363 |
| 1521 | CB | SER | 917 | 6.870 | 29.626 | 17.987 |
| 1522 | C | SER | 917 | 9.183 | 30.515 | 18.428 |
| 1523 | O | SER | 917 | 9.504 | 31.695 | 18.551 |
| 1524 | OG | SER | 917 | 7.069 | 28.618 | 18.985 |
| 1525 | N | TRP | 918 | 9.706 | 29.564 | 19.191 |
| 1526 | CA | TRP | 918 | 10.703 | 29.976 | 20.171 |
| 1527 | CB | TRP | 918 | 11.036 | 28.870 | 21.173 |
| 1528 | CG | TRP | 918 | 9.927 | 28.533 | 22.068 |
| 1529 | CD2 | TRP | 918 | 9.500 | 29.264 | 23.223 |
| 1530 | CE2 | TRP | 918 | 8.375 | 28.589 | 23.743 |
| 1531 | CE3 | TRP | 918 | 9.997 | 30.377 | 23.915 |
| 1532 | CD1 | TRP | 918 | 9.049 | 27.515 | 21.909 |
| 1533 | NE1 | TRP | 918 | 8.105 | 27.543 | 22.904 |
| 1534 | CZ2 | TRP | 918 | 7.679 | 29.048 | 24.864 |
| 1535 | CZ3 | TRP | 918 | 9.310 | 30.830 | 25.041 |
| 1536 | CH2 | TRP | 918 | 8.179 | 30.144 | 25.521 |
| 1537 | C | TRP | 918 | 11.992 | 30.412 | 19.490 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1538 | O | TRP | 918 | 12.819 | 31.066 | 20.124 |
| 1539 | N | GLN | 919 | 12.196 | 30.066 | 18.225 |
| 1540 | CA | GLN | 919 | 13.434 | 30.506 | 17.602 |
| 1541 | CB | GLN | 919 | 13.751 | 29.719 | 16.327 |
| 1542 | CG | GLN | 919 | 15.178 | 29.976 | 15.774 |
| 1543 | CD | GLN | 919 | 16.294 | 29.818 | 16.827 |
| 1544 | OE1 | GLN | 919 | 16.925 | 30.802 | 17.240 |
| 1545 | NE2 | GLN | 919 | 16.541 | 28.501 | 17.267 |
| 1546 | C | GLN | 919 | 13.363 | 31.996 | 17.296 |
| 1547 | O | GLN | 919 | 14.341 | 32.711 | 17.482 |
| 1548 | N | ARG | 920 | 12.196 | 32.462 | 16.855 |
| 1549 | CA | ARG | 920 | 12.005 | 33.878 | 16.529 |
| 1550 | CB | ARG | 920 | 10.734 | 34.075 | 15.714 |
| 1551 | CG | ARG | 920 | 10.632 | 35.476 | 15.165 |
| 1552 | CD | ARG | 920 | 9.499 | 35.588 | 14.195 |
| 1553 | NE | ARG | 920 | 9.351 | 36.947 | 13.680 |
| 1554 | CZ | ARG | 920 | 10.163 | 37.510 | 12.794 |
| 1555 | NH1 | ARG | 920 | 11.233 | 36.851 | 12.254 |
| 1556 | NH2 | ARG | 920 | 9.894 | 38.785 | 12.424 |
| 1557 | C | ARG | 920 | 11.937 | 34.762 | 17.765 |
| 1558 | O | ARG | 920 | 12.311 | 35.938 | 17.723 |
| 1559 | N | PHE | 921 | 11.434 | 34.203 | 18.855 |
| 1560 | CA | PHE | 921 | 11.348 | 34.951 | 20.092 |
| 1561 | CB | PHE | 921 | 10.608 | 34.150 | 21.146 |
| 1562 | CG | PHE | 921 | 10.407 | 34.891 | 22.422 |
| 1563 | CD1 | PHE | 921 | 9.321 | 35.745 | 22.576 |
| 1564 | CD2 | PHE | 921 | 11.319 | 34.774 | 23.458 |
| 1565 | CE1 | PHE | 921 | 9.140 | 36.456 | 23.747 |
| 1566 | CE2 | PHE | 921 | 11.152 | 35.483 | 24.639 |
| 1567 | CZ | PHE | 921 | 10.062 | 36.330 | 24.784 |
| 1568 | C | PHE | 921 | 12.770 | 35.210 | 20.568 |
| 1569 | O | PHE | 921 | 13.050 | 36.218 | 21.215 |
| 1570 | N | TYR | 922 | 13.662 | 34.277 | 20.237 |
| 1571 | CA | TYR | 922 | 15.065 | 34.379 | 20.609 |
| 1572 | CB | TYR | 922 | 15.769 | 33.040 | 20.370 |
| 1573 | CG | TYR | 922 | 17.233 | 33.089 | 20.718 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1574 | CD1 | TYR | 922 | 17.649 | 33.208 | 22.045 |
| 1575 | CE1 | TYR | 922 | 18.990 | 33.371 | 22.367 |
| 1576 | CD2 | TYR | 922 | 18.200 | 33.117 | 19.719 |
| 1577 | CE2 | TYR | 922 | 19.542 | 33.282 | 20.028 |
| 1578 | CZ | TYR | 922 | 19.930 | 33.410 | 21.353 |
| 1579 | OH | TYR | 922 | 21.264 | 33.555 | 21.664 |
| 1580 | C | TYR | 922 | 15.769 | 35.471 | 19.806 |
| 1581 | O | TYR | 922 | 16.503 | 36.284 | 20.368 |
| 1582 | N | GLN | 923 | 15.547 | 35.472 | 18.494 |
| 1583 | CA | GLN | 923 | 16.146 | 36.468 | 17.608 |
| 1584 | CB | GLN | 923 | 15.770 | 36.199 | 16.141 |
| 1585 | CG | GLN | 923 | 16.408 | 34.952 | 15.508 |
| 1586 | CD | GLN | 923 | 15.943 | 34.684 | 14.065 |
| 1587 | OE1 | GLN | 923 | 14.740 | 34.616 | 13.775 |
| 1588 | NE2 | GLN | 923 | 16.959 | 34.451 | 13.110 |
| 1589 | C | GLN | 923 | 15.639 | 37.849 | 18.011 |
| 1590 | O | GLN | 923 | 16.413 | 38.766 | 18.286 |
| 1591 | N | LEU | 924 | 14.322 | 37.996 | 18.060 |
| 1592 | CA | LEU | 924 | 13.753 | 39.275 | 18.415 |
| 1593 | CB | LEU | 924 | 12.239 | 39.179 | 18.452 |
| 1594 | CG | LEU | 924 | 11.639 | 38.894 | 17.090 |
| 1595 | CD1 | LEU | 924 | 10.143 | 38.920 | 17.222 |
| 1596 | CD2 | LEU | 924 | 12.105 | 39.929 | 16.088 |
| 1597 | C | LEU | 924 | 14.234 | 39.874 | 19.721 |
| 1598 | O | LEU | 924 | 14.514 | 41.064 | 19.777 |
| 1599 | N | THR | 925 | 14.326 | 39.063 | 20.771 |
| 1600 | CA | THR | 925 | 14.746 | 39.581 | 22.071 |
| 1601 | CB | THR | 925 | 14.343 | 38.610 | 23.184 |
| 1602 | OG1 | THR | 925 | 14.997 | 37.365 | 23.094 |
| 1603 | CG2 | THR | 925 | 12.846 | 38.297 | 23.091 |
| 1604 | C | THR | 925 | 16.252 | 39.851 | 22.121 |
| 1605 | O | THR | 925 | 16.738 | 40.653 | 22.938 |
| 1606 | N | LYS | 926 | 16.966 | 39.170 | 21.224 |
| 1607 | CA | LYS | 926 | 18.408 | 39.302 | 21.059 |
| 1608 | CB | LYS | 926 | 18.884 | 38.210 | 20.087 |
| 1609 | CG | LYS | 926 | 20.327 | 37.729 | 20.247 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1610 | CD | LYS | 926 | 20.583 | 37.132 | 21.640 |
| 1611 | CE | LYS | 926 | 21.660 | 37.911 | 22.436 |
| 1612 | NZ | LYS | 926 | 21.351 | 39.365 | 22.780 |
| 1613 | C | LYS | 926 | 18.601 | 40.715 | 20.459 |
| 1614 | O | LYS | 926 | 19.507 | 41.464 | 20.846 |
| 1615 | N | LEU | 927 | 17.717 | 41.077 | 19.525 |
| 1616 | CA | LEU | 927 | 17.748 | 42.395 | 18.889 |
| 1617 | CB | LEU | 927 | 16.650 | 42.521 | 17.825 |
| 1618 | CG | LEU | 927 | 16.952 | 43.492 | 16.671 |
| 1619 | CD1 | LEU | 927 | 15.672 | 43.904 | 15.970 |
| 1620 | CD2 | LEU | 927 | 17.674 | 44.711 | 17.194 |
| 1621 | C | LEU | 927 | 17.501 | 43.451 | 19.959 |
| 1622 | O | LEU | 927 | 18.214 | 44.445 | 20.041 |
| 1623 | N | LEU | 928 | 16.460 | 43.234 | 20.759 |
| 1624 | CA | LEU | 928 | 16.108 | 44.154 | 21.828 |
| 1625 | CB | LEU | 928 | 15.010 | 43.560 | 22.699 |
| 1626 | CG | LEU | 928 | 13.624 | 43.652 | 22.083 |
| 1627 | CD1 | LEU | 928 | 12.611 1 | 43.023 | 22.996 |
| 1628 | CD2 | LEU | 928 | 13.294 | 45.105 | 21.840 |
| 1629 | C | LEU | 928 | 17.312 | 44.432 | 22.686 |
| 1630 | O | LEU | 928 | 17.554 | 45.576 | 23.080 |
| 1631 | N | ASP | 929 | 18.061 | 43.370 | 22.975 |
| 1632 | CA | ASP | 929 | 19.268 | 43.466 | 23.789 |
| 1633 | CB | ASP | 929 | 19.844 | 42.076 | 24.061 |
| 1634 | CG | ASP | 929 | 19.233 | 41.406 | 25.276 |
| 1635 | OD1 | ASP | 929 | 18.373 | 42.017 | 25.955 |
| 1636 | OD2 | ASP | 929 | 19.631 | 40.251 | 25.555 |
| 1637 | C | ASP | 929 | 20.338 | 44.318 | 23.110 |
| 1638 | O | ASP | 929 | 20.934 | 45.189 | 23.739 |
| 1639 | N | SER | 930 | 20.588 | 44.068 | 21.833 |
| 1640 | CA | SER | 930 | 21.606 | 44.841 | 21.147 |
| 1641 | CB | SER | 930 | 21.860 | 44.274 | 19.748 |
| 1642 | OG | SER | 930 | 20.780 | 44.324 | 18.832 |
| 1643 | C | SER | 930 | 21.253 | 46.324 | 21.059 |
| 1644 | O | SER | 930 | 22.043 | 47.121 | 20.568 |
| 1645 | N | MET | 931 | 20.067 | 46.710 | 21.527 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1646 | CA | MET | 931 | 19.704 | 48.121 | 21.477 |
| 1647 | CB | MET | 931 | 18.200 | 48.329 | 21.657 |
| 1648 | CG | MET | 931 | 17.349 | 47.940 | 20.468 |
| 1649 | SD | MET | 931 | 17.684 | 48.878 | 18.960 |
| 1650 | CE | MET | 931 | 17.557 | 50.532 | 19.494 |
| 1651 | C | MET | 931 | 20.439 | 48.873 | 22.571 |
| 1652 | O | MET | 931 | 20.633 | 50.084 | 22.468 |
| 1653 | N | HIS | 932 | 20.836 | 48.177 | 23.632 |
| 1654 | CA | HIS | 932 | 21.571 | 48.865 | 24.678 |
| 1655 | CB | HIS | 932 | 21.769 | 47.988 | 25.927 |
| 1656 | CG | HIS | 932 | 20.521 | 47.749 | 26.732 |
| 1657 | CD2 | HIS | 932 | 19.798 | 46.617 | 26.920 |
| 1658 | ND1 | HIS | 932 | 19.934 | 48.714 | 27.513 |
| 1659 | CE1 | HIS | 932 | 18.898 | 48.190 | 28.159 |
| 1660 | NE2 | HIS | 932 | 18.798 | 46.923 | 27.815 |
| 1661 | C | HIS | 932 | 22.927 | 49.210 | 24.064 |
| 1662 | O | HIS | 932 | 23.499 | 50.242 | 24.386 |
| 1663 | N | ASP | 933 | 23.438 | 48.363 | 23.168 |
| 1664 | CA | ASP | 933 | 24.730 | 48.640 | 22.525 |
| 1665 | CB | ASP | 933 | 25.262 | 47.385 | 21.776 |
| 1666 | C | ASP | 933 | 24.619 | 49.831 | 21.578 |
| 1667 | O | ASP | 933 | 25.430 | 50.750 | 21.640 |
| 1668 | CG | ASP | 933 | 26.686 | 47.474 | 21.207 |
| 1669 | OD1 | ASP | 933 | 26.999 | 47.013 | 20.119 |
| 1670 | OD2 | ASP | 933 | 27.547 | 48.139 | 22.035 |
| 1671 | N | LEU | 934 | 23.613 | 49.857 | 20.716 |
| 1672 | CA | LEU | 934 | 23.537 | 50.986 | 19.807 |
| 1673 | CB | LEU | 934 | 22.477 | 50.754 | 18.709 |
| 1674 | C | LEU | 934 | 23.149 | 52.278 | 20.522 |
| 1675 | O | LEU | 934 | 23.643 | 53.348 | 20.158 |
| 1676 | CG | LEU | 934 | 22.732 | 49.537 | 17.784 |
| 1677 | CD1 | LEU | 934 | 21.508 | 49.360 | 16.873 |
| 1678 | CD2 | LEU | 934 | 24.040 | 49.676 | 16.975 |
| 1679 | N | VAL | 935 | 22.288 | 52.203 | 21.533 |
| 1680 | CA | VAL | 935 | 21.926 | 53.425 | 22.243 |
| 1681 | CB | VAL | 935 | 20.942 | 53.176 | 23.392 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1682 | CG1 | VAL | 935 | 20.838 | 54.420 | 24.251 |
| 1683 | CG2 | VAL | 935 | 19.578 | 52.840 | 22.834 |
| 1684 | C | VAL | 935 | 23.198 | 54.025 | 22.820 |
| 1685 | O | VAL | 935 | 23.482 | 55.204 | 22.626 |
| 1686 | N | SER | 936 | 23.964 | 53.210 | 23.530 |
| 1687 | CA | SER | 936 | 25.213 | 53.684 | 24.096 |
| 1688 | CB | SER | 936 | 26.126 | 52.532 | 24.592 |
| 1689 | C | SER | 936 | 25.962 | 54.526 | 23.037 |
| 1690 | O | SER | 936 | 26.249 | 55.702 | 23.270 |
| 1691 | OG | SER | 936 | 27.299 | 53.007 | 25.241 |
| 1692 | N | ASP | 937 | 26.245 | 53.942 | 21.872 |
| 1693 | CA | ASP | 937 | 26.940 | 54.653 | 20.786 |
| 1694 | CB | ASP | 937 | 27.064 | 53.786 | 19.544 |
| 1695 | CG | ASP | 937 | 28.095 | 52.733 | 19.691 |
| 1696 | OD1 | ASP | 937 | 29.205 | 53.001 | 20.143 |
| 1697 | C | ASP | 937 | 26.250 | 55.917 | 20.337 |
| 1698 | O | ASP | 937 | 26.897 | 56.926 | 20.066 |
| 1699 | OD2 | ASP | 937 | 27.576 | 51.470 | 19.631 |
| 1700 | N | LEU | 938 | 24.939 | 55.841 | 20.209 |
| 1701 | CA | LEU | 938 | 24.194 | 56.994 | 19.767 |
| 1702 | CB | LEU | 938 | 22.745 | 56.613 | 19.505 |
| 1703 | CG | LEU | 938 | 22.526 | 55.761 | 18.258 |
| 1704 | CD1 | LEU | 938 | 21.053 | 55.804 | 17.907 |
| 1705 | CD2 | LEU | 938 | 23.359 | 56.294 | 17.101 |
| 1706 | C | LEU | 938 | 24.278 | 58.136 | 20.765 |
| 1707 | O | LEU | 938 | 24.502 | 59.281 | 20.380 |
| 1708 | N | LEU | 939 | 24.128 | 57.811 | 22.048 |
| 1709 | CA | LEU | 939 | 24.149 | 58.797 | 23.131 |
| 1710 | CB | LEU | 939 | 23.652 | 58.115 | 24.413 |
| 1711 | CG | LEU | 939 | 22.540 | 58.624 | 25.350 |
| 1712 | CD1 | LEU | 939 | 21.445 | 59.418 | 24.648 |
| 1713 | CD2 | LEU | 939 | 21.956 | 57.384 | 26.015 |
| 1714 | C | LEU | 939 | 25.495 | 59.512 | 23.375 |
| 1715 | O | LEU | 939 | 25.512 | 60.736 | 23.544 |
| 1716 | N | GLU | 940 | 26.613 | 58.777 | 23.399 |
| 1717 | CA | GLU | 940 | 27.930 | 59.403 | 23.627 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1718 | CB | GLU | 940 | 29.094 | 58.382 | 23.486 |
| 1719 | C | GLU | 940 | 28.154 | 60.548 | 22.659 |
| 1720 | O | GLU | 940 | 28.576 | 61.643 | 23.029 |
| 1721 | CG | GLU | 940 | 30.519 | 58.995 | 23.561 |
| 1722 | CD | GLU | 940 | 30.834 | 59.804 | 24.872 |
| 1723 | OE1 | GLU | 940 | 30.234 | 59.461 | 25.896 |
| 1724 | OE2 | GLU | 940 | 31.666 | 60.706 | 24.729 |
| 1725 | N | PHE | 941 | 27.880 | 60.238 | 21.398 |
| 1726 | CA | PHE | 941 | 28.004 | 61.147 | 20.273 |
| 1727 | CB | PHE | 941 | 27.702 | 60.368 | 19.001 |
| 1728 | CG | PHE | 941 | 28.400 | 60.835 | 17.757 |
| 1729 | CD1 | PHE | 941 | 28.546 | 62.194 | 17.467 |
| 1730 | CE1 | PHE | 941 | 29.203 | 62.614 | 16.306 |
| 1731 | CD2 | PHE | 941 | 28.923 | 59.900 | 16.866 |
| 1732 | CE2 | PHE | 941 | 29.578 | 60.293 | 15.710 |
| 1733 | CZ | PHE | 941 | 29.716 | 61.652 | 15.428 |
| 1734 | C | PHE | 941 | 26.980 | 62.268 | 20.405 |
| 1735 | O | PHE | 941 | 27.032 | 63.260 | 19.681 |
| 1736 | N | CYS | 942 | 26.029 | 62.091 | 21.313 |
| 1737 | CA | CYS | 942 | 24.983 | 63.082 | 21.515 |
| 1738 | CB | CYS | 942 | 23.667 | 62.395 | 21.861 |
| 1739 | SG | CYS | 942 | 22.249 | 63.518 | 22.095 |
| 1740 | C | CYS | 942 | 25.364 | 64.028 | 22.630 |
| 1741 | O | CYS | 942 | 25.093 | 65.227 | 22.560 |
| 1742 | N | PHE | 943 | 25.975 | 63.457 | 23.665 |
| 1743 | CA | PHE | 943 | 26.444 | 64.211 | 24.817 |
| 1744 | CB | PHE | 943 | 26.870 | 63.257 | 25.950 |
| 1745 | CG | PHE | 943 | 25.725 | 62.493 | 26.600 |
| 1746 | CD1 | PHE | 943 | 24.403 | 62.897 | 26.446 |
| 1747 | CD2 | PHE | 943 | 25.992 | 61.399 | 27.431 |
| 1748 | CE1 | PHE | 943 | 23.356 | 62.227 | 27.103 |
| 1749 | CE2 | PHE | 943 | 24.953 | 60.720 | 28.094 |
| 1750 | CZ | PHE | 943 | 23.635 | 61.137 | 27.930 |
| 1751 | C | PHE | 943 | 27.657 | 65.018 | 24.331 |
| 1752 | O | PHE | 943 | 27.739 | 66.230 | 24.546 |
| 1753 | N | TYR | 944 | 28.580 | 64.328 | 23.661 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1754 | CA | TYR | 944 | 29.792 | 64.947 | 23.117 |
| 1755 | CB | TYR | 944 | 30.614 | 64.019 | 22.183 |
| 1756 | C | TYR | 944 | 29.352 | 66.200 | 22.336 |
| 1757 | O | TYR | 944 | 29.713 | 67.316 | 22.712 |
| 1758 | CG | TYR | 944 | 31.628 | 64.830 | 21.403 |
| 1759 | CD1 | TYR | 944 | 32.695 | 65.534 | 22.068 |
| 1760 | CD2 | TYR | 944 | 31.503 | 64.959 | 19.972 |
| 1761 | CE1 | TYR | 944 | 33.605 | 66.358 | 21.317 |
| 1762 | CE2 | TYR | 944 | 32.419 | 65.780 | 19.229 |
| 1763 | CZ | TYR | 944 | 33.480 | 66.497 | 19.889 |
| 1764 | OH | TYR | 944 | 34.350 | 67.294 | 19.176 |
| 1765 | N | THR | 945 | 28.287 | 66.230 | 21.337 |
| 1766 | CA | THR | 945 | 27.800 | 67.322 | 20.501 |
| 1767 | C | THR | 945 | 27.089 | 68.377 | 21.331 |
| 1768 | O | THR | 945 | 27.152 | 69.566 | 21.020 |
| 1769 | CB | THR | 945 | 26.837 | 66.791 | 19.405 |
| 1770 | OG1 | THR | 945 | 27.460 | 65.817 | 18.573 |
| 1771 | CG2 | THR | 945 | 26.356 | 67.912 | 18.511 |
| 1772 | N | PHE | 946 | 26.423 | 67.928 | 22.391 |
| 1773 | CA | PHE | 946 | 25.684 | 68.805 | 23.294 |
| 1774 | C | PHE | 946 | 26.683 | 69.701 | 24.034 |
| 1775 | O | PHE | 946 | 26.466 | 70.902 | 24.160 |
| 1776 | CB | PHE | 946 | 24.899 | 67.961 | 24.302 |
| 1777 | CG | PHE | 946 | 24.055 | 68.766 | 25.255 |
| 1778 | CD1 | PHE | 946 | 22.905 | 69.411 | 24.823 |
| 1779 | CD2 | PHE | 946 | 24.442 | 68.901 | 26.584 |
| 1780 | CE1 | PHE | 946 | 22.143 | 70.187 | 25.697 |
| 1781 | CE2 | PHE | 946 | 23.690 | 69.673 | 27.468 |
| 1782 | CZ | PHE | 946 | 22.537 | 70.319 | 27.025 |
| 1783 | N | ARG | 947 | 27.770 | 69.110 | 24.527 |
| 1784 | CA | ARG | 947 | 28.799 | 69.887 | 25.225 |
| 1785 | C | ARG | 947 | 29.519 | 70.858 | 24.274 |
| 1786 | O | ARG | 947 | 29.941 | 71.947 | 24.678 |
| 1787 | CB | ARG | 947 | 29.802 | 68.926 | 25.920 |
| 1788 | CG | ARG | 947 | 30.861 | 69.622 | 26.815 |
| 1789 | CD | ARG | 947 | 31.844 | 68.628 | 27.446 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1790 | NE | ARG | 947 | 32.810 | 69.389 | 28.278 |
| 1791 | CZ | ARG | 947 | 33.806 | 68.863 | 28.977 |
| 1792 | NH1 | ARG | 947 | 34.078 | 67.592 | 29.025 |
| 1793 | NH2 | ARG | 947 | 34.549 | 69.668 | 29.650 |
| 1794 | N | GLU | 948 | 29.648 | 70.476 | 23.005 |
| 1795 | CA | GLU | 948 | 30.336 | 71.328 | 22.034 |
| 1796 | C | GLU | 948 | 29.348 | 72.084 | 21.156 |
| 1797 | O | GLU | 948 | 29.743 | 72.682 | 20.152 |
| 1798 | CB | GLU | 948 | 31.277 | 70.432 | 21.180 |
| 1799 | CG | GLU | 948 | 32.464 | 69.730 | 21.920 |
| 1800 | CD | GLU | 948 | 33.610 | 70.599 | 22.443 |
| 1801 | OE1 | GLU | 948 | 33.987 | 71.616 | 21.876 |
| 1802 | OE2 | GLU | 948 | 34.168 | 70.135 | 23.595 |
| 1803 | N | SER | 949 | 28.070 | 72.071 | 21.533 |
| 1804 | CA | SER | 949 | 27.034 | 72.719 | 20.729 |
| 1805 | C | SER | 949 | 27.350 | 74.132 | 20.238 |
| 1806 | O | SER | 949 | 27.131 | 74.442 | 19.071 |
| 1807 | CB | SER | 949 | 25.690 | 72.711 | 21.471 |
| 1808 | OG | SER | 949 | 25.673 | 73.463 | 22.684 |
| 1809 | N | HIS | 950 | 27.863 | 74.996 | 21.105 |
| 1810 | CA | HIS | 950 | 28.165 | 76.351 | 20.660 |
| 1811 | C | HIS | 950 | 29.304 | 76.341 | 19.648 |
| 1812 | O | HIS | 950 | 29.279 | 77.087 | 18.665 |
| 1813 | CB | HIS | 950 | 28.515 | 77.260 | 21.850 |
| 1814 | CG | HIS | 950 | 27.466 | 77.222 | 22.923 |
| 1815 | ND1 | HIS | 950 | 26.098 | 77.400 | 22.732 |
| 1816 | CE1 | HIS | 950 | 25.669 | 77.261 | 24.001 |
| 1817 | NE2 | HIS | 950 | 26.596 | 77.025 | 24.969 |
| 1818 | CD2 | HIS | 950 | 27.768 | 76.999 | 24.259 |
| 1819 | N | ALA | 951 | 30.284 | 75.470 | 19.863 |
| 1820 | CA | ALA | 951 | 31.426 | 75.374 | 18.965 |
| 1821 | C | ALA | 951 | 31.055 | 74.760 | 17.623 |
| 1822 | O | ALA | 951 | 31.589 | 75.140 | 16.583 |
| 1823 | CB | ALA | 951 | 32.532 | 74.549 | 19.617 |
| 1824 | N | LEU | 952 | 30.135 | 73.806 | 17.654 |
| 1825 | CA | LEU | 952 | 29.702 | 73.117 | 16.446 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1826 | C | LEU | 952 | 28.543 | 73.820 | 15.762 |
| 1827 | O | LEU | 952 | 28.123 | 73.419 | 14.680 |
| 1828 | CB | LEU | 952 | 29.305 | 71.683 | 16.797 |
| 1829 | CG | LEU | 952 | 30.429 | 70.781 | 17.301 |
| 1830 | CD1 | LEU | 952 | 29.852 | 69.453 | 17.772 |
| 1831 | CD2 | LEU | 952 | 31.442 | 70.567 | 16.184 |
| 1832 | N | LYS | 953 | 28.044 | 74.878 | 16.387 |
| 1833 | CA | LYS | 953 | 26.919 | 75.618 | 15.851 |
| 1834 | C | LYS | 953 | 25.688 | 74.721 | 15.769 |
| 1835 | O | LYS | 953 | 24.879 | 74.855 | 14.856 |
| 1836 | CB | LYS | 953 | 27.275 | 76.202 | 14.455 |
| 1837 | CG | LYS | 953 | 28.396 | 77.270 | 14.502 |
| 1838 | CD | LYS | 953 | 28.727 | 77.915 | 13.154 |
| 1839 | CE | LYS | 953 | 29.796 | 78.997 | 13.360 |
| 1840 | NZ | LYS | 953 | 30.242 | 79.500 | 12.048 |
| 1841 | N | VAL | 954 | 25.548 | 73.805 | 16.726 |
| 1842 | CA | VAL | 954 | 24.396 | 72.901 | 16.749 |
| 1843 | C | VAL | 954 | 23.448 | 73.310 | 17.879 |
| 1844 | O | VAL | 954 | 23.850 | 73.383 | 19.035 |
| 1845 | CB | VAL | 954 | 24.841 | 71.440 | 16.958 |
| 1846 | CG1 | VAL | 954 | 23.611 | 70.519 | 17.045 |
| 1847 | CG2 | VAL | 954 | 25.752 | 71.000 | 15.796 |
| 1848 | N | GLU | 955 | 22.197 | 73.600 | 17.536 |
| 1849 | CA | GLU | 955 | 21.214 | 73.984 | 18.537 |
| 1850 | C | GLU | 955 | 20.405 | 72.802 | 19.068 |
| 1851 | O | GLU | 955 | 19.952 | 71.973 | 18.288 |
| 1852 | CB | GLU | 955 | 20.238 | 75.010 | 17.965 |
| 1853 | CG | GLU | 955 | 19.118 | 75.361 | 18.944 |
| 1854 | CD | GLU | 955 | 18.123 | 76.367 | 18.393 |
| 1855 | OE1 | GLU | 955 | 18.298 | 76.824 | 17.240 |
| 1856 | OE2 | GLU | 955 | 17.160 | 76.703 | 19.123 |
| 1857 | N | PHE | 956 | 20.094 | 72.231 | 20.212 |
| 1858 | CA | PHE | 956 | 19.219 | 71.221 | 20.770 |
| 1859 | CB | PHE | 956 | 19.929 | 70.498 | 21.911 |
| 1860 | CG | PHE | 956 | 20.927 | 69.468 | 21.447 |
| 1861 | CD1 | PHE | 956 | 22.196 | 69.830 | 20.999 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1862 | CD2 | PHE | 956 | 20.607 | 68.119 | 21.520 |
| 1863 | CE1 | PHE | 956 | 23.121 | 68.857 | 20.622 |
| 1864 | CE2 | PHE | 956 | 21.518 | 67.143 | 21.149 |
| 1865 | CZ | PHE | 956 | 22.780 | 67.511 | 20.716 |
| 1866 | C | PHE | 956 | 17.952 | 71.918 | 21.259 |
| 1867 | O | PHE | 956 | 18.024 | 72.911 | 21.981 |
| 1868 | N | PRO | 957 | 16.764 | 71.402 | 20.888 |
| 1869 | CD | PRO | 957 | 16.416 | 70.032 | 20.472 |
| 1870 | CA | PRO | 957 | 15.575 | 72.103 | 21.362 |
| 1871 | CB | PRO | 957 | 14.447 | 71.365 | 20.659 |
| 1872 | CG | PRO | 957 | 14.903 | 69.963 | 20.757 |
| 1873 | C | PRO | 957 | 15.475 | 72.040 | 22.880 |
| 1874 | O | PRO | 957 | 16.409 | 71.607 | 23.553 |
| 1875 | N | ALA | 958 | 14.331 | 72.467 | 23.410 |
| 1876 | CA | ALA | 958 | 14.097 | 72.501 | 24.852 |
| 1877 | CB | ALA | 958 | 12.877 | 73.367 | 25.145 |
| 1878 | C | ALA | 958 | 13.909 | 71.138 | 25.508 |
| 1879 | O | ALA | 958 | 14.633 | 70.777 | 26.439 |
| 1880 | N | MET | 959 | 12.912 | 70.398 | 25.039 |
| 1881 | CA | MET | 959 | 12.643 | 69.079 | 25.575 |
| 1882 | CB | MET | 959 | 11.709 | 68.321 | 24.623 |
| 1883 | CG | MET | 959 | 11.377 | 66.927 | 25.090 |
| 1884 | SD | MET | 959 | 10.899 | 66.977 | 26.813 |
| 1885 | CE | MET | 959 | 9.291 | 67.636 | 26.628 |
| 1886 | C | MET | 959 | 13.970 | 68.335 | 25.741 |
| 1887 | O | MET | 959 | 14.433 | 68.125 | 26.862 |
| 1888 | N | LEU | 960 | 14.579 | 67.968 | 24.613 |
| 1889 | CA | LEU | 960 | 15.864 | 67.258 | 24.563 |
| 1890 | CB | LEU | 960 | 16.367 | 67.174 | 23.116 |
| 1891 | CG | LEU | 960 | 16.274 | 65.922 | 22.248 |
| 1892 | CD1 | LEU | 960 | 14.839 | 65.532 | 21.975 |
| 1893 | CD2 | LEU | 960 | 16.973 | 66.214 | 20.943 |
| 1894 | C | LEU | 960 | 16.935 | 67.952 | 25.394 |
| 1895 | O | LEU | 960 | 17.790 | 67.299 | 26.003 |
| 1896 | N | VAL | 961 | 16.902 | 69.276 | 25.378 |
| 1897 | CA | VAL | 961 | 17.856 | 70.091 | 26.120 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1898 | CB | VAL | 961 | 17.706 | 71.666 | 26.224 |
| 1899 | C | VAL | 961 | 17.957 | 69.563 | 27.547 |
| 1900 | O | VAL | 961 | 19.039 | 69.199 | 28.011 |
| 1901 | CG1 | VAL | 961 | 18.703 | 72.407 | 25.295 |
| 1902 | CG2 | VAL | 961 | 17.886 | 72.321 | 27.621 |
| 1903 | N | GLU | 962 | 16.806 | 69.518 | 28.210 |
| 1904 | CA | GLU | 962 | 16.651 | 69.055 | 29.593 |
| 1905 | CB | GLU | 962 | 15.206 | 69.219 | 30.010 |
| 1906 | CG | GLU | 962 | 14.904 | 70.411 | 30.842 |
| 1907 | CD | GLU | 962 | 13.434 | 70.465 | 31.145 |
| 1908 | OE1 | GLU | 962 | 12.721 | 69.520 | 30.737 |
| 1909 | OE2 | GLU | 962 | 12.990 | 71.440 | 31.785 |
| 1910 | C | GLU | 962 | 17.019 | 67.610 | 29.895 |
| 1911 | O | GLU | 962 | 17.672 | 67.319 | 30.900 |
| 1912 | N | ILE | 963 | 16.530 | 66.715 | 29.043 |
| 1913 | CA | ILE | 963 | 16.735 | 65.276 | 29.160 |
| 1914 | CB | ILE | 963 | 15.854 | 64.552 | 28.143 |
| 1915 | CG2 | ILE | 963 | 15.968 | 63.057 | 28.308 |
| 1916 | CG1 | ILE | 963 | 14.407 | 64.986 | 28.335 |
| 1917 | CD1 | ILE | 963 | 13.573 | 64.754 | 27.120 |
| 1918 | C | ILE | 963 | 18.189 | 64.865 | 28.960 |
| 1919 | O | ILE | 963 | 18.644 | 63.878 | 29.547 |
| 1920 | N | ILE | 964 | 18.914 | 65.604 | 28.115 |
| 1921 | CA | ILE | 964 | 20.324 | 65.308 | 27.887 |
| 1922 | CB | ILE | 964 | 20.867 | 65.955 | 26.563 |
| 1923 | CG2 | ILE | 964 | 22.384 | 65.814 | 26.499 |
| 1924 | CG1 | ILE | 964 | 20.292 | 65.222 | 25.339 |
| 1925 | CD1 | ILE | 964 | 20.013 | 66.096 | 24.116 |
| 1926 | C | ILE | 964 | 21.097 | 65.825 | 29.105 |
| 1927 | O | ILE | 964 | 21.970 | 65.128 | 29.622 |
| 1928 | N | SER | 965 | 20.757 | 67.031 | 29.570 |
| 1929 | CA | SER | 965 | 21.391 | 67.626 | 30.757 |
| 1930 | CB | SER | 965 | 20.865 | 69.070 | 31.026 |
| 1931 | C | SER | 965 | 21.061 | 66.760 | 31.975 |
| 1932 | O | SER | 965 | 21.902 | 66.526 | 32.844 |
| 1933 | OG | SER | 965 | 19.502 | 69.092 | 31.464 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1934 | N | ASP | 966 | 19.818 | 66.295 | 32.019 |
| 1935 | CA | ASP | 966 | 19.303 | 65.434 | 33.081 |
| 1936 | CB | ASP | 966 | 17.883 | 64.988 | 32.699 |
| 1937 | CG | ASP | 966 | 17.209 | 64.126 | 33.760 |
| 1938 | OD1 | ASP | 966 | 17.765 | 63.130 | 34.230 |
| 1939 | C | ASP | 966 | 20.211 | 64.210 | 33.212 |
| 1940 | O | ASP | 966 | 20.949 | 64.053 | 34.184 |
| 1941 | OD2 | ASP | 966 | 16.115 | 64.728 | 34.317 |
| 1942 | N | GLN | 967 | 20.139 | 63.372 | 32.187 |
| 1943 | CA | GLN | 967 | 20.858 | 62.106 | 32.055 |
| 1944 | CB | GLN | 967 | 20.407 | 61.462 | 30.754 |
| 1945 | CG | GLN | 967 | 18.958 | 61.134 | 30.781 |
| 1946 | CD | GLN | 967 | 18.665 | 60.195 | 31.918 |
| 1947 | OE1 | GLN | 967 | 18.006 | 60.557 | 32.892 |
| 1948 | NE2 | GLN | 967 | 19.196 | 58.911 | 31.958 |
| 1949 | C | GLN | 967 | 22.379 | 62.075 | 32.087 |
| 1950 | O | GLN | 967 | 22.987 | 61.168 | 32.664 |
| 1951 | N | LEU | 968 | 22.965 | 63.066 | 31.431 |
| 1952 | CA | LEU | 968 | 24.404 | 63.194 | 31.282 |
| 1953 | CB | LEU | 968 | 24.767 | 64.662 | 30.896 |
| 1954 | C | LEU | 968 | 25.260 | 62.800 | 32.495 |
| 1955 | O | LEU | 968 | 26.306 | 62.165 | 32.333 |
| 1956 | CG | LEU | 968 | 26.255 | 65.071 | 30.998 |
| 1957 | CD1 | LEU | 968 | 27.125 | 64.301 | 29.982 |
| 1958 | CD2 | LEU | 968 | 26.401 | 66.600 | 30.833 |
| 1959 | N | PRO | 969 | 24.827 | 63.139 | 33.720 |
| 1960 | CD | PRO | 969 | 23.761 | 64.056 | 34.147 |
| 1961 | CA | PRO | 969 | 25.651 | 62.756 | 34.869 |
| 1962 | CB | PRO | 969 | 25.002 | 63.513 | 36.025 |
| 1963 | CG | PRO | 969 | 24.385 | 64.700 | 35.350 |
| 1964 | C | PRO | 969 | 25.669 | 61.252 | 35.114 |
| 1965 | O | PRO | 969 | 26.724 | 60.620 | 35.093 |
| 1966 | N | LYS | 970 | 24.489 | 60.678 | 35.323 |
| 1967 | CA | LYS | 970 | 24.382 | 59.253 | 35.612 |
| 1968 | CB | LYS | 970 | 23.093 | 58.967 | 36.395 |
| 1969 | CG | LYS | 970 | 21.918 | 58.557 | 35.516 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 1970 | CD | LYS | 970 | 20.970 | 59.705 | 35.241 |
| 1971 | CE | LYS | 970 | 19.993 | 59.845 | 36.399 |
| 1972 | NZ | LYS | 970 | 18.955 | 60.941 | 36.202 |
| 1973 | C | LYS | 970 | 24.465 | 58.260 | 34.443 |
| 1974 | O | LYS | 970 | 24.428 | 57.052 | 34.683 |
| 1975 | N | VAL | 971 | 24.557 | 58.730 | 33.195 |
| 1976 | CA | VAL | 971 | 24.646 | 57.790 | 32.064 |
| 1977 | CB | VAL | 971 | 24.076 | 58.386 | 30.763 |
| 1978 | C | VAL | 971 | 26.088 | 57.388 | 31.819 |
| 1979 | O | VAL | 971 | 26.434 | 56.940 | 30.727 |
| 1980 | CG1 | VAL | 971 | 22.556 | 58.673 | 30.818 |
| 1981 | CG2 | VAL | 971 | 24.289 | 57.551 | 29.471 |
| 1982 | N | GLU | 972 | 26.913 | 57.538 | 32.852 |
| 1983 | CA | GLU | 972 | 28.335 | 57.225 | 32.772 |
| 1984 | CB | GLU | 972 | 29.130 | 58.508 | 32.516 |
| 1985 | C | GLU | 972 | 28.875 | 56.589 | 34.050 |
| 1986 | O | GLU | 972 | 29.789 | 55.764 | 34.002 |
| 1987 | CG | GLU | 972 | 29.130 | 59.589 | 33.646 |
| 1988 | CD | GLU | 972 | 29.832 | 60.922 | 33.377 |
| 1989 | OE1 | GLU | 972 | 29.856 | 61.838 | 34.188 |
| 1990 | OE2 | GLU | 972 | 30.426 | 60.991 | 32.153 |
| 1991 | N | SER | 973 | 28.308 | 56.988 | 35.189 |
| 1992 | CA | SER | 973 | 28.731 | 56.522 | 36.514 |
| 1993 | CB | SER | 973 | 28.038 | 57.356 | 37.602 |
| 1994 | C | SER | 973 | 28.536 | 55.048 | 36.864 |
| 1995 | O | SER | 973 | 29.487 | 54.346 | 37.233 |
| 1996 | OG | SER | 973 | 28.513 | 58.706 | 37.656 |
| 1997 | N | GLY | 974 | 27.293 | 54.593 | 36.770 |
| 1998 | CA | GLY | 974 | 26.947 | 53.226 | 37.110 |
| 1999 | C | GLY | 974 | 25.688 | 53.370 | 37.941 |
| 2000 | O | GLY | 974 | 25.613 | 52.946 | 39.095 |
| 2001 | N | ASN | 975 | 24.696 | 53.998 | 37.334 |
| 2002 | CA | ASN | 975 | 23.438 | 54.253 | 38.004 |
| 2003 | CB | ASN | 975 | 23.152 | 55.769 | 37.918 |
| 2004 | CG | ASN | 975 | 24.345 | 56.632 | 38.410 |
| 2005 | OD1 | ASN | 975 | 25.508 | 56.343 | 38.100 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 2006 | ND2 | ASN | 975 | 24.052 | 57.602 | 39.400 |
| 2007 | C | ASN | 975 | 22.307 | 53.415 | 37.380 |
| 2008 | O | ASN | 975 | 21.467 | 52.864 | 38.093 |
| 2009 | N | ALA | 976 | 22.343 | 53.284 | 36.050 |
| 2010 | CA | ALA | 976 | 21.348 | 52.557 | 35.245 |
| 2011 | CB | ALA | 976 | 21.235 | 53.216 | 33.853 |
| 2012 | C | ALA | 976 | 21.560 | 51.042 | 35.030 |
| 2013 | O | ALA | 976 | 22.631 | 50.601 | 34.612 |
| 2014 | N | LYS | 977 | 20.512 | 50.260 | 35.283 |
| 2015 | CA | LYS | 977 | 20.559 | 48.811 | 35.128 |
| 2016 | CB | LYS | 977 | 19.815 | 48.166 | 36.285 |
| 2017 | CG | LYS | 977 | 19.808 | 46.674 | 36.278 |
| 2018 | CD | LYS | 977 | 18.743 | 46.214 | 37.234 |
| 2019 | CE | LYS | 977 | 18.641 | 44.708 | 37.259 |
| 2020 | NZ | LYS | 977 | 17.452 | 44.172 | 38.042 |
| 2021 | C | LYS | 977 | 19.920 | 48.379 | 33.808 |
| 2022 | O | LYS | 977 | 18.698 | 48.379 | 33.665 |
| 2023 | N | PRO | 978 | 20.759 | 47.994 | 32.857 |
| 2024 | CA | PRO | 978 | 20.302 | 47.585 | 31.535 |
| 2025 | CB | PRO | 978 | 21.489 | 47.759 | 30.556 |
| 2026 | C | PRO | 978 | 19.723 | 46.150 | 31.515 |
| 2027 | O | PRO | 978 | 20.467 | 45.190 | 31.365 |
| 2028 | CG | PRO | 978 | 22.674 | 47.424 | 31.477 |
| 2029 | CD | PRO | 978 | 22.310 | 48.109 | 32.798 |
| 2030 | N | LEU | 979 | 18.404 | 46.005 | 31.678 |
| 2031 | CA | LEU | 979 | 17.752 | 44.683 | 31.668 |
| 2032 | CB | LEU | 979 | 16.226 | 44.819 | 31.756 |
| 2033 | CG | LEU | 979 | 15.695 | 45.767 | 32.823 |
| 2034 | CD1 | LEU | 979 | 14.170 | 45.795 | 32.863 |
| 2035 | CD2 | LEU | 979 | 16.246 | 45.305 | 34.139 |
| 2036 | C | LEU | 979 | 18.084 | 43.939 | 30.374 |
| 2037 | O | LEU | 979 | 18.209 | 44.559 | 29.320 |
| 2038 | N | TYR | 980 | 18.217 | 42.619 | 30.438 |
| 2039 | CA | TYR | 980 | 18.517 | 41.850 | 29.230 |
| 2040 | CB | TYR | 980 | 19.994 | 41.458 | 29.180 |
| 2041 | C | TYR | 980 | 17.684 | 40.579 | 29.171 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 2042 | O | TYR | 980 | 17.437 | 39.953 | 30.199 |
| 2043 | CG | TYR | 980 | 21.010 | 42.605 | 29.135 |
| 2044 | CD1 | TYR | 980 | 21.693 | 42.974 | 30.299 |
| 2045 | CD2 | TYR | 980 | 21.256 | 43.294 | 27.943 |
| 2046 | CE1 | TYR | 980 | 22.606 | 44.024 | 30.272 |
| 2047 | CE2 | TYR | 980 | 22.170 | 44.344 | 27.920 |
| 2048 | CZ | TYR | 980 | 22.844 | 44.708 | 29.082 |
| 2049 | OH | TYR | 980 | 23.738 | 45.741 | 29.055 |
| 2050 | N | PHE | 981 | 17.248 | 40.207 | 27.972 |
| 2051 | CA | PHE | 981 | 16.476 | 38.990 | 27.793 |
| 2052 | CB | PHE | 981 | 15.719 | 39.019 | 26.466 |
| 2053 | CG | PHE | 981 | 14.478 | 39.838 | 26.516 |
| 2054 | CD1 | PHE | 981 | 13.293 | 39.284 | 26.973 |
| 2055 | CD2 | PHE | 981 | 14.525 | 41.204 | 26.255 |
| 2056 | CE1 | PHE | 981 | 12.168 | 40.081 | 27.180 |
| 2057 | CE2 | PHE | 981 | 13.408 | 42.012 | 26.459 |
| 2058 | CZ | PHE | 981 | 12.228 | 41.449 | 26.927 |
| 2059 | C | PHE | 981 | 17.465 | 37.848 | 27.815 |
| 2060 | O | PHE | 981 | 17.124 | 36.725 | 28.178 |
| 2061 | N | HIS | 982 | 18.705 | 38.164 | 27.441 |
| 2062 | CA | HIS | 982 | 19.793 | 37.189 | 27.394 |
| 2063 | CB | HIS | 982 | 20.119 | 36.846 | 25.950 |
| 2064 | CG | HIS | 982 | 18.928 | 36.424 | 25.171 |
| 2065 | CD2 | HIS | 982 | 18.431 | 36.849 | 23.989 |
| 2066 | ND1 | HIS | 982 | 18.030 | 35.495 | 25.655 |
| 2067 | CE1 | HIS | 982 | 17.028 | 35.374 | 24.807 |
| 2068 | NE2 | HIS | 982 | 17.245 | 36.186 | 23.786 |
| 2069 | C | HIS | 982 | 21.054 | 37.669 | 28.067 |
| 2070 | O | HIS | 982 | 21.554 | 38.757 | 27.770 |
| 2071 | N | ARG | 983 | 21.586 | 36.847 | 28.961 |
| 2072 | CA | ARG | 983 | 22.811 | 37.196 | 29.655 |
| 2073 | CB | ARG | 983 | 22.886 | 36.464 | 31.006 |
| 2074 | CG | ARG | 983 | 22.124 | 35.132 | 31.099 |
| 2075 | C | ARG | 983 | 24.076 | 36.946 | 28.818 |
| 2076 | O | ARG | 983 | 24.800 | 37.896 | 28.521 |
| 2077 | CD | ARG | 983 | 22.237 | 34.496 | 32.495 |

TABLE 11 (continued)

| THREE-DIMENSIONAL COORDINATES OF MR OBTAINED FROM HOMOLOGY MODELING OF THE CRYSTAL STRUCTURE COORDINATES OF GRα IN COMPLEX WITH FP | | | | | | |
|---|---|---|---|---|---|---|
| ATOM | ATOM TYPE | RESIDUE | RESIDUE # | X | Y | Z |
| 2078 | NE | ARG | 983 | 21.504 | 33.197 | 32.517 |
| 2079 | CZ | ARG | 983 | 21.405 | 32.388 | 33.567 |
| 2080 | NH1 | ARG | 983 | 20.738 | 31.285 | 33.433 |
| 2081 | NH2 | ARG | 983 | 21.942 | 32.642 | 34.727 |
| 2082 | N | LYS | 984 | 24.308 | 35.693 | 28.408 |
| 2083 | CA | LYS | 984 | 25.503 | 35.276 | 27.631 |
| 2084 | CB | LYS | 984 | 25.244 | 35.274 | 26.094 |
| 2085 | CG | LYS | 984 | 26.211 | 34.352 | 25.247 |
| 2086 | CD | LYS | 984 | 27.026 | 35.124 | 24.164 |
| 2087 | CE | LYS | 984 | 27.905 | 34.230 | 23.243 |
| 2088 | NZ | LYS | 984 | 29.205 | 33.731 | 23.855 |
| 2089 | C | LYS | 984 | 26.718 | 36.154 | 27.961 |
| 2090 | O | LYS | 984 | 26.967 | 37.154 | 27.246 |
| 2091 | OXT | LYS | 984 | 27.399 | 35.837 | 28.965 |

It will be understood that various details of the invention may be changed without departing from the scope of the invention. Furthermore, the foregoing description is for the purpose of illustration only, and not for the purpose of limitation the invention being defined by the claims.

## Claims

**1.** A crystalline GR polypeptide complex comprising an expanded binding pocket.

**2.** The polypeptide complex of claim 1, wherein an AF2 helix is located in an active position, and where atoms in residues Met560, Met639, Gln642, Cys643, Met646, and Tyr735 have shifted from their positions in a GR/Dex structure, **characterized by** the atomic structural coordinates of Table 3, by one of a heavy-atom RMS deviation of at least about 0.50 angstroms and by a backbone heavy-atom RMS deviation of at least about 0.35 angstroms.

**3.** The polypeptide complex of claim 1, wherein an AF2 helix is located in an active position, and wherein atoms in residues Met560, Met639, Gln642, Cys643, Met646, and Tyr735 have shifted from their positions in a GR/Dex structure, **characterized by** the atomic structural coordinates of Table 3, so as to increase the volume of the main binding pocket by at least about 5%, compared with a GR/Dex structure **characterized by** the atomic structural coordiates of Table 3.

**4.** The polypeptide complex of claim 1, wherein an AF2 helix is located in an active position, and wherein atoms in and around a ligand binding site have shifted from their positions in a GR/Dex structure, **characterized by** the atomic structural coordinates of Table 3, so as to accommodate, without atomic overlap, a steroidal ligand with 17-$\alpha$ substituents comprising 2-20 atoms.

**5.** The polypeptide complex of claim 1, wherein an AF2 helix is located in an active position, and wherein atoms in and around a ligand binding site have shifted from their positions in a GR/Dex structure, **characterized by** the atomic coordinates of Table 3, so as to accommodate, without atomic overlap, a non-steroidal ligand.

**6.** The polypeptide complex of claim 5, wherein the non-steroidal ligand is selected from the group consisting of benzoxazin-1-one and A-222977.

**7.** The polypeptide complex of claim 1, wherein an AF2 helix is located in an active position, and wherein atoms in and around a ligand binding site have shifted from their positions in a GR/Dex structure, **characterized by** the atomic coordinates of Table 3, such that fluticasone propionate can be docked into a binding site with a favorable

binding energy and wherein all atoms in the polypeptide are held fixed.

**8.** The polypeptide complex of claim 7, wherein the non-steroidal ligand is selected from the group consisting of benzoxazin-1-one and A-222977.

**9.** The polypeptide complex of claim 1, further comprising fluticasone propionate and a co-activator peptide.

**10.** The polypeptide complex of claim 9, wherein the crystalline form comprises lattice constants of a = b = 127.656 Å, c = 87.725 Å, $\alpha = 90°$, $\beta = 90°$, $\gamma = 120°$.

**11.** The polypeptide complex of claim 9, wherein the co-activator peptide is a TIF2 peptide.

**12.** The polypeptide complex of claim 9, wherein the complex comprises a hexagonal crystalline form.

**13.** The polypeptide complex of claim 9, wherein the crystalline form has a space group of $P6_1$.

**14.** The polypeptide complex of claim 9, wherein the GR polypeptide comprises a GR$\alpha$ ligand binding domain.

**15.** The polypeptide complex of claim 15, wherein the GR$\alpha$ polypeptide has the amino acid sequence shown in any one of SEQ ID NOs: 6 or 8.

**16.** The polypeptide complex of claim 15, further **characterized by** the atomic structure coordinates shown in Table 2.

**17.** The polypeptide complex of claim 15, wherein the crystalline form comprises two GR$\alpha$ ligand binding domain polypeptides in the asymmetric unit.

**18.** The polypeptide complex of claim 15, wherein the complex is such that the three-dimensional structure of the crystallized GR$\alpha$ ligand binding domain polypeptide can be determined to a resolution of about 3.0 Å or better.

**19.** The polypeptide complex of claim 9, wherein the complex comprises one or more atoms having a molecular weight of 40 grams/mol or greater.

**20.** A method for determining the three-dimensional structure of a crystallized GR polypeptide complex comprising an expanded binding pocket to a resolution of about 3.0 Å or better, the method comprising:

(a) crystallizing a GR ligand binding domain polypeptide; and
(b) analyzing the GR ligand binding domain polypeptide to determine the three-dimensional structure of the crystallized GR ligand binding domain polypeptide, whereby the three-dimensional structure of a crystallized GR polypeptide complex comprising an expanded binding pocket is determined to a resolution of about 3.0 Å or better.

**21.** The method of claim 20, wherein the reservoir solution comprises 60mM bis-Tris-propane, pH 7.5-8.5, and 1.5-1.7 M magnesium sulfate.

**22.** The method of claim 20, wherein the co-activator peptide is a TIF2 peptide.

**23.** The method of claim 20, wherein the GR ligand binding domain comprises one of SEQ ID NO: 6 and SEQ ID NO: 8.

**24.** A method of generating a crystallized GR polypeptide complex comprising an expanded binding pocket and a ligand known or suspected to be unable to associate with a known GR structure, the method comprising:

(a) providing a solution comprising a GR polypeptide and a ligand known or suspected to be unable to associate with a known GR structure; and
(b) crystallizing the GR ligand binding domain polypeptide using the hanging drop method, whereby a crystallized GR polypeptide complex comprising an expanded binding pocket and a ligand known or suspected to be unable to associate with a known GR structure is generated.

**25.** The method of claim 24, wherein the solution comprises 475 mM ammonium acetate, 25 mM NaCl, 50 mM Tris, pH 8.0, 10% glycerol, 10 mM dithiothreitol (DTT), 0.5mM EDTA and 0.05% β-octyl-glucoside.

**26.** The method of claim 24, wherein a crystallization reservoir solution comprises 60mM bis-Tris-propane, pH 7.5-8.5, and 1.5-1.7 M magnesium sulfate.

**28.** The method of claim 24, wherein the co-activator peptide is a TIF2 peptide.

**29.** The method of claim 24, wherein the GR polypeptide comprises one of SEQ ID NO: 6 and SEQ ID NO: 8.

**30.** A method for identifying a GR modulator, the method comprising:

(a) providing atomic coordinates of a GR polypeptide complex comprising an expanded binding pocket to a computerized modeling system;and
(b) modeling a ligand that fits spatially into the large pocket volume of the GR polypeptide complex to thereby identify a GR modulator.

**31.** A method of designing a modulator that selectively modulates the activity of a GRα polypeptide comprising an expanded binding pocket, the method comprising:

(a) providing a crystalline form of a GRα polypeptide complex comprising an expanded binding pocket;
(b) determining the three-dimensional structure of the crystalline form of the GRα ligand binding domain polypeptide; and
(c) synthesizing a modulator based on the three-dimensional structure of the crystalline form of the GRα ligand binding domain polypeptide, whereby a modulator that selectively modulates the activity of a GRα polypeptide comprising an expanded binding pocket is designed.

**32.** The method of claim 31, wherein the method further comprises contacting a GRα polypeptide with the potential modulator; and assaying the GRα polypeptide for binding of the potential modulator, for a change in activity of the GRα polypeptide, or both.

**33.** A method of forming a homology model of an NR, the method comprising:

(a) providing a template amino acid sequence comprising a GR polypeptide comprising an expanded binding pocket;
(b) providing a target NR amino acid sequence;
(c) aligning the target sequence and the template sequence to form a homology model.

**34.** The method of claim 33, further comprising assigning structural coordinates to the homology model.

**35.** The method of claim 33, wherein the NR is selected from the group consisting of AR, PR, ER, GR and MR.

**36.** The method of claim 33, wherein the template amino acid sequence comprises one of the atomic coordinates of Table 2 and a subset of the coordinates of Table 2.

**37.** The method of claim 33, wherein the template amino acid sequence comprises spatial coordinates characterizing an AF2 helix located in an active position, and wherein the spatial coordinates further characterize atoms in residues Met560, Met639, Gln642, Cys643, Met646, and Tyr735 that have shifted from their positions in a GR/Dex structure, **characterized by** the atomic structural coordinates of Table 3, by one of a heavy-atom RMS deviation of at least about 0.50 angstroms and by a backbone heavy-atom RMS deviation of at least about 0.35 angstroms.

**38.** The method of claim 33, wherein the template amino acid sequence comprises spatial coordinates characterizing an AF2 helix located in an active position, and wherein the spatial coordinates further characterize atoms in residues Met560, Met639, Gln642, Cys643, Met646, and Tyr735 that have shifted from their positions in a GR/Dex structure, **characterized by** the atomic structural coordinates of Table 3, so as to increase the volume of a binding pocket by at least about 5%, compared with a GR/Dex structure **characterized by** the atomic structural coordiates of Table 3.

**39.** The method of claim 33, wherein the template amino acid sequence comprises spatial coordinates characterizing an AF2 helix located in an active position, and wherein the spatial coordinates further characterize atoms in and around a ligand binding site that have shifted from their positions in a GR/Dex structure, **characterized by** the atomic structural coordinates of Table 3, so as to accommodate, without atomic overlap, a steroidal ligand with C17-$\alpha$ substituents comprising 2-20 atoms.

**40.** The method of claim 33, wherein the template amino acid sequence comprises spatial coordinates characterizing an AF2 helix located in an active position, and wherein the spatial coordinates further characterize atoms in and around a ligand binding site that have shifted from their positions in a GR/Dex structure, **characterized by** the atomic coordinates of Table 3, so as to accommodate, without atomic overlap, a non-steroidal ligand.

**41.** The method of claim 33, wherein the template amino acid sequence comprises spatial coordinates characterize an AF2 helix located in an active position, and wherein the spatial coordinates further characterize atoms in and around a ligand binding site that have shifted from their positions in a GR/Dex structure, **characterized by** the atomic coordinates of Table 3, such that fluticasone propionate can be docked into a binding site with a favorable binding energy and wherein all atoms in the polypeptide are held fixed.

**42.** The method of claim 33, wherein the template amino acid sequence comprises spatial coordinates characterizing an AF2 helix is located in an active position, and wherein the spatial coordinates further characterize atoms in and around the ligand binding site that have shifted from their positions in a GR/Dex structure, **characterized by** the atomic coordinates of Table 3, such that a non-steroidal GR ligand can be docked into the binding site with a favorable binding energy, as computed with molecular modeling software, and wherein all atoms in the polypeptide are held fixed.

**43.** A method of designing a modulator of a nuclear receptor, the method comprising:

(a) designing a potential modulator of a nuclear receptor that will make interactions with amino acids in the ligand binding site of the nuclear receptor based upon atomic structure coordinates of a NR polypeptide complex comprising an expanded binding pocket;
(b) synthesizing the modulator; and
(c) determining whether the potential modulator modulates the activity of the nuclear receptor, whereby a modulator of a nuclear receptor is designed.

**44.** The method of claim 46, wherein the potential modulator is a non-steroidal compound.

**45.** The method of claim 46, wherein the potential modulator is a steroid compound.

**46.** A method of modeling an interaction between an NR and a non-steroid ligand, the method comprising:

(a) providing a homology model of a target NR generated using a crystalline GR polypeptide complex comprising an expanded binding pocket;
(b) providing atomic coordinates of a non-steroid ligand; and
(c) docking the non-steroid ligand with the homology model to form a NR/ligand model.

**47.** A method of designing a non-steroid modulator of a target NR using a homology model, the method comprising:

(a) modeling an interaction between a target NR and a non-steroid ligand using a homology model generated using a crystalline GR polypeptide complex comprising an expanded binding pocket;
(b) evaluating the interaction between the target NR and the non-steroid ligand to determine a first binding efficiency;
(c) modifying the structure of the non-steroid ligand to form a modified ligand;
(d) modeling an interaction between the modified ligand and the target NR;
(e) evaluating the interaction between the target NR and the modified ligand to determine a second binding efficiency; and
(f) repeating steps (c)-(e) a desired number of times if the second binding efficiency is less than the first binding efficiency .

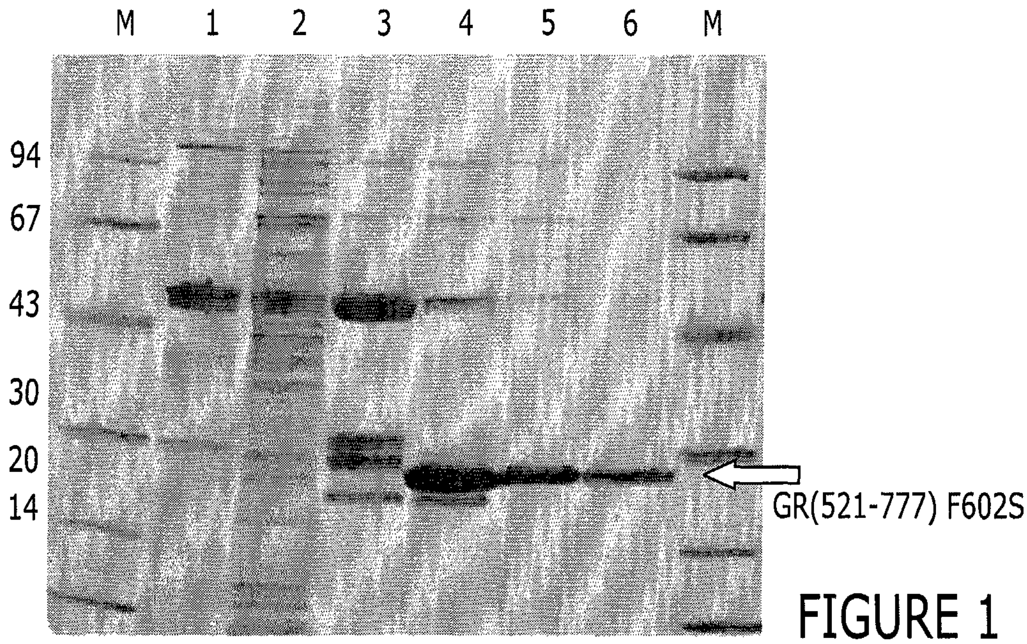


FIGURE 1

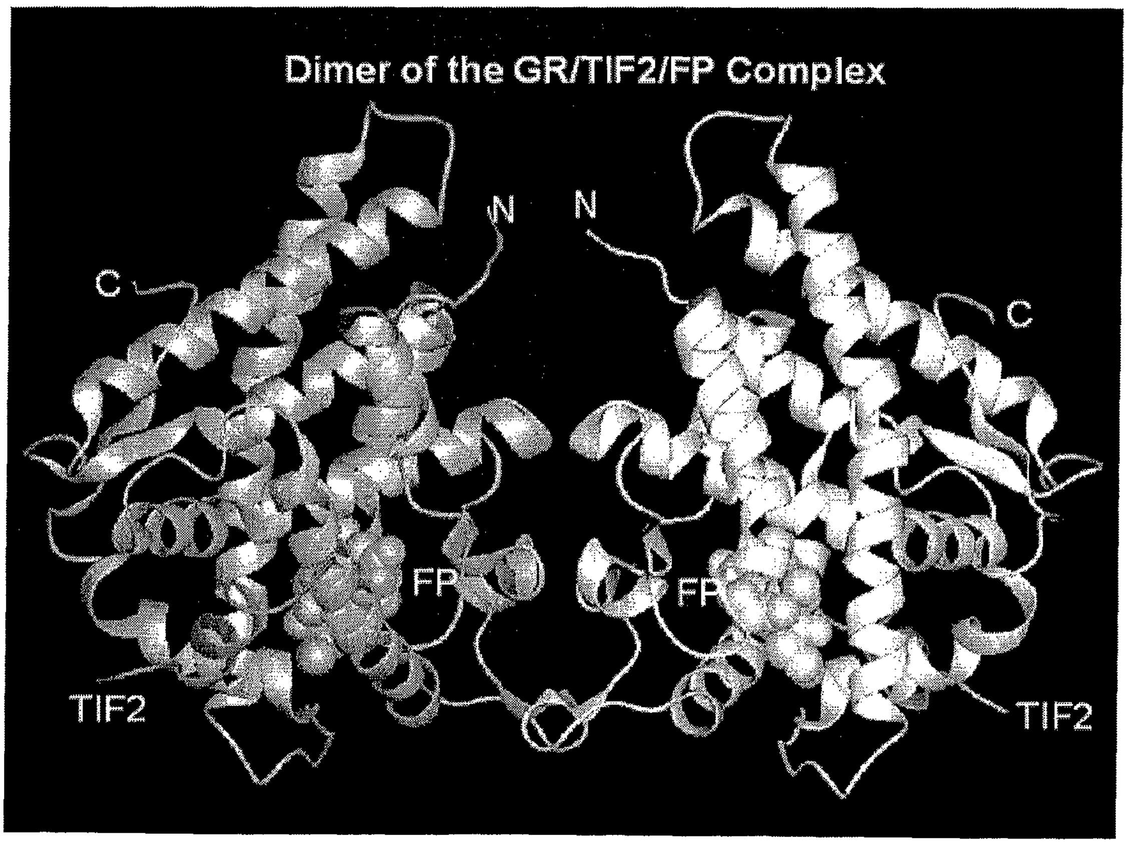


FIGURE 2

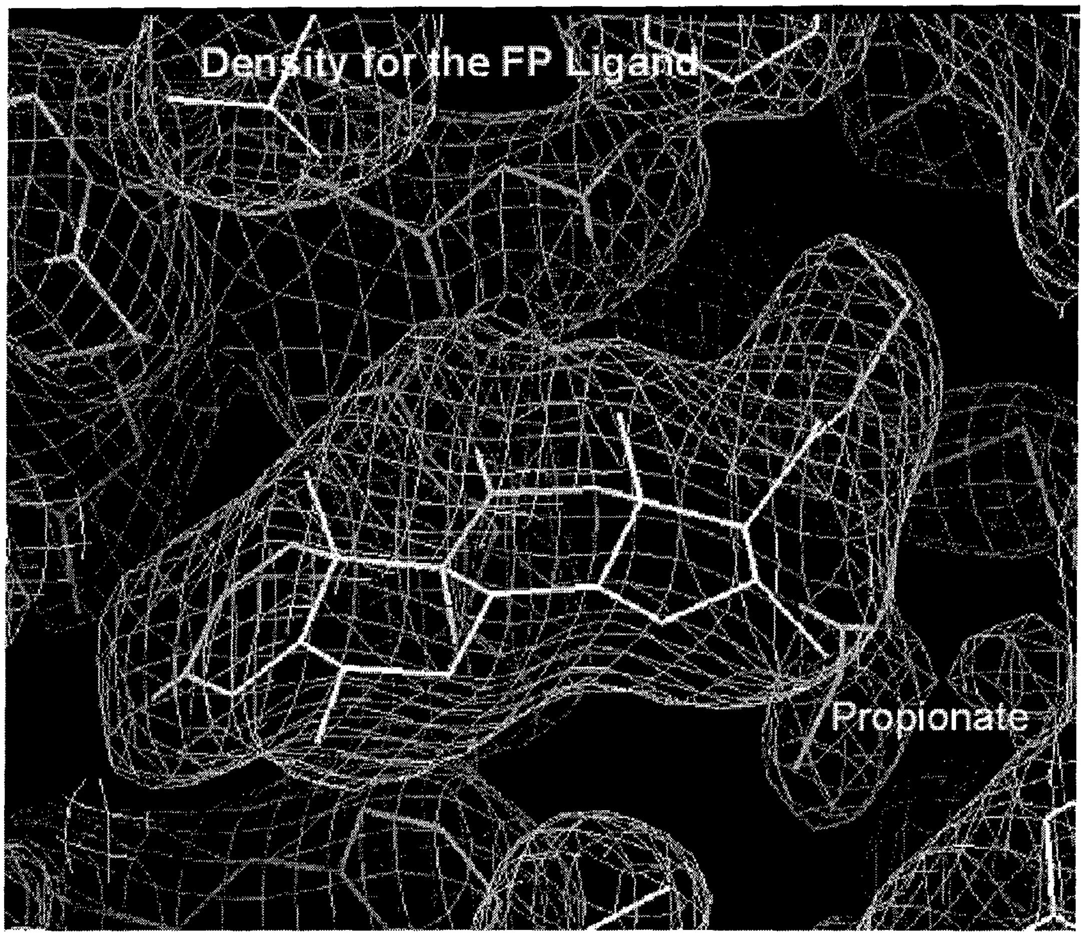


FIGURE 3

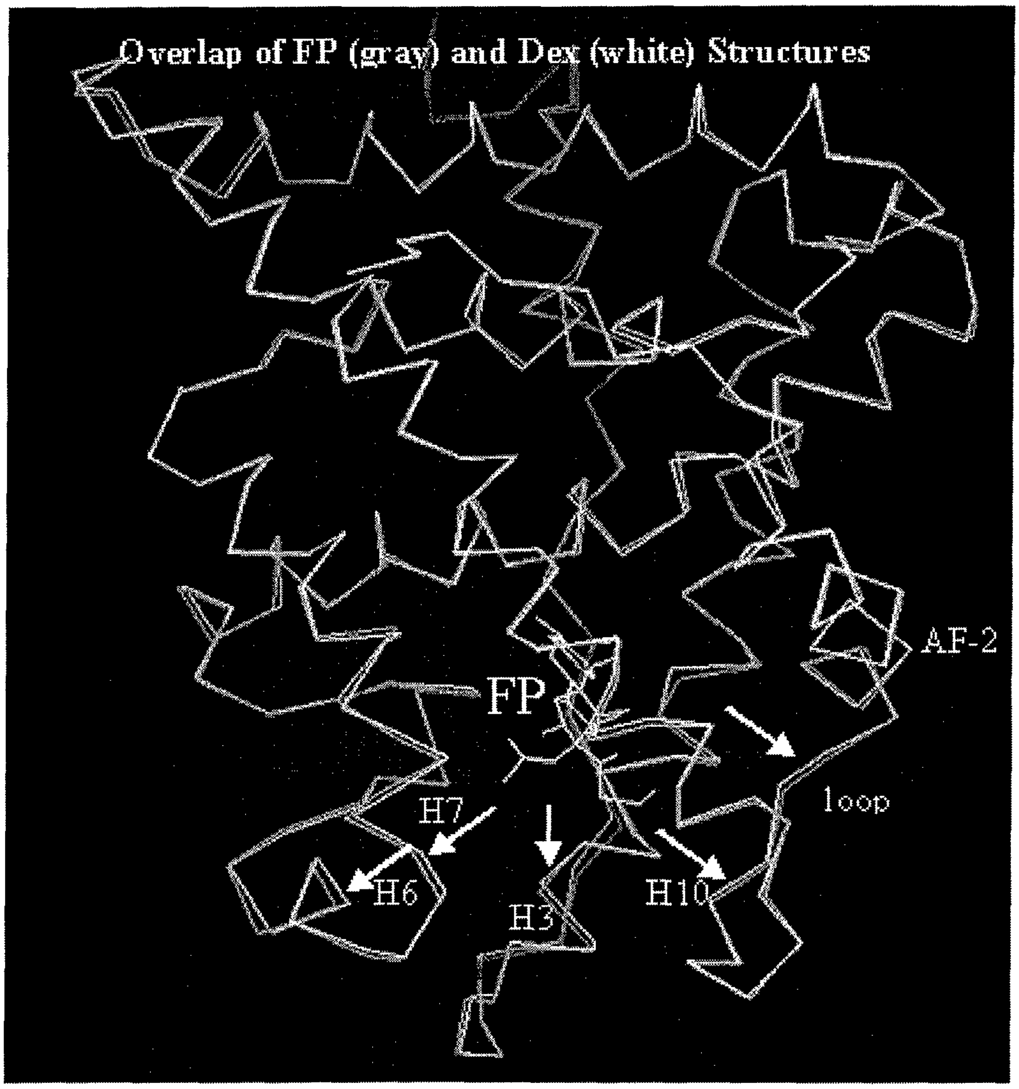
Overlap of FP (gray) and Dex (white) Structures
AF-2
FP
loop
H7
H6
H3
H10

FIGURE 4

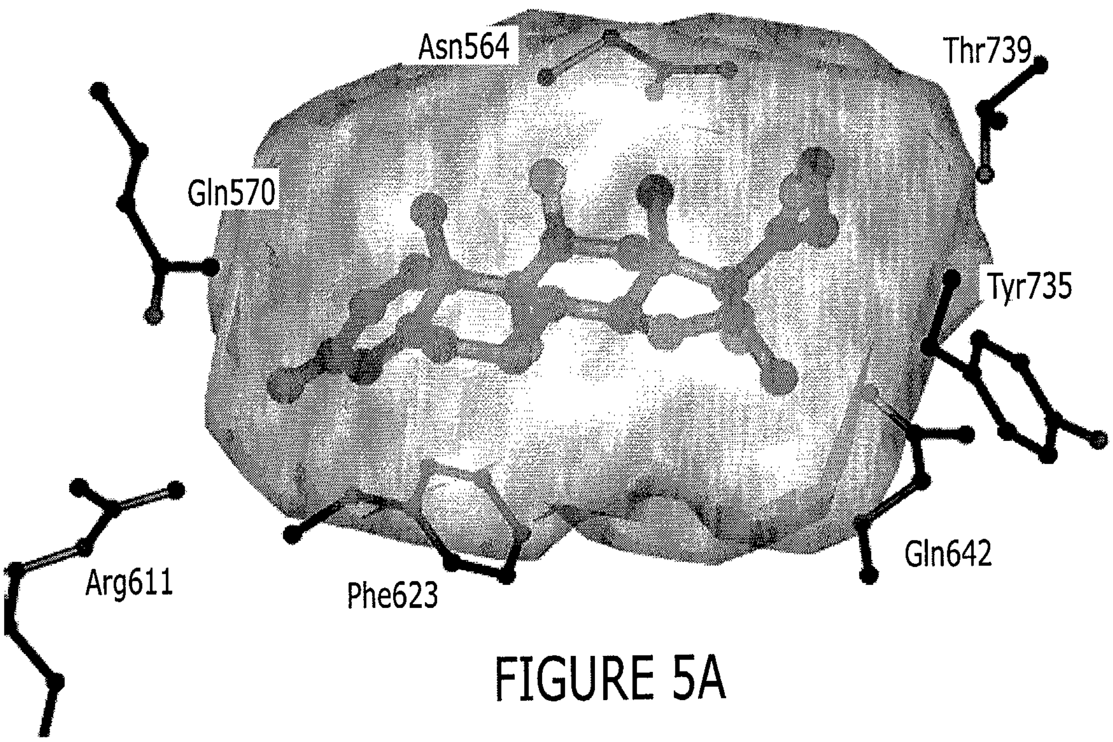


FIGURE 5A

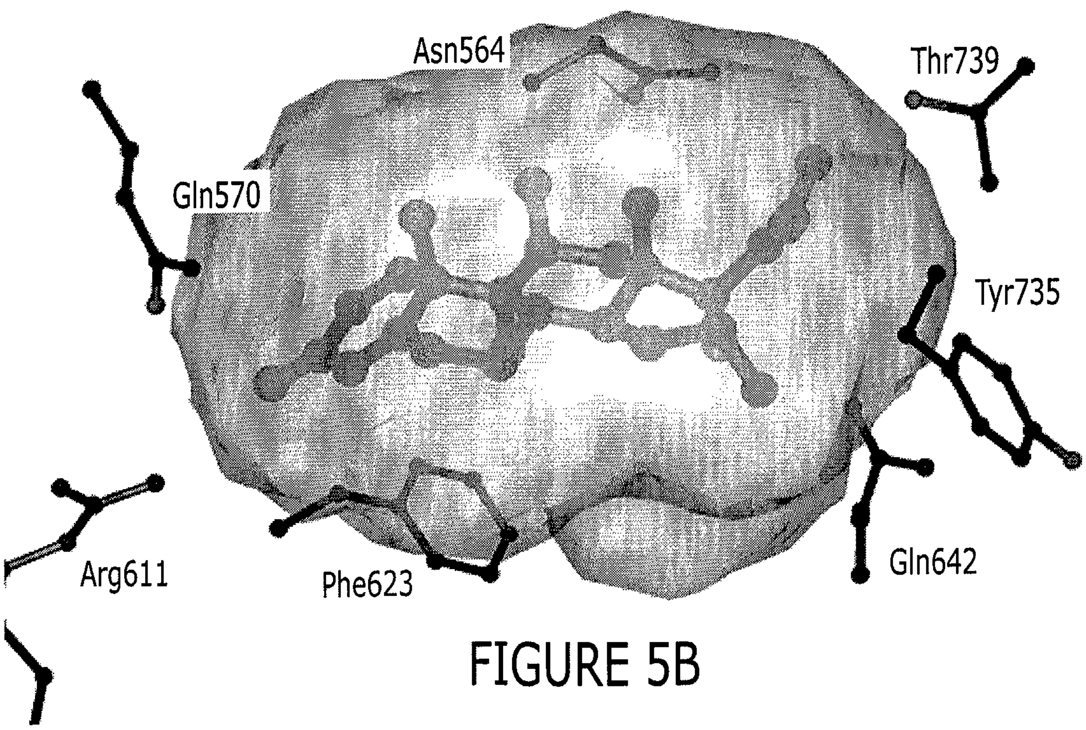


FIGURE 5B

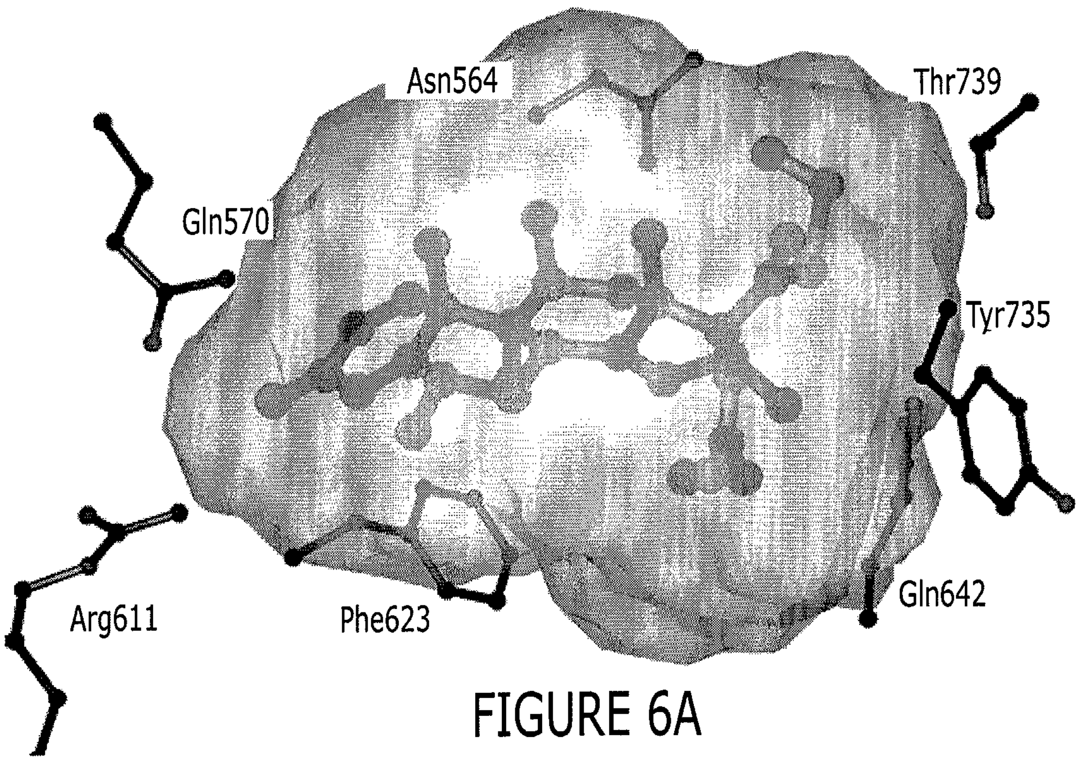


FIGURE 6A

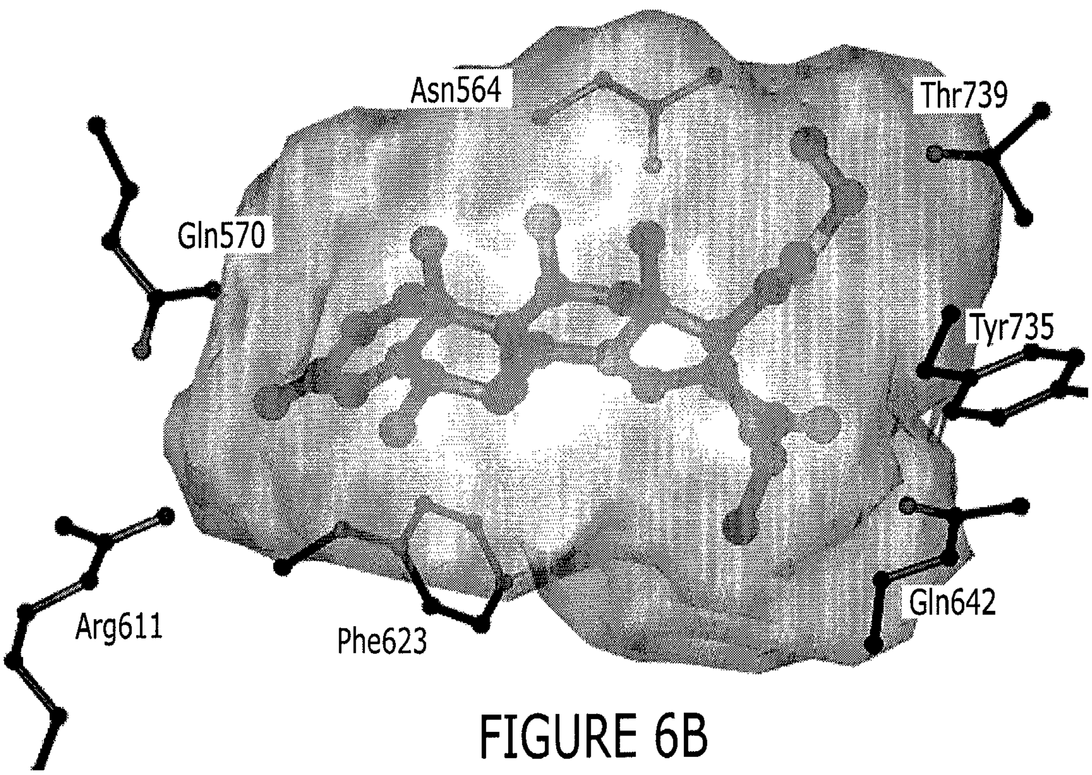


FIGURE 6B

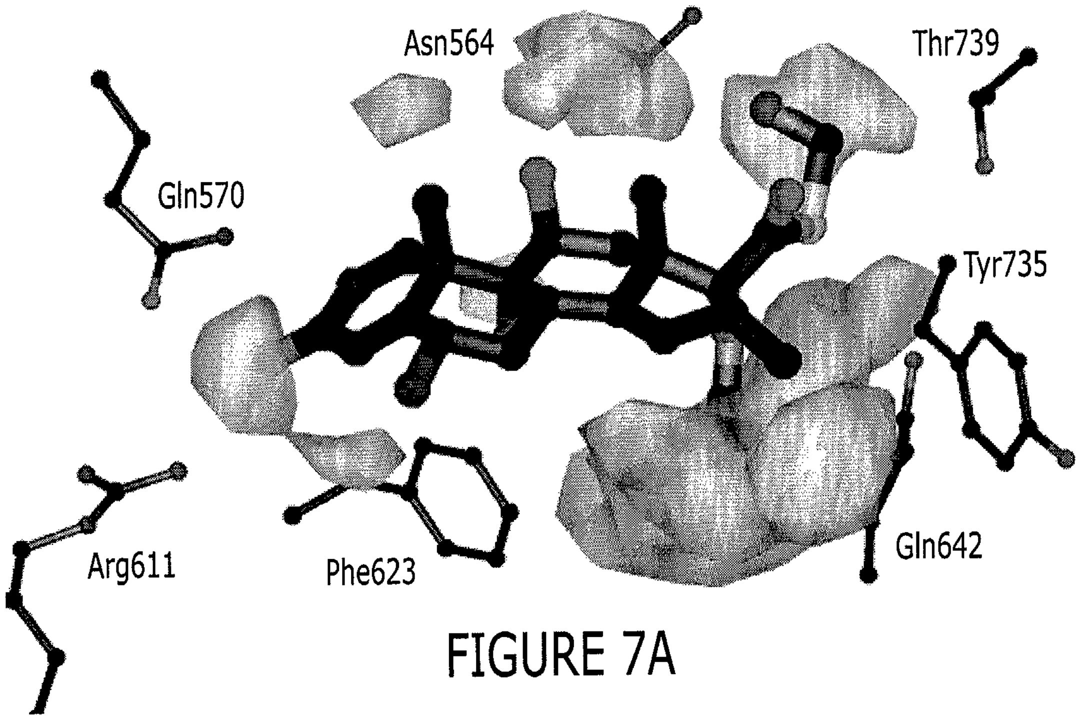


FIGURE 7A

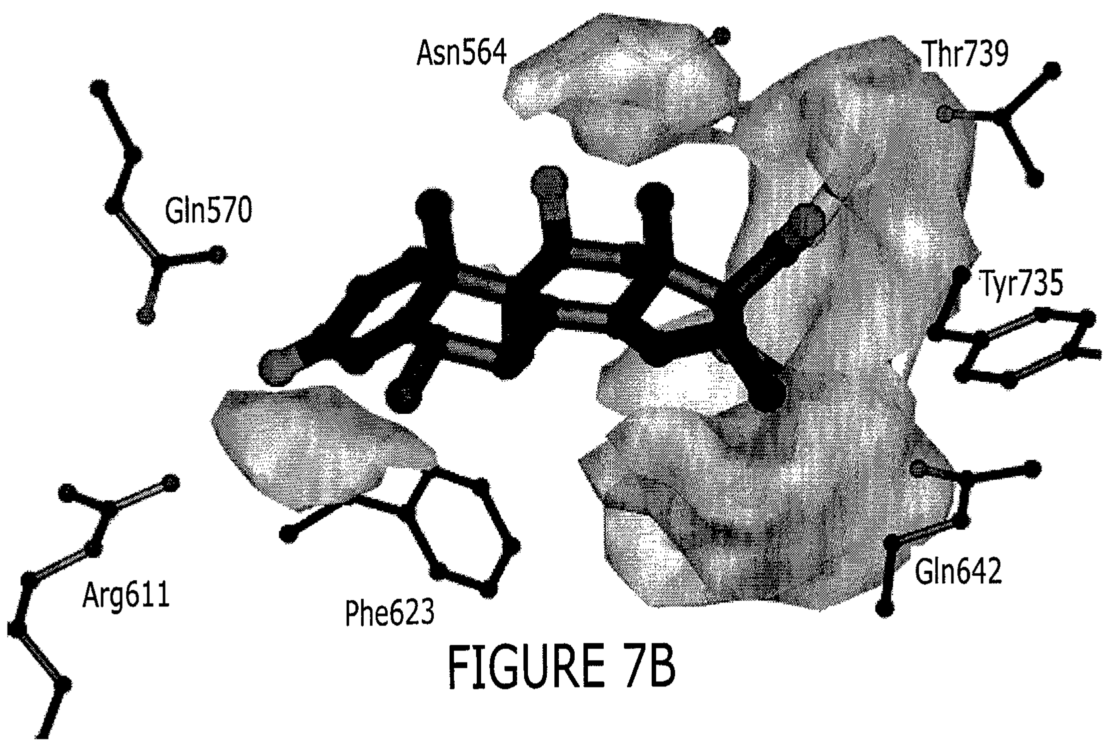


FIGURE 7B

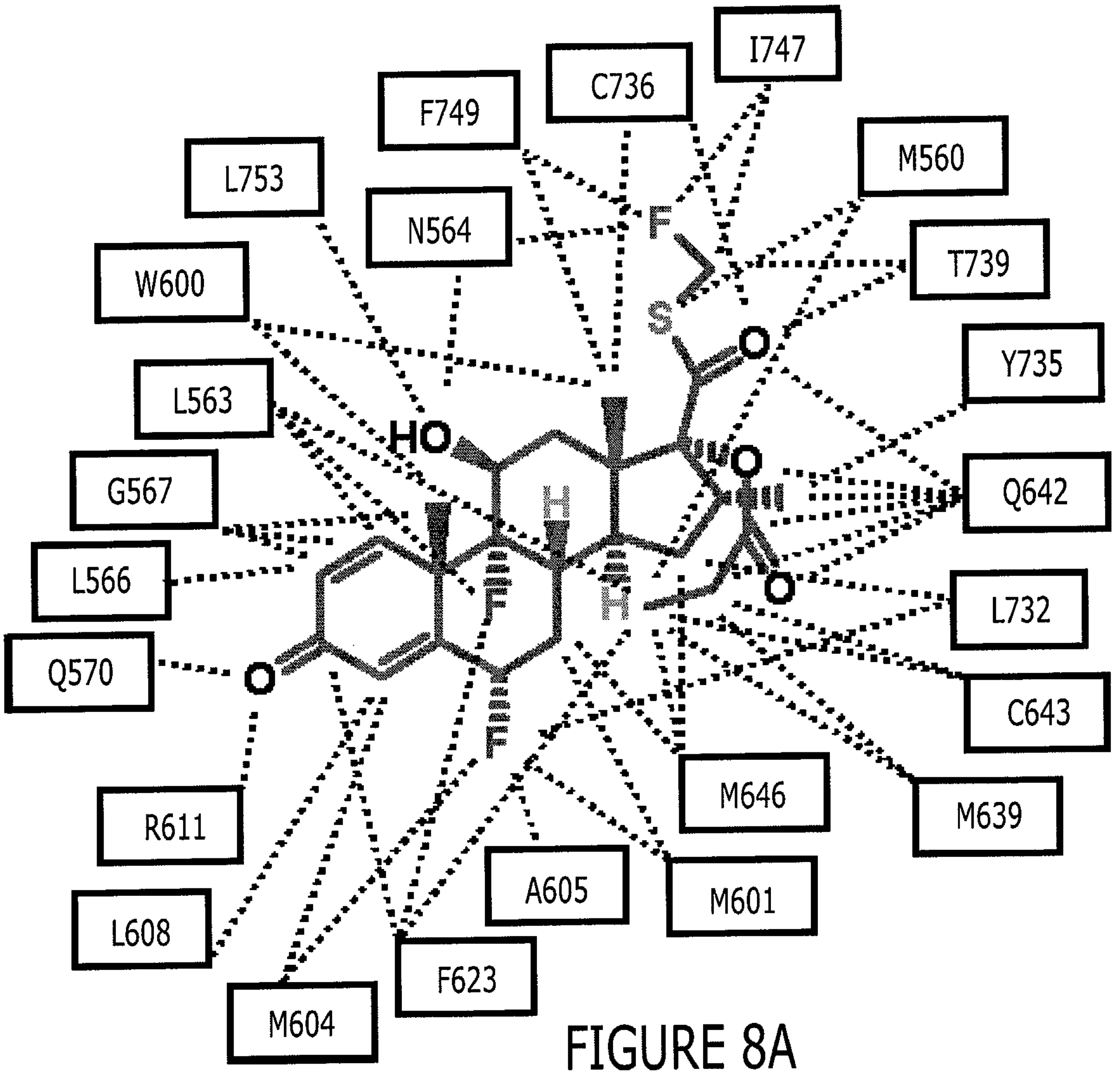
I747
C736
F749
M560
L753
N564
T739
W600
Y735
L563
G567
Q642
L566
L732
Q570
C643
M646
M639
R611
A605
M601
L608
F623
M604

FIGURE 8A

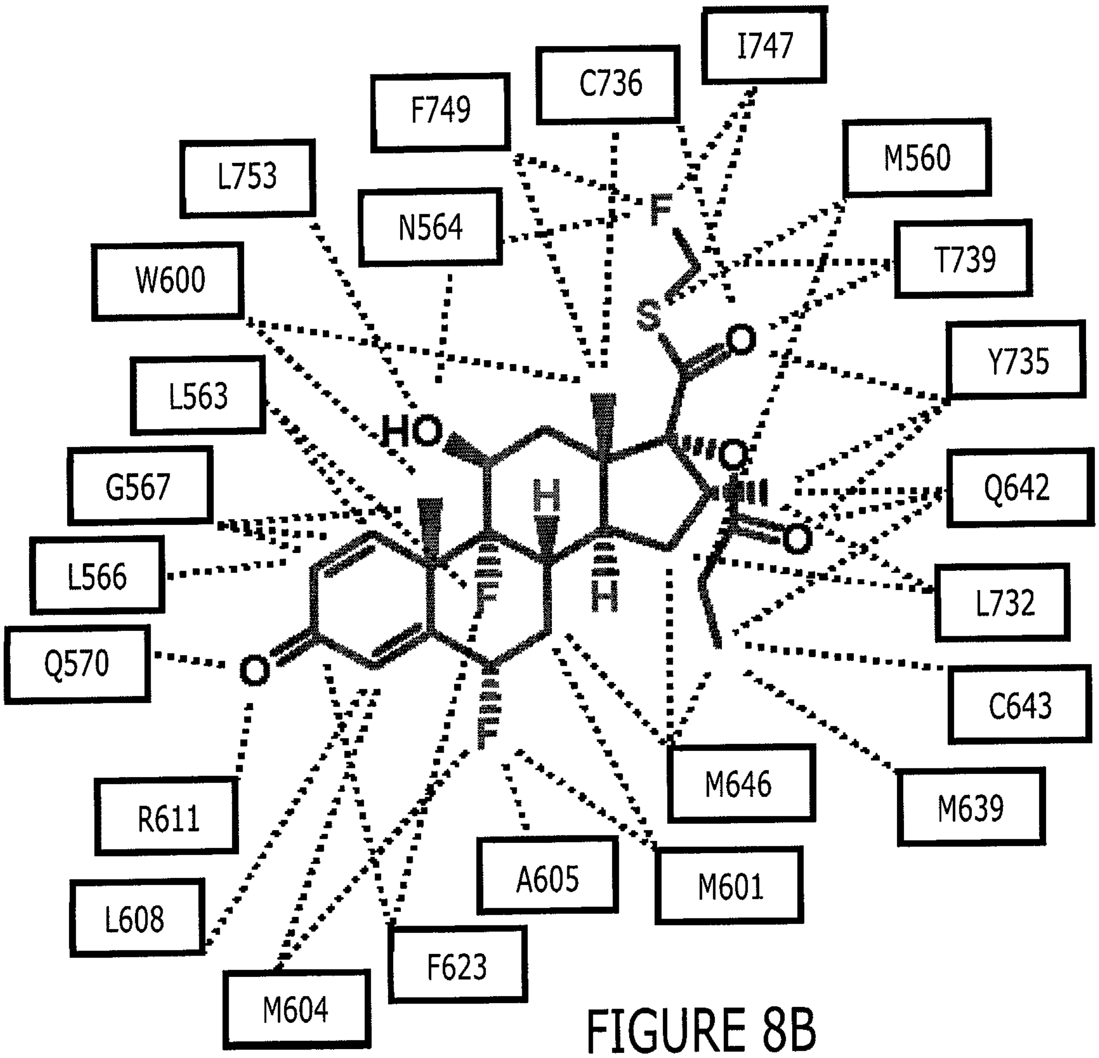
I747
C736
F749
M560
L753
N564
T739
W600
Y735
L563
HO
G567
Q642
L566
L732
Q570
C643
M646
M639
R611
A605
M601
L608
F623
M604

FIGURE 8B

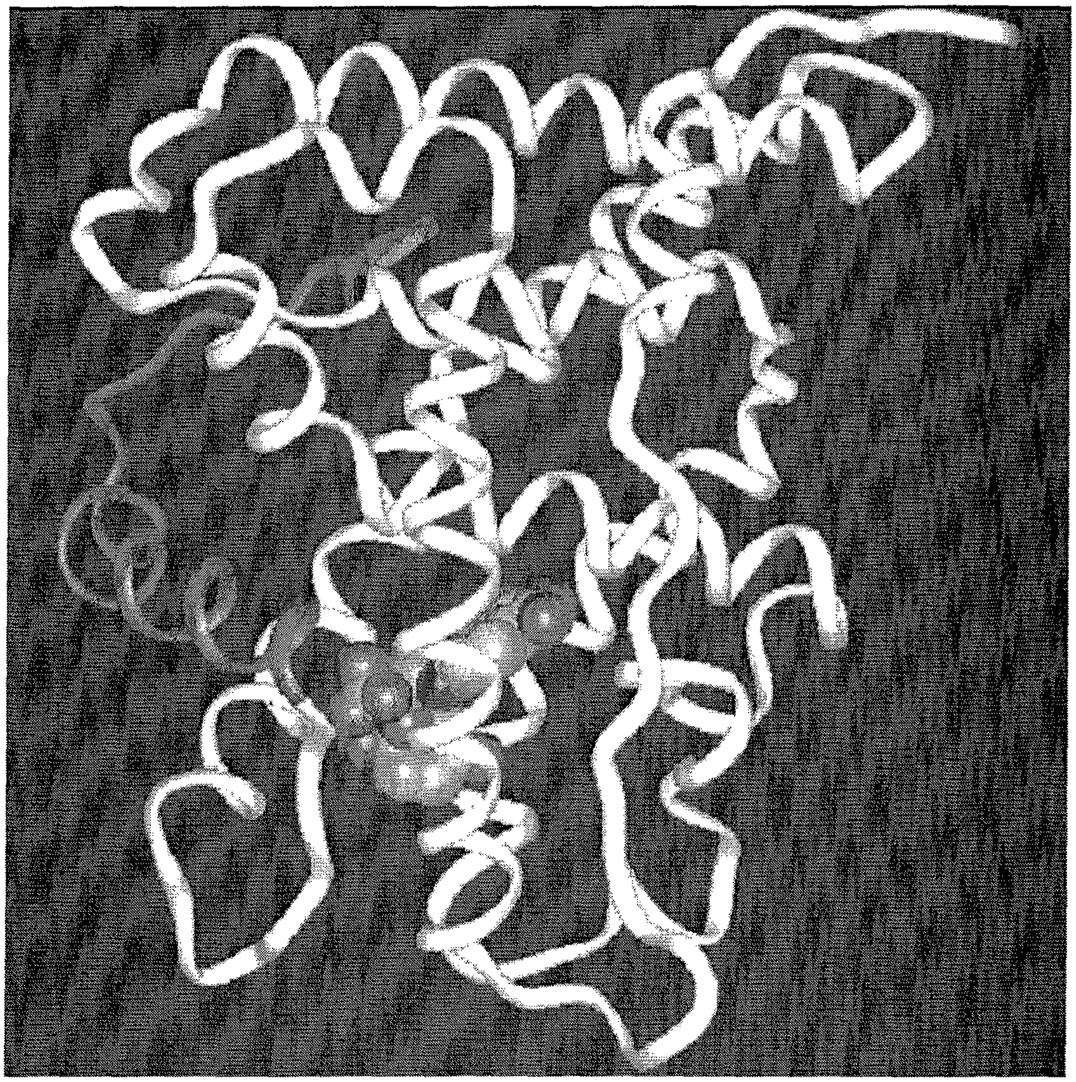

FIGURE 9

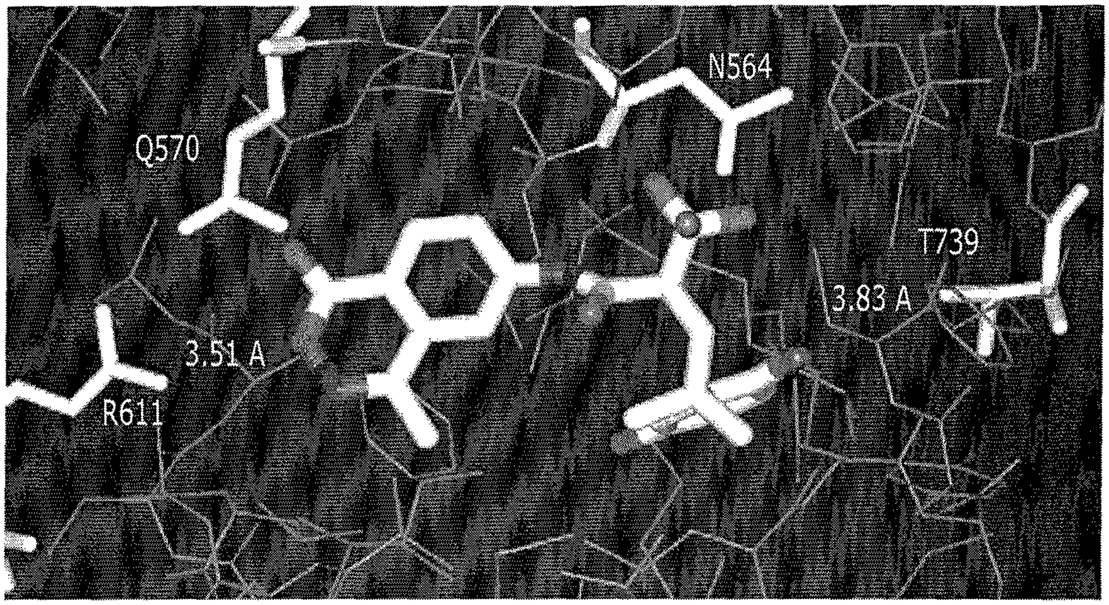


FIGURE 10

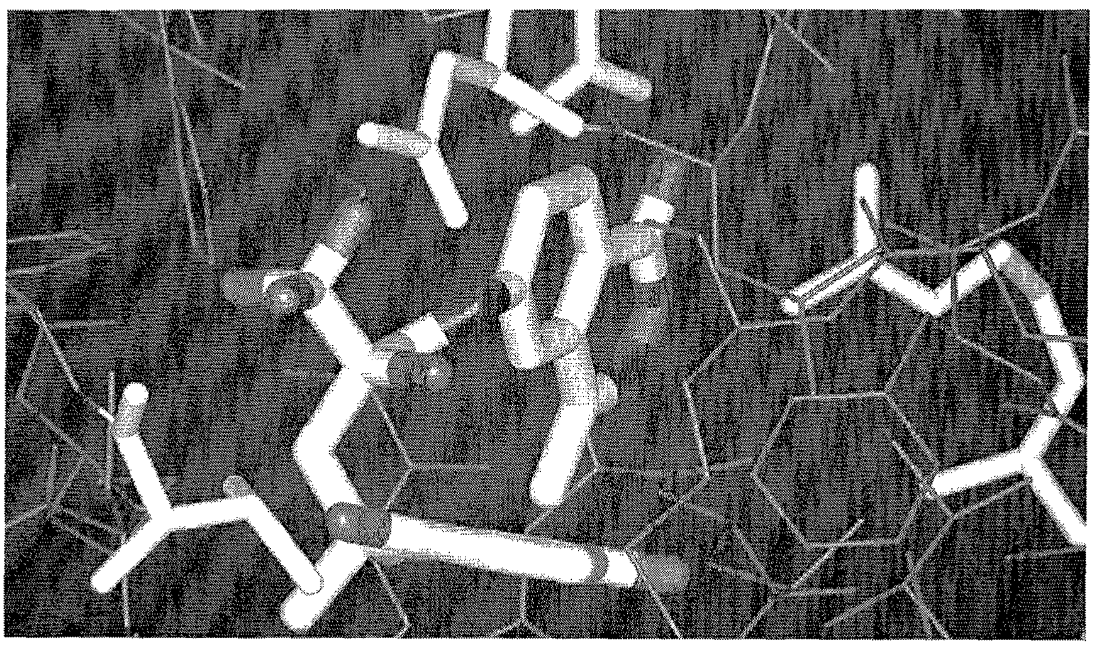

FIGURE 11

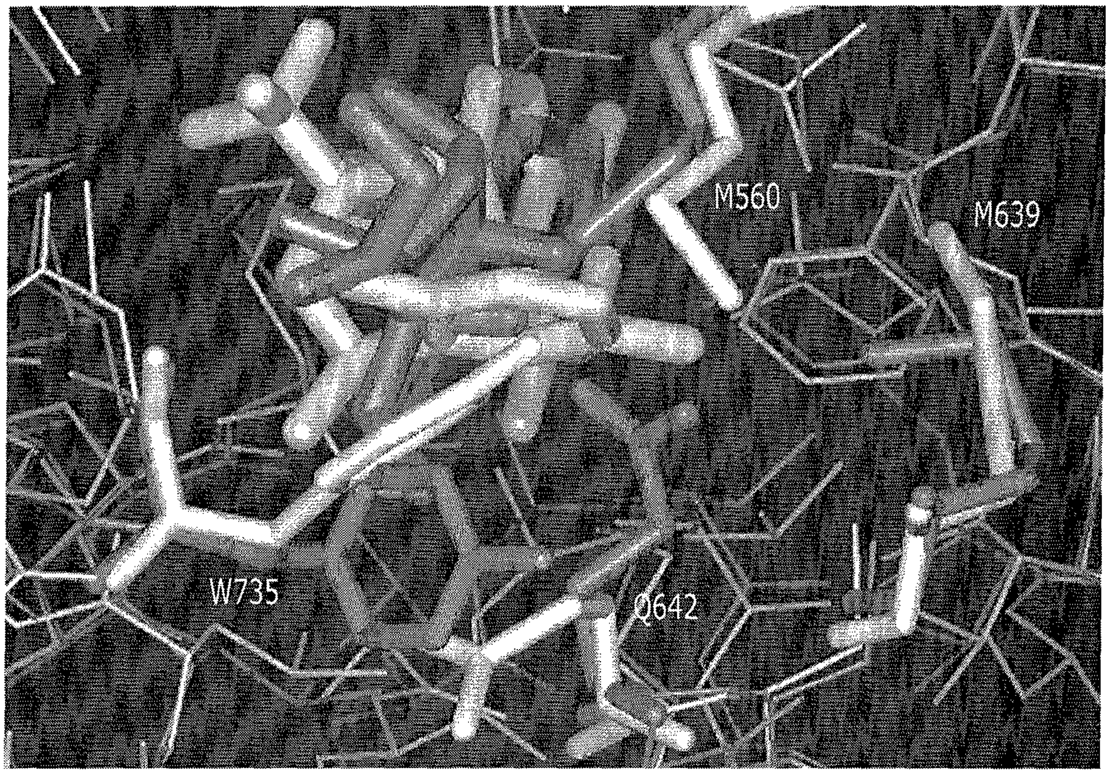


FIGURE 12

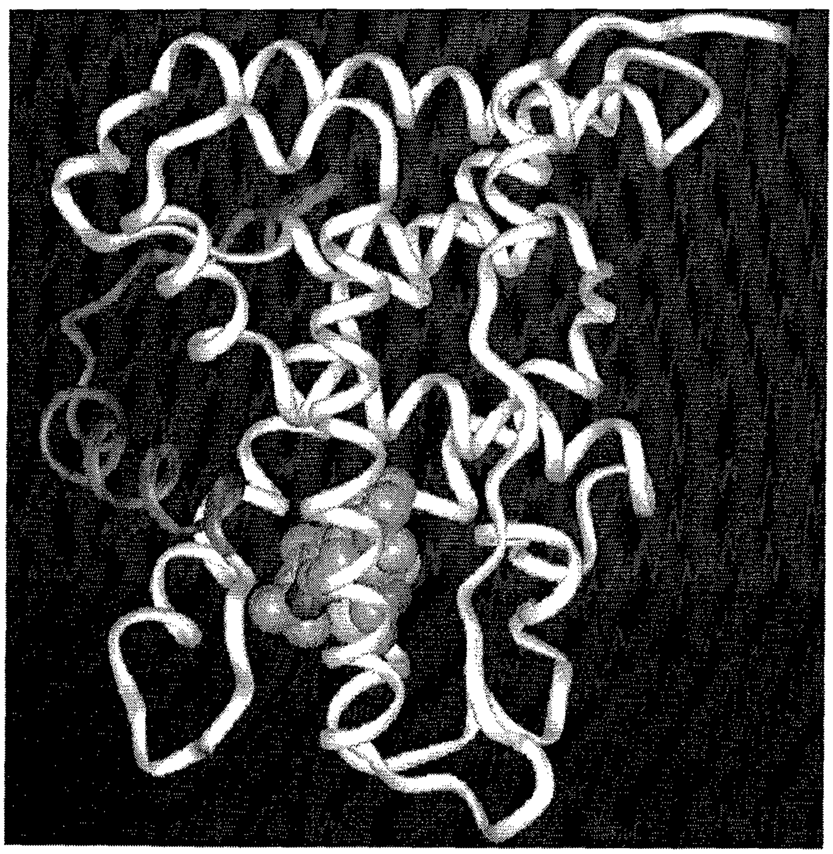

FIGURE 13

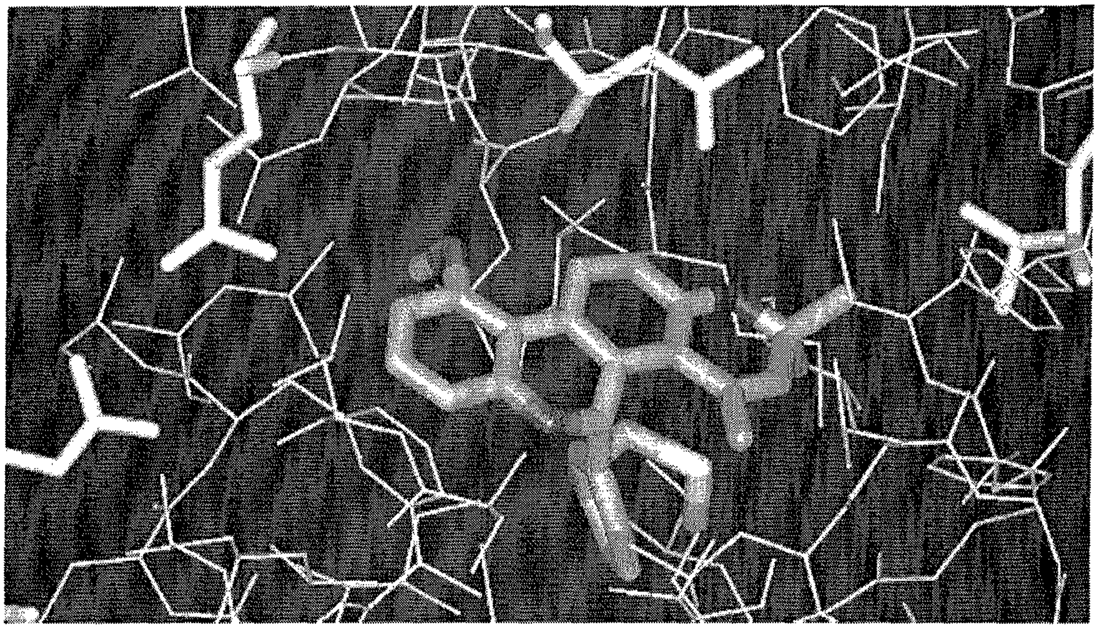

FIGURE 14

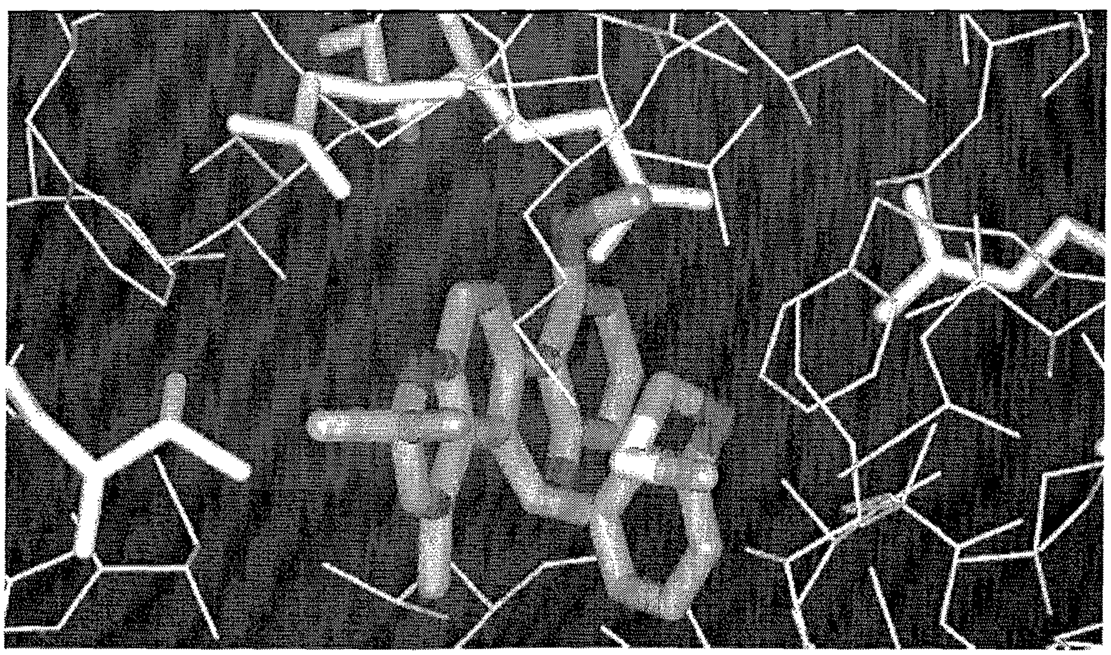

FIGURE 15

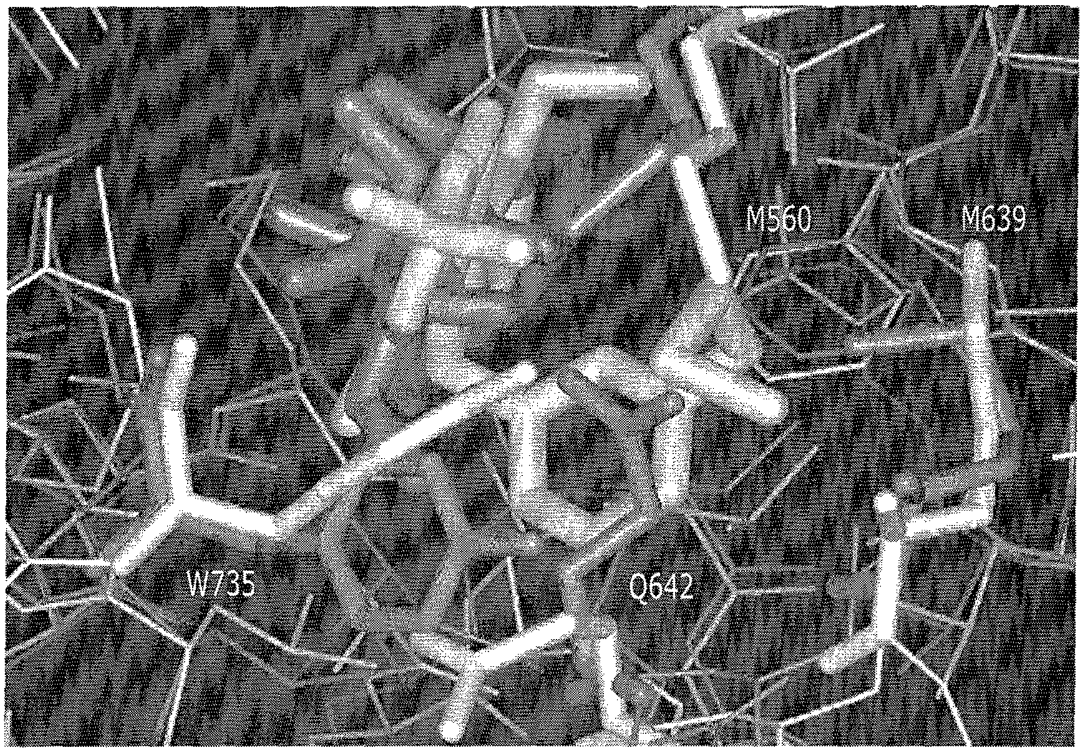


FIGURE 16

```
GR 527  QLTPTLVSLLEVIEPEVLYAGYDSSVPDSTWRIMTTL
MR 733  ALTPSPVMVLENIEPEIVYAGYDSSKPDTAENLLSTL
PR 682  QLIPPLINLLMSIEPDVIYAGHDNTKPDTSSSLLTSL
AR 668  ECQPIFLNVLEAIEPGVVCAGHDNNQPDSFAALLSSL
            helix-1

GR 564  NMLGGRQVIAAVKWAKAIPGFRNLHLDDQMTLLQYSW
MR 770  NRLAGKQMIQVVKWAKVLPGFKNLPLEDQITLIQYSW
PR 719  NQLGERQLLSVVKWSKSLPGFRNLHIDDQITLIQYSW
AR 705  NELGERQLVHVVKWAKALPGFRNLGVDDQMAVIQYSW
          helix-3           helix-3'  helix-4

GR 601  MSIMAFALGWRSYRQSSANLLCFAPDLIINEQRMTLP
MR 807  MCLSSFALSWRSYKHTNSQFLYFAPDLVFNEEKMHQS
PR 756  MSIMVFGLGWRSYKHVSGQMLYFAPDLILNEQRMKES
AR 742  MGLMVFAMGWRSFTNVNSRMLYFAPDLVFNEYRMHKS
            helix-5          beta-3 beta-4 helix-6

GR 638  CMYDQCKHMLYVSSELHRLQVSYEEYLCMKTLLLLSS
MR 844  AMYELCQGMHQISLQFVRLQLTFEEYTIMKVLLLLST
PR 793  SFYSLCLTMWQIPQEFVKLQVSQEEFLCMKVLLLLNT
AR 779  RMYSQCVRMRHLSQEFGWLQITPQEFLCMKALLLFSI
               helix-7             helix-8

GR 675  VPKDGLKSQELFDEIRMTYIKELGKAIVKREGNSSQN
MR 881  IPKDGLKSQAAFEEMRTNYIKELRKMVTKCPNNSGQS
PR 830  IPLEGLRSQTQFEEMRSSYIRELIKAIGLRQKGVVSS
AR 816  IPVDGLKNQKFFDELRMNYIKELDRIIACKRKNPTSC
     beta-5             helix-9

GR 712  WQRFYQLTKLLDSMHEVVENLLNYCFQTFLD-KTMSI
MR 918  WQRFYQLTKLLDSMHDLVSDLLEFCFYTFRESHALKV
PR 867  SQRFYQLTKLLDNLHDLVKQLHLYCLNTFIQSRALSV
AR 853  SRRFYQLTKLLDSWQPIARELHQFTFDLLIKSHMVSV
                 helix-10

GR 748  EFPEMLAEIITNQIPKYSNGNIKKLLFHQK
MR 955  EFPAMLVEIISDQLPKVESGNAKPLYFHRK
PR 904  EFPEMMSEVIAAQLPKILAGMVKPLLFHKK
AR 890  DFPEMMAEIISVQVPKILSGKVKPIYFHTQ
            helix-AF       beta-6
```

FIGURE 17

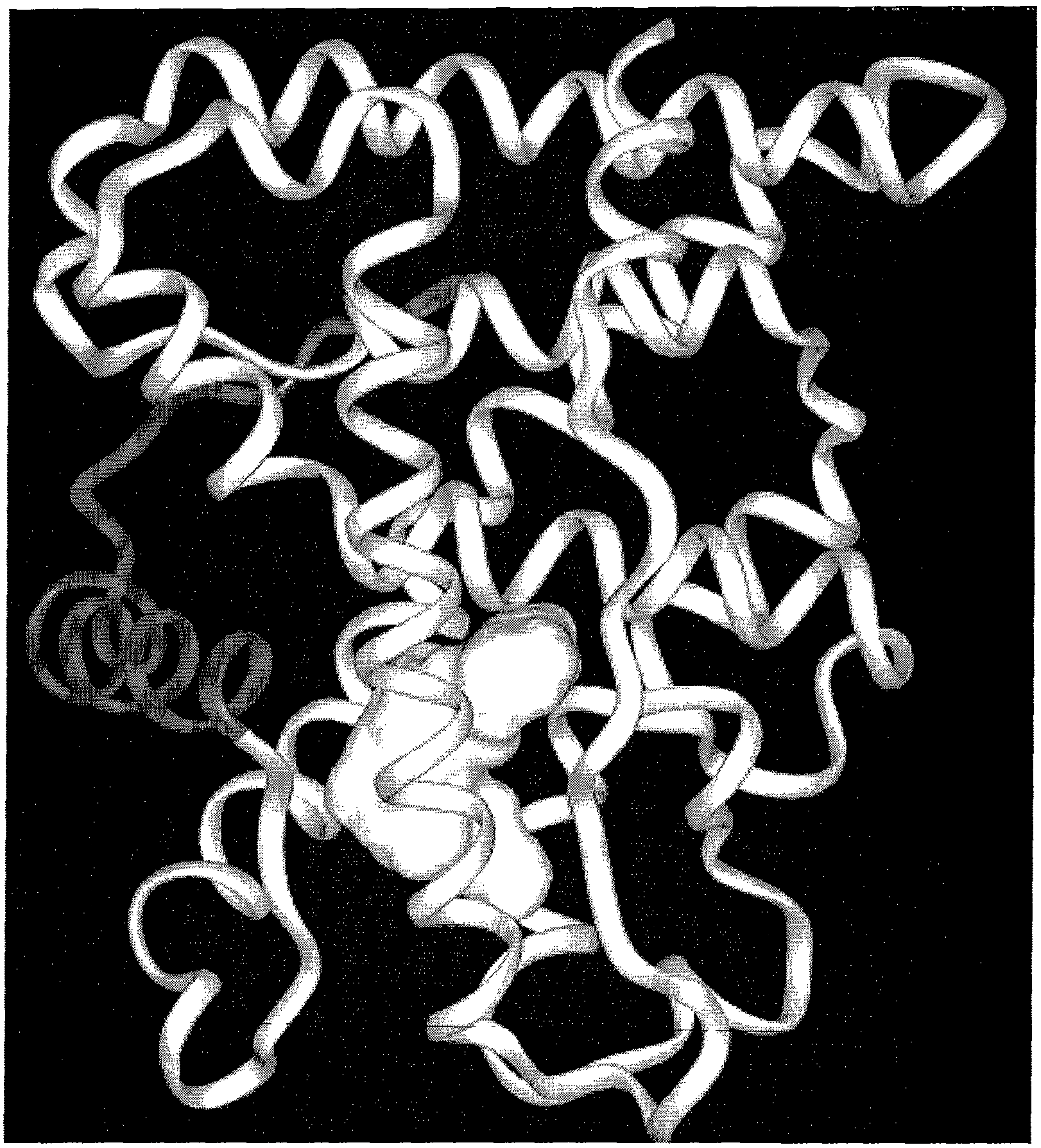

FIGURE 18A

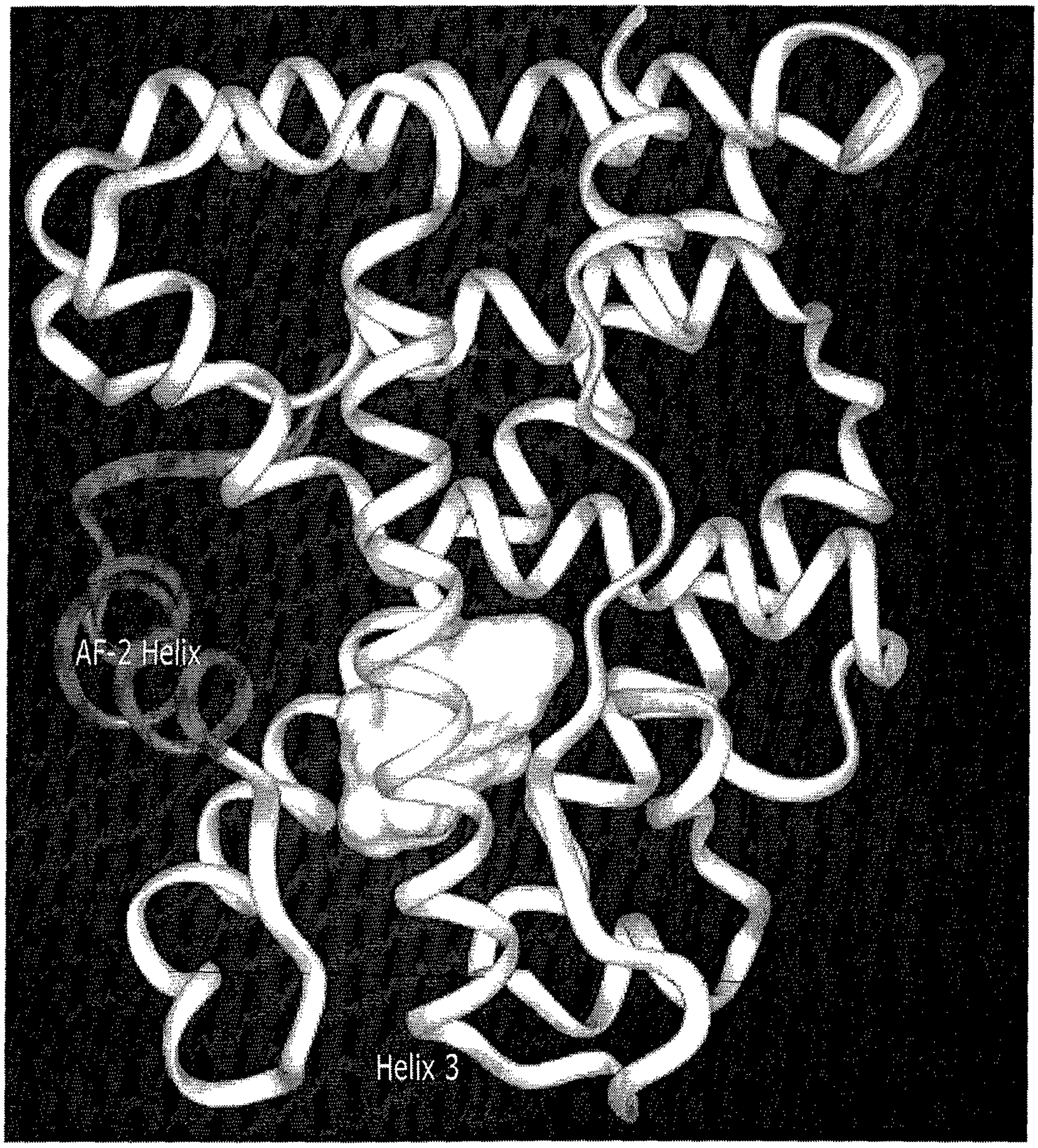


FIGURE 18B

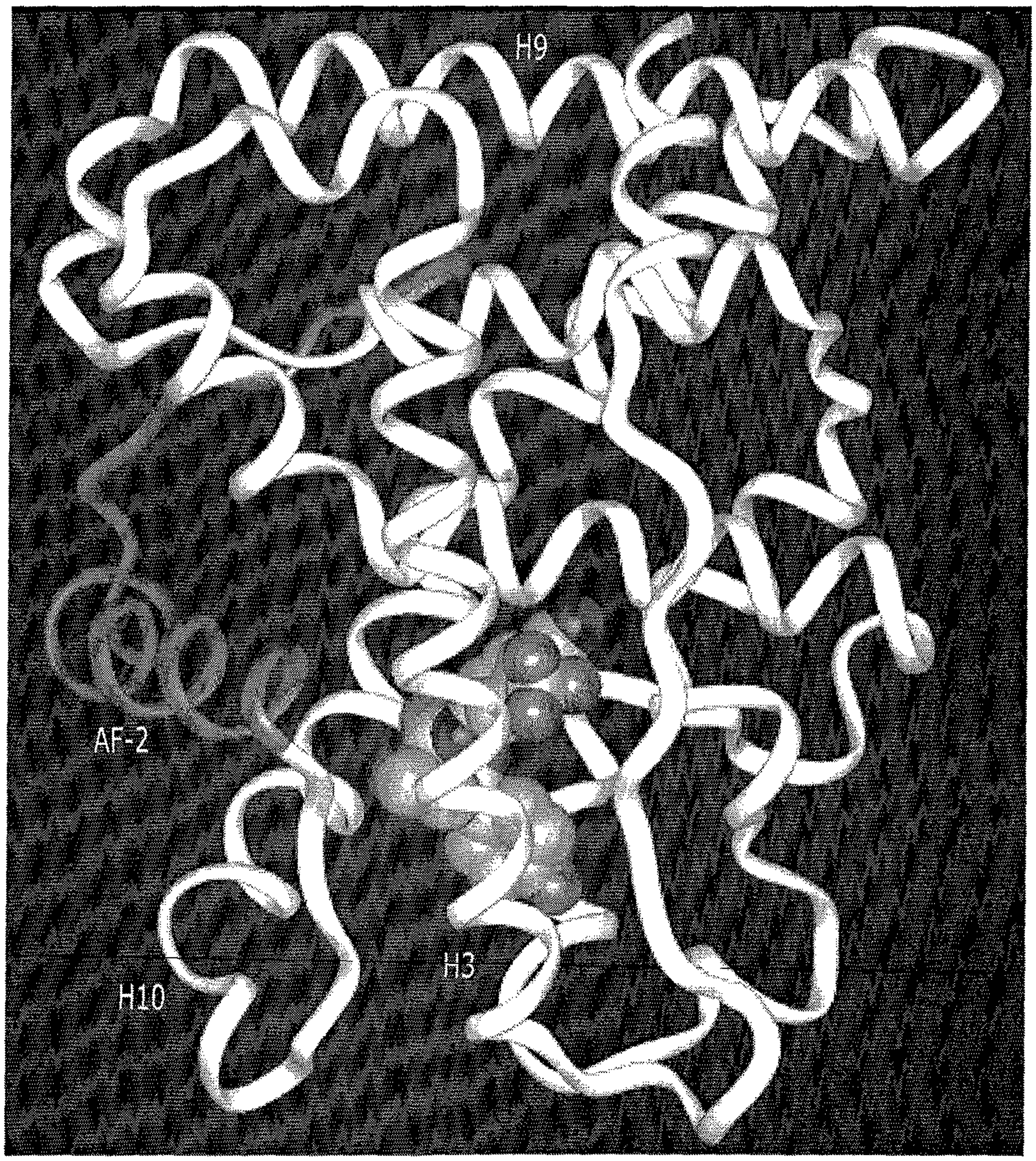


FIGURE 19

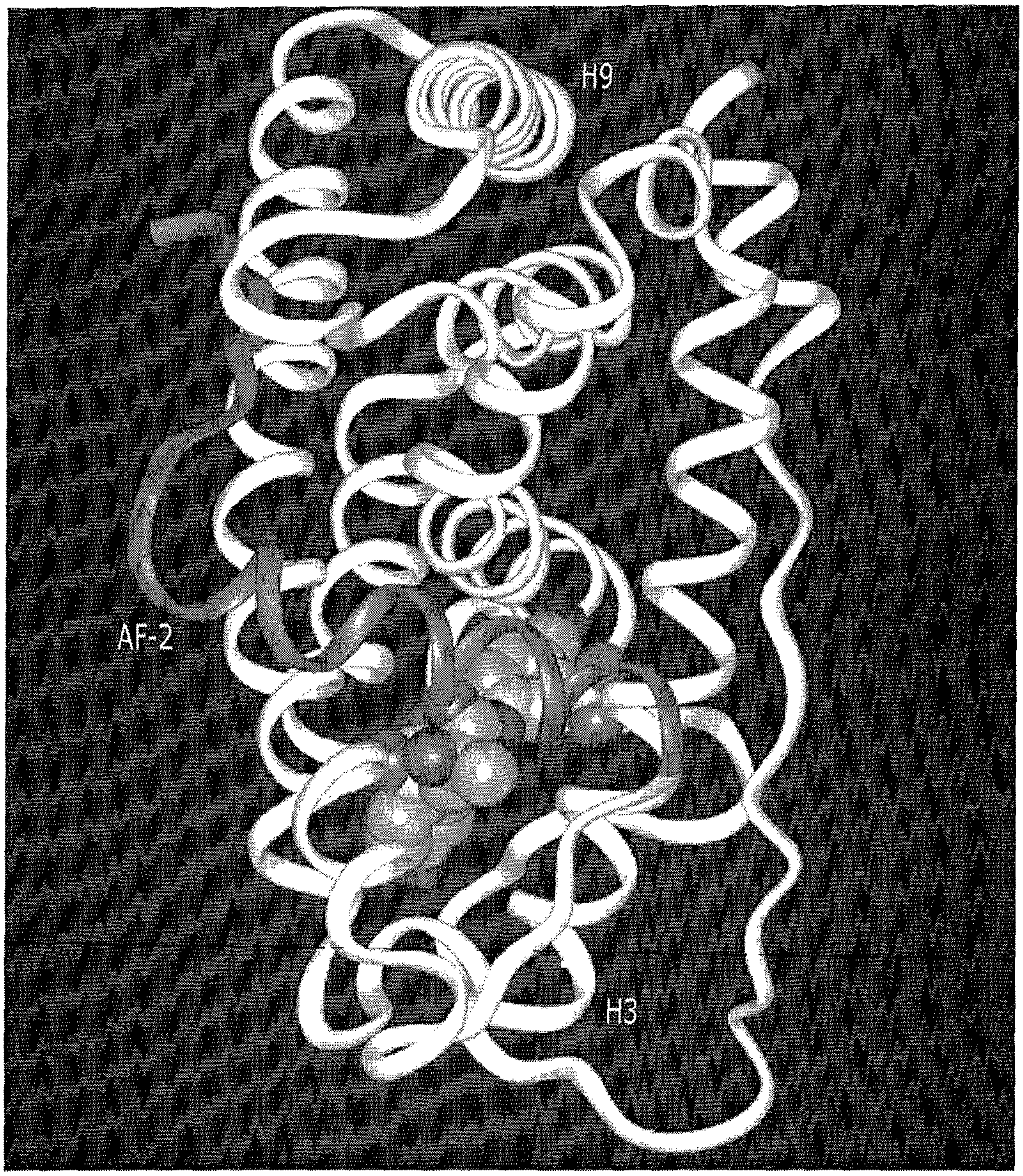


FIGURE 20

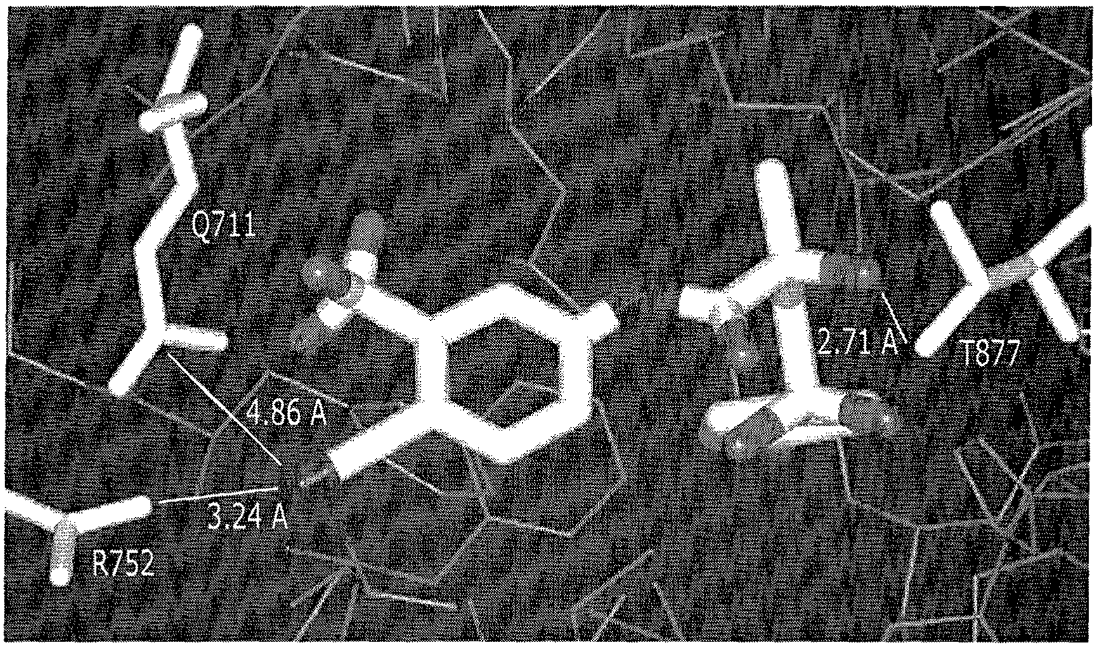


FIGURE 21

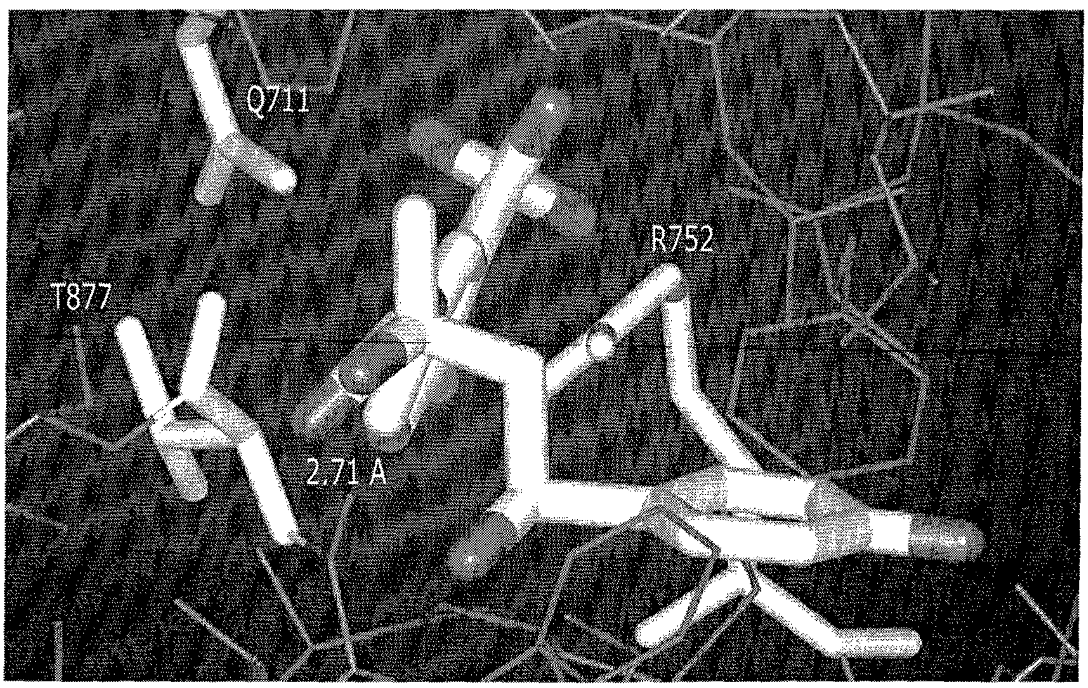


FIGURE 22

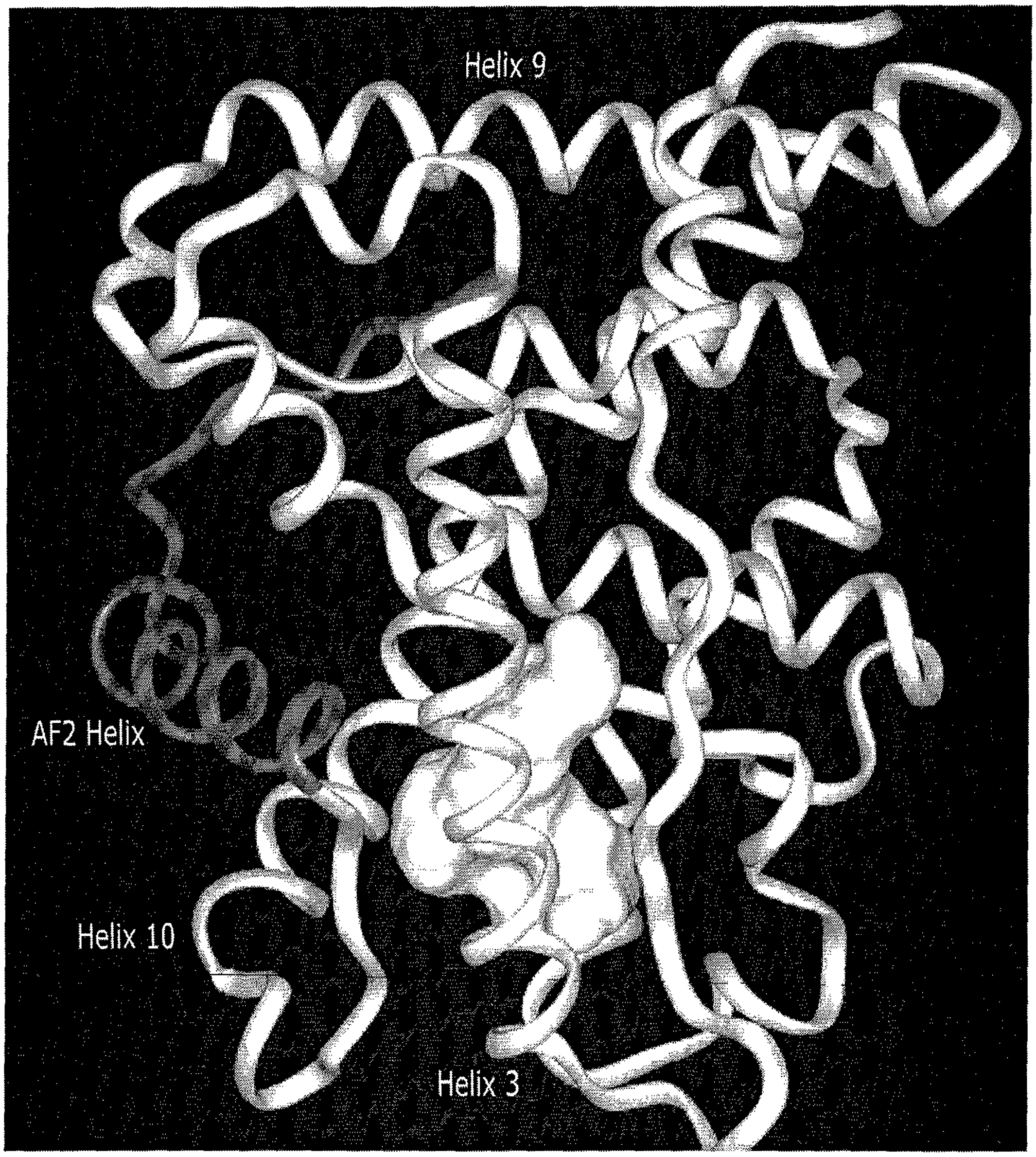


FIGURE 23A

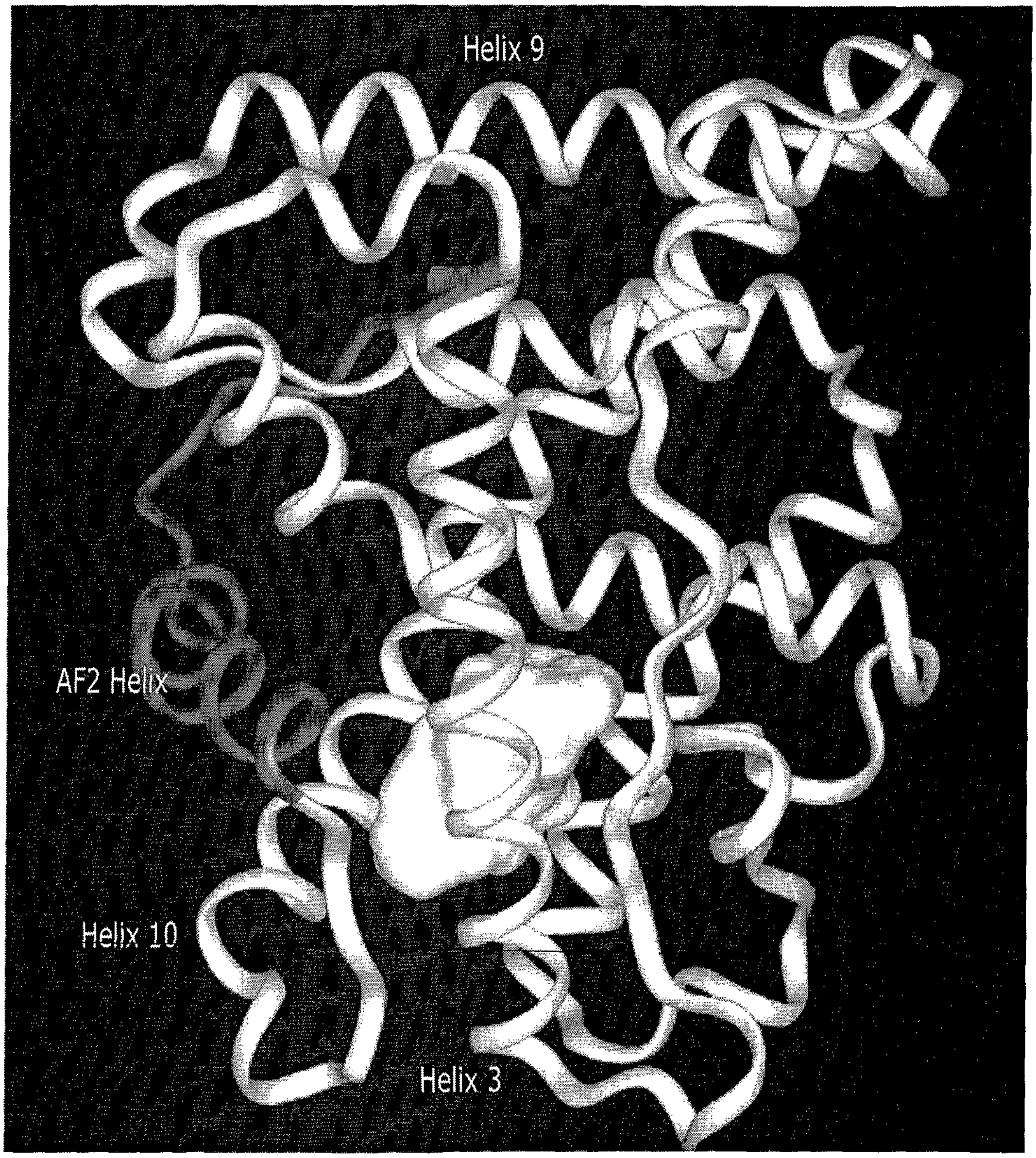


FIGURE 23B

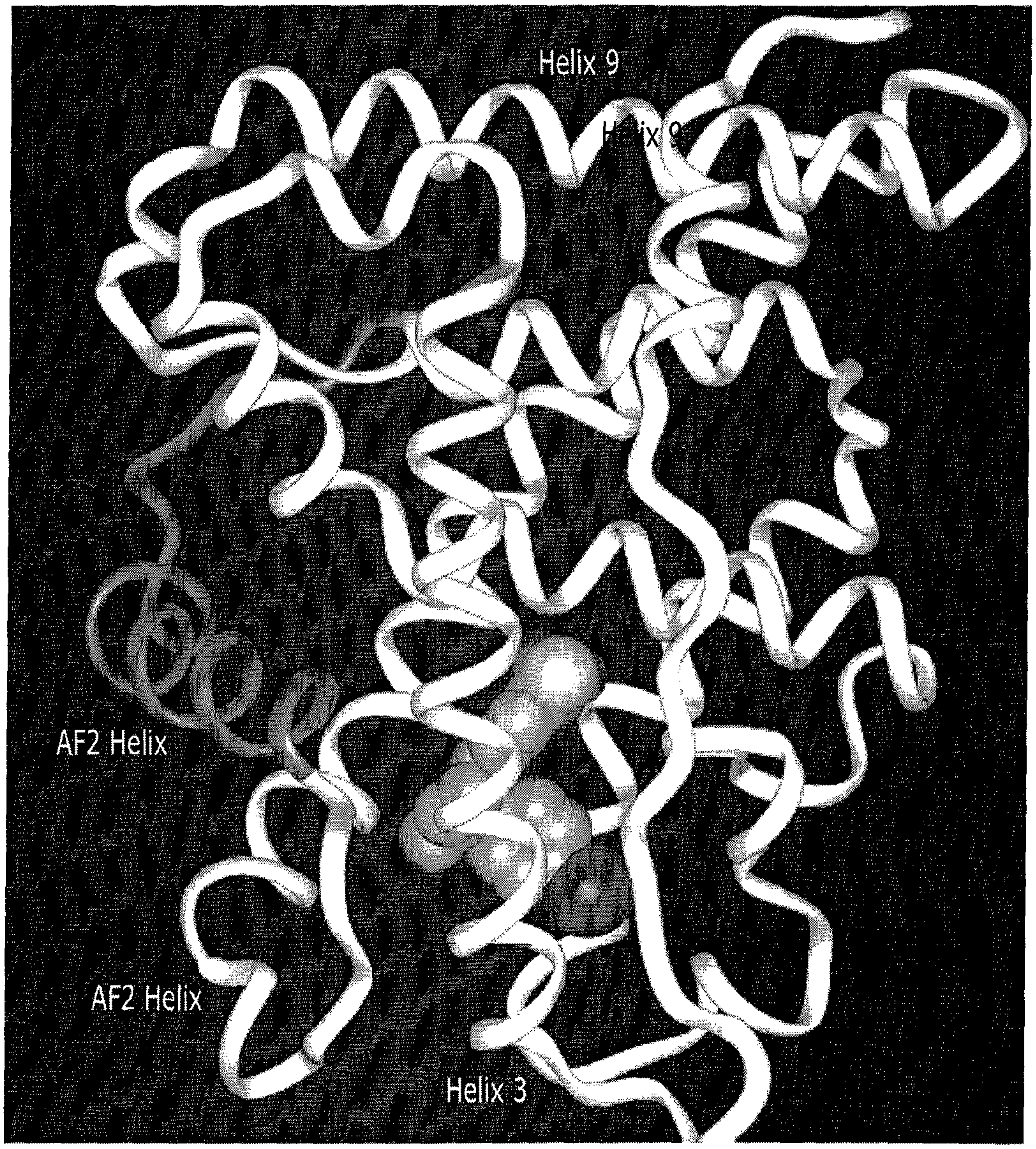


FIGURE 24

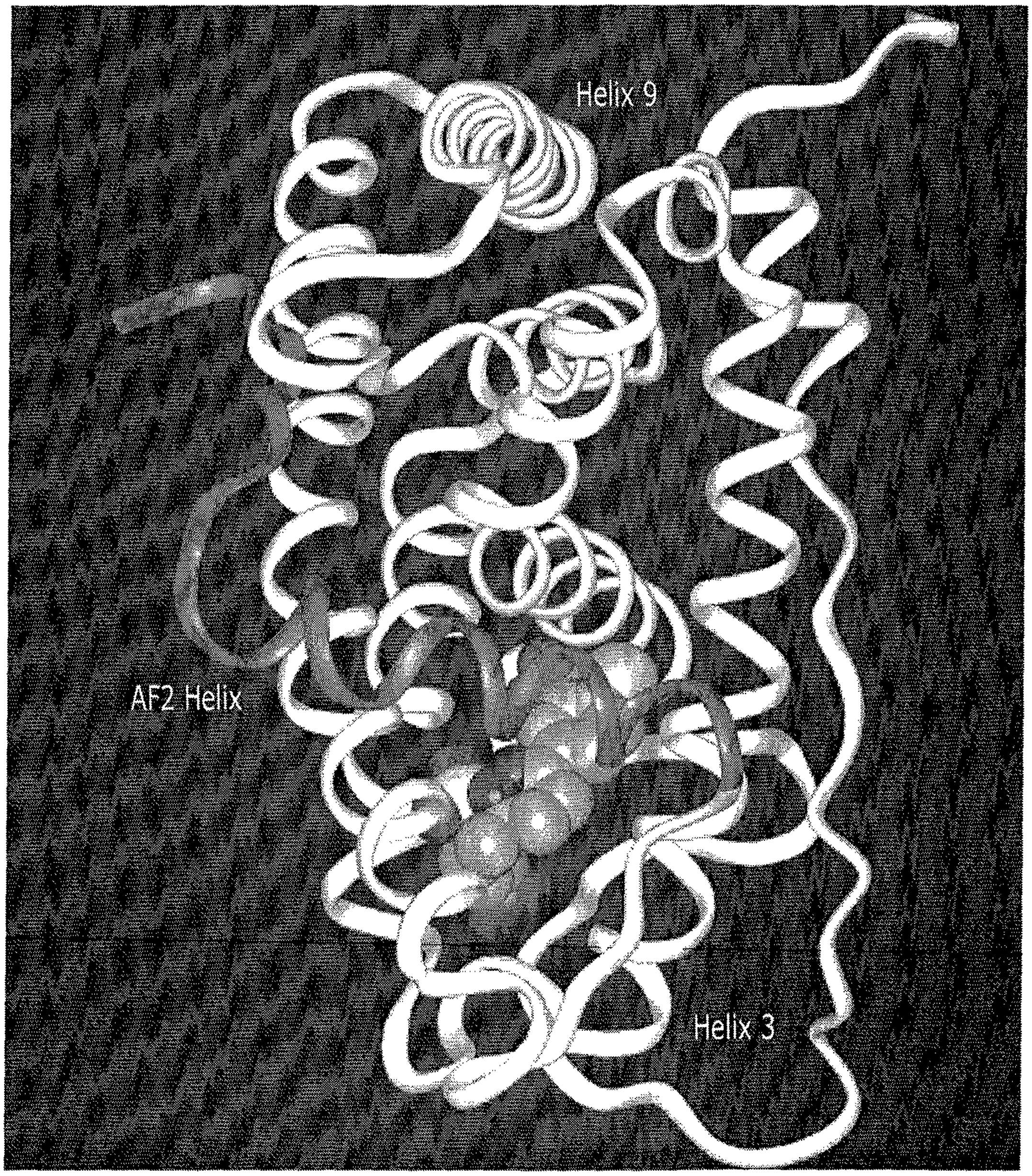


FIGURE 25

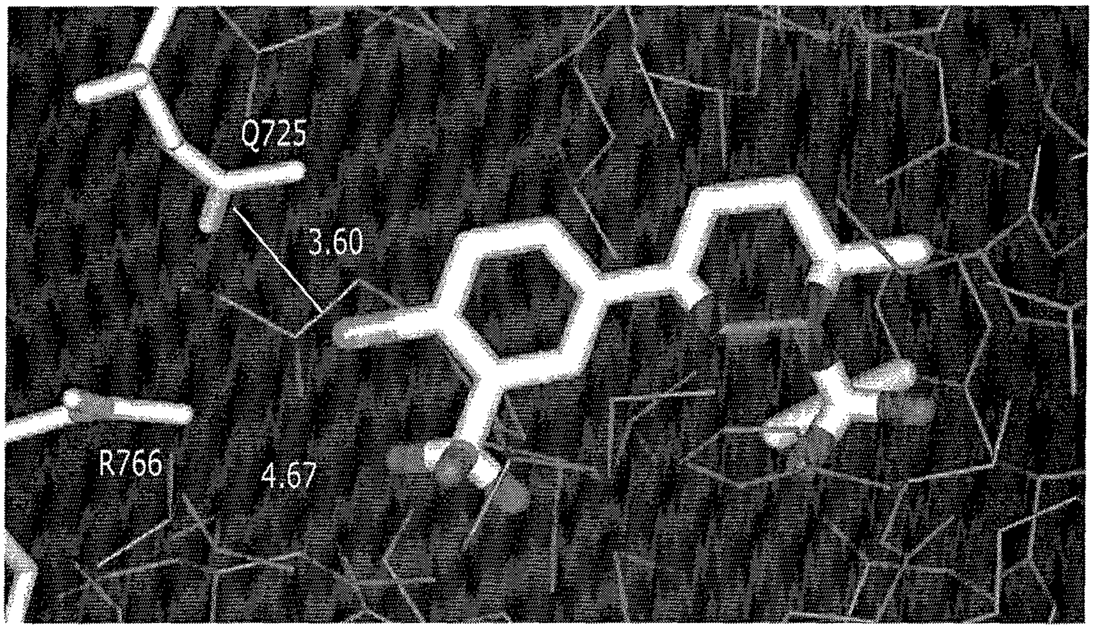


FIGURE 26

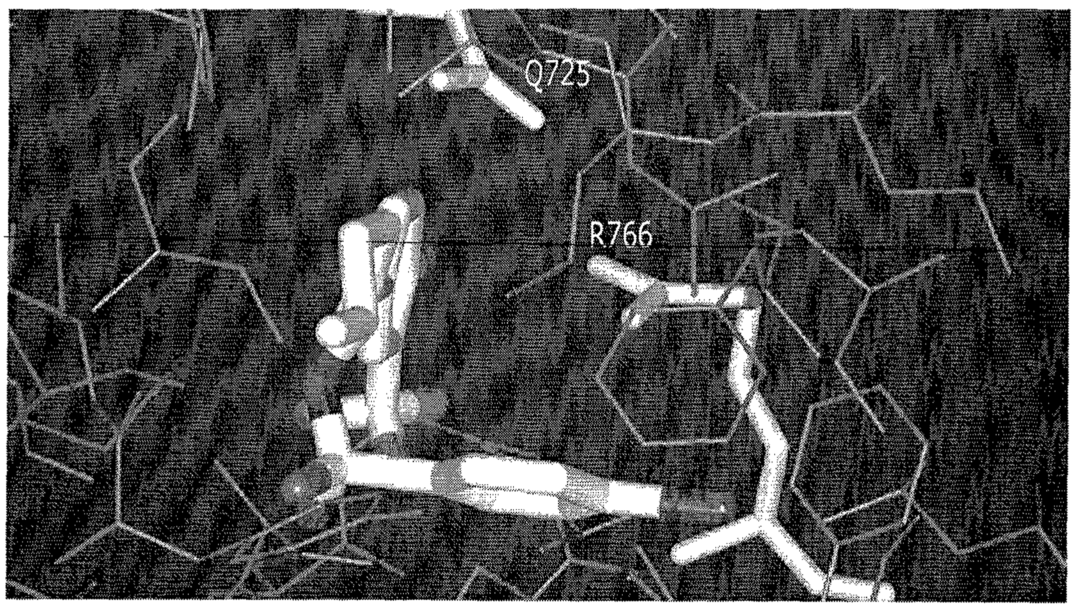


FIGURE 27

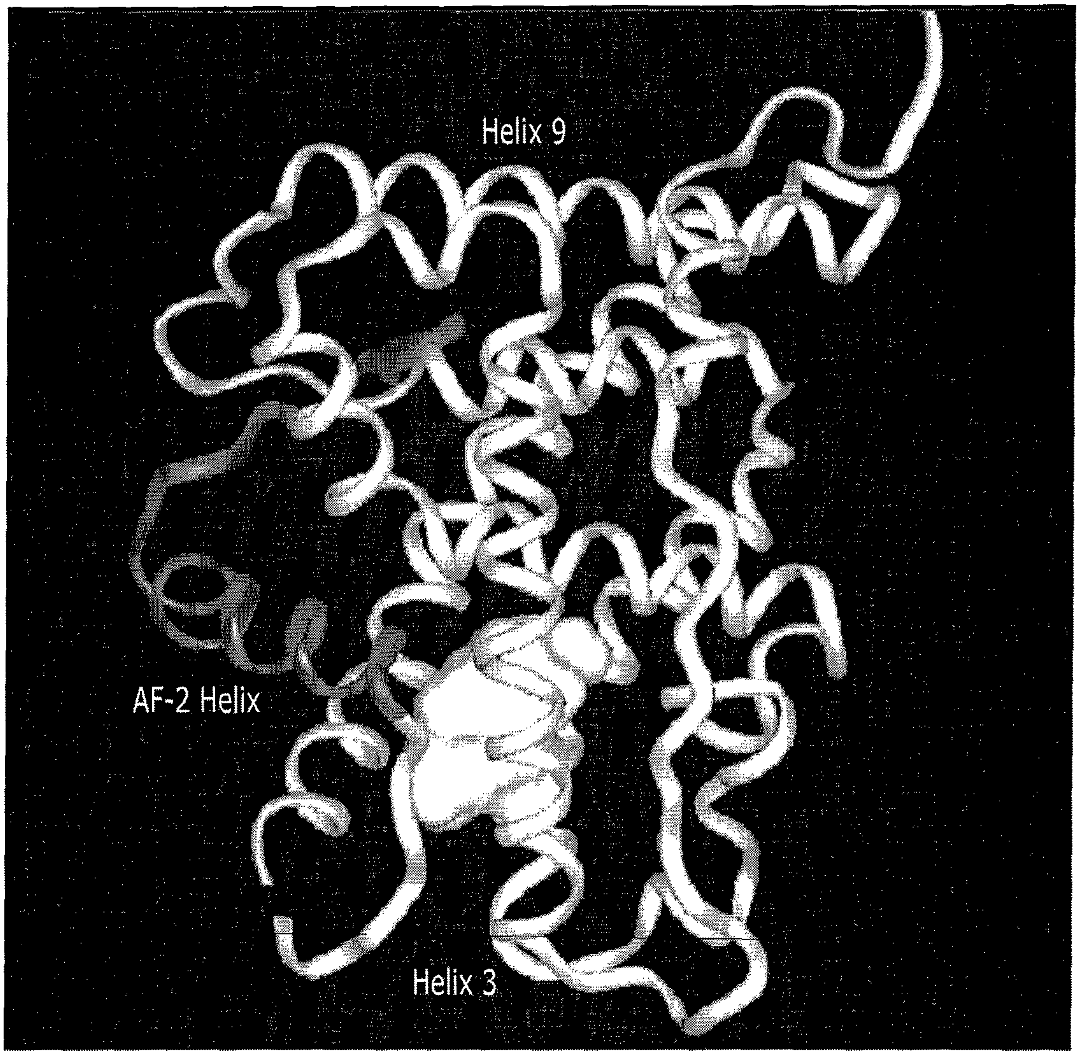


FIGURE 28A

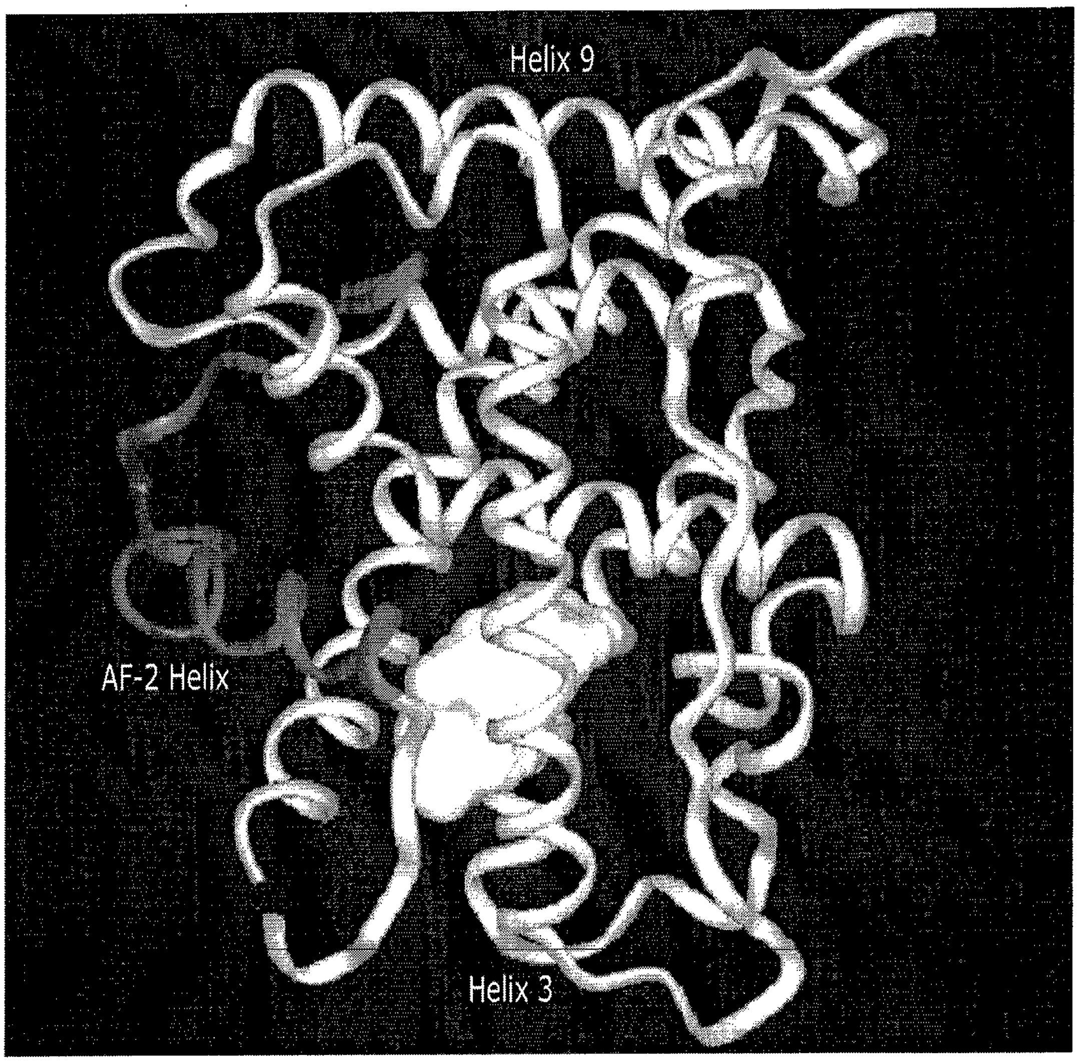


FIGURE 28B

European Patent Office

# EUROPEAN SEARCH REPORT

Application Number
EP 03 07 6899

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | HOLLENBERG S M ET AL: "PRIMARY STRUCTURE AND EXPRESSION OF A FUNCTIONAL HUMAN GLUCOCORTICOID RECEPTOR CDNA" NATURE, MACMILLAN JOURNALS LTD. LONDON, GB, vol. 318, 19 December 1985 (1985-12-19), pages 635-641, XP000611470 ISSN: 0028-0836 * the whole document * --- | 1-47 | C07K14/72 G06F19/00 |
| A | STEVENS R C: "High-throughput protein crystallization" CURRENT OPINION IN STRUCTURAL BIOLOGY, CURRENT BIOLOGY LTD., LONDON, GB, vol. 10, no. 5, October 2000 (2000-10), pages 558-563, XP002208529 ISSN: 0959-440X * the whole document * --- | 1-47 | |
| A | WILLIAMS SHAWN P ET AL: "Atomic structure of progesterone complexed with its receptor" NATURE, MACMILLAN JOURNALS LTD. LONDON, GB, vol. 393, no. 6683, 28 May 1998 (1998-05-28), pages 392-396, XP002173773 ISSN: 0028-0836 * the whole document * --- | 1-47 | TECHNICAL FIELDS SEARCHED (Int.Cl.7) C07K G06F |
| A | WO 02 36606 A (MATRIX THERAPEUTICS LTD ;MIEL HUGHES JEAN PIERRE (GB); RAY DAVID W) 10 May 2002 (2002-05-10) * examples 1-3 * --- -/-- | 1-47 | |

The present search report has been drawn up for all claims

| Place of search | Date of completion of the search | Examiner |
|---|---|---|
| MUNICH | 21 August 2003 | Petri, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
&: member of the same patent family, corresponding document

EPO FORM 1503 03.82 (P04C01)

European Patent Office

# EUROPEAN SEARCH REPORT

Application Number
EP 03 07 6899

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DEY R ET AL: "Homology modelling of the ligand-binding domain of glucocorticoid receptor: binding site interactions with cortisol and corticosterone" PROTEIN ENGINEERING, OXFORD UNIVERSITY PRESS, SURREY, GB, vol. 14, no. 8, 2001, pages 565-571, XP002964886 ISSN: 0269-2139 * the whole document * --- | 1-47 | |
| P,X | WO 03 015692 A (APOLITO CHRISTOPHER J ;LAMBERT MILLARD H III (US); SMITHKLINE BEEC) 27 February 2003 (2003-02-27) * the whole document * --- | 1-47 | |
| P,X | BLEDSOE R K ET AL: "Crystal structure of the glucocorticoid receptor ligand binding domain reveals a novel mode of receptor dimerization and coactivator recognition" CELL, CELL PRESS, CAMBRIDGE, NA, US, vol. 110, 12 July 2002 (2002-07-12), pages 93-105, XP002964887 ISSN: 0092-8674 * the whole document * --- | 1-47 | TECHNICAL FIELDS SEARCHED (Int.Cl.7) |
| A | WO 00 52050 A (GREENIDGE PAULETTE ;GILLNER MIKAEL (SE); KAROBIO AB (SE)) 8 September 2000 (2000-09-08) * the whole document * ----- | 1-47 | |

The present search report has been drawn up for all claims

| Place of search | Date of completion of the search | Examiner |
|---|---|---|
| MUNICH | 21 August 2003 | Petri, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 07 6899

This annex lists the patent family members relating to the patent documents cited in the above-mentioned European search report.
The members are as contained in the European Patent Office EDP file on
The European Patent Office is in no way liable for these particulars which are merely given for the purpose of information.

21-08-2003

| Patent document cited in search report | | Publication date | Patent family member(s) | | Publication date |
|---|---|---|---|---|---|
| WO 0236606 | A | 10-05-2002 | AU | 1076802 A | 15-05-2002 |
| | | | CA | 2427569 A1 | 10-05-2002 |
| | | | EP | 1335928 A1 | 20-08-2003 |
| | | | WO | 0236606 A1 | 10-05-2002 |
| WO 03015692 | A | 27-02-2003 | WO | 03015692 A2 | 27-02-2003 |
| WO 0052050 | A | 08-09-2000 | AU | 2818200 A | 21-09-2000 |
| | | | WO | 0052050 A2 | 08-09-2000 |

EPO FORM P0459

For more details about this annex : see Official Journal of the European Patent Office, No. 12/82